# EUROPEAN PATENT APPLICATION

(11) **EP 4 134 104 A1**
(43) Date of publication of application: **15.02.2023**
(21) Application number: 21784504.9
(22) Date of filing: 09.04.2021
(51) Int. Cl.: A61K 47/66, C07D 487/04, A61P 35/00

(54) **TARGETED PROTEASE DEGRADATION (TED) PLATFORM**

(30) Priority: 09.04.2020 CN 202010276301
(71) Applicant: Eubulus Biotherapeutics Inc., Jiaxing, Zhejiang 314000 (CN)
(72) Inventor: CAO, Sheldon, Jiaxing, Zhejiang 314000 (CN); WANG, Xiaolei, Jiaxing, Zhejiang 314000 (CN); HUANG, Chaoran, Jiaxing, Zhejiang 314000 (CN)
(74) Representative: Cabinet Laurent & Charras
(86) International application number: PCT/IB2021/052954
(87) International publication number: WO 2021/205391

(57) **Abstract**

The present invention relates to a targeted protease degradation (TED) platform, and specifically to a conjugate of target molecule-linker-E3 ligase ligand as shown in formula I, R_{T}-L1-R_{E3} (formula I), wherein R_{T} is a monovalent group of the target molecule, R_{E3} is a monovalent group of the E3 ligase ligand, L1 is the linker linking A and B, and L1 is as shown in formula II below:-W-L2-W²-(II).

## Description

### TECHNICAL FIELD

The present invention belongs to biomedicine, specifically, relates to a Targeted Protease Degradation (TED) platform.

### BACKGROUND

Expression level of proteins is regulated on three basic levels according to modern molecular biology. Firstly, at the DNA level, the target protein DNA is inactivated through gene knock-out. Secondly, it binds to the mRNA of the target protein through small molecule RNA, thereby inhibiting the translation and expression of mRNAi. Thirdly, at the protein level, the amount and activity of the target protein can be regulated by modificating of the target protein after translation, such as methylation, phosphorylation, glycosylation, etc.

In terms of the overall development of drug research and development, both small molecule and macromolecule drug forms have their own advantages and disadvantages. For example, the development of small molecule drugs has been facing crucial challenges such as how to maintain drug concentration in the body and drug resistance. The shapes of some target sites are adverse to design of small molecule drugs, thus becoming non-drugable targets. For these targets, no effective regulatory methods have yet been found. Although, compared to small molecules, monoclonal antibodies have the advantages of high affinity and high selectivity, and easy to develop highly effective and highly selective drugs, but the biggest drawback thereof is that they cannot penetrate cell membranes and therefore cannot act on intracellular targets. Antibody-drug conjugates (ADC) utilize endocytic antibodies to provide targeting and serve as carriers to deliver super toxin drugs to the targeted site. The bottleneck encountered in the development of ADC drugs is that the treatment window is not wide enough. In addition to the side effects caused by the antibody itself, the super toxins will fall off before reaching the targeting site due to the heterogeneity of coupling, and causing serious side effects. In addition, normal physiological function of ubiquitin- proteasome system is responsible for cleaning up denatured, mutated or harmful proteins in cells.

Summary, there is an urgent need in the art to develop a compound that is able to degrade target proteins more efficiently and re-usably so as to treat related diseases.

### SUMMARY OF THE INVENTION

The purpose of the present application is to provide a compound that is able to degrade target proteins more efficiently and re-usably so as to treat related diseases.

In the first aspect of the present invention, provided is a conjugate of formula I, and the pharmaceutically acceptable salts thereof, wherein

R_{T}-L1-R_{E3} (I)

wherein
(a) the R_{E3} is a moiety of E3 Ligase Ligand;
(b) the R_{T} is a moiety of target molecule;
(c) the L1 is a linker connecting the moieties of R_{E3} and R_{T}, and L1 is shown in formula II;

   -W¹-L2-W²- (II)

   wherein
   W¹ and W² are each independently-(W)ₛ-;
   W is each independently selected from the group consisting of null, -C(R^{b})₂-, -O-, -S-, - N(R^{a})-, -C(=O)-, -SOz-, -SO-, -PO₃-, -C(R^{b})=C(R^{b})-, -C=C-, NR, substituted or unsubstituted C3-8 cycloalkyl, substituted or unsubstituted 4 to 10 membered heterocycloalkyl, substituted or unsubstituted C6-10 aryl, and substituted or unsubstituted 5 to 10 membered heteroaryl;
   s = 0, 1, 2, 3, or 4;
   L2 is shown in formula III,

   -(M^{L})ₒ- (III)

   wherein
   M^{L} is each independently M, M^{T} or M^{N};
   wherein,
   o is an integer of 5 to 50;
   M is each independently divalent group selected from the group consisting of -C(R^{b})₂-, - O-, -S-, -N(R^{a})-, -C(=O)-, -SOz-, -SO-, -PO₃-, -C(R^{b})=C(R^{b})-, -C=C-, substituted or unsubstituted C₃₋₈ cycloalkyl, substituted or unsubstituted 4 to 10 membered heterocycloalkyl, substituted or unsubstituted C₆₋₁₀ aryl, substituted or unsubstituted 5 to 10 membered heteroaryl, and amino acid residue;
   M^{N} is each independently divalent group selected from the group consisting of -N(R')-, - N(4 to 10 membered heterocycloalkyl containing N(R') as ring atom)-, 4 to 10 membered heterocycloalkyl containing N(R') as ring atom, -C(R^{b})₂- substituted with at least one -N(R^{b})R' (preferably, -NHR'), C₃₋₈ cycloalkyl, 4 to 10 membered heterocycloalkyl, C₆₋₁₀ aryl, and 5 to 10 membered heteroaryl;
   M^{T} is each independently divalent group selected from the group consisting of -N(R")-, - N(4 to 10 membered heterocycloalkyl containing N(R") as ring atom)-, 4 to 10 membered heterocycloalkyl containing N(R") as ring atom, -C(R^{b})₂- substituted with at least one - N(R^{b})R" (preferably, -NHR"), C₃₋₈ cycloalkyl, 4 to 10 membered heterocycloalkyl, C₆₋₁₀ aryl, and 5 to 10 membered heteroaryl;
   RisR' or R";
   R' is each independently selected from the group consisting of H, C₁₋₆ alkyl, OH, SH, - COO-C₁₋₆ alkyl, -OC(O)-C₁₋₆alkyl, and amino protecting group;
   R" is -W³-L3-W⁴-(R_{P})_{q};
   W³ and W⁴ are each independently -(W)ₛ-; and the definitions of W and s are the same as definitions used in W¹ and W²;
   L3 is a divalent linker group;
   R_{P} is a polypeptide element or target molecule T;
   q is >0 (preferably, m is 0.1 to 10, more preferably, 0.2 to 5);
   R^{a} is each independently selected from the group consisting of H, OH, SH, substituted or unsubstituted C₁₋₆ alkyl, amino protecting group, 4 to 10 membered heterocycloalkyl containing N(R^{c}) as ring atom;
   R^{b} is each independently selected from the group consisting of H, halogen, OH, SH, substituted or unsubstituted C₁₋₆ alkyl, substituted or unsubstituted C₂₋₆ alkenyl, substituted or unsubstituted C₂₋₆ alkynyl, substituted or unsubstituted C₁₋₆ alkoxy, substituted or unsubstituted C₁₋₆ alkanoyl (-C(O)-C₁₋₆ alkyl), carboxyl, -COO-C₁₋₆ alkyl, -OC(O)-C₁₋₆alkyl; or, two R^{b} on the same atom together with the carbon to which they are attached form substituted or unsubstituted C₃₋₈ cycloalkyl, substituted or unsubstituted 4 to 10 membered heterocycloalkyl.
   R^{c} is each independently selected from the group consisting of H, OH, SH, substituted or unsubstituted C₁₋₆ alkyl, and amino protecting group;
   unless otherwise specified, the substituted means that one or more (such 1, 2, or 3) hydrogen atoms in the group are substituted with substituents selected from the group consisting of halogen (preferably, F, Cl, Br or I), cyano(CN), oxo (=O), thio (=S), C₁₋₆ alkyl, C₁₋₆ haloalkyl, C₂₋₆ alkenyl, C₂₋₆ alkynyl, C₁₋₆ alkoxy, C₁₋₆ alkanoyl (C₁₋₆ alkyl-C(O)-), -COO-C₁₋₆ alkyl, -OC(O)-C₁₋₆ alkyl, NH₂, NH(C₁₋₆ alkyl), and N(C₁₋₆ alkyl)₂.

In another preferred embodiment, W is not NR.

In another preferred embodiment, there is no -O-O- in L2.

In another preferred embodiment, in L2, at least one of M^{L} is M^{T} or M^{N}.

In another preferred embodiment, in L2, all of M^{L} is M.

In another preferred embodiment, in L2, when two or more of M^{L} are M^{T} or M^{N}, L2 comprises M^{T} and M^{N}, or L2 comprises only M^{T}, or L2 only comprises M^{N}.

In another preferred embodiment, in L2, at least one of M^{L} is M^{N}.

In another preferred embodiment, in L2, at least one of M^{L} is M^{T}.

In another preferred embodiment, in L2, 1, 2 or 3 of M^{L} are each independently M^{T} or M^{N}.

In another preferred embodiment, in L2, 1, 2 or 3 of M^{L} are each independently M^{N}.

In another preferred embodiment, in L2, 1, 2 or 3 of M^{L} are each independently M^{T}.

In another preferred embodiment, L2 is L5, and L5 is shown in formula IIIc;

-(M)ₒ₁-(M')-(M)ₒ₂- (IIIc)

wherein
M' is each independently M^{T} or M^{N};
M, M^{T} and M^{N} are as defined in formula I;
o1 and o2 are each independently integers of 1 to 50, and 4≤o1+o2≤49.

In another preferred embodiment, L2 is L6, and L6 is shown in formula IIIa;

-(M)ₒ₁-(M^{N})-(M)ₒ₂- (IIIa)

wherein
M, and M^{N} are defined as above;
o1 and o2 are each independently integers of 1 to 50, and 4≤o1+o2≤49.

In another preferred embodiment, o1 and o2 are each independently 1, 2, 3, 4, 5, 6, 7 or 8.

In another preferred embodiment, in L6, M is each independently selected from the group consisting of -CH₂-, -CH(C₁₋₄ alkyl)-, -CH(NH₂)-, -O- -NH-, -N(C₁₋₄ alkyl)-, 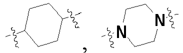 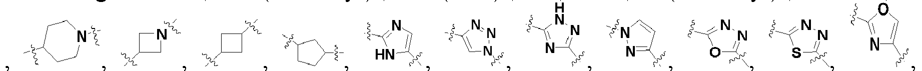 and

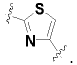

In another preferred embodiment, the conjugate is shown in formula IV;

R_{T}-W¹-L6-W²-R_{E3} (IV)

wherein, L6, W¹, W², R_{T} and R_{E3} are defined as in formula I.

In another preferred embodiment, L2 is L7, and L7 is shown informula IIIb;

-(M)ₒ₁-(M^{T})-(M)ₒ₂- (IIIb)

wherein M, and M^{T} are defined as above;
o1 and o2 are each independently integers of 1 to 50, and 4≤o1+o2≤49.

In another preferred embodiment, o1 and o2 are each independently 1, 2, 3, 4, 5, 6, 7 or 8.

In another preferred embodiment, the conjugate is shown in formula V;

R_{T}-W¹-L7-W²-R_{E3} (V);

wherein L7, W¹, W², R_{T} and R_{E3} are defined in formula I.

In another preferred embodiment, the conjugate is shown in formula 1-1, 1-2, 1-3, 2 or 3;

R_{T}-W¹-L5-W^{b}-C≡C-R_{E3} (1-1);

R_{T}-W¹-L5-CO-R_{E3} (1-2);

R_{T}-W¹-L5-CONH-R_{E3} (1-3);

R_{T}-W^{a}-Cr¹-W^{a}-Cr²-L5-W²-R_{E3} (2)

R_{T}-Ar1-L5-W²-R_{E3} (3)

wherein
Ar1 is -5 or 6 membered heteroaryl containing nitrogen atom-;
Cr¹ is null, or C₄₋₇ cycloalkyl unsubstituted or substituted with C₁₋₄ alkyl, or 4 to 6 membered heterocyclyl unsubstituted or substituted with C₁₋₄ alkyl;
Cr² is 4 to 6 membered heterocyclyl containing nitrogen unsubstituted or substituted with C₁₋₄ alkyl, and at least one of nitrogen heteroatom in Cr² is attached with L5;
the definitions of W^{a} and W^{b} are the same as W; and W, W¹, W², R_{T}, R_{E3} and L5 are defined as above.

In another preferred embodiment, the conjugate is shown in formula 1a-1, 1a-2, 1a-3, 2a or 3a;

R_{T}-W¹-L6-W^{b}-C≡C-R_{E3} (1a-1);

R_{T}-W¹-L6-CO-R_{E3} (1a-2);

R_{T}-W¹-L6-CONH-R_{E3} (1a-3);

R_{T}-W^{a}-Cr¹-W^{a}-Cr²-L6-W²-R_{E3} (2a)

R_{T}-Ar1-L6-W²-R_{E3} (3a)

wherein
Ar1, Cr¹, Cr², W^{a}, W^{b}, W¹, W², R_{T}, R_{E3} and L6 are defined as above.

In another preferred embodiment, the conjugate is shown in formula 1b-1, 1b-2, 1b-3, 2b or 3b;

R_{T}-W¹-L7-W^{b}-C≡C-R_{E3} (1b-1);

R_{T}-W¹-L7-CO-R_{E3} (1b-2);

R_{T}-W¹-L7-CONH-R_{E3} (1b-3);

R_{T}-W^{a}-Cr¹-W^{a}-Cr²-L7-W²-R_{E3} (2b)

R_{T}-Ar1-L7-W²-R_{E3} (3b)

wherein
Ar1 is 5 or 6 membered heteroaryl containing nitrogen atom;
Cr¹ is null, or C₄₋₇ cycloalkyl unsubstituted or substituted with C₁₋₄ alkyl, or 4 to 6 membered heterocyclyl unsubstituted or substituted with C₁₋₄ alkyl;
Cr² is 4 to 6 membered heterocyclyl containing nitrogen that is unsubstituted or substituted with C₁₋₄ alkyl, and at least one of nitrogen heteroatom in Cr² is attached with L7;
the definitions of W^{a} and W^{b} are the same as W; and W, W¹, W², R_{T}, R_{E3} and L7 are defined as above.

In another preferred embodiment, L2 is L8, and L8 is shown in formula IIId;

-(M)ₒ₃- (IIId)

wherein M is defined as above (preferably, M is CH₂), o3 is 1, 2, 3, 4 or 5.

In another preferred embodiment, the conjugate is shown inR_{T}-W¹-L8-W²-R_{E3}; wherein R_{T}, W¹, L8, W², and R_{E3} are defined as above. Preferably, W¹ is W^{a}-Cr¹-Cr² (more preferably, is NH-Cr¹-Cr²), Cr¹ and Cr² are defined as above.

In another preferred embodiment, when the heterocycloalkyl (such as 4 to 10 membered heterocycloalkyl) is a divalent group, the 4 to 10 membered heterocycloalkyl includes 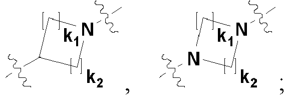 wherein k1 and k2 are each independently 0, 1, 2 or 3; preferably, the 4 to 10 membered heterocycloalkyl is selected from the group consisting of

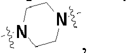

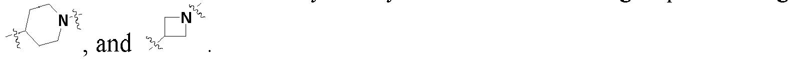

In another preferred embodiment, when the cycloalkyl (such as C₃₋₈ cycloalkyl) is a divalent group, the cycloalkyl (such as C₃₋₈ cycloalkyl) includes 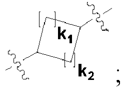 wherein k1 and k2 are each independently 1, 2 or 3; preferably, the C₃₋₈ cycloalkyl is selected from the group consisting of

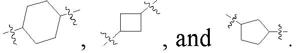

In another preferred embodiment, when the heteroaryl (such as 5 to 10 membered heteroaryl) is a divalent group, the heteroaryl such as 5 to 10 membered heteroaryl) is 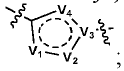 wherein V₁, V₂ and V₄ are each independently selected from the group consisting of -O-, -S-, -N=, -NH-, -CH=, and -CH₂-; V₃ is selected from the group consisting of -N=, and -CH=; preferably, the 5 to 10 membered heteroaryl is selected from the group consisting of

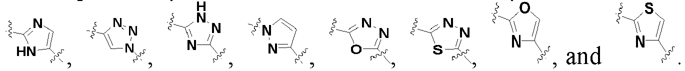

In another preferred embodiment, M is each independently selected from the group consisting of -CH₂-, -CH(C₁₋₄ alkyl)-, -CH(NH₂)-, -O-, -NH-, -N(C₁₋₄ alkyl)-,

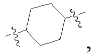

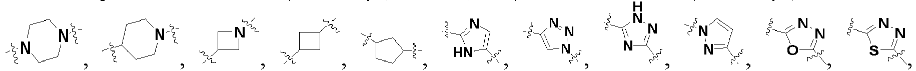

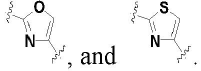

In another preferred embodiment, when the 4 to 10 membered heterocycloalkyl containing N(R) as ring atom is a divalent group, the 4 to 10 membered heterocycloalkyl containing N(R) as ring atom is selected from the group consisting of 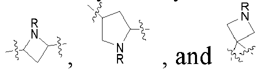 ; wherein, R is R' or R".

In another preferred embodiment, M^{T} is each independently selected from the group consisting of -N(R")-, -C(R^{b})(NHR")-,

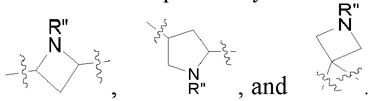

In another preferred embodiment, M^{N} is each independently selected from the group consisting of N(R')-, -C(R^{b})(NHR')-,

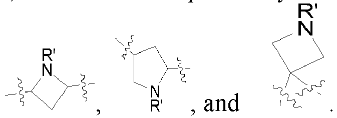

In another preferred embodiment, M is each independently selected from the group consisting of O, and C(R^{b})₂; preferably, wherein R^{b} is each independently H or C₁₋₆ alkyl (such as methyl).

In another preferred embodiment, W is selected from the group consisting of null, - C(R^{b})₂-, -O- -S-, -N(R^{a})-, -C(=O)-, -SO₂-, -SO-, -PO₃-, -C(R^{b})=C(R^{b})-, -C≡C- or W is substituted or unsubstituted group selected from the group consisting of 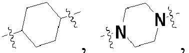 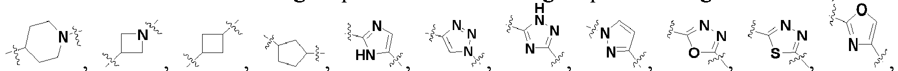 and

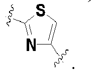

In another preferred embodiment, R^{a} is each independently H or C₁₋₆ alkyl (such as methyl).

In another preferred embodiment, R^{b} is each independently H or C₁₋₆ alkyl (such as methyl).

In another preferred embodiment, R^{c} is each independently H or C₁₋₆ alkyl (such as methyl).

In another preferred embodiment, L3 is -(M^{a})ₚ-; wherein M^{a} is defined as M, p is an integer of 1 to 50.

In another preferred embodiment, p=1, 2, 3, 4, 5, 6, 7, 8, 9, 10, 11, 12, 13, 14, or 15.

In another preferred embodiment, M^{a} is each independently the divalent group selected from the group consisting of -C(R^{b})₂-, -O-, -S-, -N(R^{a})-, -C(=O)-, -SOz-, -SO-, -PO₃-, - C(R^{b})=C(R^{b})-, -C=C-, substituted or unsubstituted -C3-8 cycloalkyl-, substituted or unsubstituted -4 to 10 membered heterocycloalkyl, substituted or unsubstituted -C6-10 aryl, substituted or unsubstituted 5 to 10 membered heteroaryl, and amino acid residue.

In another preferred embodiment, -W³-L3-W⁴-R_{P} is selected from the group consisting of 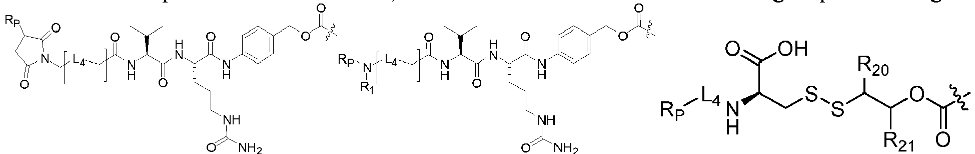 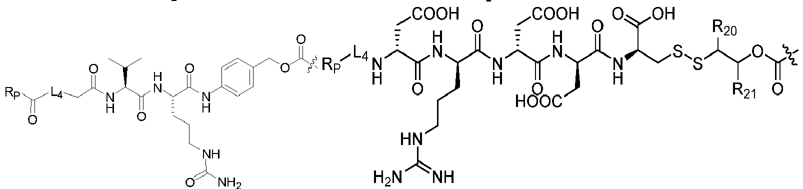 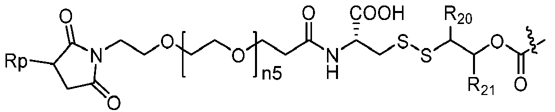 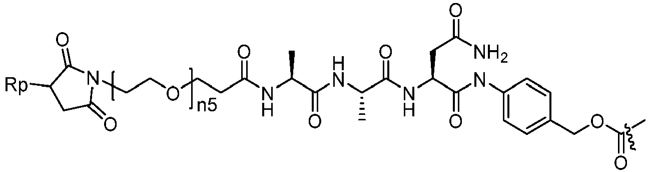
wherein L4 is -(M)_{q}-, wherein M is defined as in L2;
q is an integer of 0 to 50 and q is less than p (preferably, q = an integer of 0 to 30; more preferably, q= 0, 1, 2, 3, 4, 5, 6, 7, 8, 9, or 10), n5 is an integer of 0 to 30 (preferably, n5=0, 1, 2, 3, 4, 5, 6, 7, 8, 9, or 10); R₂₀ and R₂₁ are each independently selected from the group consisting of -H, -Me, -Et, -nPr, iPro, and cPro.

In another preferred embodiment, the conjugate is not those specific compounds disclosed in PCT/CN2019/110225.

In another preferred embodiment, the conjugate is not those specific compounds disclosed in Table B1-11 in PCT/CN2019/110225, and the specific compounds described in Table B1-11 are as follows:

| **Example** | **Compound structure** | **Example** | **Compound structure** | **Example** | **Compound structure** |
|---|---|---|---|---|---|
| **UB-180501** | 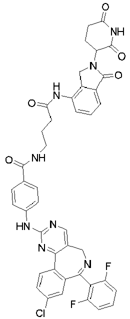 | **UB-180505** | 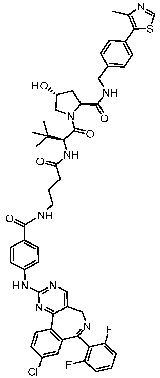 | **UB-180572** | 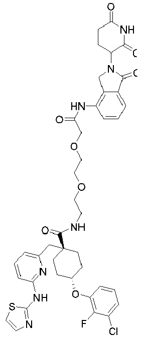 |
| **UB-180502** | 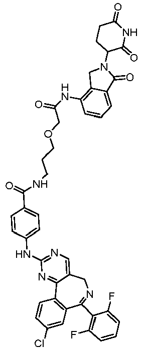 | **UB-180506** | 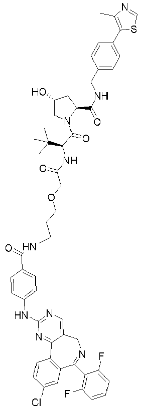 | **UB-180573** | 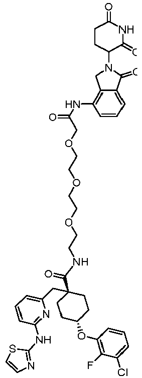 |
| **UB-180503** | 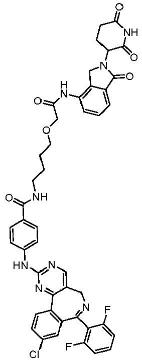 | **UB-180514** | 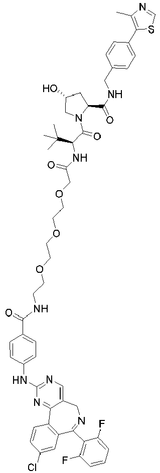 | **UB-180574** | 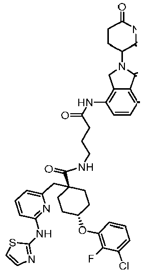 |
| **UB-180504** | 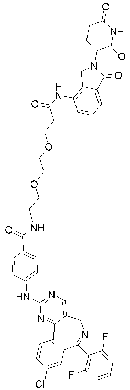 | **UB-180527** | 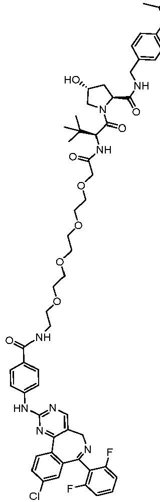 | **UB-180575** | 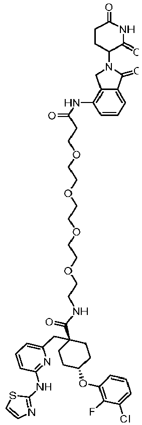 |
| **UB-180509** | 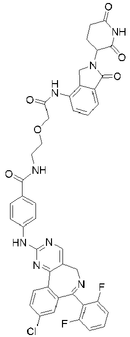 | **UB-180531** | 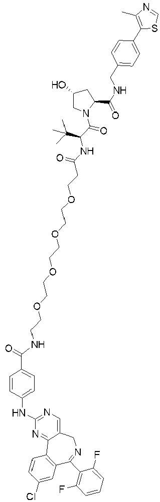 | **UB-180576** | 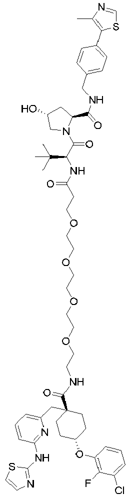 |
| **UB-180520** | 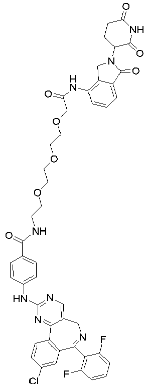 | **UB-180532** | 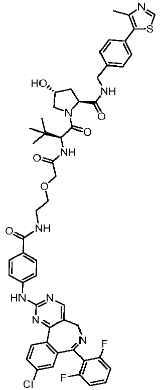 | **UB-180577** | 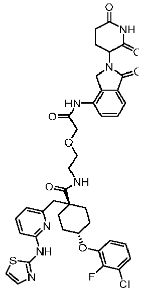 |
| **UB-180530** | 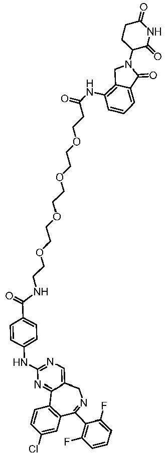 | **UB-180533** | 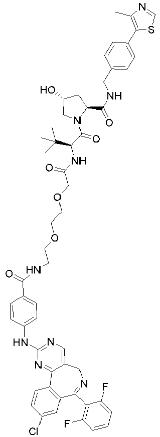 | **UB-180581** | 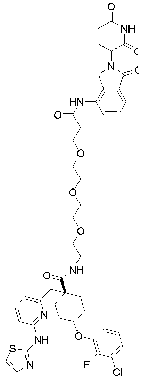 |
| **UB-180534** | 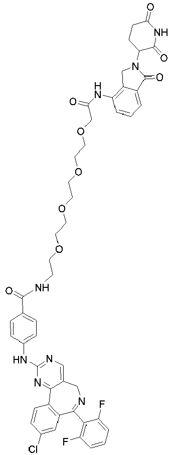 | **UB-180536** | 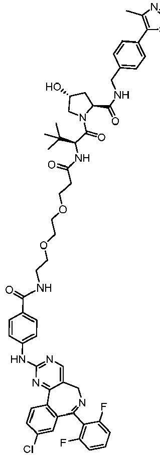 | **UB-180582** | 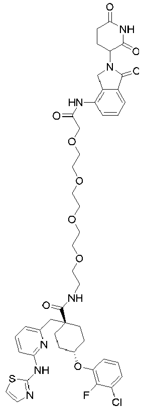 |
| **UB-180535** | 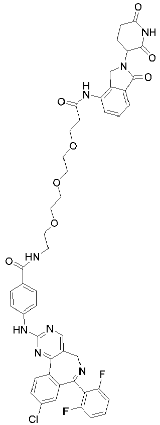 | **UB-180558** | 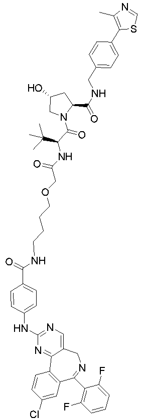 | **UB-180590** | 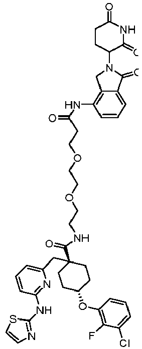 |
| **UB-180559** | 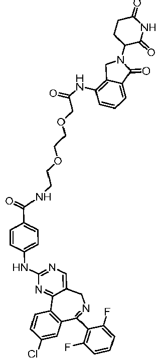 | | **UB-180586** | 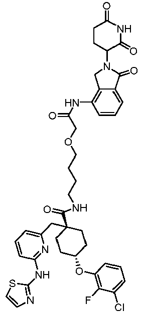 | 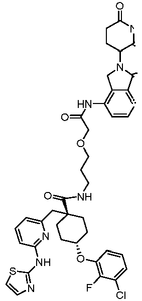 |

| **Example** | **Compound structure** | **Example** | **Compound structure** | **Example** | **Compound structure** |
|---|---|---|---|---|---|
| **UB-180583** | 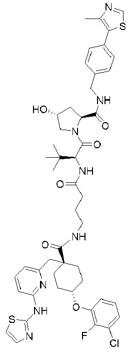 | **UB-180512** | 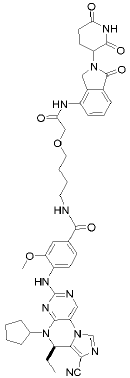 | **UB-180519** | 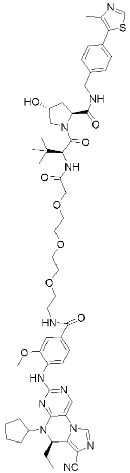 |
| **UB-180584** | 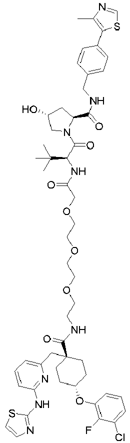 | **UB-180518** | 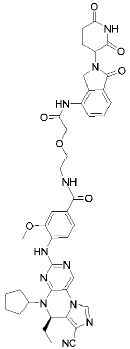 | **UB-180525** | 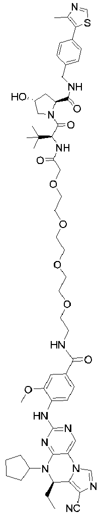 |
| **UB-180585** | 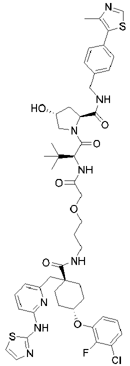 | **UB-180537** | 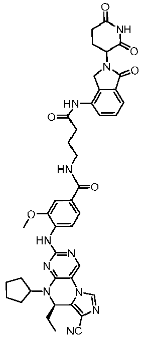 | **UB-180529** | 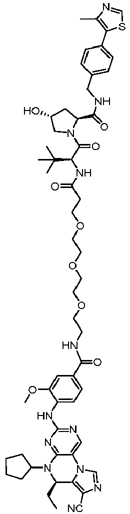 |
| **UB-180587** | 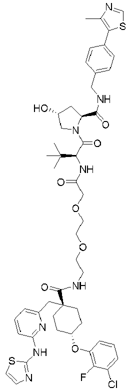 | **UB-180538** | 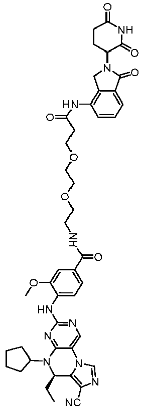 | **UB-180542** | 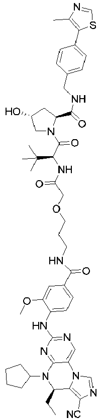 |
| **UB-180589** | 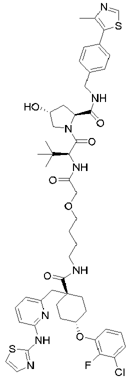 | **UB-180545** | 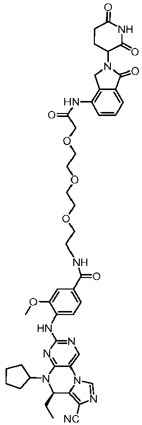 | **UB-180543** | 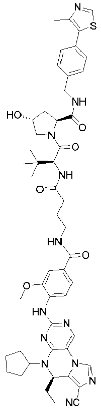 |
| **UB-180599 (199)** | 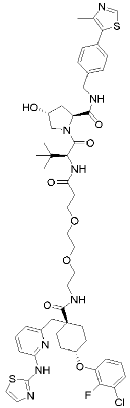 | **UB-180548** | 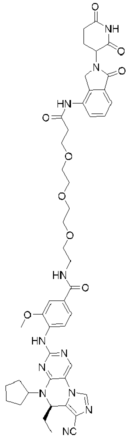 | **UB-180544** | 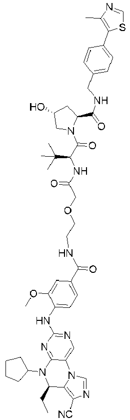 |
| **UB-180576** | 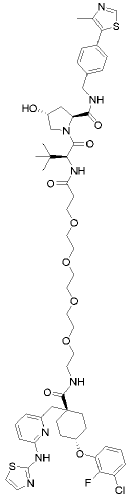 | **UB-180551** | 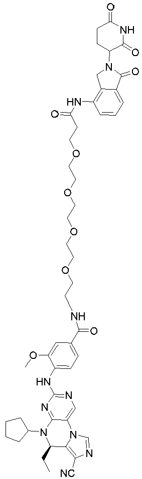 | **UB-180552** | 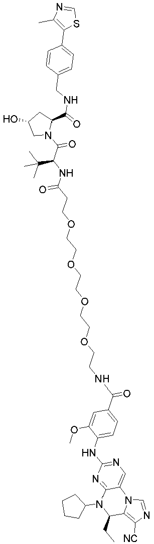 |
| **UB-180578** | 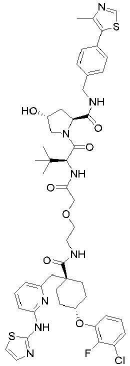 | **UB-180557** | 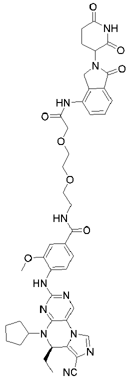 | **UB-180554** | 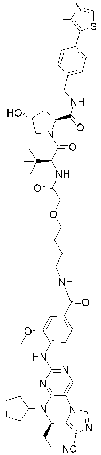 |
| **UB-180580** | 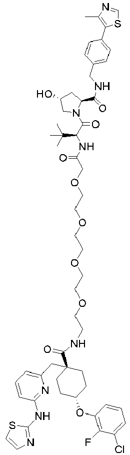 | **UB-180560** | 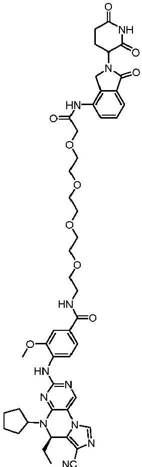 | **UB-180563** | 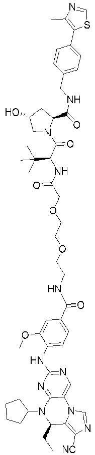 |

| **Example** | **Structural data analysis** |
|---|---|
| **UB-180600 (100)** | 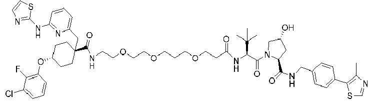 |
| **UB-180609 (109)** | 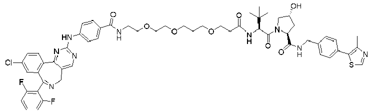 |
| **UB-180610 (110)** | 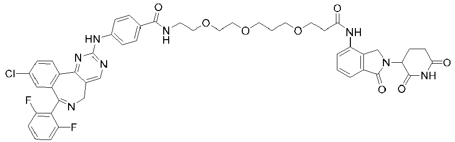 |
| **UB-180611 (111)** | 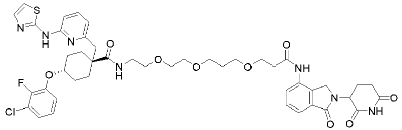 |
| **UB-180612 (112)** | 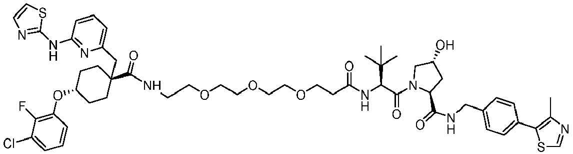 |
| **UB-180613 (113)** | 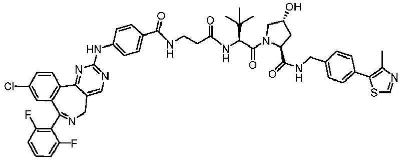 |
| **UB-180614 (114)** | 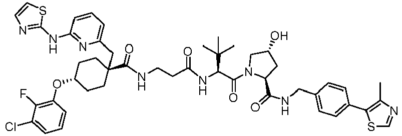 |
| **UB-180615 (115)** | 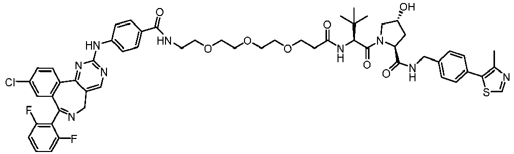 |
| **UB-180616 (116)** | 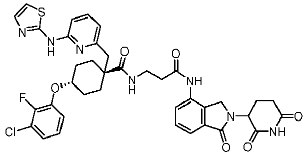 |
| **UB-180617 (117)** | 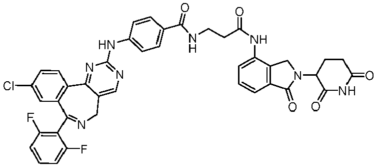 |
| **UB-180726 (226)** | 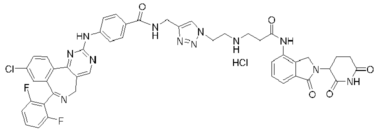 |
| **UB-180727 (227)** | 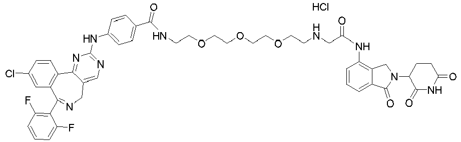 |
| **UB-180728 (228)** | 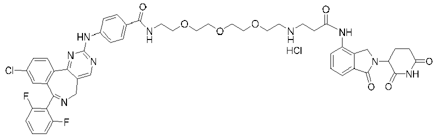 |
| **UB-180729 (229)** | 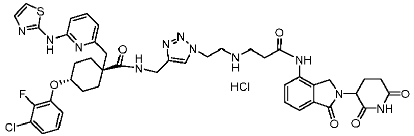 |
| **UB-180730 (230)** | 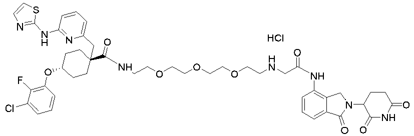 |
| **UB-180731 (231)** | 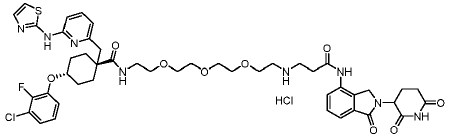 |
| **UB-180732 (232)** | 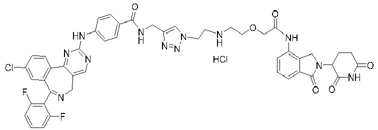 |
| **UB-180735 (235)** | 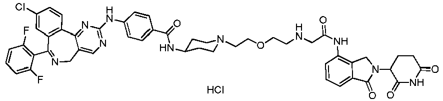 |
| **UB-180736 (236)** | 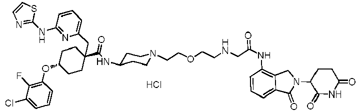 |
| **UB-180738 (238)** | 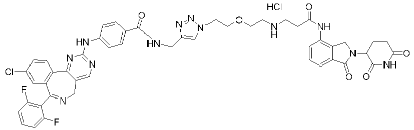 |
| **UB-180739 (239)** | 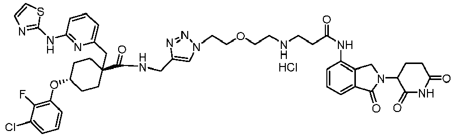 |
| **UB-180740 (240)** | 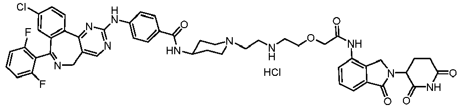 |
| **UB-180741 (241)** | 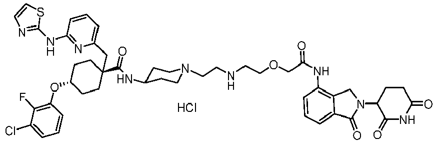 |
| **UB-180742 (242)** | 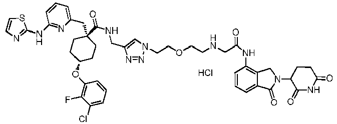 |
| **UB-180743 (243)** | 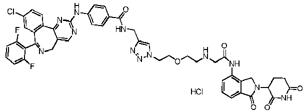 |
| **UB-180744 (244)** | 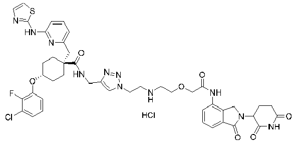 |
| **UB-180748 (248)** | 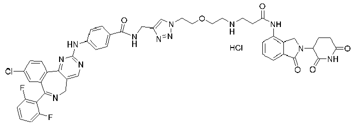 |
| **UB-180749 (249)** | 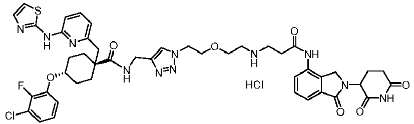 |
| **UB-180761 (261)** | 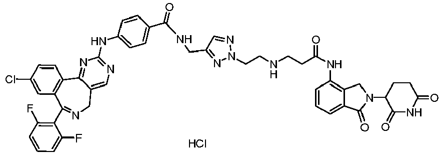 |
| **UB-180768 (268)** | 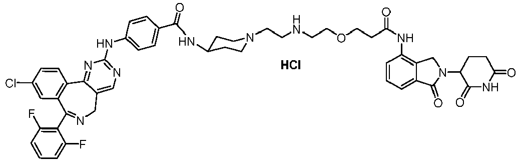 |
| **UB-180769 (269)** | 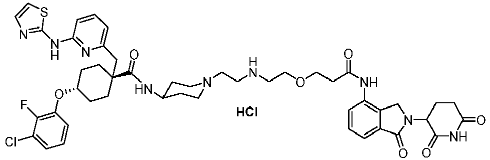 |
| **UB-180770 (270)** | 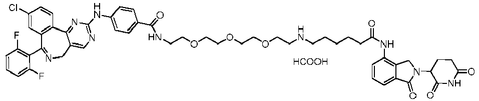 |
| **UB-180771 (271)** | 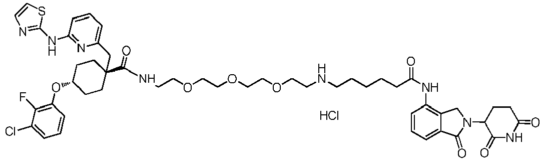 |
| **UB-180772 (272)** | 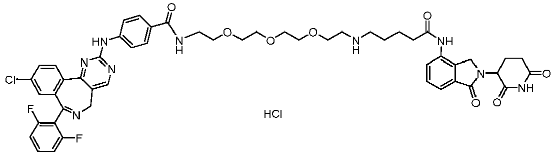 |
| **UB-180773 (273)** | 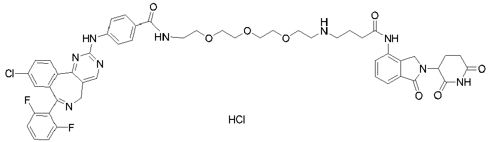 |
| **UB-180774 (274)** | 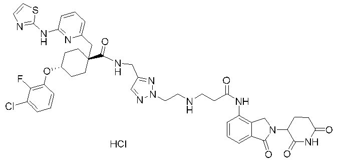 |
| **UB-180775 (275)** | 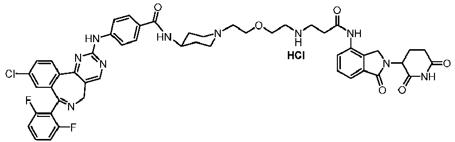 |
| **UB-180776 (276)** | 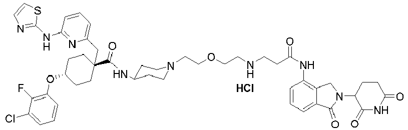 |
| **UB-180777 (277)** | 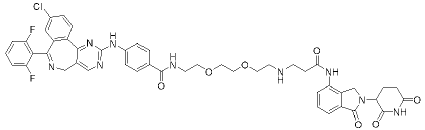 |
| **UB-180778 (278)** | 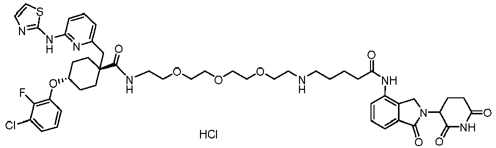 |
| **UB-180779 (279)** | 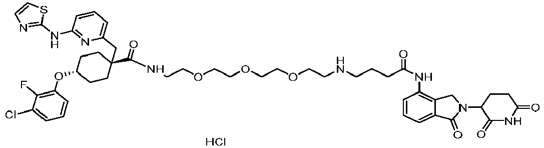 |
| **UB-180780 (280)** | 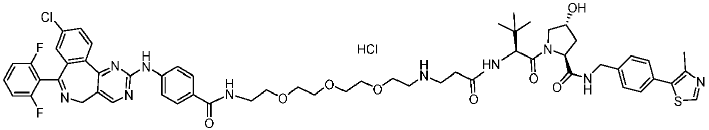 |
| **UB-180781 (281)** | 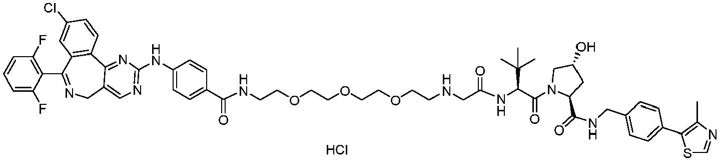 |
| **UB-180782 (282)** | 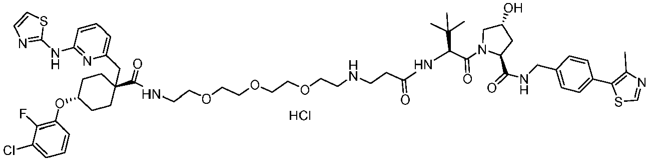 |
| **UB-180783 (283)** | 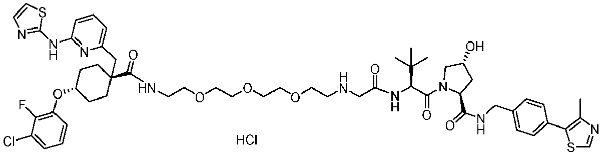 |
| **UB-180784 (284)** | 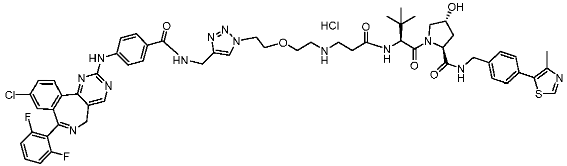 |
| **UB-180785 (285)** | 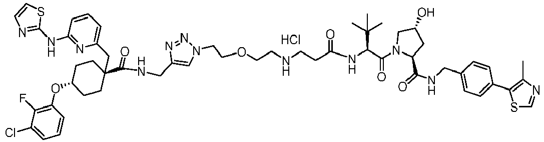 |
| **UB-180786 (286)** | 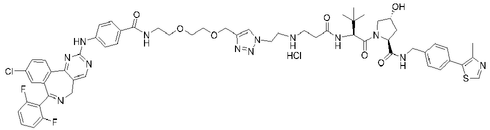 |
| **UB-180787 (287)** | 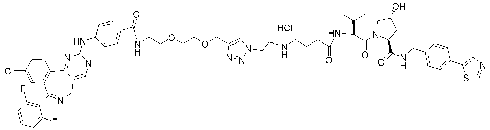 |
| **UB-180788 (288)** | 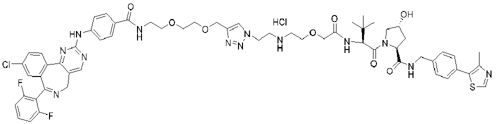 |
| **UB-180789 (289)** | 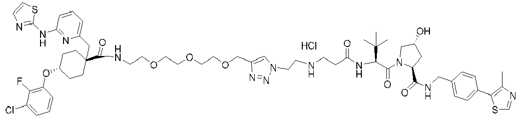 |
| **UB-180790 (290)** | 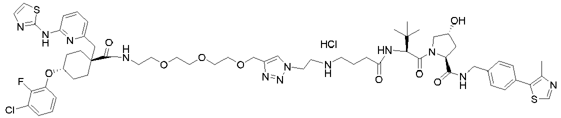 |
| **UB-180791 (291)** | 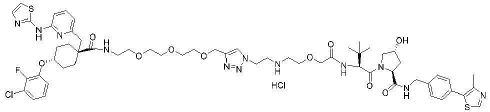 |
| **UB-180792 (292)** | 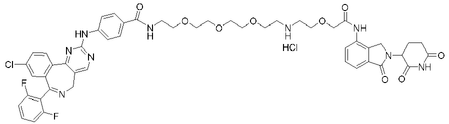 |
| **UB-180793 (293)** | 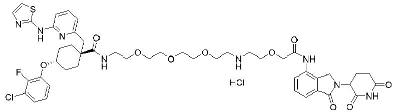 |
| **UB-180794 (294)** | 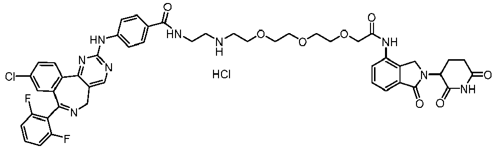 |
| **UB-180795 (295)** | 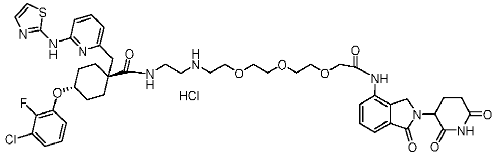 |
| **UB-180796 (296)** | 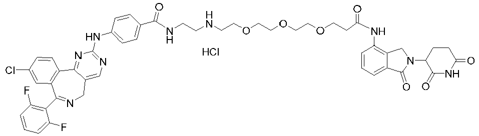 |
| **UB-180797 (297)** | 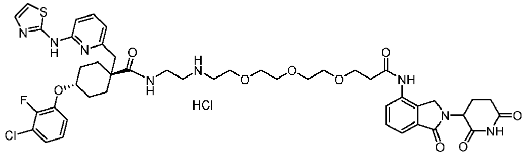 |
| **UB-180798 (298)** | 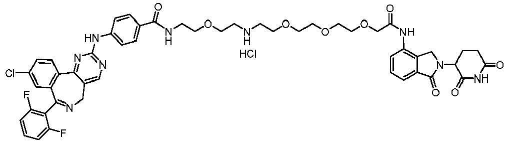 |
| **UB-180799 (299)** | 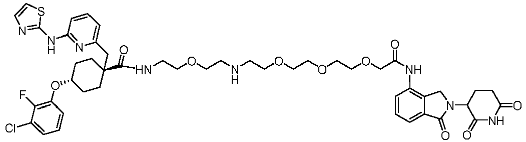 |
| **UB-180801 (301)** | 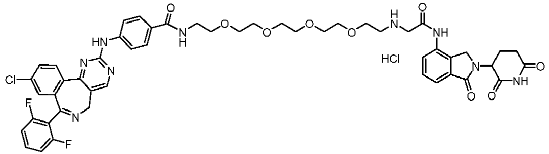 |
| **UB-180802 (302)** | 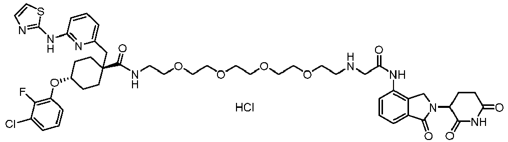 |
| **UB-180803 (303)** | 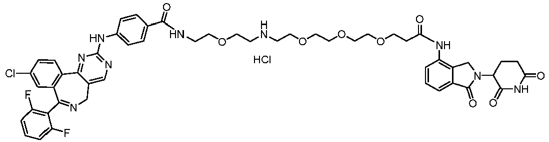 |
| **UB-180804 (304)** | 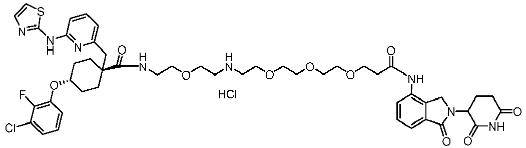 |
| **UB-180807 (307)** | 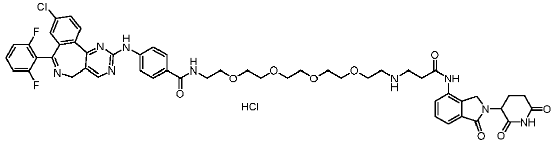 |
| **UB-180808 (308)** | 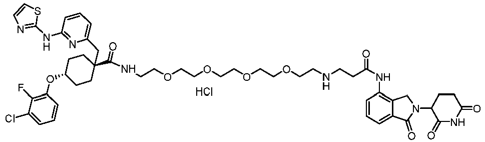 |

| **Example** | **Compound structure** | **Example** | **Compound structure** | |
|---|---|---|---|---|
| **UB-180510** | 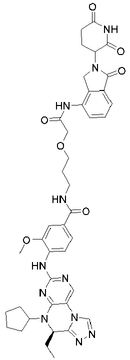 | **UB-180516** | 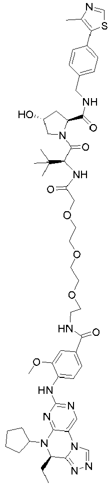 | |
| **UB-180511** | 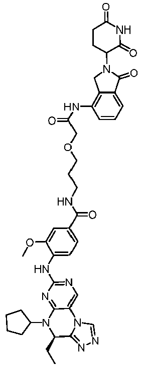 | **UB-180522** | 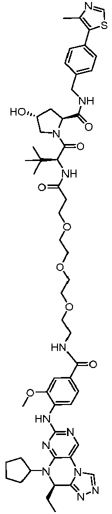 | |
| **UB-180521** | 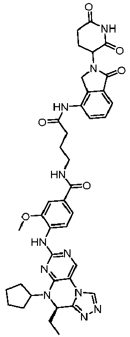 | **UB-180523** | 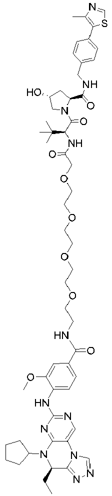 | |
| **UB-180524** | 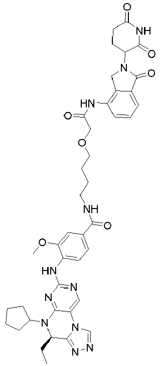 | **UB-180539** | 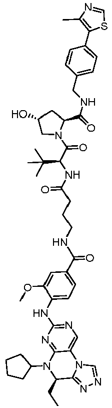 | |
| **UB-180546** | 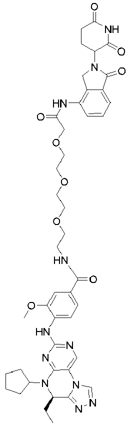 | **UB-180540** | 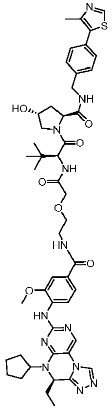 | |
| **UB-180547** | 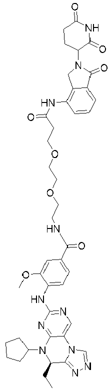 | **UB-180541** | 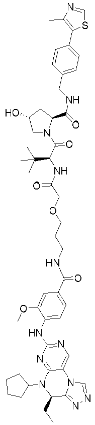 | |
| **UB-180549** | 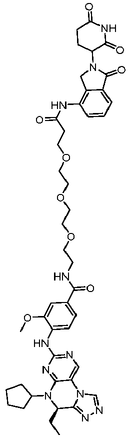 | **UB-180550** | 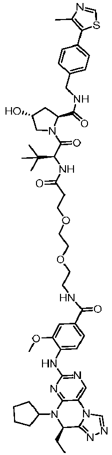 | |
| **UB-180556** | 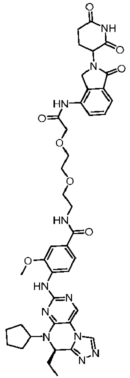 | **UB-180553** | 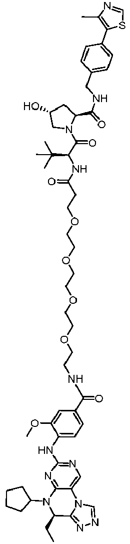 | |
| **UB-180561** | 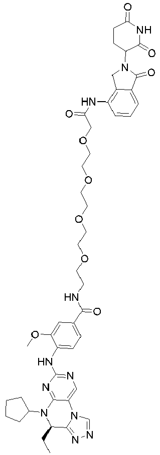 | **UB-180555** | 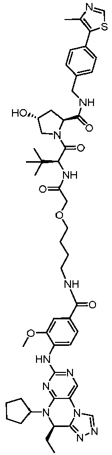 | |
| **UB-180562** | 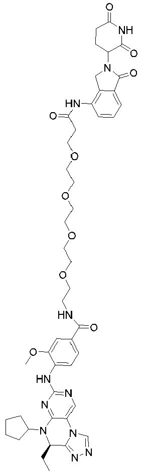 | **UB-180564** | 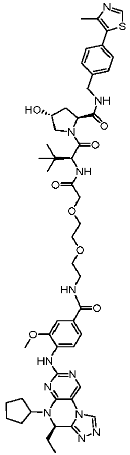 | |

| **Example** | **Structural data analysis** |
|---|---|
| **UB-180551 (51)** | 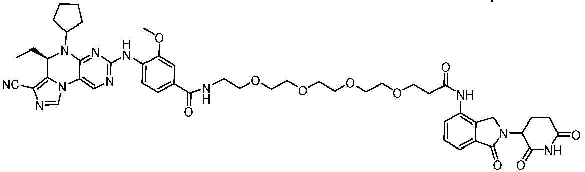 |
| **UB-180552 (52)** | 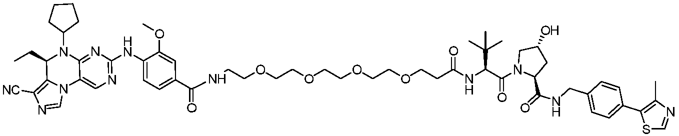 |
| **UB-180554 (54)** | 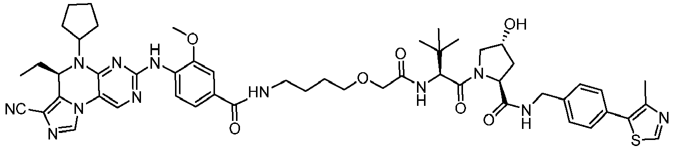 |
| **UB-180557 (57)** | 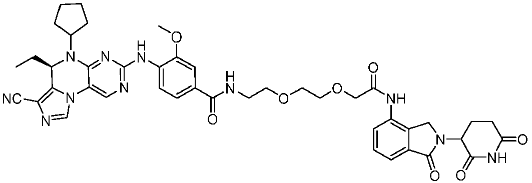 |
| **UB-180560 (60)** | 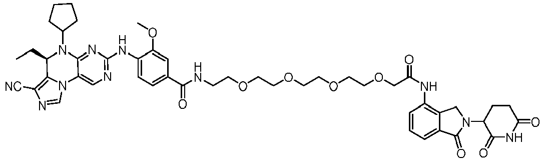 |
| **UB-180566 (66)** | 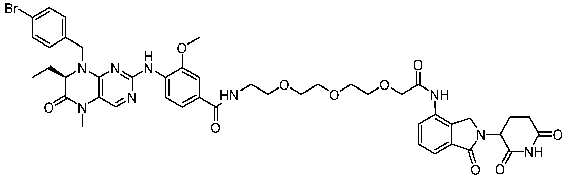 |
| **UB-180567 (67)** | 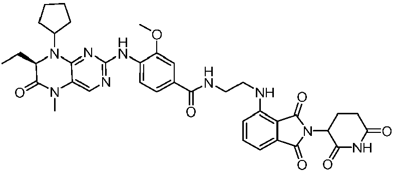 |
| **UB-180568 (68)** | 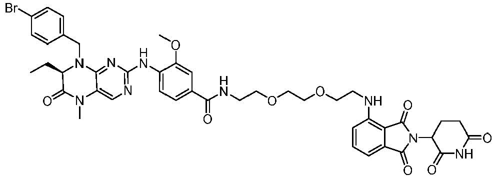 |
| **UB-180569 (69)** | 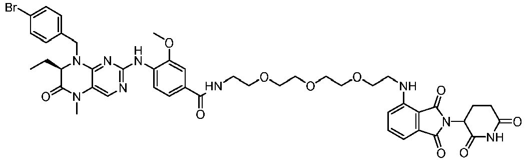 |
| **UB-180570 (70)** | 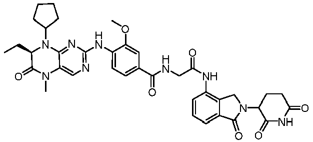 |
| **UB-180571 (71)** | 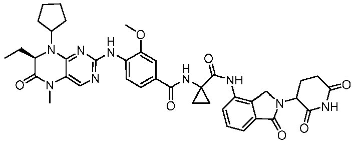 |
| **UB-180579 (79)** | 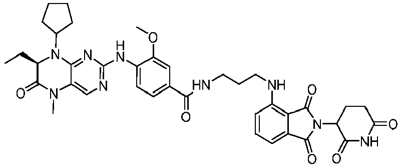 |
| **UB-180591 (91)** | 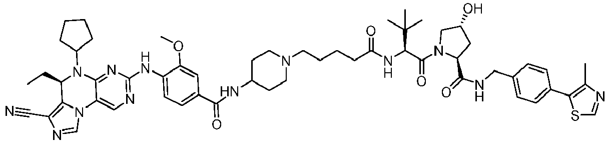 |
| **UB-180592 (92)** | 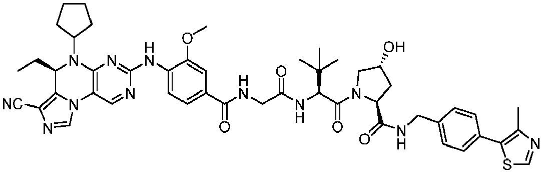 |
| **UB-180593 (93)** | 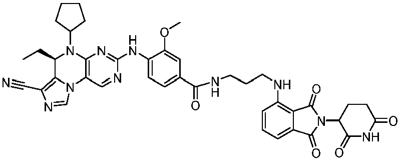 |
| **UB-180594 (94)** | 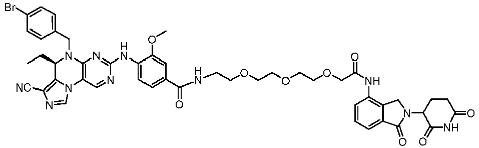 |
| **UB-180595 (95)** | 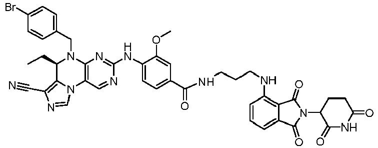 |
| **UB-180596 (96)** | 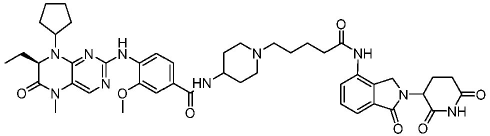 |
| **UB-180597 (97)** | 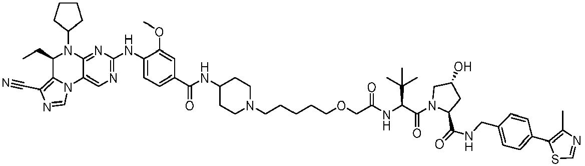 |
| **UB-180598 (98)** | 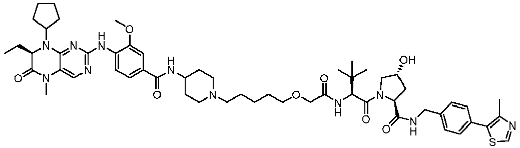 |
| **UB-180601 (101)** | 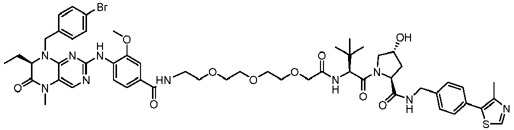 |
| **UB-180602 (102)** | 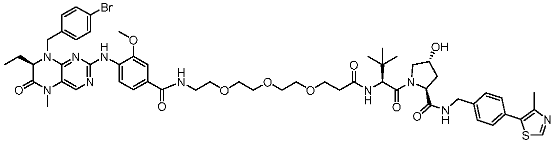 |
| **UB-180605 (105)** | 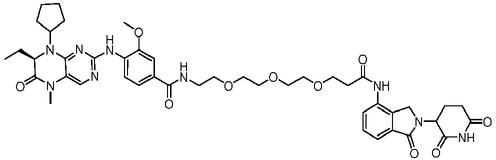 |
| **UB-180606 (106)** | 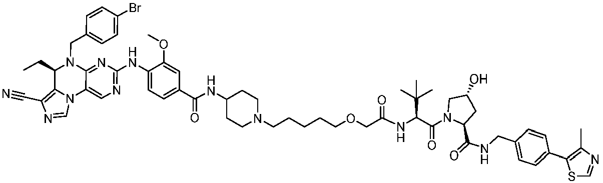 |
| **UB-180607 (107)** | 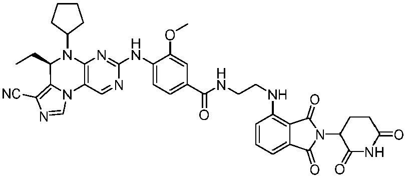 |
| **UB-180608 (108)** | 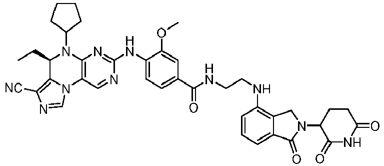 |
| **UB-180618 (118)** | 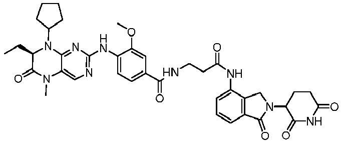 |
| **UB-180619 (119)** | 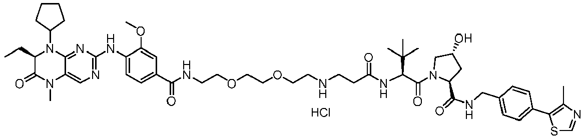 |
| **UB-180620 (120)** | 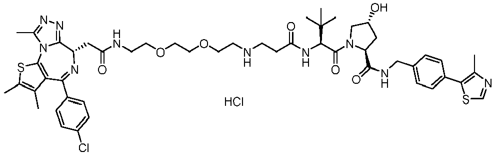 |
| **UB-180621 (121)** | 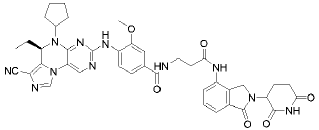 |
| **UB-180622 (122)** | 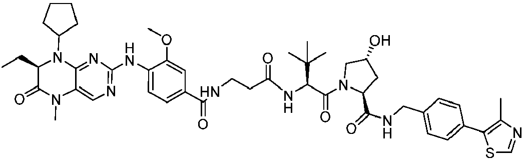 |
| **UB-180623 (123)** | 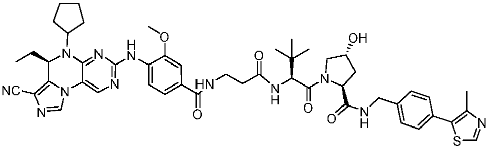 |
| **UB-180624 (124)** | 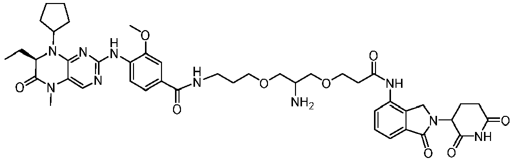 |
| **UB-180625 (125)** | 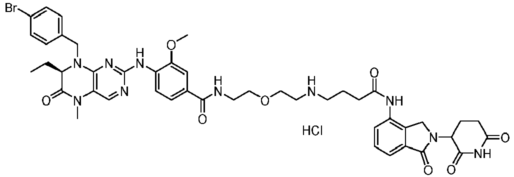 |
| **UB-180626 (126)** | 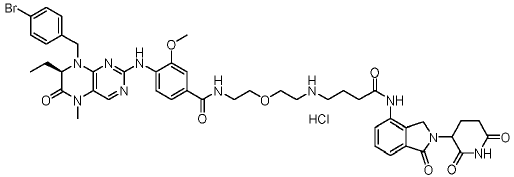 |
| **UB-180627 (127)** | 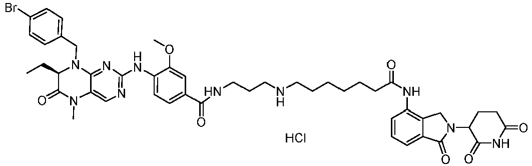 |
| **UB-180628 (128)** | 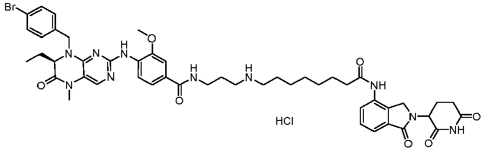 |
| **UB-180629 (129)** | 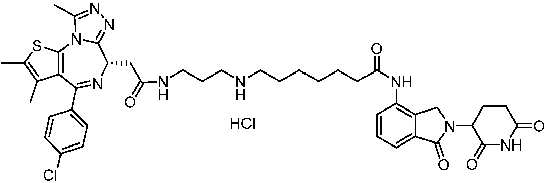 |
| **UB-180630 (130)** | 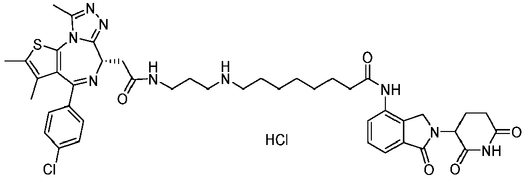 |
| **UB-180631 (131)** | 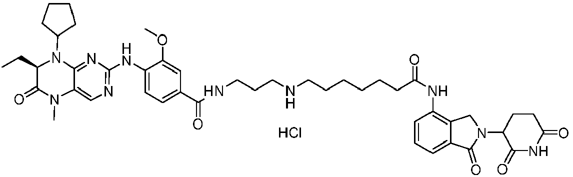 |
| **UB-180632 (132)** | 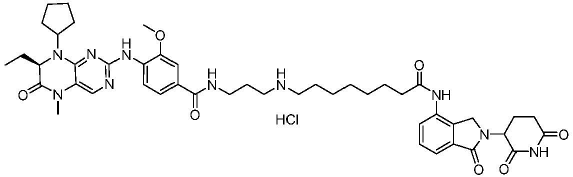 |
| **UB-180633 (133)** | 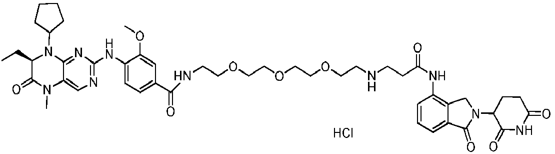 |
| **UB-180634 (134)** | 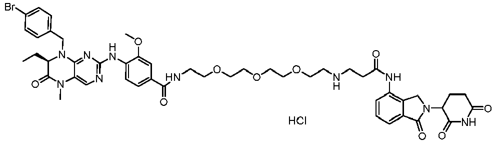 |
| **UB-180635 (135)** | 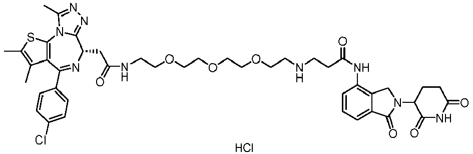 |
| **UB-180636 (136)** | 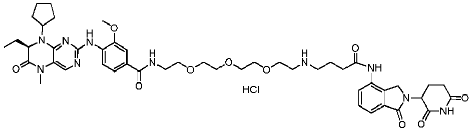 |
| **UB-180637 (137)** | 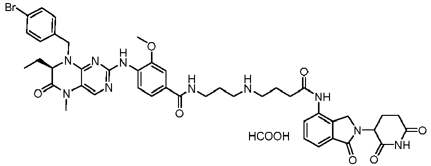 |
| **UB-180638 (138)** | 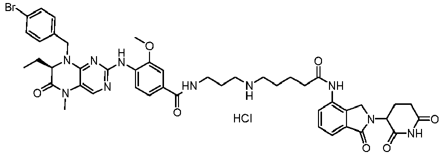 |
| **UB-180639 (139)** | 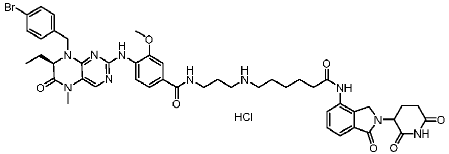 |
| **UB-180640 (140)** | 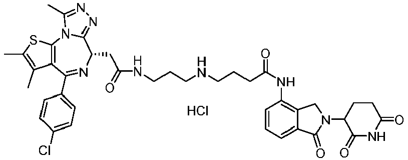 |
| **UB-180641 (141)** | 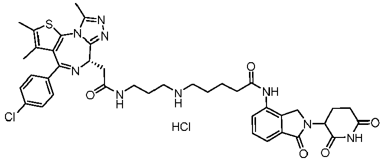 |
| **UB-180642 (142)** | 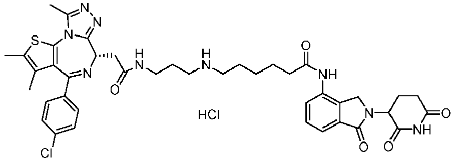 |
| **UB-180643 (143)** | 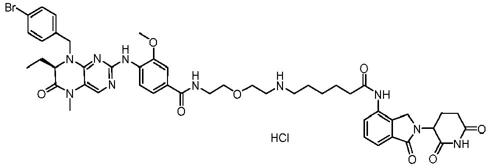 |
| **UB-180644 (144)** | 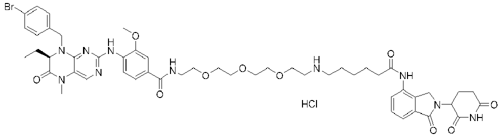 |
| **UB-180645 (145)** | 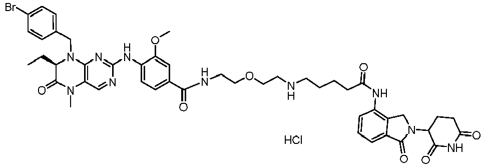 |
| **UB-180647 (147)** | 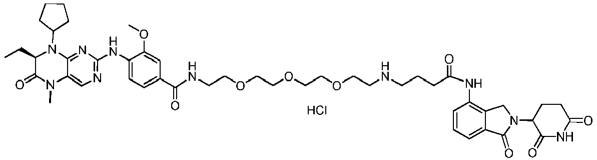 |
| **UB-180648 (148)** | 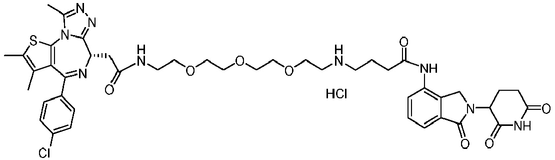 |
| **UB-180649 (149)** | 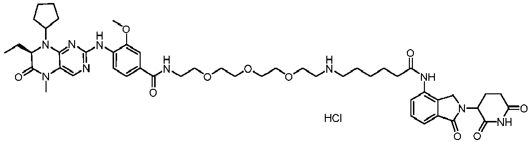 |
| **UB-180650 (150)** | 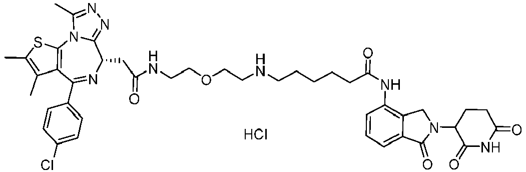 |
| **UB-180651 (151)** | 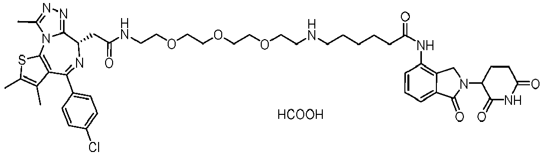 |
| **UB-180652 (152)** | 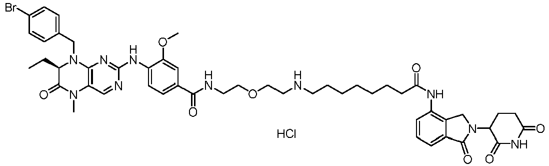 |
| **UB-180653 (153)** | 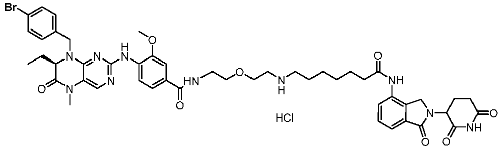 |
| **UB-180654 (154)** | 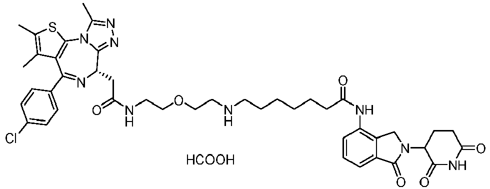 |
| **UB-180655 (155)** | 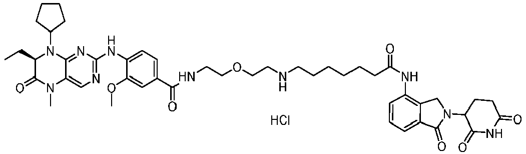 |
| **UB-180656 (156)** | 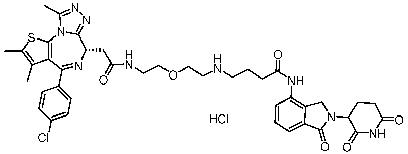 |
| **UB-180657 (157)** | 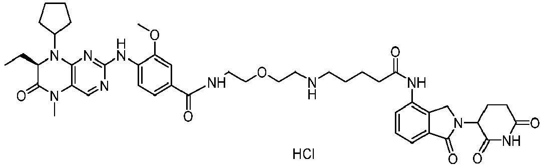 |
| **UB-180658 (158)** | 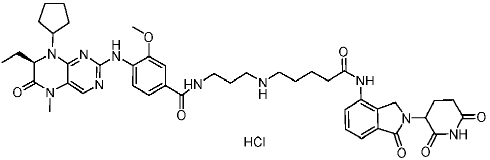 |
| **UB-180659 (159)** | 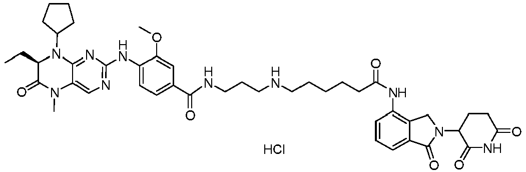 |
| **UB-180660 (160)** | 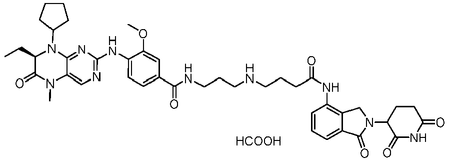 |
| **UB-180661 (161)** | 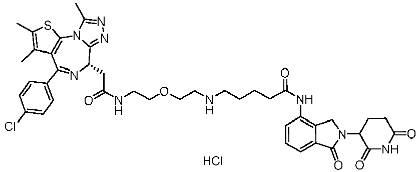 |
| **UB-180662 (162)** | 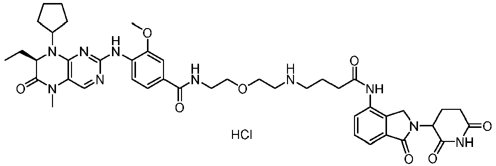 |
| **UB-180663 (163)** | 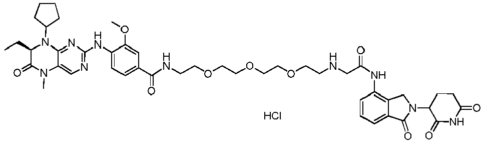 |
| **UB-180664 (164)** | 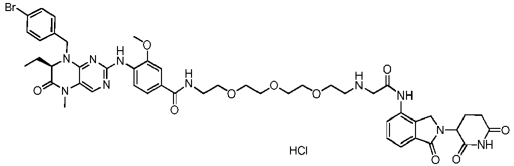 |
| **UB-180665 (165)** | 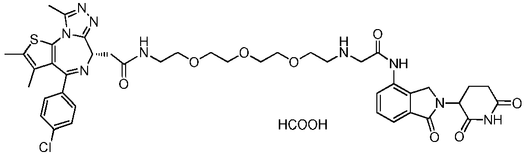 |
| **UB-180666 (166)** | 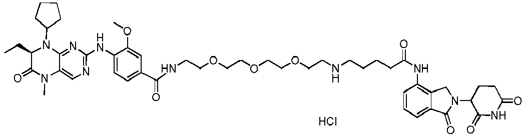 |
| **UB-180667 (167)** | 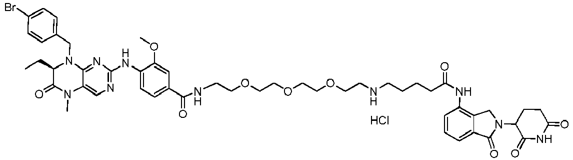 |
| **UB-180668 (168)** | 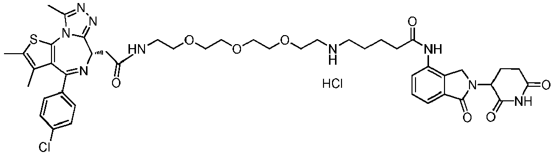 |
| **UB-180670 (170)** | 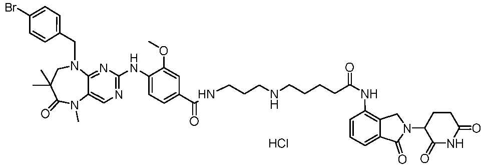 |
| **UB-180671 (171)** | 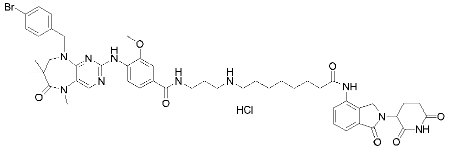 |
| **UB-180672 (172)** | 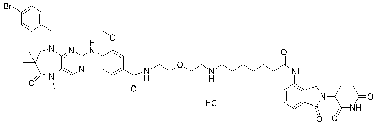 |
| **UB-180673 (173)** | 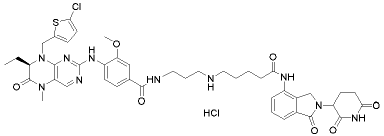 |
| **UB-180674 (174)** | 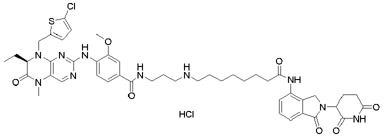 |
| **UB-180675 (175)** | 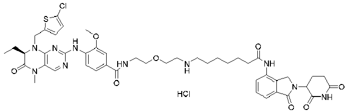 |
| UB-180676 **(**176) | 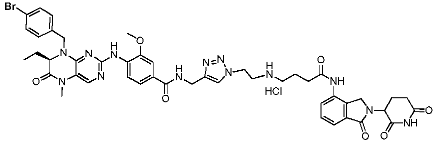 |
| **UB-180677 (177)** | 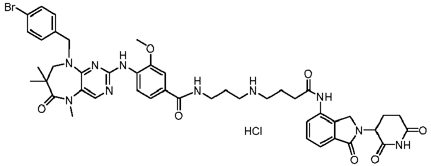 |
| **UB-180678 (178)** | 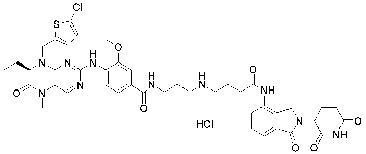 |
| **UB-180679 (179)** | 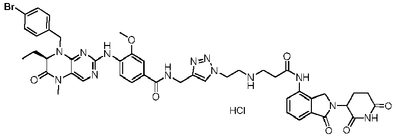 |
| **UB-180680 (180)** | 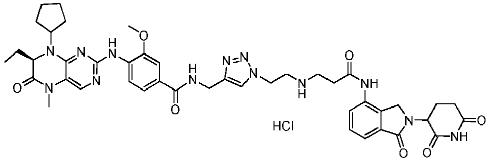 |
| **UB-180681 (181)** | 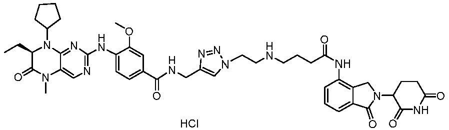 |
| **UB-180682 (182)** | 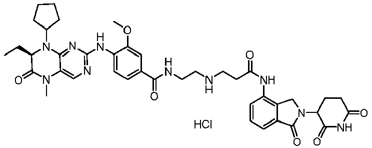 |
| **UB-180683 (183)** | 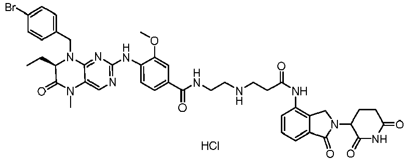 |
| **UB-180684 (184)** | 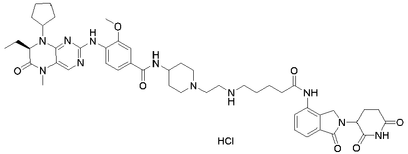 |
| **UB-180685 (185)** | 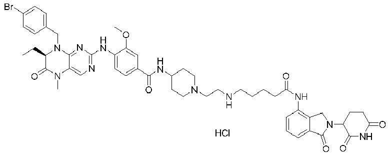 |
| **UB-180687 (187)** | 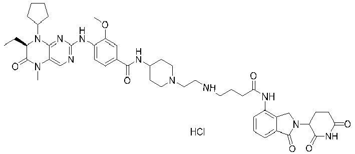 |
| **UB-180688 (188)** | 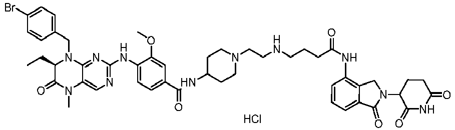 |
| **189 (189)** | 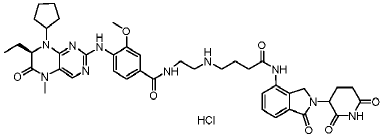 |
| **UB-180690 (189)** | 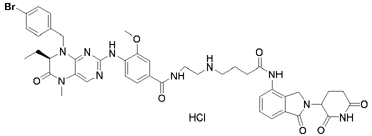 |
| **UB-180691 (191)** | 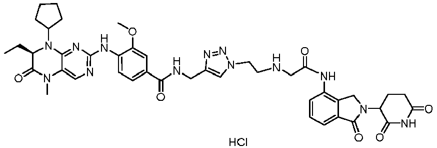 |
| **UB-180692 (192)** | 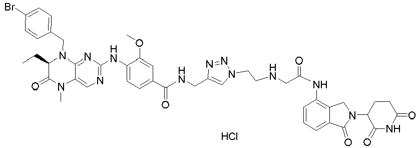 |
| **UB-180693 (193)** | 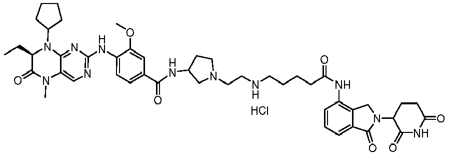 |
| **UB-180694 (194)** | 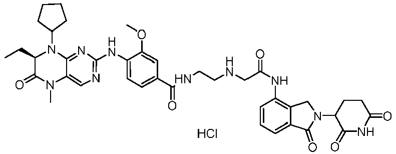 |
| **UB-180695 (195)** | 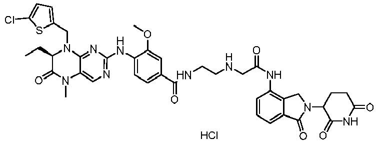 |
| **UB-180696 (196)** | 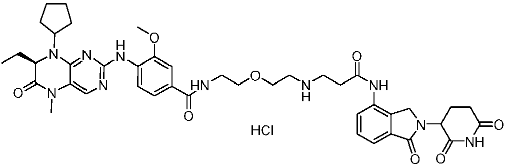 |
| **UB-180697 (197)** | 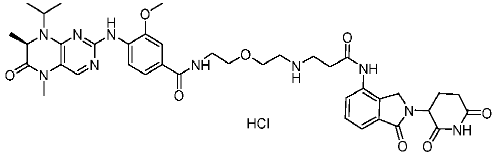 |
| **UB-180698 (198)** | 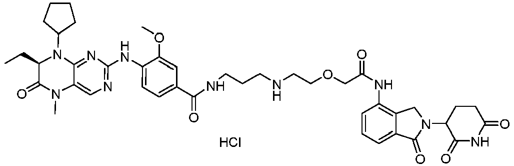 |
| **UB-180699 (199)** | 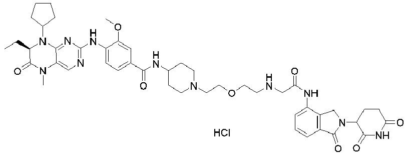 |
| **UB-180700 (200)** | 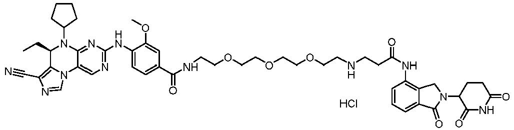 |
| **UB-180701 (201)** | 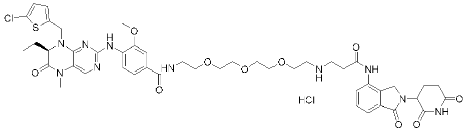 |
| **UB-180702 (202)** | 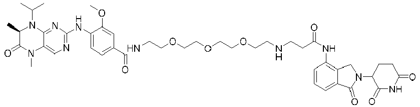 |
| **UB-180703 (203)** | 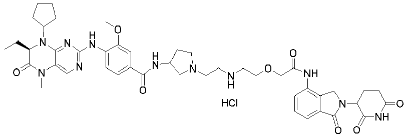 |
| **UB-180705 (205)** | 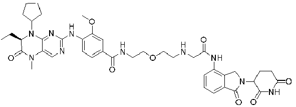 |
| **UB-180706 (206)** | 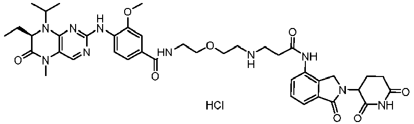 |
| **UB-180707 (207)** | 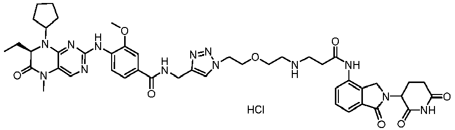 |
| **UB-180708 (208)** | 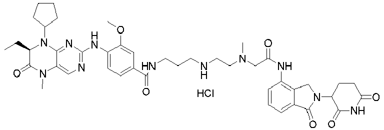 |
| **UB-180709 (209)** | 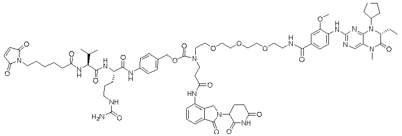 |
| **UB-180710 (210)** | 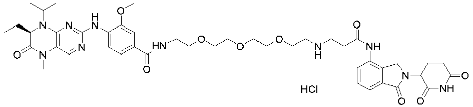 |
| **UB-180711 (211)** | 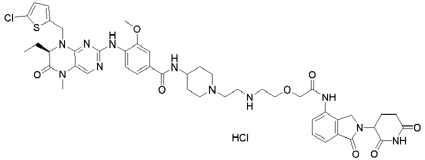 |
| **UB-180712 (212)** | 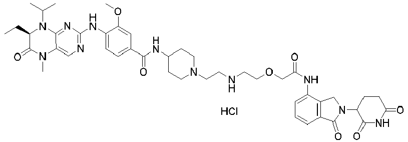 |
| **UB-180713 (213)** | 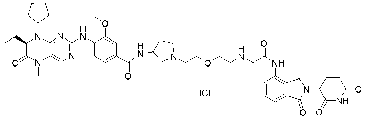 |
| **UB-180714 (214)** | 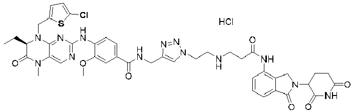 |
| **UB-180715 (215)** | 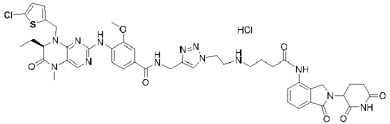 |
| **UB-180716 (216)** | 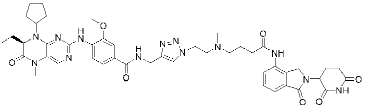 |
| **UB-180717 (217)** | 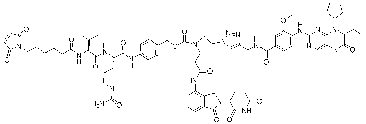 |
| **UB-180718 (218)** | 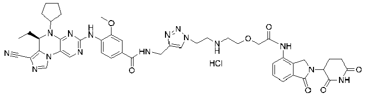 |
| **UB-180719 (219)** | 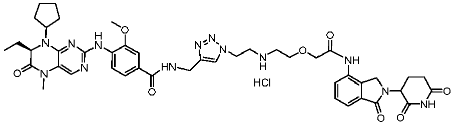 |
| **UB-180720 (220)** | 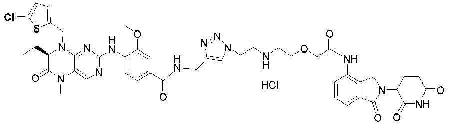 |
| **UB-180723 (223)** | 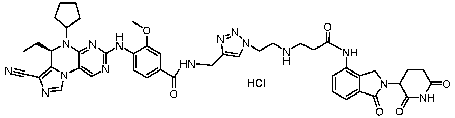 |
| **UB-180725 (225)** | 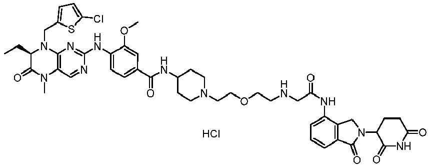 |
| **UB-180733 (233)** | 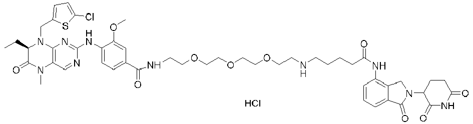 |
| **UB-180734 (234)** | 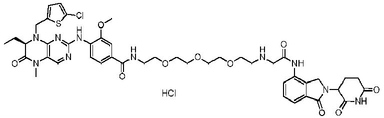 |
| **UB-180745 (245)** | 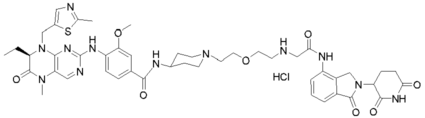 |
| **UB-180746 (246)** | 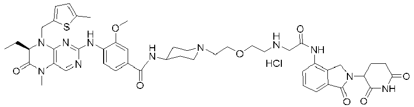 |
| **UB-180747 (247)** | 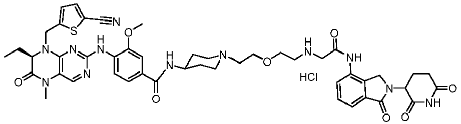 |
| **UB-180763 (263)** | 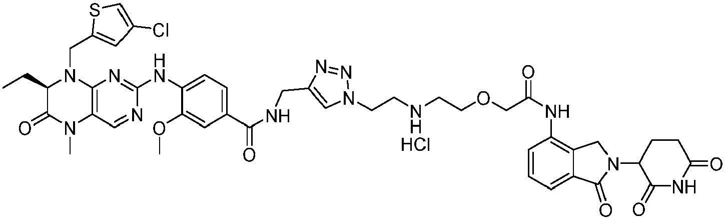 |
| **UB-180764 (264)** | 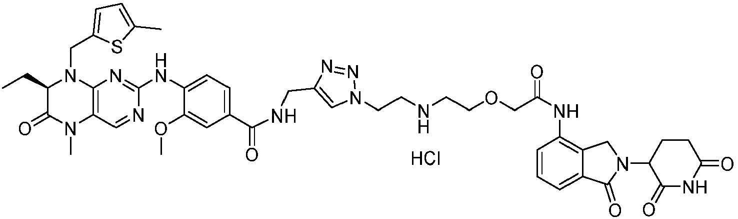 |
| **UB-180765 (265)** | 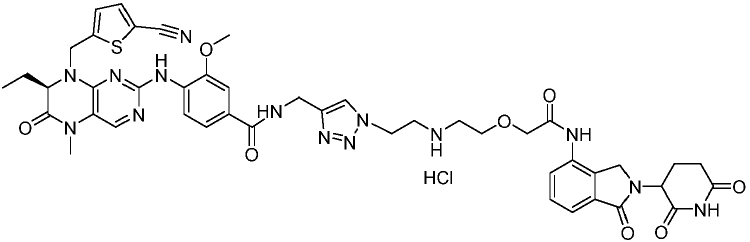 |
| **UB-180766 (266)** | 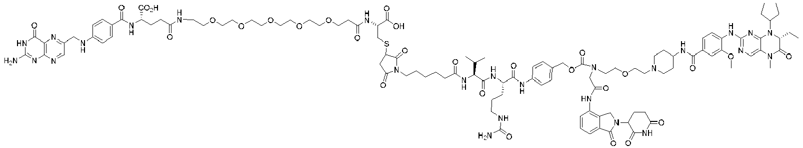 |
| **UB-180767 (267)** | 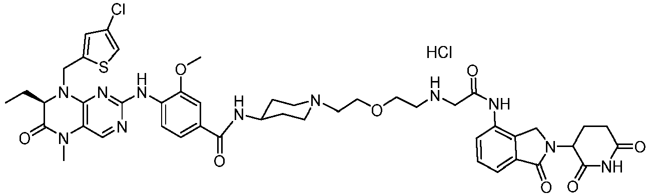 |

| | |
|---|---|
| **UB-180809 (809)** | 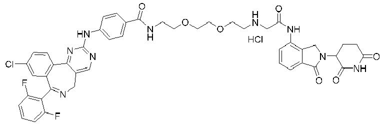 |
| **UB-180810 (810)** | 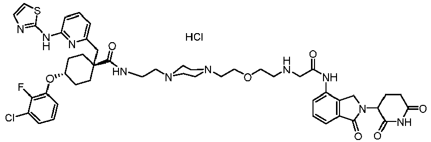 |
| **UB-180811 (811)** | 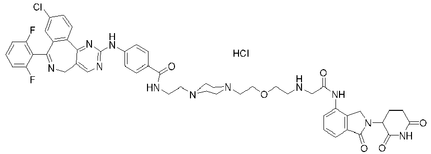 |
| **UB-180812 (812)** | 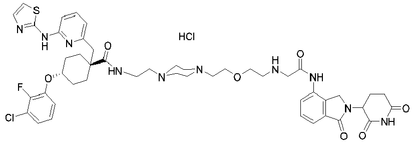 |
| **UB-180813 (813)** | 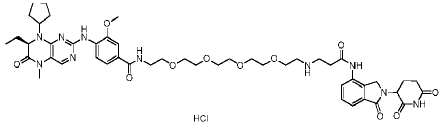 |
| **UB-180814 (814)** | 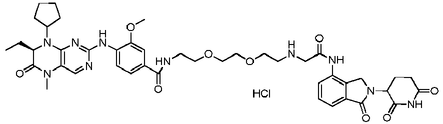 |
| **UB-180815 (815)** | 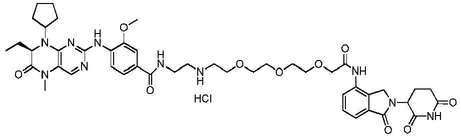 |
| **UB-180820 (820)** | 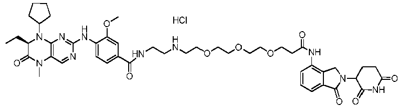 |
| **UB-180821 (821)** | 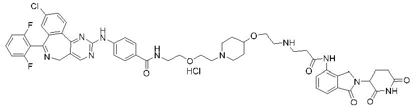 |
| **UB-180822 (822)** | 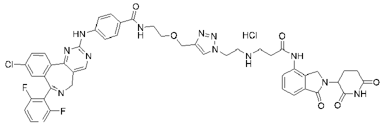 |
| **UB-180828 (828)** | 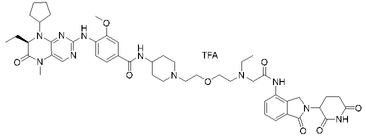 |
| **UB-180833 (833)** | 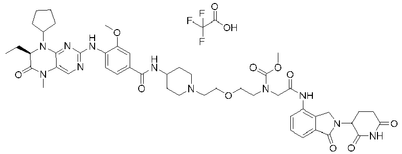 |
| **UB-180834 (834)** | 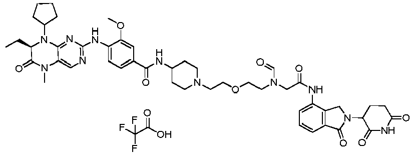 |
| **UB-180835 (835)** | 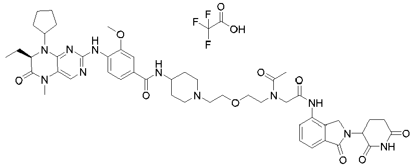 |
| **UB-180836 (836)** | 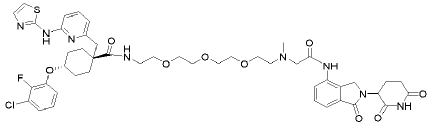 |
| **UB-180840 (840)** | 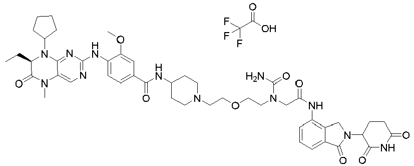 |
| **UB-180844 (844)** | 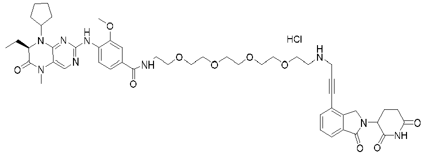 |
| **UB-180845 (845)** | 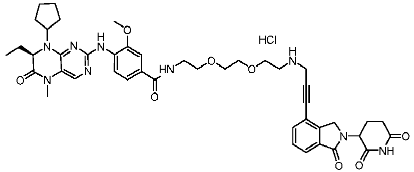 |
| **UB-180846 (846)** | 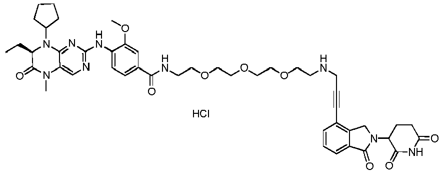 |
| **UB-180847 (847)** | 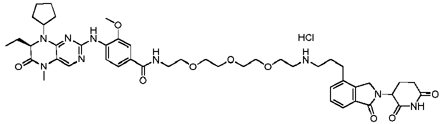 |
| **UB-180848 (848)** | 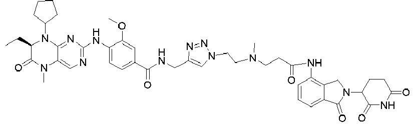 |
| **UB-180849 (849)** | 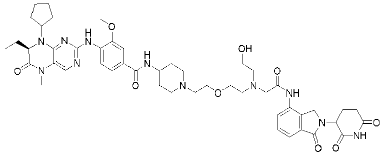 |

| | |
|---|---|
| **UB-180855 (855)** | 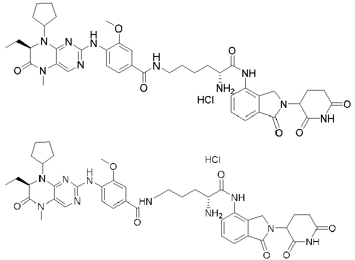 |
| **UB-180856 (856)** | 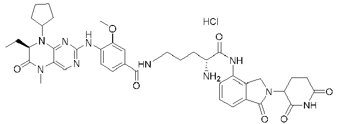 |

In another preferred embodiment, the conjugate is not those specific compounds described in Table D in PCT/CN2019/110225, and the specific compounds described in Table D are as follows:

| | |
|---|---|
| **838** | 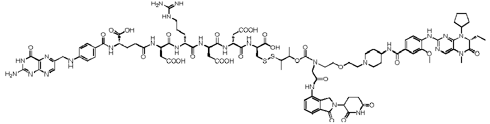 |
| **839** | 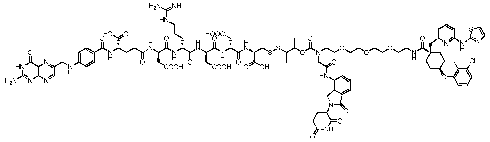 |
| **829** | 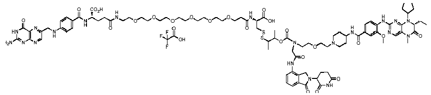 |

In another preferred embodiment, the conjugate is a conjugate selected from Group 1, Group 2 and Group 3.

In another preferred embodiment, the conjugate is a conjugate selected from Group 1a, Group 2a and Group 3a.

In another preferred embodiment, the conjugate is a conjugate selected from Group 1, Group 2 and Group 3; wherein Rand R¹ are R" (i.e. R and R¹ are each independently -W³-L3-W⁴ -(R_{P})_{q}).

In another preferred embodiment, the conjugate of formula I is a conjugate of formula X

R_{P}-(W⁴-L3-W³-R_{TED})ₜ (X)

wherein t=1 /q (preferably, t = 1 to 8; more preferably, t = 2 to 7);
R_{P} is defined as above, preferably R_{P} is polypeptide element, more preferably, antibody;
R_{TED}-W⁴-L3-W³- is the remain part of the conjugate of formula I after loss of R_{P}.

In another preferred embodiment, R_{TED} is a monovalent group derived from conjugates in Tables A1, A2 and A3, conjugates in Group 1a, Group 2a and Group 3a, or specific compounds of Example 1.5 (wherein, the derived means a monovalent group formed by the specific compounds shown in Tables A1, A2 and A3 or specific compounds shown in Example 1.5 losing a hydrogen from NH or NH₂ on the main chain or the branch chain of the linker group).

In another preferred embodiment, Ab is connected with W⁴-L3 W³- of formula III (preferably, 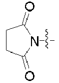 or -NH₂ group in W⁴-L3-W³-), through amino acid at N-terminal or C-terminal, or a side chain of amino acid (preferably, a side chain of amino acid selected from the group consisting of Lys, and Cys), or a sulfhydryl formed by reducing and opening disulfide bond.

In another preferred embodiment, the target molecule is target molecule A or target molecule T.

In another preferred embodiment, the target molecule A or T includes small molecules, nanocarriers, or combinations thereof.

In another preferred embodiment, the target molecule A and T are each independently selected from the group consisted of folic acid, HSP90, TINFRm, TNFR2, NADPH oxidase, BclIBax, C5a receptor, HMG-CoA reductase, PDE I-V, Squalene cyclase inhibitors, CXCR1, CXCR2, Nitric oxide (NO)synthase, cyclo-oxygenase 1-2, 5HT receptors, dopamine receptors, G-proteins, Gq, Histamine receptors, Lipoxygenases, Tryptase serine protease, Thymidylate synthase, Purine nucleotide phosphorylase, GAPDH trypanosomal, Glycogen phosphorylase, Carbonic anhydrase, Chemokine receptors, JAW STAT, RXR and the like, HIV 1 protease, HIV 1 integrase, Influenza, hepatitis B reverse transcriptase, neuraminidase, Sodium channel, MDR, protein P-glycoprotein, Tyrosine kinases, CD23, CD124, TK p56 lck, CD4, CD5, IL-1 receptor, IL-2 receptor, TNF-aR, ICAM1, Ca+ channels, VCAM, VLA-4 integrin, VLA-4 integrin, Selectins, CD40/40L, Newokinins and receptors, Inosine monophosphate dehydrogenase, p38 MAP kinase, Interleukin-1 converting enzyme, Caspase, HCV NS3 protease, HCV-NS3 RNA helicase, Glycinamide ribonucleotide formyl transferase, rhinovirus 3C protease, HSV-I, CMV, ADP-polymerae, CDK, VEGF, oxytoxin receptor, msomalmsomal transfer protein inhibitor, Bile acid transfer protein inhibitor, 5-a reductase, Angiotensin 11, Glycine receptors, noradrenaline reuptake receptor, Endothelin receptors, Neuropeptide Y and receptors, Estrogen receptors, AMP, AMP deaminase, ACC, EGFR, and Farnesyltransferase.

In another preferred embodiment, the peptide element includes antibody, protein, or combinations thereof.

In another preferred embodiment, the antibody comprises a nanobody and/or small molecule antibody (minibody), or combinations thereof.

In another preferred example, the polypeptide element is an antibody; preferably, the antibody comprises a nanobody and/or a small molecule antibody (minibody).

In another preferred embodiment, the antibody can bind to the antigen or receptor selected from the group consisting of DLL3, EDAR, CLL1, BMPR1B, E16, STEAP1, 0772P, MPF, 5T4, NaPi2b, Sema 5b, PSCA hlg, ETBR, MSG783, STEAP2, TrpM4, CRIPTO, CD21, CD22, CD79b, CD19, CD37, CD138, FcRH2, B7-H4, HER2, NCA, MDP, IL20Rα, Brevican, EphB2R, ASLG659, PSCA, GEDA, BAFF-R, CD79a, CXCR5, HLA-DOB, P2X5, CD72, LY64, FcRH1, IRTA2, TENB2, PMEL17, TMEFF1, GDNF-Ra1, Ly6E, TMEM46, Ly6G6D, LGR5, RET, LY6K, GPR19, GPR54, ASPHD1, Tyrosinase, TMEM118, GPR172A, MUC1, CD70, CD71, MUC16, methothelin, FOLR1, Trop-2, gpNMB, EGFR, ENPP3, PSMA, CA6, GPC-3, PTK7, CD44, CD56, TIM-1, Cadherin-6, ASG-15ME, ASG-22ME, CanAg, AXL, CEACAM5, EphA4, cMet, FGFR2, FGFR3, CD123, Her3, LAMP1, LRRC15, TDGF1, CD66, CD25, BCMA, GCC, Noch3, cMet, EGFR and CD33.

In another preferred embodiment, R_{T} is selected from groups shown in Table B.

In another preferred embodiment, the moiety of E3 ligase ligand A1 is selected from the group consisting of the A¹ groups in WO2017/176957 A1 (preferably, corresponding moiety of A-10, A-11, A-15, A-28, A- 48, A-69, A-85, A-93, A-98, A-99 or A-101 in WO2017/176957 A1):

In another preferred embodiment, the moiety of E3 ligase ligand is selected from:

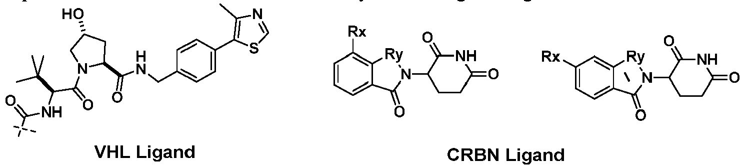

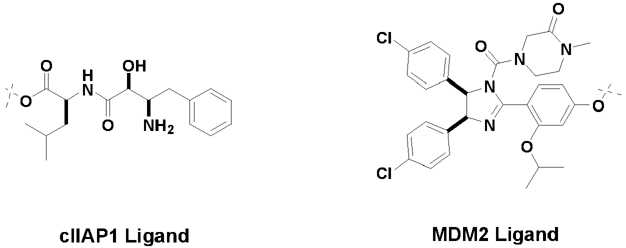

In each formula, a dotted line indicates the position connected with other parts (i.e., the position connected with R_{T}-L1);
wherein Rx is each independently selected from the group consisting of null, NH, NH-CO, O, S, SO, SOz, SOz(NHz)NH, C1-C4 alkylene, C2-C5 alkenylene, and C2-C5 alkynylene; R_{y} is C=O, C=S or CH₂.

In another preferred embodiment, the moiety of E3 ligase ligand is selected from the groups shown in Table C.

In another preferred embodiment, when R_{E3} is 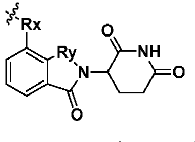 (A1)(preferably, A1.2 in Table B), the conjugate of formula I is of formula 1-1, R_{T}-W¹-L5-W^{b}-C≡C-R_{E3} (1-1); preferably, at least one of M in L5 is O and/or W¹ is NH or NH-Cr², and/or W^{b} is CH²; more preferably, in L5, 7≤o1+o2≤12.

In another preferred embodiment, when R_{E3} is 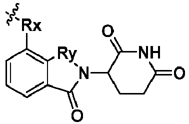 (A1)(preferably, A1.2 in Table B), the conjugate of formula I is of R_{T}-W^{a}-Cr¹-Cr²-(M)ₒ₃-W²-R_{E3}, and neither of Cr¹ and Cr² is null; preferably, L2 is -(M)ₒ₃-, and subscript o3 is 1, 2, 3, 4, or 5.

In another preferred embodiment, R_{T}, R_{E3}, R_{P}, L1, L2, L3, L4, L5, L6, L7, W¹, W², W³, W⁴, W^{a}, W^{b}, W, M^{L}, M, M', M^{T}, M^{N}, subscript s, subscript p, subscript q, subscript o, subscript o1, subscript o2, R^{a}, R^{b}, R^{c}, R, R', R", Cr¹, Cr², Ar1 are each independently corresponding groups in specific compound or general formula herein; preferably, the corresponding groups in specific compounds or general formula shown in Gorup 1, Gorup 2, Gorup 3, Gorup 1a, Gorup 2a, Gorup 3a, Table A1, Table A2, A3, Table B, Table C, and Table D.

In another preferred embodiment, the conjugate is the TED compoundof the sixth aspect.

In another preferred embodiment, the conjugate is the ACTED compound of the seventh aspect.

In the second aspect of the present invention, a pharmaceutical composition is provided, wherein the pharmaceutical composition includes the conjugate of the first aspect and pharmaceutically acceptable carriers.

In the third aspect of the present invention, provided is a use of the conjugate of the first aspect in preparation of a drug for the treatment or prevention of diseases associated with an excess of a target protein.

In the forth aspect of the present invention, provided is a use of the conjugate of the first aspect for treatment or prevention of diseases associated with an excess of a target protein.

In the fifth aspect of the present invention, a method for reducing the content of target proteins in a cell is provided, wherein the cell is contacted with the conjugate of the first aspect, thereby reducing the content of the target proteins in the cell.

In another preferred embodiment, the method is *in vitro.*

In another preferred embodiment, the method is non-diagnostic and non-therapeutic.

In the sixth aspect of the present invention, provided is a TED compound or the pharmaceutically acceptable salts thereof, wherein the TED compound is shown in formula VI;

R_{T} W¹-(M^{L})ₒ-W²-R_{E3} (VI)

wherein
M^{L} is each independently M or M^{N}
M, M^{N}, R_{E3}, R_{T}, W¹, W² and subscript o are defined as in formula I.

In another preferred embodiment, the TED compound is shown in formula IV.

In another preferred embodiment, the TED compound is shown in formula 1a-1, 1a-2, 1a-3, 2a or 3a.

In another preferred embodiment, the TED compound is used for coupling with R_{P}.

In another preferred embodiment, the TED compound is coupled with R^{P} through -W³-L3-W⁴-.

In another preferred embodiment, the TED compound is a compound selected from Group 1, Group 2 and Group 3, and R and R¹ are each independently R'.

In another preferred embodiment, the TED compound is a compound selected from Table A1, A2 and A3, Group 1a, Group 2a and Group 3a.

In the sixth aspect of the present invention, provided is an ACTED compound or the pharmaceutically acceptable salts thereof, wherein the ACTED compound is shown in formula VII;

R_{T} W¹-(M^{L})ₒ-W²-R_{E3} (VII)

wherein
M^{L} is each independently M or M^{T}
M, M^{T}, R_{E3}, R_{T}, W¹, W² and subscript o are defined as in formula I.

In another preferred embodiment, the ACTED compound is shown in formula V.

In another preferred embodiment, the ACTED compound is shown in formula X.

In another preferred embodiment, the ACTED compound is shown in formula 1b-1, 1b-2, 1b-3, 2b or 3b.

In another preferred embodiment, the ACTED compound is a compound selected from Group 1, Group 2 and Group 3, and R and R¹ are each independently R".

In another preferred embodiment, the ACTED compound is selected from: 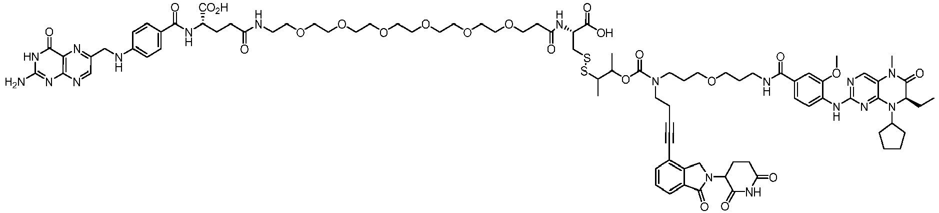 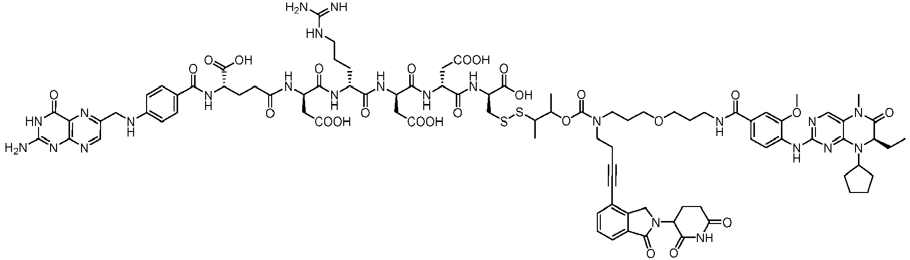 compounds 1216, 1229, 1231, and 1233 in Table D.

It should be understood that within the scope of the present invention, the above technical features of the present invention and the technical features specifically described in the following (e.g., exmples) can be combined with each other, thereby forming a new or preferred technical solution. Due to space limitations, it will not be repeated herein.

### DESCRIPTION OF THE DRAWINGS

Figure 1 shows the degradation of BRD4 and PLK1 in the MV4;11 cell line by the compounds of the present invention.
Figure 2 shows the degradation of BRD4 and PLK1 in the MV4;11 cell line by the compounds of the present invention.
Figure 3 shows the degradation of BRD4 and PLK1 in the TMD-8 cell line by the compounds of the present invention.
Figure 4 shows the degradation of BRD4 and PLK1 in the MV4;11 cell line by the compounds of the present invention

### DETAILED DESCRIPTION OF THE INVENTION

After extensively and deeply researching, the inventors developed TED conjugates with novel structures for the first time, and the conjugates of the present invention have a structure of formula I. In addition, the conjugates of the present invention are very suitable for further connected with polypeptide elements (especially antibodies, protein ligands) and/or other molecules with targeting properties, or after further connecting with polypeptide elements and/or other molecules with targeting properties and the like, or further connecting with polypeptide elements and/or other molecules with targeting properties in the conjugates with polypeptide elements and/or other molecules with targeting properties, thereby possessing excellent dual targeting properties(such as specificity of targeting of tumour cells), improving drug selectivity, implementing more precise degradation of pathogenic proteins, reducing the possible systemic toxicity induced by non-specific degradation, and is possible to overcome the difficulties encountered in drug absorption and metabolism, and eliminate the possibility for producing drug resistance. The inventor has completed the present invention on this basis.

### Terms

As used herein, the term "compound of the present invention", and "conjugate of the present invention" are used interchangeably and refers to the compound or the conjugate of formula I described in the first aspect of the present invention.

As used herein, unless otherwise stated, the term "alkyl", by itself or as part of another substituent means a straight or branched chain hydrocarbon radical, having the number of carbon atoms designated (i.e. C₁₋₆ means 1 - 6 carbons). Preferably, alkyl contains 1 to 4 carbons, i.e. C₁₋₄ alkyl. Examples of alkyl include, but not limited to, methyl, ethyl, n-propyl, isopropyl, n-butyl, t-butyl, iso-butyl, sec-butyl, n-pentyl, n-hexyl, n-heptyl, n-octyl, and the like. The term "alkenyl" refers to an unsaturated alkyl having one or more double bonds. Preferably, alkenyl contains 2 to 4 carbons, i.e. C₂₋₄ alkenyl. Similarly, the term "alkynyl" refers to an unsaturated alkyl having one or more triple bonds. Preferably, alkynyl contains 2 to 4 carbons, i.e. C₂₋₄ alkynyl. Examples of such unsaturated alkyl include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. The term "cycloalkyl" refers to hydrocarbon rings having the indicated number of ring atoms (e.g., C₃₋₆ cycloalkyl) and being fully saturated or having no more than one double bond between ring vertices.

As used herein, the term "cycloalkyl" refers to hydrocarbon rings having the indicated number of ring atoms (e.g., C₃₋₈ cycloalkyl) and being fully saturated or having no more than one double bond between ring vertices. This term is also meant to contain bicyclic and polycyclic hydrocarbon rings such as bicyclo[2.2.1]heptane, bicyclo[2.2.2]octane, etc. The term "heterocycloalkyl" refers to a cycloalkyl that contains one to five heteroatoms selected from N, O, and S, wherein the nitrogen and sulfur atoms are optionally oxidized, and the nitrogen atom(s) are optionally quaternized. The heterocycloalkyl may be a monocyclic, a bicyclic or a polycylic ring system. Non limiting examples of heterocycloalkyl include pyrrolidine, imidazolidine, pyrazolidine, butyrolactam, valerolactam, imidazolidinone, hydantoin, dioxolane, phthalimide, piperidine, 1,4-dioxane, morpholine, thiomorpholine, thiomorpholine-S-oxide, thiomorpholine-S,S-oxide, piperazine, pyran, pyridone, 3-pyrroline, thiopyran, pyrone, tetrahydrofuran, tetrhydrothiophene, quinuclidine, and the like. The heterocycloalkyl can be attached to the rest of the molecule via a ring carbon or a heteroatom. For terms such as cycloalkylalkyl and heterocycloalkylalkyl, it is meant that a cycloalkyl or a heterocycloalkyl is attached through an alkyl or alkylene linker to the rest of the molecule. For example, cyclobutylmethyl - is a cyclobutyl ring that is attached to a methylene linker to the rest of the molecule.

The term "alkylene" by itself or as part of another substituent means a divalent radical derived from an alkane, as exemplified by -CH₂CH₂CH₂CH₂-. Typically, an alkyl (or alkylene) will have from 1 to 24 carbon atoms, with those groups having 10 or fewer carbon atoms being preferred in the present disclosure. A "lower alkyl" or "lower alkylene" is a shorter chain alkyl or alkylene, generally having four or fewer carbon atoms. Similarly, "alkenylene" and "alkynylene" refer to the unsaturated forms of "alkylene" having double or triple bond, respectively

Unless otherwise specified, the term "heteroalkyl" by itself or in combination with other terms refers to a stable linear or branched or cyclic hydrocarbon group or a combination thereof, consisting of a specified number of carbon atoms and 1 to 3 heteroatoms selected from O, N, Si and S, and wherein nitrogen and sulfur atoms are optionally oxidized, and nitrogen heteroatoms can be optionally quaternized. The heteroatoms O, N and S can be placed at any internal position of the heteroalkyl. The heteroatom Si may be placed at any position of the heteroalkyl, including the position at which the alkyl is attached to the rest of the molecule. Examples include -CH₂-CH₂-O-CH₃, -CH₂-CH₂-NH-CH₃, -CH₂-CH₂-N(CH₃)-CH₃, -CH₂-S-CH₂-CH₃, -CH₂-CH₂-S(O)-CH₃, -CH₂-CH₂-S(O)₂-CH₃, -CH=CH-O-CH₃, -Si(CH₃)₃, -CH₂-CH=N-OCH₃, and -CH=CH-N(CH₃)-CH₃. Up to two heteroatoms may be consecutive, such as -CH₂-NH-OCH₃ and -CH₂-O-Si(CH₃)₃. Similarly, unless otherwise specified, terms "heteroalkenyl" and "heteroalkynyl" by themselves or in combination with another term refer to alkenyl or alkynyl, respectively, that contain the stated number of carbons and 1 to 3 heteroatoms selected from O, N, Si and S, and wherein the nitrogen and sulfur atoms may optionally be oxidized and the nitrogen heteroatom may optionally be quaternized. The heteroatoms O, N and S can be placed at any internal position of the heteroalkyl.

The term "heteroalkylene" by itself or as part of another substituent means a saturated or unsaturated or polyunsaturated divalent radical, derived from heteroalkyl, as exemplified by - CH₂-CH₂-S-CH₂CH₂- and -CH₂-S-CH₂-CH₂-NH-CH₂-, -O-CHz-CH=CH-, -CHz-CH=C(H)CHz-O-CHz- and -S-CH₂-C≡C-. For heteroalkylene, heteroatoms can also occupy either or both of the chain termini (e.g., alkyleneoxy, alkylenedioxy, alkyleneamino, alkylenediamino, and the like).

The terms "alkoxy", "alkylamino" and "alkylthio" (or thioalkoxy) are used in their conventional meanings, refer to those alkyl attached to the rest of the molecule via an oxygen atom, amino, or a sulfur atom, respectively. Additionally, for dialkylamino, the alkyl portions can be the same or different and can also be combined to form a 3-7 membered ring with the nitrogen atom to which each is attached. Accordingly, a group represented as -NR^{a}R^{b} is meant to include piperidinyl, pyrrolidinyl, morpholinyl, azetidinyl and the like.

Unless otherwise stated, the terms "halo" or "halogen" by themselves or as part of another substituent, mean, a fluorine, chlorine, bromine, or iodine atom. Additionally, terms such as "haloalkyl," are meant to include monohaloalkyl or polyhaloalkyl. For example, the term "C₁₋₄ haloalkyl" is mean to include trifluoromethyl, 2,2,2-trifluoroethyl, 4-chlorobutyl, 3-bromopropyl, and the like.

Unless otherwise stated, the term "aryl" means, a polyunsaturated, typically aromatic, hydrocarbon group which can be a single ring or multiple rings (up to three rings) which are fused together or linked covalently. The term "heteroaryl" refers to aryl (or rings) that contains one to five heteroatoms selected from N, O, and S, wherein the nitrogen and sulfur atoms are optionally oxidized, and the nitrogen atom(s) are optionally quaternized. A heteroaryl can be attached to the rest of the molecule through a heteroatom. Non-limiting examples of aryl include phenyl, naphthyl and biphenyl, while non-limiting examples of heteroaryl include pyridyl, pyridazinyl, pyrazinyl, pyrimindinyl, triazinyl, quinolinyl, quinoxalinyl, quinazolinyl, cinnolinyl, phthalaziniyl, benzotriazinyl, purinyl, benzimidazolyl, benzopyrazolyl, benzotriazolyl, benzisoxazolyl, isobenzofuryl, isoindolyl, indolizinyl, benzotriazinyl, thienopyridinyl, thienopyrimidinyl, pyrazolopyrimidinyl, imidazopyridinyl, benzothiaxolyl, benzofuranyl, benzothienyl, indolyl, quinolyl, isoquinolyl, isothiazolyl, pyrazolyl, indazolyl, pteridinyl, imidazolyl, triazolyl, tetrazolyl, oxazolyl, isoxazolyl, thiadiazolyl, pyrrolyl, thiazolyl, furyl, thienyl and the like. Substituents for above-stated aryl and heteroaryl ring systems are selected from the group of acceptable substituents described below.

For brevity, the term "aryl" when used in combination with other terms (e.g., aryloxy, arylthio, arylalkyl) includes both aryl and heteroaryl rings as defined above. Thus, the term "arylalkyl" is meant to include those radicals in which an aryl is attached to an alkyl that is attached to the rest of the molecule (e.g., benzyl, phenethyl, pyridylmethyl and the like).

The above terms(e.g., "alkyl," "aryl" and "heteroaryl"), in some embodiments, will include both substituted and unsubstituted forms of the indicated radical. The preferred substituents for each type of group are provided below. For brevity, the terms aryl and heteroaryl will refer to substituted or unsubstituted versions as provided below, while the term "alkyl" and related aliphatic radicals is meant to refer to unsubstituted version, unless indicated to be substituted.

Substituents for the alkyl (including those groups often referred to as alkylene, alkenyl, alkynyl and cycloalkyl) can be a variety of groups selected from -halogen, -OR', -NR'R", -SR', -SiR'R"R"', -OC(O)R', -C(O)R', -CO₂R', -CONR'R", -OC(O)NR'R", -NR"C(O)R', -NR'-C(O)NR"R"', -NR"C(O)₂R', -NH-C(NH₂)=NH, -NR'C(NH₂)=NH, -NH-C(NH₂)=NR', -S(O)R', -S(O)₂R', -S(O)₂NR'R", -NR'S(O)₂R", -CN and -NOz, in a number ranging from zero to (2M'+1), wherein M' is the total number of carbon atoms in such radical. R', R" and R‴ are each independently refer to hydrogen, unsubstituted C₁₋₈ alkyl, unsubstituted heteroalkyl, unsubstituted aryl, aryl substituted with 1-3 halogens, unsubstituted C₁₋₈ alkyl, C₁₋₈ alkoxy or C₁₋₈ thioalkoxy, or unsubstituted aryl-C₁₋₄ alkyl. When R' and R" are attached to the same nitrogen atom, they can be combined with the nitrogen atom to form a 3-, 4-, 5-, 6-, or 7-membered ring. For example, -NR'R" is meant to include 1-pyrrolidinyl and 4-morpholinyl. The term "acyl" as used by itself or as part of another group refers to groups wherein two H on the carbon that is closest to the point of attachment for the radical is replaced with the substituent =O (e.g., C(O)CH₃, -C(O)CH₂CH₂OR' and the like).

Similarly, substituents for the aryl and heteroaryl are varied and are generally selected from -halogen, -OR', -OC(O)R', -NR'R", -SR', -R', -CN, -NO₂, -CO₂R', -CONR'R", -C(O)R', - OC(O)NR'R", -NR"C(O)R', -NR"C(O)₂R', -NR'-C(O)NR"R"', -NH-C(NH₂)=NH, - NR'C(NH₂)=NH, -NH-C(NH₂)=NR', -S(O)R', -S(O)₂R', -S(O)₂NR'R", -NR'S(O)₂R", -N₃, perfluoro(C₁-C₄)alkoxy, and perfluoro(C₁-C₄)alkyl, in a number ranging from 0 to the total number of open valences on the aromatic ring system; and wherein R', R" and R‴ are independently selected from hydrogen, C₁₋₈ alkyl, C₃₋₆ cycloalkyl, C₂₋₈ alkenyl, C₂₋₈ alkynyl, unsubstituted aryl and heteroaryl, (unsubstituted aryl)-C₁₋₄ alkyl, and unsubstituted aryloxy-C₁₋₄ alkyl. Other suitable substituents include each of the above aryl substituents attached to a ring atom by an alkylene containing 1-4 carbon atoms.

Two of the substituents on adjacent atoms of the aryl or heteroaryl ring may optionally be replaced with a substituent of the formula -T-C(O)-(CH₂)_{q}-U-, wherein T and U are independently -NH-, -O-, -CH₂- or a single bond, and q is an integer of from 0 to 2. Alternatively, two of the substituents on adjacent atoms of the aryl or heteroaryl ring may optionally be replaced with formula -A-(CH₂)ᵣ-B-, wherein A and B are independently -CH₂-, - O-, -NH-, -S-, -S(O)-, -S(O)₂-, -S(O)₂NR'- or a single bond, and r is an integer of from 1 to 3. One of the single bonds of the new ring so formed may optionally be replaced with a double bond. Alternatively, two of the substituents on adjacent atoms of the aryl or heteroaryl ring may optionally be replaced with a substituent of the formula -(CH₂)ₛ-X-(CH₂)ₜ-, wherein s and t are independently integers of from 0 to 3, and X is -O-, -NR'-, -S-, -S(O)-, -S(O)₂- or - S(O)₂NR'-. The substituent R' in -NR'- and -S(O)₂NR- is selected from hydrogen or unsubstituted C₁₋₆ alkyl.

In the present invention, when a cycloalkyl or heterocycloalkyl is a divalent group, the cycloalkyl or heterocycloalkyl may lose two hydrogens on the same ring atom (on ring carbon atom) thereby connecting with other chain atoms on the chain (forming a structure similar to a spirocyclic ring), or may lose two hydrogens on different ring atoms thereby connect with other chain atoms on the chain (such as -cyclopentylidene-).

As used herein, the term "heteroatom" is meant to include oxygen (O), nitrogen (N), sulfur (S) and silicon (Si).

For the compounds provided herein, a bond that is drawn from a substituent (typically an R group) to the center of an aromatic ring (e.g., benzene, pyridine, and the like) will be understood to refer to a bond providing a connection at any of the available vertices of the aromatic ring. In some embodiments, the depiction will also include connection at a ring which is fused to the aromatic ring. For example, a bond drawn to the center of the benzene portion of an indole, will indicate a bond to any available vertex of the six- or five-membered ring portions of the indole.

As used herein, term "amino acid residue" refers to a group formed by the removal of an H from -NH₂ at the N-terminal and the removal of -OH from -COOH at the C-terminal of the an amino acid. Unless otherwise defined, used herein, amino acids include natural or nonnatural amino acids, including D and/or L-type amino acids. Examples of amino acids include, but are not limited to, Ala (A), Arg (R), Asn (N), Asp (D), Cys (C), Gln (Q) Glu (E), Gly (G), His (H), Ile (I), Leu (L), Lys (K), Met (M), Phe (F), Pro (P), Ser (S), Thr (T), Trp (W), Tyr (Y), Val (V). Preferably, the amino acid used herein is an amino acid selected from the group consisting of L-glycine(L-Gly), L-alanine (L-Ala), β-alanine (β-Ala), L-glutamic acid(L-Glu), L-aspartic acid (L-Asp), L-histidine (L-His), L-Arginine (L-Arg), L-Lysine (L-Lys), L-Valine (L-Val), L-Serine (L-Ser), and L-Threonine(L-Thr).

The term "pharmaceutically acceptable salts" is meant to include salts of the active compounds which are prepared with relatively nontoxic acids or bases, depending on the particular substituents found on the compounds described herein. When compounds of the present disclosure contain relatively acidic functionalities, base addition salts can be obtained by contacting the neutral form of such compounds with a sufficient amount of the desired base, either neat or in a suitable inert solvent. Examples of salts derived from pharmaceutically acceptable inorganic bases include aluminum, ammonium, calcium, copper, iron, ferrous, lithium, magnesium, manganese, manganous, potassium, sodium, zinc, and the like. Salts derived from pharmaceutically-acceptable organic bases include salts of primary, secondary and tertiary amines, including substituted amines, cyclic amines, naturally-occuring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like. When compounds of the present disclosure contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, malonic, benzoic, succinic, suberic, fumaric, mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like. Certain specific compounds of the present disclosure contain both basic and acidic functionalities that allow the compounds to be converted into either base or acid addition salts.

The neutral forms of the compounds may be regenerated by contacting the salt with a base or acid and isolating the parent compound in the conventional manner. The parent form of the compound differs from the various salt forms thereof in certain physical properties, such as solubility in polar solvents, but in addition to the above, those salts are equivalent to the parent form of the compound for the purposes of the present invention.

In addition to salt forms, the present disclosure provides compounds which are in a prodrug form. Prodrugs of the compounds described herein are those compounds that readily undergo chemical changes under physiological conditions to provide the compounds of the present disclosure. Additionally, prodrugs can be converted to the compounds of the present disclosure by chemical or biochemical methods in an ex vivo environment. For example, when placed in a transdermal patch reservoir containing suitable enzymes or chemical reagents, the prodrug can be slowly converted to the compound of the invention.

Certain compounds of the present disclosure can exist in unsolvated forms as well as solvated forms, including hydrated forms. The solvated forms are generally equivalent to the non-solvated forms and should be included in the scope of the present invention. Certain compounds of the present disclosure may exist in polycrystallie or amorphous forms. Generally, as for the application considered in the present invention, all physical forms are equivalent and should be included in the scope of the present invention.

Certain compounds of the present disclosure possess asymmetric carbon atoms (optical centers) or double bond; the racemates, diastereomers, geometric isomers, regioisomers and individual isomers (e.g., separate enantiomers) are all intended to be encompassed within the scope of the present disclosure. When compounds are provided herein with an identified stereochemistry (indicated as R or S, or with dashed or wedge bond designations), those compounds will be understood by one of skill in the art to be substantially free of other isomers (e.g., at least 80%, 90%, 95%, 98%, 99%, and up to 100% free of the other isomer).

The compounds of the present disclosure may also contain unnatural proportions of isotope atomic isotopes at one or more of isotopic atoms that constitute such compounds. The unnatural proportions of certain isotope can be defined as the amount from the naturally found amount of the atom discussed to 100% of that atom. For example, the compounds may incorporate radioactive isotopes, such as tritium (³H), iodine-125 (¹²⁵I) or carbon-14 (¹⁴C), or non-radioactive isotopes, such as deuterium (²H) or carbon-13 (¹³C). Such isotopic variants may provide additional uses in addition to those described in this application. For instance, isotopic variants of the compounds of the disclosure may find additional utility, including but not limited to, as diagnostic and/or imaging reagents, or as cytotoxic/radiotoxic therapeutic agents. Additionally, isotopic variants of the compounds of the disclosure can have altered pharmacokinetic and pharmacodynamic characteristics which can contribute to enhanced safety, tolerability or efficacy during treatment. All isotopic variations of the compounds of the present disclosure, whether radioactive or not, should be encompassed within the scope of the present disclosure.

### Targeted Enzyme Degradation (TED) Platform

The present invention provides a Targeted Enzyme Degradation (TED) platform on basis of the conjugate of the present invention, which utilizes the "intracellular cleaner" - ubiquitin proteasome system.

Typically, according to the TED technology of the present invention, which can utilize cell's intrinsic protein destruction mechanism to remove specific oncogenic and pathogenic proteins from the cell, therefore it is an alternative method of targeted therapy.

Different from action mechanism of conventional protein inhibitors, TED technology of the present invention relates to a bifunctional hybrid compound, one side of which is used to bind target proteins, and another side is used to bind E3 ligases, enabling the target proteins binding the E3 ligases, and the target proteins being ubiquitinated, thereby being degraded by the proteome. Theoretically, TED technology only provides binding activity without functional activity that directly inhibiting the target protein, and can be reused. Therefore, TED technology has excellent application prospects.

### Polypeptide element

As used herein, the term "polypeptide element" includes peptide fragments (such as oligopeptide comprising 3-20 aa) or proteins. In addition, this term also includes intact proteins or fragments thereof. Preferred polypeptide elements include antibodies (such as intact antibodies, single-chain antibodies, nanobodies, Fab), especially those antibodies against tumor cell markers (such as tumor markers located on the surface of tumor cells, such as receptors on the cell surface) or inflammatory factors (such as inflammatory factors associated with autoimmune diseases).

As used herein, term "antibody" or "immunoglobulin" is a heterotetrameric glycoprotein of about 150,000 daltons with the same structural characteristics, which consists of two identical light chains (L) and two identical heavy chains (H). Each light chain is connected to the heavy chain by a covalent disulfide bond, and the number of disulfide bonds between the heavy chains of different immunoglobulin isotypes are different. Each heavy and light chain also has regularly spaced intrachain disulfide bonds. Each heavy chain has a variable region (VH) at one end, followed by multiple constant regions. There are a variable region (VL) at one end of each light chain and a constant region at the other end. The constant region of the light chain is opposite the first constant region of the heavy chain, and the variable region of the light chain is opposite the variable region of the heavy chain. Special amino acid residues form an interface between the variable regions of the light chain and the heavy chain.

As used herein, terms "single-domain antibody" and "nanobody" have the same meaning, and refer to cloning the variable region of the heavy chain of an antibody, and constructing a single-domain antibody consisting of only one heavy chain variable region, which is the smallest antigen-binding fragment that having complete functions. Usually, after obtaining an antibody naturally missing constant region 1 (CH1) of light chain and heavy chain, the variable region of the antibody heavy chain is cloned to construct a single domain antibody consisting of only one heavy chain variable region.

As used herein, term "variable" means that certain parts of the variable region of the antibody are different in sequence, which forms the binding and specificity to specific antigens of various specific antibodies. However, variabilities are not evenly distributed throughout the variable regions of antibodies. It is concentrated in three fragments that are called complementarity determining regions (CDR) or hypervariable regions in the variable regions of light chain and heavy chain. More conservative parts of the variable region are called the framework region (FR). The variable regions of the natural heavy and light chains each contain four FR regions, which are in a roughly β-folded conformation and are linked by three CDRs that form a linking loop, which in some cases can form a partially folded structure. The CDRs in each chain are closely placed together through the FR regions and form the antigen binding site of the antibody together with the CDRs in other chain (see Kabat et al., NIH Publ. No. 91-3242, Volume I, pages 647-669 (1991)). Constant regions do not directly participate in the binding of antibodies to antigens, but they exhibit different effector functions, such as participating in antibody-dependent cytotoxicity of antibodies.

The "light chains" of vertebrate antibodies (immunoglobulins) can be classified in one of two distinct categories (called κ and λ) based on the amino acid sequence of constant regions thereof. According to the amino acid sequence of the constant region in heavy chain thereof, immunoglobulins can be classified into different types. There are five main classes of immunoglobulins: IgA, IgD, IgE, IgG and IgM, some of which can be further classified into subclasses (isotypes), such as IgG1, IgG2, IgG3, IgG4, IgA and IgA2. The constant regions in heavy chains corresponding to different classes of immunoglobulins are called α, δ, ε, γ, and µ respectively. The subunit structures and three-dimensional configurations of different classes of immunoglobulins are well known to those skilled in the art.

Generally, the antigen-binding properties of antibodies can be described by 3 specific regions located in the variable regions of the heavy and light chains, called variable regions (CDR), which are divided into 4 framework regions (FRs). The amino acid sequence of 4 FRs is relatively conservative and does not directly participate in the binding reaction. These CDRs form a loop structure, and the β-pleated sheet formed by the FRs in between are close to each other in space structure, and the CDRs on the heavy chain and the corresponding CDRs on the light chain constitute the antigen binding site of the antibody. It can be determined by comparing the amino acid sequences of antibodies of the same type which amino acids constitute the FR or CDR regions.

In the present invention, the polypeptide elements can include not only intact antibodies, but also fragments of antibodies with immunological activity (such as Fab or (Fab) ₂ fragment; heavy chain of antibodies; or light chain of antibodies) or fusion proteins formed by antibodies and other sequences. Therefore, the present invention also includes fragments, derivatives and analogs of the antibodies.

### Targeting Ligand

Targeting ligands (or moiety of target protein or target protein ligand or ligand) are small molecules that capable of binding to interesting target protein.

Some embodiments of this application relate to target molecules. Representative target molecules include but are not limited to folic acid, Hsp90 inhibitors, kinase inhibitors, MDM2 inhibitors, compounds targeting proteins containing human BET bromodomain, compounds targeting cytoplasmic signaling protein FKBP12, HDAC inhibitors, human lysine methyltransferase inhibitors, angiogenesis inhibitors, immunosuppressive compounds and compounds targeting aryl hydrocarbon receptor (AHR).

In certain embodiments, the targeting ligand is capable of binding kinases, BET bromodomain-containing proteins, cytoplasmic signaling proteins (such as FKBP12), nucleoproteins, histone deacetylases, lysine methyl transferase, protein regulating angiogenesis, proteins regulating immune response, aromatic hydrocarbon receptors (AHRs), estrogen receptors, androgen receptors, glucocorticoid receptors, or transcription factor (e.g., SMARCA4, SMARCA2, TRIM24).

In certain embodiments, kinases, to which targeting ligands are able to bind, include, but not limited to Tyrosine kinases (for example, AATK, ABL, ABL2, ALK, AXL, BLK, BMX, BTK, CSF1R, CSK, DDR1, DDR2, EGFR, EPHA1, EPHA2, EPHA3, EPHA4, EPHA5, EPHA6, EPHA7, EPHA8, EPHA10, EPHB1, EPHB2, EPHB3, EPHB4, EPHB6, ERBB2, ERBB3, ERBB4, FER, FES, FGFR1, FGFR2, FGFR3, FGFR4, FGR, FLT1, FLT3, FLT4, FRK, FYN, GSG2, HCK, HRAS, HSP90, IGF1R, ILK, INSR, INSRR, IRAK4, ITK, JAK1, JAK2, JAK3, KDR, KIT, KRAS, KSP, KSR1, LCK, LMTK2, LMTK3, LTK, LYN, MATK, MERTK, MET, MLTK, MST1R, MUSK, NPR1, NRAS, NTRK1, NTRK2, NTRK3, PDGFRA, PDGFRB, PLK4, PTK2, PTK2B, PTK6, PTK7, RET, ROR1, ROR2, ROS1, RYK, SGK493, SRC, SRMS, STYK1, SYK, TEC, TEK, TEX14, TIE1, TNK1, TNK2, TNNI3K, TXK, TYK2, TYRO3, YES1 or ZAP70), Serine/threonine kinase (such as Casein Kinase 2, protein kinase A, protein kinase B, protein kinase C, Raf kinase, CaM kinase, AKT1, AKT2, AKT3, ALK1, ALK2, ALK3, ALK4, Aurora A, Aurora B, Aurora C, CHK1, CHK2, CLK1, CLK2, CLK3, DAPK1, DAPK2, DAPK3, DMPK, ERK1, ERK2, ERK5, GCK, GSK3, HIPK, KHS1, LKB1, LOK, MAPKAPK2, MAPKAPK, MEK, MNK1, MSSK1, MST1, MST2, MST4, NDR, NEK2, NEK3, NEK6, NEK7, NEK9, NEK11, PAK1, PAK2, PAK3, PAK4, PAK5, PAK6, PIM1, PIM2, PLK1, RIP2, RIP5, RSK1, RSK2, SGK2, SGK3, SIK1, STK33, TAO1, TAO2, TGF-β, TLK2, TSSK1, TSSK2, MLK1 or MLK2), cyclin-dependent kinases (such as Cdk1-Cdk11) and Leucine-rich repetitive kinase (such as LRRK2).

### Target molecule

In the conjugates of formula I of the present application, the conjugate binds to target proteins through R^{T} (the moiety of target molecule) .

In the present invention, the target molecule can be a target molecule A, a target molecule T, or the combination thereof.

In the present invention, the target molecule can be any inhibitor of the target protein. The target molecule can be a highly effective inhibitor of the target protein, or an inhibitor with relatively poor activity. Specifically, the target molecule of the present invention may be a small molecule inhibitor known in the art against any target protein in the art.

In some embodiments, the target molecule used herein has a radical, such as -O-, -NR^{a}-(wherein R^{a} is H, or substituents such as C1-C6 alkyl, -CO-, -COO-, and the like), that is able to connect to a linker molecule of the present invention (e.g. L1 in the present invention) monovalently to form an ether, an amine, an amide and the like, thereby forming the moiety of target molecule.

The target protein may be a variety of target proteins known in the art, representative examples include, but are not limited to MDM2, AKT, BCR-ABL, Tau, BET(BRD2, BRD3, BRD4), ERRα, FKBP12, RIPK2, ERBB3, androgen receptor, MetAP2, TACC3, FRS2α, PI3K, DHFR, GST, Halo Tag, CRABPI, CRABPII, RAR, aromatic hydrocarbon receptor, estrogen receptor. Different target proteins and some corresponding inhibitors can be obtained commercially or prepared by conventional methods. For example, as for MDM2, the inhibitors thereof can be referred to documents such as WO 2017176957, WO2017176958A1.

In another specific embodiment, R^{T} is selected from Table B

**Table B**

| | | | |
|---|---|---|---|
| 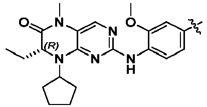 | 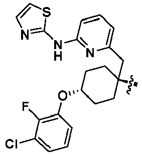 | 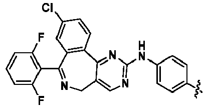 | 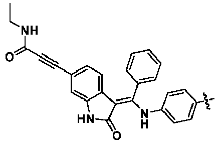 |
| P1 | P2 | P3 | P4 |

### E3 Ligase Ligand

In the present invention, the moiety of E3 ligase ligand (R_{E3}) is used for binding E3 ligase.

In a specific embodiment, representative moieties of E3 ligase ligand have a structure of formula A1 or A2:

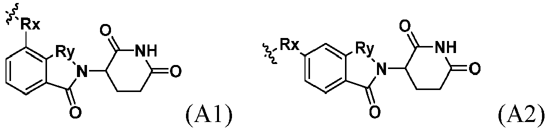

In formula A, R_{X} is selected from null, C1-C6 alkyl, C2-C6 alkenyl, C2-C6 alkynyl, O, NH, S, CO or SOₙ (n is 1 or 2) and the like; R_{Y} is CH₂, C=S, CO; and the E3 ligase ligand (R_{E3} in formula I) is able to connect to L1 of the present invention via Rx group in the E3 ligase ligand, such as -Rₓ-L1-R_{T} (such as -O-L1-R_{T});
or, representative moieties of E3 ligase ligand have a structure of formula A1b: 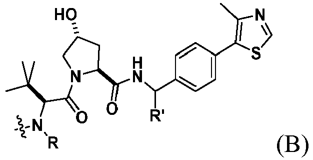 in formula A1b, R' is H or C1-C6 alkyl (such as Me), R is H, or C1-C6 alkyl (such as Me or Et).

In some embodiments, the E3 ligase ligand used herein has a radical, such as -O-, -NR^{a}-(wherein R^{a} is H, or substituents such as C1-C6 alkyl and the like, -CO-, -COO-, and the like), that is able to connect to a linker molecule of the present invention (e.g. L1 in the present invention and the like) monovalently to form an ether, an amine, an amide and the like.

In another specific embodiment, R_{E3}(moiety of E3 ligase ligand) used herein is selected from Table C:

**Table C**

| | | | |
|---|---|---|---|
| 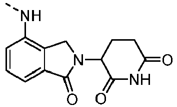 | 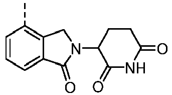 | 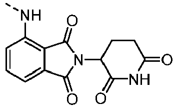 | 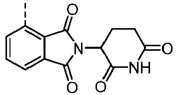 |
| A1.1 | A1.2 | A 1.3 | A1.4 |
| 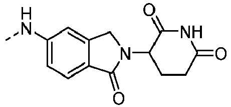 | 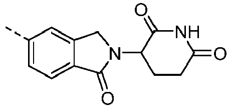 | 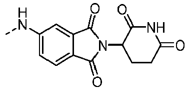 | 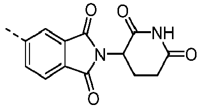 |
| A2.1 | A2.2 | A2.3 | A2.4 |
| 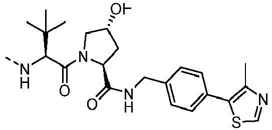 | | | |
| B1 | | | |

### Linker molecule (L1 as described herein)

The linker molecules of the present invention are used for connecting the target molecule and the E3 ligase ligand. For example, it can be connected to the target molecule or the E3 ligase ligand through functional groups at both ends (such as -OH, -SH, -NH₂, -NHR, -SOOH or -COOH) ; wherein R is selected from: substituted or unsubstituted C1-C10 alkyl, -(C=O)-R', (C=O)NH-R', -NH(C=O)- R', -SO₂-R', -NHSO₂-R', -SOzNH-R ', -SO-R', -NHSO-R', -SONH-R', -PO₃-R', -NHCOO-R', -COO-R' or -NH-CO-NH- R', -NH -CO-O-R' or -X'-L3-Z; where L3 is a linking group, and Z is a polypeptide element (such as a ligand, antibody or peptide fragment, etc.) or a targeting molecule such as a targeting function Small molecules (such as folic acid, HSP90 inhibitors, etc.).

### Linker and coupling method

The linker L1 of the present invention is used for connecting the target molecule (moiety) P1 and the E3 ligase ligand (moiety) A1.

Preferably, the target molecule (moiety) or the E3 ligase ligand (moiety) can be connected with the linker through -O-, -S-, -NH-, -NR-, -(C=O)-, -(C=O)O-, -SOz- and other groups.

The linker of the present invention may further contain a varity of other functional groups, such as -OH, -NHR, -SH and the like.

Typically, the linker of the present invention L1, can be represented by the following general formula II:

-W¹-L2-W²-

In the formula, the definition of W¹, L2, and W² are as described in the first aspect of the present invention.

In another preferred embodiment, W¹ and W² are each independently divalent groups formed by the loss of 1 hydrogen atom forming bivalence from the following monovalent groups: -OH, -NH₂, -SH, -COOH, -SOzH and the like. For example, the connection mode of the linker and the target molecule can a connection through the linker group shown as below: 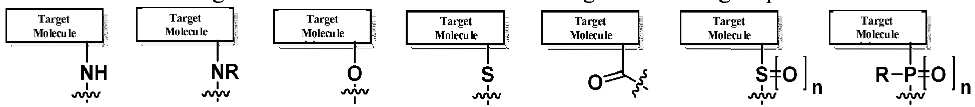 alternatively, W¹ and W² each independently comprise a divalent linking group having a rigid portion (e.g., a portion of 4-membered, 5-membered, or 6-membered aliphatic ring (saturated carbocyclic ring) , or a portion of 5-membered or 6-membered aromatic heterocyclic ring, etc.), exemplary examples of which are shown below and in examples. 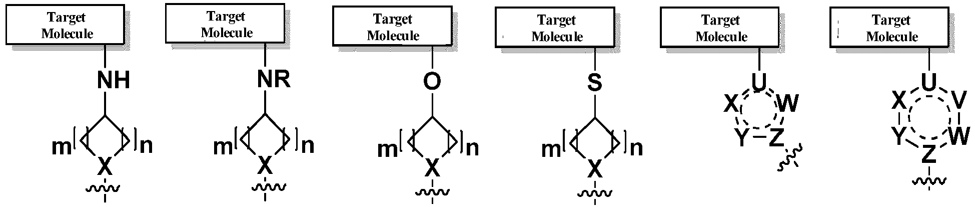 wherein, R in the each of above formulas is defined as above; n is 1 or 2 or 3.

In a specific embodiment, W¹ and W² are each independently selected from the group consisting of
null, -N(R^{a})-, -C(R^{b})₂-, -N(R^{a})-C(R^{b})₂-, -C(=O)-, -C(O)-N(R^{a})-, -C(R^{b})₂-C≡C-, -C=C-, - C(O)-C=C-, -CH(OH)-C=C-, -O-, -S-, -SOz-, -SO-, -PO₃-, -C(R^{b})=C(R^{b})-, substituted or unsubstituted C3-8 cycloalkyl, substituted or unsubstituted 4 to 10 membered heterocycloalkyl, substituted or unsubstituted C6-10 aryl, and substituted or unsubstituted 5 to 10 membered heteroaryl.

### Active Ingredients

As used herein, the term "compound of the invention" refers to the compound or the conjugate of formula I. The term also comprises the crystal forms, or pharmaceutically acceptable salts of compound of formula (I).

Specifically, the present invention provides a class of conjugates of formula I that are suitable for further attaching with the polypeptide elements (e.g., an antibody, a protein ligand, etc.) or target molecule T, or that are coupled with polypeptide elements or target molecule T:

R_{T}-L1-R_{E3} (I)

wherein R_{L} is a moiety of E3 Ligase Ligand; R_{T} is a moiety of target molecule; L1 is a linker connecting the moieties of A1 and P1.

Preferably, R_{L}, R_{T} and L1 are defined as above.

In a specific embodiment, the conjugate provided by the present invention that is suitable for further attaching with the polypeptide elements or the target molecule T is shown in formula IV:

R_{T}-W¹-L6-W²-R_{E3} (IV)

wherein R_{T}, R_{E3}, W¹, W² and L7 are defined as above.

In a specific embodiment, the conjugate provided by the present invention that is attached with the polypeptide elements or the target molecule T is shown in formula V;

R_{T}-W¹-L7-W²-R_{E3} (V);

wherein R_{T}, R_{E3}, W¹, W² and L7 are defined as above.

In a specific embodiment, the present invention further provides the conjugate as shown in R_{T}-W¹-L5-W^{b}-C≡C-R_{E3}(1-1), R_{T}-W¹-L5-CO-R_{E3}(1-2), or R_{T}-W¹-L5-CONH-R_{E3}(1-3); wherein W^{b} is defined the same as W; W¹, R_{T}, R_{E3} and L5 are defined as above.

In another preferred embodiment, in formula 1-1, W¹ is selected from the group consisting of NH, and O; preferably, W is NH.

In another preferred embodiment, in formula 1-1, W^{b} is selected from the group consisting of null, -CH₂-, -CH(OH)-, and -C(=O)-.

In a specific embodiment, the present invention provides the conjugate as shown in below 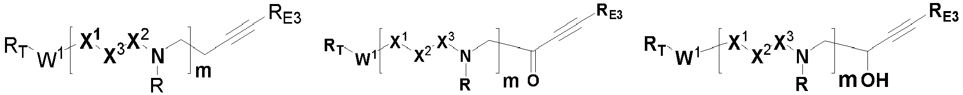
wherein W¹, R_{T}, R_{E3} and R are defined as above; preferably, R is H, C1-6 alkyl (such as Me, Et, etc.);
m=0, 1 2 3, etc. (preferably, m is not 0);
X¹, X² and X³ are each independently selected from O, C₁₋₄ alkylene,

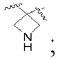

Preferably, W¹ is W, and W is defined as above. More preferably, W¹ is NH.

In a specific embodiment, the present invention further provides the conjugate as shown in below; 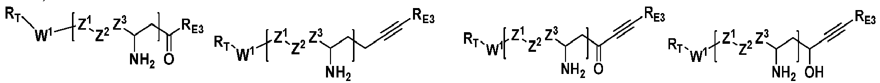 in each formula,
R, R₁, R_{T} and R_{E3} are defined as above;
Z¹, Z² and Z³ are each independently selected from O, C₁₋₄ alkylene, -CH(OH)-, 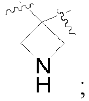
m=0, 1, 2, 3, 4 and other integers.

In another specific embodiment, the conjugate is a conjugate selected from Group 1:

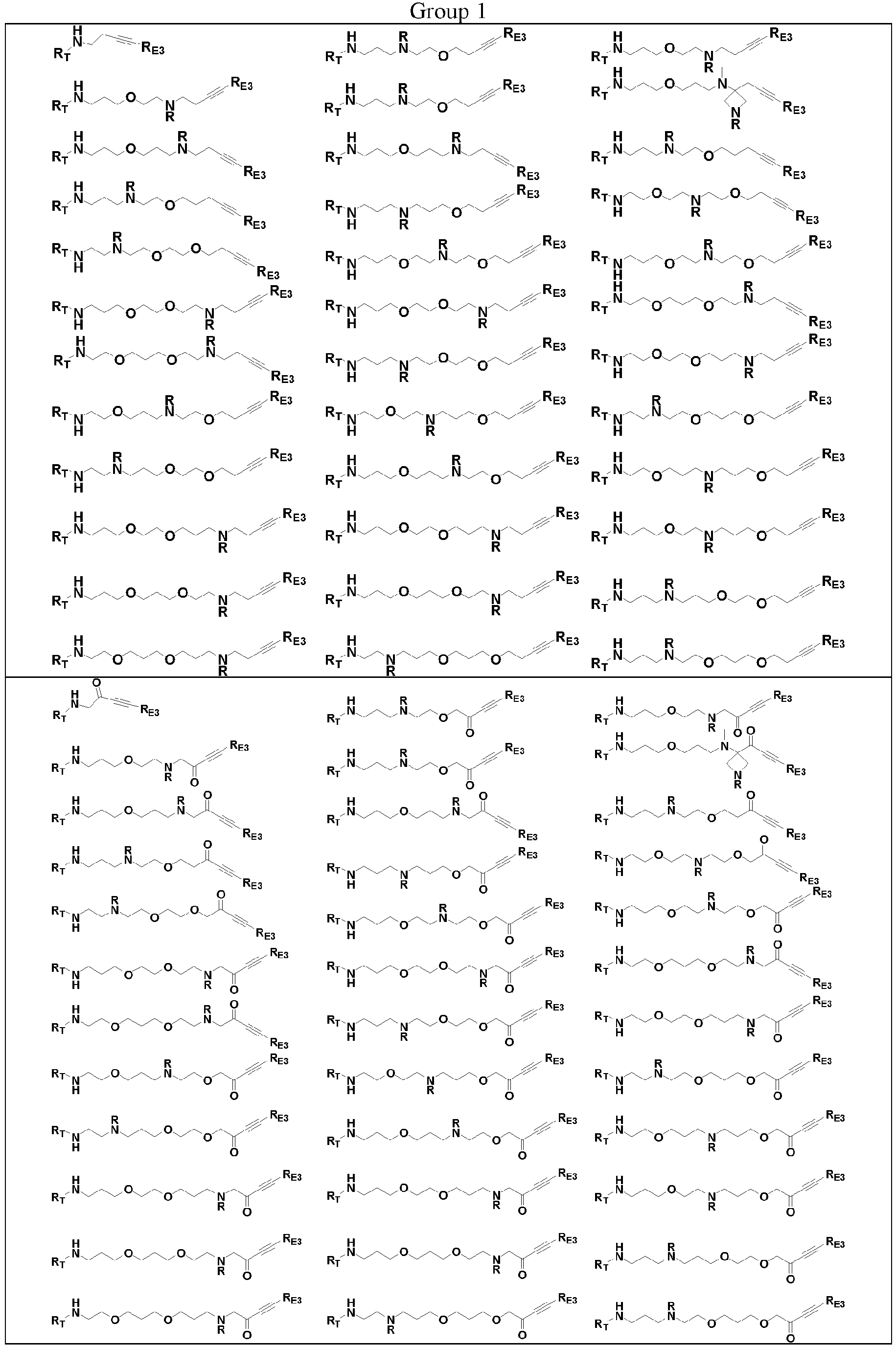

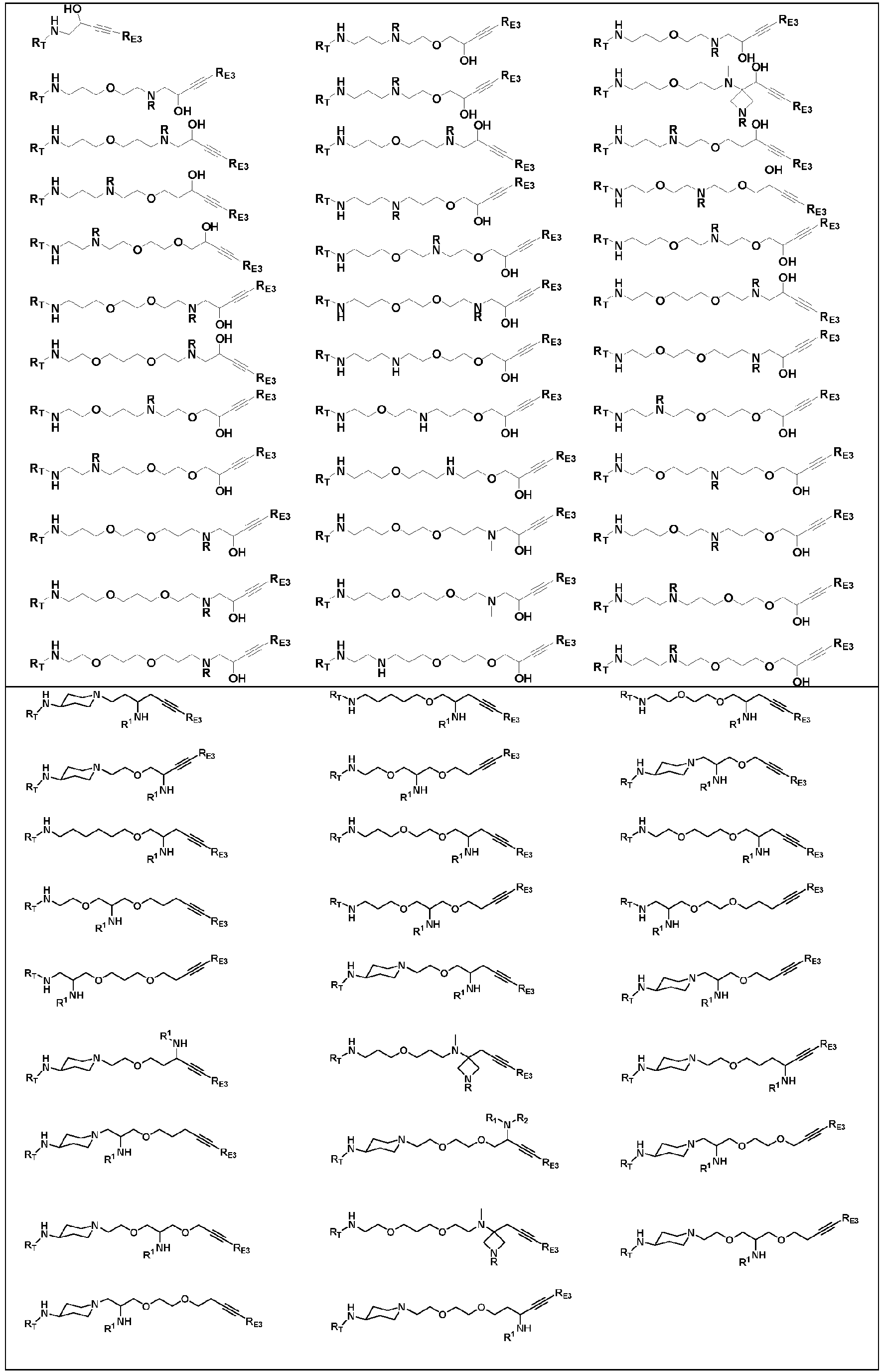

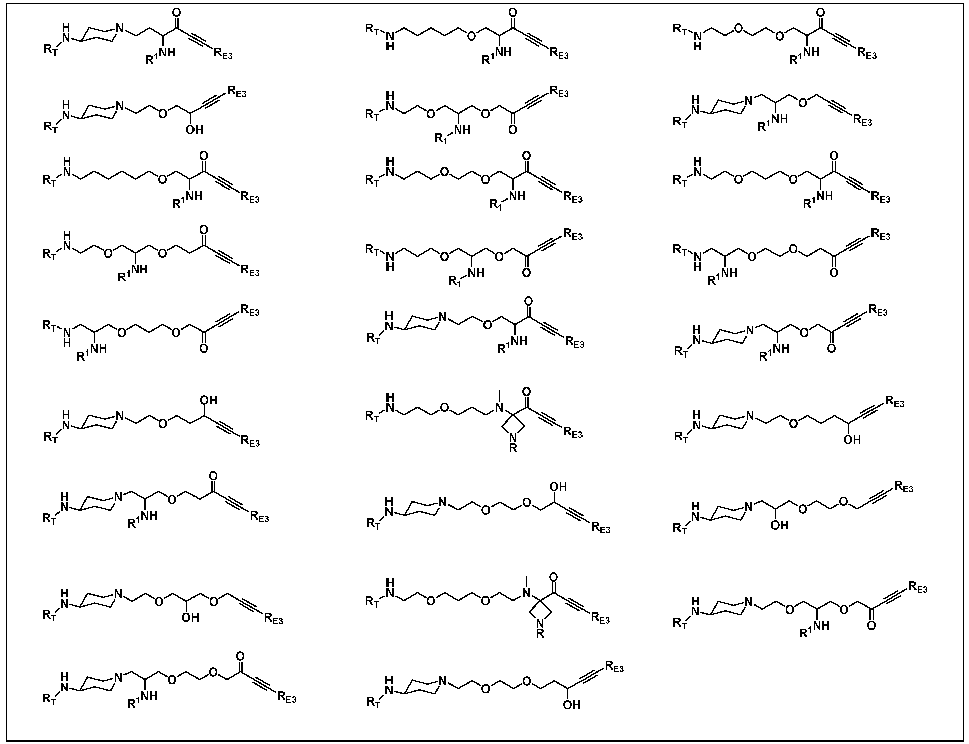

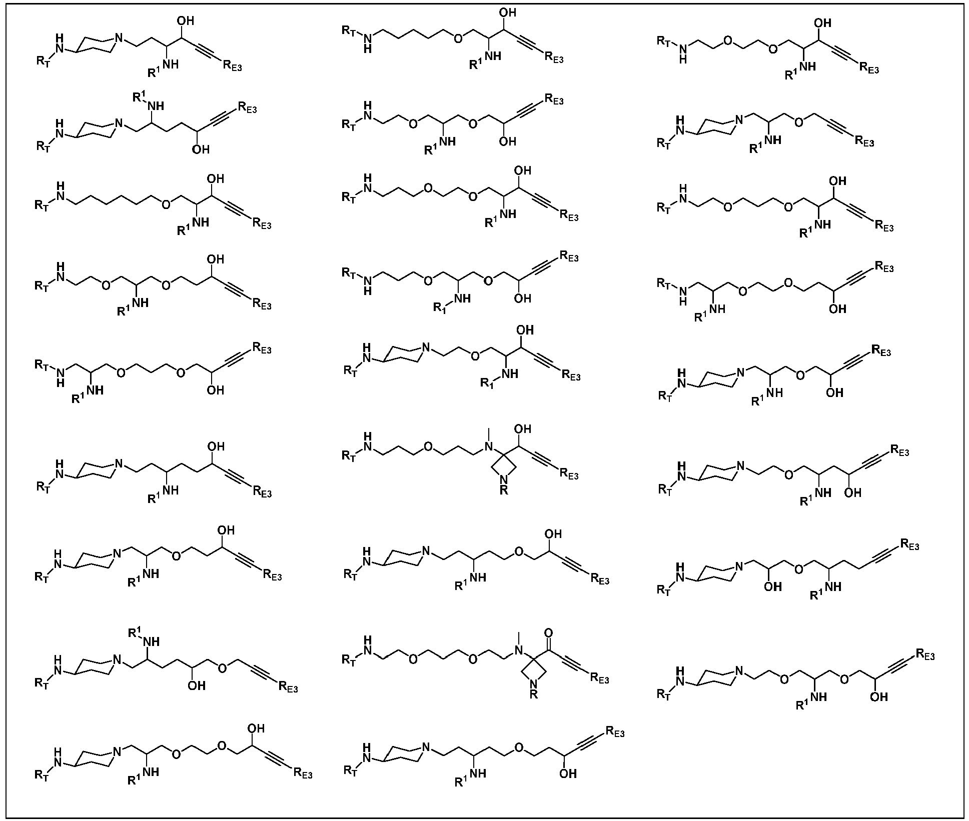

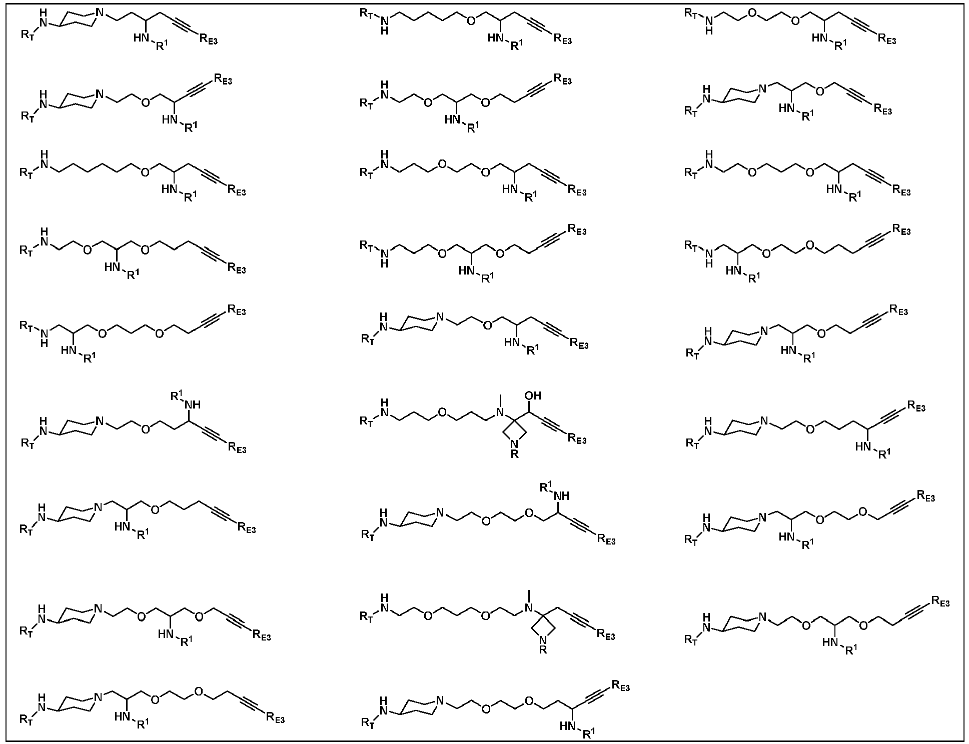

wherein R_{T}, R_{E3}, R and R₁ are defined as above; preferably, R and R¹ are each independently -W³-L3-W⁴-(Rp)_{q}, wherein W³, L3, W⁴, R_{P} and m are defined as above.

In a specific embodiment, the present invention further provides the conjugate as shown in R_{T}-W¹-L6-W^{b}-C≡C-R_{E3}(1a-1), R_{T}-W¹-L6-CO-R_{E3}(1a-2), or R_{T}-W¹-L6-CONH-R_{E3}(1a-3);
wherein W^{b} is defined the same as W; W¹, R_{T}, R_{E3} and L5 are defined as above.

In a specific embodiment, the present invention further provides the conjugate as shown in R_{T}-W^{a}-L6-W^{b}-C≡C-R_{E3}; wherein W^{a} and W^{b} are defined the same as W; R_{T}, R_{E3} and L6 are defined as above.

In another preferred embodiment, W^{a} is selected from the group consisting of NH, and O; preferably, W is NH.

In another preferred embodiment, W^{b} is selected from the group consisting of null, -CH₂-, -CH(OH)-, and -C(=O)-.

In another specific embodiment, the conjugate is a conjugate selected from Group 1a:

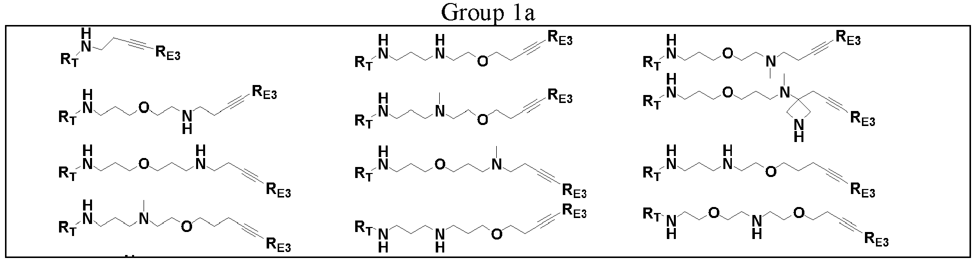

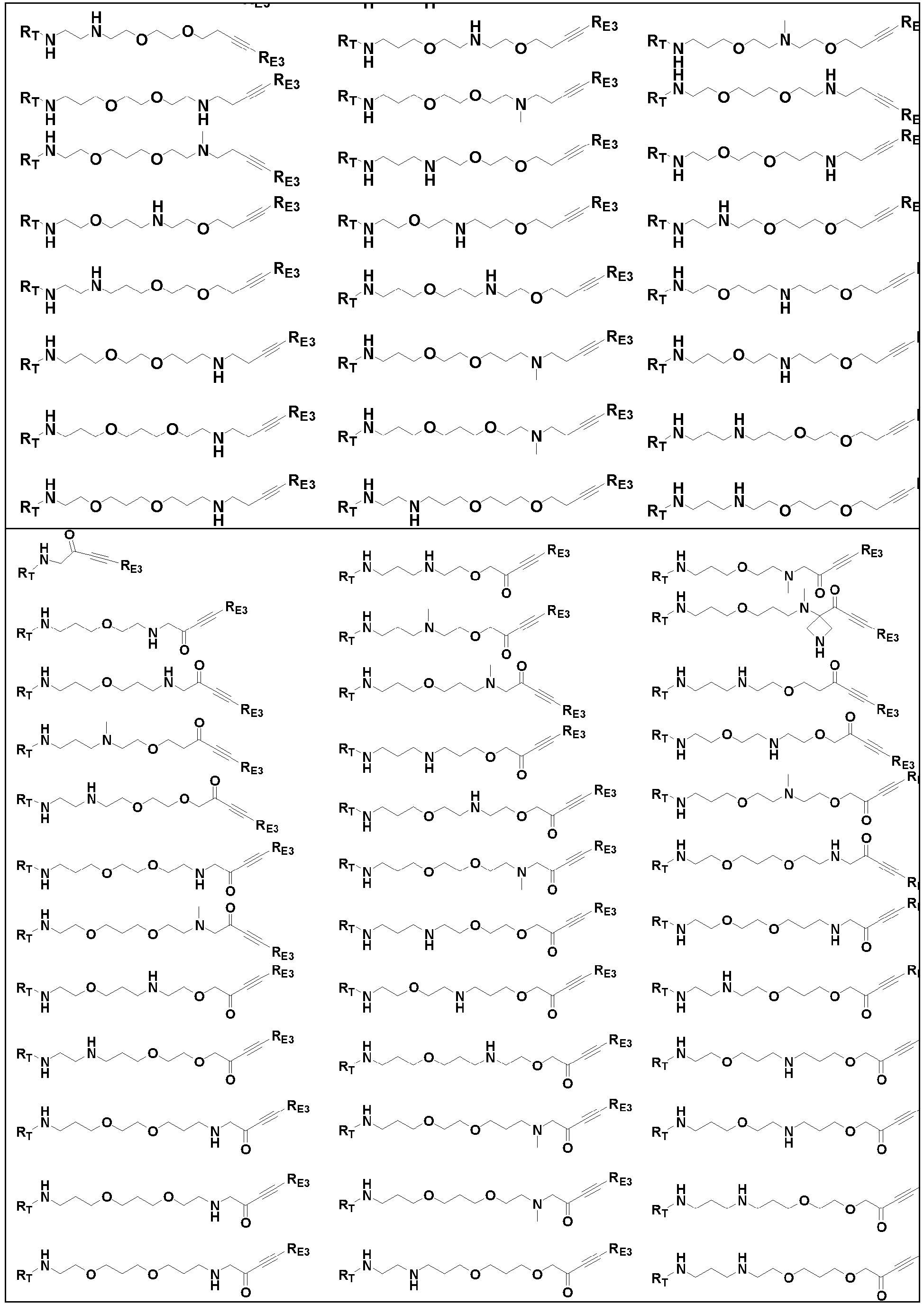

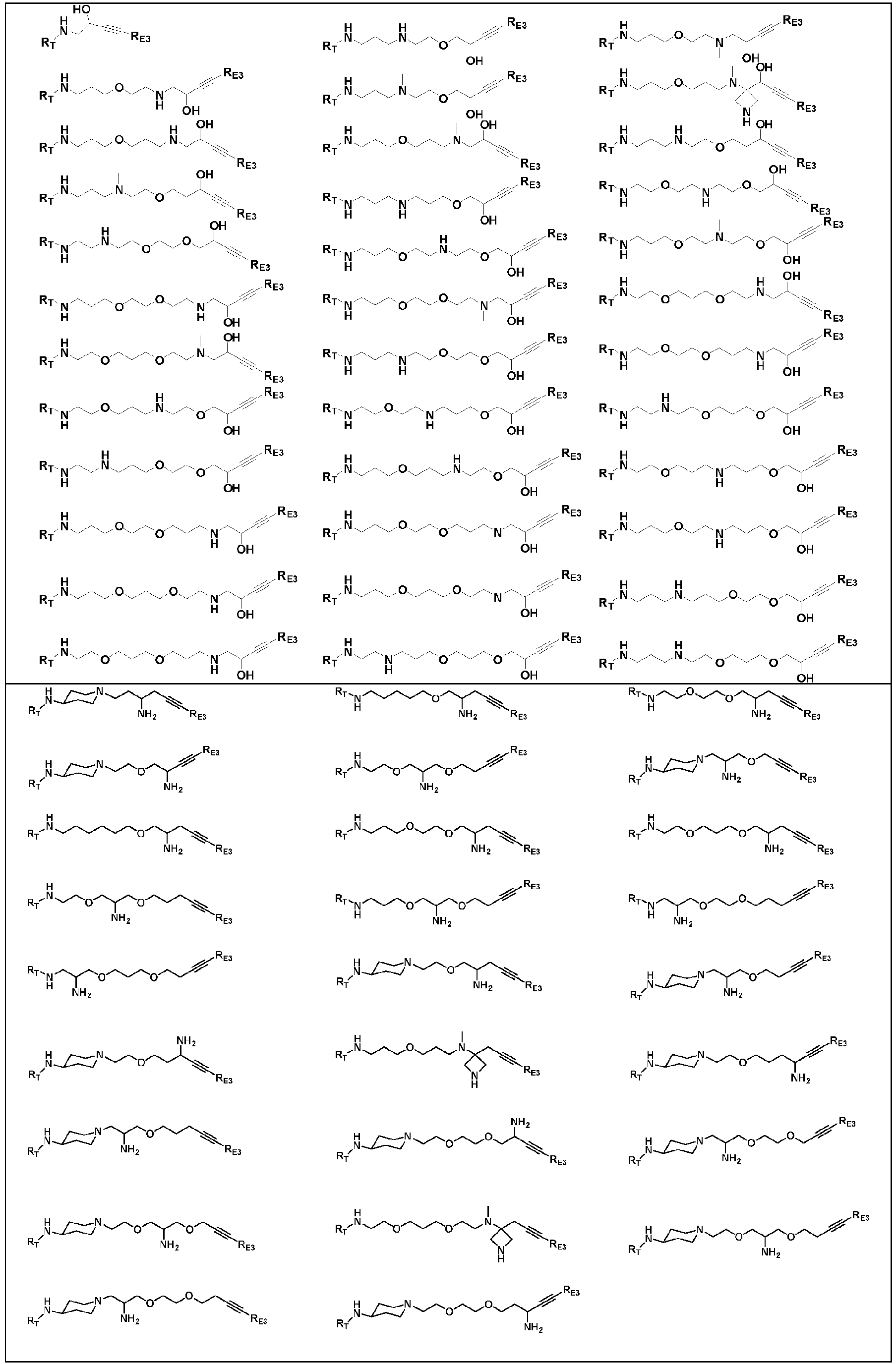

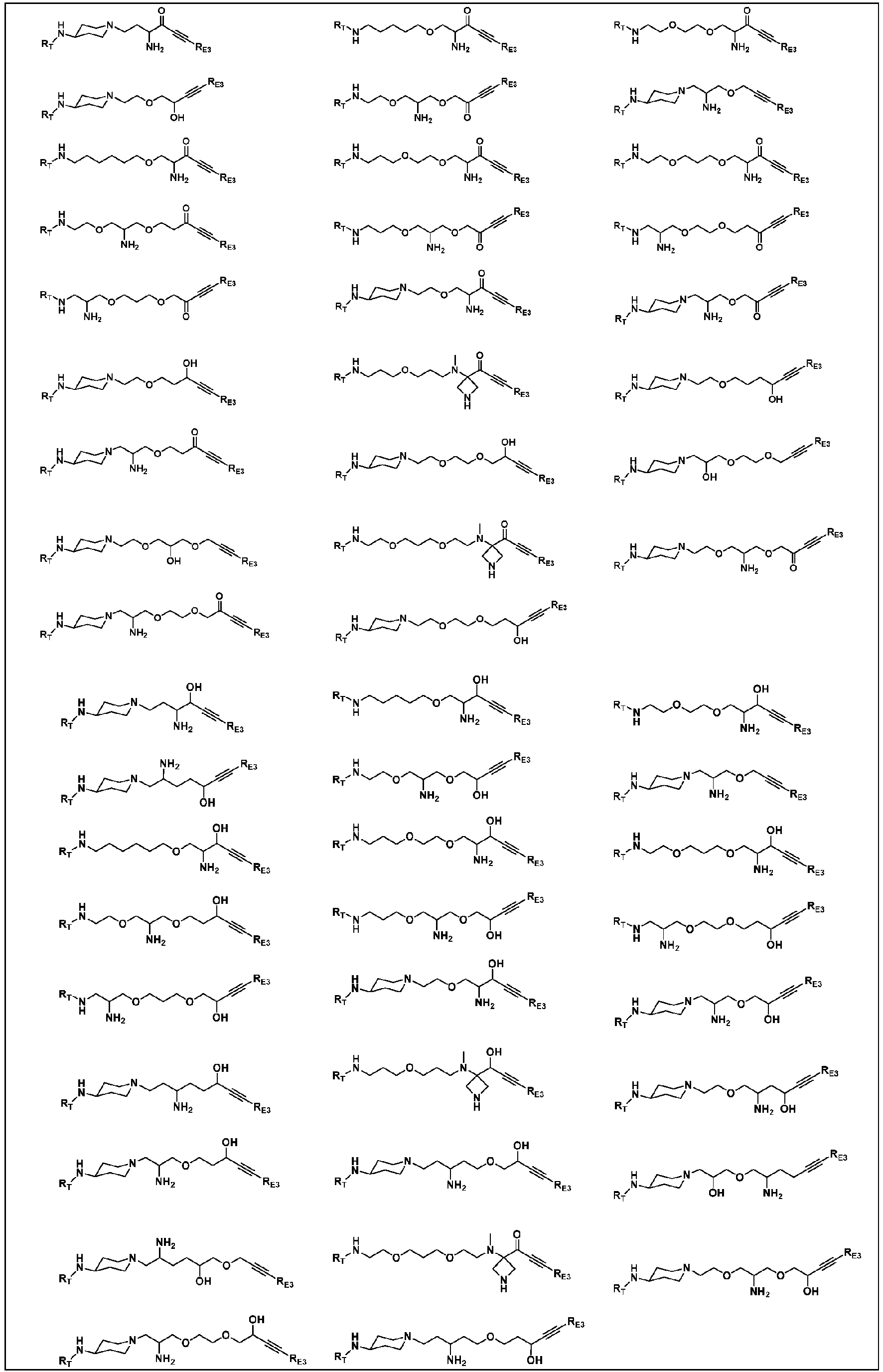

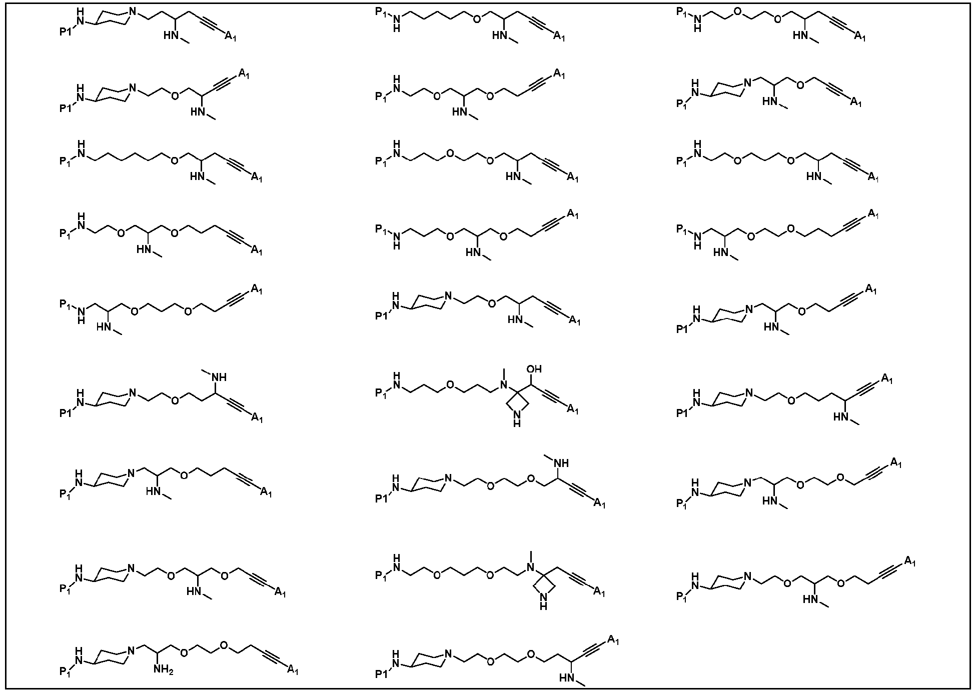

wherein R_{T} and R_{E3} are defined as above.

In a specific embodiment,
the present invention further provides the conjugate as shown in R_{T}-W^{a}-Cr¹-W^{a}-Cr²-L₅-W²-R_{E3} (2);
wherein,
W^{a} is defined the same as W;
Cr¹ is null, or C₄₋₇ cycloalkyl unsubstituted or substituted with C₁₋₄ alkyl, or 4 to 6 membered heterocyclyl unsubstituted or substituted with C₁₋₄ alkyl;
Cr² is 4 to 6 membered heterocyclyl containing nitrogen unsubstituted or substituted with C₁₋₄ alkyl, and at least one of nitrogen heteroatom in Cr² is attached with L5;
W, R_{T}, R_{E3}, W² and L5 are defined as above.

In another preferred embodiment, W² is selected from the group consisting of W^{b}-C≡C, C(=O), and C(=O)NH.

In another specific embodiment, the present invention further provides the conjugate as shown in R_{T}-W^{a}-Cr¹-Cr²-L5-W^{b}-C≡C-R_{E3};
wherein W^{a} and W^{b} are defined the same as W;
Cr¹ is null, or C₄₋₇ cycloalkyl unsubstituted or substituted with C₁₋₄ alkyl, or 4 to 6 membered heterocyclyl unsubstituted or substituted with C₁₋₄ alkyl;
Cr² is 4 to 6 membered heterocyclyl containing nitrogen unsubstituted or substituted with C₁₋₄ alkyl, and at least one of nitrogen heteroatom in Cr² is attached with L5;
R_{T}, R_{E3} and L5 are defined as above.

Preferably, W^{a} is selected from the group consisting of NH, and O; preferably, W^{a} is NH. preferably, W^{b} is selected from the group consisting of null, - CH₂-, -CH(OH)-, and - C(=O)-.

Preferably, the conjugates are selected from the group consisted of:

R_{T}-NH-Cr¹-Cr²-L5-CH₂-C≡C-R_{E3};

R_{T}-NH-Cr¹-Cr²-L5-C(=O)-C≡C-R_{E3};

R_{T}-NH-Cr¹-Cr²-L5-CH(OH)-C≡C-R_{E3};

and R_{T}-NH-Cr¹-Cr²-L5-C≡C-R_{E3} in each formula, R_{T}, R_{E3}, Cr¹, Cr² and L5 are defined as above.

Preferably, the conjugates are selected from the group consisted of

R_{T}-NH-Cr¹-Cr²-L8-C≡C-R_{E3};

wherein R_{T}, R_{E3}, Cr¹, Cr² and L8 are defined as above.

In another preferred embodiment, Cr¹ is null or 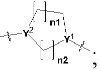 wherein Y¹ are Y² are each independently selected from CH and N; n1=0, 1 or 2; and n2=1 or 2. In another preferred embodiment, Cr² is 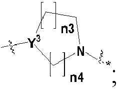 wherein * indicates the position connected with L5; Y³ is selected from CH and N, n3 = 0, 1 or 2; and n4=1 or 2.

In another preferred embodiment, Cr¹ is selected from the group consisting of null, 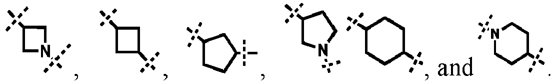 In another preferred embodiment, Cr² is selected from the group consisting of

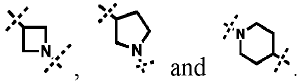

In a specific embodiment, the present invention provides the conjugate as shown in below; 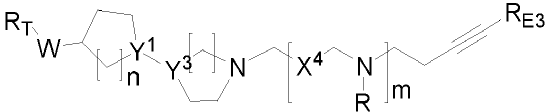 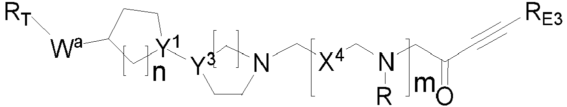 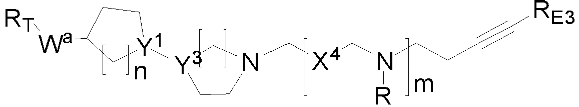 wherein
X⁴ is selected from the group consisting of CH₂, O, NH, and NR;
Y¹ and Y³ are each independently selected from the group consisting of CH, and N;
W^{a} is selected from the group consisting of NH, and O;
m=0, 1, 2, 3, etc. (preferably, m is not 0);
n = 0, 1, 2, 3, etc. (preferably, n is not 0);

R_{T}, R_{E3} and R are defined as above; preferably, R is H, C1-6 alkyl (such as Me, Et, etc.), Ac, CHO, and CONH₂.

In another specific embodiment, the conjugate is a conjugate selected from Group 2:

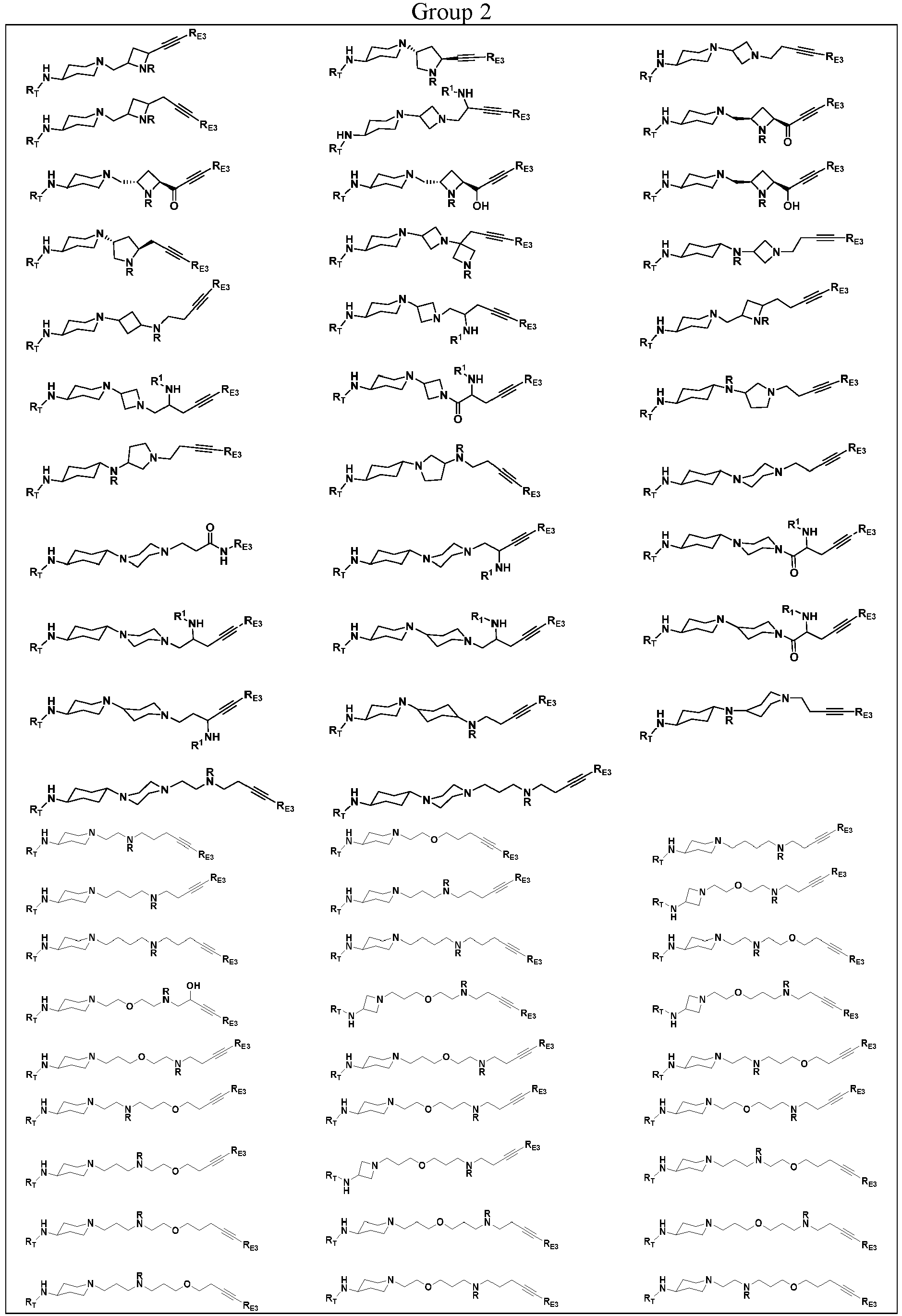

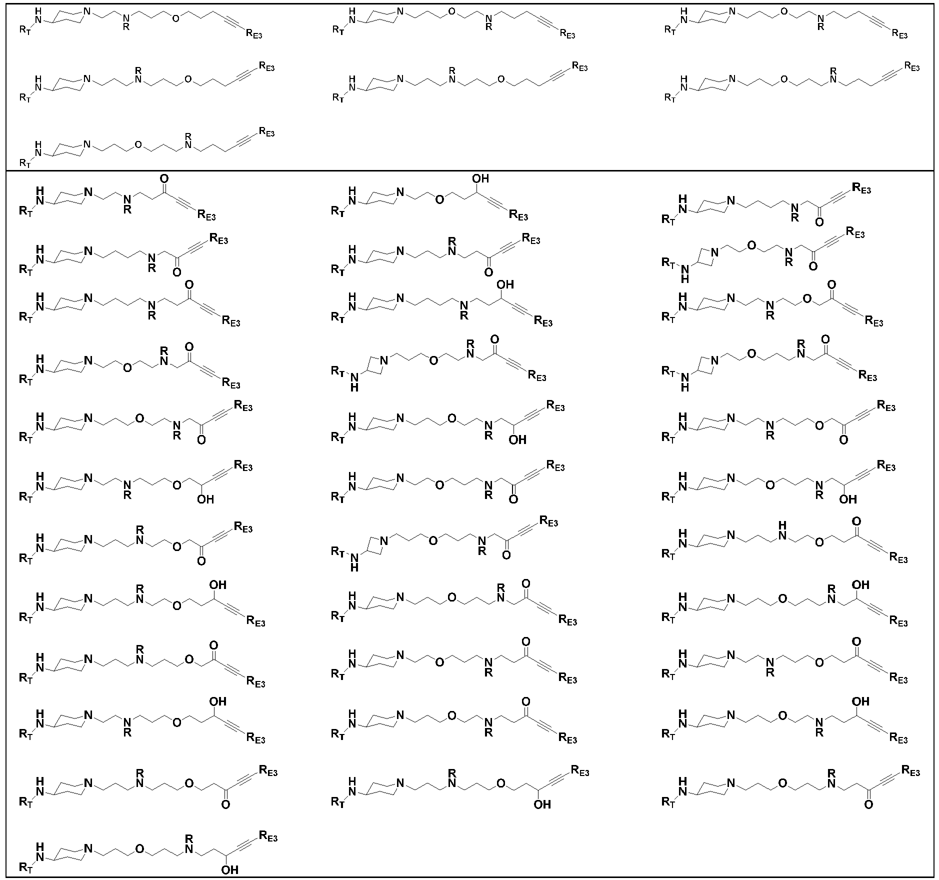

wherein R^{T} R_{E3}, R and R¹ are defined as above; preferably, R and R¹ are each independently -W³-L3-W⁴-(Rp)_{q}, wherein W³, L3, W⁴, R_{P} and m are defined as above.

In a specific embodiment, the present invention further provides the conjugate as shown in R_{T}-W^{a}-Cr¹-W^{a}-Cr²-L6-W²-R_{E3}(I-2a);
wherein,
W^{a} is defined the same as W;
Cr¹ is null, or C₄₋₇ cycloalkyl unsubstituted or substituted with C₁₋₄ alkyl, or 4 to 6 membered heterocyclyl unsubstituted or substituted with C₁₋₄ alkyl;
Cr² is 4 to 6 membered heterocyclyl containing nitrogen unsubstituted or substituted with C₁₋₄ alkyl, and at least one of nitrogen heteroatom in Cr² is attached with L5;
W, R_{T}, R_{E3}, W² and L5 are defined as above.

In another preferred embodiment, W² is selected from the group consisting of W^{b}-C≡C, C(=O), and C(=O)NH.

In a specific embodiment, the present invention further provides the conjugate as shown in R_{T}-W^{a}-Cr¹-Cr²-L6-W^{b}-C≡C-R_{E3}; wherein W^{a}, W^{b}, Cr¹, Cr², R_{T}, R_{E3} and L5 are defined as above.

Preferably, the conjugates are selected from the group consisted of

R_{T}-NH-Cr¹-Cr²-L6-CH₂-C≡C-R_{E3};

R_{T}-NH-Cr¹-Cr²-L6-C(=O)-C≡C-R_{E3};

R_{T}-NH-Cr¹-Cr²-L6-CH(OH)-C≡C-R_{E3};

and

R_{T}-NH-Cr¹-Cr²-L6-C≡C-R_{E3};

in each formula, R_{T}, R_{E3}, Cr¹, Cr² and L6 are defined as above.

In another specific embodiment, the conjugate is a conjugate selected from Group 2a:

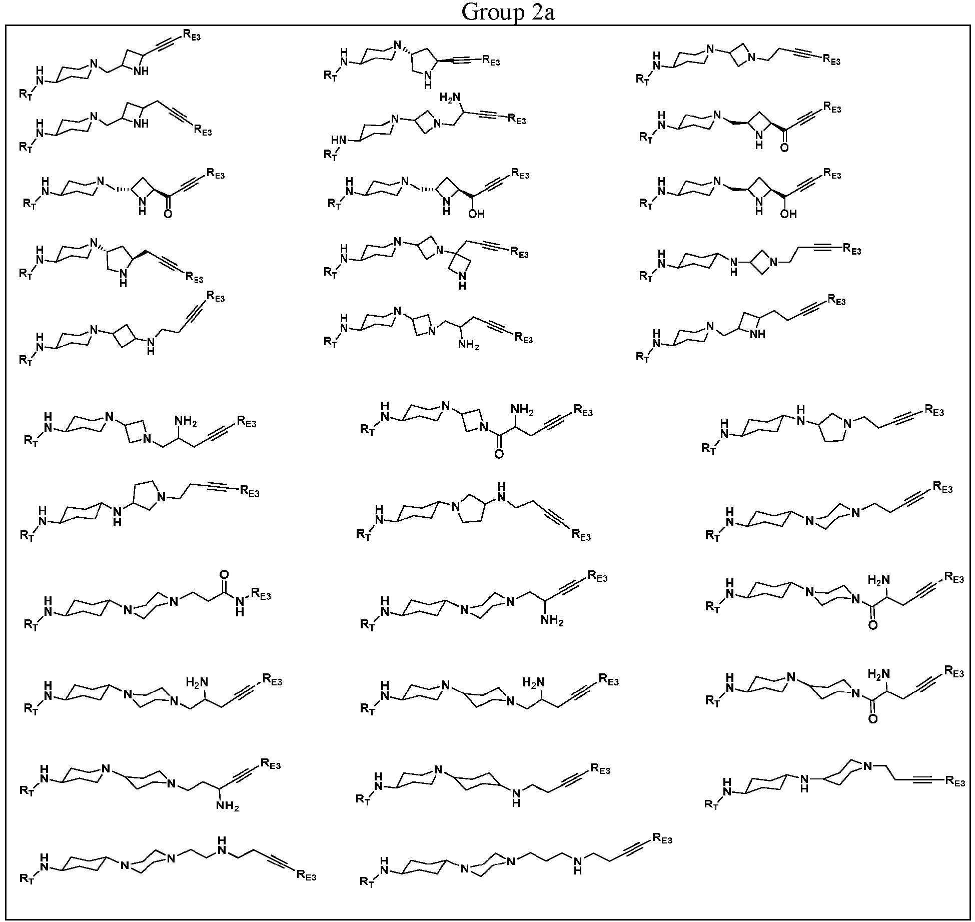

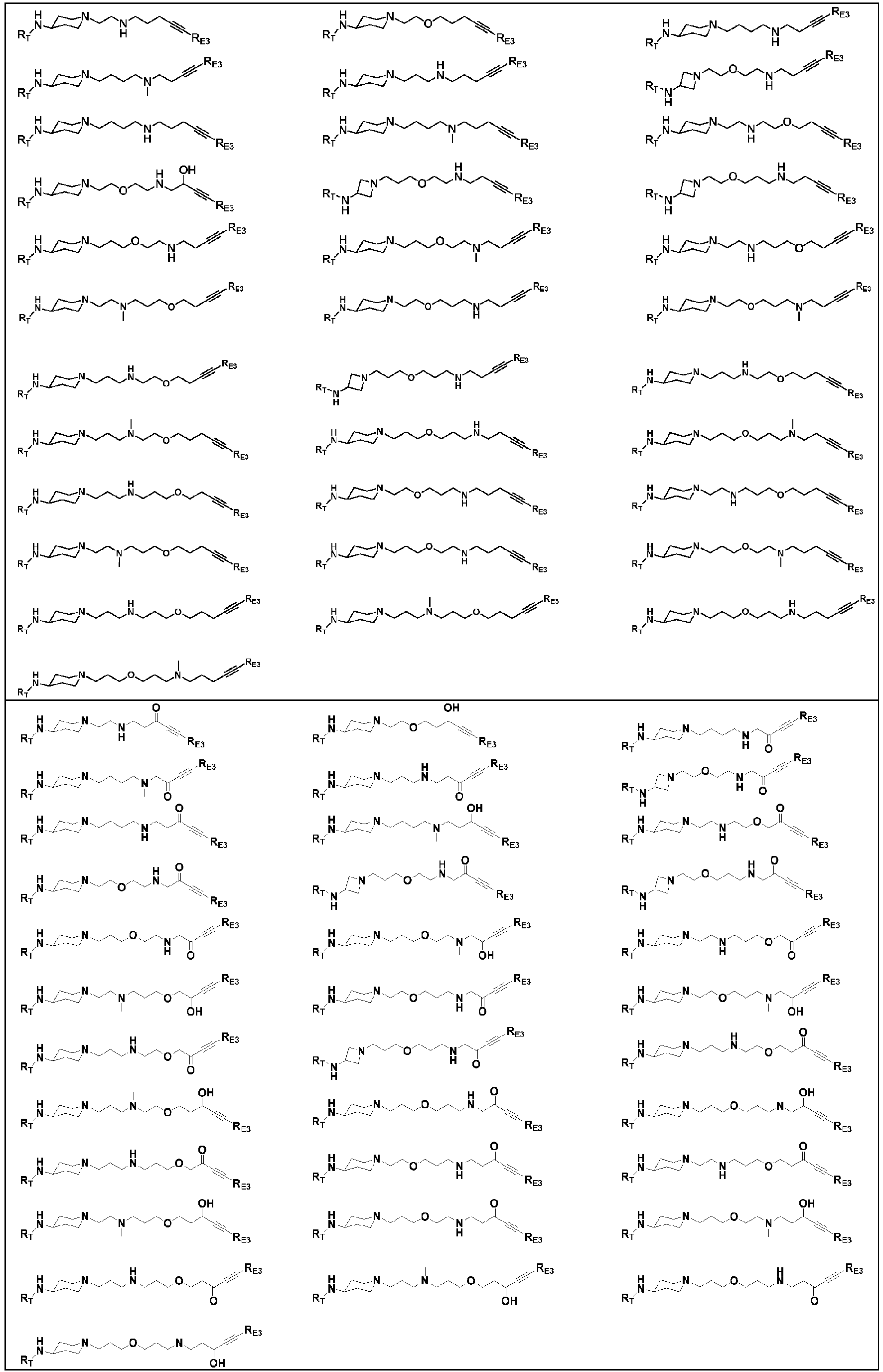

In a specific embodiment, the present invention provides the conjugate as shown in R_{T}-Ar1-L5-W²-R_{E}(3);
wherein Ar1 is -5 or 6 membered heteroaryl containing nitrogen atom-; L5, R_{T}, W2 and R_{E3} are defined as above.

In another preferred embodiment, W2 is selected from -CONH-, -CO-, -CONH-, and - W^{b}-C≡C-.

In a specific embodiment, the present invention provides the conjugate as shown in R_{T}-Ar1-L5-CONH-R_{E3}, R_{T}-Ar1-L5-CO-R_{E3} or R_{T}-Ar1-L5-W^{b}-C≡C-R_{E3};
wherein Ar1 is -5 or 6 membered heteroaryl containing nitrogen atom-; L5, R^{T} and R_{E3} are defined as above.

In another preferred embodiment, Ar1 is 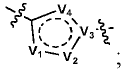 wherein V₁, V₂ and V₄ are each independently selected from -O-, -S-, -N=, -NH-, -CH=, -CH₂-; V₃ is selected from the group consisting of-N=, and -CH=.

In a specific embodiment, the present invention provides the conjugate as shown in below; 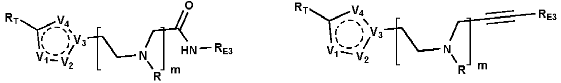 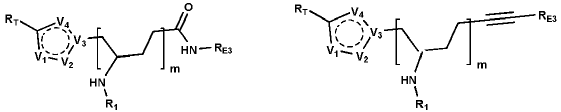 in each formula,
V₁, V₂ and V₄ are each independently selected from -O-, -S-, -N=, -NH-, -CH=, -CH₂-;
V₃ is selected from the group consisting of -N=, and -CH=;
R, R₁, R_{T} and R_{E3} are defined as above;
m = 0, 1, 2, 3, 4 and other integers (preferably m is not 0).

In a specific embodiment, the present invention provides the conjugate as shown in below;

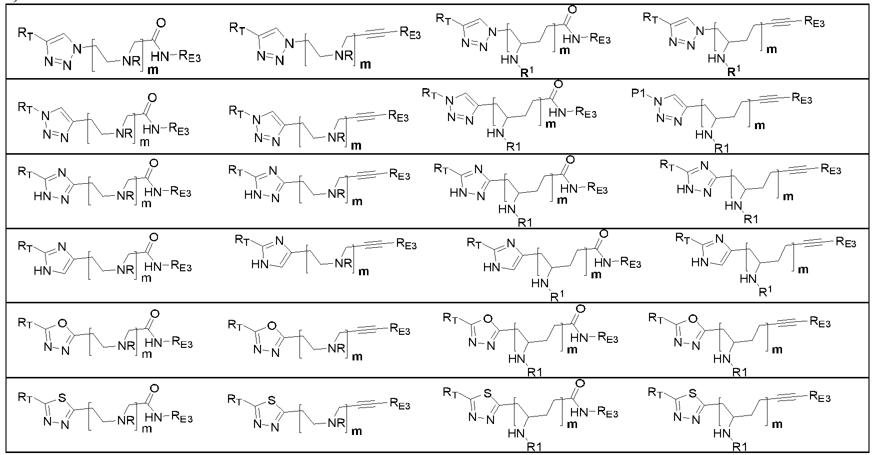

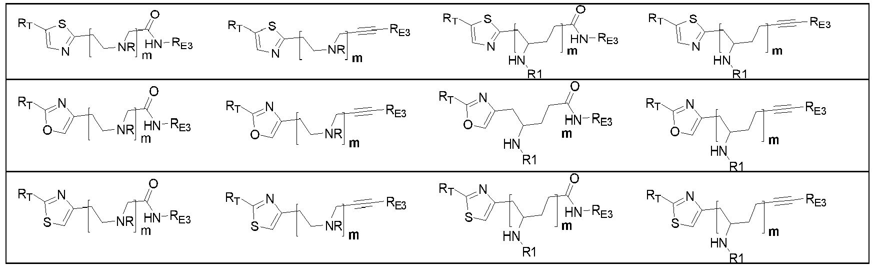

in each formula,
R, R₁, R_{T} and R_{E3} are defined as above;
m = 0, 1, 2, 3, 4 and other integers (preferably m is not 0).

In another specific embodiment, the conjugate is selected from Group 3:

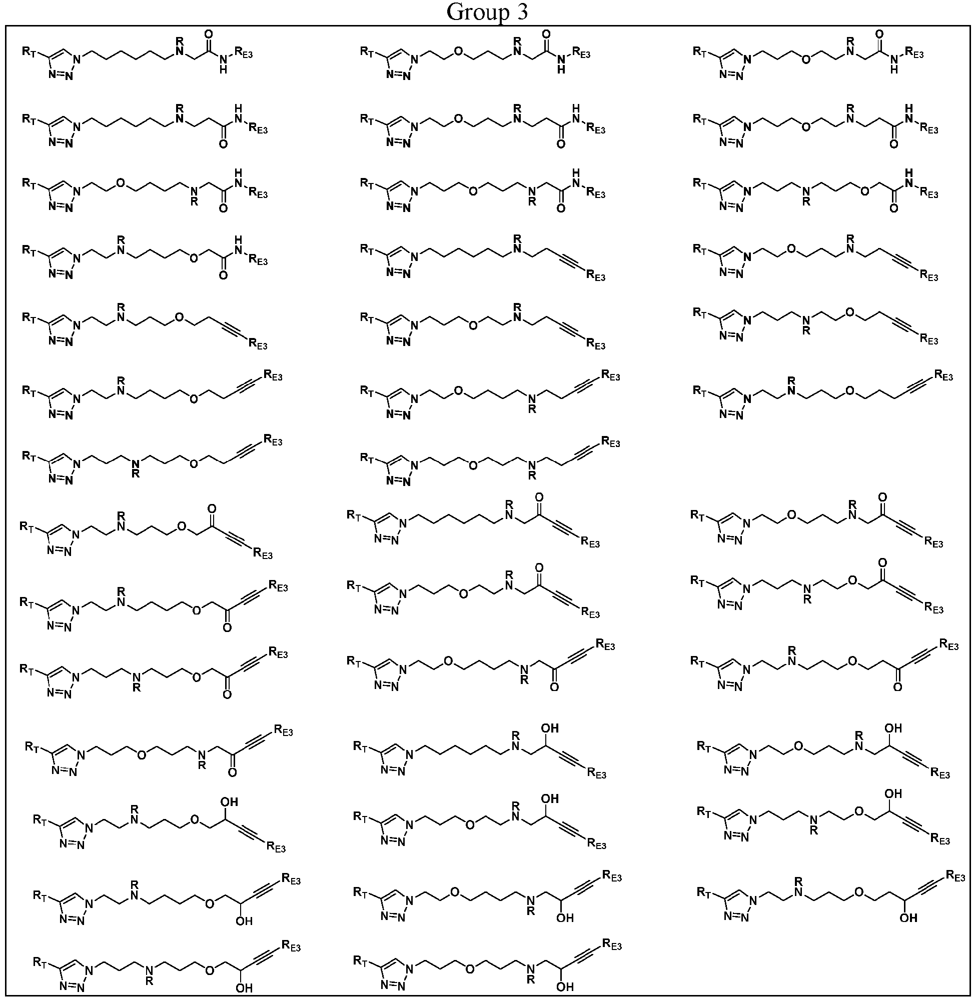

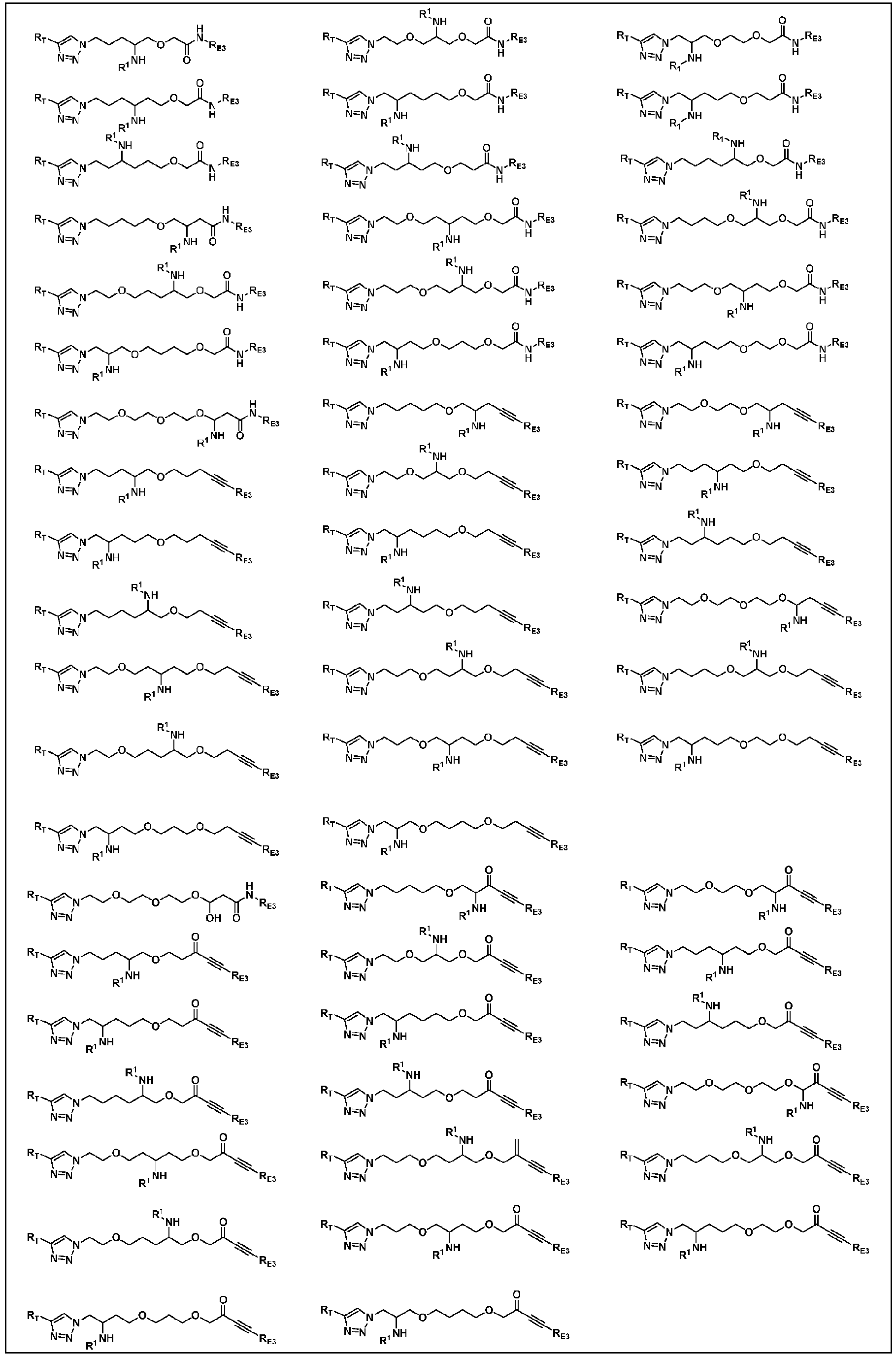

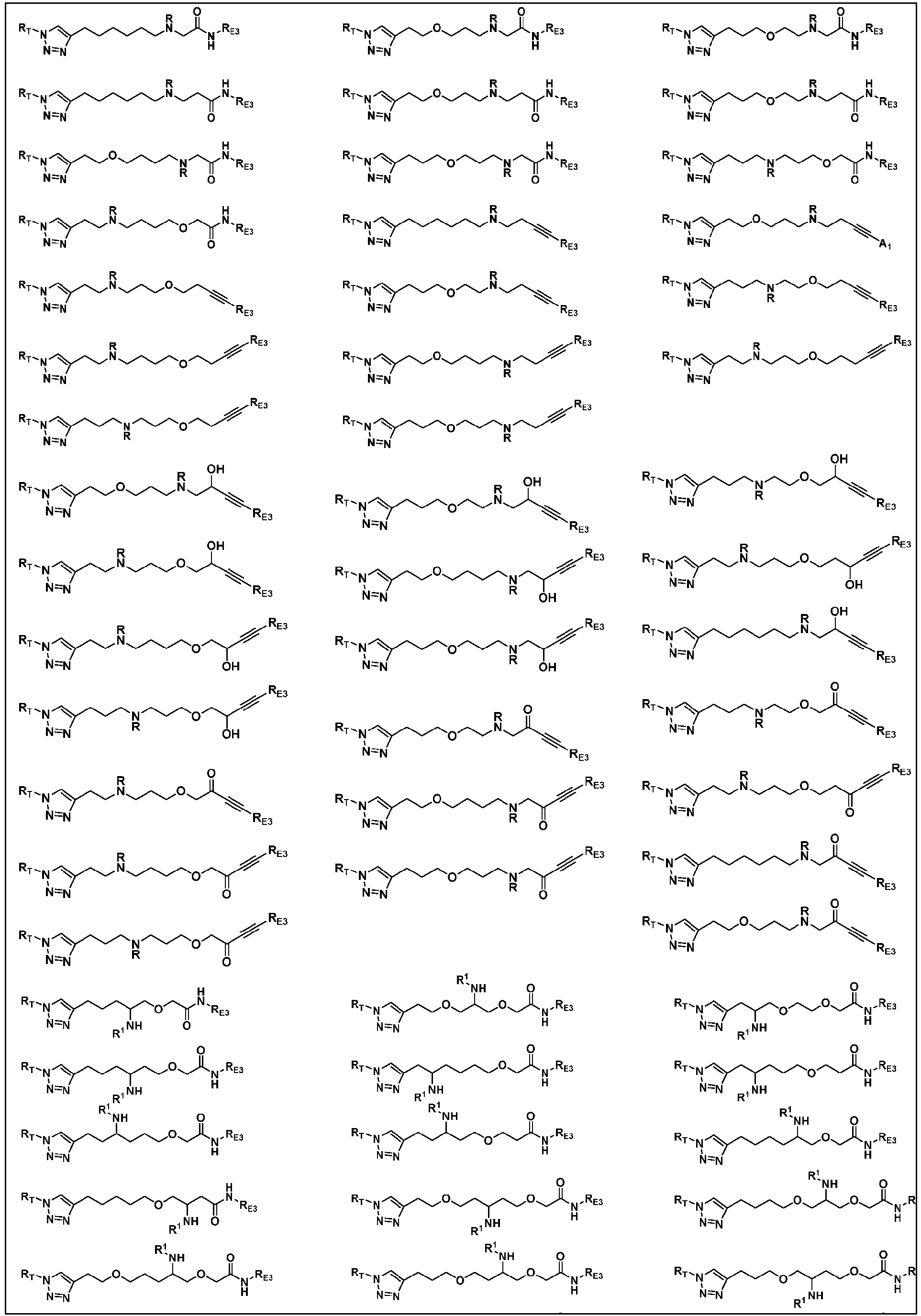

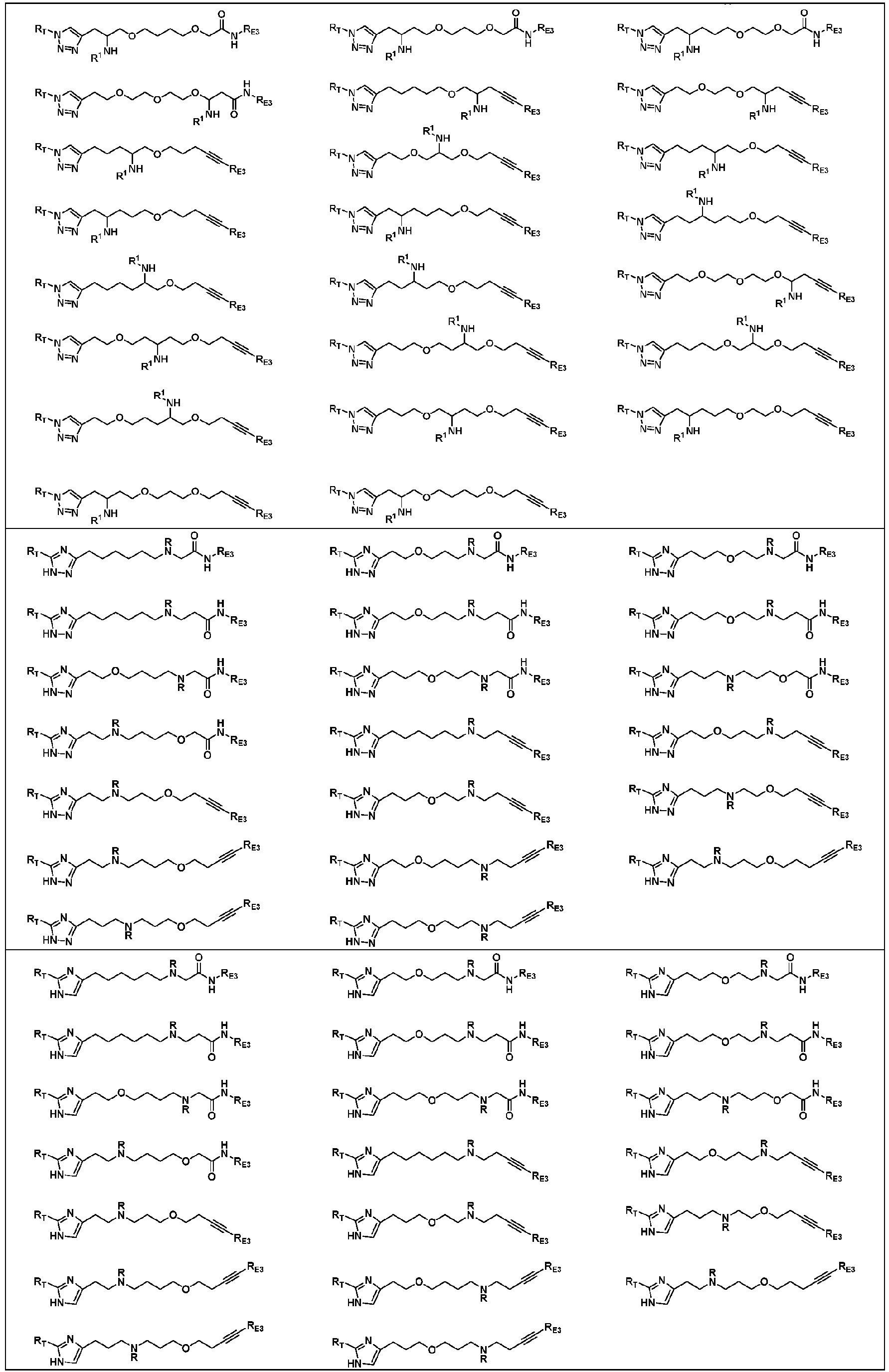

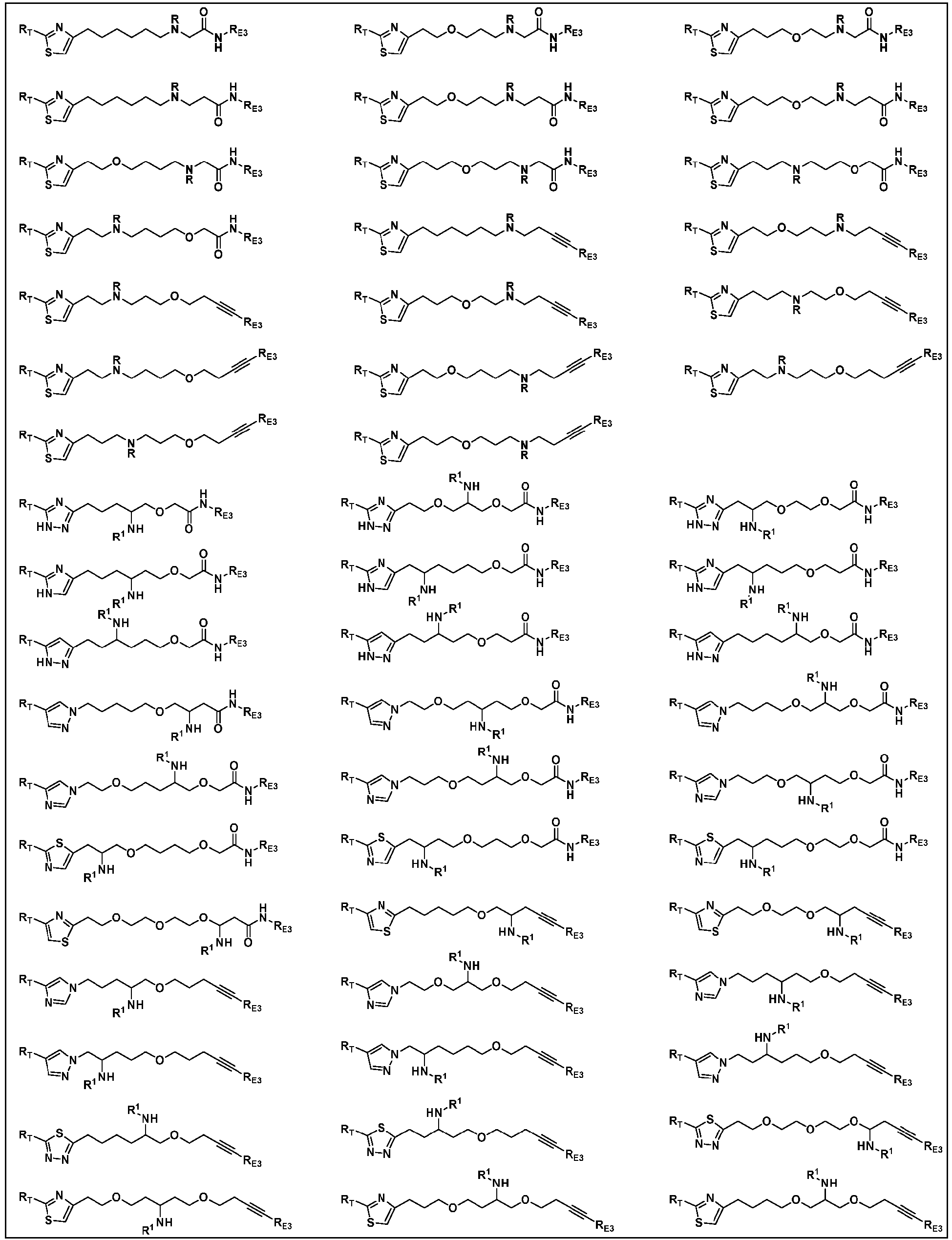

wherein R_{T}, R_{E3}, R and R₁ are defined as above; preferably, R and R¹ are each independently -W³-L3-W⁴-(Rp)_{q}, wherein W³, L3, W⁴, R_{P} and m are defined as above.

In a specific embodiment, the present invention provides the conjugate as shown in R_{T}-Ar1-L6-W2-R_{E};
wherein Ar¹, L5, R_{T}, W² and R_{E3} are defined as above.

In a specific embodiment, the present invention provides the conjugate as shown in R_{T}-Ar¹-L6-CONH-R_{E3}, R_{T}-Ar¹-L6-CO-R_{E3} or R_{T}-Ar¹-L6-W^{b}-C≡C-R_{E3}; wherein Ar¹, L6, R^{T} and R_{E3} are as defined as above.

In another specific embodiment, the conjugate is selected from Group 3a-1 and Group 3a-5:

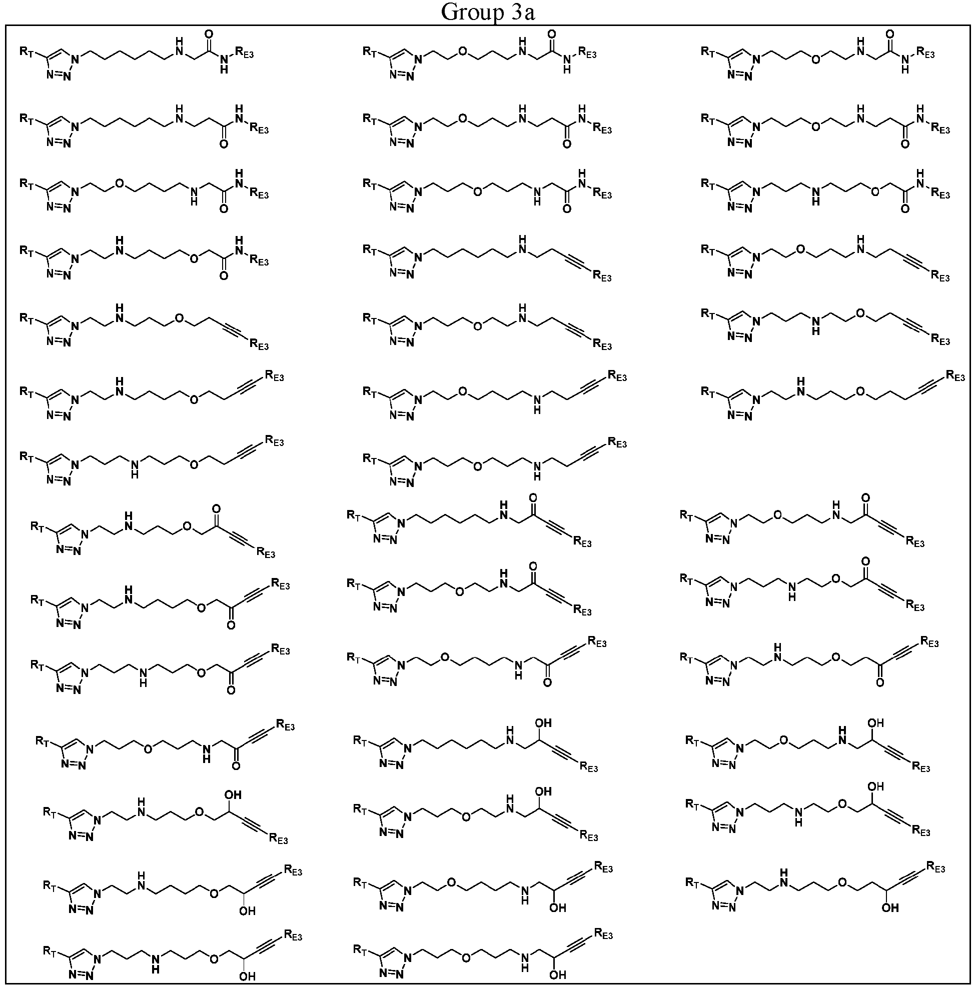

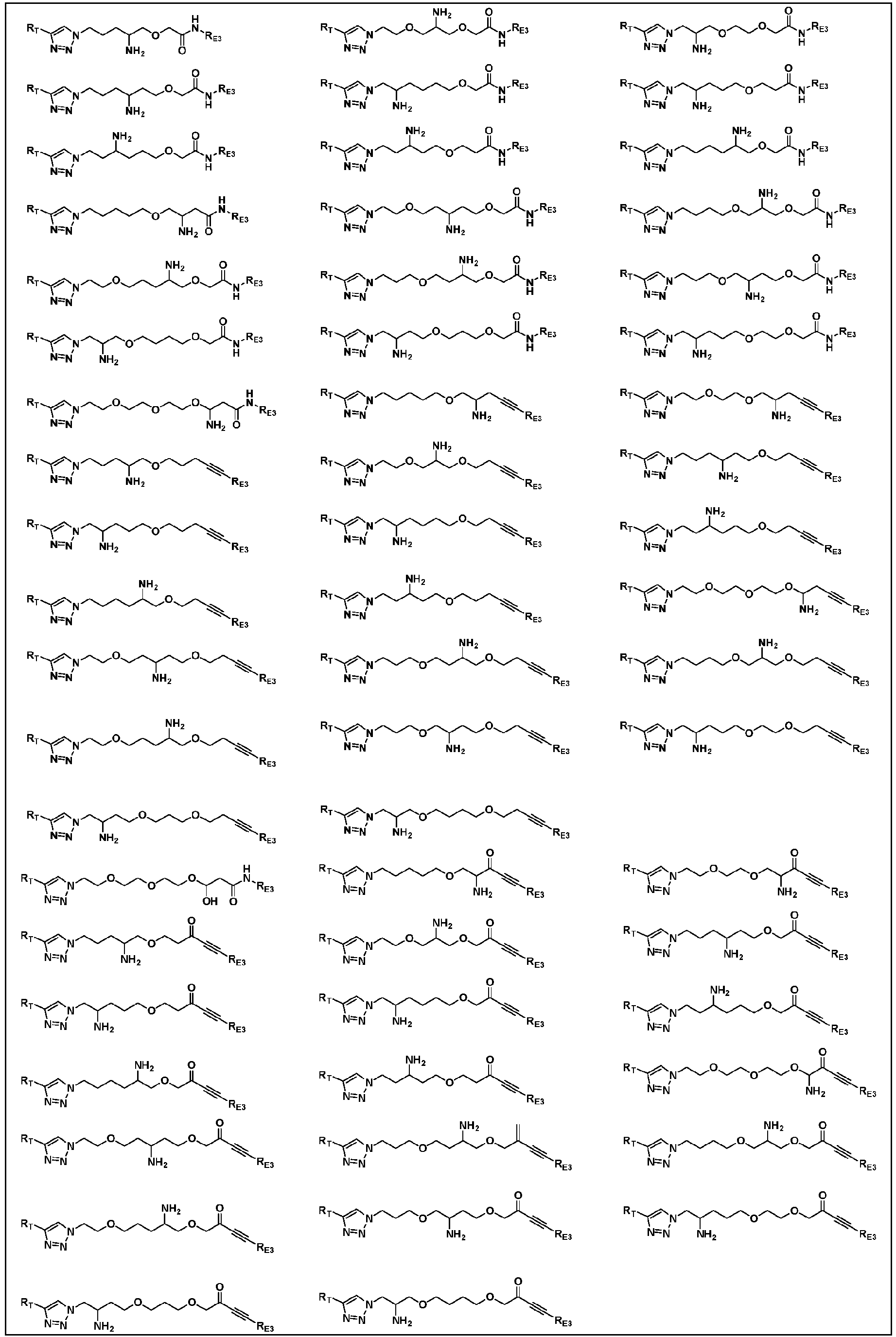

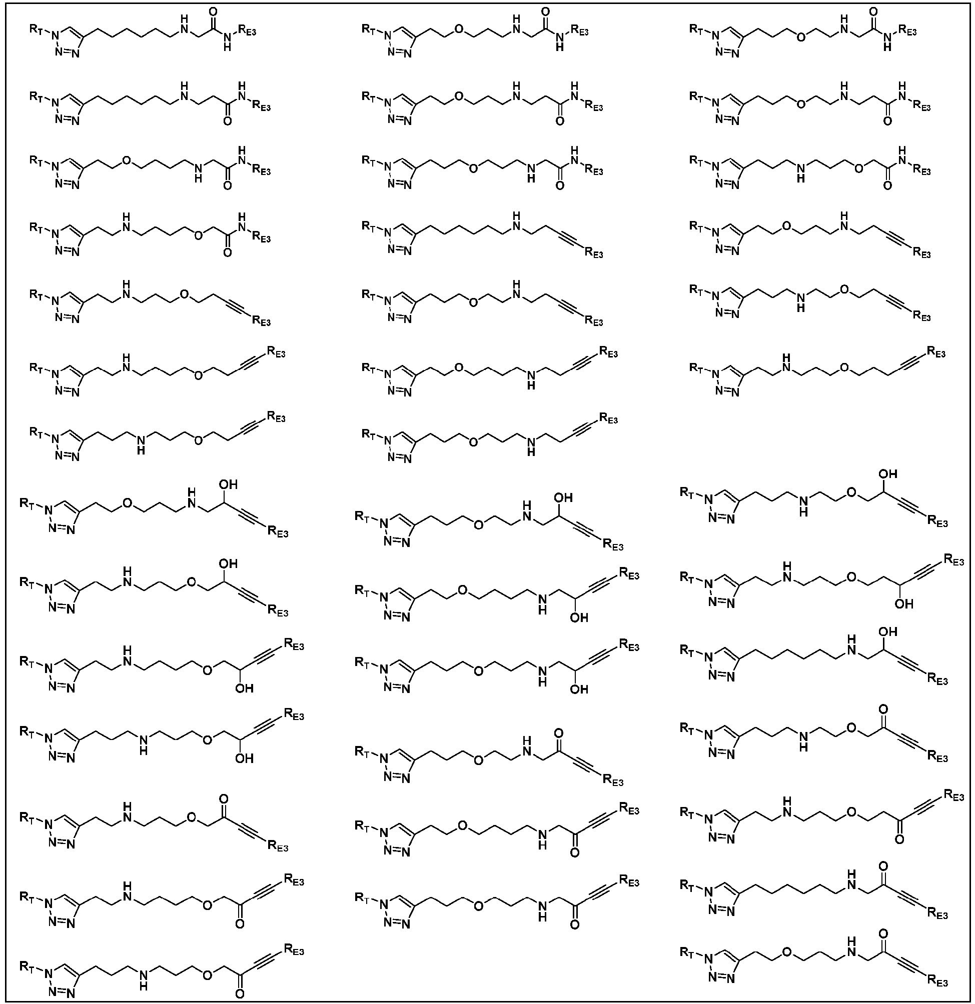

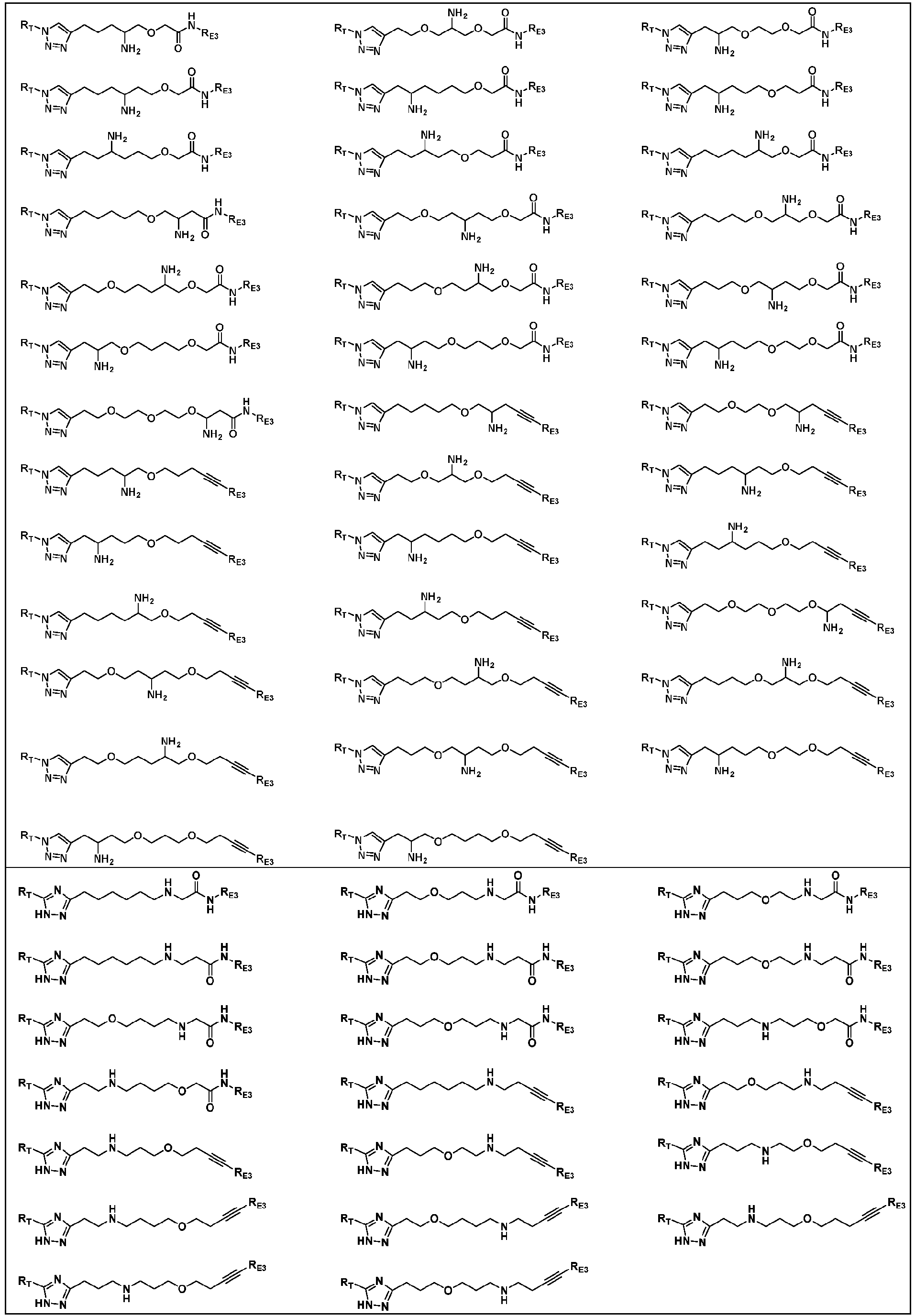

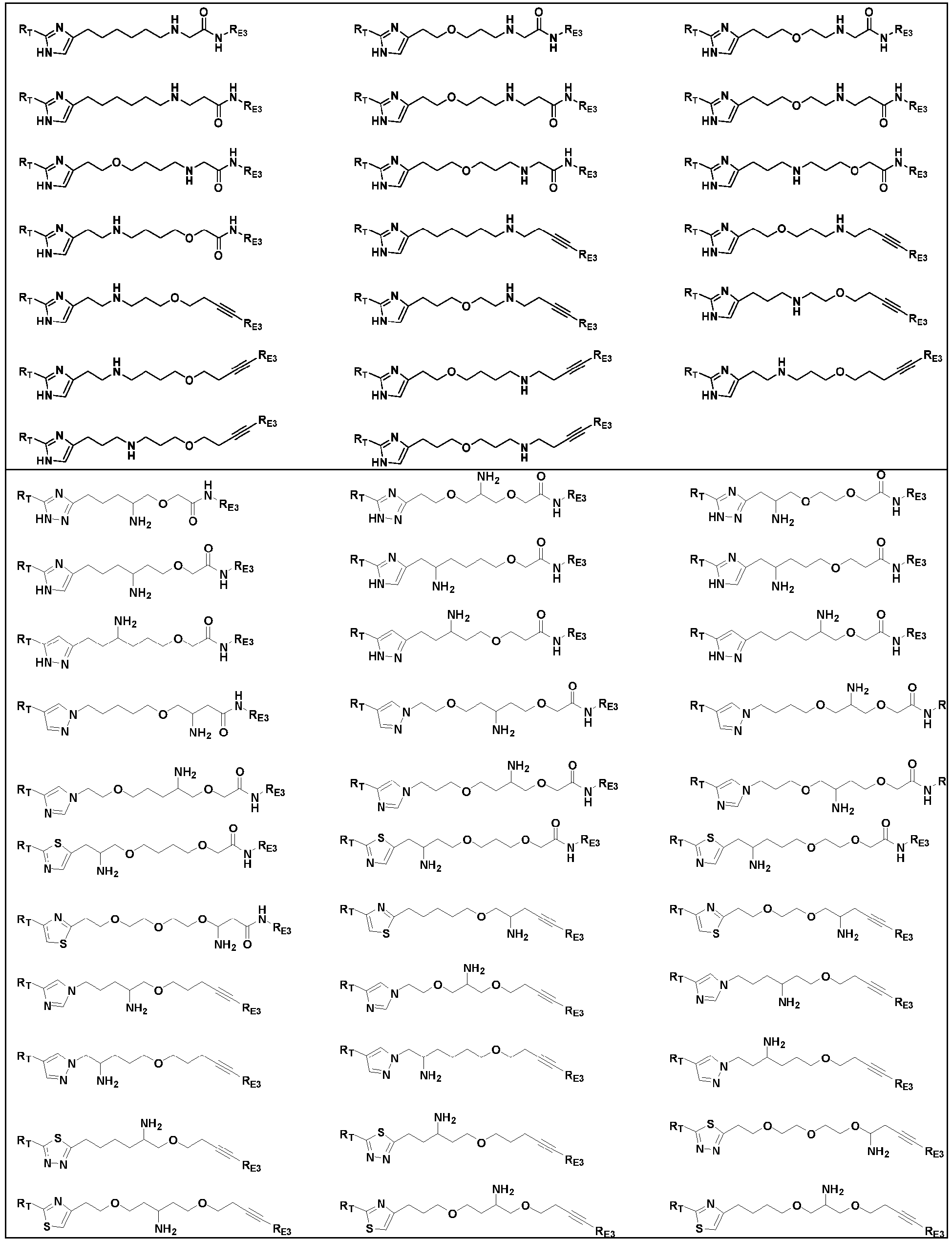

wherein R^{T} and R_{E3} are defined as above.

### ACTED

In the present invention, when the target molecule is an antibody or peptides, or cyclic peptides, or folate receptor ligands, or HSP90 ligands, or other extracellular target protein ligands, the conjugate of the present invention also can be referred to as ACTED or ACTED molecule or ACTED compound for short.

Some ACTED compounds are listed below: 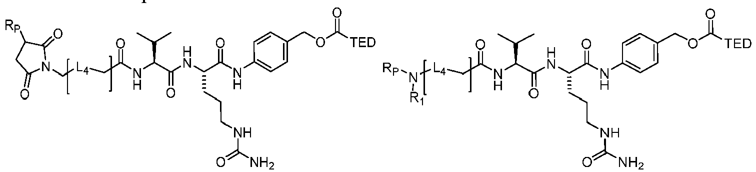 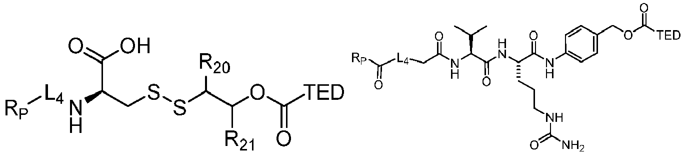 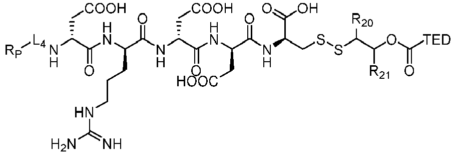
wherein TED refers the monovalent group formed by the loss of the group on N of conjugate of formula I or TED compound of formula VI;
R_{P}, and L4 are as defined as above.

In a specific embodiment, the examples of ACTED of the present invention include but not limit to compound or conjugate selected from the group consisting of

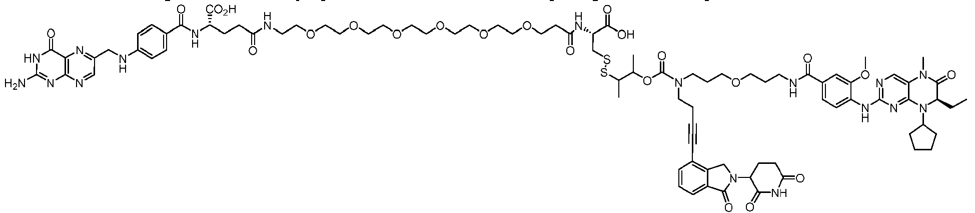

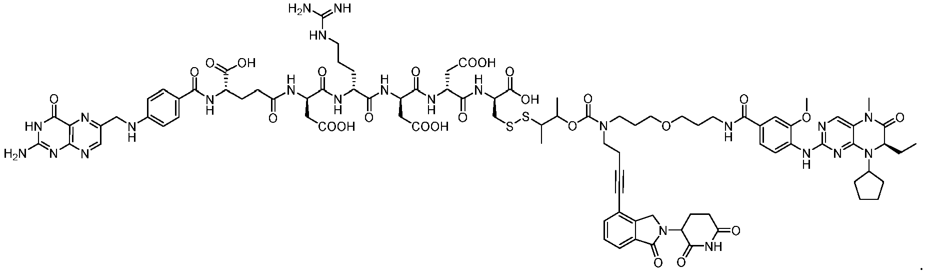

### The main advantages of the present invention include:

(a) The conjugate TED of the present invention has high activity on tumor cells, has selectivity on cells and has good safety.
(b) The conjugate TED of the present invention can exert the effect of inhibiting cell proliferation in a catalytic amount. The intracellular degradation of target proteins can be circulated to reduce the dose and prolong the dosing cycle to achieve safe and effective antitumor effects.
(c) The conjugate TED of the present invention, the linker (L1) portion of which carries an active site that can be linked to a drug delivery vehicle (e.g., antibody, peptide, other small molecule ligands).

The present invention was further described hereafter in combination with specific examples. It should be understood that these examples are only used to illustrate and not to limit the scope of the invention. The experimental methods without specific conditions in the following examples generally follow the conventional conditions or the conditions suggested by the manufacturer. Unless otherwise stated, percentages and parts are percentages by weight and parts by weight.

Unless otherwise specified, the starting materials or compounds used in examples are commercially available or can be prepared by methods known to those skilled in the art.

### General Method 1:

### Synthesis method of Compound P1- Linker - Ligand A

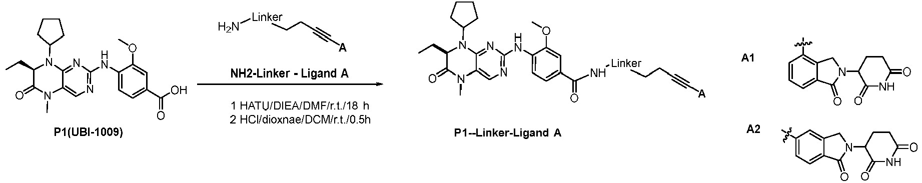

In formula, A is a structure shown in A1 or A2. Under N₂ protection, compound **P1** (20 mg, 1eq.), **Linker- Ligand** A (1 eq. ), **HATU** (2eq.) and **DIEA** (3eq.) were dissolved in **DMF** (2 mL), and reacted at room temperature for 18 hours. The reaction solution was poured into 5 mL of water and extracted with ethyl acetate (5 mL*3). The organic phases were combined and washed with saturated brine (10 mL*3), dried over anhydrous Na₂SO₄, concentrated under reduced pressure to obtain the crude product, which was isolated by thin-layer chromatography on silica gel plates (DCM/MeOH = 10/1) to obtain target product.

The above target product was dissolved in DCM (3mL), 0.5mL (HCl/dioxane 4 M) was added, and reacted at room temperature for 1 hour. The reaction solution was concentrated and washed with ether (5mL *3), and filtered to obtain target product **P1- Linker** - **Ligand A** as a white solid.

### General Method 2:

### Synthesis method of Compound P1 - Linker g -Ligand A

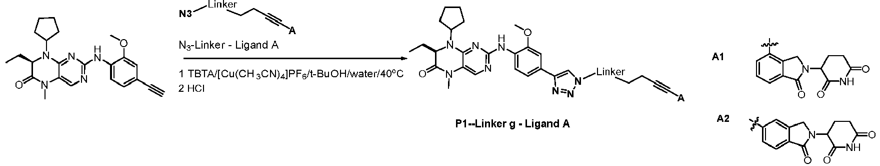

In formula, A is a structure shown in A1 or A2.

Compound **(R)-8-cyclopentyl-7-ethyl-2-((4-ethynyl-2-methoxyphenyl)amino)-5-methyl-7,8-dihydropteridin-6(5H)-one** (1eq) , **N3-linker** - **Ligand A** (1 eq. ), **TBTA** (1eq.), and **[Cu(CH₃CN)₄]PF₆** (Cat.) were dissolved in tert-butanol (5mL) and water, the mixture was reacted at room temperature for 16 hours to 4 days. The reaction solution was concentrated under reduced pressure and purified by silica gel column (MeOH/DCM=10%) to obtain the compound as a white solid.

The above target product was dissolved in DCM (3mL), 0.5mL (HCl/dioxane 4 M) was added, and reacted at room temperature for 1 hour. The reaction solution was concentrated and washed with ether (5mL *3), and filtered to obtain target product **P1- Linker** - **Ligand** A as a white solid.

### General Method 3:

### Synthesis method of Compound R1 - Linker - Ligand A

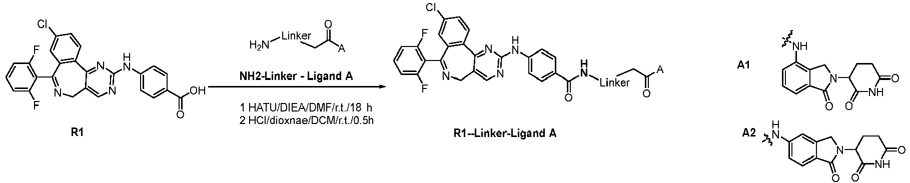

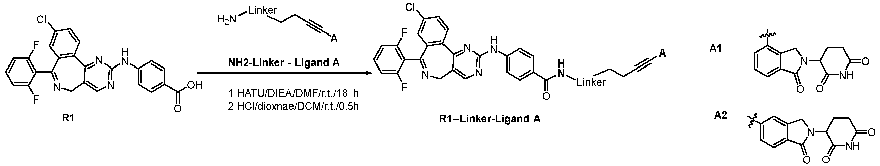

In formula, A is a structure shown in A1 or A2.

After addition of compound **R1/R2** (20 mg, 1eq.), **Linker- Ligand A** (1 eq.), **HATU** (2eq.) and **DIEA** (3eq.), the reaction was carried out at room temperature under nitrogen protection for 18 hours. The reaction solution was poured into 5 mL of water and extracted with ethyl acetate (5 mL*3). The organic phases were combined and washed with saturated brine (10 mL*3), dried over anhydrous Na₂SO₄, concentrated by rotary evaporation under reduced pressure to obtain the crude product, which was isolated by thin-layer chromatography on silica gel plates (DCM/MeOH = 10/1) to obtain target product.

The above target product was dissolved in DCM (3mL), 0.5mL (HCl/dioxane 4 M) was added, and reacted at room temperature for 1 hour. The reaction solution was concentrated and washed with ether (5mL *3), and filtered to obtain target product **R1-Linker** - **Ligand A** as a white solid.

### General Method 4:

### Synthesis method of Compound R2 - Linker - Ligand A

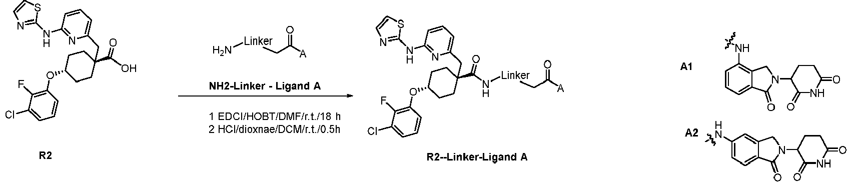

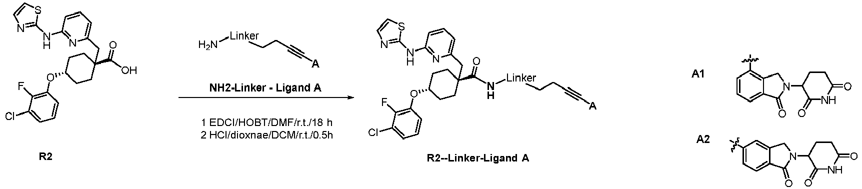

In formula, A is a structure shown in A1 or A2.

**Compound R1/R2** (20 mg, 1eq.), **Linker- Ligand A** (1 eq.), **EDCI** (2eq.), **HOBT** (2eq.), and **DIEA** (3eq.) were dissolved in **DMF** (2 mL), after addition, the reaction was carried out at room temperature under nitrogen protection for 18 hours. The reaction solution was poured into 5 mL of water and extracted with ethyl acetate (5 mL*3). The organic phases were combined and washed with saturated brine (10 mL*3), dried over anhydrous Na₂SO₄, concentrated by rotary evaporation under reduced pressure to obtain the crude product, which was isolated by thin-layer chromatography on silica gel plates (DCM/MeOH = 10/1) to obtain target product.

The above target product was dissolved in DCM (3mL), 0.5mL (HCl/dioxane 4 M) was added, and reacted at room temperature for 1 hour. The reaction solution was concentrated and washed with ether (5mL *3), and filtered to obtain target product **R2- Linker** - **Ligand A** as a white solid.

### General Method 5:

### Synthesis method of Compound R1/R2 - Linker - Ligand A

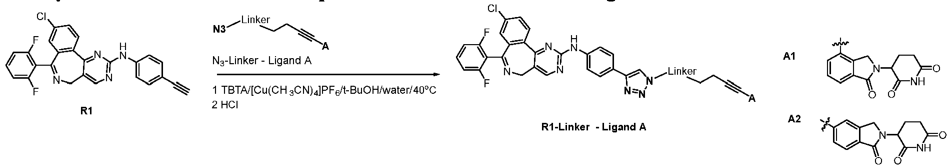

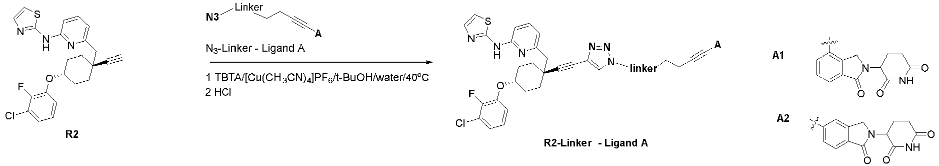

In formula, A is a structure shown in A1 or A2.

**Compound R1/R2** (1 eq.), **N3-Linker** - **Ligand** A (1eq.), **TBTA** (1eq.), and **[Cu(CH₃CN)₄]PF₆** (Cat.) were dissolved in *t*-BuOH (5mL) and water (1mL), and reacted at room temperature for 16 hours to 4 days. After the reaction was completed, it was concentrated to obtain the crude product, which was purified by silica gel column to obtain a white solid.

The above target product was dissolved in DCM (3mL), 0.5mL (HCl/dioxane 4 M) was added, and reacted at room temperature for 1 hour. The reaction solution was concentrated and washed with ether (5mL *3), and filtered to obtain target product **R1/R2- Linker** - **Ligand A** as a white solid.

### General Method 6:

### Synthesis method of Compound M - Linker - Ligand A

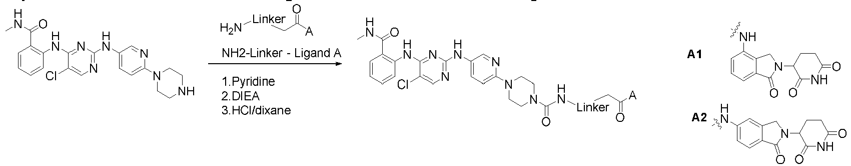

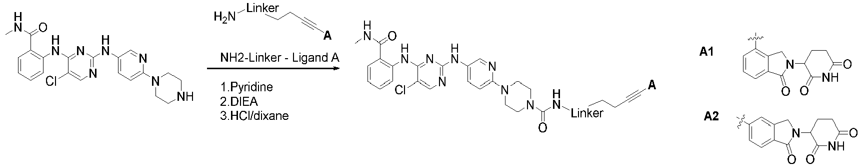

In formula, A is a structure shown in A1 or A2.

Compound **NH2-Linker** - **Ligand A** (1eq.) was dissolved in pyridine, then bis(*p-*nitrophenyl)carbonate(1eq) was added, and reacted for 2 hours. The yellow reaction solution was then obtained by adding **M** (1eq) and **DIPEA,** followed by the reaction at room temperature for 1 hour. The reaction solution was concentrated and purified by silica gel column to obtain a white solid.

The above target product was dissolved in DCM (3mL), 0.5mL (HCl/dioxane 4 M) was added, and reacted at room temperature for 1 hour. The reaction solution was concentrated and washed with ether (5mL *3), and filtered to obtain target product **M- Linker** - **Ligand** A as a white solid.

### General Method 7:

### Synthesis method of Compound R3- Linker - Ligand E

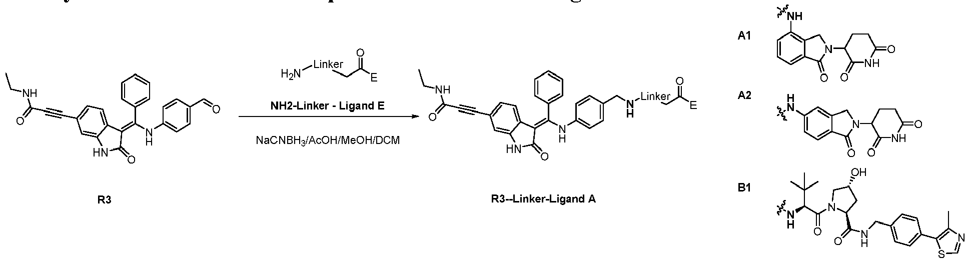

In formula, E is a structure shown in A1, A2 or B1.

**Compound R3** (20mg, 1eq.), **Linker- Ligand E** (2eq.) and a catalytic amount of AcOH (1drop) were dissolved in **methanol/dichloromethane=1/10** (10 mL) and reacted at room temperature for 18 hours. Then **NaCNBH₃**(3eq.) was added and the reaction was continued at room temperature for 3 hours. The reaction solution was concentrated and washed once with water (5 mL), extracted twice with ethyl acetate (10 mL), and the organic phase was concentrated to obtain the target product **R3- Linker** - **Ligand E.**

### Example 1

### Example 1.1 Synthesis of P1-Linker b- A1

### Example 1.1.1 Synthesis of compound UBI-1289 (NH₂-11b-A1)

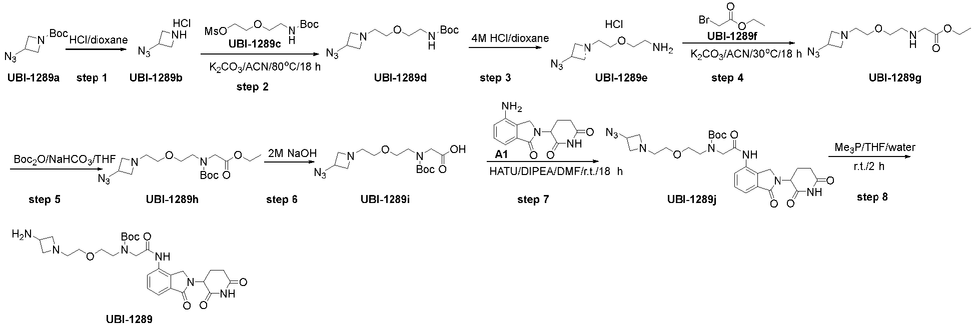

**Step 1: Synthesis of UBI-1289b (V1179-123): UBI-1289a** (7.2 g, 36.3 mmol) was added with 4M HCl/dioxane (25 mL) under ice bath and reacted overnight. Ether (25 mL) was added, and the mixture was slurried and filtered to obtain **UBI-1289b** (4.4 g, yield 89%) as a white solid.

**Step 2: Synthesis of UBI-1289d (V1179-126): UBI-1289b** (4.2 g, 31.2 mmol) was dissolved in acetonitrile (150 mL), K₂CO₃ (13 g, 93.6 mmol) and **UBI-1289c** (8.8 g, 31.2 mmol) were added, the mixture was warmed to 80°C and reacted overnight. The reaction solution was filtered, concentrated and passed through the column (dichloromethane/methanol = 0% to 10%) to obtain product **UBI-1289d** (5 g, yield 56%) as a yellow oil. LCMS [M+H]⁺ = 286.2.

**Step 3: Synthesis of UBI-1289e (V1179-127): UBI-1289d** (5 g, 17.5 mmol) was added with 4M HCl/dioxane (10 mL) under ice bath and reacted at room temperature for 1 hour. The reaction was concentrated to obtain product **UBI-1289e** (7.8 g) as a white solid.

**Step 4: UBI-1289g (V1179-130): UBI-1289e** (7.2 g, 16.2 mmol) was dissolved in acetonitrile (100 mL), K₂CO₃ (4.5 g, 32.4 mmol) and **UBI-1289f** (3 g, 17.9 mmol) were added, and the mixture was reacted over weekend. The reaction solution was filtered, and the filtrate was concentrated and passed through the column (dichloromethane/methanol = 0% to 10%) to obtain product **UBI-1289g** (1 g, 23% yield) as a yellow oil. LCMS [M+H]⁺ =272.3

**Step 5: UBI-1289h (V1179-131): UBI-1289g** (1 g, 3.7 mmol) was dissolved in THF (20 mL), NaHCO₃ (621 mg, 7.4 mmol) and Boc₂O (800 mg, 3.7 mmol) were added, and the mixture was reacted at room temperature for 3 hours. The reaction was filtered, concentrated and passed through the column (dichloromethane/methanol = 0% to 3%) to obtain crude product **UBI-1289h** (490 mg) as a yellow oil. LCMS [M+H]⁺ = 372.2

**Step 6: UBI-1289i (V1179-138): UBI-1289h** (490 mg, 1.32 mmol) was dissolved in ethanol (10 mL), 2M NaOH (1.5 mL, 3 mmol) was added, and the mixture was reacted at room temperature overnight. Brine (15 mL) was added, and the mixture was extracted with ether (20 mL*2). The aqueous layer was adjusted to pH~5 using hydrochloric acid. Then it was extracted with ethyl acetate (40 mL*3), the organic phases were combined, dried over anhydrous sodium sulfate, filtered and dried by rotary dryer to obtain crude **UBI-1289i** (300 mg) as a colorless oil. LCMS[MS+H]⁺=344.1

**Step 7: UBI-1289j (V2111-001): UBI-1289i** (240 mg, 0.69 mmol), **A1** (181 mg, 0.69 mmol), HATU (524 mg, 1.38 mmol), and DIPEA (0.5 mL) were dissolved in DMF (3 mL), and the mixture was reacted at room temperature overnight. The reaction was concentrated and passed through the column chromatography (dichloromethane/methanol = 0% to 30%) to obtain **UBI-1289j (V2111-001,** 140 mg) as a yellow oil. LCMS [M+H]⁺ = 585.4

**Step 8: UBI-1289 (V2111-002): UBI-1289j** (140 mg, 0.24 mmol) was dissolved in THF (2 mL), and 1M Me₃P (0.36 mL, 0.36 mmol) was added, and the mixture was reacted at room temperature for 1 hour. Water (0.5 mL) was added, the mixture was reacted for another 1 hour. The reaction solution was concentrated and passed through reversed-phase chromatography (acetonitrile/water = 0% to 30%) to obtain **UBI-1289** (40 mg, yield 30 %) as a yellow solid.

LCMS [M+H]⁺ = 559.3

### Example 1.2 Synthesis of P1-Linker c- A1

### Example 1.2.1 Synthesis of compound UBI- 1267

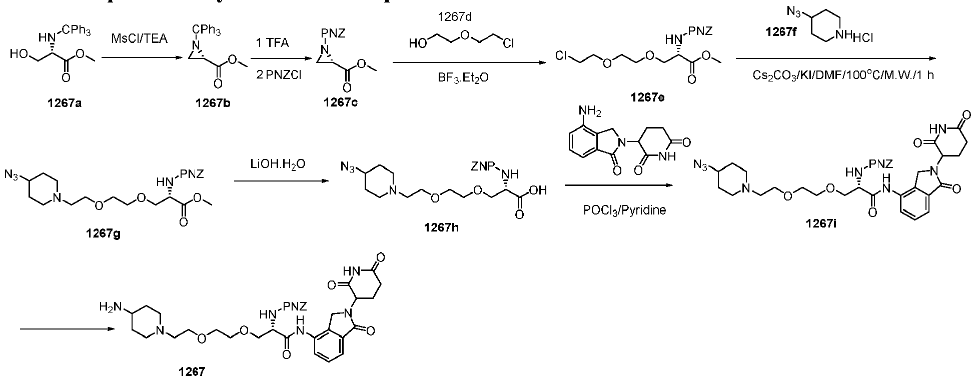

### Step 1: UBI-1267b (V879-078)

**UBI1267a** (10g, 27.2mmol) and TEA (8.4mL, 60.94mmol) were dissolved in THF (250mL), and cooled to 0°C, then MsCl (2.35mL,33.2mmol) was added, and the mixture was reacted at 80°C for 48 hours. The reaction solution was cooled to room temperature, filtered, and the filtrate was concentrated under pressure to obtain an oil. Then aqueous solution of HCl (1M) was added, the mixture was extracted with dichloromethane (20mL*3), and the organic phase was washed successively with saturated aqueous NaHCO₃, water, and saturated brine, dried over anhydrous Na₂SO₄, and concentrated to obtain a crude solid. The solid was recrystallized by adding into 50mL petroleum ether/ethyl acetate(3/1) to obtain target product **UBI-1267b** (4g, yield 42%) as a white solid.

1H NMR (400 MHz, chloroform-*d*) δ 7.54 - 7.41 (m, 5H), 7.36 - 7.09 (m, 10H), 3.76 (s, 3H), 2.26 (s, 1H), 1.91 - 1.82 (m, 1H), 1.47 - 1.33 (m, 1H).

### Step 2: UBI-1267c (V907-082)

Compound **UBI-1267b** (2 g, 5.8 mmol) was dissolved in dichloromethane (30 mL) and triethylamine (5 mL) was added at 0 °C. Then the reaction was reacted at room temperature for 3 hours. The reaction solution was added with water(30 mL), and washed with dichloromethane (20 mL) once. The aqueous phase was adjusted to pH > 8 with NaHCO3. The aqueous phase was slowly added a solution of PNZCl (7.6 g, 0.04 mol) in ethyl acetate (150 mL) dropwise to the aqueous phase at 0°C. Then the reaction was reacted at room temperature for 16 hours. The reaction solution was extracted with ethyl acetate (20 mL) twice. The organic phase was dried over anhydrous sodium sulfate and dried by rotary dryer to obtain the crude product. The crude product was separated by column chromatography (petroleum ether/dichloromethane=1/2) to obtain target product UBI-1267c (6.7 g, yield 61%) as a white solid. LCMS [M+H]+=101. ¹H NMR (400 MHz, chloroform-*d*) δ 8.37 - 8.03 (m, 2H), 7.74 - 7.43 (m, 2H), 5.46 - 4.94 (m, 2H), 3.76 (s, 3H), 3.16 (dd, *J=* 5.3, 3.2 Hz, 1H), 2.64 (dd, *J*= 3.2, 1.3 Hz, 1H), 2.52 (dd, *J* = 5.3, 1.3 Hz, 1H).

### Step 3: UBI-1267e (V1686-112)

Compound **UBI-1267c** (3 g, 10.7 mmol), and **UBI-1267d** (30 mL) were cooled to 0°C. Then BF₃ Et₂O (456 mg, 3.2 mmol) was slowly added dropwise into reaction solution, and the mixture was slowly warmed to room temperature and reacted for 2 hours. The reaction solution was directly purified via reversed-phase column chromatography (methanol/water = 5 % to 95 %, 40 minutes) to obtain target product **UBI-1267e** (3.6 g, yield 84%) as a light yellow oil. LCMS [M+H]⁺=225

1H NMR (400 MHz, chloroform-*d*) δ 8.45 - 8.09 (m, 2H), 7.53 (d, *J* = 8.3 Hz, 2H), 5.86 (d, *J=* 8.8 Hz, 1H), 5.21 (dd, *J=* 13.3, 4.3 Hz, 2H), 4.47 (d, *J=* 8.6 Hz, 1H), 4.00 (dd, *J=* 9.8, 3.2 Hz, 2H), 3.85 - 3.70 (m, 6H), 3.68 - 3.55 (m, 5H).

### Step 4: UBI-1267g (V1686-124)

Compound **UBI-1267f** (160 mg, 0.99 mmol) was dissolved in N,N-dimethylformamide (10 mL), then cesium carbonate (646 mg, 1.98 mmol), potassium iodide (247 mg, 1.49 mmol) and **UBI-1267e** (400 mg, 0.99 mmol) were added, and the mixture was reacted for 2 hours by being heated to 100°C via microwave. The reaction solution was filtered, and the filtrate was concentrated to obtain crude product. The crude product was purified by reversed-phase column chromatography (methanol/water = 5% to 95%, 40 minutes, collected at 75%) to obtain target product **UBI-1267g** (300 mg, yield 61%) as a brown oil. LCMS [M+H]⁺=315. ¹H NMR (400 MHz, chloroform-*d*) δ 8.22 (dd, *J=* 9.3, 2.6 Hz, 2H), 7.67 - 7.38 (m, 2H), 5.35 - 5.06 (m, 2H), 4.60 - 4.34 (m, 1H), 3.77 (s, 3H), 3.69 - 3.54 (m, 7H), 3.44 (m, 1H), 2.85 (d, *J* = 12.9 Hz, 2H), 2.63 (s, 2H), 1.96 (d, *J=* 9.0 Hz, 3H), 1.71 (m, 2H).

### Step 5: UBI-1267h (V1686-137)

Compound **UBI-1267g** (530 mg, 1.07 mmol) was dissolved in methanol (2 mL), tetrahydrofuran(6 mL) and water (2 mL), then lithium hydroxide (68 mg, 1.61 mmol) was added, and the mixture was reacted overnight. The reaction solution was washed once with ethyl acetate (20 mL), and the aqueous phase was adjusted to pH=6 with 3N diluted hydrochloric acid and concentrated to obtain 100 mg of the target product **UBI-1267h** (450 mg, yield 87%) as a white solid. The crude product was directly used in the next reaction.
LCMS [M+H]⁺=301

### Step 6: UBI-1267i (V1686-139)

Compound **UBI-1267h** (100 mg, 0.21 mmol) and lenalidomide (54 mg, 0.21mmol) were dissolved in dry pyridine (3 mL), then the mixture was cooled to 0°C in ice bath, then phosphorus oxychloride (319 mg, 2.1 mmol) was slowly added dropwise over 10 minutes(color was changed from brown to light yellow). The reaction was warmed to room temperature for 10 minutes (the color turned to black). The reaction solution was quenched with water and directly concentrated to obtain a crude product. The crude product was purified by reversed-phase column (methanol/water = 5% to 95%, 40 minutes, collected at 60%) to obtain target product **UBI-1267i** (70 mg, yield 47%) as a brown solid. LCMS [M+H]⁺=542. ¹H NMR (400 MHz, DMSO-*d₆*) δ 11.02 (t, *J=* 5.6 Hz, 1H), 8.24 (d, *J=* 8.4 Hz, 2H), 7.79 (s, 2H), 7.64 (d, *J=* 8.2 Hz, 2H), 7.53 (d, *J=* 10.3 Hz, 3H), 5.26 - 5.00 (m, 3H), 4.51 (s, 1H), 4.20 (m, 5H), 3.80 - 3.48 (m, 12H), 3.17 (s, 2H), 2.92 (m, 5H), 2.12 - 1.64 (m, 8H).

### Step 7: UBI-1267 (V1686-141)

Compound **UBI-1267i** (70 mg, 0.10 mmol) was dissolved in tetrahydrofuran(5 mL) and water (0.5 mL), then triphenylphosphine resin (100 mg, 0.15 mmol) was added, and the mixture was heated to 75°C and reacted for 18 hours. The reaction solution was filtered, and the filtrate was concentrated to obtain target product **UBI-1267** (60 mg, yield 90%) as a brown oil. LCMS [M+H]⁺=516

### Example 1.2.2 Synthesis of compound UBI-1276 (P1-11c-COOH)

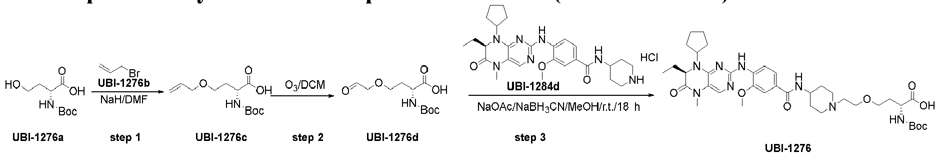

### Step 1: UBI-1276c (V1899-053)

60% NaH (857 mg, 20.4 mmol) was added to anhydrous DMF under ice bath and then **UBI-1276a** (2 g, 8.58 mmol) was added, and the mixture was reacted for 15 minutes. Then **UBI-1276b** (1.2 g,10.7 mmol) was added and the reaction was warmed to room temperature for 2 hours. The reaction solution was dried by rotary dryer and slurried with small amount of ether and water, the upper organic phase was discarded and the aqueous layer was adjusted to pH ~ 3 with hydrochloric acid. Then it was extracted with ethyl acetate, and the organic phases were combined, dried over anhydrous sodium sulfate, filtered and dried by rotary dryer. The crude product was passed through the column (dichloromethane/methanol = 0% to 20%) to obtain product **UBI-1276c** (1.148 g, yield 48%) as a yellow oil.

### Step 2: UBI-1276d (V1685-061)

**UBI-1276b** (500mg, 0.49mmol) were dissolved in DCM and cooled to -78°C. Ozone gas was passed through the reaction solution, bubbling for 20 minutes until the reaction solution turned blue. N₂ was passed into reaction solution for 20 minutes and then cooled to -78°C, then dimethyl sulfide was added and the mixture was warmed slowly to room temperature and stirred for 16 hours. The reaction liquid was concentrated to obtain crude product, which was directly used in the next reaction. LCMS [M-H]⁺ = 260.1

### Step 3: UBI-1276 (V1782-105)

**UBI-1276d** (115 mg, 0.44 mmol) were dissolved in methanol (4 mL), **UBI-1284d** (120 mg, 0.22 mmol) and NaOAc (18 mg, 0.22 mmol) were added and reacted for another 15 minutes. Then NaBH₃CN (28 mg, 0.44 mmol) was added and reacted for another 1 hour. The reaction solution was dried by rotary dryer, and the crude product passed through the column (dichloromethane/methanol= 0% to 100%) to obtain **UBI-1276** (170 mg, yield 34%) as a yellow oil. LCMS [M+H]⁺ = 753.2.

1H NMR (400 MHz, DMSO-*d₆*) δ 8.44 - 8.37 (m, 1H), 8.09 (d, *J* = 7.7 Hz, 1H), 7.85 (s, 1H), 7.59 (s, 1H), 7.48 (dq, *J* = 4.7, 1.9 Hz, 2H), 6.37 (s, 2H), 4.36 (t, *J=* 8.1 Hz, 1H), 4.23 (dd, *J* = 7.6, 3.6 Hz, 1H), 3.94 (s, 3H), 3.76 (d, *J=* 7.1 Hz, 2H), 3.70 (d, *J=* 7.4 Hz, 2H), 3.46 (d, *J=* 6.0 Hz, 2H), 3.39 (d, *J* = 2.8 Hz, 2H), 3.25 (s, 3H), 2.93 (d, *J* = 11.2 Hz, 2H), 2.14 - 1.97 (m, 4H), 1.89 (d, J= 3.0 Hz, 3H), 1.81 - 1.71 (m, 7H), 1.67 - 1.57 (m, 5H), 1.37 (s, 9H), 0.76 (t, J= 7.4 Hz, 3H).

### Example 1.2.3 Synthesis of compound UBI-1287 (P1-10c-A1)

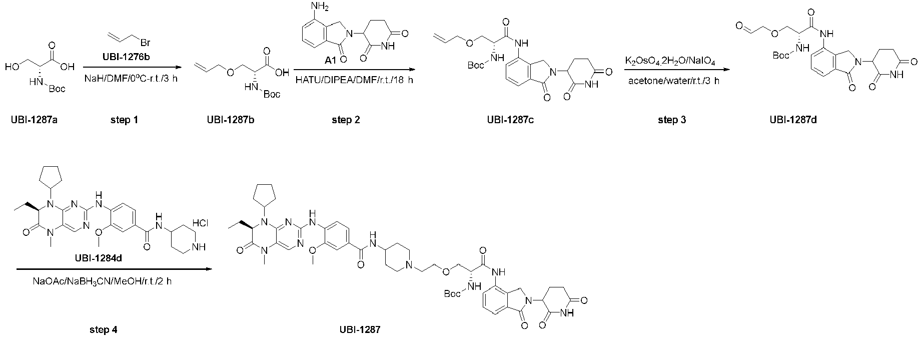

### Step 1: UBI-1287b (V1782-116)

60% NaH (4.88 g, 122 mmoL) was added to anhydrous DMF (30 mL) under ice bath, **UBI-1287a** (10 g, 48.8 mmoL) was dissolved in anhydrous DMF (20 mL) and added into the above reaction solution. The mixture was reacted for half an hour, then **UBI-1276b** (5.3 mL, 61 mmoL) was added and the reaction was warmed to room temperature for 2 hours. The reaction solution was dried by rotary dryer and slurried with small amount of ether and water, the upper organic phase was discarded and the aqueous layer was adjusted to pH ~ 3 with hydrochloric acid. Then it was extracted with ethyl acetate, the organic phases were combined, dried over anhydrous sodium sulfate, filtered and dried by rotary dryer. The crude product was passed through the column (dichloromethane/methanol = 0% to 10%) to obtain product **UBI-1287b** (10.5 g, yield 88%) as a colorless oil. LCMS [M-H]⁺ = 244.3

¹H NMR (400 MHz, DMSO-*d₆*) δ 12.67 (s, 1H), 6.90 (d, *J* = 8.3 Hz, 1H), 5.85 (ddt, *J* = 17.4, 10.5, 5.3 Hz, 1H), 5.24 (dq, *J* = 17.3, 1.8 Hz, 1H), 5.14 (dq, *J* = 10.4, 1.5 Hz, 1H), 4.14 (dt, *J=* 8.4, 5.5 Hz, 1H), 3.94 (dq, *J* = 5.3, 1.7 Hz, 2H), 3.60 (dt, *J* = 6.5, 3.8 Hz, 2H), 1.38 (s, 9H).

### Step 2: UBI-1287c (V1782-134)

**UBI-1287b** (245 mg, 1 mmol), **A1** (260 mg, 1 mmol), HATU (760 mg, 2 mmol), and DIPEA (390 mg, 3 mmol) were dissolved in DMF (5 mL) and the mixture was reacted at room temperature overnight. The reaction solution was concentrated and passed through the column (dichloromethane/methanol= 0% to 10%) to obtain product **UBI-1287c** (400 mg, yield 82%) as a light yellow solid. LCMS [M+H]⁺ =487.1

### Step 3: UBI-1287d (V1782-141)

**UBI-1287c** (200 mg, 0.4 mmol) was added to water (3 mL) and acetone (15 mL), a catalytic amount of K₂OₛO₄.2H₂O (cat.) and NaIO₄ (263 mg, 1.2 mmol) were added, and the mixture was reacted at room temperature for 2 hours. The reaction was filtered and concentrated, added with water, extracted with ethyl acetate (20 mL*2). The organic phases were combined, dried over anhydrous sodium sulfate, and filtered; and filtrate was directly used in the next step reaction. LCMS [M+H]⁺ =489.3

### Step 4: UBI-1287 (V1782-142)

**UBI-1287d** (V1782-141) and **UBI-1284d** (40 mg, 0.073 mmol) were dissolved in methanol (5 mL), NaOAc (6 mg, 0.073 mmol) was added, and the mixture was reacted at room temperature for 15 minutes. Then NaBH₃CN (9 mg, 0.15 mmol) was added and reacted at room temperature for 1 hour. The reaction was concentrated to obtain product **UBI-1287** (8 mg, yield 11%) as a white solid. LCMS [M+H]⁺ = 980.2.

### Example 1.2.4 Synthesis of compound UBI-1288 (P1-12c-A1)

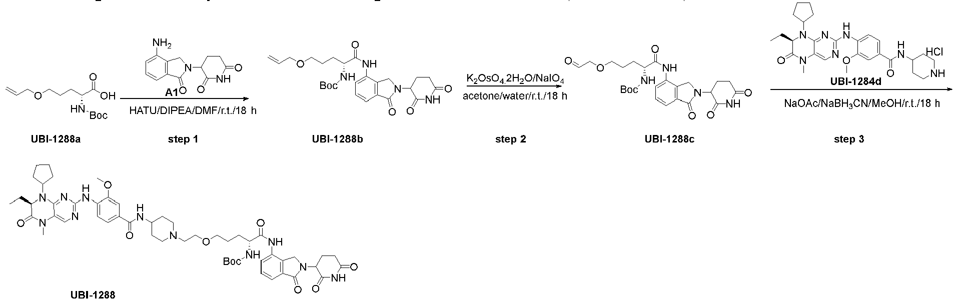

### Step 1: UBI-1288b (V1782-140)

**UBI-1288a** (500 mg, 1.83 mmol), **A1** (473 mg, 1.83 mmol), HATU (1.39 g, 3.66 mmol), and DIPEA (715 mg, 5.49 mmol) were dissolved in DMF (5 mL) and the mixture was reacted at room temperature overnight. The reaction solution was concentrated and passed through the column (dichloromethane/methanol= 0% to 10%) to obtain product **UBI-1288b** (320 mg, yield 34%) as a white solid. LCMS [M+H]⁺ =515.2

### Step 2: UBI-1288c (V1782-143)

**UBI-1288b** (150 mg, 0.3 mmol) was added to water (2 mL) and acetone (8 mL), then a catalytic amount of K₂O_{S}O₄.2H₂O was added and the mixture was reacted at room temperature for 2 hours. Then NaIO4 (192 mg, 0.9 mmol) was added, and the mixture was reacted at room temperature overnight. The reaction was filtered and concentrated, added with water, and extracted with ethyl acetate (20 mL*2). The organic phases were combined, dried over anhydrous sodium sulfate, and filtered, filtrate was directly used in the next step reaction.

LCMS [M+H]⁺ =517.3

### Step 3: UBI-1288 (V1782-145)

**UBI-1288c** and **UBI-1284d** (40 mg, 0.073 mmol) were dissolved in methanol (5 mL), NaOAc (6 mg, 0.073 mmol) and a catalytic amount of HOAc was added and the mixture was reacted for 20 minutes. Then NaBH₃CN (9 mg, 0.15 mmol) was added and reacted at room temperature for 1hour. The reaction solution was prepared to obtain **UBI-1288** (25 mg, yield 33%) as a white solid. LCMS [M+H]⁺ = 1008.2.

1H NMR (400 MHz, DMSO-*d₆*) δ 11.03 (s, 1H), 9.94 (s, 1H), 9.42 (s, 1H), 8.39 (d, *J=* 7.1 Hz, 1H), 8.10 (s, 1H), 7.80 (s, 1H), 7.74 (t, *J* = 9.3 Hz, 1H), 7.52 (m, 4H), 7.13 (m, 1H), 5.17 (m, 1H), 4.42 - 4.21 (m, 5H), 4.13 (m, 1H), 3.93 (s, 3H), 3.72 (m, 2H), 3.28 (m, 2H), 3.23 (s, 3H), 3.13 (m, 2H), 2.91 (m, 1H), 2.61 (m, 2H), 2.46 - 2.44 (m, 1H), 2.27 (m, 1H), 2.02 (m, 6H), 1.89 - 1.72 (m, 7H), 1.63 (m, 5H), 1.52 (m, 2H), 1.39 (s, 9H), 0.76 (t, *J=* 7.4 Hz, 3H).

### Example 1.3 Synthesis report of P1-Linker h- A1

### Example 1.3.1 Synthesis of compound UBI-1268 (P1-13h)

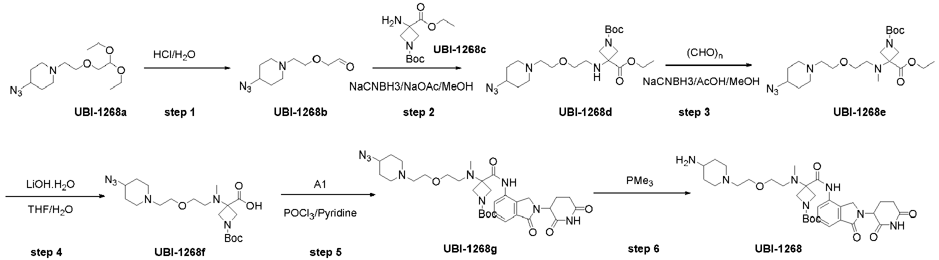

### Step 1: UBI-1268b(V2037-023)

Compound **UBI-1268a** (2.1 g, 7.3 mmol) was dissolved in 3N diluted hydrochloric acid (50 mL), then reacted at room temperature for 2 hours. The reaction solution was extracted with dichloromethane (30 mL), and the organic phase was dried over anhydrous sodium sulfate and then used directly in the next reaction. LCMS [M+H]⁺=287

### Step 2: UBI-1268d(V2037-024)

Compound **UBI-1268c** (600 mg, 2.46 mmol) was dissolved in methanol (30 mL), then sodium acetate (403 mg, 4.92 mmol) and **UBI-1268b** (1.56 g, 7.38 mmol) were added and the mixture was reacted at 25 °C for 1 hour. Then sodium cyanoborohydride (463 mg, 7.38 mol) was added. The reaction was carried out at room temperature overnight. The reaction solution was concentrated and then washed with water (20 mL) once, and extracted with ethyl acetate (30 mL), and the organic phase was concentrated, then isolated by column chromatography (methanol/dichloromethane=0 - 10%) to obtain target product **UBI-1268d** (790 mg, yield 25%) as a colorless oil. LCMS [M+H]⁺=441. **¹H NMR (400** MHz, chloroform-*d*) δ 4.27 (q, *J* = 7.1 Hz, 2H), 4.18 (d, *J* = 8.8 Hz, 2H), 3.83 (dd, *J* = 12.0, 6.9 Hz, 5H), 3.63 (t, *J* = 5.0 Hz, 2H), 3.12 (d, *J=* 27.2 Hz, 6H), 2.73 (t, *J=* 5.0 Hz, 2H), 2.27 (m, 2H), 1.94 (d, *J=* 14.0 Hz, 2H), 1.45 (s, 9H), 1.32 (t, *J = 7.1* Hz, 3H).

### Step 3: UBI-1268e (V2037-025)

Compound **UBI-1268d** (220 mg, 0.5 mmol) was dissolved in methanol (10 mL), then one drop of acetic acid and paraformaldehyde (23 mg, 0.75 mmol) were added and the mixture was reacted at 25 °C for 1 hour. Then sodium cyanoborohydride (94 mg, 1.5mol) was added. The reaction was carried out at room temperature overnight. The reaction solution was washed with water (10 mL) once, and extracted with ethyl acetate (20 mL), and the organic phase was concentrated to obtain target product **UBI-1268e** (200 mg, yield 25%) as a colorless oil. The crude product was directly used in the next reaction. LCMS [M+H]⁺=455

### Step 4: UBI-1268f (V2037-027)

Compound **UBI-1268e** (220 mg, 0.48 mmol) was dissolved in ethanol (1 mL), tetrahydrofuran(3 mL) and water (1 mL), then lithium hydroxide (31 mg, 0.73 mmol) was added, and the mixture was reacted at room temperature overnight. The reaction solution was washed once with ethyl acetate (10 mL), and the aqueous phase was adjusted to pH=6 with 3N HCl and lyophilizated to obtain target product **UBI-1268f** (250 mg, yield 100%) (containing salt) as a white solid. The crude product was directly used in the next reaction. LCMS [M+H]⁺=427.

### Step 5: UBI-1268g (V2037-032)

Compound **UBI-1268f** (200 mg, 0.47 mmol) and lenalidomide (121 mg, 0.47 mmol) were dissolved in dry pyridine (5 mL), and the mixture was cooled to 0°C, then phosphorus oxychloride (718 mg, 4.69 mmol) was slowly added dropwise over 10 minutes(color was changed from brown to light yellow). Then the mixture was reacted at room temperature for 10 minutes (the color turned to black). The reaction solution was quenched with water (5 mL) and concentrated to obtain a crude product. The crude product was purified by reversed-phase column chromatography (methanol/water = 5% to 95%, 40 minutes, collected at 60%) to obtain target product **UBI-1268g** (100 mg, yield 32%) as a brown solid. LCMS [M+H]⁺=668

### Step 6: UBI-1268 (V2037-033)

Compound **UBI-1268g** (30 mg, 0.04 mmol) was dissolved in dichloromethane (8 mL) and methanol (2 mL), then a catalytic amount of Pd/C was added. The reaction solution was reacted at room temperature for 4 hours under hydrogen condition. The reaction solution was filtered, and the filtrate was concentrated to obtain target product **UBI-1268g** (10 mg, yield 35%) as a yellow oil. The crude product was directly used in the next reaction. LCMS [M+H]⁺=642

### Example 1.3.2 compound UBI-1269 (P1-13h)

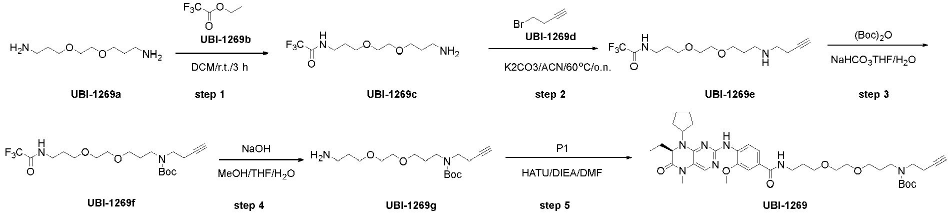

### Step 1: UBI-1269c (V2037-041)

Compound **1269a** (2 g, 11.4 mmol) was dissolved in dichloromethane (60 mL), the mixture was cooled to 0 °C, then ethyl trifluoroacetate (1.6 g, 11.4 mmol) was added. The reaction was carried out at room temperature overnight. The reaction solution was concentrated and isolated by column chromatography (methanol/dichloromethane = 1/10) to obtain target product **UBI-1269c** (1.5 g, yield 48%) as a colorless oil. LCMS [M+H]⁺=273. 1H NMR (400 MHz, chloroform-*d*) δ 8.23 (s, 1H), 3.72 - 3.53 (m, 8H), 3.52 - 3.43 (m, 2H), 2.81 (t, *J=* 6.6 Hz, 2H), 1.98 - 1.81 (m, 4H), 1.73 (p, *J=* 6.3 Hz, 2H).

### Step 2: UBI-1269e (V2037-043)

Compound **UBI-1269c** (600 mg, 2.2 mmol) was dissolved in acetonitrile (15 mL) and potassium carbonate (365 mg, 2.6 mmol) and **UBI-1269d** (293 mg, 2.2 mmol) were added, the reaction solution was heated to 60°C, then reacted for 16 hours. The reaction solution was filtered, and the filtrate was concentrated to obtain crude product. The crude product was isolated by column chromatography (methanol/dichloromethane = 0-10%) to obtain target product **UBI-1269e** (240 mg, yield 34%) as a colorless oil. LCMS [M+H]⁺=325. ¹H NMR (400 MHz, chloroform-*d*) δ 7.91 (s, 1H), 3.69 - 3.62 (m, 2H), 3.62 - 3.55 (m, 5H), 3.54 - 3.43 (m, 4H), 2.83 (dt, *J* = 12.0, 6.6 Hz, 4H), 2.46 (td, *J* = 6.6, 2.7 Hz, 2H), 2.07 - 1.96 (m, 1H), 1.93 - 1.72 (m, 4H).

### Step 3: UBI-1269f (V2037-044)

**Compound UBI-1269e** (250 mg, 0.77 mmol) was dissolved in tetrahydrofuran (15 mL), then added sodium bicarbonate (97 mg, 1.16 mmol) and Boc₂O (200 mg, 0.93 mmol) and reacted at room temperature for 1 hour. The reaction solution was concentrated and isolated by column chromatography (methanol/dichloromethane = 1/20) to obtain target product **UBI-1269f** (130 mg, yield 40%) as a colorless oil. LCMS [M+H]⁺=425

### Step 4 : UBI-1269g (V2037-047)

**Compound UBI-1269f** (150 mg, 2.9 mmol) was dissolved in methanol/tetrahydrofuran/water (5/5/3 mL), then sodium hydroxide(97 mg, 1.16 mmol) was added, and the mixture was reacted at room temperature for 1 hour. The reaction solution was concentrated to obtain target product **UBI-1269g** (110 mg, yield 95%) as a colorless oil. The crude product was directly used in the next reaction. LCMS [M+H]⁺=329

### Step 5 : UBI-1269 (V2037-049)

Compound **P1** (149 mg, 0.35 mmol) was dissolved in N,N-dimethylformamide (2 mL) and HATU (266 mg, 070 mmol) and DIEA (136 mg, 1.05 mmol) were added. After reacting at room temperature for 20 minutes, **UBI-1269g** (115 mg, 0.35 mmol) was added, and then reacting at room temperature for 16 hours. The reaction solution was added with water (10 mL), and extracted once with ethyl acetate (10 mL), the organic phase was concentrated, then isolated by column chromatography (methanol/dichloromethane=0 - 10%) to obtain target product **UBI-1269** (150 mg, yield 58%) as a brown oil. LCMS [M+H]⁺=736

### Example 1.3.3 Synthesis method of Compound UBI-1270 (P1-12h)

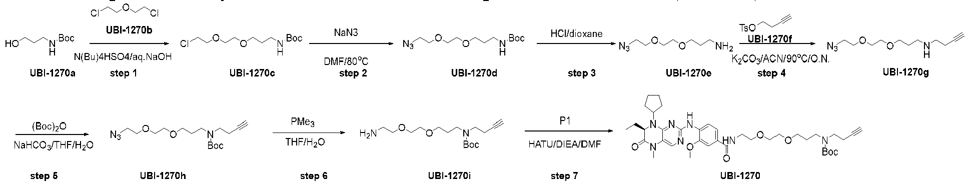

### Step 1: UBI-1270c (V2037-055)

Compound **UBI-1270a** (3 g, 17.1 mmol) was dissolved in **UBI-1270b** (10 mL) and 50% aq.NaOH (50 mL), and N(Bu)₄HSO₄ (11.7 g, 34.3 mmol) was added, and the reaction was reacted at 40°C for 16 hours. The reaction solution was washed with water (20 mL) once, and extracted with dichloromethane (50 mL), the organic phase was concentrated, then isolated by column chromatography (methanol/dichloromethane=0 -10%) to obtain target product **UBI-1270c** (4 g, yield 83%) as a colorless oil. LCMS [M+H]⁺=282

### Step 2: UBI-1270d (V2037-057)

Compound **UBI-1270c** (5.5 g, 19.6 mmol) was dissolved in N,N-dimethylformamide (50 mL), and sodium azide (1.9 g, 29.4 mmol) was added, the mixture was reacted at 80°C for 4 hours. The reaction solution was added with ice water(30 mL), and extracted with dichloromethane (20 mL) twice. The organic phase was concentrated and isolated by column chromatography (methanol/dichloromethane = 1/20) to obtain target product **UBI-1270d** (1.2 g, yield 21%) as a colorless oil. LCMS [M+H]⁺=289. ¹H NMR (400 MHz, chloroform-*d*) δ 4.91 (s, 1H), 3.72 - 3.63 (m, 5H), 3.63 - 3.58 (m, 2H), 3.58 - 3.51 (m, 2H), 3.40 (t, *J=* 5.0 Hz, 2H), 3.23 (d, *J=* 5.9 Hz, 2H), 1.88 - 1.70 (m, 2H), 1.44 (d, *J=* 0.8 Hz, 9H).

### Step 3: UBI-1270e (V2037-063)

Compound **UBI-1270d** (1.2 g, 4.2 mmol) was dissolved in dichloromethane (15 mL), then HCl/dioxane (15 mL) was added and the mixture was reacted at room temperature for 2 hours. The reaction solution was concentrated to obtain target product **UBI-1270e** (1 g, yield 100%) as a yellow oil. LCMS [M+H]⁺=189

### Step 4: UBI-1270g (V2037-065)

Compound **UBI-1270e** (500 mg, 2.2 mmol) was dissolved in acetonitrile (40 mL), then potassium carbonate (676 mg, 4.9 mmol), a catalytic amount of potassium iodide and **UBI-1270f** (499 mg, 2.2 mmol) were added, and the reaction solution was reacted at 90°C for 16 hours. The reaction solution was filtered. The filtrate was concentrated and isolated by column chromatography (methanol/dichloromethane = 0% to 10%) to obtain target product **UBI-1270g** (280 mg, yield 52%) as a yellow oil. LCMS [M+H]⁺=241. ¹H NMR (400 MHz, chloroformchloroform-*d*) δ 5.48 (s, 1H), 3.85 - 3.57 (m, 8H), 3.44 (dd, *J=* 5.5, 4.4 Hz, 2H), 3.04 (dt, *J* = 13.3, 6.4 Hz, 4H), 2.67 (td, *J* = 6.7, 2.7 Hz, 2H), 2.11 (t, *J* = 2.7 Hz, 1H), 2.02 (dq, *J* = 8.8, 6.0 Hz, 2H).

### Step 5: UBI-1270h (V2037-067)

Compound **UBI-1270g** (280 mg, 1.2 mmol) was dissolved in tetrahydrofuran (10 mL) and water (2 mL), then (Boc)₂O (381 mg, 1.7 mmol) and sodium bicarbonate(196 mg, 2.3 mmol) were added, and the mixture was reacted at room temperature for 1 hour. The reaction solution was washed with water (10 mL) once, and extracted with ethyl acetate (15 mL), and the organic phase was concentrated, then isolated by column chromatography (methanol/dichloromethane=1/10) to obtain target product **UBI-1270h** (270 mg, yield 68%) as a colorless oil. LCMS [M+H]⁺=341

### Step 6: UBI-1270i (V2037-069)

Compound **UBI-1270h** (270 mg, 0.08 mmol) was dissolved in tetrahydrofuran (5 mL), trimethylphosphine (1.2 mL) was added, and the mixture was reacted at 45°C for 1 hour. After adding water (1 mL), the mixture was reacted at 45 °C for 1 hour. The reaction solution was concentrated to obtain target product **UBI-1270i** (250 mg, yield 100%) as a colorless oil. The crude product was directly used in the next reaction. LCMS [M+H]⁺=315

### Step 7: UBI-1270 (V2037-070)

Compound **P1** (338 mg, 0.80 mmol) was dissolved in N,N-dimethylformamide (6 mL), then HATU (363 mg, 0.96 mmol) and DIEA (308 mg, 2.39 mmol) were added. After reacting at room temperature for 20 minutes, **UBI-1270i** (250 mg, 0.80 mmol) was added. Then the reaction was carried out at room temperature overnight. The reaction solution was added with water (10 mL), and extracted with ethyl acetate (15 mL), and the organic phase was concentrated, then isolated by column chromatography (methanol/dichloromethane=1/10) to obtain target product **UBI-1270** (400 mg, yield 70%) as a light yellow oil. LCMS [M+H]⁺=722. ¹H NMR (400 MHz, DMSO-*d*₆) δ 8.47 - 8.30 (m, 2H), 7.85 (s, 1H), 7.60 (s, 1H), 7.49 (d, *J* = 8.0 Hz, 2H), 4.41 - 4.30 (m, 1H), 4.24 (dd, *J* = 7.6, 3.6 Hz, 1H), 3.93 (s, 3H), 3.59 - 3.51 (m, 4H), 3.49 (dd, *J=* 6.1, 3.7 Hz, 2H), 3.42 (q, *J=* 5.9 Hz, 2H), 3.36 (t, *J=* 6.2 Hz, 2H), 3.24 (d, *J* = 4.0 Hz, 4H), 3.21 (d, *J* = 5.3 Hz, 1H), 3.20 - 3.15 (m, 2H), 2.81 (s, 1H), 2.33 (s, 2H), 1.89 (d, *J=* 8.5 Hz, 2H), 1.77 (ddd, *J=* 14.6, 7.8, 3.7 Hz, 4H), 1.64 (dt, *J=* 14.4, 4.7 Hz, 5H), 1.37 (s, 9H), 0.76 (t, *J=* 7.5 Hz, 3H).

### Example 1.3.4 Synthesis method of Compound UBI-1271 (P1-13h)

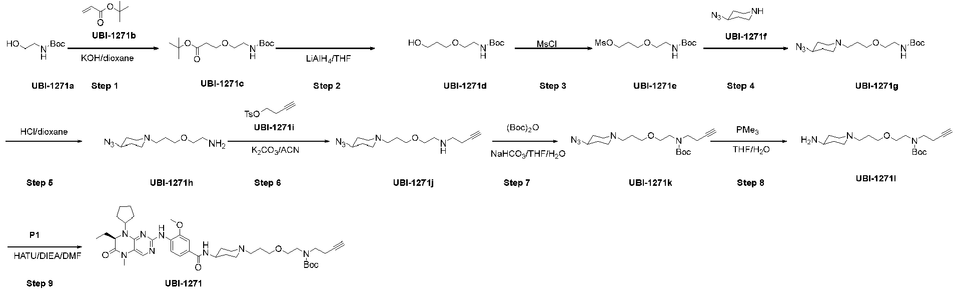

### Step 1: UBI-1271c (V)

Compound **UBI-1271a** (10 g, 64.6 mmol) was dissolved in dioxane (25 mL) and 60% potassium hydroxide (10 mL), and **UBI-1271b** (15 g, 129.3 mmol) was added, the mixture was reacted 25 °C for 18 hours. The reaction solution was concentrated and extracted three times with dichloromethane (30 mL). The organic phase was concentrated and isolated by column chromatography (petroleum ether/ethyl acetate= 0-100%) to obtain target product **UBI-1271c** (8 g, yield 43%) as a colorless oil. LCMS [M+H]⁺ = 290. ¹H NMR (400 MHz, DMSO-*d*₆) δ 6.81 - 6.58 (m, 1H), 3.56 (t, *J* = 6.2 Hz, 2H), 3.35 (t, *J=* 6.1 Hz, 2H), 3.04 (q, *J* = 6.0 Hz, 2H), 2.41 (t, *J* = 6.2 Hz, 2H), 1.38 (d, *J* = 11.9 Hz, 18H).

### Step 2: UBI-1271d (V2037-056)

Compound **UBI-1271c** (8 g, 28 mmol) was dissolved in tetrahydrofuran (80 mL), and the mixture was cooled to 0°C followed by adding LiAlH₄/THF (30.4 mL), and reacted at room temperature for 1 hour. The reaction solution was quenched by adding water (100 mL), and extracted with ethyl acetate (150 mL), the organic phase was concentrated, then isolated by column chromatography (methanol/dichloromethane=1/10) to obtain target product **UBI-1271d** (4.6 g, yield 75 %) as a colorless oil. LCMS [M+H]⁺=220. ¹H NMR (400 MHz, chloroform-*d*) δ 4.84 (s, 1H), 3.76 (t, *J=* 5.7 Hz, 2H), 3.63 (t, *J=* 5.8 Hz, 2H), 3.54 - 3.42 (m, 2H), 3.31 (t, *J* = 5.2 Hz, 2H), 2.12 (s, 1H), 1.83 (p, *J* = 5.8 Hz, 2H), 1.45 (s, 9H).

### Step 3: UBI-1271e (V2037-060)

Compound **UBI-1271d** (1g, 4.6 mmol) was dissolved in dichloromethane (10 mL), then triethylamine (553 mg, 5.5 mmol) was added, then methanesulfonyl chloride (680 mg, 5.9 mmol) dissolved in dichloromethane (10 mL) was slowly added to the reaction solution dropwise. After the reaction solution was reacted at room temperature for 2 hours, water (10 mL) was added, and the reaction solution was extracted with dichloromethane (20 mL), and the organic phase was concentrated to obtain target product **UBI-1271e** (1.2 g, yield 88 %) as a yellow oil. The crude product was directly used in the next reaction. LCMS [M+H]⁺=298

### Step 4: UBI-1271g (V2037-061)

Compound **UBI-1271f** (742 mg, 4.6 mmol) was dissolved in acetonitrile (15 mL), then potassium carbonate (1.4 g, 10.0 mmol), and **UBI-1271e** (1.4 g, 4.6 mmol) were added, and the mixture was heated to 60°C and reacted for 16 hours. The reaction solution was filtered. The filtrate was concentrated and isolated by column chromatography (methanol/dichloromethane = 0% to 10%) to obtain target product **UBI-1271g** (1.2 g, yield 60%) as a colorless oil. LCMS [M+H]⁺=328

¹H NMR (400 MHz, chloroform-*d*) δ 5.04 (s, 1H), 3.85 - 3.69 (m, 2H), 3.58 (m, 2H), 3.51 (q, *J* = 6.3, 5.7 Hz, 2H), 3.30 (q, *J* = 5.3 Hz, 2H), 2.92 (m, 4H), 1.93 - 1.79 (m, 2H), 1.57 (m, 4H), 1.45 (s, 9H).

### Step 5: UBI-1271h (V2037-064)

Compound **UBI-1271g** (1.6 g, 4.9 mmol) was dissolved in dichloromethane (15 mL), then HCl/dioxane (15 mL) was added and the mixture was reacted at room temperature for 2 hours. The reaction solution was concentrated to obtain target product **UBI-1271h** (1.2 g, yield 100 %) as a yellow oil. The crude product was directly used in the next reaction. LCMS [M+H]⁺=228

### Step 6: UBI-1271j (V2037-066)

Compound **UBI-1271h** (600 mg, 2.3 mmol) was dissolved in acetonitrile (40 mL), then potassium carbonate (691 mg, 5.0 mmol), a catalytic amount of potassium iodide and **UBI-1271i** (510 mg, 2.3 mmol) were added, then the reaction solution was heated to 90°C and reacted for 16 hours. The reaction solution was filtered. The filtrate was concentrated and isolated by column chromatography (methanol/dichloromethane = 0% to 10%) to obtain target product **UBI-1271j** (230 mg, yield 36 %) as a yellow oil. LCMS [M+H]⁺=280. ¹H NMR (400 MHz, chloroform-*d*) δ 4.05 (t, *J=* 5.1 Hz, 3H), 3.81 - 3.51 (m, 5H), 3.41 - 2.92 (m, 8H), 2.59 (m, 2H), 2.28 - 1.91 (m, 5H).

### Step 7: UBI-1271k (V2037-068)

Compound **UBI-1271j** (120 mg, 0.43 mmol) was dissolved in tetrahydrofuran (10 mL) and water (2 mL), then (Boc)₂O (141 mg, 0.65 mmol) and sodium bicarbonate (72 mg, 0.86 mmol) were added, and the mixture was reacted at room temperature for 1 hour. The reaction solution was added with water (10 mL), then extracted with ethyl acetate (15 mL), and the organic phase was concentrated, then isolated by column chromatography (methanol/dichloromethane=1/10) to obtain target product **UBI-1271k** (110 mg, yield 67 %) as a colorless oil. LCMS [M+H]⁺=380

### Step 8: UBI-12711 (V2037-072)

Compound **UBI-1271k** (230 mg, 0.61 mmol) was dissolved in tetrahydrofuran (5 mL), then triphenylphosphine (0.73 mL) was added, and the mixture was heated at 45°C for 1 hour. Then water (1 mL) was added, the mixture was reacted for another 1 hour. The reaction solution was concentrated to obtain target product **UBI-1271l** (210 mg, yield 100 %) as a colorless oil. The crude product was directly used in the next reaction. LCMS [M+H]⁺=354

### Step 9: UBI-1271 (V2037-073)

Compound **P1** (253 mg, 0.59 mmol) was dissolved in N,N-dimethylformamide (6 mL), then HATU (271 mg, 0.71 mmol) and DIEA (230 mg, 1.78 mmol) were added, and the mixture was reacted at room temperature for 20 minutes, followed by adding **UBI-12711** (210 mg, 0.59 mmol) and reacting at room temperature for 16 hours. The reaction solution was added with water (10 mL), and extracted with ethyl acetate (20 mL), and the organic phase was concentrated, then isolated by column chromatography (methanol/dichloromethane=1/10) to obtain target product **UBI-1271** (360 mg, yield 80 %) as a light yellow oil. LCMS [M+H]⁺=761

### Example 1.3.5 Synthesis method of Compound UBI-1272 (P1-14h)

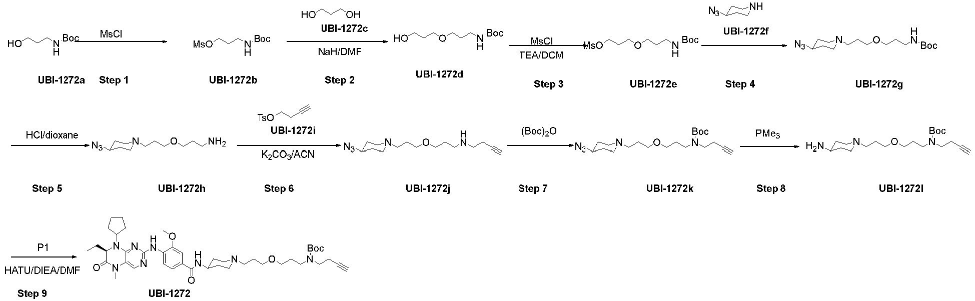

### Step 1: UBI-1272b (V2128-008)

To a solution of compound **UBI-1272a** (5g, 28.5 mmol) and triethylamine (3.5g, 34.2 mmol) in dry dichloromethane (50 ml) was slowly added methanesulfonyl chloride (4.2g, 37.1 mmol), the reaction was stirred at 0°C for 30 minutes, then warmed up to room temperature and reacted for 5 hours. The reaction solution was added with water(10 mL), and extracted with dichloromethane (10 mL) three times. The organic layer was washed with brine, dried over anhydrous sodium sulfate and concentrated to obtain target product **UBI-1272b** (7.2 g, yield 100 %) as a colorless oil.

### Step 2: UBI-1272d (V2128-009)

At 0°C, to the solution of compound **UBI-1272c** (12.6g, 165.8mmol) in N,N-dimethylformamide (80ml) was added 60% sodium hydride (2.2g, 55.3mmol). After reacting at room temperature for 30 minutes, **UBI-1272b** (7g, 27.6mmol) in N,N-dimethylformamide (20ml) was slowly added, then the mixture was reacted at room temperature for 12 hours. The reaction was quenched with saturated ammonium chloride solution at 0 °C. The resulting mixture was concentrated under reduced pressure. The residue was diluted with brine and extracted with ethyl acetate. The combined organic layers were washed with brine and dried over anhydrous sodium sulfate, filtered and concentrated. The residue was purified by silica gel column chromatography, and eluted with 33% ethyl acetate in petroleum ether to obtain target product **UBI-1272d** (2.6 g, yield 40 %) as a light yellow oil.

¹H NMR (400 MHz, DMSO-*d*₆) δ 6.75 (s, 1H), 4.36 (t, *J=* 5.2 Hz, 1H), 3.52 - 3.33 (m, 6H), 2.98 - 2.92 (m, 2H), 1.60 (dt, *J* = 17.0, 6.6 Hz, 4H), 1.37 (s, 9H).

### Step 3: UBI-1272e (V2037-076)

Compound **UBI-1272d** (1.5 g, 6.4 mmol) was dissolved in dichloromethane (20 mL), and triethylamine (780 mg, 7.7 mmol) was added. Methanesulfonyl chloride (958 mg, 8.4 mmol) dissolved in dichloromethane (10 mL) was slowly added to the reaction solution, and the mixture was reacted at room temperature for 2 hours. The reaction solution was added with water (10 mL), extracted with dichloromethane (15 mL), and the organic phase was dried by rotary dryer to obtain target product **UBI-1272e** (2.0 g, yield 100 %) as a light yellow oil. The crude product was directly used in the next reaction. LCMS [M+H]⁺=312

### Step 4: UBI-1272g(V2037-079)

Compound **UBI-1272f** (1.0 g, 6.4 mmol) was dissolved in acetonitrile (50 mL), then potassium carbonate (2.0g, 14.2 mmol), a catalytic amount of potassium iodide and **UBI-1272e** (2.0 g, 6.4 mmol) were added, and the mixture was heated to 90°C and reacted for 16 hours. The reaction solution was filtered. The filtrate was concentrated and isolated by column chromatography (methanol/dichloromethane = 0% to 10%) to obtain target product **UBI-1272g** (550 mg, yield 26 %) as a yellow oil. LCMS [M+H]⁺=342. ¹H NMR (400 MHz, chloroform-*d*) δ 4.90 (s, 1H), 3.47 (dd, *J=* 11.7, 5.8 Hz, 6H), 3.22 (q, *J=* 6.3 Hz, 2H), 2.84 (m, 2H), 2.53 (m, 2H), 2.34 (m, 1H), 2.02 (m, 2H), 1.89 - 1.62 (m, 6H), 1.44 (s, 9H).

### Step 5: UBI-1272h(V2037-080)

Compound **UBI-1272g** (550 mg, 1.61 mmol) was dissolved in dichloromethane (5 mL), then HCl/dioxane (3 mL) was added and the mixture was reacted at room temperature for 2 hours. The reaction solution was dried by rotary dryer to obtain crude product **UBI-1272h** (500 mg, yield 100 %) as a yellow oil. The crude product was directly used in the next reaction.

LCMS [M+H]⁺=242

### Step 6: UBI-1272j(V2037-081)

Compound **UBI-1272h** (570 mg, 2.0 mmol) was dissolved in acetonitrile (40 mL), then potassium carbonate (621 mg, 4.5 mmol), a catalytic amount of potassium iodide and **UBI-1272i** (458 mg, 2.0 mmol) were added, then the mixture was heated to 90°C and reacted for 16 hours. The reaction solution was filtered. The filtrate was dried by rotary dryer and isolated by column chromatography (methanol/dichloromethane = 0% to 10%) to obtain target product **UBI-1272j** (470 mg, yield 78 %) as a yellow oil. LCMS [M+H]⁺=294

### Step 7: UBI-1272k(V2037-082)

Compound **UBI-1272j** (470 mg, 1.6 mmol) was dissolved in tetrahydrofuran (10 mL) and water (2 mL), then (Boc)₂O (525 mg, 2.4 mmol) and sodium bicarbonate (269 mg, 3.2 mmol) were added, and the mixture was reacted at room temperature for 1 hour. The reaction solution was added with water (10 mL), and extracted with ethyl acetate (20 mL), the organic phase was dried by rotary dryer, then isolated by column chromatography (methanol/dichloromethane= 1/10) to obtain target product **UBI-1272k** (250 mg, yield 40 %) as a colorless oil. LCMS [M+H]⁺=394. ¹H NMR (400 MHz, chloroform-*d*) δ 3.50 - 3.22 (m, 9H), 2.79 (m, 2H), 2.42 (m, 4H), 2.28 - 2.10 (m, 2H), 2.05 - 1.86 (m, 3H), 1.77 (tt, *J* = 14.2, 6.7 Hz, 6H), 1.46 (s, 9H).

### Step 8: UBI-1272l (V2037-083)

Compound **UBI-1272k** (250 mg, 0.64 mmol) was dissolved in tetrahydrofuran (5 mL), then triphenylphosphine (0.95 mL) was added, and the mixture was heated to 45°C and reacted for 1 hour. Water (1 mL) was added, the mixture was continued to react at 45 °C for 1 hour. The reaction solution was dried by rotary dryer to obtain target product **UBI-1272l** (230 mg, yield 99 %) as a colorless oil. The crude product was directly used in the next reaction. LCMS [M+H]⁺=368

### Step 9: UBI-1272 (V2037-084)

Compound **P1** (266 mg, 0.63 mmol) was dissolved in N,N-dimethylformamide (6 mL), then HATU (286 mg, 0.75 mmol) and DIEA (243 mg, 1.88 mmol) were added. After reacting at room temperature for 20 minutes, **UBI-1272l** (230 mg, 0.63 mmol) was added, and then reacting at room temperature for 16 hours. The reaction solution was added with water (10 mL), and extracted with ethyl acetate (20 mL), the organic phase was dried by rotary dryer, then isolated by column chromatography (methanol/dichloromethane=1/10) to obtain target product **UBI-1272** (380 mg, yield 78%) as a yellow oil. LCMS [M+H]⁺=775

### Example 1.3.6 Synthesis method of Compound UBI-1273 (P1-15h)

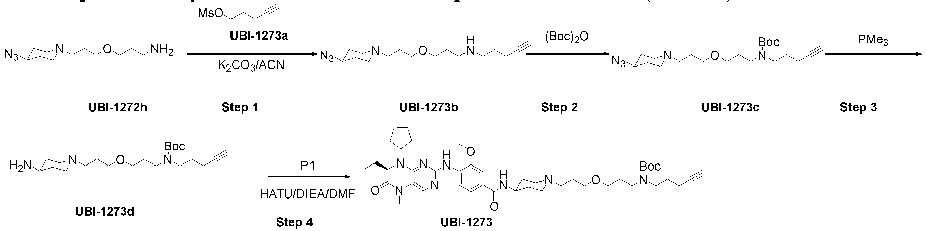

### Step 1: UBI-1273b (V2037-087)

Compound **UBI-1272h** (570 mg, 2.1 mmol) was dissolved in acetonitrile (40 mL), then potassium carbonate (621 mg, 4.5 mmol), a catalytic amount of potassium iodide and **UBI-1273a** (333 mg, 2.1 mmol) were added, and the mixture was warmed up to 90°C and reacted for 16 hours. The reaction solution was filtered. The filtrate was dried by rotary dryer and isolated by column chromatography (methanol/dichloromethane = 0% to 10%) to obtain target product **UBI-1273b** (400 mg, yield 19 %) as a yellow oil.

LCMS [M+H]⁺=308

### Step 2: UBI-1273c (V2037-089)

Compound **UBI-1273b** (500 mg, 1.6 mmol) was dissolved in tetrahydrofuran (10 mL) and water (2 mL), then (Boc)₂O (532 mg, 2.4 mmol) and sodium bicarbonate (274 mg, 3.3 mmol) were added, and the mixture was reacted at room temperature for 1 hour. The reaction solution was added with water (10 mL), and extracted with ethyl acetate (20 mL), the organic phase was dried by rotary dryer, then isolated by column chromatography (methanol/dichloromethane=1/10) to obtain target product **UBI-1273c**(250 mg, yield 38%) as a colorless oil.

LCMS [M+H]⁺=408

¹H NMR (400 MHz, chloroform-*d*) δ 3.57 - 3.36 (m, 5H), 3.27 (m, 4H), 2.80 (s, 2H), 2.45 (s, 2H), 2.19 (td, *J=* 7.1, 2.7 Hz, 3H), 1.96 (t, *J=* 2.7 Hz, 3H), 1.86 - 1.54 (m, 8H), 1.45 (s, 9H).

### Step 3: UBI-1273d (V2037-091)

Compound **UBI-1273c** (250 mg, 0.61 mmol) was dissolved in tetrahydrofuran (5 mL), then triphenylphosphine (0.92 mL) was added, and the mixture was heated to 45°C and reacted for 1 hour. Then water (1 mL) was added, the mixture was continued to react at 45 °C for 1 hour. The reaction solution was dried by rotary dryer to obtain target product **UBI-1273d** (230 mg, yield 100 %) as a colorless oil. The target product was directly used in the next reaction.

LCMS [M+H]⁺=382

### Step 4: UBI-1273 (V2037-093)

Compound **P1** (257 mg, 0.60 mmol) was dissolved in N,N-dimethylformamide (6 mL), then HATU (275 mg, 0.72 mmol) and DIEA (234 mg, 1.81 mmol) were added. After reacting at room temperature for 20 minutes, **UBI-1273d** (230 mg, 0.60 mmol) was added, and continued to react at room temperature for 16 hours. The reaction solution was added with water (10 mL), and extracted with ethyl acetate (20 mL), the organic phase was dried by rotary dryer, then isolated by column chromatography (methanol/dichloromethane=1/10) to obtain target product **UBI-1273** (330 mg, yield 69 %) as a light yellow oil.

LCMS [M+H]⁺=790

### Example 1.3.7 Synthesis method of Compound UBI-1275 (P1-13h)

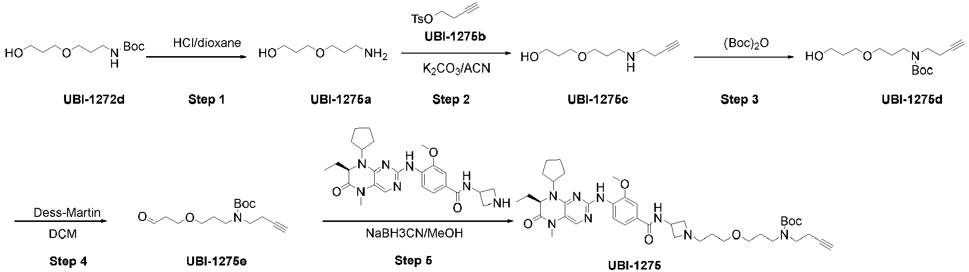

### Step 1: UBI-1275a (V2037-109)

Compound **UBI-1272d** (600 mg, 2.6 mmol) was dissolved in dichloromethane (10 mL), then HCl/dioxane (5 mL) was added and the mixture was reacted at room temperature for 2 hours. The reaction solution was dried by rotary dryer to obtain target product **UBI-1275a** (500 mg, yield 100 %) as a yellow oil. The crude product was directly used in the next reaction.

LCMS [M+H]⁺=134

### Step 2: UBI-1275c (V2037-110)

Compound **UBI-1275a** (436 mg, 2.6 mmol) was dissolved in acetonitrile (40 mL), then potassium carbonate (782 mg, 5.7 mmol), a catalytic amount of potassium iodide and **UBI-1275b** (577 mg, 2.6 mmol) were added, and the mixture was warmed up to 90°C and reacted for 16 hours. The reaction solution was filtered. The filtrate was dried by rotary dryer and isolated by column chromatography (methanol/dichloromethane = 0% to 10%) to obtain target product **UBI-1275c** (400 mg, yield 86 %) as a yellow oil.

LCMS [M+H]⁺=186

### Step 3: UBI-1275d (V2037-111)

Compound **UBI-1275c** (600 mg, 3.2 mmol) was dissolved in tetrahydrofuran (10 mL) and water (2 mL), then (Boc)₂O (1.1 g, 4.9 mmol) and sodium bicarbonate (545 mg, 6.5 mmol) were added, and the mixture was reacted at room temperature for 1 hour. The reaction solution was added with water (10 mL), and extracted with ethyl acetate (20 mL), the organic phase was dried by rotary dryer, then isolated by column chromatography (methanol/dichloromethane=1/10) to obtain target product **UBI-1275d** (260 mg, yield 28 %) as a colorless oil.

LCMS [M+H]⁺=286

### Step 4: UBI-1275e (V2128-054)

Compound **UBI-1275d** (245 mg, 0.86 mmol) was dissolved in dichloromethane (8 ml), then Dess-Martin Periodinane (546 mg, 1.3 mmol) was added and the mixture was reacted at room temperature for 1 hour. After filtration, the reaction solution was directly used in the next step.

LCMS [M+1]⁺=284.

### Step 5: UBI-1275 (V2128-056)

Compound **UBI-1275e** (220 mg, 0.78 mmol) and **UBI-1275f** (280 mg, 0.54 mmol) were dissolved in dichloromethane/methanol=10/1 (16 ml), and sodium acetate (106mg, 0.78mmol) was added, the mixture was continued to react at room temperature for 1 hour. Then the mixture was added with sodium cyanoborohydride(49mg, 0.78mmol), and continued to react at room temperature for 3 hours. The reaction solution was added water and extracted with dichloromethane three times, washed with water and brine, dried over anhydrous sodium sulfate, filtered and concentrated to obtain the crude product. The crude product was isolated by column chromatography (methanol/dichloromethane = 0-10%) to obtain target product **UBI-1275** (55mg, yield 9%) as a yellow solid.

LCMS [M + H] + = 747.

### Example 1.3.8 Synthesis method of Compound UBI-1274 (NH₂-10h-A1)

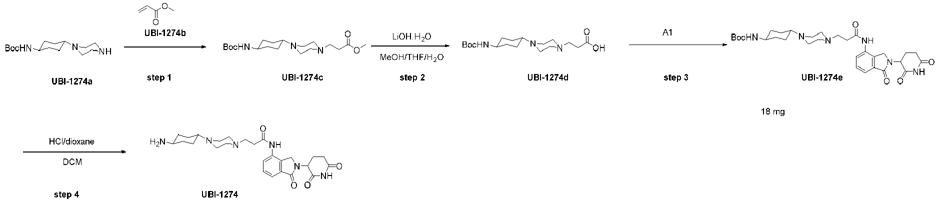

### Step 1: UBI-1274c (V2127-034)

Compound **UBI-1274a** (900 mg, 3.18 mmol), DIEA (410 mg, 3.18 mmol) and **UBI-1274b** (411 mg, 4.77 mmol) were dissolved in acetonitrile (30 mL), and then heated to 80°C and reacted for 18 hours. The reaction solution was dried by rotary dryer and isolated by column chromatography (petroleum ether/ethyl acetate = 50% to 100%, 20 minutes, then methanol/dichloromethane = 0% to 10%, 40 minutes) to obtain target product **UBI-1274c** (1.0 g, yield 86%) as a white solid.

### Step 2: UBI-1274d (V2037-116)

Compound **UBI-1274c** (260 mg, 0.70 mmol) was dissolved in methanol (2 mL), tetrahydrofuran (6 mL) and water (2 mL), then lithium hydroxide (36 mg, 0.85 mmol) was added, and the mixture was reacted at room temperature for 1 hour. The reaction solution was washed once with ethyl acetate (15 mL), and the aqueous phase was adjusted to pH=6 with 3N HCl and concentrated to obtain target product **UBI-1274d** (330 mg, yield 100 %) as a white solid.

LCMS [M+H]⁺=356

### Step 3: UBI-1274e (V2037-113)

Compound **UBI-1274d** (250 mg, 0.70 mmol) was dissolved in N,N-dimethylformamide (6 mL) and then added HATU (321 mg, 0.85 mmol) and DIEA(191 mg, 0.50 mmol), followed by DIEA (273 mg, 2.11 mmol). After reacting at room temperature for 20 minutes, lenalidomide (182 mg, 0.70 mmol) was added, and continued to react at room temperature for 16 hours. The reaction solution was added with water (10 mL), and extracted with ethyl acetate (20 mL), and the organic phase was dried by rotary dryer, then isolated by preparation plate (methanol/dichloromethane=1/10) to obtain target product **UBI-1274e** (18 mg, yield 4 %) as a yellow oil.

LCMS [M+H]⁺=597

### Step 4: UBI-1274 (V2037-114)

Compound **UBI-1274e** (20 mg, 0.03 mmol) was dissolved in dichloromethane (2 mL), then HCl/dioxane (0.3 mL) was added and the mixture was reacted at room temperature for 2 hours. The reaction solution was dried by rotary dryer to obtain target product **UBI-1274** (15 mg, yield 100 %) as a yellow oil. The crude product was directly used in the next reaction.

LCMS [M+H]⁺=497

### Example 1.3.9 Synthesis method of Compound UBI-1278 (9h-A1)

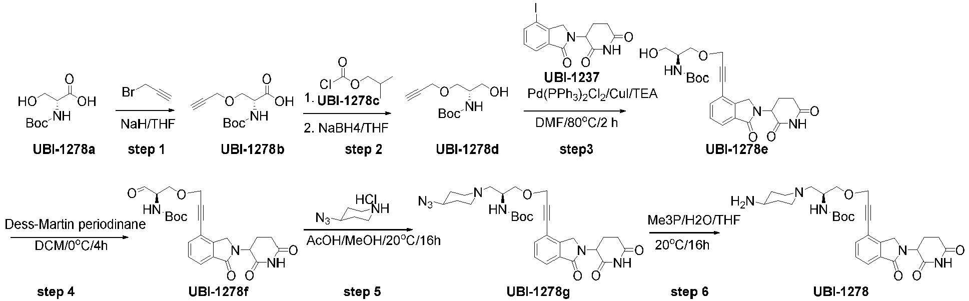

### Step 1: UBI-1278b (V1661-133)

UBI-1278a (4.197 g, 20.5 mmol) was dissolved in anhydrous DMF (60 mL), and cooled to 0°C, sodium hydride (1.64 g, 41 mmol) was added to above solution. The reaction mixture was stirred at 0°C for 30 min. Propargyl bromide (80% in toluene, 2.2 mL, 20.5 mmol) was added slowly dropwise to the system (15 min), and the reaction was continued at 0°C for 2 h until the reaction was completed. The reaction solution was quenched with water (50 mL), and extracted with ethyl acetate (50 mL*3). The organic phases were combined, and dried over anhydrous sodium sulfate. The reaction solution was concentrated and isolated by silica gel column chromatography (petroleum ether: ethyl acetate = 2:1) to obtain UBI-1278b (4.86 g, yield 98%). LC-MS: [M-56] ⁺= 188.2

### Step 2: UBI-1278d (V1661-136)

UBI-1278b (1 g, 6.94 mmol), and triethylamine(1.1mL, 7.8mmol) were dissolved in tetrahydrofuran(20 mL), cooled to -10°C. UBI-1278c (1.0 mL, 7.8mmol) was added dropwise, the mixture was stirred at -10°C for 1 hour. The reaction system was heated to 0°C. A mixture of NaBH4 (0.78 g, 20.8 mmol) dissolved in tetrahydrofuran (10mL) and water (2mL) was added dropwise into the above system. The mixture was stirred at 0°C for 1 hour. The mixture was poured into a 20% aqueous solution of citric acid. The mixture was extracted with ethyl acetate (30 mL×3). The organic phases were combined, washed with saturated brine, dried (MgSO4), and filtered. The mixture was concentrated and isolated by silica gel column chromatography (petroleum ether: ethyl acetate = 8:1) to obtain UBI-1278d (0.9 g, yield 100%). LC-MS: [M-56] ⁺= 174.2

### Step 3: UBI-1278e (V2031-017)

UBI-1278d (300 mg, 1.3 mmol), UBI-1237(480 mg, 1.3 mmol), PdCl₂(PPh₃)₂(46 mg, 0.06 mmol), copper iodide (25 mg), and triethylamine (400 mg) were added to anhydrous DMF (10mL) under nitrogen protection, the reaction system was heated to 80°C, and stirred for 2 hours. After cooling to room temperature, the mixture was added into water, extracted with dichloromethane, washed with water, dried over sodium sulfate, and filtered, and the reaction solution was concentrated and then isolated by silica gel column chromatography (dichloromethane/methanol=10%) to obtain UBI-1278e (200 mg, yield 32.4%). LC-MS: [M+H] ⁺= 472.5

### Step 4: UBI-1278f (V2031-018)

Dess-Martin Periodinane (555 mg, 1.31 mmol) was added to a solution of UBI-1278e (200 mg,0.87 mmol) in dichloromethane (10mL), and the mixture was stirred at 0°C for 1 hour. The reaction was quenched with saturated sodium thiosulfate solution and extracted with dichloromethane. The organic phase was washed with saturated sodium bicarbonate solution, brine and water, and dried over sodium sulfate. The reaction solution concentrated and then purified by silica gel column chromatography(dichloromethane /methanol =10%) to obtain UBI -1278f (110 mg, yield 55.3%) as a yellow solid. LC-MS: [M+H] ⁺= 580.2

### Step 5: UBI-1278h (V2031-027)

UBI- 1278f (40mg,0.24mmol), UBI- 1278g (1 13mg,0.24mmol), and acetic acid (10mg) were added to methanol(2mL), and the mixture was stirred for 1 hour. Then sodium cyanoborohydride (30 mg, 0.48 mmol) was added and the reaction system was stirred at 60°C for 3 h. After the reaction was completed (5 mL) and extracted with ethyl acetate (20 mL × 3). The organic phases were combined, and dried over anhydrous sodium sulfate. The reaction solution concentrated and then purified by silica gel column chromatography(dichloromethane /methanol =10%) to obtain compound UBI-1278h (40 mg, yield 81%). LC-MS: [M+H] ⁺= 580.2

### Step 6: UBI-1278 (V2031-035)

PMe3 (1mL) was added to UBI-1278h (40 mg, 0.07 mmol) in tetrahydrofuran(5 mL). The system was stirred at 45°C for 1 h, water (0.5 mL) was added, the system was stirred at 20°C for 16 h, concentrated under reduced pressure to obtain target product UBI-1278 (38 mg, 100% yield) as a colorless oil. LCMS: [M+H]⁺ = 554.3

### Example 1.3.10 Synthesis method of Compound UBI-1279 (P1-9h)

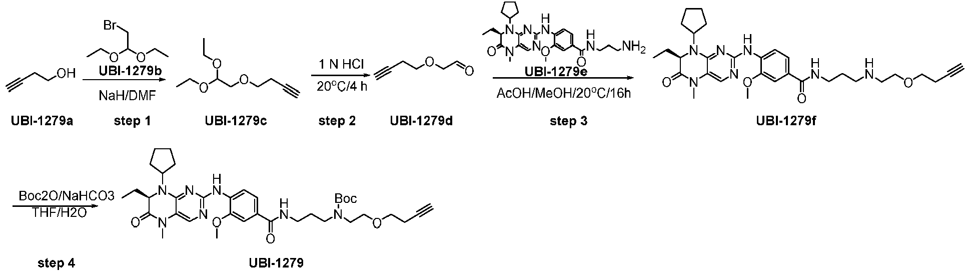

### Step 1: UBI-1279c (V2031-047)

Under argon protection, UBI-1279a (500mg, 7.14 mmol) was added to anhydrous DMF (10mL), sodium hydride (571 mg, 14.3 mmol) was added to the system in batches. After stirring the reaction mixture for 30 min, UBI-1279b (2.11 g,10.7 mmol) was added. After completion of addition, the reaction system was stirred at 0°C for 2 hours, until being completely transformed (TLC analysis monitoring, ethyl acetate: petroleum ether = 1:10). The reaction was quenched with water (20 mL), and solvent was removed under reduced pressure. The mixture was extracted with ethyl acetate (30mL×3), dried over anhydrous sodium sulfate. The reaction solution was concentrated and then isolated by silica gel column chromatography (0% -20% ethyl acetate: petroleum ether) to obtain UBI-1279c (0.72 g, 54.1% yield) as a colorless oil. LCMS: [M+H]⁺ = 187.2

¹H NMR (400 MHz, chloroform-*d*) δ 4.63 (t, J = 5.1 Hz, 1H), 3.72 - 3.57 (m, 6H), 3.53 (d, J = 5.2 Hz, 2H), 2.48 (td, J = 7.0, 2.7 Hz, 2H), 1.97 (t, J = 2.7Hz, 1H), 1.22 (dd, J = 7.1, 1.4 Hz, 6H).

### Step 2: UBI-1279d (V2031-062)

UBI-1279c (0.72 g, 3.87 mmol) was added to 1N HCl (20mL) aqueous solution. The mixture was stirred at room temperature for 2 hours. After the completion of the reaction, the mixture was extracted with dichloromethane (30mL×2), dried over anhydrous sodium sulfate, filtered, concentrated to obtain UBI-1279d(430 mg, yield 100%). LCMS: [M+H]⁺ = 113.1

### Step 3: UBI-1205f (V2031-062)

UBI-1279d (50mg,0.45mmol), UBI-1279e(230mg,0.45mmol), and acetic acid (50mg) were added to methanol(5mL), the reaction system was stirred for 1 hour. Then sodium cyanoborohydride (56mg,0.89mmol) was added, and the reaction was stirred at °C for 16 hours. After completion of the reaction, it was quenched with water (20mL), and extracted with ethyl acetate(30mL×3). The organic layers were combined, dried over anhydrous sodium sulfate and filtered. The reaction solution was concentrated and then isolated by silica gel column chromatography (dichloromethane /methanol =0 to 10%) to obtain UBI -1279f (115 mg, yield 44.6% as a white solid LCMS: [M+H]⁺ = 578.3

### Step 4: UBI-1279 (V2031-063)

UBI-1279f (100mg, 0.48 mmol), tert-butyl dicarbonate (57 mg, 0.26mmol), sodium bicarbonate(43 mg), and water (2 mL) were added into tetrahydrofuran (5 mL). The reaction was stirred at room temperature for 3 hours. After completion of reaction, the reaction solution was concentrated and then performed chromatographic elution by silica gel column chromatographic separation (dichloromethane/methanol=0 to 10%) to obtain UBI-1279 (50 mg,42.6% yield) as a yellow oil. LCMS:[M+H]⁺ = 678.3

### Example 1.3.11 Synthesis method of Compound UBI-1280 (P1-11h)

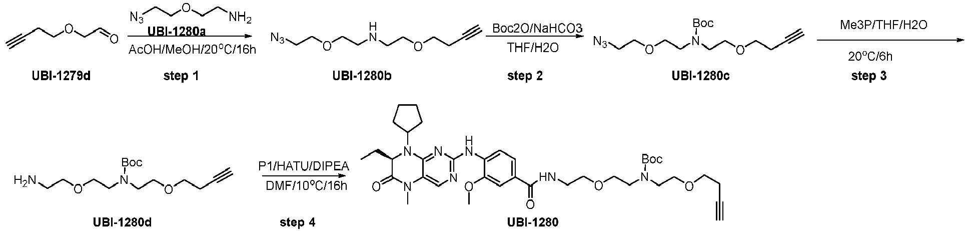

### Step 1: UBI-1280b (V2031-075)

UBI-1279d(250mg,2.23mmol), BI-1280a(290mg,2.23mmol), and acetic acid (50mg) were added into methanol (5mL), the reaction system was stirred for 1 hour. Sodium cyanoborohydride (281.3mg,4.46mmol) was added to system, and the mixture was stirred at 20°C for 16 hours. After completion of the reaction, the reaction solution was concentrated and then isolated by silica gel column chromatography (dichloromethane /methanol = 0 to 20%) to obtain UBI -1280b (200mg, yield 39.6%) as a yellow oil. LCMS: [M+H]⁺ = 227.2

### Step 2: UBI-1280c (V2031-078)

UBI-1280b (200mg, 0.88 mmol), tert-butyl dicarbonate (290 mg, 1.33mmol), sodium bicarbonate (223 mg), and water (2 mL) were added into tetrahydrofuran (5 mL). The reaction was stirred at room temperature for 3 hours. After completion of the reaction, the reaction solution was concentrated and then isolated by silica gel column chromatography (petroleum ether/ethyl acetate = 1 /1) to obtain UBI-1280c (200 mg, yield 69.3%) as a yellow oil. LCMS: [M+H]⁺ = 327.2

### Step 3: UBI-1280d (V2031-080)

1 mL 1 M triphenylphosphine (in tetrahydrofuran) was added to UBI-1280c (200 mg) in 5mL tetrahydrofuran and 0.1 mL water at room temperature. The reaction system was stirred overnight, after the reaction was completed. The reaction was dried under vacuum to give UBI-1280d (170 mg, 95.9% yield), which was not purified and used directly in the next step. LCMS: [M+H] ⁺= 301.2

### Step 4: UBI-1280 (V2031-086)

P₁ (170 mg, 0.4mmol), UBI-1280d (150 mg,0.5 mol), HATU (285 mg, 0.75 mmol), and diisopropylethylamine (193.5mg, 1.5 mol) were dissolved in DMF (10 mL), and the mixture was stirred at room temperature for 18 hours. After the reaction was completed, the mixture was poured into water (20 mL) and then extracted with ethyl acetate (20 mL × 3). The combined organic layer was washed with water, dried over anhydrous sodium sulfate and concentrated and then isolated and purified by silica gel column chromatography (dichloromethane /methanol =10%) to obtain UBI -1280 (100 mg, yield 35.4%) as a yellow solid. LCMS: [M+H] ⁺= 708.4

### Example 1.3.12 Synthesis method of Compound UBI-1281 (P1-12h)

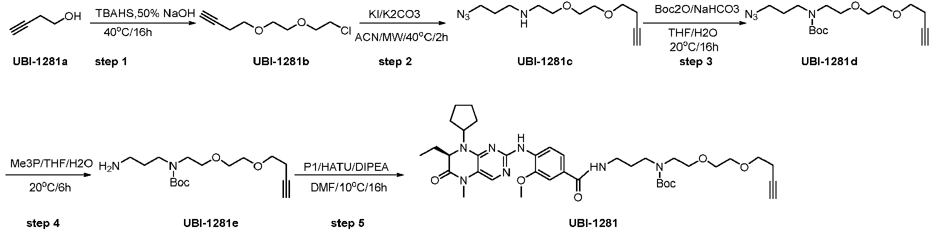

### Step 1: UBI-1281b (V2031-076)

UBI-1281a (2.0 g, 28.57 mmol), tetrabutylammonium bisulfate (0.97 g, 2.86 mmol) and bis(2-chloroethyl) ether (12.2 g, 85.7 mmol) were added to 50% aqueous sodium hydroxide solution (12 mL) and the reaction system was stirred vigorously at 40°C for 16 h. After the reaction was completed (monitored by TLC), the reaction mixture was added to a mixture of 80 mL of water and 80 mL of dichloromethane and extracted with dichloromethane. The organic layers were combined, and dried over sodium sulfate. The reaction solution was concentrated and isolated by silica gel column chromatography (petroleum ether: ethyl acetate = 1:1) to obtain UBI-1281b (3.5 g, yield 69.4%) as a colorless oil. LCMS: [M+H] ⁺= 177.1

¹H NMR (400 MHz, chloroform-d) δ 3.80 - 3.75 (m, 2H), 3.68 - 3.62 (m, 8H), 2.49 (d, J = 2.6 Hz, 2H), 1.98 (s, 1H).

### Step 2: UBI-1281c (V2031-079)

UBI-1281b (200 mg, 1.13 mmol), 3-azidopropylamine (97.7 mg, 1.13 mmol), potassium iodide (188.6 mg, 1.13 mmol), and potassium carbonate (313.6 mg, 2.27 mmol) were added to acetonitrile (5 mL), and the reaction mixture was stirred at 80 °C for 16 h. After completion of reaction, water (5 mL) was added, the mixture was extracted with ethyl acetate, dried (sodium sulfate), filtered and concentrated, and then isolated by silica gel column chromatography (dichloromethane /methanol = 10%) to obtain UBI-1281c (200mg, yield 73.5%) as a colorless oil.

LCMS: [M+H] ⁺= 241.2, ¹H NMR (400 MHz, chloroform-*d*) δ 3.98 - 3.87 (m, 2H), 3.74 - 3.67 (m, 4H), 3.64 (t, J = 6.7 Hz, 2H), 3.57 (t, J = 6.3 Hz, 2H), 3.28 - 3.21 (m, 2H), 3.18(t, J = 7.3 Hz, 2H), 2.50 (td, J = 6.7, 2.7 Hz, 2H), 2.21 - 2.11 (m, 2H), 2.06 (t, J = 2.7 Hz, 1H).

### Step 3: UBI-1281d (V2031-078)

UBI-1281c (200mg, 0.88 mmol), tert-butyl dicarbonate (290 mg, 1.33mmol), sodium bicarbonate (223 mg), and water (2 mL) were added into tetrahydrofuran (5 mL). The reaction system was carried out at room temperature for 3 hours. After completion of the reaction, the reaction solution was concentrated and then isolated by silica gel column chromatography (dichloromethane /methanol = 10%) to obtain UBI-1281d (200 mg, yield 70.6%) as yellow oil. LCMS: [M+H] ⁺= 341.2

### Step 4: UBI-1281e(V2031-081)

At room temperature, 0.88 mL 1 M triphenylphosphine (in tetrahydrofuran) was added to UBI-1281d (200 mg) in 5mL tetrahydrofuran and 0.1 mL water. The reaction system was stirred at room temperature overnight. After the reaction was completed, the mixture was dried by the oil pump to obtain UBI-1281e (150 mg, 81.2% yield). LCMS: [M+H] ⁺= 314.2

### Step 5: UBI-1281 (V2031-082)

P1 (162 mg, 0.38mmol), UBI-1281e (150 mg,0.48 mol), HATU (272 mg, 0.72 mmol), diisopropylethylamine (185mg, 1.43 mmol) were dissolved in DMF (10 mL), the mixture was stirred at room temperature overnight. The mixture was poured into water (20 mL) and then extracted with ethyl acetate (20 mL × 3). The organic layers were combined, washed with brine, dried over sodium sulfate, filtered and concentrated and then isolated by silica gel column chromatography (dichloromethane /methanol =10%) to obtain UBI -1281 (100 mg, yield 36.4%) as a yellow solid. LCMS: [M+H] ⁺= 722.4

### Example 1.3.13 Synthesis method of Compound UBI-1282 (P1-10h)

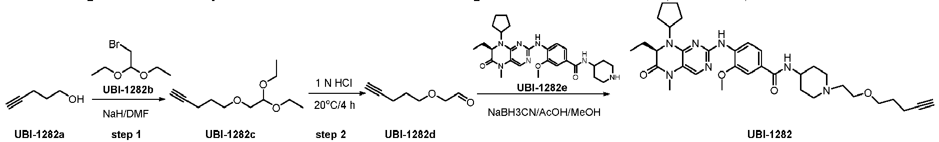

### Step 1: UBI-1282c (V2031-48)

UBI-1282a (500mg, 5.94 mmol) was dissolved in anhydrous DMF (10mL), and sodium hydroxide (356.6mg, 8.92 mmol) was add portionwise. After stirring the reaction system for 30 min, UBI-1282b (1.76 g, 8.92mmol) was added. Then the reaction system was continued to stir at 0°C for 2 hours, until complete transformation (TLC analysis monitoring, ethyl acetate: petroleum ether = 1:10). The reaction was quenched with water (20 mL) after completion, extracted with ethyl acetate (30 mL×3) and dried over sodium sulfate. The organic phases were combined, dried over anhydrous sodium sulfate. The filtrate was concentrated and then isolated by silica gel column chromatography (dichloromethane /methanol =10%) to obtain UBI-1282c (0.9 g, yield 75.6%)

LCMS: [M+H] ⁺= 201.2

¹H NMR (400 MHz, chloroform-*d*) δ 4.62 (t, J = 5.2 Hz, 1H), 3.70 (dq, J = 9.4, 7.0 Hz, 2H), 3.62 - 3.53 (m, 4H), 3.49 (d, J = 5.3 Hz, 2H), 2.29 (td, J = 7.0,2.6 Hz, 2H), 1.94 (t, J = 2.7 Hz, 1H), 1.80 (tt, J = 7.1, 6.2 Hz, 2H), 1.22 (t, J = 7.1 Hz, 6H).

### Step 2: UBI-1282d (V2031-062)

UBI-1282c (0.8 g, 4 mmol) was added to 1 N HCl (20 mL). The reaction system was stirred at room temperature for 2 hours. After the completion of the reaction, the mixture was extracted with dichloromethane (30mL×2), dried over anhydrous sodium sulfate, filtered, concentrated to obtain UBI-1282d (350 mg, yield 69.4%). The crude product was directly used in the next step. LCMS: [M+H] ⁺= 127.1

### Step 3: UBI-1282 (V2031-090)

UBI-1282d (50mg,0.45mmol), UBI-1282e (230mg,0.45mmol), and acetic acid (10mg) were added to methanol (5mL), the mixture was stirred at room temperature for 1 hour. Then sodium cyanoborohydride (56mg,0.89mmol) was added, the mixture was stirred at 20 °C for 16 hours. The reaction solution was concentrated and then isolated by silica gel column chromatography to obtain UBI -1282 (55mg, yield 40%) as a yellow solid.

LCMS: [M+H] ⁺= 618.4

### Example 1.3.14 Synthesis method of Compound UBI-1254 (P1-14h)

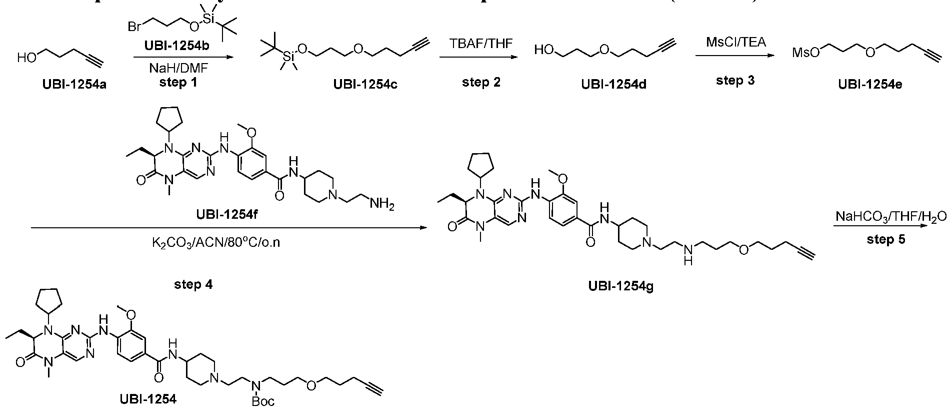

### Step 1: UBI-1254c (V2118-034)

**UBI-1254a** (1.0 g, 12 mmol) was dissolved in DMF(30 mL), NaH (60%, 576 mg, 24 mmol) was added at 0°C, the mixture was warmed up to room temperature and reacted for 1 hour. **UBI-1254b** (3 g, 12 mmol) was added to reaction solution, continued to stir at room temperature for 6 hours. After the completion of the reaction, the reaction solution was poured to ice water, extracted three times by adding EA. EA layers were combined, and washed with water twice, brine once. The mixture was dried, filtered, and purified by dry loading column chromatography (PE/EA) to obtain product UBI-1254c (1 g, 33% yield) as a colorless oil.

¹H NMR (400 MHz, CDCl₃) δ 3.64 (t, *J* = 6.2 Hz, 2H), 3.45 (td, *J* = 6.3, 1.2 Hz, 4H), 2.23 (td, *J* = 7.1, 2.7 Hz, 2H), 1.89 (t, *J=* 2.7 Hz, 1H), 1.73 (pd, *J=* 6.6, 4.8 Hz, 4H), 0.91 - 0.75 (m, 9H), 0.05-0 (m, 6H).

### Step 2: UBI-1254d (V2118-035)

**UBI-1254b** (1 g, 4 mmol) was dissolved in 30 mL THF, to which was added 10 mL of TBAF in THF (1M), the mixture was reacted at room temperature overnight (about 16 hours). After completion of the reaction, the reaction was quenched by adding saturated ammonium chloride solution, and THF was removed by rotary evaporation. The mixture was extracted with dichloromethane for three times. The organic layers were combined, dried, filtered, and purified by dry loading column chromatography (PE/EA) to obtain product BI-1254d (480 mg, 85% yield) as a colorless oil. LC-MS: [M+H] ⁺= 143

### Step 3: UBI-1254e (V2118-036)

**UBI-1254d** (110 mg, 0.77 mmol) was dissolved in DCM (10 mL), TEA (157 mg, 1.55 mmol) was added, MsCl (106 mg, 0.92 mmol) was added slowly at 0°C. After dropwise addition, the reaction was carried out at room temperature for one hour, and the completion of the reaction was monitored by TLC. To the solution was added a saturated solution of NaHCO₃ solution, the mixture was extracted with DCM (20 mL*2) twice. The organic layers were combined, dried, filtered, and purified by dry loading column chromatography (PE/EA) to obtain product **UBI-1254e** (160 mg, 94% yield) as yellow oil. LC-MS: [M+H] ⁺= 221

### Step 4: UBI-1254g (V2118-037)

**UBI-1254e** (160 mg, 0.72 mmol), **UBI-1254f** (450 mg, 0.82 mmol), and K₂CO₃ (113 mg, 0.82) were added to ACN (50 mL), and the mixture was reacted at 80°C for 16 hours. The mixture was cooled and filtered to remove solids. The filtrate was added with water and then extracted three times with DCM (50 mL*3). The organic layers were combined, dried and filtrated. The sample was purified by dry loading column chromatography (DCM/MeOH) to obtain product **UBI-1254g** (160 mg, purity 75%) as yellow solid.

LC-MS: [M+H] ⁺ =675

### Step 5: UBI-1254h (V2118-038)

**UBI-1254g** (160 mg, 0.24 mmol)was dissolved in 10mL THF, to which was added Boc₂O (110 mg, 0.5 mmol) and saturated NaHCO₃ (84 mg, 1 mmol in 10 mL H₂O) solution, the mixture was reacted at room temperature for 2 hours. The completion of the reaction was monitored by TLC, the reaction was added with water and extracted three times with DCM:MeOH=10:1 (10mL *3). The organic layers were combined, dried and filtrated. The sample was purified by dry loading column chromatography (DCM/MeOH) to obtain product **UBI-1254h** (120mg) as a yellow solid, yield 25% for 2 steps.

LC-MS: [M+H] ⁺ =775

### Example 1.3.15 Synthesis of compound UBI-1251 (P1-15h)

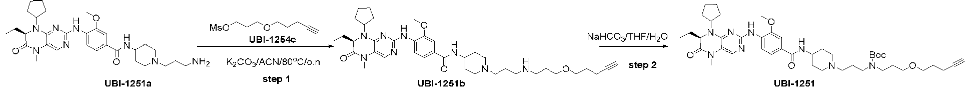

### Step 1: UBI-1251b (V2118-044)

**UBI-1254e** (250 mg, 1.14 mmol), **UBI-1251a** (670 mg, 1.19 mmol), and K₂CO₃ (164 mg, 1.19) were added to DMF (20 mL), and the mixture was reacted at 80°C for 16 hours (overnight reaction). The reaction was cooled and filtered to remove excess solids. The filtrate was added with 50 mL of water and then extracted twice with DCM (50 mL*2). The organic layers were combined, dried over Na₂SO₄, filtered, samples were stirred with silica gel and purified by column machine (DCM/MeOH). Finally, yellow solid product **UBI-1251b** (140 mg, 65% purity) was obtained, the purity was not high, and there were some raw materials and by-products, which were removed in the next purification step.

LC-MS: [M+H] ⁺=689

### Step 5: UBI-1251b(V2118-045)

**UBI-1251a** (140 mg, 0.2 mmol) was dissolved in 10mL THF, to which was added BoczO (87 mg, 0.4 mmol) and NaHCO₃ solution(84 mg, 1 mmol in 10 mL H₂O), the mixture was stirred at room temperature for 2 hours. After the completion of the reaction monitored by TLC, the reaction was extracted three times with DCM:MeOH=10:1 (10mL *3). The organic layers were combined, dried by adding Na₂SO₄ and filtrated and purified by dry loading column chromatography (DCM/MeOH) to obtain final product **UBI-1251b** (85 mg, 9.5% yield for 2 steps) as a yellow solid.

LC-MS: [M+H] ⁺=789

### Example 1.3.16 Synthesis method of Compound UBI-1252 (P1-10h)

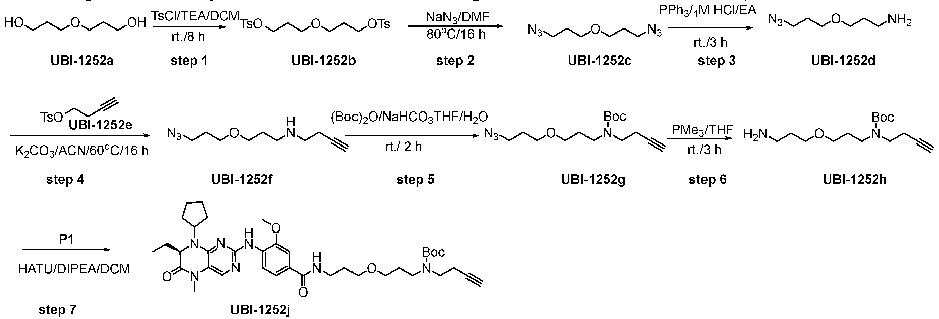

### Step 1: UBI-1252b (V2118-007)

**UBI-1252a** (20 g, 149 mmol) was dissolved in DCM (200 mL), TsCl (85 g, 447 mmol), TEA (124 mL, 894 mmol) and DMAP (1.8 g, 14.9 mmol) were added at 0 °C, then the mixture was reacted at room temperature for 8 hours. The solids were removed by filtration, and the filtrate was purified by dry loading column chromatography (PE/EA) with PE and EA as mobile phases, resulting in product **UBI-1252b** (39 g, 88.2 mmol, yield 59%) as a yellow oil.

¹H NMR (400 MHz, CDCl₃) δ 7.90 - 7.67 (m, 4H), 7.44 - 7.28 (m, 4H), 4.72 - 4.47 (m, 1H), 3.94 - 3.65 (m, 3H), 3.64 - 3.25 (m, 3H), 2.45 (d, *J=* 4.3 Hz, 6H), 1.08 - 0.92 (m, 3H).

LCMS [M+H]⁺ = 443

### Step 2: UBI-1252c (V2118-008)

**UBI-1252b** (39 g, 88 mmol) was dissolved in 200 mL DMF, the mixture was gently stirred, and NaN₃ (17 g, 264 mmol) was carefully added to the solution and reacted at 80°C overnight (16 h). Product disappeared monitored by TLC(PE/EA = 10/1, 1/1), new spots were generated. The excess NaN3 was removed by filtration, then 200 mL of water was added and extracted twice with EA (200 mL*2). EA layers were combined, and washed with 200mL water twice, 100mL saturated saline once, then dried over Na₂SO₄, filtrated, dried by rotary dryer to obtain product **UBI-1252c** (15 g, yield 94%) as a yellow oil.

LCMS [M+H]⁺ = 185

### Step 3: UBI-1252d (V2118-009)

At 0°C, UBI-1252c (15 g, 82 mmol) and 1M HCl solution (123 mL, 123 mmol) were added to Et₂O (100 mL)/EtOAc (200 mL)/THF (50 mL) and stirred. PPh₃ (21.5 g, 82 mmol) was dissolved in EtOAc (150 mL)/Et₂O (50 mL) and was slowly added dropwise to the above mixture over one hour. The mixture was reacted at room temperature for 3 hours. The aqueous layer was washed twice with EA (100 mL) and the EA was discarded. 2M NaOH solution was added dropwise to the aqueous layer until the pH reached about 10, and then the mixture was extracted with DCM (200mL*2) twice. DCM layers were combined and dried by adding sodium sulfate, filtrated, dried by rotary dryer to obtain crude product **UBI-1252d** (12 g, yield 92%) as a yellow oil.

LCMS [M+H]⁺ = 159

### Step 4: UBI-1252f (V2118-010)

**UBI-1252d** (1 g, 6.3 mmol), **UBI-1252e** (1.4 g, 6.3 mmol) and K₂CO₃ (2.6 g, 19 mmol) were added into 150 mL ACN, the mixture was reacted at 60°C overnight (about 16 hours). The reaction was cooled and filtered to remove solids, and the filtrate was dried by rotary dryer and purified by passing through the column (DCM/MeOH = 0 to 10 %) to obtain product **UBI-1252f** (820 mg, yield 62%) as a yellow oil.

LCMS [M+H]⁺ = 211

### Step 5: UBI-1252g (V2118-011)

**UBI-1252f** (820 mg, 3.9 mmol) was dissolved in 10 mL THF, NaHCO₃ solution (983 mg, 11.7 mmol) dissolved in 10 mL H₂O) and BoczO (1.27 g, 5.85 mmol) were added, the mixture was reacted at 20°C for 2 hours. After the completion of the reaction, EA (20 mL*2) was added for extraction twice. The organic layers were combined, dried and filtrated, and purified by column chromatography (DCM/MeOH= 0 to 10 %) to obtain product **UBI-1252g** (850 mg, yield 70%) as a yellow oil.

¹H NMR (400 MHz, CDCl₃) δ 3.77 - 3.00 (m, 8H), 2.43 (s, 2H), 1.96 (s, 1H), 1.52 - 1.38 (m, 9H), 1.24 - 1.06 (m, 6H).

LCMS [M+H]⁺ = 311

### Step 6: UBI-1252h (V2118-054)

**UBI-1252g** (200 mg, 0.64 mmol) was dissolved in 10 mL THF, PMe₃ (98 mg, 1.28 mmol) was added, the mixture was reacted at room temperature for 3 hours. The completion of the reaction was monitored by TLC. The reaction was dried by rotary dryer to obtain product UBI-1252h (272 mg), which was directly used in the next step.

LCMS [M+H]⁺ = 285

### Step 7: UBI-1252j (V2118-055)

**UBI-1252h** (272 mg, 0.64 mmol), and **UBI-1252i** (183, 0.64 mmol) were dissolved in 20mL DCM, to which was added HATU (243 mg, 0.64 mmol) and DIPEA (248 mg, 1.92 mmol), the mixture was reacted at room temperature for one hour. The completion of the reaction was monitored by TLC. The sample was dried by rotary dryer and purified dry loading column chromatography (DCM/MeOH= 10:1) to obtain product UBI-1252j 105 mg as a yellow solid, yield 23.5% for 2 steps.

LCMS [M+H]⁺ = 692.

### Example 1.3.17 Synthesis method of Compound UBI-1253 (P1-11h)

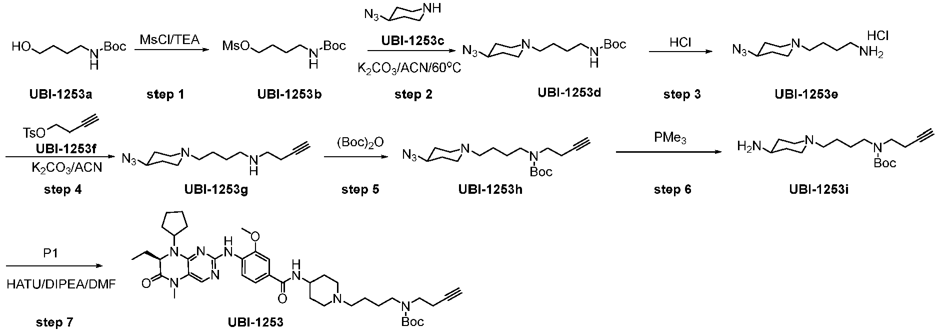

### Step 1: UBI-1253a (V2118-029)

**UBI-1253a** (1 g, 5.3 mmol) was dissolved in DCM (50 mL), TEA (1.6 g, 15.9 mmol) was added, then the mixture was cooled to 0°C, and MsCl (736 mg, 6.4 mmol) was slowly added dropwise. After dropwise addition, the reaction was carried out at room temperature for one hour. The reaction was quenched with NaHCO₃ saturated solution and extracted twice with DCM (50 mL*2). The organic layers were combined, dried, filtered, and purified by dry loading column chromatography (PE/EA) to obtain product **UBI-1253b** (1.2 g, 86% yield) as a yellow oil.

LC-MS: [M+H] ⁺= 268

### Step 2: UBI-1253d (V2118-030)

**UBI-1253b** (1.2 g, 4.5 mmol), **UBI-1253c(566** mg, 4.5 mmol), and K₂CO₃ (1.2 g, 9 mmol) were added to ACN (50 mL), and the mixture was reacted at 60°C for 16 hours. The reaction was cooled and filtrated, the filtrate was dried by rotary dryer to remove acetonitrile, then 50 mL of water was added, and the mixture was extracted twice with DCM. The organic layers were combined, dried and filtrated, and purified by dry loading column chromatography (DCM/MeOH) to obtain product **UBI-1253d** (1.2 g, yield 89%) as a yellow oil.

¹H NMR (400 MHz, CDCl₃) δ 5.39 (s, 1H), 3.37 (dd, *J* = 47.0, 13.8 Hz, 2H), 3.20 - 3.03 (m, 2H), 2.83 - 2.63 (m, 2H), 2.34 (t, *J=* 6.8 Hz, 2H), 2.15 (s, 2H), 1.99 - 1.76 (m, 3H), 1.76 - 1.60 (m, 2H), 1.59 - 1.48 (m, 3H), 1.45 (d, *J=* 8.0 Hz, 9H).

LC-MS: [M+H] ⁺=298

### Step 3: UBI-1253e (V2118-031)

**UBI-1253d** (1.2 g, 4 mmol) was dissolved in DCM (20 mL), to which was added 4M HCl in dioxane (2 mL), the mixture was reacted at room temperature for one hour. The mixture was stood, and supernatant was poured off. The solids were washed twice with Et₂O, and Et₂O was poured off. The remaining solids was dried by an oil pump to give **product UBI-1253e** (HCl salt) (900 mg, yield 96 %) as yellow solid.

LCMS [M+H]⁺ = 198

### Step 4: UBI-1253g (V2118-032)

To 100mL ACN was added **UBI-1253e** (900 mg, 4.7 mmol), **UBI-1253f** (1 g, 4.7 mmol) and K₂CO₃(1.3 g, 9.4 mmol), and the mixture was reacted at 60°C overnight (about 16 hours). The reaction was cooled and filtrated, the filtrate was dried by rotary dryer, and purified by dry loading column chromatography (DCM:MeOH = 0 to 10 %) to obtain product **UBI-1253g** ( 530 mg, purity 75%) as a yellow oil. The purity was not high, and there were some raw materials and by-products, which were removed in the next purification step.

LCMS [M+H]⁺ = 250

### Step 5: UBI-1253h (V2118-033)

**UBI-1253g** (530 mg, 2 mmol) was dissolved in 30 mL THF, NaHCO₃ solution (504 mg, 6mmol in 10 mL H₂O) and BoczO (872 mg, 4 mmol) were added, the mixture was reacted at room temperature for 2 hours. The mixture was added with water and extracted twice with DCM (50 mL*2). The organic layers were combined, dried and filtrated. The sample was dried by rotary dryer and purified by dry loading column chromatography (DCM/MeOH) to obtain product **UBI-1253h** (510 mg) as a yellow solid, yield 36% for 2 steps.

LCMS [M+H]⁺ = 350

### Step 6: UBI-1253i (V2118-042)

**UBI-1253h** (510 mg, 1.46 mmol) were dissolved in 20 mL THF, and PMe₃ (222 mg, 2.9 mmol) was added, the mixture was reacted at room temperature for 3 hours, dried by rotary dryer to obtain product **UBI-1253i** (324 mg, yield: 69%), which was directly used in the next reaction.

LCMS [M+H]⁺ = 324

### Step 7: UBI-1253k (V2118-043)

**UBI-1253i** (324 mg, 1 mmol), **UBI-1253j** (292 mg, 1 mmol), and HATU (456 mg, 1.2 mmol) were successively added to DMF (15 mL), then DIPEA (387 mg, 3 mmol) was added, and the mixture was reacted at room temperature for 2 hours. The mixture was dried by rotary dryer and directly purified by dry loading column chromatography (DCM/MeOH) to obtain **product UBI-1253k** (70 mg, yield 9.6 %) as a white solid.

LCMS [M+H]⁺ = 731

### Example 1.3.18 Synthesis method of Compound UBI-1277 (NH₂-12h-A1)

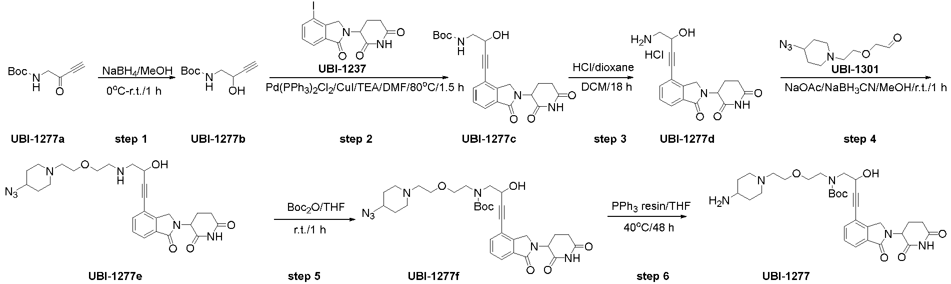

### Step 1: UBI-1277b (V1782-093)

**UBI-1277a** (600 mg, 3.27 mmol) were dissolved in methanol (12 mL), NaBH₄ (247 mg, 6.55 mmol) was added in the ice bath. The reaction was slowly warmed up to room temperature and reacted for 2 hours. The reaction was quenched with saturated NH₄Cl (30 mL), then extracted with ethyl acetate(20 mL*3). The organic phases were combined, dried over anhydrous sodium sulfate, filtered and dried by rotary dryer to obtain product (580 mg, yield 100%) as a yellow oil. LCMS [M-100]⁺ =86.3

### Step 2: UBI-1277c (V1782-096)

**UBI-1277b** (580 mg, 2.7 mmol), **UBI-1237** (500 mg, 1.35 mmol), Pd(PPh₃)₂Cl₂ (95 mg, 0.135 mmol), CuI (51 mg, 0.27 mmol), and TEA (136 mg, 1.35 mmol) were dissolved in anhydrous DMF (12 mL), the mixture was heated to 80°C and reacted for 1.5 hours under N₂ protection. The reaction was cooled to room temperature and dried by rotary dryer, the crude product was passed through column (petroleum ether/ethyl acetate= 0% to 100%) to obtain product **UBI-1277c** (500 mg) as a brown oil. LCMS [M+H]⁺ = 428.2

### Step 3: UBI-1277d (V1782-121)

**UBI-1277c** (400 mg, 0.94 mmol) was dissolved in DCM (5 mL), 4M HCl/dioxane (5 mL) was added, the mixture was reacted at room temperature overnight. Crude product was obtained by drying by rotary dryer to remove solvent and was slurried by adding ether. Solid was filtered and dried to obtain product **UBI-1277d** (340 mg) as a brown solid. LCMS [M+H]⁺ = 328.4

### Step 4: UBI-1277e (V1782-127)

**UBI-1277d** (300 mg, 0.82 mmol) was dissolved in methanol (20 mL), **UBI-1301** (600 mg, 2.47 mmol) and NaOAc (130 mg, 1.64 mmol) were added, the mixture was reacted for 1 hour. Then NaBH₃CN (77 mg, 1.23 mmol) was added and continued to react for another 1 hour. The reaction was quenched with saturated brine, extracted with dichloromethane. The organic phase was dried, then filtered, and the filtrate was used directly in the next step. LCMS [M+H]⁺ = 524.2.

### Step 5: UBI-1277f (V1782-130)

**UBI-1277e** (500 mg, crude) was dissolved in THF (5 mL), BoczO (1 mL) was added. The mixture was reacted at room temperature for 1 hour. The reaction solution was dried by rotary dryer and passed through the column (dichloromethane/methanol= 0% to 10%) to obtain **product UBI-1277f** (100 mg) as a colorless oil. LCMS: [MS+H]⁺=624.3

### Step 6: UBI-1277 (V1782-131)

**UBI-1277f** (100 mg, 0.16 mmol) was dissolved in THF (5 mL), triphenylphosphine resin (200 mg) was added, and the mixture was reacted at 40°C for 48 hours. The reaction was filtrated and dried by rotary dryer to obtain product **UBI-1277f** (40 mg, yield 42%) as a yellow solid. LCMS [M+H]⁺ =598.4.

### Example 1.3.19 Synthesis method of Compund UBI-1290 (P1-10h)

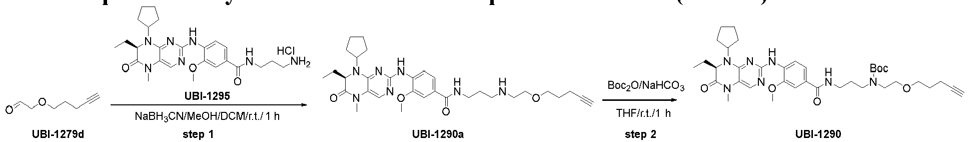

### Step 1: UBI-1290a (V2111-014)

**UBI-1279d** (200 mg, 0.38 mmol) was dissolved in methanol (3 mL), **UBI-1295** (72 mg, 0.57 mmol) was added in ice bath, the mixture was reacted at room temperature for 1 hour. Then NaBH₃CN (48 mg, 0.76 mmol) was added, the mixture was reacted for 10 minutes. Water (5 mL) was added, the mixture was extracted with dichloromethane (15 mL), the organic phases were combined, dried over anhydrous sodium sulfate, and the filtrate was used directly in the next reaction.

### Step 2: UBI-1290 (V2111-015)

**UBI-1290a** was dissolved in THF (3 mL), BoczO (0.5 mL) and saturated NaHCO₃ (0.5 mL) were added, the mixture was reacted at room temperature for 1 hour. Water (5 mL) was added and the mixture was extracted with DCM (15 mL). The organic phases were combined, dried over anhydrous sodium sulfate, and filtered. The crude product was subjected to column chromatography (dichloromethane/methanol = 0% to 10%) to obtain **UBI-1290** (120 mg) as a yellow oil. LCMS [M+H]⁺ =692.3

### Synthesis method of Compound UBI-1291 (P1-2h)

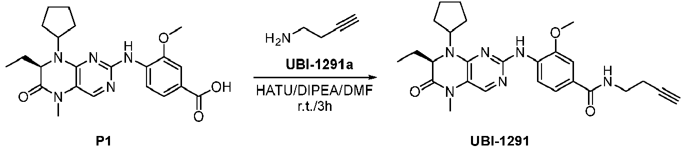

### Step 1: UBI-1291 (V2111-049)

**P1** (37 mg, 0.35 mmol), **UBI-1291a** (100 mg, 0.23 mmol), HATU (175 mg, 0.46 mmol), and DIPEA (89 mg, 0.69 mmol) were dissolved in DMF (3 mL), the mixture was reacted at room temperature for 3 hours. The reaction solution was filtered, dried by rotary dryer and passed through the column (dichloromethane/methanol = 0% to 10%) to obtain **UBI-1291** (130 mg, yield 100%) as yellow solid. LCMS [M+H]⁺ =477.4

¹H NMR (400 MHz, DMSO-*d*₆) δ 8.52 (t, *J=* 5.8 Hz, 1H), 8.43 (d, *J=* 8.3 Hz, 1H), 7.85 (s, 1H), 7.62 (s, 1H), 7.48 (d, *J* = 8.0 Hz, 2H), 4.42 - 4.31 (m, 1H), 4.24 (m, 1H), 3.94 (s, 3H), 3.62 (m, 2H), 3.41 - 3.35 (m, 2H), 3.25 (s, 3H), 3.14 (m, 2H), 2.85 (m, 1H), 2.42 (m, 2H), 2.04 (m, 1H), 1.89 (m, 2H), 1.84 - 1.73 (m, 4H), 1.65 - 1.59 (m, 2H), 0.76 (t, *J=* 7.5 Hz, 3H).

### Synthesis method of Compound UBI-1292 (NH₂-13h-A1)

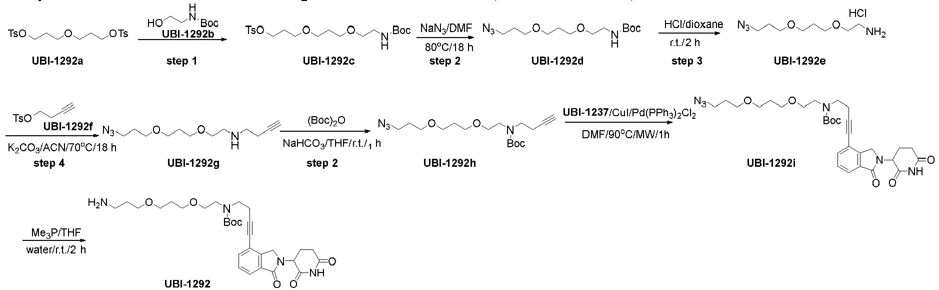

### Step 1: UBI-1292c (V2111-035)

**UBI-1292b** (2.7 g, 16.7 mmol), **UBI-1292a** (37 g, 83.6 mmol), Bu₄NHSO₄ (12.4 g, 36.6 mmol) and 60% KOH (30 mL) were reacted at 40°C overnight. Water was added and the mixture was extracted with DCM (50 mL*3), the organic phase was dried by rotary dryer, and the crude was passed through column (petroleum ether/ethyl acetate= 0% to 100%) to obtain product **UBI-1292c** (2 g, yield 27%) as a colorless oil. LCMS [M-100]⁺ = 332.3

### Step 2: UBI-1292d (V2111-034)

**UBI-1292c** (700 mg, 1.6 mmol) was dissolved in anhydrous DMF (10 mL), NaN₃ (320 mg, 4.8 mmol) was added, the mixture was reacted at 80°C for 5 hours. Water (10 mL) was added and the mixture was extracted with ethyl acetate (20 mL*3), the organic phase was dried by rotary dryer, and passed through column (petroleum ether/ethyl acetate= 0% to 100%) to obtain product **UBI-1292d** (430 mg, yield 89%) as a colorless oil. LCMS [M-100]⁺ = 203.2

### Step 3: UBI-1292e (V2111-036)

**UBI-1292d** (430 mg, 1.42 mmol) was dissolved in dichloromethane (3 mL), HCl/dioxane (7 mL, 28 mmol) was added, the mixture was reacted at room temperature for 2 hours. The reaction solution was concentrated to obtain product **UBI-1292e** (300 mg) as a yellow oil.

LCMS [M+H]⁺ =203.3

### Step 4: UBI-1292g (V2111-037)

**UBI-1292e** (340 mg, 1.42 mmol) was dissolved in acetonitrile (7 mL), **UBI-1292f** (318 mg, 1.42 mmol), and K₂CO₃ (390 mg, 2.84 mmol) were added, the mixture was reacted at 70°C overnight. The reaction was cooled down and filtered, and the filtrate was used directly in the next step. LCMS [M+H]⁺ =255.4

### Step 5: UBI-1292h (V2111-039)

**UBI-1292g** was dissolved in THF (6 mL), BoczO (0.5 mL) and NaHCO₃ (300 mg) were added, the mixture was reacted at room temperature for 1 hour. Water (5 mL) was added and the mixture was extracted with dichloromethane (15 mL), the organic phase was concentrated to dryness, filtered, dried by rotary dryer, and passed through column (petroleum ether/ethyl acetate= 0% to 100%) to obtain product **UBI-1292h** (200 mg) as a yellow oil. LCMS [M-100]⁺ = 255.4

### Step 6: UBI-1292i (V2111-045)

**UBI-1292h** (130 mg, 0.36 mmol), **UBI-1237** (120 mg, 0.32 mmol), Pd(PPh₃)₂Cl₂ (60 mg, 0.085 mmol), CuI (40 mg, 0.21 mmol), and TEA (3 drop) were dissolved in anhydrous DMF (5 mL), the mixture was reacted at 40°C for 1 hour under the protection of N₂. The reaction was dried by rotary dryer, passed through the column (dichloromethane/methanol = 0% to 10%) to obtain product **UBI-1292j** (200 mg mixture) as a yellow oil.

### Step 7: UBI-1292 (V2111-047)

**UBI-1292i** (15 mg crude) was dissolved in THF (2 mL), 1M Me₃P (1 mL) was added, the mixture was reacted at room temperature for 1 hour. Water (0.5 mL) was added, the mixture was reacted for another 1 hour. The reaction solution was concentrated and then passed through reversed-phase chromatography (acetonitrile/water = 0% to 100%) to obtain a red solid **(V2111-047,** 20 mg). LCMS [M+H]⁺ =571.5

¹H NMR (400 MHz, DMSO-*d*₆) δ 7.72 (d, *J=* 7.6 Hz, 1H), 7.63 (d, *J=* 7.5 Hz, 1H), 7.53 (t, *J =* 7.5 Hz, 1H), 5.16 (m, 1H), 4.45 (m, 1H), 4.30 (m, 1H), 3.62 (m, 1H), 3.56 - 3.37 (m, 10H), 2.99 - 2.90 (m, 1H), 2.69 (m, 2H), 2.60 (m, 2H), 2.44 (m, 2H), 2.02 (m, 1H), 1.90 (m, 2H), 1.43 - 1.35 (m, 9H), 1.09 - 0.96 (m, 6H).

### Example 1.3.20 Synthesis method of Compound UBI-1293 (P1-13h):

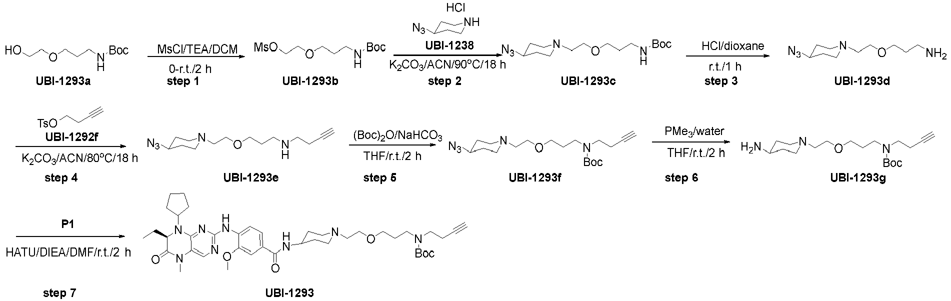

### Step 1: UBI-1293b (V2111-063)

**UBI-1293a** (1.5 g, 6.83 mmol), and TEA (1.5 mL, 10.8 mmol) were dissolved in anhydrous dichloromethane (15 mL), MsCl (1.5 mL, 18.9 mmol) was added in ice bath, the mixture was reacted at room temperature for 2 hours. Water (30 mL) was added and the mixture was extracted with dichloromethane (20 mL*3). The organic phases were combined, dried over anhydrous sodium sulfate, and filtered, and the filtrate was dried by rotary dryer to obtain product **UBI-1293b** (1.78 g, yield 87%) as yellow oil, which was directly used in the next reaction. LCMS [M-100]⁺ = 198.2

### Step 2: UBI-1293c (V2111-063)

**UBI-1293b** (1.78 g, 6 mmol) was dissolved in acetonitrile (50 mL), **UBI-1238** (1.07 g, 6.6 mmol), and K₂CO₃ (1.65 g, 12 mmol) were added, the mixture was reacted at 80°C overnight. The reaction solution was filtered, concentrated and passed through the column (acetonitrile/water = 0% to 30%) to obtain product **UBI-1293c** (400 mg, yield 20%) as a yellow oil. LCMS [M+H]⁺ =328

### Step 3: UBI-1293d (V2111-064)

**UBI-1293c** (0.4 g, 1.2 mmol) was added with 4M HCl/dioxane (4 mL, 20 mmol), and the mixture was reacted at room temperature for 2 hours. The reaction solution was concentrated to obtain product **UBI-1293d** (0.3 g, yield 100%) as a yellow oil. LCMS [M+H]⁺ =228

### Step 4: UBI-1293e (V2111-069)

**UBI-1293d** (724 mg, 2.75 mmol) was dissolved in acetonitrile (70 mL), **UBI-1292f** (616 mg, 2.75 mmol), K₂CO₃ (760 mg, 5.5 mmol) and KI (228 mg, 1.37 mmol) were added, and the mixture was reacted at 80°C overnight. The reaction solution was cooled down and filtered, and the filtrate was used directly in the next reaction. LCMS [M+H]⁺ =280.2

### Step 5: UBI-1293f (V2111-070)

**UBI-1293e** was dissolved in THF (6 mL), BoczO (2 mL, 8.25 mmol) and NaHCO₃ (700 mg, 8.25 mmol) were added, and the mixture was reacted at room temperature for 1 hour. Water (5 mL) was added and the mixture was extracted with dichloromethane (15 mL). The organic phases were combined, dried over anhydrous sodium sulfate, and filtered, and the filtrate was dried by rotary dryer and passed through column (dichloromethane/methanol = 0% to 100%) to obtain product **UBI-1293f** (350 mg mixture) as a yellow oil. LCMS [M+H]⁺ =380.2

### Step 6: UBI-1293g (V2111-073)

**UBI-1293f** (350 mg, 0.46 mmol) was dissolved in THF (2 mL), 1M Me₃P (3 mL, 3 mmol) was added, and the mixture was reacted at 50°C for 1 hour. Water (0.5 mL) was added, and the mixture was reacted at 50 °C for 1 hour. The reaction was concentrated and directly used in the next reaction. LCMS [M+H]⁺ =354.3

### Step 7: UBI-1293 (V2111-072)

**UBI-1293g** (60 mg, 0.17 mmol), **P1** (50 mg, 0.11 mmol), HATU (130 mg, 0.34 mmol), and DIPEA (66 mg, 0.51 mmol) were dissolved in DMF (1 mL), the mixture was reacted at room temperature for 3 hours. The mixture was added with water, then extracted with ethyl acetate (10 mL×2). The organic phases were combined, dried over anhydrous sodium sulfate, and filtered, and the filtrate was dried by rotary dryer and purified by preparative thin layer chromatography (dichloromethane/methanol = 0% to 10%) to obtain product **UBI-1293** (25 mg, yield 20%) as a white solid. LCMS [M+H]⁺ =761.5

¹H NMR (400 MHz, DMSO-*d*₆) δ 8.43 (d, *J* = 8.3 Hz, 1H), 8.30 (s, 1H), 7.85 (s, 1H), 7.62 (s, 1H), 7.49 (d, *J* = 11.2 Hz, 2H), 4.35 (m, 1H), 4.24 (m, 1H), 4.05 (d, *J* = 7.1 Hz, 1H), 3.94 (s, 3H), 3.71 (m, 2H), 3.55 (m, 2H), 3.44 (m, 3H), 3.29 (m, 2H), 3.27 (m, 2H), 3.25 (s, 3H), 3.17 - 3.07 (m, 2H), 2.85 (m, 1H), 2.37 (m, 2H), 2.02 (m, 3H), 1.93 - 1.71 (m, 10H), 1.65 (m, 4H), 1.40 (s, 9H), 0.77 (t, *J=* 7.4 Hz, 3H).

### Example 1.3.21 Synthesis method of Compound UBI-1294 (P1-14h):

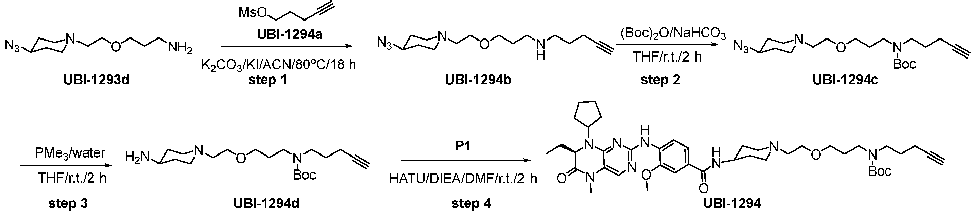

### Step 1: UBI-1294b (V2111-077)

**UBI-1293d** (443 mg, 1.68 mmol) was dissolved in acetonitrile (70 mL), **UBI-1294a** (272 mg, 1.68 mmol), and K₂CO₃ (464 mg, 3.36 mmol) were added, and the mixture was reacted at 80°C overnight. The reaction solution was cooled down, then filtered, and the filtrate was used directly in the next reaction. LCMS [M+H]⁺ =294.3

### Step 2: UBI-1294c (V2111-079)

**UBI-1294b** was dissolved in THF (6 mL), BoczO (2 mL, 9.17 mmol) and saturated NaHCO₃ (2 mL), the mixture was reacted at room temperature overnight. Water (5 mL) was added and the mixture was extracted with dichloromethane (15 mL). The organic phases were combined, dried over anhydrous sodium sulfate, and filtered, and the filtrate was dried by rotary dryer and purified by passing through column (dichloromethane/methanol = 0% to 100%) to obtain product **UBI-1294c** (400 mg mixture) as a yellow oil. LCMS [M+H]⁺ =394.2

### Step 3: UBI-1294e (V2111-081)

**UBI-1294d** (400 mg, 1 mmol) was dissolved in THF (2 mL), 1M Me₃P (1.5 mL, 1.5 mmol) was added, and the mixture was reacted at 50°C for 1 hour. Water (0.5 mL) was added, the mixture was reacted at 50 °C for 1 hour. The solvent was concentrated and dried by rotary dryer, and directly used in the next step. LCMS [M+H]⁺ =368

### Step 4: UBI-1294 (V2111-083)

**UBI-1294d** (360 mg, 1 mmol), **P1** (300 mg, 0.7 mmol), HATU (760 mg, 2 mmol), and DIPEA (390 mg, 3 mmol) were dissolved in DMF (5 mL), the mixture was reacted at room temperature overnight. Water was added and the mixture was extracted with ethyl acetate (10 mL*2), and the organic phases were combined, concentrated, and subjected to column (dichloromethane/methanol= 0% to 10%) to obtain product **UBI-1294** (230 mg, yield 30%) as a white solid. LCMS [M+H]⁺ =775.3

¹H NMR (400 MHz, DMSO-*d*₆) δ 8.42 (d, *J =* 8.2 Hz, 1H), 8.29 (d, *J =* 7.3 Hz, 1H), 7.84 (s, 1H), 7.63 (m, 1H), 7.53 - 7.45 (m, 2H), 4.42 - 4.32 (m, 1H), 4.31 - 4.23 (m, 1H), 3.95 (m, 4H), 3.70 (m, 3H), 3.55 (m, 2H), 3.45 (m, 3H), 3.25 (s, 3H), 3.20 (m, 5H), 2.81 (m, 1H), 2.16 - 2.12 (m, 2H), 2.10 - 1.98 (m, 4H), 1.88 (m, 2H), 1.76 (m, 8H), 1.63 (m, 5H), 1.40 (d, *J =* 1.6 Hz, 9H), 0.77 (t, *J* = 7.4 Hz, 3H).

### Example 1.3.22 Synthesis method of Compound UBI-1260 (P1-11h):

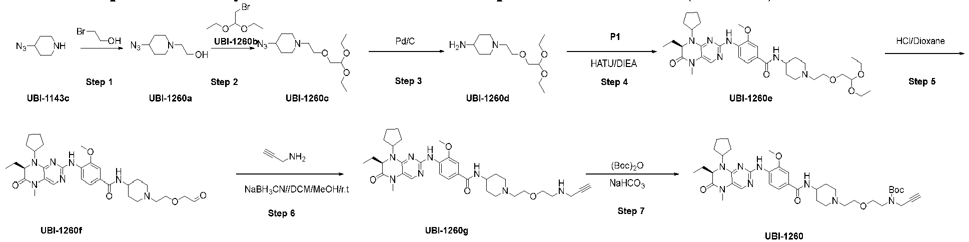

### Step 1: UBI-1260a (V1685-067)

**UBI-1143c** (40g, 247mmol) and K₂CO₃ (68.2g, 494mmol) were dissolved in acetonitrile, bromoethanol (21g, 494mmol) was added, and the mixture was reacted at 80 °C for16 hours. The reaction was filtered after completion, and the filtrate was concentrated by rotary evaporation under reduced pressure to obtain crude product. And the mixture was isolated by silica gel column chromatography (dichloromethane/methanol = 50/1) to obtain target product **UBI-1260a** (23g, yield 27.3%) as a colorless transparent oil.

¹H NMR (400 MHz, chloroform-d) δ 3.65 - 3.56 (m, 2H), 3.49 - 3.40 (m, 1H), 2.84 - 2.78 (m, 2H), 2.53 (t, 2H), 2.32 - 2.21 (m, 2H), 1.98 - 1.88 (m, 2H), 1.74 - 1.62 (m, 2H)

### Step 2: UBI-1260c (V1685-068)

**UBI-1260a** (15g, 88mmol) was dissolved in THF (300mL) and cooled to 0°C, NaH (4.2g, 105.6mmol) was added. The mixture was reacted at 50°C for 1 hour. Then **UBI-1260b** (22.5g, 114mmol) was added, the mixture was reacted at 70°C for 16 hours. HCl (2M) was added until pH=8 as soon as the reaction solution cooled, and the mixture was concentrated by rotary evaporation under reduced pressure to obtain crude product, and it was isolated by silica gel column chromatography (dichloromethane/methanol = 20/1) to obtain target product **UBI-1260c** (11g, yield 44%) as a colorless transparent oil.

¹H NMR (400 MHz, chloroform-d) δ 4.63 (t, *J =* 5.3 Hz, 1H), 3.79 - 3.52 (m, 6H), 3.50 (d, *J =* 5.3 Hz, 2H), 3.39 (q, *J =* 5.1, 4.5 Hz, 1H), 2.82 (dd, *J =* 11.2, 5.6 Hz, 2H), 2.58 (t, *J =* 5.8 Hz, 2H), 2.23 (t, *J=* 10.0 Hz, 2H), 1.97 - 1.85 (m, 2H), 1.68 (dtd, *J=* 13.2, 9.6, 3.7 Hz, 2H), 1.22 (t, *J=* 7.1 Hz, 6H).

### Step 3: UBI-1260d (V1685-082)

**UBI-1260c** (1g, 3.5mmol) was dissolved in methanol(20mL), the mixture was reacted at room temperature for 16 hours under N2 protection. After the reaction was completed, it was filtered and the filtrate was concentrated by rotary evaporation under reduced pressure to obtain crude product which was directly used in the next step. LC-MS: [M+H] ⁺= 261.2

### Step 4: UBI-1260d (V1685-094)

**UBI-1260d** (1g, 3.8mmol), P1 (1.6g, 3.8mmol), HATU (2.9g, 7.7mmol) were dissolved in DIPEA (1.4mL, 7.7mmol) and DMF (10mL), the mixture was reacted at room temperature for 2 hours. After the completion of the reaction, the reaction solution was separated by reversed-phase column (water/methanol =5%/95% 45 minutes) to obtain **UBI-1260d** (1.5g, yield 60%) as a white solid. LC-MS: [M+H] ⁺=: 668.2

### Step 5: UBI-1260f (V1685-095)

**UBI-1260d** (1.5g, 2.25mmol) was dissolved in hydrochloric acid (3mL) and water (12mL), the mixture was reacted at room temperature for 16 hours. After the completion of the reaction, saturated NaHCO₃ aqueous solution was added to adjust the pH=7. The mixture was extracted with dichloromethane(5mL*3). The organic phase was dried over Na₂SO₄ and concentrated to obtain crude product **UBI-1260f** (1g, yield 76.9%) as a light yellow oil. The mixture was directly used in the next step. LC-MS: [M+H] ⁺=: 594.2

### Step 6: UBI-1260g (V1685-096)

Propargylamine (24mg, 0.26mmol) was dissolved in MeOH (10mL), **UBI-1260f** (300mg, 0.51) and a drop of acetic acid were added. The reaction was reacted at room temperature for 1 hour. Then NaBH₃CN (36mg, 0.51mmol) was added, the mixture was reacted at room temperature for 1 hour. The reaction solution was quenched with saturated NaHCO₃ aqueous solution, concentrated to obtain crude product. The crude product was dissolved in THF, filtered, and the filtrate was used directly in the next step. LC-MS: [M+H] ⁺= 633.2.

### Step 7: UBI-1260 (V1685-098)

**UBI-1260g** (200mg, 0.31mmol), (Boc)2O (200mg, 0.93mmol), and NaHCO3 (78mg, 0.93mmol) were dissolved in THF, the mixture was reacted at room temperature for 1 hour. After concentration, the reaction solution was isolated by Pre-TLC(dichloromethane /methanol =10/1) to obtain **UBI-1260** (40 mg, yield 17.6%) as a white solid. LC-MS: [M+H] ⁺= 733.4

### Example 1.3.23 Synthesis method of Compound UBI-1261 (P1-12h):

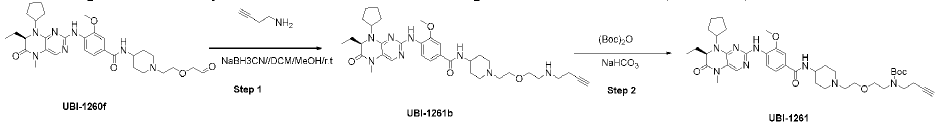

### Step 1: UBI-1261b (V1685-097)

But-3-yn-1-amine (34mg, 0.26mmol) was dissolved in methanol (10mL), **UBI-1260f** (300mg, 0.51) and a drop of acetic acid were added. The reaction was reacted at room temperature for 1 hour. Then NaBH₃CN (36mg, 0.51mmol) was added, the mixture was reacted at room temperature for 1 hour. The reaction solution was quenched with saturated NaHCO₃ aqueous solution, concentrated to obtain crude product. The crude product was dissolved in THF, filtered, and the filtrate was used directly in the next step. LC-MS: [M+H] ⁺= 647.2

### Step 2: UBI-1261 (V1685-099)

**UBI-1261b** (200mg, 0.31mmol), (Boc)2O (200mg, 0.93mmol), and NaHCO3 (78mg, 0.93mmol) were dissolved in THF, the mixture was reacted at room temperature for 1 hour. After concentration, the reaction solution was isolated by Pre-TLC(dichloromethane /methanol =10/1) to obtain **UBI-1260** (40 mg, yield 17.6%) as a white solid. LC-MS: [M+H] ⁺= 747.4

### Example 1.3.24 Synthesis method of Compound UBI-1262 (P1-9h):

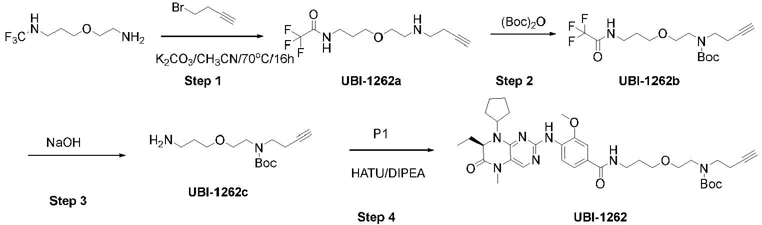

### Step 1: UBI-1262a (V1685-111)

**UBI-1285g** (1.38g, 6.45mmol) and K₂CO₃ (890mg, 6.45mmol) were dissolved in acetonitrile(100mL), 4-bromobutyne (681mg, 5.16mmol) was added. The mixture was reacted at 70°C for 16 hours, filtered after the completion of the reaction, the filtrate was concentrated by rotary evaporation under reduced pressure to obtain crude product. And the mixture was isolated by silica gel column chromatography (dichloromethane/methanol = 20/1) to obtain target product **UBI-1262a** (370mg, yield 28.4%) as a yellow transparent oil. LC-MS: [M+H] ⁺= 267.2.

### Step 2: UBI-1262b (V1685-112)

**UBI-1262a** (370mg, 1.4mmol), (Boc)₂O (606mg, 2.8mmol), NaHCO3 (176mg, 2.1mmol) were dissolved in THF(10mL), the mixture was reacted at room temperature for 1 hour. The reaction solution was extracted with ethyl acetate (10 mL*3). The organic phase was dried over Na₂SO₄.

The mixture was concentrated to obtain crude product, and isolated by silica gel column chromatography (dichloromethane/methanol = 20/1 to 100%) to obtain target product **UBI-1262b** (337 mg, yield 65.8%) as white solid.

¹H NMR (400 MHz, chloroform-*d*) δ 3.67 - 3.29 (m, 10H), 2.43 (dt, *J=* 8.5, 4.1 Hz, 2H), 1.98 (t, *J=* 2.7 Hz, 1H), 1.84 (s, 2H), 1.46 (s, 9H).

### Step 3: UBI-1262c (V1685-113)

**UBI-1262b** (337mg, 0.92mmol) **was dissolved** in H₂O (1mL) and THF (10mL), NaOH (73mg, 1.8mmol) was added. The reaction was reacted at room temperature for 16 hours. The reaction liquid was concentrated to obtain crude product, which was directly used in the next step. LC-MS: [M+H] ⁺= 267.2.

### Step 4: UBI-1262 (V1685-114)

**UBI-1262c** (400mg, 1.48mmol), P1 (630mg, 1.48mmol), and HATU (1.1g, 2.96mmol) were dissolved in DIPEA (545ul, 2.96mmol) and DMF (10mL). The reaction was reacted at room temperature for 2 hours. After the completion of the reaction, the reaction solution was separated by reversed-phase column (water/methanol =5%/95% 45 minutes) to obtain **UBI-1262** (100mg, yield 10%) as a white solid.

1H NMR (400 MHz, DMSO-d6) δ 8.41 (d, J = 8.4 Hz, 1H), 8.36 - 8.32 (m, 1H), 7.84 (s, 1H), 7.60 (s, 1H), 7.48 (d, J = 7.8 Hz, 2H), 4.45 - 4.29 (m, 1H), 4.24 (dd, J = 7.6, 3.6 Hz, 1H), 3.94 (s, 3H), 3.45 (t, J = 5.8 Hz, 4H), 3.38 - 3.27 (m, 6H), 3.25 (s, 4H), 2.82 (d, J = 3.5 Hz, 1H), 2.37 (d, J = 7.6 Hz, 3H), 2.06 - 1.98 (m, 1H), 1.95 - 1.72 (m, 8H), 1.63 (dd, J = 14.2, 7.1 Hz, 3H), 1.39 (s, 9H), 0.76 (t, J = 7.4 Hz, 3H).

### Example 1.3.25 Synthesis method of Compound UBI-1263 (P1-13h):

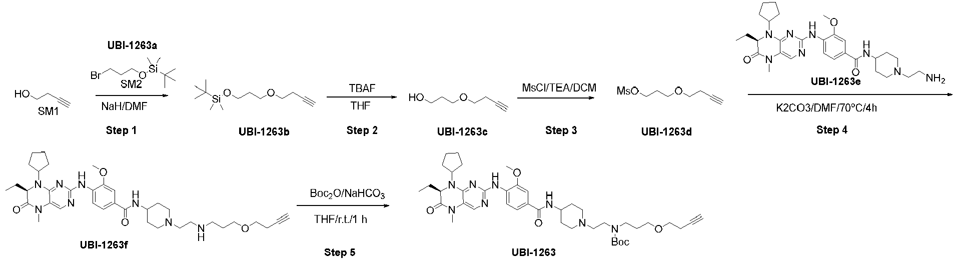

### Step 1: UBI-1263b (V1685-121)

**UBI-1263b** (5g, 71.4mmol) was dissolved in DMF (200mL) and cooled to 0°C, NaH (3.4g, 85.7mmol) was added. The mixture was reacted at 0°C for 1 hour. **UBI-1263a** (21.6g, 85.7mmol) was added, and then reacting at room temperature for 16 hours. The reaction was added to saturated NH₄Cl aqueous solution and extracted with dichloromethane (10mL*3). The organic phase was dried over Na₂SO₄ and concentrated to obtain crude product, which was isolated by silica gel column chromatography (petroleum ether/dichloromethane=3/1) to obtain **UBI-1263b** (5g, yield 29%) as a colorless oil.

¹H NMR (400 MHz, chloroform-d) δ 3.65 (t, *J=* 6.1 Hz, 2H), 3.50 (td, *J=* 6.6, 3.4 Hz, 4H), 2.41 (t, *J=* 7.0, 2.7 Hz, 2H), 1.92 (t, *J=* 2.7 Hz, 1H), 1.73 (p, *J=* 6.2 Hz, 2H), 0.84 (s, 9H), 0.00 (s, 6H).

### Step 2: UBI-1263c (V1685-123)

**UBI-1263b** (3g, 12.4mmol) was dissolved in THF (100mL), TBAF (4.85g, 18.6mmol) was added. The reaction was reacted at room temperature for 16 hours. The reaction solution was added water, and extracted with ethyl acetate (10 mL*3). The organic phase was dried over Na₂SO₄ and concentrated to obtain crude product, which was isolated by silica gel column chromatography (dichloromethane/methanol=20/1) to obtain **UBI-1263c** (1.3g, yield 82.2%) as a colorless oil.

¹H NMR (400 MHz, chloroform-*d*) δ 3.82 - 3.75 (m, 2H), 3.67 (t, *J=* 5.8 Hz, 2H), 3.58 (t, *J=* 6.7 Hz, 2H), 2.47 (td, *J =* 6.7, 2.7 Hz, 2H), 2.34 (s, 1H), 2.00 (t, *J =* 2.6 Hz, 1H), 1.85 (p, *J =* 5.7 Hz, 2H).

### Step 3: UBI-1263d (V1685-129)

**UBI-1263c** (128mg. 1mmol) was dissolved in DCM (10mL), then TEA (88ul, 2mmol) and MsCl (155ul, 2mmol) were added. The reaction was reacted at room temperature for 16 hours. The reaction solution was added water, and extracted with dichloromethane (10 mL*3). The organic phase was dried and concentrated to give the crude product, which was directly used in the next step.

### Step 4: UBI-1263f (V1685-130)

**UBI-1263e** (213mg, 0.388mmol), **UBI-1263d** (214mg,0.388mmol), and K2CO3 (160mg, 1.16mmol) were dissolved in DMF (10mL), the mixture as reacted at 70°C for 4 hours. The reaction solution was added water, and extracted with ethyl acetate (50 mL*3). The organic phase was dried over Na₂SO₄ and concentrated to obtain crude product, and it was isolated by silica gel column chromatography (dichloromethane/methanol=20/1), then the silica gel column was rinsed with MeOH/NH3. H2O =1/1 to obtain **UBI-1263f** (200mg, yield 78.1%).

LC-MS: [M+H] ⁺= 661.2

### Step 5: UBI-1263 (V1685-131)

**UBI-1263f** (200mg, 0.3mmol), (Boc)₂O (327mg, 1.5mmol), and NaHCO3 (80mg, 0.9mmol) were dissolved in THF. The reaction was reacted at room temperature for 1 hour. The reaction solution was added water, and extracted with ethyl acetate (10 mL*3). The organic phase was dried over Na₂SO₄ and concentrated to obtain crude product, which was isolated by silica gel column chromatography (dichloromethane/methanol=20/1 to 100%) to obtain target product **UBI-1263** (80mg, yield 34.7%) as white solid. LC-MS: [M+H] ⁺= 761.5

### Example 1.3.26 Synthesis method of Compound UBI-1264 (P1-14h):

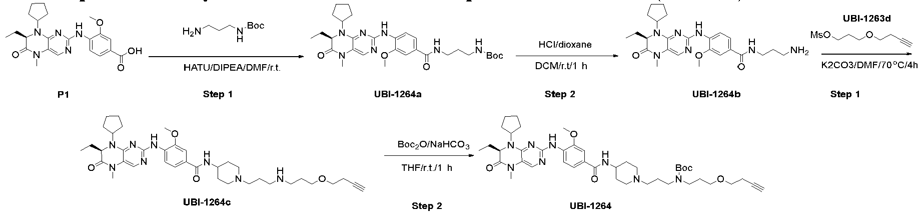

### Step 1: UBI-1264a (V1899-108)

P1 (1.43 g, 8.22 mmol), N-Boc-1,3-propanediamine (3.5 g, 8.22 mmol), HATU (4.7 g, 16.44 mmol) were dissolved in DIPEA (3.2 g, 24.66 mmol) and DMF (40 mL). The reaction was reacted at room temperature for 18 hours. The reaction solution was added to 100mL water, and solid appeared, which was filtered to obtain yellow target compound **UBI-1264a** (3.5 g, yield 73%)

¹H NMR (400 MHz, DMSO-*d*₆) δ 8.43 (d, *J=* 8.3 Hz, 1H), 8.31 (t, *J=* 5.8 Hz, 1H), 7.85 (s, 1H), 7.61 (s, 1H), 7.51 - 7.43 (m, 2H), 6.82 (t, *J=* 5.8 Hz, 1H), 4.35 (t, *J=* 8.2 Hz, 1H), 4.24 (dd, *J =* 7.6, 3.6 Hz, 1H), 3.94 (s, 3H), 3.33 (s, 1H), 3.25 (s, 4H), 2.98 (q, *J =* 6.6 Hz, 2H), 2.06 - 1.97 (m, 1H), 1.90 (td, *J* = 7.9, 2.5 Hz, 2H), 1.78 (qd, *J=* 6.8, 4.3, 3.5 Hz, 4H), 1.68 - 1.55 (m, 5H), 1.38 (s, 9H), 0.76 (t, *J =* 7.4 Hz, 3H). LCMS [M+H] ⁺=582

### Step 2: UBI-1264b (V1899-110)

**UBI-1264a** (300 mg, 0.52 mmol) was cooled to 0°C, then 4M HCl/dioxane (0.6 mL) was added. The reaction was reacted at room temperature for 2 hours. The reaction solution was concentrated to obtain product **UBI-1264b** (200 mg, yield 85%) as a yellow oil. LCMS [M+H] ⁺=482

### Step 3: UBI-1264c (V1685-134)

**UBI-1264b** (600mg, 1mmol), **UBI-1263d** (206mg, 1mmol), and K2CO3 (414mg, 3mmol) were dissolved in DMF, the mixture was reacted at 70°C for 16 hours. After the completion of the reaction, the reaction solution was directly separated by reversed-phase column (water/methanol =5%/95% 45 minutes). to obtain crude product **UBI-1260c** (200mg, yield 29.6%) as a white solid. LC-MS: [M+H] ⁺= 675.5

### Step 2: UBI-1264 (V1685-135)

**UBI-1264c** (200mg, 0.3mmol), (Boc)₂O (327mg, 1.5mmol), and NaHCO3 (80mg, 0.9mmol) were dissolved in THF, the mixture was reacted at room temperature for 1 hour. The reaction solution was added water, and extracted with ethyl acetate (10 mL*3). The organic phase was dried over Na₂SO₄ and concentrated to obtain crude product, which was isolated by silica gel column chromatography (dichloromethane/methanol=20/1 to 100%) to obtain target product **UBI-1264** (80mg, yield 34.5%) as a white solid. LC-MS: [M+H] ⁺= 774.5

### Example 1.3.27 Synthesis method of Compound UBI-1265 (P1-10h):

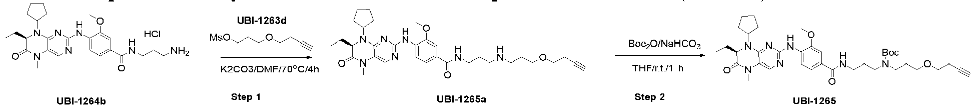

### Step 1: UBI-1265a (V1685-144)

**UBI-1264b** (200mg, 0.41mmol), **UBI-1263d** (85mg, 0.41mmol), and K2CO3 (113mg, 0.82mmol) were dissolved in DMF, the mixture was reacted at 70°C for 16 hours. The reaction solution was directly separated by reversed-phase column (water/methanol =5%/95% 45 minutes). to obtain crude product **UBI-1265a** (200mg, yield 82.6%) as a white solid. LC-MS: [M+H] ⁺= 592.2

### Step 2: UBI-1265 (V1685-148)

**UBI-1265a** (200mg, 0.3mmol), (Boc)₂O (327mg, 1.5mmol), and NaHCO3 (80mg, 0.9mmol) were dissolved in THF, the mixture was reacted at room temperature for 1 hour. The reaction solution was added water, and extracted with ethyl acetate (10 mL*3). The organic phase was dried over Na₂SO₄ and concentrated to obtain crude product, which was isolated by silica gel column chromatography (dichloromethane/methanol=20/1 to 100%) to obtain target product **UBI-1265** (90mg, yield 34.5%) as a white solid. LC-MS: [M+H] ⁺= 692.5

### Example 1.3.28 Synthesis method of Compound UBI-1258 (P1-14h)

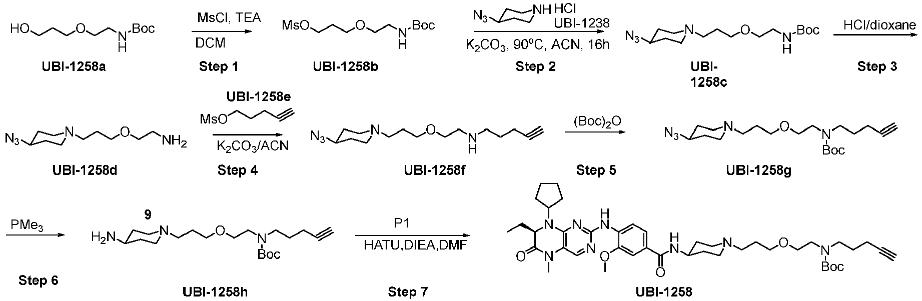

### Step 1: UBI-1258b(V2126-010)

Compound **UBI-1258a** (0.5 g, 2.28 mmol), **and** compound TEA(0.7 g, 6.84 mmol) were dissolved in DCM (20 mL). At 0°C, to the reaction solution was added with MsCl (0.4 g, 3.42 mmol), then reacted at room temperature for 1 hour. The reaction solution was washed with water, and the organic phases was dried, and dried by rotary dryer to obtain crude product **UBI-1097c** (670mg, yield 100% ) as a colorless oil. LCMS [M+H]⁺ =298.2

### Step 2: UBI-1258c(V2126-011)

Compound **UBI-1258b** (670 mg, 2.28 mmol), compound **UBI-1238** (400 mg, 2.46mmol), K₂CO₃ (690 mg, 5 mmol), and KI (40 mg, 0.23 mmol) were dissolved in CH₃CN (20mL), then the mixture was reacted at 90°C for 16 hours. The reaction solution was washed with water (50 mL), extracted with dichloromethane(100mL *3). The organic phases were dried over anhydrous sodium sulfate, and dried by rotary dryer under reduced pressure to obtain crude product. The crude product was isolated by silica gel column chromatography (dichloromethane/methanol = 10/1) to obtain target product **UBI-1258c** (600 mg, yield 80%) as a white solid. LCMS [M+H]⁺ =328.3

### Step 3: UBI-1258d(V2126-012)

Compound **UBI-1258c** (600 mg, 1.83 mmol) was dissolved in 4N HCl/dioxane (10mL), then the mixture was reacted at room temperature for 1 hour. The reaction solution was dried by rotary dryer and concentrated under reduced pressure to obtain the target product **UBI-1258d (500 mg,** yield: 90%) as a white solid. LCMS [M+H]⁺ =228.3

### Step 4: UBI-1258f(V2126-017)

Compound **UBI-1258d** (400 mg, 1.33 mmol), compound **UBI-1258e** (215 mg, 1.33 mmol), K₂CO₃ (550 mg, 4 mmol), and KI (25 mg, 0.13 mmol) were dissolved in CH₃CN (20mL), then the mixture was reacted at 90°C for 3 hours. The reaction solution was filtered, the filtrate was concentrated by rotary evaporation under reduced pressure to obtain crude product, and separated by reverse C18 column chromatography, lyophilizated to obtain target product **UBI-1258f** (350 mg, yield 90%, purity50%) as a colorless oil. LCMS [M+H]⁺ =294.2

### Step 5: UBI-1258g(V2126-022)

**Compound UBI-1258f** (350 mg, 1.2 mmol), compound di-tert-butyl carbonate (392 mg, 1.8 mmol) and NaHCO₃ (300 mg, 3.6 mmol) were dissolved in THF (20mL) and reacted at room temperature for 2 hours. The reaction solution was filtered, the filtrate was concentrated by rotary evaporation under reduced pressure to obtain crude product, and it was isolated by silica gel column chromatography(ethyl acetate: petroleum ether=1:1) to obtain **UBI-1258g** (200 mg, yield 42% ) as a white solid. LCMS [M+H]⁺ =394.3

### Step 6: UBI-1258h(V2126-024)

Compound **UBI-1258g**(200mg, 0.51ml), and 1N triphenylphosphine in tetrahydrofuran(1mL, 1mmol) were dissolved in CH₃CN (10mL), then the mixture was reacted at room temperature for 1 hour. The reaction was added with water (1 mL), and reacted at 40 °C for 2 hours. The reaction solution was dried by rotary dryer and concentrated under reduced pressure to obtain the target product **UBI-1258h (180 mg,** yield: 100%) as a white solid. LCMS [M+H]⁺ =368.3

### Step 7: UBI-1258(V2126-025)

Compound **UBI-1258h** (180 mg, 0.5 mmol), compound **P1** (210 mg, 0.50 mmol), HATU(230 mg, 0.6 mmol), and diisopropylethylamine (190 mg, 1.5 mmol) were dissolved in DMF (5 mL), then the mixture was reacted at room temperature for 1 hour. The reaction solution was concentrated by rotary evaporation under reduced pressure to obtain crude product, the crude product was diluted by adding dichloromethane, and washed with water. The organic phase was dried over anhydrous sodium sulfate and concentrated, and isolated by silica gel column chromatography (dichloromethane/methanol = 0:1) to obtain **UBI-1258** (300 mg, yield 78%) as a white solid. LCMS [M+H]⁺ =775.5

### Example 1.3.29 Synthesis method of Compound UBI-1257(P1-12h)

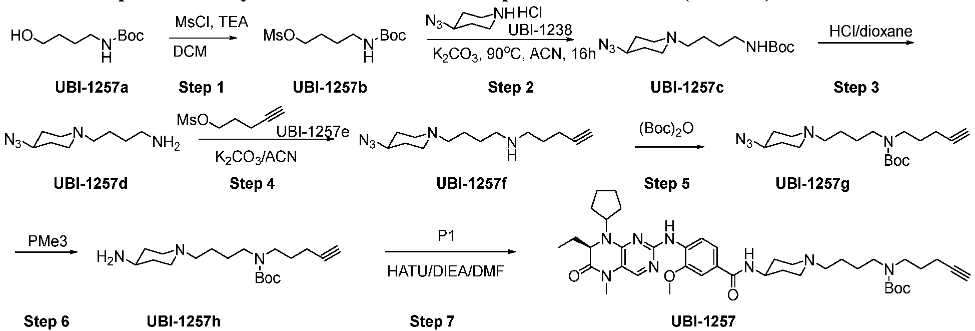

### Step 1: UBI-1257b(V2126-015)

Compound **UBI-1257a** (4 g, 21 mmol), **and** triethylamine(6.4 g, 63 mmol) were dissolved in dichloromethane (200 mL), the reaction solution was added dropwise methylsufonyl chloride (3.6 g, 31.5 mmol) at 0°C, then reacted at room temperature for 1 hour. The reaction solution was washed with water, and the organic phases was dried over anhydrous sodium sulfate, and concentrated by rotary evaporation under reduced pressure to obtain crude product **UBI-1257b** (5.6 g, yield 100%) as a colorless oil. LCMS [M+H]⁺ =268.2

### Step 2: UBI-1257c(V2126-016)

Compound **UBI-1257b** (5.6 g, 21 mmol), compound **UBI-1238** (3.4 g, 21mmol), K2CO3 (5.8 g, 42 mmol), and KI (400 mg, 2.1 mmol) were dissolved in CH3CN (200 mL), then the mixture was reacted at 90 °C for 16 hours. The reaction solution was filtered. The filtrate was concentrated by rotary evaporation under reduced pressure to obtain crude product, and it was isolated by silica gel column chromatography (dichloromethane: methanol = 10: 1) to obtain target product **UBI-1260c** (4.5 g, yield 72%) as white solid. LCMS [M+H]⁺ =298.3

### Step 3: UBI-1257d(V2126-020)

Compound **UBI-1257c** (1.5 g, 5 mmol) was dissolved in 4N HCl/dioxane (30mL), then the mixture was reacted at room temperature for 1 hour. The reaction solution concentrated by rotary evaporation under reduced pressure to obtain crude product **UBI-1257d** (1.3 g, yield 95%). LCMS [M+H]⁺ =198.3

### Step 4: UBI-1257f(V2126-023)

Compound **UBI-1257d** (800 mg, 2.96 mmol), compound **UBI-1257e** (480 mg, 2.96 mmol), K₂CO₃ (1.25 g, 8.88 mmol), and KI (60 mg, 0.3 mmol) were dissolved in CH₃CN (30mL), then the mixture was reacted at 90°C for 3 hours. The reaction solution was filtered, the filtrate was concentrated by rotary evaporation under reduced pressure to obtain crude product, and separated by reverse C18 column chromatography, lyophilizated to obtain target product **UBI-1257f** (500 mg, yield 60% , purity50%) as a colorless oil. LCMS [M+H]⁺ =264.3

### Step 5: UBI-1257g(V2126-026)

Compound **UBI-1257f** (500 mg, 1.9 mmol), compound di-tert-butyl carbonate (620 mg, 2.8 mmol), and NaHCO₃ (480 mg, 5.7 mmol) were dissolved in THF (20 mL), then the mixture was reacted at room temperature for 2 hours. The reaction solution was filtered, the filtrate was concentrated by rotary evaporation under reduced pressure to obtain crude product, and it was isolated by silica gel column chromatography(ethyl acetate: petroleum ether=1:1) to obtain target product **UBI-1257g** (250 mg, 36% yield) as a white solid. LCMS [M+H]⁺ =364.3

### Step 6: UBI-1257h(V2126-028)

Compound **UBI-1257g** (250mg, 0.69ml), and 1N triphenylphosphine in tetrahydrofuran (1.4mL, 1.4mmol) were dissolved in THF (10mL), then the mixture was reacted at room temperature for 1 hour. The reaction was added with water (1 mL), and reacted at 40 °C for 2 hours. The reaction solution was concentrated by rotary evaporation under reduced pressure to obtain the target product **UBI-1257h (230 mg,** yield: 100%) as a white solid. LCMS [M+H]⁺ =338.4

### Step 7: UBI-1257(V2126-029)

Compound **UBI-1257h** (230 mg, 0.69 mmol), compound **P1** (290 mg, 0.69 mmol), HATU(315 mg, 0.83 mmol), and diisopropylethylamine (270 mg, 2.1 mmol) were dissolved in DMF (5 mL), then the mixture was reacted at room temperature for 1 hour. The reaction solution was concentrated by rotary evaporation under reduced pressure to obtain crude product, the crude product was diluted by adding dichloromethane, washed with water. The organic phase was dried over anhydrous sodium sulfate and concentrated. and isolated by silica gel column chromatography (dichloromethane:methanol = 10:1) to obtain **UBI-1257** (400 mg, yield 78%) as a white solid. LCMS [M+H]⁺ =745.5

### Example 1.4 Synthesis method of Compound Linker b- A1

### Example 1.4.1 Synthesis method of Compound UBI-1302 (NH₂-12e-A1)

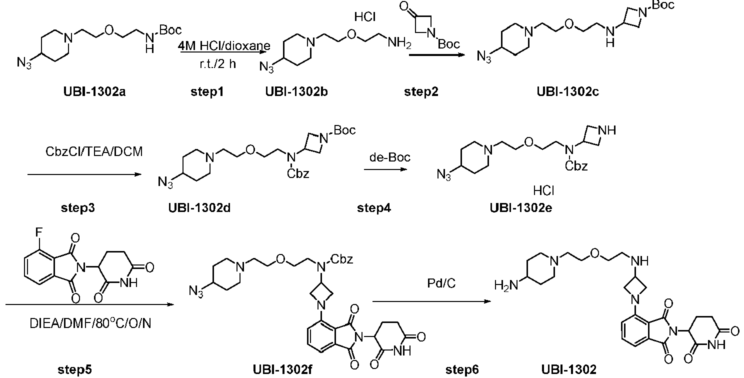

### Step 1: UBI-1302b (V1895-009)

Compound **UBI-1302a** (660 mg, 2.11 mmol) and HCl/dioxane (10 mL, 4 N) were added to tetrahydrofuran (10mL), the mixture was reacted at room temperature for 2 hours. After the completion of the reaction, the mixture was concentrated by rotary evaporation under reduced pressure to obtain compound **UBI-1302b** (525 mg, yield 100%).

### Step 2: UBI-1302c(V1895-015)

Compound **UBI-1302b(525** mg, 2.11 mmol), and tert-butyl 3-oxoazetidine-1-carboxylate (361 mg, 2.11 mmol) were added to dichloromethane (10 mL), sodium triacetoxyborohydride (530 mg, 2.51 mmol) was added. After the completion of the reaction, the reaction was poured into 10 mL of water and extracted with dichloromethane (5 mL*3). The organic phases were combined, then washed with saturated brine, dried over anhydrous Na₂SO₄, and concentrated by rotary evaporation under reduced pressure to obtain crude product. The crude product was purified by silica gel column chromatography (DCM/MeOH = 10/1) to obtain compound **UBI-1302c** (240 mg) as a white solid. Yield: 31%.

### Step 3: UBI-1302d(V1895-017)

**UBI-1302c** (240 mg, 0.65 mmol), benzyl chloroformate (144 mg, 0.85 mmol) and sodium bicarbonate (771 mg, 9.18 mmol) were added to tetrahydrofuran (20 ml), the mixture was reacted at room temperature for 12 h, then poured to 10 mL water and extracted with dichloromethane (10 ml*3). The organic layer was dried over anhydrous Na2SO4 and concentrated to give product **UBI-1302d** (198 mg, 77% yield).

### Step 4: UBI-1302e(V1895-018)

**UBI-1302d** (198mg, 0.39mmol), and hydrochloric acid/dioxane (10mL, 4N) were added to tetrahydrofuran (10mL), the mixture was reacted at room temperature for 2 hours. After the completion of the reaction, the mixture was concentrated under reduced pressure to obtain compound **UBI-1302e** (159mg, yield 100%).

### Step 5: UBI-1302f(V1895-019)

**UBI-1302e(159** mg, 0.39 mmol), DIEA(50 mg, 0.39 mmol) and 2-(2,6-dioxopiperidin-3-yl)-4-fluoroisoindole-1,3-dione (107 mg, 0.39 mmol) were added to DMF (30 mL), the reaction was stirred at 80°C for 18 h, then isolated by silica gel column chromatography to obtain compound **UBI-1302f** (80mg, yield 31%).

### Step 6: UBI-1302 (V1895-020)

UBI-180857f (41mg, 0.06mmol), and 10% palladium on carbon (20mg) were added to a mixed solvent of methanol/dichloromethane (1 mL/10 mL), and the mixture was reacted at room temperature for 2 hours under hydrogen atmosphere. After filtration, the filtrate was concentrated to obtain the crude product. The crude product was washed with cold ether (10 mL*3), and dried to obtain compound **UBI-1302** (19mg, yield 49%).

### Example 1.4.2 Synthesis method of Compound UBI-1303 (NH₂-12e-A1)

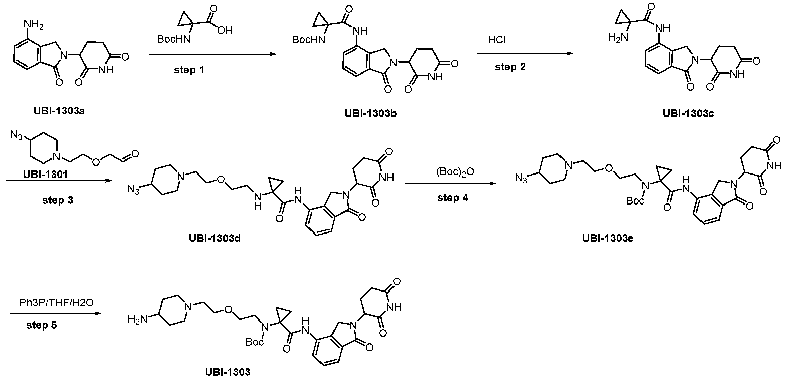

### Step 1: UBI-1303b(V1895-025)

1-(tert-Butoxycarbonyl)cyclopropanecarboxylic acid (500mg, 2.49mmol), lenalidomide (644mg, 2.49mmol), HATU(1015mg, 2.67mmol) and DIPEA(0.3mL) were added to DMF(3mL), the mixture was stirred at room temperature for 16h, purified by reversed-phase column (MeOH/H2O=5% to 95%, 45min) to obtain product UBI-1303b (846mg, 77% yield ).

### Step 2: UBI-1303c(V1895-028)

**UBI-1303b** (846 mg, 1.91 mmol) was dissolved in dichloromethane (5mL) and methanol(5mL), then HCl/dioxane (0.5mL) was added, and the mixture was reacted at room temperature for 1 hour. The reaction solution was concentrated to obtain crude product target compound **UBI-1303c** (654 mg, yield 100%) (76 mg, yield 100%).

### Step 3: UBI-1303d(V1895-059)

Compound **UBI-1303c** (305 mg, 1.44 mmol), and **UBI-1301** (361 mg, 2.11 mmol) were added to dichloromethane (10 mL), sodium triacetoxyborohydride (530 mg, 2.51 mmol) was added. After the completion of the reaction, the reaction was poured into 10 mL of water and extracted with dichloromethane (5 mL*3). The organic phases were combined, then washed with saturated brine, dried over anhydrous Na₂SO₄, concentrated by rotary evaporation under reduced pressure to obtain crude product. The crude product was purified by silica gel column chromatography (DCM/MeOH = 10/1) to obtain compound **UBI-1303d** (240 mg) as a white solid. Yield: 31%.

### Step 4: UBI-1303e (V1895-061)

**UBI-1303d** (240 mg, 0.45 mmol), and tert-butyl dicarbonate (146 mg, 0.67 mmol) were added to dioxane (30 mL), the mixture was reacted at room temperature for 2 h, then concentrated and extracted with ethyl acetate (10 mL*3), filtered and concentrated to obtain desired compound **UBI-1303e**(233mg, yield 82%).

### Step 5: UBI-1303(V1895-062)

**UBI-1303e** (233 mg, 0.37mmol), and 10% palladium on carbon (20mg) was added to a mixed solvent of methanol/dichloromethane (1 mL/10 mL), and reacted at room temperature for 2 hours under hydrogen atmosphere. After filtration, the filtrate was concentrated to obtain the crude product. The crude product was washed with cold ether (10 mL*3), and dried to obtain compound **UBI-1303** (221 mg, yield 100 %).

LCMS: (M+H) ⁺= 613.3

### Example 1.4.3 Synthesis method of Compound UBI-1304 (NH₂-12e-A1)

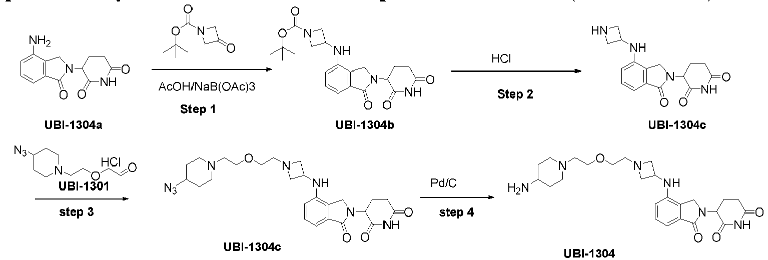

### Step 1: UBI-1304b (V1685-046)

Lenalidomide (1g, 3.8mmol) was dissolved in AcOH (20ml), then N-Boc-3-hydroxyazetidine (1.31g, 7.7mmol) was added, the mixture was reacted at 80°C for 1 hour. The reaction solution was cooled to 30 °C, the reaction solution room was reacted at room temperature for 16 hours after adding NaBH(OAc)₃. The reaction solution was concentrated, then isolated by silica gel column (dichloromethane /methanol = 20/1) to obtain target compound **UBI-1304b** (1.15g, yield 73.2%) as white solid. LC-MS: (M+H)⁺= 415.1

### Step 2: UBI-1304c (V1685-047)

**UBI-1304b** (100mg, 0.24mmol) was dissolved in dichloromethane (5mL) and methanol(5mL), then HCl/dioxane (0.5mL) was added, the mixture was reacted at room temperature for 1 hour. The reaction solution was concentrated to obtain crude product of target compound (76mg, yield 100%), the crude product was directly used in the next reaction.

### Step 3: UBI-1304d (V1895-065)

**UBI-1304c** (300 mg, 1.42 mmol), and **UBI-1301** (452 mg, 1.44 mmol) were added to dichloromethane (10 mL), sodium triacetoxyborohydride (530 mg, 2.51 mmol) was added. After the completion of the reaction, the reaction was poured into 10 mL of water and extracted with dichloromethane (5 mL*3). The organic phases were combined, then washed with saturated brine, dried over anhydrous Na₂SO₄, concentrated by rotary evaporation under reduced pressure to obtain crude product. The crude product was purified by silica gel column chromatography (DCM/MeOH = 10/1) to obtain compound **UBI-1304d** (215 mg) as a white solid. Yield: 30%.

### Step 4: UBI-1304e (V1895-066)

**UBI-1304d** (215 mg, 0.42 mmol), and 10% palladium on carbon (20mg) were added to a mixed solvent of methanol/dichloromethane (1 mL/10 mL), and reacted at room temperature for 16 hours under hydrogen atmosphere. After filtration, the filtrate was concentrated to obtain the crude product. The crude product was washed with cold ether (10 mL*3), and dried to obtain compound **UBI-1304e** (139 mg, yield 71%).

LCMS: (M+H) ⁺= 485.1

### Example 1.4.3 Synthesis method of Compound UBI-13045(NH₂-12e-A1)

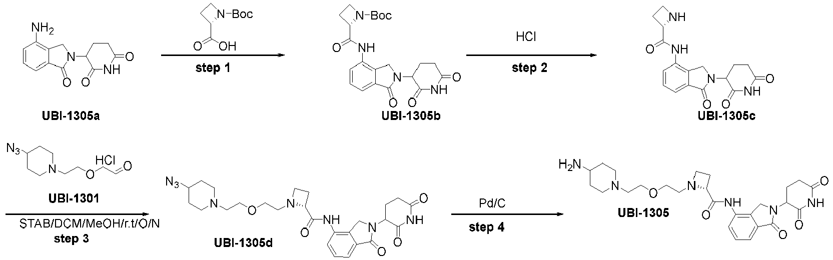

### Step 1: UBI-1305b (V1895-026)

1-Boc-L-azetidine-2-carboxylic acid (500mg, 2.49mmol), lenalidomide (644mg, 2.49mmol), HATU(1015mg, 2.67mmol) and DIPEA(0.3mL) were added to DMF(3mL), and stirred at room temperature for 16 h, and the reaction solution was purified by reversed-phase column (MeOH/H2O=5% to 95%, 45min) to obtain product **UBI-1305b** (846mg, 77% yield ).

### Step 2: UBI-1305c(V1895-027)

**UBI-1303b** (846 mg, 1.91mmol) was dissolved in dichloromethane (5mL) and methanol (5mL), then HCl/dioxane (0.5mL) was added, the mixture was reacted at room temperature for 1 hour. The reaction solution was concentrated to obtain crude product target compound **UBI-1303c** (654 mg, yield 100%) (76 mg, yield 100%).

### Step 3: UBI-1305d(V1895-029)

Compound **UBI-1305c** (352 mg, 1.42 mmol), and **UBI-1301** (485 mg, 1.42 mmol) were added to dichloromethane (10 mL), sodium triacetoxyborohydride (530 mg, 2.51 mmol) was added. After the completion of the reaction, the reaction was poured into 10 mL of water and extracted with dichloromethane (5 mL*3). The organic phases were combined, then washed with saturated brine, dried over anhydrous Na₂SO₄, concentrated by rotary evaporation under reduced pressure to obtain crude product. The crude product was purified by silica gel column chromatography (DCM/MeOH = 10/1) to obtain compound **UBI-1305d** (229 mg) as a white solid. Yield: 30%.

### Step 4: UBI-1305 (V1895-070)

**UBI-1305d** (229 mg, 0.43 mmol), and 10% palladium on carbon (20mg) were added to a mixed solvent of methanol/dichloromethane (1 mL/10 mL), and reacted at room temperature for 16 hours under hydrogen atmosphere. After filtration, the filtrate was concentrated to obtain the crude product. The crude product was washed with cold ether (10 mL*3), and dried to obtain compound **UBI-1305** (139 mg, yield 71%).

LCMS: (M+H) ⁺= 513.1

### Example 1.4.4 Synthesis method of Compound UBI-1306 (NH₂-12e-A1)

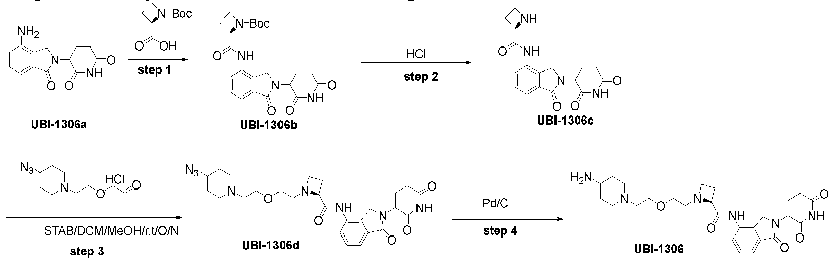

### Step 1: UBI-1306b (V1895-057)

1-Boc-R-azetidine-2-carboxylic acid (500mg, 2.49mmol), lenalidomide (644mg, 2.49mmol), HATU(1015mg, 2.67mmol) and DIPEA(0.3mL) were added to DMF(3mL), the mixture was stirred at room temperature for 16h, purified by reversed-phase column (MeOH/H2O=5% to 95%, 45min) to obtain **UBI-1306b** (840mg, yield 76%).

### Step 2: UBI-1306c(V1895-058)

**UBI-1306b** (840 mg, 1.91mmol) was dissolved in dichloromethane (5mL) and methanol (5mL), then HCl/dioxane (0.5mL) was added, the mixture was reacted at room temperature for 1 hour. The reaction solution was concentrated to obtain crude product target compound **UBI-1306c** (650 mg, yield 100%) (76 mg, yield 100%).

### Step 3: UBI-1306d(V1895-072)

**UBI-1301** (352 mg, 1.42 mmol), and **UBI-1306c** (485 mg, 1.42 mmol) were added to dichloromethane (10 mL), sodium triacetoxyborohydride (530 mg, 2.51 mmol) was added. After the completion of the reaction, the reaction was poured into 10 mL of water and extracted with dichloromethane (5 mL*3). The organic phases were combined, then washed with saturated brine, dried over anhydrous Na₂SO₄, concentrated under reduced pressure to obtain crude product. The crude product was purified by silica gel column chromatography (DCM/MeOH = 10/1) to obtain compound **UBI-1306d** (229 mg) as a white solid. Yield: 30%.

### Step 4: UBI-1306 (V1895-073)

**UBI-1306d** (229 mg, 0.43 mmol), and 10% palladium on carbon (20 mg) were added to a mixed solvent of methanol/dichloromethane (1 mL/10 mL), and reacted at room temperature for 16 hours under hydrogen atmosphere. After filtration, the filtrate was concentrated to obtain the crude product. The crude product was washed with cold ether (10 mL*3), and dried to obtain compound **UBI-1306** (154 mg, yield 71%).

LCMS: (M+H) ⁺= 513.1

### Example 1.4.5 Synthesis method of Compound UBI-1307(NH₂-12e-A1)

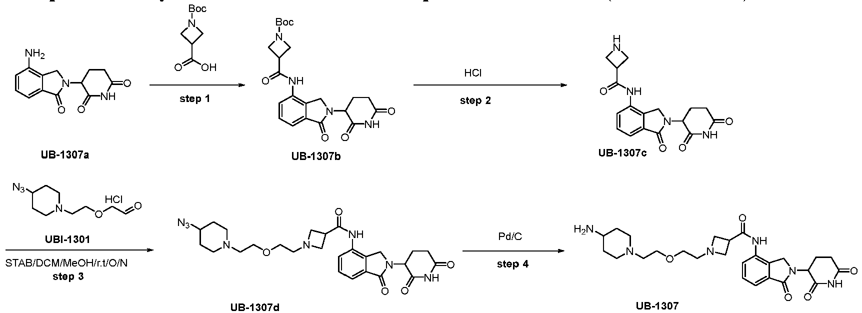

### Step 1: UBI-1307b (V1895-057)

1-{[(1,1-Dimethylethyl)oxy]carbonyl}-3-hydroxyazetidine-3-carboxylic acid(500mg, 2.49mmol), lenalidomide (644mg, 2.49mmol), HATU(1015mg, 2.67mmol) and DIPEA(0.3mL) were added to DMF(3mL), the mixture was stirred at room temperature for 16h, purified by reversed-phase column (MeOH/H2O=5% to 95%, 45min) to obtain **UBI-1307b** (840mg, yield 76%).

### Step 2: UBI-1307c(V1895-061)

**UBI-13076b** (840 mg, 1.91mmol) was dissolved in dichloromethane (5mL) and methanol (5mL), then HCl/dioxane (0.5mL) was added, the mixture was reacted at room temperature for 1 hour. The reaction solution was concentrated to obtain crude product target compound **UBI-1307c** (650 mg, yield 100%).

### Step 3: UBI-1307d(V1895-075)

Compound **UBI-1307c** (352 mg, 1.42 mmol), and **UBI-1301** (485 mg, 1.42 mmol) were added to dichloromethane (10 mL), sodium triacetoxyborohydride (530 mg, 2.51 mmol) was added. After the completion of the reaction, the reaction was poured into 10 mL of water and extracted with dichloromethane (5 mL*3). The organic phases were combined, then washed with saturated brine, dried over anhydrous Na₂SO₄, concentrated by rotary evaporation under reduced pressure to obtain crude product. The crude product was purified by silica gel column chromatography (DCM/MeOH = 10/1) to obtain compound **UBI-1307d** (230 mg) as a white solid. Yield: 30%.

### Step 4: UBI-1307 (V1895-076)

**UBI-1307d** (229 mg, 0.43 mmol), and 10% palladium on carbon (20 mg) were added to a mixed solvent of methanol/dichloromethane (1 mL/10 mL), and reacted at room temperature for 16 hours under hydrogen atmosphere. After filtration, the filtrate was concentrated to obtain the crude product. The crude product was washed with cold ether (10 mL*3), and dried to obtain compound **UBI-1307** (154 mg, yield 71%).

LCMS: (M+H) ⁺= 513.1

### Example 1.4.6 Synthesis method of Compound UBI-1308 (NH₂-12e-A1)

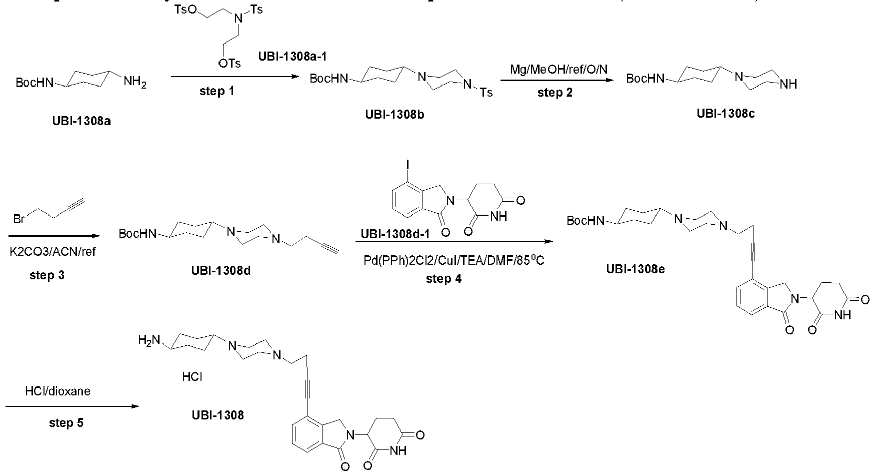

### Step 1: UBI-1308b (V1895-091)

**UBI-1308a** (1.0 g, 4.67 mmol), **UBI-1308a-1** (2.6 g, 4.67 mmol) and N,N-diisopropylethylamine (1.8 g, 14.01 mmol) were added to acetonitrile(20 mL), the mixture was reacted at 80°C for 18 hours. The reaction solution was filtered through Celite, and the filtrate was concentrated to obtain crude product. The crude product was purified via flash chromatography (DCM/MeOH=0% to10%, 20min) to obtain compound **UBI-1308b**(1.5g, yield 72%).

### Step 2: UBI-1308c (V1895-092)

**UBI-1308b** (1.1 g, 2.5 mmol) was dissolved in MeOH (500 mL), magnesium chips (5.6 g, 2.3 mmol) were added, the mixture was reacted at 65°C overnight. The reaction solution was filtered through Celite, and the filtrate was concentrated to obtain crude product, which was purified by flash chromatography (eluted with DCM/MeOH=0% to 10%) to obtain product **UBI-1308c** (0.29g) as a white solid. Yield: 41%.

### Step 3: UBI-180889 d(V1895-093)

**UBI-180889c(500** mg, 1.77 mmol) and 4-bromo-1-alkyne(280 mg, 2.12 mmol) were added to acetonitrile (10 mL), potassium carbonate (733 mg, 5.31 mmol) was added under N2. The reaction solution was filtered through Celite, and the filtrate was concentrated to obtain crude product, which was purified by flash chromatography (eluted with DCM/MeOH=0% to 10% for 20 minutes) to obtain product **UBI-180889d** (473mg, yield 80%) as a white solid.

### Step 4: UBI-1308d (V1895-094)

Compound **UBI-1308c** (400 mg, 1.49 mmol) was dissolved in DMF (10 mL), dichlorobis(triphenylphosphonium)palladium (104 mg,0.149 mmol), cuprous iodide (57 mg, 0.07 mmol) and triethylamine (150 mg, 1.49 mmol) were added, the mixture was reacted at 80°C overnight under nitrogen. The reaction solution was filtered through Celite, and the filtrate was concentrated to obtain crude product, which was purified by flash t chromatography (eluted with DCM/MeOH=0% to 10% for 30 minutes) to obtain product **UBI-1308d** (260mg) as a white solid. Yield: 38%.

### Step 5: UBI-1308e (V1895-095)

**UBI-1308c** (900 mg, 3.15 mmol), and hydrochloric acid/dioxane (10mL, 4N) were added to tetrahydrofuran (10mL), the mixture was reacted at room temperature for 2 hours. After the completion of the reaction, the mixture was concentrated under reduced pressure to obtain compound **UBI-1308** (215 mg, yield 100%).

### Example 1.4.7 Synthesis method of Compound UBI-1309 (NH₂-12e-A1)

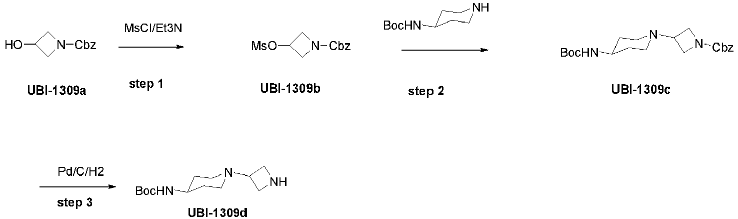

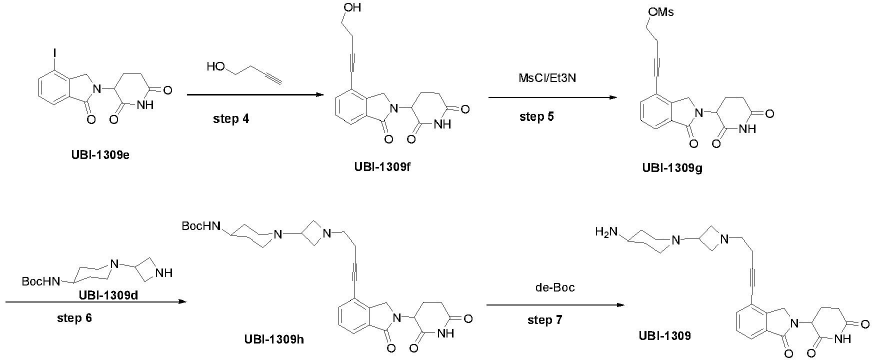

### Step 1: UBI-1309b(V2127-011)

**UBI-1309a(20.7** g, 100 mmol) and triethylamine(17.1 g, 150 mmol) were dissolved in DCM(60 mL), methanesulfonyl chloride (12.5 g, 110 mmol) was slowly added. After reacting at room temperature for 1 hour, the reaction was extracted with dichloromethane (50 mL×3), the combined organic layers was washed with brine(50 mL), the organic was dried over anhydrous Na2SO4, concentrated under reduced pressure, purified by flash chromatography (DCM:MeOH=10:1) to obtain **UBI-1309b**(26.5g, yield 93%) as a colorless oil.

### Step 2: UBI-1309c (V2127-013)

**UBI-1309b** (285 mg, 1.00 mmol), tert-butyl piperidin-4-yl carbamate (200 mg, 1.00 mmol), and potassium carbonate (414 mg, 3.00 mmol) were dissolved in DMF(9 mL), the mixture was reacted at 120°C in microwave for 1 hour. Resulting crude product was purified via flash chromatography (petroleum ether/ethyl acetate=80% to 100%, 20min, dichloromethane/methanol=0% to 10%, 20min) to obtain **UBI-1309c** (1 10mg, yield 28%) as a colorless oil.

### Step 3: UBI-1309d (V2127-014)

**UBI-1309c** (110mg, 27.3mmol), and 10% palladium on carbon (581mg, 5.46mmol) were added to methanol(20ml), the mixture was reacted at room temperature for 12h under hydrogen atmosphere. The reaction solution was filtered on Celite. The filtrate was concentrated to obtain **UBI-1309d** (72mg, yield 100%) as a white solid.

### Step 4: UBI-1309f (V2127-024)

**UBI-1309d** (500 mg, 1.35 mmol), 3-butyne-1-ol (94 mg, 1.35 mmol), Pd(PPh3)2Cl2(94 mg, 0.135 mmol) and cuprous iodide (51 mg, 0.27 mmol) were added to DMF (2 mL), the mixture was reacted at 80°C for 16 hours under N₂ protection. The mixture was purified by reversed- phase chromatography column (MeOH/H2O=5%-95%, 45min), collected at 60% to obtain compound **UBI-1309f** (215mg, yield 54%) as a white solid.

### Step 5: UBI-1309g (V2127-026)

Compound **UBI-1309f** (312mg, 1.00 mmol) and triethylamine (171 mg, 1.50 mmol) were added to dichloromethane (60 mL), methanesulfonyl chloride (125 mg, 1.10 mmol) was slowly added. After reacting at room temperature for 1 hour, the reaction was extracted with DCM (50 mL×3), the combined organic layers was washed with brine (50 mL), dried over anhydrous Na2SO4, concentrated under reduced pressure, purified by column chromatography (DCM:MeOH=10:1) to obtain **UBI-1309g** (171 mg, yield 99%) as a white solid.

### Step 6: UBI-1309h(V2127-027)

Compound **UBI-1309g** (78 mg, 0.20 mmol), N,N-diisopropylethylamine (50 mg, 0.39 mmol) and compound **UBI-1309d** (51 mg, 0.20 mmol) were added to acetonitrile (30 mL), the mixture was reacted at 80°C for 18 h, concentrated to obtain crude product, which was then purified via silica gel column chromatography (PE/EtOAc=70% to 100%, 20 min, MeOH/DCM=0% to 10%, 40 min) to obtain compound **UBI-1309h**(51mg, yield 46%).

### Step 7: UBI-1309 (V2127-029)

**UBI-1309h** (51 mg, 0.09 mmol), and hydrochloric acid/dioxane (10mL, 4N) were added to tetrahydrofuran (10mL), the mixture was reacted at room temperature for 2 hours. After the completion of the reaction, the mixture was concentrated under reduced pressure to obtain compound **UBI-1309** (41 mg, yield 100%).

### Example 1.5 Synthesis method of Compound P1- linker h - A1

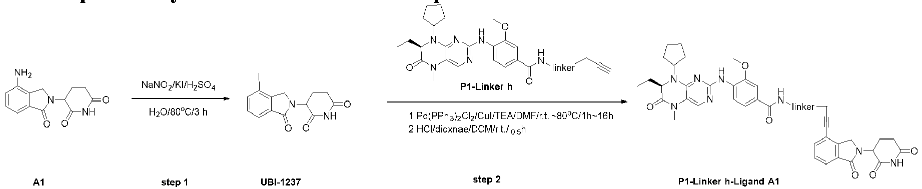

### Step 1: UBI-1237 (V1782-042)

**A1** (5.2 g, 20 mmol), and NaNO₂ (4.15 g, 60 mmol) were added to water (200 mL), diluted H₂SO₄ (20 mL of concentrated sulfuric acid plus 50 mL of water) was slowly added dropwise over about 75 minutes. Then after reacting at room temperature for 30 minutes, 100 mL aqueous solution of KI (16.6 g, 100 mmol) was added dropwise in ice bath, then the mixture was reacted at 80°C for 3 hours, and stood till cooling down. The mixture was filtered. The filter cake was washed repeatedly with petroleum ether and water. The solid was recrystallized with ethanol to obtain product **UBI-1237** (5.4 g, yield 72%) as a brownish yellow solid. LCMS [M+H]⁺ = 371

¹H NMR (400 MHz, DMSO-*d*₆) δ 11.01 (s, 1H), 8.04 (d, *J =* 7.7 Hz, 1H), 7.77 (t, *J =* 5.6 Hz, 1H), 7.35 (t, *J=* 7.6 Hz, 1H), 5.15 (m, 1H), 4.29 (d, *J=* 17.5 Hz, 1H), 4.14 (d, *J=* 17.5 Hz, 1H), 2.91 (m, 1H), 2.66 - 2.55 (m, 1H), 2.49 - 2.42 (m, 1H), 2.02 (m, 1H).

### Step 2: P1- linker h - A1

**Compound P1-linker h** (20 mg, 1eq.), **UBI-1237** (1 eq.), Pd(PPh₃)₂Cl₂ (0.2eq.), CuI (0.2eq.), and TEA (1eq.) were dissolved in anhydrous DMF(4 mL), the mixture was reacted at room temperature to 100°C for 1 hour under N2 protection. After cooling, the reaction solution was poured into 5 mL of water and extracted with ethyl acetate (5 mL*3). The organic phases was washed with water, dried over Na₂SO₄ and concentrated to give the crude product. The crude product was isolated by preP1-TLC (DCM/MeOH = 10/1) to obtain target compound.

Above target compound was dissolved in dichloromethane (3 mL), then 0.5mL (HCl/dioxane 4 M) was added, the mixture was reacted at room temperature for 1 hour. The reaction solution was concentrated by rotary evaporation under reduced pressure to obtain the crude product, which was washed anhydrous ether (5 mL *3), the solid was dried in vacuum after filtration to obtain the target Compound **P1-linker h-A11**

### Synthesis method of Compound UB-180925

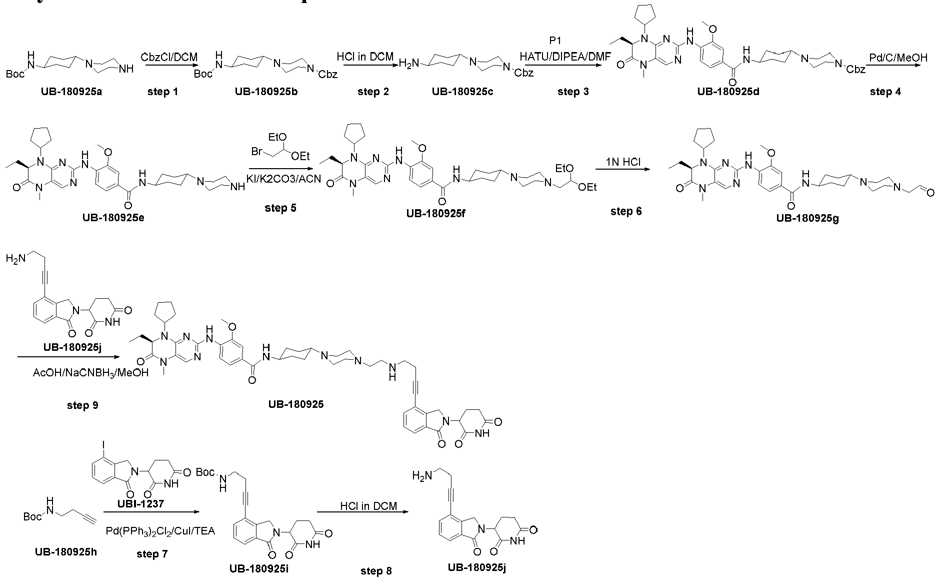

### Step 1: UB-180925b (V2031-110)

Cbz-Cl (361 mg, 2.12mmol) was added to UB-180925a (400 mg, 1.41 mmol) and TEA (285 mg, 2.83mmol) in dichloromethane (10 mL). The reaction system was stirred at 0°C for 3 hours. After the completion of the reaction, the reaction was concentrated, then isolated by silica gel column chromatography(dichloromethane /methanol =10%) to obtain UB-180925b (380 mg, yield 64%) as a yellow oil. LCMS: [M+H] ⁺= 418.2

### Step 2: UB-180925c (V2031-111)

4N hydrochloric acid in dioxane (2 mL) was added to UB-180925b(380 mg, 0.91 mmol) in dichloromethane (10 mL). The reaction system was stirred at 20°C for 4 hours, After the completion of the reaction, the reaction was concentrated to obtain UB-180925c (250 mg,86.5% yield) as a white solid. LCMS: [M+H] ⁺= 318.2

### Step 3: UB-180925d (V2031-112)

P₁ (268 mg, 0.63mmol), UB-180925c (250 mg,0.79 mol), HATU (450mg, 1.18mmol), and diisopropylethylamine (305mg, 2.37 mmol) were dissolved in anhydrous DMF (10 mL), the mixture was stirred at room temperature for 16 hours. After the reaction was completed, the mixture was poured into water (20 mL) and then extracted with ethyl acetate (20 mL × 3). The organic phases were combined, washed with brine, dried over sodium sulfate, concentrated and then isolated by silica gel column chromatography (dichloromethane /methanol =10%) to obtain UB-180925d (250 mg, yield 54.8%) as a yellow solid. LCMS: [M+H] ⁺ = 725.4

¹H NMR (400 MHz, chloroform-d) δ 11.22 (s, 2H), 8.65 - 8.52 (m, 1H), 8.28 (dd, J = 31.2, 8.4 Hz, 3H), 7.59 (s, 1H), 7.46 (d, J = 1.8 Hz, 1H), 7.35 (qd, J= 6.4, 5.5, 1.9 Hz, 5H), 6.62 (d, J = 7.8 Hz, 1H), 5.30 (s, 1H), 5.14 (s, 2H), 4.46 (q, J = 8.1 Hz, 1H), 4.27 (dd, J = 7.7, 3.5 Hz, 1H), 3.98 (s, 7H), 3.66 (pd, J= 6.6, 3.1 Hz, 7H), 3.49 (s, 2H), 3.31 (s, 3H), 3.09 (qd, J = 7.5, 3.5 Hz, 8H), 2.29 (dd, J = 32.4, 11.8 Hz, 4H), 2.13 (d, J = 11.6 Hz, 1H), 2.01 - 1.62 (m, 10H), 0.87 (t, J = 7.5 Hz, 3H).

### Step 4: UB-180925e (V2031-113)

10% palladium carbon (30 mg) was added to a solution of UB-180925d (200 mg, 0.28 mol) in methanol (5 mL). The reaction mixture was stirred under a H2 ball for 4 h. After completion of the reaction, the reaction was filtered and the filter cake was washed with methanol. The filtrate was concentrated to obtain UB-180925e (110 mg, yield 67.5%) as a white solid. LCMS: [M+H] ⁺ = 591.4

### Step 5: UB-180925f (V2031-119)

UB-180925e (250 mg, 0.42 mmol), UBI-1282 (125 mg, 0.635 mmol), potassium iodide (70.3 mg, 0.42 mmol), and potassium carbonate (117mg,0.85 mmol) were added to acetonitrile (5 mL) solution, and the reaction mixture was stirred at 80 °C for 16 h. After completion of reaction, water (5 mL) was added, the organics was separated, dried (sodium sulfate), filtered, the reaction solution was concentrated, and then isolated by silica gel column chromatography (dichloromethane /methanol = 10%) to obtain UB-180925f (200mg, yield 66.9%) as a yellow solid. LCMS: [M+H] ⁺ = 707.4

¹H NMR (400 MHz, DMSO-*d6*) δ 9.03 (s, 1H), 8.41 (d, J = 8.6 Hz, 1H), 8.10 (s, 1H), 7.84 (s, 1H), 7.60 (s, 1H), 7.51 - 7.43 (m, 2H), 4.60 (s, 1H), 4.35(q, J = 8.1 Hz, 1H), 4.24 (dd, J = 7.8, 3.6 Hz, 1H), 3.94 (s, 3H), 3.75 (s, 1H), 3.60 (t, J = 8.2 Hz, 2H), 3.48 (t, J = 8.3 Hz, 3H), 3.06 (s, 2H), 2.18 - 1.71(m, 12H), 1.63 (dd, J = 14.7, 7.2 Hz, 4H), 1.40 (d, J = 13.2 Hz, 2H), 1.12 (t, J = 7.0 Hz, 6H), 0.76 (t, J = 7.4 Hz, 3H).

### Step 6: UB-180925g (V2031-122)

UB-180925f (150 mg, 0.21 mmol) was added to 1N HCl (10mL). The reaction system was reacted at 30°C for 4 hours, after completion of reaction, the reaction was extracted with dichloromethane, dried over sodium sulfate, filtered and concentrated in vacuum to obtain UB-180925g (110 mg). LCMS: [M+H] ⁺ = 633.4

### Step 7: UB-180925i (V2031-121)

UBI-1237 (400 mg, 1.08 mmol), UB-180925h (274 mg, 1.62mmol), PdCl2(PPh3)2 (38 mg, 0.05 mmol), copper iodide(21 mg), and triethylamine (491 mg) was added to anhydrous DMF (5 mL). The reaction system was stirred at 80°C for 2 hours, the reaction was cooled down to room temperature after completion. The mixture was added into water, extracted with dichloromethane, brine (30 mL), dried over sodium sulfate, filtered, and concentrated, then isolated by silica gel column chromatography (dichloromethane/methanol=10%) to obtain UB-180925i (250 mg, yield 56.2%) as a yellow solid.

LCMS: [M+H] ⁺ = 412.2 ¹H NMR (400 MHz, DMSO-*d6*) δ 11.01 (s, 1H), 7.71 (dd, J = 7.6, 1.1 Hz, 1H), 7.64 (dt, J = 8.1, 1.9 Hz, 1H), 7.51 (d, J = 7.6 Hz, 1H), 7.11 - 6.97 (m,1H), 5.15 (dd, J = 13.3, 5.1 Hz, 1H), 3.18 (q, J = 6.6 Hz, 2H), 3.04 (q, J = 6.6 Hz, 1H), 2.98 - 2.89 (m, 1H), 2.62 (s, 1H), 2.61 - 2.54 (m, 2H), 2.45(dd, J = 13.1, 4.4 Hz, 1H), 2.39 (t, J = 6.7 Hz, 1H), 2.02 (dd, J = 8.9, 3.6 Hz, 1H), 1.37 (d, J = 4.7 Hz, 9H).

### Step 8: UB-180925j (V2031-123)

4N hydrochloric acid in dioxane(1 mL) was added to UB-180925i (150 mg, 0.21 mmol) in dichloromethane (5 mL). The reaction system was stirred at 20°C for 4 hours, after completion of reaction, the reaction was added with water, extracted with dichloromethane, dried over sodium sulfate, filtered and concentrated to obtain UB-180925j (110 mg, yield 97%). LCMS: [M+H] ⁺ = 312.1

### Step 9: UB-180925 (V2031-125)

UBI-180925 (50mg,0.17mmol), UBI- 180925 j(35.7mg,0.17mmol), and acetic acid (10mg) were added to methanol (2mL), the reaction system was stirred for 1 hour, then continued to stirred at 60°C for 3 hours after adding sodium cyanoborohydride(15mg,0.24mmol). After completion of reaction, UBI- 180925 (2.6 mg, 3.6% yield) as a white solid was prepared. LCMS: [M+H] ⁺ = 928.5

### Synthesis method of Compound UB-180933

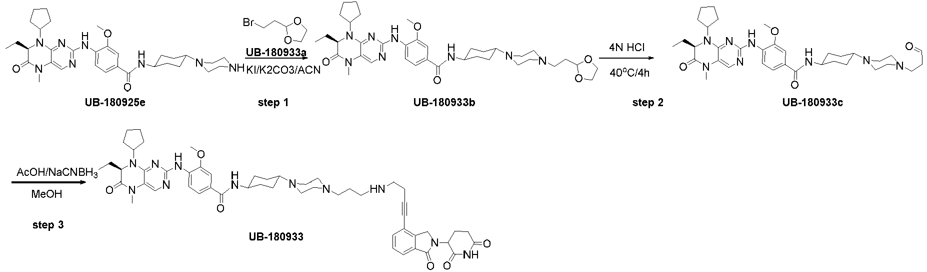

### Step 1: UB-180933b (V2031-124)

UB-180933a (115 mg, 0.635 mmol), potassium iodide (70.3 mg,0.42 mmol), potassium carbonate (117mg,0.85 mmol) were added to acetonitrile (5 mL), and the reaction system was stirred at 80°C for 16 h. After completion of the reaction, water (5 mL) was added, the mixture was extracted with ethyl acetate, dried (sodium sulfate), filtered, the reaction solution was concentrated, and then isolated by silica gel column chromatography (dichloromethane /methanol = 10%) to obtain UB-180933b (150mg, yield 51.3%) as a yellow solid. LCMS: [M+H] ⁺ = 691.4

### Step 2: UB-180933c (V2031-126)

UB-180933b (150 mg, 0.22 mmol) was added to 4N hydrochloric acid in dioxane (5 mL). The reaction system was reacted at 30°C for 4 hours, after completion of the reaction, 10 mL water was added, the mixture was extracted with dichloromethane, dried over sodium sulfate, filtered, concentrated to obtain UB-180933c (113 mg, yield 80.5%). LCMS: [M+H] ⁺ = 647.4

### Step 3: UB-180933 (V2031-133)

UBI- 180925j (50mg,0.45mmol), UBI- 180933c (113mg,0.22mmol), and acetic acid (10mg) were added to methanol (5mL). The system was stirred at room temperature for 1 hour and then sodium cyanoborohydride (56 mg, 0.89 mmol) was added and stirring was continued overnight. After completion of reaction, UBI-180933 (5mg, 3.1% yield) was prepared. LCMS: [M+H] ⁺ = 942.5

¹H NMR (400 MHz, DMSO-*d6*) δ 12.02 (s, 1H), 11.01 (s, 1H), 9.69 (s, 1H), 8.38 (d, J = 7.8 Hz, 1H), 7.82 (t, J = 4.1 Hz, 2H), 7.74 (did, J = 14.9, 7.7, 1.1Hz, 2H), 7.65 - 7.51 (m, 3H), 5.17 (dd, J = 13.3, 5.1 Hz, 1H), 4.63 - 4.29 (m, 3H), 4.17 (p, J = 8.7 Hz, 1H), 3.91 (s, 3H), 3.58 (d, J = 12.0 Hz, 4H), 3.45 (s,2H), 3.31 (s, 1H), 3.21 (s, 3H), 3.10 (s, 2H), 3.01 - 2.86 (m, 1H), 2.63 (s, 1H), 2.19 (d, J = 9.2 Hz, 2H), 2.08 - 1.74 (m, 10H), 1.66 (t, J = 11.9 Hz, 2H), 1.57- 1.34 (m, 6H), 0.75 (t, J = 7.4 Hz, 3H).

As used herein Compound No.UB-18XXXX, can also be simplified to No. XXXX, for example **UB-180925 is Compound 925(0925).**

Other compounds shown in Table A1 were prepared by similar methods.

**Table A1**

| **Compound No.** | **Structure and Data Analysis** |
|---|---|
| **850** | 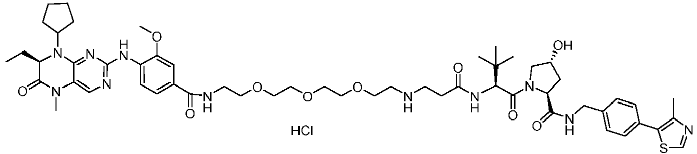 |
| | ¹H NMR (400 MHz, DMSO-*d*₆) δ 9.79 (s, 1H), 9.05 (s, 1H), 8.89 (s, 2H), 8.70 (t, *J* = 5.6 Hz, 1H), 8.62 (t, *J* = 6.1 Hz, 1H), 8.31 (d, *J* = 9.3 Hz, 1H), 7.90 - 7.79 (m, 2H), 7.64 (s, 1H), 7.56 (d, *J* = 8.3 Hz, 1H), 7.41 (q, *J* = 8.2 Hz, 4H), 4.55 (d, *J* = 9.3 Hz, 1H), 4.50 (dd, *J* = 6.4, 3.2 Hz, 1H), 4.48 - 4.39 (m, 2H), 4.36 (s, 1H), 4.24 (d, *J* = 5.5 Hz, 1H), 4.22 - 4.07 (m, 1H), 3.90 (s, 3H), 3.56 (s, 3H), 3.44 (q, *J* = 6.0 Hz, 3H), 3.22 (s, 3H), 3.11 (dp, *J* = 11.4, 6.3, 5.8 Hz, 4H), 2.71 (td, *J* = 7.5, 4.1 Hz, 3H), 2.45 (s, 5H), 2.14 - 1.98 (m, 3H), 1.92 (dtd, *J* = 13.3, 8.6, 7.9, 5.3 Hz, 3H), 1.81 (dt, *J* = 13.6, 7.2 Hz, 3H), 1.57 - 1.38 (m, 5H), 1.24 (d, *J* = 3.5 Hz, 1H), 0.95 (s, 9H), 0.75 (t, *J* = 7.4 Hz, 3H). LCMS [M+H]⁺ =1084 |
| **851** | 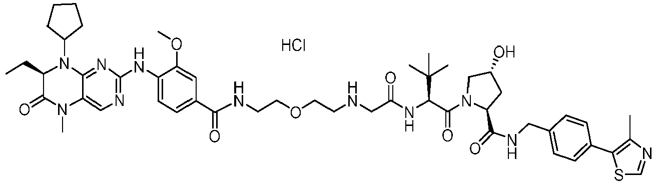 |
| | ¹H NMR (400 MHz, DMSO-*d*₆) δ 9.80 (d, *J* = 12.2 Hz, 1H), 9.12 - 8.97 (m, 3H), 8.80 (d, *J* = 9.0 Hz, 1H), 8.67 (d, *J* = 9.1 Hz, 2H), 7.92 - 7.79 (m, 2H), 7.75 - 7.65 (m, 1H), 7.59 (d, *J* = 8.3 Hz, 1H), 7.41 (q, *J* = 8.4 Hz, 4H), 4.57 (d, *J* = 9.1 Hz, 1H), 4.50 (dd, *J* = 6.4, 3.1 Hz, 1H), 4.48 - 4.40 (m, 2H), 4.38 (s, 1H), 4.24 (d, *J* = 5.5 Hz, 1H), 4.22 - 4.09 (m, 2H), 3.92 (s, 3H), 3.86 (q, *J* = 5.9 Hz, 3H), 3.57 (s, 2H), 3.53 - 3.47 (m, 3H), 3.22 (s, 3H), 3.15 (q, *J* = 5.7 Hz, 2H), 2.45 (s, 3H), 2.07 (dd, *J* = 12.6, 8.2 Hz, 1H), 1.91 (ddd, *J* = 12.8, 8.2, 4.0 Hz, 4H), 1.81 (p, *J* = 7.2 Hz, 3H), 1.57 - 1.36 (m, 4H), 1.24 (d, *J* = 3.8 Hz, 1H), 0.97 (s, 9H), 0.75 (t, *J* = 7.4 Hz, 3H). LCMS [M+H]⁺ =982.6 |
| **852** | 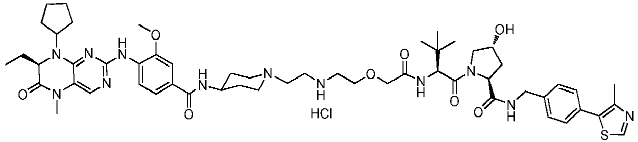 |
| | ¹H NMR (400 MHz, DMSO-*d*₆) δ 9.50 (s, 2H), 8.99 (s, 1H), 8.73 - 8.42 (m, 2H), 7.93 (d, *J* = 8.0 Hz, 1H), 7.87 (s, 1H), 7.80 (d, *J* = 9.3 Hz, 1H), 7.62 (s, 1H), 7.58 (d, *J* = 8.3 Hz, 1H), 7.40 (s, 4H), 4.59 (d, *J* = 9.4 Hz, 1H), 4.51 - 4.32 (m, 4H), 4.31 - 4.15 (m, 3H), 4.10 (d, *J* = 6.0 Hz, 3H), 3.92 (s, 3H), 3.82 (t, *J* = 5.3 Hz, 2H), 3.71 - 3.60 (m, 7H), 3.22 (m, 6H), 2.44 (s, 3H), 2.14 - 1.69 (m, 13H), 1.62 - 1.41 (m, 4H), 0.96 (s, 9H), 0.76 (t, *J* = 7.4 Hz, 3H). LCMS [M+1]⁺ =1066.3 |
| **853** | 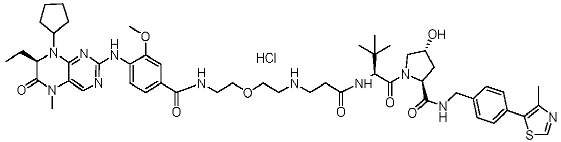 |
| | ¹H NMR (400 MHz, DMSO-*d*₆) δ 9.38 (s, 1H), 8.99 (s, 1H), 8.57 (dq, *J* = 10.7, 6.3 Hz, 3H), 8.31 (d, *J* = 9.2 Hz, 1H), 7.89 (d, *J* = 8.3 Hz, 1H), 7.79 (s, 1H), 7.59 (s, 1H), 7.54 (d, *J* = 7.8 Hz, 1H), 7.40 (q, *J* = 8.1 Hz, 4H), 4.56 (d, *J* = 9.3 Hz, 2H), 4.44 (td, *J* = 9.1, 8.1, 4.7 Hz, 4H), 4.37 (s, 2H), 4.24 (d, *J* = 5.5 Hz, 1H), 4.22 - 4.10 (m, 4H), 3.91 (s, 3H), 3.22 (s, 3H), 3.15 (dq, *J* = 10.9, 5.9 Hz, 5H), 2.67 (q, *J* = 7.3 Hz, 3H), 2.50 (d, *J* = 2.2 Hz, 10H), 2.44 (s, 3H), 2.05 (dd, *J* = 12.8, 7.9 Hz, 2H), 1.92 (ddt, *J* = 13.2, 9.1, 4.8 Hz, 5H), 1.78 (dt, *J* = 14.3, 7.2 Hz, 3H), 1.61 - 1.35 (m, 5H), 1.24 (d, *J* = 3.6 Hz, 2H), 0.95 (s, 9H), 0.75 (t, *J* = 7.4 Hz, 3H). LCMS [M+H]⁺ =996.7 |
| **854** | 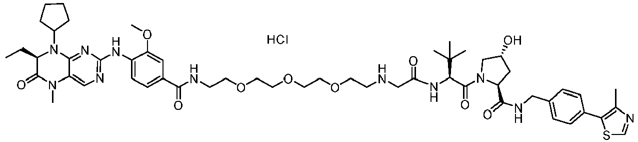 |
| | ¹H NMR (400 MHz, DMSO-*d*₆) δ 9.74 (s, 1H), 9.01 (s, 1H), 8.91 (t, *J* = 6.3 Hz, 2H), 8.65 (dt, *J* = 16.7, 6.3 Hz, 3H), 7.84 (d, *J* = 9.7 Hz, 2H), 7.63 (d, *J* = 1.9 Hz, 1H), 7.55 (dd, *J* = 8.3, 1.8 Hz, 1H), 7.40 (q, *J* = 8.2 Hz, 4H), 4.58 (d, *J* = 9.2 Hz, 1H), 4.50 (dd, *J* = 6.5, 3.2 Hz, 1H), 4.48 - 4.41 (m, 2H), 4.38 (s, 1H), 4.24 (d, *J* = 5.5 Hz, 1H), 4.21 - 4.07 (m, 2H), 3.90 (s, 3H), 3.84 (q, *J* = 5.7 Hz, 3H), 3.72 (d, *J* = 4.0 Hz, 1H), 3.67 (t, *J* = 5.3 Hz, 5H), 3.55 (s, 13H), 3.22 (s, 3H), 3.12 (t, *J* = 5.7 Hz, 2H), 2.44 (s, 3H), 2.08 - 2.02 (m, 1H), 1.92 (tq, *J* = 9.4, 5.2 Hz, 5H), 1.81 (p, *J* = 6.9 Hz, 3H), 1.60 - 1.37 (m, 5H), 1.24 (d, *J* = 3.3 Hz, 2H), 0.97 (s, 9H), 0.75 (t, *J* = 7.4 Hz, 3H). LCMS [M+H]⁺ =1072 |
| **855** | 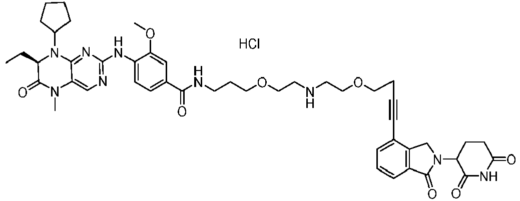 |
| | ¹H NMR (400 MHz, DMSO-*d*₆) δ 11.01 (s, 1H), 8.74 (s, 2H), 8.58 (s, 2H), 7.81 (s, 1H), 7.72 (d, *J* = 7.5 Hz, 1H), 7.65 - 7.61 (m, 1H), 7.59 (s, 1H), 7.52 (t, *J* = 7.9 Hz, 2H), 5.16 (dd, *J* = 13.2, 5.0 Hz, 1H), 4.45 (d, *J* = 17.8 Hz, 2H), 4.31 (d, *J* = 17.6 Hz, 1H), 3.92 (d, *J* = 1.4 Hz, 3H), 3.78 (t, *J* = 5.2 Hz, 2H), 3.69 (t, *J* = 6.6 Hz, 2H), 3.64 (t, *J* = 5.0 Hz, 2H), 3.46 (d, *J* = 6.2 Hz, 2H), 3.23 (s, 3H), 2.78 (t, *J* = 6.6 Hz, 2H), 2.64 (d, *J* = 22.5 Hz, 1H), 2.37 - 2.27 (m, 1H), 1.93 (s, 4H), 1.79 (dd, *J* = 13.4, 6.7 Hz, 6H), 1.55 (d, *J* = 40.5 Hz, 5H), 1.37 (s, 4H), 1.24 (s, 1H), 0.75 (t, *J* = 7.4 Hz, 3H). LCMS [M+H]⁺ =795.58 |
| **856** | 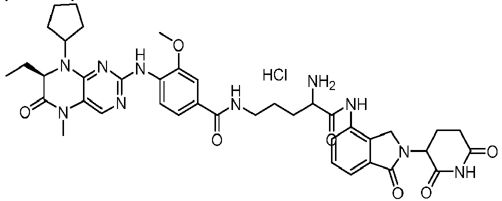 |
| | ¹H NMR (400 MHz, DMSO-*d*₆) δ 11.02 (d, *J* = 3.3 Hz, 1H), 10.87 (d, *J* = 26.0 Hz, 1H), 8.77 (t, *J* = 9.1 Hz, 1H), 8.42 (s, 3H), 8.02 - 7.73 (m, 3H), 7.70 - 7.43 (m, 4H), 5.16 (dd, *J* = 13.2, 5.8 Hz, 1H), 4.54 - 4.30 (m, 3H), 4.18 (dd, *J* = 21.9, 12.6 Hz, 2H), 3.89 (d, *J* = 1.9 Hz, 3H), 3.55 - 3.42 (m, 1H), 3.22 (s, 4H), 2.96 - 2.86 (m, 1H), 2.63 (d, *J* = 31.1 Hz, 1H), 2.28 (p, *J* = 10.1, 9.4 Hz, 1H), 2.07 - 1.63 (m, 12H), 1.48 (d, *J* = 35.1 Hz, 4H), 1.24 (s, 2H), 0.75 (t, *J* = 7.4 Hz, 3H). LCMS [M+H]⁺ =781.56 |
| **857** | 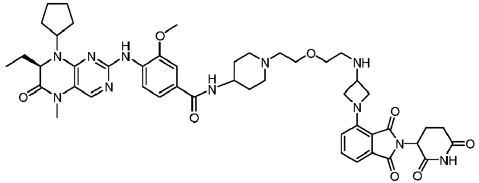 |
| | ¹H NMR (400 MHz, DMSO-d₆)δ 11.03 (s, 1H), 8.41 (d, *J* = 8.9 Hz, 1H), 8.04 (d, *J* = 7.7 Hz, 1H), 7.85 (s, 1H), 6.78- 7.75 (m, 6H), 5.16 (dd, *J* = 13.0, 4.8 Hz, 1H),4.39-4.12 (m, 5H), 3.92 (s, 3H), 3.77-3.53(m, 9H),3.24(s, 3H), 2.92 - 2.61 (m, 10H), 2.24 - 1.93 (m, 3H), 1.91-1.79 (m, 12H), 0.76 (t, *J* = 7.4 Hz, 3H). LCMS [M+H]⁺ =906.1 |
| **858** | 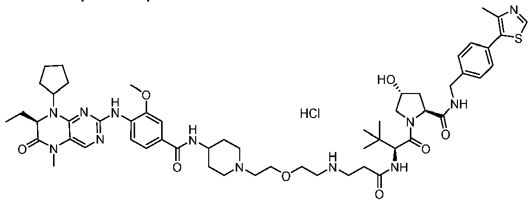 |
| | ¹H NMR (400 MHz, DMSO-*d*₆) δ 10.57 (s, 1H), 9.73 (s, 1H), 9.41 - 9.14 (m, 2H), 9.00 (d, *J* = 3.6 Hz, 1H), 8.70 - 8.55 (m, 2H), 8.30 (d, *J* = 9.4 Hz, 1H), 7.93 - 7.80 (m, 2H), 7.64 (s, 1H), 7.59 (d, *J* = 8.4 Hz, 1H), 7.45 - 7.35 (m, 4H), 4.56 - 4.51 (m, 1H), 4.50 - 4.40 (m, 3H), 4.38 - 4.33 (m, 1H), 4.24 - 4.15 (m, 2H), 4.10 - 4.02 (m, 1H), 3.93 (d, *J* = 15.6 Hz, 3H), 3.84 - 3.80 (m, 2H), 3.76 - 3.73 (m, 2H), 3.65 - 3.63 (m, 2H), 3.31 - 3.27 (m, 2H), 3.22 (s, 3H), 3.18 - 3.08 (m, 6H), 2.85 - 2.76 (m, 2H), 2.44 (d, *J* = 5.2 Hz, 3H), 2.26 - 2.11 (m, 2H), 2.09 - 1.76 (m, 10H), 1.63 - 1.36 (m, 4H), 0.95 (d, *J* = 4.8 Hz, 9H), 0.75 (t, *J* = 7.6 Hz, 3H). |
| **859** | 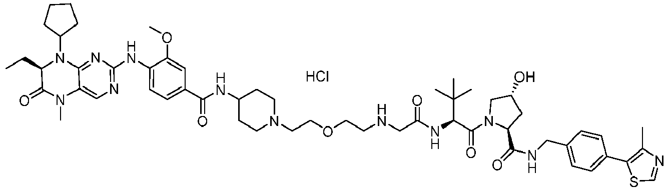 |
| | ¹H NMR (400 MHz, DMSO-*d*₆) δ 8.98 (s, 1H), 8.58 (t, *J* = 6.0 Hz, 1H), 8.41 (d, *J* = 8.8 Hz, 1H), 8.19 (s, 2H), 8.07 (d, *J* = 7.6 Hz, 1H), 8.01 (d, *J* = 9.6 Hz, 1H), 7.84 (s, 1H), 7.59 (s, 1H), 7.52 - 7.45 (m, 2H), 7.46 - 7.34 (m, 4H), 4.54 (d, *J* = 9.6 Hz, 1H), 4.45 - 4.40 (m, 1H), 4.39 - 4.33 (m, 2H), 4.29 - 4.21 (m, 2H), 3.93 (s, 3H), 3.77 - 3.73 (m, 1H), 3.70 - 3.66 (m, 1H), 3.63 - 3.59 (m, 1H), 3.55 - 3.42 (m, 6H), 3.25 (s, 3H), 3.20 (d, *J* = 8.8 Hz, 2H), 2.95 - 2.87 (m, 2H), 2.73 - 2.61 (m, 2H), 2.44 (s, 3H), 2.14 - 1.97 (m, 4H), 1.95 - 1.84 (m, 3H), 1.83 - 1.70 (m, 6H), 1.65 - 1.53 (m, 4H), 0.95 (s, 9H), 0.76 (t, *J* = 7.6 Hz, 3H). |
| **860** | 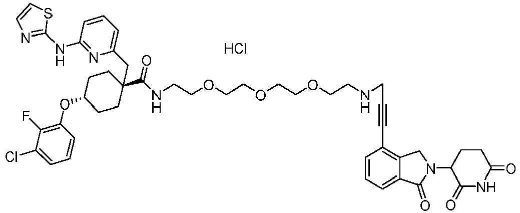 |
| | ¹H NMR (400 MHz, DMSO-*d*₆) δ 11.22 (s, 1H), 11.05 (s, 1H), 9.50 (d, *J* = 47.6 Hz, 2H), 8.65 (d, *J* = 86.6 Hz, 2H), 7.78 (dd, *J* = 32.1, 7.6 Hz, 1H), 7.68 - 7.48 (m, 2H), 7.44 - 7.27 (m, 2H), 7.19 - 7.06 (m, 2H), 7.01 - 6.84 (m, 1H), 6.64 (d, *J* = 7.3 Hz, 1H), 5.19 (dd, *J* = 13.4, 5.1 Hz, 1H), 4.54 (d, *J* = 17.4 Hz, 1H), 4.37 (d, *J* = 18.0 Hz, 1H), 4.22 (s, 1H), 3.72 (t, *J* = 5.1 Hz, 2H), 3.59 (dt, *J* = 13.5, 3.6 Hz, 3H), 3.53 - 3.46 (m, 3H), 3.09 (td, *J* = 7.3, 4.8 Hz, 6H), 2.91 (s, 2H), 2.09 - 1.94 (m, 2H), 1.77 (t, *J* = 11.5 Hz, 2H), 1.65 (d, *J* = 12.3 Hz, 2H), 1.46 (s, 2H), 1.30 - 1.23 (m, 10H), 0.97 (s, 1H), 0.85 (t, *J* = 6.3 Hz, 2H). LCMS [M+H]⁺ =916.5 |
| **861** | 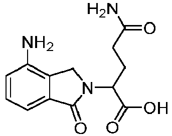 |
| | ¹H NMR (400 MHz, DMSO-*d*₆) δ 7.26 (s, 1H), 7.18 (t, *J* = 7.6 Hz, 1H), 6.90 (d, *J* = 7.4 Hz, 1H), 6.80 (d, *J* = 7.9 Hz, 1H), 6.75 (s, 1H), 4.74 (dd, *J* = 11.1, 4.5 Hz, 1H), 4.34 - 4.15 (m, 4H), 2.37 - 2.18 (m, 1H), 2.12 - 1.87 (m, 3H). LCMS [M+H]⁺ =278.2 |
| **862** | 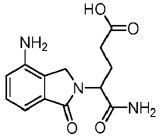 |
| | ¹H NMR (400 MHz, DMSO-*d*₆) δ 7.55 (s, 1H), 7.25 - 7.10 (m, 2H), 6.93 (d, *J* = 7.4 Hz, 1H), 6.81 (d, *J* = 7.9 Hz, 1H), 4.73 (dd, *J* = 10.5, 4.1 Hz, 1H), 4.44 (d, *J* = 17.5 Hz, 1H), 4.21 (d, *J* = 17.5 Hz, 2H), 3.99 (s, 2H), 2.26 - 2.07 (m, 3H), 2.06 - 1.86 (m, 1H). LCMS [M+H]⁺ =278.2 |
| **863** | 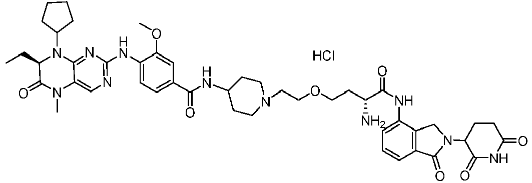 |
| | ¹H NMR (400 MHz, DMSO-d6) δ 11.02 (m, J = 15.3 Hz, 2H), 10.58 (brm, 1H), 8.51 (m, 4H), 7.94 (brm, 1H), 7.83 (m, 2H), 7.58 (m, 4H), 5.17 (m, 1H), 4.52 (m, 1H), 4.40 (m, 3H), 4.11 (m, 3H), 3.93 (s, 3H), 3.80 (m, 2H), 3.69 - 3.53 (m, 4H), 3.28 (m, 2H), 3.23 (s, 3H), 3.09 (d, J = 11.2 Hz, 2H), 2.93 (m, 1H), 2.64 (m, 1H), 2.34 - 2.20 (m, 2H), 2.16 - 1.92 (m, 8H), 1.91 - 1.76 (m, 4H), 1.52 (m, 4H), 0.76 (t, J = 7.4 Hz, 3H). LCMS [M+H]⁺ = 894.7 |
| **864** | 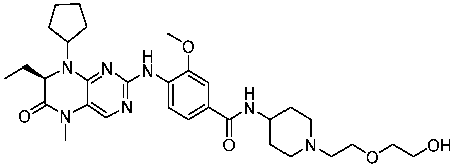 |
| | ¹H NMR (400 MHz, DMSO-*d*₆) δ 9.25 (s, 1H), 8.38 (d, *J* = 7.5 Hz, 1H), 8.14 (s, 1H), 7.80 (d, *J* =4.0 Hz, 1H), 7.62 - 7.44 (m, 2H), 4.37 (s, 1H), 4.27 (s, 1H), 3.93 (d, *J* = 3.1 Hz, 3H), 3.77 (t, *J* = 4.9 Hz, 3H), 3.55 (d, *J* = 4.7 Hz, 4H), 3.29 (s, 3H), 3.24 (s, 3H), 3.16 (t, *J* = 11.7 Hz, 3H), 2.12 - 1.46 (m, 16H), 0.76 (t, *J* = 7.4 Hz, 3H). LCMS [M+H]⁺ =956.3 |
| **865** | 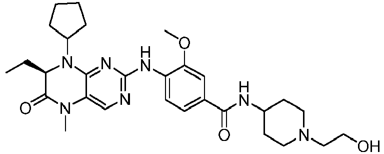 |
| | ¹H NMR (400 MHz, DMSO-*d*₆) δ 8.50 - 8.35 (m, 1H), 8.19 (dd, *J* = 40.3, 7.8 Hz, 1H), 7.85 (s, 1H), 7.60 (s, 1H), 7.53 - 7.41 (m, 2H), 4.35 (q, *J* = 8.3 Hz, 1H), 4.24 (dd, *J* = 7.6, 3.6 Hz, 1H), 3.94 (s, 3H), 3.83 (d, *J* = 6.8 Hz, 1H), 3.56 (t, *J* = 6.1 Hz, 2H), 3.25 (s, 3H), 3.07 (d, *J* = 11.3 Hz, 2H), 2.64 (d, *J* = 28.2 Hz, 3H), 2.33 (dt, *J* = 3.7, 2.0 Hz, 2H), 2.10 - 1.47 (m, 14H), 0.76 (t, *J* = 7.4 Hz, 3H). LCMS [M+H]⁺ =552.3 |
| **866** | 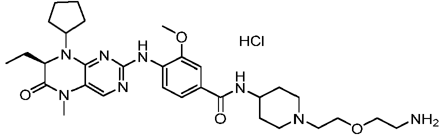 |
| | ¹H NMR (400 MHz, DMSO-*d*₆) δ 10.56 (d, *J* = 90.5 Hz, 1H), 9.73 (s, 1H), 8.67 (dd, *J* = 33.7, 7.1 Hz, 1H), 8.36 (s, 2H), 8.03 - 7.78 (m, 2H), 7.74 - 7.43 (m, 2H), 4.49 (dd, *J* = 6.5, 3.3 Hz, 1H), 4.26 - 4.12 (m, 1H), 4.11 - 3.99 (m, 1H), 3.94 (d, *J* = 14.9 Hz, 3H), 3.82 (t, *J* = 4.9 Hz, 2H), 3.68 (dd, *J* = 10.4, 5.4 Hz, 3H), 3.64 - 3.55 (m, 3H), 3.28 (q, *J* = 5.1 Hz, 2H), 3.18 - 3.06 (m, 2H), 3.01 (q, *J* = 5.3 Hz, 2H), 2.18 (d, *J* = 12.7 Hz, 2H), 2.07 - 1.73 (m, 8H), 1.60 - 1.40 (m, 4H), 0.76 (t, *J* = 7.4 Hz, 3H). LCMS [M+H]⁺ =595.4 |
| **867** | 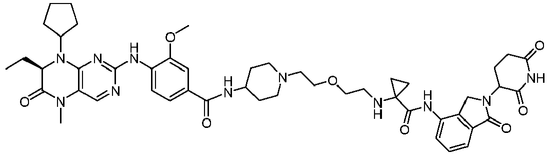 |
| | ¹H NMR (400 MHz, DMSO-*d*₆) 11.01 (s, 1H), 10.10 (s, 1H), 8.41 (*d, J* = 8.9 Hz, 1H), 7.98 - 7.81 (m, 2H), 7.57 (d, *J* = 20.9 Hz, 1H), 7.55 - 7.39 (m, 5H), 5.14 (dd, *J* = 13.2, 5.1 Hz, 1H), 4.52 - 3.99 (m, 4H), 3.94 (s, 3H), 3.29 - 3.16 (m, 4H), 3.24 (s, 3H), 2.96 - 2.78 (m, 2H), 2.96 - 2.70 (m, 4H), 2.84 (dt, *J* = 8.4, 10.6 Hz, 2H), 1.90 (d, *J* = 7.4 Hz, 12H), 1.77 (d, *J* = 10.5 Hz, 2H), 1.29 (d, *J* = 6.8 Hz, 4H), 1.18 (s, 2H), 0.74 (t, *J* = 7.4 Hz, 3H). |
| | LCMS [M+H]⁺ =920.6 |
| **868** | 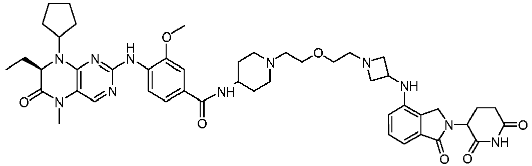 |
| | ¹H NMR (400 MHz, DMSO-d₆)δ 11.03 (s, 1H), 10.33 - 10.12 (m, 2H), 9.14 (s, 1H), 8.73 (s, 1H), 8.41 (d, *J* = 8.9 Hz, 1H),7.63-6.15 (m, 7H), 5.14 (dd, *J* = 13.2, 5.1 Hz, 1H), 4.54 (d, *J* = 5.5 Hz, 2H), 4.42 - 4.12 (m, 8H), 3.94 (s, 3H), 3.29 - 3.16 (m, 4H), 3.24 (s, 3H), 3.30 - 3.00 (m, 4H), 2.96 - 2.55 (m, 4H), 2.46 - 2.39 (m, 2H), 2.05 (s, 4H), 1.85 (s, 6H), 1.75 (d, *J* = 9.1 Hz, 3H), 0.76 (t, *J* = 7.3 Hz, 3H). |
| | LCMS [M+H]⁺ =892.7 |
| **869** | 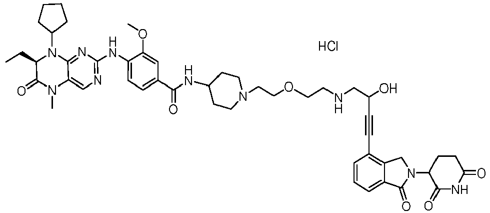 |
| | ¹H NMR (400 MHz, DMSO-d6) δ 11.03 (s, 1H), 10.49 (s, 1H), 9.40 (s, 1H), 9.14 (s, 1H), 8.59 (d, J = 7.1 Hz, 1H), 7.91 (s, 1H), 7.85 (s, 1H), 7.76 (m, 2H), 7.67 - 7.52 (m, 3H), 6.54 (s, 1H), 5.22 - 5.10 (m, 2H), 4.58 - 4.45 (m, 2H), 4.37 (d, J = 17.9 Hz, 1H), 4.19 (m, 1H), 4.04 (m, 1H), 3.93 (d, J = 11.8 Hz, 3H), 3.86 - 3.73 (m, 4H), 3.60 (m, 2H), 3.26 (m, 4H), 3.22 (s, 3H), 3.12 (m, 2H), 2.98 - 2.89 (m, 1H), 2.61 (m, 1H), 2.46 - 2.40 (m, 1H), 2.16 (m, 2H), 2.09 - 1.70 (m, 10H), 1.51 (m, 4H), 0.76 (t, J = 7.4 Hz, 3H). LCMS [M+H]⁺ = 905.6 |
| **870** | 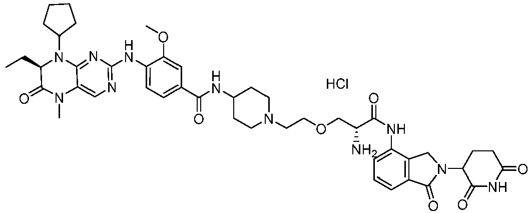 |
| | 1H NMR (400 MHz, DMSO-d6) δ 11.05 (s, 1H), 10.92 (d, J = 12.7 Hz, 1H), 10.57 (s, 1H), 8.65 (s, 3H), 8.50 (d, J = 7.4 Hz, 1H), 7.90 - 7.82 (m, 2H), 7.57 (m, 4H), 5.17 (m, 1H), 4.50 (m, 2H), 4.43 - 4.34 (m, 2H), 4.28 (m, 1H), 4.03 (m, 2H), 3.92 (m, 5H), 3.58 (m, 2H), 3.24 (s, 3H), 3.12 (m, 2H), 2.94 (m, 2H), 2.69 - 2.64 (m, 1H), 2.62 (m, 1H), 2.35 - 2.30 (m, 1H), 2.15 - 1.79 (m, 12H), 1.66 (m, 2H), 1.54 (m, 2H), 0.76 (t, J = 7.4 Hz, 3H). LCMS [M+H]⁺ = 880.5 |
| **871** | 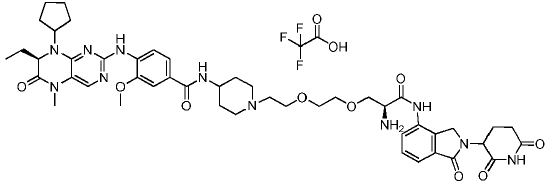 |
| | ¹H NMR (400 MHz, DMSO-*d*₆) δ 11.06 (s, 1H), 10.58 (s, 1H), 9.76 (s, 1H), 8.41 (s, 3H), 8.11 (d, *J* = 25.8 Hz, 2H), 7.87 - 7.79 (m, 2H), 7.61 - 7.42 (m, 4H), 5.20 (dt, *J* = 13.3, 5.1 Hz, 1H), 4.54 - 4.15 (m,6H), 4.00 (s, 1H), 3.93 (s, 3H), 3.91 (d, *J* = 4.8 Hz, 1H), 3.75 (s, 2H), 3.66 (t, *J* = 4.0 Hz, 2H), 3.61 (s, 2H), 3.24 (s, 3H), 3.10 (s, 2H), 2.95 (t, *J* = 13.3 Hz, 2H), 2.68 - 2.58 (m, 2H), 2.34 (s, 1H), 2.09 - 1.92 (m, 6H), 1.83 (d, *J* = 14.9 Hz, 6H), 1.64 (s, 2H), 1.52 (m, 3H), 0.76 (t, *J* = 7.4 Hz, 3H). LCMS [M+1]⁺ =924.6 |
| **872** | 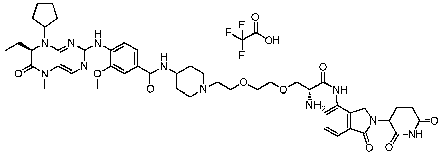 |
| | ¹H NMR (400 MHz, ) δ 11.05 (d, *J* = 8.4 Hz, 1H), 10.58 (d, *J* = 5.7 Hz, 1H), 9.78 (s, 1H), 8.42 (s, 3H), 8.15 (s, 2H), 7.84 (d, *J* = 7.1 Hz, 2H), 7.71 - 7.38 (m, 4H), 5.20 (dt, *J* = 13.2, 5.1 Hz, 1H), 4.53 - 4.16 (m, 6H), 4.00 (s, 1H), 3.93 (s, 3H), 3.91 (d, *J* = 4.8 Hz, 1H), 3.75 (s, 2H), 3.66 (t, *J* = 4.0 Hz, 2H), 3.62 (d, *J* = 4.7 Hz, 2H), 3.24 (s, 3H), 3.10 (s, 2H), 2.99 - 2.90 (m, 2H), 2.71 - 2.59 (m, 2H), 2.33 - 2.20 (m, 1H), 2.15 - 1.93 (m, 6H), 1.89 - 1.74 (m, 6H), 1.64 (s, 2H), 1.57 - 1.42 (m, 3H), 0.76 (t, *J* = 7.4 Hz, 3H). LCMS [M+1]⁺ =924.6 |
| **873** | 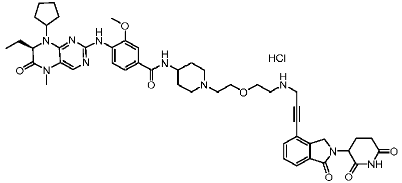 |
| | ¹H NMR (400 MHz, DMSO-d6) δ 11.04 (s, 1H), 10.57 (s, 1H), 10.05 (s, 1H), 9.65 - 9.25 (m, 1H), 8.68 - 8.52 (m, 1H), 7.98 - 7.55 (m, 7H), 5.24 - 5.12 (m, 1H), 4.64 (d, J = 18.2 Hz, 1H), 4.54 - 4.40 (m, 2H), 4.29 - 4.14 (m, 3H), 4.09 - 3.89 (m, 6H), 3.82 (d, J = 8.1 Hz, 4H), 3.69 - 3.58 (m, 2H), 3.33 - 3.26 (m, 3H), 3.22 (s, 3H), 3.18 - 3.09 (m, 2H), 2.99 - 2.86 (m, 1H), 2.71 - 2.59 (m, 2H), 2.24 - 2.13 (m, 2H), 2.11 - 1.77 (m, 11H), 1.65 - 1.42 (m, 4H), 0.76 (t, J = 7.4 Hz,3H). LCMS [M+H]⁺ =875.6 |
| **874** | 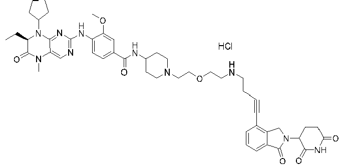 |
| | ¹H NMR (400 MHz, DMSO-*d*₆) δ 11.01 (s, 1H), 10.41 (s, 1H), 9.50 (s, 2H), 8.56 (d, *J* = 7.0 Hz, 1H), 7.97 (s, 1H), 7.83 (s, 1H), 7.78 - 7.66 (m, 2H), 7.64 - 7.49 (m, 3H), 5.24 - 5.10 (m, 1H), 4.58 - 4.29 (m, 3H), 4.04 (s, 1H), 3.99 - 3.86 (m, 1H), 3.84 - 3.75 (m, 4H), 3.66 - 3.58 (m, 4H), 3.22 (s, 4H), 3.17 - 3.03 (m, 6H), 2.71 - 2.58 (m, 4H), 2.22 - 2.11 (m, 2H), 2.07 - 1.79 (m, 10H), 1.68 - 1.47 (m, 4H), 0.76 (t, *J* = 7.4 Hz, 3H).LCMS [M+H]⁺ =889.6 |
| **875** | 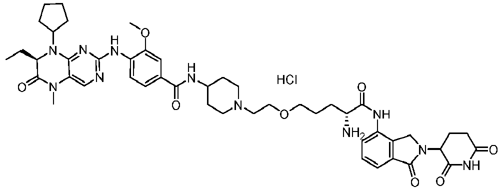 |
| | ¹H NMR (400 MHz, DMSO-*d*₆) δ 11.05 (s, 1H), 10.55 (s, 1H), 9.69 (s, 3H), 8.37 (s, 1H), 8.21 (s, 1H), 7.88 (d, J = 7.6 Hz, 1H), 7.81 (s, 1H), 7.60 (t, J = 5.8 Hz, 2H), 7.51 (d, J = 7.2 Hz, 2H), 5.32 (t, J = 4.8 Hz, 1H), 5.19 (dd, J = 13.2, 5.2 Hz, 1H), 4.47 - 4.32 (m, 4H), 4.27 (d, J = 8.8 Hz, 3H), 4.02 (s, 2H), 3.93 (s, 3H), 3.79 (s, 5H), 3.24 (s, 3H), 2.66 (dt, J = 7.5, 2.5 Hz, 2H), 2.61 (s, 1H), 2.00 (dq, J = 13.1, 7.8, 6.2 Hz, 7H), 1.90 - 1.78 (m, 6H), 1.69 (dd, J = 14.4, 7.1 Hz, 3H), 1.53 (t, J = 6.3 Hz, 2H), 1.46 (d, J = 7.1 Hz, 2H), 0.81 (dt, J = 36.9, 7.0 Hz, 6H). LCMS [M+H]⁺ = 992 |
| **876** | 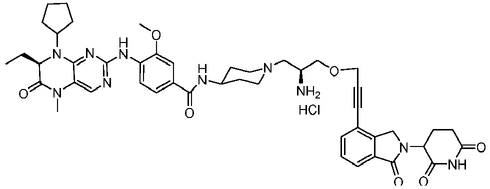 |
| | NMR: None.LCMS [M+H]⁺ =861.6 |
| **877** | 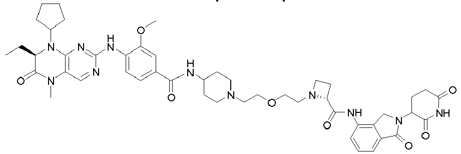 |
| | ¹H NMR (400 MHz, DMSO-d₆)δ 11.03 (s, 1H), 9.14 (s, 1H), 8.41 (d, *J=* 8.9 Hz, 1H), 7.94 - 7.85 (m, 7H), 5.14 (dd, *J* = 13.2, 5.1 Hz, 1H), 4.46 - 4.25 (m, 4H), 3.94 (s, 3H), 3.29 - 3.16 (m, 4H), 3.25(s, 3H), 3.27 - 2.72 (m, 8H), 3.03 - 2.70 (m, 3H), 2.61 (dd, *J* = 8.1, 13.3 Hz, 2H), 2.35 (d, *J* = 10.7 Hz, 4H), 2.06 (dd, *J* = 11.0, 9.2Hz, 5H), 1.91 - 1.58 (m, 14H), 0.99 - 0.76 (m, 1H), 0.75 (s, 3H). |
| | LCMS [M+H]⁺ =920.6 |
| **878** | 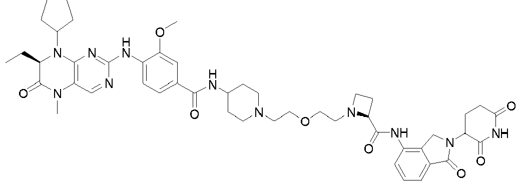 |
| | ¹H NMR (400 MHz, DMSO-*d*₆) δ11.02 (s, 1H), 9.14 (s, 1H), 8.41 (d, *J*= = 8.9 Hz, 1H), 8.04 (d, *J* = 7.7 Hz, 1H), 7.85 (s, 1H), 7.72-7.55 (m, 6H), 5.16 (dd, *J* = 13.0, 4.8 Hz, 1H), 4.45 (d, *J* = 9.0 Hz, 1H), 4.29 (ddd, *J* = 14.0, 11.2, 5.9 Hz, 3H), 3.94 (s, 3H), 3.29 - 3.16 (m, 4H), 3.25(s, 3H), 2.35 - 2.01 (m, 1H), 1.94 - 1.54 (m, 18H), 0.99 - 0.76 (m, 1H), 0.75 (s, 3H). LCMS [M+H]⁺ =920.6 |
| **879** | 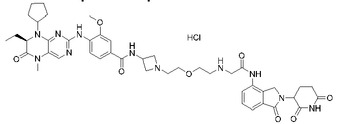 |
| | ¹H NMR (400 MHz, DMSO-*d₆*) δ 11.03 (s, 1H), 10.73 (m, 2H), 9.67 (m, 1H), 9.34 (m, 2H), 7.99 (m, 1H), 7.89 - 7.68 (m, 3H), 7.59 (m, 3H), 5.16 (m, 1H), 4.92 - 4.80 (m, 1H), 4.44 (m, 7H), 4.26 - 4.09 (m, 3H), 3.92 (d, J = 7.2 Hz, 3H), 3.73 (m, 4H), 3.61 - 3.53 (m, 2H), 3.29 (m, 2H), 3.22 (s, 3H), 2.94 (m, 1H), 2.62 (m, 1H), 2.35 - 2.25 (m, 1H), 2.05 (m, 1H), 2.01 - 1.74 (m, 6H), 1.62 - 1.41 (m, 4H), 0.76 (t, J = 7.4 Hz, 3H). LCMS [M+H]⁺ = 866 |
| **880** | 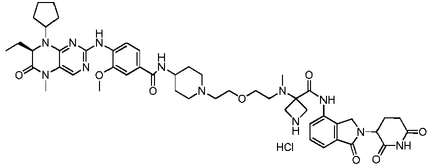 |
| | LCMS [M+1]⁺ =950.4 |
| **881** | 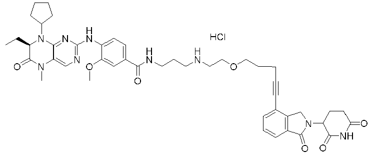 |
| | ¹H NMR (400 MHz, DMSO-*d*₆) δ 11.00 (s, 1H), 9.60 (s, 1H), 8.94 (s, 2H), 8.87 (t, *J* = 5.7 Hz, 1H), 7.86 (d, *J* = 10.7 Hz, 2H), 7.75 - 7.49 (m, 5H), 5.15 (m, 1H), 4.51 - 4.41 (m, 2H), 4.31 (d, *J* = 17.7 Hz, 1H), 4.16 (m, 1H), 3.91 (s, 3H), 3.70 (m, 2H), 3.58 (m, 2H), 3.40 - 3.33 (m, 2H), 3.22 (s, 3H), 3.12 (m, 2H), 3.03 - 2.86 (m, 3H), 2.58 (m, 3H), 2.42 (m, 1H), 2.06 - 1.72 (m, 11H), 1.49 (m, 4H), 0.75 (t, *J* = 7.4 Hz, 3H). LCMS [M+H]⁺ = 834 |
| **882** | 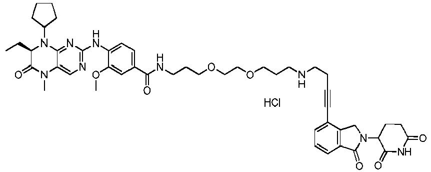 |
| | ¹H NMR (400 MHz, DMSO-*d*₆) δ 11.01 (s, 1H), 9.60 (s, 1H), 9.21 (s, 2H), 8.59 (d, *J* = 5.9 Hz, 1H), 7.88 - 7.79 (m, 2H), 7.74 (d, *J* = 7.4 Hz, 1H), 7.68 (dd, *J* = 7.6, 1.1 Hz, 1H), 7.61 (d, *J* = 1.9 Hz, 1H), 7.54 (ddd, *J* = 7.6, 4.4, 2.5 Hz, 2H), 5.16 (dd, *J* = 13.3, 5.1 Hz, 1H), 4.66 - 4.43 (m, 2H), 4.34 (d, *J* = 17.9 Hz, 1H), 4.20 - 4.10 (m, 1H), 3.90 (s, 3H), 3.49 - 3.41 (m, 5H), 3.33 (q, *J* = 6.7 Hz, 2H), 3.22 (s, 5H), 3.09 - 2.91 (m, 6H), 2.82 (d, *J* = 7.6 Hz, 1H), 2.60 (d, *J* = 17.4 Hz, 1H), 2.46 - 2.38 (m, 1H), 2.14 - 1.69 (m, 12H), 1.48 (d, *J* = 26.4 Hz, 4H), 0.75 (t, *J* = 7.4 Hz, 3H). LCMS [M+1]⁺ =879.3 |
| **883** | 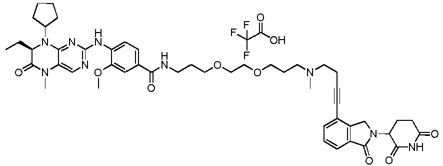 |
| | ¹H NMR (400 MHz, DMSO-*d*6) δ 11.03 (s, 1H), 9.41 (s, 1H), 8.41 (s, 1H), 8.11 (s, 1H), 7.79 (s, 1H), 7.75 (d, *J* = 7.5 Hz, 1H), 7.66 (d, *J* = 7.7 Hz, 1H), 7.59 - 7.45 (m, 4H), 5.17 (dd, *J* = 13.5, 5.0 Hz, 1H), 4.46 (d, *J* = 17.6 Hz, 1H), 4.27 (d, *J* = 25.4 Hz, 2H), 3.91 (s, 3H), 3.50 (dd, *J* = 6.1, 2.0 Hz, 7H), 3.23 (s, 3H), 3.02 - 2.94 (m, 3H), 2.86 (s, 3H), 2.79 - 2.74 (m, 1H), 2.63 (s, 1H), 2.06 - 1.73 (m, 15H), 1.64 (s, 2H), 1.52 (s, 2H), 0.76 (t, *J* = 7.5 Hz, 3H). LCMS [M+1]⁺ =892.6 |
| **884** | 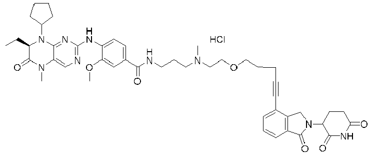 |
| | ¹H NMR (400 MHz, DMSO-*d*₆) δ 11.00 (s, 1H), 10.46 (s, 1H), 9.73 (s, 1H), 8.89 (m, 1H), 7.86 (m, 2H), 7.61 (m, 5H), 5.15 (m, 1H), 4.54 - 4.40 (m, 2H), 4.32 (m, 1H), 4.17 (m, 1H), 3.91 (s, 3H), 3.79 (m, 2H), 3.62 - 3.53 (m, 5H), 3.22 (m, 5H), 3.16 - 3.00 (m, 2H), 2.96 - 2.87 (m, 1H), 2.78 (m, 3H), 2.59 (m, 2H), 2.46 - 2.32 (m, 1H), 2.08 - 1.74 (m, 11H), 1.60 - 1.39 (m, 4H), 0.75 (t, *J* = 7.5 Hz, 3H). LCMS [M+H]⁺ = 848 |
| **885** | 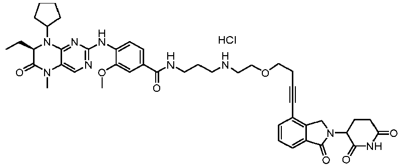 |
| | ¹H NMR (400 MHz, DMSO-*d6*) δ 11.00 (s, 1H), 9.04 (s, 2H), 8.86 (s, 1H), 7.96 (s, 1H), 7.85 (s, 1H), 7.74 - 7.45 (m, 5H), 5.15 (dd, J = 13.3, 5.2 Hz, 1H),4.45 (d, J = 16.2 Hz, 2H), 4.35 - 4.27 (m, 1H), 4.19 (s, 1H), 3.92 (s, 3H), 3.78 (t, J = 5.2 Hz, 2H), 3.68 (t, J = 6.7 Hz, 2H), 3.22 (s, 3H), 3.13 (s, 2H), 2.98(s, 2H), 2.77 (t, J = 6.7 Hz, 2H), 2.60 (d, J = 15.5 Hz, 1H), 1.93 (d, J = 7.3 Hz, 8H), 1.53 (d, J = 32.8 Hz, 4H), 0.75 (t, J = 7.4 Hz, 3H).LCMS [M+H]⁺ =820.5 |
| **886** | 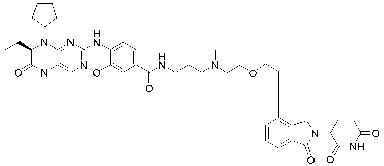 |
| | ¹H NMR (400 MHz, DMSO-*d6*) δ 10.90 (s, 1H), 8.32 (dd, J = 24.0, 6.8 Hz, 2H), 7.70 (s, 1H), 7.58 (d, J = 7.5 Hz, 1H), 7.49 (d, J = 7.0 Hz, 2H), 7.42 - 7.32 (m, 3H), 5.02 (dd, J = 13.3, 5.2 Hz, 1H), 4.31 (dd, J = 17.9, 3.1 Hz, 1H), 4.22 (d, J = 12.7 Hz, 1H), 4.12 (dd, J = 7.6, 3.6 Hz, 2H), 3.82 (s, 4H),3.48 (s, 5H), 3.13 (s, 3H), 2.56 (t, J = 6.7 Hz, 2H), 2.47 (d, J = 4.9 Hz, 2H), 2.36 - 2.33 (m, 2H), 2.14 (s, 3H), 1.85 (dt, J = 48.2, 8.0 Hz, 10H), 1.53(ddd, J = 24.9, 12.2, 5.8 Hz, 6H), 0.65 (d, J = 7.4 Hz, 3H).LCMS [M+H]⁺ =834.5 |
| **887** | 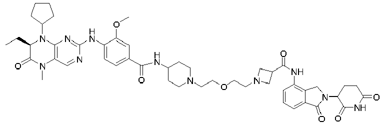 |
| | ¹H NMR (400 MHz, DMSO-*d*₆) δ11.02 (s, 1H), 10.33 (s, 1H),8.41 (d, *J* = 8.9 Hz, 1H), 8.04 (d, *J* = 7.7 Hz, 1H), 7.85 (s, 1H), 7.72 (d, *J* = 7.3 Hz, 1H), 7.55 (ddd, *J* = 23.7, 17.9, 6.2 Hz, 5H), 5.16 (dd, *J* = 13.0, 4.8 Hz, 1H), 4.45 (d, *J* = 9.0 Hz, 1H), 4.29 (ddd, *J* = 14.0, 11.2, 5.9 Hz, 3H), 3.94 (s, 3H), 3.29 - 3.16 (m, 4H), 3.25(s, 3H),3.14 - 2.58 (m, 2H), 2.56 - 1.56 (m, 12H), 1.13 - 0.92 (m, 2H), 0.79 (dd, *J* = 12.6, 9.1 Hz, 3H). |
| | LCMS [M+H]⁺ =920.6 |
| **888** | 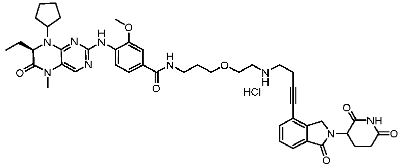 |
| | ¹H NMR (400 MHz, DMSO-*d*₆) δ 11.01 (s, 1H), 9.19 (s, 2H), 8.71 (t, *J=* 5.6 Hz, 1H), 7.88 (d, *J=* 8.6 Hz, 1H), 7.82 (s, 1H), 7.78 - 7.64 (m, 3H), 7.61 - 7.50 (m, 2H), 5.16 (dd, *J=* 13.3, 5.1 Hz, 1H), 4.48 (d, *J=* 6.8 Hz, 1H), 4.34 (d, *J=* 17.8 Hz, 1H), 4.17 (t, *J=* 8.8 Hz, 1H), 3.91 (s, 3H), 3.70 (d, *J=* 5.6 Hz, 2H), 3.56 - 3.48 (m, 3H), 3.30 - 3.17 (m, 6H), 3.00 (t, *J=* 7.5 Hz, 3H), 2.97 - 2.85 (m, 1H), 2.67 - 2.57 (m, 1H), 2.09 - 1.75 (m, 10H), 1.57 - 1.42 (m, 4H), 0.75 (t, *J=* 7.4 Hz, 3H). LCMS [M+H]⁺ =820.5 |
| **889** | 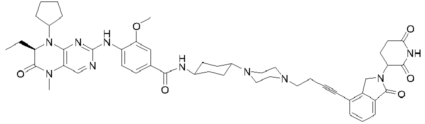 |
| | ¹H NMR (400 MHz, DMSO-*d*₆)δ11.02 (s, 1H), 8.41 (d, *J=* 8.9 Hz, 1H), 8.04 (d, *J=* 7.7 Hz, 1H), 7.85 (s, 1H), 7.72 (d, *J=* 7.3 Hz, 1H), 7.55 (ddd, *J* = 23.7, 17.9, 6.2 Hz, 5H), 5.16 (dd, *J=* 13.0, 4.8 Hz, 1H), 4.45 (d, *J=* 18.0 Hz, 1H), 4.29 (ddd, *J* = 14.0, 11.2, 5.9 Hz, 3H), 3.84 (d, *J* = 9.4Hz, 4H), 3.40 (s, 2H), 3.36 (d, *J=* 9.4 Hz, 3H), 2.92 (d, *J=* 12.3 Hz, 1H), 2.33 (s, 3H), 2.29 - 2.14 (m, 1H), 2.14 - 1.87 (m, 8H), 1.79 (s, 3H), 1.65 (dd, *J* = 13.7, 6.6 Hz, 2H), 1.49 (d, *J=* 10.1 Hz, 4H), 1.23 (s, 2H), 0.76 (t, *J=* 7.4 Hz, 3H). |
| | LCMS [M+H]⁺ =885.6 |
| **890** | 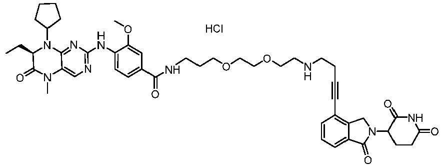 |
| | ¹H NMR (400 MHz, DMSO-*d*₆) δ 11.03 (s, 1H), 8.65 (s, 2H), 8.40 (t, *J* = 5.5 Hz, 1H), 8.21 (s, 2H), 7.80 (s, 1H), 7.77 - 7.73 (m, 1H), 7.67 (dd, *J* = 7.6, 1.1 Hz, 1H), 7.55 (t, *J* = 7.6 Hz, 1H), 7.52 - 7.46 (m, 2H), 4.47 (d, *J* = 17.8 Hz, 1H), 4.38 - 4.29 (m, 2H), 3.92 (s, 3H), 3.70 (t, *J* = 5.1 Hz, 2H), 3.61 (dd, *J* = 5.8, 3.0 Hz, 7H), 3.24 (s, 3H), 2.91 (q, *J* = 7.2, 6.5 Hz, 4H), 2.06 - 1.94 (m, 4H), 1.88 (s, 3H), 1.78 (q, *J* = 6.7 Hz, 3H), 1.73 - 1.64 (m, 3H), 1.55 (d, *J* = 7.1 Hz, 3H), 1.24 (d, *J* = 3.0 Hz, 3H), 0.76 (t, *J* = 7.4 Hz, 3H). LCMS [M+H]⁺ =864 |
| **891** | 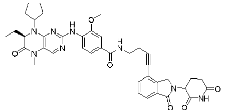 |
| | ¹H NMR (400 MHz, DMSO-*d*₆) δ 10.98 (d, *J* = 2.0 Hz, 1H), 8.72 (m, 2H), 8.03 (d, *J* = 8.2 Hz, 1H), 7.78 (d, *J* = 1.9 Hz, 1H), 7.71 (d, *J* = 7.5 Hz, 1H), 7.64 (d, *J* = 7.5 Hz, 1H), 7.58 (d, *J* = 1.8 Hz, 1H), 7.56 - 7.50 (m, 2H), 5.07 (m, 1H), 4.44 - 4.34 (m, 2H), 4.30 - 4.17 (m, 2H), 3.90 (s, 3H), 3.55 (m, 2H), 3.23 (s, 3H), 2.92 - 2.84 (m, 1H), 2.78 (t, *J* = 6.7 Hz, 2H), 2.56 (m, 1H), 2.24 (m, 1H), 2.00 - 1.72 (m, 8H), 1.58 (m, 2H), 1.49 - 1.39 (m, 2H), 0.75 (t, *J* = 7.4 Hz, 3H). LCMS [M+H]⁺ = 719 |
| **892** | 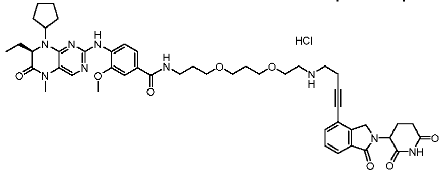 |
| | ¹H NMR (400 MHz, DMSO-*d*₆) δ 11.01 (s, 1H), 9.27 (s, 2H), 7.84 (s, 1H), 7.73 (d, *J* = 7.6 Hz, 1H), 7.67 (d, *J* = 6.3 Hz, 1H), 7.54 (m, 3H), 7.39 (s, 1H), 7.26 (s, 1H), 7.14 (s, 1H), 5.15 (m, 1H), 4.50 (m, 2H), 4.34 (m, 4H), 3.94 - 3.87 (s, 3H), 3.75 (m, 4H), 3.39 (m, 4H), 3.23 (s, 3H), 3.13 (m, 2H), 2.97 (m, 2H), 2.61 (m, 2H), 2.45 (m, 2H), 1.99 (m, 6H), 1.83 (m, 4H), 1.46 (m, 6H), 0.78 - 0.72 (m, 3H). LCMS [M+H]⁺ = 878 |
| **893** | 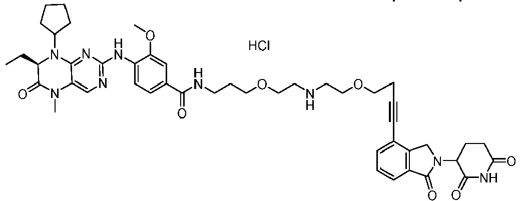 |
| | ¹H NMR (400 MHz, DMSO-*d*₆) δ 11.01 (s, 1H), 8.74 (s, 2H), 8.58 (s, 2H), 7.81 (s, 1H), 7.72 (d, *J* = 7.5 Hz, 1H), 7.65 - 7.61 (m, 1H), 7.59 (s, 1H), 7.52 (t, *J* = 7.9 Hz, 2H), 5.16 (dd, *J* = 13.2, 5.0 Hz, 1H), 4.45 (d, *J* = 17.8 Hz, 2H), 4.31 (d, *J* = 17.6 Hz, 1H), 3.92 (d, *J* = 1.4 Hz, 3H), 3.78 (t, *J* = 5.2 Hz, 2H), 3.69 (t, *J* = 6.6 Hz, 2H), 3.64 (t, *J* = 5.0 Hz, 2H), 3.46 (d, *J* = 6.2 Hz, 2H), 3.23 (s, 3H), 2.78 (t, *J* = 6.6 Hz, 2H), 2.64 (d, *J* = 22.5 Hz, 1H), 2.37 - 2.27 (m, 1H), 1.93 (s, 4H), 1.79 (dd, *J* = 13.4, 6.7 Hz, 6H), 1.55 (d, *J* = 40.5 Hz, 5H), 1.37 (s, 4H), 1.24 (s, 1H), 0.75 (t, *J* = 7.4 Hz, 3H). LCMS [M+H]⁺ =864.7 |
| **894** | 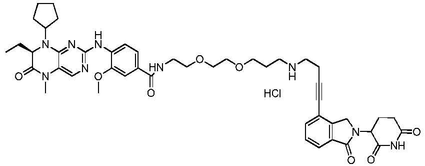 |
| | ¹H NMR (400 MHz, DMSO-*d*₆) δ 11.01 (s, 1H), 9.74 (s, 1H), 9.32 (s, 2H), 8.70 (t, *J* = 5.6 Hz, 1H), 7.92 - 7.79 (m, 2H), 7.77 - 7.67 (m, 2H), 7.64 (d, *J* = 1.9 Hz, 1H), 7.59 - 7.50 (m, 2H), 5.16 (dd, *J* = 13.3, 5.1 Hz, 1H), 4.57 - 4.46 (m, 2H), 4.35 (d, *J* = 17.8 Hz, 1H), 4.15 (s, 2H), 3.90 (s, 3H), 3.63 - 3.38 (m, 9H), 3.21 (s, 3H), 3.17 (t, *J* = 6.3 Hz, 2H), 3.04 - 2.87 (m, 5H), 2.71 - 2.53 (m, 2H), 2.10 - 1.63 (m, 9H), 1.67 - 1.25 (m, 4H), 0.75 (t, *J* = 7.4 Hz, 3H). LCMS [M+1]⁺ =864.5 |
| **895** | 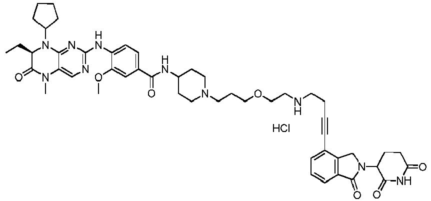 |
| | ¹H NMR (400 MHz, DMSO-*d*₆) δ 11.02 (s, 1H), 10.81 (s, 1H), 9.79 (s, 1H), 9.52 (s, 2H), 8.64 (d, *J* = 7.3 Hz, 1H), 7.95 - 7.81 (m, 2H), 7.80 - 7.63 (m, 3H), 7.62 - 7.46 (m, 2H), 5.16 (dd, *J* = 13.2, 5.2 Hz, 1H), 4.66 - 4.45 (m, 2H), 4.36 (d, *J* = 17.8 Hz, 1H), 4.17 (t, *J* = 8.8 Hz, 1H), 4.08 - 3.99 (m, 1H), 3.97 (s, 1H), 3.92 (s, 3H), 3.73 (t, *J* = 5.0 Hz, 2H), 3.37 (d, *J* = 15.7 Hz, 1H), 3.31 - 3.15 (m, 9H), 3.07 (q, *J* = 9.1, 7.1 Hz, 4H), 2.93 (td, *J* = 13.8, 13.3, 6.7 Hz, 1H), 2.68 - 2.55 (m, 1H), 2.20 - 1.71 (m, 14H), 1.63 - 1.37 (m, 4H), 0.75 (t, *J* = 7.5 Hz, 3H). LCMS [M+1]⁺ =903.5 |
| **896** | 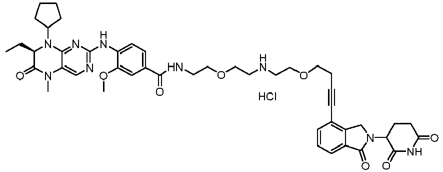 |
| | ¹H NMR (400 MHz, DMSO-*d6*) δ 10.90 (s, 1H), 8.32 (dd, J = 24.0, 6.8 Hz, 2H), 7.70 (s, 1H), 7.58 (d, J = 7.5 Hz, 1H), 7.49 (d, J = 7.0 Hz, 2H), 7.42 - 7.32 (m, 3H), 5.02 (dd, J = 13.3, 5.2 Hz, 1H), 4.31 (dd, J = 17.9, 3.1 Hz, 1H), 4.22 (d, J = 12.7 Hz, 1H), 4.12 (dd, J = 7.6, 3.6 Hz, 2H), 3.82 (s, 4H),3.48 (s, 5H), 3.13 (s, 3H), 2.56 (t, J = 6.7 Hz, 2H), 2.47 (d, J = 4.9 Hz, 2H), 2.36 - 2.33 (m, 2H), 2.14 (s, 3H), 1.85 (dt, J = 48.2, 8.0 Hz, 10H), 1.53(ddd, J = 24.9, 12.2, 5.8 Hz, 6H), 0.65 (d, J = 7.4 Hz, 3H).LCMS [M+H]⁺ =850.5 |
| **897** | 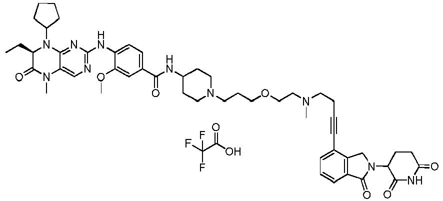 |
| | ¹H NMR (400 MHz, DMSO-*d*₆) δ 11.04 (s, 1H), 9.63 (d, *J* = 68.0 Hz, 3H), 8.40 (d, *J* = 7.4 Hz, 1H), 8.18 (s, 1H), 7.86 - 7.64 (m, 3H), 7.60 - 7.46 (m, 3H), 5.19 (dd, *J* = 13.3, 5.1 Hz, 1H), 4.54 - 4.20 (m, 5H), 4.03 (d, *J* = 6.9 Hz, 1H), 3.93 (s, 3H), 3.75 (t, *J* = 4.9 Hz, 2H), 3.53 (t, *J* = 6.2 Hz, 2H), 3.24 (s, 3H), 3.13 - 2.93 (m, 7H), 2.89 (s, 3H), 2.64 (dd, *J* = 16.1, 12.7 Hz, 2H), 2.41 (s, 2H), 2.10 - 1.42 (m, 19H), 0.76 (t, *J* = 7.4 Hz, 3H). LCMS [M+1]⁺ =917.7 |
| **898** | 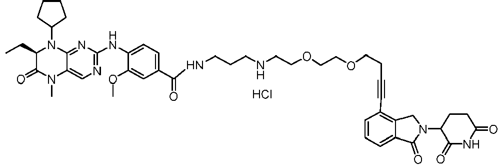 |
| | ¹H NMR (400 MHz, DMSO-*d6*) δ 11.01 (s, 1H), 9.49 (s, 1H), 9.15 (s, 2H), 9.00 (t, J = 5.6 Hz, 1H), 7.95 (d, J = 8.4 Hz, 1H), 7.86 (s, 1H), 7.78 -7.66 (m, 2H), 7.62 (dd, J = 7.9, 1.3 Hz, 2H), 7.52 (d, J = 7.6 Hz, 1H), 5.15 (dd, J = 13.3, 5.1 Hz, 1H), 4.56 - 4.15 (m, 4H), 3.93 (s, 3H), 3.74 (t, J =5.2 Hz, 2H), 3.67 - 3.61 (m, 7H), 3.22 (s, 3H), 3.15 (d, J = 6.1 Hz, 4H), 2.92 (ddd, J = 18.0, 13.6, 5.4 Hz, 1H), 2.73 (t, J = 6.8 Hz, 2H), 2.62 (d, J =3.5 Hz, 1H), 2.46 - 2.38 (m, 1H), 2.06 - 1.68 (m, 8H), 1.63 - 1.43 (m, 4H), 0.75 (t, J = 7.4 Hz, 3H).LCMS [M+H]⁺ =864.5 |
| **899** | 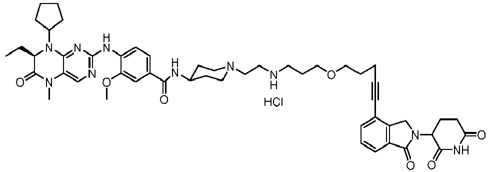 |
| | ¹H NMR (400 MHz, DMSO) δ 10.95 (d, *J* = 47.4 Hz, 1H), 9.70 (s, 1H), 9.42 (s, 1H), 8.61 (d, *J* = 7.3 Hz, 1H), 7.95 - 7.37 (m, 7H), 5.16 (dd, *J* = 13.3, 5.1 Hz, 1H), 4.54 - 4.23 (m, 3H), 4.15 (dd, *J* = 26.4, 17.9 Hz, 3H), 3.93 (d, *J* = 12.1 Hz, 6H), 3.66 (d, *J* = 11.1 Hz, 2H), 3.58 - 3.37 (m, 9H), 3.22 (s, 3H), 3.17 (s, 2H), 3.03 (s, 2H), 2.98 - 2.83 (m, 1H), 2.71 - 2.53 (m, 5H), 2.46 - 2.31 (m, 1H), 2.10 - 1.72 (m, 15H), 1.60 - 1.36 (m, 4H), 0.76 (t, *J* = 7.4 Hz, 3H). LCMS [M+H]⁺ =917.5 |
| **900** | 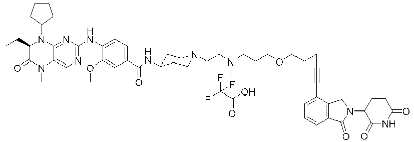 |
| | ¹H NMR (400 MHz, DMSO) δ 11.02 (s, 1H), 10.06 (s, 1H), 8.22 (s, 2H), 7.64 (ddd, *J* = 47.7, 25.7, 18.2 Hz, 5H), 5.16 (dd, *J* = 13.4, 5.2 Hz, 1H), 4.39 (dd, *J* = 58.2, 17.7 Hz, 4H), 3.94 (s, 3H), 2.99 - 2.73 (m, 2H), 2.70 - 2.64 (m, 3H), 2.36 - 2.30 (m, 2H), 1.82 (ddd, *J* = 111.1, 77.5, 45.9 Hz, 15H), 1.20 (d, *J* = 34.9 Hz, 1H), 1.06 (t, *J* = 7.0 Hz, 1H), 0.77 (t, *J* = 7.4 Hz, 3H). LCMS [M+H]⁺ =931.5 |
| **901** | 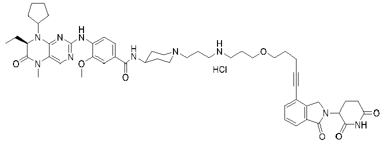 |
| | ¹H NMR (400 MHz, DMSO) δ 11.01 (s, 2H), 9.70 (s, 1H), 9.09 (s, 2H), 8.62 (d, *J* = 7.5 Hz, 1H), 7.92 - 7.43 (m, 7H), 5.16 (dd, *J* = 13.3, 5.1 Hz, 1H), 4.39 (dd, *J* = 57.8, 17.7 Hz, 3H), 4.15 (dd, *J* = 25.9, 17.4 Hz, 2H), 3.93 (d, *J* = 10.0 Hz, 4H), 3.58 - 3.43 (m, 8H), 3.22 (s, 3H), 3.18 - 2.84 (m, 9H), 2.72 - 2.54 (m, 2H), 2.33 (s, 2H), 2.21 - 1.73 (m, 17H), 1.48 (d, *J* = 24.3 Hz, 4H), 0.76 (t, *J* = 7.4 Hz, 3H). LCMS [M+H]⁺ =931.51 |
| **902** | 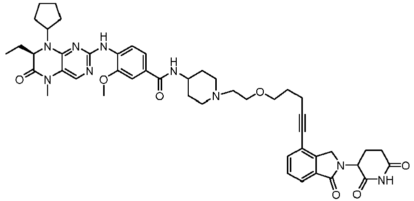 |
| | ¹H NMR (400 MHz, DMSO-*d6*) δ 11.00 (s, 1H), 10.85 (s, 1H), 8.42 (d, J = 8.7 Hz, 2H), 7.85 (s, 1H), 7.71 (dd, J = 7.6, 1.1 Hz, 1H), 7.66 (d, J = 7.6 Hz,1H), 7.61 (s, 1H), 7.53 (d, J = 7.1 Hz, 3H), 5.15 (dd, J = 13.3, 5.1 Hz, 1H), 4.47 (d, J = 17.7 Hz, 1H), 4.40 - 4.30 (m, 2H), 4.24 (dd, J = 7.6, 3.6 Hz, 1H),3.95 (s, 3H), 3.84 (s, 2H), 3.58 (s, 3H), 3.12 (s, 2H), 3.07 - 2.85 (m, 2H), 2.56 (d, J = 7.1 Hz, 3H), 2.44 (dd, J = 13.2, 4.3 Hz, 1H), 2.11 - 1.55 (m, 18H),0.76 (t, J = 7.4 Hz, 3H). LCMS [M+H]⁺ =860.6 |
| 903 | 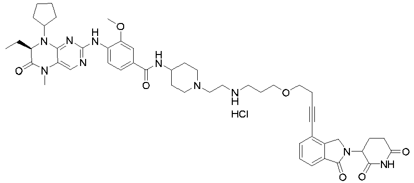 |
| | ¹H NMR (400 MHz, DMSO-*d*₆) δ 11.01 (s, 1H), 10.77 (s, 1H), 9.27 (s,2H), 8.55 (d, *J* = 6.7 Hz, 1H), 8.00 (s, 1H), 7.82 (s, 2H), 7.68 - 7.52 (m, 4H), 5.16 (dd, *J* = 13.2, 5.1 Hz, 1H), 4.47 - 4.41 (m, 1H), 4.27 - 4.19 (m, 1H), 4.14 - 4.05 (m, 2H), 3.93 (s, 3H), 3.72 - 3.44 (m, 14H), 3.23 (s, 3H), 3.09 - 3.01 (m, 2H), 2.76 (t, *J* = 6.7 Hz, 2H), 2.70 - 2.64 (m, 1H), 2.58 (d, *J* = 6.0 Hz, 2H), 2.10 - 1.76 (m, 12H), 1.54 (d, *J* = 39.3 Hz, 4H), 1.26 - 1.22 (m, 2H), 0.76 (t, *J* = 7.4 Hz, 3H). LCMS [M+H]⁺ =903.6 |
| 904 | 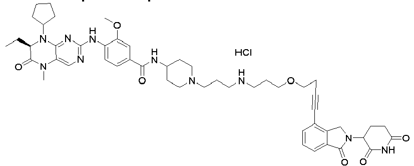 |
| | ¹H NMR (400 MHz, DMSO-*d*₆) δ 10.99 (d, *J* = 20.2 Hz, 2H), 9.52 (s, 1H), 9.14 (s, 2H), 8.61 (d, *J* = 7.3 Hz, 1H), 7.88 (d, *J* = 23.6 Hz, 2H), 7.75 - 7.43 (m, 5H), 5.16 (dd, *J* = 13.2, 5.1 Hz, 1H), 4.53 - 4.42 (m, 2H), 4.32 (d, *J* = 17.7 Hz, 1H), 4.19 (t, *J* = 8.9 Hz, 1H), 4.08 (s, 1H), 3.92 (s, 3H), 3.63 (t, *J* = 6.7 Hz, 2H), 3.56 (t, *J* = 6.0 Hz, 2H), 3.50 (d, *J* = 11.1 Hz, 2H), 3.22 (s, 3H), 2.97 (m,9H), 2.75 (t, *J* = 6.7 Hz, 2H), 2.61 (m, 1H), 2.45 (d, *J* = 4.5 Hz, 1H), 2.22 - 1.72 (m, 17H), 1.61 - 1.41 (m, 4H), 1.24 (d, *J* = 3.4 Hz, 1H), 0.76 (t, *J=* 7.4 Hz, 3H). LCMS [M+H]⁺ =917.6 |
| **905** | 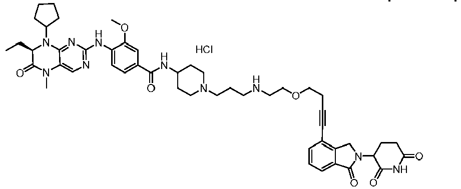 |
| | ¹H NMR (400 MHz, DMSO-*d*₆) δ 11.00 (d, *J=* 16.6 Hz, 2H), 9.09 (s, 2H), 8.59 (d, *J* = 7.4 Hz, 1H), 7.84 (s, 1H), 7.73 (dd, *J* = 7.4, 3.5 Hz, 1H), 7.66 (dt, *J* = 7.7, 1.8 Hz, 1H), 7.62 (d, *J* = 1.8 Hz, 1H), 7.60 - 7.55 (m, 1H), 7.53 (d, *J* = 7.6 Hz, 1H), 5.17 (dd, *J* = 13.3, 5.1 Hz, 1H), 4.47 (d, *J* = 17.7 Hz, 2H), 4.33 (d, *J* = 17.7 Hz, 1H), 4.20 (t, *J* = 8.7 Hz, 1H), 4.09 (d, *J* = 14.2 Hz, 1H), 3.94 (d, *J* = 9.5 Hz, 3H), 3.78 (t, *J* = 5.1 Hz, 2H), 3.71 (t, *J* = 6.7 Hz, 2H), 3.22 (s, 3H), 3.19 - 2.99 (m, 8H), 2.97 - 2.87 (m, 1H), 2.81 (t, *J* = 6.7 Hz, 3H), 2.70 - 2.56 (m, 2H), 2.47 - 2.39 (m, 1H), 2.20 - 1.73 (m, 14H), 1.52 (d, *J=* 29.5 Hz, 4H), 0.76 (t, *J* = 7.4 Hz, 3H). LCMS [M+H]⁺ =903.6 |
| **906** | 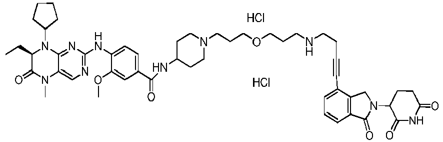 |
| | ¹H NMR (400 MHz, DMSO-*d*₆) δ 11.02 (s, 1H), 10.68 (s, 1H), 9.54 (s, 1H), 9.31 (s, 2H), 8.61 (d, *J* = 7.5 Hz, 1H), 7.93 - 7.82 (m, 2H), 7.79 - 7.67 (m, 2H), 7.64 (d, *J* = 1.8 Hz, 1H), 7.62 - 7.51 (m, 2H), 5.17 (dd, *J* = 13.3, 5.1 Hz, 1H), 4.58 - 4.43 (m, 2H), 4.41 - 4.30 (m, 1H), 4.19 (p, *J* = 8.4 Hz, 1H), 4.06 (d, *J* = 8.7 Hz, 1H), 3.92 (s, 3H), 3.58 - 3.43 (m, 7H), 3.22 (m, 5H), 3.12 - 2.95 (m, 7H), 2.97 - 2.87 (m, 1H), 2.61 (d, *J* = 17.9 Hz, 1H), 2.48 - 2.41 (m, 1H), 2.13 - 1.73 (m, 15H), 1.63 - 1.32 (m, 4H), 0.76 (t, *J* = 7.4 Hz, 3H). LCMS [M+1]⁺ =917.6 |
| **907** | 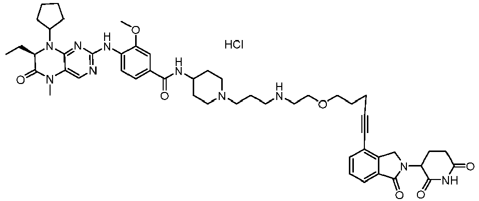 |
| | ¹H NMR (400 MHz, DMSO-*d*₆) δ 11.01 (s, 2H), 9.12 (s, 2H), 8.49 - 8.28 (m, 2H), 7.85 (s, 1H), 7.72 (d, *J* = 7.6 Hz, 1H), 7.65 (d, *J* = 7.5 Hz, 1H), 7.54 (d, *J* = 7.7 Hz, 2H), 5.16 (dd, *J* = 13.3, 5.1 Hz, 1H), 4.47 (d, *J* = 17.7 Hz, 1H), 4.38 - 4.21 (m, 3H), 3.95 (s, 3H), 3.71 (t, *J* = 5.1 Hz, 2H), 3.59 (t, *J* = 6.2 Hz, 3H), 3.50 (d, *J* = 11.8 Hz, 3H), 3.25 (s, 5H), 3.13 (s, 9H), 2.99 - 2.84 (m, 2H), 2.60 (t, *J* = 7.2 Hz, 4H), 2.02 (s, 6H), 1.92 - 1.49 (m, 13H), 1.25 - 1.12 (m, 1H), 0.76 (t, *J* = 7.4 Hz, 3H). LCMS [M+H]⁺ =917.6 |
| **908** | 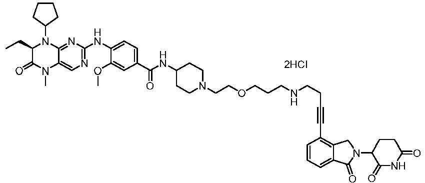 |
| | ¹H NMR (400 MHz, DMSO-*d*₆) δ 11.01 (s, 1H), 10.79 (s, 1H), 9.68 (s, 1H), 9.35 (s, 2H), 8.62 (d, *J* = 7.3 Hz, 1H), 7.85 (q, *J* = 5.8, 5.1 Hz, 2H), 7.77 - 7.66 (m, 2H), 7.63 (d, *J* = 1.9 Hz, 1H), 7.61 - 7.49 (m, 2H), 5.16 (m, 1H), 4.58 - 4.45 (m, 2H), 4.35 (m, 1H), 4.17 (m, 1H), 4.09 (m, 1H), 3.95 (m, 1H), 3.91 (s, 3H), 3.62 - 3.51 (m, 4H), 3.22 (m, 6H), 3.14 (m, 1H), 3.02 (m, 4H), 2.96 - 2.86 (m, 1H), 2.60 (m, 1H), 2.47 - 2.39 (m, 1H), 2.16 - 1.71 (m, 13H), 1.47 (m, 4H), 0.75 (t, *J* = 7.4 Hz, 3H). LCMS [M+H]⁺ = 903 |
| **909** | 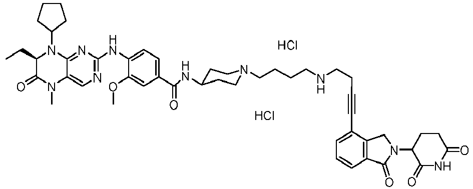 |
| | ¹H NMR (400 MHz, DMSO) δ 11.03 (s, 1H), 10.76 (s, 1H), 9.54 (s, 1H), 9.22 (s, 1H), 8.59 (d, *J* = 7.6 Hz, 1H), 7.97 - 7.46 (m, 5H), 5.18 (dd, *J* = 13.3, 5.0 Hz, 1H), 4.52 (t, *J* = 16.2 Hz, 2H), 4.36 (d, *J* = 17.9 Hz, 1H), 4.27 - 3.97 (m, 2H), 3.93 (d, *J* = 8.6 Hz, 2H), 3.22 (s, 3H), 3.07 - 2.77 (m, 5H), 2.70 - 2.55 (m, 1H), 2.33 (s, 1H), 2.14 - 1.58 (m, 10H), 1.50 (d, *J* = 25.4 Hz, 3H), 0.76 (t, *J* = 7.4 Hz, 3H). |
| | LCMS [M+H]⁺ =873 |
| **910** | 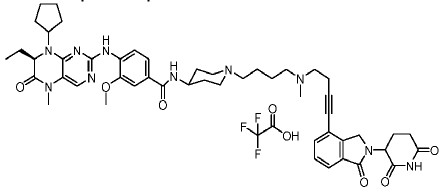 |
| | LCMS [M+H]⁺ =887.6 |
| **911** | 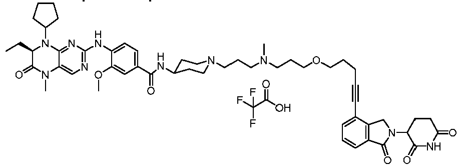 |
| | ¹H NMR (400 MHz, DMSO) δ 11.01 (s, 1H), 9.45 (s, 2H), 8.47 - 8.08 (m, 2H), 7.89 - 7.40 (m, 5H), 5.17 (dd, *J* = 13.4, 5.0 Hz, 1H), 4.38 (dd, *J* = 59.1, 17.6 Hz, 4H), 3.99 (d, *J* = 46.4 Hz, 4H), 3.57 - 3.50 (m, 4H), 3.24 - 2.99 (m, 6H), 3.00 - 2.86 (m, 3H), 2.80 (s, 4H), 2.66 (dd, *J* = 11.1, 9.3 Hz, 2H), 2.36 - 2.28 (m, 1H), 2.15 - 1.51 (m, 22H), 1.47 (s, 5H), 0.76 (t, *J* = 7.5 Hz, 3H). |
| | LCMS [M+H]⁺ =945.5 |
| **912** | 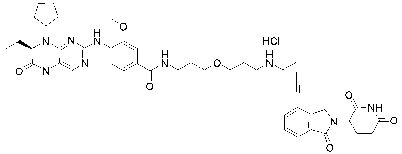 |
| | ¹H NMR (400 MHz, DMSO) δ 11.01 (s, 1H), 9.60 - 8.33 (m, 3H), 7.95 - 7.42 (m, 7H), 5.16 (d, *J* = 9.2 Hz, 1H), 4.33 (dd, *J* = 65.2, 49.8 Hz, 5H), 3.93 (d, *J* = 4.8 Hz, 4H), 3.63 - 3.53 (m, 15H), 3.22 (s, 7H), 3.07 - 2.84 (m, 4H), 2.65 (dd, *J* = 42.6, 20.6 Hz, 2H), 2.33 (s, 2H), 1.96 (dd, *J* = 71.5, 44.7 Hz, 8H), 1.65 - 1.35 (m, 4H), 1.34 - 1.01 (m, 8H), 0.75 (t, *J* = 5.8 Hz, 3H). |
| | LCMS [M+H]⁺ =834.4 |
| **913** | 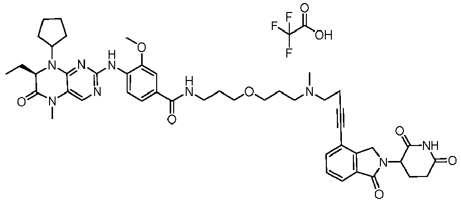 |
| | LCMS [M+H]⁺ =887.6 |
| **914** | 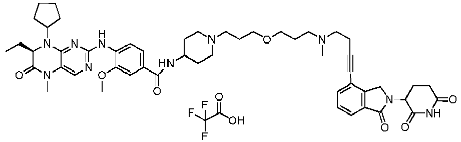 |
| | ¹H NMR (400 MHz, DMSO-*d*₆) δ 11.03 (s, 1H), 10.29 (d, *J* = 32.0 Hz, 2H), 8.48 (d, *J* = 7.4 Hz, 1H), 8.12 (s, 1H), 7.82 (s, 1H), 7.76 (d, *J* = 7.5 Hz, 1H), 7.69 (d, *J* = 7.4 Hz, 1H), 7.61 - 7.49 (m, 3H), 5.18 (dd, *J* = 13.4, 4.9 Hz, 1H), 4.55 - 4.18 (m, 5H), 4.05 (s, 2H), 3.93 (s, 3H), 3.58 - 3.39 (m, 7H), 3.36 (m, 2H), 3.23 (s, 3H), 3.05 (t, *J* = 6.6 Hz, 5H), 2.85 (d, *J* = 3.7 Hz, 2H), 2.63 (t, *J* = 16.3 Hz, 1H), 2.37 (d, *J* = 29.3 Hz, 2H), 2.11 - 1.37 (m, 20H), 0.76 (t, *J* = 7.4 Hz, 3H). LCMS [M+1]⁺ =931.6 |
| **915** | 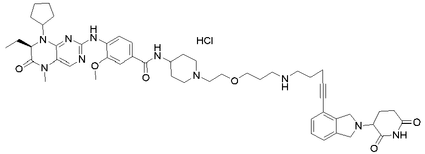 |
| | ¹H NMR (400 MHz, DMSO-*d6*) δ 11.00 (s, 1H), 10.74 (s, 1H), 9.54 (s, 1H), 9.14 (s, 2H), 8.59 (d, *J* = 6.9 Hz, 1H), 7.86 (d, *J* = 13.6 Hz, 2H), 7.74 - 7.61 (m, 3H), 7.60 - 7.47 (m, 2H), 5.16 (m, 1H), 4.57 - 4.45 (m, 2H), 4.34 (d, *J* = 17.8 Hz, 1H), 4.18 (m, 1H), 4.04 (m, 1H), 3.93 (d, *J* = 13.1 Hz, 3H), 3.82 (m, 2H), 3.62 - 3.53 (m, 4H), 3.24 (m, 6H), 3.17 - 2.85 (m, 8H), 2.69 - 2.56 (m, 3H), 2.10 - 1.80 (m, 14H), 1.50 (m, 4H), 0.76 (t, J = 7.4 Hz, 3H). LCMS [M+H]⁺ = 903 |
| **916** | 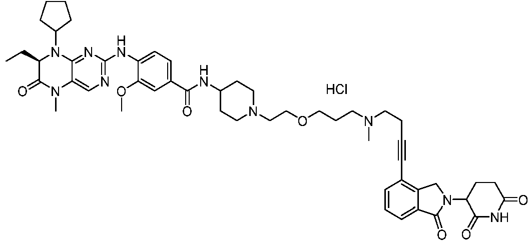 |
| | ¹H NMR (400 MHz, DMSO-*d6*) δ 11.19 (d, *J* = 36.7 Hz, 2H), 11.02 (s, 1H), 9.75 (s, 1H), 8.65 (d, *J* = 7.3 Hz, 1H), 7.94 - 7.83 (m, 2H), 7.78 - 7.62 (m, 3H), 7.56 (m, 2H), 5.16 (m, 1H), 4.58 - 4.45 (m, 2H), 4.35 (m, 1H), 4.23 - 4.14 (m, 1H), 4.06 (m, 1H), 3.93 (d, J = 18.8 Hz, 3H), 3.84 (m, 2H), 3.62 - 3.50 (m, 6H), 3.22 (m, 5H), 3.12 (m, 5H), 2.99 - 2.90 (m, 1H), 2.81 (d, *J* = 4.6 Hz, 3H), 2.66 - 2.58 (m, 1H), 2.48 - 2.40 (m, 1H), 2.17 - 1.77 (m, 14H), 1.55 - 1.40 (m, 4H), 0.75 (t, *J* = 7.4 Hz, 3H). LCMS [M+H]⁺ = 917 |
| **917** | 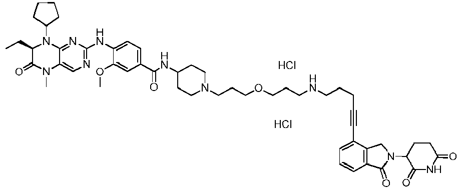 |
| | ¹H NMR (400 MHz, DMSO-*d*₆) δ 11.00 (s, 1H), 10.72 (s, 1H), 9.73 (s, 1H), 9.23 (s, 2H), 8.64 (d, *J* = 7.4 Hz, 1H), 7.85 (d, *J* = 8.9 Hz, 2H), 7.75 - 7.48 (m, 5H), 5.16 (dd, *J* = 13.2, 5.0 Hz, 1H), 4.57 - 4.47 (m, 2H), 4.35 (d, *J* = 17.8 Hz, 1H), 4.19 (q, *J* = 8.8 Hz, 1H), 4.06 (s, 1H), 3.92 (s, 3H), 3.57 - 3.42 (m, 6H), 3.34 (d, *J* = 6.8 Hz, 1H), 3.22 (s, 3H), 3.11 - 2.87 (m, 8H), 2.73 - 2.52 (m, 4H), 2.19 - 1.73 (m, 17H), 1.49 (d, *J* = 23.9 Hz, 4H), 0.75 (t, *J* = 7.4 Hz, 3H). LCMS [M+1]⁺ =931.6 |
| **918** | 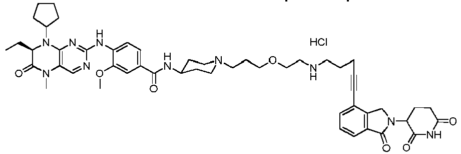 |
| | ¹H NMR (400 MHz, DMSO-*d*₆) δ 11.01 (s, 1H), 10.34 (s, 1H), 8.99 (s, 1H), 8.90 (s, 1H), 8.53 (s, 1H),8.01 (s, 1H), 7.80 (s, 1H), 7.74 (d, *J* = 7.6 Hz, 1H), 7.67 (d, *J* = 7.6 Hz, 1H),7.59-7.54 (m, 4H), 5.17 (dd, *J* = 13.2 Hz, 4.8 Hz, 1H), 4.50 (d, *J* = 18 Hz, 1H), 4.44 (s, 1H), 4.33 (d, *J* = 18 Hz, 1H), 4.21 (s, 1H), 4.06 (s, 1H), 3.93 (s, 3H), 3.68 (t, *J* = 4.8 Hz, 2H), 3.54 (t, *J* = 5.6 Hz, 4H), 3.23 (s, 3H), 3.19-3.02 (m, 8H), 2.99-2.89 (m, 1H), 2.68-2.65 (m, 1H), 2.63 (d, *J* = 10.8 Hz, 2H), 2.33 (m, 1H), 2.07-1.90 (m, 10H), 1.89-1.72 (m, 4H), 1.58 (s, 2H), 1.49 (s, 2H), 0.76 (t, *J* = 7.6Hz, 3H). LCMS [M+H]⁺ =917.6 |
| **919** | 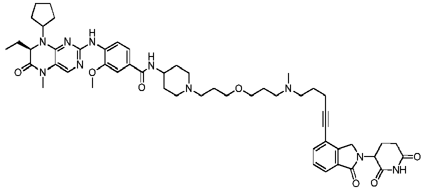 |
| | ¹H NMR (400 MHz, DMSO-*d*₆) δ 11.08 (s, 1H), 8.48 (d, *J* = 8.3 Hz, 1H), 8.39 - 8.25 (m, 1H), 7.91 (s, 1H), 7.81 - 7.64 (m, 3H), 7.58 (dt, *J* = 12.0, 4.0 Hz, 3H), 5.22 (dd, *J* = 13.2, 5.2 Hz, 1H), 4.61 - 4.50 (m, 1H), 4.47 - 4.35 (m, 2H), 4.30 (dd, *J* = 7.6, 3.6 Hz, 1H), 4.01 (s, 3H), 4.00 - 3.91 (m, 2H), 3.64 (d, *J* = 66.9 Hz, 2H), 3.31 (s, 3H), 2.99 (ddd, *J* = 17.8, 13.7, 5.4 Hz, 2H), 2.77 - 2.59 (m, 6H), 2.46 - 2.24 (m, 7H), 2.07 (tt, *J* = 9.5, 5.0 Hz, 4H), 2.04 - 1.74 (m, 16H), 1.69 (dq, *J* = 12.3, 6.6, 5.7 Hz, 4H), 0.83 (t, *J* = 7.4 Hz, 3H). LCMS [M+1]⁺ =945.7 |
| **920** | 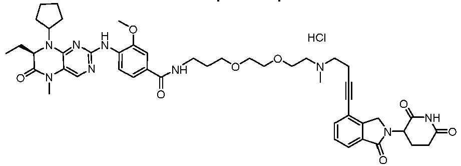 |
| | ¹H NMR (400 MHz, DMSO-*d*₆) δ 11.01 (s, 1H), 8.42 (d, *J* = 8.3 Hz, 1H), 8.30 (t, *J* = 5.6 Hz, 1H), 7.84 (s, 1H), 7.70 (d, *J* = 7.6 Hz, 1H), 7.62 (s, 1H), 7.55 - 7.47 (m, 2H), 4.44 (d, *J* = 17.6 Hz, 3H), 4.34 (d, *J* = 9.3 Hz, 1H), 4.26 - 4.23 (m, 3H), 3.93 (s, 3H), 3.60 - 3.42 (m, 12H), 3.25 (s, 3H), 2.71 - 2.54 (m, 8H), 2.26 (s, 3H), 2.08 - 1.90 (m, 3H), 1.85 - 1.72 (m,5H), 1.68 - 1.56 (m, 2H), 1.24 (s, 2H), 0.77 (t, *J* = 7.4 Hz, 3H). LCMS [M+H]⁺ =878.5 |
| **921** | 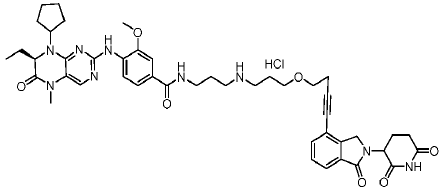 |
| | ¹H NMR (400 MHz, DMSO-*d*₆) δ 11.00 (s, 1H), 9.14 (s, 2H), 8.94 - 8.90 (m, 1H), 7.99 - 7.84 (m, 2H), 7.75 - 7.46 (m, 5H), 5.15 (dd, *J* = 13.2, 5.0 Hz, 1H), 4.58 - 4.41 (m, 2H), 4.35 - 4.27 (m, 1H), 4.18 (t, *J* = 8.8 Hz, 1H), 3.92 (s, 3H), 3.69 - 3.48 (m, 6H), 3.22 (s, 3H), 2.98 - 2.87 (m, 4H), 2.74 (t, *J* = 6.9 Hz, 2H), 2.64 - 2.54 (m, 2H), 2.47 (s, 2H), 2.06 - 1.73 (m, 10H), 1.60 - 1.44 (m, 4H), 1.25 - 1.21 (m, 2H), 0.75 (t, *J* = 7.4 Hz, 3H). LCMS [M+H]⁺ = 834.4 |
| **922** | 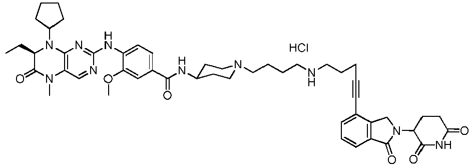 |
| | ¹H NMR (400 MHz, DMSO-*d*₆) δ 11.01 (s, 1H), 10.33 (s, 1H), 8.99 (s, 1H), 8.90 (s, 1H), 8.51 (s, 1H), 7.97 (s, 1H), 7.80 (s, 1H), 7.74 (d, *J* = 7.6 Hz, 1H), 7.67 (d, *J* = 7.6 Hz, 1H),7.60-7.52 (m, 4H), 5.17 (dd, *J* = 13.2 Hz, 4.8 Hz, 1H), 4.50 (d, *J* = 18 Hz, 1H), 4.43 (s, 1H), 4.33 (d, *J* = 18 Hz, 1H), 4.22 (s, 1H), 4.05 (s, 1H), 3.93 (s, 3H), 3.68 (t, *J* = 4.8 Hz, 2H), 3.52 (t, *J* = 5.6 Hz, 4H), 3.23 (s, 3H), 3.19-3.02 (m, 8H), 2.99-2.89 (m, 1H), 2.68-2.65 (m, 1H), 2.63 (d, *J* = 10.8 Hz, 2H), 2.33 (m, 1H), 2.07-1.90 (m, 8H), 1.89-1.72 (m, 4H), 1.58 (s, 2H), 1.49 (s, 2H), 0.76 (t, *J* = 7.6Hz, 3H). LCMS [M+H]⁺ =887.6 |
| **923** | 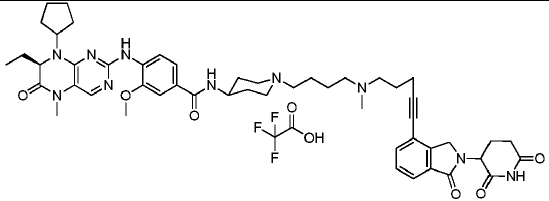 |
| | ¹H NMR (400 MHz, DMSO-*d*₆) δ 11.02 (s, 1H), 9.48 (s, 1H), 9.39 (s, 1H), 8.36 (s, 1H), 8.34 (s, 1H), 7.83 (s, 1H), 7.75 (d, *J* = 7.6 Hz, 1H), 7.67 (d, *J* = 7.6 Hz, 1H), 7.56 (t, *J* = 7.6 Hz, 1H), 7.53-7.49 (m, 2H), 5.18 (dd, *J* = 13.2 Hz, 4.8 Hz, 1H), 4.48 (d, *J* = 18 Hz, 1H), 4.33 (d, *J* = 18 Hz, 1H), 4.32 (s, 2H), 4.06 (s, 1H), 3.94 (s, 3H), 3.51 (t, *J* = 8.8 Hz, 2H), 3.25 (s, 3H), 3.11-3.01 (m, 4H), 2.99-2.89 (m, 1H), 2.82 (s, 3H), 2.62 (t, *J* = 6.4 Hz, 2H), 2.11-1.95 (m, 8H), 1.92-1.85 (m, 3H), 1.83-1.65 (m, 12H), 1.58 (s, 2H), 0.76 (t, *J* = 7.6Hz, 3H). LCMS [M+H]⁺ =901.6 |
| **924** | 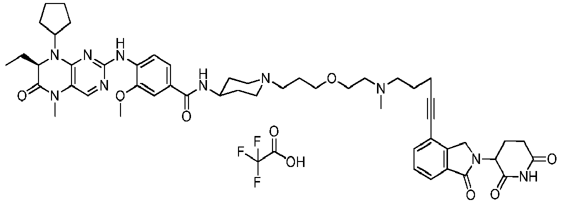 |
| | ¹H NMR (400 MHz, DMSO-*d*₆) δ 11.03 (s, 1H), 9.43 (s, 1H), 9.31 (s, 1H), 8.36 (s, 1H), 8.25 (s, 1H), 7.83 (s, 1H), 7.75 (d, *J* = 7.6 Hz, 1H), 7.67 (d, *J* = 7.6 Hz, 1H), 7.56 (t, *J* = 7.6 Hz, 1H), 7.53-7.49 (m, 2H), 5.18 (dd, *J* = 13.2 Hz, 4.8 Hz, 1H), 4.48 (d, *J* = 18 Hz, 1H), 4.33 (d, *J* = 18 Hz, 1H), 4.32 (s, 2H), 4.06 (s, 1H), 3.94 (s, 3H), 3.72 (t, *J* = 4.8 Hz, 2H), 3.52 (t, *J* = 8.8 Hz, 2H), 3.25 (s, 3H), 3.12-3.03 (m, 4H), 2.99-2.89 (m, 1H), 2.62 (t, *J* = 6.4 Hz, 2H), 2.01-1.86 (m, 16H), 1.70 (s, 2H), 1.57 (s, 2H), 0.76 (t, *J* = 7.6Hz, 3H). LCMS [M+H]⁺ =931.6 |
| **925** | 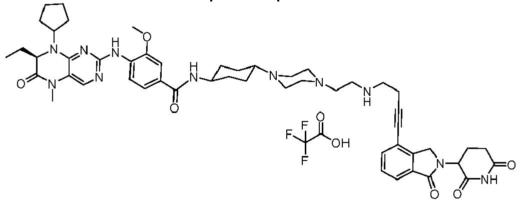 |
| | NMR:None.LCMS [M+H]⁺ =928.7 |
| **926** | 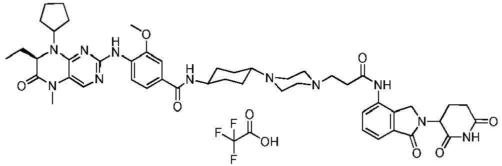 |
| | ¹H NMR (400 MHz, DMSO-*d*₆) δ 11.04 (s, 1H), 9.98 (s, 1H), 9.01 (br, 1H), 8.25 (d, *J* = 7.7 Hz, 1H), 7.96 (s, 1H), 7.87 - 7.81 (m, 1H), 7.76 (s, 1H), 7.62 - 7.37 (m, 4H), 5.17 (dd, *J* = 13.3, 5.2 Hz, 1H), 4.50 - 4.28 (m, 3H), 4.21 (t, *J* = 8.7 Hz, 1H), 3.92 (s, 3H), 3.78 (m, 2H), 3.23 (s, 3H), 3.01 - 2.87 (m, 3H), 2.82 - 2.54 (m, 6H), 2.37 - 2.18 (m, 2H), 2.13 - 1.68 (m, 12H), 1.67 - 1.34 (m, 8H), 1.25 (dd, *J* = 11.0, 4.7 Hz, 5H), 0.76 (t, *J* = 7.4 Hz, 3H). LCMS [M+1]⁺ =904.7 |
| **927** | 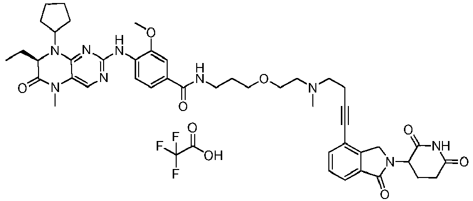 |
| | ¹H NMR (400 MHz, DMSO-*d*₆) δ 11.02 (s, 1H), 9.49 (s, 1H), 8.83 (s, 1H), 8.48 (t, *J* = 5.8 Hz, 1H), 8.02 (s, 1H), 7.85 - 7.70 (m, 2H), 7.67 (d, *J* = 7.6 Hz, 1H), 7.60 - 7.38 (m, 2H), 5.16 (dd, *J* = 13.3, 5.1 Hz, 1H), 4.47 (d, *J* = 17.7 Hz, 1H), 4.40 (dd, *J* = 7.0, 3.4 Hz, 1H), 4.33 (d, *J* = 17.7 Hz, 1H), 4.20 (t, *J* = 8.8 Hz, 1H), 3.90 (s, 3H), 3.74 (t, *J* = 5.0 Hz, 2H), 3.54 - 3.49 (m, 4H), 3.36 (s, 4H), 3.23 (s, 3H), 3.02 (t, *J* = 7.4 Hz, 2H), 2.91 (s, 3H), 2.62 (dd, *J* = 20.4, 16.7 Hz, 2H), 2.45 - 2.28 (m, 1H), 2.10 - 1.67 (m, 9H), 1.65 - 1.40 (m, 4H), 0.75 (t, *J* = 7.4 Hz, 3H). LCMS [M+1]⁺ =834.5 |
| **928** | 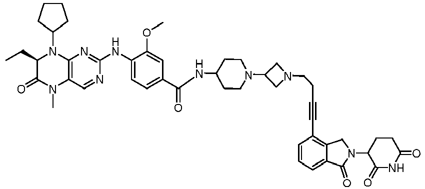 |
| | ¹H NMR (400 MHz, DMSO-*d*₆)δ11.02 (s, 1H), 8.42 (d, *J* = 8.9 Hz, 1H), 7.97 (d, *J* = 5.7 Hz, 1H), 7.84 - 7.80 (m, 1H), 7.72 (d, *J* = 7.5 Hz, 1H), 7.56 (ddd, *J* = 8.0, 9.8, 6.6 Hz, 5H), 5.16 (dd, *J* = 13.3, 5.0 Hz, 1H), 4.44 (t, *J* = 20.3 Hz, 1H), 4.29 (ddd, *J* = 11.7, 11.2, 5.9 Hz, 3H), 3.94 (s, 3H), 3.76 (s, 1H), 3.59 - 3.54 (m, 1H), 3.25 (s, 4H), 2.92 (dd, *J* = 21.3, 9.3 Hz, 3H), 2.76 - 2.44 (m, 3H), 2.44 - 2.35 (m, 1H), 2.33 (s, 2H), 2.07 (dd, *J* = 10.3, 9.3 Hz, 5H), 2.44 - 1.30 (m, 4H),1.15 - 1.04 (m, 2H), 0.76 (t, *J* = 7.4 Hz, 3H). LCMS [M+H]⁺ =857.5 |
| **930** | 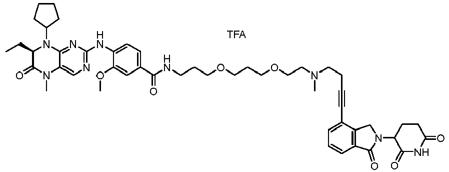 |
| | ¹H NMR (400 MHz, DMSO-*d*₆) δ 11.03 (s, 1H), 9.51 (d, *J* = 32.1 Hz, 1H), 8.86 (s, 1H), 8.16 (t, *J* = 8.7 Hz, 1H), 8.01 (m, 1H), 7.81 - 7.73 (m, 2H), 7.66 (m, 1H), 7.58 - 7.47 (m, 3H), 5.17 (m, 1H), 4.52 - 4.39 (m, 2H), 4.32 (m, 1H), 4.25 - 4.16 (m, 2H), 3.91 (m, 3H), 3.80 (m, 4H), 3.23 (s, 3H), 2.98 (m, 3H), 2.88 (s, 3H), 2.69 - 2.58 (m, 2H), 2.43 - 2.32 (m, 2H), 2.08 - 1.72 (m, 9H), 1.59 (m, 2H), 1.48 (m, 3H), 1.18 - 1.05 (m, 6H), 0.75 (t, *J* = 7.4 Hz, 3H). LCMS [M+H]⁺ = 892 |
| **931** | 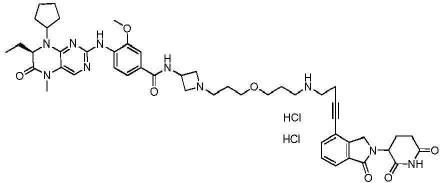 |
| | ¹H NMR (400 MHz, DMSO-*d*₆) δ 11.01 (s, 1H), 10.74 (d, *J* = 61.8 Hz, 1H), 9.92 - 9.49 (m, 2H), 9.30 (d, *J* = 32.7 Hz, 2H), 7.89 (d, *J* = 8.5 Hz, 1H), 7.82 (s, 1H), 7.69 (dddd, *J* = 25.2, 11.6, 8.3, 2.5 Hz, 4H), 7.58 - 7.50 (m, 1H), 5.16 (dd, *J* = 13.4, 5.0 Hz, 1H), 4.85 (d, *J* = 8.0 Hz, 1H), 4.60 - 4.47 (m, 2H), 4.35 (qd, *J* = 16.6, 14.7, 7.5 Hz, 5H), 4.18 (t, *J* = 8.8 Hz, 1H), 3.95 (d, *J* = 3.9 Hz, 3H), 3.41 - 3.29 (m, 4H), 3.22 (s, 5H), 3.12 - 2.87 (m, 6H), 2.60 (d, *J* = 17.0 Hz, 1H), 2.45 (dd, *J* = 13.3, 4.5 Hz, 1H), 2.13 - 1.68 (m, 12H), 1.63 - 1.39 (m, 4H), 0.75 (t, *J* = 7.3 Hz, 3H). LCMS [M+1]⁺ =889.6 |
| **932** | 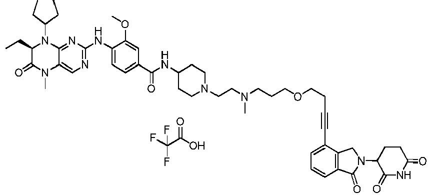 |
| | ¹H NMR (400 MHz, DMSO-*d*₆) δ 11.01 (s, 1H), 10.01 (s, 1H), 8.58 (s, 1H), 8.37 (s, 1H), 8.12 (s, 1H), 7.80 (s, 1H), 7.73 (d, *J* = 7.5 Hz, 1H), 7.64 (d, *J* = 7.6 Hz, 1H), 7.56 - 7.45 (m, 3H), 5.17 (dd, *J* = 13.3, 5.1 Hz, 1H), 4.45 (d, *J* = 17.7 Hz, 1H), 4.37 (dd, *J* = 7.2, 3.4 Hz, 1H), 4.31 (d, *J* = 17.7 Hz, 1H), 4.26 (q, *J* = 8.6 Hz, 2H), 4.02 (s, 1H), 3.93 (s, 3H), 3.63 (t, *J* = 6.7 Hz, 3H), 3.55 (t, *J* = 6.0 Hz, 6H), 3.24 (s, 3H), 3.17 (m, 2H), 2.94 (ddd, *J* = 17.9, 13.5, 5.3 Hz, 2H), 2.85 - 2.72 (m, 4H), 2.69 - 2.55 (m, 1H), 2.47 - 2.40 (m, 1H), 2.17 - 1.39 (m, 17H), 1.33 - 1.18 (m, 3H), 0.76 (t, *J* = 7.4 Hz, 3H). LCMS [M+1]⁺ =917.6 |
| **933** | 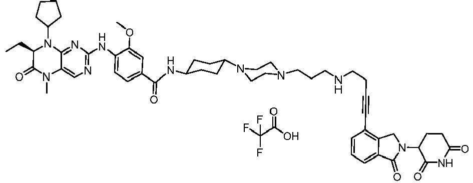 |
| | ¹H NMR (400 MHz, DMSO-*d6*) δ 12.02 (s, 1H), 11.01 (s, 1H), 9.69 (s, 1H), 8.38 (d, J = 7.8 Hz, 1H), 7.82 (t, J = 4.1 Hz, 2H), 7.74 (ddd, J = 14.9, 7.7, 1.1 |
| | Hz, 2H), 7.65 - 7.51 (m, 3H), 5.17 (dd, J = 13.3, 5.1 Hz, 1H), 4.63 - 4.29 (m, 3H), 4.17 (p, J = 8.7 Hz, 1H), 3.91 (s, 3H), 3.58 (d, J = 12.0 Hz, 4H), 3.45 (s,2H), 3.31 (s, 1H), 3.21 (s, 3H), 3.10 (s, 2H), 3.01-2.86 (m, 1H), 2.63 (s, 1H), 2.19 (d, J = 9.2 Hz, 2H), 2.08 - 1.74 (m, 10H), 1.66 (t, J = 11.9 Hz, 2H), 1.57- 1.34 (m, 6H), 0.75 (t, J = 7.4 Hz, 3H). LCMS [M+H]⁺ = 942.7 |

### Synthesis method of Compound M5

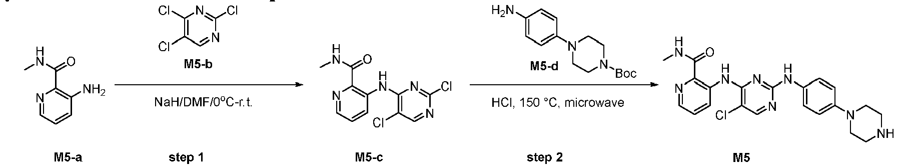

### Step 1: M5-c (V2591-149)

To **M5-a** (200 mg, 1.3 mmol) in tert-butanol (10mL) solution was added **M5-b** (243 mg, 1.3 mmol) and 1 ml DIPEA, then the mixture was stirred at 90°C for 18 hours, the mixture was concentrated in vacuum to obtain solid. The mixture was added ether and ultrasound for 10 minutes, then filtered to obtain desired product **M5-c** (270 mg, 69 % yield ) as a yellow solid.

LCMS [M+1] ⁺ =298.2.

### Step 2: M5 (V2876-001)

To **M5-c** (245 mg, 0.8 mmol) in n-butanol (10mL) solution was added **M5-d** (228 mg, 0.8mmol), hydrochloric acid (1ml) was added to the mixture, then the mixture was stirred at 80°C. The mixture was heated by microwave at 150°C for 2 hours under nitrogen protection, the reaction mixture was added to ether and filtered to obtain M5 (385 mg) as a yellow solid.

¹H NMR (400 MHz, DMSO) δ 12.49 (s, 1H), 9.39 (s, 2H), 9.28 - 9.09 (m, 3H), 8.36 - 8.16 (m, 2H), 7.52 (d, *J=* 8.6 Hz, 3H), 6.98 (d, *J=* 8.8 Hz, 2H), 3.40 - 3.36 (m, 2H), 3.23 (s, 2H), 2.85 (d, *J=* 4.8 Hz, 3H), 1.40 - 1.26 (m, 2H), 0.86 (t, *J=* 7.2 Hz, 2H). LCMS [M+1] ⁺ =439.4

### Synthesis of Compound UB-180961

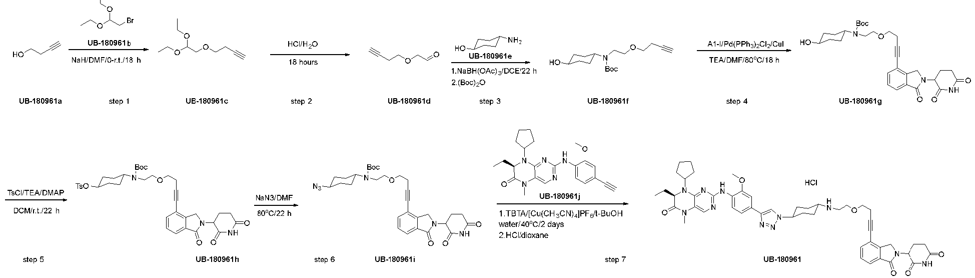

### Step 1: UB-180961c(V2407-041)

Compound **UB-180961a** (20 g, 71.1 mmol) was dissolved in dry DMF (80 mL) and cooled to 0°C, then NaH (16.8 g, 107 mmol) was added to reaction solution. After half an hour, **UB-180961b(21.1** g, 107 mmol) was dissolved in dry DMF (20 mL) and added to reaction solution, the mixture was reacted at room temperature overnight. To reaction solution was added ice water(100 mL) and extracted with EtOAc (60 mL) three times, the organic phases were combined, then isolated by column chromatography (PE/EtOAc = 0-100%) to obtain target product **UB-180961c** (25g, 47% yield) as a colorless oil.

¹H NMR (400 MHz, CDCl₃) δ 4.63 (t, *J=* 5.2 Hz, 1H), 3.72 - 3.52 (m, 8H), 2.47 (td, *J=* 7.0, 2.6 Hz, 2H), 1.98 (t, *J* = 2.7 Hz, 1H), 1.28-1.21 (m, 6H).

### Step 2: UB-180961d(V2407-042)

Compound **UB-180961c(15** g, 285.4 mmol) was dissolved in water (40 mL), then concentrated HCl (10 mL) was added and the mixture was reacted at room temperature overnight. The reaction solution was extracted with DCM (50 mL) three times, the organic phase was dried over anhydrous sodium sulfate, then concentrated to obtain target product **UB-180961d** (7.6 g) as a colorless oil. The crude product was directly used in the next reaction.

¹H NMR (400 MHz, CDCl₃) δ 9.74 (d, *J=* 0.8 Hz, 1H), 4.15 (d, *J=* 0.8 Hz, 2H), 3.72 - 3.67 (m, 2H), 2.54 (t, *J* = 2.7 Hz, 2H), 2.02 (t, *J* = 3.4 Hz, 1H).

### Step 3: UB-180961f(V2407-044)

Compound **UB-180961d** (7.8 g, 68 mmol) and **UB-180961e** (7.6 g, 68 mmol) were dissolved in DCE (100 mL), then the mixture was reacted at room temperature for 1 hour, then NaBH(OAc)3 (29.6 g, 136 mmol) was added and continued to react at room temperature overnight. To the reaction solution was added TEA (5 mL, sat) and BoczO (6 g, 23.8 mmol), then the mixture was reacted at room temperature for 18 hours, the reaction solution was extracted twice with EtOAc (15 mL). The organic phase was dried over anhydrous Na₂SO₄, then isolated by column chromatography (PE/EtOAc = 0-100%) to obtain target product **UB-180961f** (4.6 g, 57% yield) as a yellow oil.

¹H NMR (400 MHz, CDCl₃) δ 4.14 (ddd, *J* = 28.9, 14.7, 7.4 Hz, 5H), 2.67 (t, *J=* 11.7 Hz, 2H), 1.82 (dd, *J=* 14.1, 7.0 Hz, 2H), 1.70 (d, *J=* 12.3 Hz, 2H), 1.59 (d, *J=* 18.5 Hz, 4H), 1.46 (s, 9H), 1.19 (dd, *J* = 12.2, 4.0 Hz, 2H).

### Step 4: UB-180961g(V2407-047)

Compound **UB-180961f** (4.6 g, 15 mmol) and **A1-I** (3.7 g, 10 mmol), Pd(PPh₃)₂Cl₂ (701 mg, 1 mmol), CuI (190 mg,1mmol), and TEA (4.2 ml, 30 mmol) were dissolved in dry DMF (120 mL), then the mixture was reacted at 80°C overnight. The reaction solution was extracted twice with EtOAc (15 mL), The organic phase was dried over anhydrous Na₂SO₄, then isolated by column chromatography (PE/EtOAc = 0-100%) to obtain target product **UB-180961g** (3.6 g, 57% yield) as a yellow solid. ¹H NMR (400 MHz, DMSO) δ 11.00 (s, 1H), 7.72 (d, *J* = 6.9 Hz, 1H), 7.63 (d, *J* = 7.2 Hz, 1H), 7.53 (t, *J* = 7.6 Hz, 1H), 5.15 (dd, *J* = 13.3, 5.1 Hz, 1H), 4.46 (dd, *J* = 18.0, 11.1 Hz, 2H), 4.30 (d, *J=* 17.7 Hz, 1H), 3.62 (t, *J* = 6.6 Hz, 2H), 3.47 (t, *J*= 6.5 Hz, 2H), 3.30 - 3.15 (m, 3H), 2.98 - 2.87 (m, 1H), 2.71 (t, *J* = 6.7 Hz, 2H), 2.59 (d, *J =* 18.0 Hz, 1H), 2.44 (dd, *J* = 13.1, 4.4 Hz, 1H), 2.07 - 1.98 (m, 1H), 1.79-1.77 (d, *J* = 11.1 Hz, 2H), 1.53 (s, 4H), 1.37 (s, 9H), 1.21 - 1.08 (m, 2H).

### Step 5: UB-180961h(V2407-048)

Compound **UB-180961g** (3.6 g, 33.8 mmol), TEA (10.3 g, 10.2 mmol) and DMAP (12.4 g, 10.2 mmol) were dissolved in dry DMF (140 mL), then TsCl (14.6 g, 7.7 mmol) was added at 0 °C. The reaction solution was warmed up to 30 °C and reacted for 15 hours. The reaction solution was extracted twice with DCM (50 mL), The organic phase was concentrated, then isolated by column chromatography (PE/EtOAc = 0-100%) to obtain target product **UB-180961h** (3.6 g, 86% yield) as a white solid.

¹H NMR (400 MHz, DMSO) δ 11.01 (s, 1H), 7.78 (d, *J=* 8.3 Hz, 2H), 7.73 (dd, *J=* 7.5, 0.8 Hz, 1H), 7.63 - 7.59 (m, 1H), 7.52 (t, *J=* 7.6 Hz, 1H), 7.46 (d, *J=* 8.0 Hz, 2H), 5.16 (dd, *J* = 13.3, 5.1 Hz, 1H), 4.37 (dt, *J* = 41.1, 17.7 Hz, 3H), 3.60 (t, *J* = 6.6 Hz, 2H), 3.44 (t, *J* = 6.5 Hz, 2H), 3.19 (s, 2H), 2.91 (d, *J=* 12.3 Hz, 1H), 2.70 (t, *J=* 6.6 Hz, 2H), 2.59 (d, *J=* 16.2 Hz, 1H), 2.46 - 2.37 (m, 4H), 2.05 - 2.00 (m, 1H), 1.78 (d, *J=* 8.3 Hz, 2H), 1.64 - 1.43 (m, 6H), 1.36 (d, *J=* 5.1 Hz, 9H).

### Step 6: UB-180961i(V2407-049)

Compound **V2407-048** (3.6 g, 5.1 mmol) and NaN3 (667 mg, 10.2 mmol) were dissolved in DMF (10 mL), then the mixture was reacted at 80°C overnight. The reaction solution was extracted twice with EtOAc (100 mL). The organic phase was dried, then isolated by column chromatography (PE/EtOAc = 0-100%) to obtain target product **UB-180961i** (2.4 g, 68% yield) as a white solid.

¹H NMR (400 MHz, DMSO) δ 11.00 (s, 1H), 7.72 (d, *J=* 7.4 Hz, 1H), 7.63 (d, *J=* 7.3 Hz, 1H), 7.52 (t, *J* = 7.6 Hz, 1H), 5.15 (dd, *J* = 13.3, 5.1 Hz, 1H), 4.44 (d, *J* = 17.7 Hz, 1H), 4.31 (d, *J=* 17.7 Hz, 1H), 3.92 (s, 1H), 3.63 (t, *J=* 6.6 Hz, 2H), 3.49 (t, *J=* 6.3 Hz, 2H), 3.24 (s, 2H), 3.00 - 2.85 (m, 1H), 2.73 (t, *J=* 6.6 Hz, 2H), 2.61 (s, 1H), 2.46 - 2.37 (m, 1H), 2.02 (d, *J* = 5.5 Hz, 1H), 1.93 - 1.44 (m, 8H), 1.38 (s, 10H).

### Step 7: The method is similar to General Method 2

### UB-180961(V2240-090)

LCMS [M+H]⁺ = 884.6

¹H NMR (400 MHz, DMSO-*d₆*) δ 13.08 (s, 1H), 11.01 (s, 1H), 9.62 (s, 1H), 8.96 (m, 3H), 7.76 - 7.68 (m, 3H), 7.66 - 7.61 (m, 2H), 7.56 - 7.49 (m, 2H), 5.16 (dd, *J=* 13.3, 5.1 Hz, 1H), 4.71 (m, 1H), 4.51 - 4.44 (m, 2H), 4.32 (d, *J* = 17.8 Hz, 1H), 3.91 (s, 3H), 3.80 (t, *J* = 5.3 Hz, 2H), 3.69 (t, *J=* 6.7 Hz, 2H), 3.32 (m, 1H), 3.21 (s, 3H), 3.16 (m, 2H), 2.97 - 2.89 (m, 1H), 2.79 (t, *J* = 6.7 Hz, 2H), 2.59 (m, 1H), 2.44 (m, 1H), 2.05 - 1.73 (m, 14H), 1.46 (m, 2H), 1.41 - 1.33 (m, 2H), 0.75 (t, *J=* 7.4 Hz, 3H).

### Synthesis of Compound UB-180937

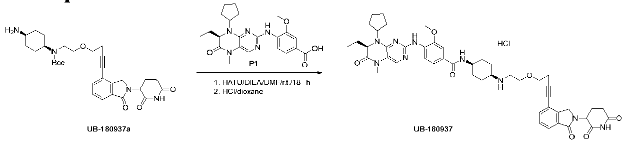

### The method is similar to General Method 1

### UB-180937 (V2768-119)

¹H NMR (400 MHz, DMSO-*d₆*) δ 11.01 (s, 1H), 9.70 (s, 1H), 8.98 (s, 2H), 7.95 (d, *J* = 5.4 Hz, 1H), 7.87 - 7.80 (m, 2H), 7.73 (dd, *J=* 7.6, 1.1 Hz, 1H), 7.68 - 7.58 (m, 3H), 7.53 (t, *J* = 7.6 Hz, 1H), 5.16 (dd, *J=* 13.3, 5.1 Hz, 1H), 4.51 - 4.45 (m, 2H), 4.33 (s, 1H), 4.17 (d, *J=* 8.8 Hz, 1H), 3.94 (s, 4H), 3.81 (t, *J=* 5.3 Hz, 2H), 3.70 (t, *J=* 6.7 Hz, 2H), 3.18 (d, *J=* 26.3 Hz, 6H), 2.99 - 2.88 (m, 1H), 2.80 (t, *J* = 6.7 Hz, 2H), 2.59 (d, *J* = 17.8 Hz, 1H), 2.46 (dd, *J*= 13.1, 4.2 Hz, 1H), 2.09 - 1.73 (m, 13H), 1.63 - 1.39 (m, 6H), 0.76 (t, *J=* 7.4 Hz, 3H). LCMS [M/2+1]⁺ = 431.1

### Synthesis method of Compound UB-180934

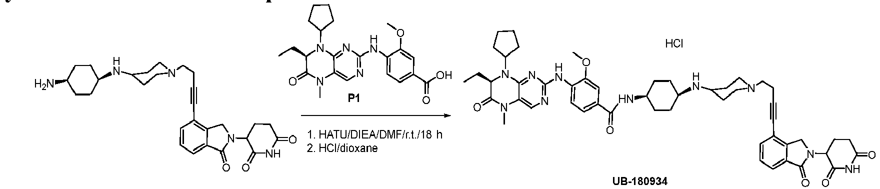

### The method is similar to General Method 1

LCMS [M+1]⁺ = 899.7

¹H NMR (400 MHz, ) δ 11.02 (s, 1H), 8.41 (d, *J=* 8.5 Hz, 1H), 7.84 (d, *J=* 12.8 Hz, 2H), 7.69 (t, *J=* 14.8 Hz, 2H), 7.57 - 7.51 (m, 2H), 7.51 - 7.44 (m, 2H), 5.17 (dd, *J=* 13.2, 5.2 Hz, 1H), 4.46 (d, *J=* 17.7 Hz, 1H), 4.39 - 4.28 (m, 2H), 4.25 (dd, *J=* 7.7, 3.6 Hz, 1H), 3.95 (d, *J* = 7.9 Hz, 4H), 3.25 (s, 3H), 2.96 (d, *J=* 18.8 Hz, 2H), 2.72 - 2.63 (m, 2H), 2.59 (s, 1H), 2.43 (s, 1H), 2.06 (d, *J* = 18.5 Hz, 7H), 1.94 - 1.71 (m, 11H), 1.71 - 1.50 (m, 9H), 0.77 (t, *J=* 7.4 Hz, 3H).

### Synthesis method of Compound UB-181010

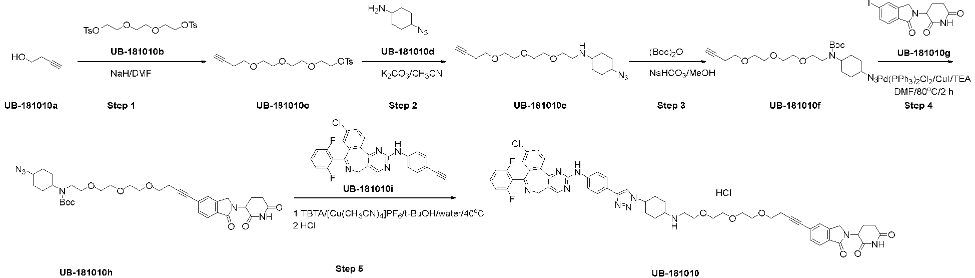

### Step 1: UB-181010c (V2141-112)

Compound **UB-181010a** (700mg, 10mmol) was dissolved in DMF (10mL), the mixture was cooled down to 0°C and added NaH (400mg, 10mmol). After reacting at room tempera for 16 hours, the reaction solution was quenched with water, extracted with ethyl acetate(30mL*3). The organic phases were combined, then dried, concentrated, and purified by silica gel column (PE/EA=3/1) to obtain target product **UB-181010c** (650mg 18% yield) as a white solid. ¹H NMR (400 MHz, Chloroform-*d*) δ 7.80 (d, *J=* 8.4 Hz, 2H), 7.41 - 7.31 (m, 2H), 3.66 (d, *J* = 4 Hz, 4H), 3.61 (d, *J=* 11.8 Hz, 10H), 2.51 - 2.43 (m, 3H).

### Step 2: UB-181010e (V2141-114)

Compound **UB-181010c** (500mg, 1.4mmol) was dissolved in CH₃CN (10mL), then K₂CO₃ (390mg, 2.8mmol), and **UB-181010d** (200mg, 1.4mmol) were added. The mixture was reacted at 80°C for 16 hours. The reaction solution was filtrated, and the filtrate was concentrated to obtain the crude product. The crude product was purified by silica gel column to obtain target product **UB-181010e(300mg,** 65.9% yield) as a colorless oil. LC-MS: [M+H] ⁺=324.3

### Step 3: UB-181010f (V2141-115)

Compound **UB-181010e** (300mg, 0.92mmol) was dissolved in THF (10mL), (BOC)₂O (400mg. 1.85mmol) and NaHCO₃ (155mg, 1.85mmol) were added. The reaction was reacted at room temperature for 2 hours. The reaction solution was added water, and extracted with ethyl acetate(30 mL*3). The combined organic phases were dried and the crude product was concentrated (390mg, 99% yield ), and directly used in the next step. LC-MS: [M+H] ⁺=425.4

### Step 4: UB-181010h (V2141-117)

Under the N₂ protection, **UB-181010f** (300mg, 0.7mmol), **UB-181010g** (260mg, 0.7mmol), Pd(PPh₃)₂Cl₂ (50mg, 0.07mmol), and CuI (266mg, 1.4mmol) were dissolved in TEA (200uL) and DMF (10mL), the mixture was reacted at 80°C for 2 hours. The reaction solution was filtered, concentrated to obtain crude product, then the crude product was purified by preparative TLC (DCM/MeOH=10/1) to obtain target product **UB-181010h**(230mg, 49% yield ) as a white solid. LC-MS: [M+H] ⁺=666.7

### Step 4: UB-181010 (V2141-124)

**The method is similar to General Method 3**

¹H NMR (400 MHz, DMSO-*d₆*) δ 11.00 (s, 1H), 10.03 (s, 1H), 9.00 (s, 2H), 8.80 (s, 1H), 8.70 (s, 1H), 8.31 (d, *J* = 8.5 Hz, 1H), 7.97 - 7.77 (m, 5H), 7.69 (d, *J* = 7.8 Hz, 1H), 7.62 (s, 1H), 7.59 - 7.47 (m, 2H), 7.34 (d, *J*= 2.3 Hz, 2H), 7.18 (t, *J=* 8.8 Hz, 2H), 5.11 (dd, *J=* 13.3, 5.0 Hz, 1H), 4.70 (s, 1H), 4.52 - 4.28 (m, 2H), 3.96 - 3.90 (m, 9H), 3.75 (t, *J=* 5.4 Hz, 2H), 3.62 (t, *J* = 6.7 Hz, 2H), 3.30 (s, 1H), 3.12 (p, *J* = 5.5 Hz, 2H), 2.91 (m, 1H), 2.72 (t, *J* = 6.7 Hz, 2H), 2.62 - 2.56 (m, 1H), 2.37 (m, 1H), 2.06 - 1.78 (m, 6H). LC-MS: [M+H] ⁺ =903, 452

### Synthesis method of Compound UB-181011

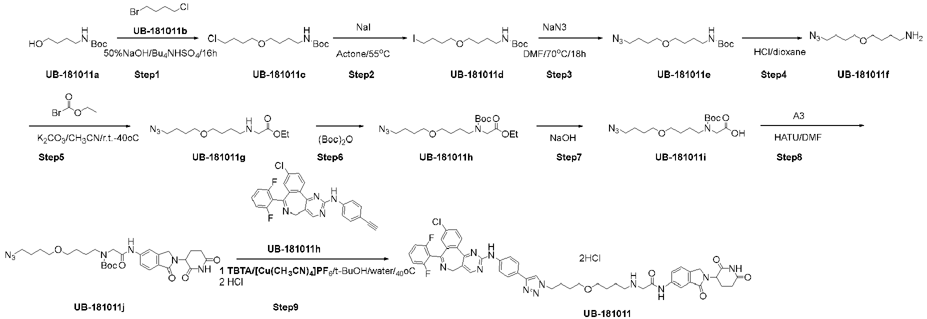

### Step 1: UB-181011c (V2141-128)

Compound **UB-181011a** (5g, 26mmol) **was dissolved in UB-181011b** (10mL), Bu₄NHSO₄ (17.7g, 52mmol) and 50%NaOH were added. The mixture was reacted at 50°C for 16 hours. The reaction solution was added water, extracted with dichloromethane (30 mL*3). The organic phases were combined, then dried and concentrated to give the crude product. The crude product was purified by silica gel column (PE/EA=3/1) to obtain target product **UB-181011c** (4.4g, 60% yield) as a colorless oil. LC-MS: [M+H] ⁺ =280.3

### Step 2: UB-181011d (V2141-129)

Compound **UB-181011c** (4.4g, 15.7mmol) **was dissolved in** acetone (200mL), NaI (23.5g, 15.7mmol) was added, and the mixture was reacted at 80°C for 2 days. The reaction solution was added water after concentration, and extracted with dichloromethane (20 mL*3). The organic phases were combined, then dried and concentrated to give the crude product **UB-181011d** (5.2g, 90% yield), which was directly used in the next step. LC-MS: [M+H] ⁺ =372.3

### Step 3: UB-181011e (V2141-130)

Compound **UB-181011d** (5.2g, 14mmol) and NaN₃ (1.82g, 28mmol) were dissolved in DMF (50mL), the mixture was reacted at 80°C for 16 hours. The reaction solution was added water (20 mL), and extracted with ethyl acetate(20 mL*3). The combined organic phases were dried and concentrated to give the crude product **UB-181011e** (4.2g, yield 100%). LC-MS: [M+H] ⁺ =286.3

### Step 4: UB-181011f (V2141-131)

Compound **UB-181011e** (4.2g, 14.6mmol) was dissolved in HCl/dioxane (50mL), and the mixture was reacted at room temperature for 2 hours. The reaction solution was concentrated to give the crude product **UB-181011f** (4g, 100% yield), which was directly used in the next step. LC-MS: [M+H] ⁺ =186.3

### Step 5: UB-181011g (V2141-132)

Compound **UB-181011f** (2.5g, 13.4mmol) was dissolved in CH3CN (200mL), K₂CO₃ (3.7g, 26.8mmol) and 2-bromoethyl acetate (2.23g, 13.4mmol) were added. The mixture was reacted at room temperature for 16 h. The reaction solution was filtered, and concentrated to obtain crude product. The crude product was purified by silica gel column to obtain target product **UB-181011g** (820mg, 22.7% yield). LC-MS: [M+H] ⁺ =272.3

### Step 6: UB-181011h (V2141-133)

To compound **UB-181011g** (820mg, 3mmol) was dissolved in THF (50mL), (BOC)₂O (241mg. 6mmol) and NaHCO₃ (504mg, 6mmol) were added. The mixture was reacted at room temperature for 2 h. The reaction solution was added water (20 mL), and extracted with ethyl acetate(20 mL*3). The combined organic phases were dried and concentrated to give the crude product **UB-181011h** (800mg, 73.2% yield ), which was directly used in the next step. LC-MS: [M+H] ⁺ =372.3

### Step 7: UB-181011i (V2141-134)

Compound **UB-181011h** (800mg, 2.15mmol), and NaOH (344mg, 8.6mmol) were dissolved in THF (20mL), methanol (20mL) and water (2mL), the mixture was reacted at 50°C for 2 hours. The reaction solution was concentrated, added water (20 mL), and extracted with ethyl acetate(20 mL*3). The aqueous phase was acidified to pH=3 using 1M HCl. Then the mixture was extracted with dichloromethane (20 mL*3). The organic phases of dichloromethane were combined, dried and concentrated to give the crude product **UB-181011i** (600mg, 81% yield), which was directly used in the next step. LC-MS: [M+H] ⁺ =345.3

### Step 8: UB-181011j (V2141-135)

Compound **UB-181011i** (300mg, 0.87mmol), A3 (226mg, 0.87mmol), and HATU (661mg, 1.74mmol) were dissolved in DMF (5mL) and DIPEA (1mL), the mixture was reacted at room temperature for 2 hours. The reaction solution was added water (20 mL), and extracted with ethyl acetate(20 mL*3). The combined organic phases were dried and concentrated to give the crude product. The crude product was purified by silica gel column (DCM/MeOH=20/1) to obtain target product **UB-181011j** (270mg, 53% yield). LC-MS: [M+H] ⁺ =587.3

### Step 9: UB-181011 (V2141-138)

**The method is similar to General Method 3**

¹H NMR (400 MHz, DMSO-*d₆*) δ 11.23 (s, 1H), 10.98 (s, 1H), 10.01 (s, 1H), 9.16 (s, 2H), 8.69 (s, 1H), 8.51 (s, 1H), 8.30 (d, *J=* 8.5 Hz, 1H), 7.96 - 7.87 (m, 3H), 7.80 (d, *J=* 8.4 Hz, 2H), 7.71 (s, 2H), 7.59 - 7.44 (m, 1H), 7.34 (d, *J=* 2.2 Hz, 1H), 7.17 (t, *J=* 8.8 Hz, 2H), 5.09 (dd, *J=* 13.3, 5.1 Hz, 1H), 4.50 - 4.27 (m, 4H), 4.02 (t, *J=* 5.7 Hz, 2H), 3.44 - 3.34 (m, 4H), 3.08 - 2.86 (m, 3H), 2.71 - 2.59 (m, 1H), 2.42 - 2.32 (m, 1H), 2.05 - 1.87 (m, 3H), 1.76 - 1.67 (m, 2H), 1.58 - 1.44 (m, 4H). LC-MS: [M+H] ⁺ =942.8

### Synthesis method of Compound UB-181013

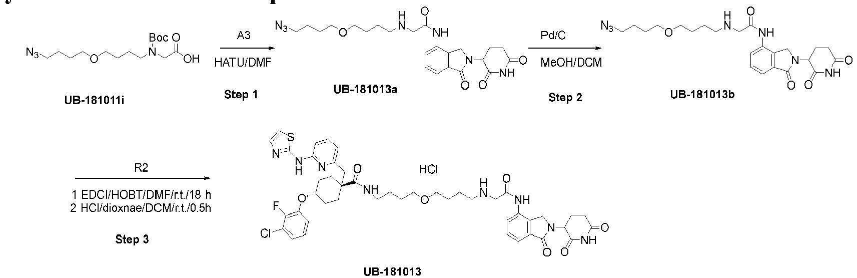

### Step 1: UB-181013a (V2141-135)

Compound **UB-181011i** (300mg, 0.87mmol), A3 (226mg, 0.87mmol), and HATU (661mg, 1.74mmol) were dissolved in DMF (5mL) and DIPEA (1mL), the mixture was reacted at room temperature for 2 hours. The reaction solution was added water (20 mL), and extracted with ethyl acetate(20 mL*3). The combined organic phases were dried and concentrated to give the crude product. The crude product was purified by silica gel column (DCM/MeOH=20/1) to obtain target product **UB-181013a** (270mg, 53% yield). LC-MS: LC-MS: [M+H] ⁺ =587.3

### Step 2: UB-181013b (V2141-139)

Compound **UB-181013a** (50mg, 0.085mmol), and Pd/C (5mg) were dissolved in DCM/MeOH=10/1 (10mL), the mixture was reacted at room temperature for 2 hours under H₂ atmosphere. The reaction solution was added 100mL DCM/MeOH=10/1, filtered after being stirred to fully dissolve the product. The filtrate was concentrated to obtain target product **UB-181013b** (50mg, 100% yield) as a colorless oil. LC-MS: [M+H] ⁺ =560.3

### Step 3: UB-181013 (V2141-146)

### The method is similar to General Method 2

¹H NMR (400 MHz, DMSO-*d₆*) δ 12.29 - 12.03 (m, 1H), 11.05 (s, 1H), 10.86 (s, 1H), 9.29 (s, 2H), 7.88 (d, *J=* 7.4 Hz, 1H), 7.73 - 7.47 (m, 5H), 7.24 - 6.98 (m, 5H), 6.75 (d, *J=* 7.4 Hz, 1H), 5.17 (dd, *J* = 13.2, 5.1 Hz, 1H), 4.55 - 4.26 (m, 2H), 4.03 (s, 2H), 3.43 - 3.28 (m, 4H), 2.99 (d, *J* = 18.9 Hz, 6H), 2.62 (d, *J* = 17.2 Hz, 2H), 2.36 - 2.17 (m, 1H), 2.12 - 1.99 (m, 1H), 1.96 - 1.88 (m, 2H), 1.82 - 1.53 (m, 10H), 1.43 - 1.36 (m, 4H). LC-MS: [M+H] ⁺ =903, 452

### Synthesis method of Compound UB-181019

### Step 1: UB-181019 (V2141-147)

### The method is similar to General Method 2

¹H NMR (400 MHz, DMSO-*d₆*) δ 12.16 - 11.78 (m, 1H), 11.30 (s, 1H), 10.99 (s, 1H), 9.22 (s, 2H), 7.96 (s, 1H), 7.76 - 7.61 (m, 4H), 7.47 (d, *J=* 3.9 Hz, 1H), 7.18 - 6.98 (m, 5H), 6.74 (d, *J* = 7.4 Hz, 1H), 5.09 (dd, *J* = 13.3, 5.1 Hz, 1H), 4.56 (s, 1H), 4.50 - 4.25 (m, 2H), 4.03 (s, 2H), 3.41 - 3.29 (m, 4H), 2.93 - 2.87 (m, 7H), 2.63 - 2.57 (m, 1H), 2.42 - 2.31 (m, 1H), 2.05 - 1.87 (m, 3H), 1.84 - 1.53 (m, 10H), 1.48 - 1.39 (m, 4H). LC-MS: [M+H] ⁺ =903, 452

### Synthesis method of Compound UB-181020

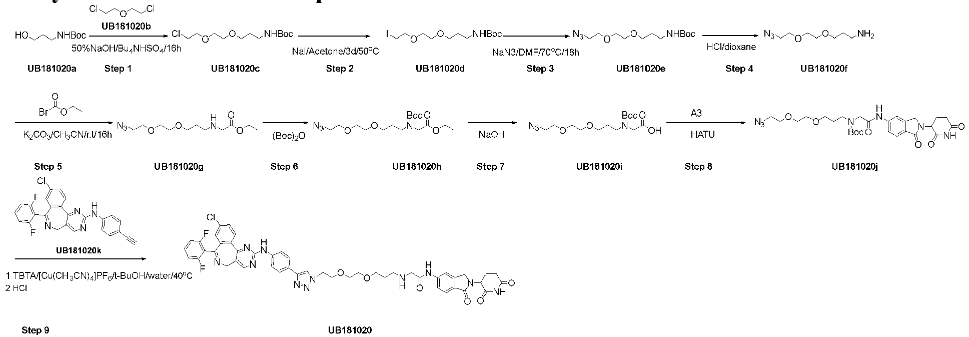

### Step 1: UB-181020c (V2141-108)

Compound **UB-181020a** (5g, 28.5mmol) was dissolved in **UB-181020b** (10mL), Bu₄NHSO₄ (19g, 57mmol) was added. The mixture was reacted at 50°C for 16 hours. The reaction solution was added water, and extracted with dichloromethane (20 mL*3). The combined organic phases were dried and concentrated to give the crude product. The crude product was purified by silica gel column (PE/EA=3/1) to obtain target product **UB-181020c** (4.4g, 60% yield) as a colorless oil. LC-MS: [M+H] ⁺ =282.3

### Step 2: UB-181020d (V2141-116)

Compound **UB-181020c** (3g, 10.6mmol) **was dissolved** in acetone (300mL), NaI (16g, 106mmol) was added, and the mixture was reacted at 80°C for 2 days. The reaction solution was added water after concentration, and extracted with dichloromethane (20 mL*3). The organic phases were combined, dried and concentrated to give the crude product **UB-181020d** (3.4g), which was directly used in the next step. LC-MS: [M+H] ⁺ =374.3

### Step 3: UB-181020e (V2141-127)

Compound **UB-181020d** (3.4g, 9.1mmol) and NaN₃ (1.82g, 27mmol) were dissolved in DMF (50mL), the mixture was reacted at 80°C for 16 hours. The reaction solution was added water, and extracted with ethyl acetate(20 mL*3). The combined organic phases were dried and concentrated to give the crude product **UB-181020e** (3g). LC-MS: [M+H] ⁺ =289.3

### Step 4: UB-181020f (V2141-143)

Compound **UB-181020e** (6g, 20.8mmol) was dissolved in HCl/dioxane (50mL), and the mixture was reacted at room temperature for 2 hours. The reaction solution was concentrated to give crude product **UB-181020f** (6g, 100% yield). LC-MS: [M+H] ⁺ =188.3

### Step 5: UB-181020g (V2141-144)

Compound **UB-181020f** (4.8g, 25.5mmol) was dissolved in CH3CN (200mL), K₂CO₃ (4.28g, 25.5mmol) and 2-bromoethyl acetate (4.2g, 25.5mmol) were added. The reaction was reacted at room temperature for 16 hours. The reaction solution was filtered, and concentrated to obtain crude product. The crude product was purified by silica gel column (DCM/MeOH=20/1) to obtain target product **UB-181020g** (2.1g, 30% yield). LC-MS: [M+H] ⁺ =274.3

### Step 6: UB-181020h (V2141-145)

Compound **UB-181020g** (2.1g, 7.66mmol) was dissolved in THF (100mL), (BOC)²O (612mg. 15.3mmol) and NaHCO₃ (1.28mg, 15.3mmol) were added. The reaction was reacted at room temperature for 2 hours. The reaction solution was added water, and extracted with ethyl acetate (20 mL*3). The organic phases were combined, dried and concentrated to give **UB-181020h** (3g, yield 100%). LC-MS: [M+H] ⁺ =374.3

### Step 7: UB-181020i (V2141-148)

Compound **UB-181020h** (3g, 8mmol), and NaOH (1.28g, 32mmol) were dissolved in THF (20mL), methanol (20mL) and water (2mL), the mixture was reacted at 50°C for 2 hours. The reaction solution was concentrated, then added water (20 mL), and extracted with ethyl acetate(10 mL*3). The aqueous phase was acidified to pH=3 using 1M HCl. Then the mixture was extracted with dichloromethane (20 mL*3). The organic phases were combined, dried and concentrated to give **UB-181020i** (2g, 74% yield ). LC-MS: [M+H] ⁺ =347.3

### Step 8: UB-181020j (V2141-149)

Compound **UB-181020i** (346mg, 1mmol), A3 (259mg, 1mmol), and HATU (760mg, 2mmol) were dissolved in DMF (3mL) and DIPEA (0.3mL), the mixture was reacted at room temperature for 2 hours. The reaction solution was added water, and extracted with ethyl acetate (20 mL*3). The organic phases were combined, dried and concentrated to give the crude product. The crude product was purified by silica gel column (DCM/MeOH=20/1) to obtain target product **UB-181011j** (380mg, 64.7% yield). LC-MS: [M+H] ⁺ =588.3

### Step 9: UB-181020 (V2537-001)

### The method is similar to General Method 3

¹H NMR (400 MHz, DMSO-*d₆*) δ 11.24 (s, 1H), 10.99 (s, 1H), 10.02 (s, 1H), 9.14 (s, 2H), 8.69 (s, 1H), 8.47 (s, 1H), 8.31 (d, *J* = 8.5 Hz, 1H), 7.97 - 7.86 (m, 4H), 7.80 (d, *J=* 8.5 Hz, 2H), 7.70 (s, 2H), 7.55 (td, *J* = 8.5, 4.1 Hz, 1H), 7.34 (d, *J* = 2.2 Hz, 1H), 7.17 (t, *J* = 9.0 Hz, 2H), 5.09 (dd, *J=* 13.3, 5.1 Hz, 1H), 4.50 - 4.24 (m, 6H), 4.04 - 3.86 (m, 4H), 3.62 - 3.42 (m, 6H), 3.10 - 2.98 (m, 2H), 2.91 (ddd, *J=* 18.0, 13.5, 5.4 Hz, 1H), 2.67 - 2.55 (m, 2H), 2.41 - 2.28 (m, 1H), 2.05 - 1.84 (m, 3H). LC-MS: [M+H] ⁺ =904.9, 473.2

### Synthesis method of Compound UB-181032

### Step 1: UB-181032 (V2537-005)

### The method is similar to General Method 3

¹H NMR (400 MHz, DMSO-*d₆*) δ 10.99 (s, 1H), 8.98 (s, 2H), 7.93 (s, 1H), 7.71 (t, *J* = 11.6 Hz, 3H), 7.56 (s, 1H), 7.38 (d, *J* = 4.5 Hz, 1H), 7.22 - 7.07 (m, 3H), 6.99 (d, *J* = 3.6 Hz, 1H), 6.94 - 6.81 (m, 1H), 6.75 - 6.65 (m, 1H), 5.09 (dd, *J=* 13.2, 5.1 Hz, 1H), 4.54 (s, 1H), 4.50 - 4.26 (m, 2H), 4.03 (s, 2H), 3.63 - 3.49 (m, 4H), 3.37 (t, *J* = 6.3 Hz, 2H), 3.23 - 3.16 (m, 2H), 3.06 (s, 2H), 2.99 - 2.83 (m, 2H), 2.70 - 2.57 (m, 1H), 2.42 - 2.25 (m, 1H), 2.07 - 1.70 (m, 9H), 1.69 - 1.59 (m, 2H). LC-MS: [M+H] ⁺ =906

### Synthesis method of Compound UB-181035

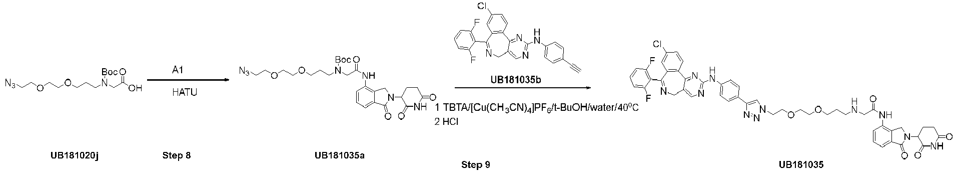

### Step 1: UB-181035a (V2537-002)

Compound **UB-181020j** (180mg, 0.3mmol), and Pd/C (30mg) were dissolved in DCM/MeOH=10/1 (10mL), the mixture was reacted at room temperature for 2 hours under H₂ atmosphere. The reaction solution was added 100mL DCM/MeOH=10/1, filtered after being enough stirring, the filtrate was concentrated to obtain target product **UB-181035a** (160mg, 100% yield) as a white solid. LC-MS: [M+H] ⁺ =588.3

### Step 2: UB-181035 (V2537-009)

### The method is similar to General Method 3

¹H NMR (400 MHz, DMSO-*d₆*) δ 11.24 (s, 1H), 10.99 (s, 1H), 10.02 (s, 1H), 9.14 (s, 2H), 8.69 (s, 1H), 8.47 (s, 1H), 8.31 (d, *J* = 8.5 Hz, 1H), 7.97 - 7.86 (m, 4H), 7.80 (d, *J* = 8.5 Hz, 2H), 7.70 (s, 2H), 7.55 (td, *J* = 8.5, 4.1 Hz, 1H), 7.34 (d, *J* = 2.2 Hz, 1H), 7.17 (t, *J* = 9.0 Hz, 2H), 5.09 (dd, *J=* 13.3, 5.1 Hz, 1H), 4.50 - 4.24 (m, 6H), 4.04 - 3.86 (m, 4H), 3.62 - 3.42 (m, 6H), 3.10 - 2.98 (m, 2H), 2.91 (ddd, *J* = 18.0, 13.5, 5.4 Hz, 1H), 2.67 - 2.55 (m, 2H), 2.41 - 2.28 (m, 1H), 2.05 - 1.84 (m, 3H). LC-MS: [M+H] ⁺ =904.9, 473.2

### Synthesis method of Compound UB-181040

### Step 1: UB-181040 (V2537-012)

### The method is similar to General Method 4

¹H NMR (400 MHz, DMSO-*d₆*) δ 11.97 (s, 1H), 11.05 (s, 1H), 10.87 (s, 1H), 10.04 (s, 1H), 9.26 (s, 2H), 8.91 (s, 1H), 8.63 (s, 1H), 8.32 (s, 1H), 7.86 (d, *J=* 23.5 Hz, 2H), 7.73 - 7.38 (m, 6H), 7.21 (s, 1H), 6.91 (s, 1H), 5.17 (m, 1H), 4.65 - 4.33 (m, 3H), 4.06 (s, 3H), 3.46 - 3.36 (m, 7H), 3.23 (s, 4H), 3.06 (s, 4H), 2.81 (s, 3H), 2.68 - 2.61 (m, 1H), 2.36 - 2.23 (m, 1H), 2.11 - 1.87 (m, 3H), 1.34 - 1.22 (m, 2H). LC-MS: [M+H] ⁺ =925.7

### Synthesis method of Compound UB-181047

### Step 1: UB-181047 (V2537-018)

### The method is similar to General Method 4

¹H NMR (400 MHz, DMSO-*d₆*) δ 12.06 (s, 1H), 11.43 (s, 1H), 10.99 (s, 1H), 10.24 (s, 1H), 9.28 (s, 2H), 8.96 (d, *J* = 4.9 Hz, 1H), 8.59 (d, *J* = 8.4 Hz, 1H), 8.34 (s, 1H), 7.99 (s, 1H), 7.85 (d, *J* = 7.8 Hz, 1H), 7.77 - 7.60 (m, 6H), 7.55 (t, *J* = 7.9 Hz, 1H), 7.22 (t, *J* = 7.6 Hz, 1H), 5.09 (dd, *J* = 13.2, 5.0 Hz, 1H), 4.51 - 4.29 (m, 2H), 4.06 (d, *J* = 6.0 Hz, 2H), 3.63 - 3.40 (m, 12H), 3.24 (d, *J=* 6.3 Hz, 2H), 3.07 (p, *J=* 6.7 Hz, 2H), 3.00 - 2.78 (m, 4H), 2.60 (d, *J=* 16.9 Hz, 1H), 2.45 - 2.33 (m, 1H), 1.98 (dh, *J* = 27.2, 6.6 Hz, 3H), 1.36 - 1.23 (m, 1H). LC-MS: [M+H] ⁺ =925.7

### Synthesis method of Compound UB-181048

### Step 1: UB-181048 (V2537-019)

### The method is similar to General Method 4

¹H NMR (400 MHz, DMSO-*d₆*) δ 11.99 (s, 1H), 11.05 (s, 1H), 10.90 (s, 1H), 10.11 (d, *J* = 16.7 Hz, 1H), 9.38 - 9.20 (m, 2H), 8.93 (t, *J=* 5.4 Hz, 1H), 8.61 (d, *J=* 8.4 Hz, 1H), 8.32 (s, 1H), 7.96 - 7.75 (m, 2H), 7.72 - 7.50 (m, 7H), 7.30 - 7.15 (m, 1H), 5.16 (dd, *J=* 13.2, 5.1 Hz, 1H), 4.56 - 4.33 (m, 2H), 4.04 (s, 2H), 3.40 (d, *J* = 18.0 Hz, 8H), 3.05 - 2.85 (m, 7H), 2.81 (d, *J=* 4.3 Hz, 3H), 2.63 (d, *J=* 16.9 Hz, 1H), 2.28 (m, 1H), 2.03 (m, 1H), 1.78 - 1.71 (m, 2H), 1.59 - 1.46 (m, 6H), 1.27 (d, *J* = 6.8 Hz, 2H). LC-MS: [M+H] ⁺ =925.7

### Synthesis method of Compound UB-181049

### Step 1: UB-181049 (V2537-020)

### The method is similar to General Method 4

¹H NMR (400 MHz, DMSO-*d₆*) δ 11.95 (s, 1H), 11.30 (s, 1H), 10.99 (s, 1H), 10.01 (s, 1H), 9.21 (s, 2H), 8.89 (d, *J=* 4.7 Hz, 1H), 8.63 (d, *J=* 8.5 Hz, 1H), 8.31 (s, 1H), 7.97 (s, 1H), 7.82 (d, *J=* 7.8 Hz, 1H), 7.69 (d, *J=* 26.9 Hz, 4H), 7.53 (t, *J=* 8.0 Hz, 3H), 7.20 (t, *J=* 7.5 Hz, 1H), 5.09 (dd, *J=* 13.3, 5.1 Hz, 1H), 4.54 - 4.25 (m, 2H), 4.03 (t, *J=* 5.6 Hz, 2H), 3.42 - 3.35 (m, 8H), 3.15 - 2.85 (m, 7H), 2.81 (d, *J* = 4.5 Hz, 3H), 2.64 - 2.58 (m, 1H), 2.46 - 2.31 (m, 1H), 2.03 - 1.96 (m, 2H), 1.78 - 1.70 (m, 2H), 1.64 - 1.49 (m, 6H), 1.24 (d, *J=* 3.6 Hz, 2H). LC-MS: [M+H] ⁺ =925.7

### Synthesis method of Compound UB-181059

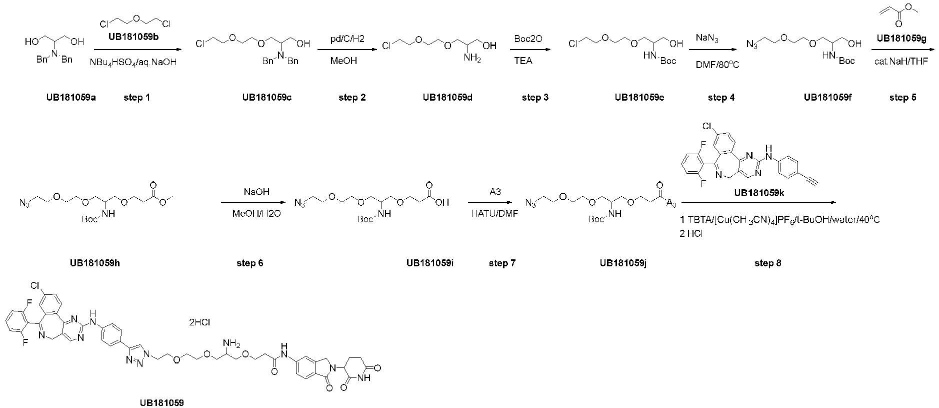

### Step 1: UB-181059c (V907-066)

Compound **UBI-1059a** (10 g, 37 mmol) was dissolved in dichloroether (50 mL), N(B_{U})₄HSO₄ (12.5 g, 37 mmol) was added. The mixture was cooled to 0°C, slowly added 50% NaOH aqueous solution (100 mL). The reaction was reacted at room temperature for 18 hours. The reaction solution was added water, and extracted with dichloromethane (80 mL*2). The dichloromethane layers were combined, dried and concentrated to give the crude product. The crude product was purified by silica gel column (PE/EA=1/5) to obtain target product **UB-181059c** (8.8 g, 63% yield) as a yellow oil. ¹H NMR (400 MHz, Chloroform-*d*) δ 7.43 - 7.14 (m, 10H), 3.86 (d, *J=* 13.4 Hz, 2H), 3.81 - 3.75 (m, 3H), 3.72 - 3.52 (m, 11H), 3.29 - 3.00 (m, 1H), 2.89 (s, 1H).

### Step 2: UB-181059d (V2312-139)

**UB-181059c** (5 g, 13.2 mmol) was dissolved in methanol, Pd/C (2 g) was added under nitrogen protection, the reaction system was purged with hydrogen for three times, reacted at room temperature for 2 h under hydrogen atmosphere. The reaction solution was filtered and evaporated to dryness in vacuum to give UB-181059d (2.8 g, 90% yield) as yellow oil.

¹H NMR (400 MHz, Chloroform-*d*) δ 3.80 - 3.73 (m, 2H), 3.66 (dddd, *J* = 8.2, 6.0, 3.2, 1.5 Hz, 7H), 3.60 - 3.46 (m, 4H), 3.16 (tt, *J* = 6.3, 4.5 Hz, 1H), 3.01 (s, 4H). LCMS [M+H]⁺ = 198.

### Step 3: UB-181059e (V2312-144)

**UB-181059d** (2.8 g, 14.2 mmol) and BOC2O (4.0 g, 17.04 mmol) were dissolved in THF, then NaHCO3(aq) (2.4 g, 28.4 mmol) was added, reaction solution was reacted at room temperature for 18 h. The reaction solution was extracted with water and ethyl acetate, washed with saturated brine. The combined organic layers were dried and filtered over Na2SO4. The solvent was removed in vacuum to obtain crude product, and the crude product was purified via silica gel column chromatography (EA/PE=1/5) to obtain **UB-181059e** (3.0 g, 71% yield) as a light yellow oil.

¹H NMR (400 MHz, Chloroform-*d*) δ 5.25 (s, 1H), 3.86 - 3.59 (m, 12H), 2.53 (s, 1H), 1.45 (s, 9H). LCMS [M+H] ⁺ = 298.

### Step 4: UB-181059f (V2312-148)

**UB-181059e** (3.0 g, 10.1 mmol) was dissolved in DMF (30 mL), then NaN3 (1.32 g, 20.2 mmol) was added, the reaction solution was warmed up to 80 °C and reacted for 16 h. The reaction solution was filtered, then extracted with water and ethyl acetate, washed with saturated brine. The combined organic layers were dried and filtered over Na2SO4. The solvent was removed in vacuum to obtain crude product, and the crude product was purified via silica gel column chromatography (EA/PE=1/10) to obtain **UB-181059f** (2.1 g, 68% yield) as a yellow oil.

¹H NMR (400 MHz, Chloroform-*d*) δ 5.29 (d, *J* = 14.5 Hz, 1H), 3.88 - 3.73 (m, 2H), 3.72 - 3.55 (m, 9H), 3.40 (dd, *J=* 5.6, 4.3 Hz, 2H), 2.67 (s, 1H), 1.45 (s, 9H). LCMS [M+H]⁺ = 305.

### Step 5: UB-181059h (V2595-003)

**UB-181059f** (500 mg, 1.64 mmol) and **UB-181059g** (283 mg, 3.3 mmol) were dissolved in anhydrous THF, then NaH (4 mg, 0.08 mmol) was added. The reaction solution was reacted at room temperature for 18h. The reaction solution was extracted with water and ethyl acetate, washed with saturated brine. The combined organic layers were dried and filtered over Na2SO4. The solvent was removed in vacuum to obtain crude product, and the crude product was purified via silica gel column chromatography (EA/PE=1/5) to obtain **UB-181059h** (140 mg, 22% yield) as a light yellow oil.

¹H NMR (400 MHz, Chloroform-*d*) δ 3.73 (td, *J* = 6.3, 1.7 Hz, 2H), 3.70 - 3.65 (m, 5H), 3.63 (s, 4H), 3.57 (dd, *J=* 9.5, 4.3 Hz, 2H), 3.48 (ddd, *J=* 9.4, 6.1, 3.4 Hz, 2H), 3.42 - 3.36 (m, 2H), 2.57 (t, *J=* 6.3 Hz, 2H), 1.62 (s, 3H), 1.44 (s, 9H). LCMS [M+H]⁺ = 390.

### Step 6: UB-181059i (V2595-031)

**UB-181059h** (140 mg, 0.376 mmol) was dissolved in methanol, then NaOH (60 mg, 1.5 mmol, aqueous solution) was added, and the mixture was reacted at room temperature for 2 h. To the reaction solution was added water, and methanol was removed by rotary evaporation in vacuum, the reaction solution was extracted with ethyl acetate, and resulting aqueous phase was adjusted with 1 M diluted hydrochloric acid to pH about 4, then extracted with ethyl acetate again, and the resulting organic phase was rotary evaporated in vacuum to obtain UB-181059i (110 mg, 80% yield) as a yellow oil. LCMS [M+H] ⁺ = 377.

### Step 7: UB-181059j (V2595-018)

**UB-181059i** (320 mg, 0.85 mmol), A3 (220 mg, 085 mmol), HATU (810 mg, 2.12 mmol), and DIPEA (330 mg, 2.55 mmol) were dissolved in DMF, the mixture was reacted at room temperature for 18 hours. The reaction solution was filtered, then extracted with water and ethyl acetate, washed with saturated brine. The combined organic layers were dried and filtered over Na2SO4. The solvent was removed in vacuum to obtain crude product, and the crude product was purified via silica gel column chromatography (MeOH/DCM=1/15) to obtain **UB-181059j** (110 mg, 60% yield) as a yellow solid. LCMS [M+H] ⁺ = 618.

### Step 8: UB-181059 (V2537-026)

### The method is similar to General Method 3

¹H NMR (400 MHz, DMSO-*d₆*) δ 10.98 (s, 1H), 10.55 (s, 1H), 10.01 (s, 1H), 8.69 (s, 1H), 8.45 (s, 1H), 8.30 (d, *J=* 8.4 Hz, 1H), 8.10-8.01 (m, 4H), 7.93 - 7.78 (m, 4H), 7.72 - 7.51 (m, 3H), 7.33 (s, 1H), 7.26 - 7.11 (m, 2H), 5.09 (d, *J=* 5.1 Hz, 1H), 4.56 (t, *J=* 5.2 Hz, 2H), 4.45 - 4.22 (m, 2H), 3.93 - 3.67 (m, 3H), 3.39 (s, 6H), 2.91 (t, *J=* 16.4 Hz, 2H), 2.72 - 2.54 (m, 1H), 2.42 - 2.26 (m, 5H), 2.09 - 1.90 (m, 1H), 1.24 (s, 2H). LC-MS: [M+H] ⁺ =974.3

### Synthesis method of Compound UB-181064

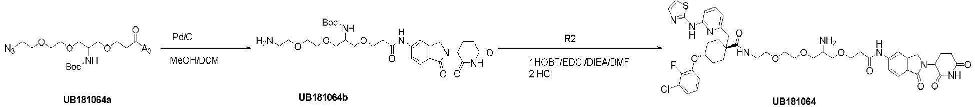

### Step 1: UB-181064b (V2537-028)

Compound **UB-181064a** (V2537-028), Pd/C (10mg) were dissolved in DCM/MeOH=10/1 (10mL), the mixture was reacted at room temperature for 2 hours under H₂ atmosphere. The reaction solution was added 100mL DCM/MeOH=10/1, filtered after being enough stirring, the filtrate was concentrated to obtain target product (180mg, 94.7% yield) as a white solid. LC-MS: [M+H] ⁺ =561.3

### Step 2: UB-181064 (V2537-031)

### The method is similar to General Method 2

¹H NMR (400 MHz, DMSO-*d₆*) δ 10.98 (s, 1H), 10.59 (s, 1H), 8.14 (s, 3H), 8.01 (s, 1H), 7.80 - 7.64 (m, 4H), 7.49 (t, *J=* 3.1 Hz, 1H), 7.20 - 7.04 (m, 5H), 6.81 (d, *J=* 7.4 Hz, 1H), 5.14 - 5.07 (m, 1H), 4.55 (s, 1H), 4.46 - 4.23 (m, 2H), 3.76 (q, *J=* 5.9 Hz, 2H), 3.66 - 3.46 (m, 8H), 3.38 (m, 3H), 3.19 (t, *J* = 5.9 Hz, 2H), 3.01 - 2.85 (m, 3H), 2.68 (t, *J* = 6.2 Hz, 2H), 2.63 - 2.56 (m, 1H), 2.43 - 2.30 (m, 1H), 2.03 - 1.63 (m, 9H). LC-MS: [M+H] ⁺ =936

### Synthesis method of Compound UB-181065

### Step 1: UB-181065 (V2537-032)

### The method is similar to General Method 4

¹H NMR (400 MHz, DMSO-*d₆*) δ 11.96 (s, 1H), 10.98 (s, 1H), 10.64 (s, 1H), 10.01 (s, 1H), 8.97 - 8.88 (m, 1H), 8.63 (d, *J* = 8.5 Hz, 1H), 8.31 (s, 1H), 8.18 (d, *J=* 5.3 Hz, 3H), 8.03 (s, 1H), 7.82 (d, *J* = 7.7 Hz, 1H), 7.74 - 7.63 (m, 3H), 7.57 - 7.43 (m, 3H), 7.20 (t, *J* = 7.5 Hz, 1H), 6.90 (s, 1H), 5.12 - 5.09 (m, 1H), 4.46 - 4.29 (m, 2H), 3.54 (s, 6H), 3.45 - 3.36 (m, 10H), 3.23 (d, *J* = 6.2 Hz, 2H), 2.98 - 2.77 (m, 4H), 2.71 (d, *J* = 6.2 Hz, 2H), 2.60 (d, *J* = 17.2 Hz, 1H), 2.43 - 2.31 (m, 1H), 2.05 - 1.95 (m, 2H). LC-MS: [M+H] ⁺ =956.

### Synthesis method of Compound UB-181073

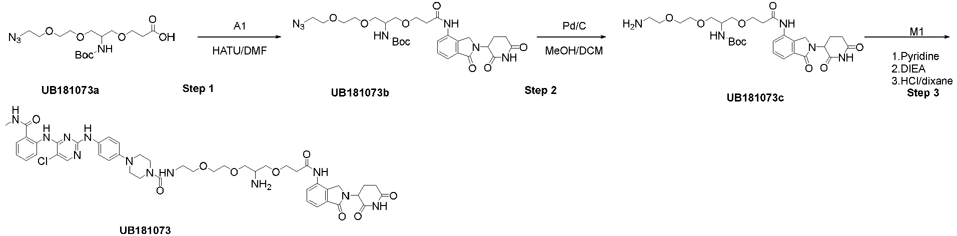

### Step 1: UB-181073b (V2595-032)

**UB-181073a** (185 mg, 0.5 mmol), A1 (127 mg, 0.5 mmol), HATU (467 mg, 1.25 mmol), and DIPEA (190 mg, 1.5 mmol) were dissolved in DMF, and the mixture was reacted at room temperature for 18 hours. The reaction solution was filtered, then extracted with water and ethyl acetate, washed with saturated brine. The combined organic layers were dried and filtered over Na₂SO₄. The solvent was removed in vacuum to obtain crude product, and the crude product was purified via silica gel column chromatography (MeOH/DCM=1/15) to obtain **UB-181073b** (85 mg, 28% yield) as a yellow solid. LCMS [M+H]⁺ = 618.

### Step 2: UB-181073c (V2595-034)

**UB-181073b** (85 mg, 0.14mmol) was dissolved in DCM/MeOH(v/v = 10 mL), Pd/C (50 mg) was added under nitrogen protection, the reaction system was purged with hydrogen for three times, reacted at room temperature for 2 h under hydrogen atmosphere. The reaction solution was filtered and evaporated to dryness in vacuum to give **UB-181073c** (70 mg, 86% yield) as yellow solid. LCMS [M+H]⁺ = 592.

### Step 3: UB-181073 (v2537-039)

### The method is similar to General Method 4

¹H NMR (400 MHz, DMSO-*d₆*) δ 11.89 (s, 1H), 11.02 (s, 1H), 10.13 (d, *J* = 10.5 Hz, 1H), 9.84 (d, *J=* 21.1 Hz, 1H), 8.86 (s, 1H), 8.67 (s, 1H), 8.37 - 8.13 (m, 4H), 7.94 - 7.81 (m, 2H), 7.63 (s, 2H), 7.59 - 7.34 (m, 5H), 7.18 (t, *J=* 7.7 Hz, 1H), 6.86 (s, 1H), 5.15 (dd, *J=* 13.3, 5.0 Hz, 1H), 4.41 (q, *J* = 17.6 Hz, 2H), 3.56 - 3.17 (m, 19H), 3.00 - 2.78 (m, 5H), 2.75 - 2.60 (m, 4H), 2.30 (d, *J* = 20.0 Hz, 2H), 2.03 (s, 2H), 1.33 - 1.26 (m, 2H). LC-MS: [M+H] ⁺ =956.

### Synthesis method of Compound UB-181085

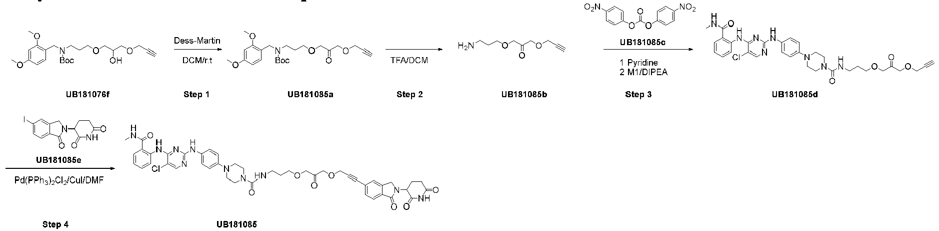

### Step 1: UB-181085a (V2595-077)

UB-181076f (1.1 g, 2.5 mmol) was dissolved in DCM (50 ml), then Dess-Martin (2.4 g) was added, the mixture was reacted at room temperature for 40 min. The reaction mixture was diluted with distilled water/brine and the product was extracted with ethyl acetate (80 ml * 3). The combined organic layers were dried and filtered over Na2SO4. The solvent was removed in vacuum to obtain crude product, and the crude product was purified via silica gel column chromatography (MeOH/DCM=1/10) to obtain **UB-181085a** (750 mg, 70% yield) as a yellow oil. LCMS [M+H] ⁺ =436.

### Step 2: UB-181085b (V2537-053)

**UB-181085a** (80mg, 0.18mmol) was dissolved in TFA (1mL) and DCM (3mL), the mixture was reacted at room temperature for 1 hour. The reaction solution was concentrated to obtain **UB-181085b** (50mg, 100% yield). LC-MS: [M+H] ⁺ = 186.2

### Step 3: UB-181085c (V2537-054)

Compound **UB-181085b** (22mg, 0.12mmol), and **UB-181085c** (40mg, 0.12mmol) were dissolved in pyridine, the mixture was reacted at room temperature for 2 hours. M1 (44mg, 0.1mmol) and DIPEA (0.3mL) were added, then continued to react for 1 hour. The reaction solution was concentrated, then purified by preparative TLC (DCM/MeOH=15/1) to obtain white target product **UB-181085c** (20mg, 26% yield). LC-MS: [M+H] ⁺ = 649

### Step 4: UB-181085 (V2537-057)

Under the N₂ protection, **UB-181085c** (30mg, 0.045mmol), **UB-181085e** (36mg, 0.045mmol), Pd(PPh₃)₂Cl₂ (3mg, 0.0045mmol), and CuI (6mg, 0.045mmol) were dissolved in TEA (13ul, 0.09mmol) and DMF (6mL), the mixture was reacted at 40°C for 16 hours. The reaction solution was concentrated, then purified by preparative TLC to obtain **UB-181085** (1mg, 2.56% yield). LC-MS: [M+H] ⁺= 892

### Synthesis method of Compound UB-181181

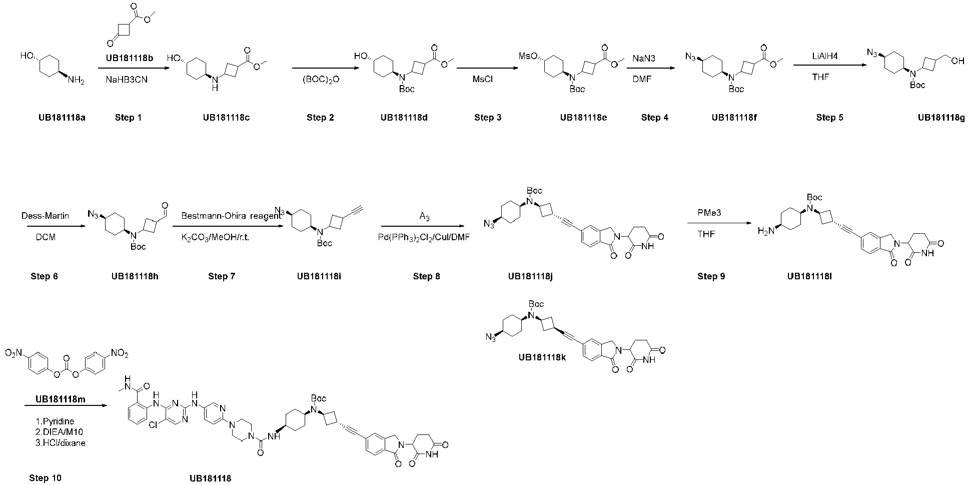

### Step 1: UB-181181c (V2954-006)

Compound **UB-181181a** (5g, 43mmol), and **UB-181181a** (5.5g, 43mmol) were dissolved in methanol (100mL), AcOH (10mL) was added. The mixture was reacted at 40°C for 16 hours. The reaction solution was added NaBH₃CN (5.4g, 86mmol), followed by reacting at room temperature for 2 hours. The reaction liquid was directly used in the next step. LC-MS: [M+H] ⁺ = 228

### Step 2: UB-181181d (V2954-010)

The reaction solution of **UB-181181c** (5g, 22mmol) was added to methanol (100mL), NaHCO₃ (3.7g, 44mmol) and (Boc)2O (4.8g, 22mmol) were added, the mixture was reacted at 40°C for 30 minutes. The reaction solution was filtered, and the filtrate was concentrated to obtain crude product. The crude product was purified by silica gel column (PE/WA=1/1) to obtain target product **UB-181181d** (7g, 97% yield). LC-MS: [M+H] ⁺ = 328

### Step 3: UB-181181e (V2954-012)

Compound **UB-181181d** (10g, 31mmol) was dissolved in DCM (50mL), MsCl (11g, 92mmol) and TEA (13mL, 92mmol) were added, the mixture was reacted at 40°C for 2 hours. The reaction solution was added water, and extracted with dichloromethane (10 mL*3). The organic phases were combined, dried and concentrated to give the crude product, which was directly used in the next step.

### Step 4: UB-181181f (V2954-013)

Compound **UB-181181e** (7g, 17mmol) was dissolved in DMF (20mL), NaN₃ (1.68g, 26mmol) was added, the mixture was reacted at 80°C for 16 hours. The reaction solution was added water, and extracted with ethyl acetate (10 mL*3). The organic phases were combined, dried and concentrated to give the crude product. The crude product was purified by silica gel column (PE/EA=10/1) to obtain target product **UB-181181f** (4g, 66.6% yield) as a colorless oi. ¹H NMR (400 MHz, Chloroform-*d*) δ 4.16 - 3.98 (m, 1H), 3.86 (t, *J=* 3.0 Hz, 1H), 3.68 (s, 3H), 3.61 - 3.44 (m, 1H), 2.81 - 2.54 (m, 3H), 2.48 - 2.24 (m, 2H), 2.12 - 1.88 (m, 4H), 1.50 (s, 14H). LC-MS: [M+H] ⁺= 354

### Step 5: UB-181181g (V2954-015)

Compound **UB-181181f** (2g, 5.7mmol) was dissolved in THF (30mL) and cooled to 0°C, LiAlH4 (6.8mL, 6.8mmol) was added slowly. The mixture was reacted at 0°C for 2 hours. To the reaction solution was added 10H₂O Na₂SO₄, then stirred for 30 minutes. The mixture was filtered and the filtrate was concentrated to obtain the crude product, which was purified by silica gel column to obtain the target product **UB-181181g** (700mg, 37.8% yield) as a colorless oil. ¹H NMR (400 MHz, Chloroform-*d*) δ 4.01 - 3.89 (m, 1H), 3.86 (p, *J* = 3.0 Hz, 1H), 3.62 (d, *J=* 5.0 Hz, 3H), 2.32 - 2.11 (m, 4H), 1.94 (m, 4H), 1.50 (m, 13H). LC-MS: [M+H] ⁺= 325

### Step 6: UB-181181h (V2954-017)

Compound **UB-181181g** (700mg, 2.16mmol) was dissolved in DCM (50mL), Dess-Martin (1.1g, 2.59mmol) was added at 0°C, then the mixture was reacted at room temperature for 4 hours. The reaction solution was added NaHCO₃ aqueous solution, and extracted with dichloromethane. The organic phases were combined, dried and concentrated to give the crude product. The crude product was purified by silica gel column to obtain target product **UB-181181h** (470mg, 67% yield). LC-MS: [M+H] ⁺= 323

### Step 7: UB-181181i (V2954-020)

Compound **UB-181181h** (100mg, 0.31mmol) in MeOH (10mL) was added Bestmann-Ohira reagent (71mg, 0.37mmol) and K₂CO₃ (86mg, 0.37mmol). The mixture was stirred at 40°C for 2h.Then the mixture was concentrated and purified by silica gel column (PE/EA=10/1) to get **UB-181181i** (50mg, 50.5% yield). ¹H NMR (400 MHz, Chloroform-*d*) δ 3.91 - 3.84 (m, 2H), 3.67 (s, 1H), 2.98 - 2.80 (m, 1H), 2.71 - 2.54 (m, 2H), 2.49 - 2.39 (m, 2H), 2.12 (t, *J=* 0.8 Hz, 1H), 1.98 - 1.89 (m, 4H), 1.76 - 1.46 (m, 13H). LC-MS: [M+H] ⁺= 319

### Step 8: UB-181181j (V2954-021)

Under the N₂ protection, **UB-181181i** (44mg, 0.12mmol), **A3** (50mg, 0.12mmol), Pd(PPh₃)₂Cl₂ (8mg, 0.012mmol), and CuI (5mg, 0.024mmol) were dissolved in TEA (35uL, 0.24mmol) and DMF (8mL), the mixture was reacted at 80°C for 2 hours. The reaction solution was filtered, and concentrated to obtain crude product. The crude product was purified by Prep-HPLC to obtain **UB-181181j** (8mg, 12% yield) and **UB-181181jk** (2mg, 3% yield).

**UB-181181j:** ¹H NMR (400 MHz, Chloroform-*d*) δ 8.04 (s, 1H), 7.80 (d, *J=* 7.9 Hz, 1H), 7.54 - 7.39 (m, 2H), 5.22 (dd, *J=* 13.2, 5.2 Hz, 1H), 4.55 - 4.24 (m, 2H), 3.94 - 3.78 (m, 2H), 3.74 (s, 1H), 3.02 - 2.70 (m, 5H), 2.58 - 2.47 (m, 2H), 2.42 - 2.16 (m, 2H), 2.01 - 1.89 (m, 4H), 1.71 - 1.45 (m, 13H). LC-MS: [M+H] ⁺= 561

**UB-181181k:¹H** NMR (400 MHz, Chloroform-d) δ 8.04 (s, 1H), 7.82 (d, J = 7.8 Hz, 1H), 7.58 - 7.43 (m, 2H), 5.22 (dd, J = 13.3, 5.2 Hz, 1H), 4.56 - 4.25 (m, 3H), 3.87 (s, 1H), 3.70 (m, 1H), 3.20 (t, J = 10.3 Hz, 1H), 3.05 - 2.72 (m, 4H), 2.42 - 2.17 (m, 4H), 2.04 - 1.84 (m, 4H), 1.75 - 1.43 (m, 13H). LC-MS: [M+H] ⁺= 561

### Step 9: UB-181181l (V2954-022)

**UB-181181j** (50mg, 0.07mmol) was dissolved in THF (5mL), PMe₃ (1mL) was added, the mixture was reacted at 50°C for 6 hours. The reaction solution was concentrated, then purified by preparative TLC (MeOH/DCM=1/10) to obtain target product **UB-181181l** (15mg, 40.5% yield) as a white solid. LC-MS: [M+H] ⁺= 535

### Step 10: UB-181181 (V2954-025)

### The method is similar to General Method 4

¹H NMR (400 MHz, DMSO-*d₆*) δ 12.23 (s, 1H), 11.00 (s, 1H), 9.27 (s,2H), 8.99 - 8.81 (m, 2H), 8.38 (s, 1H), 8.05 - 7.91 (m, 2H), 7.84 (d, *J=* 7.9 Hz, 1H), 7.81 - 7.48 (m, 5H), 7.24 (t, *J* = 7.6 Hz, 1H), 6.12 (d, *J* = 4.5 Hz, 1H), 5.11 (dd, *J=* 13.3, 5.1 Hz, 1H), 4.50 - 4.29 (m, 2H), 3.71 (d, *J=* 37.9 Hz, 2H), 3.46 - 3.34 (m, 4H), 3.21 - 3.00 (m, 6H), 2.82 (d, *J=* 4.5 Hz, 3H), 2.73 - 2.58 (m, 3H), 2.44 - 2.31 (m, 1H), 2.01 (dd, *J=* 10.3, 4.8 Hz, 2H), 1.90 - 1.68 (m, 6H), 1.57 - 1.46 (m, 2H), 1.25 - 1.16 (m, 2H). LC-MS: [M+H] ⁺ = 900

### Synthesis method of Compound UB-181050

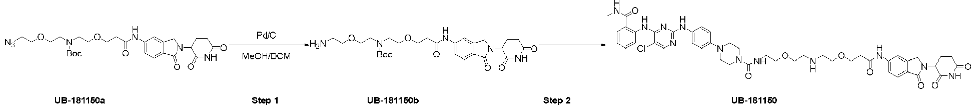

### Step 1: UB-181050b (V2235-144)

Compound **UB-181050a** (30 mg, 0.05 mmol) was dissolved in methanol /dichloromethane (3/6 mL), then a catalytic amount of palladium carbon was added, and the mixture was reacted at room temperature for 1 hour under hydrogen condition. The reaction solution was filtered, the filtrate was concentrated to obtain crude product **UB-181050b** (30 mg, yield 100%) as a yellow oil. The crude product was directly used in the next reaction. LCMS [M+H] ⁺ = 562.2

### Step 2: UB-181050 (V2235-148)

### The method is similar to General Method 3

¹H NMR (400 MHz, DMSO-*d₆*) δ 11.94 (s, 1H), 10.97 (s, 1H), 10.59 (s, 1H), 9.94 (s, 1H), 9.02 - 8.77 (m, 3H), 8.65 (d, *J=* 8.3 Hz, 1H), 8.29 (s, 1H), 8.01 (d, *J=* 1.7 Hz, 1H), 7.80 (dd, *J* = 8.0, 1.6 Hz, 1H), 7.67 (td, *J* = 9.2, 8.3, 4.4 Hz, 4H), 7.56 - 7.40 (m, 3H), 7.20 (td, *J* = 7.6, 1.2 Hz, 1H), 6.96 (s, 1H), 5.08 (dd, *J* = 13.3, 5.1 Hz, 1H), 4.54 - 4.23 (m, 4H), 3.76 (dt, *J* = 10.7, 5.7 Hz, 6H), 3.66 (t, *J=* 5.1 Hz, 2H), 3.46 (t, *J=* 5.7 Hz, 2H), 3.36 (s, 4H), 3.26 (d, *J=* 5.8 Hz, 2H), 3.14 (q, *J=* 5.7 Hz, 4H), 2.91 (ddd, *J* = 17.1, 13.5, 5.4 Hz, 1H), 2.81 (d, *J* = 4.4 Hz, 3H), 2.70 (t, *J=* 6.1 Hz, 2H), 2.64 - 2.56 (m, 1H), 2.42 - 2.30 (m, 1H), 2.05 - 1.91 (m, 1H). LCMS [M+H]⁺ = 926.0

### Synthesis method of Compound UB-181051

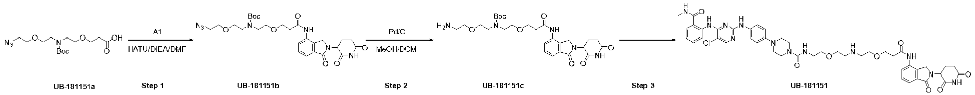

### Step 1: UB-181151b (V2591-001)

Compound **UB-181151a** (400 mg, 1.16 mmol) and HATU (879 mg, 2.13 mmol) were dissolved in DMF (20 ml), then DIPEA (447 mg, 3.47 mmol) was added, the mixture was reacted at room temperature for 1 hour. Then A1 (269 mg, 1.04 mmol) was added, and continued to react at room temperature for 12 hours. The reaction solution dried by rotary dryer to remove solvent and isolated by column chromatography (methanol/dichloromethane = 1/10) to obtain target product **UB-181151b** (315 mg, yield 46%) as a white solid.

¹H NMR (400 MHz, DMSO) δ 10.97 (s, 1H), 10.28 (s, 1H), 7.99 (s, 1H), 7.65 (d, *J* = 8.3 Hz, 1H), 7.59 (d, *J* = 9.4 Hz, 1H), 5.08 (dd, *J* = 13.3, 5.1 Hz, 1H), 4.35 (dd, *J* = 55.4, 17.3 Hz, 2H), 4.09 (q, *J=* 5.3 Hz, 2H), 3.71 (s, 2H), 3.54 - 3.45 (m, 6H), 3.40 - 3.35 (m, 2H), 3.18 (s, 2H), 2.95 - 2.85 (m, 1H), 2.63 - 2.56 (m, 3H), 2.41 - 2.32 (m, 1H), 2.03 - 1.95 (m, 1H), 1.37 (s, 9H). LCMS: [M+H] ⁺ = 588.6

### Step 2: UB-181051c (V2235-146)

Compound **UB-181051b** (30 mg, 0.05 mmol) was dissolved in methanol /dichloromethane (3/6 mL), and a catalytic amount of palladium carbon was added, and the mixture was reacted at room temperature for 1 hour under hydrogen condition. The reaction solution was filtered, the filtrate was concentrated to obtain crude product **UB-181051c** (30 mg, yield 100%) as a yellow oil. The crude product was directly used in the next reaction. LCMS [M+H] ⁺ = 562.2

### Step 3: UB-181051 (V2596-003)

### The method is similar to General Method 3

¹H NMR (400 MHz, DMSO-*d₆*) δ 11.91 (s, 1H), 11.02 (s, 1H), 10.12 (s, 1H), 9.89 (s, 1H), 9.12 - 8.77 (m, 3H), 8.66 (s, 1H), 8.29 (s, 1H), 7.83 (ddd, *J=* 18.0, 7.6, 1.7 Hz, 2H), 7.76 - 7.58 (m, 3H), 7.50 (tt, *J* = 13.8, 7.0 Hz, 4H), 7.19 (t, *J* = 7.6 Hz, 1H), 6.98 (s, 1H), 5.15 (dd, *J* = 13.3, 5.1 Hz, 1H), 4.56 - 4.30 (m, 2H), 4.22 (t, *J=* 6.5 Hz, 1H), 3.76 (m, 8H), 3.52 - 3.43 (m, 2H), 3.34 (s, 3H), 3.25 (d, *J=* 5.8 Hz, 2H), 3.15 (dt, *J* = 11.4, 4.2 Hz, 4H), 2.98 - 2.87 (m, 1H), 2.81 (d, *J* = 4.5 Hz, 3H), 2.70 (q, *J* = 7.3, 6.7 Hz, 1H), 2.66 - 2.57 (m, 2H), 2.40 - 2.26 (m, 1H), 2.18 - 1.93 (m, 1H), 1.75 - 1.55 (m, 1H), 1.50 - 1.33 (m, 1H). LCMS [M+H] ⁺ = 925.9

### Synthesis method of Compound UB-181053

### Step 1: UB-181053 (V2596-004)

### Method analogous to General Method 3

¹H NMR (400 MHz, DMSO-*d₆*) δ 12.00 (s, 1H), 11.03 (s, 1H), 10.30 (s, 1H), 10.11 (s, 1H), 9.18 (s, 2H), 8.89 (q, *J=* 4.5 Hz, 1H), 8.61 (d, *J=* 8.4 Hz, 1H), 8.33 (s, 1H), 7.84 (ddd, *J* = 15.5, 7.6, 1.7 Hz, 2H), 7.69 - 7.37 (m, 7H), 7.33 - 6.96 (m, 2H), 5.15 (dd, *J=* 13.3, 5.1 Hz, 1H), 3.66 - 3.33 (m, 10H), 3.24 (q, *J* = 6.6 Hz, 2H), 3.07 (p, *J* = 6.0 Hz, 2H), 3.02 - 2.87 (m, 3H), 2.81 (d, *J* = 4.4 Hz, 3H), 2.70 - 2.57 (m, 1H), 2.42 - 2.25 (m, 1H), 2.13 - 1.91 (m, 1H). LCMS [M+H]⁺ = 837.7

### Synthesis method of Compound UB-181054

### Step 1: UB-181054 (V2596-005)

### The method is similar to General Method 3

¹H NMR (400 MHz, DMSO-*d₆*) δ 11.94 (s, 1H), 10.98 (s, 1H), 10.76 (d, *J=* 5.2 Hz, 1H), 9.99 (s, 1H), 9.10 (s, 2H), 8.87 (t, *J=* 4.6 Hz, 1H), 8.64 (d, *J=* 8.3 Hz, 1H), 8.30 (s, 1H), 7.97 (s, 1H), 7.81 (dd, *J=* 8.0, 1.7 Hz, 1H), 7.81 - 7.44 (m, 6H), 7.20 (t, *J=* 7.5 Hz, 2H), 5.08 (dd, *J* = 13.2, 5.1 Hz, 1H), 4.58 - 4.14 (m, 2H), 3.48 - 3.32 (m, 8H), 3.25 (p, *J=* 6.8 Hz, 2H), 3.10 (ddt, *J=* 16.9, 9.8, 4.6 Hz, 3H), 3.01 - 2.86 (m, 3H), 2.81 (d, *J* = 4.5 Hz, 3H), 2.67 - 2.55 (m, 2H), 2.37 (td, *J=* 13.3, 4.7 Hz, 1H), 2.06 - 1.89 (m, 1H). LCMS [M+H]⁺ = 837.8

### Synthesis method of Compound UB-181084

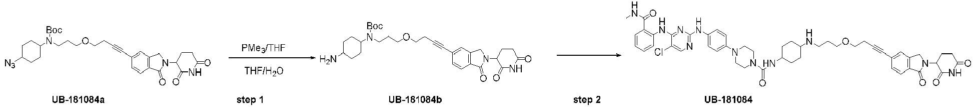

### Step 1: UB-181084b (V2596-043)

Compound **UB-181084a** (30 mg, 0.05 mmol) was dissolved in THF (3 mL), then 1M/L solution of trimethylphosphine in tetrahydrofuran (0.1 mL) was added, the mixture was reacted at 50°C for 1 hour. Then water (0.5 mL) was added, the mixture was reacted at 50 °C for 1 hour. The reaction solution was concentrated to obtain product **UB-181084b** (30 mg, yield 100%) as a yellow oil. The crude product was directly used in the next reaction. LCMS [M+H] ⁺ = 567.2

### Step 2: UB-181084 (V2596-045)

### The method is similar to General Method 3

¹H NMR (400 MHz, DMSO-*d₆*) δ 11.84 (s, 1H), 11.00 (s, 1H), 9.75 (s, 1H), 8.88 - 8.77 (m, 2H), 8.67 (s, 1H), 8.26 (s, 1H), 7.79 (dd, *J=* 8.0, 1.6 Hz, 1H), 7.70 (d, *J=* 7.8 Hz, 1H), 7.63 (d, *J=* 11.0 Hz, 2H), 7.51 (dt, *J* = 8.2, 4.0 Hz, 2H), 7.34 (s, 2H), 7.18 (t, *J=* 7.6 Hz, 1H), 6.16 (s, 1H), 5.11 (dd, *J=* 13.3, 5.1 Hz, 1H), 4.54 - 4.27 (m, 2H), 3.73 - 3.65 (m, 3H), 3.61 (t, *J=* 6.5 Hz, 3H), 3.56 (t, *J=* 5.9 Hz, 3H), 3.29 (s, 4H), 2.96 (d, *J=* 15.7 Hz, 3H), 2.89 (dd, *J=* 13.1, 5.0 Hz, 1H), 2.81 (d, *J* = 4.5 Hz, 2H), 2.73 (t, *J* = 6.5 Hz, 2H), 2.66 - 2.55 (m, 1H), 2.39 (qd, *J=* 13.3, 4.4 Hz, 1H), 2.08 - 1.71 (m, 9H), 1.57 - 1.40 (m, 2H). LCMS [M+H]⁺ = 930.8

### Synthesis method of Compound UB-181087

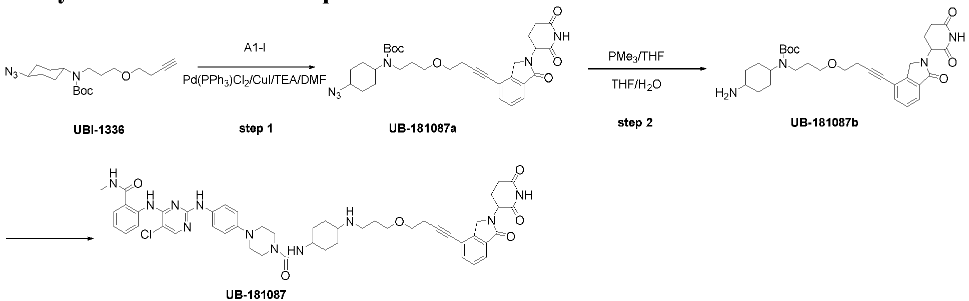

### Step 1: UB-181087a (V2596-050)

Compound **UBI-1338** (42 mg, 0.12 mmol), **A1-I** (32 mg, 0.12 mmol), Pd(PPh₃)₂Cl₂ (17 mg, 0.02 mmol), CuI (5 mg,0.02 mmol), and TEA (12 mg, 0.12 mmol) were dissolved in DMF (10 mL), then the mixture was reacted at 80°C for 2 hours. The reaction solution was filtered. Then the filtrate was concentrated and isolated by column chromatography (methanol/dichloromethane = 1/10) to obtain target product **UB-181087a(30** mg, yield 42%) as a brown oil. LCMS [M+H]⁺ = 593.7

### Step 2: UB-181087b (V2596-052)

Compound **UB-181084a** (30 mg, 0.05 mmol) was dissolved in THF (3 mL), then 1M/L solution of trimethylphosphine in tetrahydrofuran (0.1 mL) was added, the mixture was reacted at 50°C for 1 hour. Then water (0.5 mL) was added, the mixture was reacted at 50 °C for 1 hour. The reaction solution was concentrated to obtain crude product **UB-181087b** (30 mg, yield 100%) as a yellow oil. The crude product was directly used in the next reaction. LCMS [M+H] ⁺ = 567.2

### Step 3: UB-181087 (V2596-054)

**The method is similar to General Method 3**

¹H NMR (400 MHz, DMSO-*d₆*) δ 11.89 (s, 1H), 11.01 (s, 1H), 9.90 (s, 1H), 8.97 (s, 2H), 8.87 (q, *J=* 4.6 Hz, 1H), 8.66 (d, *J=* 8.4 Hz, 1H), 8.29 (s, 1H), 7.81 (dd, *J=* 7.9, 1.6 Hz, 1H), 7.72 (d, *J=* 7.5 Hz, 1H), 7.68 - 7.63 (m, 2H), 7.53 (t, *J=* 7.6 Hz, 3H), 7.19 (t, *J=* 7.5 Hz, 1H), 6.33 - 6.07 (m, 1H), 5.16 (dd, *J=* 13.3, 5.1 Hz, 1H), 4.56 - 4.05 (m, 2H), 3.71 - 3.67 (m, 3H), 3.63 (d, *J=* 6.8 Hz, 3H), 3.42 - 3.30 (m, 4H), 3.10 - 2.87 (m, 5H), 2.81 (d, *J=* 4.5 Hz, 2H), 2.75 (t, *J* = 6.6 Hz, 2H), 2.67 - 2.56 (m, 1H), 2.45 (d, *J* = 4.6 Hz, 1H), 2.08 - 1.90 (m, 4H), 1.81 (dd, *J=* 22.5, 12.8 Hz, 5H), 1.47 (d, *J=* 10.2 Hz, 2H), 1.20 (t, *J=* 7.3 Hz, 3H). LCMS [M+H]⁺ = 931.0

### Synthesis method of Compound UB-181095

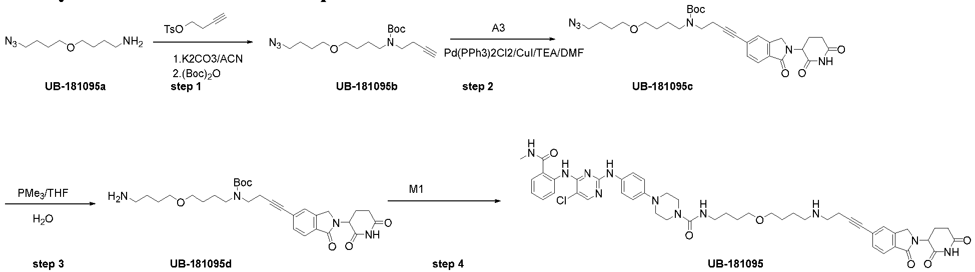

### Step 1: UB-181095b (V2596-059)

Compound **UB-181095a** (200 mg, 0.90 mmol) was dissolved in MeCN (30 mL), then K₂CO₃ (273 mg, 1.98 mmol) and butynyl p-toluenesulfonate(201 mg, 0.90 mmol) were added, the mixture was reacted at 70°C for 16 hours. Then (Boc)₂O (294 mg, 1.35 mmol) was added, and continued to react at room temperature for 2 hours. The reaction solution was washed once with water (10 mL), and extracted once with ethyl acetate (20 mL), the organic phase was concentrated, then isolated by column chromatography (ethyl acetate/petroleum ether=1/3) to obtain target product **UB-181095b** (150 mg, yield 28%) as a yellow oil. LCMS [M+H] ⁺ = 339.3

### Step 2: UB-181095c (V2596-060)

Compound **UB-181095b** (80 mg, 0.24 mmol), **A3-I** (88 mg, 0.24 mmol), Pd(PPh₃)₂Cl₂ (33 mg, 0.05 mmol), CuI (9 mg, 0.05 mmol), and TEA (24 mg, 0.24 mmol) were dissolved in DMF (10 mL), then the mixture was heated to 80°C for 2 hours by microwave reactor. The reaction solution was filtered. Then the filtrate was concentrated and isolated by column chromatography (methanol/dichloromethane = 1/10) to obtain target product **UB-181095c** (28 mg, yield 20%) as a brown solid. LCMS [M+H] ⁺ = 581.5

### Step 3: UB-181095d (V2596-062)

Compound **UB-181095c** (28 mg, 0.05 mmol) was dissolved in THF (3 mL), then 1M/L solution of trimethylphosphine in tetrahydrofuran (0.14 mL) was added, the mixture was reacted at 50°C for 1 hour. Then water (0.5 mL) was added, the mixture was reacted at 50 °C for 1 hour. The reaction solution was concentrated to obtain crude product **UB-181095d** (25 mg, yield 82%) as a yellow oil. The crude product was directly used in the next reaction. LCMS [M+H] ⁺ = 555.3

### Step 4: UB-181095 (V2596-066)

### The method is similar to General Method 3

¹H NMR (400 MHz, DMSO-*d₆*) δ 11.87 (s, 1H), 11.00 (s, 1H), 9.85 (s, 1H), 9.26 (s, 2H), 8.90 (dd, *J=* 18.2, 5.4 Hz, 2H), 8.66 (d, *J=* 8.5 Hz, 1H), 8.57 (s, 1H), 8.28 (s, 1H), 8.05 (t, *J* = 6.9 Hz, 1H), 7.81 (d, *J* = 7.9 Hz, 1H), 7.75 - 7.67 (m, 2H), 7.64 (d, *J* = 8.4 Hz, 1H), 7.58 (d, *J* = 7.9 Hz, 1H), 7.52 (t, *J* = 7.9 Hz, 1H), 7.43 (s, 1H), 7.19 (t, *J* = 7.5 Hz, 1H), 5.11 (dd, *J* = 13.2, 5.0 Hz, 1H), 4.58 - 4.11 (m, 2H), 3.41 - 3.29 (m, 9H), 3.15 (q, *J* = 6.6 Hz, 3H), 3.07 (d, *J* = 8.7 Hz, 2H), 2.96 (t, *J* = 7.3 Hz, 4H), 2.89 (dd, *J* = 12.9, 4.8 Hz, 1H), 2.81 (d, *J* = 4.5 Hz, 3H), 2.60 (d, *J* = 16.9 Hz, 2H), 2.45 - 2.29 (m, 1H), 2.08 - 1.88 (m, 2H), 1.71 (q, *J* = 7.8 Hz, 2H), 1.57 (q, *J=* 6.9 Hz, 2H), 1.52 - 1.38 (m, 4H). LCMS [M+H] ⁺ = 919.0

### Synthesis method of Compound UB-181098

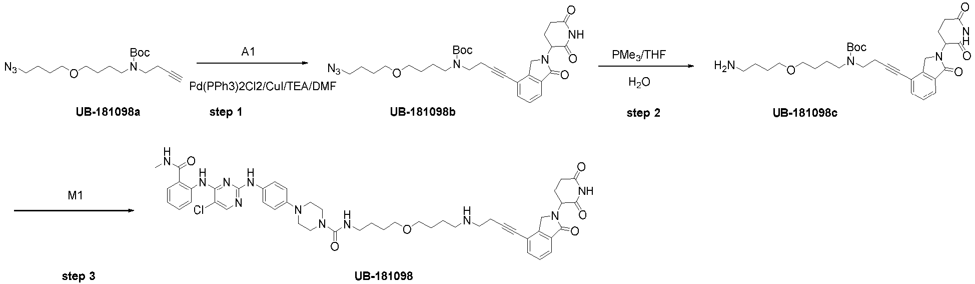

### Step 1: UB-181098b (V2596-061)

Compound **UB-181098a** (80 mg, 0.24 mmol), **A1-I** (88 mg, 0.24 mmol), Pd(PPh₃)₂Cl₂ (33 mg, 0.05 mmol), CuI (9 mg, 0.05 mmol), and TEA (24 mg, 0.24 mmol) were dissolved in DMF (10 mL), then the mixture was heated to 80°C and reacted for 2 hours by microwave synthesizer. The reaction solution was filtered. Then the filtrate was concentrated and isolated by column chromatography (methanol/dichloromethane = 1/10) to obtain target product **UB-181098b** (35 mg, yield 25%) as a brown solid. LCMS [M+H] ⁺ = 581.7

### Step 2: UB-181098c (V2596-064)

Compound **UB-181098b** (35 mg, 0.06 mmol) was dissolved in THF (3 mL), then 1M/L solution of trimethylphosphine in tetrahydrofuran (0.18 mL) was added, the mixture was reacted at 50°C for 1 hour. Then water (0.5 mL) was added, the mixture was reacted at 50 °C for 1 hour. The reaction solution was concentrated to obtain product **UB-181098c** (32 mg, yield 94%) as a yellow oil. The crude product was directly used in the next reaction. LCMS [M+H] ⁺ = 555.3

### Step 3: UB-181098 (V2596-067)

### The method is similar to General Method 3

LCMS [M+H] ⁺ = 918.8

### Compound UB-181102

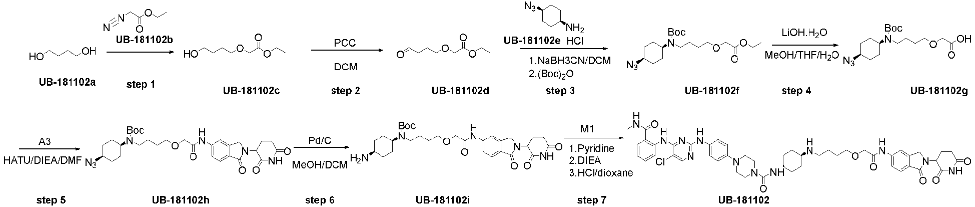

### Step 1: UB-181102c (V2596-056)

Compound **UB-181102a** (1.8 g, 20 mmol) was dissolved in dichloromethane (50 mL), then **UB-181102b** (1.15 g, 10 mmol) and BF3.Et₂O (0.127 mL) were added at 0°C, then the mixture was reacted at room temperature overnight. The reaction solution was concentrated and isolated by column chromatography (ethyl acetate/petroleum ether= 1/1) to obtain target product **UB-181102c** (1.4 g, yield 80%) as a colorless oil. LCMS [M+H] ⁺ = 177.2

### Step 2: UB-181102d (V2596-057)

Compound **UB-181102c** (400 mg, 2.27 mmol) was dissolved in dichloromethane (15 mL), then PCC (980 mg, 4.55 mmol) was added and the mixture was reacted at room temperature for 2 hours. The reaction solution was concentrated to obtain crude product **UB-181102d** (380 mg, yield 95%) as a brown solid. The crude product was directly used in the next reaction. LCMS [M+H] ⁺= 175.2

### Step 3: UB-181102f (V2596-058)

Compound **UB-181102d** (203 mg, 1.15 mmol) was dissolved in ethanol (10 mL), then a drop of acetic acid and **UB-181102e** (400 mg, 2.30 mmol) were added, the mixture was reacted at room temperature for 16 hours, then continue to react at room temperature for 2 hours after adding NaCNBH₃ (217 mg, 3.45 mmol). Then (Boc)₂O (376 mg, 1.73 mmol) and NaHCO₃ (145 mg, 1.73 mmol) were added, and continued to react at room temperature for 2 hours. The reaction solution was filtered. The filtrate was concentrated and isolated by column chromatography (methanol/dichloromethane = 1/10) to obtain target product **UB-181102f**(230 mg, yield 50%) as a yellow oil. LCMS [M+H] ⁺ = 399.4

### Step 4: UB-181102g (V2596-069)

**Compound UB-181102f** (230 mg, 0.58 mmol) was dissolved in methanol/tetrahydrofuran/water (1/6/2 mL), then LiOH.H₂O (36 mg, 0.87 mmol) was added, and the mixture was reacted at room temperature for 3 hours. The reaction solution was concentrated and washed once with water (10 mL) and ethyl acetate (10 mL), the aqueous phase was acidified 1 to pH=6 with 1 M HCl, and extracted once with ethyl acetate (15 mL), and the organic phase was concentrated to obtain crude product **UB-181102g** (220 mg, 95% yield) as a colorless oil. The crude product was directly used in the next reaction. LCMS [M+H] ⁺ = 371.4

### Step 5: UB-181102h (V2596-073)

Compound **UB-181102g** (100 mg, 0.27 mmol) was dissolved in DMF (8 mL), then HATU (205 mg, 0.54 mmol) and DIEA (105 mg, 0.81 mmol) were added, and the mixture was reacted at room temperature for 20 minutes, followed by adding **A1** (63 mg, 0.24 mmol) and continued to react at room temperature for 2 hours. The reaction solution was washed with water (10 mL) once, and extracted with ethyl acetate (15 mL), the organic phase was concentrated, then isolated by column chromatography (methanol/dichloromethane=1/10) to obtain target product **UB-181102h** (80 mg, yield 48%) as a yellow oil. LCMS [M+H] ⁺ = 612.7

### Step 6: UB-181102i (V2596-074)

Compound **UB-181102h** (40 mg, 0.07 mmol) was dissolved in methanol /dichloromethane (1/10 mL), then a catalytic amount of palladium carbon was added, and the mixture was reacted at room temperature for 1 hour under hydrogen condition. The reaction solution was filtered, the filtrate was concentrated to obtain crude product **UB-181102i** (40 mg, yield 100%) as a yellow oil. The crude product was directly used in the next reaction. LCMS [M+H] ⁺ = 586.7

### Step 7: UB-181102 (V2596-077)

### The method is similar to General Method 3

¹H NMR (400 MHz, DMSO-*d₆*) δ 11.87 (s, 1H), 11.01 (s, 1H), 9.90 (s, 1H), 9.85 (s, 1H), 9.21 - 8.79 (m, 3H), 8.67 (d, *J=* 8.3 Hz, 1H), 8.28 (s, 1H), 7.79 (ddd, *J=* 12.0, 7.8, 1.4 Hz, 2H), 7.65 (s, 2H), 7.59 - 7.43 (m, 3H), 7.30 - 7.00 (m, 1H), 6.19 (s, 1H), 5.15 (dd, *J=* 13.3, 5.1 Hz, 1H), 4.40 (q, *J=* 17.5 Hz, 2H), 4.14 (s, 2H), 3.71 (s, 3H), 3.55 (d, *J=* 5.7 Hz, 5H), 3.34 (s, 4H), 3.04 (s, 1H), 2.98 - 2.88 (m, 3H), 2.81 (d, *J=* 4.4 Hz, 2H), 2.68 - 2.56 (m, 1H), 2.43 - 2.26 (m, 1H), 2.01 (ddd, *J* = 16.0, 8.0, 4.3 Hz, 2H), 1.91 - 1.72 (m, 7H), 1.67 (q, *J* = 7.2, 6.7 Hz, 2H), 1.47 (d, *J=* 13.7 Hz, 2H). LCMS [M+H]⁺ = 950.0

### Synthesis method of Compound UB-181109

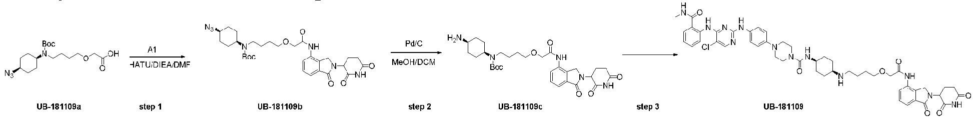

### Step 1: UB-181109b (V2596-070)

Compound **UB-181109a** (100 mg, 0.27 mmol) was dissolved in DMF (8 mL), then HATU (205 mg, 0.54 mmol) and DIEA (105 mg, 0.81 mmol) were added, and the mixture was reacted at room temperature for 20 minutes, followed by adding A1 (63 mg, 0.24 mmol) and continued to react at room temperature for 2 hours. The reaction solution was washed once with water (10 mL), and extracted once with ethyl acetate (15 mL), the organic phase was concentrated, then isolated by column chromatography (methanol/dichloromethane=1/10) to obtain target product **UB-181109b** (80 mg, yield 48%) as a yellow oil. LCMS [M+H] ⁺ = 612.7

### Step 2: UB-181109c (V2596-071)

Compound **UB-181109b** (40 mg, 0.07 mmol) was dissolved in methanol /dichloromethane (1/10 mL), then a catalytic amount of palladium carbon was added, and the mixture was reacted at room temperature for 1 hour under hydrogen condition. The reaction solution was filtered, the filtrate was concentrated to obtain target product **UB-181109c** (40 mg, yield 100%) as a yellow oil. The crude product was directly used in the next reaction. LCMS [M+H] ⁺ = 586.7

### Step 3: UB-181109 (V2596-080)

### The method is similar to General Method 3

LCMS [M+H]⁺ = 950.0

### Synthesis method of Compound UB-181112

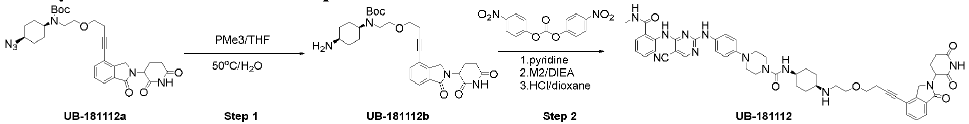

### Step 1: UB-181112b (V2596-082)

Compound **UB-181112a** (60 mg, 0.10 mmol) was dissolved in THF (10 mL), then 1M/L solution of trimethylphosphine in tetrahydrofuran (0.31 mL) was added, the mixture was reacted at 50°C for 1 hour. Then water (0.5 mL) was added, the mixture was reacted at 50 °C for 1 hour. The reaction solution was concentrated and isolated by column chromatography on preparative plate (methanol/dichloromethane = 1/10) to obtain target product **UB-181112b** (30 mg, yield 48%) as a brown oil. LCMS [M+H] ⁺ = 553.6

### Step 2: UB-181112 (V2596-088)

### The method is similar to General Method 3

¹H NMR (400 MHz, DMSO-*d₆*) δ 11.83 (s, 1H), 11.01 (s, 1H), 10.21 (s, 1H), 9.16 - 8.91 (m, 2H), 8.86 (d, *J* = 5.0 Hz, 1H), 8.61 (s, 1H), 8.51 (s, 1H), 7.81 (dd, *J* = 8.0, 1.6 Hz, 1H), 7.76 - 7.63 (m, 3H), 7.54 (q, *J=* 7.2 Hz, 3H), 7.22 (d, *J=* 7.0 Hz, 1H), 6.21 (s, 1H), 5.16 (dd, *J* = 13.3, 5.1 Hz, 1H), 4.55 - 4.22 (m, 3H), 3.80 (t, *J* = 5.3 Hz, 7H), 3.70 (t, *J* = 6.6 Hz, 4H), 3.40 (d, *J =* 7.6 Hz, 3H), 3.12 (t, *J =* 7.2 Hz, 3H), 2.92 (ddd, *J =* 17.2, 13.6, 5.4 Hz, 1H), 2.81 (d, *J* = 4.9 Hz, 4H), 2.66 - 2.55 (m, 1H), 2.43 (dd, *J* = 13.3, 4.5 Hz, 1H), 2.01 (ddt, *J* = 12.6, 9.9, 4.9 Hz, 1H), 1.82 (d, *J=* 11.8 Hz, 5H), 1.45 (s, 2H). LCMS [M+H] ⁺ = 907.9

### Synthesis method of Compound UB-181114

### Step 1: UB-181114 (V2596-094)

### The method is similar to General Method 3

¹H NMR (400 MHz, DMSO-*d₆*) δ 11.83 (s, 1H), 11.00 (s, 1H), 10.21 (s, 1H), 9.03 - 8.90 (m, 2H), 8.86 (d, *J* = 4.8 Hz, 1H), 8.60 (s, 1H), 8.17 - 8.01 (m, 1H), 7.81 (dd, *J* = 8.0, 1.6 Hz, 1H), 7.70 (d, *J=* 7.8 Hz, 2H), 7.65 (s, 1H), 7.61 - 7.47 (m, 2H), 7.21 (t, *J=* 7.6 Hz, 1H), 6.23 (s, 1H), 5.11 (dd, *J* = 13.3, 5.1 Hz, 1H), 4.52 - 4.25 (m, 3H), 3.80 (t, *J* = 5.1 Hz, 5H), 3.72 - 3.63 (m, 5H), 3.59 (d, *J=* 15.2 Hz, 1H), 3.41 (q, *J=* 6.2, 5.3 Hz, 3H), 3.14 (s, 3H), 3.00 - 2.85 (m, 1H), 2.87 - 2.74 (m, 3H), 2.64 - 2.57 (m, 1H), 2.39 (dd, *J* = 13.2, 4.5 Hz, 1H), 2.11 - 1.91 (m, 1H), 1.85 (q, *J* = 11.3, 8.4 Hz, 5H), 1.70 (d, *J* = 11.0 Hz, 1H), 1.47 (s, 2H). LCMS [M+H] ⁺ = 907.8

### Synthesis method of Compound UB-181115

### Step 1: UB-181115 (V2596-097)

### The method is similar to General Method 3

¹H NMR (400 MHz, DMSO-*d₆*) δ 11.96 (s, 1H), 11.00 (s, 1H), 10.04 (s, 1H), 9.00 (s, 2H), 8.94 - 8.81 (m, 1H), 8.63 (d, *J* = 8.2 Hz, 1H), 8.31 (s, 1H), 7.82 (dd, *J* = 7.9, 1.6 Hz, 1H), 7.74 - 7.63 (m, 3H), 7.60 - 7.47 (m, 3H), 7.21 (d, *J=* 7.6 Hz, 1H), 6.59 (s, 1H), 5.11 (dd, *J=* 13.3, 5.1 Hz, 1H), 4.55 - 4.23 (m, 3H), 3.80 (t, *J=* 5.2 Hz, 3H), 3.69 (t, *J=* 6.7 Hz, 5H), 3.49 - 3.34 (m, 4H), 3.14 (qd, *J* = 7.0, 4.7, 3.9 Hz, 2H), 3.06 - 2.95 (m, 1H), 2.92 - 2.85 (m, 1H), 2.80 (dd, *J* = 12.8, 5.6 Hz, 4H), 2.66 - 2.54 (m, 1H), 2.39 (dd, *J* = 13.2, 4.6 Hz, 1H), 2.12 (d, *J* = 11.8 Hz, 2H), 2.06 - 1.94 (m, 1H), 1.87 (d, *J* = 11.7 Hz, 2H), 1.47 (q, *J=* 12.2 Hz, 2H), 1.37 - 1.24 (m, 3H). LCMS [M+H] ⁺ = 916.8

### Synthesis method of Compound UB-181119

### Step 1: UB-181119 (V2596-106)

### The method is similar to General Method 3

¹H NMR (400 MHz, DMSO-*d₆*) δ 11.81 (s, 1H), 11.02 (s, 1H), 10.09 (s, 1H), 9.05 - 8.88 (m, 3H), 8.83 (d, *J=* 4.9 Hz, 1H), 8.58 (s, 1H), 8.07 (dd, *J=* 7.9, 6.4 Hz, 1H), 7.79 (dd, *J=* 7.9, 1.6 Hz, 1H), 7.73 (d, *J* = 7.5 Hz, 1H), 7.65 (t, *J* = 8.5 Hz, 2H), 7.53 (q, *J* = 7.8 Hz, 2H), 7.35 (s, 2H), 7.19 (t, *J* = 7.5 Hz, 1H), 6.49 (s, 1H), 5.17 (dd, *J* = 13.3, 5.1 Hz, 1H), 4.59 - 4.17 (m, 3H), 3.79 (t, *J* = 5.2 Hz, 4H), 3.70 (t, *J* = 6.7 Hz, 4H), 3.47 - 3.38 (m, 2H), 3.36 - 3.24 (m, 4H), 3.13 (dd, *J* = 7.7, 3.8 Hz, 2H), 3.01 - 2.86 (m, 2H), 2.81 (d, *J* = 4.9 Hz, 4H), 2.67 - 2.56 (m, 1H), 2.48 - 2.37 (m, 1H), 2.19 - 1.94 (m, 4H), 1.85 (d, *J=* 12.0 Hz, 2H), 1.45 (p, *J=* 10.8, 9.2 Hz, 2H). LCMS [M+H] ⁺ = 907.7

### Synthesis method of Compound UB-181120

### Step 1: UB-181120 (V2596-107)

### The method is similar to General Method 3

¹H NMR (400 MHz, DMSO-*d₆*) δ 11.93 (s, 1H), 11.02 (s, 1H), 9.98 (s, 1H), 9.04 (s, 2H), 8.88 (d, *J=* 4.7 Hz, 1H), 8.65 (d, *J=* 8.5 Hz, 1H), 8.30 (s, 1H), 7.82 (dd, *J=* 8.0, 1.6 Hz, 1H), 7.73 (d, *J=* 7.5 Hz, 1H), 7.66 (d, *J=* 7.2 Hz, 2H), 7.53 (td, *J=* 7.8, 3.6 Hz, 3H), 7.20 (t, *J=* 7.6 Hz, 1H), 6.56 (s, 1H), 5.17 (dd, *J=* 13.3, 5.1 Hz, 1H), 4.63 - 4.25 (m, 3H), 3.80 (t, *J=* 5.3 Hz, 5H), 3.70 (t, *J=* 6.7 Hz, 5H), 3.38 (s, 5H), 3.13 (dd, *J=* 7.4, 3.9 Hz, 2H), 3.04 - 2.85 (m, 2H), 2.93 - 2.76 (m, 4H), 2.69 - 2.55 (m, 1H), 2.45 (d, *J=* 4.4 Hz, 1H), 2.11 (d, *J=* 9.5 Hz, 2H), 2.06 - 1.94 (m, 1H), 1.93 - 1.74 (m, 2H), 1.62 - 1.39 (m, 2H). LCMS [M+H] ⁺ = 916.7

### Synthesis method of Compound UB-181121

### Step 1: UB-181121 (V2596-111)

### The method is similar to General Method 3

¹H NMR (400 MHz, DMSO-*d₆*) δ 11.62 (s, 1H), 11.02 (s, 1H), 9.39 (s, 1H), 8.77 (dd, *J=* 10.0, 6.3 Hz, 2H), 8.20 (s, 1H), 7.75 (ddd, *J=* 13.4, 7.8, 1.3 Hz, 2H), 7.65 (dd, *J=* 7.7, 1.1 Hz, 1H), 7.62 - 7.41 (m, 4H), 7.14 (t, *J* = 7.5 Hz, 3H), 6.21 (d, *J* = 7.6 Hz, 1H), 5.17 (dd, *J* = 13.3, 5.2 Hz, 1H), 4.55 - 4.20 (m, 2H), 4.09 (d, *J* = 12.8 Hz, 2H), 3.71 (dt, *J* = 19.2, 6.0 Hz, 4H), 3.09 (s, 2H), 3.00 - 2.86 (m, 2H), 2.85 - 2.77 (m, 5H), 2.74 - 2.56 (m, 5H), 2.44 (d, *J=* 4.6 Hz, 1H), 2.01 (dt, *J* = 15.6, 4.7 Hz, 4H), 1.83 (d, *J* = 12.0 Hz, 2H), 1.79 - 1.65 (m, 2H), 1.55 - 1.30 (m, 5H). LCMS [M+H] ⁺ = 916.9

### Synthesis method of Compound UB-181122

### Step 1: UB-181122 (V2596-112)

### The method is similar to General Method 3

¹H NMR (400 MHz, DMSO-*d₆*) δ 11.83 (s, 1H), 11.00 (s, 1H), 9.70 (s, 1H), 9.03 - 8.77 (m, 3H), 8.68 (d, *J=* 8.4 Hz, 1H), 8.26 (s, 1H), 7.79 (dd, *J=* 7.9, 1.6 Hz, 1H), 7.70 (d, *J=* 7.9 Hz, 1H), 7.65 (s, 1H), 7.53 (dd, *J=* 8.1, 4.9 Hz, 3H), 7.47 (s, 1H), 7.18 (t, *J=* 7.7 Hz, 2H), 6.25 (s, 1H), 5.11 (dd, *J=* 13.3, 5.1 Hz, 1H), 4.56 - 4.24 (m, 2H), 4.10 (d, *J=* 12.7 Hz, 2H), 3.78 (t, *J* = 5.2 Hz, 2H), 3.69 (t, *J* = 6.7 Hz, 3H), 3.40 (p, *J* = 7.8 Hz, 2H), 3.14 (tt, *J* = 6.9, 3.9 Hz, 2H), 2.97 (d, *J=* 9.3 Hz, 1H), 2.95 - 2.85 (m, 1H), 2.85 - 2.76 (m, 4H), 2.74 - 2.54 (m, 5H), 2.39 (qd, *J=* 13.1, 4.4 Hz, 1H), 2.10 (d, *J=* 11.7 Hz, 2H), 2.03 (dd, *J* = 7.3, 4.4 Hz, 1H), 1.92 - 1.80 (m, 2H), 1.73 (d, *J* = 12.5 Hz, 2H), 1.61 - 1.34 (m, 4H). LCMS [M+H] ⁺ = 916.1

### Synthesis method of Compound UB-181123

### Step 1: UB-181123 (V2596-113)

### The method is similar to General Method 3

¹H NMR (400 MHz, DMSO-*d₆*) δ 11.74 (s, 1H), 11.00 (s, 1H), 9.90 (s, 1H), 8.78 (d, *J*= 4.8 Hz, 1H), 8.52 (s, 1H), 8.44 (s, 1H), 7.77 (d, *J=* 7.8 Hz, 1H), 7.71 (d, *J=* 7.9 Hz, 1H), 7.64 (s, 1H), 7.56 - 7.35 (m, 3H), 7.26 - 7.09 (m, 1H), 6.98 - 6.79 (m, 2H), 6.35 (d, *J=* 7.6 Hz, 1H), 5.11 (dd, *J* = 13.3, 5.1 Hz, 1H), 4.58 - 4.18 (m, 2H), 3.69 (dd, *J* = 15.4, 8.7 Hz, 4H), 3.42 (d, *J=* 5.2 Hz, 4H), 3.38 (s, 1H), 3.10 (s, 2H), 3.04 (s, 3H), 2.91 (ddd, *J=* 17.6, 13.6, 5.5 Hz, 2H), 2.85 - 2.77 (m, 4H), 2.68 - 2.53 (m, 2H), 2.39 (dd, *J* = 13.1, 4.5 Hz, 1H), 2.17 - 1.93 (m, 4H), 1.84 (d, *J=* 12.0 Hz, 2H), 1.40 - 1.28 (m, 4H). LCMS [M+H] ⁺ = 908.0

### Synthesis method of Compound UB-181132

### Step 1: UB-181132 (V2596-128)

### The method is similar to General Method 3

¹H NMR (400 MHz, DMSO-*d₆*) δ 11.02 (s, 1H), 9.74 (d, *J=* 40.0 Hz, 2H), 9.00 (s, 2H), 8.28 (s, 1H), 7.93 (dd, *J* = 7.8, 1.5 Hz, 1H), 7.80 (td, *J* = 7.8, 1.6 Hz, 1H), 7.73 (dd, *J* = 7.6, 1.0 Hz, 1H), 7.69 7.60 (m, 2H), 7.58 7.47 (m, 2H), 7.44 (d, *J=* 8.3 Hz, 2H), 7.33 (s, 2H), 6.54 (s, 1H), 5.17 (dd, *J* = 13.3, 5.1 Hz, 1H), 4.58 4.27 (m, 2H), 3.82 3.75 (m, 3H), 3.70 (t, *J* = 6.7 Hz, 6H), 3.36 (d, *J=* 31.2 Hz, 5H), 3.13 (s, 2H), 3.04 2.82 (m, 2H), 2.80 (t, *J=* 6.7 Hz, 2H), 2.68 2.55 (m, 1H), 2.45 (d, *J* = 4.6 Hz, 1H), 2.10 (d, *J* = 11.7 Hz, 2H), 2.06 1.93 (m, 2H),1.85 (d, *J* = 11.9 Hz, 2H), 1.45 (q, *J* = 12.5 Hz, 2H), 1.33 1.25 (m, 2H). LCMS [M+H] ⁺ = 884.8

### Synthesis method of Compound UB-181133

### Step 1: UB-181133 (V2596-129)

### The method is similar to General Method 3

¹H NMR (400 MHz, DMSO-*d₆*) δ 11.00 (s, 1H), 9.80 (d, *J* = 46.1 Hz, 2H), 8.99 (s, 2H), 8.29 (s, 1H), 7.94 (dd, *J=* 7.8, 1.5 Hz, 1H), 7.81 (td, *J* = 7.8, 1.6 Hz, 1H), 7.70 (d, *J=* 7.9 Hz, 1H), 7.67 7.62 (m, 2H), 7.53 (dd, *J* = 7.7, 1.2 Hz, 2H), 7.44 (d, *J* = 8.1 Hz, 2H), 7.38 (s, 1H), 6.58 (s, 1H), 5.11 (dd, *J* = 13.3, 5.1 Hz, 1H), 4.52 4.24 (m, 3H), 3.79 (t, *J* = 5.2 Hz, 3H), 3.69 (t, *J* = 6.7 Hz, 3H), 3.38 (d, *J* = 25.4 Hz, 5H), 3.13 (d, *J* = 7.2 Hz, 2H), 3.06 2.84 (m, 2H), 2.79 (t, *J* = 6.6 Hz, 2H), 2.67 2.53 (m, 1H), 2.39 (qd, *J* = 13.2, 4.5 Hz, 1H), 2.12 (d, *J* = 11.7 Hz, 2H), 2.06 1.95 (m, 2H), 1.91 1.79 (m, 2H), 1.47 (q, *J* = 12.8, 12.3 Hz, 2H), 1.33 1.25 (m, 2H). LCMS [M+H] ⁺ = 884.8

### Synthesis method of Compound UB-181134

### Step 1: UB-181134 (V2596-133)

**The method is similar to General Method 3**¹H NMR (400 MHz, DMSO-*d₆*) δ 11.02 (s, 1H), 9.61 (s, 2H), 8.94 (s, 2H), 8.25 (s, 1H), 7.93 (dd, *J=* 7.8, 1.5 Hz, 1H), 7.79 (td, *J=* 7.9, 1.6 Hz, 1H), 7.73 (d, *J* = 7.5 Hz, 1H), 7.65 (t, *J* = 7.9 Hz, 2H), 7.52 (dt, *J* = 11.8, 7.6 Hz, 2H), 7.30 (d, *J* = 8.1 Hz, 2H), 6.93 (d, *J* = 8.3 Hz, 2H), 6.22 (s, 1H), 5.17 (dd, *J* = 13.3, 5.1 Hz, 1H), 4.52 4.22 (m, 2H), 4.07 (d, *J=* 13.2 Hz, 2H), 3.79 (t, *J=* 5.3 Hz, 2H), 3.70 (t, *J=* 6.7 Hz, 2H), 3.46 - 3.30 (m, 2H), 3.13 (t, *J=* 5.8 Hz, 2H), 2.94 (ddd, *J=* 13.6, 10.9, 6.9 Hz, 2H), 2.80 (t, *J* = 6.7 Hz, 2H), 2.72 2.60 (m, 3H), 2.43 (d, *J=* 9.0 Hz, 1H), 2.18 1.93 (m, 4H), 1.91 1.76 (m, 2H), 1.71 1.59 (m, 2H), 1.52 1.32 (m, 5H). LCMS [M+H] ⁺ = 884.0

### Synthesis method of Compound UB-181135

### Step 1: UB-181135 (V2596-134)

### The method is similar to General Method 3

¹H NMR (400 MHz, DMSO-*d₆*) δ 11.00 (s, 1H), 9.58 (s, 2H), 8.92 (dt, *J=* 6.2, 3.2 Hz, 2H), 8.24 (s, 1H), 8.05 (dd, *J=* 7.8, 6.4 Hz, 1H), 7.93 (dd, *J=* 7.7, 1.5 Hz, 1H), 7.79 (td, *J* = 7.8, 1.6 Hz, 1H), 7.75 7.60 (m, 3H), 7.60 7.42 (m, 2H), 7.30 (d, *J* = 8.1 Hz, 2H), 6.93 (d, *J* = 8.3 Hz, 2H), 6.24 (s, 1H), 5.11 (dd, *J* = 13.3, 5.1 Hz, 1H), 4.61 4.22 (m, 2H), 4.07 (d, *J* = 12.7 Hz, 2H), 3.78 (t, *J* = 5.2 Hz, 2H), 3.69 (t, *J* = 6.7 Hz, 2H), 3.47 3.34 (m, 2H), 3.15 (q, *J* = 5.6 Hz, 2H), 2.92 (s, 1H), 2.79 (t, *J* = 6.7 Hz, 2H), 2.58 (m, 4H), 2.39 (dd, *J* = 13.1, 4.6 Hz, 1H), 2.17 2.05 (m, 2H), 2.06 1.96 (m, 2H), 1.92 1.77 (m, 2H), 1.731.50 (m, 2H), 1.48 1.33 (m, 5H). LCMS [M+H] ⁺ = 883.9

### Synthesis method of Compound UB-181139

### Step 1: UB-181139 (V2596-139)

### The method is similar to General Method 3

¹H NMR (400 MHz, DMSO-*d₆*) δ 11.02 (s, 1H), 9.59 (s, 1H), 9.43 (s, 1H), 8.93 (s, 2H), 8.48 (d, *J=* 8.1 Hz, 1H), 8.29 (s, 1H), 7.94 (dd, *J=* 8.0, 1.6 Hz, 1H), 7.74 (dd, *J=* 8.1, 5.3 Hz, 2H), 7.66 (d, *J=* 7.5 Hz, 1H), 7.59 7.34 (m, 4H), 7.09 (d, *J=* 8.3 Hz, 2H), 6.23 (s, 1H), 5.17 (dd, *J=* 13.3, 5.2 Hz, 1H), 4.63 4.24 (m, 2H), 4.09 (d, *J=* 12.7 Hz, 2H), 3.79 (t, *J=* 5.2 Hz, 2H), 3.70 (t, *J* = 6.7 Hz, 2H), 3.39 (s, 2H), 3.27 (s, 3H), 3.13 (p, *J* = 5.4 Hz, 2H), 3.06 2.86 (m, 2H), 2.80 (t, *J* = 6.7 Hz, 2H), 2.69 (t, *J* = 11.9 Hz, 2H), 2.63 2.56 (m, 2H), 2.49 2.33 (m, 1H), 2.17 1.96 (m, 4H), 1.84 (d, *J* = 11.3 Hz, 2H), 1.78 1.60 (m, 2H), 1.56 1.35 (m, 4H). LCMS [M+H] ⁺ = 936.9

### Synthesis method of Compound UB-181140

### Step 1: UB-181140 (V2596-140)

### The method is similar to General Method 3

¹H NMR (400 MHz, DMSO-*d₆*) δ 11.00 (s, 1H), 9.58 (s, 1H), 9.43 (s, 1H), 8.85 (s, 2H), 8.48 (d, *J* = 8.2 Hz, 1H), 8.29 (s, 1H), 7.94 (dd, *J* = 8.0, 1.6 Hz, 1H), 7.82 7.58 (m, 3H), 7.60 7.26 (m, 4H), 7.09 (d, *J* = 8.4 Hz, 2H), 6.24 (s, 1H), 5.11 (dd, *J* = 13.3, 5.1 Hz, 1H), 4.59 4.25 (m, 2H), 4.09 (d, *J=* 12.8 Hz, 2H), 3.78 (t, *J=* 5.1 Hz, 2H), 3.69 (t, *J=* 6.7 Hz, 2H), 3.27 (s, 3H), 3.15 (q, *J* = 6.2, 5.5 Hz, 2H), 3.02 2.85 (m, 3H), 2.79 (t, *J* = 6.7 Hz, 2H), 2.73 2.54 (m, 5H), 2.45 2.30 (m, 1H), 2.10 (d, *J* = 11.5 Hz, 2H), 2.00 (dp, *J* = 12.1, 4.4, 3.5 Hz, 2H), 1.85 (d, *J* = 12.2 Hz, 2H), 1.74 1.64 (m, 2H), 1.43 (dd, *J* = 12.5, 4.1 Hz, 4H). LCMS [M+H] ⁺ = 936.9

### Synthesis method of Compound UB-181141

### Step 1: UB-181141 (V2596-145)

### The method is similar to General Method 3

¹H NMR (400 MHz, DMSO-*d₆*) δ 11.96 (s, 1H), 11.02 (s, 1H), 9.94 (s, 1H), 9.12 (s, 2H), 8.91 (d, *J* = 4.8 Hz, 1H), 8.71 (s, 1H), 8.44 (s, 1H), 8.31 (s, 1H), 8.25 (dd, *J* = 9.7, 2.6 Hz, 1H), 7.82 (dd, *J* = 7.9, 1.6 Hz, 1H), 7.73 (d, *J* = 7.5 Hz, 1H), 7.66 (d, *J* = 7.5 Hz, 1H), 7.54 (td, *J* = 7.5, 4.7 Hz, 2H), 7.45 (d, *J* = 9.8 Hz, 1H), 7.18 (t, *J* = 7.5 Hz, 1H), 6.50 (s, 1H), 5.17 (dd, *J* = 13.3, 5.1 Hz, 1H), 4.62 4.20 (m, 3H), 3.81 (t, *J=* 5.3 Hz, 2H), 3.70 (t, *J=* 6.6 Hz, 6H), 3.59 3.41 (m, 3H), 3.42 (d, *J* = 11.5 Hz, 1H), 3.11 (d, *J* = 6.3 Hz, 2H), 2.99 2.88 (m, 2H), 2.87 2.71 (m, 4H), 2.66 2.57 (m, 1H), 2.44 (dd, *J* = 13.2, 4.4 Hz, 1H), 2.23 2.06 (m, 2H), 2.06 1.92 (m, 1H), 1.84 (d, J = 11.9 Hz, 2H), 1.54 -1.38 (m, 2H), 1.36 1.24 (m, 3H). LCMS [M+H] ⁺ = 917.8

### Synthesis method of Compound UB-181142

### Step 1: UB-181142 (V2596-146)

### The method is similar to General Method 3

¹H NMR (400 MHz, DMSO-*d₆*) δ 11.84 (s, 1H), 11.00 (s, 1H), 9.66 (s, 1H), 8.79 (d, *J*= 45.6 Hz, 4H), 8.42 (s, 1H), 8.26 (s, 1H), 8.14 (s, 1H),7.79 (d, *J=* 7.9 Hz, 1H), 7.70 (d, *J=* 7.9 Hz, 1H), 7.65 (s, 1H), 7.53 (d, *J* = 7.8 Hz, 2H), 7.24 (d, *J=* 52.1 Hz, 1H), 7.15 (d, *J* = 7.5 Hz, 1H), 6.44 (d, *J* = 7.1 Hz, 1H), 5.11 (dd, *J* = 13.3, 5.1 Hz, 1H), 4.62 4.19 (m, 2H), 3.77 (d, *J* = 5.4 Hz, 2H), 3.69 (t, *J* = 6.7 Hz, 2H), 3.60 (s, 4H), 3.15 (d, *J* = 8.2 Hz, 2H), 3.02 2.85 (m, 3H), 2.84 2.71 (m, 4H), 2.68 2.56 (m, 2H), 2.39 (qd, *J* = 14.3, 13.7, 5.1 Hz, 1H), 2.10 (d, *J* = 12.3 Hz, 2H), 2.05 1.92 (m, 2H), 1.85 (d, *J* = 12.0 Hz, 2H), 1.58 1.37 (m, 2H), 1.34 1.22 (m, 4H). LCMS [M+H] ⁺ = 918.8

### Synthesis method of Compound UB-181184

### Step 1: UB-181184 (V2891-057)

### The method is similar to General Method 3

¹H NMR (400 MHz, DMSO-*d₆*) δ 11.75 (s, 1H), 11.00 (s, 1H), 9.83 (s, 1H), 9.23 (s, 2H), 8.84 (d, *J* = 4.8 Hz, 1H), 8.71 (d, *J* = 8.4 Hz, 1H), 8.28 (s, 1H), 7.90 (s, 1H), 7.84 7.78 (m, 2H),7.74 7.68 (m, 3H), 7.56 (dd, *J* = 16.5, 8.0 Hz, 2H), 7.18 (t, *J* = 7.5 Hz, 1H), 5.11 (dd, *J* = 13.3, 5.1 Hz, 1H), 4.54 -4.27 (m, 2H), 3.82 3.73 (m, 3H), 3.39 (s, 2H), 3.19 (d, *J=* 16.2 Hz, 3H), 2.99 (t, *J* = 7.5 Hz, 2H), 2.81 (d, *J* = 4.5 Hz, 2H), 2.67 (s, 2H), 2.45 2.27 (m, 1H), 2.18 (s, 2H), 2.10 1.93 (m, 3H), 1.89 1.70 (m, 6H), 1.63 1.37 (m, 3H), 1.20 (t, *J* = 7.3 Hz, 1H). LCMS [M+H] ⁺ = 871.7

### Synthesis method of Compound UB-181186

### Step 1: UB-181186 (V2891-061)

### The method is similar to General Method 3

LCMS [M+H] ⁺ = 871.8

### Synthesis method of Compound UB-181187

### Step 1: UB-181187 (V2891-064)

### The method is similar to General Method 3

¹H NMR (400 MHz, DMSO-*d₆*) δ 12.29 (s, 1H), 11.57 (s, 1H), 11.02 (s, 1H), 9.30 (s, 2H), 9.00 8.91 (m, 1H), 8.86 (t, *J* = 7.8 Hz, 2H), 8.52 8.23 (m, 2H), 8.07 (d, *J* = 9.6 Hz, 1H), 8.02 7.87 (m, 2H), 7.86 (d, *J=* 7.9 Hz, 1H), 7.73 (dd, *J* = 15.2, 7.6 Hz, 2H), 7.59 (ddd, *J* = 26.4, 12.5, 6.5 Hz, 2H), 7.26 (d, *J* = 6.4 Hz, 1H), 6.13 (s, 1H), 5.17 (dd, *J* = 13.3, 5.2 Hz, 1H), 4.62 4.25 (m, 3H), 3.71 (s, 2H), 3.14 (dt, *J* = 15.3, 5.0 Hz, 5H), 3.05 2.91 (m, 5H), 2.82 (d, *J* = 4.4 Hz, 3H), 2.70 2.56 (m, 3H), 2.31 (d, *J* = 20.3 Hz, 1H), 2.07 1.95 (m, 2H), 1.88 (d, *J* = 15.3 Hz, 5H), 1.50 (s, 3H). LCMS [M+H] ⁺ = 873.8

### Synthesis method of Compound UB-181188

### Step 1: UB-181188 (V2891-065)

### The method is similar to General Method 3

¹H NMR (400 MHz, DMSO-*d₆*) δ 12.35 (s, 1H), 11.79 (s, 1H), 11.00 (s, 1H), 9.29 (s, 2H), 8.97 (t, *J* = 4.6 Hz, 1H), 8.84 (d, *J* = 8.4 Hz, 1H), 8.41 (s, 1H), 8.13 (dd, *J* = 9.6, 2.9 Hz, 1H), 7.98 7.82 (m, 2H), 7.80 7.68 (m, 2H), 7.61 (dd, *J* = 10.0, 7.6 Hz, 3H), 7.26 (t, *J* = 7.6 Hz, 1H), 6.15 (s, 1H), 5.11 (dd, *J* = 13.3, 5.1 Hz, 1H), 4.55 4.17 (m, 2H), 3.51 (d, *J* = 14.4 Hz, 4H), 3.18 (q, *J* = 6.3, 5.0 Hz, 8H), 3.00 (t, *J* = 7.4 Hz, 2H), 2.95 2.86 (m, 1H), 2.82 (d, *J* = 4.5 Hz, 3H), 2.65 2.55 (m, 1H), 2.39 (dd, *J* = 13.3, 4.7 Hz, 1H), 2.01 (tt, *J* = 10.5, 4.9 Hz, 2H), 1.87 (d, *J=* 15.6 Hz, 5H), 1.51 (dt, *J=* 14.7, 7.5 Hz, 2H). LCMS [M+H] ⁺ = 873.8

### Synthesis method of Compound UB-181170&181179

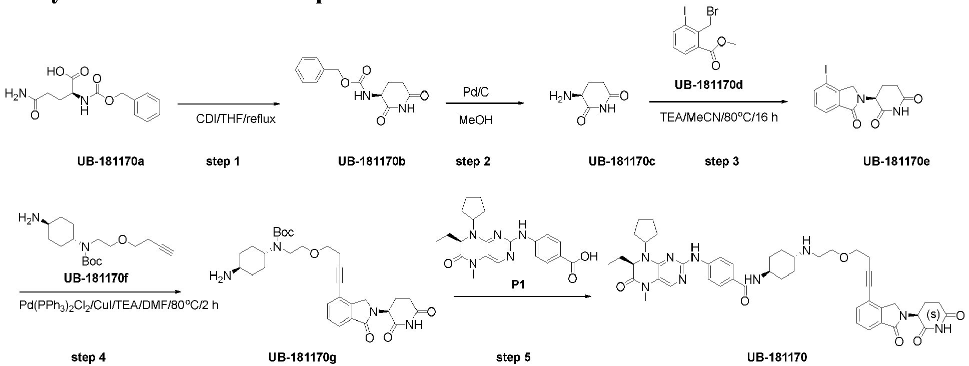

### Step 1: UB-181170b (V2891-013)

Compound **UB-181179a** (1 g, 3.57 mmol) was dissolved in THF (30 mL), then CDI (694 mg, 4.29 mmol) was added and the mixture was reacted under reflux for 18 hours. The reaction solution was filtered, solids was slurried with ether, and filtered again to obtain target product **UB-181170b** (1 g, yield 100%) as a white solid. LCMS [M+H] ⁺ = 263.2

### Step 2: UB-181170c (V2891-020)

Compound **UB-181170b** (500 mg, 1.91 mmol) was dissolved in ethanol(20 mL), then a catalytic amount of palladium carbon was added, and the mixture was reacted at room temperature for 6 hours under hydrogen condition. The reaction solution was filtered, the filtrate was concentrated to obtain crude product **UB-181170c** (260 mg, yield 100%) as a yellow oil. The crude product was directly used in the next reaction. LCMS [M+H] ⁺ = 129.0

### Step 3: UB-181170e (V2891-022)

Compound **UB-181170c** (260 mg, 2.03 mmol) was dissolved in MeCN (20 mL), then **TEA** (339 mg, 3.35 mmol) and DIEA (1.1 g, 3.05 mmol) and **UB-181170d** (1.1 g, 3.05 mmol) were added, and the mixture was reacted under reflux for 18 hours. The reaction solution was washed once with water (20 mL), and extracted with dichloromethane, the organic phase was concentrated, then isolated by column chromatography (methanol/dichloromethane=1/10) to obtain target product **UB-181170e** (180 mg, yield 24%) as a white solid. LCMS [M+H] ⁺ = 371.1

### Step 4: UB-181170g (V2891-025)

Compound **UB-181170f** (70 mg, 0.23 mmol), **UB-181170e** (84 mg, 0.23 mmol), Pd(PPh₃)₂Cl₂ (16 mg, 0.02 mmol), CuI (9 mg, 0.05 mmol), and TEA (23 mg, 0.23 mmol) were dissolved in DMF (5 mL), then the mixture was heated to 80°C and reacted for 2 hours by microwave synthesizer. The reaction solution was filtered. Then the filtrate was concentrated and isolated by column chromatography (methanol/dichloromethane = 1/10) to obtain target product **UB-181170g(30** mg, yield 24%) as a brown solid.

### Step 5: UB-181170 (V2891-027)

Compound **P1** (21 mg, 0.05 mmol) was dissolved in DMF (5 mL), then HATU (31 mg, 0.08 mmol) and DIEA (14 mg, 0.11 mmol) were added, and the mixture was reacted at room temperature for 20 minutes, followed by adding **UB-181170g** (30 mg, 0.05 mmol) and continued to react at room temperature for 2 hours. The reaction solution was washed with water (10 mL) once, and extracted with ethyl acetate (10 mL) twice, the organic phase was concentrated, then subject to column chromatography on preparative plate (methanol/dichloromethane=1/10) to obtain 20 mg white solid. The solid was dissolved in dichloromethane (3 mL) and added HCl/dioxane (0.3 mL), the mixture was reacted at room temperature for 20 minutes. The reaction solution was concentrated to obtain target product **UB-181170** (14.5 mg, yield 32%) as a light yellow solid.

¹H NMR (400 MHz, DMSO-*d₆*) δ 11.02 (s, 1H), 10.45 (s, 1H), 9.00 (s, 2H), 8.22 (d, *J*= 7.8 Hz, 1H), 7.94 - 7.82 (m, 2H), 7.73 (d, *J=* 7.5 Hz, 1H), 7.65 (dd, *J=* 11.3, 7.9 Hz, 2H), 7.53 (t, *J* = 7.6 Hz, 1H), 5.17 (dd, *J* = 13.3, 5.1 Hz, 2H), 4.62 - 4.40 (m, 2H), 4.42 - 4.23 (m, 3H), 3.91 - 3.76 (m, 3H), 3.71 (t, *J=* 6.7 Hz, 3H), 3.23 (s, 2H), 3.15 (q, *J=* 5.6 Hz, 1H), 3.04 (s, 1H), 2.96 - 2.88 (m, 2H), 2.81 (t, *J=* 6.7 Hz, 1H), 2.64 - 2.57 (m, 1H), 2.45 (d, *J=* 4.6 Hz, 1H), 2.15 (d, *J* = 11.7 Hz, 2H), 2.07 - 1.80 (m, 7H), 1.65 (d, *J* = 7.8 Hz, 2H), 1.56 - 1.32 (m, 6H), 0.77 (t, *J=* 7.4 Hz, 3H). LCMS [M+H] ⁺ = 830.8

### Synthesis method of Compound UB-180959

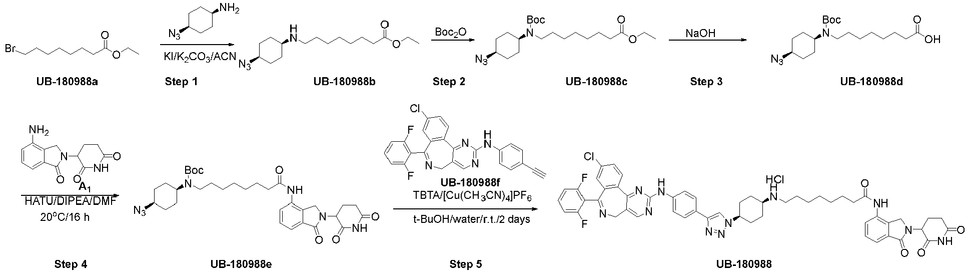

### Step 1: UB-180959b (V2228-053)

10% palladium carbon (90 mg) was added to a solution of **UB-180959a** (300 mg, 1.06 mol) in methanol (10 mL). The reaction mixture was stirred under a H2 ball for 4 h. After completion of the reaction, the reaction was filtered and the filter cake was washed with methanol. The filtrate was concentrated to obtain product **UB-180959b** (195 mg, yield 95%) as a yellow oil. LCMS: [M+H] ⁺ = 193.2

### Step 2: UB-180959c (V2228-054)

Compound **UB-180959b** (160 mg, 0.83mmol) was dissolved in dichloromethane (20 mL), added TEA (252.7 mg, 2.5mmol) and MsCl (191 mg, 1.66mmol) in ice bath, and reacted for 1 hour in the ice bath, followed by at room temperature for 2 hours. The reaction solution was washed with saturated brine, dried over anhydrous sodium sulfate, and concentrated by rotary evaporation under reduced pressure to obtain product **UB-180959c** (180 mg, yield 80%) as a yellow oil. LCMS: [M+H] ⁺ = 271.1

### Step 3: UB-180959e (V2228-055)

**UB-180959c** (180 mg, 0.67mmol), **UB-180959c** (381 mg, 0.66mmol), potassium iodide (110 mg, 0.66mmol), and potassium carbonate (276mg, 2mmol) were added to acetonitrile (5 mL) solution, and the reaction mixture was stirred at 80 °C for 16 h. After completion of reaction, water (5 mL) was added, the organics was isolated, dried(sodium sulfate), filtered, the reaction solution was concentrated to obtain crude product **UB-180959e** (250 mg), which was directly used in the next reaction without purification. LCMS: [M+H] ⁺ = 707.4

### Step 4: UB-180959f (V2228-055)

Compound **UB-180959e** (250 mg) was dissolved in tetrahydrofuran (10 mL). Di-*tert*-butyl dicarbonate (0.5 mL) was added. Reaction was allowed at room temperature for 3 hours. After completion of the reaction, the filtrate was concentrated to obtain the crude product, which was isolated by silica gel column chromatography (dichloromethane/methanol = 0% to 5%) to obtain target product **UB-180959f** (80 mg, yield 20%) as a yellow transparent oil. LCMS [M+H]⁺ = 874.3

### Step 5: UB-180959g (V2228-056)

Compound **UB-180959i** (30 mg, 0.04mmol) was dissolved in ethanol (2 mL), 2M NaOH (2 mL) was added. The mixture was reacted at room temperature for 18 hours. The reaction solution was concentrated and added water (3 mL), then extracted with ether (10 mL *3) to remove organic impurities. The aqueous phase was neutralized with 1M HCl to pH~6 and lyophilized to obtain product **UB-180959j** (20 mg) as white solid. LCMS [M+H]⁺ = 833.3

### Step 6: UB-180959 (V2228-070)

### General Method 3:

LCMS [M+H]⁺ = 974.4

### Synthesis method of Compound UB-180960

### Step 1: UB-180960 (V2228-071)

### General Method 1:

LCMS [M+H]⁺ = 974.4

### Synthesis method of Compound UB-180966

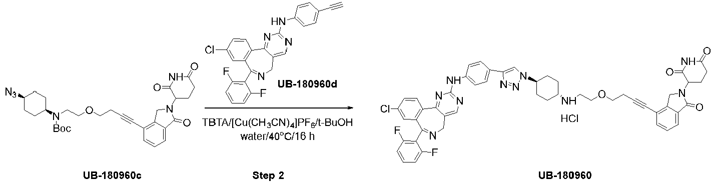

### Step 1: UB-180966 (V2228-086)

### General Method 3:

¹H NMR (400 MHz, *DMSO-d6)* δ 11.00 (s, 1H), 10.02 (s, 1H), 8.97 (s, 2H), 8.77 (d, J = 3.0 Hz, 1H), 8.70 (s, 1H), 8.31 (d, J = 8.5 Hz, 1H), 7.96 -7.85(m, 3H), 7.81 (d, J = 8.5 Hz, 2H), 7.73 - 7.64 (m, 2H), 7.57 - 7.49 (m, 2H), 7.17 (t, J = 8.8 Hz, 2H), 5.68 (s, 1H), 5.11 (dd, J = 13.2, 5.1 Hz, 1H), 4.73 -4.55 (m, 2H), 4.47 - 4.27 (m, 3H), 3.81 (t, J = 5.2 Hz, 2H), 3.69 (d, J = 6.9 Hz, 2H), 3.60 (s, 2H), 2.78 (t, J = 6.7 Hz, 2H), 2.62 (s, 1H), 2.39 - 2.33 (m,1H), 1.98 (qt, J = 12.6, 9.3, 6.8 Hz, 6H), 1.83 (t, J = 10.3 Hz, 2H). LCMS [M+H]⁺ = 935.3

### Synthesis method of Compound UB-180975

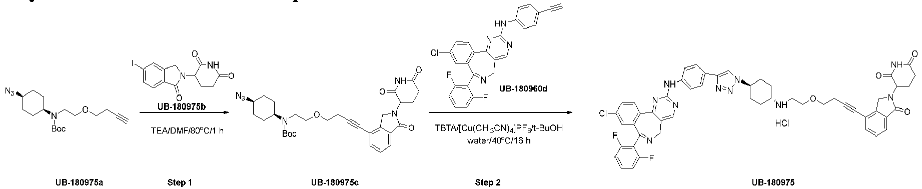

### Step 1: UB-180975 (V2228-083)

**UB-1809**75a (80 mg, 0.22mmol), **UB-1809**75b (51 mg, 0.22mmol), PdCl2(PPh3)2 (7.6 mg), copper iodide(4.1 mg), and triethylamine (66 mg) were added to anhydrous DMF (2 mL). The reaction system was stirred at 80°C for 2 hours, the reaction was cooled down to room temperature after completion. The mixture was added into water, extracted with dichloromethane, brine (30 mL), dried over sodium sulfate, filtered, and concentrated, then isolated by silica gel column chromatography (dichloromethane/methanol=10%) to obtain **UB-1809**75c (53 mg, yield 38.5%). LCMS: [M+H] ⁺ = 579.3

### Step 2: UB-180975 (V2228-089)

### General Method 3:

¹H NMR (400 MHz, *DMSO-d6)* δ 11.01 (s, 1H), 10.02 (s, 1H), 8.96 (s, 2H), 8.76 (s, 1H), 8.71 (d, J = 7.4 Hz, 1H), 8.31 (d, J = 8.5 Hz, 1H), 8.12 -7.97 (m, 1H), 7.91 (td, J = 6.0, 5.5, 2.1 Hz, 2H), 7.84 - 7.79 (m, 2H), 7.72 (d, J = 7.6 Hz, 1H), 7.65 (d, J = 7.4 Hz, 1H), 7.53 (t, J = 7.6 Hz, 2H), 7.39 -7.31 (m, 4H), 7.16 (d, J = 9.5 Hz, 2H), 5.67 (s, 1H), 5.16 (dd, J = 13.3, 5.2 Hz, 1H), 4.68 (s, 1H), 4.47 (d, J = 17.8 Hz, 1H), 4.32 (d, J = 17.8 Hz, 1H),3.81 (t, J = 5.3 Hz, 2H), 3.69 (t, J = 6.8 Hz, 2H), 3.16 (s, 2H), 2.90 (d, J = 12.4 Hz, 1H), 2.79 (t, J = 6.7 Hz, 2H), 2.62 (s, 1H), 2.44 (d, J = 4.3 Hz, 1H),2.07 - 1.91 (m, 7H), 1.81 (s, 2H). [M+H]⁺ = 935.5

### Synthesis method of Compound UB-180980

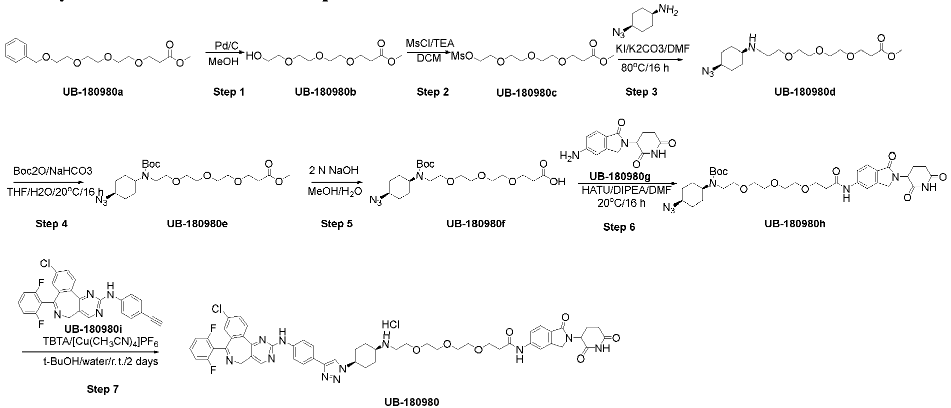

### Step 1: UB-180980b (V2228-060)

10% palladium carbon (60 mg) was added to a solution of **UB-18098**0a (150 mg, 0.46mol) in methanol (5 mL). The reaction mixture was stirred under a H2 ball for 4 h. After completion of the reaction, the reaction was filtered and the filter cake was washed with methanol. The filtrate was concentrated to obtain **UB-180980b** (90 mg, yield 82.9%) as a white solid. LCMS: [M+H] ⁺ = 237.2

### Step 2: UB-180980c (V2228-100)

**UB-180980b** (200 mg, 0.85mmol) was dissolved in dichloromethane (10 mL), TEA (257 mg, 2.54mmol) and MsCl (194 mg, 1.69mmol) were added in ice bath, and reacted for 1 hour in the ice bath, followed by at room temperature for 2 hours. The reaction solution was washed with saturated brine, dried over anhydrous sodium sulfate, and concentrated by rotary evaporation under reduced pressure to obtain product **UB-18098**0c (210 mg, yield 79%) as a yellow oil. LCMS: [M+H] ⁺ = 315.1

### Step 3: UB-180980d (V2228-102)

**UB-180980c** (50 mg, 0.19mmol), cis-4-Azidocyclohexylamine (30 mg, 0.19mmol), potassium iodide (30 mg, 0.19mmol), and potassium carbonate (63mg,0.19mmo) were added to acetonitrile (5 mL) solution, and the reaction mixture was stirred at 80 °C for 16 h. After completion of reaction, water (5 mL) was added, the organics was isolated, dried(sodium sulfate), filtered, the reaction solution was concentrated to obtain crude product, which was directly used in the next reaction without purification. LCMS: [M+H]⁺ =359.2

### Step 4: UB-180980e (V2228-105)

Compound **UB-18098**0d (80 mg, 0.13mmol) was dissolved in tetrahydrofuran (3 mL). Di-tert-butyl dicarbonate (0.2 mL) was added. Reaction was allowed at room temperature for 3 hours. After completion of the reaction, the filtrate was concentrated to obtain the crude product, which was isolated by silica gel column chromatography (dichloromethane/methanol = 0% to 5%) to obtain target product **UB-18098**0e(40 mg, 34% for 2 steps) as a yellow transparent oil. LCMS [M+H]⁺ = 459.3

### Step 5: UB-180980f (V2228-106)

Compound **UB-18098**0e (180 mg, 0.39mmol) was dissolved in ethanol (5 mL), 2M NaOH (2 mL) was added. The mixture was reacted at room temperature for 18 hours. The reaction solution was concentrated and added water (3 mL), then extracted with ether (10 mL *3) to remove organic impurities. The aqueous phase was neutralized with 1M HCl to pH~6 and lyophilized to obtain product **UB-18098**0f (120 mg, yield 67.8%) as a white solid. LCMS [M+H]⁺ = 445.3

### Step 6: UB-180980h (V2228-107)

**A3** (28 mg, 0.11mmol), **UB-18098**0f (60 mg,0.135 mol), HATU (77 mg, 0.2mmol), and diisopropylethylamine (52 mg, 0.4mmol) were dissolved in anhydrous DMF (5 mL), the mixture was stirred at room temperature for 16 hours. After the reaction was completed, the mixture was poured into water (20 mL) and then extracted with ethyl acetate (20 mL × 3). The organic phases were combined, washed with brine, dried over sodium sulfate, concentrated and then isolated by silica gel column chromatography (dichloromethane /methanol =10%) to obtain **UB-18098**0h (55 mg, yield 59.4%) as a yellow solid. LCMS: [M+H] ⁺ = 686.4

### Step 7: UB-180980 (V2228-111)

### General Method 3:

¹H NMR (400 MHz, *DMSO-d6)* δ 10.98 (s, 1H), 10.39 (s, 1H), 10.02 (s, 1H), 8.69 (d, J = 5.3 Hz, 4H), 8.31 (d, J = 8.5 Hz, 1H), 7.99 (s, 1H), 7.91 (s, 3H),7.83 (s, 2H), 7.69 - 7.60 (m, 2H), 7.55 (ddd, J = 14.8, 8.3, 6.4 Hz, 1H), 7.34 (d, J = 2.2 Hz, 1H), 7.17 (s, 2H), 5.08 (dd, J = 13.2, 5.1 Hz, 1H), 4.68 (s, 1H),4.44 - 4.25 (m, 2H), 3.72 (s, 5H), 3.28 (s, 6H), 3.11 (d, J = 8.8 Hz, 3H), 2.95 - 2.87 (m, 1H), 2.62 (s, 3H), 2.38 - 2.32 (m, 1H), 1.96 (s, 5H), 1.73 (d, J =10.9 Hz, 2H), 1.31 - 1.24 (m, 6H). LCMS [M+H]⁺ = 1009.7

### Synthesis method of Compound UB-180981

### Step 1: UB-180981 (V2228-115)

### General Method 3:

¹H NMR (400 MHz, *DMSO-d6*) δ 10.97 (s, 1H), 10.43 (s, 1H), 10.02 (s, 1H), 8.69 (d, J = 7.6 Hz, 4H), 8.31 (d, J = 8.5 Hz, 1H), 8.00 (s, 1H), 7.92 (d, J =8.8 Hz, 2H), 7.88 (dd, J = 8.5, 2.2 Hz, 1H), 7.81 (d, J = 8.6 Hz, 2H), 7.65 (d, J = 2.2 Hz, 2H), 7.60 - 7.50 (m, 1H), 7.34 (d, J = 2.2 Hz, 1H), 7.18 (d, J =7.9 Hz, 2H), 5.08 (dd, J = 13.2, 5.1 Hz, 1H), 4.65 (d, J = 10.7 Hz, 1H), 4.47 - 4.27 (m, 2H), 4.05 (q, J = 7.1 Hz, 1H), 3.72 (dt, J = 10.7, 4.9 Hz, 4H), 3.62- 3.59 (m, 2H), 3.11 - 3.05 (m, 3H), 2.95 - 2.86 (m, 2H), 2.64 (d, J = 6.0 Hz, 1H), 2.37 (dd, J = 13.3, 4.5 Hz, 1H), 1.95 (s, 5H), 1.75 (d, J = 13.3 Hz, 2H),1.29 - 1.26 (m, 6H). LCMS [M+H]⁺ = 998.72

### Synthesis method of Compound UB-180988

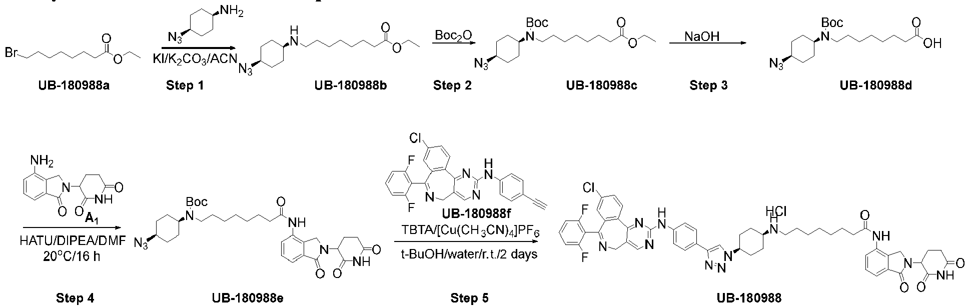

### Step 1: UB-180988b (V2228-110)

**UB-18098**8a (100 mg, 0.38mmol), cis-4-Azidocyclohexylamine (125 mg, 0.635mmol), potassium iodide (62 mg, 0.38mmol), and potassium carbonate (131mg,0.95mmol) were added to acetonitrile (5 mL) solution, and the reaction mixture was stirred at 80 °C for 16 h. After completion of reaction, water (5 mL) was added, the organics was isolated, dried(sodium sulfate), filtered, the reaction solution was concentrated to obtain crude product **UB-180988b** (100 mg), which was directly used in the next reaction without purification. LCMS: [M+H] ⁺ = 311.3

### Step 2: UB-180988c (V2228-112)

Compound **UB-18098**8b(100 mg, 0.28mmol) was dissolved in tetrahydrofuran (3 mL). Di-*tert*-butyl dicarbonate (0.2 g) was added. Reaction was allowed at room temperature for 3 hours. After completion of the reaction, the filtrate was concentrated to obtain the crude product, which was isolated by silica gel column chromatography (dichloromethane/methanol = 0% to 5%) to obtain target product **UB-18098**8c(80 mg, yield 60.5%) as a yellow transparent oil. LCMS [M+H]⁺ = 411.3

### Step 3: UB-180988d (V2228-113)

Compound **UB-18098**8c (120 mg, 0.34mmol) was dissolved in ethanol (3 mL), 2M NaOH (3 mL) was added. The mixture was reacted at room temperature for 18 hours. The reaction solution was concentrated and added water (3 mL), then extracted with ether (10 mL *3) to remove organic impurities. The aqueous phase was neutralized with 1M HCl to pH~6 and lyophilized to obtain product **UB-180988d** (100 mg, yield 89.5%) as a white solid. LCMS [M+H]⁺ = 383.2

### Step 4: UB-180988e (V2228-117)

A1 (21.8 mg, 0.08mmol), **UB-180988d** (40 mg,0.1 mol), HATU (60 mg, 0.16mmol), and diisopropylethylamine (40.7 mg, 0.32mmol) were dissolved in anhydrous DMF (5 mL), the mixture was stirred at room temperature for 16 hours. After the reaction was completed, the mixture was poured into water (20 mL) and then extracted with ethyl acetate (20 mL × 3). The organic phases were combined, washed with brine, dried over sodium sulfate, concentrated and then isolated by silica gel column chromatography (dichloromethane /methanol =10%) to obtain **UB-180988e** (35 mg, yield 53.6%) as a yellow solid. LCMS: [M+H] ⁺ = 624.4

### Step 5: UB-180988 (V2228-122)

### General Method 3:

¹H NMR (400 MHz, *DMSO-d6)* δ 11.02 (s, 1H), 10.02 (s, 1H), 9.88 (s, 1H), 8.78 (d, J = 10.5 Hz, 2H), 8.70 (s, 1H), 8.31 (d, J = 8.5 Hz, 1H), 7.94 - 7.79(m, 5H), 7.61 - 7.45 (m, 3H), 7.41 - 7.30 (m, 2H), 7.17 (t, J = 8.7 Hz, 2H), 5.15 (dd, J = 13.2, 5.1 Hz, 1H), 4.68 (d, J = 15.9 Hz, 1H), 4.45 - 4.32 (m, 2H),3.62 - 3.57 (m, 3H), 3.50 (d, J = 5.5 Hz, 1H), 3.12 (qd, J = 7.3, 4.2 Hz, 2H), 2.90 (t, J = 6.3 Hz, 3H), 2.61 (d, J = 16.8 Hz, 1H), 2.37 (d, J = 7.3 Hz, 3H),1.99 (d, J = 16.6 Hz, 5H), 1.79 (d, J = 8.8 Hz, 2H), 1.64 (s, 4H), 1.33 (s, 6H). LCMS [M+H]⁺ = 980.79

### Synthesis method of Compound UB-180989

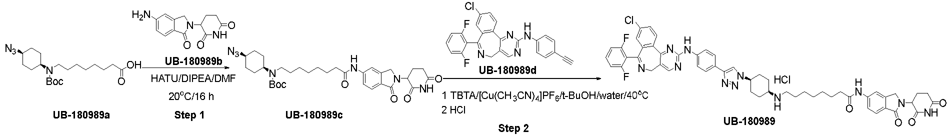

### Step 1: UB-180989c (V2228-116)

A3 (21.8 mg, 0.08mmol), **UB-180989a** (40 mg,0.1 mol), HATU (60 mg, 0.16mmol), and diisopropylethylamine (40.7 mg, 0.32mmol) were dissolved in anhydrous DMF (5 mL), the mixture was stirred at room temperature for 16 hours. After the reaction was completed, the mixture was poured into water (20 mL) and then extracted with ethyl acetate (20 mL × 3). The organic phases were combined, washed with brine, dried over sodium sulfate, concentrated and then isolated by silica gel column chromatography (dichloromethane /methanol =10%) to obtain **UB-180989c** (30 mg, yield 46.0%) as yellow solid. LCMS: [M+H] ⁺ = 624.4

### Step 2: UB-180989 (V2228-123)

### General Method 3:

¹H NMR (400 MHz, *DMSO-d6)* δ 11.02 (s, 1H), 10.02 (s, 1H), 9.88 (s, 1H), 8.78 (d, J = 10.5 Hz, 2H), 8.70 (s, 1H), 8.31 (d, J = 8.5 Hz, 1H), 7.94 - 7.79(m, 5H), 7.61 - 7.45 (m, 3H), 7.41 - 7.30 (m, 2H), 7.17 (t, J = 8.7 Hz, 2H), 5.15 (dd, J = 13.2, 5.1 Hz, 1H), 4.68 (d, J = 15.9 Hz, 1H), 4.45 - 4.32 (m, 2H),3.62 - 3.57 (m, 3H), 3.50 (d, J = 5.5 Hz, 1H), 3.12 (qd, J = 7.3, 4.2 Hz, 2H), 2.90 (t, J = 6.3 Hz, 3H), 2.61 (d, J = 16.8 Hz, 1H), 2.37 (d, J = 7.3 Hz, 3H),1.99 (d, J = 16.6 Hz, 5H), 1.79 (d, J = 8.8 Hz, 2H), 1.64 (s, 4H), 1.33 (s, 6H). LCMS [M+H]⁺ = 980.79

### Synthesis method of Compound UB-180996

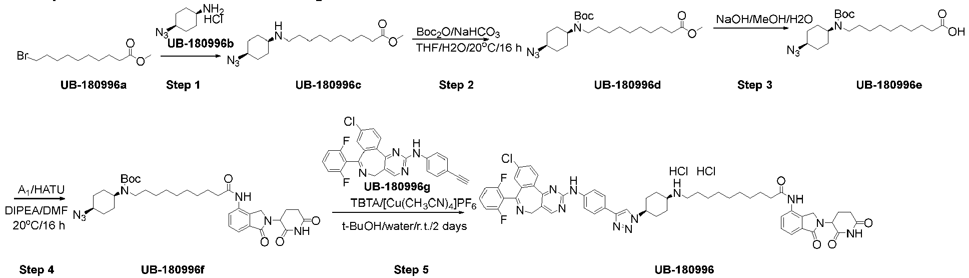

### Step 1: UB-180996 (V2228-109)

**UB-18099**6a (100 mg, 0.40mmol), **UB-18099**6b(125 mg, 0.635mmol), potassium iodide (66 mg, 0.40mmol), and potassium carbonate (138mg,1.0mmol) were added to acetonitrile (10 mL) solution, and the reaction mixture was stirred at 80 °C for 16 h. After completion of reaction, water (5 mL) was added, the organics was isolated, dried(sodium sulfate), filtered, the reaction solution was concentrated to obtain crude product **UB-180996c** (100 mg), which was directly used in the next reaction without purification. LCMS: [M+H] ⁺ = 325.3

### Step 2: UB-180996d (V2228-112)

Compound **UB-18099**6c(100mg, 0.28mmol) was dissolved in tetrahydrofuran (3 mL). Di-tert-butyl dicarbonate (0.2 g) was added. Reaction was allowed at room temperature for 3 hours. After completion of the reaction, the filtrate was concentrated to obtain the crude product, which was isolated by silica gel column chromatography (dichloromethane/methanol = 0% to 5%) to obtain target product **UB-18099**6d(120mg**,** yield 91%) as a yellow transparent oil. LCMS [M+H]⁺ = 425.4

### Step 3: UB-180996e (V2228-113)

Compound **UB-18099**6d (120 mg, 0.34mmol) was dissolved in ethanol (5 mL), 2M NaOH (3 mL) was added. The mixture was reacted at room temperature for 18 hours. The reaction solution was concentrated and added water (3 mL), then extracted with ether (10 mL *3) to remove organic impurities. The aqueous phase was neutralized with 1M HCl to pH~6 and lyophilized to obtain product **UB-18099**6e (80 mg, 46%) as white solid. LCMS [M+H]⁺ = 411.4

### Step 4: UB-180996f (V2228-117)

A1 (21.8 mg, 0.084mmol), **UB-18099**6e (40 mg,0.1 mol), HATU (60mg, 0.16mmol), and diisopropylethylamine (41mg, 2.37mmol) were dissolved in anhydrous DMF (5 mL), the mixture was stirred at room temperature for 16 hours. After the reaction was completed, the mixture was poured into water (20 mL) and then extracted with ethyl acetate (20 mL × 3). The organic phases were combined, washed with brine, dried over sodium sulfate, concentrated and then isolated by silica gel column chromatography (dichloromethane /methanol =10%) to obtain UB-180996f (35 mg, yield 53.6%) as a yellow solid. LCMS: [M+H] ⁺ = 652.4

### Step 5: UB-180996 (V2228-128)

### General Method 3:

¹H NMR (400 MHz, *DMSO-d6)* δ 11.03 (s, 1H), 10.02 (s, 1H), 9.89 (s, 1H), 9.36 - 9.20 (m, 1H), 8.84 (s, 2H), 8.78 (s, 1H), 8.70 (s, 1H), 8.31 (d, J = 8.5 Hz,1H), 7.95 - 7.84 (m, 3H), 7.83 - 7.79 (m, 2H), 7.59 - 7.44 (m, 3H), 7.33 (d, J = 2.2 Hz, 1H), 7.17 (t, J = 8.8 Hz, 2H), 5.15 (dd, J = 13.3, 5.1 Hz, 1H), 4.74 -4.66 (m, 1H), 4.37 (q, J = 17.6 Hz, 2H), 3.57 (d, J = 2.8 Hz, 4H), 3.50 (s, 2H), 3.26 (s, 2H), 3.12 (qd, J = 7.4, 4.2 Hz, 2H), 2.90 (dq, J = 14.2, 8.6, 6.8 Hz,3H), 2.65 - 2.58 (m, 1H), 2.41 - 2.30 (m, 3H), 1.99 (ddq, J = 20.2, 9.5, 4.9 Hz, 5H), 1.80 (d, J = 10.8 Hz, 2H), 1.63 (dt, J = 14.1, 7.5 Hz, 4H), 1.30 (d, J =2.1 Hz, 6H). LCMS [M+H]⁺ = 1008.8

### Synthesis method of Compound UB-180997

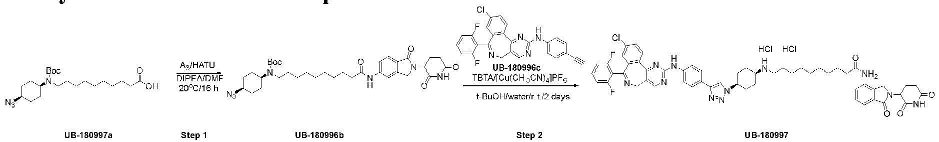

### Step 1: UB-180997b (V2228-124)

A1 (24.5 mg, 0.01mmol), **UB-180997a** (45 mg,0.12 mol), HATU (67.5mg, 0.18mmol), and diisopropylethylamine (45.8mg, 0.35mmol) were dissolved in anhydrous DMF (5 mL), the mixture was stirred at room temperature for 16 hours. After the reaction was completed, the mixture was poured into water (20 mL) and then extracted with ethyl acetate (20 mL × 3). The organic phases were combined, washed with brine, dried over sodium sulfate, concentrated and then isolated by silica gel column chromatography (dichloromethane /methanol =10%) to obtain **UB-18099**7b (38 mg, yield 53.2%) as a yellow solid. LCMS: [M+H] ⁺ = 652.4

### Step 2: UB-180997 (V2228-129)

### General Method 3:

¹H NMR (400 MHz, *DMSO-d6*) δ 10.97 (s, 1H), 10.35 (s, 1H), 10.02 (s, 1H), 8.80 (d, J = 15.4 Hz, 3H), 8.70 (s, 1H), 8.31 (d, J = 8.5 Hz, 1H), 8.00 (s, 1H),7.94 - 7.86 (m, 3H), 7.86 - 7.78 (m, 2H), 7.63 (d, J = 2.0 Hz, 2H), 7.59 - 7.50 (m, 1H), 7.40 - 7.28 (m, 2H), 7.17 (t, J = 8.6 Hz, 2H), 5.08 (dd, J = 13.3, 5.1 Hz,1H), 4.70 (s, 1H), 4.46 - 4.25 (m, 2H), 3.65 - 3.57 (m, 3H), 3.49 (p, J = 4.2, 3.7 Hz, 3H), 3.27 (s, 2H), 3.12 (tt, J = 7.5, 3.8 Hz, 1H), 2.88 (d, J = 9.8 Hz, 3H),2.59 (d, J = 17.1 Hz, 1H), 2.37 (dd, J = 9.2, 5.6 Hz, 3H), 2.05 - 1.90 (m, 5H), 1.80 (s, 2H), 1.71 - 1.54 (m, 4H), 1.29 (s, 6H). LCMS [M+H]⁺ = 1008.8

### Synthesis method of Compound UB-181005

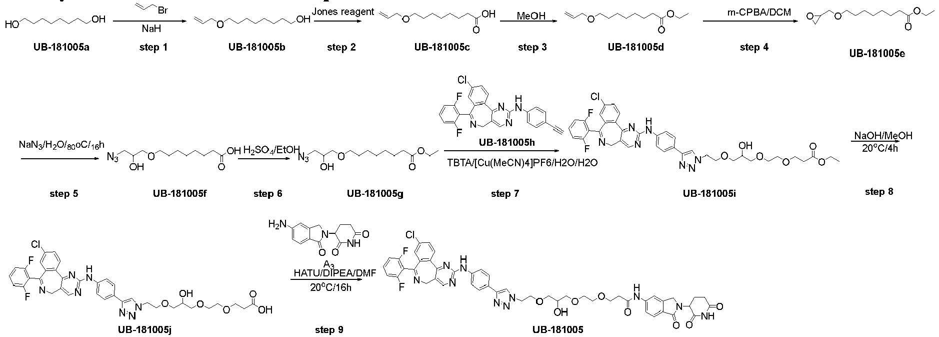

### Step 1: UB-181005b (V2141-091)

**Compound UB-181005a** (7g, 48mmol) was dissolved in tetrahydrofuran (20 mL), **UB-18100**5a (7g, 48mmol) was added in ice bath, and the mixture was reacted for 1 hour. Then allyl bromide (5.75g, 48mmol) was added, and the mixture was reacted at room temperature for 16 hours. the reaction was filtered. The filtrate was concentrated, added (50 mL) and extracted with ethyl acetate (50 mL*3). The organic phases were combined, washed with water (50 mL*3), and saturated brine (50 mL*3) successively, dried over anhydrous sodium sulfate, and concentrated, then isolated by silica gel column chromatography (petroleum ether/ethyl acetate=1/1) to obtain product **UB-18100**5b (4.4g, yield 49.4%) as colorless oil. ¹H NMR (400 MHz, Chloroform-*d*) δ 6.02 - 5.83 (m, 1H), 5.33 - 5.13 (m, 2H), 3.96 (dd, *J* = 5.6, 1.6 Hz, 2H), 3.67 - 3.58 (m, 2H), 3.42 (t, *J=* 6.7 Hz, 2H), 1.67 - 1.49 (m, 5H), 1.34 (d, *J=* 8.0 Hz, 9H).

### Step 2: UB-181005c (V2141-092)

Compound **UB-181005b** (4.2g, 18.4mmol) was dissolved in ethanol (100 mL), Jones Reagent (17 mL (2.2M), 36.8mmol) was added in ice bath, the mixture was reacted for 2 hours. The reaction was filtered. The filtrate was concentrated and the crude was isolated by silica gel column chromatography (dichloromethane/ methanol= 5%) to obtain product **UB-181005c** (3.5g, yield 95.1%) as a yellow oil. ¹H NMR (400 MHz, *DMSO-d6)* δ 11.97 (s, 1H), 5.96 - 5.75 (m, 1H), 5.17 (dd, *J=* 42.8, 13.9 Hz, 2H), 3.90 (d, *J=* 5.3 Hz, 2H), 3.47 - 3.25 (m,2H), 2.19 (t, *J* = 7.4 Hz, 2H), 1.65 - 1.11 (m, 12H).

### Step 3: UB-181005d (V2141-093)

Compound **UB-181005c** (3.5g, 17.5mmol) was dissolved in ethanol (100 mL), sulfuric acid (3.6g, 21mmol) was added, the reaction solution was reacted at 80°C for 16 hours. After completion of reaction, it was quenched with sodium bicarbonate solution (50 mL), then the mixture was extracted with ethyl acetate (50 mL*3). The organic phases were combined, washed with water (50 mL*3), and saturated brine (50 mL*3) successively, dried over anhydrous sodium sulfate, and concentrated, then isolated by silica gel column chromatography (petroleum ether/ethyl acetate=1/1) to obtain product **UB-18100**5d (2.2g, yield 55%) as a colorless oil. ¹H NMR (400 MHz, Chloroform-*d*) δ 5.98 - 5.81 (m, 1H), 5.29 - 5.13 (m, 2H), 4.12 (q, *J* = 7.1 Hz, 2H), 3.96 (dt, *J* = 5.6, 1.5 Hz, 2H), 3.42 (t, *J* = 6.6 Hz, 2H), 2.28 (dd, *J=* 8.3, 6.8 Hz, 2H), 1.70 - 1.52 (m, 4H), 1.33 (t, *J* = 1.9 Hz, 6H), 1.25 (t, *J* = 7.1 Hz, 3H).

### Step 4: UB-181005e (V2141-094)

Compound **UB-181005d** (2.2g, 9.6mmol) was dissolved in dichloromethane (100 mL), m-chloroperoxybenzoic acid(2g) was added, the reaction solution was reacted at room temperature for 2 hours. After completion of reaction, it was quenched with sodium bicarbonate solution, extracted with dichloromethane (10 mL*3). The organic phases were combined, washed with water, and saturated brine successively, dried over anhydrous sodium sulfate, and concentrated, then isolated by silica gel column chromatography (petroleum ether/ethyl acetate=3/1) to obtain product **UB-18100**5e (2g, yield 85.5%) as a colorless oil. ¹H NMR (400 MHz, Chloroform-*d*) δ 4.12 (q, *J* = 7.2 Hz, 2H), 3.70 (dd, *J* = 11.5, 3.1 Hz, 1H), 3.53 - 3.43 (m, 2H), 3.39 - 3.33 (m, 1H), 3.14 (ddt, *J=* 5.8, 4.1, 2.9 Hz, 1H), 2.86 - 2.79 (m, 1H), 2.61 (dd, *J=* 5.1, 2.7 Hz, 1H), 2.29 (t, *J=* 7.5 Hz, 2H), 1.65 - 1.53 (m, 5H), 1.39 - 1.29 (m, 6H), 1.25 (t, *J* = 7.1 Hz, 3H).

### Step 5: UB-181005f (V2141-099)

**UB-181005e** (1.9g, 7.78mmol), and NaN₃ (320 mg, 4.8mmol) were dissolved in water 20 mL water, the reaction was reacted at 80°C for 16 hours. The mixture was extracted with dichloromethane (20 mL*3), the organic phase was dried by rotary dryer, and passed through column (dichloromethane/methanol = 0/10) to obtain product **UB-18100**5f (1.8g, yield 89.5%) as a colorless oil. ¹H NMR (400 MHz, *DMSO-d6)* δ 5.69 - 5.43 (m, 1H), 3.77 (qd, *J* = 6.2, 3.8 Hz, 1H), 3.47 - 3.07 (m, 76H), 1.87 (t, *J=* 7.4 Hz, 2H), 1.44 (dt, *J=* 17.8, 6.9 Hz, 4H), 1.24 (s, 6H).

### Step 6: UB-181005g (V2141-100)

**UB-181005f** (1.8g, 6.9mmol) was dissolved in ethanol (20 mL), 3 drops of sulfuric acid were added, then the reaction system was reacted at 80°C for 16 hours. After the completion of the reaction, the reaction was neutralized with sodium bicarbonate, and extracted with ethyl acetate (20 mL*3), the organic phase was dried by rotary dryer, and passed through column (petroleum ether/ethyl acetate= 1/1) to obtain product **UB-181005g** (1.5g, yield 75%) as a colorless oil. ¹H NMR (400 MHz, Chloroform-*d*) δ 4.13 (q, *J* = 7.1 Hz, 1H), 3.94 (tt, *J=* 6.1, 4.6 Hz, 1H), 3.55 - 3.31 (m, 6H), 2.93 (s, 1H), 2.29 (t, *J=* 7.5 Hz, 2H), 1.75 - 1.48 (m, 4H), 1.33 (dd, *J* = 3.8, 2.0 Hz, 6H), 1.26 (t, *J = 7.1* Hz, 3H).

### Step 7: UB-181005i (V2228-141)

Compound **UB-181005i** (100 mg,0.22mmol), and **UB-181005h** (88.8mg,0.22mmol) were dissolved in t-BuOH (5 mL) and water(2.5 mL), then TBTA (3 mg) and [Cu(CH₃CN)₄]PF₆ (5 mg) were added. The mixture was reacted at room temperature overnight. Water (15 mL) was added, and the mixture was extracted with EtOAc (10 mL*2), the organic phases were combined, concentrated, and then isolated by column chromatography (methanol/dichloromethane=1/10) to obtain target product **UB-181005i** (120 mg, yield 45.2 %) as a yellow solid. LCMS [M+H]⁺ = 762.4

### Step 8: UB-181005j (V2228-147)

Compound **UB-181005i** (30 mg, 0.04mmol) was dissolved in ethanol (2 mL), 2M NaOH (2 mL) was added. The mixture was reacted at room temperature for 18 hours. The reaction solution was concentrated and added water (3 mL), then extracted with ether (10 mL *3) to remove organic impurities. The aqueous phase was neutralized with 1M HCl to pH~6 and lyophilized to obtain product **UB-181005j** (20 mg) as white solid. LCMS [M+H]⁺ = 733.2

### Step 9: UB-181005 (V2228-148)

### General Method 1:

¹H NMR (400 MHz, *DMSO-d6)* δ 10.97 (s, 1H), 10.23 (s, 1H), 10.00 (s, 1H), 8.69 (s, 1H), 8.38 (s, 1H), 8.31 (d, J = 8.5 Hz, 1H), 8.01 - 7.77 (m,7H), 7.68 - 7.48 (m, 3H), 7.33 (d, J = 2.2 Hz, 1H), 7.17 (s, 2H), 5.32 (d, J = 5.5 Hz, 1H), 5.07 (dd, J = 13.2, 5.1 Hz, 1H), 4.54 - 4.22 (m, 5H), 4.02(s, 1H), 3.46 - 3.37 (m, 4H), 3.35 (d, J = 7.7 Hz, 2H), 3.31 (s, 2H), 2.90 (ddd, J = 18.3, 13.5, 5.5 Hz, 1H), 2.67 - 2.56 (m, 2H), 2.36 (s, 3H), 1.99 (t,J = 5.8 Hz, 2H), 1.68 - 1.48 (m, 5H), 1.32 (s, 7H). LCMS [M+H] ⁺ = 957.8

### Synthesis method of Compound UB-181006

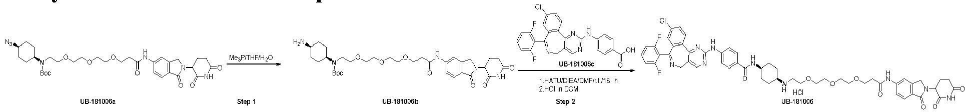

### Step 1: UB-181006b (V2228-131)

**UB-18100**6a (30 mg, 0.04mmol) was dissolved in THF (2 mL), and 1M Me₃P (0.36 mL, 0.36mmol) was added, and reacted at room temperature for 1 hour. Water (0.5 mL) was added and reacted for another 1 hour. The reaction solution was concentrated and passed through reversed-phase chromatography (acetonitrile/water = 0%-20%) to give a yellow solid **UB-181006b** (10 mg, yield 30 %). LCMS [M+H]⁺ = 660.4

### Step 2: UB-181006 (V2228-146)

### General Method 1:

¹H NMR (400 MHz, *DMSO-d6)* δ 10.97 (s, 1H), 10.52 (d, J = 31.8 Hz, 1H), 10.16 (d, J = 2.8 Hz, 1H), 8.99 - 8.65 (m, 4H), 8.29 (d, J = 8.5 Hz, 1H), 8.00 (d, J= 4.5 Hz, 1H), 7.88 (dd, J = 8.2, 3.7 Hz, 5H), 7.65 (d, J = 5.4 Hz, 2H), 7.54 (p, J = 7.2 Hz, 1H), 7.33 (d, J = 2.1 Hz, 1H), 7.16 (t, J = 8.8 Hz, 2H), 5.07 (dd, J =13.3, 5.1 Hz, 1H), 4.44 - 4.25 (m, 2H), 4.16 - 4.08 (m, 1H), 3.97 (d, J = 4.8 Hz, 2H), 3.73 (s, 2H), 3.62 (s, 4H), 3.12 - 3.05 (m, 4H), 2.93 - 2.83 (m, 2H), 2.66- 2.55 (m, 3H), 2.42 - 2.31 (m, 1H), 2.04 - 1.92 (m, 4H), 1.85 (h, J = 8.4, 6.9 Hz, 4H), 1.55 (tt, J = 10.6, 5.7 Hz, 2H), 1.38 (dd, J = 10.1, 6.5 Hz, 4H). LCMS [M+H]⁺ = 1018.9

### Synthesis method of Compound UB-181007

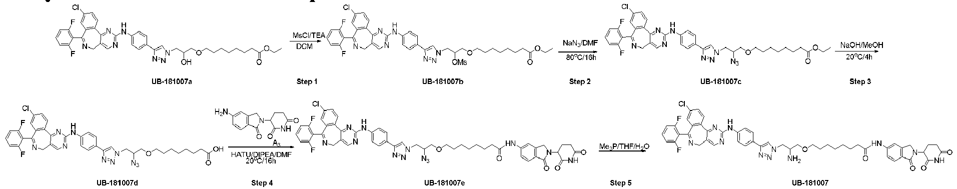

### Step 1: UB-181007b (V2228-143)

**Compound UB-181007a** (90 mg, 0.13mmol) was dissolved in dichloromethane (5 mL), TEA (0.2 g, 111.9 mmol) and MsCl (30 mg, 0.26mmol) were added in ice bath, and reacted for 1 hour in the ice bath, followed by at room temperature for 2 hours. The reaction solution was washed with saturated brine, dried over anhydrous sodium sulfate, and concentrated by rotary evaporation under reduced pressure to obtain product **UB-18100**7b (100mg, yield 100%) as a yellow oil. LCMS [M+H]⁺ = 822.3

### Step 2: UB-181007c (V2228-144)

**BI-181007b** (100 mg, 0.13mmol) was dissolved in anhydrous DMF (3 mL), NaN₃ (12.6 mg, 0.19mmol) was added, the mixture was reacted at 80°C for 16 hours. Water (10 mL) was added and the mixture was extracted with ethyl acetate (20 mL*3), the organic phase was dried by rotary dryer, and passed through column (petroleum ether/ethyl acetate= 0% to 100%) to obtain product **UB-18100**7c (90 mg, 96%) as a colorless oil. LCMS [M+H]⁺ = 769.3

### Step 3: UB-181007d (V2228-149)

Compound **UB-18100**7c (23 mg, 0.03mmol) was dissolved in ethanol (2 mL), 2M NaOH (2 mL) was added. The mixture was reacted at room temperature for 18 hours. The reaction solution was concentrated and added water (3 mL), then extracted with ether (10 mL *3) to remove organic impurities. The aqueous phase was neutralized with 1M HCl to pH~6 and lyophilized to obtain product **UB-18100**7d (15 mg) as white solid. LCMS [M+H]⁺ = 769.3

### Step 4: UB-181007e (V2228-150)

A3 (4.4 mg, 0.02mmol), **UB-181007d** (15 mg,0.02 mol), HATU (12mg, 0.03mmol), and diisopropylethylamine (8.12mg, 0.06mmol) were dissolved in anhydrous DMF (5 mL), the mixture was stirred at room temperature for 16 hours. After the reaction was completed, the mixture was poured into water (20 mL) and then extracted with ethyl acetate (20 mL × 3). The organic phases were combined, washed with brine, dried over sodium sulfate, concentrated and then isolated by silica gel column chromatography (dichloromethane /methanol =10%) to obtain UB-181007e (11 mg, yield 55%) as a yellow solid. LCMS: [M+H] ⁺ = 982.4

### Step 5: UB-181007 (V2511-002)

**UB-181007e** (25 mg, 0.03mmol) was dissolved in THF (5 mL), and 1M Me₃P (0.5 mL) was added, and reacted at room temperature for 1 hour. Water (0.5 mL) was added, the mixture was reacted for another 1 hour. The reaction solution was concentrated and passed through reversed-phase chromatography (acetonitrile/water = 0% to 30%) to obtain **UB-181007** (1.5 mg, yield 6%) as a whit solid. LCMS [M+H]⁺ = 956.4

### Synthesis method of Compound UB-181012

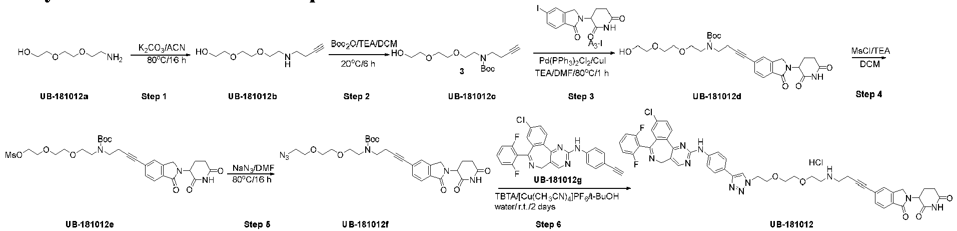

### Step 1: UB-181012b(V2511-024)

**UB-181012a** (1.0 g, 5.8mmol), bromobutyne(773 mg, 5.81mmol), and potassium iodide (1.2 g, 8.72mmol) were added to acetonitrile (5 mL) solution, and the reaction mixture was stirred at 80 °C for 16 h. After completion of reaction, water (5 mL) was added, the organics was separated, dried (sodium sulfate), filtered, the reaction solution was concentrated, and then isolated by silica gel column chromatography (dichloromethane /methanol = 10%) to obtain **UB-181012b** (320mg, yield 26%) as a yellow oil. LCMS: [M+H] ⁺ = 202.2

### Step 2: UB-181012c(V2511-025)

Compound **UB-18101**2b(350 mg, 1.74mmol) was dissolved in tetrahydrofuran (20 mL). Di-*tert*-butyl dicarbonate (760 mg, 3.48mmol) was added. Reaction was allowed at room temperature for 3 hours. After completion of the reaction, the filtrate was concentrated to obtain the crude product, which was isolated by silica gel column chromatography (dichloromethane/methanol = 0% to 5%) to obtain target product **UB-181012c** (400 mg, yield 76.3%) as a yellow transparent oil. LCMS [M+H]⁺ = 302.2

### Step 3: UB-181012d(V2511-027)

**UB-181012c** (150 mg, 1.35mmol), **A3I** (52 mg, 0.75mmol), PdCl2(PPh3)2 (17.5 mg, 0.025mmol), copper iodide(9.5 mg) , and triethylamine (151 mg) were added to anhydrous DMF (5 mL). The reaction system was stirred at 80°C for 2 hours, the reaction was cooled down to room temperature after completion. The mixture was added into water, extracted with dichloromethane, brine (30 mL), dried over sodium sulfate, filtered, and concentrated, then isolated by silica gel column chromatography (dichloromethane/methanol=10%) to obtain UB-181012d (150 mg, yield 56.2%) as a yellow solid. LCMS [M+H]+ = 544.3

### Step 4: UB-181012e(V2511-028)

Compound **UB-181012d** (150 mg, 0.28mmol) was dissolved in dichloromethane (10 mL), TEA (88.7 mg, 0.83mmol) and MsCl (63.3 mg, 0.55mmol) were added in ice bath, and reacted for 1 hour in the ice bath, followed by at room temperature for 2 hours. The reaction solution was washed with saturated brine, dried over anhydrous sodium sulfate, and concentrated by rotary evaporation under reduced pressure to obtain product **UB-181012e** (120 mg, yield 70%) as a colorless oil. LCMS [M+H]⁺ = 622.3

### Step 5: UB-181012f(V2511-029)

**BI-181002e** (120mg, 0.22mmol) was dissolved in anhydrous DMF (10 mL), NaN₃ (320 mg, 4.8mmol) was added, the mixture was reacted at 80°C for 5 hours. Water (10 mL) was added and the mixture was extracted with ethyl acetate (20 mL*3), the organic phase was dried by rotary dryer, and passed through column (petroleum ether/ethyl acetate= 0% to 100%) to obtain product **UB-181012f** (90 mg, yield 82%) as a colorless oil. LCMS [M+H]⁺ = 568.3

### Step 6: UB-181012(V2511-038)

### General Method 3:

¹H NMR (400 MHz, *DMSO-d6)* δ 11.00 (s, 1H), 10.02 (s, 1H), 8.99 (s, 2H), 8.68 (s, 1H), 8.46 (s, 1H), 8.30 (d, J = 8.5 Hz, 1H), 7.94 - 7.86 (m, 3H), 7.83- 7.78 (m, 2H), 7.69 (d, J = 7.9 Hz, 1H), 7.64 (s, 1H), 7.55 (t, J = 7.4 Hz, 2H), 7.40 - 7.31 (m, 3H), 7.18 (q, J = 8.6, 7.7 Hz, 2H), 5.11 (dd, J = 13.3, 5.1Hz, 1H), 4.58 (t, J = 5.2 Hz, 2H), 4.45 - 4.28 (m, 2H), 3.90 (t, J = 5.2 Hz, 2H), 3.68 (t, J = 5.1 Hz, 2H), 3.59 (tt, J = 5.4, 2.6 Hz, 4H), 3.21 - 3.08 (m, 4H),2.95 - 2.83 (m, 3H), 2.59 (d, J = 17.1 Hz, 1H), 2.42 - 2.32 (m, 1H), 2.04 - 1.94 (m, 1H). LCMS [M+H]⁺ = 925.9

### Synthesis method of Compound UB-181014

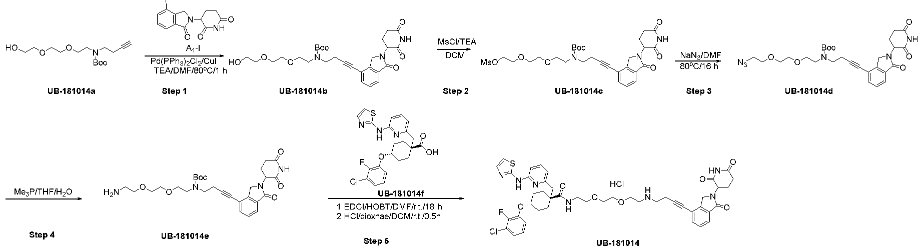

### Step 1: UB-181014b(V2511-030)

**UB-181014a** (150 mg, 0.5mmol), **A1I** (52.3 mg, 0.75mmol), PdCl2(PPh3)2 (17.5 mg), copper iodide(21 mg), and triethylamine (151 mg) were added to anhydrous DMF (5 mL). The reaction system was stirred at 80°C for 2 hours, the reaction was cooled down to room temperature after completion. The mixture was added into water, extracted with dichloromethane, brine (30 mL), dried over sodium sulfate, filtered, and concentrated, then isolated by silica gel column chromatography (dichloromethane/methanol=10%) to obtain UB-181014b (140 mg, 51.7%) as a yellow solid. LCMS [M+H]+ = 544.3

### Step 2: UB-181014c(V2511-039)

Compound **UB-181014b** (150 mg, 0.28mmol) was dissolved in dichloromethane (10 mL), TEA (83 mg, 0.83mmol) and MsCl (63 mg, 0.55mmol) were added in ice bath, and reacted for 1 hour in the ice bath, followed by at room temperature for 2 hours. The reaction solution was washed with saturated brine, dried over anhydrous sodium sulfate, and concentrated by rotary evaporation under reduced pressure to obtain product **UB-18101**4c (115 mg, yield 65%) as a yellow oil. LCMS [M+H]⁺ = 622.3

### Step 3: UB-181014d(V2511-040)

**BI-181004c** (115 mg, 0.21mmol) was dissolved in anhydrous DMF (5 mL), NaN₃ (27.5 mg, 0.42mmol) was added, the mixture was reacted at 80°C for 5 hours. Water (10 mL) was added and the mixture was extracted with ethyl acetate (20 mL*3), the organic phase was dried by rotary dryer, and passed through column (petroleum ether/ethyl acetate= 0% to 100%) to obtain product **UB-18101**4d (80 mg, yield 76%) as a colorless oil. LCMS [M+H]⁺ = 569.3

### Step 4: UB-181014e(V2511-041)

**UB-181014d** (50 mg, 0.1mmol) was dissolved in THF (2 mL), and 1M Me₃P (0.5 mL) was added, and reacted at room temperature for 1 hour. Water (0.1 mL) was added, the mixture was reacted for another 1 hour. The reaction solution was concentrated and passed through reversed-phase chromatography (acetonitrile/water = 0% to 30%) to obtain **UB-181014e** (25 mg, 52.4 %) as a yellow solid. LCMS [M+H]⁺ = 542.3

### Step 5: UB-181014(V2511-056)

### General Method 2:

¹H NMR (400 MHz, *DMSO-d6)* δ 11.65 (s, 1H), 11.02 (s, 1H), 9.30 (d, J = 13.7 Hz, 2H), 7.74 (d, J = 7.2 Hz, 2H), 7.69 (d, J = 7.6 Hz, 1H), 7.61 (t, J = 7.8 Hz,1H), 7.54 (t, J = 7.6 Hz, 1H), 7.43 (d, J = 3.8 Hz, 1H), 7.14 (dtd, J = 26.3, 8.5, 4.8 Hz, 3H), 7.05 (d, J = 3.8 Hz, 1H), 6.96 (d, J = 8.1 Hz, 1H), 6.72 (d, J = 7.3Hz, 1H), 5.15 (dd, J = 13.3, 5.1 Hz, 1H), 4.59 - 4.33 (m, 3H), 3.74 (s, 3H), 3.00 - 2.93 (m, 4H), 2.63 (s, 1H), 2.10 - 1.57 (m, 12H). LCMS [M+H]⁺ = 886.8

### Synthesis method of Compound UB-181015

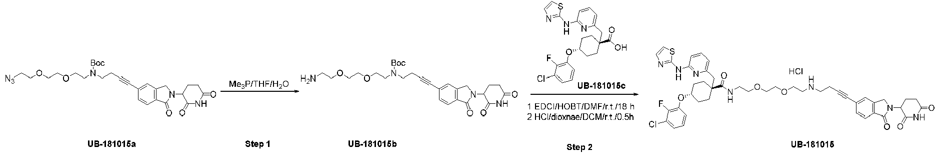

### Step 1: UB-181015b(V2511-045)

**UB-181015a** (50 mg, 0.1mmol) was dissolved in THF (5 mL), and 1M Me₃P (0.5 mL) was added, and reacted at room temperature for 1 hour. Water (0.1 mL) was added, the mixture was reacted for another 1 hour. The reaction solution was concentrated and passed through reversed-phase chromatography (acetonitrile/water = 0% to 30%) to give a yellow solid **UB-18101**5b (25 mg, yield 52.4 %). LCMS [M+H]⁺ = 543.3

### Step 2: UB-181015 (V2511-052)

### General Method 2:

¹H NMR (400 MHz, *DMSO-d6)* δ 11.58 (s, 1H), 11.00 (s, 1H), 9.20 (s, 2H), 7.71 (dd, J = 16.3, 8.4 Hz, 3H), 7.65 - 7.53 (m, 2H), 7.41 (d, J = 3.7 Hz, 1H), 7.19- 7.08 (m, 3H), 7.03 (d, J = 3.7 Hz, 1H), 6.95 (d, J = 8.2 Hz, 1H), 6.71 (d, J = 7.4 Hz, 1H), 5.11 (dd, J = 13.2, 5.1 Hz, 1H), 4.59 - 4.29 (m, 3H), 3.73 (s, 2H),2.98 - 2.91 (m, 4H), 2.62 (s, 2H), 2.00 (dt, J = 9.7, 6.4 Hz, 2H), 1.90 (d, J = 13.3 Hz, 2H), 1.84 - 1.73 (m, 4H), 1.65 (d, J = 13.4 Hz, 2H). LCMS [M+H]⁺ = 886.7

### Synthesis method of Compound UB-181027

### Step 1: UB-181027(V2511-053)

### General Method 3:

¹H NMR (400 MHz, *DMSO-d6)* δ 11.01 (s, 1H), 10.01 (s, 1H), 9.20 (s, 2H), 8.68 (s, 1H), 8.47 (s, 1H), 8.30 (d, J = 8.5 Hz, 1H), 7.95 - 7.84 (m, 3H), 7.80(d, J = 8.4 Hz, 2H), 7.68 (dd, J = 24.2, 7.5 Hz, 2H), 7.53 (dt, J = 18.2, 7.1 Hz, 2H), 7.42 - 7.29 (m, 4H), 7.16 (q, J = 6.2, 3.5 Hz, 2H), 5.68 (s, 1H), 5.15 (dd,J = 13.3, 5.1 Hz, 1H), 4.58 (t, J = 5.2 Hz, 2H), 4.53 - 4.35 (m, 2H), 3.89 (t, J = 5.3 Hz, 2H), 3.69 (t, J = 5.2 Hz, 2H), 3.62 - 3.56 (m, 4H), 3.14 (d, J = 27.1Hz, 4H), 2.98 - 2.91 (m, 2H), 2.61 (s, 1H), 2.44 - 2.35 (m, 1H), 2.01 (s, 1H). LCMS [M+H]⁺ = 925.8

### Synthesis method of Compound UB-181028

### Step 1: UB-181028(V2511-051)

### General Method 2:

¹H NMR (400 MHz, *DMSO-d6)* δ 11.00 (s, 1H), 10.17 (s, 1H), 9.62 - 9.39 (m, 5H), 9.26 (s, 2H), 8.72 (s, 1H), 8.47 (t, J = 5.6 Hz, 1H), 8.29 (d, J = 8.5 Hz,1H), 7.89 (q, J = 8.8 Hz, 5H), 7.73 - 7.65 (m, 2H), 7.55 (t, J = 7.6 Hz, 2H), 7.33 (d, J = 2.1 Hz, 1H), 7.17 (t, J = 9.2 Hz, 2H), 5.10 (dd, J = 13.2, 5.1 Hz,1H), 4.43 (d, J = 17.5 Hz, 2H), 3.10 (q, J = 3.4 Hz, 6H), 2.93 (dd, J = 15.7, 8.1 Hz, 4H), 2.57 (s, 1H), 2.42 - 2.33 (m, 1H), 2.05 - 1.94 (m, 2H). LCMS [M+H]⁺ = 901.7

### Synthesis method of Compound UB-181029

### Step 1: UB-181028(V2511-060)

### General Method 1:

¹H NMR (400 MHz, *DMSO-d6)* δ 11.02 (s, 1H), 10.15 (d, J = 3.7 Hz, 1H), 9.06 (s, 2H), 8.71 (d, J = 5.2 Hz, 1H), 8.43 (t, J = 5.7 Hz, 1H), 8.29(d, J = 8.5 Hz, 1H), 7.93 - 7.80 (m, 6H), 7.70 (dd, J = 22.6, 7.5 Hz, 2H), 7.53 (qd, J = 8.0, 2.2 Hz, 2H), 7.41 - 7.24 (m, 2H), 7.16 (t, J = 9.6 Hz,2H), 5.14 (dd, J = 13.3, 5.1 Hz, 1H), 4.50 (d, J = 17.8 Hz, 1H), 4.34 (d, J = 17.9 Hz, 1H), 3.60 (s, 4H), 2.93 (s, 3H), 2.07 - 1.95 (m, 2H). LCMS [M+H]⁺ = 901.8

### Synthesis method of Compound UB-181037

### Step 1: UB-181037(V2511-063)

### General Method 2:

¹H NMR (400 MHz, *DMSO-d6)* δ 11.20 (s, 1H), 11.03 (s, 1H), 10.10 (s, 1H), 9.96 (s, 1H), 8.72 (s, 2H), 8.05 (d, J = 11.4 Hz, 1H), 7.83 (dd, J = 7.1, 2.0 Hz,1H), 7.58 - 7.47 (m, 3H), 7.44 (t, J = 7.7 Hz, 2H), 7.39 - 7.24 (m, 5H), 7.19 - 7.07 (m, 3H), 6.97 (d, J = 3.7 Hz, 1H), 6.88 (d, J = 8.2 Hz, 1H), 6.62 (d, J = 7.4Hz, 1H), 5.15 (dd, J = 13.3, 5.1 Hz, 1H), 4.55 (s, 1H), 4.37 (q, J = 17.5 Hz, 2H), 3.72 (t, J = 6.3 Hz, 2H), 3.65 (d, J = 5.7 Hz, 2H), 3.06 (s, 2H), 2.91 (s, 4H),2.64 (d, J = 5.8 Hz, 2H), 2.31 (s, 2H), 2.02 (dd, J = 17.0, 9.6 Hz, 5H), 1.88 (s, 2H), 1.80 (d, J = 9.7 Hz, 4H), 1.72 (d, J = 12.0 Hz, 2H), 1.64 (d, J = 12.2 Hz,2H), 1.29 (d, J = 6.6 Hz, 4H). LCMS [M+H]⁺ = 1003.4

### Synthesis method of Compound UB-181038

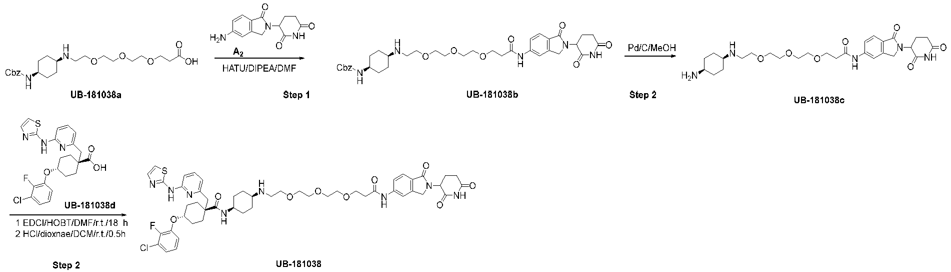

### Step 1: UB-181038b(V2511-047)

A3 (14 mg, 0.05mmol), **UB-181038a** (30 mg,0.05 mol), HATU (31 mg, 0.08mmol), and diisopropylethylamine (22.5 mg, 0.16mmol) were dissolved in anhydrous DMF (2 mL), the mixture was stirred at room temperature for 16 hours. After the reaction was completed, the mixture was poured into water (20 mL) and then extracted with ethyl acetate (20 mL × 3). The organic phases were combined, washed with brine, dried over sodium sulfate, concentrated and then isolated by silica gel column chromatography (dichloromethane /methanol =10%) to obtain **UB-18103**8b (30 mg, yield 65.2%) as a yellow solid. LCMS: [M+H] ⁺ = 694.3

### Step 2: UB-181038c(V2511-059)

10% palladium carbon (15 mg) was added to a solution of UB-181038b (100 mg, 0.13 mol) in methanol (5 mL). The reaction mixture was stirred under a H2 ball for 4 h. After completion of the reaction, the reaction was filtered and the filter cake was washed with methanol. The filtrate was concentrated to obtain UB-181038c (58 mg, yield 71.9%) as a white solid. LCMS: [M+H] + = 560.3

### Step 3: UB-181038(V2511-064)

### General Method 2:

¹H NMR (400 MHz, ) δ 11.19 (s, 1H), 10.98 (s, 1H), 10.43 (s, 1H), 8.69 (s, 2H), 8.00 (s, 1H), 7.70 - 7.62 (m, 2H), 7.56 (t, J = 7.8 Hz, 1H), 7.40 - 7.33(m, 2H), 7.23 - 7.06 (m, 5H), 6.97 (d, J = 3.6 Hz, 1H), 6.88 (d, J = 8.2 Hz, 1H), 6.69 - 6.60 (m, 2H), 5.33 (t, J = 4.8 Hz, 1H), 5.08 (dd, J = 13.3, 5.1 Hz,1H), 4.56 (s, 1H), 4.43 (d, J = 17.3 Hz, 1H), 4.29 (d, J = 17.3 Hz, 1H), 3.72 (d, J = 6.2 Hz, 2H), 3.66 (s, 2H), 2.91 (s, 4H), 2.62 (d, J = 6.0 Hz, 2H), 2.57(s, 1H), 2.42 - 2.32 (m, 1H), 2.06 - 1.96 (m, 7H), 1.90 (d, J = 13.2 Hz, 2H), 1.80 (d, J = 10.8 Hz, 4H), 1.73 (d, J = 13.8 Hz, 2H), 1.64 (d, J = 12.2 Hz,2H), 1.43 (d, J = 21.9 Hz, 4H). LCMS [M+H]⁺ = 1003.4

### Synthesis method of Compound UB-181071

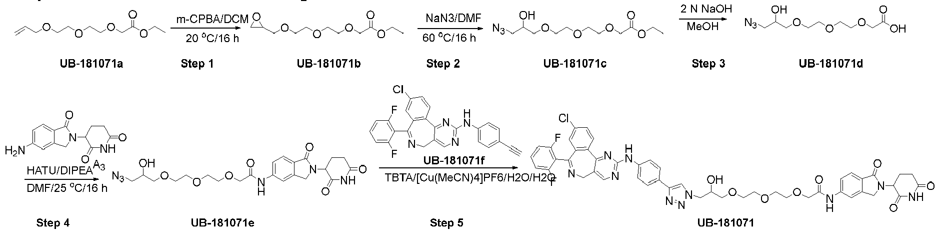

### Step 1: UB-181071b(V2511-081)

Compound **UB-181071a** (100 mg, 0.46mmol) was dissolved in dichloromethane (10 mL), m-chloroperoxybenzoic acid(157 mg) was added, the reaction solution was reacted for 6.5 hours in ice bath. After completion of reaction, it was quenched with sodium bicarbonate solution, extracted with dichloromethane (10 mL*3). The organic phases were combined, washed with water, saturated brine successively, dried over anhydrous sodium sulfate, and concentrated, then isolated by silica gel column chromatography (petroleum ether/ethyl acetate=3/1) to obtain product **UB-1810**71b (70 mg, yield 65.5%) as a colorless oil.

### Step 2: UB-181071c(V2511-083)

Compound **UB-181071b** (80 mg, 0.34mmol), NaN3(51.3 mg,0.68mmol) were dissolved in saturated ammonium chloride solution, the reaction solution was reacted at 80"C for 12 hours. After the completion of the reaction, it was extracted with ethyl acetate (10 mL*3). The organic phases were combined, washed with water, saturated brine successively, dried over anhydrous sodium sulfate, and concentrated, then isolated by silica gel column chromatography (petroleum ether/ethyl acetate=4/1) to obtain product **UB-181071c** (50 mg, yield 53.3%) as a colorless oil. ¹H NMR (400 MHz, Chloroform-d) δ 4.22 (q, J = 7.1 Hz, 2H), 4.15 (d, J = 2.9 Hz, 2H), 3.96 (tdd, J = 6.2, 5.1, 3.8 Hz, 1H), 3.77 - 3.65 (m, 8H), 3.62 -3.50 (m, 2H), 3.36 (dd, J = 5.5, 1.4 Hz, 2H), 1.29 (t, J = 7.2 Hz, 3H).LCMS: [M+H] ⁺ = 292.2

### Step 3: UB-181071d(V2511-085)

Compound **UB-181071c** (70 mg, 0.14mmol) was dissolved in ethanol (5 mL), 2M NaOH (2 mL) was added. The mixture was reacted at room temperature for 18 hours. The reaction solution was concentrated and added water (3 mL), then extracted with ether (10 mL *3) to remove organic impurities. The aqueous phase was neutralized with 1M HCl to pH~6 and lyophilized to obtain product **UB-181071d** (42 mg, yield 63.2%) as a white solid. LCMS [M+H]⁺ = 264.2

### Step 4: UB-181071e(V2511-086)

A3 (30 mg, 0.11mmol), **UB-181071d** (28 mg,0.11 mol), HATU (61.7 mg, 0.16mmol), and diisopropylethylamine (44.8mg, 0.32mmol) were dissolved in anhydrous DMF (2 mL), the mixture was stirred at room temperature for 16 hours. After the reaction was completed, the mixture was poured into water (20 mL) and then extracted with ethyl acetate (20 mL × 3). The organic phases were combined, washed with brine, dried over sodium sulfate, concentrated and then isolated by silica gel column chromatography (dichloromethane /methanol =10%) to obtain UB-181071e (45 mg, yield 74.3%) as a yellow solid. LCMS: [M+H] + = 505.2

### Step 5: UB-181071(V2511-089)

### General Method 3:

¹H NMR (400 MHz, *DMSO-d6)* δ 11.00 (s, 1H), 10.00 (s, 1H), 9.70 (s, 1H), 8.69 (s, 1H), 8.37 (s, 1H), 8.31 (d, J = 8.6 Hz, 1H), 7.89 (ddd, J = 9.2, 7.8, 2.1Hz, 3H), 7.82 - 7.77 (m, 2H), 7.74 (dd, J = 7.7, 1.3 Hz, 1H), 7.33 (d, J = 2.1 Hz, 1H), 7.18 (q, J = 7.6 Hz, 2H), 6.85 (ddd, J = 46.2, 7.7, 0.9 Hz, 1H), 5.43 -5.32 (m, 2H), 5.13 (dd, J = 13.0, 5.1 Hz, 1H), 4.48 (dd, J = 13.9, 3.7 Hz, 1H), 4.37 (d, J = 8.8 Hz, 1H), 4.33 - 4.26 (m, 1H), 4.19 - 4.07 (m, 3H), 4.01 (s, 1H),3.71 (dd, J = 5.9, 3.4 Hz, 2H), 3.65 (dd, J = 6.0, 3.4 Hz, 2H), 3.62 - 3.57 (m, 4H), 3.40 (d, J = 5.5 Hz, 1H), 2.91 (ddd, J = 18.6, 12.9, 5.6 Hz, 2H), 2.57 (s,1H), 2.34 (d, J = 4.7 Hz, 1H), 2.00 (q, J = 7.0 Hz, 4H). LCMS [M+H]⁺ = 961.3

### Synthesis method of Compound UB-181072

### Step 1: UB-181072(V2511-090)

### General Method 1:

¹H NMR (400 MHz, *DMSO-d6)* δ 10.95 (d, J = 21.0 Hz, 1H), 10.13 - 9.95 (m, 2H), 8.69 (d, J = 1.9 Hz, 2H), 8.43 - 8.35 (m, 2H), 8.31 (dd, J = 8.5,1.2 Hz, 2H), 7.89 (td, J = 8.0, 7.3, 2.1 Hz, 4H), 7.80 (dd, J = 8.8, 3.2 Hz, 3H), 7.73 - 7.48 (m, 3H), 7.33 - 7.26 (m, 3H), 7.17 (t, J = 8.4 Hz, 4H), 5.50- 5.35 (m, 2H), 5.08 (dd, J = 13.3, 5.1 Hz, 1H), 4.54 - 4.40 (m, 2H), 4.31 (dq, J = 11.5, 3.8 Hz, 2H), 4.15 (s, 1H), 4.13 - 4.07 (m, 1H), 4.05 (d, J =14.7 Hz, 2H), 3.83 (s, 2H), 3.72 - 3.64 (m, 2H), 3.61 (s, 2H), 3.59 (s, 4H), 3.58 (s, 2H), 2.97 - 2.80 (m, 2H), 2.58 (d, J = 16.8 Hz, 1H), 2.41 - 2.34 (m,1H), 2.19 - 1.84 (m, 4H). LCMS [M+H]⁺ = 961.3

### Synthesis method of Compound UB-181075

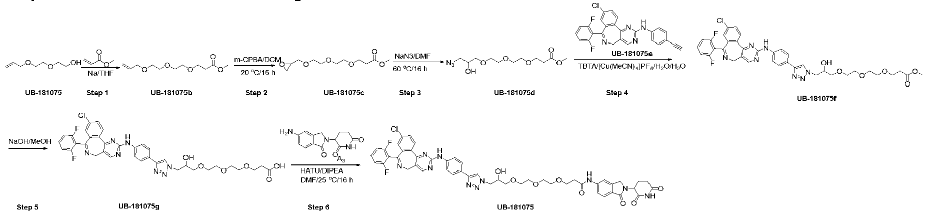

### Step 1: UB-181075b(V2511-080)

**Compound UB-18107**5a(100 mg, 0.68mmol) was dissolved in tetrahydrofuran (20 mL), Na (5 mg) was added in ice bath, and the mixture was reacted for ten minutes. Then methyl acrylate (117 mg, 1.37mmol) was added, and the mixture was reacted at room temperature for 16 hours. The reaction solution was filtered. The filtrate was concentrated, added (20 mL) and extracted with ethyl acetate (20 mL*3). The organic phases were combined, washed with water (20 mL*3), and saturated brine (20 mL*3) successively, dried over anhydrous sodium sulfate, and concentrated, then isolated by silica gel column chromatography (methanol/ dichloromethane = 0% to 10%)) to obtain product **UB-181075b** (100mg, yield 62.9%) as a yellow oil. LCMS: [M+H] ⁺ = 233.3

### Step 2: UB-181075c(V2511-082)

Compound **UB-181075b** (100 mg, 0.43mmol) was dissolved in dichloromethane (10 mL), m-chloroperoxybenzoic acid(148mg) was added, the reaction solution was reacted for 6.5 hours in ice bath. After completion of reaction, it was quenched with sodium bicarbonate solution, extracted with dichloromethane (10 mL*3). The organic phases were combined, washed with water, and saturated brine successively, dried over anhydrous sodium sulfate, and concentrated, then isolated by silica gel column chromatography (petroleum ether/ethyl acetate=3/1) to obtain product **UB-181075c** (80 mg, yield 74.8%) as a colorless oil.

### Step 3: UB-181075d (V2511-083)

Compound **UB-181075c** (100 mg, 0.43mmol), and NaN3(51.3 mg,0.68mmol) were dissolved in saturated ammonium chloride solution, the reaction solution was reacted at 80"C for 12 hours. After the completion of the reaction, it was extracted with ethyl acetate (10 mL*3). The organic phases were combined, washed with water, and saturated brine successively, dried over anhydrous sodium sulfate, and concentrated, then isolated by silica gel column chromatography (petroleum ether/ethyl acetate=4/1) to obtain product **UB-181075d** (80 mg, yield 70%) as a colorless oil. ¹H NMR (400 MHz, Chloroform-d) δ 4.22 (q, J = 7.1 Hz, 2H), 4.15 (d, J = 2.9 Hz, 2H), 3.96 (tdd, J = 6.2, 5.1, 3.8 Hz, 1H), 3.77 - 3.65 (m, 8H), 3.62 - 3.50 (m, 2H), 3.36 (dd, J = 5.5, 1.4 Hz, 2H), 1.29 (t, J = 7.2 Hz, 3H). LCMS: [M+H] ⁺ = 292.2

### Step 4: UB-181075f(V2511-084)

**UB-181075d** (400 mg, 1.08mmol), **UB-181075e** (274 mg, 1.62mmol), PdCl2(PPh3)2 (38 mg, 0.05mmol), copper iodide(21 mg), and triethylamine (491 mg) was added to anhydrous DMF (5 mL). The reaction system was stirred at 80°C for 2 hours, the reaction was cooled down to room temperature after completion. The mixture was added into water, extracted with dichloromethane, brine (30 mL), dried over sodium sulfate, filtered, and concentrated, then isolated by silica gel column chromatography (dichloromethane/methanol=10%) to obtain UB-181075f (250 mg, 56.2%) as a yellow solid. LCMS: [M+H] + = 748.3

### Step 5: UB-181075g(V2511-085)

Compound **UB-181075f** (70 mg, 0.14mmol) was dissolved in ethanol (5 mL), 2M NaOH (2 mL) was added. The mixture was reacted at room temperature for 18 hours. The reaction solution was concentrated and added water (3 mL), then extracted with ether (10 mL *3) to remove organic impurities. The aqueous phase was neutralized with 1M HCl to pH~6 and lyophilized to obtain product **UB-181075g** (42 mg, yield 61.2%) as a white solid. LCMS [M+H]⁺ = 733.2

### Step 6: UB-181075(V2511-096)

### General Method 1:

¹H NMR (400 MHz, *DMSO-d6)* δ 10.95 (s, 1H), 10.50 (d, J = 3.7 Hz, 2H), 10.02 (s, 1H), 8.65 (s, 1H), 8.24 (d, J = 8.4 Hz, 1H), 7.99 (s, 1H), 7.87 - 7.77(m, 4H), 7.70 - 7.59 (m, 3H), 7.57 - 7.34 (m, 4H), 7.33 - 7.24 (m, 2H), 7.14 (s, 2H), 5.32 (dd, J = 45.6, 40.2 Hz, 2H), 5.07 (dd, J = 13.2, 5.1 Hz, 1H), 4.18(s, 1H), 3.99 (d, J = 27.8 Hz, 3H), 3.69 - 3.63 (m, 2H), 3.45 (d, J = 4.9 Hz, 5H), 2.94 - 2.85 (m, 1H), 2.60 (d, J = 5.6 Hz, 3H), 2.40 - 2.31 (m, 2H), 2.04 -1.93 (m, 2H). LCMS [M+H]⁺ = 975.3

### Synthesis method of Compound UB-181081

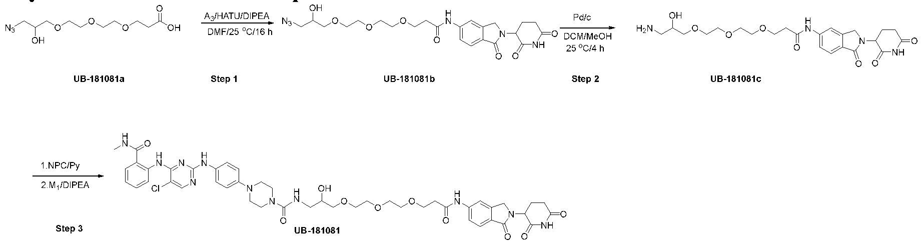

### Step 1: UB-181081b(V2511-100)

A1 (46.7 mg, 0.18mmol), **UB-181081a** (44 mg,0.14 mol), HATU (82.3 mg, 0.21mmol), and diisopropylethylamine (60mg, 0.43mmol) were dissolved in anhydrous DMF (2 mL), the mixture was stirred at room temperature for 16 hours. After the reaction was completed, the mixture was poured into water (20 mL) and then extracted with ethyl acetate (20 mL × 3). The organic phases were combined, washed with brine, dried over sodium sulfate, concentrated and then isolated by silica gel column chromatography (dichloromethane /methanol =10%) to obtain **UB-181081b** (40 mg, yield 48.6%) as a yellow solid. LCMS: [M+H] ⁺ = 519.2

### Step 2: UB-181081c (V2511-102)

10% palladium carbon (20 mg) was added to a solution of UB-181081b (30 mg, 0.14 mol) in methanol (5 mL). The reaction mixture was stirred under a H2 ball for 4 h. After completion of the reaction, the reaction was filtered and the filter cake was washed with methanol. The filtrate was concentrated to obtain **UB-181081c** (25 mg, yield 55%) as a white solid. LCMS: [M+H] ⁺ = 493.4

### Step 3: UB-181081 (V2511-104)

### General Method 4:

¹H NMR (400 MHz, *DMSO-d6)* δ 11.62 (s, 1H), 10.97 (s, 1H), 10.31 (s, 1H), 9.26 (s, 1H), 8.83 - 8.70 (m, 2H), 8.17 (s, 1H), 7.99 (s, 1H), 7.75 (dd, J =7.9, 1.6 Hz, 1H), 7.70 - 7.57 (m, 2H), 7.54 - 7.44 (m, 3H), 7.19 - 7.09 (m, 1H), 6.94 (s, 2H), 6.60 (s, 1H), 5.08 (dd, J = 13.3, 5.1 Hz, 1H), 4.48 - 4.25(m, 2H), 3.75 - 3.70 (m, 2H), 3.70 - 3.57 (m, 2H), 3.51 (q, J = 1.8 Hz, 4H), 3.30 - 3.26 (m, 2H), 3.18 (dt, J = 12.9, 6.2 Hz, 3H), 3.03 (s, 4H), 2.90 (ddd,J = 17.9, 13.6, 5.4 Hz, 1H), 2.81 (d, J = 4.5 Hz, 3H), 2.60 (q, J = 10.5, 8.4 Hz, 3H), 2.37 (dd, J = 13.2, 4.5 Hz, 1H), 2.04 - 1.92 (m, 1H). LCMS [M+H]⁺ = 956.9

### Synthesis method of Compound UB-181082

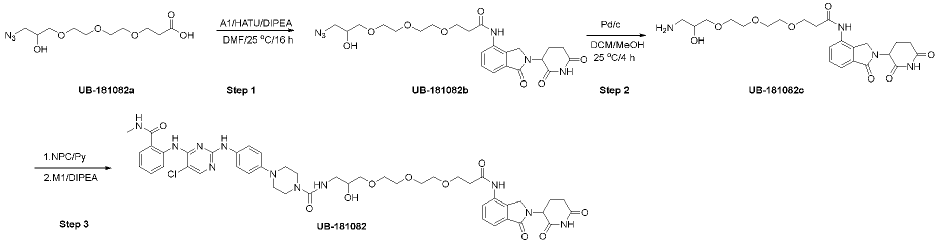

### Step 1: UB-181082b(V2511-099)

A1 (46.7 mg, 0.18mmol), **UB-181082a** (80 mg,0.18 mol), HATU (102.7 mg, 0.27mmol), and diisopropylethylamine (74.6mg, 0.54mmol) were dissolved in anhydrous DMF (2 mL), the mixture was stirred at room temperature for 16 hours. After the reaction was completed, the mixture was poured into water (20 mL) and then extracted with ethyl acetate (20 mL × 3). The organic phases were combined, washed with brine, dried over sodium sulfate, concentrated and then isolated by silica gel column chromatography (dichloromethane /methanol =10%) to obtain **UB-181082b** (40 mg, yield 26.7%) as a yellow solid. LCMS: [M+H] ⁺ = 519.2

### Step 2: UB-181082c (V2511-101)

10% palladium carbon (20 mg) was added to a solution of **UB-181082b** (30 mg, 0.14 mol) in methanol (5 mL). The reaction mixture was stirred under a H2 ball for 4 h. After completion of the reaction, the reaction was filtered and the filter cake was washed with methanol. The filtrate was concentrated to obtain **UB-181082c** (22 mg, yield 57.9%) as a white solid. LCMS: [M+H] ⁺ = 493.4

### Step 3: UB-181082 (V2511-109)

### General Method 4:

LCMS [M+H]⁺ = 956.6

### Synthesis method of Compound UB-181092

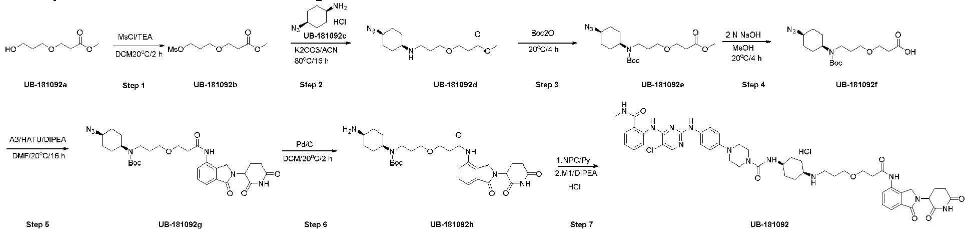

### Step 1: UB-181092b(V2511-106)

Compound **UB-18109**2a (120 mg, 0.74mmol) was dissolved in dichloromethane (5 mL), TEA (150 mg, 1.48mmol) and MsCl (92.3 mg, 0.81mmol) were added in ice bath, and reacted for 1 hour in the ice bath, followed by at room temperature for 2 hours. The reaction solution was washed with saturated brine, dried over anhydrous sodium sulfate, and concentrated by rotary evaporation under reduced pressure to obtain product **UB-18109**2b (80 mg, yield 45%) as a yellow oil. LCMS: [M+H] ⁺ = 241.1

### Step 2: UB-181092d(V2511-107)

**UB-18109**2b (80 mg, 0.47mmol), **UB-18109**2c (65 mg, 0.47mmol), and potassium iodide (96 mg, 0.70mmol) were added to acetonitrile (5 mL) solution, and the reaction mixture was stirred at 80 °C for 16 h. After completion of the reaction, water (5 mL) was added, the organics was isolated, dried(sodium sulfate), filtered, the reaction solution was concentrated to obtain crude product UB-181092d (150 mg), which was directly used in the next reaction without purification.

### Step 3: UB-181092e(V2511-110)

Compound **UB-18109**2d(150 mg, 0.4mmol) was dissolved in tetrahydrofuran (20 mL). Di-tert-butyl dicarbonate (0.5 mL) was added. Reaction was allowed at room temperature for 3 hours. After completion of the reaction, the filtrate was concentrated to obtain the crude product, which was isolated by silica gel column chromatography (dichloromethane/methanol = 0% to 5%) to obtain target product **UB-18109**2e(85 mg, yield 41.9%) as a yellow transparent oil. LCMS [M+H]⁺ = 385.3

### Step 4: UB-181092f(V2511-114)

Compound **UB-18109**2e (100 mg, 0.26mmol) was dissolved in ethanol (5 mL), 2M NaOH (3 mL) was added. The mixture was reacted at room temperature for 18 hours. The reaction solution was concentrated and added water (3 mL), then extracted with ether (10 mL *3) to remove organic impurities. The aqueous phase was neutralized with 1M HCl to pH~6 and lyophilized to obtain product **UB-18109**2f (80 mg, yield 83%) as a white solid. LCMS [M+H]⁺ = 371.3

### Step 5: UB-181092g(V2511-115)

A1 (28 mg, 0.11mmol), **UB-18109**2f (40 mg,0.11 mol), HATU (61.6mg, 0.16mmol), and and diisopropylethylamine (44.7 mg, 0.32mmol) were dissolved in anhydrous DMF (2 mL), the mixture was stirred at room temperature for 16 hours. After the reaction was completed, the mixture was poured into water (20 mL) and then extracted with ethyl acetate (20 mL × 3). The organic phases were combined, washed with brine, dried over sodium sulfate, concentrated and then isolated by silica gel column chromatography (dichloromethane /methanol =10%) to obtain **UB-181092g** (25 mg, yield 37.8%) as a yellow solid. LCMS: [M+H] ⁺ = 612.4

### Step 6: UB-181092h(V2511-123)

10% palladium carbon (30 mg) was added to a solution of UB-181092g (50 mg, 0.1 mol) in methanol (5 mL). The reaction mixture was stirred under a H2 ball for 4 h. After completion of the reaction, the reaction was filtered and the filter cake was washed with methanol. The filtrate was concentrated to obtain **UB-181092h** (35 mg, yield 73.1%) as a white solid. LCMS: [M+H] ⁺ = 586.4

### Step 7: UB-181092 (V2511-130)

### General Method 4:

¹H NMR (400 MHz, *DMSO-d6)* δ 11.62 (s, 1H), 11.02 (s, 1H), 10.07 (s, 1H), 9.25 (s, 1H), 8.95 - 8.69 (m, 4H), 8.16 (s, 1H), 7.81 (ddd, J = 34.2, 7.6, 1.7 Hz,2H), 7.55 - 7.40 (m, 5H), 7.17 - 7.09 (m, 1H), 6.92 (d, J = 9.0 Hz, 2H), 6.04 (d, J = 4.9 Hz, 1H), 5.15 (dd, J = 13.3, 5.1 Hz, 1H), 4.40 (q, J = 17.5 Hz, 2H),3.70 (dt, J = 11.0, 4.9 Hz, 4H), 3.60 (dd, J = 6.6, 3.9 Hz, 2H), 3.12 - 3.09 (m, 1H), 3.03 (d, J = 5.4 Hz, 4H), 2.94 (d, J = 13.4 Hz, 4H), 2.81 (d, J = 4.5 Hz,3H), 2.70 - 2.58 (m, 4H), 2.39 - 2.29 (m, 2H), 2.03 (dd, J = 10.7, 5.0 Hz, 2H), 1.95 - 1.87 (m, 2H), 1.76 (d, J = 22.5 Hz, 6H), 1.44 (s, 2H). LCMS [M+H]⁺ = 950.0

### Synthesis method of Compound UB-181093

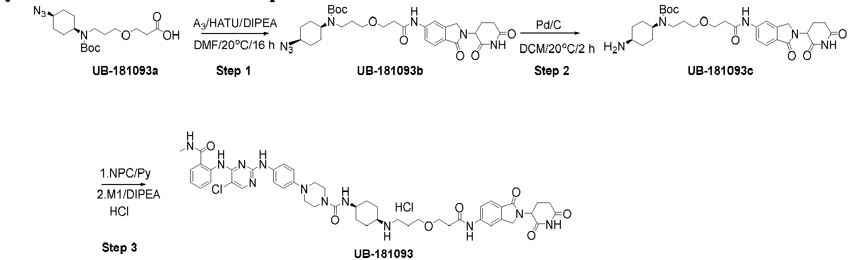

### Step 1: UB-181093b(V2511-116)

A3 (23.3 mg, 0.09mmol), **UB-18109**2a (40 mg,0.09 mol), HATU (51.35 mg, 0.135mmol), and diisopropylethylamine (37.3 mg, 0.27mmol) were dissolved in anhydrous DMF (2 mL), the mixture was stirred at room temperature for 16 hours. After the reaction was completed, the mixture was poured into water (20 mL) and then extracted with ethyl acetate (20 mL × 3). The organic phases were combined, washed with brine, dried over sodium sulfate, concentrated and then isolated by silica gel column chromatography (dichloromethane /methanol =10%) to obtain **UB-18109**2b (35 mg, yield 53.0%) as a yellow solid. LCMS: [M+H] ⁺ = 612.4

### Step 2: UB-181093c(V2511-123)

10% palladium carbon (30 mg) was added to a solution of **UB-18109**3b (50 mg, 0.09 mol) in methanol (5 mL). The reaction mixture was stirred under a H2 ball for 4 h. After completion of the reaction, the reaction was filtered and the filter cake was washed with methanol. The filtrate was concentrated to obtain **UB-18109**3c (35 mg, yield 73%) as a white solid. LCMS: [M+H] ⁺ = 586.4

### Step 3: UB-181093(V2511-131)

### General Method 4:

¹H NMR (400 MHz, *DMSO-d6)* δ 11.81 (s, 1H), 10.98 (s, 1H), 10.57 (d, J = 2.4 Hz, 1H), 9.70 (s, 1H), 8.96 - 8.62 (m, 4H), 8.25 (s, 1H), 8.02 (d, J = 1.7Hz, 1H), 7.80 (dd, J = 7.9, 1.6 Hz, 1H), 7.74 - 7.58 (m, 4H), 7.51 (t, J = 7.6 Hz, 1H), 7.30 (d, J = 14.5 Hz, 1H), 7.17 (td, J = 7.6, 1.2 Hz, 1H), 6.14 (s, 1H),5.08 (dd, J = 13.3, 5.1 Hz, 1H), 4.48 - 4.27 (m, 2H), 3.73 (s, 2H), 3.50 (s, 2H), 3.26 (s, 4H), 3.15 - 3.09 (m, 1H), 3.02 - 2.84 (m, 4H), 2.81 (d, J = 4.5 Hz,3H), 2.69 - 2.57 (m, 3H), 2.39 (td, J = 13.1, 4.4 Hz, 1H), 1.99 (ddd, J = 9.6, 5.3, 2.6 Hz, 1H), 1.94 - 1.68 (m, 8H), 1.51 - 1.39 (m, 2H).LCMS [M/2+H]⁺ = 475.7

### Synthesis method of Compound UB-181097

### Step 1: UB-181097 (V2511-131)

### General Method 3:

¹H NMR (400 MHz, *DMSO-d6)* δ 11.64 (s, 1H), 11.00 (s, 1H), 9.25 (s, 1H), 8.88 (d, J = 4.7 Hz, 1H), 8.77 (s, 1H), 8.16 (s, 1H), 7.88 - 7.78 (m, 2H),7.69 (d, J = 7.9 Hz, 1H), 7.65 (s, 1H), 7.49 (q, J = 6.9 Hz, 5H), 7.12 (t, J = 7.6 Hz, 2H), 6.92 (d, J = 9.0 Hz, 2H), 5.29 (d, J = 6.0 Hz, 1H), 5.11 (dd, J= 13.2, 5.2 Hz, 2H), 4.93 (d, J = 5.2 Hz, 1H), 4.70 (t, J = 5.7 Hz, 1H), 4.39 (d, J = 34.7 Hz, 2H), 4.03 (dd, J = 11.1, 4.2 Hz, 2H), 3.98 (d, J = 5.4 Hz,2H), 3.92 - 3.88 (m, 2H), 3.01 (s, 4H), 2.80 (s, 3H), 2.28 (s, 2H). LCMS [M+H]⁺ = 902.9

### Synthesis method of Compound UB-181100

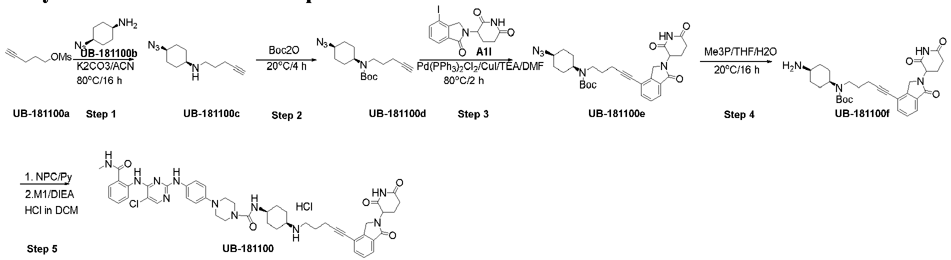

### Step 1: UB-181100c(V2511-136)

**UB-181100a** (500mg, 3.58mmol), **UB-18110**0b (580 mg, 3.58mmol), and potassium iodide (750 mg, 5.36mmol) were added to acetonitrile (20 mL) solution, and the reaction mixture was stirred at 80 °C for 16 h. After completion of reaction, water (5 mL) was added, the organics was isolated, dried(sodium sulfate), filtered, the reaction solution was concentrated to obtain crude product UB-181100c (500 mg), which was directly used in the next reaction without purification. LCMS: [M+H] ⁺ = 207.2

### Step 2: UB-181100d(V2511-136)

Compound **UB-18110**0c(500 mg, 2.42mmol) was dissolved in tetrahydrofuran (20 mL). Di-tert-butyl dicarbonate (1.0 g, 4.85mmol) was added. Reaction was allowed at room temperature for 3 hours. After completion of the reaction, the filtrate was concentrated to obtain the crude product, which was isolated by silica gel column chromatography (dichloromethane/methanol = 0% to 5%) to obtain target product (300 mg, yield 40%) as a yellow transparent oil. ¹H NMR (400 MHz, ) δ 3.86 (t, J = 2.8 Hz, 1H), 3.19 (s, 2H), 2.20 (dd, J = 7.0, 2.7 Hz, 2H), 1.99 (t, J = 2.6 Hz, 1H), 1.98 - 1.90 (m, 2H), 1.83 - 1.68 (m,4H), 1.66 - 1.55 (m, 4H), 1.48 (s, 9H).LCMS [M+H]⁺ = 307.2

### Step 3: UB-18100e(V2511-137)

**UB-181100d** (80 mg, 0.26mmol), **A1I** (96.7 mg, 0.26mmol), PdCl2(PPh3)2 (9.2 mg), copper iodide(5 mg), and triethylamine (52.8 mg) was added to anhydrous DMF (5 mL). The reaction system was stirred at 80°C for 2 hours, the reaction was cooled down to room temperature after completion. The mixture was added into water, extracted with dichloromethane, brine (30 mL), dried over sodium sulfate, filtered, and concentrated, then isolated by silica gel column chromatography (dichloromethane/methanol=10%) to obtain **UB-181100e** (50 mg, yield 42.7%). LCMS [M+H]⁺ = 549.3

### Step 4: UB-18100f(V2511-139)

**UB-181100e** (50 mg, 0.1mmol) was dissolved in THF (5 mL), and 1M Me₃P (0.5 mL) was added, and reacted at room temperature for 1 hour. Water (0.1 mL) was added, the mixture was reacted for another 1 hour. The reaction solution was concentrated and passed through reversed-phase chromatography (acetonitrile/water = 0% to 30%) to obtain **UB-18110**0f (25 mg, 53.1 %) as a yellow solid. LCMS [M+H]⁺ = 523.3

### Step 5: UB-18100(V2511-146)

### General Method 4:

¹H NMR (400 MHz, *DMSO-d6)* δ 11.83 (s, 1H), 11.00 (s, 1H), 9.76 (s, 1H), 9.02 (s, 2H), 8.85 (d, J = 4.8 Hz, 1H), 8.68 (s, 1H), 8.26 (s, 1H), 7.80 (dd, J =7.8, 1.6 Hz, 1H), 7.71 (d, J = 7.8 Hz, 1H), 7.64 (d, J = 15.9 Hz, 3H), 7.52 (dd, J = 13.0, 7.8 Hz, 2H), 7.39 (s, 1H), 7.18 (t, J = 7.5 Hz, 1H), 6.18 (s, 1H), 5.11(dd, J = 13.3, 5.1 Hz, 1H), 4.51 - 4.25 (m, 2H), 3.30 (s, 4H), 3.08 (d, J = 26.9 Hz, 3H), 2.96 - 2.85 (m, 1H), 2.81 (d, J = 4.4 Hz, 3H), 2.70 - 2.56 (m, 3H),2.39 (dd, J = 13.2, 4.4 Hz, 1H), 1.99 (q, J = 8.3, 7.2 Hz, 3H), 1.86 (d, J = 20.2 Hz, 6H), 1.51 (s, 2H). LCMS [M+H]⁺ = 887.0

### Synthesis method of Compound UB-181101

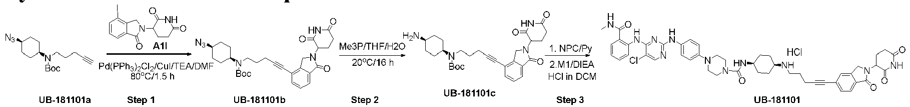

### Step 1: UB-181101b(V2511-138)

**UB-181101a** (80 mg, 0.26mmol), **A1I** (96.73 mg, 0.26mmol), PdCl2(PPh3)2 (9.2 mg), copper iodide(5 mg), and triethylamine (53 mg) was added to anhydrous DMF (5 mL). The reaction system was stirred at 80°C for 2 hours, the reaction was cooled down to room temperature after completion. The mixture was added into water, extracted with dichloromethane, brine (30 mL), dried over sodium sulfate, filtered, and concentrated, then isolated by silica gel column chromatography (dichloromethane/methanol=10%) to obtain **UB-181101b** (45 mg, yield 31.4%) as a yellow solid. LCMS [M+H]⁺ = 549.2

### Step 2: UB-181101c(V2511-140)

**UB-181101b** (40 mg, 0.1mmol) was dissolved in THF (5 mL), and 1M Me₃P (0.5 mL) was added, and reacted at room temperature for 1 hour. Water (0.1 mL) was added, the mixture was reacted for another 1 hour. The reaction solution was concentrated and passed through reversed-phase chromatography (acetonitrile/water = 0% to 30%) to give a yellow solid **UB-181101c** (28 mg, yield 73.5 %). LCMS [M+H]⁺ = 523.3

### Step 3: UB-181101 (V2511-147)

### General Method 4:

¹H NMR (400 MHz, *DMSO-d6)* δ 11.83 - 11.71 (m, 1H), 11.00 (s, 1H), 9.65 (s, 1H), 9.02 (s, 2H), 8.83 (d, J = 4.9 Hz, 1H), 8.70 (s, 1H), 8.24 (s, 1H), 7.79(dd, J = 7.9, 1.6 Hz, 1H), 7.73 (d, J = 7.5 Hz, 1H), 7.67 (d, J = 7.6 Hz, 1H), 7.60 (s, 2H), 7.52 (dt, J = 12.8, 7.7 Hz, 2H), 7.25 (s, 1H), 7.17 (t, J = 7.6 Hz,1H), 6.14 (s, 1H), 5.16 (dd, J = 13.2, 5.1 Hz, 1H), 4.43 (dd, J = 71.3, 17.7 Hz, 2H), 3.24 (s, 4H), 3.08 (ddt, J = 24.0, 9.4, 4.5 Hz, 5H), 2.96 - 2.88 (m, 1H),2.81 (d, J = 4.5 Hz, 3H), 2.64 (q, J = 7.8, 7.4 Hz, 3H), 2.57 (d, J = 4.0 Hz, 1H), 2.44 (s, 1H), 2.00 (dt, J = 14.9, 6.3 Hz, 4H), 1.86 (d, J = 27.4 Hz, 6H), 1.50(s, 2H). LCMS [M+H]⁺ = 887.0

### Synthesis method of Compound UB-181116

### Step 1: UB-181106(V2777-013)

### General Method 4:

¹H NMR (400 MHz, *DMSO-d6)* δ 11.01 (s, 1H), 10.08 (s, 1H), 9.18 (s, 1H), 8.94 (s, 2H), 8.61 (s, 1H), 8.32 (d, J = 4.3 Hz, 1H), 7.72 (d, J = 7.4 Hz, 1H), 7.65(d, J = 7.5 Hz, 1H), 7.53 (t, J = 7.5 Hz, 4H), 7.23 (d, J = 35.1 Hz, 2H), 6.07 (s, 1H), 5.16 (dd, J = 13.2, 5.0 Hz, 1H), 4.56 - 4.25 (m, 2H), 3.80 (t, J = 5.2 Hz,3H), 3.70 (t, J = 6.7 Hz, 6H), 3.16 (d, J = 29.1 Hz, 8H), 2.99 - 2.88 (m, 1H), 2.85 (d, J = 4.7 Hz, 3H), 2.80 (t, J = 6.6 Hz, 2H), 2.60 (d, J = 16.9 Hz, 1H), 2.45(d, J = 4.3 Hz, 1H), 2.10- 1.98 (m, 1H), 1.82 (d, J = 10.1 Hz, 6H), 1.44 (s, 2H) LCMS [M+H]⁺ = 908.9

### Synthesis method of Compound UB-181117

### Step 1: UB-181107(V2777-014)

### General Method 4:

¹H NMR (400 MHz, *DMSO-d6)* δ 12.65 (s, 1H), 11.00 (s, 1H), 10.05 (s, 1H), 9.18 (s, 1H), 8.86 (s, 2H), 8.60 (s, 1H), 8.32 (d, J = 4.3 Hz, 1H), 7.70 (d, J =7.8 Hz, 1H), 7.65 (s, 1H), 7.58 - 7.40 (m, 3H), 7.20 (d, J = 40.2 Hz, 2H), 6.08 (s, 1H), 5.11 (dd, J = 13.2, 5.1 Hz, 1H), 4.51 - 4.26 (m, 2H), 3.79 (t, J = 5.1Hz, 2H), 3.69 (t, J = 6.6 Hz, 4H), 3.16 (s, 7H), 2.97 - 2.87 (m, 1H), 2.85 (d, J = 4.9 Hz, 3H), 2.79 (t, J = 6.6 Hz, 2H), 2.60 (d, J = 16.6 Hz, 1H), 2.40 -2.31 (m, 1H), 2.06 - 1.95 (m, 1H), 1.81 (s, 6H), 1.44 (d, J = 21.5 Hz, 2H). LCMS [M+H]⁺ = 908.9

### Synthesis method of Compound UB-181124

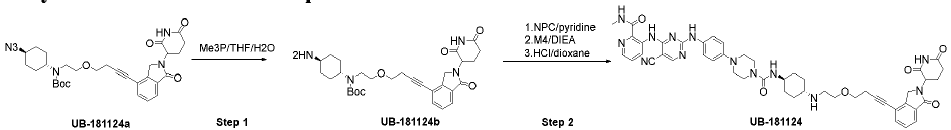

### Step 1: UB-181124b(V2777-009)

**UB-18112**4a (70 mg, 0.12mmol) was dissolved in THF (5 mL), and 1M Me₃P (0.5 mL) was added, and reacted at room temperature for 1 hour. Water (0.1 mL) was added, the mixture was reacted for another 1 hour. The reaction solution was concentrated and passed through reversed-phase chromatography (acetonitrile/water = 0% to 30%) to give a yellow solid UB-**181124b** (30 mg, yield 44.8 %). LCMS [M+H]⁺ = 553.3

### Step 2: UB-181124(V2777-022)

### General Method 4:

¹H NMR (400 MHz, *DMSO-d6)* δ 11.83 (s, 1H), 11.00 (s, 1H), 9.70 (s, 1H), 9.01 - 8.80 (m, 3H), 8.68 (d, J = 8.4 Hz, 1H), 8.26 (s, 1H), 7.79 (dd, J = 8.0, 1.6Hz, 1H), 7.70 (d, J = 7.9 Hz, 1H), 7.65 (s, 1H), 7.53 (dd, J = 8.2, 4.9 Hz, 3H), 7.50 - 7.42 (m, 1H), 7.18 (t, J = 7.7 Hz, 3H), 6.25 (s, 1H), 5.11 (dd, J = 13.3,5.1 Hz, 1H), 4.49 - 4.29 (m, 2H), 4.10 (d, J = 12.7 Hz, 2H), 3.78 (t, J = 5.2 Hz, 2H), 3.69 (s, 2H), 3.41 (s, 2H), 3.14 (td, J = 6.7, 3.2 Hz, 2H), 3.02 - 2.89 (m,2H), 2.84 - 2.75 (m, 5H), 2.75 - 2.56 (m, 5H), 2.41 (td, J = 13.1, 4.5 Hz, 1H), 2.10 (d, J = 11.8 Hz, 2H), 2.01 (tt, J = 9.2, 4.4 Hz, 2H), 1.93 - 1.80 (m, 2H),1.73 (d, J = 12.5 Hz, 2H), 1.53 - 1.39 (m, 4H), 1.33 - 1.22 (m, 5H). LCMS [M+H]⁺ = 908.9

### Synthesis method of Compound UB-181125

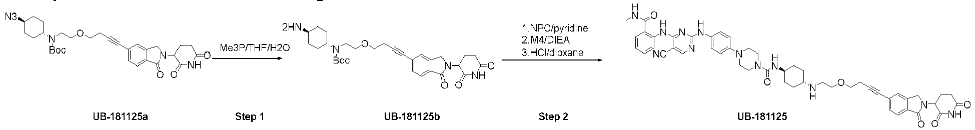

### Step 1: UB-181125b(V2777-010)

**UB-18112**4a (50 mg, 0.1mmol) was dissolved in THF (5 mL), and 1M Me₃P (0.5 mL) was added, and reacted at room temperature for 1 hour. Water (0.1 mL) was added, the mixture was reacted for another 1 hour. The reaction solution was concentrated and passed through reversed-phase chromatography (acetonitrile/water = 0% to 30%) to give a yellow solid **UB-181124b** (25 mg, yield 52.4 %). LCMS [M+H]⁺ = 553.3

### Step 2: UB-181125(V2777-023)

### General Method 4:

¹H NMR (400 MHz, *DMSO-d6)* δ 11.61 (s, 1H), 11.02 (s, 1H), 9.39 (s, 1H), 8.76 (dd, J = 10.0, 6.3 Hz, 2H), 8.20 (s, 1H), 7.75 (ddd, J = 13.4, 7.8, 1.3 Hz, 2H),7.65 (dd, J = 7.7, 1.1 Hz, 1H), 7.60 - 7.44 (m, 4H), 7.14 (t, J = 7.5 Hz, 3H), *DMSO-d66*.21 (d, J = 7.6 Hz, 1H), 5.16 (dd, J = 13.3, 5.2 Hz, 1H), 4.51 - 4.27 (m, 3H), 4.09(d, J = 12.8 Hz, 2H), 3.73 (t, J = 5.2 Hz, 2H), 3.68 (d, J = 6.7 Hz, 2H), 3.08 (s, 2H), 2.96 - 2.88 (m, 2H), 2.83 - 2.75 (m, 5H), 2.75 - 2.57 (m, 5H), 2.45 - 2.40(m, 1H), 2.11 - 1.97 (m, 4H), 1.83 (d, J = 12.0 Hz, 2H), 1.75 - 1.67 (m, 2H), 1.51 - 1.32 (m, 5H). LCMS [M+H]⁺ = 908.9

### Synthesis method of Compound UB-181180

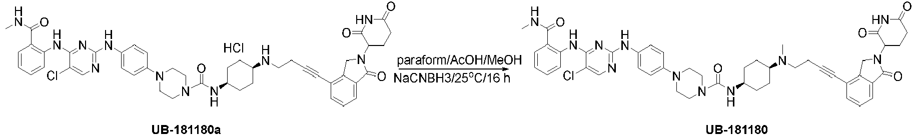

### Step 1: UB-181180(V2777-081)

**UB-181180a** (6 mg, 0.01mmol) was dissolved in methanol (2 mL), acetic acid(0.1 mL) and paraformaldehyde(1 mg, 0.03mmol) were added, the mixture was reacted at room temperature for 1 hour. Sodium cyanoborohydride (1mg, 0.1mmol) was added, the mixture was reacted for another 16 hours. The reaction solution was concentrated and isolated by thin-layer chromatography (MeOH /DCM =10%) to obtain **UB-18118**0 (3.1 mg, yield 40%) as a white solid. LCMS [M+H]⁺ = 886.7

### Synthesis method of Compound UB-1811805

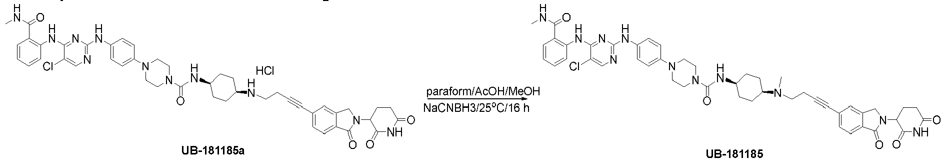

### Step 1: UB-181185(V2777-087)

**UB-181185a** (5 mg, 0.01mmol) was dissolved in methanol (2 mL), acetic acid(0.1 mL) and paraformaldehyde(1 mg, 0.03mmol) were added, the mixture was reacted at room temperature for 1 hour. Sodium cyanoborohydride (1mg, 0.1mmol) was added, the mixture was reacted for another 16 hours. The reaction solution was concentrated and isolated by thin-layer chromatography (MeOH /DCM =10%) to obtain **UB-181185** (2.4 mg, yield 47.2%) as a white solid.

¹H NMR (400 MHz, DMSO-*d*₆) δ 12.05 (d, *J=* 360.4 Hz, 1H), 10.99 (s, 1H), 9.23 (s, 1H), 8.96 - 8.82 (m, 1H), 8.75 (d, *J=* 5.1 Hz, 1H), 8.20 (d, *J=* 28.3 Hz, 1H), 7.79 - 7.44 (m, 6H), 7.24 (d, *J=* 8.7 Hz, 1H), 7.16 - 7.09 (m, 1H), 6.98 (dd, *J=* 50.4, 8.7 Hz, 2H), 6.12 (s, 1H), 5.11 (dd, *J=* 13.3, 5.1 Hz, 1H), 4.53 - 4.27 (m, 2H), 3.80 (s, 1H), 3.49 (s, 5H), 3.17 (s, 2H), 3.03 (s, 4H), 2.80 (t, *J* = 4.8 Hz, 5H), 2.59 (d, *J* = 16.8 Hz, 2H), 2.38 (dd, *J* = 12.8, 4.4 Hz, 2H), 2.28 (s, 3H), 2.00 (q, *J=* 7.3 Hz, 3H), 1.75 (s, 5H), 1.48 (s, 2H). LCMS [M+H]⁺ = 886.7

### Synthesis method of Compound UB-181076 (M1-11cg-A3)

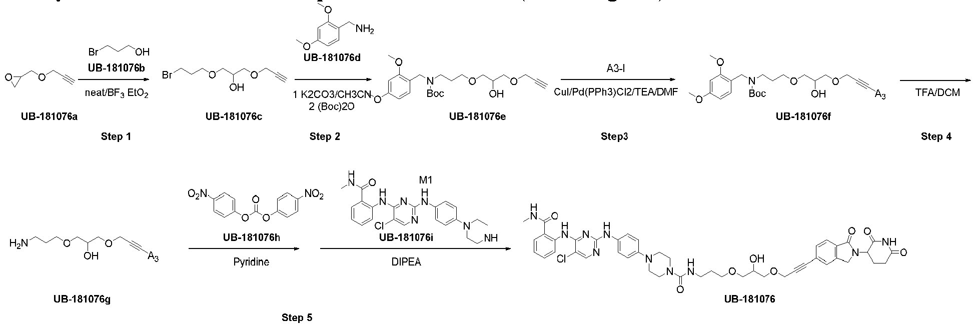

### Step 1: UB-181076c (V2595-046)

**UB-181076a** (2.0 g, 17.86 mmol) was dissolved in **UB-181076b** (25 ml), cooled to 0°C, then BF₃Et₂O (320 mg, 1.8 mmol) was added, the mixture was reacted at room temperature for 2 h. The reaction solution was dried by rotary dryer and passed through column (DCM/MeOH=20/1) to obtain **UB-181076c** (2.7 g, 69% yield) as a yellow oil.

¹H NMR (400 MHz, Chloroform-*d*) δ 4.21 (d, *J=* 2.4 Hz, 1H), 3.99 (tt, *J=* 6.2, 4.4 Hz, 1H), 3.69 - 3.46 (m, 11H), 2.47 (t, *J=* 2.4 Hz, 1H), 2.17 - 2.04 (m, 6H).LCMS [M+H]⁺=251.

### Step 2: UB-181076e (V2595-058)

**UB-181076c** (100 mg, 0.4 mmol) and **UB-181076d** (70 mg, 0.4 mmol) were dissolved in ACN (10 ml), K2CO3 (110 mg, 0.8 mmol) was added. The reaction solution was heated to 100 °C to react for 40min. Then returned to room temperature, to the reaction solution was added Boc2O(100 mg), the mixture was continued to react at room temperature for 1 h. The reaction solution was diluted with water, extracted with EtOAc(80 mL*3). Then the combined organic layers were washed with saturated saline, dried over Na2SO4 and filtered. The solvent was removed in vacuum to obtain crude product, and the crude product was purified via silica gel column chromatography (DCM/MeOH=10/1) to obtain **UB-181076e** (35 mg, 30% yield) as an oil.

¹H NMR (400 MHz, Chloroform-*d*) δ 7.37 - 7.23 (m, 1H), 6.53 - 6.38 (m, 2H), 5.30 (s, 1H), 4.18 (dd, *J=* 7.2, 2.4 Hz, 1H), 4.01 (d, *J=* 1.5 Hz, 1H), 3.86 (d, *J=* 3.0 Hz, 2H), 3.84 - 3.76 (m, 5H), 3.69 - 3.61 (m, 2H), 3.60 - 3.47 (m, 3H), 2.97 (dt, *J* = 15.3, 5.9 Hz, 1H), 2.71 - 2.61 (m, 1H), 2.08 - 1.63 (m, 3H).LCMS [M+H]⁺=438.

### Step 3: UB-181076f (V2595-057)

**UB-181076e** (160 mg, 0.37 mmol), I-A3 (135 mg, 0.37 mmol), Pd(PPh3)2Cl2 (15 mg, 0.07 mmol), CuI (10 mg, 0.07 mmol), and TEA (40 mg, 0.37 mmol) were dissolved in anhydrous DNF. The reaction solution was purged with N2 for three times and heated to 80 °C to react for 2 h under N2 atmosphere. The reaction solution was diluted with water, extracted with EtOAc(80 mL*3). Then the combined organic layers were washed with saturated saline, dried over Na2SO4 and filtered. The solvent was removed in vacuum to obtain crude product, and the crude product was purified via silica gel column chromatography (DCM/MeOH=20/1) to obtain **UB-181076f** (35 mg, 30% yield) as a yellow solid.

¹H NMR (400 MHz, DMSO-*d*₆) δ 11.00 (s, 1H), 7.79 - 7.65 (m, 2H), 7.56 (dd, *J=* 7.9, 1.4 Hz, 1H), 6.97 (d, *J=* 8.3 Hz, 1H), 6.57 - 6.40 (m, 2H), 5.12 (dd, *J=* 13.3, 5.1 Hz, 1H), 4.87 (d, *J=* 5.2 Hz, 1H), 4.49 - 4.16 (m, 6H), 3.75 (d, *J=* 12.8 Hz, 7H), 3.57 - 3.38 (m, 3H), 3.37 (d, *J* = 6.2 Hz, 2H), 3.32 - 3.26 (m, 1H), 3.20 - 3.10 (m, 3H), 2.97 - 2.83 (m, 1H), 2.60 (ddd, *J* = 17.2, 4.4, 2.3 Hz, 1H), 2.39 (qd, *J* = 13.6, 4.8 Hz, 1H), 2.06 - 1.95 (m, 1H), 1.64 (ddd, *J* = 9.0, 5.7, 2.4 Hz, 2H), 1.43 - 1.31 (m, 9H).LCMS [M+H]⁺ = 680.

### Step 4: UB-181076g (V2595-062)

**UB-181076f** (70 mg, 0.1 mmol) was dissolved in DCM, then TFEA was added, the mixture was heated to 40°C and reacted for 2 h. The reaction solution was filtered and concentrated in vacuum to obtain **UB-181076g** (50 mg, 100% yield) as a yellow solid. LCMS [M+H]⁺ =430.

### Step 5: UB-181076 (V2595-064)

**UB-181076g,** and **UB-181076h** were dissolved in anhydrous pyridine, the mixture was stirred at room temperature for 2 h. Then M1 and DIPEA were added and continued to stir for 1 h. The reaction solution was concentrated in vacuum to obtain crude product, and the crude product was purified via silica gel column chromatography (DCM/MeOH=15/1) to obtain **UB-181076** (5 mg, 13% yield) as a white solid.

¹H NMR (400 MHz, DMSO-*d*₆) δ 11.98 (s, 1H), 11.00 (s, 1H), 10.04 (s, 1H), 8.87 (q, *J* = 4.6 Hz, 1H), 8.62 (d, *J* = 8.5 Hz, 1H), 8.31 (s, 1H), 7.91 - 7.39 (m, 9H), 7.21 (t, *J* = 7.6 Hz, 1H), 6.75 (s, 1H), 5.11 (dd, *J* = 13.2, 5.1 Hz, 1H), 4.56 - 4.24 (m, 5H), 3.55 (dd, *J* = 9.8, 4.6 Hz, 3H), 3.41 (qdt, *J=* 15.7, 9.9, 5.9 Hz, 12H), 3.13 (t, *J=* 6.9 Hz, 2H), 2.90 (td, *J* = 13.3, 6.8 Hz, 2H), 2.81 (d, *J* = 4.4 Hz, 3H), 2.60 (d, *J* = 18.0 Hz, 1H), 2.39 (qd, *J* = 13.3, 4.4 Hz, 1H), 2.01 (dt, *J* = 11.0, 5.2 Hz, 1H), 1.68 (q, *J=* 6.6 Hz, 2H).LC-MS: [M+H]+ = 894

### Synthesis method of Compound UB-181083 (M1-11cg-A1)

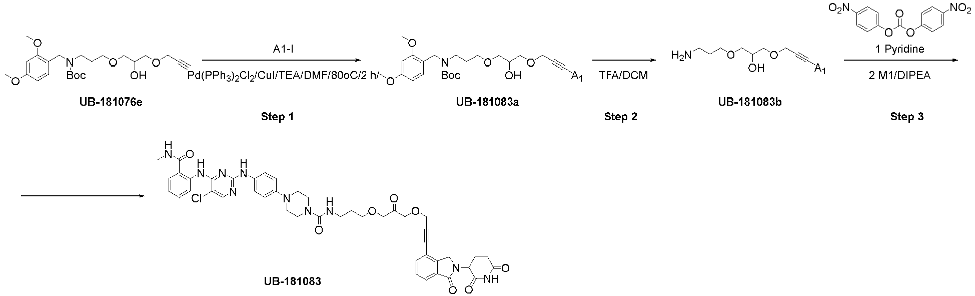

### Step 1: UB-181083a (V2595-067)

**UB-181076f** (200 mg, 0.45 mmol), I-A1 (170 mg, 0.45 mmol), Pd(PPh₃)₂Cl₂ (15 mg, 0.07 mmol), CuI (10 mg, 0.07 mmol), and TEA (100 mg, 0.9 mmol) were dissolved in anhydrous DNF. The reaction solution was purged with N2 for three times and heated to 80 °C to react for 2 h under N2 atmosphere. The reaction solution was diluted with water, extracted with EtOAc(80 mL*3). Then the combined organic layers were washed with saturated saline, dried over Na2SO4 and filtered. The solvent was removed in vacuum to obtain crude product, and the crude product was purified via silica gel column chromatography (DCM/MeOH=20/1) to obtain **UB-181083a** (40 mg, 13% yield) as a yellow solid. LCMS [M+H]⁺ = 680

### Step 2: UB-181083b (V2595-071)

**UB-181083a** (10 mg, 0.015 mmol) was dissolved in DCM, then TFEA was added, the mixture was heated to 40°Cand reacted for 2 h. The reaction solution was filtered and concentrated in vacuum to obtain **UB-181083b** (8 mg, 100% yield) as a yellow solid. LCMS [M+H]⁺ =430.

### Step 3: UB-181083 (V2595-072)

**UB-181083b,** and **UB-181083c** were dissolved in pyridine, the mixture was stirred at room temperature for 2 h. Then M1 and DIPEA were added and continued to stir for 1 h. The reaction solution was concentrated in vacuum to obtain crude product, and the crude product was purified via silica gel column chromatography (DCM/MeOH=15/1) to obtain **UB-181083** (5 mg, 40% yield) as a white solid.

¹H NMR (400 MHz, DMSO-*d*₆) δ 11.90 (s, 1H), 11.01 (s, 1H), 9.82 (s, 1H), 8.83 (q, *J*= 4.6 Hz, 1H), 8.66 (d, *J=* 8.2 Hz, 1H), 8.27 (s, 1H), 7.83 - 7.68 (m, 3H), 7.56 (ddd, *J=* 27.1, 19.2, 8.2 Hz, 4H), 7.37 (s, 2H), 7.19 (t, *J=* 7.6 Hz, 1H), 6.67 (s, 1H), 5.15 (dd, *J=* 13.3, 5.1 Hz, 1H), 4.55 - 4.27 (m, 5H), 3.78 (p, *J=* 5.6 Hz, 3H), 3.42 (t, *J=* 6.3 Hz, 5H), 3.39 - 3.34 (m, 3H), 3.32 (t, *J=* 6.8 Hz, 5H), 3.11 (t, *J=* 7.0 Hz, 3H), 2.81 (d, *J=* 4.4 Hz, 3H), 2.07 - 1.87 (m, 2H), 1.65 (p, *J=* 6.6 Hz, 2H), 1.24 (d, *J=* 3.4 Hz, 2H).LC-MS: [M+H]⁺ = 894

### Synthesis method of Compound UB-181110 (M1-11g-A3)

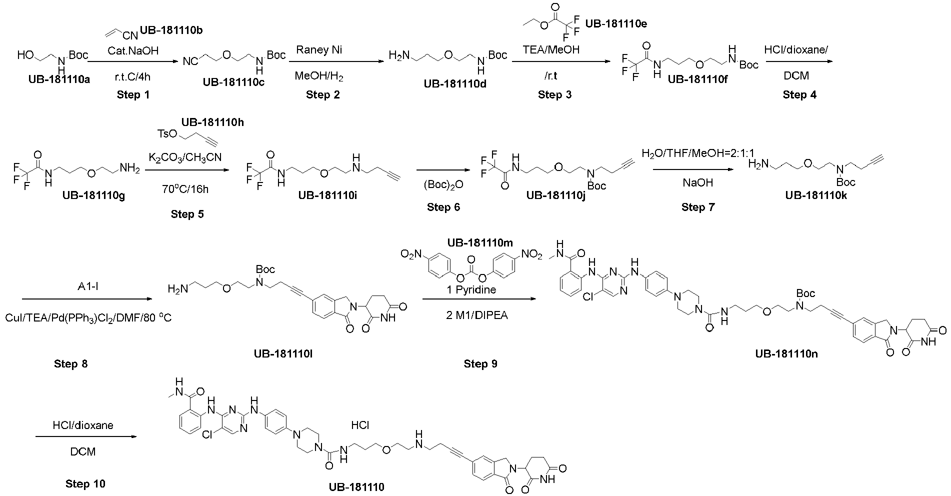

### Step 1: UB-181110c (V2595-081)

**UB-181110a** (20 g, 124 mmol) was dissolved in **UB-181110b** (100 ml), sodium hydroxide (250 mg, 6.2 mmol, in water) was added, the mixture was stirred at room temperature for 4 hours. The mixture was concentrated to obtain crude product **UB-181110c**(13 g, yield 89%) as a yellow oil.

¹H NMR (400 MHz, Chloroform-*d*) δ 3.72 - 3.52 (m, 6H), 3.48 (t, *J* = 5.0 Hz, 2H), 2.71 - 2.53 (m, 4H), 1.53 - 1.40 (m, 9H). LCMS [M+H]⁺=229.

### Step 2: UB-181110d (V2595-083)

Under the protection of nitrogen, Raney nickel (500 mg) was added to the solution of **UB-181110c** (22 g, 102 mmol) in methanol. The suspension was degassed under vacuum and replaced several times with hydrogen. The reaction mixture was stirred at room temperature for 2 hours under hydrogen atmosphere, the mixture was filtered and concentrated in vacuum to obtain **UB-181110d**(22 g, yield 80 %) as a yellow oil.

¹H NMR (400 MHz, Chloroform-*d*) δ 3.72 - 3.19 (m, 8H), 3.06 - 2.47 (m, 3H), 1.74 (t, *J* = 49.9 Hz, 2H), 1.58 - 1.35 (m, 9H). LCMS [M+H]⁺=219.

### Step 3: UB-181110f (V2595-085)

**UB-181110d** (11 g, 50 mmol) and **UB-181110e** (8 g, 50 mmol) were dissolved in methanol (200 mL), triethylamine (6 g, 50 mmol) was added, the mixture was stirred at room temperature for 2 hours. The mixture was filtered, concentrated, and the residue was purified by silica gel chromatography (PE/EA = 5/1) to give **UB-181110f** (4 g, 28% yield) as a yellow oil.

¹H NMR (400 MHz, Chloroform-*d*) δ 3.63 - 3.42 (m, 8H), 3.40 - 3.26 (m, 2H), 1.92 - 1.81 (m, 2H), 1.51 - 1.41 (m, 9H). LCMS [M+H]⁺=315.

### Step 4: UB-181110g (V2595-086)

**UB-181110f** (4 g, 12.7 mmol) was dissolved in DCM, cooled to 0°C, and 4 M hydrochloric acid/dioxane (10 mL) was added, the reaction was stirred at room temperature for 2 hours. The solvent was concentrated to give **UB-181110f** (3.18 g, 100%yield) as a yellow oil. LCMS [M+H]⁺=215.

### Step 5: UB-181110i (V2595-088)

**UB-181110g** (3.18 g, 14.86 mmol) and **UB-181110h** (2.85 g, 14.86 mmol) were dissolved in CAN, K2CO3 (3.5 g, 29.72 mmol) was added, the reaction solution was heated to 70°C and reacted for 18 h. The reaction was dried over Na2SO4 and filtered. The solvent was removed in vacuum to obtain crude product, and the crude product was purified via silica gel column chromatography (DCM/MeOH=10/1) to obtain **UB-181110i** (950 mg, 25% yield) as a yellow oil. LCMS [M+H]⁺=267.

### Step 6: UB-181110j (V2595-110)

**UB-181110i** and Boc₂O were dissolved in THF, then Na₂CO₃ was added, the reaction was reacted at room temperature for 1 h. The reaction solution was diluted with water, extracted with EtOAc(80 mL*3). Then the combined organic layers were washed with saturated saline, dried over Na2SO4 and filtered. The solvent was removed in vacuum to obtain crude product, and the crude product was purified via silica gel column chromatography (PE/EA=10/1) to obtain **UB-181110j** (950 mg, 90% yield) as a yellow oil.

¹H NMR (400 MHz, Chloroform-*d*) δ 3.66 - 3.28 (m, 10H), 2.43 (td, *J=* 7.1, 2.7 Hz, 2H), 1.98 (t, *J* = 2.7 Hz, 1H), 1.91 - 1.78 (m, 2H), 1.46 (s, 9H). LCMS [M+H]⁺=367.

### Step 7: UB-181110k (V2790-036)

**UB-181110j** (950 mg, 2.6 mmol) was dissolved in MeOH:THF:H2O=2:2:1, then NaOH (420 mg, 10.4 mmol, aqueous solution) was added, the mixture was reacted at room temperature for 2 h. The reaction solution was dried in vacuum to give **UB-181110k** (400 mg, 60% yield) as a yellow oil.

¹H NMR (400 MHz, Chloroform-*d*) δ 3.61 - 3.27 (m, 9H), 3.05 (s, 2H), 2.42 (td, *J* = 7.0, 2.6 Hz, 2H), 2.02 - 1.83 (m, 4H), 1.45 (s, 9H). LCMS [M+H]⁺=271.

### Step 8: UB-181110l (V2595-115)

**UB-181110k** (200 mg, 0.74 mmol), **I-A3** (275 mg, 0.74 mmol), Pd(PPh₃)₂Cl₂ (26 mg, 0.04 mmol), CuI (7 mg, 0.04 mmol), and TEA (150 mg, 1.5 mmol) were dissolved in anhydrous DMF, The reaction solution was purged with N2 for three times and heated to 80 °C to react for 2 h under N2 atmosphere. The reaction solution was diluted with water, extracted with EtOAc(80 mL*3). Then the combined organic layers were washed with saturated saline, dried over Na2SO4 and filtered. The solvent was removed in vacuum to obtain crude product, and the crude product was purified via silica gel column chromatography (DCM/MeOH=10/1) to obtain **UB-181110l** (110 mg, 30% yield) as a yellow solid.

¹H NMR (400 MHz, DMSO-*d*₆) δ 7.70 (d, *J=* 7.9 Hz, 1H), 7.62 (s, 1H), 7.50 (dd, *J=* 7.9, 1.3 Hz, 1H), 5.11 (dd, *J=* 13.3, 5.1 Hz, 1H), 4.52 - 4.25 (m, 2H), 3.47 (dq, *J* = 21.3, 7.3, 6.5 Hz, 8H), 3.00 - 2.78 (m, 3H), 2.76 - 2.54 (m, 2H), 2.39 (qd, *J* = 13.2, 4.4 Hz, 1H), 2.04 - 1.92 (m, 2H), 1.78 (s, 2H), 1.40 (s, 9H). LCMS [M+H]⁺ = 513.

### Step 9: UB-181110n (V2595-117)

**UB-181110l,** and **UB-181110m** were dissolved in pyridine, the mixture was stirred at room temperature for 2 h. Then M1 and DIPEA were added and continued to stir for 1 h. The reaction solution was concentrated in vacuum to obtain crude product, and the crude product was purified via silica gel column chromatography (DCM/MeOH=15/1) to obtain **UB-181110n** (200 mg, 85% yield) as a white solid. LC-MS: [M+H]⁺ = 976.

### Step 10: UB-181110 (V2595-120)

**UB-181110n** (40 mg, 0.04 mmol) was dissolved in DCM, then cooled to 0°C, then 4 M hydrochloric acid in dioxane was added, the mixture was reacted at room temperature for 2 h. The reaction solution was directly dried by rotary dryer under vacuum to obtain **UB-181110** (19 mg, 80% yield) as a white solid.

¹H NMR (400 MHz, DMSO-*d*₆) δ 11.89 (s, 1H), 11.00 (s, 1H), 9.80 (s, 1H), 9.06 (s, 2H), 8.84 (d, *J=* 4.8 Hz, 1H), 8.67 (s, 1H), 8.27 (s, 1H), 7.79 (dd, *J=* 7.9, 1.6 Hz, 1H), 7.74 - 7.68 (m, 2H), 7.66 - 7.55 (m, 3H), 7.51 (t, *J=* 7.9 Hz, 1H), 7.35 (s, 2H), 7.21 - 7.12 (m, 1H), 6.84 (s, 1H), 5.11 (dd, *J=* 13.3, 5.1 Hz, 1H), 3.69 (t, *J=* 5.1 Hz, 4H), 3.48 (t, J= 6.3 Hz, 3H), 3.30 (s, 4H), 3.27 - 3.22 (m, 3H), 3.17 (dt, *J=* 10.8, 6.8 Hz, 5H), 3.13 - 3.09 (m, 1H), 2.97 (t, *J=* 7.3 Hz, 2H), 2.81 (d, J= 4.5 Hz, 3H), 2.06 - 1.90 (m, 2H), 1.70 (t, *J=* 6.4 Hz, 3H), 1.33 - 1.15 (m, 4H). LCMS [M+H]⁺=877.

### Synthesis method of Compound UB-181111 (M1-11g-A1)

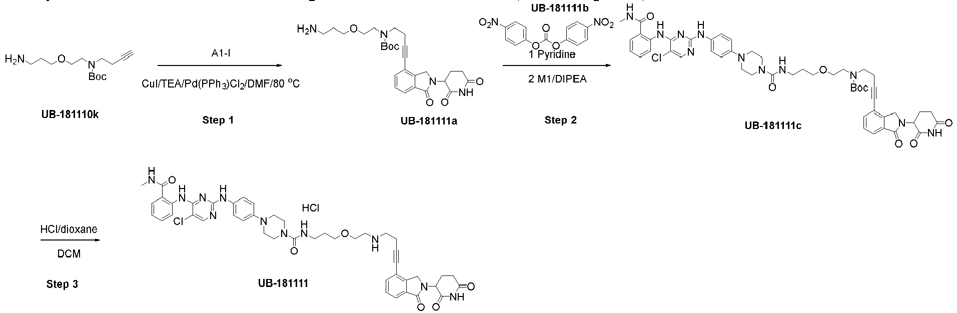

### Step 1: UB-181111a (V2595-123)

**UB-181110k** (200 mg, 0.74 mmol), I-A1 (275 mg, 0.74 mmol), Pd(PPh₃)₂Cl₂ (26 mg, 0.04 mmol), CuI (7 mg, 0.04 mmol), and TEA (150 mg, 1.5 mmol) were dissolved in anhydrous DMF, The reaction solution was purged with N2 for three times and heated to 80 °C to react for 2 h under N2 atmosphere. The reaction solution was diluted with water, extracted with EtOAc(80 mL*3). Then the combined organic layers were washed with saturated saline, dried over Na2SO4 and filtered. The solvent was removed in vacuum to obtain crude product, and the crude product was purified via silica gel column chromatography (DCM/MeOH=10/1) to obtain **UB-181111a** (30 mg, 10% yield) as a yellow solid. LCMS [M+H]⁺ = 513.

### Step 9: UB-181111c (V2595-127)

**UB-1811111a,** and **UB-181111b** were dissolved in pyridine, the mixture was stirred at room temperature for 2 h. Then M1 and DIPEA were added and continued to stir for 1 h. The reaction solution was concentrated in vacuum to obtain crude product, and the crude product was purified via silica gel column chromatography (DCM/MeOH=15/1) to obtain **UB-181110l** (30 mg, 85% yield) as a white solid. LC-MS: [M+H]⁺ = 976.

### Step 10: UB-181111 (V2595-130)

**UB-181111cn** (30 mg, 0.04 mmol) was dissolved in DCM, then cooled to 0°C, then 4 M hydrochloric acid in dioxane was added, the mixture was reacted at room temperature for 2 h. The reaction solution was directly dried by rotary dryer under vacuum to obtain **UB-181111** (9 mg, 80% yield) as a white solid.

¹H NMR (400 MHz, DMSO-*d*₆) δ 11.97 (s, 1H), 11.02 (s, 1H), 10.04 (s, 1H), 9.29 (s, 2H), 8.89 (d, *J* = 4.8 Hz, 1H), 8.63 (d, *J* = 8.7 Hz, 1H), 8.31 (s, 1H), 7.82 (dd, *J* = 7.9, 1.6 Hz, 1H), 7.77 - 7.62 (m, 4H), 7.54 (td, *J* = 7.4, 3.8 Hz, 3H), 7.20 (td, *J* = 7.6, 1.2 Hz, 1H), 6.93 (s, 1H), 5.16 (dd, *J* = 13.3, 5.1 Hz, 1H), 4.57 - 4.25 (m, 3H), 3.70 (t, *J* = 5.1 Hz, 4H), 3.52 - 3.32 (m, 6H), 3.20 (dq, *J* = 26.2, 6.7 Hz, 6H), 3.06 - 2.87 (m, 3H), 2.81 (d, *J* = 4.4 Hz, 3H), 2.70 - 2.53 (m, 2H), 2.48 - 2.31 (m, 1H), 2.02 (dq, *J=* 9.2, 3.5, 3.0 Hz, 1H), 1.70 (p, *J* = 6.5 Hz, 2H), 1.36 - 1.18 (m, 2H). LCMS [M+H]⁺=877.

### Synthesis method of Compound UB-181149 (MC-Ala-Ala-Asn-PAB-961)

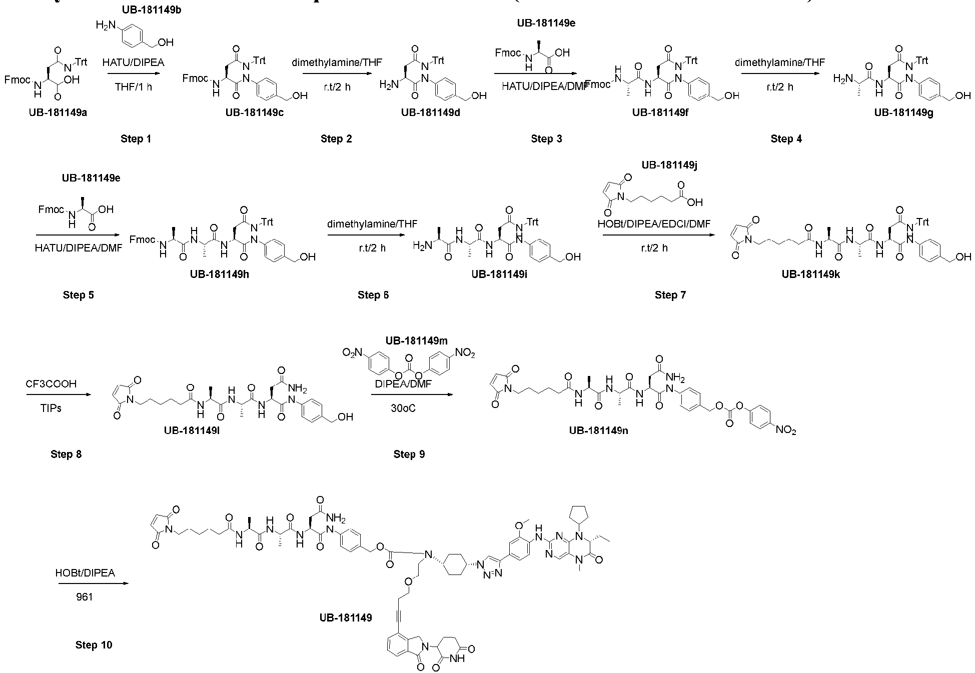

### Step 1: UB-181149c (V2790-075)

**UB-181149a** (50 g, 84 mmol), **UB-181149b** (10.3 g, 84 mmol) and HATU (38.2 g, 126 mmol) were dissolved in THF (600ML), then DIEA (21.6 g, 210 mmol) was added and the mixture was reacted at room temperature for 2 h. The reaction solution was filtered, dried in vacuum to obtain crude product, then it was slurried three times with ether to obtain UB-181149c (70 g, 80% yield) as a yellow solid. LCMS [M+H]⁺ = 702.

### Step 2: UB-181149d (V2790-077)

**UB-181149c**(70 g, 99.8 mmol) was dissolved in THF(300 ml), then **dimethylamine**(300 ml) was added, the mixture was reacted at room temperature for 2 h. The reaction solution was dried in vacuum to obtain crude product, then it was slurried with ether to obtain **UB-181149d** (40 g, 80% yield) as a yellow solid. LCMS [M+H]⁺ =480.

### Step 3: UB-181149f (V2790-089)

To a solution of UB-181149d (70 g, 146 mmol), UB-181149e(43 g, 132 mmol) and HATU(67 g, 175.1 mmol) in DMF(1.5 l) was added with DIEA (57 g, 292 mmol). The reaction solution was stirred at room temperature for 2 hours. The reaction mixture was poured into water. The reaction mixture was filtered, the filter cake was washed with ether and dried in vacuum to obtain UB-181149f (70 g, 80% yield ) as a yellow solid. LC-MS: [M+H]⁺ = 844.

### Step 4: UB-181149g (V2790-031)

UB-181149f(13 g, 16.8 mmol) was dissolved in THF(200 ml), then **dimethylamine**(300 ml) was added, the mixture was reacted at room temperature for 2 h. The reaction solution was dried in vacuum to obtain crude product, then it was slurried with ether to obtain **UB-181149g** (8.0 g, 80% yield) as a yellow solid. LC-MS: [M+H]⁺ = 551.

### Step 5: UB-181149h (V2790-032)

To a solution of **UB-181149g** (5.0 g, 9.1 mmol), **UB-181149e** (2.8 g, 9.1 mmol) and HATU(5.2 g, 13.6 mmol) in DMF (200ml) was added with DIEA (3.5 g, 27.3 mmol). The reaction solution was stirred at room temperature for 2 hours. The reaction mixture was poured into water. The reaction mixture was filtered, the filter cake was washed with ether and dried in vacuum to obtain **UB-181149h** (4.8 g, 80% yield) as a yellow solid. LC-MS: [M+H]⁺ = 843.

### Step 6: UB-181149i (V2790-035)

UB-181149h (2.6 g, 3.1 mmol) was dissolved in THF(100 ml), then **dimethylamine**(40ml) was added, the mixture was reacted at room temperature for 2 h. The reaction solution was dried in vacuum to obtain crude product, then it was slurried with ether to obtain **UB-181149i** (1.0 g, 80% yield) as yellow solid. LC-MS: [M+H]⁺ = 622.

### Step 7: UB-181149k (V2790-036)

**UB-181149j,** and HOBT were dissolved in DMF, then DIEA (119 mg, 0.92 mmol) was added. The reaction solution was cooled to 0 °C, then **UB-181149i and** EDCI in DMF were added dropwise, the mixture was reacted at room temperature for 7 hours. The reaction solution was poured into water, the mixture was extracted with ethyl acetate, the obtained organic phase was washed once with diluted hydrochloric acid and saturated brine, and dried over anhydrous sodium sulfate, combined organic phase was evaporated to dryness in vacuum and purified via silica gel chromatography (DCMIDCM:MeOH:THF(10:0.5:0.5)=0-96%) to obtain **UB-181149k** (107 mg, yield%) as a white solid. LC-MS: [M+H]⁺ =815.

### Step 8: UB-181149l (V2790-038)

UB-181149k (100 mg, 0.10 mmol) was dissolved in TIS (1 mL), cooled to 0 °C, then CF3COOH (2 ml) was added, the mixture was reacted at 0 °C for 15min. The reaction solution was filtered, and purified via silica gel chromatography (H2O: acetonitrile =0%-12%) to obtain **UB-181149l** (40 mg, 30% yield) as a white solid. LC-MS: [M+H]⁺=573.

### Step 9: UB-181149n (V2790-039)

**UB-181149l** (40 mg, 0.07 mmol) and **UB-181149m** (43 mg, 0.14 mmol) were dissolved in DMF(2.5 ml), then DIPEA (50 mg, 0.36 mmol) was added, the mixture was reacted at room temperature for 18 h. The reaction solution was dried by rotary dryer under vacuum, and purified by silica gel column chromatography (water: acetonitrile =0%-12%) to obtain **UB-181149n** (30 mg, 75% yield) as a white solid. LC-MS: [M+H]⁺ =738.

### Step 10: UB-181149 (V2790-042)

**UB-181149n** (27 mg, 0.039 mmol), 961 (31.5 mg, 0.032 mmol), HOBt (10 mg, 0.073 mmol), and DIPEA (14 mg, 0.11 mmol) were dissolved in DMF (5 mL), the mixture was reacted at room temperature for 18 hours. The reaction solution was purified by HPLC to obtain **UB-181149** (10 mg, 45% yield) as a white solid. LC-MS: [M+H]⁺ =1483.

### Synthesis method of Compound UB-180947

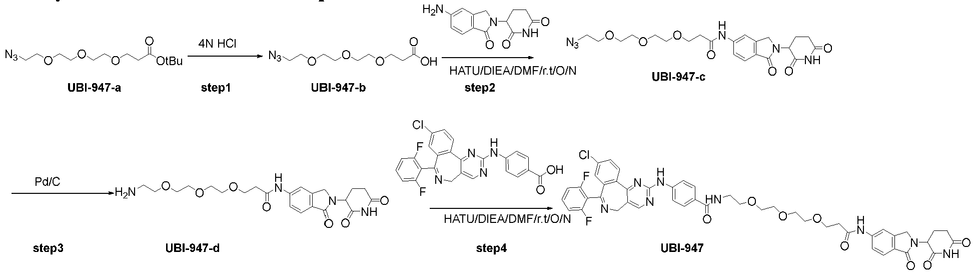

### Step 1: UB-180947b (V2127-049)

Compound **UB-180947a** (800 mg, 2.64 mmol) and hydrochloric acid in dioxane (10 mL, 4 N) were added to tetrahydrofuran (10mL), the mixture was reacted at room temperature for 2 hours. After the completion of the reaction, the mixture was concentrated by rotary evaporation under reduced pressure to obtain compound **UB-947b** (652 mg, yield 100%). LC-MS: [M+H] ⁺ =248.1

### Step 2: UB-180947c (V2127-051)

Compound **UB-180947b** (652 mg, 2.64 mmol) and 3-(5-amino-1-oxo-2,3-dihydro-1H-isoindol-2-yl)piperidine-2,6-dione(684 mg, 2.64 mmol), and HATU (1015 mg, 2.67 mmol) were added to a mixture of DMF (3mL) and DIPEA(0.3mL) successively, the mixture was reacted at room temperature for 16 h. The crude product was purified by reversed- phase chromatography column (MeOH/H₂O=5% to 95%, 45mins). to obtain product **UB-947c** (846 mg, yield 66%). LC-MS: [M+H] ⁺ =489.2

### Step 3: UB-180947d (V2127-053)

**UB-180947c** (50 mg, 0.10 mmol), and 10% palladium on carbon (5 mg) was added to a mixed solvent of methanol/dichloromethane (1 mL/10 mL), and reacted at room temperature for 16 hours under hydrogen atmosphere. After filtration, the filtrate was concentrated to obtain the crude product. The crude product was washed with cold ether (10 mL*3), and dried to obtain target compound **UB-947d** (39 mg, yield 83%). LCMS: (M+H) ⁺ =463.3

### Step 4: UB-180947d (V2127-057)

**The method is similar to General Method 1**

¹H NMR (400 MHz, *d6-DMSO*) δ 10.95 (s, 1H), 10.29 (s, 1H), 10.16 (s, 1H), 8.72 (s, 1H), 8.49 - 8.25 (m, 2H), 7.98 (s, 1H), 7.87 (ddd, *J=* 18.0, 13.3, 8.9 Hz, 5H), 7.71 - 7.38 (m, 3H), 7.34 (d, *J* = 2.1 Hz, 1H), 7.17 (t, *J* = 8.5 Hz, 2H), 5.06 (dt, *J* = 33.1, 16.6 Hz, 1H), 4.42 (d, *J* = 17.3 Hz, 1H), 4.36 - 4.20 (m, 1H), 3.56 - 3.48 (m, 11H), 3.41 - 3.32 (m, 2H), 2.94 - 2.71 (m, 1H), 2.60 (t, *J=* 6.1 Hz, 3H), 2.37 (qd, *J=* 13.3, 4.4 Hz, 1H), 2.08 - 1.85 (m, 1H). LCMS [M+H]⁺ =921.4

### Synthesis method of Compound UB-180948

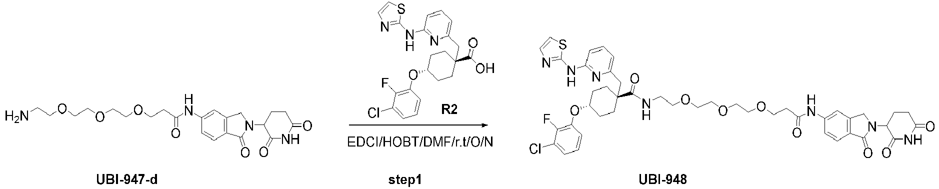

### Step 1: UB-180948 (V2127-058)

**The method is similar to General Method 2**

¹H NMR (400 MHz, *d6-DMSO*) δ 11.12 (s, 1H), 10.97 (s, 1H), 10.29 (s, 1H), 7.98 (s, 1H), 7.58 (ddt, *J* = 9.2, 7.2, 14.0 Hz, 4H), 7.39 - 7.01 (m, 4H), 7.01 - 6.80 (m, 1H), 6.86 (d, *J* = 8.1 Hz, 1H), 6.64 (t, *J=* 6.0 Hz, 1H), 5.08 (dd, *J=* 13.3, 5.1 Hz, 1H), 4.53(d, *J=* 10.5 Hz, 1H), 4.34 - 4.17 (m, 2H), 3.70 (t, *J=* 6.2 Hz, 2H), 3.55 - 3.46 (m, 8H), 3.26 - 3.08 (m, 2H), 2.99 - 2.82 (m, 2H), 2.69 - 2.55 (m, 3H), 1.98 (dd, *J* = 6.7, 3.8 Hz, 1H), 1.87 (dd, *J* = 18.0, 7.2 Hz, 2H), 1.75 (dd, *J* = 7.0, 4.0 Hz, 4H), 1.59 (t, *J* = 11.1 Hz, 2H). LCMS [M+H]⁺ =906.4

### Synthesis method of Compound UB-180950

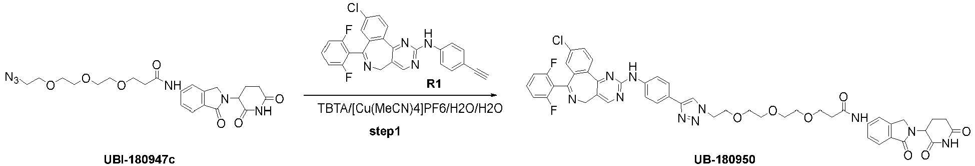

### Step 1: UB-180950 (V2127-064)

**The method is similar to General Method 3**

¹H NMR (400 MHz, *d6-DMSO*) δ 10.97 (s, 1H), 10.26 (s, 1H), 10.00 (s, 1H), 8.68 (s, 1H), 8.40 (s, 1H), 8.30 (d, *J=* 8.5 Hz, 1H), 7.97 (s, 1H), 7.96 - 7.84 (m, 4H), 7.79 (d, *J=* 8.7 Hz, 2H), 7.57 (ddt, *J=* 15.0, 8.3, 7.5 Hz, 4H), 7.33 (d, *J=* 2.1 Hz, 1H), 7.17 (t, *J=* 8.4 Hz, 2H), 5.04 (dt, *J* = 5.8, 2.4 Hz, 1H), 4.54 (t, *J* = 5.1 Hz, 2H), 4.27 (d, *J* = 17.3 Hz, 2H), 3.84 (t, *J* = 5.1 Hz, 2H), 3.69 (t, *J* = 6.2 Hz, 2H), 3.61 - 3.44 (m, 8H), 3.03 - 2.79 (m, 1H), 2.75 - 2.67 (m, 3H), 2.36 (qd, *J=* 13.3, 4.6 Hz, 1H), 2.09 - 1.84 (m, 1H).LCMS [M+H]⁺ =945.3

### Synthesis method of Compound UB-180953

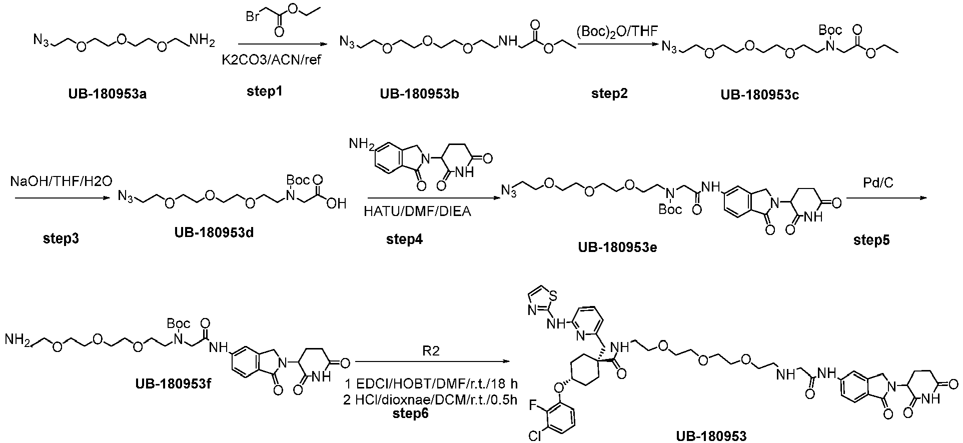

### Step 1: UB-180953b (V2127-068)

Compound UBI-180953a (970 mg, 4.45 mmol) , bromo ethyl acetate (970 mg, 4.45 mmol), and potassium carbonate (2.06 g, 14.9 mmol) were added to anhydrous acetonitrile (20 mL), the mixture was reacted at 80°C for 18 hours. After the completion of the reaction, the reaction solution was concentrated. The crude was isolated by silica gel column chromatography (DCM/MeOH = 10/1) to obtain compound **UB-180953b** (710 mg, yield 51%) as a colorless oil.

### Step 2: UB-180953c(V2127-069)

**Compound UB-180953b** (710 mg, 2.34 mmol), di-tert-butyl dicarbonate (938 mg, 4.30 mmol) and sodium bicarbonate (360 mg, 4.29mmol) were added to tetrahydrofuran (20mL) successively and reacted at room temperature for 2 hours. After the completion of the reaction, the reaction solution was poured into 10 mL of water and extracted with dichloromethane (10 mL*3). The organic phases were combined, then washed with saturated brine, dried over anhydrous Na₂SO₄, and concentrated by rotary evaporation under reduced pressure to obtain compound **UB-180953c(670** mg, yield 71%). LCMS: [M+H]⁺ =405.3

### Step 3: UB-180953d (V2127-071)

Compound **UB-1095c** (1.5g, 6.0 mmol), and sodium hydroxide (69.6 mg, 1.74mmol) were added to water (5 mL) successively, reacted at 30°C for 12 hours. After the completion of the reaction, it was concentrated and the aqueous phase was acidified to pH=5 using hydrochloric acid (1M). Then it was extracted with dichloromethane (10ml*3), the combined organic layer was dried over anhydrous Na₂SO₄, and concentrated to obtain the desired compound **UB-180953d** (567 mg, yield 91%). LCMS: [M+H]⁺ =377.4

### Step 4: UB-180953e(V2127-072)

Compound **UB-180953c** (670 mg, 1.66 mmol), 3-(5-amino-1-oxo-2,3-dihydro-1H-isoindol-2-yl)piperidine-2,6-dione(390 mg, 1.51 mmol), HATU (714 mg, 0.97) and DIPEA (0.3mL) were added to anhydrous DMF (10 mL). The mixture was reacted at room temperature for 16 hours. After the completion of the reaction, concentrated to obtain crude product which was isolated by reverse chromatography column chromatography (MeOH/H₂O = 5% to 95%, 45mins) to obtain **UBI-180953e** (576 mg, yield 62%) as a colorless oil. [M+H]⁺ =618.2

### Step 5: UB-180953f (V2127-073)

**UB-180953e(200** mg, 0.32 mmol), and 10% palladium on carbon (20 mg) was added to a mixed solvent of methanol/dichloromethane (1 mL/10 mL), and reacted at room temperature for 2 hours under hydrogen atmosphere. After filtration, the filtrate was concentrated to obtain the crude product. The crude product was washed with cold ether (10 mL*3), and dried to obtain target compound **UB-180953f** (170 mg, yield 89 %).

LCMS: (M+H) ⁺ = 592.6

### Step 6: UB-180953(V2127-075)

### The method is similar to General Method 2

¹H NMR (400 MHz, DMSO-*d*₆) δ 12.69-12.64 (m, 1H), 11.32 (s, 1H), 11.04 - 10.83 (m, 1H), 9.21 (s, 2H), 8.06 (s, 1H), 7.91 - 7.58 (m, 4H), 7.55 (t, *J=* 7.0 Hz, 1H), 7.35 - 7.06 (m, 4H), 7.06 - 6.64 (m, 1H), 6.77 (s, 1H), 6.77 (s, 2H), 5.14 - 5.01 (m, 2H), 4.51 (d, *J* = 31.1 Hz, 1H), 4.43 (s, 1H), 4.40 - 4.19 (m, 2H), 4.06 (s, 2H), 3.73 (t, *J* = 5.0 Hz, 2H), 3.54 (d, *J=* 15.3 Hz, 3H), 3.53 - 3.39 (m, 5H), 3.27 (dt, *J* = 9.0, 4.9 Hz, 4H), 3.19 - 2.98 (m, 2H), 2.98 (s, 2H), 3.04 - 2.83 (m, 1H), 2.83 - 2.69 (m, 1H), 2.62 (t, *J=* 19.5 Hz, 1H), 2.45 - 2.20 (m, 1H), 2.01 - 1.34 (m, 9H)..LCMS [M+ H]⁺ =935.4

### Synthesis method of Compound UB-180954

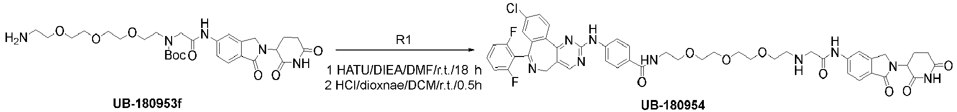

### Step 1: UB-180954(V2127-074)

### The method is similar to General Method 1

¹H NMR (400 MHz, *d6-DMSO*) δ 10.98 (s, 1H), 10.17 (s, 1H), 9.10 - 9.05 (m, 1H), 8.73 (s, 1H), 8.38 (s, 9H), 8.29 (d, *J=* 8.6 Hz, 1H), 8.00 - 7.75 (m, 6H), 7.75 - 7.46 (m, 2H), 7.34 (d, *J* = 1.9 Hz, 1H), 7.17 (t, *J* = 8.5 Hz, 2H), 5.09 (dd, *J* = 13.2, 5.0 Hz, 1H), 4.45 (d, *J* = 17.7 Hz, 1H), 4.31 (d, *J=* 17.5 Hz, 1H), 4.04 (s, 2H), 3.81 - 3.53 (m, 12H), 3.52 (s, 2H), 3.41 (d, *J* = 5.8 Hz, 2H), 3.24 - 2.69 (m, 1H), 2.85 - 2.77 (m, 2H), 2.11 - 1.96 (m, 2H). LCMS [M+ H]⁺ =950.4

### Synthesis method of Compound UB-180965

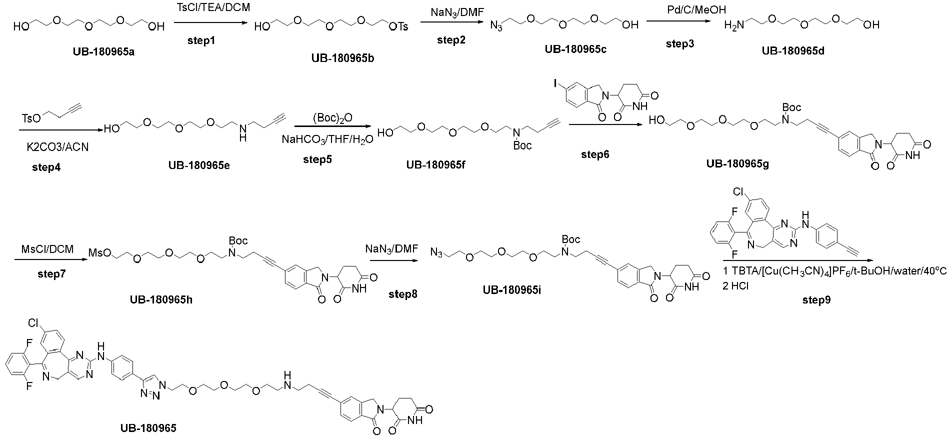

### Step 1: UB-180965b (V2127-087)

Compound **UBI-180965a** (8.0 g, 41 mmol), p-toluenesulfonyl chloride (7.81 g, 41 mmol) and triethylamine (10.2 g, 101 mmol) were successively added to dichloromethane (100 mL), the mixture was reacted at 25°C overnight. After the completion of the reaction, the reaction was poured into 10 mL of water and extracted with dichloromethane (10 mL*3). The organic phases were combined, then washed with saturated brine, dried over anhydrous Na₂SO₄, and concentrated by rotary evaporation under reduced pressure to obtain crude product. The crude product was purified by silica gel column chromatography (DCM/MeOH = 10/1) to obtain compound **UBI-180965b** (14 g, yield 99%) as a white solid. LCMS [M+ H]⁺ =349.4.

### Step 2: UB-180965c (V2127-088)

**UB-180965b** (14 g, 40 mmol) was dissolved in DMF(100 mL), sodium azide (6.43 g, 99 mmol) was added, the mixture was stirred at 85°C under N₂ overnight. After completion of the reaction, the reaction was filtered and concentrated in vacuum to obtain crude product. The crude product was purified by silica gel column chromatography (DCM/MeOH = 30/1) to obtain **UB-180965c** (8.1 g, yield 92%) as a colorless oil. LCMS[M+H]+=220.2

### Step 3: UB-180965d(V2127-090)

Compound **UB-180965c** (8.1 g, 37 mmol), and 10% palladium on carbon (200 mg) were added to a mixed solvent of methanol/dichloromethane (1 mL/10 mL), and reacted at room temperature for 2 hours under hydrogen atmosphere. After filtration, the filtrate was concentrated to obtain the crude product. The crude product was washed with cold ether (10 mL*3), and dried to obtain compound **UB-180965d** (7.14 g, yield 100%). LCMS [M+ H]⁺ =194.3

### Step 4: UB-180965e(V2127-091)

Compound **UB-180965d** (2 g, 10.4 mmol), and 3-butynyl p-toluenesulfonate (1.27 g, 7.6 mmol) were successively added to toluene (100 mL), the mixture was reacted at 80°C for 18 hours. After completion of reaction, the reaction was concentrated by rotary evaporation under reduced pressure to obtain crude product. The crude product was purified by silica gel column chromatography (DCM/MeOH = 10/1) to obtain compound **UB-180965e** (1.4g, yield 30%) as a colorless oil. LCMS [M+ H]⁺ =246.4.

### Step 5: UBI-180965f (V2127-092)

**UB-180965e** (1.4 g, 5.7 mmol), and tert-butyl dicarbonate (2.3g, 10.7mmol) were added to dioxane (30 mL), the mixture was reacted at room temperature for 2 h, then concentrated and extracted with ethyl acetate(10 mL*3). The crude product was purified by silica gel column chromatography (DCM/MeOH=10/1) to obtain desired compound **UB-180965f** (1.4g, yield 71%). LCMS[M+ H]⁺ =346.4

### Step 6: UB-180965g (V2127-093)

Compound **UB-180965f** (500 mg, 1.45mmol) and 3-(5-iodo-1-oxo-2,3-dihydro-1H-isoindol-2-yl)piperidine-2,6-dione (535 mg, 1.45 mmol) were dissolved in DMF (10 mL), dichlorobis(triphenylphosphonium)palladium (30 mg,0.045mmol), cuprous iodide (60 mg, 0.45mmol ) and triethylamine (13 ul, 0.09mmol) were added, the mixture was reacted at 80°C overnight under nitrogen. The reaction solution was filtered through Celite, and the filtrate was concentrated to obtain crude product, which was purified by flash chromatography (eluted with DCM/MeOH=0% to 20% for 30 minutes) to obtain product **UB-180965g** (610 mg, yield 72%). LC-MS: M+H) ⁺= 588.

### Step 7: UB-180965h (V2127-094)

**UB-180965g** (610 mg, 1.03 mmol) was dissolved in DCM(30 mL), methanesulfonyl chloride (153 mg, 1.34 mmol) and triethylamine (166 mg, 1.65 mmol) were added, the mixture was stirred at 25°C overnight. After completion of reaction, water was added and the mixture extracted with DCM (10 mL*3). The organic layer was dried over Na2SO4 and concentrated to obtain crude product, which was purified by flash chromatography (DCM/MeOH=30/1) to obtain product **UB-180965h** (691 mg, yield 99%). LC-MS:(M+H)⁺=666.7

### Step 8: UB-180965i (V2127-095)

**UBI-180965h** (691 mg, 1.03 mmol) was dissolved in DMF(15 mL), sodium azide (87 mg, 1.34 mmol) was added, the mixture was stirred 85°C overnight. After completion of reaction, the reaction was filtered and concentrated in vacuum to obtain crude product. The crude product was purified by silica gel column chromatography (DCM/MeOH = 30/1) to obtain **UBI-180965i** (585 mg, yield: 92%.) LC-MS: M+H) ⁺= 613.6

### Step 9: UB-180965 (V2127-096)

### The method is similar to General Method 3

¹H NMR (400 MHz, DMSO-*d*₆) δ 11.00 (s, 1H), 10.02 (s, 1H), 9.06 (s, 2H), 8.69 (s, 1H), 8.44 (d, *J=* 5.1 Hz, 1H), 8.30 (d, *J=* 8.5 Hz, 1H), 7.97 - 7.84 (m, 3H), 7.80 (d, *J=* 8.7 Hz, 1H), 7.74 - 7.62 (m, 1H), 7.61 - 7.49 (m, 1H), 7.35 (t, *J=* 6.3 Hz, 1H), 7.17 (t, *J=* 8.6 Hz, 1H), 5.09 - 4.78 (m, 1H), 4.82 - 4.50 (m, 2H), 4.37 (dt, *J=* 17.7, 14.1 Hz, 3H), 3.91 - 3.74 (m, 2H), 3.71 - 3.46 (m, 12H), 3.37 - 2.97 (m, 4H), 2.97 - 2.75 (m, 3H), 2.60 (dd, *J* = 5.8, 16.8 Hz, 1H), 2.43 - 2.06 (m, 2H), 1.98 (dd, *J=* 4.9, 4.8 Hz, 1H).LCMS [M+H]⁺ =969.4

### Synthesis method of Compound UB-180973

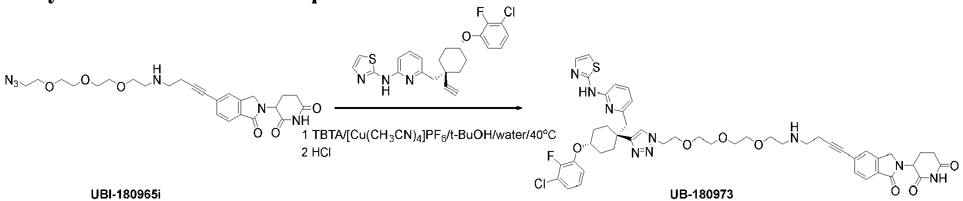

### The method is similar to General Method 3

¹H NMR (400 MHz, DMSO-*d*₆) δ 11.00 (s, 1H) 8.95 (s, 2H), 7.70 (dd, *J* = 14.2, 9.5 Hz, 3H), 7.41 (dt, *J* = 3.7, 13.9 Hz, 2H), 7.18 - 7.07 (m, 5H), 6.17 (t, *J=* 8.6 Hz, 1H), 5.09 - 4.98 (m, 1H), 4.52 - 4.26 (m, 6H), 3.72 (s, 6H), 3.42 - 2.87 (m, 12H), 2.65 - 2.57 (m, 2H), 2.49 - 2.46 (m, 2H), 2.09 - 1.87 (m, 6H), 1.86-1.75 (m, 2H), 1.56-1.44 (m, 2H). LCMS [M+H]⁺ =954.4

### Synthesis method of Compound UB-180974

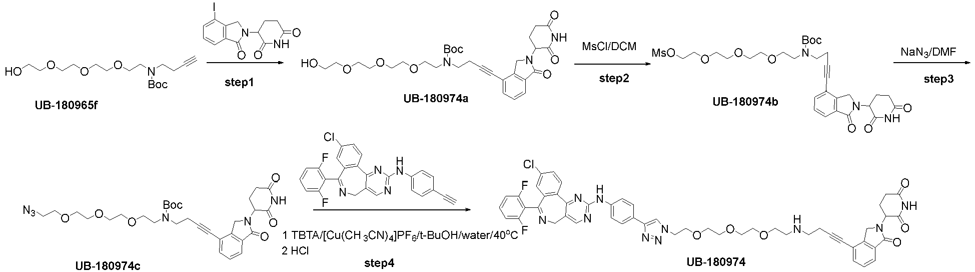

### Step 1: UB-180974a (V2127-120)

Compound **UB-180965f** (500 mg, 1.45mmol) and 3-(4-iodo-1-oxo-2,3-dihydro-1H-isoindol-2-yl)piperidine-2,6-dione (535 mg, 1.45 mmol) were dissolved in DMF (10 mL), dichlorobis(triphenylphosphonium)palladium (30 mg, 0.045mmol), cuprous iodide (60 mg, 0.45mmol ) and triethylamine (13 ul, 0.09mmol) were added, the mixture was reacted at 80°C overnight under nitrogen. The reaction solution was filtered through Celite, and the filtrate was concentrated to obtain crude product, which was purified by flash chromatography (eluted with DCM/MeOH=0% to 20% for 30 minutes) to obtain product **UB-180965g** (610 mg, yield 72%). LC-MS: M+H) ⁺= 588.

### Step 2: UB-180974b(V2127-121)

**Compound UB-180974a** (610 mg, 1.03 mmol) was dissolved in DCM(30 mL), methanesulfonyl chloride (153 mg, 1.34 mmol) and triethylamine (166 mg, 1.65 mmol) were added, the mixture was stirred at 25°C overnight. After completion of reaction, water was added and the mixture extracted with DCM (10 mL*3). The organic layer was dried over Na2SO4 and concentrated to obtain crude product, which was purified by flash chromatography (DCM/MeOH=30/1) to obtain product **UB-180974b** (691 mg, yield 99%). LC-MS:M+H)⁺=666.7

### Step 3: UB-180974c (V2127-123)

**UB-180974b** (691 mg, 1.03 mmol) was dissolved in DMF(15 mL), sodium azide (87 mg, 1.34 mmol) was added, the mixture was stirred 85°C overnight. After completion of reaction, the reaction was filtered and concentrated in vacuum to obtain crude product. The crude product was purified by silica gel column chromatography (DCM/MeOH = 30/1) to obtain **UB-180974c** (585 mg, yield 92%.) LC-MS: M+H) ⁺= 613.6

### Step 4: UBI-180974 (V2127-129)

### The method is similar to General Method 3

¹H NMR (400 MHz, DMSO-*d*₆) δ 11.02 (s, 1H), 10.02 (s, 1H), 9.19 (s, 2H), 8.69 (s, 1H), 8.45 (s, 1H), 8.30 (d, *J* = 8.5 Hz, 1H), 7.89 (dd, *J* = 14.6, 5.5 Hz, 3H), 7.83 - 7.49 (m, 6H), 7.36 (dd, *J* = 14.2, 3.9 Hz, 2H), 7.17 (t, *J* = 8.5 Hz, 2H), 5.16 (dd, *J* = 13.3, 5.0 Hz, 1H), 4.56 - 4.31 (m, 5H), 3.69 (t, *J=* 5.0 Hz, 3H), 3.64 - 3.37 (m, 12H), 3.18 (dd, *J* = 5.5, 3.6 Hz, 2H), 2.89 (dt, *J* = 8.2, 4.2 Hz, 3H), 2.66 - 2.54 (m, 1H), 2.49 - 2.47 (m, 1H), 2.01 (d, *J* = 5.4 Hz, 1H).LCMS [M+H]⁺ =969.4

### Synthesis method of Compound UBI-180976

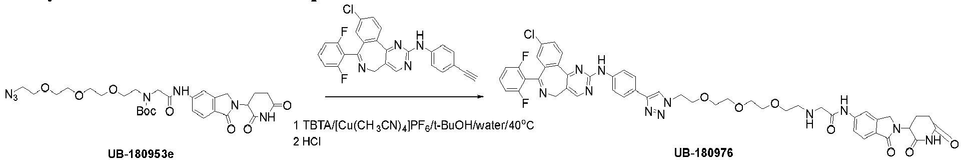

### Step 1: UB-180976 (V2127-107)

### The method is similar to General Method 3

¹H NMR (400 MHz, DMSO-*d*₆) δ 11.43 (s, 1H), 10.99 (s, 1H), 10.07 (s, 1H), 9.26 (s, 2H), 8.71 (s, 1H), 8.49 (s, 1H), 8.32 (d, *J=* 8.5 Hz, 1H), 7.96 (d, *J=* 9.0 Hz, 1H), 7.93 - 7.81 (m, 3H), 7.81 - 7.63 (m, 4H), 7.63 - 7.18 (m, 1H), 7.20 (d, *J=* 8.4 Hz, 2H), 7.19 (t, *J=* 8.3 Hz, 2H), 5.12 - 5.06 (m, 1H), 4.41 (t, *J=* 5.7 Hz, 2H), 4.30 (d, *J=* 8.4 Hz, 2H), 4.92 - 3.83 (m, 2H), 4.18 - 3.75 (m, 2H), 3.81 (d, *J* = 5.8 Hz, 2H), 3.80 (d, *J* = 4.3 Hz, 2H), 3.73 (s, 2H), 3.73 - 3.46 (m, 2H), 3.23 - 2.99 (m, 2H), 2.93 - 2.71 (m, 1H), 2.73 - 2.69 (m, 1H), 2.53 - 2.43 (m, 1H), 1.99 (d, *J=* 5.2 Hz, 1H). LCMS [M+H]⁺ =974.6

### Synthesis method of Compound UBI-180982

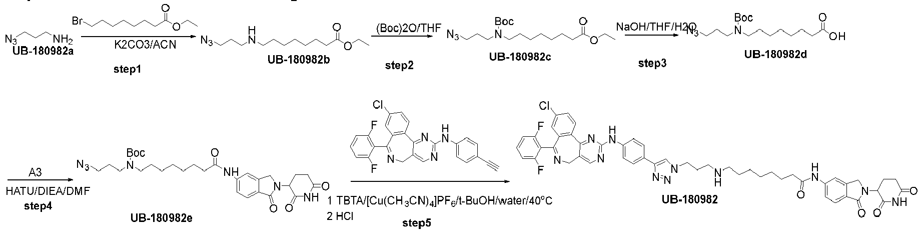

### Step 1: UBI-180982b (V2127-119)

**UB-180982a** (990 mg, 9.90 mmol) and ethyl 8-bromooctanoate (2484 mg, 9.90 mmol) were added to acetonitrile (20 mL), then potassium carbonate (2.06 g, 14.9 mmol) was added. The mixture was stirred at 80°C overnight, the filtrate was concentrated to obtain crude product, which was purified via flash chromatography (DCM/MeOH=0% to 10%) to obtain **UB-180982b** (962 mg, yield 36%) as a colorless oil. LCMS [M+H]⁺ =271.3

### Step 2: UBI-180982c(V2127-125)

**UB-180982b** (962 mg, 3.56 mmol), tert-butyl dicarbonate (938 mg, 4.30 mmol) and sodium bicarbonate (360 mg, 4.29mmol) were added to tetrahydrofuran (20 mL), the mixture was reacted at room temperature for 2 h. Water (10mL) was added, the mixture was concentrated and extracted with ethyl acetate(10 mL*3). The organic phase was dried and concentrated to obtain **product UBI-180982c** (988 mg, yield 75%). LCMS [M+H]⁺ =371.3

### Step 3: UBI-180982d (V2127-126)

Compound **UBI-180982c** (988 mg, 2.67 mmol), and sodium hydroxide (140 mg, 3.48 mmol) were added to water (5 mL) successively, reacted at 30°C for 16 hours. After the completion of the reaction, it was concentrated and the aqueous phase was acidified to pH=5 using hydrochloric acid (1M). Then it was extracted with dichloromethane (10ml*3), the combined organic layer was dried over anhydrous Na₂SO₄, and concentrated to obtain the target compound **UBI-180982d** (803 mg, yield 88%). LCMS: [M+H]⁺ =343.4

### Step 4: UB-180982e(V2127-127)

Compound **UB-180982d** (812 mg, 2.37 mmol), 3-(5-amino-1-oxo-2,3-dihydro-1H-isoindol-2-yl)piperidine-2,6-dione(780 mg, 3.02mmol), HATU (1428 mg, 3.76 mmol) and DIPEA (0.3mL) were added to anhydrous DMF (10 mL). The mixture was reacted at room temperature for 16 hours. After the completion of the reaction, concentrated to obtain crude product which was isolated by reverse chromatography column chromatography (MeOH/H₂O = 5% to 95%, 45mins) to obtain **UBI-180982e** (913 mg, yield 66%) as a colorless oil. [M+H]⁺ =584.6

### Step 5: UB-180982 (V2127-128)

### The method is similar to General Method 3

¹H NMR (400 MHz, DMSO-*d*₆) δ 10.97 (s, 1H), 10.37 (s, 1H), 10.03 (s, 1H), 8.96 (s, 2H), 8.70 (s, 1H), 8.54 (s, 1H), 8.31 (d, *J* = 8.5 Hz, 1H), 8.00 (s, 1H), 7.96 - 7.83 (m, 3H), 7.80 (d, *J* = 8.7 Hz, 2H), 7.67 - 7.55 (m, 2H), 7.55 - 7.34 (m, 1H), 7.20 (dd, *J=* 5.3, 6.7 Hz, 2H), 5.08 (dd, *J=* 13.3, 5.0 Hz, 1H), 4.54(t, *J=* 6.6 Hz, 2H), 2.87 (dt, *J=* 8.5, 4.3 Hz, 5H), 2.59 (d, *J=* 6.6 Hz, 1H), 2.55 - 2.46 (m, 2H), 2.44 - 2.02 (m, 2H), 2.05 - 1.84 (m, 1H), 1.61 (s, 4H), 1.36 - 1.23 (m, 6H)..LCMS [M+H]⁺ =940.7

### Synthesis method of Compound UB-180983

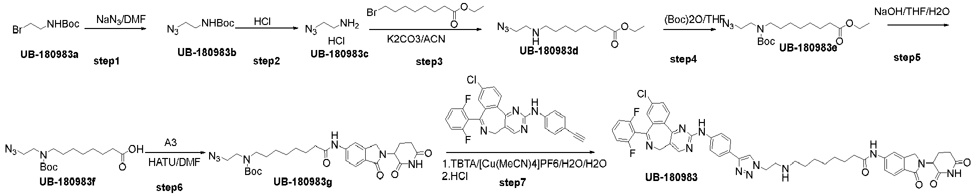

### Step 1: UB-180983b (V2127-120)

**UB-180983a** (14 g, 63 mmol) was dissolved in DMF(100 mL), sodium azide (6.43 g, 99 mmol) was added, the mixture was stirred 85°C overnight. After the completion of the reaction, the filtrate was obtained by filtration and concentrated in vacuum to obtain crude product. The crude product was separated and purified by chromatographic column chromatography (DCM/MeOH = 30/1) to obtain **UB-180983b** (11.1 g, yield 95%) as colorless oil. LCMS[M+H]+=187.3

¹H NMR (400 MHz, CDCl₃) δ4.86 - 4.78 (m, 1H), 3.64 - 3.19 (m, 4H), 1.54 - 1.26 (m, 9H)

### Step 2: UB-180983c (V2127-122)

Compound **UB-180983b** (11.1 g, 59.6 mmol) and hydrochloric acid in dioxane (10 mL, 4 N) were added to tetrahydrofuran (10mL), the mixture was reacted at room temperature for 2 hours. After the completion of the reaction, the mixture was concentrated by rotary evaporation under reduced pressure to obtain compound **UB-180983c** (7.33 g, yield 100%). LC-MS: [M+H]⁺ =87.1

¹H NMR (400 MHz, DMSO) δ, 8.28(s, 3H), 3.63 (dd, *J=* 3.5, 7.6 Hz, 2H), 2.89 (s, 2H).

### Step 3: UB-180983d (V2127-123)

Compound **UB-180983c** (7.33 g, 60.1 mmol), ethyl 8-bromooctanoate (15.01 g, 60.1 mmol) and potassium carbonate (20.6 g, 149 mmol) were added to anhydrous acetonitrile (100 mL), the mixture was reacted at 80°C for 18 hours. After the completion of the reaction, the reaction solution was concentrated. The crude product was isolated by silica gel column chromatography (DCM/MeOH = 10/1) to obtain compound **UB-180983d** (5.07 g, yield 33%) as a colorless oil. LCMS [M+H]⁺ =257.3

### Step 4: UBI-180983e(V2127-124)

**UB-180983d** (5.07g, 19.83 mmol), tert-butyl dicarbonate (8.64 g, 39.65 mmol) and sodium bicarbonate (3.6 g, 42.9mmol) were added to tetrahydrofuran (20 mL), the mixture was reacted at room temperature for 2 h. Water (10mL) was added, the mixture was concentrated and extracted with ethyl acetate(10 mL*3). The organic phase was dried and concentrated to obtain product **UBI-180983e** (5.28 g, yield 75%). LCMS [M+H]+ =371.3

### Step 5: UB-180983f (V2127-125)

Compound **UB-180983e** (5.28 g, 14.8 mmol), and sodium hydroxide (1.4 g, 34.8 mmol) were added to water (5 mL) successively, reacted at 30°C for 16 hours. After the completion of the reaction, it was concentrated and the aqueous phase was acidified to pH=5 using hydrochloric acid (1M). Then it was extracted with dichloromethane (10ml*3), the combined organic layer was dried over anhydrous Na₂SO₄, and concentrated to obtain the desired compound **UB-180983f** (4.28g, yield 88%). [M+H]⁺ =329.4

### Step 6: UBI-180983g(V2127-129)

Compound **UB-180983f** (4.28 g, 13.04mmol), 3-(5-amino-1-oxo-2,3-dihydro-1H-isoindol-2-yl)piperidine-2,6-dione(3.38 g, 13.04 mmol), HATU (5.95 g, 15.64 mmol) and DIPEA (3mL) were added to anhydrous DMF (10 mL). The mixture was reacted at room temperature for 16 hours. After the completion of the reaction, concentrated to obtain crude product which was isolated by reverse chromatography column chromatography (MeOH/H₂O = 5% to 95%, 45mins) to obtain **UB-180983g** (4.90 g, yield 66%) as a colorless oil. [M+H]⁺ =570.6

### Step 7: UBI-180983(V2127-130)

### The method is similar to General Method 3

¹H NMR (400 MHz, DMSO-*d*₆) δ10.97 (s, 1H), 10.34 (s, 1H), 10.04 (s, 1H), 9.07 (s, 2H), 8.70 (s, 1H), 8.57 (s, 1H), 8.31 (d, *J=* 8.5 Hz, 1H), 7.99 (s, 1H), 7.97 - 7.86 (m, 3H), 7.81 (d, *J* = 8.7Hz, 2H), 7.75 - 7.45 (m, 3H), 7.34 (d, *J* = 2.1 Hz, 1H), 7.17 (t, *J* = 8.4 Hz, 2H), 5.08 (dd, *J=* 13.2, 5.0 Hz, 1H), 4.79 (t, *J* = 6.0 Hz, 2H), 4.41 (d, *J* = 7.3 Hz, 1H), 4.27 (d, *J* = 7.4 Hz, 1H), 3.68-3.55 (m, 2H), 2.93 - 2.84 (m, 3H), 2.59-2.50 (m, 1H), 2.38 (dd, *J=* 5.3, 8.0 Hz, 3H), 2.06 -1.84 (m, 1H), 1.60 (d, *J=* 5.8 Hz, 4H), 1.28 (d, *J=* 3.6 Hz, 6H). LCMS [M+H]⁺ =926.8

### Synthesis method of Compound UBI-180986

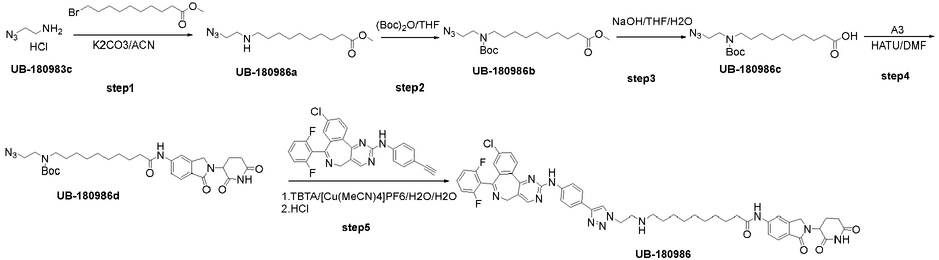

### Step 1: UB-180986a (V2127-133)

Compound **UB-180983c** (7.5 g, 61.5 mmol), methyl 10-bromodecanoate (16.2 g, 61.5 mmol) and potassium carbonate (20.6 g, 149 mmol) were added to anhydrous acetonitrile (100 mL), the mixture was reacted at 80°C for 18 hours. After the completion of the reaction, the reaction solution was concentrated. The crude was isolated by silica gel column chromatography (DCM/MeOH = 10/1) to obtain compound **UB-180986a** (5.81 g, yield 35%). LCMS [M+H]⁺=271.3

### Step 2: UB-180986b(V2127-134)

**UB-180986a** (5.81g, 21.52 mmol), tert-butyl dicarbonate (8.64 g, 39.65 mmol) and sodium bicarbonate (3.6 g, 42.9mmol) were added to tetrahydrofuran (20 mL), the mixture was reacted at room temperature for 2 h. Water (10mL) was added, the mixture was concentrated and extracted with ethyl acetate(10 mL*3). The organic phase was dried and concentrated. The crude was isolated by silica gel column chromatography (DCM/MeOH = 10/1) to obtain compound **UBI-180986b** (5.89 g, yield 74%).

.LCMS [M+H]⁺ =371.3

### Step 3: UB-180986c (V2127-136)

**Compound UB-180986b** (5.89 g, 15.9 mmol) and sodium hydroxide (1.4 g, 34.8 mmol) were added to a system of water/methanol/tetrahydrofuran (0.5mL/3mL/3mL), and reacted at room temperature for 16 hours. After the completion of the reaction, the organic solvent was removed by concentration, and the aqueous phase was acidified to pH=5 using hydrochloric acid (1M). Then it was extracted with dichloromethane (10ml*3), the combined organic phase was dried over anhydrous Na₂SO₄, and concentrated to obtain the desired compound **UB-180986c**(4.99g, yield 88%). LCMS [M+H]⁺ = 357.4

### Step 4: UB-180986d (V2127-137)

Compound **UB-180986c** (4.99 g, 14.0mmol), 3-(5-amino-1-oxo-2,3-dihydro-1H-isoindol-2-yl)piperidine-2,6-dione(3.63 g, 14.0 mmol), HATU (5.95 g, 15.64 mmol) and DIPEA (3mL) were added to anhydrous DMF (10 mL). The mixture was reacted at room temperature for 16 hours. After the completion of the reaction, the organic solvent was removed. The crude product was isolated by reverse chromatography column chromatography (method: MeOH/H₂O = 5% to 95%, 45mins, collected at 60%) to obtain **UB-180986d** (5.77 g, yield 69%) as a colorless oil. LCMS [M+H]⁺ =598.7

### Step 5: UBI-180986(V2127-140)

### The method is similar to General Method 3

¹H NMR (400 MHz, DMSO-*d*₆) δ10.95 (s, 1H), 10.33 (s, 1H), 10.03 (s, 1H), 9.07 (s, 2H), 8.70 (s, 1H), 8.57 (s, 1H), 8.31 (d, *J=* 8.5 Hz, 1H), 7.99 (s, 1H), 7.97 - 7.86 (m, 3H), 7.81 (d, *J* = 8.7 Hz, 2H), 7.75 - 7.45 (m, 3H), 7.34 (d, *J* = 2.1 Hz, 1H), 7.17 (t, *J* = 8.4 Hz, 2H), 5.08 (dd, *J* = 13.2, 5.0 Hz, 1H), 4.80 (t, *J* = 6.0 Hz, 2H), 4.41 (d, *J* = 7.3 Hz, 1H), 4.27 (d, *J* = 7.4 Hz, 1H), 2.98 - 2.85 (m, 5H), 2.68 - 2.55 (m, 1H), 2.26 (dd, *J=* 8.1, 7.9 Hz, 2H), 2.06 - 1.84 (m, 1H), 1.64 (d, *J=* 4.1 Hz, 4H), 1.24 (s,10H)..LCMS [M+H]⁺ =954.8

### Synthesis method of Compound UB-180987

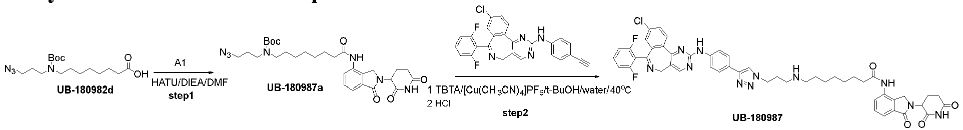

### Step 1: UB-180987a(V2127-141)

**Compound UB-180982d** (812 mg, 2.37mmol), lenalidomide (780 mg, 3.02mmol), HATU (1428 mg, 3.76 mmol) and DIPEA (0.3mL) were added to anhydrous DMF (10 mL). The mixture was reacted at room temperature for 16 hours. After the completion of the reaction, the organic solvent was removed by concentration. The crude product was isolated by reverse chromatography on silica gel column chromatography (method: MeOH/H₂O = 5% to 95%, 45mins, collected at 70%) to obtain compound **UB-180987a** (915 mg, 67% yield) as a colorless oil.

### Step 2: UB-180987 (V2127-144)

### The method is similar to General Method 3

¹H NMR (400 MHz, DMSO-*d*₆) δ11.02 (s, 1H), 10.03 (s, 1H), 9.79 (s, 1H), 8.50 (s, 1H), 7.96 - 7.72 (m, 6H), 7.62 - 7.43 (m, 3H), 7.17 (s, 2H), 5.15 (dd, *J* = 13.4, 4.9 Hz, 1H), 4.52 (s, 1H), 4.36 (q, *J* = 7.5 Hz, 2H), 2.83 (t, *J* = 9.1 Hz, 5H), 2.68 - 2.56 (m, 1H), 2.41 - 2.30 (m, 5H), 2.13 (d, *J=* 6.0 Hz, 1H), 1.64 (d, *J=* 4.0 Hz, 4H), 1.24 (s,6H).LCMS [M+H]⁺ =940.7

### Synthesis method of Compound UB-180990

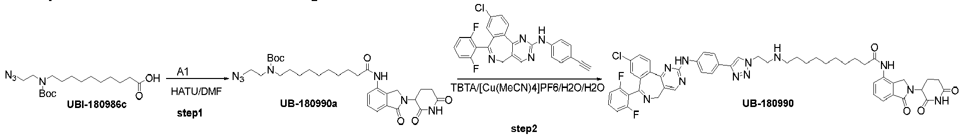

### Step 1: UB-180990a (V2127-142)

Compound **UB-180986c** (4.99 g, 14.0mmol), lenalidomide (3.63 g, 14.0 mmol), HATU (5.95 g, 15.64 mmol) and DIPEA (0.3mL) were added to anhydrous DMF (10 mL). The mixture was reacted at room temperature for 16 hours. After the completion of the reaction, the reaction was concentrated. The crude product was isolated by reverse silica gel column chromatography (method: MeOH/H₂O = 5% to 95%, 45mins, collected at 70%) to obtain compound **UB-180990a** (5.77 g, 69% yield).

### Step 2: UB-180990(V2127-146)

### The method is similar to General Method 3

¹H NMR (400 MHz, DMSO-*d*₆) δ11.02 (s, 1H), 10.04 (s, 1H), 9.82 (s, 1H), 8.83 (d, *J* = 10.5 Hz, 2H), 8.70 (s, 1H), 8.55 (s, 1H), 8.31 (d, *J=* 8.5 Hz, 1H), 7.99 - 7.84 (m, 3H), 7.80 (d, *J* = 8.7 Hz, 3H), 7.67 - 7.40 (m, 3H), 7.34 (d, *J* = 2.1 Hz, 1H), 7.17 (t, *J* = 8.4 Hz, 2H), 5.15 (dd, *J* = 13.3, 5.1 Hz, 1H), 4.77 (t, *J* = 6.0 Hz, 2H), 4.36 (q, *J* = 7.4 Hz, 2H), 3.44 - 3.38 (m, 2H), 3.08 - 2.73 (m, 3H), 2.63 (t, *J* = 6.8 Hz, 1H), 2.50 - 2.10 (m, 3H), 2.10 - 1.90 (m, 1H), 1.64 (d, *J=* 4.0 Hz, 4H), 1.28 (s, 10H). LCMS [M+H]⁺ = 954.7

### Synthesis method of Compound UB-180991

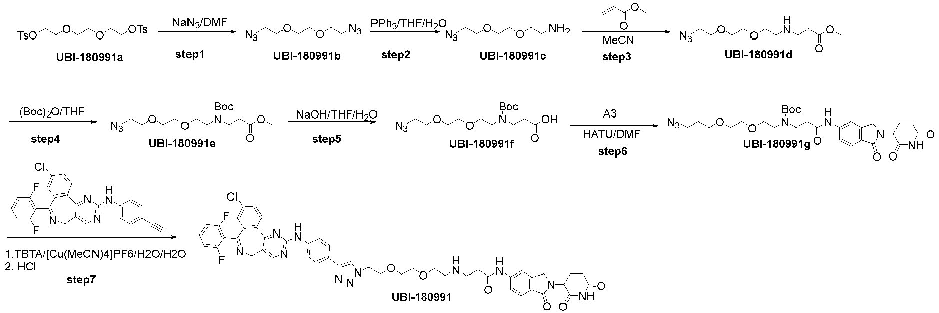

### Step 1: UB-180991b (V2127-131)

**UB-180991a** (10 g, 22 mmol) and sodium azide (3.21 g, 49 mmol) were dissolved in DMF(100 ml), the mixture was stirred at room temperature overnight, after completion of the reaction, the reaction was diluted with H₂O (300 ml) and extracted with ether (2x150 ml). The organic phase was washed with H₂O (3×100 ml) and brine (1×100 ml), dried over MgSO₄, filtered, and the solvent was removed under reduced pressure to obtain **UBI-180991b** (4.15 g, yield 95%) as a colorless oil.

### Step 2: UB-180991c (V2127-135)

**UB-180991b** (4.15 g,20.7 mmol), and PPh3(5.44 g, 20.7 mmol) were added to a mixed solvent of water (10 mL), con-HCl (1 mL) and THF(10 mL), the mixture was stirred at room temperature for 16 h, then concentrated and extracted with ether (10 mL*3). The aqueous phase was adjusted to pH=13 with NaOH (1M), extracted with DCM (10mL*3), and the combined organic layers were dried over anhydrous Na2SO4 and concentrated to give compound **UB-180991c** (3.18g, 88% yield)

### Step 3: UB-180991d (V2127-139)

**Compound UB-180991c** (1.0g, 5.75 mmol) and methyl acrylate (1.6g, 7.69 mmol) were dissolved in toluene (15 mL), the mixture was reacted at 80°C for 18 hours. After the completion of the reaction, the reaction solution was poured into 5 mL of water, extracted with ethyl acetate (5 mL*3). The organic phases were combined, then washed with saturated brine, dried over anhydrous Na2SO4, and concentrated by rotary evaporation under reduced pressure to obtain crude product, which was isolated by silica gel column chromatography (DCM/MeOH = 10/1) to obtain compound **UB-180991d** (1.27 g, yield 75 %) as a colorless oil.

### Step 4: UB-180991e (V2127-143)

**Compound UB-180991d** (350 mg, 1.35 mmol), di-tert-butyl dicarbonate (441 mg, 2.03 mmol) and sodium bicarbonate (771 mg, 9.18 mmol) were added to dioxane (13 mL) and the mixture was reacted at room temperature for 2 hours. After the completion of the reaction, it was poured into 10 mL of water and extracted with dichloromethane (5 mL*3). The organic phases were combined, then washed with saturated brine, dried over anhydrous Na₂SO₄, and concentrated by rotary evaporation under reduced pressure to obtain **UB-180991e** (422 mg, yield 64%) as a colorless oil.

### Step 5: UB-180991f (V2127-145)

**Compound UB-180991e** (390 mg, 1.09 mmol) and sodium hydroxide (1.19g, 21.2mmol) were added to a system of water/methanol/tetrahydrofuran (0.5mL/3mL/3mL), and reacted at room temperature for 12 hours. After the completion of the reaction, the organic solvent was removed by concentration, and the aqueous phase was acidified to pH=5 using hydrochloric acid (1M). Then it was extracted with dichloromethane (10ml*3), the combined organic phase was dried over anhydrous Na₂SO₄, and concentrated to obtain **UB-180991f** (326 mg, yield 87%) as a colorless oil.

### Step 6: UB-180991g (V2127-147)

Compound **UB-180991f** (210 mg, 0.607 mmol), A3 (160 mg, 0.618 mmol), HATU (305 mg, 0.803 mmol) and DIPEA (0.5 mL) were added to anhydrous DMF (5 mL). The mixture was reacted at room temperature for 16 hours. After the completion of the reaction, the reaction was concentrated to obtain crude product. The crude product was isolated by reverse silica gel column chromatography (method: MeOH/H₂O = 5% to 95%, 45mins, collected at 60%) to obtain compound **UB-180991g** (153 mg, 42% yield) as a colorless oil.

### Step 7: UB-180991 (V2127-149)

### The method is similar to General Method 3

¹H NMR (400 MHz, DMSO-*d*₆) δ10.98 (s, 1H), 10.77 (s, 1H), 10.02 (s, 1H), 9.01 (s, 2H), 8.69 (d, *J=* 6.4 Hz, 1H), 8.49 (s, 1H), 8.31 (dd, *J=* 8.5, 4.0 Hz, 1H), 8.02 - 7.86 (m, 5H), 7.82 (s, 1H), 7.82 - 7.42 (m, 5H), 7.36 - 7.01 (m, 4H), 5.04 (dt, *J* = 9.2, 9.6 Hz, 1H), 4.74 - 4.56 (m, 2H), 3.86- 3.74 (m, 2H), 3.67 - 3.64 (m, 2H), 3.63- 3.54 (m, 4H), 3.32 - 3.12 (m, 2H), 3.12 - 2.87 (m, 2H), 2.87 - 2.78 (m, 3H), 2.69 - 2.55 (m, 1H), 2.36 (dt, *J* = 13.2, 9.0 Hz, 1H), 2.01 - 1.84 (m, 1H). LCMS [M+H]⁺ =944.7

### Synthesis method of Compound UB-180994

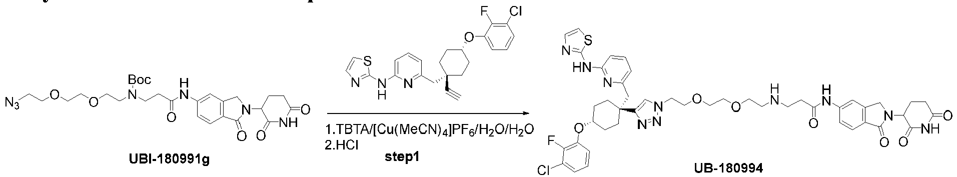

### Step 1: UB-180994 (V2395-002)

### The method is similar to General Method 3

¹H NMR (400 MHz, DMSO-*d*₆) δ10.98 (s, 1H), 10.68 (s, 1H), 10.18 (s, 1H), 8.86 (s, 2H), 8.02 -7.66(m, 4H),7.52 -7.42 (m, 2H), 7.26 - 6.91 (m, 5H), 6.24- 6.19 (m, 2H), 5.17-5.21(m,1H),4.51-4.22(m,5H), 3.75 - 3.61 (m, 6H), 3.25 - 2.78 (m, 8H), 2.63 (t, *J=* 6.3 Hz, 2H), 2.40 - 2.14 (m, 1H), 2.04 (dd, *J* = 14.3, 9.0 Hz, 6H), 1.66 - 1.54 (m, 2H). LCMS [M+H]⁺ =929.7

### Synthesis method of Compound UB-180995

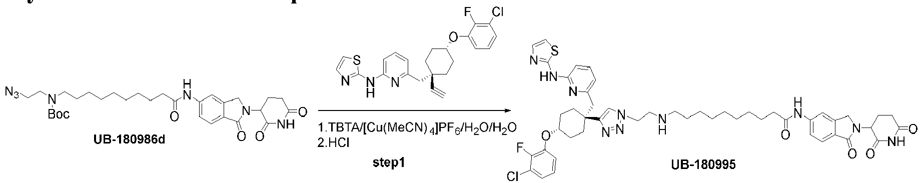

### Step 1: UB-180995(V2395-005)

### The method is similar to General Method 3

¹H NMR (400 MHz, DMSO-*d*₆) δ11.03 (s, 1H), 10.29 (s, 1H), 9.06 (s, 2H), 7.92 - 7.63 (m, 4H), 7.47 (q, *J=* 8.2 Hz, 2H), 7.54 - 7.07 (m, 3H), 7.07-6.21 (m, 3H) , 5.17-5.21(m,1H), 4.64 - 4.37 (m, 5H), 3.11 - 2.58 (m, 8H), 2.35 (dd, *J=* 13.3, 5.7 Hz, 4H), 2.14 - 1.82 (m, 6H), 1.61 - 1.52 (m, 6H), 1.26 (d, *J* = 8.4 Hz, 10H). LCMS [M+H]⁺ = 939.7

### Synthesis method of Compound UB-181000

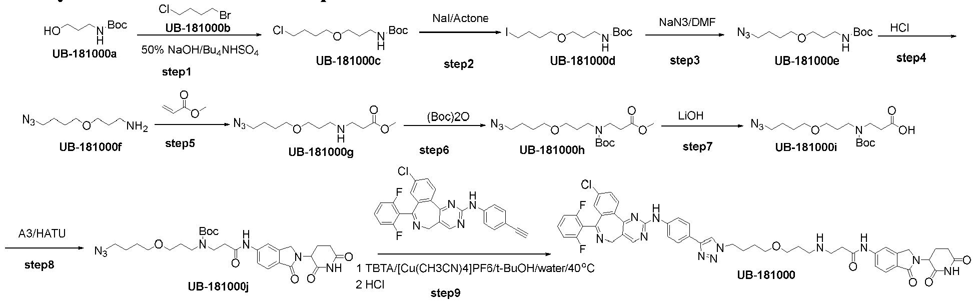

### Step 1: UB-181000c (V2395-003)

**UB-181000a** (5g, 26mmol) was added to **UB-181000b**(10mL), then Bu4NHSO4(17.7g, 52mmol) was added. To above solution was added 50% NaOH (50mL), the mixture was stirred at 50°C for 16h. Water was added, the mixture was extracted with DCM(30mL*3). The organic phase was dried and concentrated to give the crude product. The crude product was purified by silica gel column chromatography (PE/EA=3/1) to obtain **UB-181000c** (4.3g, yield 58%) as a colorless oil. LC-MS: [M+H] +=266.3

### Step 2: UB-181000d (V V2395-004)

A solution of **UB-181000c** (4.3g, 16.2 mmol) stored in acetone(200mL) was added with NaI(23.5g, 15.7mmol), the mixture was stirred at 80°C for 2 days. The reaction solution was concentrated and added water, extracted with DCM (20mL*3). The organic layer was dried and concentrated to obtain product **UB-181000d** (5.2g, yield 90%). LC-MS: [M+H]+=358.3

### Step 3: UB-181000e (V2395-006)

**UB-181000d** (5.2g, 14mmol) and sodium azide (1.82g, 28mmol) were mixed and dissolved in DMF(50 ml), the mixture was stirred at 80°C overnight, after completion of the reaction, the reaction was diluted with H2O (300 ml) and extracted with ether (2x150 ml). The organic phase was washed with H₂O (3×100 ml) and brine (1×100 ml), dried over MgSO₄, filtered, and the solvent was removed under reduced pressure to obtain product **UB-181000e** (4.2g, yield 100%). LC-MS: [M+H]⁺ =273.3

### Step 4: UB-181000f (V2395-007)

Compound **UB-181011e** (4.2g, 14.6mmol) and hydrochloric acid in dioxane (10 mL, 4 N) were added to tetrahydrofuran (10mL), the mixture was reacted at room temperature for 2 hours. After the completion of the reaction, the mixture was concentrated by rotary evaporation under reduced pressure to obtain compound **UB-181000f** (4g, yield 100%). LC-MS: [M+H]⁺ =173.31

### Step 5: UB-181000g (V2395-009)

**Compound UB-181000f** (2.5g, 13.4mmol), bromo ethyl acetate (2.23g, 13.4mmol) and potassium carbonate (3.7g, 26.8mmol) were added to anhydrous acetonitrile (200 mL). The mixture was reacted at 80 °C for 18 hours, concentrated after the completion of the reaction. The crude product was isolated by silica gel column chromatography (DCM/MeOH = 10/1) to obtain compound **UB-181000g** (820mg, yield 22.7% ) as a colorless oil. LC-MS: [M+H]⁺ =259.3

### Step 6: UB-181000h (V2395-010)

**Compound UB-181000g** (820mg, 3mmol), di-tert-butyl dicarbonate (241mg, 6mmol) and sodium bicarbonate (504mg, 6mmol) were added to tetrahydrofuran (50mL) successively and reacted at room temperature for 2 hours. After the completion of the reaction, the reaction solution was poured into 10 mL of water and extracted with ethyl acetate (10 mL*3). The organic phases were combined, then washed with saturated brine, dried over anhydrous Na₂SO₄, and concentrated by rotary evaporation under reduced pressure to obtain compound **UB-181000h** (800mg, yield 73.2%). LC-MS: [M+H]⁺ =359.3

### Step 7: UB-181000i (V2395-011)

Compound **UB-181000h** (800mg, 2.15mmol), and lithium hydroxide (344mg, 8.6mmol) were added to water (50 mL) successively, reacted at 50°C for 2 hours. After the completion of the reaction, it was concentrated and the aqueous phase was acidified to pH=5 using hydrochloric acid (1M). Then it was extracted with dichloromethane (10ml*3), the combined organic layer was dried over anhydrous Na₂SO₄, and concentrated to obtain the desired compound **UB-181000i** (600mg, yield 81%). LC-MS: [M+H]⁺ =359.3

### Step 8: UB-181000j (V2395-012)

**Compound UB-181000i** (300mg, 0.87mmol), **A3** (226mg, 0.87mmol), HATU (661mg, 1.74mmol) and DIPEA (0.5mL) were added to anhydrous DMF (5 mL). The mixture was reacted at room temperature for 2 hours, concentrated after the completion of the reaction. The crude product was isolated by silica gel column chromatography (DCM/MeOH=10/1) to obtain compound **UB-181000j** (270mg, 53% yield). LC-MS: [M+H]+ =587.3

### Step 9: UB-181000 (V2395-013)

### The method is similar to General Method 3

¹H NMR (400 MHz, DMSO-*d*₆) δ 10.98 (s, 1H), 10.74 (s, 1H), 10.01 (s, 1H), 8.94 (s, 2H), 8.69 (s, 1H), 8.51 (s, 1H), 8.31 (d, *J=* 8.5 Hz, 1H), 7.95 (d, *J=* 15.7 Hz, 1H), 7.89 (dd, *J=* 13.1, 5.4 Hz, 3H), 7.80 (d, *J=* 8.7 Hz, 2H), 7.67 (s, 2H), 7.65 - 7.42 (m, 1H), 7.34 (d, *J=* 2.0 Hz, 1H), 7.17 (t, *J* = 8.3 Hz, 2H), 5.08 (dd, *J* = 13.2, 5.0 Hz, 1H), 4.96 - 4.91 (m, 3H), 4.42 (dd, *J* = 12.0, 4.8 Hz, 1H), 3.51 - 3.12 (m, 6H), 3.06 - 2.85 (m, 5H), 2.59 (d, *J* = 7.3 Hz, 1H), 2.47 - 2.16 (m, 1H), 1.94 (ddd, *J* = 20.1, 12.7, 5.8 Hz, 5H), 1.54 (dd, *J* = 14.3, 6.5 Hz, 2H). LCMS [M+H]⁺ = 939.7

### Synthesis method of Compound UBI-181001

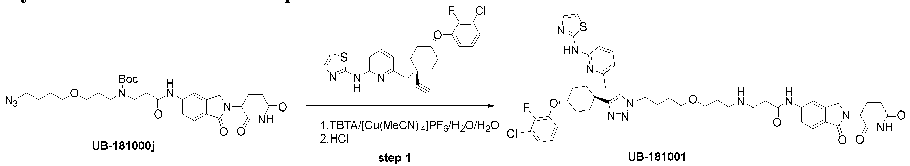

### Step 1: UBI-181001(V2395-014)

### The method is similar to General Method 3

¹H NMR (400 MHz, DMSO-*d*₆) δ 10.98 (s, 1H), 10.71 (d, *J* = 5.4 Hz, 1H), 8.92 (d, *J* = 3.6 Hz, 3H), 7.99 (d, *J* = 3.9 Hz, 51H), 7.75 - 7.45 (m, 5H), 7.17 (ddd, *J* = 17.3, 12.4, 11.9 Hz, 1H), 7.13 - 7.04 (m, 3H), 6.97 (d, *J=* 8.1 Hz, 1H), 6.27-6.25 (m, 1H) , 5.08 (dd, *J=* 13.2, 5.0 Hz, 1H), 4.67- 4.20 (m, 5H), 3.66 - 3.41 (m, 6H), 3.15 - 2.49 (m, 6H), 2.65 - 2.49 (m, 1H), 2.45 - 2.32 (m, 1H), 2.04 (dd, *J* = 26.5, 11.8 Hz, 4H), 1.92 - 1.82 (m,5H), 1.78 - 1.12 (m, 7H). LCMS [M+H]⁺ = 927.7

### Synthesis method of Compound UBI-181002

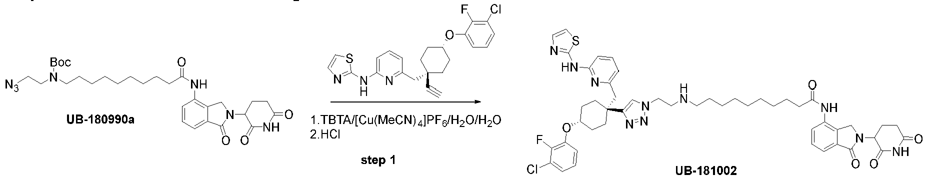

### Step 1: UBI-181002(V2395-015)

### The method is similar to General Method 3

¹H NMR (400 MHz, DMSO-*d*₆) δ 10.98 (s, 1H), 9.84(s, 1H), 9.19 (s, 2H), 7.81 (d, *J=* 6.9 Hz, 2H), 7.76 - 7.36 (m, 4H), 7.21 - 6.93 (m, 5H), 6.27-6.25 (m, 1H) ,5.15 (dd, *J=* 13.2, 5.1 Hz, 1H), 4.55 (d, *J* = 8.1 Hz, 3H), 4.37 (dd, *J* = 3.7, 7.5 Hz, 2H), 3.45 (d, *J* = 4.7 Hz, 2H), 3.02 (ddd, *J* = 8.4, 3.3, 3.6 Hz, 2H), 2.87 - 2.68 (m, 3H), 2.67 - 2.54 (m, 1H), 2.55 - 2.46(m, 3H), 2.39 - 1.87 (m,5H), 1.86 - 1.53 (m, 8H), 1.26 (d, *J=* 8.6 Hz, 10H),. LCMS [M+H]⁺ = 939.8

### Synthesis method of Compound UBI-181003

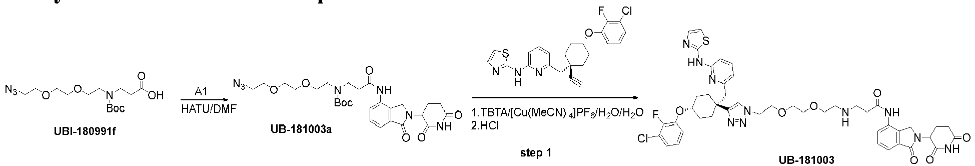

### Step 1: UBI-1810003a (V2395-008)

**Compound UBI-180991a** (210 mg, 0.607 mmol), lenalidomide (160 mg, 0.618 mmol), HATU (305 mg, 0.803 mmol) and DIPEA (0.5mL) were added to anhydrous DMF (5 mL). The mixture was reacted at room temperature for 16 hours, concentrated after the completion of the reaction. The crude product was isolated by silica gel column chromatography (DCM/MeOH=10/1) to obtain compound **UBI-1810003a** (153 mg, 42% yield).

### Step 2: UBI-1810003 (V2395-016)

### The method is similar to General Method 3

¹H NMR (400 MHz, DMSO-*d*₆) δ 11.03 (s, 1H), 10.16 (d, *J=* 5.4 Hz, 1H), 8.92 (d, *J* = 3.6 Hz, 2H), 7.89 (d, *J* = 3.9 Hz, 1H), 7.85 - 7.45 (m, 5H), 7.13 - 7.04 (m, 4H), 6.97 (d, *J* = 8.1 Hz, 1H), 6.27-6.25 (m, 1H), 5.08 (dd, *J* = 13.2, 5.0 Hz, 1H), 4.63 - 4.34 (m, 5H), 3.66 - 3.41 (m, 8H), 3.15 - 2.69 (m, 6H), 2.65 - 2.59 (m, 1H), 2.45 - 2.32 (m, 1H), 2.04 (dd, *J* = 6.5, 9.8 Hz, 4H), 1.78 - 1.52 (m, 2H). LCMS [M+H]⁺ = 929.8

### Synthesis method of Compound UBI-181016

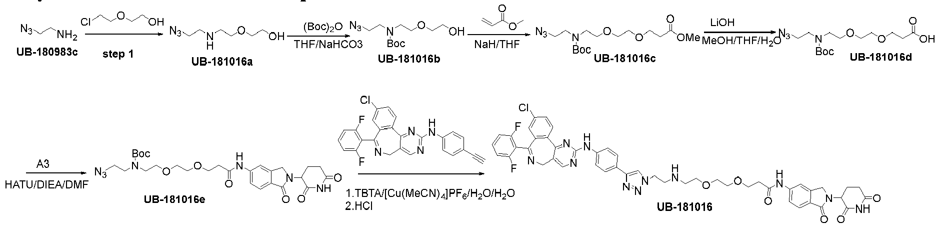

### Step 1: UB-181016a (V2395-047)

**Compound UB-180983c** (7.5 g, 87.2 mmol), 2-chloroethoxyethanol (10.8 g, 87.2 mmol) and potassium carbonate (20.6 g, 149 mmol) were added to anhydrous acetonitrile (100 mL). The mixture was reacted at 80 °C for 18 hours, concentrated after the completion of the reaction. The crude product was isolated by silica gel column chromatography (DCM/MeOH = 10/1) to obtain compound **UB-181016a** (5.31 g, yield 35%) as a colorless oil. LCMS [M+H]⁺ = 175.2

### Step 2: UB-181016b(V2395-048)

**Compound UB-181016a** (5.31g, 30.5 mmol), di-tert-butyl dicarbonate (8.64 g, 39.65 mmol) and sodium bicarbonate (3.6 g, 42.9mmol) were added to tetrahydrofuran (20mL) successively and reacted at room temperature for 2 hours. After the completion of the reaction, the reaction solution was poured into 10 mL of water and extracted with dichloromethane (10 mL*3). The organic phases were combined, then washed with saturated brine, dried over anhydrous Na₂SO₄, and concentrated by rotary evaporation under reduced pressure to obtain compound **UB-181016b(6.19** g, yield 74%). LCMS [M+H]⁺ = 275.3

### Step 3: UB-181016c (V2395-049)

**UB-181016b** (1.00g, 3.65 mmol) was dissolved in THF(10mL), cooled to 0°C, NaH(0.251g, 6.28mmol) was added, the mixture was stirred for 10min. Then methyl acrylate (0.405g, 4.71mmol) was added, and the mixture was reacted at room temperature for 16 h. The mixture was filtered, the filtrate was concentrated under reduced pressure. Water (50ml) was added, the mixture was extracted with ethyl acetate(50ml*3). The combined organic layer was washed with water (50 mL*3) and brine (50 mL*3), dried over anhydrous Na2SO4, concentrated under reduced pressure to obtain the crude product, which was purified by flash chromatography (eluted with MeOH/DCM=0% to 3%) to give the product **UB-181016c** (696 mg, yield 53%). LCMS[M+H]+=361.3

### Step 4: UB-181016d (V2395-051)

Compound **UB-181016c** (696 mg, 1.93 mmol), and lithium hydroxide (1.4 g, 34.8 mmol) were added to water (50 mL) successively, reacted at 30°C for 12 hours. After the completion of the reaction, it was concentrated and the aqueous phase was acidified to pH=5 using hydrochloric acid (1M). Then it was extracted with dichloromethane (10ml*3), the combined organic layer was dried over anhydrous Na₂SO₄, and concentrated to obtain the desired compound **UB-181016d** (588 mg, yield 88%). LCMS [M+H]⁺ =347.3

### Step 5: UB-181016e (V2395-053)

Compound **UB-181016d** (588 mg, 1.70 mmol), A3 (440 mg, 1.70 mmol), HATU (646 mg, 1.70 mmol) and DIPEA (0.5mL) were added to anhydrous DMF (5 mL). The mixture was reacted at room temperature for 16 hours, concentrated after the completion of the reaction. The crude product was isolated by silica gel column chromatography (DCM/MeOH=10/1) to obtain compound **UB-181016e** (688 mg, 69% yield). LCMS [M+H]⁺ =588.6

### Step 6: UB-181016 (V2395-054)

### The method is similar to General Method 3

¹H NMR (400 MHz, DMSO-*d*₆) δ10.98 (s, 1H), 10.54 (d, *J=* 8.5 Hz, 1H), 10.05 (s, 1H), 9.25 (s, 1H), 8.70 (s, 1H), 8.57 (s, 1H), 8.32 (d, *J=* 8.5 Hz, 1H), 8.05 - 7.87 (m, 4H), 7.82 (s, 1H), 7.81 - 7.51 (m, 4H), 7.35 (d, *J* = 2.0 Hz, 1H), 7.31 - 7.06 (m, 2H), 5.05 (dt, *J* = 7.8, 3.9 Hz, 1H), 4.81 (t, *J=* 6.2 Hz, 2H), 4.41 (dd, *J=* 7.3, 8.0 Hz, 1H), 4.34 (ddd, *J=* 4.9, 7.3, 7.9 Hz, 1H), 3.75 - 3.56 (m, 12H), 3.37 (d, *J=* 2.8 Hz, 2H), 3.07 - 2.97 (m, 1H), 2.97 - 2.83 (m, 3H), 2.48 - 2.26 (m, 1H), 2.13 - 1.83 (m, 1H). LCMS [M+H]⁺ = 929.8

### Synthesis method of Compound UBI-181018

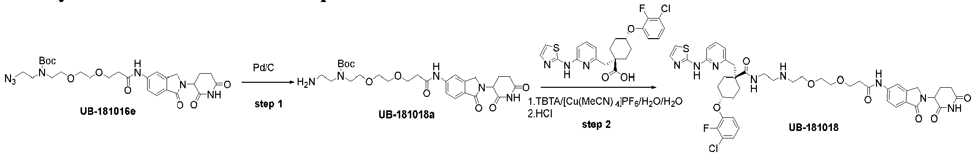

### Step 1: UB-181018a(V2395-060)

Compound **UB-181016e** (200 mg, 0.32 mmol), and 10% palladium on carbon (20 mg) were added to a mixed solvent of methanol/dichloromethane (1 mL/10 mL), and reacted at room temperature for 2 hours under hydrogen atmosphere. After filtration, the filtrate was concentrated to obtain the crude product. The crude product was washed with cold ether (10 mL*3), and dried to obtain target compound **UB-181018a** (170 mg, yield 89 %).

[M+H]⁺ = 562.6

### Step 2: UB-181018 (V2395-061)

### The method is similar to General Method 1

¹H NMR (400 MHz, DMSO-*d*₆) δ10.98 (s, 1H), 10.44 (s, 1H), 8.86 (s, 2H), 7.99 (s, 2H), 7.83 - 7.53 (m, 3H), 7.35 (d, *J*= 2.0 Hz, 1H), 7.31 - 7.06 (m, 4H), 6.98 - 6.70 (m, 2H), 5.05 (dt, *J=* 7.8, 3.9 Hz, 1H), 4.41 (dd, *J=* 7.3, 8.0 Hz, 1H), 4.34 (ddd, *J=* 4.9, 7.3, 7.9 Hz, 1H), 3.75 - 3.56 (m, 9H), 3.07 - 2.77 (m, 7H), 2.71-2.63 (m, 3H), 2.48 - 2.36 (m, 1H), 2.03 - 163 (m, 9H). LCMS [M+H]⁺ = 905.8

### Synthesis method of Compound UB-181031

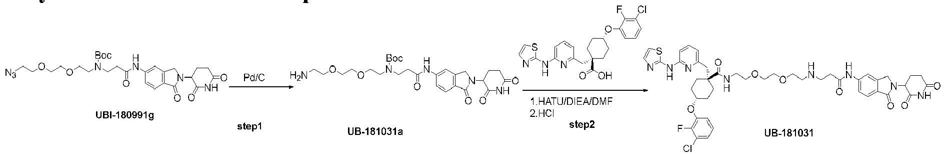

### Step 1: UB-181031a (V2395-062)

Compound **UB-180953e** (200 mg, 0.32 mmol), and 10% palladium on carbon (20 mg) were added to a mixed solvent of methanol/dichloromethane (1 mL/10 mL), and reacted at room temperature for 2 hours under hydrogen atmosphere. After filtration, the filtrate was concentrated to obtain the crude product. The crude product was washed with cold ether (10 mL*3), and dried to obtain target compound **UB-181031a** (170 mg, yield 89 %).

[M+H]⁺ = 562.6

### Step 2: UBI-181031 (V2395-066)

### The method is similar to General Method 1

¹H NMR (400 MHz, DMSO-*d*₆) δ 10.98 (s, 1H), 10.73 (d, *J* = 7.9 Hz, 1H), 8.96 (d, *J*= 4.28 Hz, 2H), 7.96 (s, 1H), 7.80 7.53 (m, 4H), 7.53 7.30 (m, 1H), 7.28 6.97(m, 5H), 6.78 (dd, *J=* 5.3, 7.3 Hz, 1H), 5.08 (dd, *J=* 13.2, 5.1 Hz, 2H), 4.49 (d, *J=* 4.91 Hz, 3H), 4.02-3.53 (m, 17H), 3.40 3.16 (m, 8H), 2.86 2.64 (m, 4H), 2.69 2.47 (m, 1H), 2.44 2.26 (m, 1H), 2.06 1.77 (m, 9H). LCMS [M+H]⁺ = 905.9

### Synthesis method of Compound UBI-181036

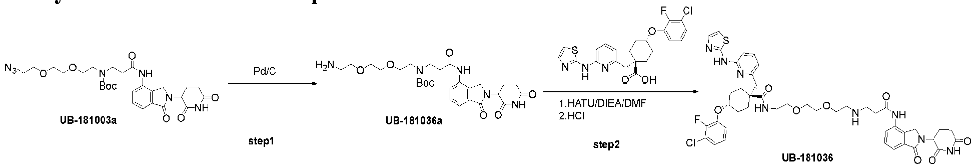

### Step 1: UB-181036a (V2395-063)

Compound **UB-181003a** (200 mg, 0.32 mmol), and 10% palladium on carbon (20 mg) were added to a mixed solvent of methanol/dichloromethane (1 mL/10 mL), and reacted at room temperature for 2 hours under hydrogen atmosphere. After filtration, the filtrate was concentrated to obtain the crude product. The crude product was washed with cold ether (10 mL*3), and dried to obtain target compound **UB-181036a** (170 mg, yield 89 %).

[M+H]⁺ = 562.6

### Step 2: UBI-181036 (V2395-073)

### The method is similar to General Method 1

¹H NMR (400 MHz, DMSO-*d*₆) δ 10.98 (s, 1H), 10.17(d, *J* = 7.9 Hz, 1H), 8.86 (d, *J* = 4.28 Hz, 2H), 7.96-7.82 (m, 1H), 7.80 7.53 (m, 4H), 7.48 7.40 (m, 1H), 7.28 6.87(m, 5H), 6.78 (dd, *J=* 5.3, 7.3 Hz, 1H), 5.08 (dd, *J=* 13.2, 5.1 Hz, 1H), 4.49 (d, *J=* 4.91 Hz, 3H), 4.02-3.53 (m, 6H), 3.40 3.16 (m, 6H), 2.86 2.64 (m, 4H), 2.69 2.47 (m, 1H), 2.44 2.26 (m, 2H), 2.06 1.87 (m, 1H). 1.83 1.58 (m, 7H). LCMS [M+H]⁺ = 905.7

### Synthesis method of Compound UBI-181039

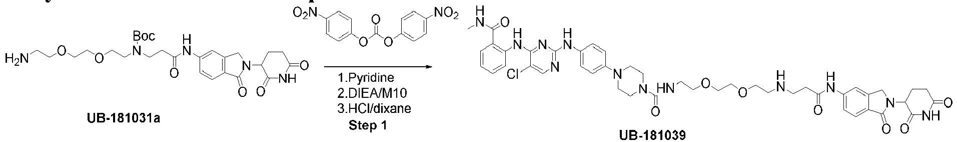

### Step 1: UBI-181039 (V2395-075)

### The method is similar to General Method 4

¹H NMR (400 MHz, DMSO-*d*₆) δ 12.01 (s, 1H), 10.98 (s, 1H), 10.83 (s, 1H),10.14 (s, 1H), 9.18 8.84 (m, 2H), 8.56 (t, *J=* 3.6 Hz, 1H), 8.33 (s, 1H), 7.98 (s, 1H), 7.94 7.63 (m, 9H), 7.42 7.17 (m, 2H), 5.08 (dd, *J* = 13.2, 5.1 Hz, 1H), 4.45 4.34 (m, 2H), 3.79 3.03 (m, 19H), 3.08 2.70 (m,5H), 2.98 2.64 (m, 7H), 2.64 2.59 (m, 2H), 2.44 2.17 (m, 2H), 2.24 1.97 (m, 1H). LCMS [M+H]⁺ = 925.8

### Synthesis method of Compound UBI-181043

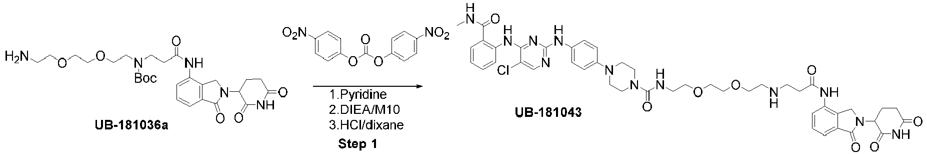

### Step 1: UBI-181043 (V2395-081)

### The method is similar to General Method 4

¹H NMR (400 MHz, DMSO-*d*₆) δ 12.01 (s, 1H), 10.98 (s, 1H), 10.23 (s, 1H),9.94 (s, 1H), 9.18 8.84 (m, 3H), 8.56 (t, *J* = 3.6 Hz, 1H), 8.33 (s, 1H), 7.94 7.43 (m, 9H), 7.42 7.17 (m, 2H), 5.08 (dd, *J=* 13.2, 5.1 Hz, 1H), 4.45 - 4.34 (m, 2H), 3.79 3.03 (m, 16H), 3.01 2.70 (m,4H), 2.98 2.64 (m, 3H), 2.64 2.59 (m, 2H), 2.44 2.17 (m, 1H), 2.24 1.97 (m, 1H). LCMS [M+H]⁺ = 925.9

### Synthesis method of Compound UBI-181044

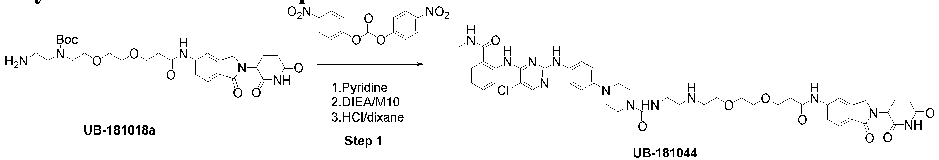

### Step 1: UBI-181044 (V2395-084)

### The method is similar to General Method 4

¹H NMR (400 MHz, DMSO-*d*₆) δ 12.01 (s, 1H), 10.98 (s, 1H), 10.23 (s, 1H),9.94 (s, 1H), 9.18 8.84 (m, 3H), 8.56 (t, *J* = 3.6 Hz, 1H), 8.33 (s, 1H), 7.94 7.43 (m, 9H), 7.42 7.17 (m, 2H), 5.08 (dd, *J* = 13.2, 5.1 Hz, 1H), 4.45 4.34 (m, 2H), 3.79 3.03 (m, 16H), 3.01 2.70 (m,4H), 2.98 2.64 (m, 3H), 2.64 2.59 (m, 2H), 2.44 2.17 (m, 1H), 2.24 1.97 (m, 1H). LCMS [M+H]⁺ = 925.9

### Synthesis method of Compound UBI-181060

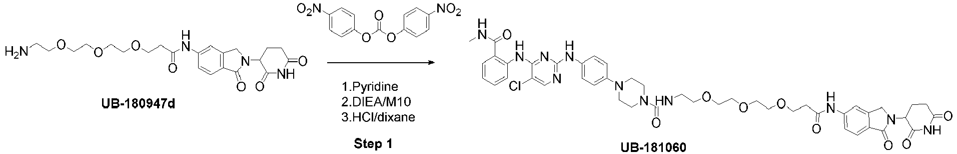

### Step 1: UBI-181060 (V2395-092)

### The method is similar to General Method 4

¹H NMR (400 MHz, DMSO-*d*₆) δ 11.81 (s, 1H), 10.98 (s, 1H), 10.43 (s, 1H), 9.63 (s, 1H), 9.34 (s, 3H), 8.89 8.79 (m, 1H), 8.66 8.58 (m, 1H),8.34 (s, 1H), 8.03 (s, 1H), 7.95 7.74 (m, 1H), 7.74 7.36 (m, 4H), 7.42 7.36 (m, 3H), 5.08 (dd, *J* = 13.2, 5.1 Hz, 1H), 4.33 4.24 (m,2H), 3.78 3.46 (m, 10H), 3.42 3.05 (m, 9H), 3.21 2.81 (m, 1H), 2.81 (s, 3H), 2.80 2.58 (m, 3H), 2.45 2.29 (m, 1H), 2.01 1.88 (m, 1H), 1.52 1.39 (m, 1H), 1.28 (dt, *J* = 11.0, 8.6 Hz, 1H) LCMS [M+H]⁺ = 927.0

### Synthesis method of Compound UB-181074

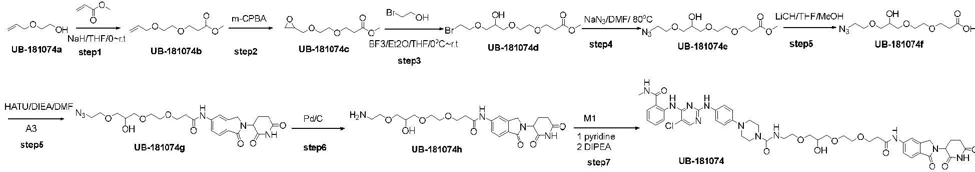

### Step 1: UB-181074b (V2395-097)

**UB-181074a** (10.0g, 98.04 mmol) was dissolved in THF(100mL), cooled to 0°C, NaH(0.251g, 6.28mmol) was added, the mixture was stirred for 10min. Then methyl acrylate (12.65 g, 147.1mmol) was added, and the mixture was reacted at room temperature for 16 h. The mixture was filtered, the filtrate was concentrated under reduced pressure. Water (50ml) was added, the mixture was extracted with ethyl acetate(50ml*3). The combined organic layer was washed with water (50 mL*3) and brine (50 mL*3), dried over anhydrous Na2SO4, concentrated under reduced pressure, purified by flash chromatography (eluted with MeOH/DCM=0% to 3%) to give the compound **UB-181074b** (9.2 g, yield 51%). LCMS[M+H]+= 189.2

¹H NMR (400 MHz, CDCl₃) δ6.01 5.76 (m, 1H), 5.41 5.14 (m, 2H), 4.06 4.01 (m, 2H), 3.78-3.76(m, 2H), 3.69-3.64(m, 2H),3.56-3.23(m, 2H), 2.83 2.52 (m, 86H).

### Step 2: UB-181074c (V2395-112)

**UB-181074b(10** g, 53 mmol)was dissolved in DCM(30 mL), cooled to 0°C, mCPBA(11 g, 64 mmol) was added. After completion of the reaction, the reaction mixture was added with water and stirred for 10minutes, filtered and concentrated in vacuum to obtain crude product, which was purified by flash chromatography (DCM/MeOH = 10/1) to obtain **UB-181074c** (2.5 g, yield 22%) as a white solid. LCMS [M+H]⁺ = 205.2

### Step 3: UB-181074d (V2395-113)

**UB-181074c**(1g, 4.9 mmol) and boron trifluoride ethyl ether (0.136 mL, 1.08 mmol) were dissolved in 20 mL THF. In an ice bath, 2-bromoethan-1-ol (1.11 g, 9 mmol) was slowly added dropwise. After completion, the mixture was heated in 45°C oil bath for 90 minutes. After completion of the reaction, the reaction solution was concentrated to obtain **UB-181074d** (1.2g, yield 75%) as an oil. LCMS [M+H]⁺ = 330.2

### Step 4: UB-181074e (V2395-120)

**UB-181074d** (1 g, 3.04 mmol) and sodium azide (321 mg, 4.9 mmol) were mixed and dissolved in DMF(10 ml), the mixture was stirred at room temperature overnight, after completion of the reaction, the reaction was diluted with H2O (300 ml) and extracted with ether (2x150 ml). The organic phase was washed with H₂O (3×100 ml) and brine (1×100 ml), dried over MgSO₄, filtered, and concentrated to obtain crude product, which was purified by flash chromatography to obtain product **UB-181074e** (880 mg, yield :97%.) LCMS [M+H]⁺ = 292.2

### Step 5: UB-181074f (V2395-121)

Compound **UB-181074e** (696 mg, 2.39 mmol), and lithium hydroxide (1.4 g, 34.8 mmol) were added to water (50 mL) successively, reacted at 30°C for 12 hours. After the completion of the reaction, it was concentrated and the aqueous phase was acidified to pH=5 using hydrochloric acid (1M). Then it was extracted with dichloromethane (10ml*3), the combined organic layer was dried over anhydrous Na₂SO₄, and concentrated to obtain the desired compound **UB-181074f** (563 mg, yield 85%). LCMS [M+H]⁺ = 278.2

### Step 6: UB-181074g(V2395-122)

Compound **UB-181074f** (563 mg, 2.03 mmol), A3 (880 mg, 3.40 mmol), HATU (1292 mg, 3.40 mmol) and DIPEA (0.5mL) were added to anhydrous DMF (5 mL). The mixture was reacted at room temperature for 16 hours, concentrated after the completion of the reaction. The crude product was isolated by silica gel column chromatography (DCM/MeOH=10/1) to obtain compound **UB-181074**g (694mg, 66% yield). LCMS [M+H]⁺ =519.5

### Step 7: UB-181074h (V2395-128)

Compound **UB-181074**g (200 mg, 0.39 mmol), and 10% palladium on carbon (20 mg) were added to a mixed solvent of methanol/dichloromethane (1 mL/10 mL), and reacted at room temperature for 2 hours under hydrogen atmosphere. After filtration, the filtrate was concentrated to obtain the crude product. The crude product was washed with cold ether (10 mL*3), and dried to obtain compound **UB-181074**h (172 mg, yield 91 %). [M+H]⁺ = 493.5

### Step 8: UB-181074(V2395-129)

### The method is similar to General Method 4

¹H NMR (400 MHz, DMSO-*d*₆) 11.63 (s, 1H), 10.97 (s, 1H), 10.31 (s, 1H), 9.27 (s, 1H), 8.77 (s, 2H), 8.17 (s, 1H), 7.99 (s, 1H), 7.75 (d, *J=* 7.9 Hz, 1H), 7.63 (dd, *J=* 8.4, 8.3 Hz, 2H), 7.48 (dd, *J=* 13.8, 7.2 Hz, 3H), 7.09 (dd, *J=* 44.3, 37.1 Hz, 3H), 6.61 (s, 1H), 5.08 (dd, *J=* 13.2, 5.1 Hz, 1H), 4.42 (d, *J* = 17.4 Hz, 1H), 4.28 (d, *J* = 17.3 Hz, 1H), 3.70 (dt, *J* = 11.7, 5.8 Hz, 3H), 3.64 3.56 (m, 1H), 3.56 3.25 (m, 19H), 3.23 2.88 (m, 6H), 2.88 2.67 (m, 4H), 2.62 2.24 (m, 1H), 1.96 (dd, *J* = 2.2, 5.1 Hz, 1H) LCMS [M+H]⁺ = 956.9

### Synthesis method of Compound UB-181077

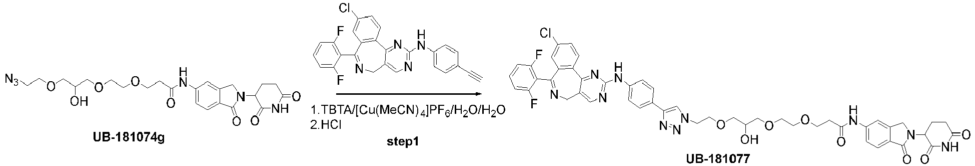

### Step 1: UB-181077(V2395-130)

### The method is similar to General Method 3

¹H NMR (400 MHz, DMSO-*d*₆)δ 10.97 (s, 1H), 10.27 (s,1H), 10.00 (s, 1H), 8.69 (d, *J=* 3.5 Hz, 1H), 8.47 (dd, *J* = 13.6, 7.5 Hz, 2H), 8.31 (dd, *J* = 8.5, 2.8 Hz, 1H), 8.01 7.77 (m, 2H), 7.77 7.48 (m, 1H), 7.36 7.24 (m, 5H), 7.24-7.11 (m, 4H), 5.07 (dd, *J* = 13.2, 5.0 Hz, 1H), 4.54 (d, *J* = 4.4 Hz, 2H), 4.48 4.31 (m, 2H), 3.83 (dd, *J* = 12.4, 7.5 Hz, 2H), 3.78 3.61 (m, 5H), 3.61 3.10 (m,3H), 3.05 2.79 (m, 3H), 2.66 2.55 (m, 3H), 2.41 2.05 (m, 1H), 2.12 1.95 (m, 1H), 1.32 1.18 (m, 1H) .LCMS [M+H]⁺ = 975.9

### Synthesis method of Compound UB-181079

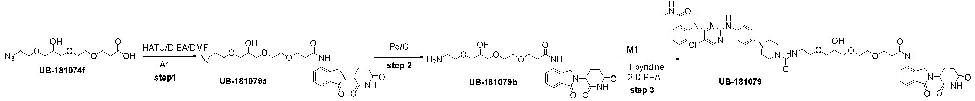

### Step 1: UB-181079a (V2395-135)

Compound **UB-181074f** (563 mg, 2.03 mmol), lenalidomide (880 mg, 3.40 mmol), HATU (1292 mg, 3.40 mmol) and DIPEA (0.5mL) were added to anhydrous DMF (5 mL). The mixture was reacted at room temperature for 16 hours, concentrated after the completion of the reaction. The crude product was isolated by silica gel column chromatography (DCM/MeOH=10/1) to obtain **UB-181079a** (694mg, 66% yield). LCMS [M+H]⁺ =519.5

### Step 2: UB-181079b (V2395-136)

Compound **UB-181079a** (200 mg, 0.39 mmol), and 10% palladium on carbon (20 mg) were added to a mixed solvent of methanol/dichloromethane (1 mL/10 mL), and reacted at room temperature for 2 hours under hydrogen atmosphere. After filtration, the filtrate was concentrated to obtain the crude product. The crude product was washed with cold ether (10 mL*3), and dried to obtain compound **UB-181079b** (172 mg, yield 91 %). [M+H]⁺ = 493.5

### Step 3: UB-181079(V2395-137)

### The method is similar to General Method 4

¹H NMR (400 MHz, DMSO-*d*₆)δ 11.64 (s, 1H), 11.02 (s, 1H), 9.91 (s, 1H), 9.21 (d, *J*= 9.6 Hz, 2H), 8.78 (d, *J* = 4.6 Hz, 2H), 8.17 (s, 1H), 7.79 (dd, *J* = 6.0, 7.3 Hz, 2H), 7.70 7.05 (m, 6H), 7.09 (dd, *J* = 4.4, 3.9 Hz, 2H), 6.63 (s, 1H), 5.14 (dd, *J* = 13.3, 5.0 Hz, 1H), 4.81 4.25 (m, 2H), 4.02 3.51 (m, 15H), 3.41 3.02 (m, 8H), 3.01 2.59 (m, 7H), 2.41 2.31 (m, 1H), 2.12 1.95 (m, 1H). LCMS [M+H]⁺ = 956.8

### Synthesis method of Compound UB-181086

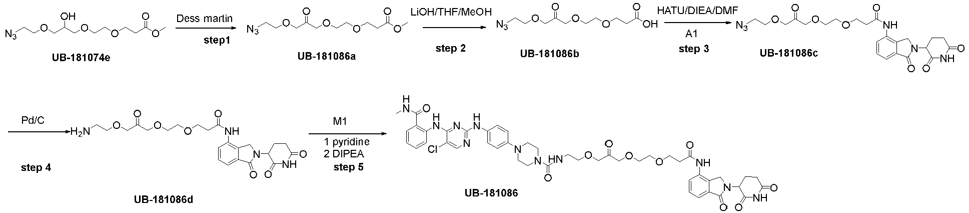

### Step 1: UB-181086a (V2395-115)

**UB-181074e** (1 g, 3.44 mmol) was dissolved in DCM (10 mL), Dess Martin reagent (2.18 g, 5.15 mmol) was added, the mixture was stirred at 85°C overnight. After completion of the reaction, the reaction mixture was filtered and the filtrate was concentrated to obtain crude product. The crude product was isolated by flash column chromatography (DCM/MeOH = 30/1) to obtain **UB-181086a** (880 mg, yield 90%) as a colorless oil.

LCMS [M+H]⁺ = 290.2

### Step 2: UB-181086b (V2395-121)

Compound **UB-181086a** (694 mg, 2.39 mmol), and lithium hydroxide (1.4 g, 34.8 mmol) were added to water (50 mL) successively, reacted at 30°C for 16 hours. After the completion of the reaction, the mixture was acidified to pH=5 using hydrochloric acid (1M). Then it was extracted with dichloromethane (10ml*3), the combined organic layer was dried over anhydrous Na₂SO₄, and concentrated to obtain UB-181086b (561mg, yield 85%). LCMS [M+H]⁺ = 276.2

### Step 3: UB-181086c(V2395-122)

Compound **UB-181086b** (561 mg, 2.04 mmol), A3 (880 mg, 3.40 mmol), HATU (1292 mg, 3.40 mmol) and DIPEA (0.5mL) were added to anhydrous DMF (5 mL). The mixture was reacted at room temperature for 16 hours, concentrated after the completion of the reaction. The crude product was isolated by silica gel column chromatography (DCM/MeOH=10/1) to obtain compound **UB-181086c** (692 mg, 66% yield). LCMS [M+H]⁺ =517.5

### Step 4: UB-181086d (V2395-128)

Compound **UB-181086c** (200 mg, 0.39 mmol), and 10% palladium on carbon (20 mg) were added to a mixed solvent of methanol/dichloromethane (1 mL/10 mL), and reacted at room temperature for 2 hours under hydrogen atmosphere. After filtration, the filtrate was concentrated to obtain the crude product. The crude product was washed with cold ether (10 mL*3), and dried to obtain compound **UB-181086d** (171 mg, yield 91 %). [M+H]⁺ = 491.5

### Step 5: UB-181086 (V2395-149)

### The method is similar to General Method 4

¹H NMR (400 MHz, DMSO-*d*₆)δ 11.65 (s, 1H), 11.03 (s, 1H), 9.90 (s, 1H), 9.21 (d, *J* = 9.6 Hz, 2H), 8.75 (d, *J* = 4.6 Hz, 2H), 8.15 (s, 1H), 7.79 (dd, *J* = 6.0, 7.3 Hz, 2H), 7.70 7.05 (m, 6H), 7.10 (dd, *J=* 4.1, 5.2 Hz, 2H), 6.63 (s, 1H), 5.12 (dd, *J=* 9.3, 7.2 Hz, 1H), 4.81 4.25 (m, 2H), 4.02 3.51 (m, 15H), 3.41 3.02 (m, 8H), 3.01 2.59 (m, 7H), 2.41 2.31 (m,1H), 2.12 1.95 (m, 1H). LCMS [M+H]⁺ = 954.9

### Synthesis method of Compound UB-181088

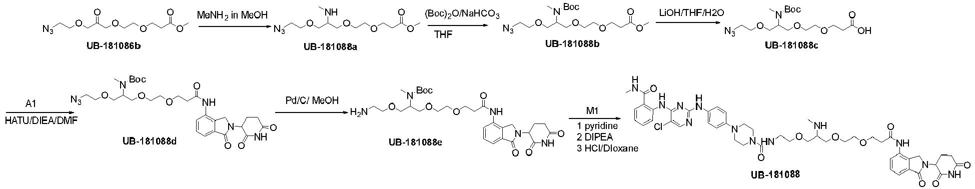

### Step 1: UB-181086a (V2395-117)

Compound **UB-181086b** (1 g, 3.46 mmol), and methylamine aqueous solution (1N, 6.92 ml, 6.92 mmol) were added to dichloromethane (10 mL), sodium cyanoborohydride (644 mg, 10.4 mmol) was added. After the completion of the reaction, the reaction was poured into 10 mL of water and extracted with dichloromethane (5 mL*3). The organic phases were combined, then washed with saturated brine, dried over anhydrous Na₂SO₄, concentrated by rotary evaporation under reduced pressure to obtain crude product. The crude product was purified by silica gel column chromatography (DCM/MeOH = 10/1) to obtain compound **UB-181088a** (947 mg, yield 90%). LCMS [M+H]⁺ = 305.3

**Step 2: UB-181088b (V2395119)Compound UB-181088a** (947 mg, 3.12 mmol), di-tert-butyl dicarbonate (864 mg, 3.96 mmol) and sodium bicarbonate (360 mg, 4.29 mmol) were added to dioxane (20mL) successively and reacted at room temperature for 2 hours. After the completion of the reaction, the reaction solution was poured into 10 mL of water and extracted with ethyl acetate (10 mL*3). The organic phases were combined, then washed with saturated brine, dried over anhydrous Na₂SO₄, and concentrated by rotary evaporation under reduced pressure to obtain compound **UB-181088b** (931 mg, yield 74%). LCMS [M+H]⁺ =405.3

### Step 3: UB-181088c (V2395-121)

Compound **UB-181088b** (931 mg, 2.31 mmol), and lithium hydroxide (700 mg, 17.4 mmol) were added to water (5 mL) successively, reacted at room temperature for 12 hours. After the completion of the reaction, it was concentrated and the aqueous phase was acidified to pH=5 using hydrochloric acid (1M). Then it was extracted with dichloromethane (10ml*3), the combined organic layer was dried over anhydrous Na₂SO₄, and concentrated to obtain **UB-181088c** (764 mg, yield 85%). LCMS [M+H]⁺ = 391.3

### Step 4: UB-181088d (V2395-122)

Compound **UB-181088c** (560 mg, 1.44 mmol), lenalidomide (372 mg, 1.44 mmol), HATU (820 mg, 2.16 mmol) and DIPEA (0.5mL) were added to anhydrous DMF (5 mL). The mixture was reacted at room temperature for 16 hours, concentrated after the completion of the reaction. The crude product was isolated by silica gel column chromatography (DCM/MeOH=10/1) to obtain compound **UB-181088d** (589 mg, 65% yield). LCMS [M+H]⁺ =632.6

### Step 5: UB-181088e (V2395-128)

Compound **UB-181088d** (200 mg, 0.39 mmol), and 10% palladium on carbon (20 mg) were added to a mixed solvent of methanol/dichloromethane (1 mL/10 mL), and reacted at room temperature for 2 hours under hydrogen atmosphere. After filtration, the filtrate was concentrated to obtain the crude product. The crude product was washed with cold ether (10 mL*3), and dried to obtain target compound **UB-181088e** (182 mg, yield 95 %). LCMS [M+H]⁺ =606.6

### Step 6: UB-181088 (V2714-008)

### The method is similar to General Method 4

¹H NMR (400 MHz, DMSO-*d*₆)δ 11.86 (s, 1H), 11.03 (s, 1H), 9.98 (s, 1H), 9.73 (s, 1H), 8.83 (d, J=4.3 Hz, 3H), 8.68 (s, 1H), 8.26 (s, 1H), 7.82 - 7.55 (m, 7H), 7.42 - 7.09 (m, 3H), 6.88 (s, 1H), 5.15 (dd, *J=* 13.2, 5.1 Hz, 1H), 4.38 (dd, *J=* 3.5, 7.5 Hz, 2H), 3.78 - 3.50 (m, 14H), 3.50-3.02 (m, 8H), 2.98-2.66 (m, 7H), 2.66-2.23 (m, 8H), 2.05(s, 1H) . LCMS [M+H]⁺ = 939.9

### Synthesis method of Compound UB-181090

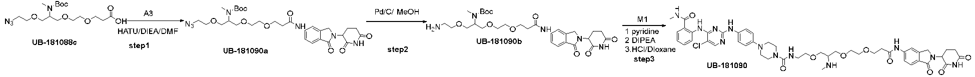

### Step 1: UB-181090a (V2395-122)

Compound **UB-181088c** (560 mg, 1.44 mmol), lenalidomide (372 mg, 1.44 mmol), HATU (820 mg, 2.16 mmol) and DIPEA (0.5mL) were added to anhydrous DMF (5 mL). The mixture was reacted at room temperature for 16 hours, concentrated after the completion of the reaction. The crude product was isolated by silica gel column chromatography (DCM/MeOH=10/1) to obtain compound **UB-181090a** (589 mg, 65% yield). LCMS [M+H]⁺ =632.6

### Step 2: UB-181090b (V2395-128)

Compound **UB-181090a** (200 mg, 0.39 mmol), and 10% palladium on carbon (20 mg) were added to a mixed solvent of methanol/dichloromethane (1 mL/10 mL), and reacted at room temperature for 2 hours under hydrogen atmosphere. After filtration, the filtrate was concentrated to obtain the crude product. The crude product was washed with cold ether (10 mL*3), and dried to obtain target compound **UB-181090b** (182 mg, yield 95 %). LCMS [M+H]⁺ =606.6

### Step 3: UB-181090 (V2714-010)

### The method is similar to General Method 4

¹H NMR (400 MHz, DMSO-*d*₆)δ12.03 (s, 1H), 11.01 (s, 1H), 10.52 (d, *J* = 3.5 Hz, 1H), 9.87 (s, 3H),9.11-8.94 (m, 3H),8.77-8.04 (m, 3H),7.87 -7.44 (m, 7H),7.22(t, *J* = 7.5 Hz, 1H), 5.15 (dd, *J=* 13.2, 5.1 Hz, 1H), 4.38 (dd, *J=* 3.5, 7.5 Hz, 2H), 3.86 (s, 3H), 3.77-3.53(m, 4H),3.49 (s, 6H),3.46 (d, *J=* 4.5 Hz, 1H),3.39 (dd, *J=* 6.1, 3.6 Hz, 2H), 3.18-2.99 (m, 7H), 2.96-2.59 (m, 10H), 2.53-2.36(m,1H),2.02-1.96(m, 1H). LCMS [M+H]⁺ = 970.1

### Synthesis method of Compound UB-181103

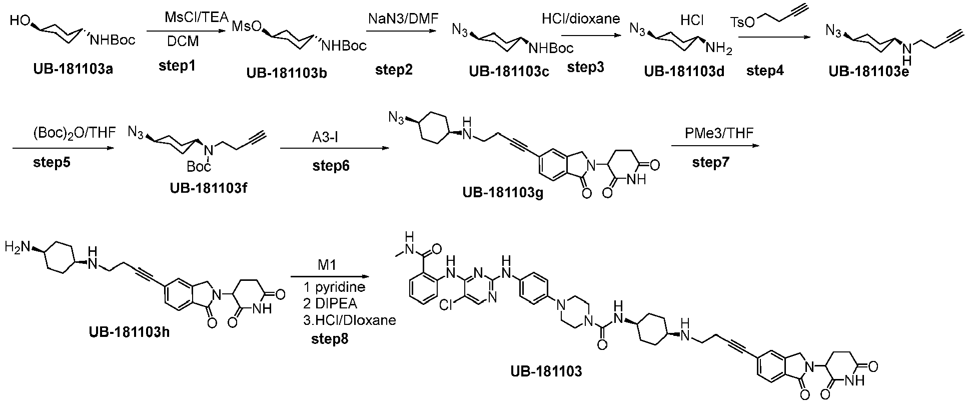

### Step 1: UB-181103b(V2714-018)

**To** a solution of **UB-181103a** (10 g, 22 mmol) and triethylamine (7.05 g, 70 mmol) in dichloromethane (10 ml) was added methanesulfonyl chloride (6.89 g, 60 mmol) dropwise, and the mixture was stirred overnight at room temperature. After completion of the reaction, to the mixture was added water (10ml) and extracted with DCM (10ml*3). The organic layer was dried over Na₂SO₄ and concentrated to obtain white solids **UB-181103b** (13g, yield: 98%.) LCMS [M+H]⁺ =294.3

### Step 2: UB-181103c(V2714-019)

**UB-181103b** (13 g, 44 mmol) and sodium azide (3.75 g, 58 mmol) were mixed and dissolved in DMF(10 ml), the mixture was stirred at room temperature overnight, after completion of the reaction, the reaction was diluted with H2O (300 ml) and extracted with ether (2x150 ml). The organic phase was washed with H₂O (3×100 ml) and brine (1×100 ml), dried over MgSO₄, filtered, and the solvent was removed under reduced pressure to obtain product **UB-181103c** (9 g, yield: 88%) LCMS [M+H]⁺ =241.3

### Step 3: UB-181103d(V2714-020)

Compound **UB-181103c** (10 g, 0.042 mmol) and hydrochloric acid in dioxane (100 mL, 4 N) were added to tetrahydrofuran (10mL), the mixture was reacted at room temperature for 2 hours. After the completion of the reaction, the mixture was concentrated by rotary evaporation under reduced pressure to obtain compound **UB-181103d** (5.8 g, yield 99%). LCMS [M+H]⁺ =141.3

### Step 4: UB-181103e(V2714-027)

### Compound UB-1811103d (1.0g, 5.68 mmol), 3-butynyl p-toluenesulfonate

(1.27 g, 5.68 mmol) and triethylamine (6.06 g, 60mmol) were mixed, then dissolved in toluene (20 mL), the mixture was reacted at 80°C for 18 hours, filtered after the completion of the reaction, the filtrate was concentrated by rotary evaporation under reduced pressure, and isolated by silica gel column chromatography (DCM/MeOH =10/1) to obtain **UB-181103e** (818 mg, yield 75 %) as a colorless oil. LCMS [M+H]⁺ =193.3

### Step 5: UB-181103f (V2714-032)

**Compound UB-181103e** (350 mg, 1.82 mmol), di-tert-butyl dicarbonate (441 mg, 2.03 mmol) and sodium bicarbonate (360 mg, 4.29mmol) were added to tetrahydrofuran (20mL) successively and reacted at room temperature for 2 hours. After the completion of the reaction, the reaction was poured into 10 mL of water and extracted with dichloromethane (5 mL*3). The organic phases were combined, then washed with saturated brine, dried over anhydrous Na₂SO₄, and concentrated by rotary evaporation under reduced pressure to obtain compound **UB-181103f** (463 mg, yield 87%) as a colorless oil. LCMS [M+H]⁺ =293.3

### Step 6: UB-181103g (V2714-033)

Compound **UB-181103f** ( 30 mg, 0.103 mmol) and A1-I (38 mg, 0.103 mmol) were dissolved in DMF (10 mL), dichlorobis(triphenylphosphonium)palladium (7.2 mg,0.010 mmol), cuprous iodide (3.91 mg, 0.021 mmol) and triethylamine (150 mg, 1.49 mmol) were added, the mixture was reacted at 80°C overnight. The reaction solution was filtered through Celite, and the filtrate was concentrated to obtain crude product, which was purified by flash chromatography (eluted with DCM/MeOH=0% to 20% for 30 minutes) to obtain product **UB-181103g** (9 mg, yield 17%). LCMS [M+H]⁺ =435.5

### Step 7: UB-181103h(V2714-034)

**UB-181103g** (1 g, 1.87 mmol) was dissolved in THF(10 mL), trimethylphosphine (402 mg, 1.87 mmol) was added, the mixture was reacted at room temperature overnight, concentrated after completion of the reaction to obtain crude product, which was purified by flash chromatography (DCM/MeOH=10/1) to obtain product **UB-181103h**(510 mg, yield 91%). LCMS [M+H]⁺ =409.5

### Step 8: UB-181103(V2714-035)

### The method is similar to General Method 3

¹H NMR (400 MHz, DMSO-*d*₆) δ11.85 (s, 1H), 11.00 (s, 1H), 9.78 (s, 1H), 9.16 (s, 2H), 8.84 (d, *J=* 4.5 Hz, 1H), 8.69 (s, 1H), 8.27 (s, 1H), 7.80 (d, *J=* 6.8 Hz, 1H), 7.70 (dd, *J=* 8.3, 2.3 Hz, 2H), 7.56 (dt, *J=* 15.9, 9.9 Hz, 3H), 7.20 (dd, *J=* 18.0, 10.0 Hz, 1H), 6.20 (s, 1H), 5.11 (dd, *J=* 13.3, 5.0 Hz, 1H), 4.46 (d, *J=* 7.7 Hz, 1H), 4.33 (d, *J=* 7.7 Hz, 1H), 3.80 (d, *J=* 7.9 Hz, 4H), 3.31 (t, *J=* 6.2 Hz, 4H), 3.30-3.15 (m, 4H), 2.98-2.76 (m, 3H), 2.67 (dd, *J=* 3.4, 5.7 Hz, 3H), 2.65 - 2.51 (m, 2H), 2.45 - 2.31 (m, 2H), 2.05 - 1.97 (m, 1H), 1.93-1.77 (m, 4H), 1.70-1.59 (m, 2H). LCMS [M+H]⁺ = 872.9

### Synthesis method of Compound UB-181104

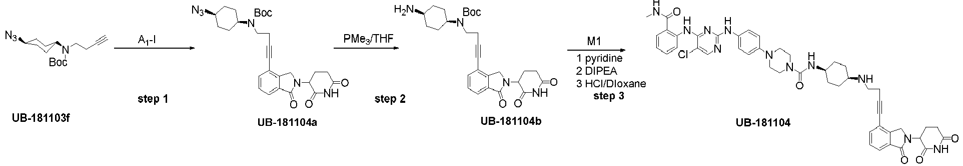

### Step 1: UB-181104a(V2714-027)

Compound **UBI-181103f** ( 300 mg, 1.03 mmol) and A1-I (380 mg, 1.03 mmol) were dissolved in DMF (10 mL), dichlorobis(triphenylphosphonium)palladium (72 mg,0.10 mmol), cuprous iodide (39.1 mg, 0.21 mmol) and triethylamine (150 mg, 1.49 mmol) were added, the mixture was reacted at 80°C overnight. The reaction solution was filtered through Celite, and the filtrate was concentrated to obtain crude product, which was purified by flash chromatography (eluted with DCM/MeOH=0% to 20% for 30 minutes) to obtain product **UB-181104a** (90 mg, yield 17%). LCMS [M+H]⁺ =435.5

### Step 2: UB-181104b(V2714-033)

**UB-181104a** (1 g, 1.87 mmol) was dissolved in THF(10 mL), trimethylphosphine (402 mg, 1.87 mmol) was added, the mixture was reacted at room temperature overnight, concentrated after completion of the reaction to obtain crude product, which was purified by flash chromatography (DCM/MeOH=10/1) to obtain product **UB-181104b**(510 mg, yield 91%). LCMS [M+H]⁺ =409.5

### Step 3: UB-181104(V2714-037)

### The method is similar to General Method 4

¹H NMR (400 MHz, DMSO-*d*₆)δ11.87 (s, 1H), 11.02 (s, 1H), 9.81 (s, 1H), 9.18 (s, 2H), 8.84 (d, *J=* 4.5 Hz, 1H), 8.67 (s, 1H), 8.43 - 8.08 (m, 1H), 7.64 (dddd, *J=* 5.0, 5.8, 3.0, 5.2 Hz, 8H), 7.42 (d, *J=* 9.2 Hz, 2H), 7.45 - 6.78 (m, 1H), 6.19 (s, 1H), 5.17 (dd, *J=* 13.3, 5.1 Hz, 1H), 4.54 (d, *J=* 7.8 Hz, 1H), 4.36 (d, *J=* 7.8 Hz, 1H), 3.79 (s, 2H), 3.59 - 3.23 (m, 8H), 3.18 - 2.74 (m, 7H), 2.70 - 2.51 (m, 1H), 2.51 - 2.37 (m, 1H), 2.10 -2.05(m, 1H), 1.95- 1.75 (m, 7H), 1.46 (d, *J=* 6.7 Hz, 2H). LCMS [M+H]⁺ = 872.9

### Synthesis method of Compound UB-181127

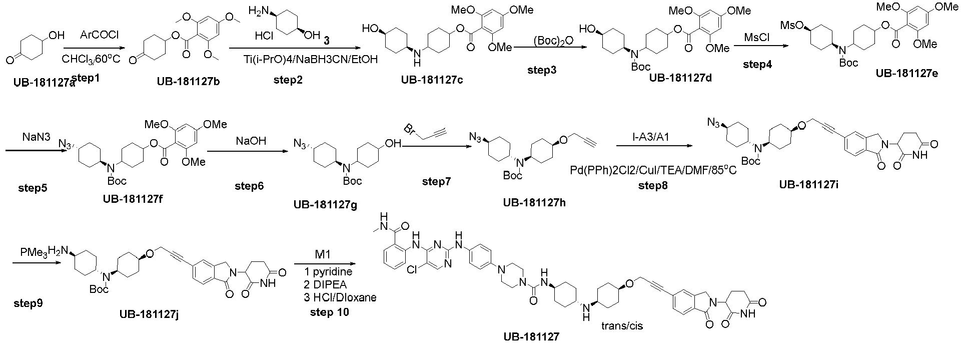

### Step 1: UB-181127b(V2714-048)

3,4,5-Trimethoxybenzoic acid (1008 mg, 4.38 mmol) and SOCl2(2 equivalent) were added to 5 mL CHCl₃, the mixture was reacted at 60°C for 4 h. The reaction mixture was cooled to room temperature, concentrated under reduced pressure to obtain chloride in CHCl3, 4-hydroxycyclohexanone (0.5 g, 4.38 mmol) was added. At 60°C, the mixture was reacted for 8 hours, then cooled to room temperature, treated with CH₂Cl₂, the organic layer was washed with 10%NaOH solution. The mixture was dried over Na2S04, then solvent was removed under reduced pressure, the system was eluted with cyclohexane/ethyl acetate(7:3), the residue was purified by flash chromatography to obtain **product UBI-181127b** (390mg, yield 29%). LCMS [M+H]⁺ =309.3

1H NMR(CDCl3): δ 7.25 (s, 2H, aromatics); 5.41-5.32 (m, 1H, CHO); 3.85(s, 9H, 3OCH3); 2.57-2.53 (m, 2H, CH2); 2.51-2.40 (m, 2H, CH2);2.21-2.16 (m, 4H, 2CH2).

### Step 2: UB-181127c(V2714-050)

Compound **UB-181127b** (420 mg, 2.76 mmol), trans-4-aminocyclohexanol (851 mg, 2.76 mmol) and titanium isopropoxide(IV)(3.1 mL, 8.3 mmol) were mixed and reacted for 3 hours, anhydrous ethanol (2.5 mL) and NaBH3CN(170 mg, 1.79 mmol) were added and stirred for 20 hours. Water (10 mL) was added, the organic solvent was removed under reduced pressure, the crude product was dissolved with CH2Cl2, and the organic layer was washed with saturated NaHCO3 solution. After drying with Na2SO4, the solvent was removed under reduced pressure and purified by flash chromatography using CHCl3/MeOH (9:1) as the elution system to obtain Compound **UB-181127c** (410 mg, yield 74%). LCMS [M+H]+ =408.5

1H NMR (CDCl3): *δ* 7.27 (s, 2H,aromatics); 5.12 (bs, 0.5H, CHO); 4.92 (tt, J=10.8 Hz, *J* = 4.4 Hz, 0.5H, CHO); 3.87 (s, 9H, 3OCH3); 3.70-3.48 (m, 1H, CHOH);2.73-2.49 (m, 2H, 2NCH); 2.20-1.01 (m, 18H, 8CH2, OH and NH) ppm.

### Step 3: UB-181127d(V2714-051)

**Compound UB-181127c** (20 mg, 0.049 mmol), di-tert-butyl dicarbonate (16 mg, 0.074 mmol) and tBuOK (8.2 mg) were added to tBuOH (10mL) successively, the mixture was reacted at 60 °C for 8 hours. After the completion of the reaction, the reaction solution was poured into 10 mL of water and extracted with ethyl acetate (10 mL*3). The organic phases were combined, then washed with saturated brine, dried over anhydrous Na₂SO₄, and concentrated by rotary evaporation under reduced pressure to obtain compound **UB-181127d** (18 mg, yield 75%). LCMS [M+H]⁺ =508.5

### Step 4: UB-181127e(V2714-052)

**UBI-181127d** (500 mg, 0.984 mmol) and triethylamine (149 mg, 1.476 mmol) were dissolved in DCM(10 mL), methanesulfonyl chloride (145 mg, 1.28 mmol) was slowly added. After reacting at room temperature for 1 hour, the reaction was extracted with dichloromethane (50 mL×3), the combined organic layers was washed with brine (50 mL), the organic phase was dried over anhydrous Na2SO4, concentrated under reduced pressure, purified by flash chromatographic column chromatography (DCM:MeOH=10:1) to obtain **UB-181127e** (560 mg, yield 97%). LCMS [M+H]⁺ =586.7

### Step 5: UB-181127f(V2714-068)

**UB-181127e** (3 g, 5 mmol) and sodium azide (0.43 g, 7 mmol) were mixed and dissolved in DMF(10 ml), the mixture was stirred at 85°C overnight, after completion of the reaction, the reaction was diluted with H2O (300 ml) and extracted with ether (2x150 ml). The organic phase was washed with H₂O (3×100 ml) and brine (1×100 ml), dried over MgSO₄, filtered, and the solvent was removed under reduced pressure to obtain **UB-181127f** (2.7 g, yield 98%). LCMS [M+H]⁺ =533.6

### Step 6: UB-181127g(V2714-069)

Compound **UB-181127f** (6g, 18 mmol), and aqueous solution of sodium hydroxide (4N, 10 mL) were sequentially added into water (50 mL), and reacted at 30 ° C for 12 hours. After the completion of the reaction, it was concentrated and the aqueous phase was acidified to pH=5 using hydrochloric acid (1M). Then it was extracted with dichloromethane (10ml*3), the combined organic layer was dried over anhydrous Na₂SO₄, and concentrated to obtain compound **UB-181127g** (3.5 g, yield 81%). LCMS [M+H]⁺ = 339.4

### Step 7: UB-181127h(V2714-071)

**UB-181127g** (100 mg, 0.296 mmol) was dissolved in DMF (10 mL), NaH(60%, 76.5 mg) and 3-bromopropyne (51 mg, 0.385 mmol) were added, the mixture was stirred overnight. Water (20 mL) was added, the mixture was extracted by adding CH2Cl2, the organic layer was washed with saturated NaHCO3 solution. The mixture was dried over Na2SO4, concentrated under reduced pressure to obtain crude product, which was purified by flash chromatography (DCM/MeOH=10/1) to obtain product **UB-181127h**(35 mg, yield 30%). LCMS [M+H]⁺ =377.5

### Step 8: UB-181127i(V2714-072)

Compound **UBI-181127h** (30 mg, 0.08 mmol) and 3-(5-iodo-1-oxo-2,3-dihydro-1H-isoindol-2-yl)piperidine-2,6-dione (29 mg, 0.08 mmol) were dissolved in DMF (10 mL), dichlorobis(triphenylphosphonium)palladium (7.2 mg,0.010 mmol), cuprous iodide (3.91 mg, 0.021 mmol ) and triethylamine (13 ul, 0.09mmol) were added, the mixture was reacted at 80°C overnight. The reaction solution was filtered through Celite, and the filtrate was concentrated to obtain crude product, which was purified by flash chromatography (eluted with DCM/MeOH=0% to 20% for 30 minutes) to obtain product

### UB-181127i (27 mg, yield53%.) LCMS [M+H]⁺ =619.7

### Step 9: UB-181127j(V2714-073)

**UB-181127i** (50 mg, 0.081 mmol) was dissolved in THF(10 mL), then trimethylphosphine (0.5mL, 1M) was added dropwise. The solvent was removed under reduced pressure, the mixture was concentrated in vacuum to obtain crude product, which was purified by TLC(DCM/MeOH=10/1) to obtain product **UB-181127j**(45 mg, yield 96%, as a white solid. LCMS [M+H]⁺ =593.3

### Step 10: UB-181127(V2714-074)

### The method is similar to General Method 4

¹H NMR (400 MHz, DMSO-*d*₆)δ11.74 (s, 1H), 11.00 (s, 1H), 9.51 (s, 1H), 8.83-8.58 (m, 4H), 8.21 (s, 1H), 7.64-7.42 (m, 8H), 7.45 - 6.78 (m, 2H), 6.49 (s, 1H), 5.17 (dd, *J=* 13.3, 5.1 Hz, 1H), 4.54-4.36 (m, 5H), 3.29 - 3.03 (m, 6H), 3.00 - 2.74 (m, 4H), 2.70 - 2.51 (m, 3H),2.51-2.37(m, 3H), 2.10-1.97(m,5H),1.95-1.75(m, 2H), 1.49-1.35 (m, 4H), 1.49-1.35 (m, 4H). LCMS [M+H]⁺ = 956.8

### Synthesis method of Compound UB-181131

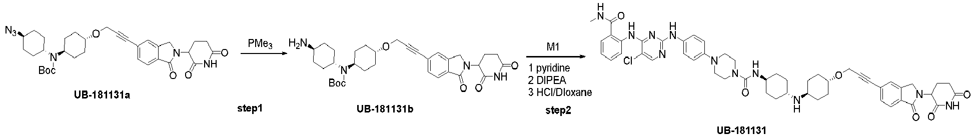

### Step 1: UB-181131b(V2714-073)

**UB-181131a**(50 mg, 0.0 81 mmol) was dissolved in THF(10 mL), then trimethylphosphine (0.5mL, 1M) was added dropwise. The solvent was removed under reduced pressure, the mixture was concentrated in vacuum to obtain crude product, which was purified by TLC(DCM/MeOH=10/1) to obtain product **UB-181131b**(45 mg, yield 96%) as a white solid. LCMS [M+H]⁺ =593.3

### Step 2: UB-181131(V2714-075)

### The method is similar to General Method 4

LCMS [M+H]⁺ = 956.8

### Synthesis method of Compound UB-181137

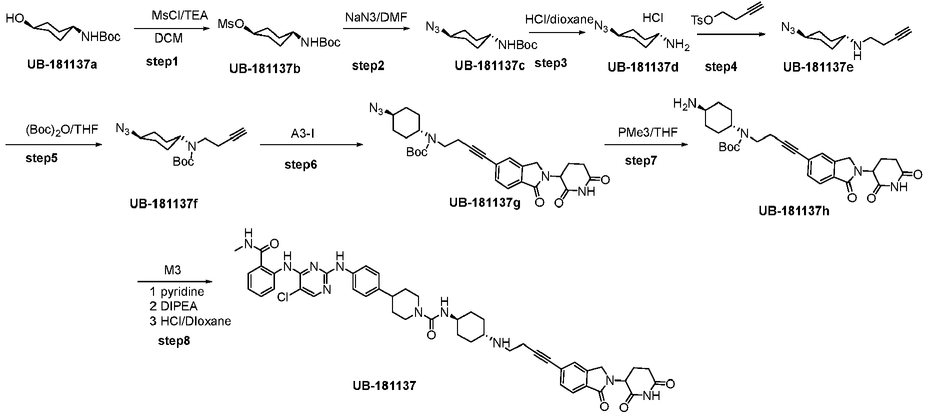

### Step 1: UB-181137b(V2714-018)

**To** a solution of **UB-181137a** (10 g, 22 mmol) and triethylamine (7.05 g, 70 mmol) in dichloromethane (10 ml) was added methanesulfonyl chloride (6.89 g, 60 mmol) dropwise, and the mixture was stirred overnight at room temperature. After completion of the reaction, to the mixture was added water (10ml) and extracted with DCM (10ml*3). The organic layer was dried over Na₂SO₄ and concentrated to obtain **UB-181137b** as white solid (13g, yield 98%.) LCMS [M+H]⁺ =294.3

### Step 2: UB-181137c(V2714-019)

**UB-181137b** (13 g, 44 mmol) and sodium azide (3.75 g, 58 mmol) were mixed and dissolved in DMF(10 ml), the mixture was stirred at room temperature overnight, after completion of the reaction, the reaction was diluted with H2O (300 ml) and extracted with ether (2x150 ml). The organic phase was washed with H₂O (3×100 ml) and brine (1×100 ml), dried over MgSO₄, filtered, and the solvent was removed under low pressure to obtain product **UB-181137c** (9 g, yield 88%) LCMS [M+H]⁺ =241.3

### Step 3: UB-181137d(V2714-020)

Compound **UB-181137c** (10 g, 0.042 mmol) and hydrochloric acid in dioxane (100 mL, 4 N) were added to tetrahydrofuran (10mL), the mixture was reacted at room temperature for 2 hours. After the completion of the reaction, the mixture was concentrated by rotary evaporation under reduced pressure to obtain compound **UB-181137d** (5.8 g, yield 99%). LCMS [M+H]⁺ =141.3

### Step 4: UBI-181137e(V2714-027)

### Compound UB-181137d (1.0g, 5.68 mmol), 3-butynyl p-toluenesulfonate

(1.27 g, 5.68 mmol) and triethylamine (6.06 g, 60mmol) were mixed, then dissolved in toluene (20 mL), the mixture was reacted at 80°C for 18 hours, filtered after the completion of the reaction, the filtrate was concentrated by rotary evaporation under reduced pressure, and isolated by silica gel column chromatography (DCM/MeOH =10/1) to obtain **UBI-181137e** (818 mg, yield 75 %) as a colorless oil. LCMS [M+H]⁺ =193.3

### Step 5: UB-181137f (V2714-032)

**Compound UB-181137e** (350 mg, 1.82 mmol), di-tert-butyl dicarbonate (441 mg, 2.03 mmol) and sodium bicarbonate (360 mg, 4.29mmol) were added to tetrahydrofuran (20mL) successively and reacted at room temperature for 2 hours. After the completion of the reaction, the reaction was poured into 10 mL of water and extracted with dichloromethane (5 mL*3). The organic phases were combined, then washed with saturated brine, dried over anhydrous Na₂SO₄, and concentrated by rotary evaporation under reduced pressure to obtain compound **UB-181137f** (463 mg, yield 71%) as a colorless oil. LCMS [M+H]⁺ =293.3

### Step 6: UB-181137g (V2714-033)

Compound **UBI-181137f** (30 mg, 0.103 mmol) and A3-I (38 mg, 0.103 mmol) were dissolved in DMF (10 mL), dichlorobis(triphenylphosphonium)palladium (7.2 mg,0.010 mmol), cuprous iodide (3.91 mg, 0.021 mmol) and triethylamine (150 mg, 1.49 mmol) were added, the mixture was reacted at 80°C overnight under nitrogen. The reaction solution was filtered through Celite, and the filtrate was concentrated to obtain crude product, which was purified by fast chromatography (eluted with DCM/MeOH=0% to 20% for 30 minutes) to obtain product **UB-181137g** (9 mg, yield 17%). LCMS [M+H]⁺ =535.5

### Step 7: UB-181137h(V2714-034)

**UB-181137g** (1 g, 1.87 mmol) was dissolved in THF(10 mL), trimethylphosphine (402 mg, 1.87 mmol) was added. The mixture was reacted at room temperature overnight, concentrated after completion of the reaction to obtain crude product, which was purified by flash chromatography (DCM/MeOH=10/1) to obtain product **UB-181137h**(510 mg, yield 91%). LCMS [M+H]⁺ =509.5

### Step 8: UB-181137(V2714-074)

### The method is similar to General Method 4

¹H NMR (400 MHz, DMSO-*d*₆)δ11.74 (s, 1H), 11.00 (s, 1H), 9.46 (s, 1H), 9.45-9.38 (m, 5H), 8.86-8.68 (m, 2H), 8.21 (s, 1H), 7.64-7.42 (m, 7H), 7.14 (t, *J=* 7.7 Hz, 3H), 6.27 (d, *J* = 7.5 Hz, 1H), 5.12 (dd, *J* = 13.2, 5.1 Hz, 1H), 4.67 - 4.34 (m, 2H), 4.34 (d, *J* = 7.6 Hz, 1H), 4.10 (d, *J* = 12.0 Hz, 2H), 3.83 - 3.47 (m, 4H), 3.35 -3.20 (m, 6H), 2.96 - 2.85 (m, 2H), 2.85 - 2.65 (m, 6H), 2.60 (d, *J* = 12.9 Hz, 3H), 2.55 - 2.47 (m, 1H), 2.43 (dd, *J* = 13.2, 4.5 Hz, 1H), 2.39 - 1.97 (m, 5H), 1.97-1.60 (m, 6H), 1.65 - 1.22 (m, 2H). LCMS [M+H]⁺ = 871.8

### Synthesis method of Compound UB-181138

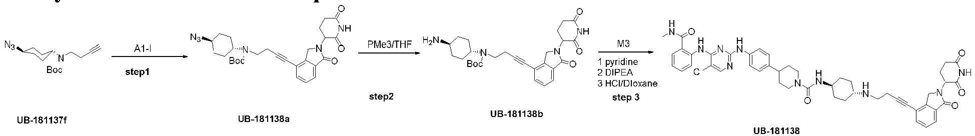

### Step 1: UB-181137g (V2714-093)

Compound **UBI-181137f** (30 mg, 0.103 mmol) and A1-I (38 mg, 0.103 mmol) were dissolved in DMF (10 mL), dichlorobis(triphenylphosphonium)palladium (7.2 mg,0.010 mmol), cuprous iodide (3.91 mg, 0.021 mmol) and triethylamine (150 mg, 1.49 mmol) were added, the mixture was reacted at 80°C overnight. The reaction solution was filtered through Celite, and the filtrate was concentrated to obtain crude product, which was purified by fast chromatography (eluted with DCM/MeOH=0% to 20% for 30 minutes) to obtain product **UB-181138a** (9 mg, yield 17%). LCMS [M+H]⁺ =535.2

### Step 2: UB-181138b(V2714-094)

**UB-181138a** (1 g, 1.87 mmol) was dissolved in THF(10 mL), trimethylphosphine (402 mg, 1.87 mmol) was added, the mixture was reacted at room temperature overnight, concentrated after completion of the reaction to obtain crude product, which was purified by rapid chromatography (DCM/MeOH=10/1) to obtain product **UB-181138b**(510 mg, yield 91%). LCMS [M+H]⁺ =509.6

### Step 3: UB-181138(V2714-095)

### The method is similar to General Method 4

¹H NMR (400 MHz, DMSO-*d*₆)δ11.81(s, 1H), 11.03 (s, 1H), 9.66 (s, 1H), 9.20 (s, 2H), 8.86-8.68 (m, 2H), 8.21 (s, 1H), 7.84-7.42 (m, 8H), 7.14 (t, *J* = 7.7 Hz, 3H), 6.27 (d, *J* = 7.5 Hz, 1H), 5.12 (dd, *J* = 13.2, 5.1 Hz, 1H), 4.67 - 4.34 (m, 2H), 4.34 (d, *J* = 7.6 Hz, 1H), 4.10 (d, *J* = 12.0 Hz, 2H), 3.35 -3.20 (m, 2H), 3.10 - 2.85 (m, 4H), 2.85 - 2.55 (m, 8H), 2.55 - 2.47 (m, 1H), 2.41 (d, *J=* 12.9 Hz, 2H), 2.10 (dd, *J* = 3.2, 1.8 Hz, 2H), 1.90 (ddd, *J* = 6.4, 5.8, 9.4 Hz, 2H), 1.46 (dd, *J* = 2.1, 1.7 Hz, 4H), 1.25 (d, *J* = 5.4 Hz, 2H). LCMS [M+H]⁺ = 871.8

### Synthesis method of Compound UB-181146

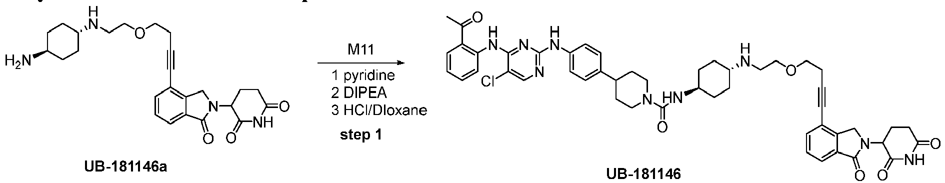

### Step 1: UB-181146(V2714-113)

### The method is similar to General Method 4

¹H NMR (400 MHz, DMSO-*d*₆)δ11.84 (s, 1H), 11.02 (s, 1H), 9.64 (s, 1H), 8.85 (s, 3H), 8.30 (s, 1H), 8.13 (d, *J* = 7.9 Hz, 1H), 7.73 (d, *J* = 7.5 Hz, 1H), 7.70 - 7.38 (m, 5H), 7.31 - 7.10 (m, 3H), 6.23 (s, 1H), 5.17 (dd, *J=* 13.1, 4.8 Hz, 1H), 4.47 (d, *J=* 8.0 Hz, 1H), 4.32 (d, *J* = 7.7 Hz, 1H), 4.23-4.15 (m, 2H), 3.91 - 3.60 (m, 2H), 3.10 (s, 2H), 3.08 (d, *J* = 8.4 Hz, 2H), 3.02 - 2.5(m, 11H), 2.45 (s, 1H), 2.04 (dd, *J* = 2.1, 8.1 Hz, 3H), 1.79 (dd, *J* = 5.2, 11.2 Hz, 2H), 1.73 (d, *J=* 12.0 Hz, 2H), 1.84 - 1.36 (m, 4H), 1.33 - 1.13 (m, 3H),LCMS [M+H]⁺ = 871.8

### Synthesis method of Compound UB-181147

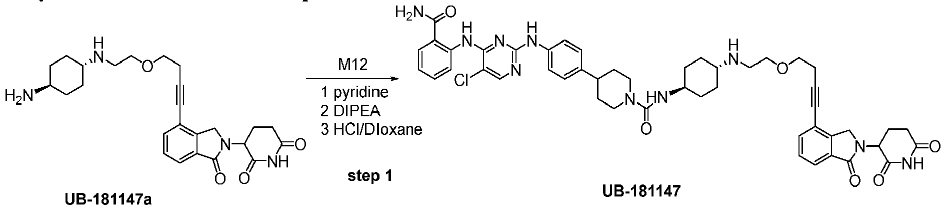

### Step 1: UB-181147(V2714-114)

### The method is similar to General Method 4

¹H NMR (400 MHz, DMSO-*d*₆)δ11.92 (s, 1H), 11.02 (s, 1H), 9.46 (s, 1H), 8.81-8.65 (m, 3H), 8.27 (d, *J=* 3.3 Hz, 2H), 7.86 - 7.49 (m, 9H), 7.14 (t, *J=* 8.7 Hz, 1H),6.23 (s, 1H), 5.17 (dd, *J=* 13.1, 4.8 Hz, 1H), 4.47 (d, *J=* 8.0 Hz, 1H), 4.32 (d, *J=* 7.7 Hz, 1H), 4.23-4.15 (m, 2H), 3.91 - 3.60 (m, 4H), 3.45 - 3.05 (m, 4H), 3.11 - 2.51 (m, 8H), 2.03 (d, *J=* 6.3 Hz, 4H), 2.24 - 1.86 (m, 2H), 1.84 (s, 2H), 1.73 (d, *J* = 12.0 Hz, 2H), 1.39 (dd, *J* = 4.8, 6.1 Hz, 3H), 1.25 (dt, *J=* 15.5, 8.4 Hz, 2H).LCMS [M+H]⁺ = 901.8

### Synthesis method of Compound UB-181151

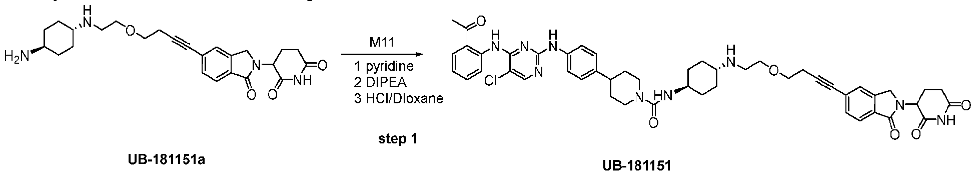

### Step 1: UB-181151(V2714-117)

### The method is similar to General Method 4

¹H NMR (400 MHz, DMSO-*d*₆)δ 11.82 (s, 1H), 11.00 (s, 1H), 9.52 (s, 1H), 8.95 (s, 1H), 8.69 (s,2H), 8.28 (s, 1H), 8.13 (d, *J* = 8.0 Hz, 1H), 7.76 - 7.65 (m, 2H), 7.65 - 7.44 (m, 4H), 7.28 - 7.08 (m, 3H), 6.24 (s, 1H), 5.11 (dd, *J=* 13.1, 5.2 Hz, 1H), 4.45 (d, *J* = 6.7 Hz, 1H), 4.33 (d, *J=* 7.4 Hz, 1H), 3.88- 3.69 (m, 4H), 2.88 (ddd, *J* = 6.7, 7.9, 9.2 Hz, 5H), 2.78 (dd, *J=* 7.9, 1.3 Hz, 3), 2.83 - 1.52 (m, 3H), 2.39 (d, *J* = 13.3 Hz, 8H), 2.31 (d, *J* = 9.9 Hz, 3H), 2.11 (s, 1H), 2.44 - 1.52 (m, 3H), 2.11 - 1.60 (m, 5H), 1.49 - 1.31 (m, 7H). LCMS [M+H]⁺ = 900.8

### Synthesis method of Compound UB-181152

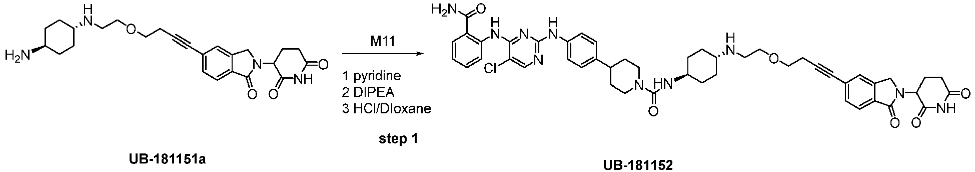

### Step 1: UB-181152(V2714-118)

### The method is similar to General Method 4

¹H NMR (400 MHz, DMSO-*d*₆)δ 12.00 (s, 1H), 11.07 (s, 1H), 9.55 (s, 1H), 8.85 (s, 1H), 8.79 (s,2H), 8.28 (s, 1H), 8.13 (d, *J* = 8.0 Hz, 1H), 7.76 - 7.65 (m, 5H), 7.65 - 7.44 (m, 5H), 7.28 - 7.08 (m, 3H), 6.24 (s, 1H), 5.11 (dd, *J=* 13.1, 5.2 Hz, 1H), 4.45 (d, *J* = 6.7 Hz, 1H), 4.33 (d, *J=* 7.4 Hz, 1H), 3.88- 3.69 (m, 4H), 3.27 (s, 2H), 3.09 - 2.51 (m, 9H), 2.36 (d, *J=* 6.7 Hz, 1H), 2.50 - 2.03 (m, 4H), 1.96 (dd, *J=* 5.3, 3.3 Hz,2H), 1.65 (d, *J=* 10.1 Hz, 2H), 1.48 (d, *J=* 11.7 Hz, 3H), 1.38 - 1.21 (m, 2H). LCMS [M+H]⁺ = 900.8

### Synthesis method of Compound UB-181168

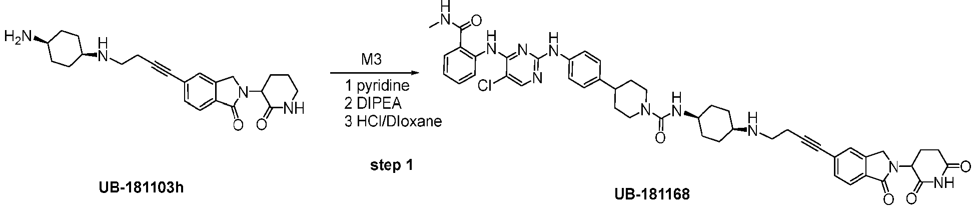

### Step 1: UB-181168(V2714-138)

### The method is similar to General Method 4

¹H NMR (400 MHz, DMSO-*d*₆)δ11.75 (s, 1H), 11.00 (s, 1H), 9.57 (s,1H), 9.01 - 8.77 (m, 2H), 8.71 (d, *J=* 7.4 Hz, 2H), 8.22 (s 1H), 7.82 - 7.68 (m, 3H), 7.68 - 7.51 (m, 3H), 7.47 (t, *J* = 7.9 Hz, 1H), 7.17 (dd, *J* = 7.5, 5.4 Hz, 3H), 5.93 (s, 1H), 5.11 (dd, *J* = 13.3, 5.1 Hz, 1H), 4.46 (d, *J=* 7.7 Hz, 1H), 4.33 (d, *J=* 7.7 Hz, 1H),3.68 (s, 1H), 3.19 (d, *J=* 7.7 Hz, 3H), 3.21 - 2.90 (m, 3H), 2.90 - 2.49 (m, 7H), 2.37 (ddd, *J* = 6.2, 10.5, 7.3 Hz, 2H), 2.04 - 1.99 (m, 2H), 1.83 (dd, *J* = 2.7, 9.1 Hz, 1H), 1.65 (d, *J* = 4.4 Hz, 8H), 1.57 - 1.27 (m, 4H).LCMS [M+H]⁺ = 871.8.

### Synthesis method of Compound UB-181169

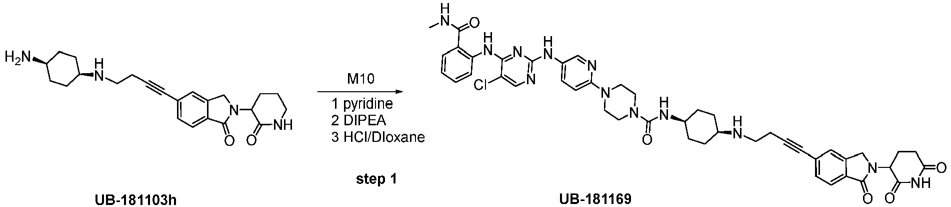

### Step 1: UB-181169(V2714-139)

### The method is similar to General Method 4

¹H NMR (400 MHz, DMSO-*d*₆)δ 12.25 (s, 1H), 11.00 (s, 1H), 9.27 - 8.76 (m, 4H), 8.46 (d, *J=* 8.1 Hz, 1H), 8.15 - 7.99 (m, 1H), 7.98 - 7.82 (m, 3H), 7.81 - 7.76 (m, 3H), 7.76 - 7.69 (m, 3H), 7.32 - 6.97 (m, 2H), 6.13 (s, 1H), 5.12 (dd, *J* = 13.3, 5.0 Hz, 1H), 4.46 (d, *J* = 7.6 Hz, 1H), 4.34 (d, *J=* 7.6 Hz, 1H), 3.68 - 3.27 (m, 8H), 3.08 (dd, *J* = 9.4, 7.1 Hz, 4H), 2.96 - 2.84 (m, 3H), 2.96 - 2.84 (m, 3H), 2.96 - 2.71 (m, 3H), 2.69 (d, *J* = 13.2 Hz, 1H), 2.60 (d, *J* = 7.4 Hz, 2H), 2.27 (d, *J=* 8.6 Hz, 2H), 2.02 - 1.68 (m, 6H), 1.68 - 1.21 (m, 3H).

LCMS [M+H]⁺ = 909.8

### Synthesis method of Compound UB-181174

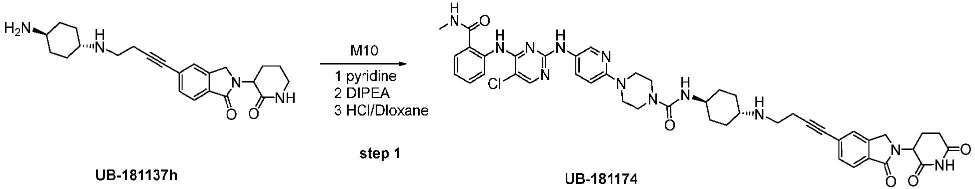

### Step 1: UB-181174(V2714-146)

### The method is similar to General Method 4

¹H NMR (400 MHz, DMSO-*d*₆)δ 12.32 (s, 1H), 11.00 (s, 1H), 9.27 - 8.76 (m, 2H), 8.46 (d, *J=* 8.1 Hz, 1H), 8.15 - 7.99 (m, 1H), 7.98 - 7.82 (m, 2H), 7.81 - 7.76 (m, 3H), 7.76 - 7.69 (m, 3H), 6.82 - 6.67 (m, 1H), 6.43 (s, 1H), 5.12 (dd, *J* = 13.3, 5.0 Hz, 1H), 4.46 (d, *J* = 7.7 Hz, 1H), 4.34 (d, *J=* 7.7 Hz, 1H), 3.68 - 3.57 (m, 9H), 3.08 (dd, *J* = 9.4, 7.1 Hz, 2H), 2.96 - 2.84 (m, 3H), 2.81 - 2.71 (m, 1H), 2.27 (d, *J=* 8.6 Hz, 2H), 2.02 - 1.68 (m, 4H), 1.68 - 1.21 (m, 11H). LCMS [M+H]⁺ = 873.8

### Synthesis method of Compound UB-181182

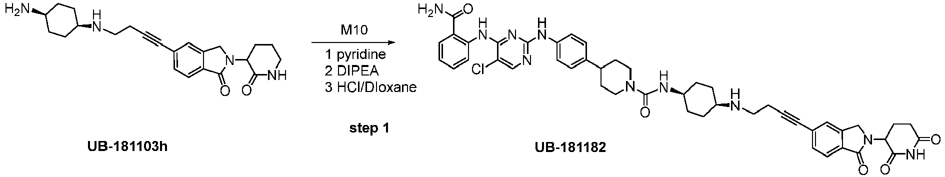

### Step 1: UB-181182(V2962-021)

### The method is similar to General Method 4

¹H NMR (400 MHz, DMSO-*d*₆)δ 12.02 (s, 1H), δ 11.01 (s, 1H), 9.55 (s, 1H), 8.85 (s, 1H), 8.79 (s,2H), 8.28 (s, 1H), 8.13 (d, *J* = 8.0 Hz, 1H), 7.76 - 7.65 (m, 5H), 7.65 - 7.44 (m, 5H), 7.28 - 7.08 (m, 3H), 6.24 (s, 1H), 5.11 (dd, *J=* 13.1, 5.2 Hz, 1H), 4.45 (d, *J* = 6.7 Hz, 1H), 4.33 (d, *J=* 7.4 Hz, 1H), 3.88- 3.69 (m, 4H), 3.27 (s, 2H), 3.09 - 2.51 (m, 9H), 2.36 (d, *J=* 6.7 Hz, 1H), 2.50 - 2.03 (m, 4H), 1.96 (dd, *J=* 5.3, 3.3 Hz,2H), 1.65 (d, *J=* 10.1 Hz, 2H), 1.48 (d, *J=* 11.7 Hz, 3H), 1.38 - 1.21 (m, 2H).

LCMS [M+H]⁺ = 858.3

### Synthesis method of Compound UB-181183

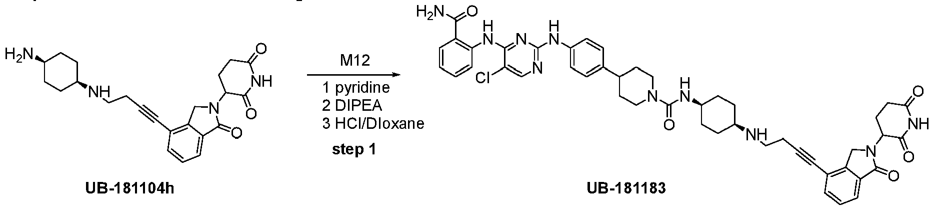

### Step 1: UB-181183(V2962-023)

### The method is similar to General Method 4

LCMS [M+H]⁺ = 857.3

### Synthesis method of Compound UB-181189

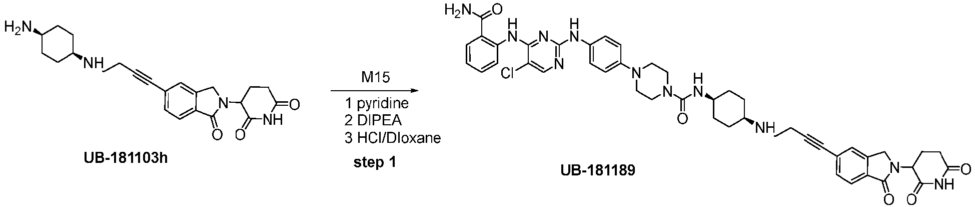

### Step 1: UB-181189(V2962-030)

### The method is similar to General Method 4

LCMS [M+H]⁺ = 859.4

### Synthesis method of Compound UB-181190

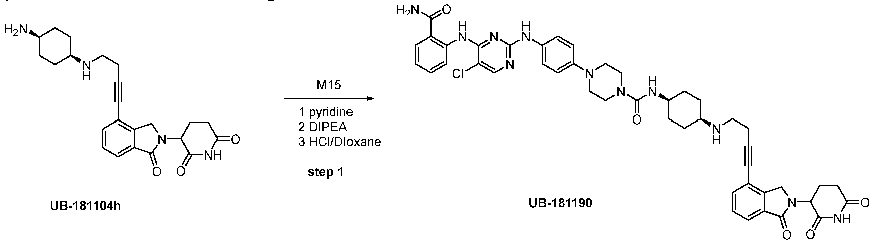

### Step 1: UB-181190(V2962-031)

### The method is similar to General Method 4

LCMS [M+H]⁺ = 859.4

### Synthesis method of Compound UB-180943

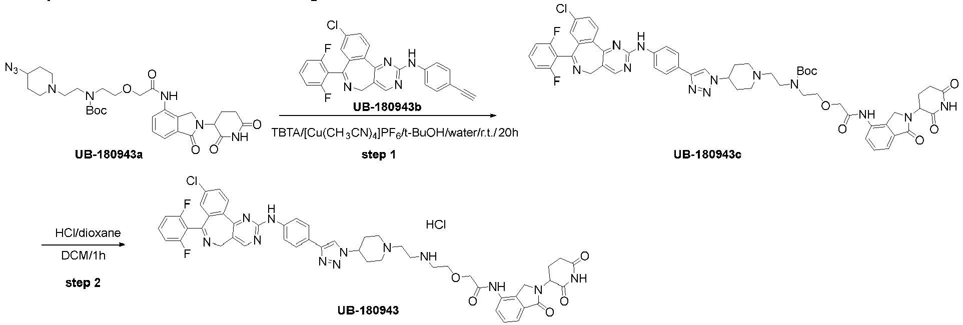

### Step 1: UB-180943c (V2240-015)

Compound **UB-180943b** (35 mg, 0.076 mmol), and **UB-180943a** (70 mg, 0.11 mmol) were dissolved in t-BuOH (5 mL), water (2.5 mL) was added with TBTA (3 mg) and [Cu(CH₃CN)₄]PF₆ (4 mg), the mixture was reacted at room temperature overnight. Water (15 mL) was added, the mixture was extracted with EtOAc (10 mL*2), the organic phases were combined and concentrated. The crude product was purified by thin layer chromatography (dichloromethane /methanol =15/1) to obtain target product **UB-180943c** (45 mg, yield 55%) as a yellow solid. LCMS [M+H]⁺ = 1069.3

### Step 2: UB-180943 (V2240-020)

Compound **UB-180943c** (45 mg, 0.042 mmol) was dissolved in DCM (3 mL), HCl/dioxane (1.5 mL) was added, and the mixture was reacted at room temperature for 1 hour. The reaction supernatant was removed, the solid was dried to obtain target product **UB-180943** (33 mg, yield 80%) as a yellow solid. LCMS [M+H]⁺ = 969.7

¹H NMR (400 MHz, DMSO-*d*₆) δ 11.02 (s, 1H), 10.03 (m, 2H), 9.47 (m, 2H), 8.70 (s, 1H), 8.56 (s, 1H), 8.31 (d, *J=* 8.5 Hz, 1H), 7.95 - 7.73 (m, 6H), 7.63 - 7.50 (m, 3H), 7.34 (d, *J*= 2.2 Hz, 1H), 7.17 (t, *J=* 9.0 Hz, 2H), 5.16 (dd, *J=* 13.3, 5.1Hz, 1H), 4.90 (d, *J=* 35.8 Hz, 2H), 4.50 (d, *J* = 17.6 Hz, 1H), 4.39 (s, 1H), 4.24 (s, 2H), 3.85 (d, *J* = 18.4 Hz, 4H), 3.31 (s, 4H), 2.97 - 2.89 (m, 1H), 2.68 - 2.57 (m, 4H), 2.45 - 2.33 (m, 3H), 2.07 - 1.96 (m, 2H).

### Synthesis method of Compound UB-180944

### Step 1: UB-180944 (V2240-022)

### See general formula 3 for Synthesis method

¹H NMR (400 MHz, DMSO-*d*₆) δ 11.01 (s, 1H), 10.01 (s, 1H), 8.96 (s, 2H), 8.69 (s, 1H), 8.56 (s, 1H), 8.30 (d, *J* = 8.5 Hz, 1H), 7.88 (td, *J* = 8.5, 2.1Hz, 3H), 7.80 (d, *J* = 8.6 Hz, 2H), 7.73 (d, *J* = 7.6 Hz, 1H), 7.65 (d, *J* = 7.6 Hz, 1H), 7.53 (dt, *J* = 12.5, 8.0 Hz, 2H), 7.34 (d, *J* = 2.2 Hz, 1H), 7.17 (t, *J* = 8.9 Hz, 2H), 5.15 (dd, *J* = 13.3, 5.1Hz, 1H), 4.63 (t, *J* = 5.0 Hz, 2H), 4.48 (d, *J* = 17.7 Hz, 1H), 4.33 (d, *J* = 17.8 Hz, 1H), 3.93 (t, *J=* 5.1Hz, 2H), 3.75 (t, *J=* 5.1Hz, 2H), 3.19 (s, 4H), 2.93 - 2.86 (m, 3H), 2.59 (d, *J=* 17.6 Hz, 2H), 2.40 (dd, *J=* 13.1, 4.6 Hz, 2H), 2.05 - 1.96 (m, 2H). LCMS [M+H]⁺ = 881.3

### Synthesis method of Compound UB-180945

### Step 1: UB-180945 (V2240-021)

### See general formula 3 for Synthesis method

¹H NMR (400 MHz, DMSO-*d*₆) δ 11.03 (s, 1H), 10.72 (s, 1H), 10.54 (s, 1H), 10.03 (d, *J=* 2.6 Hz, 1H), 9.41 (s, 2H), 8.70 (s, 1H), 8.51 (s, 1H), 8.31 (dd, *J* = 8.5, 1.5 Hz, 1H), 7.94 - 7.76 (m, 6H), 7.59 - 7.49 (m, 3H), 7.34 (d, *J* = 2.1Hz, 1H), 7.17 (t, *J* = 8.9 Hz, 2H), 5.17 (m, 1H), 5.01 - 4.81 (m, 2H), 4.49 (d, *J* = 17.6 Hz, 1H), 4.38 (d, *J* = 17.7 Hz, 1H), 4.10 (d, *J* = 5.0 Hz, 2H), 3.82 (m, 6H), 3.40 (m, 2H), 3.29 (m, 4H), 2.94 (m, 1H), 2.60 (m, 4H), 2.43 (m, 2H), 2.32 - 2.26 (m, 1H), 2.08 - 1.92 (m, 2H). LCMS [M+H]⁺ = 969.3

### Synthesis method of Compound UB-180946

### Step 1: UB-180946 (V2240-030)

### See general formula 3 for Synthesis method

¹H NMR (400 MHz, DMSO-*d*₆) δ 11.01 (s, 1H), 10.00 (t, *J=* 3.7 Hz, 1H), 9.13 (s, 1H), 8.87 (d, *J=* 41.4 Hz, 1H), 8.71 - 8.54 (m, 2H), 8.30 (dd, *J=* 8.5, 2.0 Hz, 1H), 7.93 - 7.85 (m, 3H), 7.85 - 7.78 (m, 2H), 7.74 - 7.63 (m, 2H), 7.60 - 7.49 (m, 2H), 7.33 (d, *J=* 2.2 Hz, 1H), 7.17 (t, *J=* 9.0 Hz, 2H), 5.15 (d, *J=* 13.0 Hz, 1H), 5.03 - 4.90 (m, 1H), 4.62 - 4.44 (m, 2H), 4.33 (d, *J=* 17.8 Hz, 1H), 4.03 - 3.74 (m, 4H), 3.26 - 3.07 (m, 3H), 3.01 - 2.85 (m, 4H), 2.58 (d, *J=* 17.1Hz, 2H), 2.00 (q, *J=* 7.4 Hz, 2H), 1.55 (q, *J=* 6.6, 5.2 Hz, 1H), 1.20 - 1.13 (m, 2H), 1.07 (dd, *J* = 8.8, 6.0 Hz, 1H), 0.85 (t, *J* = 6.5 Hz, 1H). LCMS [M+H]⁺ = 909.5

### Synthesis method of Compound UB-181008

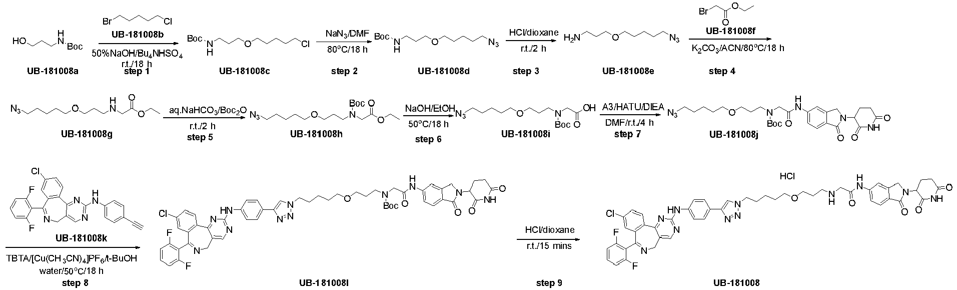

### Step 1: UB-181008c (V2240-116)

Compound **UB-181008a** (3.5 g, 20 mmol), and **UB-181008b** (4 g, 21 mmol), 50% NaOH (4 g, 4 mL water, 100 mmol), Bu₄NHSO₄ (6.7 g, 20 mmol) were added and reacted at room temperature overnight. Water (30 mL) was added, the mixture was extracted with EtOAc (30 mL), the organic phases were combined and concentrated. The crude product was isolated by column chromatography (petroleum ether/ethyl acetate= 0-100%) to obtain target product **UB-181008c** (1.6 g, yield 27%) as a colorless oil. LCMS [M+H]⁺ = 180.1

¹H NMR (400 MHz, Chloroform-*d*) δ 4.85 (s, 1H), 3.53 (t, *J=* 5.9 Hz, 2H), 3.43 (t, *J=* 6.3 Hz, 2H), 3.20 (t, *J=* 6.9 Hz, 2H), 1.82 (m, 4H), 1.77 (m, 2H), 1.68 - 1.58 (m, 2H), 1.44 (s, 9H).

### Step 2: UB-181008d (V2240-119)

Compound **UB-181008c** (1.6 g, 5.7 mmol) and NaN₃ (1.1 g, 17.1 mmol) were dissolved in DMF (20mL), the mixture was reacted at 80°C overnight. The reaction solution was poured to brine (40 mL), the mixture was extracted with EtOAc (30 mL^{∗}2) , the organic phases were combined, then washed with brine, dried over anhydrous sodium sulfate, filtered, and the filtrate was concentrated to obtain target product **UB-181008d** (1.5 g) as a colorless oil. LCMS [M-56]⁺ = 231.2

¹H NMR (400 MHz, Chloroform-*d*) δ 4.85 (s, 1H), 3.48 (t, *J=* 5.9 Hz, 2H), 3.41 (t, *J=* 6.3 Hz, 2H), 3.28 (t, *J=* 6.9 Hz, 2H), 3.22 (q, *J=* 6.0 Hz, 2H), 1.75 (p, *J=* 6.2 Hz, 2H), 1.68 - 1.58 (m, 4H), 1.44 (s, 11H).

### Step 3: UB-181008e (V2240-120)

Compound **UB-181008d** (1.5 g, 5.23 mmol) was dissolved in DCM (10 mL), 4M HCl/dioxane (5 mL) was added, and the mixture was reacted at room temperature for 1 hour. The reaction was concentrated to obtain target product **UB-181008e** (1.1 g, yield 100%) as a yellow oil.

### Step 4: UB-181008g (V2240-122)

**Compound UB-181008e** (500 mg, 2.2 mmol) was dissolved in ACN (30 mL), K₂CO₃ (608 mg, 4.4 mmol), and **UB-181008f(375** mg, 2.2 mmol) were added, the mixture was reacted at 80°C overnight. The reaction was cooled down to room temperature and directly used in the next step. LCMS [M+H]⁺ = 273.4

### Step 5: UB-181008h (V2240-123)

Compound UB-181008g was added to saturated NaHCO₃ (9 mL) and BoczO (9 mL), the mixture was reacted at room temperature overnight. Then the mixture was extracted with EtOAc (15 mL*2), the organic phase was dried over anhydrous sodium sulfate and filtered, the filtrate was concentrated and the crude product was separated by column chromatography (petroleum ether/ethyl acetate= 0-100%) to obtain target product **UB-181008h** (0.5 g, yield 60% for two steps) as a yellow oil. LCMS [M-100]⁺ = 237.

¹H NMR (400 MHz, Chloroform-*d*) δ 3.48 (m, 2H), 3.41 (m, 4H), 3.27 (m, 4H), 3.22 (d, *J* = 6.1 Hz, 2H), 1.76 (m, 2H), 1.64 - 1.58 (m, 6H), 1.44 (s, 9H), 1.28 - 1.23 (m, 3H).

### Step 6: UB-181008i (V2240-124)

Compound **UB-181008h** (500 mg, 1.34 mmol) were dissolved in EtOH (5 mL), 2M NaOH (5 mL) was added, the mixture was reacted at 60°C for 2 hours. Brine (15 mL) was added, the mixture was extracted with Et₂O (20 mL*2). The aqueous phase was acidified to pH~3 with 1M hydrochloric acid, and the mixture was extracted with DCM (20 mL*3) and organic phases were combined and concentrated to obtain product **UB-181008i** (110 mg) as colorless oil. LCMS [M-100]⁺ = 245.3

### Step 7: UB-181008i (V2240-124)

Compound **UB-181008i** (110 mg, 0.32 mmol), **A3** (83 mg, 0.64 mmol), HATU (243 mg, 0.64 mmol), DIPEA (123 mg, 0.96 mmol) were dissolved in DMF (5 mL), the mixture was reacted at room temperature for 3 hours. The reaction solution was concentrated and the crude product was isolated by column chromatography (dichloromethane/methanol = 0 - 10%) to obtain target product (120 mg, yield 64%) as yellow oil. LCMS [M+H]⁺ = 586.7

### Steps 8&9: UB-181008 (V2240-131)

### See general formula 3 for Synthesis method

¹H NMR (400 MHz, DMSO-*d*₆) δ 10.96 (m, 2H), 10.01 (s, 1H), 8.93 (m, 2H), 8.69 (s, 1H), 8.48 (s, 1H), 8.30 (d, *J* = 8.5 Hz, 1H), 7.97 - 7.84 (m, 4H), 7.83 - 7.75 (m, 2H), 7.72 (d, *J* = 8.3 Hz, 1H), 7.65 (d, *J* = 8.4 Hz, 1H), 7.55 (m, 1H), 7.34 (d, *J* = 2.2 Hz, 1H), 7.17 (t, *J* = 8.9 Hz, 2H), 5.09 (dd, *J=* 13.3, 5.1Hz, 1H), 4.48 - 4.29 (m, 4H), 4.01 (t, *J=* 5.8 Hz, 2H), 3.45 (m, 5H), 3.05 (s, 2H), 2.94 - 2.87 (m, 1H), 2.59 (m, 2H), 2.42 - 2.29 (m, 2H), 2.04 - 1.81 (m, 6H), 1.60 - 1.51 (m, 2H), 1.37 - 1.30 (m, 2H). LCMS [M+H]⁺ = 943.0

### Synthesis method of Compound UB-181009

### Step 1: UB-181009 (V2240-132)

### See general formula 3 for Synthesis method

¹H NMR (400 MHz, DMSO-*d*₆) δ 11.22 (s, 1H), 10.98 (d, *J* = 6.1 Hz, 2H), 8.96 (br, 2H), 7.93 (d, *J=* 1.7 Hz, 1H), 7.72 (d, *J=* 8.2 Hz, 1H), 7.70 - 7.62 (m, 2H), 7.43 - 7.33 (m, 2H), 7.21 - 7.08 (m, 3H), 6.97 (d, *J=* 3.6 Hz, 1H), 6.82 (d, *J=* 8.2 Hz, 1H), 6.07 (d, *J=* 7.3 Hz, 1H), 5.09 (dd, *J=* 13.3, 5.1 Hz, 1H), 4.57 - 4.42 (m, 2H), 4.31 (d, *J=* 17.4 Hz, 1H), 4.21 (t, *J* = 6.9 Hz, 2H), 4.03 (m, 2H), 3.46 (m, 2H), 3.07 (m, 2H), 2.95 (m, 3H), 2.59 (m, 2H), 2.38 (m, 1H), 2.13 - 1.79 (m, 10H), 1.70 (m, 2H), 1.59 - 1.45 (m, 4H), 1.17 (m, 2H). LCMS [M+H]⁺ = 928.0

### Synthesis method of Compound UB-181017

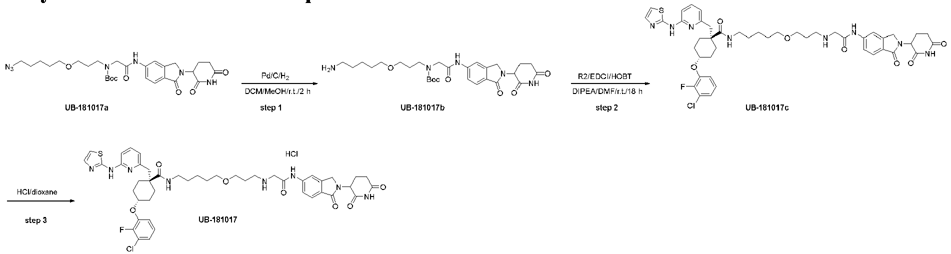

### Step 1: UB-181017b (V2240-149)

Compound **UB-181017a** (50 mg, 0.08 mmol) was dissolved in MeOH (3 mL) and DCM (15 mL), Pd/C (20 mg) was added, the mixture was reacted at room temperature for 2 hours under H₂ protection. The mixture was filtered, the filtrate was concentrated to obtain crude product **UB-181017b** (40 mg) as a yellow oil. LCMS [M+H]⁺ = 560.6

### Step 3: UB-181017 (V2531-004)

### See general formula 2 for Synthesis method

¹H NMR (400 MHz, DMSO-*d*₆) δ 11.3 (m, 2H),10.99 (m, 2H), 8.98 (m, 2H), 7.93 (s, 1H), 7.79 - 7.52 (m, 3H), 7.39 (m, 1H), 7.20 - 7.08 (m, 2H), 7.04 - 6.83 (m, 2H), 6.64 (d, *J=* 8.5 Hz, 1H), 5.09 (dd, *J=* 13.3, 5.1 Hz, 1H), 4.55 (m, 1H), 4.45 (d, *J=* 17.4 Hz, 1H), 4.32 (d, *J* = 17.4 Hz, 1H), 4.03 (m, 2H), 3.44 (m, 4H), 3.38 (m, 2H), 3.32 (m, 2H), 3.02 (m, 4H), 2.90 (m, 3H), 2.59 (m, 2H), 2.42 - 2.32 (m, 1H), 2.07 - 1.55 (m, 10H), 1.42 (m, 4H). LCMS [M+H]⁺ = 903.9

### Synthesis method of Compound UB-181030

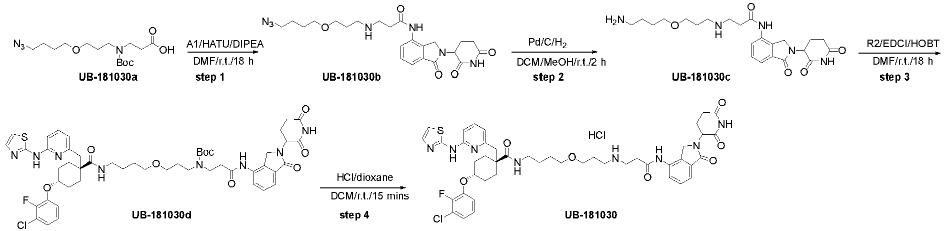

### Step 1: UB-181030b (V2531-009)

Compound **UB-181030a** (250 mg, 0.72 mmol), **A1** (205 mg, 0.79 mmol), HATU (551 mg, 1.45 mmol), and DIPEA (280 mg, 2.16 mmol) were dissolved in DMF (5 mL), the mixture was reacted at room temperature overnight. The reaction solution was concentrated and the crude product was isolated by column chromatography (dichloromethane/methanol = 0 - 10%) to obtain target product **UB-181030b** (240 mg, yield 57%) as a yellow oil. LCMS [M+H]⁺ = 586.7

### Step 2: UB-181030c (V2531-013)

Compound **UB-181030b** (100 mg, 0.17 mmol) was dissolved in MeOH (1 mL) and DCM (15 mL), Pd/C (30 mg) was added, the mixture was reacted at room temperature for 2 hours under H₂ protection. The mixture was filtered, the filtrate was concentrated to obtain crude product **UB-181030c** (95 mg) as colorless oil. LCMS [M+H]⁺ = 560.7

### Step 3&4: UB-181030 (V2531-014)

### See general formula 3 for Synthesis method

¹H NMR (400 MHz, DMSO-*d*₆) δ 11.27 (br, 1H), 11.04 (s, 1H), 10.12 (br, 1H), 8.58 (br, 2H), 7.85 (dd, *J=* 7.4, 1.6 Hz, 1H), 7.64 - 7.48 (m, 4H), 7.38 (m, 1H), 7.18 - 7.08 (m, 3H), 6.99 (m, 1H), 6.89 (m, 1H), 6.63 (m, 1H), 5.17 (dd, *J* = 13.2, 5.1 Hz, 1H), 4.55 (m, 1H), 4.43 (d, *J* = 17.7 Hz, 1H), 4.33 (d, *J=* 17.7 Hz, 1H), 3.23 (m, 4H), 3.03 (m, 4H), 2.92 (m, 3H), 2.84 (m, 2H), 2.62 (m, 2H), 2.27 (m, 1H), 2.02 (m, 2H), 1.93 - 1.74 (m, 8H), 1.64 (m, 2H), 1.42 (m, 4H). LCMS [M+H]⁺ = 903.9

### Synthesis method of Compound UB-181033

### Step 1: UB-181033 (V2531-021)

### See general formula 1 for Synthesis method

¹H NMR (400 MHz, DMSO-*d*₆) δ 10.98 (s, 1H), 10.62 (s, 1H), 10.15 (s, 1H), 8.72 (s, 1H), 8.65 (br, 2H), 8.36 - 8.26 (m, 2H), 7.96 (s, 1H), 7.92 - 7.81 (m, 5H), 7.70 - 7.62 (m, 2H), 7.60 - 7.51 (m, 1H), 7.34 (d, *J=* 2.1 Hz, 1H), 7.17 (t, *J=* 8.8 Hz, 2H), 5.08 (dd, *J=* 13.3, 5.1Hz, 1H), 4.42 (d, *J=* 17.4 Hz, 1H), 4.29 (d, *J=* 17.3 Hz, 1H), 3.48 - 3.38 (m, 6H), 3.24 (dd, *J=* 11.6, 5.8 Hz, 4H), 3.02 (m, 2H), 2.94 - 2.84 (m, 3H), 2.58 (m, 1H), 2.39 - 2.30 (m, 1H), 2.02 - 1.94 (m, 1H), 1.87 (m, 2H), 1.56 (m, 4H). LCMS [M+H]⁺ = 918.7

### Synthesis method of Compound UB-181034

### Step 1: UB-181034 (V2531-022)

### See general formula 1 for Synthesis method

¹H NMR (400 MHz, DMSO-*d*₆) δ 12.29 (br, 1H), 10.98 (s, 1H), 10.73 (d, *J* = 4.3 Hz, 1H), 8.95 (s, 2H), 7.96 (s, 1H), 7.76 - 7.64 (m, 4H), 7.51 (d, *J=* 4.0 Hz, 1H), 7.20 - 7.02 (m, 5H), 6.79 (d, J= 7.1 Hz, 1H), 5.08 (dd, *J=* 13.3, 5.1 Hz, 1H), 4.55 (m, 1H), 4.42 (d, *J* = 17.3 Hz, 1H), 4.29 (d, *J* = 17.3 Hz, 1H), 3.44 (t, *J* = 5.9 Hz, 2H), 3.38 - 3.32 (m, 2H), 3.25 - 3.18 (m, 2H), 3.06 - 2.95 (m, 6H), 2.91 (m, 2H), 2.63 - 2.57 (m, 1H), 2.36 (m, 1H), 2.08 - 1.71 (m, 10H), 1.66 (m, 2H), 1.49 - 1.36 (m, 4H). LCMS [M+H]⁺ = 903.8

### Synthesis method of Compound UB-181042

### Step 1: UB-181042 (V2531-032)

### See general formula 4 for Synthesis method

¹H NMR (400 MHz, DMSO-*d*₆) δ 11.76 (s, 1H), 10.98 (s, 1H), 10.62 (s, 1H), 9.51 (s, 1H), 8.79 (m, 1H), 8.67 (m, 3H), 8.21 (s, 1H), 7.96 (s, 1H), 7.77 (m, 1H), 7.71 - 7.64 (m, 2H), 7.52 (m, 3H), 7.16 (m, 3H), 6.66 (s, 1H), 5.08 (dd, *J* = 13.2, 5.1 Hz, 1H), 4.42 (d, *J* = 17.3 Hz, 1H), 4.31 (d, *J=* 13.8 Hz, 1H), 3.25 (m, 4H), 3.15 (m, 4H), 3.08 - 3.01 (m, 4H), 2.93 - 2.84 (m, 4H), 2.81 (d, *J* = 4.5 Hz, 3H), 2.61 (m, 2H), 2.44 - 2.34 (m, 2H), 2.03 - 1.97 (m, 2H), 1.87 (m, 2H), 1.59 - 1.40 (m, 6H). LCMS [M/2]⁺ =462.7

### Synthesis method of Compound UB-181046

### Step 1: UB-181046 (V2531-032)

### See general formula 4 for Synthesis method

¹H NMR (400 MHz, DMSO-*d*₆) δ 11.96 (s, 1H), 11.03 (s, 1H), 10.27 (s, 1H), 9.99 (s, 1H), 9.00 (m, 2H), 8.87 (m, 1H), 8.63 (m, 1H), 8.30 (s, 1H), 7.91 - 7.79 (m, 2H), 7.66 (d, *J=* 8.5 Hz, 2H), 7.52 (m, 5H), 7.20 (t, *J* = 7.5 Hz, 1H), 6.79 (m, 1H), 5.16 (dd, *J* = 13.3, 5.1 Hz, 1H), 4.47 (d, *J* = 17.6 Hz, 1H), 4.37 (d, *J* = 17.5 Hz, 1H), 3.46 (d, *J* = 6.0 Hz, 2H), 3.38 (m, 6H), 3.22 (t, *J* = 6.3 Hz, 3H), 3.07 (m, 2H), 3.03 - 2.88 (m, 6H), 2.81 (m, 3H), 2.66 - 2.59 (m, 1H), 2.34 (m, 1H), 2.08 - 1.82 (m, 4H), 1.55 - 1.44 (m, 4H). LCMS [M/2]⁺ =462.7

### Synthesis method of Compound UB-181078

### Step 1: UB-181078 (V2531-100)

### See general formula 4 for Synthesis method

¹H NMR (400 MHz, DMSO-*d*₆) δ 11.72 (s, 1H), 11.02 (s, 1H), 9.41 (s, 1H), 8.77 (m, 2H), 8.55 (m, 2H), 8.20 (s, 1H), 7.79 - 7.71 (m, 2H), 7.64 (m, 1H), 7.59 - 7.45 (m, 4H), 7.16 (m, 1H), 7.05 (m, 1H), 6.04 (m, 1H), 5.17 (dd, *J* = 13.3, 5.1 Hz, 1H), 4.46 (d, *J* = 17.7 Hz, 1H), 4.31 (d, *J* = 17.7 Hz, 1H), 3.75 (t, *J* = 5.1 Hz, 2H), 3.71 (t, *J=* 6.7 Hz, 2H), 3.66 (m, 1H), 3.13 (m, 6H), 2.82 - 2.80 (m, 3H), 2.78 (m, 1H), 2.44 (m, 1H), 2.00 (m, 4H), 1.89 - 1.67 (m, 7H), 1.46 (m, 4H). LCMS [M+H]⁺ = 917.0

### Synthesis method of Compound UB-181080

### Step 1: UB-181080 (V2531-104)

### See general formula 4 for Synthesis method

¹H NMR (400 MHz, DMSO-*d*₆) δ 11.84 (s, 1H), 11.00 (s, 1H), 9.76 (s, 1H), 8.76 (m, 4H), 8.25 (m, 1H), 7.79 (m, 1H), 7.70 (m, 1H), 7.63 (m, 3H), 7.52 (m, 2H), 7.42 -7.13 (m, 3H), 6.14 (m, 1H), 5.11 (dd, *J=* 13.3, 5.1 Hz, 1H), 4.45 (d, *J* = 17.6 Hz, 1H), 4.32 (d, *J* = 17.5 Hz, 1H), 3.77 (m, 2H), 3.69 (m, 4H), 3.30 (m, 4H), 3.15 (m, 4H), 2.95 - 2.77 (m, 6H), 2.63 - 2.56 (m, 1H), 2.39 (m, 1H), 2.06 - 1.93 (m, 2H), 1.91 - 1.73 (m, 6H), 1.43 (m, 3H). LCMS [M+H]⁺ = 917.0

### Synthesis method of Compound UB-181091

### Step 1: UB-181091 (V2531-123)

### See general formula 4 for Synthesis method

¹H NMR (400 MHz, DMSO-*d*₆) δ 11.86 (brs, 1H), 11.01 (s, 1H), 9.78 (brs, 1H), 8.84 (m, 3H), 8.68 (m, 1H), 8.27 (s, 1H), 7.79 (m, 2H), 7.74 (m, 1H), 7.56 (m, 4H), 7.36 (m, 2H), 7.18 (t, *J* = 7.5 Hz, 1H), 6.17 (brs, 1H), 5.16 (dd, *J* = 13.3, 5.1 Hz, 1H), 4.54 - 4.45 (m, 3H), 4.36 (d, *J* = 17.8 Hz, 1H), 3.71 - 3.68 (m, 2H), 3.64 (m, 2H), 3.38 (m, 2H), 3.32 - 3.30 (m, 2H), 3.07 (m, 2H), 3.01 - 2.95 (m, 2H), 2.92 (m, 1H), 2.81 (d, *J=* 4.5 Hz, 3H), 2.66 - 2.57 (m, 2H), 2.45 (m, 1H), 2.08 - 1.93 (m, 4H), 1.91 - 1.74 (m, 6H), 1.49 (m, 2H). LCMS [M/2]⁺ = 459.0

### Synthesis method of Compound UB-181096

### Step 1: UB-181096 (V2531-129)

### See general formula 4 for Synthesis method

¹H NMR (400 MHz, DMSO-*d*₆) δ 11.89 (s, 1H), 11.00 (s, 1H), 9.84 (s, 1H), 8.89 - 8.74 (m, 3H), 8.67 (m, 1H), 8.28 (s, 1H), 7.80 (dd, *J* = 8.0, 1.6 Hz, 1H), 7.74 (d, *J* = 7.7 Hz, 2H), 7.69 - 7.56 (m, 3H), 7.56 - 7.30 (m, 3H), 7.22 - 7.17 (m, 1H), 6.19 (s, 1H), 5.12 (dd, *J=* 13.3, 5.1 Hz, 1H), 4.51 - 4.43 (m, 3H), 4.36 (m, 1H), 3.75 - 3.60 (m, 6H), 3.36 (m, 4H), 3.07 (m, 1H), 2.97 (m, 2H), 2.94 - 2.86 (m, 1H), 2.81 (d, *J* = 4.5 Hz, 3H), 2.60 (m, 1H), 2.39 (m, 1H), 2.07 - 1.69 (m, 10H), 1.51 (m, 2H). LCMS [M/2]⁺ = 459.0

### Synthesis method of Compound UB-181172

### Step 1: UB-181172 (V2768-075)

### See general formula 4 for Synthesis method

LCMS [M/2+H]⁺ = 436.5.

¹H NMR: NA

### Synthesis method of Compound UB-181173

### Step 1: UB-181173 (V2768-076)

### See general formula 4 for Synthesis method

¹H NMR (400 MHz, DMSO-*d*₆) δ 12.24 (s, 1H), 11.30 (s, 1H), 11.02 (s, 1H), 9.06 (s, 2H), 8.86 (dd, *J=* 21.1, 6.6 Hz, 2H), 8.38 (s, 1H), 8.00 (s, 1H), 7.92 (d, *J=* 2.8 Hz, 1H), 7.84 (dd, *J* = 7.9, 1.6 Hz, 1H), 7.76 (d, *J* = 7.6 Hz, 1H), 7.71 (d, *J* = 7.5 Hz, 1H), 7.58 (dt, *J* = 15.3, 8.0 Hz, 3H), 7.25 (t, *J* = 7.5 Hz, 1H), 6.11 (d, *J* = 4.5 Hz, 1H), 5.17 (dd, *J* = 13.3, 5.1 Hz, 1H), 4.59 - 4.50 (m, 1H), 4.36 (d, *J* = 17.8 Hz, 1H), 3.73 - 3.66 (m, 2H), 3.53 (t, *J* = 5.1 Hz, 4H), 3.15 (t, *J=* 5.0 Hz, 4H), 2.82 (d, *J=* 4.4 Hz, 3H), 2.58 (s, 1H), 2.45 (d, *J=* 4.4 Hz, 1H), 2.00 (dq, *J=* 13.4, 6.2 Hz, 4H), 1.84 (d, *J=* 8.0 Hz, 6H), 1.59 - 1.43 (m, 4H). LCMS [M/2+H]⁺ = 437.5.

### Synthesis method of Compound UB-181041:

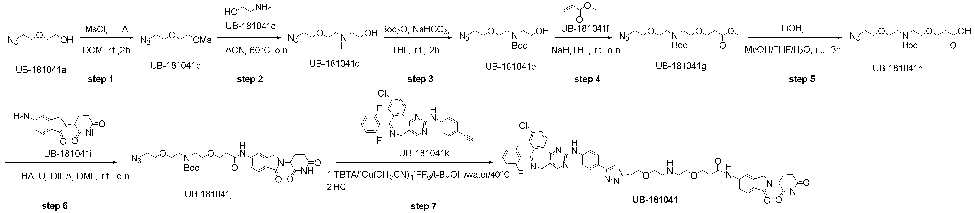

### Step 1: UB-181041b (V2330-142)

To a solution of **UB-181041a** (5 g, 38.17 mmol), and triethylamine (4.63 g, 45.8 mmol) in dry dichloromethane (80 ml) in an ice bath was slowly added methanesulfonyl chloride (5.68 g, 49.6 mmol), and the reaction mixture was stirred at 0°C for 30 min. The ice bath was removed, and the reaction mixture was stirred at room temperature for 2 hours. Water was added with stirring and the resulting mixture was transferred to a partition funnel, the layers were separated, and the aqueous layer was extracted with dichloromethane (100 ml * 3). Combined organic layer was washed with brine, dried over anhydrous sodium sulfate, and concentrated to obtain desired product **UB-181041b** (7.79g, 98% yield) as a colorless oil.

### Step 2: UB-181041d (V2330-144)

Under an ice bath, to **UB-181041b(7.79g,** 37.27mmol)in acetonitrile (30ml) solution was slowly added UB-181041c (3.4g, 55.91mmol), then the reaction mixture was stirred at 60°C overnight. The reaction mixture was concentrated to obtain crude product **UB-181041d,** which was can be used in the next step without purification. LC-MS: [M+H] ⁺ = 175.2

### Step 3: UB-181041e (V2330-145)

To **UB-181041d(6.5g,** 37.3mmol) in tetrahydrofuran (80ml) solution was added saturated sodium bicarbonate aqueous solution (5ml) and di-tert-butyl dicarbonate (11g, 48.5mmol), the reaction was stirred at room temperature for 1 hour. The reaction mixture was concentrated to obtain the crude product. The crude product was purified by silica gel chromatography (petroleum ether/ethyl acetate = 1/1) to give the desired product **UB-181041e** (3.9 g, 38%yield) as a colorless oil. LC-MS: [M+H] ⁺= 275.3

### Step 4: UB-181041g (V2330-148)

Under nitrogen protection, to **UB-181041e(1.5g,** 5.47mmol) in tetrahydrofuran (30mL) solution at 0 °Cwas added sodium hydride (22mg, 0.547mmol), the mixture was stirred at 0°C for 1 hour. Then **UB-181041f** (566 mg, 6.57 mmol) was added to the mixture and the reaction mixture was stirred at room temperature overnight. The reaction mixture was diluted with distilled water/brine and the product was extracted with ethyl acetate three times. Then the combined organic layers were washed with brine and dried over anhydrous sodium sulfate, and filtered. The solvent was removed in vacuum to obtain crude product, which was purified by silica gel chromatography to obtain product **UB-181041g** (456 mg, 51% yield) as a yellow oil. LC-MS: [M+H] ⁺= 361.2

### Step 5: UB-181041h (V2330-149)

To **UB-181041g** (730 mg, 2.028 mmol) in a solution of methanol/water/tetrahydrofuran = 1/3/1 (8 mL) was added lithium hydroxide (128 mg, 3.04 mmol), and the mixture was stirred at room temperature for 3 h. The reaction mixture was poured into water, the mixture was extracted with ethyl acetate, and the pH of the aqueous phase was adjusted to 6. Then the mixture was concentrated to obtain the desired product **UB-181041h** (600 mg, 85% yield) as a colorless oil. LC-MS: [M+H] ⁺= 347.1

### Step 6: UB-181041j (V2591-002)

To **UB-181041h(200mg,** 0.578mmol) and HATU(439mg, 1.16mmol) in N,N-dimethylformamide (20ml) solution was added DIPEA(224mg, 1.73mmol), and the mixture was stirred at room temperature for 1 hour. Then **UB-181041i** (135 mg, 0.52 mmol) was added to the mixture and the mixture was stirred at room temperature for 12 hours. The solvent was removed in vacuum, the crude product was purified via flash chromatography(dichloromethane/methanol = 10/1) to obtain desired product **UB-181041j(100mg,** 29%yield), as a colorless solid. LC-MS: [M+H] ⁺= 588.6

### Step 7: UB-181041 (V2591-004)

### Method similar to General Method 3

¹H NMR (400 MHz, DMSO) δ 10.98 (s, 1H), 10.50 (s, 1H), 10.01 (s, 1H), 8.78 (s, 2H), 8.68 (s, 1H), 8.54 (s, 1H), 8.30 (d, J = 8.5 Hz, 1H), 7.98 (s, 1H), 7.92 - 7.86 (m, 3H), 7.81 (d, J = 8.7 Hz, 2H), 7.65 (s, 2H), 7.58 - 7.51 (m, 1H), 7.33 (d, J = 2.1 Hz, 1H), 7.18 (dd, J = 16.6, 8.0 Hz, 2H), 5.07 (dd, J = 13.3, 5.1 Hz, 1H), 4.59 (t, J = 4.8 Hz, 2H), 4.33 (dd, J = 53.9, 17.3 Hz, 2H), 3.87 (t, J = 4.9 Hz, 2H), 3.69 (ddd, J = 14.7, 9.7, 5.7 Hz, 6H), 3.22 - 3.03 (m, 6H), 2.95 - 2.87 (m, 1H), 2.66 (t, J = 6.2 Hz, 2H), 2.59 (d, J = 16.9 Hz, 1H), 2.38 - 2.31 (m, 1H), 1.99 (d, J = 6.8 Hz, 1H). LC-MS: [M+H] + =944.8

### Synthesis method of Compound UB-181055:

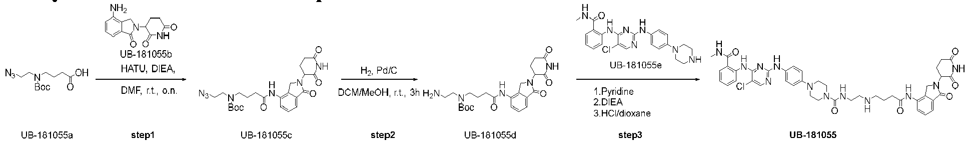

### Step 1: UB-181055c (V2591-007)

To **UB-181055a(150mg,** 0.55mmol) and HATU(419mg, 1.1mmol) in N,N-dimethylformamide (20ml) solution was added DIPEA (213mg,1.65mmol), and the mixture was stirred at room temperature for 1 hour. Then **UB-181055b** (129mg, 0.496mmol) was added to the mixture, the mixture was stirred at room temperature for 12h. The solvent was removed in vacuum, the crude product was purified via flash chromatography(DCM / MeOH = 10/1) to obtain desired product **UB-181055c(146** mg, 52% yield), as a colorless solid.

¹H NMR (400 MHz, DMSO) δ 11.02 (s, 1H), 9.82 (s, 1H), 7.80 (d, *J=* 6.6 Hz, 1H), 7.56 - 7.44 (m, 2H), 5.15 (dd, *J=* 13.3, 5.1 Hz, 1H), 4.37 (q, *J=* 17.5 Hz, 2H), 3.43 - 3.40 (m, 2H), 3.37 (d, *J=* 5.2 Hz, 2H), 3.26 - 3.21 (m, 2H), 2.96 - 2.88 (m, 1H), 2.61 (d, *J=* 16.1 Hz, 1H), 2.34 (d, *J=* 8.4 Hz, 2H), 2.19 (dd, *J=* 16.8, 9.6 Hz, 1H), 2.06 - 1.99 (m, 1H), 1.81 (s, 2H), 1.40 (d, *J=* 1.6 Hz, 9H). LC-MS: [M+H] ⁺ = 514.6

### Step 2: UB-181055d (V2591-017)

To **UB-181055c(30mg,** 0.058mmol) in dichloromethane /methanol (10ml) solution was added palladium/carbon(1.24mg, 0.012mmol), and the reaction mixture was stirred at room temperature for 1 hour under hydrogen condition. The reaction mixture was filtered and concentrated under vacuum to obtain the desired product **UB-181055d** (28 mg, 100%yield), which was a colorless solid. LC-MS: [M+H] ⁺ = 488.5

### Step 3: UB-181055 (V2591-026)

### Method similar to General Method 4

¹H NMR (400 MHz, DMSO) δ 11.95 (s, 1H), 11.02 (s, 1H), 10.10 (s, 1H), 9.98 (s, 1H), 9.07 (s, 2H), 8.87 (d, J = 4.5 Hz, 1H), 8.64 (d, J = 7.4 Hz, 1H), 8.30 (s, 1H), 7.87 - 7.79 (m, 2H), 7.65 (d, J = 7.7 Hz, 2H), 7.58 - 7.45 (m, 5H), 7.20 (t, J = 7.3 Hz, 2H), 5.15 (dd, J = 13.3, 5.1 Hz, 1H), 4.49 - 4.34 (m, 2H), 3.75 (s, 6H), 3.40 (d, J = 6.7 Hz, 4H), 3.02 (d, J = 5.4 Hz, 4H), 2.94 - 2.88 (m, 1H), 2.81 (d, J = 4.5 Hz, 3H), 2.61 (d, J = 17.1 Hz, 1H), 2.55 (d, J = 7.1 Hz, 2H), 2.35 (dd, J = 13.1, 4.4 Hz, 1H), 2.01 (dd, J = 14.1, 6.6 Hz, 3H). LCMS [M+H] ⁺ =851.9

### Synthesis method of Compound UB-181056:

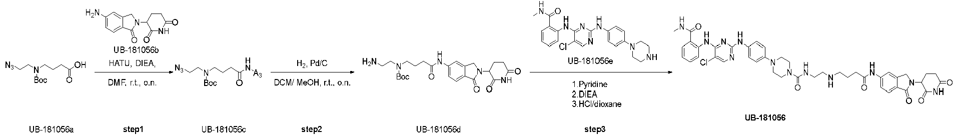

### Step 1: UB-181056c (V2591-008)

To **UB-181056a(150mg,** 0.55mmol) and HATU(419mg, 1.1mmol) in N,N-dimethylformamide (20ml) solution was added DIPEA (213mg,1.65mmol), and the mixture was stirred at room temperature for 1 hour. Then **UB-181056b** (129mg, 0.496mmol) was added to the mixture, and the mixture was stirred at room temperature for 12 hours. The solvent was removed in vacuum, and the crude product was purified via flash chromatography(DCM / MeOH= 10/1) to obtain desired product (143 mg, 51 % yield), as colorless solid.

¹H NMR (400 MHz, DMSO) δ 10.97 (s, 1H), 10.25 (s, 1H), 7.98 (s, 1H), 7.68 - 7.56 (m, 2H), 5.08 (dd, J = 13.3, 5.1 Hz, 1H), 4.35 (dd, J = 56.7, 17.3 Hz, 2H), 3.42 (d, J = 5.0 Hz, 2H), 3.39 - 3.36 (m, 2H), 3.24 (t, J = 7.2 Hz, 2H), 2.96 - 2.87 (m, 1H), 2.62 - 2.54 (m, 1H), 2.35 (t, J = 5.8 Hz, 2H), 2.16 (t, J = 7.2 Hz, 1H), 2.02 - 1.96 (m, 1H), 1.82 (d, J = 6.6 Hz, 2H), 1.39 (d, J = 4.9 Hz, 9H). LC-MS: [M+H] ⁺ = 514.6

### Step 2: UB-181056d (V2591-018)

To **UB-181056c(30mg,** 0.058mmol) in dichloromethane /methanol (10ml) solution was added palladium/carbon(1.24mg, 0.012mmol), and the reaction mixture was stirred at room temperature for 1 hour under hydrogen atmosphere. The reaction mixture was filtered and concentrated under vacuum to obtain the desired product **UB-181056d** (28 mg, 100%yield), which was a colorless solid. LC-MS: [M+H] ⁺ = 488.5

### Step 3: UB-181056 (V2591-027)

### Method similar to General Method 4

¹H NMR (400 MHz, DMSO) δ 11.93 (s, 1H), 10.96 (d, J = 10.1 Hz, 1H), 10.57 (s, 1H), 9.95 (s, 1H), 9.03 (s, 2H), 8.87 (d, J = 4.3 Hz, 1H), 8.64 (d, J = 7.3 Hz, 1H), 8.30 (s, 1H), 8.00 (s, 1H), 7.81 (d, J = 7.9 Hz, 1H), 7.69 - 7.45 (m, 7H), 7.20 (t, J = 7.4 Hz, 2H), 5.08 (dd, J = 13.3, 5.1 Hz, 1H), 4.35 (dd, J = 55.5, 17.4 Hz, 2H), 3.75 (s, 6H), 3.40 (d, J = 4.8 Hz, 4H), 3.05 - 2.97 (m, 4H), 2.92 - 2.86 (m, 1H), 2.81 (d, J = 4.5 Hz, 3H), 2.64 - 2.52 (m, 3H), 2.37 (dd, J = 12.4, 4.7 Hz, 1H), 1.98 (dd, J = 20.8, 13.3 Hz, 3H).. LCMS [M+H] ⁺ =851.9

### Synthesis method of Compound UB-181057:

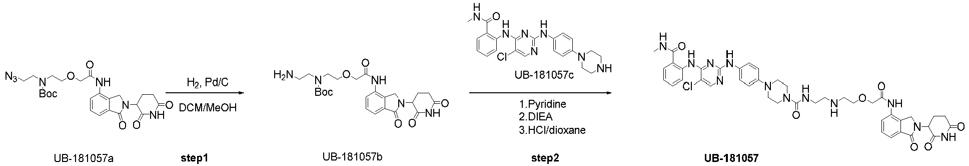

### Step 1: UB-181057b (V2591-022)

To UB-181057a (30mg, 0.057mmol) in dichloromethane /methanol (10ml) solution was added palladium/carbon(1.21mg, 0.011mmol), and the reaction mixture was stirred at room temperature for 1 hour under hydrogen atmosphere. The reaction mixture was filtered and concentrated under vacuum to obtain the desired product UB-181057b (28 mg, 100%yield), which was a colorless solid. LC-MS: [M+H] ⁺ = 488.5

### Step 2: UB-181057 (V2591-031)

### Method similar to General Method 4

¹H NMR (400 MHz, DMSO) δ 11.97 (s, 1H), 11.01 (d, J = 5.9 Hz, 1H), 10.13 (s, 1H), 10.00 (s, 1H), 9.27 (s, 2H), 8.87 (d, J = 4.4 Hz, 1H), 8.64 (s, 1H), 8.31 (s, 1H), 7.82 - 7.74 (m, 2H), 7.64 (s, 2H), 7.54 (dt, J = 15.3, 7.1 Hz, 5H), 7.20 (t, J = 7.6 Hz, 2H), 5.15 (dd, J = 13.3, 5.2 Hz, 1H), 4.51 (d, J = 17.6 Hz, 4H), 3.85 (d, J = 4.9 Hz, 2H), 3.71 (d, J = 23.2 Hz, 4H), 3.42 (d, J = 21.1 Hz, 6H), 3.24 (s, 2H), 3.10 (d, J = 5.0 Hz, 2H), 2.96 - 2.87 (m, 1H), 2.81 (d, J = 4.5 Hz, 3H), 2.60 (d, J = 16.2 Hz, 1H), 2.41 - 2.33 (m, 1H), 2.04 - 1.98 (m, 1H). LCMS [M+H] ⁺ =867.8

### Synthesis method of Compound UB-181058:

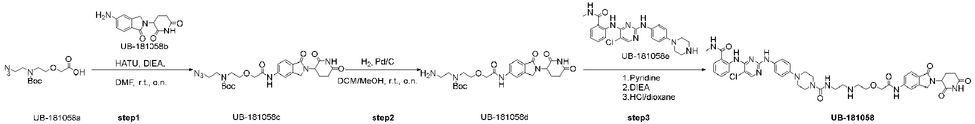

### Step 1: UB-181058c (V2591-009)

To **UB-181058a(150mg,** 0.52mmol) and HATU(396mg, 1.04mmol) in N,N-dimethylformamide (20ml) solution was added DIPEA (202mg, 1.56mmol), and the mixture was stirred at room temperature for 1 hour. Then **UB-181058b** (121 mg, 0.496 mmol) was added to the mixture, and the mixture was stirred at room temperature for 12 hours. The solvent was removed in vacuum, and the crude product was purified via flash chromatography(dichloromethane/methanol = 10/1) to obtain desired product **UB-181058c(130** mg, 47%yield), as a colorless solid.

¹H NMR (400 MHz, DMSO) δ 10.98 (s, 1H), 10.04 (s, 1H), 8.01 (s, 1H), 7.68 (s, 2H), 5.09 (dd, J = 13.3, 5.1 Hz, 1H), 4.37 (dd, J = 57.0, 17.4 Hz, 2H), 4.13 (s, 2H), 3.64 (d, J = 5.1 Hz, 2H), 3.45 (s, 6H), 2.95 - 2.86 (m, 1H), 2.62 (s, 1H), 2.42 - 2.32 (m, 1H), 2.03 - 1.94 (m, 1H), 1.40 (s, 9H). LC-MS: [M+H] ⁺ = 530.5

### Step 2: UB-181058d (V2591-023)

To **UB-181058c** (30mg, 0.057mmol) in dichloromethane /methanol (10ml) solution was added palladium/carbon(1.21mg, 0.011mmol), and the reaction mixture was stirred at room temperature for 1 hour under hydrogen atmosphere. The reaction mixture was filtered and concentrated under vacuum to obtain the desired product **UB-181058d** (28 mg, 100%yield), which was a colorless solid. LC-MS: [M+H] ⁺ = 504.5

### Step 3: UB-181058 (V2591-032)

### Method similar to General Method 4

¹H NMR (400 MHz, DMSO) δ 11.97 (s, 1H), 10.96 (d, J = 6.8 Hz, 1H), 10.36 (s, 1H), 10.01 (s, 1H), 9.27 (s, 2H), 8.87 (d, J = 4.4 Hz, 1H), 8.62 (s, 1H), 8.31 (s, 1H), 8.12 (s, 1H), 7.94 - 7.84 (m, 1H), 7.81 (d, J = 7.9 Hz, 1H), 7.71 - 7.50 (m, 6H), 7.24 - 7.17 (m, 2H), 5.08 (dd, J = 13.3, 5.0 Hz, 1H), 4.44 - 4.37 (m, 4H), 3.88 - 3.83 (m, 2H), 3.79 - 3.67 (m, 4H), 3.48 - 3.38 (m, 6H), 3.24 (s, 2H), 3.10 (s, 2H), 2.95 - 2.85 (m, 1H), 2.81 (d, J = 4.5 Hz, 3H), 2.58 (d, J = 17.9 Hz, 1H), 2.36 (dd, J = 13.3, 4.6 Hz, 1H), 2.04 - 1.92 (m, 1H). LCMS [M+H] ⁺=867.9

### Synthesis method of Compound UB-181061:

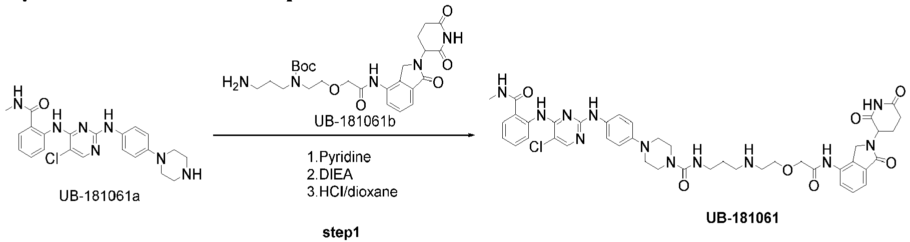

### Step 1: UB-181061 (V2591-036)

### Method similar to General Method 4

¹H NMR (400 MHz, DMSO) δ 12.03 (s, 1H), 11.01 (s, 1H), 10.15 (d, J = 9.9 Hz, 2H), 9.24 (s, 2H), 8.90 (d, J = 4.5 Hz, 1H), 8.61 (d, J = 7.6 Hz, 1H), 8.33 (s, 1H), 7.82 (dd, J = 8.0, 1.2 Hz, 1H), 7.75 (d, J = 7.7 Hz, 1H), 7.66 (d, J = 8.4 Hz, 2H), 7.55 (dt, J = 15.2, 7.1 Hz, 5H), 7.19 (dd, J = 27.1, 19.8 Hz, 2H), 5.14 (dd, J = 13.3, 5.1 Hz, 1H), 4.45 (d, J = 40.5 Hz, 2H), 3.80 - 3.70 (m, 8H), 3.40 (d, J = 4.6 Hz, 4H), 3.23 - 3.15 (m, 4H), 2.98 (d, J = 6.3 Hz, 2H), 2.94 - 2.86 (m, 1H), 2.81 (d, J = 4.5 Hz, 3H), 2.61 (d, J = 17.0 Hz, 1H), 2.37 (dd, J = 13.1, 4.6 Hz, 1H), 2.05 - 1.98 (m, 1H), 1.87 (dd, J = 13.9, 6.9 Hz, 2H). LCMS [M+H] ⁺ =881.9

### Synthesis method of Compound UB-181062:

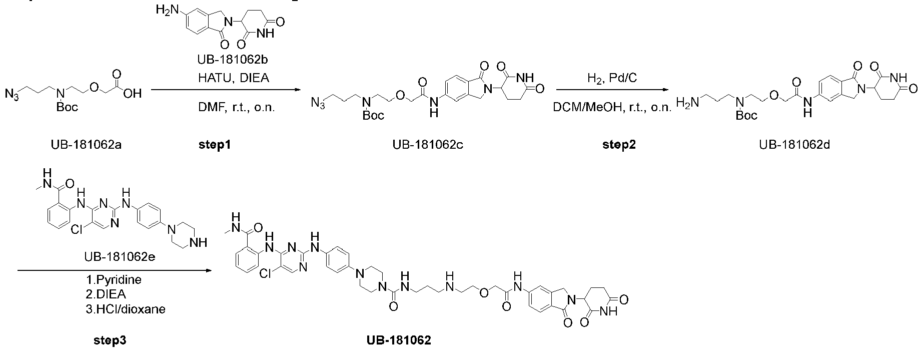

### Step 1: UB-181062c (V2591-010)

To **UB-181062a(150** mg, 0.50 mmol) and HATU(377 mg, 1.00 mmol) in N,N-dimethylformamide (20ml) solution was added DIPEA (192 mg, 1.49 mmol), and the mixture was stirred at room temperature for 1 hour. Then **UB-181062b** (116 mg, 0.447 mmol) was added to the mixture, the mixture was stirred at room temperature for 12h. The solvent was removed in vacuum, and the crude product was purified via flash chromatography(dichloromethane/methanol = 10/1) to obtain desired product **UB-181062c(70** mg, 26% yield), as a colorless solid.

¹H NMR (400 MHz, DMSO) δ 10.98 (s, 1H), 10.05 (s, 1H), 8.01 (s, 1H), 7.67 (d, J = 8.3 Hz, 2H), 5.09 (dd, J = 13.3, 5.1 Hz, 1H), 4.37 (dd, J = 56.7, 17.4 Hz, 2H), 4.13 (s, 2H), 3.63 (t, J = 5.7 Hz, 2H), 3.32 - 3.28 (m, 6H), 2.95 - 2.88 (m, 1H), 2.63 - 2.56 (m, 1H), 2.38 (dd, J = 13.1, 4.5 Hz, 1H), 1.99 (dd, J = 8.8, 3.8 Hz, 1H), 1.75 (s, 2H), 1.39 (s, 9H). LC-MS: [M+H] ⁺ = 544.6

### Step 2: UB-181062d (V2591-033)

To **UB-181062c** (30 mg, 0.055 mmol) in dichloromethane /methanol (10ml) solution was added palladium/carbon(1.18 mg, 0.011 mmol), and the reaction mixture was stirred at room temperature for 1 hour under hydrogen atmosphere. The reaction mixture was filtered and concentrated under vacuum to obtain the desired product **UB-181062d** (29 mg, 100% yield), which was a colorless solid.

### Step 3: UB-181062 (V2591-037)

### Method similar to General Method 4

¹H NMR (400 MHz, DMSO) δ 11.90 (s, 1H), 10.98 (s, 1H), 10.33 (s, 1H), 9.82 (s, 1H), 9.10 (s, 2H), 8.85 (d, J = 4.4 Hz, 1H), 8.65 (s, 1H), 8.27 (s, 1H), 8.10 (s, 1H), 7.91 - 7.82 (m, 1H), 7.80 (d, J = 6.7 Hz, 1H), 7.68 (d, J = 8.3 Hz, 1H), 7.60 (s, 2H), 7.51 (t, J = 7.6 Hz, 1H), 7.35 (s, 2H), 7.19 (t, J = 7.2 Hz, 1H), 7.03 (s, 1H), 5.08 (dd, J = 13.2, 5.0 Hz, 1H), 4.44 - 4.26 (m, 2H), 4.22 (d, J = 4.6 Hz, 2H), 3.82 (d, J = 4.9 Hz, 2H), 3.30 (s, 6H), 3.18 (s, 6H), 2.99 (s, 2H), 2.91 - 2.85 (m, 1H), 2.81 (d, J = 4.5 Hz, 3H), 2.59 (d, J = 18.2 Hz, 1H), 2.37 - 2.30 (m, 1H), 1.99 (d, J = 5.4 Hz, 1H), 1.90 - 1.81 (m, 2H). LCMS [M+H] ⁺ = 881.9

### Synthesis method of Compound UB-181066:

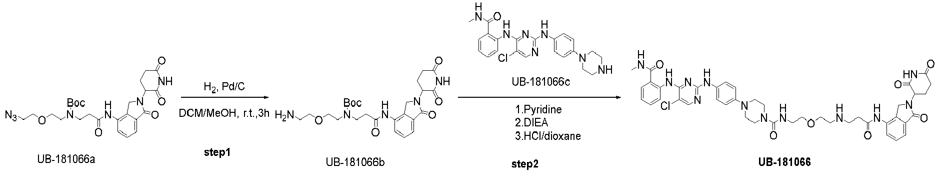

### Step 1: UB-181066b (V2591-044)

To **UB-181066a** (30 mg, 0.055 mmol) in dichloromethane /methanol (10ml) solution was added palladium/carbon(1.18 mg, 0.011 mmol), and the reaction mixture was stirred at room temperature for 1 hour under hydrogen atmosphere. The reaction mixture was filtered and concentrated under vacuum to obtain the desired product **UB-181062d** (29 mg, 100% yield), which was a colorless solid. LC-MS: [M+H] ⁺ = 518.7

### Step 2: UB-181066 (V2591-055)

### Method similar to General Method 4

¹H NMR (400 MHz, DMSO) δ 11.99 (s, 1H), 11.03 (s, 1H), 10.31 (s, 1H), 10.08 (s, 1H), 9.15 (s, 2H), 8.89 (d, J = 4.4 Hz, 1H), 8.61 (d, J = 7.4 Hz, 1H), 8.32 (s, 1H), 7.83 (t, J = 7.7 Hz, 2H), 7.66 (d, J = 8.5 Hz, 2H), 7.59 - 7.46 (m, 5H), 7.21 (t, J = 7.3 Hz, 1H), 7.05 (s, 1H), 5.14 (dd, J = 13.2, 5.1 Hz, 1H), 4.49 - 4.35 (m, 2H), 3.78 (s, 4H), 3.54 - 3.48 (m, 4H), 3.41 (s, 4H), 3.28 (d, J = 4.8 Hz, 4H), 3.16 (s, 2H), 2.97 (t, J = 7.0 Hz, 2H), 2.90 (dd, J = 17.5, 5.0 Hz, 1H), 2.81 (d, J = 4.5 Hz, 3H), 2.61 (d, J = 16.7 Hz, 1H), 2.33 (dd, J = 13.1, 4.3 Hz, 1H), 2.05 - 1.98 (m, 1H). LCMS [M+H] ⁺ = 881.9

### Synthesis method of Compound UB-181067:

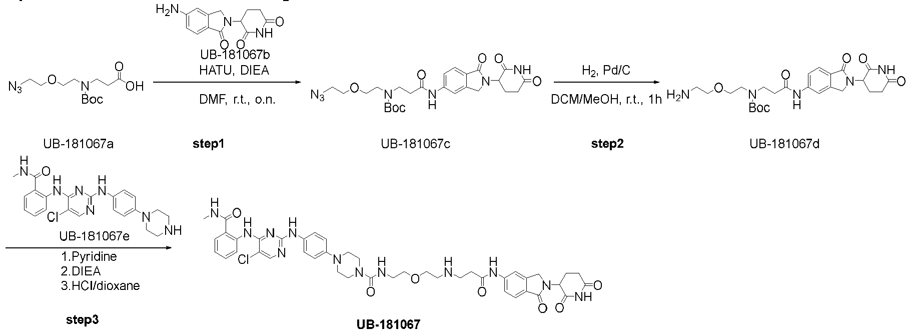

### Step 1: UB-181067c (V2591-042)

To **UB-181067a(150** mg, 0.50 mmol) and HATU(377 mg, 1.00 mmol) in N,N-dimethylformamide (20ml) solution was added DIPEA (192 mg, 1.49 mmol), and the mixture was stirred at room temperature for 1 hour. Then **UB-181067b** (116 mg, 0.447 mmol) was added to the mixture, and the mixture was stirred at room temperature for 12h. The solvent was removed in vacuum, the crude product was purified via flash chromatography(dichloromethane/methanol = 10/1) to obtain desired product **UB-181067c(135** mg, 50% yield) as a colorless solid. LC-MS: [M+H] ⁺ = 544.7

### Step 2: UB-181067d (V2591-045)

To **UB-181067c** (30 mg, 0.055 mmol) in dichloromethane /methanol (10ml) solution was added palladium/carbon(1.18 mg, 0.011 mmol), and the reaction mixture was stirred at room temperature for 1 hour under hydrogen atmosphere. The reaction mixture was filtered and concentrated under vacuum to obtain the desired product **UB-181067d** (29 mg, 100% yield), which was a colorless solid. LC-MS: [M+H] ⁺ = 518.6

### Step 3: UB-181067 (V2591-056)

### Method similar to General Method 4

¹H NMR (400 MHz, DMSO) δ 11.88 (s, 1H), 10.97 (s, 1H), 10.71 (s, 1H), 9.79 (s, 1H), 8.98 (s, 2H), 8.84 (d, J = 4.5 Hz, 1H), 8.66 (s, 1H), 8.27 (s, 1H), 7.96 (s, 1H), 7.78 (s, 1H), 7.69 - 7.58 (m, 4H), 7.50 (d, J = 7.3 Hz, 1H), 7.35 (s, 2H), 7.17 (d, J = 7.5 Hz, 1H), 6.94 (s, 1H), 5.07 (dd, J = 13.2, 5.0 Hz, 1H), 4.40 (d, J = 17.4 Hz, 1H), 4.27 (d, J = 17.5 Hz, 1H), 3.64 (s, 4H), 3.50 (d, J = 5.9 Hz, 2H), 3.28 (s, 8H), 3.17 (s, 4H), 2.94 (t, J = 7.2 Hz, 2H), 2.86 (d, J = 6.4 Hz, 1H), 2.81 (d, J = 4.5 Hz, 3H), 2.58 (d, J = 16.2 Hz, 1H), 2.37 - 2.31 (m, 1H), 2.00 - 1.94 (m, 1H). LCMS [M+H] ⁺ = 881.9

### Synthesis method of Compound UB-181068:

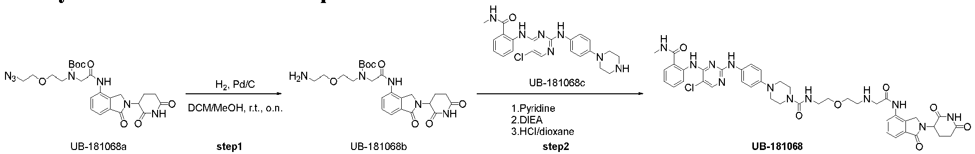

### Step 1: UB-181068b (V2591-050)

To **UB-181068a** (30 mg, 0.057 mmol) in dichloromethane /methanol (10ml) solution was added palladium/carbon(1.21 mg, 0.011 mmol), and the reaction mixture was stirred at room temperature for 1 hour under hydrogen atmosphere. The reaction mixture was filtered and concentrated under vacuum to obtain the desired product **UB-181068b** (28 mg, 100% yield), which was a colorless solid. LC-MS: [M+H] ⁺ = 504.6

### Step 2: UB-181068 (V2591-057)

### Method similar to General Method 4

¹H NMR (400 MHz, DMSO) δ 11.88 (s, 1H), 11.05 (s, 1H), 10.71 (s, 1H), 9.78 (s, 1H), 9.19 (s, 2H), 8.84 (d, J = 4.6 Hz, 1H), 8.67 (s, 1H), 8.27 (s, 1H), 7.88 (d, J = 7.5 Hz, 1H), 7.79 (d, J = 6.8 Hz, 1H), 7.64 - 7.47 (m, 5H), 7.31 (d, J = 21.7 Hz, 2H), 7.17 (d, J = 7.2 Hz, 1H), 6.91 (s, 1H), 5.17 (dd, J = 13.2, 5.1 Hz, 1H), 4.43 (dd, J = 40.4, 17.6 Hz, 2H), 4.08 (s, 2H), 3.74 - 3.71 (m, 2H), 3.64 (s, 6H), 3.25 (s, 8H), 2.95 (d, J = 13.2 Hz, 1H), 2.81 (d, J = 4.5 Hz, 3H), 2.60 (s, 1H), 2.33 - 2.26 (m, 1H), 2.02 (dd, J = 15.3, 7.7 Hz, 1H). LCMS [M+H] ⁺ = 867.9

### Synthesis method of Compound UB-181069:

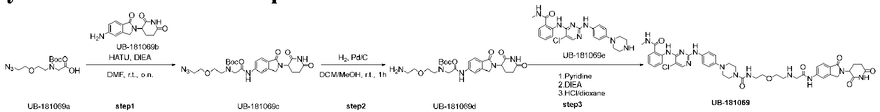

### Step 1: UB-181069c (V2591-046)

To UB-181069a(150 mg, 0.52 mmol) and HATU(395 mg, 1.04 mmol) in N,N-dimethylformamide (20ml) solution was added DIPEA (202 mg, 1.56mmol), and the mixture was stirred at room temperature for 1 hour. Then UB-181069b (121 mg, 0.47 mmol) was added to the mixture, the mixture was stirred at room temperature for 12h. The solvent was removed in vacuum, and the crude product was purified via flash CC to obtain product UB-181069c(180mg, 65% yield) as a yellow solid. LC-MS: [M + H] ⁺ = 530.6

### Step 2: UB-181069d (V2591-051)

To **UB-181069c** (30 mg, 0.057 mmol) in dichloromethane /methanol (10ml) solution was added palladium/carbon(1.21 mg, 0.011 mmol), and the reaction mixture was stirred at room temperature for 1 hour under hydrogen atmosphere. The reaction mixture was filtered and concentrated under vacuum to obtain the desired product **UB-181069d** (28 mg, 100% yield), which was a colorless solid. LC-MS: [M+H] ⁺ = 504.6

### Step 3: UB-181069 (V2591-058)

### Method similar to General Method 4

¹H NMR (400 MHz, DMSO) δ 11.88 (s, 1H), 11.31 (s, 1H), 10.97 (s, 1H), 9.81 (s, 1H), 9.23 (s, 4H), 8.85 (d, J = 4.5 Hz, 1H), 8.65 (s, 1H), 8.27 (s, 1H), 7.97 (s, 1H), 7.80 (d, J = 6.8 Hz, 1H), 7.71 (t, J = 5.4 Hz, 2H), 7.59 (s, 2H), 7.49 (d, J = 7.1 Hz, 1H), 7.32 (s, 1H), 7.18 (t, J = 7.4 Hz, 1H), 6.99 (s, 1H), 5.08 (dd, J = 13.3, 5.1 Hz, 1H), 4.41 (d, J = 17.3 Hz, 1H), 4.29 (d, J = 17.7 Hz, 1H), 4.11 (s, 2H), 3.60 (dd, J = 6.6, 2.6 Hz, 4H), 3.50 (d, J = 4.2 Hz, 2H), 3.28 (s, 8H), 3.11 (s, 2H), 2.91 (d, J = 13.1 Hz, 1H), 2.81 (d, J = 4.4 Hz, 3H), 2.60 (s, 1H), 2.34 (d, J = 8.5 Hz, 1H), 1.97 (d, J = 5.0 Hz, 1H). LCMS [M+H] ⁺ = 868.0

### Synthesis method of Compound UB-181070:

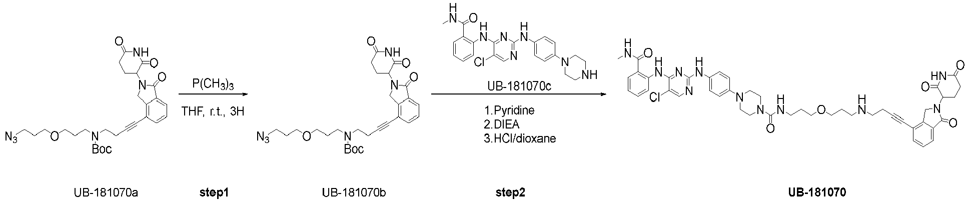

### Step 1: UB-181070b (V2591-049)

To **UB-181070a(30mg,** 0.054mmol) in tetrahydrofuran (5ml) solution was added trimethylphosphine (0.11ml), and the reaction mixture was stirred at 50°C for 1 hour. Then to the mixture was added 3ml water, and the reaction mixture was stirred for 3 hours, and the reaction mixture was filtered and concentrated in vacuum to obtain desired crude UB-181070b (29mg, 100% yield), which was colorless solid. LC-MS: [M+H] ⁺ = 527.7

### Step 2: UB-181070 (V2591-064)

### Method similar to General Method 4

LCMS [M+H] ⁺ = 890.8

### Synthesis method of Compound UB-181094:

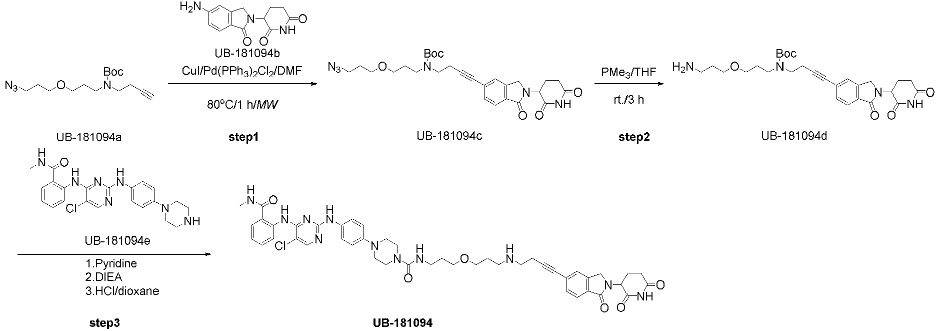

### Step 1: UB-181094c (V2591-099)

**UB-181094a(100** mg, 0.32 mmol) cuprous iodide (12.3 mg, 0.065 mmol), bistriphenylphosphonium palladium dichloride(23 mg, 0.016 mmol) and triethylamine(33 mg, 0.33 mmol) was dissolved in N,N-dimethylformamide(8 mL), **UB-181094b** (119mg, 0.32mmol) was added under nitrogen atmosphere, and the mixture was stirred at 80°C for 1 h. The reaction mixture was diluted with distilled water/brine and the product was extracted with ethyl acetate (20 ml * 3). Then the combined organic layers were washed with brine and dried over anhydrous sodium sulfate, and filtered. The solvent was removed in vacuum to obtain crude product, which was purified by silica gel chromatography on silica gel (DCM / MeOH=10/1) to obtain product **UB-181094c** (110mg, 62% yield) as a yellow solid. LC-MS: [M+H] ⁺ = 553.6

### Step 2: UB-181094d (V2591-106)

To **UB-181094c** (30mg, 0.054mmol) in tetrahydrofuran (5ml) solution was added trimethylphosphine (0.22ml), and the reaction mixture was stirred at 50°C for 1 hour. Then to the mixture was added 3ml water, and the reaction mixture was stirred for 3 hours, the reaction mixture was filtered and concentrated in vacuum to obtain desired crude **UB-181094d(50mg,** 100% yield), which was a colorless solid. LC-MS: [M+H] ⁺ = 527.7

### Step 3: UB-181094 (V2591-112)

### Method similar to General Method 4

¹H NMR (400 MHz, DMSO) δ 11.96 (s, 1H), 10.99 (s, 1H), 10.01 (s, 1H), 9.47 (s, 1H), 9.09 (d, J = 60.4 Hz, 1H), 8.88 (d, J = 4.4 Hz, 1H), 8.62 (s, 1H), 8.30 (s, 1H), 7.81 (d, J = 7.9 Hz, 1H), 7.73 - 7.42 (m, 8H), 7.19 (d, J = 7.5 Hz, 1H), 6.76 (d, J = 37.6 Hz, 1H), 5.10 (dd, J = 13.3, 4.7 Hz, 1H), 4.49 - 4.38 (m, 2H), 3.99 - 3.67 (m, 8H), 3.49 - 3.27 (m, 6H), 3.22 (d, J = 5.6 Hz, 2H), 3.03 (dt, J = 30.2, 17.0 Hz, 4H), 2.91 - 2.84 (m, 1H), 2.81 (d, J = 4.5 Hz, 3H), 2.59 (d, J = 17.2 Hz, 1H), 2.40 - 2.32 (m, 1H), 2.00 (d, J = 5.1 Hz, 1H), 1.14 - 1.08 (m, 4H). LCMS [M+H] ⁺ = 890.9

### Synthesis method of Compound UB-181128:

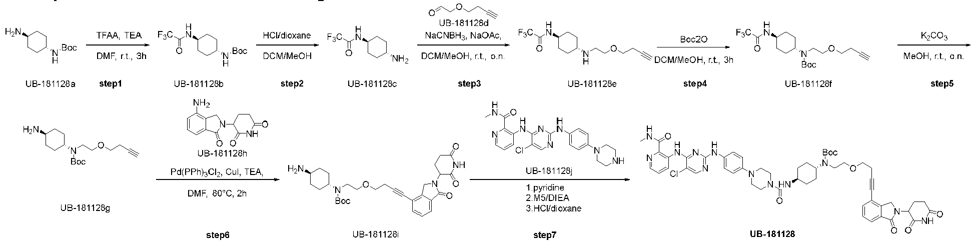

### Step 1: UB-181128b (V2591-134)

To **UB-181128a(10g,** 70mmol) in N,N-dimethylformamide(200ml) solution was added trifluoroacetic anhydride (16.2g.77mmol) and triethylamine (21g, 210mmol), the mixture was stirred at room temperature for 3 hours. The reaction mixture was poured into 1000 ml of water and filtered to obtain 20 g of solid **UB-181128b** in 92% yield as a yellow solid.

### Step 2: UB-181128c (V2591-138)

To **UB-181128b(20g,** 64.5mmol) in dichloromethane /methanol = 10/1(200ml)solution was added 4 mol/L hydrochloric acid (80ml), and the mixture was stirred at room temperature for 3 hours. The reaction mixture was concentrated to give the product **UB-181128c** (13.5 g, 100%yield) as a yellow solid. LC-MS: [M+H] ⁺ = 211.2

### Step 3: UB-181128e (V2591-141)

To **UB-181128c** (4.5g, 21.4mmol) in dichloromethane /methanol = 10/1(200ml) solution was added **UB-181128d(3g,** 23.6mmol)) and two drops of acetic acid, and the mixture was stirred at room temperature overnight. Then sodium cyanoborohydride (2.7 g, 42.9 mmol) was added and the mixture was stirred at room temperature for 1 hour. TLC board showed no raw material, and LCMS showed **UB-181128e.** To the reaction solution was directly added di-tert-butyl dicarbonate V2591-143). LC-MS: [M+H] ⁺ = 307.3

### Step 4: UB-181128f (V2591-143)

To a solution of **UB-181128e** was added di-tert-butyl dicarbonate (18.5 g, 85 mmol) and sodium bicarbonate solution (20 ml), and the mixture was stirred at room temperature for 2 h. TLC plate showed there was no SM, and LCMS showed it was the product. The reaction mixture was extracted with dichloromethane (* 3) and the organic phase was concentrated to obtain the crude product. The crude product was purified via flash chromatography (petroleum ether/ethyl acetate=3/1) to obtain **UB-181128f(5.9g,** 35 % yield ) as a white solid.

¹H NMR (400 MHz, DMSO) δ 9.24 (s, 1H), 3.64 (s, 2H), 3.49 (t, J = 6.7 Hz, 2H), 3.42 (t, J = 6.2 Hz, 2H), 3.21 (s, 2H), 2.79 (t, J = 2.6 Hz, 1H), 2.38 (td, J = 6.7, 2.6 Hz, 2H), 1.84 - 1.78 (m, 2H), 1.61 (s, 4H), 1.40 (s, 9H), 1.37 (s, 2H). LC-MS: [M+H] ⁺ = 407.4

### Step 5: UB-181128g (V2591-144)

To **UB-181128f(2g,** 4.93mmol) in methanol (20ml) solution was added potassium carbonate(1.4g, 9.85mmol), and the mixture was stirred at room temperature for 12 hours. The reaction mixture was extracted with dichloromethane, and the organic phase was concentrated to obtain the crude product. The crude product was purified via flash chromatography(dichloromethane((3‰ ammonia-methanol))/methanol = 10/1) to obtain desired product **UB-181128g**(1.04g, 68 %yield) as a colorless oil. LC-MS: [M+H] ⁺ = 311.4

### Step 6: UB-181128i (V2591-146)

**UB-181128g**(500 mg, 1.6 mmol), cuprous iodide (61 mg, 0.32 mmol), bistriphenylphosphonium palladium dichloride(113 mg, 0.08 mmol) and triethylamine(163 mg, 1.6 mmol) were dissolved in N,N-dimethylformamide(15 mL) solution, and **UB-181128h(597mg,** 1.6mmol) was added under nitrogen atmosphere, and the mixture was stirred at 80°C for 1 h. The reaction mixture was diluted with distilled water/brine and the product was extracted with ethyl acetate (50 ml * 3). Then the combined organic layers were washed with brine and dried over anhydrous sodium sulfate, and filtered. The solvent was removed in vacuum to obtain crude product, which was purified by silica gel chromatography on silica gel (dichloromethane /methanol = 10/1) to obtain product **UB-181128i** (400mg, 45 % yield) as a yellow solid.

¹H NMR (400 MHz, DMSO) δ 11.00 (s, 1H), 7.74 - 7.71 (m, 1H), 7.62 (d, J = 6.9 Hz, 1H), 7.53 (t, J = 7.6 Hz, 1H), 5.16 (dd, J = 13.3, 5.1 Hz, 1H), 4.37 (dd, J = 56.1, 17.7 Hz, 2H), 3.63 (t, J = 6.6 Hz, 2H), 3.48 (t, J = 6.5 Hz, 2H), 3.22 (s, 2H), 2.94 (td, J = 13.5, 6.8 Hz, 2H), 2.72 (t, J = 6.6 Hz, 2H), 2.59 (dd, J = 15.3, 2.1 Hz, 1H), 2.44 (dd, J = 13.2, 4.1 Hz, 1H), 2.01 (dd, J = 11.7, 6.6 Hz, 1H), 1.92 (d, J = 10.3 Hz, 2H), 1.61 (s, 4H), 1.40 (s, 1H), 1.38 (s, 9H), 1.35 - 1.30 (m, 2H). LC-MS: [M+H] ⁺ = 553.6

### Step 7: UB-181128 (V2876-009)

### Method similar to General Method 4

¹H NMR (400 MHz, ) δ 12.56 (s, 1H), 11.03 (s, 1H), 9.43 (d, J = 39.5 Hz, 2H), 9.18 (d, J = 4.9 Hz, 1H), 8.65 (s, 2H), 8.37 - 8.20 (m, 2H), 7.74 (d, J = 7.5 Hz, 1H), 7.66 (d, J = 6.8 Hz, 1H), 7.55 (t, J = 7.6 Hz, 4H), 7.10 (s, 2H), 6.39 (s, 1H), 5.18 (dd, J = 13.3, 5.1 Hz, 1H), 4.47 (d, J = 17.6 Hz, 1H), 4.33 (d, J = 17.7 Hz, 1H), 3.78 - 3.74 (m, 2H), 3.71 (d, J = 6.7 Hz, 2H), 3.52 - 3.48 (m, 4H), 3.43 - 3.36 (m, 2H), 3.15 (s, 6H), 2.96 - 2.90 (m, 1H), 2.86 (d, J = 4.9 Hz, 3H), 2.81 (t, J = 6.7 Hz, 2H), 2.65 (dd, J = 25.4, 9.1 Hz, 1H), 2.36 - 2.31 (m, 1H), 2.05 (dd, J = 18.4, 7.8 Hz, 3H), 1.87 (d, J = 10.8 Hz, 2H), 1.46 - 1.37 (m, 2H), 1.24 (d, J = 11.8 Hz, 2H). LCMS [M+H] ⁺= 917.8

### Synthesis method of Compound UB-181029:

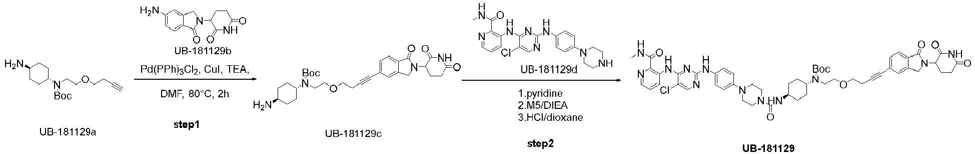

### Step 1: UB-181129c (2591-147)

**UB-181129a(500** mg, 1.6 mmol) , cuprous iodide (61 mg, 0.32 mmol), bistriphenylphosphonium palladium dichloride(113 mg, 0.08 mmol) and triethylamine(163 mg, 1.6 mmol) were dissolved in N,N-dimethylformamide(15 mL) solution, and **UB-181129b**(597mg, 1.6mmol) was added under nitrogen atmosphere, and the mixture was stirred at 80°C for 1 h. The reaction mixture was diluted with distilled water/brine and the product was extracted with ethyl acetate (50 ml * 3). Then the combined organic layers were washed with brine and dried over anhydrous sodium sulfate, and filtered. The solvent was removed in vacuum to obtain crude product, which was purified by silica gel chromatography on silica gel (dichloromethane /methanol = 10/1) to obtain product **UB-181129c** (456 mg, 51% yield) as a yellow solid.

¹H NMR (400 MHz, DMSO) δ 11.00 (s, 1H), 7.70 (d, J = 7.9 Hz, 1H), 7.62 (s, 1H), 7.49 (d, J = 8.3 Hz, 1H), 5.11 (dd, J = 13.2, 5.1 Hz, 1H), 4.38 (dd, J = 50.0, 17.6 Hz, 2H), 3.62 (t, J = 6.5 Hz, 2H), 3.49 (t, J = 6.4 Hz, 2H), 3.23 (s, 2H), 2.98 - 2.87 (m, 2H), 2.70 (dd, J = 13.9, 7.4 Hz, 2H), 2.60 (d, J = 17.6 Hz, 1H), 2.37 (dd, J = 15.9, 11.8 Hz, 1H), 2.03 - 1.98 (m, 1H), 1.92 (d, J = 10.5 Hz, 2H), 1.63 (s, 4H), 1.39 (s, 9H), 1.30 (d, J = 32.6 Hz, 3H). LC-MS: [M+H] ⁺ = 553.6

### Step 2: UB-181129 (2876-010)

### Method similar to General Method 4

¹H NMR (400 MHz, DMSO) δ 12.58 (s, 1H), 11.00 (s, 1H), 9.55 (s, 1H), 9.37 (s, 1H), 9.18 (d, J = 4.7 Hz, 1H), 8.68 (s, 2H), 8.34 - 8.24 (m, 2H), 7.71 (d, J = 7.8 Hz, 1H), 7.64 (s, 1H), 7.52 (d, J = 8.3 Hz, 4H), 7.18 (s, 2H), 6.43 (s, 1H), 5.11 (dd, J = 13.4, 5.1 Hz, 1H), 4.45 (d, J = 17.5 Hz, 1H), 4.33 (d, J = 17.6 Hz, 1H), 3.68 (s, 4H), 3.53 (s, 4H), 3.42 (s, 2H), 3.16 (s, 5H), 2.91 (s, 1H), 2.85 (d, J = 4.9 Hz, 3H), 2.79 (t, J = 6.7 Hz, 2H), 2.62 (s, 1H), 2.58 (s, 1H), 2.35 (d, J = 20.6 Hz, 1H), 2.07 (s, 2H), 2.01 (d, J = 5.4 Hz, 1H), 1.86 (d, J = 12.1 Hz, 2H), 1.42 (d, J = 13.2 Hz, 2H), 1.25 (d, J = 10.3 Hz, 2H). LCMS [M+H] ⁺ = 917.7

### Synthesis method of Compound UB-180951

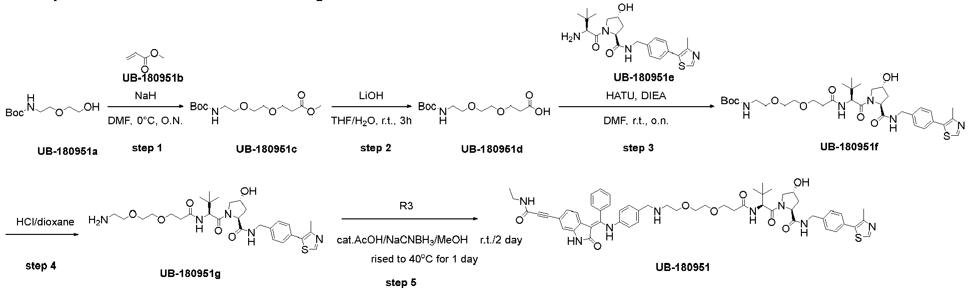

### Step 1: UB-180951c (V2128-093)

At 0°C, compound **UB-180951a** (1 g, 4.9 mmol) was dissolved in THF (10 mL), then NaH (19.49 mg, 0.49 mmol) was added, and the mixture was reacted at room temperature for 1 hour, followed by adding **UB-180951b** (419 mg, 4.9 mmol) and continued to react at room temperature overnight. The reaction solution was added with water then extracted with ethyl acetate (10 mL), the organic phase was concentrated, then isolated by column chromatography to obtain target product **UB-180951c** (440 mg, yield 32%) as a yellow oil.

¹H NMR (400 MHz, CDCl₃) δ 5.04 (s, 1H), 3.77 (t, *J=* 6.5 Hz, 2H), 3.73 - 3.65 (m, 3H), 3.65 - 3.56 (m, 4H), 3.54 (t, *J=* 5.1 Hz, 2H), 3.31 (dd, *J=* 10.0, 5.0 Hz, 2H), 2.62 (t, J= 6.4 Hz, 2H), 1.45 (s, 9H). LCMS: [M+H] ⁺ = 292.2

### Step 2: UB-180951d (V2128-120)

Compound **UB-180951c** (300 mg, 1.03 mmol) was dissolved in H₂O/THF (5 mL), then LiOH(130 mg, 3.09 mmol) was added and the mixture was reacted at room temperature for 1 hour. The reaction solution was added water, then washed with ethyl acetate(10 mL), the aqueous phase was adjusted to pH 6, and then concentrated to obtain the target crude product **UB-180951d** (286 mg, yield 100%). LCMS: [M+H] ⁺ = 278.2

### Step 3: UB-180951f (V2128-126)

Compound **UB-180951d** (143 mg, 0.52 mmol) and HATU (392 mg, 1.03 mmol) were dissolved in DMF (10 ml), then DIPEA (200 mg, 1.55 mmol) was added, and the mixture was reacted at room temperature for 1 hour. **UB-180951e** (222 mg, 0.52 mmol) was further added, and continued to react at room temperature overnight. The reaction solution was concentrated and isolated by column chromatography (dichloromethane/ methanol = 10/1) to obtain target product **UB-180951f** (70 mg, yield 20%) as a yellow solid. LCMS: [M+H] ⁺ = 690.5

### Step 4: UB-180951g (V2235-019)

Compound **UB-180951f** (25 mg, 0.04 mmol) was dissolved in dichloromethane (3 mL), then HCl/dioxane (1 mL) was added, and the mixture was reacted for 20 min. The reaction solution was concentrated to obtain target product **UB-180951g** (25 mg, yield 100%) as a yellow oil.

### Step 5: UB-180951 (V2235-037)

### The method is similar to General Method 5

¹H NMR (400 MHz, DMSO-*d*₆) δ 12.16 (s, 1H), 10.91 (s, 1H), 8.97 (d, *J=* 2.8 Hz, 1H), 8.76 - 8.41 (m, 2H), 7.91 (d, *J=* 9.6 Hz, 1H), 7.64 - 7.30 (m, 9H), 7.09 (d, *J=* 8.1 Hz, 2H), 6.98 (s, 1H), 6.84 - 6.63 (m, 3H), 5.76 (d, *J=* 8.1 Hz, 1H), 4.55 (d, *J=* 9.4 Hz, 1H), 4.49 - 4.30 (m, 4H), 4.23 (dd, *J=* 14.1, 8.6 Hz, 2H), 3.70 - 3.51 (m, 6H), 3.49 - 3.39 (m, 5H), 3.12 (p, *J* = 7.0 Hz, 2H), 2.44 (s, 3H), 2.38 - 2.27 (m, 1H), 2.08 - 1.77 (m, 4H), 1.03 (t, *J* = 7.2 Hz, 3H), 0.92 (s, 9H). LCMS [M+H]⁺ = 1009.7

### Synthesis method of Compound UB-180955

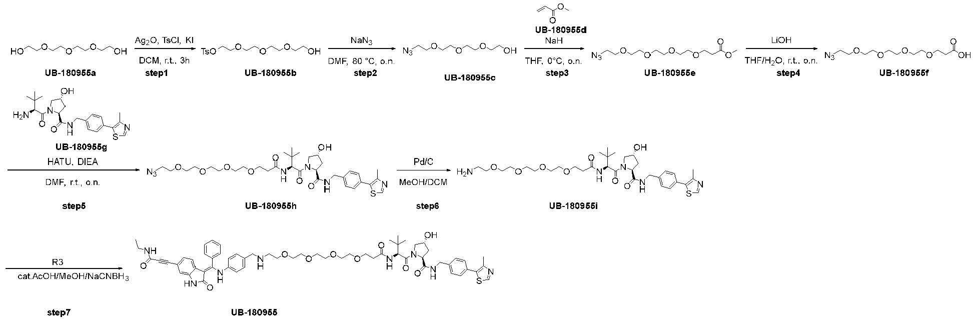

### Step 1: UB-180955b (V2128-092)

Compound **UB-180955a** (10 g, 51.5 mmol), Ag₂O (18 g, 77.3 mmol) and KI (17 g, 10.3 mmol) were dissolved in dichloromethane (40 mL), then TsCl (10.8 g, 56.7 mmol) was added and the mixture was reacted at room temperature for 3 hours. The reaction solution was filtered and washed with water, extracted with dichloromethane (20 mL). The organic phase was concentrated and isolated by column chromatography to obtain target product **UB-180955b** (9 g, yield 50%) as a colorless oil. LCMS: [M+H] ⁺ = 349.2

### Step 2: UB-180955c (V2128-095)

**Compound UB-180955b** (9 g, 258.6 mmol) was dissolved in DMF (50 mL), then NaN₃ (3.4 g, 517.2 mmol) was added, and the mixture was heated to 85°C and reacted for 12 hours. The reaction solution was filtered, then concentrated and isolated by column chromatography to obtain target product **UB-180955c** (5.3 g, yield 92%) as a colorless oil.

### Step 3: UB-180955e (V2128-138)

At 0°C, compound **UB-180955c** (2 g, 9.1 mmol) was dissolved in DMF (20 mL), then NaH (37 mg, 0.91 mmol) was added, and the mixture was reacted at room temperature for 1 hour, followed by adding **UB-180955d** (786 mg, 9.1 mmol) and continued to react at room temperature overnight. The reaction solution was washed with water, then extracted three times with ethyl acetate (20 mL), the organic phase was concentrated, then isolated by column chromatography to obtain target product **UB-180955e** (1.6 g, yield 57%) as yellow oil. ¹H NMR (400 MHz, Chloroform-*d*) δ 3.76 (t, *J* = 6.5 Hz, 2H), 3.69 (s, 4H), 3.70 - 3.58 (m, 15H), 3.40 (d, *J* = 5.1 Hz, 2H), 2.61 (t, *J* = 6.5 Hz, 2H). LCMS: [M+H] ⁺ = 306.3

### Step 4: UB-180955f (V2128-148)

Compound **UB-180955e** (150 mg, 0.49 mmol) was dissolved in H₂O/THF (4 mL), then LiOH(41 mg, 0.98 mmol) was added and the mixture was reacted at room temperature for 1 hour. The reaction solution was added water to dilute, then washed once with ethyl acetate, and the aqueous phase was adjusted to pH 3, and then concentrated to obtain the target product **UB-180955f** (130 mg, yield 95%). LCMS: [M+H] ⁺ = 292.2

### Step 5: UB-180955h (V2330-003)

Compound **UB-180955f** (115 mg, 0.395 mmol) and HATU (300 mg, 0.79 mmol) were dissolved in DMF (20 ml), then DIPEA (153 mg, 1.19 mmol) was added, the mixture was reacted at room temperature for 1 hour. **UB-180955g** (170 mg, 0.395 mmol) was further added, then continued to react at room temperature overnight. The reaction solution was concentrated and isolated by column chromatography (methanol/dichloromethane = 10/1) to obtain target product **UB-180955h** (170 mg, yield 61%) as yellow solid.

¹H NMR (400 MHz, DMSO-*d*₆) δ 8.98 (s, 1H), 8.57 (t, *J=* 6.1 Hz, 1H), 7.92 (d, *J=* 9.4 Hz, 1H), 7.40 (q, *J* = 8.4 Hz, 4H), 5.13 (d, J = 3.6 Hz, 1H), 4.55 (d, J = 9.4 Hz, 1H), 4.51 - 4.3 7 (m, 2H), 4.35 (s, 1H), 4.22 (dd, *J* = 15.9, 5.5 Hz, 1H), 3.68 - 3.44 (m, 17H), 3.39 (dd, *J* = 5.7, 4.3 Hz, 2H), 2.55 (dd, *J=* 14.5, 6.9 Hz, 1H), 2.44 (s, 3H), 2.35 (dt, *J=* 14.6, 6.1 Hz, 1H), 2.09 - 1.99 (m, 1H), 1.95 - 1.87 (m, 1H), 1.32 - 1.08 (m, 1H), 0.94 (s, 9H). LCMS: [M+H] ⁺ = 704.4

### Step 6: UB-180955i (V2330-007)

Compound **UB-180955h** (70 mg, 0.099 mmol) was dissolved in methanol (20 mL), then **Pd/C** (2.12 mg, 0.02 mmol) was added, and the mixture was reacted at room temperature for 1 hour under hydrogen condition. The reaction solution was filtered, and the filtrate was concentrated to obtain target product **UB-180955i** (60 mg, yield 90%) as a yellow solid. LCMS: [M+H] ⁺ = 678.4

### Step 5: UB-180955 (V2235-046)

### The method is similar to General Method 5

¹H NMR (400 MHz, DMSO-*d*₆) δ 12.15 (s, 1H), 10.91 (s, 1H), 8.98 (s, 1H), 8.60 (dt, *J* = 25.3, 5.8 Hz, 2H), 7.92 (d, *J* = 9.3 Hz, 1H), 7.56 (q, *J* = 6.2 Hz, 3H), 7.51 - 7.44 (m, 2H), 7.40 (q, *J=* 8.1 Hz, 4H), 7.10 (d, *J=* 8.1 Hz, 2H), 6.98 (d, *J=* 1.5 Hz, 1H), 6.76 (dd, *J=* 12.0, 8.5 Hz, 3H), 5.77 (d, *J* = 8.1 Hz, 1H), 5.13 (s, 1H), 4.55 (d, *J* = 9.4 Hz, 1H), 4.48 - 4.36 (m, 2H), 4.35 (s, 1H), 4.22 (dd, *J=* 15.9, 5.5 Hz, 1H), 3.69 - 3.61 (m, 2H), 3.57 (s, 3H), 3.47 (m, 13H), 3.42 (m, 2H), 3.18 - 3.02 (m, 2H), 2.56 (t, *J* = 5.9 Hz, 2H), 2.44 (s, 3H), 2.35 (dt, *J* = 14.7, 6.2 Hz, 1H), 2.09 - 1.97 (m, 1H), 1.95 - 1.85 (m, 1H), 1.03 (t, *J=* 7.2 Hz, 3H), 0.93 (s, 9H). LCMS [M+H]⁺ = 1097.6

### Synthesis method of Compound UB-180956

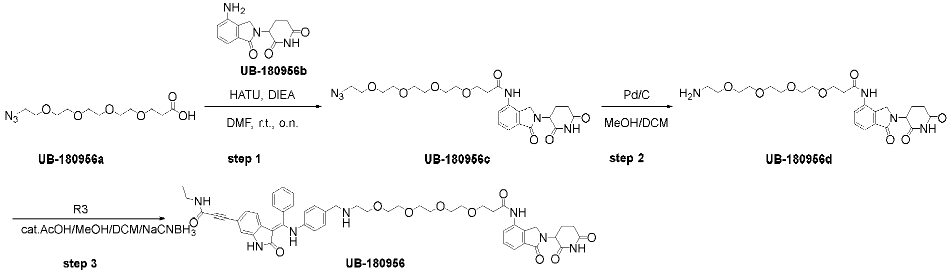

### Step 1: UB-180956c (V2235-044)

Compound **UB-180956a** (150 mg, 0.52 mmol) was dissolved in DMF (3 mL), then HATU (392 mg, 1.03 mmol) and DIEA (199 mg, 1.55 mmol) were added, and the mixture was reacted at room temperature for 20 minutes, followed by adding **UB-180956b** (134 mg, 0.52 mmol) and continued to react at room temperature overnight. The reaction solution was added water (10 mL), extracted with ethyl acetate (10 mL), the organic phase was concentrated, then isolated by column chromatography (methanol/dichloromethane=0 to 10%) to obtain target product **UB-180956c** (80 mg, yield 29%) as a colorless oil. LCMS: [M+H] ⁺ = 533.4

### Step 2: UB-180956d (V2235-047)

Under hydrogen condition, compound **UB-180956c** (80 mg, 0.15 mmol) was dissolved in methanol (4 mL) and dichloromethane (4 mL), then a catalytic amount of palladium carbon was added, then the mixture was reacted at room temperature for 20 minutes. The reaction solution was filtered, and the filtrate was concentrated to obtain target product **UB-180956d** (75 mg, yield 95%) as a colorless oil. The crude product was directly used in the next reaction. LCMS: [M+H] ⁺ = 507.3

### Step 3: UB-180956 (V2235-049)

### The method is similar to General Method 5

¹H NMR (400 MHz, DMSO-*d*₆) δ 12.15 (s, 1H), 10.92 (s, 1H), 9.83 (s, 1H), 8.63 (t, *J=* 5.6 Hz, 1H), 7.82 (dd, *J* = 7.4, 1.7 Hz, 1H), 7.64 - 7.53 (m, 3H), 7.52 - 7.42 (m, 4H), 7.14 - 7.06 (m, 2H), 6.99 (d, *J=* 1.5 Hz, 1H), 6.88 - 6.71 (m, 3H), 5.77 (d, *J=* 8.0 Hz, 1H), 5.15 (dd, *J=* 13.3, 5.1 Hz, 1H), 4.56 - 4.12 (m, 2H), 3.71 (t, *J=* 6.3 Hz, 2H), 3.57 (s, 2H), 3.51 (h, J= 2.6 Hz, 3H), 3.49 - 3.42 (m, 11H), 3.18 - 3.07 (m, 2H), 2.92 (ddd, *J=* 17.3, 13.6, 5.5 Hz, 1H), 2.59 (ddt, *J* = 19.9, 11.5, 4.9 Hz, 5H), 2.40 - 2.24 (m, 1H), 2.02 (dtd, *J* = 11.2, 5.9, 3.2 Hz, 1H), 1.03 (t, *J=* 7.2 Hz, 3H). LCMS [M+H]⁺ = 926.5

### General procedure for synthesis of UB-180957

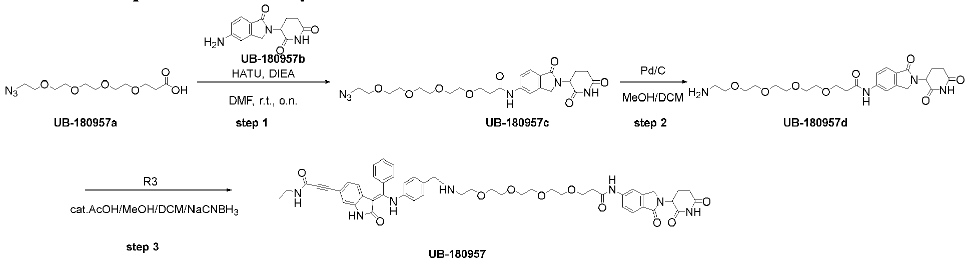

### Step 1: UB-180957c (V2235-045)

Compound **UB-180957a** (150 mg, 0.52 mmol) was dissolved in DMF (3 mL), then HATU (392 mg, 1.03 mmol) and DIEA (199 mg, 1.55 mmol) were added, and the mixture was reacted for 20 minutes, followed by adding **UB-180957b** (134 mg, 0.52 mmol) and continued to react at room temperature overnight. The reaction solution was diluted with water (10 mL), extracted with ethyl acetate (10 mL), and the organic phase was concentrated, then isolated by column chromatography (methanol/dichloromethane=0 to 10%) to obtain target product **UB-180957c** (80 mg, yield 29%) as a colorless oil.

### Step 2: UB-180957d (V2235-047)

Under hydrogen condition, compound **UB-180957c** (80 mg, 0.15 mmol) was dissolved in methanol (4 mL) and dichloromethane (4 mL), then a catalytic amount of palladium carbon was added, then the mixture was reacted at room temperature for 20 minutes. The reaction solution was filtered, then the filtrate was concentrated to obtain crude product **UB-180957d** (75 mg, yield 95%) as a colorless oil. The crude product was directly used in the next reaction. LCMS: [M+H] ⁺ = 507.3

### Step 3: UB-180957 (V2235-050)

### The method is similar to General Method 5

¹H NMR (400 MHz, DMSO-*d*₆) δ 12.15 (s, 1H), 10.92 (s, 1H), 10.31 (s, 1H), 8.64 (t, *J* = 5.6 Hz, 1H), 7.98 (s, 1H), 7.70 - 7.52 (m, 5H), 7.49 - 7.44 (m, 2H), 7.15 - 7.04 (m, 2H), 6.99 (d, *J* = 1.6 Hz, 1H), 6.85 - 6.68 (m, 3H), 5.76 (d, *J* = 8.1 Hz, 1H), 5.08 (dd, *J* = 13.3, 5.1 Hz, 1H), 4.53 - 4.19 (m, 2H), 3.70 (t, *J=* 6.2 Hz, 2H), 3.57 (m, 2H), 3.48 (ddt, *J=* 13.5, 4.5, 2.1 Hz, 8H), 3.41 (q, *J=* 6.3, 5.7 Hz, 3H), 3.17 - 3.07 (m, 2H), 2.96 - 2.84 (m, 1H), 2.63 - 2.52 (m, 4H), 2.07 - 1.90 (m, 1H), 1.03 (t, *J* = 7.2 Hz, 3H). LCMS [M+H]⁺ = 926.5

### Synthesis method of Compound UB-180958

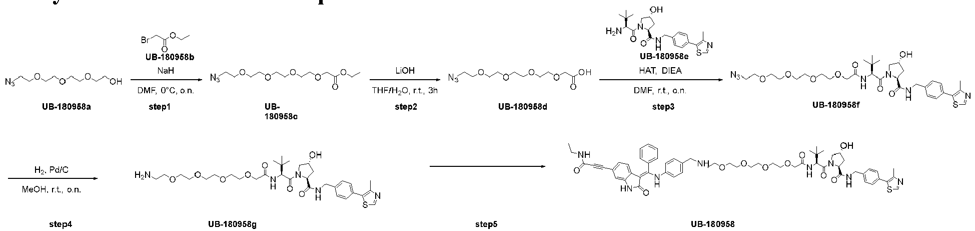

### Step 1: UB-180958c (V2128-146)

At 0°C, compound **UB-180958a** (2 g, 9.13 mmol) was dissolved in DMF(20 mL), then NaH (731 mg, 18.3 mmol) was added, and the mixture was reacted at room temperature for 1 hour, followed by adding **UB-180958b(1.5** g, 9.13 mmol) and then reacted at room temperature overnight. The reaction solution was washed with water, extracted with ethyl acetate (10 mL) three times. The organic phase was concentrated and isolated by column chromatography to obtain target product **UB-180958c** (880 mg, yield 32%) as a yellow oil. LCMS: [M+H] ⁺ = 306.2

### Step 2: UB-180958d (V2128-149)

Compound **UB-180958c** (500 mg, 1.6 mmol) was dissolved in H₂O/THF (8 mL), then LiOH(137 mg, 3.3 mmol) was added and the mixture was reacted at room temperature for 1 hour. The reaction solution was added water to dilute, then washed once with ethyl acetate, the aqueous phase was adjusted to pH 3, and then concentrated to obtain the target product **UB-180958d** (390 mg, yield 86%). LCMS: [M+H] ⁺ = 278.2

### Step 3: UB-180958f (V2330-004)

Compound **UB-180958d** (120 mg, 0.433 mmol) and HATU (329 mg, 0.87 mmol) were dissolved in DMF (20 ml), then DIPEA (168 mg, 1.3 mmol) was added, and the mixture was reacted at room temperature for 1 hour. **UB-180958e** (187 mg, 0.433 mmol) was further added, and continued to react at room temperature overnight. The reaction solution was concentrated and isolated by column chromatography (dichloromethane/ methanol = 10/1) to obtain target product **UB-180958f** (200 mg, yield 67%) as a yellow solid.

¹H NMR (400 MHz, DMSO-*d*₆) δ 8.98 (s, 1H), 8.60 (s, 1H), 7.43 (s, 1H), 7.40 (s, 4H), 5.76 (s, 1H), 5.15 (d, *J* = 3.6 Hz, 1H), 4.57 (d, *J* = 9.6 Hz, 1H), 4.45 - 4.35 (m, 2H), 4.27 (d, *J* = 5.6 Hz, 1H), 3.97 (s, 1H), 3.66 (d, *J=* 3.9 Hz, 1H), 3.62 - 3.49 (m, 14H), 3.37 (dd, *J=* 5.6, 4.2 Hz, 2H), 2.44 (s, 3H), 2.05 (dt, *J =* 9.4, 5.2 Hz, 1H), 1.90 (s, 1H), 1.28 - 1.21 (m, 2H), 0.95 (s, 9H). LCMS: [M+H] ⁺ = 690.3

### Step 4: UB-180958g (V2330-010)

Under hydrogen condition, compound **UB-180958f** (110 mg, 0.159 mmol) was dissolved in methanol (5 mL), then Pd/C (3.4 mg, 0.032 mmol) was added, and the mixture was reacted at room temperature for 1 hour. The reaction solution was filtered, and the filtrate was concentrated to obtain target crude product **UB-180958g** (96 mg, yield 91%). LCMS: [M+H] ⁺ = 664.3

### Step 5: UB-180958 (V2235-051)

### The method is similar to General Method 5

¹H NMR (400 MHz, DMSO-*d*₆) δ 12.15 (s, 1H), 10.91 (s, 1H), 8.97 (s, 1H), 8.61 (dt, *J* = 13.1, 5.8 Hz, 2H), 7.56 (q, *J=* 6.0 Hz, 3H), 7.52 - 7.34 (m, 7H), 7.17 - 7.05 (m, 2H), 6.98 (d, *J* = 1.6 Hz, 1H), 6.87 - 6.69 (m, 3H), 5.76 (d, *J* = 8.2 Hz, 1H), 5.16 (s, 1H), 4.56 (d, *J* = 9.6 Hz, 1H), 4.50 - 4.31 (m, 3H), 4.24 (dd, *J=* 15.7, 5.6 Hz, 1H), 3.96 (s, 2H), 3.75 - 3.37 (m, 17H), 3.20 - 3.04 (m, 2H), 2.54 (t, *J* = 5.8 Hz, 2H), 2.43 (s, 3H), 2.13 - 1.96 (m, 2H), 1.90 (ddd, *J*= 13.0, 8.8, 4.5 Hz, 1H), 1.03 (t, *J=* 7.2 Hz, 3H), 0.94 (s, 9H). LCMS [M+H]⁺ = 1083.6

### Synthesis method of Compound UB-180962

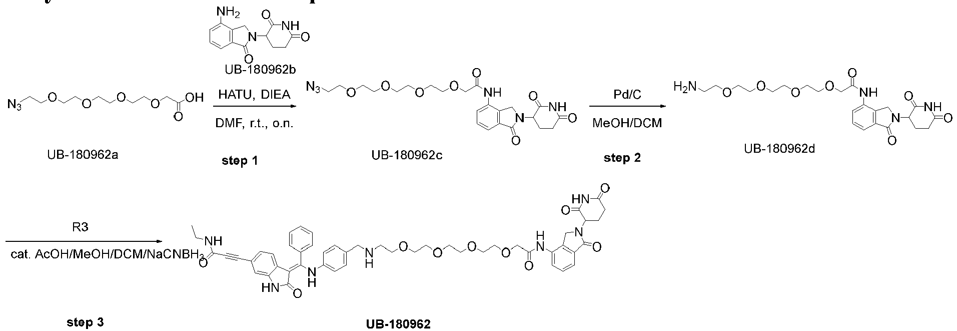

### Step 1: UB-180962c (V2330-017)

Compound **UB-180962a** (140 mg, 0.51 mmol) and HATU (384 mg, 1.01 mmol) were dissolved in DMF (10 ml), then DIPEA (196 mg, 1.5 mmol) was added, and the mixture was reacted at room temperature for 1 hour. **UB-180962b** (131 mg, 0.51 mmol) was further added, and then reacted at room temperature overnight. The reaction solution was concentrated and isolated by column chromatography (methanol/dichloromethane = 10/1) to obtain target product **UB-180962c** (125 mg, yield 48 %) as a yellow solid. LCMS: [M+H] ⁺ = 519.3

### Step 2: UB-180962d (V2330-019)

Under hydrogen condition, compound **UB-180962c** (70 mg, 0.135 mmol) was dissolved in dichloromethane/methanol (10 mL), then Pd/C (2.9 mg, 0.027 mmol) was added, and the mixture was reacted at room temperature for 1 hour. The reaction solution was filtered, then the filtrate was concentrated to obtain target product **UB-180962d** (61 mg, yield 92%) as a white solid. LCMS: [M+H] ⁺ = 493.3

### Step 3: UB-180962 (V2235-052)

### The method is similar to General Method 5

¹H NMR (400 MHz, DMSO-*d*₆) δ 12.15 (s, 1H), 10.92 (s, 1H), 9.67 (s, 1H), 8.63 (t, *J=* 5.6 Hz, 1H), 7.74 (d, *J* = 7.7 Hz, 1H), 7.63 - 7.38 (m, 7H), 7.09 (d, *J* = 8.0 Hz, 2H), 6.99 (d, *J* = 1.6 Hz, 1H), 6.86 - 6.63 (m, 3H), 5.76 (d, *J=* 8.1 Hz, 1H), 5.14 (dd, *J=* 13.3, 5.1 Hz, 1H), 4.57 - 4.25 (m, 2H), 4.13 (s, 2H), 3.68 (dd, *J=* 5.8, 3.3 Hz, 2H), 3.60 (dd, *J=* 5.8, 3.3 Hz, 2H), 3.58 - 3.46 (m, 6H), 3.46 - 3.36 (m, 8H), 3.19 - 3.05 (m, 2H), 2.91 (ddd, *J=* 18.1, 13.6, 5.4 Hz, 1H), 2.59 (ddd, *J=* 17.2, 4.3, 2.3 Hz, 1H), 2.54 (t, *J=* 5.7 Hz, 2H), 2.36 (qd, *J=* 13.2, 4.4 Hz, 1H), 2.00 (ddd, *J=* 11.2, 6.3, 3.7 Hz, 1H), 1.03 (t, *J=* 7.2 Hz, 3H). LCMS [M+H]⁺ = 912.6

### Synthesis method of Compound UB-180963

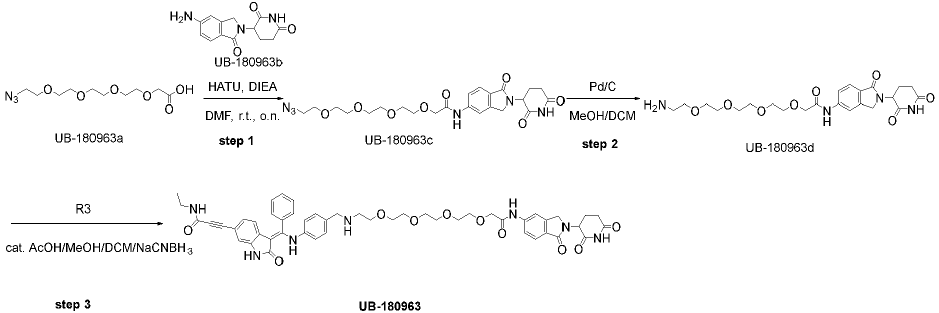

### Step 1: UB-180963c (V2330-023)

Compound **UB-180963a** (129 mg, 0.47 mmol) and HATU (354 mg, 0.93 mmol) were dissolved in DMF (10 ml), then DIPEA (180 mg, 1.40 mmol) was added, and the mixture was reacted at room temperature for 1 hour. **UB-180963b** (121 mg, 0.47 mmol) was further added, and then reacted at room temperature overnight. The reaction solution was concentrated and isolated by column chromatography (dichloromethane/ methanol = 10/1) to obtain target product **UB-180963c** (145 mg, yield 60 %) as a yellow solid. LCMS: [M+H] ⁺ = 519.3

### Step 2: UB-180963d (V2330-026)

Under hydrogen condition, compound **UB-180963c** (70 mg, 0.135 mmol) was dissolved in dichloromethane/methanol (10 mL), then Pd/C (2.9 mg, 0.027 mmol) was added, and the mixture was reacted at room temperature for 1 hour. The reaction solution was filtered, then the filtrate was concentrated to obtain target product **UB-180963d** (61 mg, yield 92%) as a white solid. LCMS: [M+H] ⁺ = 493.3

### Step 3: UB-180963 (V2235-054)

### The method is similar to General Method 5

¹H NMR (400 MHz, DMSO-*d*₆) δ 12.15 (s, 1H), 10.92 (d, J= 3.0 Hz, 1H), 9.96 (s, 1H), 8.63 (t, *J=* 5.7 Hz, 1H), 8.00 (s, 1H), 7.73 - 7.39 (m, 7H), 7.17 - 7.04 (m, 2H), 6.98 (d, *J=* 1.6 Hz, 1H), 6.83 - 6.69 (m, 3H), 5.76 (d, *J=* 8.0 Hz, 1H), 5.08 (dd, *J=* 13.3, 5.1 Hz, 1H), 4.55 - 4.23 (m, 2H), 4.12 (d, *J=* 2.3 Hz, 2H), 3.71 - 3.64 (m, 2H), 3.63 - 3.46 (m, 10H), 3.47 - 3.40 (m, 4H), 3.17 - 3.06 (m, 2H), 2.99 - 2.80 (m, 1H), 2.55 (t, *J* = 5.7 Hz, 2H), 2.41 - 2.31 (m, 1H), 2.07 (s, 1H), 1.99 (ddt, *J* = 9.7, 5.3, 2.4 Hz, 1H), 1.03 (t, *J* = 7.2 Hz, 3H). LCMS [M+H]⁺ = 912.5

### Synthesis method of Compound UB-180964

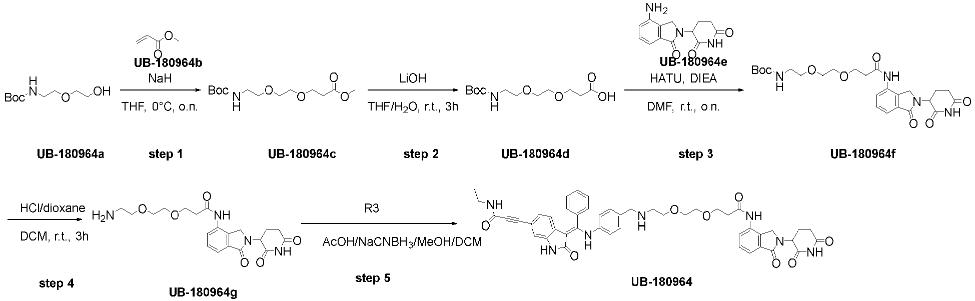

### Step 1: UB-180964c (V2128-137)

At 0°C, compound **UB-180964a** (1 g, 4.9 mmol) was dissolved in THF (10 mL), then NaH (19.49 mg, 0.49 mmol) was added, and continued to react for 1 hour. **UB-180964b** (419 mg, 4.9 mmol) was further added, and then reacted at room temperature overnight. The reaction solution was added with water then extracted with ethyl acetate, the organic phase was concentrated, then isolated by column chromatography to obtain target product **UB-180964c** (440 mg, yield 32%) as a yellow oil.

¹H NMR (400 MHz, Chloroform-*d*) δ 5.02 (s, 1H), 3.77 (t, *J* = 6.5 Hz, 2H), 3.70 (s, 3H), 3.60 (d, *J* = 1.3 Hz, 4H), 3.53 (d, *J* = 5.2 Hz, 2H), 3.31 (q, *J* = 5.0 Hz, 2H), 2.62 (t, *J* = 6.5 Hz, 2H), 1.45 (s, 9H). LCMS: [M+H] ⁺ = 292.3

### Step 2: UB-180964d (V2330-024)

Compound **UB-180964c** (500 mg, 1.72 mmol) was dissolved in H₂O/THF (8 mL), then LiOH(108 mg, 2.6 mmol) was added and the mixture was reacted at room temperature for 1 hour. The reaction solution was added water to dilute, then washed once with ethyl acetate, and the aqueous phase was adjusted to pH 6, and then concentrated to obtain the target crude product **UB-180964d** (300 mg, yield 63 %). LCMS: [M+H] ⁺ = 278.2

### Step 3: UB-180964f (V2330-025)

Compound **UB-180964d** (95 mg, 0.34 mmol) and HATU (261 mg, 0.69 mmol) were dissolved in DMF (10 ml), then DIPEA (133 mg, 1.03 mmol) was added, and the mixture was reacted at room temperature for 1 hour. **UB-180964e** (89 mg, 0.34 mmol) was further added, and then reacted at room temperature overnight. The reaction solution was concentrated and isolated by column chromatography (dichloromethane/ methanol = 10/1) to obtain target product **UB-180964f** (66 mg, yield 37%) as a yellow solid.

¹H NMR (400 MHz, DMSO) δ 11.02 (s, 1H), 9.83 (s, 1H), 7.82 (d, *J=* 7.0 Hz, 1H), 7.50 (q, *J* = 7.3 Hz, 2H), 6.74 (s, 1H), 5.15 (dd, *J* = 13.3, 5.1 Hz, 1H), 4.35 (q, *J* = 17.5 Hz, 2H), 3.71 (t, *J=* 6.3 Hz, 2H), 3.51 (d, J= 4.9 Hz, 4H), 3.36 (t, J= 6.1 Hz, 2H), 3.04 (dd, *J* = 11.7, 5.7 Hz, 2H), 2.90 (dd, *J* = 13.1, 4.7 Hz, 1H), 2.65 - 2.57 (m, 3H), 2.33 (dd, *J* = 12.9, 4.3 Hz, 1H), 2.08 - 2.00 (m, 1H), 1.36 (s, 9H). LCMS: [M+H] ⁺ = 519.4

### Step 4: UB-180964g (V2330-027)

Compound **UB-180964f** (60 mg, 0.12 mmol) was dissolved in dichloromethane (1 mL), then 4M HCl/dioxane (2 mL) was added, and the mixture was reacted at room temperature for 1 hour. The reaction solution was concentrated to obtain target product **UB-180964g** (30 mg, yield 62%). LCMS: [M+H] ⁺ = 419.3

### Step 5: UB-180964 (V2235-055)

### The method is similar to General Method 5

¹H NMR (400 MHz, DMSO-*d*₆) δ 12.15 (s, 1H), 10.91 (s, 1H), 9.83 (s, 1H), 8.63 (t, *J=* 5.6 Hz, 1H), 7.81 (d, *J* = 7.5 Hz, 1H), 7.65 - 7.31 (m, 7H), 7.07 (d, *J* = 8.2 Hz, 2H), 6.98 (s, 1H), 6.76 (dd, *J=* 8.2, 5.8 Hz, 3H), 5.76 (d, *J=* 8.1 Hz, 1H), 5.15 (dd, *J=* 13.3, 5.2 Hz, 1H), 4.44 - 4.20 (m, 2H), 3.70 (t, *J=* 6.3 Hz, 2H), 3.56 - 3.49 (m, 4H), 3.46 (dd, *J=* 6.1, 3.6 Hz, 3H), 3.40 (m, 4H), 3.12 (p, *J* = 7.0 Hz, 2H), 2.91 (ddd, *J* = 18.0, 13.7, 5.5 Hz, 1H), 2.67 - 2.55 (m, 3H), 2.32 (qd, J= 13.2, 4.2 Hz, 1H), 2.11 - 1.96 (m, 1H), 1.03 (t, *J* = 7.2 Hz, 3H). LCMS [M+H] ⁺ = 838.5

### Synthesis method of Compound UB-180967

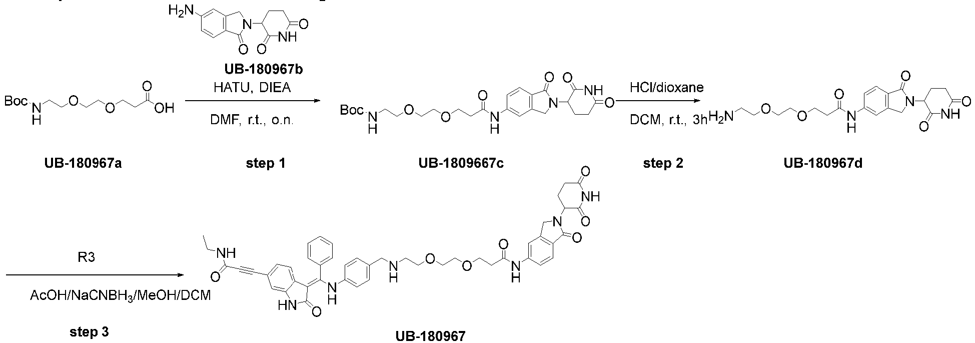

### Step 1: UB-180967c (V2330-035)

Compound **UB-180967a** (210 mg, 0.76 mmol) and HATU (576 mg, 1.52 mmol) were dissolved in DMF (10 ml), then DIPEA (293 mg, 2.27 mmol) was added, and the mixture was reacted at room temperature for 1 hour. **UB-180967b** (196 mg, 0.76 mmol) was further added, and then reacted at room temperature overnight. The reaction solution was concentrated and isolated by column chromatography (dichloromethane/methanol = 10/1) to obtain target product **UB-180967c** (180 mg, yield 46%) as a yellow solid.

¹H NMR (400 MHz, DMSO) δ 10.97 (s, 1H), 10.27 (s, 1H), 7.98 (s, 1H), 7.62 (dt, *J* = 8.3, 5.0 Hz, 2H), 6.74 (s, 1H), 5.08 (dd, *J* = 13.3, 5.1 Hz, 1H), 4.36 (dd, *J* = 56.2, 17.3 Hz, 2H), 3.71 (t, *J=* 6.2 Hz, 2H), 3.50 (ddd, *J* = 10.0, 6.3, 3.8 Hz, 4H), 3.36 (t, *J=* 6.1 Hz, 2H), 3.04 (dd, *J=* 11.9, 5.9 Hz, 2H), 2.93 - 2.85 (m, 1H), 2.60 (t, *J=* 6.2 Hz, 3H), 2.37 (dd, *J=* 13.1, 4.5 Hz, 1H), 1.99 (t, *J=* 5.2 Hz, 1H), 1.35 (d, *J=* 9.5 Hz, 9H). LCMS: [M+H] ⁺ = 519.3

### Step 2: UB-180967d (V2591-036)

Compound **UB-180967c** (50 mg, 0.1 mmol) was dissolved in dichloromethane (1 mL), then 4M HCl/dioxane (2 mL) was added, and the mixture was reacted at room temperature for 1 hour. The reaction solution was concentrated to obtain target product **UB-180967d(35** mg, yield 87%) as a white solid. LCMS: [M+H] ⁺ = 419.2

### Step 3: UB-180967 (V2235-057)

### The method is similar to General Method 5

¹H NMR (400 MHz, DMSO-*d*₆) δ 12.14 (s, 1H), 10.92 (s, 1H), 10.30 (s, 1H), 8.64 (t, *J=* 5.6 Hz, 1H), 7.98 (s, 1H), 7.69 - 7.49 (m, 5H), 7.49 - 7.40 (m, 2H), 7.08 (d, *J* = 8.2 Hz, 2H), 6.99 (d, J= 1.6 Hz, 1H), 6.76 (dq, J= 7.7, 6.0 Hz, 3H), 5.76 (d, J= 8.0 Hz, 1H), 5.07 (dd, J= 13.2, 5.2 Hz, 1H), 4.61 - 4.15 (m, 3H), 3.70 (t, *J=* 6.1 Hz, 3H), 3.56 - 3.48 (m, 5H), 3.45 (dd, *J* = 6.2, 3.7 Hz, 4H), 3.18 - 3.06 (m, 2H), 2.90 (ddd, *J* = 17.9, 13.7, 5.6 Hz, 1H), 2.58 (q, *J* = 5.5, 4.9 Hz, 3H), 2.36 (dd, *J=* 13.2, 4.6 Hz, 1H), 2.08 - 1.84 (m, 2H), 1.03 (t, *J=* 7.2 Hz, 3H). LCMS [M+H]⁺= 838.5

### Synthesis method of Compound UB-180970

### Step 1: UB-180970 (V2235-059)

### The method is similar to General Method 5

¹H NMR (400 MHz, DMSO-*d*₆) δ12.16 (s, 1H), 10.92 (s, 1H), 8.98 (d, *J=* 3.7 Hz, 1H), 8.60 (dt, *J=* 25.3, 5.8 Hz, 2H), 7.91 (d, *J=* 9.4 Hz, 1H), 7.69 - 7.51 (m, 3H), 7.50 - 7.33 (m, 6H), 7.19 - 7.05 (m, 2H), 6.98 (d, *J=* 1.6 Hz, 1H), 6.83 - 6.62 (m, 3H), 5.76 (d, *J=* 8.1 Hz, 1H), 5.23 - 5.08 (m, 1H), 4.55 (d, *J* = 9.4 Hz, 1H), 4.47 - 4.39 (m, 2H), 4.35 (s, 1H), 4.21 (dd, *J* = 15.8, 5.4 Hz, 1H), 3.71 - 3.53 (m, 6H), 3.52 - 3.44 (m, 6H), 3.41 (t, *J* = 5.8 Hz, 3H), 3.12 (qd, *J* = 7.2, 5.5 Hz, 2H), 2.55 (t, *J* = 6.0 Hz, 2H), 2.44 (s, 3H), 2.34 (dt, *J* = 14.6, 6.1 Hz, 1H), 2.02 (q, *J=* 9.7, 7.4 Hz, 2H), 1.90 (ddd, *J=* 13.0, 8.6, 4.6 Hz, 1H), 1.03 (t, *J=* 7.2 Hz, 3H), 0.93 (s, 9H). LCMS [M+H]⁺ = 1053.6

### Synthesis method of Compound UB-180971

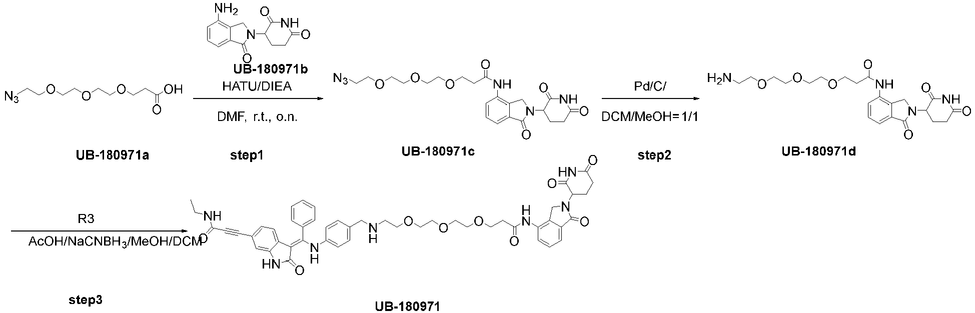

### Step 1: UB-180971c (V2330-034)

Compound **UB-180971a** (110 mg, 0.445 mmol) and HATU (338 mg, 0.89 mmol) were dissolved in DMF (10 ml), then DIPEA (172 mg, 1.34 mmol) was added, and the mixture was reacted at room temperature for 1 hour. **UB-180971b** (115 mg, 0.445 mmol) was further added, and then reacted at room temperature overnight. The reaction solution was concentrated and isolated by column chromatography (methanol/dichloromethane = 10/1) to obtain target product **UB-180971c** (65 mg, yield 30 %) as a colorless oil.

¹H NMR (400 MHz, DMSO) δ 11.02 (s, 1H), 9.83 (s, 1H), 7.82 (dd, *J* = 7.0, 1.9 Hz, 1H), 7.56 - 7.40 (m, 2H), 5.15 (dd, *J=* 13.3, 5.1 Hz, 1H), 4.35 (q, *J=* 17.5 Hz, 2H), 3.72 (t, *J=* 6.3 Hz, 2H), 3.58 - 3.48 (m, 10H), 3.38 - 3.34 (m, 2H), 2.98 - 2.86 (m, 1H), 2.61 (t, *J* = 6.4 Hz, 3H), 2.33 (dd, *J=* 13.1, 4.5 Hz, 1H), 2.06 - 1.98 (m, 1H). LCMS: [M+H] ⁺ = 489.2

### Step 2: UB-180971d (V2235-058)

Under hydrogen condition, compound **UB-180971c** (60 mg, 0.12 mmol) was dissolved in methanol /dichloromethane (3/9 mL), then a catalytic amount of palladium carbon was added, and the mixture was reacted at room temperature for 1 hour. The reaction solution was filtered, the filtrate was concentrated to obtain crude product **UB-180971d** (55 mg, yield 93 %) as a yellow solid. The crude product was directly used in the next reaction.

### Step 3: UB-180971 (V2235-060)

### The method is similar to General Method 5

¹H NMR (400 MHz, DMSO-*d*₆) δ 12.15 (s, 1H), 10.92 (s, 1H), 9.84 (s, 1H), 8.63 (t, *J=* 5.7 Hz, 1H), 7.81 (dd, *J=* 7.4, 1.6 Hz, 1H), 7.68 - 7.37 (m, 7H), 7.09 (d, *J=* 8.5 Hz, 2H), 6.98 (d, *J=* 1.6 Hz, 1H), 6.88 - 6.63 (m, 3H), 5.76 (d, *J=* 8.1 Hz, 1H), 5.15 (dd, *J=* 13.3, 5.1 Hz, 1H), 4.49 - 4.13 (m, 2H), 3.70 (t, *J=* 6.3 Hz, 2H), 3.55 (s, 2H), 3.52 - 3.45 (m, 6H), 3.45 - 3.36 (m, 4H), 3.19 - 3.06 (m, 2H), 3.05 - 2.84 (m, 1H), 2.70 - 2.56 (m, 3H), 2.55 - 2.51 (m, 2H), 2.32 (dd, *J=* 13.2, 4.5 Hz, 1H), 2.10 - 1.99 (m, 1H), 1.03 (t, *J=* 7.2 Hz, 3H). LCMS [M+H] ⁺ = 882.5

### Synthesis method of Compound UB-180972

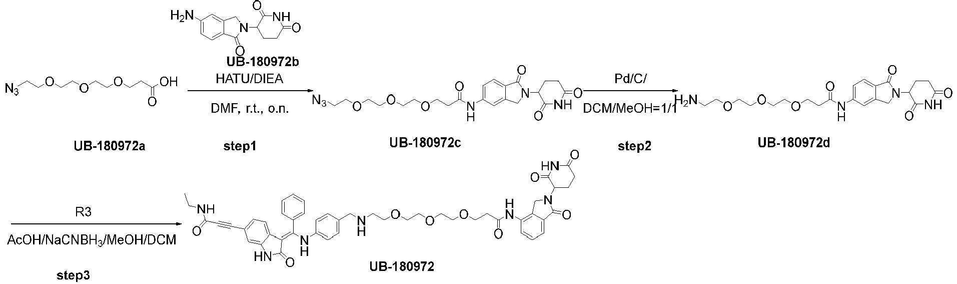

### Step 1: UB-180972c (V2330-038)

Compound **UB-180972a** (180 mg, 0.73 mmol) and HATU (554 mg, 1.5 mmol) were dissolved in DMF (10 ml), then DIPEA (282 mg, 2.2 mmol) was added, then the mixture was reacted at room temperature for 1 hour. **UB-180972b** (189 mg, 0.73 mmol) was further added, and then reacted at room temperature overnight. The reaction solution was concentrated and isolated by column chromatography (methanol/dichloromethane = 10/1) to obtain target product **UB-180972c** (80 mg, yield 22 %) as a yellow solid.

¹H NMR (400 MHz, DMSO) δ 10.97 (s, 1H), 10.29 (s, 1H), 7.99 (s, 1H), 7.62 (dt, *J* = 8.4, 4.8 Hz, 2H), 5.08 (dd, *J=* 13.3, 5.1 Hz, 1H), 4.36 (dd, *J=* 56.0, 17.3 Hz, 2H), 3.72 (t, *J=* 6.2 Hz, 2H), 3.60 - 3.46 (m, 10H), 3.38 - 3.34 (m, 2H), 2.96 - 2.84 (m, 1H), 2.61 (t, *J=* 6.1 Hz, 3H), 2.37 (dd, *J=* 13.2, 4.5 Hz, 1H), 1.99 (dd, *J=* 9.0, 3.6 Hz, 1H). LCMS: [M+H] ⁺ = 489.3

### Step 2: UB-180972d (V2330-039)

Under hydrogen condition, compound **UB-180972c** (36 mg, 0.074 mmol) was dissolved in dichloromethane/methanol (10 mL), then Pd/C (1.57 mg, 0.015 mmol) was added, and the mixture was reacted at room temperature for 1 hour. The reaction solution was filtered, then the filtrate was concentrated to obtain product **UB-180972d** (30 mg, yield 88%) as a white solid. LCMS: [M+H] ⁺ = 463.3

### Step 3: UB-180972 (V2235-061)

### The method is similar to General Method 5

¹H NMR (400 MHz, DMSO-*d*₆) δ 12.15 (s, 1H), 10.91 (s, 1H), 10.28 (s, 1H), 8.63 (t, *J=* 5.7 Hz, 1H), 7.98 (d, *J=* 1.7 Hz, 1H), 7.68 - 7.51 (m, 5H), 7.51 - 7.40 (m, 2H), 7.10 (dd, *J=* 8.1, 5.4 Hz, 2H), 6.99 (d, *J=* 1.6 Hz, 1H), 6.87 - 6.70 (m, 3H), 5.77 (dd, *J=* 8.1, 4.7 Hz, 1H), 5.08 (dd, *J* = 13.3, 5.1 Hz, 1H), 4.50 - 4.12 (m, 2H), 3.70 (t, *J* = 6.2 Hz, 2H), 3.55 (s, 2H), 3.52 - 3.45 (m, 6H), 3.40 (dt, *J* = 11.7, 5.5 Hz, 5H), 3.11 (td, *J* = 7.3, 5.5 Hz, 2H), 2.99 - 2.81 (m, 1H), 2.65 - 2.56 (m, 2H), 2.55 - 2.51 (m, 2H), 2.36 (qd, *J=* 13.1, 4.6 Hz, 1H), 1.98 (dt, *J* = 7.8, 2.7 Hz, 1H), 1.03 (t, *J* = 7.2 Hz, 3H). LCMS [M+H]⁺ = 882.4

### Synthesis method of Compound UB-181199

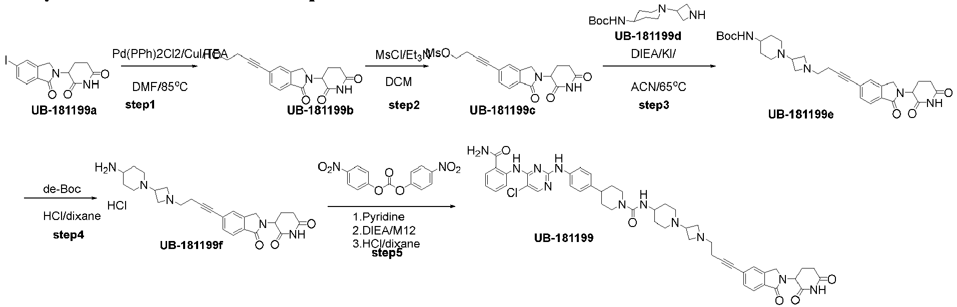

### Step 1: UB-181199b(V2962-061)

**UB-181199a** (500 mg, 1.35 mmol), 3-butyne-1-ol (94 mg, 1.35 mmol), Pd(PPh3)2Cl2(94 mg, 0.135 mmol) and cuprous iodide (51 mg, 0.27 mmol) were added to DMF (2 mL), the mixture was reacted at 80°C for 16 hours under N₂ protection. The mixture was purified by reversed- phase chromatography column (MeOH/H2O=5%-95%, 45min), collected at 60% to obtain compound **UB-181199b** (215mg, yield 54%) as a white solid. LCMS [M+H]⁺ =313.5

### Step 2: UB-181199c (V2962-062)

Compound **UB-181199b** (312mg, 1.00 mmol) and triethylamine (171 mg, 1.50 mmol) were added to dichloromethane (60 mL), methanesulfonyl chloride (125 mg, 1.10 mmol) was slowly added. After reacting at room temperature for 1 hour, the reaction was extracted with DCM (50 mL×3), the combined organic layers was washed with brine (50 mL), dried over anhydrous Na2SO4, concentrated under reduced pressure, purified by column chromatography (DCM:MeOH=10:1) to obtain **UB-181199c** (171 mg, yield 99%) as a white solid. LCMS [M+H]⁺ =391.5

### Step 3: UB-181199d (V2962-064)

Compound **UB-181199c** (78 mg, 0.20 mmol), N,N-diisopropylethylamine (50 mg, 0.39 mmol) and compound**UB-181199d** (51 mg, 0.20 mmol) were added to acetonitrile (30 mL), the mixture was reacted at 80°C for 18 h, concentrated to obtain crude product, which was then purified via silica gel column chromatography (PE/EtOAc=70% to 100%, 20 min, MeOH/DCM=0% to 10%, 40 min) to obtain compound **UB-181199d(51mg,** yield 46%). LCMS [M+H]⁺ =550.5

### Step 4: UB-181199e (V2962-065)

**UB-180972d** (51 mg, 0.09 mmol), and hydrochloric acid/dioxane (10mL, 4N) were added to tetrahydrofuran (10mL), the mixture was reacted at room temperature for 2 hours. After the completion of the reaction, the mixture was concentrated under reduced pressure to obtain compound **UB-181199e** (41 mg, yield 100%). LCMS [M+H]⁺ =450.5

### Step 5: UB-181199 (V2962-066)

### The method is similar to General Method 4

¹H NMR (400 MHz, d6-DMSO) δ11.85 (s, 1H), 10.96 (s, 1H), 9.41 (d, *J=* 14.8 Hz, 1H), 8.79 (d, *J=* 7.6 Hz, 1H), 8.40 - 8.20 (m, 1H), 7.85 - 7.65 (m, 3H), 7.65 - 7.51 (m, 3H), 7.48 (t, *J=* 8.5 Hz, 2H), 7.32 - 7.07 (m, 4H), 6.17 (d, *J=* 7.6 Hz, 1H), 5.17 - 4.99 (m, 1H), 4.44 (d, *J* = 17.6 Hz, 1H), 4.32 (d, *J* = 17.6 Hz, 1H), 4.10 (d, *J* = 13.7 Hz, 2H), 3.61 - 3.47 (m, 1H), 3.38 (d, *J=* 4.51 Hz, 1H), 3.25 (s, 2H), 2.96 - 2.59 (m, 6H), 2.70 - 2.56 (m, 7H), 2.54 - 2.17 (m, 3H), 2.17 - 1.78 (m, 6H), 1.79 - 1.43 (m, 5H), 0.85 (t, *J=* 6.8 Hz, 2H). LCMS [M+H]⁺ =898.9

### Synthesis method of Compound UB-181205

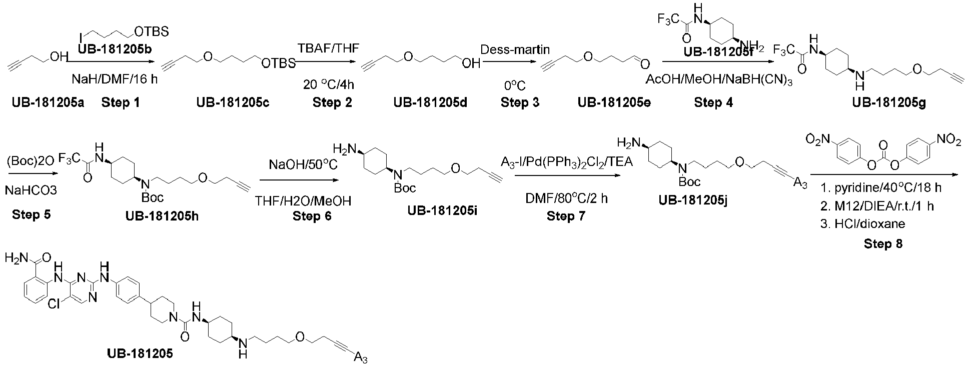

### Step 1: UB-181205c (V2790-109)

Compound **UB-181205a** was dissolved in DMF and cooled to 0°C, NaH was added over 10 minutes. **UB-181205b** in DMF was added to above solution, the mixture was reacted at room temperature for 1 hour. Water (30 mL) was added, the mixture was extracted with EtOAc (30 mL), and the organic phases were combined and concentrated. The crude product was isolated by column chromatography (PE/EA=0 to 10%, 30min) to obtain product **UB-181205c** (3.15 g, yield 80%) as a colorless oil.

¹H NMR (400 MHz, Chloroform-*d*) δ 3.68 - 3.38 (m, 6H), 2.47 - 2.37 (m, 2H), 1.93 (t, *J=* 2.7 Hz, 1H), 1.62 - 1.50 (m, 4H), 0.85 (d, *J=* 1.4 Hz, 9H), 0.00 (d, *J=* 3.4 Hz, 6H).

### Step 2: UB-181205d (V2790-110)

Compound **UB-181205c** was dissolved in THF, TBAF (1 M dissolved in THF) was added, and the mixture was reacted at room temperature overnight. The reaction solution was concentrated and isolated by column chromatography (PE/EA=0 to 50%, 30min) to obtain product **UB-1812035d** (650 mg, yield 80%) as a colorless oil.

¹H NMR (400 MHz, Chloroform-d) δ 3.66 (t, J = 5.8 Hz, 2H), 3.58 (t, J = 6.9 Hz, 2H), 3.52 (t, J = 5.8 Hz, 2H), 2.47 (td, J = 6.9, 2.7 Hz, 2H), 2.24 (s, 1H), 1.99 (t, J = 2.7 Hz, 1H), 1.68 (hd, J = 6.3, 2.7 Hz, 4H).

### Step 3: UB-181205e (V2790-111)

Compound **UB-181205d** was dissolved in DCM and cooled to 0°C, Dess-Martin was added, and the mixture was reacted at room temperature for 4 hours. The reaction was quenched with Na2CO3 solution, and extracted with DCM (10 mL*3). Organic phases were combined and concentrated. The crude product was isolated by column chromatography (PE/EA=0 to 50%, 20 min) to obtain product **UB-181205e** (65 mg, yield 22%) as a colorless oil.

### Step 4: UB-181205g (V2790-115)

Compound **UB-181205e** and **UB-181205f** were dissolved in MeOH, a catalytic amount of AcOH was added, and the mixture was reacted at room temperature for 2 hours. NaBH(CN)₃ was added, the mixture was reacted for another 2 hours. The reaction solution was directly used in next reaction without purification. LC-MS: M+H= 335

### Step 5: UB-181205h (V2790-116)

To the reaction solution **of Step 4** was added Boc2O and NaHCO3 solution, the mixture was reacted overnight. Water (30 mL) was added, the mixture was extracted with EtOAc (30 mL), and the organic phases were combined and concentrated. The crude product was isolated by column chromatography (PE/EA=3/1) to obtain product **UB-181205h** (400 mg) as a white solid. LCMS [M+H]+ = 435.

### Step 6: UB-181205i (V2790-121)

Compound **UB-181205h** was dissolved in THF: MeOH, NaOH was added, the mixture was reacted at 50°C for 3 hours. The reaction solution was concentrated. The crude product was isolated by column chromatography (MeOH/DCM=1/10) to obtain product **UB-181205i** (80 mg) as colorless oil. LCMS [M+H]+ = 339.

### Step 7: UB-181205j (V2768-130)

Compound **UB-181205i** (70 mg, 0.2 mmol), **A3-I** (76 mg, 0.2 mmol), Pd(PPh₃)₂Cl₂ (17 mg, 0.024 mmol), CuI (8 mg, 0.04 mmol), and TEA (20 mg, 0.2 mmol) were dissolved in anhydrous DMF (5 mL), and the mixture was reacted at 80°C for 2 hour under N₂ protection. The reaction solution was concentrated and purified by preparative TLC (DCM/MeOH= 10/1) to obtain product **UB-181205j** (20 mg, yield 17%). LCMS [M+H]⁺ = 581.6

### Step 8: UB-181205 (V2768-139)

### The method is similar to General Method 4

¹H NMR (400 MHz, DMSO-*d*₆) δ 11.91 (s, 1H), 11.00 (s, 1H), 9.44 (s, 1H), 8.84 - 8.67 (m, 1H), 8.35 (d, *J* = 36.4 Hz, 2H), 8.21 (s, 1H), 7.85 - 7.74 (m, 1H), 7.70 (d, *J* = 7.9 Hz, 1H), 7.63 (s, 1H), 7.57 (d, *J* = 8.2 Hz, 1H), 7.53 - 7.45 (m, 1H), 7.22 - 7.09 (m, 2H), 5.91 (d, *J* = 4.1 Hz, 1H), 5.11 (dd, *J* = 13.3, 5.1 Hz, 1H), 4.44 (d, *J* = 17.5 Hz, 1H), 4.32 (d, *J* = 17.5 Hz, 1H), 4.14 (d, *J=* 12.6 Hz, 1H), 3.72 - 3.54 (m, 3H), 3.48 (t, *J* = 6.0 Hz, 2H), 3.07 - 2.85 (m, 4H), 2.81 - 2.68 (m, 3H), 2.59 (m, 4H), 2.47 - 2.35 (m, 2H), 1.99 (m, 2H), 1.89 - 1.40 (m, 12H), 1.35 - 1.26 (m, 2H). LCMS [M/2+H]⁺ = 465.1.

### Synthesis method of Compound UB-181207

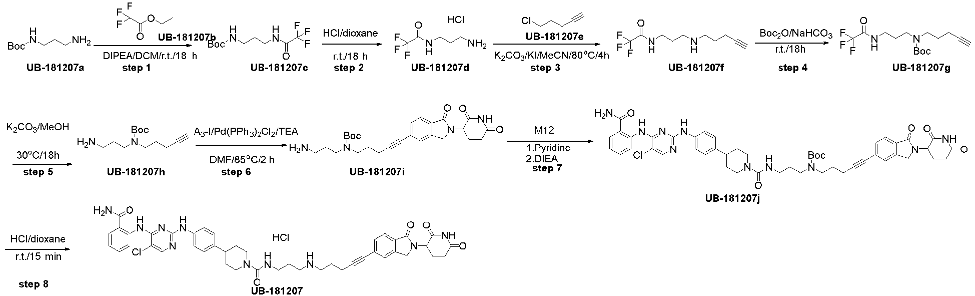

### Step 1: UB-181207c (V2768-131)

Compound **UB-181207a** (7.8 g, 44.7 mmol) was dissolved in DCM (50 mL), DIPEA (16 mL, 89.4 mmol) and **UB-181207b** (7.6 g, 53.7 mmol) were added, and the mixture was reacted at room temperature overnight. The reaction solution was concentrated and isolated by column chromatography (PE/EtOAc = 0 to 20%) to obtain product **UB-181207c** (11.5 g, yield 93%) as a white solid. LCMS [M+H]⁺ = 271.3.

### Step 2: UB-181207d (V2768-133)

Compound **UB-181207c** (11.5 g, 42.6 mmol) was dissolved in DCM (15 mL), 4M HCl/dioxane (53 mL) was added, and the mixture was reacted at room temperature overnight. The reaction liquid was concentrated to obtain crude product **UB-181207d** (9 g, 100% yield) as a white solid, which was directly used in the next reaction. LCMS [M+H]⁺ = 171.1. ¹H NMR (400 MHz, DMSO-*d*₆) δ 9.63 (d, *J* = 5.5 Hz, 1H), 8.08 (s, 3H), 3.27 (q, *J* = 6.6 Hz, 2H), 2.79 (s, 2H), 1.80 (dq, *J* = 9.3, 7.0 Hz, 2H).

### Step 3 & 4: UB-181207g (V2768-137)

**Compound UB-181207d** (1.03 g, 11 mmol) was dissolved in ACN (50 mL), K₂CO₃ (2 g, 15 mmol) and **UB-181207e** (0.61 g, 6 mmol) were added, and the mixture was reacted at 80°C for 4 hours. Boc₂O (2 mL) and NaHCO₃ (3 mL) were added, and the mixture was reacted at room temperature overnight. The reaction solution was concentrated and isolated by column chromatography (PE/EtOAc = 0 to 30%) to obtain product **UB-181207g** (160 mg, yield 10%) as a yellow solid. LCMS [M-100]⁺ = 237.2. ¹H NMR (400 MHz, DMSO-*d*₆) δ 9.39 (s, 1H), 3.22 - 3.11 (m, 6H), 2.78 (t, *J =* 2.7 Hz, 1H), 2.13 (td, *J* = 7.0, 2.7 Hz, 2H), 1.66 (m, 4H), 1.38 (s, 9H).

### Step 5: UB-181207h (V2768-140)

Compound **UB-181207g** (160 mg, 0.47 mmol) was dissolved in MeOH (10 mL), K₂CO₃ (131 mg, 0.94 mmol) was added and the mixture was reacted at room temperature overnight. The reaction solution was concentrated and isolated by column chromatography (DCM/MeOH=0 to -100%) to obtain product **UB-181207h** (100 mg, yield 88%) as a colorless oil. LCMS [M+H]⁺ = 241.3

¹H NMR (400 MHz, DMSO-*d*₆) δ 7.62 (s, 2H), 3.18 (d, *J=* 7.8 Hz, 4H), 2.80 (t, *J=* 2.6 Hz, 1H), 2.77 - 2.71 (m, 2H), 2.14 (td, *J* = 7.1, 2.7 Hz, 2H), 1.74 (q, *J=* 7.3 Hz, 2H), 1.65 (q, *J* = 7.2 Hz, 2H), 1.40 (s, 9H).

### Step 6: UB-181207i (V2768-142)

Compound **UB-181207h** (100 mg, 0.41 mmol), A3-I (120 mg, 0.32 mmol), Pd(PPh₃)₂Cl₂ (25 mg, 0.035 mmol), CuI (8 mg, 0.04 mmol), and TEA (32 mg, 0.32 mmol) were dissolved in anhydrous DMF (4 mL), and the mixture was reacted at 80°C for 2 hour under N₂ protection. The reaction solution was concentrated and isolated by column chromatography (DCM/MeOH=0 -100%) to obtain product **UB-181207i** (50 mg, yield 25%) as a brown solid. LCMS [M+H]⁺ = 483.5

### Step 7&8: UB-181207 (V2768-146)

### The method is similar to General Method 4

¹H NMR (400 MHz, DMSO-*d*₆) δ 11.92 (s, 1H), 11.00 (d, *J* = 2.7 Hz, 1H), 9.45 (s, 1H), 8.72 (d, *J* = 48.3 Hz, 3H), 8.31 (s, 1H), 8.21 (s, 1H), 7.83 (dd, *J* = 7.9, 1.6 Hz, 1H), 7.80 -7.68 (m, 2H), 7.65 (d, *J* = 6.2 Hz, 1H), 7.59 - 7.46 (m, 3H), 7.18 -7.10 (m, 2H), 6.79 (s, 1H), 5.11 (dt, *J=* 13.3, 4.6 Hz, 1H), 4.44 (dd, *J=* 17.6, 9.3 Hz, 1H), 4.31 (dd, *J=* 17.6, 6.2 Hz, 1H), 4.10 (d, *J* = 12.7 Hz, 1H), 3.16 (d, *J = 5.5* Hz, 2H), 3.12 - 2.81 (m, 8H), 2.76 (t, *J* = 12.5 Hz, 2H), 2.68 - 2.57 (m, 4H), 2.38 (dt, *J* = 13.1, 6.2 Hz, 1H), 1.96 (dq, *J=* 28.4, 7.5 Hz, 4H), 1.76 (dd, *J* = 15.5, 8.9 Hz, 4H), 1.56 - 1.40 (m, 2H). LCMS [M/2+H]⁺ = 416.5.

### Synthesis method of Compound UB-181226

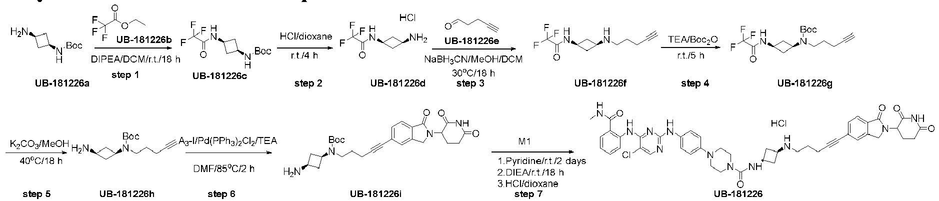

### Step 1: UB-181226c (V2768-148)

Compound **UB-181226a** (1 g, 5.3 mmol) was dissolved in DCM (20 mL), DIPEA (2 mL, 10.6 mmol) and **UB-181226b** (0.9 g, 6.4 mmol) were added, and the mixture was reacted at room temperature for 5 hours. The reaction solution was concentrated and isolated by column chromatography (PE/EtOAc = 0 to 80%) to obtain product **UB-181226c** (580 mg, yield 38%) as a white solid. LCMS [M-56]⁺ = 227.2. ¹H NMR (400 MHz, DMSO-*d*₆) δ 9.63 (d, *J* = 6.5 Hz, 1H), 7.16 (d, *J* = 7.6 Hz, 1H), 3.83 (h, *J* = 7.8 Hz, 1H), 3.66 (q, *J* = 8.2 Hz, 1H), 2.47 (d, *J* = 13.6 Hz, 2H), 2.01 - 1.88 (m, 2H), 1.37 (s, 9H).

### Step 2: UB-181226d (V2768-150)

Compound **UB-181226c** (0.58 g, 2 mmol) was dissolved in DCM (15 mL), 4M HCl/dioxane (2.5 mL) was added, and the mixture was reacted at room temperature for 4 hours. The reaction solution was concentrated to obtain crude product **UB-181226d** ( 400 mg, yield 100%) as a white solid. LCMS [M+H]⁺ = 183.1.

### Step 3&4: UB-181226g (V3160-001)

Compound **UB-181226b** (440 mg, 2 mmol), and **UB-181226e** (247 mg, 3 mmol) were dissolved in MeOH (30 mL) and DCM (50 mL), and the mixture was reacted at room temperature for 2 hours. NaBH₃CN (190 mg, 3 mmol) was added, and the mixture was reacted at room temperature overnight. The reaction solution was added with TEA (1.5 mL) and Boc₂O (2 mL), reacted at room temperature for 5 hours. The reaction solution was extracted with DCM (30 mL*2), combined, dried and concentrated. The crude product was isolated by column chromatography (PE/EtOAc = 0-40%) to obtain product **UB-181226g** (300 mg, yield 43%) as a colorless oil. LCMS [M-56]⁺ = 293.2

### Step 5: UB-181226h (V3160-002)

Compound **UB-181226g** (300 mg, 0.86 mmol) was dissolved in MeOH (15 mL), K₂CO₃ (600 mg, 4.34 mmol) was added and the mixture was reacted at 50°C overnight. The reaction solution was filtered, then concentrated, the crude product was isolated by column chromatography (DCM/MeOH=0 to -100%) to obtain product **UB-181226h** (170 mg, yield 78%) as a colorless oil. LCMS [M+H]⁺ = 253.3

¹H NMR (400 MHz, DMSO-*d*₆) δ 3.82 (s, 1H), 3.34 (s, 2H), 3.23 - 3.18 (m, 2H), 2.99 (ddd, *J=* 8.7, 6.8, 1.9 Hz, 1H), 2.80 (t, J= 2.7 Hz, 1H), 2.43 - 2.31 (m, 2H), 2.14 (td, *J=* 7.1, 2.7 Hz, 2H), 1.84 - 1.71 (m, 2H), 1.57 (dq, *J=* 8.7, 7.1 Hz, 2H), 1.38 (s, 9H).

### Step 6: UB-181226i (V3160-003)

Compound **UB-181226h** (170 mg, 0.67 mmol), **A3-I** (250 mg, 0.53 mmol), Pd(PPh₃)₂Cl₂ (47 mg, 0.067 mmol), CuI (13 mg, 0.067 mmol), and TEA (67 mg, 0.67 mmol) were dissolved in anhydrous DMF (4 mL), and the mixture was reacted at 80°C for 2 hour under N₂ protection. The reaction solution was concentrated and isolated by column chromatography (DCM/MeOH=0 -100%) to obtain product **UB-181226i** (150 mg, yield 45%) as a brown solid. LCMS [M+H]⁺ = 495.4

### Step 7: UB-181226 (V3160-012)

### The method is similar to General Method 4

¹H NMR (400 MHz, DMSO-*d*₆) δ 11.92 (s, 1H), 11.00 (s, 1H), 9.85 (s, 1H), 9.34 (s, 2H), 8.84 (d, *J=* 4.7 Hz, 1H), 8.66 (s, 1H), 8.28 (s, 1H), 7.80 (dd, *J=* 8.0, 1.6 Hz, 1H), 7.71 (d, *J=* 7.9 Hz, 1H), 7.68 - 7.32 (m, 6H), 7.20 (m, 2H), 5.11 (dd, *J* = 13.2, 5.1 Hz, 1H), 4.45 (d, *J* = 17.5 Hz, 1H), 4.33 (d, *J* = 17.5 Hz, 1H), 3.92 (m, 2H), 3.44 (d, *J* = 6.9 Hz, 4H), 3.32 (s, 4H), 2.95 (m, 3H), 2.81 (d, *J=* 4.5 Hz, 3H), 2.66 - 2.56 (m, 4H), 2.37 (m, 1H), 2.25 (d, *J=* 10.2 Hz, 2H), 2.09 - 1.88 (m, 4H). LCMS [M/2+H]⁺ = 430.

As used herein Compound No.UB-18XXXX, can also be simplified to No. XXXX, for example **UB-180941 is Compound 941(0941).**

Other compounds shown in Table A2 were prepared by similar methods.

**Table A2**

| | |
|---|---|
| **934** | 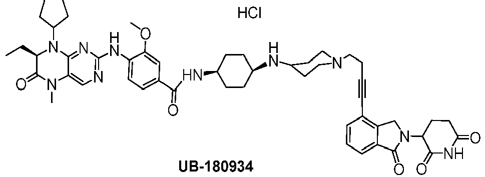 |
| **937** | 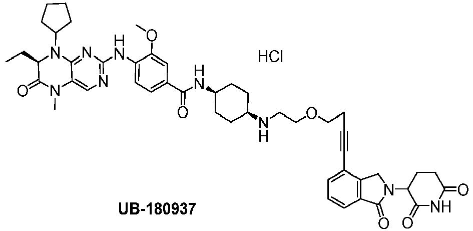 |
| | |
| **941** | 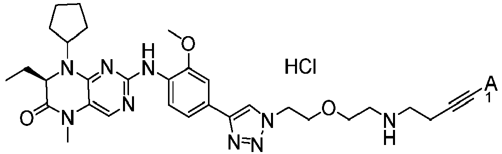 |
| | ¹H NMR (400 MHz, DMSO-*d*₆) δ 11.01 (s, 1H), 9.60 (s, 1H), 9.35 (s, 2H), 8.91 (s, 1H), 7.79 - 7.72 (m, 2H), 7.70 - 7.61 (m, 3H), 7.53 (td, *J* = 7.1, 6.4, 3.2 Hz, 2H), 5.15 (dd, *J* = 13.3, 5.1 Hz, 1H), 4.65 (t, *J* = 5.0 Hz, 2H), 4.53 - 4.42 (m, 2H), 4.32 (d, *J* = 17.8 Hz, 1H), 4.06 (q, *J* = 8.6 Hz, 2H), 3.93 (d, *J* = 5.0 Hz, 3H), 3.79 (t, *J* = 5.1 Hz, 3H), 3.17 (d, *J* = 4.1 Hz, 9H), 2.98 - 2.87 (m, 3H), 2.57 (s, 1H), 2.42 (dd, *J* = 13.2, 4.5 Hz, 1H), 2.05 - 1.69 (m, 8H), 1.42 (d, *J=* 31.5 Hz, 4H), 0.74 (t, *J* = 7.4 Hz, 3H). LCMS [M+H]⁺ = 830. |
| **943** | 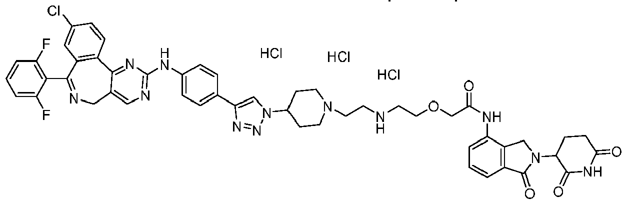 |
| | ¹H NMR (400 MHz, DMSO-*d*₆) δ 11.02 (s, 1H), 10.03 (m, 2H), 9.47 (m, 2H), 8.70 (s, 1H), 8.56 (s, 1H), 8.31 (d, *J* = 8.5 Hz, 1H), 7.95 - 7.73 (m, 6H), 7.63 - 7.50 (m, 3H), 7.34 (d, *J* = 2.2 Hz, 1H), 7.17 (t, *J* = 9.0 Hz, 2H), 5.16 (dd, *J* = 13.3, 5.1 Hz, 1H), 4.90 (d, *J* = 35.8 Hz, 2H), 4.50 (d, *J* = 17.6 Hz, 1H), 4.39 (s, 1H), 4.24 (s, 2H), 3.85 (d, *J* = 18.4 Hz, 4H), 3.31 (s, 4H), 2.97 - 2.89 (m, 1H), 2.68 - 2.57 (m, 4H), 2.45 - 2.33 (m, 3H), 2.07 - 1.96 (m, 2H). LCMS [M+H]⁺ = 969.7 |
| **944** | 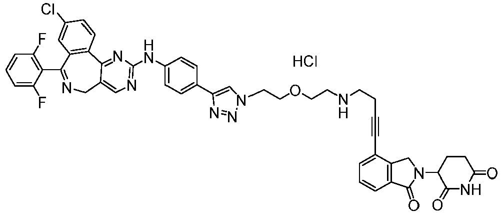 |
| | ¹H NMR (400 MHz, DMSO-*d*₆) δ 11.01 (s, 1H), 10.01 (s, 1H), 8.96 (s, 2H), 8.69 (s, 1H), 8.56 (s, 1H), 8.30 (d, *J* = 8.5 Hz, 1H), 7.88 (td, *J* = 8.5, 2.1 Hz, 3H), 7.80 (d, *J* = 8.6 Hz, 2H), 7.73 (d, *J* = 7.6 Hz, 1H), 7.65 (d, *J* = 7.6 Hz, 1H), 7.53 (dt, *J* = 12.5, 8.0 Hz, 2H), 7.34 (d, *J* = 2.2 Hz, 1H), 7.17 (t, *J* = 8.9 Hz, 2H), 5.15 (dd, *J* = 13.3, 5.1 Hz, 1H), 4.63 (t, *J* = 5.0 Hz, 2H), 4.48 (d, *J* = 17.7 Hz, 1H), 4.33 (d, *J* = 17.8 Hz, 1H), 3.93 (t, *J* = 5.1 Hz, 2H), 3.75 (t, *J* = 5.1 Hz, 2H), 3.19 (s, 4H), 2.93 - 2.86 (m, 3H), 2.59 (d, *J* = 17.6 Hz, 2H), 2.40 (dd, *J* = 13.1, 4.6 Hz, 2H), 2.05 - 1.96 (m, 2H). LCMS [M+H]⁺ = 881.3 |
| **945** | 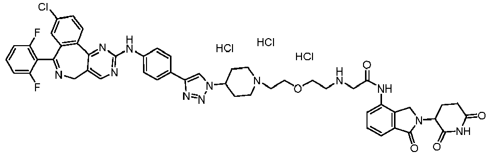 |
| | ¹H NMR (400 MHz, DMSO-*d*₆) δ 11.03 (s, 1H), 10.72 (s, 1H), 10.54 (s, 1H), 10.03 (d, *J* = 2.6 Hz, 1H), 9.41 (s, 2H), 8.70 (s, 1H), 8.51 (s, 1H), 8.31 (dd, *J* = 8.5, 1.5 Hz, 1H), 7.94 - 7.76 (m, 6H), 7.59 - 7.49 (m, 3H), 7.34 (d, *J* = 2.1 Hz, 1H), 7.17 (t, *J* = 8.9 Hz, 2H), 5.17 (m, 1H), 5.01 - 4.81 (m, 2H), 4.49 (d, *J* = 17.6 Hz, 1H), 4.38 (d, *J* = 17.7 Hz, 1H), 4.10 (d, *J* = 5.0 Hz, 2H), 3.82 (m, 6H), 3.40 (m, 2H), 3.29 (m, 4H), 2.94 (m, 1H), 2.60 (m, 4H), 2.43 (m, 2H), 2.32 - 2.26 (m, 1H), 2.08 - 1.92 (m, 2H). LCMS [M+H]⁺ = 969.3 |
| **946** | 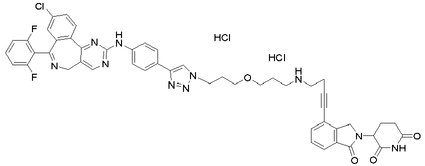 |
| | ¹H NMR (400 MHz, DMSO-*d*₆) δ 11.01 (s, 1H), 10.00 (t, *J* = 3.7 Hz, 1H), 9.13 (s, 1H), 8.87 (d, *J* = 41.4 Hz, 1H), 8.71 - 8.54 (m, 2H), 8.30 (dd, *J* = 8.5, 2.0 Hz, 1H), 7.93 - 7.85 (m, 3H), 7.85 - 7.78 (m, 2H), 7.74 - 7.63 (m, 2H), 7.60 - 7.49 (m, 2H), 7.33 (d, *J* = 2.2 Hz, 1H), 7.17 (t, *J* = 9.0 Hz, 2H), 5.15 (d, *J* = 13.0 Hz, 1H), 5.03 - 4.90 (m, 1H), 4.62 - 4.44 (m, 2H), 4.33 (d, *J* = 17.8 Hz, 1H), 4.03 - 3.74 (m, 4H), 3.26 - 3.07 (m, 3H), 3.01 - 2.85 (m, 4H), 2.58 (d, *J* = 17.1 Hz, 2H), 2.00 (q, *J* = 7.4 Hz, 2H), 1.55 (q, *J* = 6.6, 5.2 Hz, 1H), 1.20 - 1.13 (m, 2H), 1.07 (dd, *J* = 8.8, 6.0 Hz, 1H), 0.85 (t, *J* = 6.5 Hz, 1H). LCMS [M+H]⁺ = 909.5 |
| **947** | 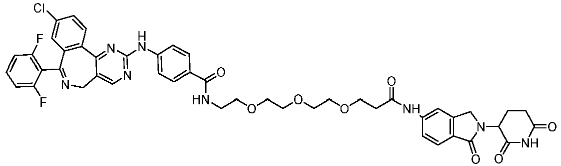 |
| | ¹H NMR (400 MHz, *d6-DMSO*) δ 10.95 (s, 1H), 10.29 (s, 1H), 10.16 (s, 1H), 8.72 (s, 1H), 8.49 - 8.25 (m, 2H), 7.98 (s, 1H), 7.87 (ddd, *J* = 18.0, 13.3, 8.9 Hz, 5H), 7.71 - 7.38 (m, 3H), 7.34 (d, *J* = 2.1 Hz, 1H), 7.17 (t, *J* = 8.5 Hz, 2H), 5.06 (dt, *J* = 33.1, 16.6 Hz, 1H), 4.42 (d, *J* = 17.3 Hz, 1H), 4.36 - 4.20 (m, 1H), 3.56 - 3.48 (m, 11H), 3.41 - 3.32 (m, 2H), 2.94 - 2.71 (m, 1H), 2.60 (t, *J* = 6.1 Hz, 3H), 2.37 (qd, *J* = 13.3, 4.4 Hz, 1H), 2.08 - 1.85 (m, 1H). LCMS [M+H]⁺ =921.4 |
| **948** | 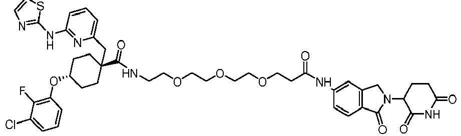 |
| | ¹H NMR (400 MHz, *d6-DMSO)* δ 11.12 (s, 1H), 10.97 (s, 1H), 10.29 (s, 1H), 7.98 (s, 1H), 7.58 (ddt, *J* = 9.2, 7.2, 14.0 Hz, 4H), 7.39 - 7.01 (m, 4H), 7.01 - 6.80 (m, 1H), 6.86 (d, *J* = 8.1 Hz, 1H), 6.64 (t, *J* = 6.0 Hz, 1H), 5.08 (dd, *J* = 13.3, 5.1 Hz, 1H), 4.53(d, *J* = 10.5 Hz, 1H), 4.34 - 4.17 (m, 2H), 3.70 (t, *J* = 6.2 Hz, 2H), 3.55 - 3.46 (m, 8H), 3.26 - 3.08 (m, 2H), 2.99 - 2.82 (m, 2H), 2.69 - 2.55 (m, 3H), 1.98 (dd, *J* = 6.7, 3.8 Hz, 1H), 1.87 (dd, *J* = 18.0, 7.2 Hz, 2H), 1.75 (dd, *J* = 7.0, 4.0 Hz, 4H), 1.59 (t, *J* = 11.1 Hz, 2H). LCMS [M+H]⁺ =906.4 |
| **950** | 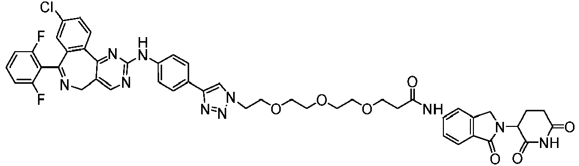 |
| | ¹H NMR (400 MHz, *d₆-DMSO*) δ 10.97 (s, 1H), 10.26 (s, 1H), 10.00 (s, 1H), 8.68 (s, 1H), 8.40 (s, 1H), 8.30 (d, *J* = 8.5 Hz, 1H), 7.97 (s, 1H), 7.96 - 7.84 (m, 4H), 7.79 (d, *J* = 8.7 Hz, 2H), 7.57 (ddt, *J* = 15.0, 8.3, 7.5 Hz, 4H), 7.33 (d, *J* = 2.1 Hz, 1H), 7.17 (t, *J* = 8.4 Hz, 2H), 5.04 (dt, *J* = 5.8, 2.4 Hz, 1H), 4.54 (t, *J* = 5.1 Hz, 2H), 4.27 (d, *J* = 17.3 Hz, 2H), 3.84 (t, *J* = 5.1 Hz, 2H), 3.69 (t, *J* = 6.2 Hz, 2H), 3.61 - 3.44 (m, 8H), 3.03 - 2.79 (m, 1H), 2.75 - 2.67 (m, 3H), 2.36 (qd, *J* = 13.3, 4.6 Hz, 1H), 2.09 - 1.84 (m, 1H).LCMS [M+H]⁺ =945.3 |
| **951** | 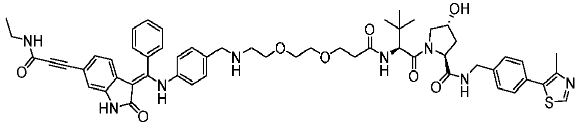 |
| | ¹H NMR (400 MHz, DMSO-*d*₆) δ 12.16 (s, 1H), 10.91 (s, 1H), 8.97 (d, *J* = 2.8 Hz, 1H), 8.76 - 8.41 (m, 2H), 7.91 (d, *J* = 9.6 Hz, 1H), 7.64 - 7.30 (m, 9H), 7.09 (d, *J* = 8.1 Hz, 2H), 6.98 (s, 1H), 6.84 - 6.63 (m, 3H), 5.76 (d, *J* = 8.1 Hz, 1H), 4.55 (d, *J* = 9.4 Hz, 1H), 4.49 - 4.30 (m, 4H), 4.23 (dd, *J* = 14.1, 8.6 Hz, 2H), 3.70 - 3.51 (m, 6H), 3.49 - 3.39 (m, 5H), 3.12 (p, *J* = 7.0 Hz, 2H), 2.44 (s, 3H), 2.38 - 2.27 (m, 1H), 2.08 - 1.77 (m, 4H), 1.03 (t, *J* = 7.2 Hz, 3H), 0.92 (s, 9H). LCMS [M+H]⁺ = 1009.7 |
| **952** | 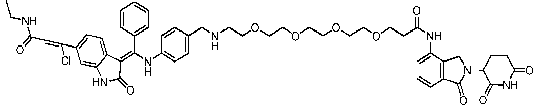 |
| | LCMS [M+H]⁺ = 962.5 |
| **953** | 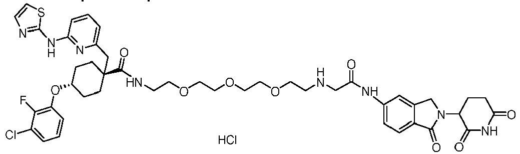 |
| | ¹H NMR (400 MHz, DMSO-*d*₆) δ 12.69-12.64 (m, 1H), 11.32 (s, 1H), 11.04 - 10.83 (m, 1H), 9.21 (s, 2H), 8.06 (s, 1H), 7.91 - 7.58 (m, 4H), 7.55 (t, *J* = 7.0 Hz, 1H), 7.35 - 7.06 (m, 4H), 7.06 - 6.64 (m, 1H), 6.77 (s, 1H), 6.77 (s, 2H), 5.14 - 5.01 (m, 2H), 4.51 (d, J= 31.1 Hz, 1H), 4.43 (s, 1H), 4.40 - 4.19 (m, 2H), 4.06 (s, 2H), 3.73 (t, *J* = 5.0 Hz, 2H), 3.54 (d, *J* = 15.3 Hz, 3H), 3.53 - 3.39 (m, 5H), 3.27 (dt, *J* = 9.0, 4.9 Hz, 4H), 3.19 - 2.98 (m, 2H), 2.98 (s, 2H), 3.04 - 2.83 (m, 1H), 2.83 - 2.69 (m, 1H), 2.62 (t, *J* = 19.5 Hz, 1H), 2.45 - 2.20 (m, 1H), 2.01 - 1.34 (m, 9H)..LCMS [M+ H]⁺ =935.4 |
| **954** | 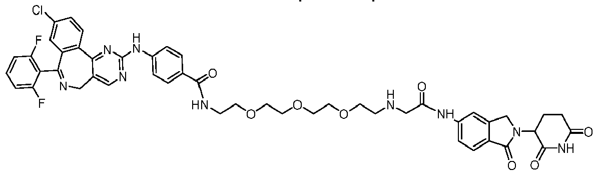 |
| | ¹H NMR (400 MHz, *d₆-DMSO*) δ 10.98 (s, 1H), 10.17 (s, 1H), 9.10 - 9.05 (m, 1H), 8.73 (s, 1H), 8.38 (s, 9H), 8.29 (d, *J* = 8.6 Hz, 1H), 8.00 - 7.75 (m, 6H), 7.75 - 7.46 (m, 2H), 7.34 (d, *J* = 1.9 Hz, 1H), 7.17 (t, *J* = 8.5 Hz, 2H), 5.09 (dd, *J* = 13.2, 5.0 Hz, 1H), 4.45 (d, *J* = 17.7 Hz, 1H), 4.31 (d, *J* = 17.5 Hz, 1H), 4.04 (s, 2H), 3.81 - 3.53 (m, 12H), 3.52 (s, 2H), 3.41 (d, *J* = 5.8 Hz, 2H), 3.24 - 2.69 (m, 1H), 2.85 - 2.77 (m, 2H), 2.11 - 1.96 (m, 2H). LCMS [M+ H]⁺ =950.4 |
| **955** | 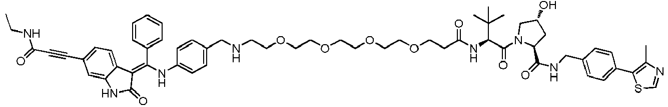 |
| | ¹H NMR (400 MHz, DMSO-*d*₆) δ 12.15 (s, 1H), 10.91 (s, 1H), 8.98 (s, 1H), 8.60 (dt, *J* = 25.3, 5.8 Hz, 2H), 7.92 (d, *J* = 9.3 Hz, 1H), 7.56 (q, *J* = 6.2 Hz, 3H), 7.51 - 7.44 (m, 2H), 7.40 (q, *J* = 8.1 Hz, 4H), 7.10 (d, *J* = 8.1 Hz, 2H), 6.98 (d, *J* = 1.5 Hz, 1H), 6.76 (dd, *J* = 12.0, 8.5 Hz, 3H), 5.77 (d, *J* = 8.1 Hz, 1H), 5.13 (s, 1H), 4.55 (d, *J* = 9.4 Hz, 1H), 4.48 - 4.36 (m, 2H), 4.35 (s, 1H), 4.22 (dd, *J* = 15.9, 5.5 Hz, 1H), 3.69 - 3.61 (m, 2H), 3.57 (s, 3H), 3.47 (m, 13H), 3.42 (m, 2H), 3.18 - 3.02 (m, 2H), 2.56 (t, *J* = 5.9 Hz, 2H), 2.44 (s, 3H), 2.35 (dt, *J* = 14.7, 6.2 Hz, 1H), 2.09 - 1.97 (m, 1H), 1.95 - 1.85 (m, 1H), 1.03 (t, *J* = 7.2 Hz, 3H), 0.93 (s, 9H). LCMS [M+H]⁺ = 1097.6 |
| **956** | 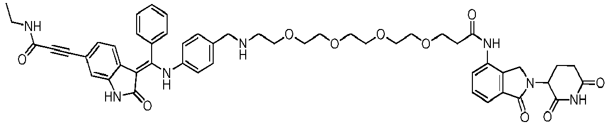 |
| | ¹H NMR (400 MHz, DMSO-*d*₆) δ 12.15 (s, 1H), 10.92 (s, 1H), 9.83 (s, 1H), 8.63 (t, *J* = 5.6 Hz, 1H), 7.82 (dd, *J* = 7.4, 1.7 Hz, 1H), 7.64 - 7.53 (m, 3H), 7.52 - 7.42 (m, 4H), 7.14 -7.06 (m, 2H), 6.99 (d, *J* = 1.5 Hz, 1H), 6.88 - 6.71 (m, 3H), 5.77 (d, *J* = 8.0 Hz, 1H), 5.15 (dd, *J* = 13.3, 5.1 Hz, 1H), 4.56 - 4.12 (m, 2H), 3.71 (t, *J* = 6.3 Hz, 2H), 3.57 (s, 2H), 3.51 (h, *J* = 2.6 Hz, 3H), 3.49 - 3.42 (m, 11H), 3.18 - 3.07 (m, 2H), 2.92 (ddd, *J* = 17.3, 13.6, 5.5 Hz, 1H), 2.59 (ddt, *J* = 19.9, 11.5, 4.9 Hz, 5H), 2.40 - 2.24 (m, 1H), 2.02 (dtd, *J* = 11.2, 5.9, 3.2 Hz, 1H), 1.03 (t, *J* = 7.2 Hz, 3H). LCMS [M+H]⁺ = 926.5 |
| **957** | 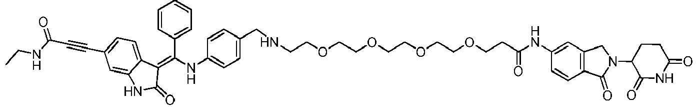 |
| | ¹H NMR (400 MHz, DMSO-*d*₆) δ 12.15 (s, 1H), 10.92 (s, 1H), 10.31 (s, 1H), 8.64 (t, *J* = 5.6 Hz, 1H), 7.98 (s, 1H), 7.70 - 7.52 (m, 5H), 7.49 - 7.44 (m, 2H), 7.15 - 7.04 (m, 2H), 6.99 (d, *J* = 1.6 Hz, 1H), 6.85 - 6.68 (m, 3H), 5.76 (d, *J* = 8.1 Hz, 1H), 5.08 (dd, *J* = 13.3, 5.1 Hz, 1H), 4.53 - 4.19 (m, 2H), 3.70 (t, *J* = 6.2 Hz, 2H), 3.57 (m, 2H), 3.48 (ddt, *J* = 13.5, 4.5, 2.1 Hz, 8H), 3.41 (q, *J* = 6.3, 5.7 Hz, 3H), 3.17 - 3.07 (m, 2H), 2.96 - 2.84 (m, 1H), 2.63 - 2.52 (m, 4H), 2.07 - 1.90 (m, 1H), 1.03 (t, *J* = 7.2 Hz, 3H). LCMS [M+H]⁺ = 926.5 |
| **958** | 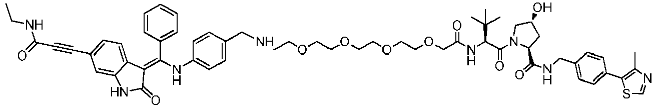 |
| | ¹H NMR (400 MHz, DMSO-*d*₆) δ 12.15 (s, 1H), 10.91 (s, 1H), 8.97 (s, 1H), 8.61 (dt, *J* = 13.1, 5.8 Hz, 2H), 7.56 (q, *J* = 6.0 Hz, 3H), 7.52 - 7.34 (m, 7H), 7.17 - 7.05 (m, 2H), 6.98 (d, *J* = 1.6 Hz, 1H), 6.87 - 6.69 (m, 3H), 5.76 (d, *J* = 8.2 Hz, 1H), 5.16 (s, 1H), 4.56 (d, *J* = 9.6 Hz, 1H), 4.50 - 4.31 (m, 3H), 4.24 (dd, *J* = 15.7, 5.6 Hz, 1H), 3.96 (s, 2H), 3.75 - 3.37 (m, 17H), 3.20 - 3.04 (m, 2H), 2.54 (t, *J* = 5.8 Hz, 2H), 2.43 (s, 3H), 2.13 - 1.96 (m, 2H), 1.90 (ddd, *J* = 13.0, 8.8, 4.5 Hz, 1H), 1.03 (t, *J* = 7.2 Hz, 3H), 0.94 (s, 9H). LCMS [M+H]⁺ = 1083.6 |
| **959** | 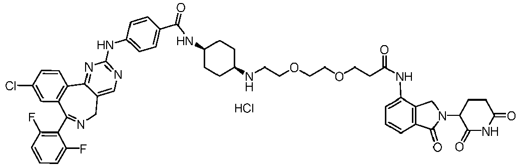 |
| | LCMS [M+H]⁺ = 974.4 |
| **960** | 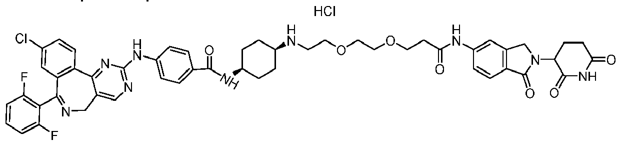 |
| | LCMS [M+H]⁺ = 974.4 |
| **961** | 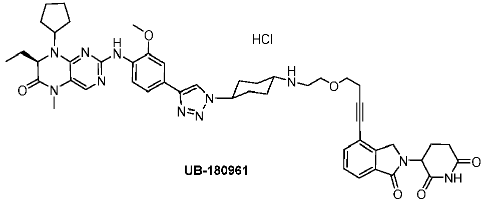 |
| | |
| **962** | 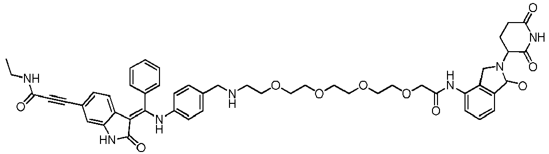 |
| | ¹H NMR (400 MHz, DMSO-*d*₆) δ 12.15 (s, 1H), 10.92 (s, 1H), 9.67 (s, 1H), 8.63 (t, *J* = 5.6 Hz, 1H), 7.74 (d, *J* = 7.7 Hz, 1H), 7.63 - 7.38 (m, 7H), 7.09 (d, *J* = 8.0 Hz, 2H), 6.99 (d, *J* = 1.6 Hz, 1H), 6.86 - 6.63 (m, 3H), 5.76 (d, *J* = 8.1 Hz, 1H), 5.14 (dd, *J* = 13.3, 5.1 Hz, 1H), 4.57 - 4.25 (m, 2H), 4.13 (s, 2H), 3.68 (dd, *J* = 5.8, 3.3 Hz, 2H), 3.60 (dd, *J* = 5.8, 3.3 Hz, 2H), 3.58 - 3.46 (m, 6H), 3.46 - 3.36 (m, 8H), 3.19 - 3.05 (m, 2H), 2.91 (ddd, *J* = 18.1, 13.6, 5.4 Hz, 1H), 2.59 (ddd, *J* = 17.2, 4.3, 2.3 Hz, 1H), 2.54 (t, *J* = 5.7 Hz, 2H), 2.36 (qd, *J* = 13.2, 4.4 Hz, 1H), 2.00 (ddd, *J* = 11.2, 6.3, 3.7 Hz, 1H), 1.03 (t, *J* = 7.2 Hz, 3H). LCMS [M+H]⁺ = 912.6 |
| **963** | 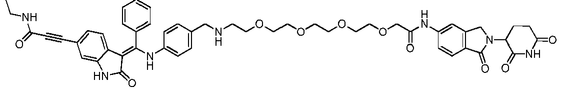 |
| | ¹H NMR (400 MHz, DMSO-*d*₆) δ 12.15 (s, 1H), 10.92 (d, *J* = 3.0 Hz, 1H), 9.96 (s, 1H), 8.63 (t, *J* = 5.7 Hz, 1H), 8.00 (s, 1H), 7.73 - 7.39 (m, 7H), 7.17 - 7.04 (m, 2H), 6.98 (d, *J* = 1.6 Hz, 1H), 6.83 - 6.69 (m, 3H), 5.76 (d, *J* = 8.0 Hz, 1H), 5.08 (dd, *J* = 13.3, 5.1 Hz, 1H), 4.55 - 4.23 (m, 2H), 4.12 (d, *J* = 2.3 Hz, 2H), 3.71 - 3.64 (m, 2H), 3.63 - 3.46 (m, 10H), 3.47 - 3.40 (m, 4H), 3.17 - 3.06 (m, 2H), 2.99 - 2.80 (m, 1H), 2.55 (t, *J* = 5.7 Hz, 2H), 2.41 - 2.31 (m, 1H), 2.07 (s, 1H), 1.99 (ddt, *J* = 9.7, 5.3, 2.4 Hz, 1H), 1.03 (t, *J* = 7.2 Hz, 3H). LCMS [M+H]⁺ = 912.5 |
| **964** | 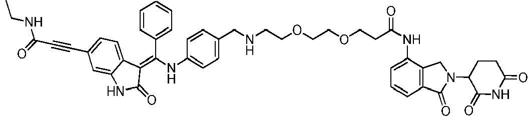 |
| | ¹H NMR (400 MHz, DMSO-*d*₆) δ 12.15 (s, 1H), 10.91 (s, 1H), 9.83 (s, 1H), 8.63 (t, *J* = 5.6 Hz, 1H), 7.81 (d, *J* = 7.5 Hz, 1H), 7.65 - 7.31 (m, 7H), 7.07 (d, *J* = 8.2 Hz, 2H), 6.98 (s, 1H), 6.76 (dd, *J* = 8.2, 5.8 Hz, 3H), 5.76 (d, *J* = 8.1 Hz, 1H), 5.15 (dd, *J* = 13.3, 5.2 Hz, 1H), 4.44 - 4.20 (m, 2H), 3.70 (t, *J* = 6.3 Hz, 2H), 3.56 - 3.49 (m, 4H), 3.46 (dd, *J* = 6.1, 3.6 Hz, 3H), 3.40 (m, 4H), 3.12 (p, *J* = 7.0 Hz, 2H), 2.91 (ddd, *J* = 18.0, 13.7, 5.5 Hz, 1H), 2.67 - 2.55 (m, 3H), 2.32 (qd, *J* = 13.2, 4.2 Hz, 1H), 2.11 - 1.96 (m, 1H), 1.03 (t, *J* = 7.2 Hz, 3H). LCMS [M+H]⁺ = 838.5 |
| **965** | 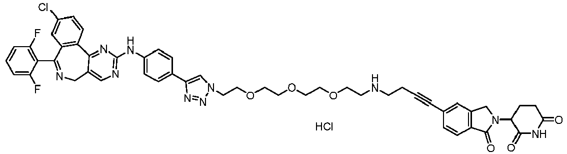 |
| | ¹H NMR (400 MHz, DMSO-*d*₆) δ 11.00 (s, 1H), 10.02 (s, 1H), 9.06 (s, 2H), 8.69 (s, 1H), 8.44 (d, *J* = 5.1 Hz, 1H), 8.30 (d, *J* = 8.5 Hz, 1H), 7.97 - 7.84 (m, 3H), 7.80 (d, *J* = 8.7 Hz, 1H), 7.74 - 7.62 (m, 1H), 7.61 - 7.49 (m, 1H), 7.35 (t, *J* = 6.3 Hz, 1H), 7.17 (t, *J* = 8.6 Hz, 1H), 5.09 - 4.78 (m, 1H), 4.82 - 4.50 (m, 2H), 4.37 (dt, *J* = 17.7, 14.1 Hz, 3H), 3.91 - 3.74 (m, 2H), 3.71 - 3.46 (m, 12H), 3.37 - 2.97 (m, 4H), 2.97 - 2.75 (m, 3H), 2.60 (dd, *J* = 5.8, 16.8 Hz, 1H), 2.43 - 2.06 (m, 2H), 1.98 (dd, *J* = 4.9, 4.8 Hz, 1H).LCMS [M+H]⁺ =969.4 |
| **966** | 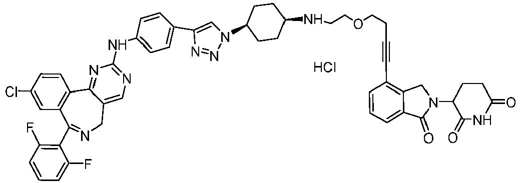 |
| | ¹H NMR (400 MHz, DMSO-d6) δ 11.00 (s, 1H), 10.02 (s, 1H), 8.97 (s, 2H), 8.77 (d, J = 3.0 Hz, 1H), 8.70 (s, 1H), 8.31 (d, J = 8.5 Hz, 1H), 7.96 - 7.85(m, 3H), 7.81 (d, J = 8.5 Hz, 2H), 7.73 - 7.64 (m, 2H), 7.57 - 7.49 (m, 2H), 7.17 (t, J = 8.8 Hz, 2H), 5.68 (s, 1H), 5.11 (dd, J = 13.2, 5.1 Hz, 1H), 4.73 -4.55 (m, 2H), 4.47 - 4.27 (m, 3H), 3.81 (t, J = 5.2 Hz, 2H), 3.69 (d, J = 6.9 Hz, 2H), 3.60 (s, 2H), 2.78 (t, J = 6.7 Hz, 2H), 2.62 (s, 1H), 2.39 - 2.33 (m,1H), 1.98 (qt, J = 12.6, 9.3, 6.8 Hz, 6H), 1.83 (t, J = 10.3 Hz, 2H). LCMS [M+H]⁺ = 935.3 |
| **967** | 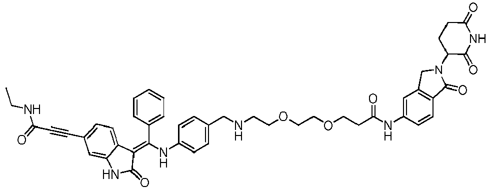 |
| | ¹H NMR (400 MHz, DMSO-*d*₆) δ 12.14 (s, 1H), 10.92 (s, 1H), 10.30 (s, 1H), 8.64 (t, *J* = 5.6 Hz, 1H), 7.98 (s, 1H), 7.69 - 7.49 (m, 5H), 7.49 - 7.40 (m, 2H), 7.08 (d, *J* = 8.2 Hz, 2H), 6.99 (d, *J* = 1.6 Hz, 1H), 6.76 (dq, *J* = 7.7, 6.0 Hz, 3H), 5.76 (d, *J* = 8.0 Hz, 1H), 5.07 (dd, *J* = 13.2, 5.2 Hz, 1H), 4.61 - 4.15 (m, 3H), 3.70 (t, J= 6.1 Hz, 3H), 3.56 - 3.48 (m, 5H), 3.45 (dd, J= 6.2, 3.7 Hz, 4H), 3.18 - 3.06 (m, 2H), 2.90 (ddd, *J* = 17.9, 13.7, 5.6 Hz, 1H), 2.58 (q, *J* = 5.5, 4.9 Hz, 3H), 2.36 (dd, *J* = 13.2, 4.6 Hz, 1H), 2.08 - 1.84 (m, 2H), 1.03 (t, *J* = 7.2 Hz, 3H). LCMS [M+H]⁺ = 838.5 |
| **970** | 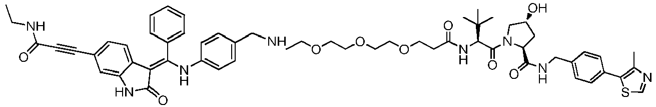 |
| | ¹H NMR (400 MHz, DMSO-*d*₆) δ12.16 (s, 1H), 10.92 (s, 1H), 8.98 (d, *J* = 3.7 Hz, 1H), 8.60 (dt, *J* = 25.3, 5.8 Hz, 2H), 7.91 (d, *J* = 9.4 Hz, 1H), 7.69 - 7.51 (m, 3H), 7.50-7.33 (m, 6H), 7.19-7.05 (m, 2H), 6.98 (d, *J* = 1.6 Hz, 1H), 6.83 - 6.62 (m, 3H), 5.76 (d, *J* = 8.1 Hz, 1H), 5.23 - 5.08 (m, 1H), 4.55 (d, *J* = 9.4 Hz, 1H), 4.47 - 4.39 (m, 2H), 4.35 (s, 1H), 4.21 (dd, *J* = 15.8, 5.4 Hz, 1H), 3.71 - 3.53 (m, 6H), 3.52 - 3.44 (m, 6H), 3.41 (t, *J* = 5.8 Hz, 3H), 3.12 (qd, *J* = 7.2, 5.5 Hz, 2H), 2.55 (t, *J* = 6.0 Hz, 2H), 2.44 (s, 3H), 2.34 (dt, *J* = 14.6, 6.1 Hz, 1H), 2.02 (q, *J* = 9.7, 7.4 Hz, 2H), 1.90 (ddd, *J* = 13.0, 8.6, 4.6 Hz, 1H), 1.03 (t, *J* = 7.2 Hz, 3H), 0.93 (s, 9H). LCMS [M+H]⁺ = 1053.6 |
| **971** | 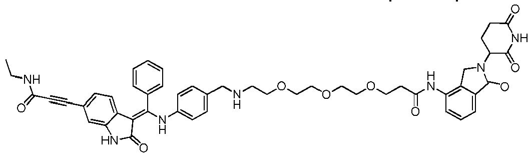 |
| | ¹H NMR (400 MHz, DMSO-*d*₆) δ 12.15 (s, 1H), 10.92 (s, 1H), 9.84 (s, 1H), 8.63 (t, *J* = 5.7 Hz, 1H), 7.81 (dd, *J* = 7.4, 1.6 Hz, 1H), 7.68 - 7.37 (m, 7H), 7.09 (d, *J* = 8.5 Hz, 2H), 6.98 (d, *J* = 1.6 Hz, 1H), 6.88 - 6.63 (m, 3H), 5.76 (d, *J=* 8.1 Hz, 1H), 5.15 (dd, *J* = 13.3, 5.1 Hz, 1H), 4.49 - 4.13 (m, 2H), 3.70 (t, *J* = 6.3 Hz, 2H), 3.55 (s, 2H), 3.52 - 3.45 (m, 6H), 3.45 - 3.36 (m, 4H), 3.19 - 3.06 (m, 2H), 3.05 - 2.84 (m, 1H), 2.70 - 2.56 (m, 3H), 2.55 - 2.51 (m, 2H), 2.32 (dd, *J* = 13.2, 4.5 Hz, 1H), 2.10 - 1.99 (m, 1H), 1.03 (t, *J* = 7.2 Hz, 3H). LCMS [M+H]⁺ = 882.5 |
| **972** | 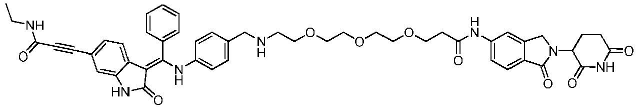 |
| | ¹H NMR (400 MHz, DMSO-*d*₆) δ 12.15 (s, 1H), 10.91 (s, 1H), 10.28 (s, 1H), 8.63 (t, *J* = 5.7 Hz, 1H), 7.98 (d, *J* = 1.7 Hz, 1H), 7.68 - 7.51 (m, 5H), 7.51 - 7.40 (m, 2H), 7.10 (dd, *J* = 8.1, 5.4 Hz, 2H), 6.99 (d, *J* = 1.6 Hz, 1H), 6.87 - 6.70 (m, 3H), 5.77 (dd, *J* = 8.1, 4.7 Hz, 1H), 5.08 (dd, *J* = 13.3, 5.1 Hz, 1H), 4.50 - 4.12 (m, 2H), 3.70 (t, *J* = 6.2 Hz, 2H), 3.55 (s, 2H), 3.52 - 3.45 (m, 6H), 3.40 (dt, *J* = 11.7, 5.5 Hz, 5H), 3.11 (td, *J* = 7.3, 5.5 Hz, 2H), 2.99 - 2.81 (m, 1H), 2.65 - 2.56 (m, 2H), 2.55 - 2.51 (m, 2H), 2.36 (qd, *J* = 13.1, 4.6 Hz, 1H), 1.98 (dt, *J=* 7.8, 2.7 Hz, 1H), 1.03 (t, *J* = 7.2 Hz, 3H). LCMS [M+H]⁺ = 882.4 |
| **973** | 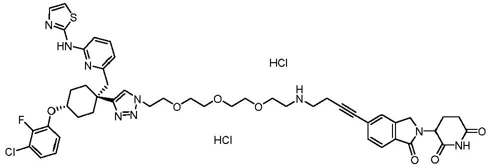 |
| | ¹H NMR (400 MHz, DMSO-*d*₆) δ 11.00 (s, 1H) 8.95 (s, 2H), 7.70 (dd, *J* = 14.2, 9.5 Hz, 3H), 7.41 (dt, *J* = 3.7, 13.9 Hz, 2H), 7.18 -7.07 (m, 5H), 6.17 (t, *J* = 8.6 Hz, 1H), 5.09 - 4.98 (m, 1H), 4.52 - 4.26 (m, 6H), 3.72 (s, 6H), 3.42 - 2.87 (m, 12H), 2.65 - 2.57 (m, 2H), 2.49 - 2.46 (m, 2H), 2.09 - 1.87 (m, 6H), 1.86-1.75 (m, 2H), 1.56-1.44 (m, 2H). LCMS [M+H]⁺ =954.4 |
| **974** | 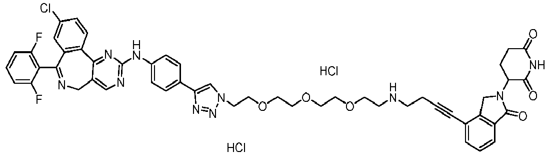 |
| | ¹H NMR (400 MHz, DMSO-*d*₆) δ 11.02 (s, 1H), 10.02 (s, 1H), 9.19 (s, 2H), 8.69 (s, 1H), 8.45 (s, 1H), 8.30 (d, *J* = 8.5 Hz, 1H), 7.89 (dd, *J* = 14.6, 5.5 Hz, 3H), 7.83 - 7.49 (m, 6H), 7.36 (dd, *J* = 14.2, 3.9 Hz, 2H), 7.17 (t, *J* = 8.5 Hz, 2H), 5.16 (dd, *J* = 13.3, 5.0 Hz, 1H), 4.56 - 4.31 (m, 5H), 3.69 (t, *J* = 5.0 Hz, 3H), 3.64 - 3.37 (m, 12H), 3.18 (dd, *J* = 5.5, 3.6 Hz, 2H), 2.89 (dt, *J* = 8.2, 4.2 Hz, 3H), 2.66 - 2.54 (m, 1H), 2.49 - 2.47 (m, 1H), 2.01 (d, *J* = 5.4 Hz, 1H).LCMS [M+H]⁺ =969.4 |
| **975** | 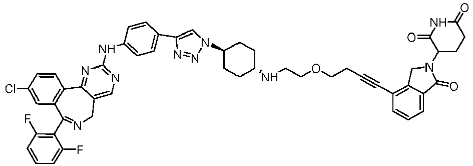 |
| | ¹H NMR (400 MHz, DMSO-d6) δ 11.01 (s, 1H), 10.02 (s, 1H), 8.96 (s, 2H), 8.76 (s, 1H), 8.71 (d, J = 7.4 Hz, 1H), 8.31 (d, J = 8.5 Hz, 1H), 8.12 -7.97 (m, 1H), 7.91 (td, J = 6.0, 5.5, 2.1 Hz, 2H), 7.84 - 7.79 (m, 2H), 7.72 (d, J = 7.6 Hz, 1H), 7.65 (d, J = 7.4 Hz, 1H), 7.53 (t, J = 7.6 Hz, 2H), 7.39 -7.31 (m, 4H), 7.16 (d, J = 9.5 Hz, 2H), 5.67 (s, 1H), 5.16 (dd, J = 13.3, 5.2 Hz, 1H), 4.68 (s, 1H), 4.47 (d, J = 17.8 Hz, 1H), 4.32 (d, J = 17.8 Hz, 1H),3.81 (t, J = 5.3 Hz, 2H), 3.69 (t, J = 6.8 Hz, 2H), 3.16 (s, 2H), 2.90 (d, J = 12.4 Hz, 1H), 2.79 (t, J = 6.7 Hz, 2H), 2.62 (s, 1H), 2.44 (d, J = 4.3 Hz, 1H),2.07 - 1.91 (m, 7H), 1.81 (s, 2H). [M+H]⁺ = 935.5 |
| **976** | 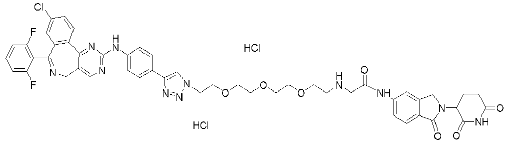 |
| | ¹H NMR (400 MHz, DMSO-*d*₆) δ 11.43 (s, 1H), 10.99 (s, 1H), 10.07 (s, 1H), 9.26 (s, 2H), 8.71 (s, 1H), 8.49 (s, 1H), 8.32 (d, *J* = 8.5 Hz, 1H), 7.96 (d, *J=* 9.0 Hz, 1H), 7.93 - 7.81 (m, 3H), 7.81 - 7.63 (m, 4H), 7.63 - 7.18 (m, 1H), 7.20 (d, *J* = 8.4 Hz, 2H), 7.19 (t, *J* = 8.3 Hz, 2H), 5.12 - 5.06 (m, 1H), 4.41 (t, *J* = 5.7 Hz, 2H), 4.30 (d, *J* = 8.4 Hz, 2H), 4.92 - 3.83 (m, 2H), 4.18 - 3.75 (m, 2H), 3.81 (d, *J* = 5.8 Hz, 2H), 3.80 (d, *J* = 4.3 Hz, 2H), 3.73 (s, 2H), 3.73 - 3.46 (m, 2H), 3.23 - 2.99 (m, 2H), 2.93 - 2.71 (m, 1H), 2.73 - 2.69 (m, 1H), 2.53 - 2.43 (m, 1H), 1.99 (d, *J* = 5.2 Hz, 1H). LCMS [M+H]⁺ =974.6 |
| **980** | 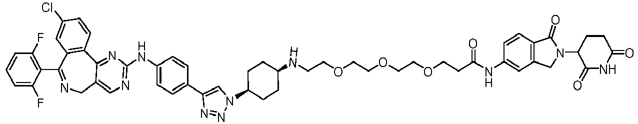 |
| | ¹H NMR (400 MHz, DMSO-d6) δ 10.98 (s, 1H), 10.39 (s, 1H), 10.02 (s, 1H), 8.69 (d, J = 5.3 Hz, 4H), 8.31 (d, J = 8.5 Hz, 1H), 7.99 (s, 1H), 7.91 (s, 3H),7.83 (s, 2H), 7.69 - 7.60 (m, 2H), 7.55 (ddd, J = 14.8, 8.3, 6.4 Hz, 1H), 7.34 (d, J = 2.2 Hz, 1H), 7.17 (s, 2H), 5.08 (dd, J = 13.2, 5.1 Hz, 1H), 4.68 (s, 1H),4.44 - 4.25 (m, 2H), 3.72 (s, 5H), 3.28 (s, 6H), 3.11 (d, J = 8.8 Hz, 3H), 2.95 - 2.87 (m, 1H), 2.62 (s, 3H), 2.38 - 2.32 (m, 1H), 1.96 (s, 5H), 1.73 (d, J =10.9 Hz, 2H), 1.31 - 1.24 (m, 6H). LCMS [M+H]⁺ = 1009.7 |
| **981** | 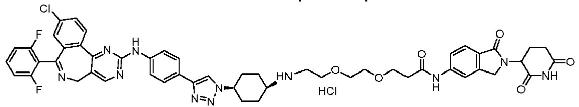 |
| | ¹H NMR (400 MHz, DMSO-d6) δ 10.97 (s, 1H), 10.43 (s, 1H), 10.02 (s, 1H), 8.69 (d, J = 7.6 Hz, 4H), 8.31 (d, J = 8.5 Hz, 1H), 8.00 (s, 1H), 7.92 (d, J =8.8 Hz, 2H), 7.88 (dd, J = 8.5, 2.2 Hz, 1H), 7.81 (d, J = 8.6 Hz, 2H), 7.65 (d, J = 2.2 Hz, 2H), 7.60 - 7.50 (m, 1H), 7.34 (d, J = 2.2 Hz, 1H), 7.18 (d, J =7.9 Hz, 2H), 5.08 (dd, J = 13.2, 5.1 Hz, 1H), 4.65 (d, J = 10.7 Hz, 1H), 4.47 - 4.27 (m, 2H), 4.05 (q, J = 7.1 Hz, 1H), 3.72 (dt, J= 10.7, 4.9 Hz, 4H), 3.62- 3.59 (m, 2H), 3.11 - 3.05 (m, 3H), 2.95 - 2.86 (m, 2H), 2.64 (d, J = 6.0 Hz, 1H), 2.37 (dd, J = 13.3, 4.5 Hz, 1H), 1.95 (s, 5H), 1.75 (d, J = 13.3 Hz, 2H),1.29 - 1.26 (m, 6H). LCMS [M+H]⁺ = 998.72 |
| **982** | 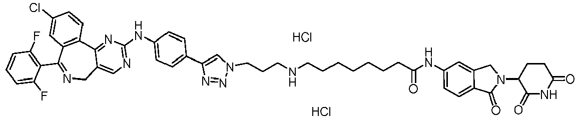 |
| | ¹H NMR (400 MHz, DMSO-*d*₆) δ 10.97 (s, 1H), 10.37 (s, 1H), 10.03 (s, 1H), 8.96 (s, 2H), 8.70 (s, 1H), 8.54 (s, 1H), 8.31 (d, *J* = 8.5 Hz, 1H), 8.00 (s, 1H), 7.96 - 7.83 (m, 3H), 7.80 (d, *J* = 8.7 Hz, 2H), 7.67 - 7.55 (m, 2H), 7.55 - 7.34 (m, 1H), 7.20 (dd, *J* = 5.3, 6.7 Hz, 2H), 5.08 (dd, *J* = 13.3, 5.0 Hz, 1H), 4.54 (t, *J* = 6.6 Hz, 2H), 2.87 (dt, *J* = 8.5, 4.3 Hz, 5H), 2.59 (d, *J* = 6.6 Hz, 1H), 2.55 - 2.46 (m, 2H), 2.44 - 2.02 (m, 2H), 2.05 - 1.84 (m, 1H), 1.61 (s, 4H), 1.36 - 1.23 (m, 6H)..LCMS [M+H]⁺ =940.7 |
| **983** | 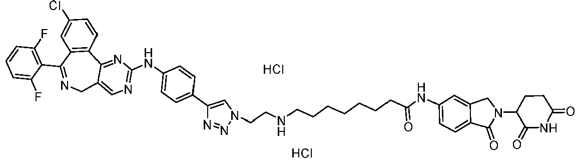 |
| | ¹H NMR (400 MHz, DMSO-*d*₆) δ10.97 (s, 1H), 10.34 (s, 1H), 10.04 (s, 1H), 9.07 (s, 2H), 8.70 (s, 1H), 8.57 (s, 1H), 8.31 (d, *J* = 8.5 Hz, 1H), 7.99 (s, 1H), 7.97 - 7.86 (m, 3H), 7.81 (d, *J* = 8.7 Hz, 2H), 7.75 - 7.45 (m, 3H), 7.34 (d, *J* = 2.1 Hz, 1H), 7.17 (t, *J* = 8.4 Hz, 2H), 5.08 (dd, *J* = 13.2, 5.0 Hz, 1H), 4.79 (t, *J* = 6.0 Hz, 2H), 4.41 (d, *J* = 7.3 Hz, 1H), 4.27 (d, *J* = 7.4 Hz, 1H), 3.68 - 3.55 (m, 2H), 2.93 - 2.84 (m, 3H), 2.59-2.50 (m, 1H), 2.38 (dd, *J* = 5.3, 8.0 Hz, 3H), 2.06 - 1.84 (m, 1H), 1.60 (d, *J* = 5.8 Hz, 4H), 1.28 (d, *J* = 3.6 Hz, 6H). LCMS [M+H]⁺ =926.8 |
| **986** | 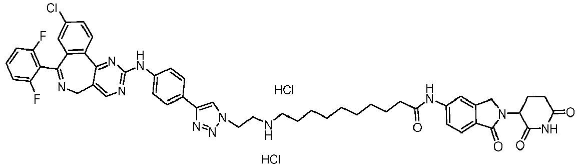 |
| | ¹H NMR (400 MHz, DMSO-*d*₆) δ10.95 (s, 1H), 10.33 (s, 1H), 10.03 (s, 1H), 9.07 (s, 2H), 8.70 (s, 1H), 8.57 (s, 1H), 8.31 (d, *J* = 8.5 Hz, 1H), 7.99 (s, 1H), 7.97 - 7.86 (m, 3H), 7.81 (d, *J* = 8.7 Hz, 2H), 7.75 - 7.45 (m, 3H), 7.34 (d, *J* = 2.1 Hz, 1H), 7.17 (t, *J* = 8.4 Hz, 2H), 5.08 (dd, *J* = 13.2, 5.0 Hz, 1H), 4.80 (t, *J* = 6.0 Hz, 2H), 4.41 (d, *J* = 7.3 Hz, 1H), 4.27 (d, *J* = 7.4 Hz, 1H), 2.98 - 2.85 (m, 5H), 2.68 - 2.55 (m, 1H), 2.26 (dd, *J* = 8.1, 7.9 Hz, 2H), 2.06 - 1.84 (m, 1H), 1.64 (d, *J* = 4.1 Hz, 4H), 1.24 (s,10H)..LCMS [M+H]⁺ =954.8 |
| **987** | 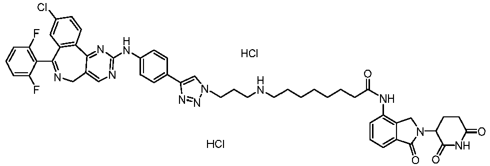 |
| | ¹H NMR (400 MHz, DMSO-*d*₆) δ11.02 (s, 1H), 10.03 (s, 1H), 9.79 (s, 1H), 8.50 (s, 1H), 7.96 - 7.72 (m, 6H), 7.62 - 7.43 (m, 3H), 7.17 (s, 2H), 5.15 (dd, *J* = 13.4, 4.9 Hz, 1H), 4.52 (s, 1H), 4.36 (q, *J* = 7.5 Hz, 2H), 2.83 (t, *J* = 9.1 Hz, 5H), 2.68 - 2.56 (m, 1H), 2.41 - 2.30 (m, 5H), 2.13 (d, *J* = 6.0 Hz, 1H), 1.64 (d, *J* = 4.0 Hz, 4H), 1.24 (s,6H).LCMS [M+H]⁺ =940.7 |
| **988** | 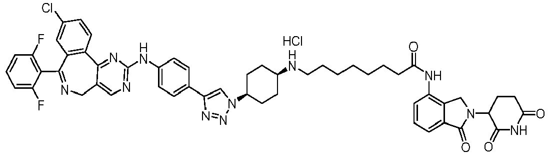 |
| | ¹H NMR (400 MHz, DMSO-d6) δ 11.02 (s, 1H), 10.02 (s, 1H), 9.88 (s, 1H), 8.78 (d, J = 10.5 Hz, 2H), 8.70 (s, 1H), 8.31 (d, J = 8.5 Hz, 1H), 7.94 - 7.79(m, 5H), 7.61 - 7.45 (m, 3H), 7.41 - 7.30 (m, 2H), 7.17 (t, J = 8.7 Hz, 2H), 5.15 (dd, J = 13.2, 5.1 Hz, 1H), 4.68 (d, J = 15.9 Hz, 1H), 4.45 - 4.32 (m, 2H),3.62 - 3.57 (m, 3H), 3.50 (d, J = 5.5 Hz, 1H), 3.12 (qd, J = 7.3, 4.2 Hz, 2H), 2.90 (t, J = 6.3 Hz, 3H), 2.61 (d, J = 16.8 Hz, 1H), 2.37 (d, J = 7.3 Hz, 3H),1.99 (d, J = 16.6 Hz, 5H), 1.79 (d, J = 8.8 Hz, 2H), 1.64 (s, 4H), 1.33 (s, 6H). LCMS [M+H]⁺ = 980.79 |
| **989** | 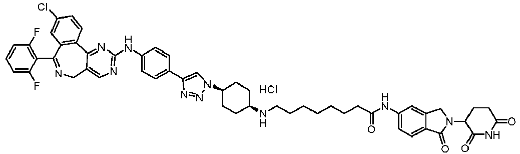 |
| | ¹H NMR (400 MHz, DMSO-d6) δ 10.97 (s, 1H), 10.39 (s, 1H), 10.02 (s, 1H), 9.31 (d, J = 2.6 Hz, 1H), 8.85 (d, J = 8.0 Hz, 2H), 8.79 (s, 1H), 8.70 (s, 1H),8.31 (d, J = 8.5 Hz, 1H), 8.00 (s, 1H), 7.94 - 7.87 (m, 3H), 7.81 (d, J = 8.6 Hz, 2H), 7.64 (d, J = 1.2 Hz, 2H), 7.55 (ddd, J = 14.9, 8.4, 6.5 Hz, 1H), 7.33 (d, J= 2.1 Hz, 1H), 7.17 (t, J = 8.9 Hz, 2H), 5.08 (dd, J = 13.3, 5.1 Hz, 1H), 4.70 (s, 1H), 4.66 - 4.55 (m, 1H), 4.46 - 4.24 (m, 3H), 3.76 (d, J = 13.1 Hz, 3H), 3. 12(tt, J = 7.4, 3.7 Hz, 2H), 2.89 (d, J = 11.6 Hz, 3H), 2.61 (s, 1H), 2.38 (t, J = 7.5 Hz, 3H), 2.03 - 1.91 (m, 5H), 1.81 (s, 2H), 1.71 - 1.57 (m, 4H), 1.33 (s, 6H). LCMS [M+H]⁺ = 980.79 |
| **990** | 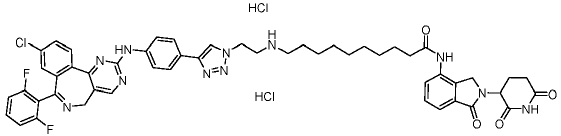 |
| | ¹H NMR (400 MHz, DMSO-*d*₆) δ11.02 (s, 1H), 10.04 (s, 1H), 9.82 (s, 1H), 8.83 (d, *J* = 10.5 Hz, 2H), 8.70 (s, 1H), 8.55 (s, 1H), 8.31 (d, *J* = 8.5 Hz, 1H), 7.99 - 7.84 (m, 3H), 7.80 (d, *J* = 8.7 Hz, 3H), 7.67 - 7.40 (m, 3H), 7.34 (d, *J* = 2.1 Hz, 1H), 7.17 (t, *J* = 8.4 Hz, 2H), 5.15 (dd, *J* = 13.3, 5.1 Hz, 1H), 4.77 (t, *J* = 6.0 Hz, 2H), 4.36 (q, *J* = 7.4 Hz, 2H), 3.44 - 3.38 (m, 2H), 3.08 - 2.73 (m, 3H), 2.63 (t, *J* = 6.8 Hz, 1H), 2.50 - 2.10 (m, 3H), 2.10 - 1.90 (m, 1H), 1.64 (d, *J* = 4.0 Hz, 4H), 1.28 (s, 10H). LCMS [M+H]⁺ = 954.7 |
| **991** | 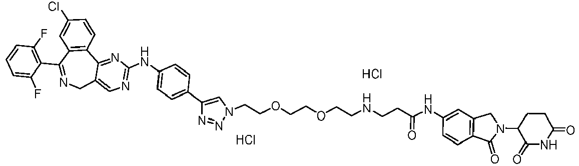 |
| | ¹H NMR (400 MHz, DMSO-*d*₆) δ10.98 (s, 1H), 10.77 (s, 1H), 10.02 (s, 1H), 9.01 (s, 2H), 8.69 (d, *J* = 6.4 Hz, 1H), 8.49 (s, 1H), 8.31 (dd, *J* = 8.5, 4.0 Hz, 1H), 8.02 - 7.86 (m, 5H), 7.82 (s, 1H), 7.82 - 7.42 (m, 5H), 7.36 - 7.01 (m, 4H), 5.04 (dt, *J* = 9.2, 9.6 Hz, 1H), 4.74 - 4.56 (m, 2H), 3.86- 3.74 (m, 2H), 3.67 - 3.64 (m, 2H), 3.63- 3.54 (m, 4H), 3.32 - 3.12 (m, 2H), 3.12 - 2.87 (m, 2H), 2.87 - 2.78 (m, 3H), 2.69 - 2.55 (m, 1H), 2.36 (dt, *J* = 13.2, 9.0 Hz, 1H), 2.01 - 1.84 (m, 1H). LCMS [M+H]⁺ =944.7 |
| **994** | 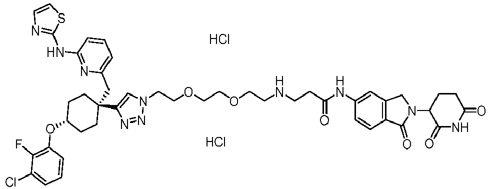 |
| | ¹H NMR (400 MHz, DMSO-*d*₆) δ10.98 (s, 1H), 10.68 (s, 1H), 10.18 (s, 1H), 8.86 (s, 2H), 8.02 -7.66(m, 4H),7.52 -7.42 (m, 2H), 7.26 - 6.91 (m, 5H), 6.24-6.19 (m, 2H), 5.17-5.21(m,1H),4.51-4.22(m,5H), 3.75 - 3.61 (m, 6H), 3.25 - 2.78 (m, 8H), 2.63 (t, *J* = 6.3 Hz, 2H), 2.40 - 2.14 (m, 1H), 2.04 (dd, *J* = 14.3, 9.0 Hz, 6H), 1.66 - 1.54 (m, 2H). LCMS [M+H]⁺ =929.7 |
| **995** | 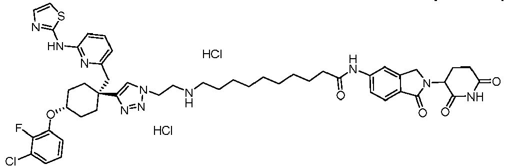 |
| | ¹H NMR (400 MHz, DMSO-*d*₆) δ11.03 (s, 1H), 10.29 (s, 1H), 9.06 (s, 2H), 7.92 - 7.63 (m, 4H), 7.47 (q, *J* = 8.2 Hz, 2H), 7.54 - 7.07 (m, 3H), 7.07-6.21 (m, 3H ) , 5.17-5.21(m,1H), 4.64 - 4.37 (m, 5H), 3.11 - 2.58 (m, 8H), 2.35 (dd, *J* = 13.3, 5.7 Hz, 4H), 2.14 - 1.82 (m, 6H), 1.61 - 1.52 (m, 6H), 1.26 (d, *J* = 8.4 Hz, 10H). LCMS [M+H]⁺ = 939.7 |
| **996** | 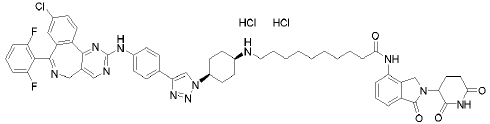 |
| | ¹H NMR (400 MHz, DMSO-d6) δ 11.03 (s, 1H), 10.02 (s, 1H), 9.89 (s, 1H), 9.36 - 9.20 (m, 1H), 8.84 (s, 2H), 8.78 (s, 1H), 8.70 (s, 1H), 8.31 (d, J = 8.5 Hz,1H), 7.95 - 7.84 (m, 3H), 7.83 - 7.79 (m, 2H), 7.59 - 7.44 (m, 3H), 7.33 (d, J = 2.2 Hz, 1H), 7.17 (t, J = 8.8 Hz, 2H), 5.15 (dd, J = 13.3, 5.1 Hz, 1H), 4.74 - 4.66 (m, 1H), 4.37 (q, J = 17.6 Hz, 2H), 3.57 (d, J = 2.8 Hz, 4H), 3.50 (s, 2H), 3.26 (s, 2H), 3.12 (qd, J = 7.4, 4.2 Hz, 2H), 2.90 (dq, J = 14.2, 8.6, 6.8 Hz,3H), 2.65 - 2.58 (m, 1H), 2.41 - 2.30 (m, 3H), 1.99 (ddq, J = 20.2, 9.5, 4.9 Hz, 5H), 1.80 (d, J = 10.8 Hz, 2H), 1.63 (dt, J = 14.1, 7.5 Hz, 4H), 1.30 (d, J =2.1 Hz, 6H). LCMS [M+H]⁺ = 1008.8 |
| **997** | 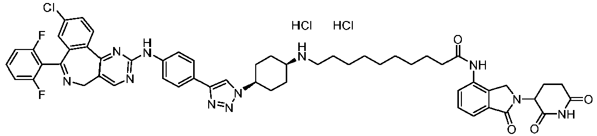 |
| | ¹H NMR (400 MHz, DMSO-d6) δ 10.97 (s, 1H), 10.35 (s, 1H), 10.02 (s, 1H), 8.80 (d, J = 15.4 Hz, 3H), 8.70 (s, 1H), 8.31 (d, J = 8.5 Hz, 1H), 8.00 (s, 1H),7.94 - 7.86 (m, 3H), 7.86 - 7.78 (m, 2H), 7.63 (d, J = 2.0 Hz, 2H), 7.59 - 7.50 (m, 1H), 7.40 - 7.28 (m, 2H), 7.17 (t, J = 8.6 Hz, 2H), 5.08 (dd, J = 13.3, 5.1 Hz,1H), 4.70 (s, 1H), 4.46 - 4.25 (m, 2H), 3.65 - 3.57 (m, 3H), 3.49 (p, J = 4.2, 3.7 Hz, 3H), 3.27 (s, 2H), 3.12 (tt, J = 7.5, 3.8 Hz, 1H), 2.88 (d, J = 9.8 Hz, 3H),2.59 (d, J = 17.1 Hz, 1H), 2.37 (dd, J = 9.2, 5.6 Hz, 3H), 2.05 - 1.90 (m, 5H), 1.80 (s, 2H), 1.71 - 1.54 (m, 4H), 1.29 (s, 6H). LCMS [M+H]⁺ = 1008.8 |
| **1000** | 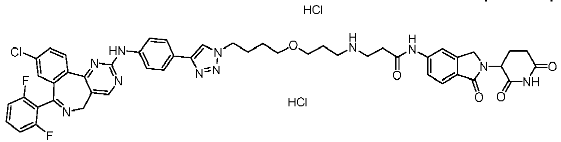 |
| | ¹H NMR (400 MHz, DMSO-*d*₆) δ 10.98 (s, 1H), 10.74 (s, 1H), 10.01 (s, 1H), 8.94 (s, 2H), 8.69 (s, 1H), 8.51 (s, 1H), 8.31 (d, *J* = 8.5 Hz, 1H), 7.95 (d, *J* = 15.7 Hz, 1H), 7.89 (dd, *J* = 13.1, 5.4 Hz, 3H), 7.80 (d, *J* = 8.7 Hz, 2H), 7.67 (s, 2H), 7.65 - 7.42 (m, 1H), 7.34 (d, *J* = 2.0 Hz, 1H), 7.17 (t, *J* = 8.3 Hz, 2H), 5.08 (dd, *J* = 13.2, 5.0 Hz, 1H), 4.96 - 4.91 (m, 3H), 4.42 (dd, *J* = 12.0, 4.8 Hz, 1H), 3.51 - 3.12 (m, 6H), 3.06 - 2.85 (m, 5H), 2.59 (d, *J* = 7.3 Hz, 1H), 2.47 - 2.16 (m, 1H), 1.94 (ddd, *J* = 20.1, 12.7, 5.8 Hz, 5H), 1.54 (dd, *J* = 14.3, 6.5 Hz, 2H). LCMS [M+H]⁺ = 939.7 |
| **1001** | 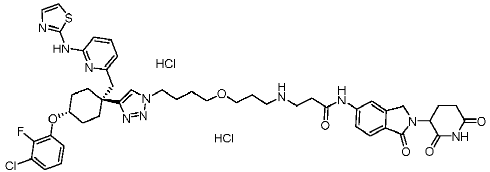 |
| | ¹H NMR (400 MHz, DMSO-*d*₆) δ 10.98 (s, 1H), 10.71 (d, *J* = 5.4 Hz, 1H), 8.92 (d, *J* = 3.6 Hz, 3H), 7.99 (d, *J* = 3.9 Hz, 51H), 7.75 - 7.45 (m, 5H), 7.17 (ddd, *J* = 17.3, 12.4, 11.9 Hz, 1H), 7.13 - 7.04 (m, 3H), 6.97 (d, *J* = 8.1 Hz, 1H), 6.27-6.25 (m, 1H), 5.08 (dd, *J* = 13.2, 5.0 Hz, 1H), 4.67- 4.20 (m, 5H), 3.66 - 3.41 (m, 6H), 3.15 - 2.49 (m, 6H), 2.65 - 2.49 (m, 1H), 2.45 - 2.32 (m, 1H), 2.04 (dd, *J* = 26.5, 11.8 Hz, 4H), 1.92 - 1.82 (m,5H), 1.78 - 1.12 (m, 7H). LCMS [M+H]⁺ = 927.7 |
| **1002** | 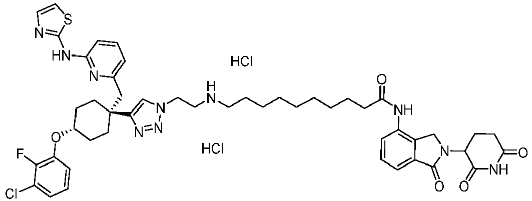 |
| | ¹H NMR (400 MHz, DMSO-*d*₆) δ 10.98 (s, 1H), 9.84(s, 1H), 9.19 (s, 2H), 7.81 (d, *J* = 6.9 Hz, 2H), 7.76 - 7.36 (m, 4H), 7.21 - 6.93 (m, 5H), 6.27-6.25 (m, 1H) ,5.15 (dd, *J* = 13.2, 5.1 Hz, 1H), 4.55 (d, *J* = 8.1 Hz, 3H), 4.37 (dd, *J* = 3.7, 7.5 Hz, 2H), 3.45 (d, *J* = 4.7 Hz, 2H), 3.02 (ddd, *J* = 8.4, 3.3, 3.6 Hz, 2H), 2.87 - 2.68 (m, 3H), 2.67 - 2.54 (m, 1H), 2.55 - 2.46(m, 3H), 2.39 - 1.87 (m,5H), 1.86 - 1.53 (m, 8H), 1.26 (d, *J* = 8.6 Hz, 10H),. LCMS [M+H]⁺ = 939.8 |
| **1003** | 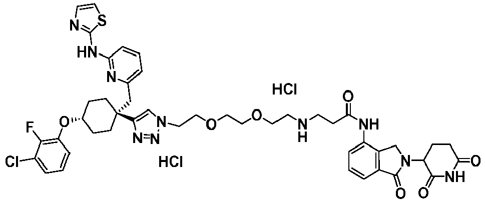 |
| | ¹H NMR (400 MHz, DMSO-*d*₆) δ 11.03 (s, 1H), 10.16 (d, *J* = 5.4 Hz, 1H), 8.92 (d, *J* = 3.6 Hz, 2H), 7.89 (d, *J* = 3.9 Hz, 1H), 7.85 - 7.45 (m, 5H), 7.13 - 7.04 (m, 4H), 6.97 (d, *J* = 8.1 Hz, 1H), 6.27-6.25 (m, 1H), 5.08 (dd, *J* = 13.2, 5.0 Hz, 1H), 4.63 - 4.34 (m, 5H), 3.66 - 3.41 (m, 8H), 3.15 - 2.69 (m, 6H), 2.65 - 2.59 (m, 1H), 2.45 - 2.32 (m, 1H), 2.04 (dd, *J* = 6.5, 9.8 Hz, 4H), 1.78 - 1.52 (m, 2H). LCMS [M+H]⁺ = 929.8 |
| **1005** | 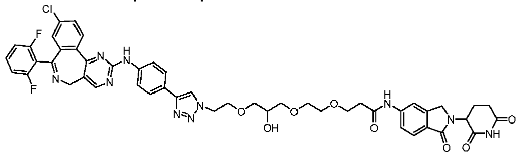 |
| | ¹H NMR (400 MHz, DMSO-d6) δ 10.97 (s, 1H), 10.23 (s, 1H), 10.00 (s, 1H), 8.69 (s, 1H), 8.38 (s, 1H), 8.31 (d, J = 8.5 Hz, 1H), 8.01 - 7.77 (m,7H), 7.68 - 7.48 (m, 3H), 7.33 (d, J = 2.2 Hz, 1H), 7.17 (s, 2H), 5.32 (d, J = 5.5 Hz, 1H), 5.07 (dd, J = 13.2, 5.1 Hz, 1H), 4.54 - 4.22 (m, 5H), 4.02(s, 1H), 3.46 - 3.37 (m, 4H), 3.35 (d, J = 7.7 Hz, 2H), 3.31 (s, 2H), 2.90 (ddd, J = 18.3, 13.5, 5.5 Hz, 1H), 2.67 - 2.56 (m, 2H), 2.36 (s, 3H), 1.99 (t,J = 5.8 Hz, 2H), 1.68 - 1.48 (m, 5H), 1.32 (s, 7H). LCMS [M+H] ⁺ = 957.8 |
| **1006** | 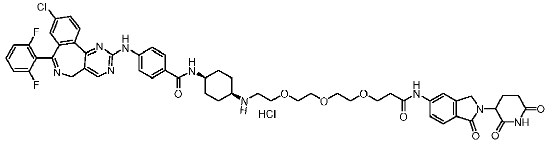 |
| | ¹H NMR (400 MHz, DMSO-d6) δ 10.97 (s, 1H), 10.52 (d, J = 31.8 Hz, 1H), 10.16 (d, J = 2.8 Hz, 1H), 8.99 - 8.65 (m, 4H), 8.29 (d, J = 8.5 Hz, 1H), 8.00 (d, J= 4.5 Hz, 1H), 7.88 (dd, J = 8.2, 3.7 Hz, 5H), 7.65 (d, J = 5.4 Hz, 2H), 7.54 (p, J = 7.2 Hz, 1H), 7.33 (d, J = 2.1 Hz, 1H), 7.16 (t, J = 8.8 Hz, 2H), 5.07 (dd, J =13.3, 5.1 Hz, 1H), 4.44 - 4.25 (m, 2H), 4.16 - 4.08 (m, 1H), 3.97 (d, J = 4.8 Hz, 2H), 3.73 (s, 2H), 3.62 (s, 4H), 3.12 - 3.05 (m, 4H), 2.93 - 2.83 (m, 2H), 2.66 - 2.55 (m, 3H), 2.42 - 2.31 (m, 1H), 2.04 - 1.92 (m, 4H), 1.85 (h, J = 8.4, 6.9 Hz, 4H), 1.55 (tt, J = 10.6, 5.7 Hz, 2H), 1.38 (dd, J = 10.1, 6.5 Hz, 4H). LCMS [M+H]⁺ = 1018.9 |
| **1007** | 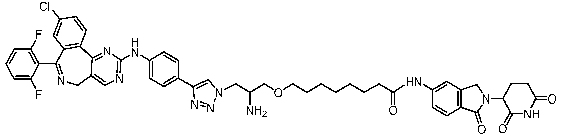 |
| | LCMS [M+H]⁺ = 956.4 |
| **1008** | 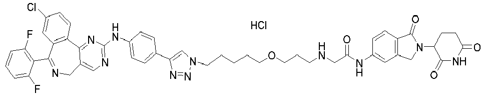 |
| | ¹H NMR (400 MHz, DMSO-*d*₆) δ 10.96 (m, 2H), 10.01 (s, 1H), 8.93 (m, 2H), 8.69 (s, 1H), 8.48 (s, 1H), 8.30 (d, *J* = 8.5 Hz, 1H), 7.97 - 7.84 (m, 4H), 7.83 - 7.75 (m, 2H), 7.72 (d, *J* = 8.3 Hz, 1H), 7.65 (d, *J* = 8.4 Hz, 1H), 7.55 (m, 1H), 7.34 (d, *J* = 2.2 Hz, 1H), 7.17 (t, *J* = 8.9 Hz, 2H), 5.09 (dd, *J* = 13.3, 5.1 Hz, 1H), 4.48 - 4.29 (m, 4H), 4.01 (t, *J* = 5.8 Hz, 2H), 3.45 (m, 5H), 3.05 (s, 2H), 2.94 - 2.87 (m, 1H), 2.59 (m, 2H), 2.42 - 2.29 (m, 2H), 2.04 - 1.81 (m, 6H), 1.60 - 1.51 (m, 2H), 1.37 - 1.30 (m, 2H). LCMS [M+H]⁺ = 943.0 |
| **1009** | 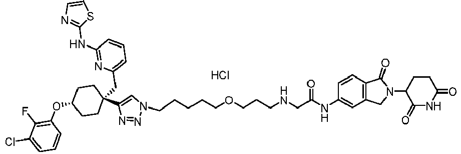 |
| | ¹H NMR (400 MHz, DMSO-*d*₆) δ 11.22 (s, 1H), 10.98 (d, *J* = 6.1 Hz, 2H), 8.96 (br, 2H), 7.93 (d, *J* = 1.7 Hz, 1H), 7.72 (d, *J* = 8.2 Hz, 1H), 7.70 - 7.62 (m, 2H), 7.43 - 7.33 (m, 2H), 7.21 - 7.08 (m, 3H), 6.97 (d, *J* = 3.6 Hz, 1H), 6.82 (d, *J* = 8.2 Hz, 1H), 6.07 (d, *J* = 7.3 Hz, 1H), 5.09 (dd, *J* = 13.3, 5.1 Hz, 1H), 4.57 - 4.42 (m, 2H), 4.31 (d, *J* = 17.4 Hz, 1H), 4.21 (t, *J* = 6.9 Hz, 2H), 4.03 (m, 2H), 3.46 (m, 2H), 3.07 (m, 2H), 2.95 (m, 3H), 2.59 (m, 2H), 2.38 (m, 1H), 2.13 - 1.79 (m, 10H), 1.70 (m, 2H), 1.59 - 1.45 (m, 4H), 1.17 (m, 2H). LCMS [M+H]⁺ = 928.0 |
| **1010** | 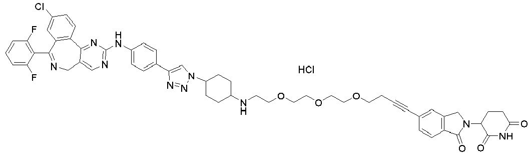 |
| | ¹H NMR (400 MHz, DMSO-*d*₆) δ 11.00 (s, 1H), 10.03 (s, 1H), 9.00 (s, 2H), 8.80 (s, 1H), 8.70 (s, 1H), 8.31 (d, *J* = 8.5 Hz, 1H), 7.97 - 7.77 (m, 5H), 7.69 (d, *J* = 7.8 Hz, 1H), 7.62 (s, 1H), 7.59 - 7.47 (m, 2H), 7.34 (d, *J* = 2.3 Hz, 2H), 7.18 (t, *J* = 8.8 Hz, 2H), 5.11 (dd, *J* = 13.3, 5.0 Hz, 1H), 4.70 (s, 1H), 4.52 - 4.28 (m, 2H), 3.96 - 3.90 (m, 9H), 3.75 (t, *J* = 5.4 Hz, 2H), 3.62 (t, *J* = 6.7 Hz, 2H), 3.30 (s, 1H), 3.12 (p, *J* = 5.5 Hz, 2H), 2.91 (m, 1H), 2.72 (t, *J* = 6.7 Hz, 2H), 2.62 - 2.56 (m, 1H), 2.37 (m, 1H), 2.06 - 1.78 (m, 6H). LC-MS: [M+H] ⁺ =903, 452 |
| **1011** | 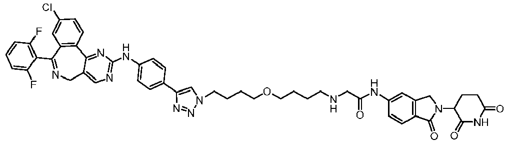 |
| | ¹H NMR (400 MHz, DMSO-*d*₆) δ 11.23 (s, 1H), 10.98 (s, 1H), 10.01 (s, 1H), 9.16 (s, 2H), 8.69 (s, 1H), 8.51 (s, 1H), 8.30 (d, *J* = 8.5 Hz, 1H), 7.96 - 7.87 (m, 3H), 7.80 (d, *J* = 8.4 Hz, 2H), 7.71 (s, 2H), 7.59 - 7.44 (m, 1H), 7.34 (d, *J* = 2.2 Hz, 1H), 7.17 (t, *J* = 8.8 Hz, 2H), 5.09 (dd, *J* = 13.3, 5.1 Hz, 1H), 4.50 - 4.27 (m, 4H), 4.02 (t, *J=* 5.7 Hz, 2H), 3.44 - 3.34 (m, 4H), 3.08 - 2.86 (m, 3H), 2.71 - 2.59 (m, 1H), 2.42 - 2.32 (m, 1H), 2.05 - 1.87 (m, 3H), 1.76 - 1.67 (m, 2H), 1.58 - 1.44 (m, 4H). LC-MS: [M+H]⁺ =942.8 |
| **1012** | 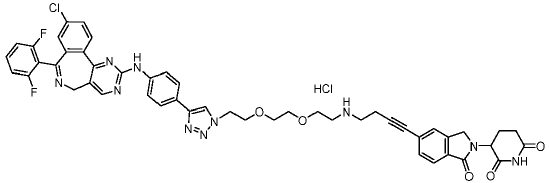 |
| | ¹H NMR (400 MHz, DMSO-d6) δ 11.00 (s, 1H), 10.02 (s, 1H), 8.99 (s, 2H), 8.68 (s, 1H), 8.46 (s, 1H), 8.30 (d, J = 8.5 Hz, 1H), 7.94 - 7.86 (m, 3H), 7.83-7.78 (m, 2H), 7.69 (d, J = 7.9 Hz, 1H), 7.64 (s, 1H), 7.55 (t, J = 7.4 Hz, 2H), 7.40 - 7.31 (m, 3H), 7.18 (q, J = 8.6, 7.7 Hz, 2H), 5.11 (dd, J = 13.3, 5.1Hz, 1H), 4.58 (t, J = 5.2 Hz, 2H), 4.45 - 4.28 (m, 2H), 3.90 (t, J = 5.2 Hz, 2H), 3.68 (t, J = 5.1 Hz, 2H), 3.59 (tt, J = 5.4, 2.6 Hz, 4H), 3.21 - 3.08 (m, 4H),2.95 - 2.83 (m, 3H), 2.59 (d, J = 17.1 Hz, 1H), 2.42 - 2.32 (m, 1H), 2.04 - 1.94 (m, 1H). LCMS [M+H]⁺ = 925.9 |
| **1013** | 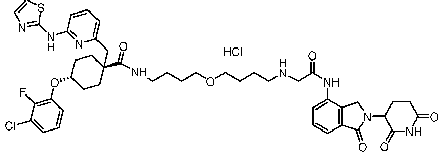 |
| | ¹H NMR (400 MHz, DMSO-*d*₆) δ 12.29 - 12.03 (m, 1H), 11.05 (s, 1H), 10.86 (s, 1H), 9.29 (s, 2H), 7.88 (d, *J* = 7.4 Hz, 1H), 7.73 - 7.47 (m, 5H), 7.24 - 6.98 (m, 5H), 6.75 (d, *J* = 7.4 Hz, 1H), 5.17 (dd, *J* = 13.2, 5.1 Hz, 1H), 4.55 - 4.26 (m, 2H), 4.03 (s, 2H), 3.43 - 3.28 (m, 4H), 2.99 (d, *J* = 18.9 Hz, 6H), 2.62 (d, *J* = 17.2 Hz, 2H), 2.36 - 2.17 (m, 1H), 2.12 - 1.99 (m, 1H), 1.96 - 1.88 (m, 2H), 1.82 - 1.53 (m, 10H), 1.43 - 1.36 (m, 4H). LC-MS: [M+H] ⁺ =903, 452 |
| **1014** | 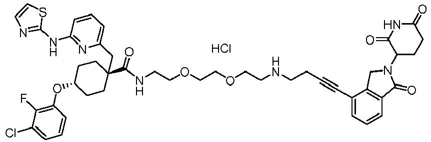 |
| | ¹H NMR (400 MHz, DMSO-d6) δ 11.65 (s, 1H), 11.02 (s, 1H), 9.30 (d, J = 13.7 Hz, 2H), 7.74 (d, J = 7.2 Hz, 2H), 7.69 (d, J = 7.6 Hz, 1H), 7.61 (t, J = 7.8 Hz,1H), 7.54 (t, J = 7.6 Hz, 1H), 7.43 (d, J = 3.8 Hz, 1H), 7.14 (dtd, J = 26.3, 8.5, 4.8 Hz, 3H), 7.05 (d, J = 3.8 Hz, 1H), 6.96 (d, J = 8.1 Hz, 1H), 6.72 (d, J = 7.3Hz, 1H), 5.15 (dd, J = 13.3, 5.1 Hz, 1H), 4.59 - 4.33 (m, 3H), 3.74 (s, 3H), 3.00 - 2.93 (m, 4H), 2.63 (s, 1H), 2.10 - 1.57 (m, 12H). LCMS [M+H]⁺ = 886.8 |
| **1015** | 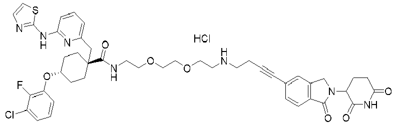 |
| | ¹H NMR (400 MHz, DMSO-d6) δ 11.58 (s, 1H), 11.00 (s, 1H), 9.20 (s, 2H), 7.71 (dd, J = 16.3, 8.4 Hz, 3H), 7.65 - 7.53 (m, 2H), 7.41 (d, J = 3.7 Hz, 1H), 7.19-7.08 (m, 3H), 7.03 (d, J = 3.7 Hz, 1H), 6.95 (d, J = 8.2 Hz, 1H), 6.71 (d, J = 7.4 Hz, 1H), 5.11 (dd, J = 13.2, 5.1 Hz, 1H), 4.59 - 4.29 (m, 3H), 3.73 (s, 2H),2.98 - 2.91 (m, 4H), 2.62 (s, 2H), 2.00 (dt, J = 9.7, 6.4 Hz, 2H), 1.90 (d, J = 13.3 Hz, 2H), 1.84 - 1.73 (m, 4H), 1.65 (d, J = 13.4 Hz, 2H). LCMS [M+H]⁺ = 886.7 |
| **1016** | 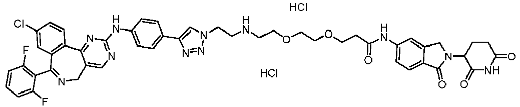 |
| | ¹H NMR (400 MHz, DMSO-*d*₆) δ10.98 (s, 1H), 10.54 (d, *J* = 8.5 Hz, 1H), 10.05 (s, 1H), 9.25 (s, 1H), 8.70 (s, 1H), 8.57 (s, 1H), 8.32 (d, *J* = 8.5 Hz, 1H), 8.05 - 7.87 (m, 4H), 7.82 (s, 1H), 7.81 - 7.51 (m, 4H), 7.35 (d, *J* = 2.0 Hz, 1H), 7.31 - 7.06 (m, 2H), 5.05 (dt, *J* = 7.8, 3.9 Hz, 1H), 4.81 (t, *J* = 6.2 Hz, 2H), 4.41 (dd, *J* = 7.3, 8.0 Hz, 1H), 4.34 (ddd, *J* = 4.9, 7.3, 7.9 Hz, 1H), 3.75 - 3.56 (m, 12H), 3.37 (d, *J* = 2.8 Hz, 2H), 3.07 - 2.97 (m, 1H), 2.97 - 2.83 (m, 3H), 2.48 - 2.26 (m, 1H), 2.13 - 1.83 (m, 1H). LCMS [M+H]⁺ = 929.8 |
| **1017** | 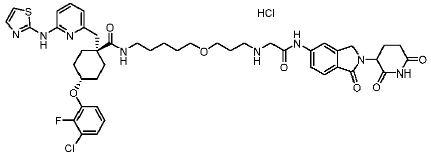 |
| | ¹H NMR (400 MHz, DMSO-*d*₆) δ 11.3 (m, 2H),10.99 (m, 2H), 8.98 (m, 2H), 7.93 (s, 1H), 7.79 - 7.52 (m, 3H), 7.39 (m, 1H), 7.20 - 7.08 (m, 2H), 7.04 - 6.83 (m, 2H), 6.64 (d, *J* = 8.5 Hz, 1H), 5.09 (dd, *J* = 13.3, 5.1 Hz, 1H), 4.55 (m, 1H), 4.45 (d, *J* = 17.4 Hz, 1H), 4.32 (d, *J* = 17.4 Hz, 1H), 4.03 (m, 2H), 3.44 (m, 4H), 3.38 (m, 2H), 3.32 (m, 2H), 3.02 (m, 4H), 2.90 (m, 3H), 2.59 (m, 2H), 2.42 - 2.32 (m, 1H), 2.07 - 1.55 (m, 10H), 1.42 (m, 4H). LCMS [M+H]⁺ = 903.9 |
| **1018** | 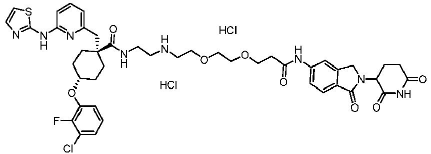 |
| | ¹H NMR (400 MHz, DMSO-*d*₆) δ10.98 (s, 1H), 10.44 (s, 1H), 8.86 (s, 2H), 7.99 (s, 2H), 7.83 - 7.53 (m, 3H), 7.35 (d, *J* = 2.0 Hz, 1H), 7.31 - 7.06 (m, 4H), 6.98 - 6.70 (m, 2H), 5.05 (dt, *J* = 7.8, 3.9 Hz, 1H), 4.41 (dd, *J* = 7.3, 8.0 Hz, 1H), 4.34 (ddd, *J* = 4.9, 7.3, 7.9 Hz, 1H), 3.75 - 3.56 (m, 9H), 3.07 - 2.77 (m, 7H), 2.71-2.63 (m, 3H), 2.48 - 2.36 (m, 1H), 2.03 - 163 (m, 9H). LCMS [M+H]⁺ = 905.8 |
| **1019** | 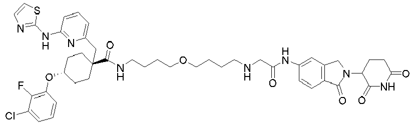 |
| | ¹H NMR (400 MHz, DMSO-*d*₆) δ 12.16 - 11.78 (m, 1H), 11.30 (s, 1H), 10.99 (s, 1H), 9.22 (s, 2H), 7.96 (s, 1H), 7.76 - 7.61 (m, 4H), 7.47 (d, *J* = 3.9 Hz, 1H), 7.18 - 6.98 (m, 5H), 6.74 (d, *J* = 7.4 Hz, 1H), 5.09 (dd, *J* = 13.3, 5.1 Hz, 1H), 4.56 (s, 1H), 4.50 - 4.25 (m, 2H), 4.03 (s, 2H), 3.41 - 3.29 (m, 4H), 2.93 - 2.87 (m, 7H), 2.63 - 2.57 (m, 1H), 2.42 - 2.31 (m, 1H), 2.05 - 1.87 (m, 3H), 1.84 - 1.53 (m, 10H), 1.48 - 1.39 (m, 4H). LC-MS: [M+H] ⁺ =903, 452 |
| **1020** | 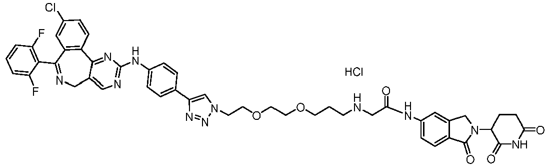 |
| | ¹H NMR (400 MHz, DMSO-*d*₆) δ 11.24 (s, 1H), 10.99 (s, 1H), 10.02 (s, 1H), 9.14 (s, 2H), 8.69 (s, 1H), 8.47 (s, 1H), 8.31 (d, *J* = 8.5 Hz, 1H), 7.97 - 7.86 (m, 4H), 7.80 (d, *J* = 8.5 Hz, 2H), 7.70 (s, 2H), 7.55 (td, *J* = 8.5, 4.1 Hz, 1H), 7.34 (d, *J* = 2.2 Hz, 1H), 7.17 (t, *J* = 9.0 Hz, 2H), 5.09 (dd, *J* = 13.3, 5.1 Hz, 1H), 4.50 - 4.24 (m, 6H), 4.04 - 3.86 (m, 4H), 3.62 - 3.42 (m, 6H), 3.10 - 2.98 (m, 2H), 2.91 (ddd, *J* = 18.0, 13.5, 5.4 Hz, 1H), 2.67 - 2.55 (m, 2H), 2.41 - 2.28 (m, 1H), 2.05 - 1.84 (m, 3H). LC-MS: [M+H] ⁺ =904.9, 473.2 |
| **1027** | 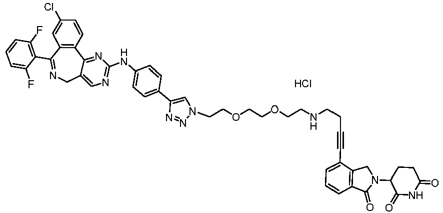 |
| | ¹H NMR (400 MHz, DMSO-d6) δ 11.01 (s, 1H), 10.01 (s, 1H), 9.20 (s, 2H), 8.68 (s, 1H), 8.47 (s, 1H), 8.30 (d, J = 8.5 Hz, 1H), 7.95 - 7.84 (m, 3H), 7.80(d, J = 8.4 Hz, 2H), 7.68 (dd, J = 24.2, 7.5 Hz, 2H), 7.53 (dt, J = 18.2, 7.1 Hz, 2H), 7.42 - 7.29 (m, 4H), 7.16 (q, J = 6.2, 3.5 Hz, 2H), 5.68 (s, 1H), 5.15 (dd,J = 13.3, 5.1 Hz, 1H), 4.58 (t, J = 5.2 Hz, 2H), 4.53 - 4.35 (m, 2H), 3.89 (t, J = 5.3 Hz, 2H), 3.69 (t, J = 5.2 Hz, 2H), 3.62 - 3.56 (m, 4H), 3.14 (d, J = 27.1Hz, 4H), 2.98 - 2.91 (m, 2H), 2.61 (s, 1H), 2.44 - 2.35 (m, 1H), 2.01 (s, 1H). LCMS [M+H]⁺ = 925.8 |
| **1028** | 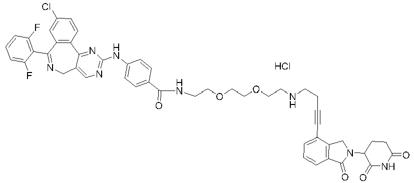 |
| | ¹H NMR (400 MHz, DMSO-d6) δ 11.00 (s, 1H), 10.17 (s, 1H), 9.62 - 9.39 (m, 5H), 9.26 (s, 2H), 8.72 (s, 1H), 8.47 (t, J = 5.6 Hz, 1H), 8.29 (d, J = 8.5 Hz,1H), 7.89 (q, J = 8.8 Hz, 5H), 7.73 - 7.65 (m, 2H), 7.55 (t, J = 7.6 Hz, 2H), 7.33 (d, J = 2.1 Hz, 1H), 7.17 (t, J = 9.2 Hz, 2H), 5.10 (dd, J = 13.2, 5.1 Hz,1H), 4.43 (d, J = 17.5 Hz, 2H), 3.10 (q, J = 3.4 Hz, 6H), 2.93 (dd, J = 15.7, 8.1 Hz, 4H), 2.57 (s, 1H), 2.42 - 2.33 (m, 1H), 2.05 - 1.94 (m, 2H). LCMS [M+H]⁺ = 901.7 |
| **1029** | 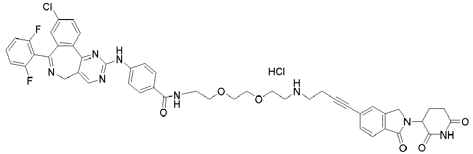 |
| | ¹H NMR (400 MHz, DMSO-d6) δ 11.02 (s, 1H), 10.15 (d, J = 3.7 Hz, 1H), 9.06 (s, 2H), 8.71 (d, J = 5.2 Hz, 1H), 8.43 (t, J = 5.7 Hz, 1H), 8.29(d, J = 8.5 Hz, 1H), 7.93 - 7.80 (m, 6H), 7.70 (dd, J = 22.6, 7.5 Hz, 2H), 7.53 (qd, J = 8.0, 2.2 Hz, 2H), 7.41 - 7.24 (m, 2H), 7.16 (t, J = 9.6 Hz,2H), 5.14 (dd, J = 13.3, 5.1 Hz, 1H), 4.50 (d, J = 17.8 Hz, 1H), 4.34 (d, J = 17.9 Hz, 1H), 3.60 (s, 4H), 2.93 (s, 3H), 2.07 - 1.95 (m, 2H). LCMS [M+H]⁺ = 901.8 |
| **1030** | 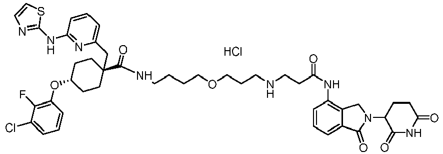 |
| | ¹H NMR (400 MHz, DMSO-*d*₆) δ 11.27 (br, 1H), 11.04 (s, 1H), 10.12 (br, 1H), 8.58 (br, 2H), 7.85 (dd, *J* = 7.4, 1.6 Hz, 1H), 7.64 - 7.48 (m, 4H), 7.38 (m, 1H), 7.18 - 7.08 (m, 3H), 6.99 (m, 1H), 6.89 (m, 1H), 6.63 (m, 1H), 5.17 (dd, *J* = 13.2, 5.1 Hz, 1H), 4.55 (m, 1H), 4.43 (d, *J* = 17.7 Hz, 1H), 4.33 (d, *J* = 17.7 Hz, 1H), 3.23 (m, 4H), 3.03 (m, 4H), 2.92 (m, 3H), 2.84 (m, 2H), 2.62 (m, 2H), 2.27 (m, 1H), 2.02 (m, 2H), 1.93 - 1.74 (m, 8H), 1.64 (m, 2H), 1.42 (m, 4H). LCMS [M+H]⁺ = 903.9 |
| **1031** | 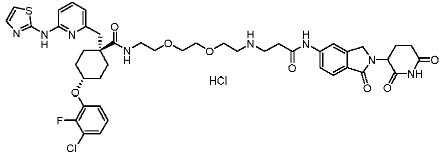 |
| | ¹H NMR (400 MHz, DMSO-*d*₆) δ 10.98 (s, 1H), 10.73 (d, *J* = 7.9 Hz, 1H), 8.96 (d, *J* = 4.28 Hz, 2H), 7.96 (s, 1H), 7.80 - 7.53 (m, 4H), 7.53 - 7.30 (m, 1H), 7.28 - 6.97(m, 5H), 6.78 (dd, *J* = 5.3, 7.3 Hz, 1H), 5.08 (dd, *J* = 13.2, 5.1 Hz, 2H), 4.49 (d, *J* = 4.91 Hz, 3H), 4.02-3.53 (m, 17H), 3.40 - 3.16 (m, 8H), 2.86 - 2.64 (m, 4H), 2.69 - 2.47 (m, 1H), 2.44 - 2.26 (m, 1H), 2.06 - 1.77 (m, 9H). LCMS [M+H]⁺ = 905.9 |
| **1032** | 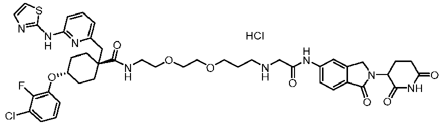 |
| | 1HNMR (400 MHz, DMSO-d6) δ 10.99 (s, 1H), 8.98 (s, 2H), 7.93 (s, 1H), 7.71 (t, *J* = 11.6 Hz, 3H), 7.56 (s, 1H), 7.38 (d, *J* = 4.5 Hz, 1H), 7.22 - 7.07 (m, 3H), 6.99 (d, *J* = 3.6 Hz, 1H), 6.94 - 6.81 (m, 1H), 6.75 - 6.65 (m, 1H), 5.09 (dd, *J* = 13.2, 5.1 Hz, 1H), 4.54 (s, 1H), 4.50 - 4.26 (m, 2H), 4.03 (s, 2H), 3.63 - 3.49 (m, 4H), 3.37 (t, *J* = 6.3 Hz, 2H), 3.23 - 3.16 (m, 2H), 3.06 (s, 2H), 2.99 - 2.83 (m, 2H), 2.70 - 2.57 (m, 1H), 2.42 - 2.25 (m, 1H), 2.07 - 1.70 (m, 9H), 1.69 - 1.59 (m, 2H). LC-MS: [M+H] + =906 |
| **1033** | 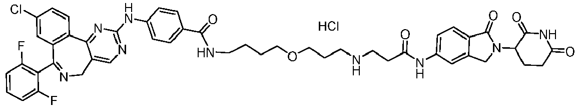 |
| | ¹H NMR (400 MHz, DMSO-*d*₆) δ 10.98 (s, 1H), 10.62 (s, 1H), 10.15 (s, 1H), 8.72 (s, 1H), 8.65 (br, 2H), 8.36 - 8.26 (m, 2H), 7.96 (s, 1H), 7.92 - 7.81 (m, 5H), 7.70 - 7.62 (m, 2H), 7.60 - 7.51 (m, 1H), 7.34 (d, *J* = 2.1 Hz, 1H), 7.17 (t, *J* = 8.8 Hz, 2H), 5.08 (dd, *J* = 13.3, 5.1 Hz, 1H), 4.42 (d, *J* = 17.4 Hz, 1H), 4.29 (d, *J* = 17.3 Hz, 1H), 3.48 - 3.38 (m, 6H), 3.24 (dd, *J* = 11.6, 5.8 Hz, 4H), 3.02 (m, 2H), 2.94 - 2.84 (m, 3H), 2.58 (m, 1H), 2.39 - 2.30 (m, 1H), 2.02 - 1.94 (m, 1H), 1.87 (m, 2H), 1.56 (m, 4H). LCMS [M+H]⁺ = 918.7 |
| **1034** | 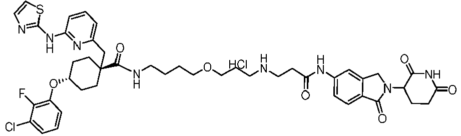 |
| | ¹H NMR (400 MHz, DMSO-*d*₆) δ 12.29 (br, 1H), 10.98 (s, 1H), 10.73 (d, *J* = 4.3 Hz, 1H), 8.95 (s, 2H), 7.96 (s, 1H), 7.76 - 7.64 (m, 4H), 7.51 (d, *J* = 4.0 Hz, 1H), 7.20 - 7.02 (m, 5H), 6.79 (d, *J* = 7.1 Hz, 1H), 5.08 (dd, *J* = 13.3, 5.1 Hz, 1H), 4.55 (m, 1H), 4.42 (d, *J* = 17.3 Hz, 1H), 4.29 (d, *J* = 17.3 Hz, 1H), 3.44 (t, *J* = 5.9 Hz, 2H), 3.38 - 3.32 (m, 2H), 3.25 - 3.18 (m, 2H), 3.06 - 2.95 (m, 6H), 2.91 (m, 2H), 2.63 - 2.57 (m, 1H), 2.36 (m, 1H), 2.08 - 1.71 (m, 10H), 1.66 (m, 2H), 1.49 - 1.36 (m, 4H). LCMS [M+H]⁺ = 903.8 |
| **1035** | 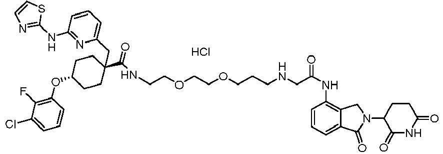 |
| | ¹H NMR (400 MHz, DMSO-*d*₆) δ 11.24 (s, 1H), 10.99 (s, 1H), 10.02 (s, 1H), 9.14 (s, 2H), 8.69 (s, 1H), 8.47 (s, 1H), 8.31 (d, *J* = 8.5 Hz, 1H), 7.97 - 7.86 (m, 4H), 7.80 (d, *J* = 8.5 Hz, 2H), 7.70 (s, 2H), 7.55 (td, *J* = 8.5, 4.1 Hz, 1H), 7.34 (d, *J* = 2.2 Hz, 1H), 7.17 (t, *J* = 9.0 Hz, 2H), 5.09 (dd, *J* = 13.3, 5.1 Hz, 1H), 4.50 - 4.24 (m, 6H), 4.04 - 3.86 (m, 4H), 3.62 - 3.42 (m, 6H), 3.10 - 2.98 (m, 2H), 2.91 (ddd, *J* = 18.0, 13.5, 5.4 Hz, 1H), 2.67 - 2.55 (m, 2H), 2.41 - 2.28 (m, 1H), 2.05 - 1.84 (m, 3H). LC-MS: [M+H] ⁺ =904.9, 473.2 |
| **1036** | 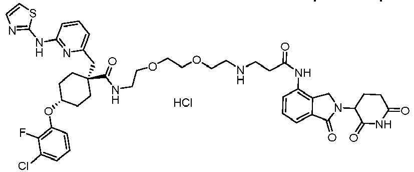 |
| | ¹H NMR (400 MHz, DMSO-*d*₆) δ 10.98 (s, 1H), 10.17(d, *J* = 7.9 Hz, 1H), 8.86 (d, *J* = 4.28 Hz, 2H), 7.96-7.82 (m, 1H), 7.80 - 7.53 (m, 4H), 7.48 - 7.40 (m, 1H), 7.28 - 6.87(m, 5H), 6.78 (dd, *J* = 5.3, 7.3 Hz, 1H), 5.08 (dd, *J* = 13.2, 5.1 Hz, 1H), 4.49 (d, *J* = 4.91 Hz, 3H), 4.02-3.53 (m, 6H), 3.40 - 3.16 (m, 6H), 2.86 - 2.64 (m, 4H), 2.69 - 2.47 (m, 1H), 2.44 - 2.26 (m, 2H), 2.06 - 1.87 (m, 1H). 1.83 - 1.58 (m, 7H). LCMS [M+H]⁺ = 905.7 |
| **1037** | 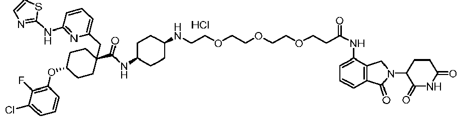 |
| | ¹H NMR (400 MHz, DMSO-d6) δ 11.20 (s, 1H), 11.03 (s, 1H), 10.10 (s, 1H), 9.96 (s, 1H), 8.72 (s, 2H), 8.05 (d, J = 11.4 Hz, 1H), 7.83 (dd, J = 7.1, 2.0 Hz, 1H), 7.58 - 7.47 (m, 3H), 7.44 (t, J = 7.7 Hz, 2H), 7.39 - 7.24 (m, 5H), 7.19 - 7.07 (m, 3H), 6.97 (d, J = 3.7 Hz, 1H), 6.88 (d, J = 8.2 Hz, 1H), 6.62 (d, J = 7.4Hz, 1H), 5.15 (dd, J = 13.3, 5.1 Hz, 1H), 4.55 (s, 1H), 4.37 (q, J = 17.5 Hz, 2H), 3.72 (t, J = 6.3 Hz, 2H), 3.65 (d, J = 5.7 Hz, 2H), 3.06 (s, 2H), 2.91 (s, 4H),2.64 (d, J = 5.8 Hz, 2H), 2.31 (s, 2H), 2.02 (dd, J = 17.0, 9.6 Hz, 5H), 1.88 (s, 2H), 1.80 (d, J = 9.7 Hz, 4H), 1.72 (d, J = 12.0 Hz, 2H), 1.64 (d, J = 12.2 Hz,2H), 1.29 (d, J = 6.6 Hz, 4H). LCMS \|M+H]⁺ = 1003.4 |
| **1038** | 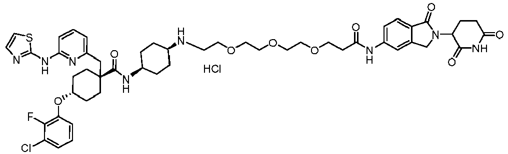 |
| | ¹H NMR (400 MHz, ) δ 11.19 (s, 1H), 10.98 (s, 1H), 10.43 (s, 1H), 8.69 (s, 2H), 8.00 (s, 1H), 7.70 - 7.62 (m, 2H), 7.56 (t, J = 7.8 Hz, 1H), 7.40 - 7.33(m, 2H), 7.23 - 7.06 (m, 5H), 6.97 (d, J = 3.6 Hz, 1H), 6.88 (d, J = 8.2 Hz, 1H), 6.69 - 6.60 (m, 2H), 5.33 (t, J = 4.8 Hz, 1H), 5.08 (dd, J = 13.3, 5.1 Hz,1H), 4.56 (s, 1H), 4.43 (d, J = 17.3 Hz, 1H), 4.29 (d, J = 17.3 Hz, 1H), 3.72 (d, J = 6.2 Hz, 2H), 3.66 (s, 2H), 2.91 (s, 4H), 2.62 (d, J = 6.0 Hz, 2H), 2.57(s, 1H), 2.42 - 2.32 (m, 1H), 2.06 - 1.96 (m, 7H), 1.90 (d, J = 13.2 Hz, 2H), 1.80 (d, J = 10.8 Hz, 4H), 1.73 (d, J = 13.8 Hz, 2H), 1.64 (d, J = 12.2 Hz,2H), 1.43 (d, J = 21.9 Hz, 4H). LCMS \|M+H]⁺ = 1003.4 |
| **1039** | 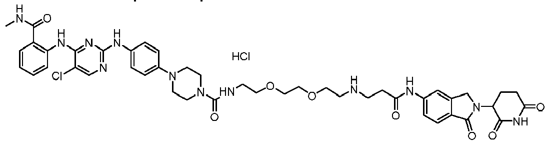 |
| | ¹H NMR (400 MHz, DMSO-*d*₆) δ 12.01 (s, 1H), 10.98 (s, 1H), 10.83 (s, 1H),10.14 (s, 1H), 9.18 - 8.84 (m, 2H), 8.56 (t, *J* = 3.6 Hz, 1H), 8.33 (s, 1H), 7.98 (s, 1H), 7.94 - 7.63 (m, 9H), 7.42 - 7.17 (m, 2H), 5.08 (dd, *J* = 13.2, 5.1 Hz, 1H), 4.45 - 4.34 (m, 2H), 3.79 - 3.03 (m, 19H), 3.08 - 2.70 (m,5H), 2.98 - 2.64 (m, 7H), 2.64 - 2.59 (m, 2H), 2.44 - 2.17 (m, 2H), 2.24 - 1.97 (m, 1H). LCMS [M+H]⁺ = 925.8 |
| **1040** | 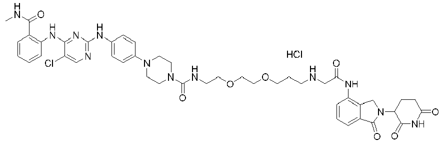 |
| | ¹H NMR (400 MHz, DMSO-*d*₆) δ 11.97 (s, 1H), 11.05 (s, 1H), 10.87 (s, 1H), 10.04 (s, 1H), 9.26 (s, 2H), 8.91 (s, 1H), 8.63 (s, 1H), 8.32 (s, 1H), 7.86 (d, *J* = 23.5 Hz, 2H), 7.73 - 7.38 (m, 6H), 7.21 (s, 1H), 6.91 (s, 1H), 5.17 (m, 1H), 4.65 - 4.33 (m, 3H), 4.06 (s, 3H), 3.46 - 3.36 (m, 7H), 3.23 (s, 4H), 3.06 (s, 4H), 2.81 (s, 3H), 2.68 - 2.61 (m, 1H), 2.36 - 2.23 (m, 1H), 2.11 - 1.87 (m, 3H), 1.34 - 1.22 (m, 2H). LC-MS: [M+H] ⁺ =925.7 |
| **1041** | 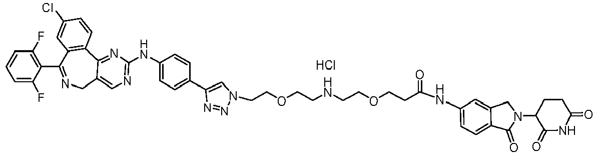 |
| | ¹H NMR (400 MHz, DMSO) δ 10.98 (s, 1H), 10.50 (s, 1H), 10.01 (s, 1H), 8.78 (s, 2H), 8.68 (s, 1H), 8.54 (s, 1H), 8.30 (d, *J* = 8.5 Hz, 1H), 7.98 (s, 1H), 7.92 - 7.86 (m, 3H), 7.81 (d, *J* = 8.7 Hz, 2H), 7.65 (s, 2H), 7.58 - 7.51 (m, 1H), 7.33 (d, *J* = 2.1 Hz, 1H), 7.18 (dd, *J* = 16.6, 8.0 Hz, 2H), 5.07 (dd, *J* = 13.3, 5.1 Hz, 1H), 4.59 (t, *J* = 4.8 Hz, 2H), 4.33 (dd, *J* = 53.9, 17.3 Hz, 2H), 3.87 (t, *J* = 4.9 Hz, 2H), 3.69 (ddd, *J* = 14.7, 9.7, 5.7 Hz, 6H), 3.22 - 3.03 (m, 6H), 2.95 - 2.87 (m, 1H), 2.66 (t, *J* = 6.2 Hz, 2H), 2.59 (d, *J* = 16.9 Hz, 1H), 2.38 - 2.31 (m, 1H), 1.99 (d, *J* = 6.8 Hz, 1H).LCMS [M+H]⁺ =944.8 |
| **1042** | 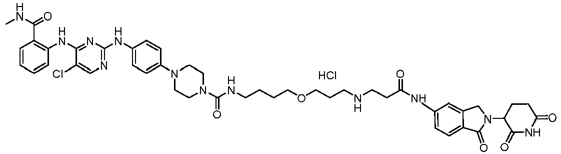 |
| | ¹H NMR (400 MHz, DMSO-*d*₆) δ 11.76 (s, 1H), 10.98 (s, 1H), 10.62 (s, 1H), 9.51 (s, 1H), 8.79 (m, 1H), 8.67 (m, 3H), 8.21 (s, 1H), 7.96 (s, 1H), 7.77 (m, 1H), 7.71 - 7.64 (m, 2H), 7.52 (m, 3H), 7.16 (m, 3H), 6.66 (s, 1H), 5.08 (dd, *J* = 13.2, 5.1 Hz, 1H), 4.42 (d, *J* = 17.3 Hz, 1H), 4.31 (d, *J* = 13.8 Hz, 1H), 3.25 (m, 4H), 3.15 (m, 4H), 3.08 - 3.01 (m, 4H), 2.93 - 2.84 (m, 4H), 2.81 (d, *J* = 4.5 Hz, 3H), 2.61 (m, 2H), 2.44 - 2.34 (m, 2H), 2.03 - 1.97 (m, 2H), 1.87 (m, 2H), 1.59 - 1.40 (m, 6H). LCMS [M/2]⁺ =462.7 |
| **1043** | 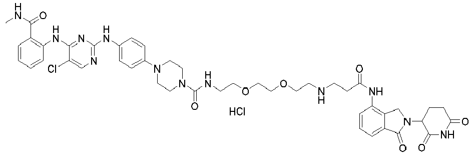 |
| | ¹H NMR (400 MHz, DMSO-*d*₆) δ 12.01 (s, 1H), 10.98 (s, 1H), 10.23 (s, 1H),9.94 (s, 1H), 9.18 - 8.84 (m, 3H), 8.56 (t, *J* = 3.6 Hz, 1H), 8.33 (s, 1H), 7.94 - 7.43 (m, 9H), 7.42 - 7.17 (m, 2H), 5.08 (dd, *J* = 13.2, 5.1 Hz, 1H), 4.45 - 4.34 (m, 2H), 3.79 - 3.03 (m, 16H), 3.01 - 2.70 (m,4H), 2.98 - 2.64 (m, 3H), 2.64 - 2.59 (m, 2H), 2.44 - 2.17 (m,1H), 2.24 - 1.97 (m, 1H). LCMS [M+H]⁺ = 925.9 |
| **1044** | 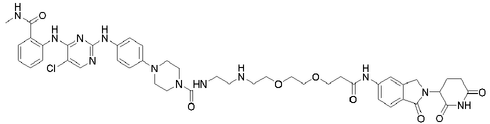 |
| | ¹H NMR (400 MHz, DMSO-*d*₆) δ 12.01 (s, 1H), 10.98 (s, 1H), 10.23 (s, 1H),9.94 (s, 1H), 9.18 - 8.84 (m, 3H), 8.56 (t, *J* = 3.6 Hz, 1H), 8.33 (s, 1H), 7.94 - 7.43 (m, 9H), 7.42 - 7.17 (m, 2H), 5.08 (dd, *J* = 13.2, 5.1 Hz, 1H), 4.45 - 4.34 (m, 2H), 3.79 - 3.03 (m, 16H), 3.01 - 2.70 (m,4H), 2.98 - 2.64 (m, 3H), 2.64 - 2.59 (m, 2H), 2.44 - 2.17 (m,1H), 2.24 - 1.97 (m, 1H). LCMS \|M+H]⁺ = 925.9 |
| **1046** | 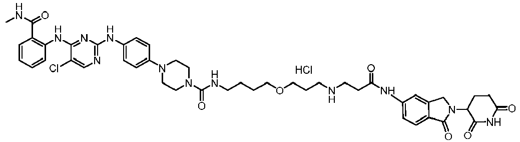 |
| | ¹H NMR (400 MHz, DMSO-*d*₆) δ 11.96 (s, 1H), 11.03 (s, 1H), 10.27 (s, 1H), 9.99 (s, 1H), 9.00 (m, 2H), 8.87 (m, 1H), 8.63 (m, 1H), 8.30 (s, 1H), 7.91 - 7.79 (m, 2H), 7.66 (d, *J* = 8.5 Hz, 2H), 7.52 (m, 5H), 7.20 (t, *J* = 7.5 Hz, 1H), 6.79 (m, 1H), 5.16 (dd, *J* = 13.3, 5.1 Hz, 1H), 4.47 (d, *J* = 17.6 Hz, 1H), 4.37 (d, *J* = 17.5 Hz, 1H), 3.46 (d, *J* = 6.0 Hz, 2H), 3.38 (m, 6H), 3.22 (t, *J* = 6.3 Hz, 3H), 3.07 (m, 2H), 3.03 - 2.88 (m, 6H), 2.81 (m, 3H), 2.66 - 2.59 (m, 1H), 2.34 (m, 1H), 2.08 - 1.82 (m, 4H), 1.55 - 1.44 (m, 4H). LCMS \|M/2]⁺ =462.7 |
| **1047** | 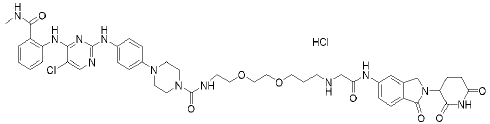 |
| | ¹H NMR (400 MHz, DMSO-*d*₆) δ 12.06 (s, 1H), 11.43 (s, 1H), 10.99 (s, 1H), 10.24 (s, 1H), 9.28 (s, 2H), 8.96 (d, *J* = 4.9 Hz, 1H), 8.59 (d, *J* = 8.4 Hz, 1H), 8.34 (s, 1H), 7.99 (s, 1H), 7.85 (d, *J* = 7.8 Hz, 1H), 7.77 - 7.60 (m, 6H), 7.55 (t, *J* = 7.9 Hz, 1H), 7.22 (t, *J* = 7.6 Hz, 1H), 5.09 (dd, *J* = 13.2, 5.0 Hz, 1H), 4.51 - 4.29 (m, 2H), 4.06 (d, *J* = 6.0 Hz, 2H), 3.63 - 3.40 (m, 12H), 3.24 (d, *J* = 6.3 Hz, 2H), 3.07 (p, *J* = 6.7 Hz, 2H), 3.00 - 2.78 (m, 4H), 2.60 (d, *J* = 16.9 Hz, 1H), 2.45 - 2.33 (m, 1H), 1.98 (dh, *J* = 27.2, 6.6 Hz, 3H), 1.36 - 1.23 (m, 1H). LC-MS: [M+H] ⁺ =925.7 |
| **1048** | 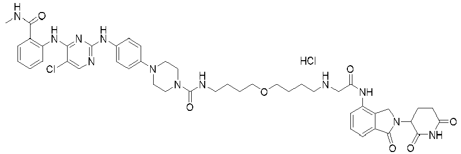 |
| | ¹H NMR (400 MHz, DMSO-*d*₆) δ 11.99 (s, 1H), 11.05 (s, 1H), 10.90 (s, 1H), 10.11 (d, *J* = 16.7 Hz, 1H), 9.38 - 9.20 (m, 2H), 8.93 (t, *J* = 5.4 Hz, 1H), 8.61 (d, *J* = 8.4 Hz, 1H), 8.32 (s, 1H), 7.96 - 7.75 (m, 2H), 7.72 - 7.50 (m, 7H), 7.30 - 7.15 (m, 1H), 5.16 (dd, *J* = 13.2, 5.1 Hz, 1H), 4.56 - 4.33 (m, 2H), 4.04 (s, 2H), 3.40 (d, *J* = 18.0 Hz, 8H), 3.05 - 2.85 (m, 7H), 2.81 (d, *J* = 4.3 Hz, 3H), 2.63 (d, *J* = 16.9 Hz, 1H), 2.28 (m, 1H), 2.03 (m, 1H), 1.78 - 1.71 (m, 2H), 1.59 - 1.46 (m, 6H), 1.27 (d, *J* = 6.8 Hz, 2H). LC-MS: [M+H] ⁺ =925.7 |
| **1049** | 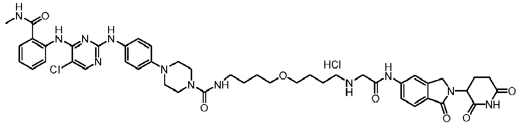 |
| | ¹H NMR (400 MHz, DMSO-*d*₆) δ 11.95 (s, 1H), 11.30 (s, 1H), 10.99 (s, 1H), 10.01 (s, 1H), 9.21 (s, 2H), 8.89 (d, *J* = 4.7 Hz, 1H), 8.63 (d, *J* = 8.5 Hz, 1H), 8.31 (s, 1H), 7.97 (s, 1H), 7.82 (d, *J* = 7.8 Hz, 1H), 7.69 (d, *J* = 26.9 Hz, 4H), 7.53 (t, *J* = 8.0 Hz, 3H), 7.20 (t, *J* = 7.5 Hz, 1H), 5.09 (dd, *J* = 13.3, 5.1 Hz, 1H), 4.54 - 4.25 (m, 2H), 4.03 (t, *J* = 5.6 Hz, 2H), 3.42 - 3.35 (m, 8H), 3.15 - 2.85 (m, 7H), 2.81 (d, *J* = 4.5 Hz, 3H), 2.64 - 2.58 (m, 1H), 2.46 - 2.31 (m, 1H), 2.03 - 1.96 (m, 2H), 1.78 - 1.70 (m, 2H), 1.64 - 1.49 (m, 6H), 1.24 (d, *J=* 3.6 Hz, 2H). LC-MS: [M+H] ⁺ =925.7 |
| **1050** | 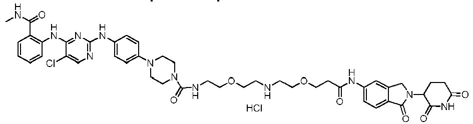 |
| | ¹H NMR (400 MHz, DMSO-*d*₆) δ 11.94 (s, 1H), 10.97 (s, 1H), 10.59 (s, 1H), 9.94 (s, 1H), 9.02 - 8.77 (m, 3H), 8.65 (d, *J* = 8.3 Hz, 1H), 8.29 (s, 1H), 8.01 (d, *J* = 1.7 Hz, 1H), 7.80 (dd, *J* = 8.0, 1.6 Hz, 1H), 7.67 (td, *J* = 9.2, 8.3, 4.4 Hz, 4H), 7.56 - 7.40 (m, 3H), 7.20 (td, *J* = 7.6, 1.2 Hz, 1H), 6.96 (s, 1H), 5.08 (dd, *J* = 13.3, 5.1 Hz, 1H), 4.54 - 4.23 (m, 4H), 3.76 (dt, *J* = 10.7, 5.7 Hz, 6H), 3.66 (t, *J* = 5.1 Hz, 2H), 3.46 (t, *J* = 5.7 Hz, 2H), 3.36 (s, 4H), 3.26 (d, *J* = 5.8 Hz, 2H), 3.14 (q, *J* = 5.7 Hz, 4H), 2.91 (ddd, *J* = 17.1, 13.5, 5.4 Hz, 1H), 2.81 (d, *J* = 4.4 Hz, 3H), 2.70 (t, *J=* 6.1 Hz, 2H), 2.64 - 2.56 (m, 1H), 2.42 - 2.30 (m, 1H), 2.05 - 1.91 (m, 1H). LCMS [M+H]⁺ = 926.0 |
| **1051** | 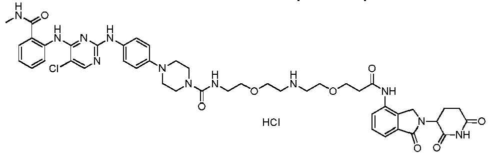 |
| | ¹H NMR (400 MHz, DMSO-*d*₆) δ 11.91 (s, 1H), 11.02 (s, 1H), 10.12 (s, 1H), 9.89 (s, 1H), 9.12 - 8.77 (m, 3H), 8.66 (s, 1H), 8.29 (s, 1H), 7.83 (ddd, *J* = 18.0, 7.6, 1.7 Hz, 2H), 7.76 - 7.58 (m, 3H), 7.50 (tt, *J* = 13.8, 7.0 Hz, 4H), 7.19 (t, *J* = 7.6 Hz, 1H), 6.98 (s, 1H), 5.15 (dd, *J* = 13.3, 5.1 Hz, 1H), 4.56 - 4.30 (m, 2H), 4.22 (t, *J* = 6.5 Hz, 1H), 3.76 (m, 8H), 3.52 - 3.43 (m, 2H), 3.34 (s, 3H), 3.25 (d, *J* = 5.8 Hz, 2H), 3.15 (dt, *J* = 11.4, 4.2 Hz, 4H), 2.98 - 2.87 (m, 1H), 2.81 (d, *J* = 4.5 Hz, 3H), 2.70 (q, *J* = 7.3, 6.7 Hz, 1H), 2.66 - 2.57 (m, 2H), 2.40 - 2.26 (m, 1H), 2.18 - 1.93 (m, 1H), 1.75 - 1.55 (m, 1H), 1.50 - 1.33 (m, 1H). LCMS [M+H]⁺ = 925.9 |
| **1053** | 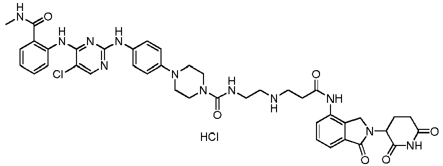 |
| | ¹H NMR (400 MHz, DMSO-*d*₆) δ 12.00 (s, 1H), 11.03 (s, 1H), 10.30 (s, 1H), 10.11 (s, 1H), 9.18 (s, 2H), 8.89 (q, *J* = 4.5 Hz, 1H), 8.61 (d, *J* = 8.4 Hz, 1H), 8.33 (s, 1H), 7.84 (ddd, *J* = 15.5, 7.6, 1.7 Hz, 2H), 7.69 - 7.37 (m, 7H), 7.33 - 6.96 (m, 2H), 5.15 (dd, *J* = 13.3, 5.1 Hz, 1H), 3.66 - 3.33 (m, 10H), 3.24 (q, *J* = 6.6 Hz, 2H), 3.07 (p, *J* = 6.0 Hz, 2H), 3.02 - 2.87 (m, 3H), 2.81 (d, *J* = 4.4 Hz, 3H), 2.70 - 2.57 (m, 1H), 2.42 - 2.25 (m, 1H), 2.13 - 1.91 (m, 1H). LCMS [M+H]⁺ = 837.7 |
| **1054** | 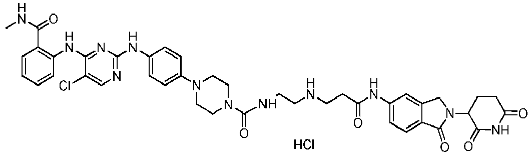 |
| | ¹H NMR (400 MHz, DMSO-*d*₆) δ 11.94 (s, 1H), 10.98 (s, 1H), 10.76 (d, *J* = 5.2 Hz, 1H), 9.99 (s, 1H), 9.10 (s, 2H), 8.87 (t, *J* = 4.6 Hz, 1H), 8.64 (d, *J* = 8.3 Hz, 1H), 8.30 (s, 1H), 7.97 (s, 1H), 7.81 (dd, *J* = 8.0, 1.7 Hz, 1H), 7.81 - 7.44 (m, 6H), 7.20 (t, *J* = 7.5 Hz, 2H), 5.08 (dd, *J* = 13.2, 5.1 Hz, 1H), 4.58 - 4.14 (m, 2H), 3.48 - 3.32 (m, 8H), 3.25 (p, *J* = 6.8 Hz, 2H), 3.10 (ddt, *J* = 16.9, 9.8, 4.6 Hz, 3H), 3.01 - 2.86 (m, 3H), 2.81 (d, *J* = 4.5 Hz, 3H), 2.67 - 2.55 (m, 2H), 2.37 (td, *J* = 13.3, 4.7 Hz, 1H), 2.06 - 1.89 (m, 1H). LCMS [M+H]⁺ = 837.8 |
| **1055** | 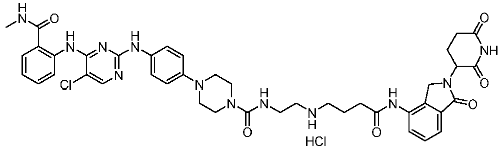 |
| | ¹H NMR (400 MHz, DMSO) δ 11.95 (s, 1H), 11.02 (s, 1H), 10.10 (s, 1H), 9.98 (s, 1H), 9.07 (s, 2H), 8.87 (d, *J* = 4.5 Hz, 1H), 8.64 (d, *J* = 7.4 Hz, 1H), 8.30 (s, 1H), 7.87 - 7.79 (m, 2H), 7.65 (d, *J* = 7.7 Hz, 2H), 7.58 - 7.45 (m, 5H), 7.20 (t, *J* = 7.3 Hz, 2H), 5.15 (dd, *J* = 13.3, 5.1 Hz, 1H), 4.49 - 4.34 (m, 2H), 3.75 (s, 6H), 3.40 (d, *J* = 6.7 Hz, 4H), 3.02 (d, *J* = 5.4 Hz, 4H), 2.94 - 2.88 (m, 1H), 2.81 (d, *J* = 4.5 Hz, 3H), 2.61 (d, *J* = 17.1 Hz, 1H), 2.55 (d, *J* = 7.1 Hz, 2H), 2.35 (dd, *J* = 13.1, 4.4 Hz, 1H), 2.01 (dd, *J* = 14.1, 6.6 Hz, 3H).LCMS [M+H]⁺ =851.9 |
| **1056** | 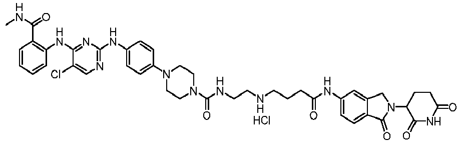 |
| | ¹H NMR (400 MHz, DMSO) δ 11.93 (s, 1H), 10.96 (d, *J* = 10.1 Hz, 1H), 10.57 (s, 1H), 9.95 (s, 1H), 9.03 (s, 2H), 8.87 (d, *J* = 4.3 Hz, 1H), 8.64 (d, *J* = 7.3 Hz, 1H), 8.30 (s, 1H), 8.00 (s, 1H), 7.81 (d, *J* = 7.9 Hz, 1H), 7.69 - 7.45 (m, 7H), 7.20 (t, *J* = 7.4 Hz, 2H), 5.08 (dd, *J* = 13.3, 5.1 Hz, 1H), 4.35 (dd, *J* = 55.5, 17.4 Hz, 2H), 3.75 (s, 6H), 3.40 (d, *J* = 4.8 Hz, 4H), 3.05 - 2.97 (m, 4H), 2.92 - 2.86 (m, 1H), 2.81 (d, *J* = 4.5 Hz, 3H), 2.64 - 2.52 (m, 3H), 2.37 (dd, *J* = 12.4, 4.7 Hz, 1H), 1.98 (dd, *J* = 20.8, 13.3 Hz, 3H).. LCMS [M+H]⁺ =851.9 |
| **1057** | 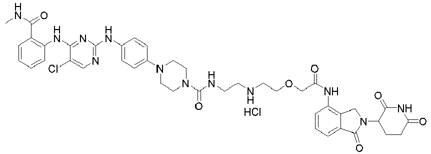 |
| | ¹H NMR (400 MHz, DMSO) δ 11.97 (s, 1H), 11.01 (d, *J* = 5.9 Hz, 1H), 10.13 (s, 1H), 10.00 (s, 1H), 9.27 (s, 2H), 8.87 (d, *J* = 4.4 Hz, 1H), 8.64 (s, 1H), 8.31 (s, 1H), 7.82 - 7.74 (m, 2H), 7.64 (s, 2H), 7.54 (dt, *J* = 15.3, 7.1 Hz, 5H), 7.20 (t, *J* = 7.6 Hz, 2H), 5.15 (dd, *J* = 13.3, 5.2 Hz, 1H), 4.51 (d, *J* = 17.6 Hz, 4H), 3.85 (d, *J* = 4.9 Hz, 2H), 3.71 (d, *J* = 23.2 Hz, 4H), 3.42 (d, *J* = 21.1 Hz, 6H), 3.24 (s, 2H), 3.10 (d, *J* = 5.0 Hz, 2H), 2.96 - 2.87 (m, 1H), 2.81 (d, *J* = 4.5 Hz, 3H), 2.60 (d, *J* = 16.2 Hz, 1H), 2.41 - 2.33 (m, 1H), 2.04 - 1.98 (m, 1H).LCMS [M+H]⁺ =867.8 |
| **1058** | 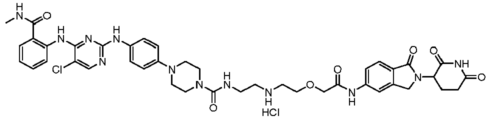 |
| | ¹H NMR (400 MHz, DMSO) δ 11.97 (s, 1H), 10.96 (d, *J* = 6.8 Hz, 1H), 10.36 (s, 1H), 10.01 (s, 1H), 9.27 (s, 2H), 8.87 (d, *J* = 4.4 Hz, 1H), 8.62 (s, 1H), 8.31 (s, 1H), 8.12 (s, 1H), 7.94 - 7.84 (m, 1H), 7.81 (d, *J* = 7.9 Hz, 1H), 7.71 - 7.50 (m, 6H), 7.24 - 7.17 (m, 2H), 5.08 (dd, *J* = 13.3, 5.0 Hz, 1H), 4.44 - 4.37 (m, 4H), 3.88 - 3.83 (m, 2H), 3.79 - 3.67 (m, 4H), 3.48 - 3.38 (m, 6H), 3.24 (s, 2H), 3.10 (s, 2H), 2.95 - 2.85 (m, 1H), 2.81 (d, *J* = 4.5 Hz, 3H), 2.58 (d, *J* = 17.9 Hz, 1H), 2.36 (dd, *J=* 13.3, 4.6 Hz, 1H), 2.04 - 1.92 (m, 1H).LCMS [M+H]⁺ =867.9 |
| **1059** | 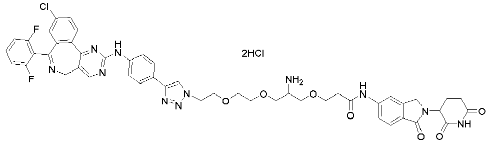 |
| | ¹H NMR (400 MHz, DMSO-*d*₆) δ 10.98 (s, 1H), 10.55 (s, 1H), 10.01 (s, 1H), 8.69 (s, 1H), 8.45 (s, 1H), 8.30 (d, *J* = 8.4 Hz, 1H), 8.10-8.01 (m, 4H), 7.93 - 7.78 (m, 4H), 7.72 - 7.51 (m, 3H), 7.33 (s, 1H), 7.26 - 7.11 (m, 2H), 5.09 (d, *J* = 5.1 Hz, 1H), 4.56 (t, *J* = 5.2 Hz, 2H), 4.45 - 4.22 (m, 2H), 3.93 - 3.67 (m, 3H), 3.39 (s, 6H), 2.91 (t, *J* = 16.4 Hz, 2H), 2.72 - 2.54 (m, 1H), 2.42 - 2.26 (m, 5H), 2.09 - 1.90 (m, 1H), 1.24 (s, 2H). LC-MS: [M+H] ⁺ =974.3 |
| **1060** | 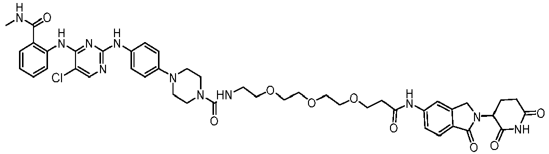 |
| | ¹H NMR (400 MHz, DMSO-*d*₆) δ 11.81 (s, 1H), 10.98 (s, 1H), 10.43 (s, 1H), 9.63 (s, 1H), 9.34 (s, 3H), 8.89- 8.79 (m, 1H), 8.66 - 8.58 (m, 1H),8.34 (s, 1H), 8.03 (s, 1H), 7.95 - 7.74 (m, 1H), 7.74 - 7.36 (m, 4H), 7.42 - 7.36 (m, 3H), 5.08 (dd, *J* = 13.2, 5.1 Hz, 1H), 4.33 - 4.24 (m,2H), 3.78 - 3.46 (m, 10H), 3.42 - 3.05 (m, 9H), 3.21 - 2.81 (m, 1H), 2.81 (s, 3H), 2.80 - 2.58 (m, 3H), 2.45 - 2.29 (m, 1H), 2.01 - 1.88 (m, 1H), 1.52 - 1.39 (m, 1H), 1.28 (dt, *J* = 11.0, 8.6 Hz, 1H) LCMS [M+H]⁺ = 927.0 |
| **1061** | 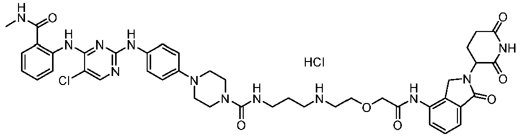 |
| | ¹H NMR (400 MHz, DMSO) δ 12.03 (s, 1H), 11.01 (s, 1H), 10.15 (d, *J* = 9.9 Hz, 2H), 9.24 (s, 2H), 8.90 (d, *J* = 4.5 Hz, 1H), 8.61 (d, *J* = 7.6 Hz, 1H), 8.33 (s, 1H), 7.82 (dd, *J* = 8.0, 1.2 Hz, 1H), 7.75 (d, *J* = 7.7 Hz, 1H), 7.66 (d, *J* = 8.4 Hz, 2H), 7.55 (dt, *J* = 15.2, 7.1 Hz, 5H), 7.19 (dd, *J* = 27.1, 19.8 Hz, 2H), 5.14 (dd, *J* = 13.3, 5.1 Hz, 1H), 4.45 (d, *J* = 40.5 Hz, 2H), 3.80 - 3.70 (m, 8H), 3.40 (d, *J* = 4.6 Hz, 4H), 3.23 - 3.15 (m, 4H), 2.98 (d, *J* = 6.3 Hz, 2H), 2.94 - 2.86 (m, 1H), 2.81 (d, *J* = 4.5 Hz, 3H), 2.61 (d, *J* = 17.0 Hz, 1H), 2.37 (dd, *J* = 13.1, 4.6 Hz, 1H), 2.05 - 1.98 (m, 1H), 1.87 (dd, *J* = 13.9, 6.9 Hz, 2H). LCMS [M+H]⁺ =881.9 |
| **1062** | 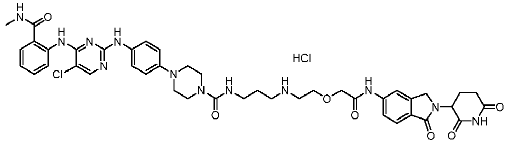 |
| | ¹H NMR (400 MHz, DMSO) δ 11.90 (s, 1H), 10.98 (s, 1H), 10.33 (s, 1H), 9.82 (s, 1H), 9.10 (s, 2H), 8.85 (d, *J* = 4.4 Hz, 1H), 8.65 (s, 1H), 8.27 (s, 1H), 8.10 (s, 1H), 7.91 - 7.82 (m, 1H), 7.80 (d, *J* = 6.7 Hz, 1H), 7.68 (d, *J* = 8.3 Hz, 1H), 7.60 (s, 2H), 7.51 (t, *J* = 7.6 Hz, 1H), 7.35 (s, 2H), 7.19 (t, *J* = 7.2 Hz, 1H), 7.03 (s, 1H), 5.08 (dd, *J* = 13.2, 5.0 Hz, 1H), 4.44 - 4.26 (m, 2H), 4.22 (d, *J* = 4.6 Hz, 2H), 3.82 (d, *J* = 4.9 Hz, 2H), 3.30 (s, 6H), 3.18 (s, 6H), 2.99 (s, 2H), 2.91 - 2.85 (m, 1H), 2.81 (d, *J* = 4.5 Hz, 3H), 2.59 (d, *J* = 18.2 Hz, 1H), 2.37 - 2.30 (m, 1H), 1.99 (d, *J* = 5.4 Hz, 1H), 1.90 - 1.81 (m, 2H). LCMS [M+H]⁺ = 881.9 |
| **1064** | 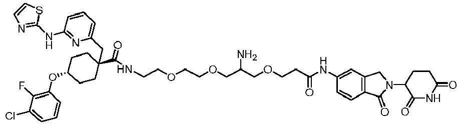 |
| | ¹H NMR (400 MHz, DMSO-*d*₆) δ 10.98 (s, 1H), 10.59 (s, 1H), 8.14 (s, 3H), 8.01 (s, 1H), 7.80 - 7.64 (m, 4H), 7.49 (t, *J* = 3.1 Hz, 1H), 7.20 - 7.04 (m, 5H), 6.81 (d, *J* = 7.4 Hz, 1H), 5.14 - 5.07 (m, 1H), 4.55 (s, 1H), 4.46 - 4.23 (m, 2H), 3.76 (q, *J* = 5.9 Hz, 2H), 3.66 - 3.46 (m, 8H), 3.38 (m, 3H), 3.19 (t, *J* = 5.9 Hz, 2H), 3.01 - 2.85 (m, 3H), 2.68 (t, *J* = 6.2 Hz, 2H), 2.63 - 2.56 (m, 1H), 2.43 - 2.30 (m, 1H), 2.03 - 1.63 (m, 9H). LC-MS: [M+H] ⁺ =936 |
| **1065** | 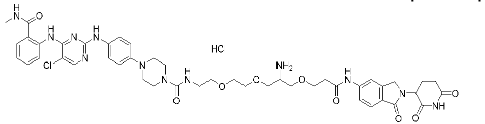 |
| | ¹H NMR (400 MHz, DMSO-*d*₆) δ 11.96 (s, 1H), 10.98 (s, 1H), 10.64 (s, 1H), 10.01 (s, 1H), 8.97 - 8.88 (m, 1H), 8.63 (d, *J* = 8.5 Hz, 1H), 8.31 (s, 1H), 8.18 (d, *J* = 5.3 Hz, 3H), 8.03 (s, 1H), 7.82 (d, *J* = 7.7 Hz, 1H), 7.74 - 7.63 (m, 3H), 7.57 - 7.43 (m, 3H), 7.20 (t, *J* = 7.5 Hz, 1H), 6.90 (s, 1H), 5.12 - 5.09 (m, 1H), 4.46 - 4.29 (m, 2H), 3.54 (s, 6H), 3.45 - 3.36 (m, 10H), 3.23 (d, *J* = 6.2 Hz, 2H), 2.98 - 2.77 (m, 4H), 2.71 (d, *J* = 6.2 Hz, 2H), 2.60 (d, *J* = 17.2 Hz, 1H), 2.43 - 2.31 (m, 1H), 2.05 - 1.95 (m, 2H). LC-MS: [M+H] ⁺ =956. |
| **1066** | 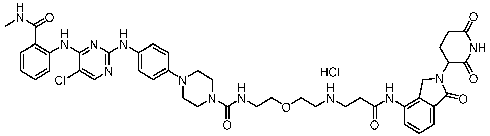 |
| | ¹H NMR (400 MHz, DMSO) δ 11.99 (s, 1H), 11.03 (s, 1H), 10.31 (s, 1H), 10.08 (s, 1H), 9.15 (s, 2H), 8.89 (d, *J* = 4.4 Hz, 1H), 8.61 (d, *J* = 7.4 Hz, 1H), 8.32 (s, 1H), 7.83 (t, *J* = 7.7 Hz, 2H), 7.66 (d, *J* = 8.5 Hz, 2H), 7.59 - 7.46 (m, 5H), 7.21 (t, *J* = 7.3 Hz, 1H), 7.05 (s, 1H), 5.14 (dd, *J* = 13.2, 5.1 Hz, 1H), 4.49 - 4.35 (m, 2H), 3.78 (s, 4H), 3.54 - 3.48 (m, 4H), 3.41 (s, 4H), 3.28 (d, *J* = 4.8 Hz, 4H), 3.16 (s, 2H), 2.97 (t, *J* = 7.0 Hz, 2H), 2.90 (dd, *J* = 17.5, 5.0 Hz, 1H), 2.81 (d, *J* = 4.5 Hz, 3H), 2.61 (d, *J* = 16.7 Hz, 1H), 2.33 (dd, *J* = 13.1, 4.3 Hz, 1H), 2.05 - 1.98 (m, 1H).LCMS [M+H]⁺ = 881.9 |
| **1067** | 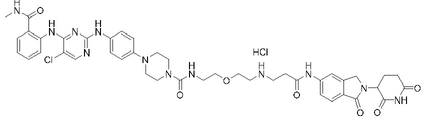 |
| | ¹H NMR (400 MHz, DMSO) δ 11.88 (s, 1H), 10.97 (s, 1H), 10.71 (s, 1H), 9.79 (s, 1H), 8.98 (s, 2H), 8.84 (d, *J* = 4.5 Hz, 1H), 8.66 (s, 1H), 8.27 (s, 1H), 7.96 (s, 1H), 7.78 (s, 1H), 7.69 - 7.58 (m, 4H), 7.50 (d, *J* = 7.3 Hz, 1H), 7.35 (s, 2H), 7.17 (d, *J* = 7.5 Hz, 1H), 6.94 (s, 1H), 5.07 (dd, *J* = 13.2, 5.0 Hz, 1H), 4.40 (d, *J* = 17.4 Hz, 1H), 4.27 (d, *J* = 17.5 Hz, 1H), 3.64 (s, 4H), 3.50 (d, *J* = 5.9 Hz, 2H), 3.28 (s, 8H), 3.17 (s, 4H), 2.94 (t, *J* = 7.2 Hz, 2H), 2.86 (d, *J* = 6.4 Hz, 1H), 2.81 (d, *J* = 4.5 Hz, 3H), 2.58 (d, *J* = 16.2 Hz, 1H), 2.37 - 2.31 (m, 1H), 2.00 - 1.94 (m, 1H).LCMS \|M+H]⁺ = 881.9 |
| **1068** | 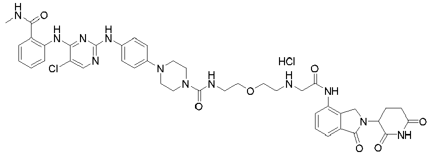 |
| | ¹H NMR (400 MHz, DMSO) δ 11.88 (s, 1H), 11.05 (s, 1H), 10.71 (s, 1H), 9.78 (s, 1H), 9.19 (s, 2H), 8.84 (d, *J* = 4.6 Hz, 1H), 8.67 (s, 1H), 8.27 (s, 1H), 7.88 (d, *J* = 7.5 Hz, 1H), 7.79 (d, *J* = 6.8 Hz, 1H), 7.64 - 7.47 (m, 5H), 7.31 (d, *J* = 21.7 Hz, 2H), 7.17 (d, *J* = 7.2 Hz, 1H), 6.91 (s, 1H), 5.17 (dd, *J* = 13.2, 5.1 Hz, 1H), 4.43 (dd, *J* = 40.4, 17.6 Hz, 2H), 4.08 (s, 2H), 3.74 - 3.71 (m, 2H), 3.64 (s, 6H), 3.25 (s, 8H), 2.95 (d, *J* = 13.2 Hz, 1H), 2.81 (d, *J* = 4.5 Hz, 3H), 2.60 (s, 1H), 2.33 - 2.26 (m, 1H), 2.02 (dd, *J* = 15.3, 7.7 Hz, 1H).LCMS \|M+H]⁺ = 867.9 |
| **1069** | 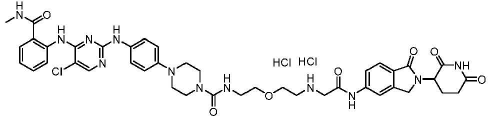 |
| | ¹H NMR (400 MHz, DMSO) δ 11.88 (s, 1H), 11.31 (s, 1H), 10.97 (s, 1H), 9.81 (s, 1H), 9.23 (s, 4H), 8.85 (d, *J* = 4.5 Hz, 1H), 8.65 (s, 1H), 8.27 (s, 1H), 7.97 (s, 1H), 7.80 (d, *J* = 6.8 Hz, 1H), 7.71 (t, *J* = 5.4 Hz, 2H), 7.59 (s, 2H), 7.49 (d, *J* = 7.1 Hz, 1H), 7.32 (s, 1H), 7.18 (t, *J* = 7.4 Hz, 1H), 6.99 (s, 1H), 5.08 (dd, *J* = 13.3, 5.1 Hz, 1H), 4.41 (d, *J* = 17.3 Hz, 1H), 4.29 (d, *J* = 17.7 Hz, 1H), 4.11 (s, 2H), 3.60 (dd, *J* = 6.6, 2.6 Hz, 4H), 3.50 (d, *J* = 4.2 Hz, 2H), 3.28 (s, 8H), 3.11 (s, 2H), 2.91 (d, *J* = 13.1 Hz, 1H), 2.81 (d, *J* = 4.4 Hz, 3H), 2.60 (s, 1H), 2.34 (d, *J* = 8.5 Hz, 1H), 1.97 (d, *J* = 5.0 Hz, 1H). LCMS \|M+H]⁺ = 868.0 |
| **1070** | 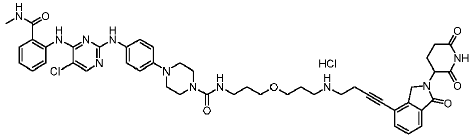 |
| | LCMS \|M+H]⁺ = 890.8 |
| **1071** | 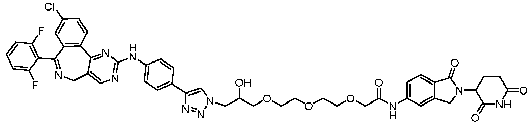 |
| | ¹H NMR (400 MHz, DMSO-d6) δ 11.00 (s, 1H), 10.00 (s, 1H), 9.70 (s, 1H), 8.69 (s, 1H), 8.37 (s, 1H), 8.31 (d, J = 8.6 Hz, 1H), 7.89 (ddd, J = 9.2, 7.8, 2.1Hz, 3H), 7.82 - 7.77 (m, 2H), 7.74 (dd, J = 7.7, 1.3 Hz, 1H), 7.33 (d, J = 2.1 Hz, 1H), 7.18 (q, J = 7.6 Hz, 2H), 6.85 (ddd, J = 46.2, 7.7, 0.9 Hz, 1H), 5.43 -5.32 (m, 2H), 5.13 (dd, J = 13.0, 5.1 Hz, 1H), 4.48 (dd, J = 13.9, 3.7 Hz, 1H), 4.37 (d, J = 8.8 Hz, 1H), 4.33 - 4.26 (m, 1H), 4.19 - 4.07 (m, 3H), 4.01 (s, 1H),3.71 (dd, J = 5.9, 3.4 Hz, 2H), 3.65 (dd, J = 6.0, 3.4 Hz, 2H), 3.62 - 3.57 (m, 4H), 3.40 (d, J = 5.5 Hz, 1H), 2.91 (ddd, J = 18.6, 12.9, 5.6 Hz, 2H), 2.57 (s,1H), 2.34 (d, J = 4.7 Hz, 1H), 2.00 (q, J = 7.0 Hz, 4H). LCMS [M+H]⁺ = 961.3 |
| **1072** | 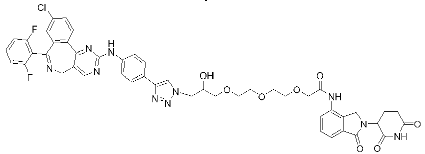 |
| | ¹H NMR (400 MHz, DMSO-d6) δ 10.95 (d, J = 21.0 Hz, 1H), 10.13 - 9.95 (m, 2H), 8.69 (d, J = 1.9 Hz, 2H), 8.43 - 8.35 (m, 2H), 8.31 (dd, J = 8.5,1.2 Hz, 2H), 7.89 (td, J = 8.0, 7.3, 2.1 Hz, 4H), 7.80 (dd, J = 8.8, 3.2 Hz, 3H), 7.73 - 7.48 (m, 3H), 7.33 - 7.26 (m, 3H), 7.17 (t, J = 8.4 Hz, 4H), 5.50- 5.35 (m, 2H), 5.08 (dd, J= 13.3, 5.1 Hz, 1H), 4.54-4.40 (m, 2H), 4.31 (dq, J = 11.5, 3.8 Hz, 2H), 4.15 (s, 1H), 4.13 - 4.07 (m, 1H), 4.05 (d, J =14.7 Hz, 2H), 3.83 (s, 2H), 3.72 - 3.64 (m, 2H), 3.61 (s, 2H), 3.59 (s, 4H), 3.58 (s, 2H), 2.97 - 2.80 (m, 2H), 2.58 (d, J = 16.8 Hz, 1H), 2.41 - 2.34 (m, 1H), 2.19 - 1.84 (m, 4H). LCMS [M+H]⁺ = 961.3 |
| **1073** | 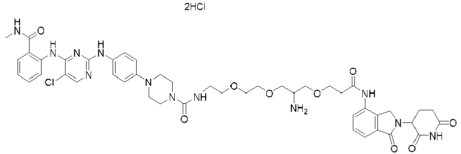 |
| | ¹H NMR (400 MHz, DMSO-*d*₆) δ 11.89 (s, 1H), 11.02 (s, 1H), 10.13 (d, *J* = 10.5 Hz, 1H), 9.84 (d, *J* = 21.1 Hz, 1H), 8.86 (s, 1H), 8.67 (s, 1H), 8.37 - 8.13 (m, 4H), 7.94 - 7.81 (m, 2H), 7.63 (s, 2H), 7.59 - 7.34 (m, 5H), 7.18 (t, *J* = 7.7 Hz, 1H), 6.86 (s, 1H), 5.15 (dd, *J* = 13.3, 5.0 Hz, 1H), 4.41 (q, *J* = 17.6 Hz, 2H), 3.56 - 3.17 (m, 19H), 3.00 - 2.78 (m, 5H), 2.75 - 2.60 (m, 4H), 2.30 (d, *J=* 20.0 Hz, 2H), 2.03 (s, 2H), 1.33 - 1.26 (m, 2H). LC-MS: [M+H] ⁺ =956. |
| **1074** | 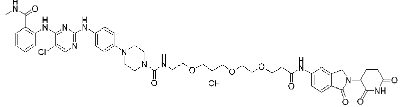 |
| | ¹H NMR (400 MHz, DMSO-*d*₆) 11.63 (s, 1H), 10.97 (s, 1H), 10.31 (s, 1H), 9.27 (s, 1H), 8.77 (s, 2H), 8.17 (s, 1H), 7.99 (s, 1H), 7.75 (d, *J* = 7.9 Hz, 1H), 7.63 (dd, *J* = 8.4, 8.3 Hz, 2H), 7.48 (dd, *J* = 13.8, 7.2 Hz, 3H), 7.09 (dd, *J* = 44.3, 37.1 Hz, 3H), 6.61 (s, 1H), 5.08 (dd, *J* = 13.2, 5.1 Hz, 1H), 4.42 (d, *J* = 17.4 Hz, 1H), 4.28 (d, *J* = 17.3 Hz, 1H), 3.70 (dt, *J* = 11.7, 5.8 Hz, 3H), 3.64 - 3.56 (m, 1H), 3.56 - 3.25 (m, 19H), 3.23 - 2.88 (m, 6H), 2.88 - 2.67 (m, 4H), 2.62 - 2.24 (m, 1H), 1.96 (dd, *J* = 2.2, 5.1 Hz, 1H) . LCMS \|M+H]⁺ = 956.9 |
| **1075** | 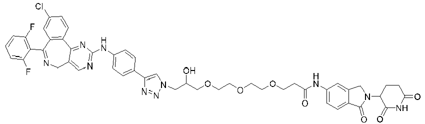 |
| | ¹H NMR (400 MHz, DMSO-d6) δ 10.95 (s, 1H), 10.50 (d, *J* = 3.7 Hz, 2H), 10.02 (s, 1H), 8.65 (s, 1H), 8.24 (d, *J* = 8.4 Hz, 1H), 7.99 (s, 1H), 7.87 - 7.77(m, 4H), 7.70 - 7.59 (m, 3H), 7.57 - 7.34 (m, 4H), 7.33 - 7.24 (m, 2H), 7.14 (s, 2H), 5.32 (dd, *J* = 45.6, 40.2 Hz, 2H), 5.07 (dd, *J* = 13.2, 5.1 Hz, 1H), 4.18(s, 1H), 3.99 (d, *J* = 27.8 Hz, 3H), 3.69 - 3.63 (m, 2H), 3.45 (d, *J* = 4.9 Hz, 5H), 2.94 - 2.85 (m, 1H), 2.60 (d, *J* = 5.6 Hz, 3H), 2.40 - 2.31 (m, 2H), 2.04 -1.93 (m, 2H). LCMS [M+H]⁺ = 975.3 |
| **1076** | 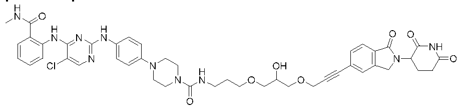 |
| | ¹H NMR (400 MHz, DMSO-*d*₆) δ 11.98 (s, 1H), 11.00 (s, 1H), 10.04 (s, 1H), 8.87 (q, *J* = 4.6 Hz, 1H), 8.62 (d, *J* = 8.5 Hz, 1H), 8.31 (s, 1H), 7.91 - 7.39 (m, 9H), 7.21 (t, *J* = 7.6 Hz, 1H), 6.75 (s, 1H), 5.11 (dd, *J* = 13.2, 5.1 Hz, 1H), 4.56 - 4.24 (m, 5H), 3.55 (dd, *J* = 9.8, 4.6 Hz, 3H), 3.41 (qdt, *J* = 15.7, 9.9, 5.9 Hz, 12H), 3.13 (t, *J* = 6.9 Hz, 2H), 2.90 (td, *J* = 13.3, 6.8 Hz, 2H), 2.81 (d, *J* = 4.4 Hz, 3H), 2.60 (d, *J* = 18.0 Hz, 1H), 2.39 (qd, *J* = 13.3, 4.4 Hz, 1H), 2.01 (dt, *J* = 11.0, 5.2 Hz, 1H), 1.68 (q, *J* = 6.6 Hz, 2H). LC-MS: [M+H]⁺ = 894. |
| **1077** | 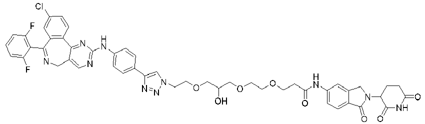 |
| | ¹H NMR (400 MHz, DMSO-*d*₆)δ 10.97 (s, 1H), 10.27 (s,1H), 10.00 (s, 1H), 8.69 (d, *J* = 3.5 Hz, 1H), 8.47 (dd, *J* = 13.6, 7.5 Hz, 2H), 8.31 (dd, *J* = 8.5, 2.8 Hz, 1H), 8.01 - 7.77 (m, 2H), 7.77 - 7.48 (m, 1H), 7.36 - 7.24 (m, 5H), 7.24-7.11 (m, 4H), 5.07 (dd, *J* = 13.2, 5.0 Hz, 1H), 4.54 (d, *J* = 4.4 Hz, 2H), 4.48 - 4.31 (m, 2H), 3.83 (dd, *J* = 12.4, 7.5 Hz, 2H), 3.78 - 3.61 (m, 5H), 3.61 - 3.10 (m,3H), 3.05 - 2.79 (m, 3H), 2.66 - 2.55 (m, 3H), 2.41 - 2.05 (m, 1H), 2.12 - 1.95 (m, 1H), 1.32 - 1.18 (m, 1H) . LCMS [M+H]⁺ = 975.9 |
| **1078** | 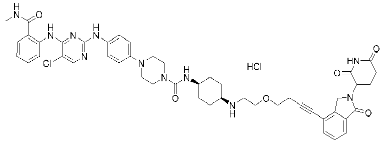 |
| | ¹H NMR (400 MHz, DMSO-*d*₆) δ 11.72 (s, 1H), 11.02 (s, 1H), 9.41 (s, 1H), 8.77 (m, 2H), 8.55 (m, 2H), 8.20 (s, 1H), 7.79 - 7.71 (m, 2H), 7.64 (m, 1H), 7.59 - 7.45 (m, 4H), 7.16 (m, 1H), 7.05 (m, 1H), 6.04 (m, 1H), 5.17 (dd, *J* = 13.3, 5.1 Hz, 1H), 4.46 (d, *J* = 17.7 Hz, 1H), 4.31 (d, *J* = 17.7 Hz, 1H), 3.75 (t, *J* = 5.1 Hz, 2H), 3.71 (t, *J* = 6.7 Hz, 2H), 3.66 (m, 1H), 3.13 (m, 6H), 2.82 - 2.80 (m, 3H), 2.78 (m, 1H), 2.44 (m, 1H), 2.00 (m, 4H), 1.89 - 1.67 (m, 7H), 1.46 (m, 4H). LCMS [M+H]⁺ = 917.0 |
| **1079** | 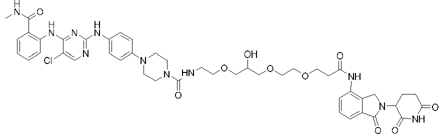 |
| | ¹H NMR (400 MHz, DMSO-*d*₆)δ 11.64 (s, 1H), 11.02 (s, 1H), 9.91 (s, 1H), 9.21 (d, *J* = 9.6 Hz, 2H), 8.78 (d, *J* = 4.6 Hz, 2H), 8.17 (s, 1H), 7.79 (dd, *J* = 6.0, 7.3 Hz, 2H), 7.70 - 7.05 (m, 6H), 7.09 (dd, *J* = 4.4, 3.9 Hz, 2H), 6.63 (s, 1H), 5.14 (dd, *J* = 13.3, 5.0 Hz, 1H), 4.81 - 4.25 (m, 2H), 4.02 - 3.51 (m, 15H), 3.41 - 3.02 (m, 8H), 3.01 - 2.59 (m, 7H), 2.41 - 2.31 (m,1H), 2.12 - 1.95 (m, 1H). LCMS [M+H]⁺ = 956.8 |
| **1080** | 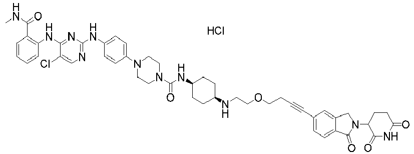 |
| | ¹H NMR (400 MHz, DMSO-*d*₆) δ 11.84 (s, 1H), 11.00 (s, 1H), 9.76 (s, 1H), 8.76 (m, 4H), 8.25 (m, 1H), 7.79 (m, 1H), 7.70 (m, 1H), 7.63 (m, 3H), 7.52 (m, 2H), 7.42 - 7.13 (m, 3H), 6.14 (m, 1H), 5.11 (dd, *J* = 13.3, 5.1 Hz, 1H), 4.45 (d*, J* = 17.6 Hz, 1H), 4.32 (d, *J* = 17.5 Hz, 1H), 3.77 (m, 2H), 3.69 (m, 4H), 3.30 (m, 4H), 3.15 (m, 4H), 2.95 - 2.77 (m, 6H), 2.63 - 2.56 (m, 1H), 2.39 (m, 1H), 2.06 - 1.93 (m, 2H), 1.91 - 1.73 (m, 6H), 1.43 (m, 3H). LCMS [M+H]⁺ = 917.0 |
| **1081** | 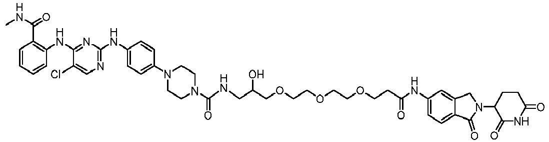 |
| | ¹H NMR (400 MHz, DMSO-d6) δ 11.62 (s, 1H), 10.97 (s, 1H), 10.31 (s, 1H), 9.26 (s, 1H), 8.83 - 8.70 (m, 2H), 8.17 (s, 1H), 7.99 (s, 1H), 7.75 (dd, J =7.9, 1.6 Hz, 1H), 7.70 - 7.57 (m, 2H), 7.54 - 7.44 (m, 3H), 7.19 - 7.09 (m, 1H), 6.94 (s, 2H), 6.60 (s, 1H), 5.08 (dd, J = 13.3, 5.1 Hz, 1H), 4.48 - 4.25(m, 2H), 3.75 - 3.70 (m, 2H), 3.70 - 3.57 (m, 2H), 3.51 (q, J = 1.8 Hz, 4H), 3.30 - 3.26 (m, 2H), 3.18 (dt, J = 12.9, 6.2 Hz, 3H), 3.03 (s, 4H), 2.90 (ddd,J = 17.9, 13.6, 5.4 Hz, 1H), 2.81 (d, J = 4.5 Hz, 3H), 2.60 (q, J = 10.5, 8.4 Hz, 3H), 2.37 (dd, J = 13.2, 4.5 Hz, 1H), 2.04 - 1.92 (m, 1H). LCMS [M+H]⁺ = 956.9 |
| **1082** | 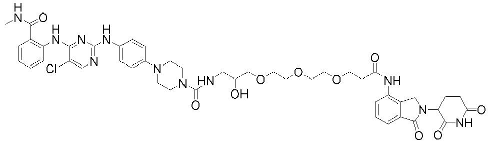 |
| | LCMS [M+H]⁺ = 956.6 |
| **1083** | 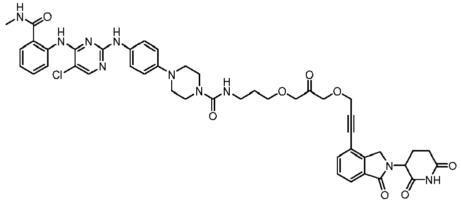 |
| | ¹H NMR (400 MHz, DMSO-*d*₆) δ 11.90 (s, 1H), 11.01 (s, 1H), 9.82 (s, 1H), 8.83 (q, *J* = 4.6 Hz, 1H), 8.66 (d, *J* = 8.2 Hz, 1H), 8.27 (s, 1H), 7.83 - 7.68 (m, 3H), 7.56 (ddd, *J* = 27.1, 19.2, 8.2 Hz, 4H), 7.37 (s, 2H), 7.19 (t, *J* = 7.6 Hz, 1H), 6.67 (s, 1H), 5.15 (dd, *J* = 13.3, 5.1 Hz, 1H), 4.55 - 4.27 (m, 5H), 3.78 (p, *J* = 5.6 Hz, 3H), 3.42 (t, *J* = 6.3 Hz, 5H), 3.39 - 3.34 (m, 3H), 3.32 (t, *J* = 6.8 Hz, 5H), 3.11 (t, *J* = 7.0 Hz, 3H), 2.81 (d, *J* = 4.4 Hz, 3H), 2.07 - 1.87 (m, 2H), 1.65 (p, *J* = 6.6 Hz, 2H), 1.24 (d, *J* = 3.4 Hz, 2H). LC-MS: [M+H]⁺ = 894 |
| **1084** | 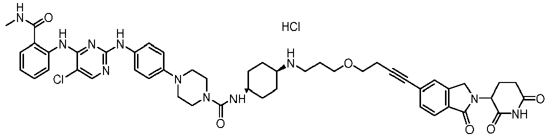 |
| | ¹H NMR (400 MHz, DMSO-*d*₆) δ 11.84 (s, 1H), 11.00 (s, 1H), 9.75 (s, 1H), 8.88 - 8.77 (m, 2H), 8.67 (s, 1H), 8.26 (s, 1H), 7.79 (dd, *J* = 8.0, 1.6 Hz, 1H), 7.70 (d, *J* = 7.8 Hz, 1H), 7.63 (d*, J* = 11.0 Hz, 2H), 7.51 (dt, *J* = 8.2, 4.0 Hz, 2H), 7.34 (s, 2H), 7.18 (t, *J* = 7.6 Hz, 1H), 6.16 (s, 1H), 5.11 (dd, *J* = 13.3, 5.1 Hz, 1H), 4.54 - 4.27 (m, 2H), 3.73 - 3.65 (m, 3H), 3.61 (t, *J* = 6.5 Hz, 3H), 3.56 (t, *J* = 5.9 Hz, 3H), 3.29 (s, 4H), 2.96 (d, *J* = 15.7 Hz, 3H), 2.89 (dd, *J* = 13.1, 5.0 Hz, 1H), 2.81 (d, *J* = 4.5 Hz, 2H), 2.73 (t, *J* = 6.5 Hz, 2H), 2.66 - 2.55 (m, 1H), 2.39 (qd, *J* = 13.3, 4.4 Hz, 1H), 2.08 - 1.71 (m, 9H), 1.57 - 1.40 (m, 2H). LCMS [M+H]⁺ = 930.8 |
| **1085** | 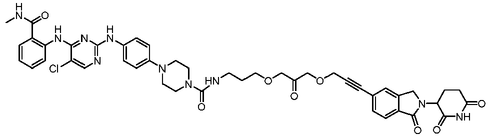 |
| | LC-MS: M+H) ⁺= 892 |
| **1086** | 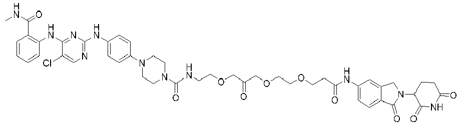 |
| | ¹H NMR (400 MHz, DMSO-*d*₆)δ 11.65 (s, 1H), 11.03 (s, 1H), 9.90 (s, 1H), 9.21 (d, *J* = 9.6 Hz, 2H), 8.75 (d, *J* = 4.6 Hz, 2H), 8.15 (s, 1H), 7.79 (dd, *J* = 6.0, 7.3 Hz, 2H), 7.70 - 7.05 (m, 6H), 7.10 (dd, *J* = 4.1, 5.2 Hz, 2H), 6.63 (s, 1H), 5.12 (dd, *J* = 9.3, 7.2 Hz, 1H), 4.81 - 4.25 (m, 2H), 4.02 - 3.51 (m, 15H), 3.41 - 3.02 (m, 8H), 3.01 - 2.59 (m, 7H), 2.41 - 2.31 (m,1H), 2.12 - 1.95 (m, 1H). LCMS [M+H]⁺ = 954.9 |
| **1087** | 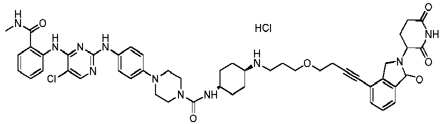 |
| | ¹H NMR (400 MHz, DMSO-*d*₆) δ 11.89 (s, 1H), 11.01 (s, 1H), 9.90 (s, 1H), 8.97 (s, 2H), 8.87 (q, *J* = 4.6 Hz, 1H), 8.66 (d, *J* = 8.4 Hz, 1H), 8.29 (s, 1H), 7.81 (dd, *J* = 7.9, 1.6 Hz, 1H), 7.72 (d, *J* = 7.5 Hz, 1H), 7.68 - 7.63 (m, 2H), 7.53 (t, *J* = 7.6 Hz, 3H), 7.19 (t, *J* = 7.5 Hz, 1H), 6.33 - 6.07 (m, 1H), 5.16 (dd, *J* = 13.3, 5.1 Hz, 1H), 4.56 - 4.05 (m, 2H), 3.71 - 3.67 (m, 3H), 3.63 (d, *J* = 6.8 Hz, 3H), 3.42 - 3.30 (m, 4H), 3.10 - 2.87 (m, 5H), 2.81 (d, *J* = 4.5 Hz, 2H), 2.75 (t, *J* = 6.6 Hz, 2H), 2.67 - 2.56 (m, 1H), 2.45 (d, *J* = 4.6 Hz, 1H), 2.08 - 1.90 (m, 4H), 1.81 (dd, *J* = 22.5, 12.8 Hz, 5H), 1.47 (d, *J* = 10.2 Hz, 2H), 1.20 (t, *J* = 7.3 Hz, 3H). LCMS \|M+H]⁺ = 931.0 |
| **1088** | 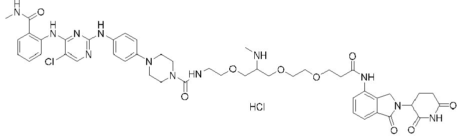 |
| | ¹H NMR (400 MHz, DMSO-*d*₆)δ 11.86 (s, 1H), 11.03 (s, 1H), 9.98 (s, 1H), 9.73 (s, 1H), 8.83 (d, J=4.3 Hz, 3H), 8.68 (s, 1H), 8.26 (s, 1H), 7.82 - 7.55 (m, 7H), 7.42 - 7.09 (m, 3H), 6.88 (s, 1H), 5.15 (dd, *J* = 13.2, 5.1 Hz, 1H), 4.38 (dd, *J* = 3.5, 7.5 Hz, 2H), 3.78 - 3.50 (m, 14H), 3.50-3.02 (m, 8H), 2.98-2.66 (m, 7H), 2.66-2.23 (m, 8H), 2.05(s, 1H) . LCMS [M+H]⁺ = 939.9 |
| **1090** | 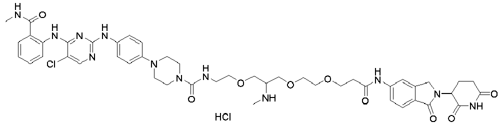 |
| | ¹H NMR (400 MHz, DMSO-*d*₆)δ12.03 (s, 1H), 11.01 (s, 1H), 10.52 (d, *J* = 3.5 Hz, 1H), 9.87 (s, 3H),9.11-8.94 (m, 3H),8.77-8.04 (m, 3H),7.87 -7.44 (m, 7H),7.22(t, *J* = 7.5 Hz, 1H), 5.15 (dd, *J* = 13.2, 5.1 Hz, 1H), 4.38 (dd, *J* = 3.5, 7.5 Hz, 2H), 3.86 (s, 3H), 3.77-3.53 (m, 4H),3.49 (s, 6H), 3.46 (d, *J* = 4.5 Hz, 1H),3.39 (dd, *J* = 6.1, 3.6 Hz, 2H), 3.18-2.99 (m, 7H), 2.96-2.59 (m, 10H), 2.53-2.36(m,1H),2.02-1.96(m, 1H). LCMS [M+H]⁺ = 970.1 |
| **1091** | 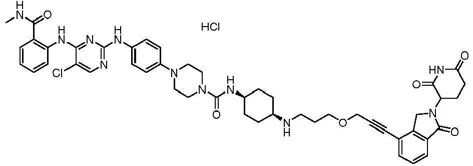 |
| | ¹H NMR (400 MHz, DMSO-*d*₆) δ 11.86 (brs, 1H), 11.01 (s, 1H), 9.78 (brs, 1H), 8.84 (m, 3H), 8.68 (m, 1H), 8.27 (s, 1H), 7.79 (m, 2H), 7.74 (m, 1H), 7.56 (m, 4H), 7.36 (m, 2H), 7.18 (t, *J* = 7.5 Hz, 1H), 6.17 (brs, 1H), 5.16 (dd, *J* = 13.3, 5.1 Hz, 1H), 4.54 - 4.45 (m, 3H), 4.36 (d, *J* = 17.8 Hz, 1H), 3.71 - 3.68 (m, 2H), 3.64 (m, 2H), 3.38 (m, 2H), 3.32 - 3.30 (m, 2H), 3.07 (m, 2H), 3.01 - 2.95 (m, 2H), 2.92 (m, 1H), 2.81 (d, *J* = 4.5 Hz, 3H), 2.66 - 2.57 (m, 2H), 2.45 (m, 1H), 2.08 - 1.93 (m, 4H), 1.91 - 1.74 (m, 6H), 1.49 (m, 2H). LCMS [M/2]⁺ = 459.0 |
| **1092** | 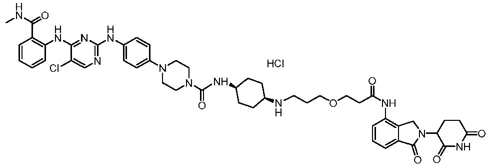 |
| | ¹H NMR (400 MHz, DMSO-d6) δ 11.62 (s, 1H), 11.02 (s, 1H), 10.07 (s, 1H), 9.25 (s, 1H), 8.95 - 8.69 (m, 4H), 8.16 (s, 1H), 7.81 (ddd, J = 34.2, 7.6, 1.7 Hz,2H), 7.55 - 7.40 (m, 5H), 7.17 - 7.09 (m, 1H), 6.92 (d, J = 9.0 Hz, 2H), 6.04 (d, J = 4.9 Hz, 1H), 5.15 (dd, J = 13.3, 5.1 Hz, 1H), 4.40 (q, J = 17.5 Hz, 2H),3.70 (dt, J = 11.0, 4.9 Hz, 4H), 3.60 (dd, J = 6.6, 3.9 Hz, 2H), 3.12 - 3.09 (m, 1H), 3.03 (d, J = 5.4 Hz, 4H), 2.94 (d, J = 13.4 Hz, 4H), 2.81 (d, J = 4.5 Hz,3H), 2.70 - 2.58 (m, 4H), 2.39 - 2.29 (m, 2H), 2.03 (dd, J = 10.7, 5.0 Hz, 2H), 1.95 - 1.87 (m, 2H), 1.76 (d, J = 22.5 Hz, 6H), 1.44 (s, 2H). LCMS [M+H]⁺ = 950.0 |
| **1093** | 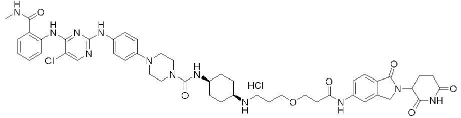 |
| | ¹H NMR (400 MHz, DMSO-d6) δ 11.81 (s, 1H), 10.98 (s, 1H), 10.57 (d, J = 2.4 Hz, 1H), 9.70 (s, 1H), 8.96 - 8.62 (m, 4H), 8.25 (s, 1H), 8.02 (d, J= 1.7Hz, 1H), 7.80 (dd, J = 7.9, 1.6 Hz, 1H), 7.74 -7.58 (m, 4H), 7.51 (t, J = 7.6 Hz, 1H), 7.30 (d, J = 14.5 Hz, 1H), 7.17 (td, J = 7.6, 1.2 Hz, 1H), 6.14 (s, 1H),5.08 (dd, J = 13.3, 5.1 Hz, 1H), 4.48 - 4.27 (m, 2H), 3.73 (s, 2H), 3.50 (s, 2H), 3.26 (s, 4H), 3.15 - 3.09 (m, 1H), 3.02 - 2.84 (m, 4H), 2.81 (d, J = 4.5 Hz,3H), 2.69 - 2.57 (m, 3H), 2.39 (td, J = 13.1, 4.4 Hz, 1H), 1.99 (ddd, J = 9.6, 5.3, 2.6 Hz, 1H), 1.94 - 1.68 (m, 8H), 1.51 - 1.39 (m, 2H).LCMS [M/2+H]⁺ = 475.7 |
| **1094** | 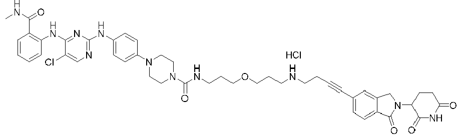 |
| | ¹H NMR (400 MHz, DMSO) δ 11.96 (s, 1H), 10.99 (s, 1H), 10.01 (s, 1H), 9.47 (s, 1H), 9.09 (d, *J* = 60.4 Hz, 1H), 8.88 (d, *J* = 4.4 Hz, 1H), 8.62 (s, 1H), 8.30 (s, 1H), 7.81 (d, *J* = 7.9 Hz, 1H), 7.73 -7.42 (m, 8H), 7.19 (d, *J* = 7.5 Hz, 1H), 6.76 (d, *J* = 37.6 Hz, 1H), 5.10 (dd, *J* = 13.3, 4.7 Hz, 1H), 4.49 - 4.38 (m, 2H), 3.99 - 3.67 (m, 8H), 3.49 - 3.27 (m, 6H), 3.22 (d, *J* = 5.6 Hz, 2H), 3.03 (dt, *J* = 30.2, 17.0 Hz, 4H), 2.91 - 2.84 (m, 1H), 2.81 (d, *J* = 4.5 Hz, 3H), 2.59 (d, *J* = 17.2 Hz, 1H), 2.40 - 2.32 (m, 1H), 2.00 (d, *J* = 5.1 Hz, 1H), 1.14 - 1.08 (m, 4H). LCMS [M+H]⁺ = 890.9 |
| **1095** | 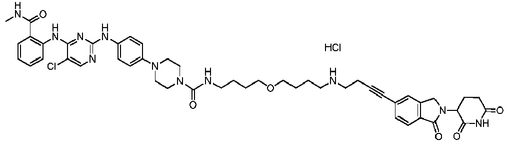 |
| | ¹H NMR (400 MHz, DMSO-*d*₆) δ 11.87 (s, 1H), 11.00 (s, 1H), 9.85 (s, 1H), 9.26 (s, 2H), 8.90 (dd, *J* = 18.2, 5.4 Hz, 2H), 8.66 (d, *J* = 8.5 Hz, 1H), 8.57 (s, 1H), 8.28 (s, 1H), 8.05 (t, *J* = 6.9 Hz, 1H), 7.81 (d, *J* = 7.9 Hz, 1H), 7.75 - 7.67 (m, 2H), 7.64 (d, *J* = 8.4 Hz, 1H), 7.58 (d, *J* = 7.9 Hz, 1H), 7.52 (t, *J* = 7.9 Hz, 1H), 7.43 (s, 1H), 7.19 (t, *J* = 7.5 Hz, 1H), 5.11 (dd, *J* = 13.2, 5.0 Hz, 1H), 4.58 - 4.11 (m, 2H), 3.41 - 3.29 (m, 9H), 3.15 (q, *J* = 6.6 Hz, 3H), 3.07 (d, *J* = 8.7 Hz, 2H), 2.96 (t, *J* = 7.3 Hz, 4H), 2.89 (dd, *J* = 12.9, 4.8 Hz, 1H), 2.81 (d, *J* = 4.5 Hz, 3H), 2.60 (d, *J* = 16.9 Hz, 2H), 2.45 - 2.29 (m, 1H), 2.08 - 1.88 (m, 2H), 1.71 (q, *J* = 7.8 Hz, 2H), 1.57 (q, *J* = 6.9 Hz, 2H), 1.52 - 1.38 (m, 4H). LCMS [M+H]⁺ = 919.0 |
| **1096** | 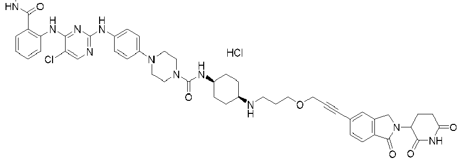 |
| | ¹H NMR (400 MHz, DMSO-*d*₆) δ 11.89 (s, 1H), 11.00 (s, 1H), 9.84 (s, 1H), 8.89 - 8.74 (m, 3H), 8.67 (m, 1H), 8.28 (s, 1H), 7.80 (dd, *J* = 8.0, 1.6 Hz, 1H), 7.74 (d, *J* = 7.7 Hz, 2H), 7.69 - 7.56 (m, 3H), 7.56 - 7.30 (m, 3H), 7.22 - 7.17 (m, 1H), 6.19 (s, 1H), 5.12 (dd, *J* = 13.3, 5.1 Hz, 1H), 4.51 - 4.43 (m, 3H), 4.36 (m, 1H), 3.75 - 3.60 (m, 6H), 3.36 (m, 4H), 3.07 (m, 1H), 2.97 (m, 2H), 2.94 - 2.86 (m, 1H), 2.81 (d, *J* = 4.5 Hz, 3H), 2.60 (m, 1H), 2.39 (m, 1H), 2.07 - 1.69 (m, 10H), 1.51 (m, 2H). LCMS [M/2]⁺ = 459.0 |
| **1097** | 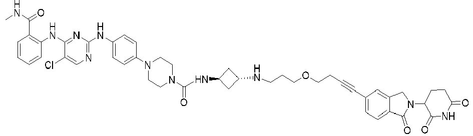 |
| | ¹H NMR (400 MHz, DMSO-d6) δ 11.64 (s, 1H), 11.00 (s, 1H), 9.25 (s, 1H), 8.88 (d, J = 4.7 Hz, 1H), 8.77 (s, 1H), 8.16 (s, 1H), 7.88 - 7.78 (m, 2H),7.69 (d, J = 7.9 Hz, 1H), 7.65 (s, 1H), 7.49 (q, J = 6.9 Hz, 5H), 7.12 (t, J = 7.6 Hz, 2H), 6.92 (d, J = 9.0 Hz, 2H), 5.29 (d, J = 6.0 Hz, 1H), 5.11 (dd, J= 13.2, 5.2 Hz, 2H), 4.93 (d, J = 5.2 Hz, 1H), 4.70 (t, J = 5.7 Hz, 1H), 4.39 (d, J = 34.7 Hz, 2H), 4.03 (dd, J = 11.1, 4.2 Hz, 2H), 3.98 (d, J = 5.4 Hz,2H), 3.92 - 3.88 (m, 2H), 3.01 (s, 4H), 2.80 (s, 3H), 2.28 (s, 2H). LCMS [M+H]⁺ = 902.9 |
| **1098** | 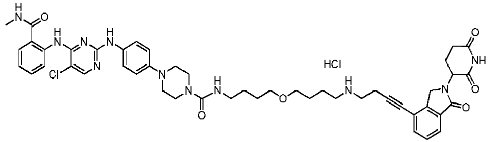 |
| | LCMS [M+H]⁺ = 918.8 |
| **1100** | 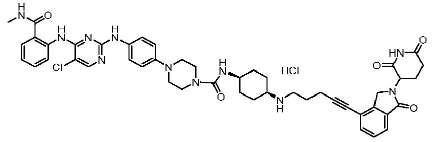 |
| | ¹H NMR (400 MHz, DMSO-d6) δ 11.83 (s, 1H), 11.00 (s, 1H), 9.76 (s, 1H), 9.02 (s, 2H), 8.85 (d, J = 4.8 Hz, 1H), 8.68 (s, 1H), 8.26 (s, 1H), 7.80 (dd, J =7.8, 1.6 Hz, 1H), 7.71 (d, J = 7.8 Hz, 1H), 7.64 (d, J = 15.9 Hz, 3H), 7.52 (dd, J = 13.0, 7.8 Hz, 2H), 7.39 (s, 1H), 7.18 (t, J = 7.5 Hz, 1H), 6.18 (s, 1H), 5.11 (dd, J = 13.3, 5.1 Hz, 1H), 4.51 - 4.25 (m, 2H), 3.30 (s, 4H), 3.08 (d, J = 26.9 Hz, 3H), 2.96 - 2.85 (m, 1H), 2.81 (d, J = 4.4 Hz, 3H), 2.70 - 2.56 (m, 3H),2.39 (dd, J = 13.2, 4.4 Hz, 1H), 1.99 (q, J = 8.3, 7.2 Hz, 3H), 1.86 (d, J = 20.2 Hz, 6H), 1.51 (s, 2H). LCMS [M+H]⁺ = 887.0 |
| **1101** | 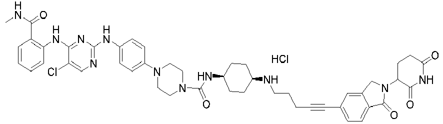 |
| | ¹H NMR (400 MHz, DMSO-d6) δ 11.83 - 11.71 (m, 1H), 11.00 (s, 1H), 9.65 (s, 1H), 9.02 (s, 2H), 8.83 (d, J = 4.9 Hz, 1H), 8.70 (s, 1H), 8.24 (s, 1H), 7.79(dd, J = 7.9, 1.6 Hz, 1H), 7.73 (d, J = 7.5 Hz, 1H), 7.67 (d, J = 7.6 Hz, 1H), 7.60 (s, 2H), 7.52 (dt, J = 12.8, 7.7 Hz, 2H), 7.25 (s, 1H), 7.17 (t, J = 7.6 Hz, 1H), 6.14 (s, 1H), 5.16 (dd, J = 13.2, 5.1 Hz, 1H), 4.43 (dd, J = 71.3, 17.7 Hz, 2H), 3.24 (s, 4H), 3.08 (ddt, J = 24.0, 9.4, 4.5 Hz, 5H), 2.96 - 2.88 (m, 1H),2.81 (d, J = 4.5 Hz, 3H), 2.64 (q, J = 7.8, 7.4 Hz, 3H), 2.57 (d, J = 4.0 Hz, 1H), 2.44 (s, 1H), 2.00 (dt, J = 14.9, 6.3 Hz, 4H), 1.86 (d, J = 27.4 Hz, 6H), 1.50(s, 2H). LCMS [M+H]⁺ = 887.0 |
| **1102** | 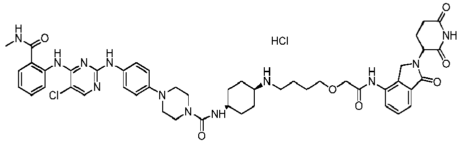 |
| | ¹H NMR (400 MHz, DMSO-*d*₆) δ 11.87 (s, 1H), 11.01 (s, 1H), 9.90 (s, 1H), 9.85 (s, 1H), 9.21 - 8.79 (m, 3H), 8.67 (d, *J* = 8.3 Hz, 1H), 8.28 (s, 1H), 7.79 (ddd, *J* = 12.0, 7.8, 1.4 Hz, 2H), 7.65 (s, 2H), 7.59 - 7.43 (m, 3H), 7.30 - 7.00 (m, 1H), 6.19 (s, 1H), 5.15 (dd, *J* = 13.3, 5.1 Hz, 1H), 4.40 (q, *J* = 17.5 Hz, 2H), 4.14 (s, 2H), 3.71 (s, 3H), 3.55 (d, *J* = 5.7 Hz, 5H), 3.34 (s, 4H), 3.04 (s, 1H), 2.98 - 2.88 (m, 3H), 2.81 (d, *J* = 4.4 Hz, 2H), 2.68 - 2.56 (m, 1H), 2.43 - 2.26 (m, 1H), 2.01 (ddd, *J* = 16.0, 8.0, 4.3 Hz, 2H), 1.91 - 1.72 (m, 7H), 1.67 (q, *J* = 7.2, 6.7 Hz, 2H), 1.47 (d, *J* = 13.7 Hz, 2H). LCMS [M+H]⁺ = 950.0 |
| **1103** | 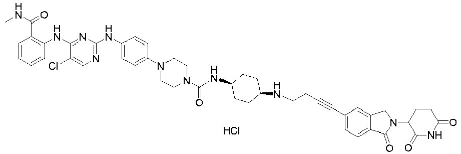 |
| | ¹H NMR (400 MHz, DMSO-*d*₆)δ11.85 (s, 1H), 11.00 (s, 1H), 9.78 (s, 1H), 9.16 (s, 2H), 8.84 (d, *J* = 4.5 Hz, 1H), 8.69 (s, 1H), 8.27 (s, 1H), 7.80 (d, *J* = 6.8 Hz, 1H), 7.70 (dd, *J* = 8.3, 2.3 Hz, 2H), 7.56 (dt, *J* = 15.9, 9.9 Hz, 3H), 7.20 (dd, *J* = 18.0, 10.0 Hz, 1H), 6.20 (s, 1H), 5.11 (dd, *J* = 13.3, 5.0 Hz, 1H), 4.46 (d, *J* = 7.7 Hz, 1H), 4.33 (d, *J* = 7.7 Hz, 1H), 3.80 (d, *J* = 7.9 Hz, 4H), 3.31 (t, *J* = 6.2 Hz, 4H), 3.30-3.15 (m, 4H), 2.98-2.76 (m, 3H), 2.67 (dd, *J* = 3.4, 5.7 Hz, 3H), 2.65 - 2.51 (m, 2H), 2.45 - 2.31 (m, 2H), 2.05 - 1.97 (m, 1H), 1.93-1.77 (m, 4H), 1.70-1.59 (m, 2H). LCMS [M+H]⁺ = 872.9 |
| **1104** | 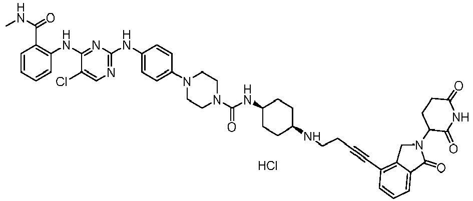 |
| | ¹H NMR (400 MHz, DMSO-*d*₆)611.87 (s, 1H), 11.02 (s, 1H), 9.81 (s, 1H), 9.18 (s, 2H), 8.84 (d, *J* = 4.5 Hz, 1H), 8.67 (s, 1H), 8.43 - 8.08 (m, 1H), 7.64 (dddd, *J* = 5.0, 5.8, 3.0, 5.2 Hz, 8H), 7.42 (d, *J* = 9.2 Hz, 2H), 7.45 - 6.78 (m, 1H), 6.19 (s, 1H), 5.17 (dd, *J* = 13.3, 5.1 Hz, 1H), 4.54 (d, *J* = 7.8 Hz, 1H), 4.36 (d, *J* = 7.8 Hz, 1H), 3.79 (s, 2H), 3.59 - 3.23 (m, 8H), 3.18 - 2.74 (m, 7H), 2.70 - 2.51 (m, 1H), 2.51 - 2.37 (m, 1H), 2.10 -2.05(m, 1H), 1.95- 1.75 (m, 7H), 1.46 (d, *J* = 6.7 Hz, 2H). LCMS [M+H]⁺ = 872.9 |
| **1109** | 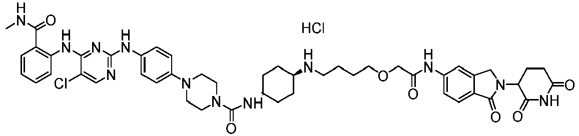 |
| | LCMS [M+H]⁺ = 950.0 |
| **1110** | 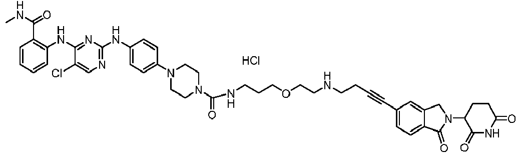 |
| | ¹H NMR (400 MHz, DMSO-*d*₆) δ 11.89 (s, 1H), 11.00 (s, 1H), 9.80 (s, 1H), 9.06 (s, 2H), 8.84 (d, *J* = 4.8 Hz, 1H), 8.67 (s, 1H), 8.27 (s, 1H), 7.79 (dd, *J* = 7.9, 1.6 Hz, 1H), 7.74 -7.68 (m, 2H), 7.66 - 7.55 (m, 3H), 7.51 (t, *J* = 7.9 Hz, 1H), 7.35 (s, 2H), 7.21 - 7.12 (m, 1H), 6.84 (s, 1H), 5.11 (dd, *J* = 13.3, 5.1 Hz, 1H), 3.69 (t, *J* = 5.1 Hz, 4H), 3.48 (t, *J* = 6.3 Hz, 3H), 3.30 (s, 4H), 3.27 - 3.22 (m, 3H), 3.17 (dt, *J* = 10.8, 6.8 Hz, 5H), 3.13 - 3.09 (m, 1H), 2.97 (t, *J* = 7.3 Hz, 2H), 2.81 (d, *J* = 4.5 Hz, 3H), 2.06 - 1.90 (m, 2H), 1.70 (t, *J* = 6.4 Hz, 3H), 1.33 - 1.15 (m, 4H). LCMS [M+H]⁺=877. |
| **1111** | 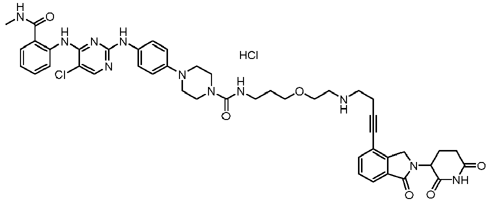 |
| | ¹H NMR (400 MHz, DMSO-*d*₆) δ 11.97 (s, 1H), 11.02 (s, 1H), 10.04 (s, 1H), 9.29 (s, 2H), 8.89 (d, *J* = 4.8 Hz, 1H), 8.63 (d, *J* = 8.7 Hz, 1H), 8.31 (s, 1H), 7.82 (dd, *J* = 7.9, 1.6 Hz, 1H), 7.77 - 7.62 (m, 4H), 7.54 (td, *J* = 7.4, 3.8 Hz, 3H), 7.20 (td, *J* = 7.6, 1.2 Hz, 1H), 6.93 (s, 1H), 5.16 (dd, *J* = 13.3, 5.1 Hz, 1H), 4.57 - 4.25 (m, 3H), 3.70 (t, *J* = 5.1 Hz, 4H), 3.52 - 3.32 (m, 6H), 3.20 (dq, *J* = 26.2, 6.7 Hz, 6H), 3.06 - 2.87 (m, 3H), 2.81 (d, *J* = 4.4 Hz, 3H), 2.70 - 2.53 (m, 2H), 2.48 - 2.31 (m, 1H), 2.02 (dq, *J* = 9.2, 3.5, 3.0 Hz, 1H), 1.70 (p, *J* = 6.5 Hz, 2H), 1.36 - 1.18 (m, 2H).LCMS [M+H]⁺=877. |
| **1112** | 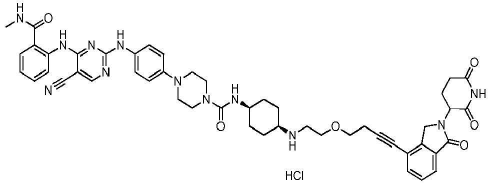 |
| | ¹H NMR (400 MHz, DMSO-*d*₆) δ 11.83 (s, 1H), 11.01 (s, 1H), 10.21 (s, 1H), 9.16 - 8.91 (m, 2H), 8.86 (d, *J* = 5.0 Hz, 1H), 8.61 (s, 1H), 8.51 (s, 1H), 7.81 (dd, *J* = 8.0, 1.6 Hz, 1H), 7.76 - 7.63 (m, 3H), 7.54 (q, *J* = 7.2 Hz, 3H), 7.22 (d, *J* = 7.0 Hz, 1H), 6.21 (s, 1H), 5.16 (dd, *J* = 13.3, 5.1 Hz, 1H), 4.55 - 4.22 (m, 3H), 3.80 (t, *J* = 5.3 Hz, 7H), 3.70 (t, *J* = 6.6 Hz, 4H), 3.40 (d, *J* = 7.6 Hz, 3H), 3.12 (t, *J* = 7.2 Hz, 3H), 2.92 (ddd, *J* = 17.2, 13.6, 5.4 Hz, 1H), 2.81 (d, *J* = 4.9 Hz, 4H), 2.66 - 2.55 (m, 1H), 2.43 (dd, *J* = 13.3, 4.5 Hz, 1H), 2.01 (ddt, *J* = 12.6, 9.9, 4.9 Hz, 1H), 1.82 (d, *J* = 11.8 Hz, 5H), 1.45 (s, 2H). LCMS [M+H]⁺ = 907.9 |
| **1114** | 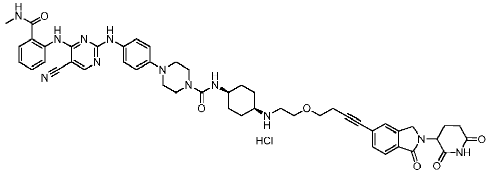 |
| | ¹H NMR (400 MHz, DMSO-*d*₆) δ 11.83 (s, 1H), 11.00 (s, 1H), 10.21 (s, 1H), 9.03 - 8.90 (m, 2H), 8.86 (d, *J* = 4.8 Hz, 1H), 8.60 (s, 1H), 8.17 - 8.01 (m, 1H), 7.81 (dd, *J* = 8.0, 1.6 Hz, 1H), 7.70 (d, *J* = 7.8 Hz, 2H), 7.65 (s, 1H), 7.61 - 7.47 (m, 2H), 7.21 (t, *J* = 7.6 Hz, 1H), 6.23 (s, 1H), 5.11 (dd, *J* = 13.3, 5.1 Hz, 1H), 4.52 - 4.25 (m, 3H), 3.80 (t, *J* = 5.1 Hz, 5H), 3.72 - 3.63 (m, 5H), 3.59 (d, *J* = 15.2 Hz, 1H), 3.41 (q, *J* = 6.2, 5.3 Hz, 3H), 3.14 (s, 3H), 3.00 - 2.85 (m, 1H), 2.87 - 2.74 (m, 3H), 2.64 - 2.57 (m, 1H), 2.39 (dd, *J* = 13.2, 4.5 Hz, 1H), 2.11 - 1.91 (m, 1H), 1.85 (q, *J* = 11.3, 8.4 Hz, 5H), 1.70 (d, *J* = 11.0 Hz, 1H), 1.47 (s, 2H). LCMS [M+H]⁺ = 907.8 |
| **1115** | 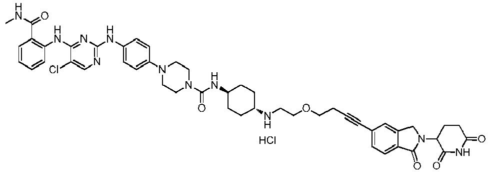 |
| | ¹H NMR (400 MHz, DMSO-*d*₆) δ 11.96 (s, 1H), 11.00 (s, 1H), 10.04 (s, 1H), 9.00 (s, 2H), 8.94 - 8.81 (m, 1H), 8.63 (d, *J* = 8.2 Hz, 1H), 8.31 (s, 1H), 7.82 (dd, *J* = 7.9, 1.6 Hz, 1H), 7.74 - 7.63 (m, 3H), 7.60 - 7.47 (m, 3H), 7.21 (d, *J* = 7.6 Hz, 1H), 6.59 (s, 1H), 5.11 (dd, *J* = 13.3, 5.1 Hz, 1H), 4.55 - 4.23 (m, 3H), 3.80 (t, *J* = 5.2 Hz, 3H), 3.69 (t, *J* = 6.7 Hz, 5H), 3.49 - 3.34 (m, 4H), 3.14 (qd, *J* = 7.0, 4.7, 3.9 Hz, 2H), 3.06 - 2.95 (m, 1H), 2.92 - 2.85 (m, 1H), 2.80 (dd, *J* = 12.8, 5.6 Hz, 4H), 2.66 - 2.54 (m, 1H), 2.39 (dd, *J* = 13.2, 4.6 Hz, 1H), 2.12 (d, *J* = 11.8 Hz, 2H), 2.06 - 1.94 (m, 1H), 1.87 (d, *J* = 11.7 Hz, 2H), 1.47 (q, *J* = 12.2 Hz, 2H), 1.37 - 1.24 (m, 3H). LCMS [M+H]⁺ = 916.8 |
| **1116** | 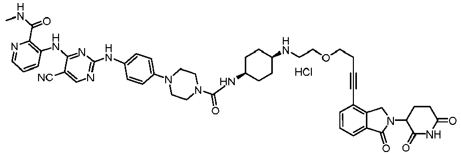 |
| | ¹H NMR (400 MHz, DMSO-d6) δ 11.01 (s, 1H), 10.08 (s, 1H), 9.18 (s, 1H), 8.94 (s, 2H), 8.61 (s, 1H), 8.32 (d, J = 4.3 Hz, 1H), 7.72 (d, J = 7.4 Hz, 1H), 7.65(d, J = 7.5 Hz, 1H), 7.53 (t, J = 7.5 Hz, 4H), 7.23 (d, J = 35.1 Hz, 2H), 6.07 (s, 1H), 5.16 (dd, J = 13.2, 5.0 Hz, 1H), 4.56 - 4.25 (m, 2H), 3.80 (t, J = 5.2 Hz,3H), 3.70 (t, J = 6.7 Hz, 6H), 3.16 (d, J = 29.1 Hz, 8H), 2.99 - 2.88 (m, 1H), 2.85 (d, J = 4.7 Hz, 3H), 2.80 (t, J = 6.6 Hz, 2H), 2.60 (d, J = 16.9 Hz, 1H), 2.45(d, J = 4.3 Hz, 1H), 2.10 - 1.98 (m, 1H), 1.82 (d, J = 10.1 Hz, 6H), 1.44 (s, 2H) LCMS [M+H]⁺ = 908.9 |
| **1117** | 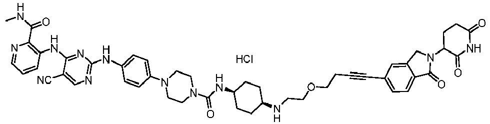 |
| | ¹H NMR (400 MHz, DMSO-d6) δ 12.65 (s, 1H), 11.00 (s, 1H), 10.05 (s, 1H), 9.18 (s, 1H), 8.86 (s, 2H), 8.60 (s, 1H), 8.32 (d, J = 4.3 Hz, 1H), 7.70 (d, J =7.8 Hz, 1H), 7.65 (s, 1H), 7.58 - 7.40 (m, 3H), 7.20 (d, J = 40.2 Hz, 2H), 6.08 (s, 1H), 5.11 (dd, J = 13.2, 5.1 Hz, 1H), 4.51-4.26 (m, 2H), 3.79 (t, J = 5.1Hz, 2H), 3.69 (t, J = 6.6 Hz, 4H), 3.16 (s, 7H), 2.97 - 2.87 (m, 1H), 2.85 (d, J = 4.9 Hz, 3H), 2.79 (t, J = 6.6 Hz, 2H), 2.60 (d, J = 16.6 Hz, 1H), 2.40 -2.31 (m, 1H), 2.06 - 1.95 (m, 1H), 1.81 (s, 6H), 1.44 (d, J = 21.5 Hz, 2H). LCMS [M+H]⁺ = 908.9 |
| **1119** | 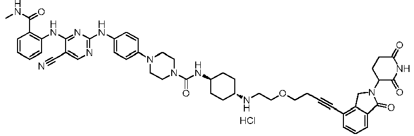 |
| | ¹H NMR (400 MHz, DMSO-*d*₆) δ 11.81 (s, 1H), 11.02 (s, 1H), 10.09 (s, 1H), 9.05 - 8.88 (m, 3H), 8.83 (d, *J* = 4.9 Hz, 1H), 8.58 (s, 1H), 8.07 (dd, *J* = 7.9, 6.4 Hz, 1H), 7.79 (dd, *J* = 7.9, 1.6 Hz, 1H), 7.73 (d, *J* = 7.5 Hz, 1H), 7.65 (t, *J* = 8.5 Hz, 2H), 7.53 (q, *J* = 7.8 Hz, 2H), 7.35 (s, 2H), 7.19 (t, *J* = 7.5 Hz, 1H), 6.49 (s, 1H), 5.17 (dd, *J* = 13.3, 5.1 Hz, 1H), 4.59 - 4.17 (m, 3H), 3.79 (t, *J* = 5.2 Hz, 4H), 3.70 (t, *J* = 6.7 Hz, 4H), 3.47 - 3.38 (m, 2H), 3.36 - 3.24 (m, 4H), 3.13 (dd, *J* = 7.7, 3.8 Hz, 2H), 3.01 - 2.86 (m, 2H), 2.81 (d, *J* = 4.9 Hz, 4H), 2.67 - 2.56 (m, 1H), 2.48 - 2.37 (m, 1H), 2.19 - 1.94 (m, 4H), 1.85 (d, *J* = 12.0 Hz, 2H), 1.45 (p, *J* = 10.8, 9.2 Hz, 2H). LCMS [M+H]⁺ = 907.7 |
| **1120** | 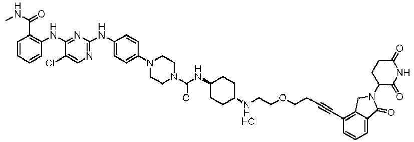 |
| | ¹H NMR (400 MHz, DMSO-*d*₆) δ 11.93 (s, 1H), 11.02 (s, 1H), 9.98 (s, 1H), 9.04 (s, 2H), 8.88 (d, *J* = 4.7 Hz, 1H), 8.65 (d, *J* = 8.5 Hz, 1H), 8.30 (s, 1H), 7.82 (dd, *J* = 8.0, 1.6 Hz, 1H), 7.73 (d, *J* = 7.5 Hz, 1H), 7.66 (d, *J* = 7.2 Hz, 2H), 7.53 (td, *J* = 7.8, 3.6 Hz, 3H), 7.20 (t, *J* = 7.6 Hz, 1H), 6.56 (s, 1H), 5.17 (dd, *J* = 13.3, 5.1 Hz, 1H), 4.63 - 4.25 (m, 3H), 3.80 (t, *J* = 5.3 Hz, 5H), 3.70 (t, *J* = 6.7 Hz, 5H), 3.38 (s, 5H), 3.13 (dd, *J* = 7.4, 3.9 Hz, 2H), 3.04 - 2.85 (m, 2H), 2.93 - 2.76 (m, 4H), 2.69 - 2.55 (m, 1H), 2.45 (d, *J* = 4.4 Hz, 1H), 2.11 (d, *J* = 9.5 Hz, 2H), 2.06 - 1.94 (m, 1H), 1.93 - 1.74 (m, 2H), 1.62 - 1.39 (m, 2H). LCMS [M+H]⁺ = 916.7 |
| **1121** | 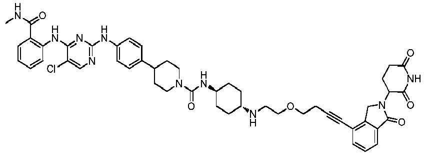 |
| | ¹H NMR (400 MHz, DMSO-*d*₆) δ 11.62 (s, 1H), 11.02 (s, 1H), 9.39 (s, 1H), 8.77 (dd, *J* = 10.0, 6.3 Hz, 2H), 8.20 (s, 1H), 7.75 (ddd, *J* = 13.4, 7.8, 1.3 Hz, 2H), 7.65 (dd, *J* = 7.7, 1.1 Hz, 1H), 7.62 - 7.41 (m, 4H), 7.14 (t, *J* = 7.5 Hz, 3H), 6.21 (d, *J* = 7.6 Hz, 1H), 5.17 (dd, *J* = 13.3, 5.2 Hz, 1H), 4.55 - 4.20 (m, 2H), 4.09 (d, *J* = 12.8 Hz, 2H), 3.71 (dt, *J* = 19.2, 6.0 Hz, 4H), 3.09 (s, 2H), 3.00 - 2.86 (m, 2H), 2.85 - 2.77 (m, 5H), 2.74 - 2.56 (m, 5H), 2.44 (d, *J* = 4.6 Hz, 1H), 2.01 (dt, *J* = 15.6, 4.7 Hz, 4H), 1.83 (d, *J* = 12.0 Hz, 2H), 1.79 - 1.65 (m, 2H), 1.55 - 1.30 (m, 5H). LCMS [M+H]⁺ = 916.9 |
| **1122** | 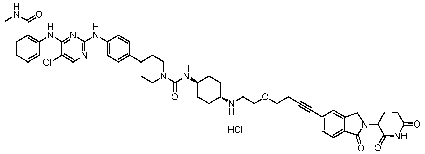 |
| | ¹H NMR (400 MHz, DMSO-*d*₆) δ 11.83 (s, 1H), 11.00 (s, 1H), 9.70 (s, 1H), 9.03 - 8.77 (m, 3H), 8.68 (d, *J* = 8.4 Hz, 1H), 8.26 (s, 1H), 7.79 (dd, *J* = 7.9, 1.6 Hz, 1H), 7.70 (d, *J* = 7.9 Hz, 1H), 7.65 (s, 1H), 7.53 (dd, *J* = 8.1, 4.9 Hz, 3H), 7.47 (s, 1H), 7.18 (t, *J* = 7.7 Hz, 2H), 6.25 (s, 1H), 5.11 (dd, *J* = 13.3, 5.1 Hz, 1H), 4.56 - 4.24 (m, 2H), 4.10 (d, *J* = 12.7 Hz, 2H), 3.78 (t, *J* = 5.2 Hz, 2H), 3.69 (t, *J* = 6.7 Hz, 3H), 3.40 (p, *J* = 7.8 Hz, 2H), 3.14 (tt, *J* = 6.9, 3.9 Hz, 2H), 2.97 (d, *J* = 9.3 Hz, 1H), 2.95 - 2.85 (m, 1H), 2.85 - 2.76 (m, 4H), 2.74 - 2.54 (m, 5H), 2.39 (qd, *J* = 13.1, 4.4 Hz, 1H), 2.10 (d, *J* = 11.7 Hz, 2H), 2.03 (dd, *J* = 7.3, 4.4 Hz, 1H), 1.92 - 1.80 (m, 2H), 1.73 (d, *J* = 12.5 Hz, 2H), 1.61 - 1.34 (m, 4H). LCMS [M+H]⁺ = 916.1 |
| **1123** | 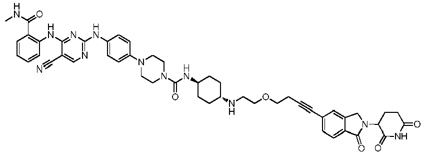 |
| | ¹H NMR (400 MHz, DMSO-*d*₆) δ 11.74 (s, 1H), 11.00 (s, 1H), 9.90 (s, 1H), 8.78 (d, *J* = 4.8 Hz, 1H), 8.52 (s, 1H), 8.44 (s, 1H), 7.77 (d, *J* = 7.8 Hz, 1H), 7.71 (d, *J* = 7.9 Hz, 1H), 7.64 (s, 1H), 7.56 - 7.35 (m, 3H), 7.26 - 7.09 (m, 1H), 6.98 - 6.79 (m, 2H), 6.35 (d, *J* = 7.6 Hz, 1H), 5.11 (dd, *J* = 13.3, 5.1 Hz, 1H), 4.58 - 4.18 (m, 2H), 3.69 (dd, *J* = 15.4, 8.7 Hz, 4H), 3.42 (d, *J* = 5.2 Hz, 4H), 3.38 (s, 1H), 3.10 (s, 2H), 3.04 (s, 3H), 2.91 (ddd, *J* = 17.6, 13.6, 5.5 Hz, 2H), 2.85 - 2.77 (m, 4H), 2.68 - 2.53 (m, 2H), 2.39 (dd, *J* = 13.1, 4.5 Hz, 1H), 2.17 - 1.93 (m, 4H), 1.84 (d, *J* = 12.0 Hz, 2H), 1.40 - 1.28 (m, 4H). LCMS [M+H]⁺ = 908.0 |
| **1124** | 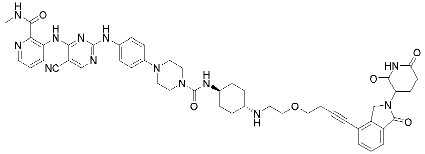 |
| | ¹H NMR (400 MHz, DMSO-d6) δ 11.83 (s, 1H), 11.00 (s, 1H), 9.70 (s, 1H), 9.01 - 8.80 (m, 3H), 8.68 (d, J = 8.4 Hz, 1H), 8.26 (s, 1H), 7.79 (dd, J = 8.0, 1.6Hz, 1H), 7.70 (d, J = 7.9 Hz, 1H), 7.65 (s, 1H), 7.53 (dd, J = 8.2, 4.9 Hz, 3H), 7.50 - 7.42 (m, 1H), 7.18 (t, J = 7.7 Hz, 3H), 6.25 (s, 1H), 5.11 (dd, J = 13.3,5.1 Hz, 1H), 4.49 - 4.29 (m, 2H), 4.10 (d, J = 12.7 Hz, 2H), 3.78 (t, J = 5.2 Hz, 2H), 3.69 (s, 2H), 3.41 (s, 2H), 3.14 (td, J = 6.7, 3.2 Hz, 2H), 3.02 - 2.89 (m,2H), 2.84 - 2.75 (m, 5H), 2.75 - 2.56 (m, 5H), 2.41 (td, J = 13.1, 4.5 Hz, 1H), 2.10 (d, J = 11.8 Hz, 2H), 2.01 (tt, J = 9.2, 4.4 Hz, 2H), 1.93 - 1.80 (m, 2H),1.73 (d, J = 12.5 Hz, 2H), 1.53 - 1.39 (m, 4H), 1.33 - 1.22 (m, 5H). LCMS [M+H]⁺ = 908.9 |
| **1125** | 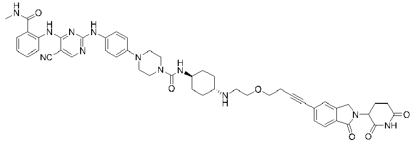 |
| | ¹H NMR (400 MHz, DMSO-d6) δ 11.61 (s, 1H), 11.02 (s, 1H), 9.39 (s, 1H), 8.76 (dd, J = 10.0, 6.3 Hz, 2H), 8.20 (s, 1H), 7.75 (ddd, J = 13.4, 7.8, 1.3 Hz, 2H),7.65 (dd, J = 7.7, 1.1 Hz, 1H), 7.60 - 7.44 (m, 4H), 7.14 (t, J = 7.5 Hz, 3H), 6.21 (d, J = 7.6 Hz, 1H), 5.16 (dd, J = 13.3, 5.2 Hz, 1H), 4.51 - 4.27 (m, 3H), 4.09(d, J = 12.8 Hz, 2H), 3.73 (t, J = 5.2 Hz, 2H), 3.68 (d, J = 6.7 Hz, 2H), 3.08 (s, 2H), 2.96 - 2.88 (m, 2H), 2.83 - 2.75 (m, 5H), 2.75 - 2.57 (m, 5H), 2.45 - 2.40(m, 1H), 2.11 - 1.97 (m, 4H), 1.83 (d, J = 12.0 Hz, 2H), 1.75 - 1.67 (m, 2H), 1.51 - 1.32 (m, 5H). LCMS [M+H]⁺ = 908.9 |
| **1127** | 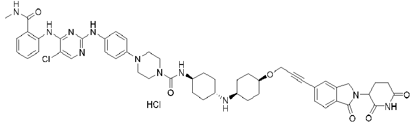 |
| | ¹H NMR (400 MHz, DMSO-*d*₆)δ11.74 (s, 1H), 11.00 (s, 1H), 9.51 (s, 1H), 8.83-8.58 (m, 4H), 8.21 (s, 1H), 7.64-7.42 (m, 8H), 7.45 - 6.78 (m, 2H), 6.49 (s, 1H), 5.17 (dd, *J* = 13.3, 5.1 Hz, 1H), 4.54-4.36 (m, 5H), 3.29 - 3.03 (m, 6H), 3.00 - 2.74 (m, 4H), 2.70 - 2.51 (m, 3H),2.51-2.37(m, 3H), 2.10-1.97(m,5H),1.95-1.75(m, 2H), 1.49-1.35 (m, 4H), 1.49-1.35 (m, 4H). LCMS [M+H]⁺ = 956.8 |
| **1128** | 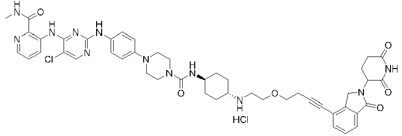 |
| | ¹H NMR (400 MHz, ) δ 12.56 (s, 1H), 11.03 (s, 1H), 9.43 (d, *J* = 39.5 Hz, 2H), 9.18 (d, *J* = 4.9 Hz, 1H), 8.65 (s, 2H), 8.37 - 8.20 (m, 2H), 7.74 (d, *J* = 7.5 Hz, 1H), 7.66 (d, *J* = 6.8 Hz, 1H), 7.55 (t, *J* = 7.6 Hz, 4H), 7.10 (s, 2H), 6.39 (s, 1H), 5.18 (dd, *J* = 13.3, 5.1 Hz, 1H), 4.47 (d, *J* = 17.6 Hz, 1H), 4.33 (d, *J* = 17.7 Hz, 1H), 3.78 - 3.74 (m, 2H), 3.71 (d, *J* = 6.7 Hz, 2H), 3.52 - 3.48 (m, 4H), 3.43 - 3.36 (m, 2H), 3.15 (s, 6H), 2.96 - 2.90 (m, 1H), 2.86 (d, *J* = 4.9 Hz, 3H), 2.81 (t, *J* = 6.7 Hz, 2H), 2.65 (dd, *J* = 25.4, 9.1 Hz, 1H), 2.36 - 2.31 (m, 1H), 2.05 (dd, *J* = 18.4, 7.8 Hz, 3H), 1.87 (d, *J* = 10.8 Hz, 2H), 1.46 - 1.37 (m, 2H), 1.24 (d, *J* = 11.8 Hz, 2H).. LCMS [M+H]⁺ = 917.8 |
| **1129** | 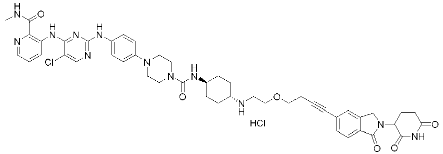 |
| | ¹H NMR (400 MHz, DMSO) δ 12.58 (s, 1H), 11.00 (s, 1H), 9.55 (s, 1H), 9.37 (s, 1H), 9.18 (d, *J* = 4.7 Hz, 1H), 8.68 (s, 2H), 8.34 - 8.24 (m, 2H), 7.71 (d, *J* = 7.8 Hz, 1H), 7.64 (s, 1H), 7.52 (d, *J* = 8.3 Hz, 4H), 7.18 (s, 2H), 6.43 (s, 1H), 5.11 (dd, *J* = 13.4, 5.1 Hz, 1H), 4.45 (d, *J* = 17.5 Hz, 1H), 4.33 (d, *J* = 17.6 Hz, 1H), 3.68 (s, 4H), 3.53 (s, 4H), 3.42 (s, 2H), 3.16 (s, 5H), 2.91 (s, 1H), 2.85 (d, *J* = 4.9 Hz, 3H), 2.79 (t, *J* = 6.7 Hz, 2H), 2.62 (s, 1H), 2.58 (s, 1H), 2.35 (d, *J* = 20.6 Hz, 1H), 2.07 (s, 2H), 2.01 (d, *J* = 5.4 Hz, 1H), 1.86 (d, *J* = 12.1 Hz, 2H), 1.42 (d, *J* = 13.2 Hz, 2H), 1.25 (d, *J* = 10.3 Hz, 2H).LCMS [M+H]⁺ = 917.7 |
| **1131** | 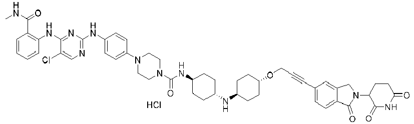 |
| | LCMS [M+H]⁺ = 956.7 |
| **1132** | 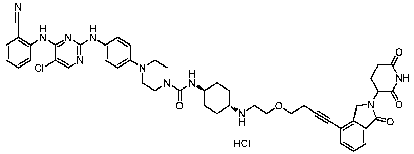 |
| | ¹H NMR (400 MHz, DMSO-*d*₆) δ 11.02 (s, 1H), 9.74 (d, *J* = 40.0 Hz, 2H), 9.00 (s, 2H), 8.28 (s, 1H), 7.93 (dd, *J* = 7.8, 1.5 Hz, 1H), 7.80 (td, *J* = 7.8, 1.6 Hz, 1H), 7.73 (dd, *J* = 7.6, 1.0 Hz, 1H), 7.69 - 7.60 (m, 2H), 7.58 - 7.47 (m, 2H), 7.44 (d, *J* = 8.3 Hz, 2H), 7.33 (s, 2H), 6.54 (s, 1H), 5.17 (dd, *J* = 13.3, 5.1 Hz, 1H), 4.58 - 4.27 (m, 2H), 3.82 - 3.75 (m, 3H), 3.70 (t, *J* = 6.7 Hz, 6H), 3.36 (d, *J* = 31.2 Hz, 5H), 3.13 (s, 2H), 3.04 - 2.82 (m, 2H), 2.80 (t, *J* = 6.7 Hz, 2H), 2.68 - 2.55 (m, 1H), 2.45 (d, *J* = 4.6 Hz, 1H), 2.10 (d, *J* = 11.7 Hz, 2H), 2.06-1.93 (m, 2H), 1.85 (d, *J* = 11.9 Hz, 2H), 1.45 (q, *J* = 12.5 Hz, 2H), 1.33 - 1.25 (m, 2H). LCMS [M+H]⁺ = 884.8 |
| **1133** | 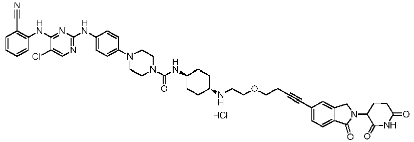 |
| | ¹H NMR (400 MHz, DMSO-*d*₆) δ 11.00 (s, 1H), 9.80 (d, *J* = 46.1 Hz, 2H), 8.99 (s, 2H), 8.29 (s, 1H), 7.94 (dd, *J* = 7.8, 1.5 Hz, 1H), 7.81 (td, *J* = 7.8, 1.6 Hz, 1H), 7.70 (d, *J* = 7.9 Hz, 1H), 7.67 - 7.62 (m, 2H), 7.53 (dd, *J* = 7.7, 1.2 Hz, 2H), 7.44 (d, *J* = 8.1 Hz, 2H), 7.38 (s, 1H), 6.58 (s, 1H), 5.11 (dd, *J* = 13.3, 5.1 Hz, 1H), 4.52 - 4.24 (m, 3H), 3.79 (t, *J* = 5.2 Hz, 3H), 3.69 (t, *J* = 6.7 Hz, 3H), 3.38 (d, *J* = 25.4 Hz, 5H), 3.13 (d, *J* = 7.2 Hz, 2H), 3.06 - 2.84 (m, 2H), 2.79 (t, *J* = 6.6 Hz, 2H), 2.67 - 2.53 (m, 1H), 2.39 (qd, *J* = 13.2, 4.5 Hz, 1H), 2.12 (d, *J* = 11.7 Hz, 2H), 2.06 - 1.95 (m, 2H), 1.91 - 1.79 (m, 2H), 1.47 (q, *J=* 12.8, 12.3 Hz, 2H), 1.33 - 1.25 (m, 2H). LCMS [M+H]⁺ = 884.8 |
| **1134** | 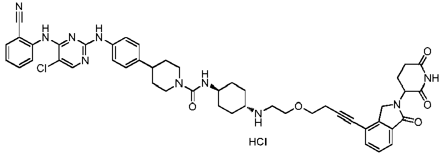 |
| | ¹H NMR (400 MHz, DMSO-*d*₆) δ 11.02 (s, 1H), 9.61 (s, 2H), 8.94 (s, 2H), 8.25 (s, 1H), 7.93 (dd, *J* = 7.8, 1.5 Hz, 1H), 7.79 (td, *J* = 7.9, 1.6 Hz, 1H), 7.73 (d, *J* = 7.5 Hz, 1H), 7.65 (t, *J* = 7.9 Hz, 2H), 7.52 (dt, *J* = 11.8, 7.6 Hz, 2H), 7.30 (d, *J* = 8.1 Hz, 2H), 6.93 (d, *J* = 8.3 Hz, 2H), 6.22 (s, 1H), 5.17 (dd, *J* = 13.3, 5.1 Hz, 1H), 4.52 - 4.22 (m, 2H), 4.07 (d, *J* = 13.2 Hz, 2H), 3.79 (t, *J* = 5.3 Hz, 2H), 3.70 (t, *J* = 6.7 Hz, 2H), 3.46 - 3.30 (m, 2H), 3.13 (t, *J* = 5.8 Hz, 2H), 2.94 (ddd, *J* = 13.6, 10.9, 6.9 Hz, 2H), 2.80 (t, *J* = 6.7 Hz, 2H), 2.72 - 2.60 (m, 3H), 2.43 (d, *J* = 9.0 Hz, 1H), 2.18 - 1.93 (m, 4H), 1.91 - 1.76 (m, 2H), 1.71 - 1.59 (m, 2H), 1.52 - 1.32 (m, 5H). LCMS [M+H]⁺ = 884.0 |
| **1135** | 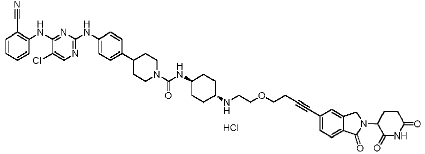 |
| | ¹H NMR (400 MHz, DMSO-*d*₆) δ 11.00 (s, 1H), 9.58 (s, 2H), 8.92 (dt, *J* = 6.2, 3.2 Hz, 2H), 8.24 (s, 1H), 8.05 (dd, *J* = 7.8, 6.4 Hz, 1H), 7.93 (dd, *J* = 7.7, 1.5 Hz, 1H), 7.79 (td, *J* = 7.8, 1.6 Hz, 1H), 7.75 - 7.60 (m, 3H), 7.60 - 7.42 (m, 2H), 7.30 (d, *J* = 8.1 Hz, 2H), 6.93 (d, *J* = 8.3 Hz, 2H), 6.24 (s, 1H), 5.11 (dd, *J =* 13.3, 5.1 Hz, 1H), 4.61 - 4.22 (m, 2H), 4.07 (d, *J* = 12.7 Hz, 2H), 3.78 (t, *J* = 5.2 Hz, 2H), 3.69 (t, *J* = 6.7 Hz, 2H), 3.47 - 3.34 (m, 2H), 3.15 (q, *J* = 5.6 Hz, 2H), 2.92 (s, 1H), 2.79 (t, *J* = 6.7 Hz, 2H), 2.58 (m, 4H), 2.39 (dd, *J* = 13.1, 4.6 Hz, 1H), 2.17 - 2.05 (m, 2H), 2.06 - 1.96 (m, 2H), 1.92 - 1.77 (m, 2H), 1.73 - 1.50 (m, 2H), 1.48 - 1.33 (m, 5H). LCMS [M+H]⁺ = 883.9 |
| **1137** | 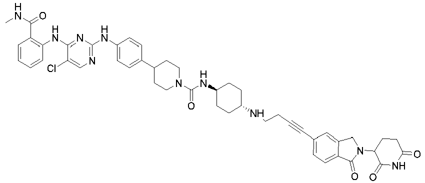 |
| | ¹H NMR (400 MHz, DMSO-*d*₆)δ11.74 (s, 1H), 11.00 (s, 1H), 9.46 (s, 1H), 9.45-9.38 (m, 5H), 8.86-8.68 (m, 2H), 8.21 (s, 1H), 7.64-7.42 (m, 7H), 7.14 (t, *J* = 7.7 Hz, 3H), 6.27 (d, *J* = 7.5 Hz, 1H), 5.12 (dd, *J* = 13.2, 5.1 Hz, 1H), 4.67 - 4.34 (m, 2H), 4.34 (d, *J* = 7.6 Hz, 1H), 4.10 (d, *J* = 12.0 Hz, 2H), 3.83 - 3.47 (m, 4H), 3.35 -3.20 (m, 6H), 2.96 - 2.85 (m, 2H), 2.85 - 2.65 (m, 6H), 2.60 (d, *J* = 12.9 Hz, 3H), 2.55 - 2.47 (m, 1H), 2.43 (dd, *J* = 13.2, 4.5 Hz, 1H), 2.39 - 1.97 (m, 5H), 1.97-1.60 (m, 6H), 1.65 - 1.22 (m, 2H). LCMS [M+H]⁺ = 871.8 |
| **1138** | 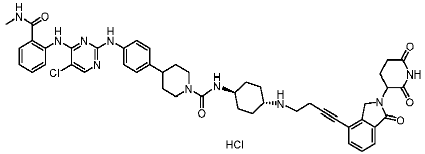 |
| | ¹H NMR (400 MHz, DMSO-*d*₆)δ11.81(s, 1H), 11.03 (s, 1H), 9.66 (s, 1H), 9.20 (s, 2H), 8.86-8.68 (m, 2H), 8.21 (s, 1H), 7.84-7.42 (m, 8H), 7.14 (t, *J* = 7.7 Hz, 3H), 6.27 (d, *J* = 7.5 Hz, 1H), 5.12 (dd, *J* = 13.2, 5.1 Hz, 1H), 4.67 - 4.34 (m, 2H), 4.34 (d, *J* = 7.6 Hz, 1H), 4.10 (d, *J* = 12.0 Hz, 2H), 3.35 -3.20 (m, 2H), 3.10 - 2.85 (m, 4H), 2.85 - 2.55 (m, 8H), 2.55 - 2.47 (m, 1H), 2.41 (d, *J* = 12.9 Hz, 2H), 2.10 (dd, *J* = 3.2, 1.8 Hz, 2H), 1.90 (ddd, *J* = 6.4, 5.8, 9.4 Hz, 2H), 1.46 (dd, *J* = 2.1, 1.7 Hz, 4H), 1.25 (d, *J* = 5.4 Hz, 2H). LCMS \|M+H]⁺ = 871.8 |
| **1139** | 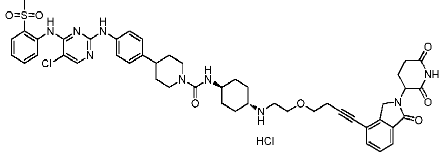 |
| | ¹H NMR (400 MHz, DMSO-*d*₆) δ 11.02 (s, 1H), 9.59 (s, 1H), 9.43 (s, 1H), 8.93 (s, 2H), 8.48 (d, *J* = 8.1 Hz, 1H), 8.29 (s, 1H), 7.94 (dd, *J* = 8.0, 1.6 Hz, 1H), 7.74 (dd, *J* = 8.1, 5.3 Hz, 2H), 7.66 (d, *J* = 7.5 Hz, 1H), 7.59 - 7.34 (m, 4H), 7.09 (d, *J* = 8.3 Hz, 2H), 6.23 (s, 1H), 5.17 (dd, *J* = 13.3, 5.2 Hz, 1H), 4.63 - 4.24 (m, 2H), 4.09 (d, *J* = 12.7 Hz, 2H), 3.79 (t, *J* = 5.2 Hz, 2H), 3.70 (t, *J* = 6.7 Hz, 2H), 3.39 (s, 2H), 3.27 (s, 3H), 3.13 (p, *J* = 5.4 Hz, 2H), 3.06 - 2.86 (m, 2H), 2.80 (t, *J* = 6.7 Hz, 2H), 2.69 (t, *J* = 11.9 Hz, 2H), 2.63 - 2.56 (m, 2H), 2.49 - 2.33 (m, 1H), 2.17 - 1.96 (m, 4H), 1.84 (d, *J* = 11.3 Hz, 2H), 1.78 - 1.60 (m, 2H), 1.56 - 1.35 (m, 4H). LCMS [M+H]⁺ = 936.9 |
| **1140** | 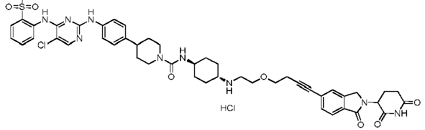 |
| | ¹H NMR (400 MHz, DMSO-*d*₆) δ 11.00 (s, 1H), 9.58 (s, 1H), 9.43 (s, 1H), 8.85 (s, 2H), 8.48 (d, *J* = 8.2 Hz, 1H), 8.29 (s, 1H), 7.94 (dd, *J* = 8.0, 1.6 Hz, 1H), 7.82 - 7.58 (m, 3H), 7.60 - 7.26 (m, 4H), 7.09 (d, *J* = 8.4 Hz, 2H), 6.24 (s, 1H), 5.11 (dd, *J* = 13.3, 5.1 Hz, 1H), 4.59 - 4.25 (m, 2H), 4.09 (d, *J* = 12.8 Hz, 2H), 3.78 (t, *J* = 5.1 Hz, 2H), 3.69 (t, *J* = 6.7 Hz, 2H), 3.27 (s, 3H), 3.15 (q, *J* = 6.2, 5.5 Hz, 2H), 3.02 - 2.85 (m, 3H), 2.79 (t, *J* = 6.7 Hz, 2H), 2.73 - 2.54 (m, 5H), 2.45 - 2.30 (m, 1H), 2.10 (d, *J* = 11.5 Hz, 2H), 2.00 (dp, *J* = 12.1, 4.4, 3.5 Hz, 2H), 1.85 (d, *J* = 12.2 Hz, 2H), 1.74 - 1.64 (m, 2H), 1.43 (dd, *J* = 12.5, 4.1 Hz, 4H). LCMS [M+H]⁺ = 936.9 |
| **1141** | 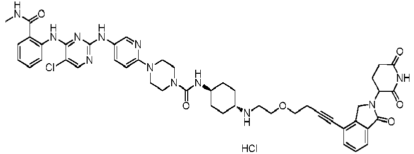 |
| | ¹H NMR (400 MHz, DMSO-*d*₆) δ 11.96 (s, 1H), 11.02 (s, 1H), 9.94 (s, 1H), 9.12 (s, 2H), 8.91 (d, *J* = 4.8 Hz, 1H), 8.71 (s, 1H), 8.44 (s, 1H), 8.31 (s, 1H), 8.25 (dd, *J* = 9.7, 2.6 Hz, 1H), 7.82 (dd, *J* = 7.9, 1.6 Hz, 1H), 7.73 (d, *J* = 7.5 Hz, 1H), 7.66 (d, *J* = 7.5 Hz, 1H), 7.54 (td, *J* = 7.5, 4.7 Hz, 2H), 7.45 (d, *J* = 9.8 Hz, 1H), 7.18 (t, *J* = 7.5 Hz, 1H), 6.50 (s, 1H), 5.17 (dd, *J* = 13.3, 5.1 Hz, 1H), 4.62 - 4.20 (m, 3H), 3.81 (t, *J* = 5.3 Hz, 2H), 3.70 (t, *J* = 6.6 Hz, 6H), 3.59 - 3.41 (m, 3H), 3.42 (d, *J* = 11.5 Hz, 1H), 3.11 (d, *J* = 6.3 Hz, 2H), 2.99 - 2.88 (m, 2H), 2.87 - 2.71 (m, 4H), 2.66 - 2.57 (m, 1H), 2.44 (dd, *J* = 13.2, 4.4 Hz, 1H), 2.23 - 2.06 (m, 2H), 2.06 - 1.92 (m, 1H), 1.84 (d, *J* = 11.9 Hz, 2H), 1.54 - 1.38 (m, 2H), 1.36 - 1.24 (m, 3H). LCMS [M+H]⁺ = 917.8 |
| **1142** | 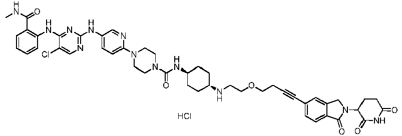 |
| | ¹H NMR (400 MHz, DMSO-*d*₆) δ 11.84 (s, 1H), 11.00 (s, 1H), 9.66 (s, 1H), 8.79 (d, *J* = 45.6 Hz, 4H), 8.42 (s, 1H), 8.26 (s, 1H), 8.14 (s, 1H),7.79 (d, *J* = 7.9 Hz, 1H), 7.70 (d, *J* = 7.9 Hz, 1H), 7.65 (s, 1H), 7.53 (d, *J* = 7.8 Hz, 2H), 7.24 (d, *J=* 52.1 Hz, 1H), 7.15 (d, *J* = 7.5 Hz, 1H), 6.44 (d, *J* = 7.1 Hz, 1H), 5.11 (dd, *J* = 13.3, 5.1 Hz, 1H), 4.62 - 4.19 (m, 2H), 3.77 (d, *J* = 5.4 Hz, 2H), 3.69 (t, *J* = 6.7 Hz, 2H), 3.60 (s, 4H), 3.15 (d, *J* = 8.2 Hz, 2H), 3.02 - 2.85 (m, 3H), 2.84 - 2.71 (m, 4H), 2.68 - 2.56 (m, 2H), 2.39 (qd, *J* = 14.3, 13.7, 5.1 Hz, 1H), 2.10 (d, *J* = 12.3 Hz, 2H), 2.05 - 1.92 (m, 2H), 1.85 (d, *J* = 12.0 Hz, 2H), 1.58 - 1.37 (m, 2H), 1.34 - 1.22 (m, 4H). LCMS [M+H]⁺ = 918.8 |
| **1146** | 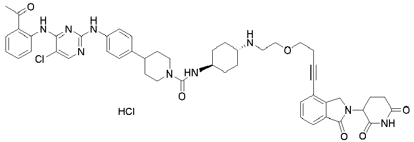 |
| | ¹H NMR (400 MHz, DMSO-*d*₆)δ11.84 (s, 1H), 11.02 (s, 1H), 9.64 (s, 1H), 8.85 (s, 3H), 8.30 (s, 1H), 8.13 (d, *J* = 7.9 Hz, 1H), 7.73 (d, *J* = 7.5 Hz, 1H), 7.70 - 7.38 (m, 5H), 7.31 - 7.10 (m, 3H), 6.23 (s, 1H), 5.17 (dd, *J* = 13.1, 4.8 Hz, 1H), 4.47 (d, *J* = 8.0 Hz, 1H), 4.32 (d, *J* = 7.7 Hz, 1H), 4.23-4.15 (m, 2H), 3.91 - 3.60 (m, 2H), 3.10 (s, 2H), 3.08 (d, *J* = 8.4 Hz, 2H), 3.02 - 2.5(m, 11H), 2.45 (s, 1H), 2.04 (dd, *J* = 2.1, 8.1 Hz, 3H), 1.79 (dd, *J* = 5.2, 11.2 Hz, 2H), 1.73 (d, *J* = 12.0 Hz, 2H), 1.84 - 1.36 (m, 4H), 1.33 - 1.13 (m, 3H),LCMS [M+H]⁺ = 871.8 |
| **1147** | 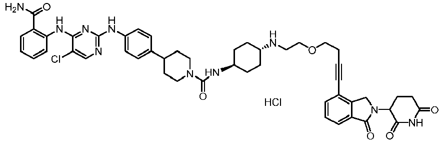 |
| | ¹H NMR (400 MHz, DMSO-*d*₆)δ11.92 (s, 1H), 11.02 (s, 1H), 9.46 (s, 1H), 8.81-8.65 (m, 3H), 8.27 (d, *J* = 3.3 Hz, 2H), 7.86 - 7.49 (m, 9H), 7.14 (t, *J* = 8.7 Hz, 1H),6.23 (s, 1H), 5.17 (dd, *J* = 13.1, 4.8 Hz, 1H), 4.47 (d, *J* = 8.0 Hz, 1H), 4.32 (d, *J* = 7.7 Hz, 1H), 4.23-4.15 (m, 2H), 3.91 - 3.60 (m, 4H), 3.45 - 3.05 (m, 4H), 3.11 - 2.51 (m, 8H), 2.03 (d, *J* = 6.3 Hz, 4H), 2.24 - 1.86 (m, 2H), 1.84 (s, 2H), 1.73 (d, *J* = 12.0 Hz, 2H), 1.39 (dd, *J* = 4.8, 6.1 Hz, 3H), 1.25 (dt, *J* = 15.5, 8.4 Hz, 2H).LCMS [M+H]⁺ = 901.8 |
| **1149** | 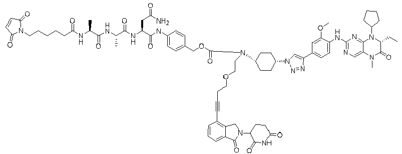 |
| | LC-MS: [M+H]⁺ =1483. |
| **1151** | 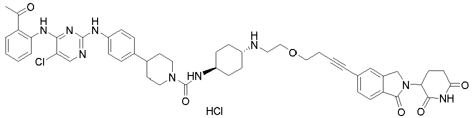 |
| | ¹H NMR (400 MHz, DMSO-*d*₆)δ 11.82 (s, 1H), 11.00 (s, 1H), 9.52 (s, 1H), 8.95 (s, 1H), 8.69 (s,2H), 8.28 (s, 1H), 8.13 (d, *J* = 8.0 Hz, 1H), 7.76 - 7.65 (m, 2H), 7.65 - 7.44 (m, 4H), 7.28 - 7.08 (m, 3H), 6.24 (s, 1H), 5.11 (dd, *J* = 13.1, 5.2 Hz, 1H), 4.45 (d, *J* = 6.7 Hz, 1H), 4.33 (d, *J* = 7.4 Hz, 1H), 3.88- 3.69 (m, 4H), 2.88 (ddd, *J* = 6.7, 7.9, 9.2 Hz, 5H), 2.78 (dd, *J* = 7.9, 1.3 Hz, 3), 2.83 - 1.52 (m, 3H), 2.39 (d, *J* = 13.3 Hz, 8H), 2.31 (d, *J* = 9.9 Hz, 3H), 2.11 (s, 1H), 2.44 - 1.52 (m, 3H), 2.11 - 1.60 (m, 5H), 1.49 - 1.31 (m, 7H). LCMS [M+H]⁺ = 900.8 |
| **1152** | 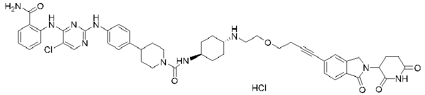 |
| | ¹H NMR (400 MHz, DMSO-*d*₆)δ 12.00 (s, 1H), 11.07 (s, 1H), 9.55 (s, 1H), 8.85 (s, 1H), 8.79 (s,2H), 8.28 (s, 1H), 8.13 (d, *J* = 8.0 Hz, 1H), 7.76 - 7.65 (m, 5H), 7.65 - 7.44 (m, 5H), 7.28 - 7.08 (m, 3H), 6.24 (s, 1H), 5.11 (dd, *J* = 13.1, 5.2 Hz, 1H), 4.45 (d, *J* = 6.7 Hz, 1H), 4.33 (d, *J* = 7.4 Hz, 1H), 3.88- 3.69 (m, 4H), 3.27 (s, 2H), 3.09 - 2.51 (m, 9H), 2.36 (d, *J* = 6.7 Hz, 1H), 2.50 - 2.03 (m, 4H), 1.96 (dd, *J* = 5.3, 3.3 Hz,2H), 1.65 (d, *J* = 10.1 Hz, 2H), 1.48 (d, *J* = 11.7 Hz, 3H), 1.38 - 1.21 (m, 2H). LCMS \|M+H]⁺ = 900.8. |
| **1168** | 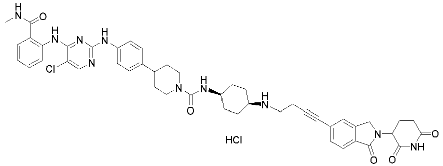 |
| | ¹H NMR (400 MHz, DMSO-*d*₆)δ11.75 (s, 1H), 11.00 (s, 1H), 9.57 (s,1H), 9.01 - 8.77 (m, 2H), 8.71 (d, *J* = 7.4 Hz, 2H), 8.22 (s 1H), 7.82 - 7.68 (m, 3H), 7.68 - 7.51 (m, 3H), 7.47 (t, *J* = 7.9 Hz, 1H), 7.17 (dd, *J* = 7.5, 5.4 Hz, 3H), 5.93 (s, 1H), 5.11 (dd, *J* = 13.3, 5.1 Hz, 1H), 4.46 (d, *J* = 7.7 Hz, 1H), 4.33 (d, *J* = 7.7 Hz, 1H),3.68 (s, 1H), 3.19 (d, *J* = 7.7 Hz, 3H), 3.21 - 2.90 (m, 3H), 2.90 - 2.49 (m, 7H), 2.37 (ddd, *J* = 6.2, 10.5, 7.3 Hz, 2H), 2.04 - 1.99 (m, 2H), 1.83 (dd, *J* = 2.7, 9.1 Hz, 1H), 1.65 (d, *J* = 4.4 Hz, 8H), 1.57 - 1.27 (m, 4H).LCMS [M+H]⁺ = 871.8. |
| **1169** | 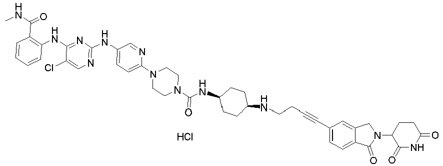 |
| | ¹H NMR (400 MHz, DMSO-*d*₆)δ 12.25 (s, 1H), 11.00 (s, 1H), 9.27 - 8.76 (m, 4H), 8.46 (d, *J* = 8.1 Hz, 1H), 8.15 - 7.99 (m, 1H), 7.98 - 7.82 (m, 3H), 7.81 - 7.76 (m, 3H), 7.76 - 7.69 (m, 3H), 7.32 - 6.97 (m, 2H), 6.13 (s, 1H), 5.12 (dd, *J* = 13.3, 5.0 Hz, 1H), 4.46 (d, *J* = 7.6 Hz, 1H), 4.34 (d, *J* = 7.6 Hz, 1H), 3.68 - 3.27 (m, 8H), 3.08 (dd, *J* = 9.4, 7.1 Hz, 4H), 2.96 - 2.84 (m, 3H), 2.96 - 2.84 (m, 3H), 2.96 - 2.71 (m, 3H), 2.69 (d, *J* = 13.2 Hz, 1H), 2.60 (d, *J* = 7.4 Hz, 2H), 2.27 (d, *J* = 8.6 Hz, 2H), 2.02 - 1.68 (m, 6H), 1.68 - 1.21 (m, 3H). LCMS [M+H]⁺ = 909.8 |
| **1170** | 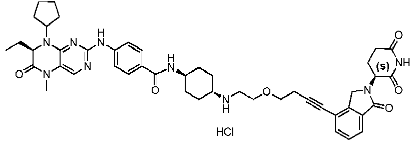 |
| | ¹H NMR (400 MHz, DMSO-*d*₆) δ 11.02 (s, 1H), 10.45 (s, 1H), 9.00 (s, 2H), 8.22 (d, *J* = 7.8 Hz, 1H), 7.94 - 7.82 (m, 2H), 7.73 (d, *J* = 7.5 Hz, 1H), 7.65 (dd, *J* = 11.3, 7.9 Hz, 2H), 7.53 (t, *J* = 7.6 Hz, 1H), 5.17 (dd, *J* = 13.3, 5.1 Hz, 2H), 4.62 - 4.40 (m, 2H), 4.42 - 4.23 (m, 3H), 3.91 - 3.76 (m, 3H), 3.71 (t, *J* = 6.7 Hz, 3H), 3.23 (s, 2H), 3.15 (q, *J* = 5.6 Hz, 1H), 3.04 (s, 1H), 2.96 - 2.88 (m, 2H), 2.81 (t, *J* = 6.7 Hz, 1H), 2.64 - 2.57 (m, 1H), 2.45 (d, *J* = 4.6 Hz, 1H), 2.15 (d, *J* = 11.7 Hz, 2H), 2.07 - 1.80 (m, 7H), 1.65 (d, *J* = 7.8 Hz, 2H), 1.56 - 1.32 (m, 6H), 0.77 (t, *J* = 7.4 Hz, 3H). LCMS [M+H]⁺ = 830.8 |
| **1172** | 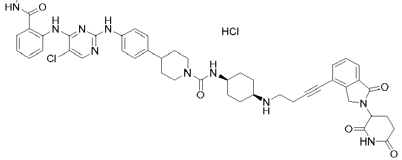 |
| | LCMS [M/2+H]⁺ = 436.5. ¹H NMR: NA |
| **1173** | 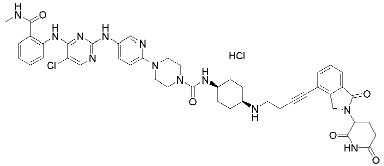 |
| | ¹H NMR (400 MHz, DMSO-*d*₆) δ 12.24 (s, 1H), 11.30 (s, 1H), 11.02 (s, 1H), 9.06 (s, 2H), 8.86 (dd, *J* = 21.1, 6.6 Hz, 2H), 8.38 (s, 1H), 8.00 (s, 1H), 7.92 (d, *J* = 2.8 Hz, 1H), 7.84 (dd, *J* = 7.9, 1.6 Hz, 1H), 7.76 (d, *J* = 7.6 Hz, 1H), 7.71 (d, *J* = 7.5 Hz, 1H), 7.58 (dt, *J* = 15.3, 8.0 Hz, 3H), 7.25 (t, *J* = 7.5 Hz, 1H), 6.11 (d, *J* = 4.5 Hz, 1H), 5.17 (dd, *J* = 13.3, 5.1 Hz, 1H), 4.59 - 4.50 (m, 1H), 4.36 (d, *J* = 17.8 Hz, 1H), 3.73 - 3.66 (m, 2H), 3.53 (t, *J* = 5.1 Hz, 4H), 3.15 (t, *J* = 5.0 Hz, 4H), 2.82 (d, *J* = 4.4 Hz, 3H), 2.58 (s, 1H), 2.45 (d, *J* = 4.4 Hz, 1H), 2.00 (dq, *J* = 13.4, 6.2 Hz, 4H), 1.84 (d, *J* = 8.0 Hz, 6H), 1.59 - 1.43 (m, 4H). LCMS [M/2+H]⁺ = 437.5. |
| **1174** | 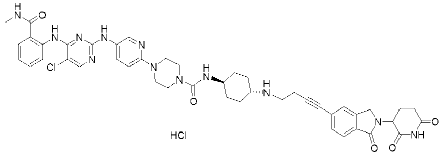 |
| | ¹H NMR (400 MHz, DMSO-*d*₆)δ 12.32 (s, 1H), 11.00 (s, 1H), 9.27 - 8.76 (m, 2H), 8.46 (d, *J* = 8.1 Hz, 1H), 8.15 - 7.99 (m, 1H), 7.98 - 7.82 (m, 2H), 7.81 - 7.76 (m, 3H), 7.76 - 7.69 (m, 3H), 6.82 - 6.67 (m, 1H), 6.43 (s, 1H), 5.12 (dd, *J* = 13.3, 5.0 Hz, 1H), 4.46 (d, *J* = 7.7 Hz, 1H), 4.34 (d, *J* = 7.7 Hz, 1H), 3.68 - 3.57 (m, 9H), 3.08 (dd, *J* = 9.4, 7.1 Hz, 2H), 2.96 - 2.84 (m, 3H), 2.81 - 2.71 (m, 1H), 2.27 (d, *J* = 8.6 Hz, 2H), 2.02 - 1.68 (m, 4H), 1.68 - 1.21 (m, 11H). LCMS [M+H]⁺ = 873.8 |
| **1179** | 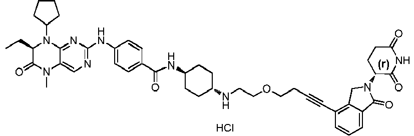 |
| | ¹H NMR (400 MHz, DMSO-*d*₆) δ 11.02 (s, 1H), 10.53 (s, 1H), 9.02 (s, 2H), 8.24 (d, *J* = 7.6 Hz, 1H), 7.92 - 7.82 (m, 3H), 7.73 (d, *J* = 7.5 Hz, 1H), 7.64 (dd, *J* = 14.5, 8.0 Hz, 3H), 7.54 (t, *J* = 7.6 Hz, 1H), 5.17 (dd, *J* = 13.3, 5.1 Hz, 1H), 4.63 - 4.18 (m, 4H), 3.81 (t, *J* = 5.3 Hz, 2H), 3.71 (t, *J* = 6.7 Hz, 3H), 3.23 (s, 2H), 3.15 (t, *J* = 6.1 Hz, 2H), 3.06 - 3.00 (m, 1H), 2.93 (ddd, *J* = 17.8, 13.6, 5.3 Hz, 1H), 2.81 (t, *J* = 6.6 Hz, 2H), 2.64 - 2.55 (m, 1H), 2.48 - 2.39 (m, 1H), 2.21 - 2.10 (m, 2H), 2.08 - 1.96 (m, 2H), 1.95 - 1.74 (m, 6H), 1.70 - 1.29 (m, 8H), 0.77 (t, *J* = 7.4 Hz, 3H). LCMS [M+H]⁺ = 830.9 |
| **1180** | 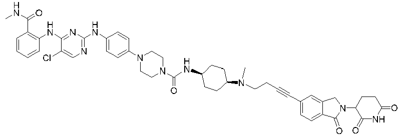 |
| | ¹H NMR (400 MHz, DMSO-*d*₆) δ 12.05 (d, *J* = 360.4 Hz, 1H), 10.99 (s, 1H), 9.23 (s, 1H), 8.96 - 8.82 (m, 1H), 8.75 (d, *J* = 5.1 Hz, 1H), 8.20 (d, *J* = 28.3 Hz, 1H), 7.79 - 7.44 (m, 6H), 7.24 (d, *J* = 8.7 Hz, 1H), 7.16 - 7.09 (m, 1H), 6.98 (dd, *J* = 50.4, 8.7 Hz, 2H), 6.12 (s, 1H), 5.11 (dd, *J* = 13.3, 5.1 Hz, 1H), 4.53 - 4.27 (m, 2H), 3.80 (s, 1H), 3.49 (s, 5H), 3.17 (s, 2H), 3.03 (s, 4H), 2.80 (t, *J* = 4.8 Hz, 5H), 2.59 (d, *J* = 16.8 Hz, 2H), 2.38 (dd, *J* = 12.8, 4.4 Hz, 2H), 2.28 (s, 3H), 2.00 (q, *J* = 7.3 Hz, 3H), 1.75 (s, 5H), 1.48 (s, 2H).LCMS [M+H]⁺ = 886.7 |
| **1181** | 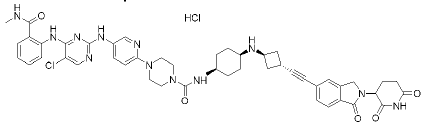 |
| | ¹H NMR (400 MHz, DMSO-*d*₆) δ 12.23 (s, 1H), 11.00 (s, 1H), 9.27 (s,2H), 8.99 - 8.81 (m, 2H), 8.38 (s, 1H), 8.05 - 7.91 (m, 2H), 7.84 (d, *J* = 7.9 Hz, 1H), 7.81 - 7.48 (m, 5H), 7.24 (t, *J* = 7.6 Hz, 1H), 6.12 (d, *J* = 4.5 Hz, 1H), 5.11 (dd, *J* = 13.3, 5.1 Hz, 1H), 4.50 - 4.29 (m, 2H), 3.71 (d, *J* = 37.9 Hz, 2H), 3.46 - 3.34 (m, 4H), 3.21 - 3.00 (m, 6H), 2.82 (d, *J* = 4.5 Hz, 3H), 2.73 - 2.58 (m, 3H), 2.44 - 2.31 (m, 1H), 2.01 (dd, *J* = 10.3, 4.8 Hz, 2H), 1.90 - 1.68 (m, 6H), 1.57 - 1.46 (m, 2H), 1.25 - 1.16 (m, 2H). LC-MS: [M+H] ⁺ = 900 |
| **1182** | 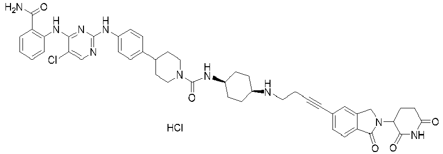 |
| | ¹H NMR (400 MHz, DMSO-*d*₆)δ 12.00 (s, 1H), δ 11.00 (s, 1H), 9.55 (s, 1H), 8.85 (s, 1H), 8.79 (s,2H), 8.28 (s, 1H), 8.13 (d, *J* = 8.0 Hz, 1H), 7.76 - 7.65 (m, 5H), 7.65 - 7.44 (m, 5H), 7.28 - 7.08 (m, 3H), 6.24 (s, 1H), 5.11 (dd, *J* = 13.1, 5.2 Hz, 1H), 4.45 (d, *J* = 6.7 Hz, 1H), 4.33 (d, *J* = 7.4 Hz, 1H), 3.88- 3.69 (m, 4H), 3.27 (s, 2H), 3.09 - 2.51 (m, 9H), 2.36 (d, *J* = 6.7 Hz, 1H), 2.50 - 2.03 (m, 4H), 1.96 (dd, *J* = 5.3, 3.3 Hz,2H), 1.65 (d, *J* = 10.1 Hz, 2H), 1.48 (d, *J* = 11.7 Hz, 3H), 1.38 - 1.21 (m, 2H). LCMS [M+H]⁺ = 858.3 |
| **1183** | 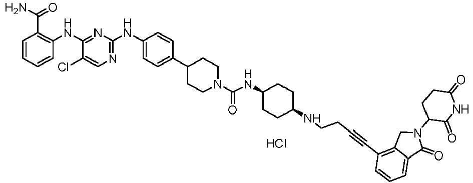 |
| | LCMS [M+H]⁺ =857.3 |
| **1184** | 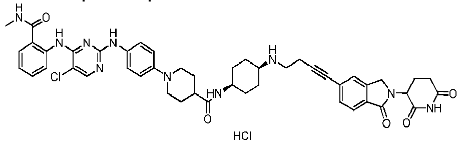 |
| | ¹H NMR (400 MHz, DMSO-*d*₆) δ 11.75 (s, 1H), 11.00 (s, 1H), 9.83 (s, 1H), 9.23 (s, 2H), 8.84 (d, *J* = 4.8 Hz, 1H), 8.71 (d, *J* = 8.4 Hz, 1H), 8.28 (s, 1H), 7.90 (s, 1H), 7.84 - 7.78 (m, 2H), 7.74 - 7.68 (m, 3H), 7.56 (dd, *J* = 16.5, 8.0 Hz, 2H), 7.18 (t, *J* = 7.5 Hz, 1H), 5.11 (dd, *J* = 13.3, 5.1 Hz, 1H), 4.54 - 4.27 (m, 2H), 3.82 - 3.73 (m, 3H), 3.39 (s, 2H), 3.19 (d, *J* = 16.2 Hz, 3H), 2.99 (t, *J* = 7.5 Hz, 2H), 2.81 (d, *J* = 4.5 Hz, 2H), 2.67 (s, 2H), 2.45 - 2.27 (m, 1H), 2.18 (s, 2H), 2.10 - 1.93 (m, 3H), 1.89 - 1.70 (m, 6H), 1.63 - 1.37 (m, 3H), 1.20 (t, *J* = 7.3 Hz, 1H). LCMS [M+H]⁺ = 871.7 |
| **1186** | 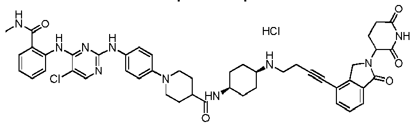 |
| | LCMS [M+H]⁺ = 871.8 |
| **1187** | 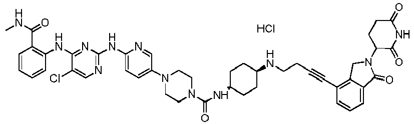 |
| | ¹H NMR (400 MHz, DMSO-*d*₆) δ 12.29 (s, 1H), 11.57 (s, 1H), 11.02 (s, 1H), 9.30 (s, 2H), 9.00 - 8.91 (m, 1H), 8.86 (t, *J* = 7.8 Hz, 2H), 8.52 - 8.23 (m, 2H), 8.07 (d, *J* = 9.6 Hz, 1H), 8.02 - 7.87 (m, 2H), 7.86 (d, *J* = 7.9 Hz, 1H), 7.73 (dd, *J* = 15.2, 7.6 Hz, 2H), 7.59 (ddd, *J* = 26.4, 12.5, 6.5 Hz, 2H), 7.26 (d, *J* = 6.4 Hz, 1H), 6.13 (s, 1H), 5.17 (dd, *J* = 13.3, 5.2 Hz, 1H), 4.62 - 4.25 (m, 3H), 3.71 (s, 2H), 3.14 (dt, *J* = 15.3, 5.0 Hz, 5H), 3.05 - 2.91 (m, 5H), 2.82 (d, *J* = 4.4 Hz, 3H), 2.70 - 2.56 (m, 3H), 2.31 (d, *J* = 20.3 Hz, 1H), 2.07 - 1.95 (m, 2H), 1.88 (d, *J* = 15.3 Hz, 5H), 1.50 (s, 3H). LCMS [M+H]⁺ = 873.8 |
| **1188** | 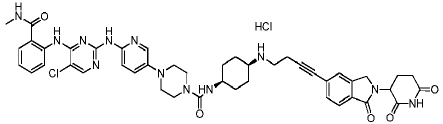 |
| | ¹H NMR (400 MHz, DMSO-*d*₆) δ 12.35 (s, 1H), 11.79 (s, 1H), 11.00 (s, 1H), 9.29 (s, 2H), 8.97 (t, *J* = 4.6 Hz, 1H), 8.84 (d, *J* = 8.4 Hz, 1H), 8.41 (s, 1H), 8.13 (dd, *J* = 9.6, 2.9 Hz, 1H), 7.98 - 7.82 (m, 2H), 7.80 - 7.68 (m, 2H), 7.61 (dd, *J* = 10.0, 7.6 Hz, 3H), 7.26 (t, *J* = 7.6 Hz, 1H), 6.15 (s, 1H), 5.11 (dd, *J* = 13.3, 5.1 Hz, 1H), 4.55 - 4.17 (m, 2H), 3.51 (d, *J* = 14.4 Hz, 4H), 3.18 (q, *J* = 6.3, 5.0 Hz, 8H), 3.00 (t, *J* = 7.4 Hz, 2H), 2.95 - 2.86 (m, 1H), 2.82 (d, *J* = 4.5 Hz, 3H), 2.65 - 2.55 (m, 1H), 2.39 (dd, *J* = 13.3, 4.7 Hz, 1H), 2.01 (tt, *J* = 10.5, 4.9 Hz, 2H), 1.87 (d, *J* = 15.6 Hz, 5H), 1.51 (dt, *J* = 14.7, 7.5 Hz, 2H). LCMS [M+H]⁺ = 873.8 |
| **1189** | 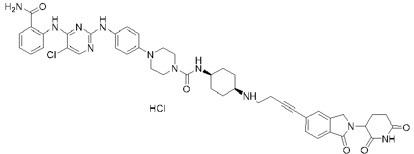 |
| | LCMS [M+H]⁺ =859.4 |
| **1190** | 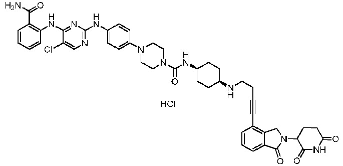 |
| | LCMS [M+H]⁺ =859.4 |
| **1194** | 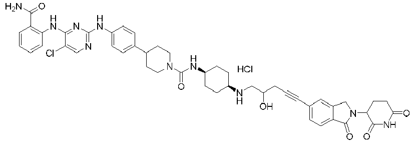 |
| | LCMS [M+H]⁺ = 887.8 |
| **1198** | 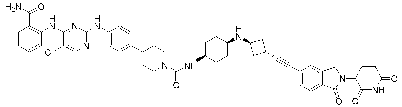 |
| | LCMS [M+H]⁺ = 883.7 |
| **1199** | 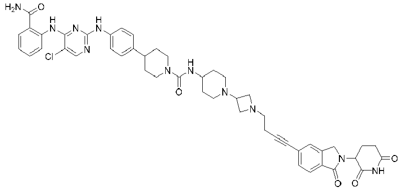 |
| | ¹H NMR (400 MHz, *d6-DMSO*) δ11.85 (s, 1H), 10.96 (s, 1H), 9.41 (d, *J* = 14.8 Hz, 1H), 8.79 (d, *J* = 7.6 Hz, 1H), 8.40 - 8.20 (m, 1H), 7.85 - 7.65 (m, 3H), 7.65 - 7.51 (m, 3H), 7.48 (t, *J* = 8.5 Hz, 2H), 7.32 - 7.07 (m, 4H), 6.17 (d, *J* = 7.6 Hz, 1H), 5.17 - 4.99 (m, 1H), 4.44 (d, *J* = 17.6 Hz, 1H), 4.32 (d, *J* = 17.6 Hz, 1H), 4.10 (d, *J* = 13.7 Hz, 2H), 3.61 - 3.47 (m, 1H), 3.38 (d, *J* = 4.51 Hz, 1H), 3.25 (s, 2H), 2.96 - 2.59 (m, 6H), 2.70 - 2.56 (m, 7H), 2.54 - 2.17 (m, 3H), 2.17 - 1.78 (m, 6H), 1.79 - 1.43 (m, 5H), 0.85 (t, *J* = 6.8 Hz, 2H). LCMS [M+H]⁺ =898.9 |
| **1203** | 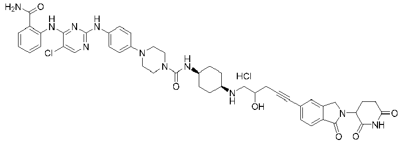 |
| | LCMS [M+H]⁺ = 888.8 |
| **1205** | 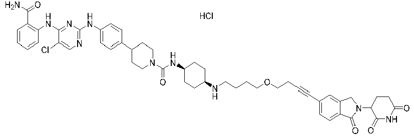 |
| | ¹H NMR (400 MHz, DMSO-*d*₆) δ 11.91 (s, 1H), 11.00 (s, 1H), 9.44 (s, 1H), 8.84 - 8.67 (m, 1H), 8.35 (d, J = 36.4 Hz, 2H), 8.21 (s, 1H), 7.85 - 7.74 (m, 1H), 7.70 (d, J = 7.9 Hz, 1H), 7.63 (s, 1H), 7.57 (d, J = 8.2 Hz, 1H), 7.53 - 7.45 (m, 1H), 7.22 - 7.09 (m, 2H), 5.91 (d, J = 4.1 Hz, 1H), 5.11 (dd, J = 13.3, 5.1 Hz, 1H), 4.44 (d, J = 17.5 Hz, 1H), 4.32 (d, J = 17.5 Hz, 1H), 4.14 (d, J = 12.6 Hz, 1H), 3.72 - 3.54 (m, 3H), 3.48 (t, J = 6.0 Hz, 2H), 3.07 - 2.85 (m, 4H), 2.81 - 2.68 (m, 3H), 2.59 (m, 4H), 2.47 - 2.35 (m, 2H), 1.99 (m, 2H), 1.89 - 1.40 (m, 12H), 1.35 - 1.26 (m, 2H). LCMS [M/2+H]+ = 465.1. |
| **1206** | 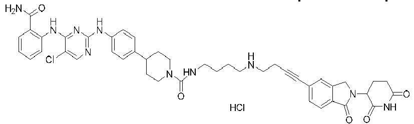 |
| | LCMS [M+H]⁺ = 831.8 |
| **1207** | 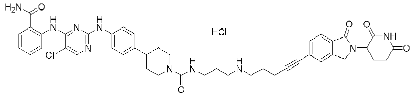 |
| | ¹H NMR (400 MHz, DMSO-*d*₆) δ 11.92 (s, 1H), 11.00 (d, *J*= 2.7 Hz, 1H), 9.45 (s, 1H), 8.72 (d, *J* = 48.3 Hz, 3H), 8.31 (s, 1H), 8.21 (s, 1H), 7.83 (dd, *J* = 7.9, 1.6 Hz, 1H), 7.80 - 7.68 (m, 2H), 7.65 (d, *J* = 6.2 Hz, 1H), 7.59 - 7.46 (m, 3H), 7.18 - 7.10 (m, 2H), 6.79 (s, 1H), 5.11 (dt, *J* = 13.3, 4.6 Hz, 1H), 4.44 (dd, *J* = 17.6, 9.3 Hz, 1H), 4.31 (dd, *J* = 17.6, 6.2 Hz, 1H), 4.10 (d, *J* = 12.7 Hz, 1H), 3.16 (d, *J* = 5.5 Hz, 2H), 3.12 - 2.81 (m, 8H), 2.76 (t, *J* = 12.5 Hz, 2H), 2.68 - 2.57 (m, 4H), 2.38 (dt, *J* = 13.1, 6.2 Hz, 1H), 1.96 (dq, *J* = 28.4, 7.5 Hz, 4H), 1.76 (dd, *J* = 15.5, 8.9 Hz, 4H), 1.56 - 1.40 (m, 2H). LCMS [M/2+H]⁺ = 416.5. |
| **1208** | 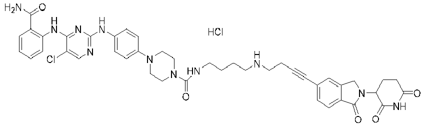 |
| | LCMS [M+H]⁺ = 832.8 |
| **1226** | 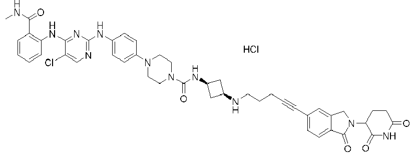 |
| | ¹H NMR (400 MHz, DMSO-*d*₆) δ 11.92 (s, 1H), 11.00 (s, 1H), 9.85 (s, 1H), 9.34 (s, 2H), 8.84 (d, *J*= 4.7 Hz, 1H), 8.66 (s, 1H), 8.28 (s, 1H), 7.80 (dd, *J* = 8.0, 1.6 Hz, 1H), 7.71 (d, *J* = 7.9 Hz, 1H), 7.68 - 7.32 (m, 6H), 7.20 (m, 2H), 5.11 (dd, *J* = 13.2, 5.1 Hz, 1H), 4.45 (d, *J* = 17.5 Hz, 1H), 4.33 (d, *J* = 17.5 Hz, 1H), 3.92 (m, 2H), 3.44 (d, *J* = 6.9 Hz, 4H), 3.32 (s, 4H), 2.95 (m, 3H), 2.81 (d, *J* = 4.5 Hz, 3H), 2.66 - 2.56 (m, 4H), 2.37 (m, 1H), 2.25 (d, *J* = 10.2 Hz, 2H), 2.09 - 1.88 (m, 4H). LCMS [M/2+H]⁺ = 430. |
| **1232** | 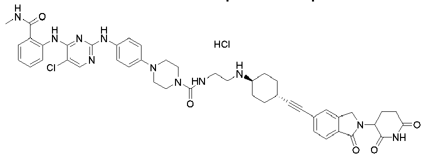 |
| | LCMS [M+H]⁺ = 873.3 |

### Synthesis of Compound UB-180938

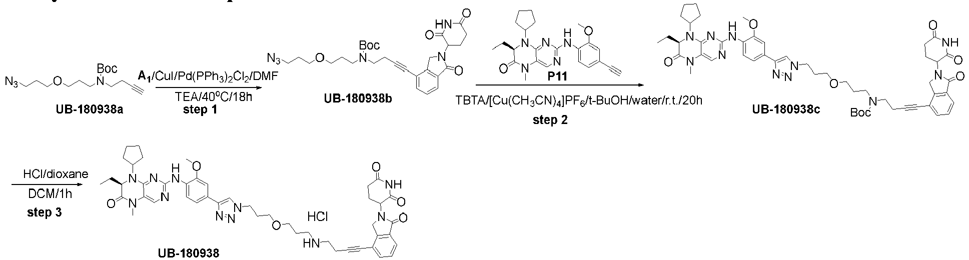

### Step 1: UB-180938 (V2111-139)

Compound **UB-180938a** (450 mg, 1.45 mmol), **A₁** (536 mg, 1.45 mmol), Pd(PPh₃)₂Cl₂ (100 mg, 0.15 mmol), CuI (68 mg, 0.35 mmol), and TEA (146 mg, 1.45 mmol) were dissolved in anhydrous DMF (5 mL), and the mixture was reacted at 40°C for overnight under N₂ protection. The reaction solution was dried by rotary dryer to remove solvent and isolated by column chromatography (methanol/dichloromethane = 1/10) to obtain target product **UB-180938b** (200 mg) as a yellow solid. LCMS [M+H]⁺ = 553.3

### Step 2: UB-180938c (V2240-005)

Compound **P11** (20 mg, 0.05 mmol), **and UB-180938b** (40 mg, 0.07 mmol) were dissolved in t-BuOH (5 mL) and water (2.5 mL), then TBTA (3 mg) and [Cu(CH3CN)4]PF6 (4 mg) were added. The mixture was reacted at room temperature overnight. Water (15 mL) was added, and the mixture was extracted with EtOAc (10 mL*2), the organic phases were combined, concentrated, and then isolated by column chromatography (methanol/dichloromethane=1/10) to obtain target product **UB-180938c** (20 mg, yield 41%) as a yellow solid. LCMS [M+H]⁺ = 958.3

### Step 3: UB-180938 (V2240-008)

Compound **UB-180938c(20** mg, 0.02 mmol) was dissolved in DCM (3 mL), 4M HCl/dioxane (0.3 mL) was added, and the mixture was reacted at room temperature for 1 hour. The supernatant was removed, and the solid was dried by rotary dryer to obtain target product **UB-180938** (24 mg, yield 85%) as a yellow solid. LCMS [M+H]⁺ = 857 ¹H NMR (400 MHz, DMSO-*d*₆) δ 11.01 (s, 1H), 9.51 (s, 1H), 9.20 (s, 1H), 9.04 (s, 1H), 8.97 - 8.87 (m, 1H), 7.84 - 7.60 (m, 5H), 7.57 - 7.45 (m, 2H), 5.16 (m, 1H), 4.97 (m, 1H), 4.53 (m, 3H), 4.32 (m, 1H), 4.06 (m, 2H), 3.94 - 3.89 (m, 3H), 3.83 (m, 2H), 3.21 (s, 3H), 2.94 (m, 2H),2.92 (m, 3H), 1.86 (m, 8H), 1.61 - 1.53 (m, 2H), 1.42 (m, 4H), 1.26 - 1.03 (m, 5H), 0.74 (t, *J* = 7.3 Hz, 3H).

### Synthesis method of Compound UB-180949

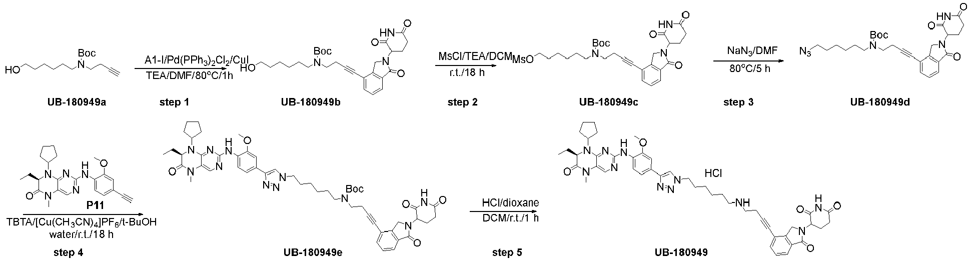

### Step 1: UB-180949b (V2240-032)

Compound **UB-180949a** (269 mg, 1 mmol) and **A1-I** (185 mg, 0.5 mmol), Pd(PPh₃)₂Cl₂ (35 mg, 0.05 mmol), CuI (38 mg,0.2 mmol), and TEA (101 mg, 1 mmol) were dissolved in anhydrous DMF (4 mL), then the mixture was reacted at 80°C for 1 hour under N₂ protection. Water (50 mL) was added, the mixture was extracted with EtOAc (20 mL), and the organic phases were combined and concentrated. The mixture was isolated by column chromatography (methanol/dichloromethane = 1/10) to obtain target product **UB-180949b** (80 mg, yield 31%) as a yellow solid. LCMS [M+H]⁺ = 512 ¹H NMR (400 MHz, DMSO-*d*₆) δ 11.01 (s, 1H), 7.72 (dd, *J=* 7.6, 1.1 Hz, 1H), 7.63 (dd, *J* = 7.7, 1.1 Hz, 1H), 7.53 (t, *J* = 7.6 Hz, 1H), 5.15 (dd, *J* = 13.3, 5.1 Hz, 1H), 4.45 (d, *J* = 17.8 Hz, 1H), 4.32 (t, *J* = 5.0 Hz, 2H), 3.41 - 3.34 (m, 4H), 3.20 (t, *J* = 7.3 Hz, 2H), 2.99 - 2.89 (m, 1H), 2.72 - 2.55 (m, 4H), 2.06 - 2.00 (m, 1H), 1.47 (m, 2H), 1.38 (d, *J=* 6.4 Hz, 9H), 1.31 - 1.19 (m, 6H).

### Step 2: UB-180949c (V2240-035)

Compound UB-**180949b** (500 mg, 0.98 mmol), and TEA (300 mg, 2.94 mmol) were dissolved in DCM (20 mL), MsCl (335 mg, 2.94 mmol) was added, and the mixture was reacted at room temperature for 2 hours. Water (5 mL) was added, and the mixture was extracted with DCM (15 mL), the organic phases were combined and concentrated, and isolated by column chromatography (methanol/dichloromethane=1/10) to obtain target product **UB-180949c** (400 mg, yield 69%) as a yellow solid. LCMS [M-56]⁺ = 534

¹H NMR (400 MHz, DMSO-*d*₆) δ 11.01 (s, 1H), 7.72 (dd, *J=* 7.6, 1.1 Hz, 1H), 7.63 (dd, *J* = 7.7, 1.1 Hz, 1H), 7.53 (t, *J* = 7.6 Hz, 1H), 5.16 (dd, *J* = 13.3, 5.1 Hz, 1H), 4.46 (d, *J* = 17.9 Hz, 1H), 4.30 (d, *J* = 17.7 Hz, 1H), 4.16 (t, *J* = 6.4 Hz, 2H), 3.40 (t, *J* = 6.9 Hz, 2H), 3.22 (d, *J=* 7.5 Hz, 2H), 3.15 (s, 3H), 2.93 (m, 1H), 2.69 (m, 2H), 2.60 (m, 1H), 2.44 (m, 1H), 2.02 (m, 1H), 1.63 (m, 2H), 1.50 (m, 2H), 1.38 (s, 9H), 1.29 (m, 4H).

### Step 3: UB-180949d (V2240-036)

Compound **UB-180949c** (400 mg, 0.68 mmol) and NaN₃ (132 mg, 2 mmol) were dissolved in DMF (10mL), and the mixture was reacted at 80°C for 5 hours. The reaction solution was poured to saturated brine (40 mL), then extracted with EtOAc (30 mL*2). The organic phase was then washed with saturated brine, dried over anhydrous sodium sulfate, filtered, and concentrated to obtain target product **UB-180949d** (400 mg) as colorless oil. LCMS [M+H]⁺ = 537

### Step 4: UB-180949e (V2240-038)

Compound **UB-180949d** (35 mg, 0.086 mmol), and **P11** (60 mg, 0.11 mmol) were dissolved in t-BuOH (5 mL) and water (2.5 mL), TBTA (8 mg) and [Cu(CH₃CN)₄]PF₆ (10 mg) were added, and the mixture was reacted at room temperature overnight. Water (15 mL) was added, the mixture was extracted with EtOAc (10 mL*2), and the organic phases were combined and concentrated. The reaction crude product was purified by preparative thin layer chromatography (dichloromethane /methanol =15/1) to obtain target product **UB-180949e** (28 mg, yield 34%) as a white solid. LCMS [M+H]⁺ = 942.5

### Step 5: UB-180949 (V2240-039)

Compound **UB-180949e** (28 mg, 0.029 mmol) was dissolved in DCM (3 mL), 4M HCl/dioxane (1.5 mL) was added, and the mixture was reacted at room temperature for 15 minutes. The reaction supernatant was removed, remaining solid was dried to obtain target product **UB-180949** (27 mg, yield 100%) as a white solid. LCMS [M+H]⁺ = 842.6

¹H NMR (400 MHz, DMSO-*d*₆) δ 13.46 - 12.78 (m, 1H), 11.02 (s, 1H), 9.71 (s, 1H), 9.35 (m, 2H), 8.74 (d, *J=* 1.2 Hz, 1H), 7.82 - 7.65 (m, 4H), 7.62 - 7.44 (m, 3H), 5.16 (dd, *J=* 13.3, 5.1 Hz, 1H), 4.58 - 4.46 (m, 2H), 4.44 - 4.31 (m, 3H), 4.08 (q, *J=* 8.9 Hz, 1H), 3.90 (s, 3H), 3.21 (s, 3H), 3.15 (q, *J* = 6.7 Hz, 2H), 2.94 (m, 5H), 2.64 - 2.58 (m, 1H), 2.44 (m, 1H), 2.07 - 1.96 (m, 2H), 1.94 - 1.74 (m, 7H), 1.67 (t, *J* = 7.6 Hz, 2H), 1.37 (m, 8H), 0.74 (t, *J* = 7.4 Hz, 3H).

### Synthesis of Compound UB-180969

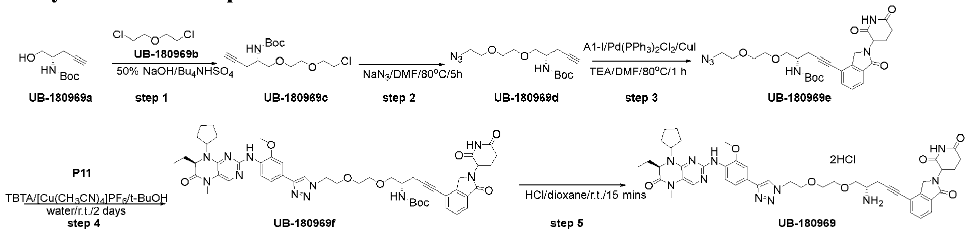

### Step 1: UB-180969c (V2240-053)

Compound **UB-180969a** (500 mg, 2.5 mmol), **and UB-180969b** (3 mL) were added with Bu₄NHSO₄ (1.7 g, 5 mmol) and 50% NaOH (4 mL), and the mixture was reacted at room temperature overnight. Water was added, and the mixture was extracted with DCM (30 mL*3). The organic phases were combined and concentrated, then isolated by column chromatography (petroleum ether/ethyl acetate= 1/1) to obtain target product **UB-180969c** (150 mg, yield 20%) as a colorless oil. LCMS [M-100]⁺ = 206 ¹H NMR (400 MHz, Chloroform-*d*) δ 3.89 (s, 1H), 3.78 - 3.74 (m, 2H), 3.72 - 3.60 (m, 8H), 3.52 (m, 1H), 2.50 (m, 2H), 1.99 (t, *J* = 2.6 Hz, 1H), 1.45 (s, 9H).

### Step 2: UB-180969d (V2240-055)

Compound **UB-180969c** (150 mg, 0.49 mmol) and NaN₃ (110 mg, 1.69 mmol) were dissolved in DMF (4mL), and the mixture was reacted at 80°C for 5 hours. The reaction solution was poured to saturated brine (40 mL), then extracted with EtOAc (30 mL*2). The organic phase was then washed with saturated brine, dried over anhydrous sodium sulfate, filtered, and concentrated to obtain target product **UB-180969d** (140 mg) as a colorless oil. LCMS [M+H]⁺ = NA

### Step 3: UB-180969e (V2240-058)

Compound **UB-180969d**(70 mg, 0.22 mmol), A1-I (75 mg, 0.2 mmol), Pd(PPh₃)₂Cl₂ (10 mg), CuI (14 mg), and TEA (2 drops) were dissolved in anhydrous DMF (2 mL), and the mixture was reacted at 80°C for 1 hour under N₂ protection. The reaction solution was concentrated and isolated by column chromatography (methanol/dichloromethane = 1/10) to obtain target product **UB-180969e** (60 mg, yield 54%) as a yellow solid. LCMS [M+H]⁺ = 555

### Step 4: UB-180969f (V2240-059)

Compound P11 (35 mg, 0.086 mmol), and **UB-180969e** (60 mg, 0.11 mmol) were dissolved in t-BuOH (5 mL) and water (2.5 mL), TBTA (8 mg) and [Cu(CH₃CN)₄]PF₆ (10 mg) were added, the mixture was reacted at room temperature for 2 days. Water (15 mL) was added, the mixture was extracted with EtOAc (10 mL*2), and the organic phases were combined and concentrated. The reaction crude product was purified by preparative thin layer chromatography (dichloromethane /methanol =20/1) to obtain target product **UB-180969f** (35 mg, yield 42%) as a white solid. LCMS [M+H]⁺ = 960.7

### Step 5: UB-180969 (V2240-063)

Compound **UB-180969f** (35 mg, 0.036 mmol) was dissolved in DCM (3 mL), 4M HCl/dioxane (1.5 mL) was added, and the mixture was reacted at room temperature for 15 minutes. The reaction supernatant was removed, remained solid was dried to obtain target product **UB-180969** (30 mg, yield 90%) as a light yellow solid. LCMS [M+H]⁺ = 860.5

¹H NMR (400 MHz, DMSO-*d*₆) δ 11.03 (s, 1H), 9.66 (s, 1H), 8.67 (s, 1H), 8.41 (d, *J* = 5.6 Hz, 3H), 7.80 - 7.65 (m, 4H), 7.59 - 7.44 (m, 3H), 5.16 (dd, *J=* 13.4, 5.2 Hz, 1H), 4.59 (t, *J* = 5.2 Hz, 2H), 4.54 - 4.45 (m, 2H), 4.35 (m, 1H), 4.06 (m, 1H), 3.90 (m, 5H), 3.69 (m, 3H), 3.61 (s, 3H), 3.21 (s, 3H), 2.98 - 2.83 (m, 3H), 2.64 - 2.57 (m, 1H), 2.43 - 2.35 (m, 1H), 1.88 (m, 8H), 1.49 - 1.32 (m, 4H), 0.74 (t, *J=* 7.4 Hz, 3H).

### Synthesis of Compound UB-180977

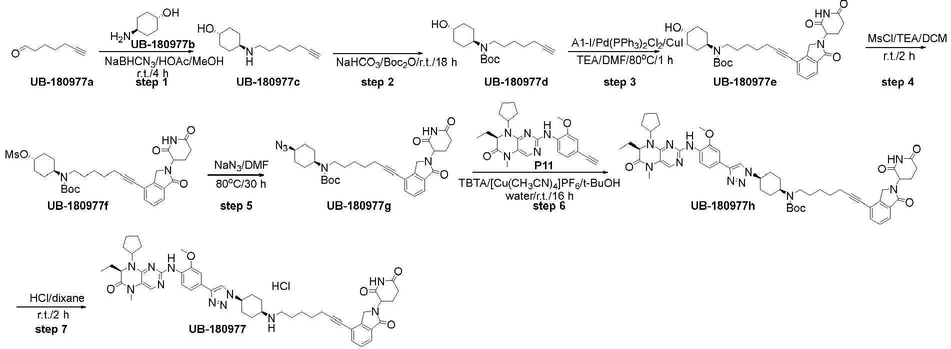

### Steps 1&2: UB-180977d (V2240-064)

Compound **UB-180977a** (300 mg, 2.7 mmol), and **UB-180977b** (310 mg, 2.7 mmol) were dissolved in MeOH (10 mL), HOAc (2 drops) was added, and the mixture was reacted at room temperature for 1 hour. NaBH₃CN (340 mg, 5.4 mmol) was added, and the mixture was reacted at room temperature for 3 hours. Then saturated NaHCO₃ (3 mL) and BoczO (1 mL) were added, and the mixture was reacted at room temperature for 18 hours. The mixture was extracted with EtOAc (15 mL*2), the organic phases were combined, and the organic phase was dried over anhydrous sodium sulfate and filtered, the filtrate was dried by rotary dryer and separated by column chromatography (petroleum ether/ethyl acetate= 0-100%) to obtain target product **UB-180977d** (150 mg, yield 18%) as a colorless oil. LCMS [M-56]⁺ = 254.2.

¹H NMR (400 MHz, Chloroform-*d*) δ 3.57 (tt, *J* = 10.7, 4.3 Hz, 1H), 3.02 (s, 2H), 2.19 (td, *J* = 7.1, 2.8 Hz, 2H), 2.04 - 1.98 (m, 2H), 1.94 (t, *J=* 2.6 Hz, 1H), 1.78 - 1.70 (m, 2H), 1.64 - 1.48 (m, 8H), 1.46 (s, 9H), 1.41 - 1.34 (m, 4H).

### Step 3: UB-180977e (V2240-066)

Compound **UB-180977d** (150 mg, 0.48 mmol), A1-I (179 mg, 0.48 mmol), Pd(PPh₃)₂Cl₂ (20 mg), CuI (10 mg), and TEA (5 drops) were dissolved in anhydrous DMF (3 mL), the mixture was reacted at 80°C for 2 hour under N₂ protection. The reaction solution was concentrated and isolated by column chromatography (methanol/dichloromethane = 1/10) to obtain target product **UB-180977e** (130 mg, yield 50%) as a yellow solid. LCMS [M-100]⁺ =452.3

¹H NMR (400 MHz, Chloroform-*d*) δ 8.05 (s, 1H), 7.82 - 7.77 (m, 1H), 7.56 (dd, *J* = 7.7, 1.1 Hz, 1H), 7.44 (t, *J* = 7.6 Hz, 1H), 5.25 (dd, *J=* 13.3, 5.1 Hz, 1H), 4.50 (d, *J=* 16.6 Hz, 1H), 4.34 (d, *J=* 16.6 Hz, 1H), 3.57 (m, 1H), 3.11 (m, 6H), 2.49 - 2.37 (m, 3H), 2.28 - 2.22 (m, 1H), 2.00 (m, 2H), 1.73 (m, 2H), 1.67 - 1.61 (m, 2H), 1.45 (m, 17H).

### Step 4: UB-180977f (V2240-068)

Compound **UB-180977e** (150 mg, 0.27 mmol), and TEA (82 mg, 0.81 mmol) were dissolved in DCM (5 mL), MsCl (0.5 mL) was added, and the mixture was reacted at room temperature for 2 hours. The reaction solution was concentrated and isolated by column chromatography (methanol/dichloromethane = 1/10) to obtain target product **UB-180977f** (150 mg, yield 88%) as a yellow solid. LCMS [M+H]⁺ = 630.5

### Step 5: UB-180977g (V2240-069)

Compound **UB-180977f** (150 mg, 0.49 mmol) and NaN₃ (156 mg, 2.4 mmol) were dissolved in DMF (5mL), and the mixture was reacted at 80°C for 30 hours. The reaction solution was poured in saturated brine (20 mL), then extracted with EtOAc (30 mL*2). The organic phase was then washed with saturated brine, dried over anhydrous sodium sulfate, filtered, and concentrated to obtain target product **UB-180977g** (120 mg) as a colorless oil. LCMS [M-56]⁺ = 521.4

### Step 6: UB-180977h (V2240-070)

Compound **P11** (35 mg, 0.086 mmol), **and UB-180977f** (60 mg, 0.10 mmol) were dissolved in t-BuOH (5 mL) and water (2.5 mL). TBTA (8 mg), and [Cu(CH₃CN)₄]PF₆ (10 mg) were added, the mixture was reacted at room temperature for 20 hours. Brine (15 mL) was added, the mixture was extracted with EtOAc (10 mL*2), and the organic phases were combined and concentrated. The reaction crude product was purified by preparative thin layer chromatography (dichloromethane /methanol =22/1) to obtain target product **UB-180977h** (33 mg, yield 39%) as a white solid. LCMS [M+H]⁺ = 983.0

### Step 7: UB-180977 (V2240-075)

Compound **UB-180977h** (33 mg, 0.033 mmol) was dissolved in DCM (1 mL), 4M HCl/dioxane (1.5 mL) was added, and the mixture was reacted at room temperature for 2 hours. The reaction supernatant was removed, and the remaining solid was dried to obtain target product **UB-180977** (30 mg, yield 93%) as a white solid. LCMS [M+H]⁺ = 883.0

¹H NMR (400 MHz, DMSO-*d*₆) δ 11.01 (s, 1H), 9.50 (s, 2H), 8.94 (s, 1H), 8.76 (s, 2H), 7.82 - 7.70 (m, 3H), 7.68 - 7.59 (m, 2H), 7.57 - 7.47 (m, 2H), 5.16 (dd, *J=* 13.2, 5.1 Hz, 1H), 4.74 (s, 1H), 4.48 (d, *J=* 17.6 Hz, 2H), 4.33 (d, *J=* 17.7 Hz, 1H), 4.11 (t, *J* = 8.9 Hz, 1H), 3.92 (s, 3H), 3.26 (m, 4H),3.23 (s, 3H), 2.99 - 2.89 (m, 3H), 2.60 (m, 1H), 2.46 (m, 1H), 2.10 - 1.59 (m, 18H), 1.45 (d, *J=* 38.9 Hz, 6H), 0.76 (t, *J=* 7.4 Hz, 3H).

### Synthesis of Compound UB-180978

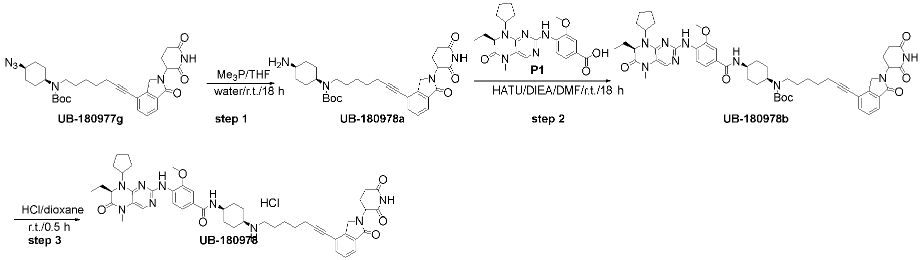

### Step 1: UB-180978a (V2240-071)

Compound **UB-180977g** (60 mg, 0.1 mmol) was dissolved in THF (2 mL) , and 1M Me₃P (0.5 mL, 0.5 mmol) was added, and the mixture was reacted at room temperature for 1 hour. Water (0.5 mL) was added, then the mixture was reacted at room temperature overnight. The reaction liquid was concentrated, then directly used in the next reaction. LCMS [M+H]⁺ = 551.3

### Step 2: UB-180978b (V2240-072)

Compound **UB-180978a** (40 mg, 0.073 mmol), **P1** (31 mg, 0.073 mmol), HATU (53 mg, 0.14 mmol), and DIPEA (28 mg, 0.22 mmol) were dissolved in DMF (3 mL ), reacted at room temperature overnight. The reaction solution was concentrated, then isolated by column chromatography (dichloromethane/methanol = 0 to 100%) to obtain target product **UB-180978b** (20 mg, yield 28%) as a white solid. LCMS [M+H]⁺ = 959.0

### Step 3: UB-180978 (V2240-076)

Compound **UB-180978b** (25 mg, 0.026 mmol) was dissolved in DCM (1 mL), 4M HCl/dioxane (1.5 mL) was added, and the mixture was reacted at room temperature for 30 minutes. The reaction supernatant was removed, and remaining solid was dried to obtain target product **UB-180978** (22 mg, yield 95%) as a white solid. LCMS [M+H]⁺ = 858.9

¹H NMR (400 MHz, DMSO-*d*₆) δ 11.01 (s, 1H), 8.64 (br, 2H), 7.95 (m, 3H), 7.81 (s, 1H), 7.73 (d, *J* = 7.5 Hz, 1H), 7.65 (d, *J* = 7.5 Hz, 1H), 7.56 (m, 3H), 5.21 - 5.15 (m, 1H), 4.48 (m, 2H), 4.33 (d, *J=* 17.7 Hz, 1H), 4.24 (m, 1H), 3.95 (m, 4H), 3.24 (s, 3H), 3.16 (m, 2H), 2.95 (m, 3H), 2.60 (m, 1H), 2.46 (m, 1H), 2.08 - 1.78 (m, 14H), 1.75 - 1.59 (m, 8H), 1.52 (m, 4H), 0.77 (t, *J* = 7.4 Hz, 3H).

### Synthesis of compound UB-180979

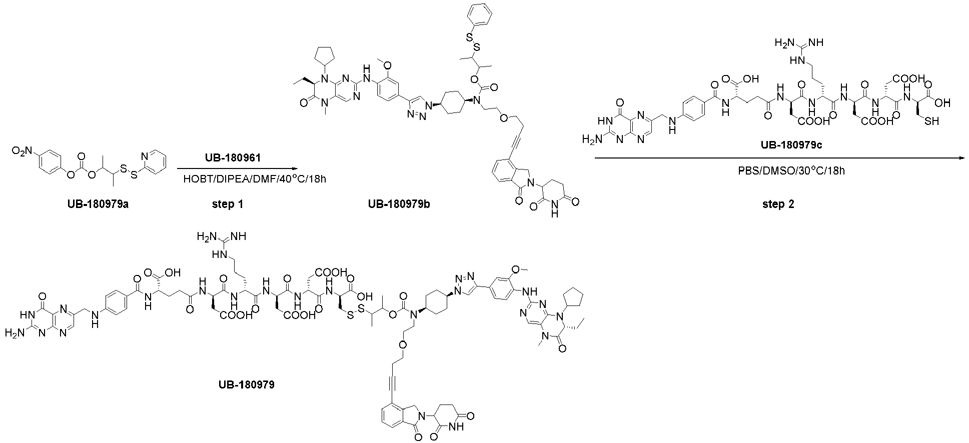

### Step 1: UB-180979b (V2240-073)

Compound **UB-180979a** (7.6 mg, 0.02 mmol), **UB-180961** (19.1 mg, 0.02 mmol), HOBT (2.7 mg, 0.02 mmol), and DIPEA (10.3 mg, 0.08 mmol) were dissolved in DMF (1 mL), and the mixture was reacted at 40°C overnight. The reaction solution was prepared to obtain target product **UB-180979b** ( 17 mg, yield 75%) as a white solid. LCMS [M+H]⁺ = 1125.9

### Step 2: UB-180979 (V2240-079)

Compound **UB-180979c** (26 mg, 0.025 mmol) was dissolved in DMSO (1.5 mL), **UB-180979b** (13 mg, 0.012 mmol) in PBS (1 mL), and DMSO (1.5 mL) were added, and the mixture was reacted at 30°C overnight. The reaction solution was purified to obtain target product **UB-180979** ( 9 mg, yield 37%) as a yellow solid. LCMS [M/3]⁺ = 687.8

¹H NMR: NA

### Synthesis of Compound UB-180984

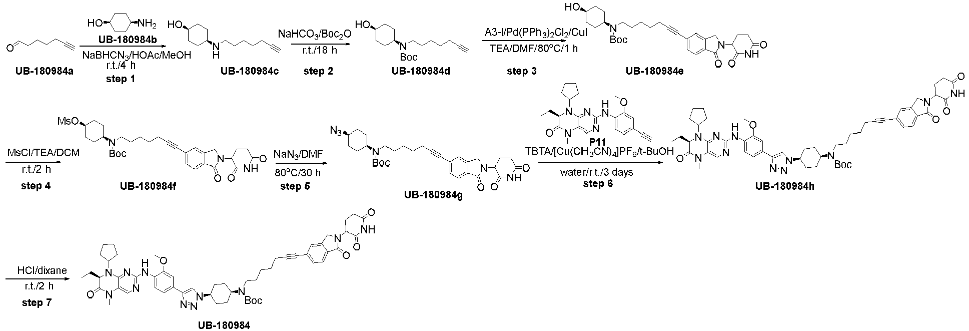

### Steps 1&2: UB-180984d (V2240-064)

Compound **UB-180984a** (300 mg, 2.7 mmol), and **UB-180984b** (310 mg, 2.7 mmol) were dissolved in MeOH (10 mL), HOAc (2 drops) was added, and the mixture was reacted at room temperature for 1 hour. Then NaBH₃CN (340 mg, 5.4 mmol) was added, and the mixture was reacted at room temperature for 3 hours. Then saturated NaHCO₃ (3 mL) and BoczO (1 mL) were added, and the mixture was reacted at room temperature for 18 hours. Then the mixture was extracted with EtOAc (15 mL*2), the organic phases were combined, and the organic phase was dried over anhydrous sodium sulfate and filtered, and the filtrate was dried by rotary dryer and separated by column chromatography (petroleum ether/ethyl acetate= 0-100%) to obtain target product **UB-180984d(150** mg, yield 18%) as a colorless oil. LCMS [M-56]⁺ = 254.2.

¹H NMR (400 MHz, Chloroform-*d*) δ 3.57 (tt, *J* = 10.7, 4.3 Hz, 1H), 3.02 (s, 2H), 2.19 (td, *J* = 7.1, 2.8 Hz, 2H), 2.04 - 1.98 (m, 2H), 1.94 (t, *J=* 2.6 Hz, 1H), 1.78 - 1.70 (m, 2H), 1.64 - 1.48 (m, 8H), 1.46 (s, 9H), 1.41 - 1.34 (m, 4H).

### Step 3: UB-180984e (V2240-077)

Compound **UB-180984d** (150 mg, 0.48 mmol), A3-I (179 mg, 0.48 mmol), Pd(PPh₃)₂Cl₂ (20 mg), CuI (10 mg), and TEA (5 drops) were dissolved in anhydrous DMF (3 mL), and the mixture was reacted at 80°C for 1 hour under N₂ protection. The reaction solution was concentrated and isolated by column chromatography (methanol/dichloromethane = 1/10) to obtain target product **UB-180984e** (180 mg, yield 68%) as a yellow solid. LCMS [M-56]⁺ =496.5

### Step 4: UB-180984f (V2240-078)

Compound **UB-180984e** (150 mg, 0.27 mmol), and TEA (82 mg, 0.81 mmol) were dissolved in DCM (5 mL), MsCl (0.5 mL) was added, and the mixture was reacted at room temperature for 2 hours. The reaction solution was concentrated to obtain crude product **UB-180984f** (300 mg) as a yellow oil. LCMS [M+H]⁺ = 630.6

### Step 5: UB-180984g (V2240-085)

Compound **UB-180984f** (160 mg, 0.49 mmol) and NaN₃ (156 mg, 2.4 mmol) were dissolved in DMF (2.5mL), the mixture was reacted at 80°C for 30 hours. The reaction solution was poured in saturated brine (20 mL), then the mixture was extracted with EtOAc (30 mL*2). The organic phase was then washed with saturated brine, dried over anhydrous sodium sulfate, filtered, and concentrated to obtain target product **UB-180984g** (120 mg) as a colorless oil. LCMS [M-56]⁺ = 521.4

### Step 6: UB-180984h (V2240-087)

Compound P11 (35 mg, 0.086 mmol), and **UB-180984g** (60 mg, 0.10 mmol) were dissolved in t-BuOH (5 mL) and water (2.5 mL), TBTA (8 mg) and [Cu(CH₃CN)₄]PF₆ (10 mg) were added, and the mixture was reacted at room temperature for 3 days. Brine (15 mL) was added, the mixture was extracted with EtOAc (10 mL*2), and the organic phases were combined and concentrated. The reaction crude product was purified by preparative thin layer chromatography (dichloromethane /methanol =22/1) to obtain target product **UB-180984h** (50 mg, yield 58%) as a yellow solid. LCMS [M+H]⁺ = 983.0

### Step 7: UB-180984 (V2240-090)

Compound **UB-180984f** (230 mg, 0.23 mmol) was dissolved in DCM (3 mL), 4M HCl/dioxane (3 mL) was added, and the mixture was reacted at room temperature for 2 hours. The reaction supernatant was removed, and remaining solid was dried to obtain target product (202 mg, yield 92%) as a light yellow solid. LCMS [M+H]⁺ = 884.6 ¹H NMR (400 MHz, DMSO-*d*₆) δ 13.08 (s, 1H), 11.01 (s, 1H), 9.62 (s, 1H), 8.96 (m, 3H), 7.76 - 7.68 (m, 3H), 7.66 - 7.61 (m, 2H), 7.56 - 7.49 (m, 2H), 5.16 (dd, *J=* 13.3, 5.1 Hz, 1H), 4.71 (m, 1H), 4.51 - 4.44 (m, 2H), 4.32 (d, *J* = 17.8 Hz, 1H), 3.91 (s, 3H), 3.80 (t, *J* = 5.3 Hz, 2H), 3.69 (t, *J=* 6.7 Hz, 2H), 3.32 (m, 1H), 3.21 (s, 3H), 3.16 (m, 2H), 2.97 - 2.89 (m, 1H), 2.79 (t, *J* = 6.7 Hz, 2H), 2.59 (m, 1H), 2.44 (m, 1H), 2.05 - 1.73 (m, 14H), 1.46 (m, 2H), 1.41 - 1.33 (m, 2H), 0.75 (t, *J=* 7.4 Hz, 3H).

### Synthesis of Compound UB-180985

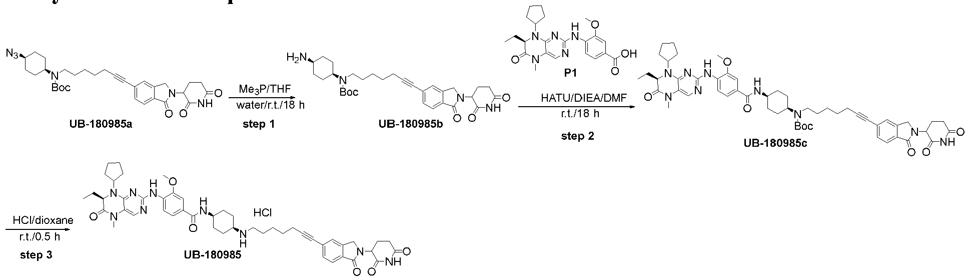

### Step 1: UB-180985b (V2240-088)

Compound **UB-180985a** (80 mg, 0.13 mmol) was dissolved in THF (2 mL), and 1M Me₃P (1 mL, 1 mmol) was added, and reacted at room temperature for 1 hour. Water (0.5 mL) was added, and the mixture was reacted at room temperature overnight. The reaction liquid was concentrated, then directly used in the next reaction. LCMS [M+H]⁺ = 551.3

### Step 2: UB-180985c (V2240-089)

Compound **UB-180985b** (40 mg, 0.073 mmol), **P1** (31 mg, 0.073 mmol), HATU (53 mg, 0.14 mmol), and DIPEA (28 mg, 0.22 mmol) were dissolved in DMF (3 mL ), and reacted at room temperature overnight. The reaction solution was concentrated, then isolated by column chromatography (dichloromethane/methanol = 0 to 100%) to obtain target product **UB-180985c** (15 mg, yield 21%) as a white solid. LCMS [M+H]⁺ = 958.9

### Step 3: UB-180985 (V2240-091)

Compound **UB-180985c** (15 mg, 0.015 mmol) was dissolved in DCM (1 mL), 4M HCl/dioxane (1 mL) was added, and the mixture was reacted at room temperature for 15 minutes. The reaction supernatant was removed, and remaining solid was dried to obtain target product **UB-180985** (12 mg, yield 85%) as a white solid. LCMS [M+H]⁺ = 858.8 ¹H NMR (400 MHz, DMSO-*d*₆) δ 11.00 (s, 1H), 8.67 (br, 3H), 7.95 (br, 2H), 7.80 (d, *J* = 2.2 Hz, 1H), 7.72 - 7.66 (m, 1H), 7.63 (s, 1H), 7.57 (m, 2H), 7.51 (d, *J* = 7.9 Hz, 1H), 5.11 (dd, *J* = 13.3, 5.1 Hz, 1H), 4.44 (m, 2H), 4.31 (d, *J* = 17.5 Hz, 1H), 4.22 (m, 1H), 3.94 (m, 4H), 3.23 (s, 3H), 3.13 (m, 2H), 2.93 (m, 3H), 2.60 (m, 1H), 2.42 - 2.37 (m, 1H), 2.05 - 1.76 (m, 14H), 1.61 (m, 8H), 1.51 (m, 4H), 0.76 (t, *J=* 7.4 Hz, 3H).

### Synthesis of Compound UB-180992

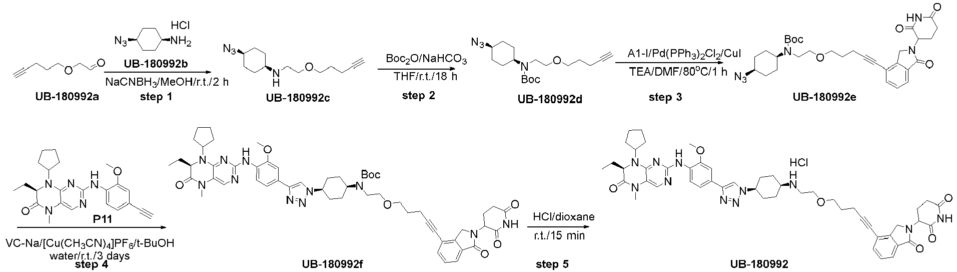

### Steps 1&2: UB-180992d (V2240-095)

Compound **UB-180992a** (100 mg, 0.79 mmol), and **UB-180992b** (140 mg, 0.79 mmol) were dissolved in MeOH (10 mL), and the mixture was reacted at room temperature for 1 hour. Then NaBH₃CN (100 mg, 1.58 mmol) was added, and the mixture was reacted at room temperature for 1 hour. Saturated NaHCO₃ (3 mL) and BoczO (0.5 mL) were added, and the mixture was reacted at room temperature overnight. The mixture was extracted with DCM (30 mL*2), the organic phases were combined, and the organic phase was dried over anhydrous sodium sulfate and filtered, the filtrate was dried by rotary dryer and separated by column chromatography (petroleum ether/ethyl acetate= 0-100%) to obtain target product **UB-180992d** (200 mg, yield 72%) as a colorless oil. LCMS [M-100]⁺ = 251.3

### Step 3: UB-180992e (V2240-098)

Compound **UB-180992d** (100 mg, 0.28 mmol), A1-I (105 mg, 0.28 mmol), Pd(PPh₃)₂Cl₂ (15 mg), CuI (11 mg), and TEA (3 drops) were dissolved in anhydrous DMF (1.5 mL), and the mixture was reacted at 80°C for 1 hour under N₂ protection. The reaction solution was concentrated and isolated by column chromatography (methanol/dichloromethane = 1/10) to obtain target product **UB-180992e** (100 mg, yield 60%) as a yellow solid. LCMS [M-56]⁺ = 537.5

### Step 4: UB-180992f (V2240-099)

Compound **P11** (30 mg, 0.074 mmol), and **UB-180992e** (44 mg, 0.074 mmol) were dissolved in t-BuOH (5 mL) and water (2.5 mL), TBTA(10 mg) (15 mg) and [Cu(CH₃CN)₄]PF₆ (10 mg) were added, and the mixture was reacted at room temperature for 3 days. Brine (15 mL) was added, the mixture was extracted with EtOAc (10 mL*2), and the organic phases were combined and concentrated. The reaction crude product was purified by preparative thin layer chromatography (dichloromethane /methanol =22/1) to obtain target product **UB-180992f** (10 mg, yield 14%) as a yellow solid. LCMS [M+H]⁺ = 999.0

### Step 5: UB-180992 (V2240-106)

Compound **UB-180992f** (10 mg, 0.01 mmol) was dissolved in DCM (1 mL), 4M HCl/dioxane (1 mL) was added, and the mixture was reacted at room temperature for 15 minutes. The reaction supernatant was removed, and the remaining solid was dried to obtain target product **UB-180992** (9 mg, yield 90%) as a yellow solid. LCMS [M+H]⁺ = 898.8 ¹H NMR (400 MHz, DMSO-*d*₆) δ 12.8 (m, 1H), 11.00 (s, 1H), 9.48 (m, 1H), 8.91 (m, 1H), 8.78 (m, 2H), 7.72 (m, 3H), 7.67 - 7.58 (m, 2H), 7.54 - 7.45 (m, 2H), 5.15 (dd, *J=* 13.2, 5.2 Hz, 1H), 4.72 (m, 1H), 4.51 - 4.42 (m, 2H), 4.31 (m, 1H), 4.10 (m, 1H), 3.91 (s, 3H), 3.70 (m, 2H), 3.59 (m, 2H), 3.22 (s, 3H), 3.16 (m, 2H), 2.97 - 2.88 (m, 1H), 2.58 (m, 4H), 2.45 (m, 4H), 2.06 - 1.93 (m, 6H), 1.89 - 1.71 (m, 8H), 1.44 (m, 4H), 0.75 (t, *J=* 7.4 Hz, 3H).

### Synthesis of Compound UB-180993

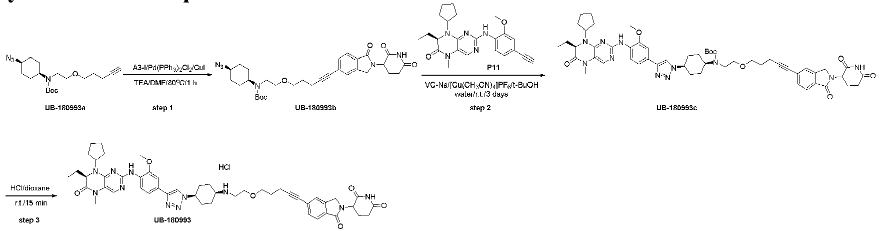

### Step 1: UB-180993b (V2240-100)

Compound **UB-180993b** (100 mg, 0.28 mmol), A3-I (105 mg, 0.28 mmol), Pd(PPh₃)₂Cl₂ (15 mg), CuI (11 mg), and TEA (3 drops) were dissolved in anhydrous DMF (1.5 mL), and the mixture was reacted at 80°C for 1 hour under N₂ protection. The reaction solution was concentrated and isolated by column chromatography (methanol/dichloromethane = 1/10) to obtain target product **UB-180993b** (90 mg, yield 60%) as a yellow solid. LCMS [M-56]⁺ = 537.5

### Step 2: UB-180993c (V2240-102)

Compound **P11** (30 mg, 0.074 mmol), and **UB-180993b** (44 mg, 0.074 mmol) were dissolved in t-BuOH (5 mL) and water (2.5 mL), TBTA (10 mg) and [Cu(CH₃CN)₄]PF₆ (10 mg) were added, and the mixture was reacted at room temperature for 3 days. Brine (15 mL) was added, the mixture was extracted with EtOAc (10 mL*2), and the organic phases were combined and concentrated. The reaction crude product was purified by preparative thin layer chromatography (dichloromethane /methanol =20/1) to obtain target product **UB-180993c** (50 mg, yield 33%) as yellow solid. LCMS [M+H]⁺ = 999.0

### Step 3: UB-180993 (V2240-107)

Compound **UB-180993c** (10 mg, 0.01 mmol) was dissolved in DCM (1 mL), 4M HCl/dioxane (1.5 mL) was added, and the mixture was reacted at room temperature for 15 minutes. The reaction supernatant was removed, and the remaining solid was dried to obtain target product **UB-180993** (40 mg, yield 80%) as yellow solid. LCMS [M+H]⁺ = 898.8 ¹H NMR (400 MHz, DMSO-*d*₆) δ 11.00 (s, 1H), 9.48 (br, 2H),8.87 (m, 1H), 8.69 (m, 2H), 7.81 - 7.66 (m, 3H), 7.62 (d, *J* = 8.7 Hz, 2H), 7.51 (dt, *J* = 7.9, 2.1 Hz, 2H), 5.11 (dd, *J* = 13.3, 5.1 Hz, 1H), 4.71 (m, 1H), 4.44 (m, 2H), 4.31 (d, *J=* 17.5 Hz, 1H), 4.10 (m, 1H), 3.91 (s, 3H), 3.69 (m, 2H), 3.59 (m, 2H), 3.22 (s, 3H), 3.16 (m, 2H), 2.95 - 2.87 (m, 1H), 2.64 - 2.53 (m, 6H), 2.38 (m, 2H), 2.00 (m, 6H), 1.82 (m, 8H), 1.48 (m, 4H), 0.75 (t, *J=* 7.4 Hz, 3H).

### Synthesis of Compound UB-180998

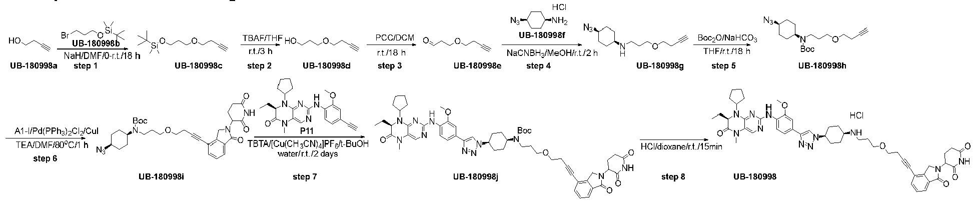

### Step 1: UB-180998c (V2240-094)

Compound **UBI-180998a** (2.1 g 30 mmol) was dissolved in anhydrous DMF (20 mL), 60% NaH (1.44 g, 36 mmol) was added in batches at 0°C, and the mixture was reacted for half an hour. **UB-180998b**(9.2 g, 36 mmol) was dissolved in anhydrous DMF (20 mL) and added to above reaction solution, and the mixture was reacted at room temperature overnight. The reaction solution was poured into ice water (100 mL) and dissolved. The mixture was extracted with EtOAc (50 mL*3), and the organic phase was concentrated and separated by column chromatography (petroleum ether/ethyl acetate= 0-100%) to obtain target product **UB-180998c** (3.4 g, yield 47%) as a colorless oil. LCMS [M+H]⁺ = 243.2

¹H NMR (400 MHz, Chloroform-*d*) δ 3.65 (t, *J* = 6.1 Hz, 2H), 3.50 (td, *J* = 6.7, 3.5 Hz, 4H), 2.41 (td, *J* = 7.0, 2.6 Hz, 2H), 1.92 (t, *J* = 2.7 Hz, 1H), 1.73 (p, *J* = 6.2 Hz, 2H), 0.84 (s, 10H), 0.00 (s, 6H).

### Step 2: UB-180998d (V2240-096)

Compound **UB-180998c** (3.4 g, 14 mmol) was dissolved in THF (10 mL), and 1M TBAF (14 mL, 14 mmol) was added, and the mixture was reacted at room temperature for 3 hours. Water (50 mL) was added. The mixture was extracted with EtOAc (20 mL*3), and the organic phase was washed with saturated ammonium chloride solution. The organic phase was dried over anhydrous sodium sulfate, concentrated, and isolated by column chromatography (petroleum ether/ethyl acetate= 0-100%) to obtain crude product **UB-180977d** (0.9 g, yield 50%) as a colorless oil. LCMS [M+H]⁺ = 129.1

¹H NMR (400 MHz, Chloroform-*d*) δ 3.83 - 3.75 (m, 2H), 3.67 (t, *J* = 5.7 Hz, 2H), 3.58 (t, *J*= 6.7 Hz, 2H), 2.47 (td, *J* = 6.7, 2.7 Hz, 2H), 2.17 (s, 1H), 2.00 (t, *J* = 2.7 Hz, 1H), 1.85 (p, *J* = 5.7 Hz, 2H).

### Step 3: UB-180998e (V2240-104)

Compound **UB-180998d** (800 mg, 6.2 mmol) was dissolved in DCM (40 mL), PCC (2.7 g, 12.4 mmol) was added, and the mixture was reacted at room temperature overnight. The reaction solution was added with Et₂O (40 mL) and silica gel (5 g) and stirred for 30 minutes. After filtration, the filtrate was concentrated to obtain crude product **UB-180998e** (0.5 g), which was directly used in the next reaction. LCMS [M+H]⁺ = NA

### Steps 4&5: UB-180998h (V2240-105)

Compound **UB-180998e** (500 mg, 3.9 mmol), and **UB-180998f** (560 mg, 3.3 mmol) were dissolved in MeOH (20 mL), and the mixture was reacted at room temperature for 1 hour. Then NaBH₃CN (410 mg, 6.6 mmol) was added, and the mixture was reacted at room temperature for 1 hour. The reaction was added saturated NaHCO₃ (10 mL) and Boc₂O (2 mL), and the mixture was reacted at room temperature overnight. The mixture was extracted with DCM (30 mL*2), the organic phases were combined, and the organic phase was dried over anhydrous sodium sulfate and filtered, and the filtrate was dried by rotary dryer and separated by column chromatography (petroleum ether/ethyl acetate= 0-100%) to obtain target product **UB-180998h**(600 mg, yield 52%) as a yellow oil. LCMS [M-100]⁺ = 251.3.

¹H NMR (400 MHz, Chloroform-*d*) δ 3.87 - 3.82 (m, 1H), 3.58 - 3.52 (m, 2H), 3.47 (t, *J* = 6.4 Hz, 2H), 3.16 (s, 2H), 2.47 (td, *J* = 7.0, 2.7 Hz, 2H), 1.99 (t, *J* = 2.7 Hz, 1H), 1.97 - 1.90 (m, 2H), 1.79 (ddt, *J=* 18.6, 10.6, 5.5 Hz, 4H), 1.67 - 1.62 (m, 1H), 1.59 - 1.52 (m, 4H), 1.46 (s, 9H).

### Step 6: UB-180998i (V2240-108)

Compound **UB-180998h** (100 mg, 0.28 mmol), **A1-I** (105 mg, 0.28 mmol), Pd(PPh₃)₂Cl₂ (15 mg), CuI (11 mg), and TEA (3 drop) were dissolved in anhydrous DMF (1.5 mL), and the mixture was reacted at 80°C for 1 hour under N₂ protection. The reaction solution was concentrated and isolated by column chromatography (methanol/dichloromethane = 1/10) to obtain target product **UB-180998i** (70 mg, yield 42%) as a yellow solid. LCMS [M-56]⁺ = 537.5

### Step7: UB-180998j (V2240-110)

Compound **P11** (68 mg, 0.16 mmol), **and UB-180998i** (100 mg, 0.16 mmol) were dissolved in t-BuOH (7 mL), and water (3 mL), [Cu(CH₃CN)₄]PF₆ (10 mg) and TBTA (10 mg) were added, and the mixture was reacted at room temperature for 3 days. Brine (15 mL) was added, the mixture was extracted with EtOAc (10 mL*2), and the organic phases were combined and concentrated. The reaction crude product was purified by preparative thin layer chromatography (dichloromethane /methanol =20/1) to obtain target product **UB-180998j** (50 mg, yield 30%) as a yellow solid. LCMS [M+H]⁺ = 999.0

### Step 8: UB-180998 (V2240-117)

Compound **UB-180998j** (50 mg, 0.05 mmol) was dissolved in DCM (1 mL), 4M HCl/dioxane (1.5 mL) was added, and the mixture was reacted at room temperature for 15 minutes. The reaction supernatant was removed, and the remaining solid was dried to obtain target product **UB-180998** (40 mg, yield 83%) as a yellow solid. LCMS [M+H]⁺ = 898.8 ¹H NMR (400 MHz, DMSO-*d*₆) δ 12.97 (br, 1H), 11.01 (s, 1H), 9.65 (s, 1H), 9.01 (s, 3H), 7.80 - 7.60 (m, 5H), 7.58 - 7.47 (m, 2H), 5.15 (dd, *J=* 13.3, 5.1 Hz, 1H), 4.72 (m, 1H), 4.52 - 4.41 (m, 2H), 4.32 (d, *J* = 17.7 Hz, 1H), 4.09 (q, *J* = 8.9 Hz, 1H), 3.91 (s, 3H), 3.62 (t, *J* = 6.6 Hz, 2H), 3.57 (d, *J* = 5.8 Hz, 2H), 3.21 (m, 4H), 3.04 - 2.87 (m, 3H), 2.75 (t, *J* = 6.6 Hz, 2H), 2.63 - 2.57 (m, 1H), 2.48 - 2.38 (m, 3H), 2.06 - 1.74 (m, 15H), 1.53 - 1.34 (m, 4H), 0.75 (t, *J=* 7.4 Hz, 3H).

### Synthesis of Compound UB-180999

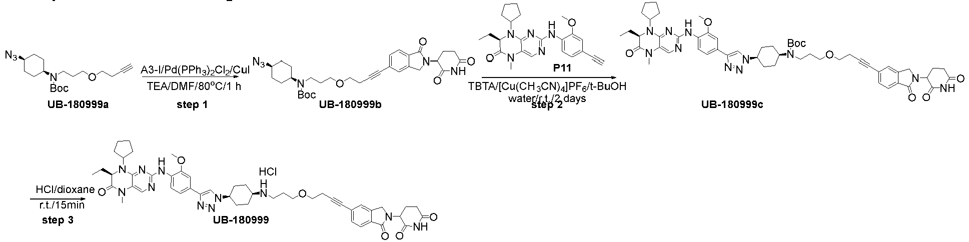

### Step 1: UB-180999b (V2240-109)

Compound **UB-180999a** (100 mg, 0.28 mmol), **A3-I** (105 mg, 0.28 mmol), Pd(PPh₃)₂Cl₂ (15 mg), CuI (11 mg), and TEA (3 drop) were dissolved in anhydrous DMF (1.5 mL), and the mixture was reacted at 80°C for 1 hour under N₂ protection. The reaction solution was concentrated and isolated by column chromatography (methanol/dichloromethane = 1/10) to obtain target product **UB-180999b** (60 mg, yield 42%) as a yellow solid. LCMS [M-56]⁺ = 537.5

### Step 2: UB-180999c (V2240-111)

Compound **UB-180999b** (90 mg, 0.15 mmol), and **P11** (61 mg, 0.15 mmol) were dissolved in t-BuOH (7 mL), and water (3 mL), TBTA (10 mg), and [Cu(CH₃CN)₄]PF₆ (10 mg) were added, and the mixture was reacted at room temperature for 3 days. Brine (15 mL) was added, the mixture was extracted with EtOAc (10 mL*2), and the organic phases were combined and concentrated. The reaction crude product was purified by preparative thin layer chromatography (dichloromethane /methanol =20/1) to obtain target product **UB-180999c** (30 mg, yield 20%) as a yellow solid. LCMS [M+H]⁺ = 999.0

### Step 3: UB-180999 (V2240-118)

Compound **UB-180999c** (30 mg, 0.03 mmol) was dissolved in DCM (1 mL), 4M HCl/dioxane (1.5 mL) was added, and the mixture was reacted at room temperature for 15 minutes. The reaction supernatant was removed, and the remaining solid was dried to obtain target product **UB-180999** (27 mg, yield 93%) as a yellow solid. LCMS [M+H]⁺ = 898.8 ¹H NMR (400 MHz, DMSO-*d*₆) δ 13.00 (br, 1H), 11.00 (s, 1H), 9.65 (s, 1H), 8.99 (m, 3H), 7.78 - 7.60 (m, 5H), 7.51 (dt, *J* = 8.1, 2.1 Hz, 2H), 5.11 (dd, *J* = 13.3, 5.1 Hz, 1H), 4.78 - 4.69 (m, 1H), 4.52 - 4.40 (m, 2H), 4.32 (m, 1H), 4.11 (d, *J=* 8.9 Hz, 1H), 3.91 (s, 3H), 3.61 (t, *J=* 6.6 Hz, 2H), 3.56 (t, *J* = 6.0 Hz, 2H), 3.21 (m, 4H), 3.05 - 2.97 (m, 2H), 2.94 - 2.85 (m, 1H), 2.73 (t, *J=* 6.5 Hz, 2H), 2.63 - 2.56 (m, 1H), 2.48 - 2.35 (m, 3H), 2.04 - 1.74 (m, 15H), 1.50 - 1.33 (m, 4H), 0.75 (t, *J=* 7.4 Hz, 3H).

### Synthesis of Compound UB-181004

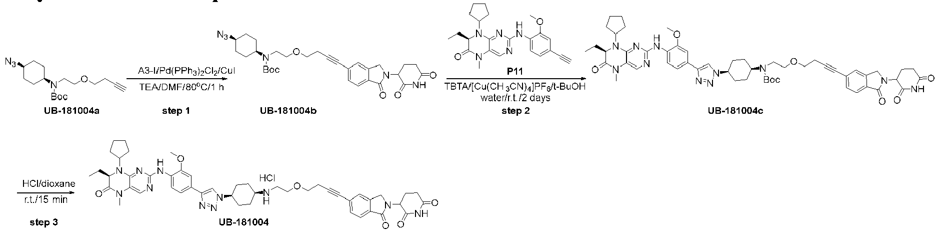

### Step 1: UB-181004b (V2240-114)

Compound **UB-181004a** (300 mg, 0.89 mmol), **A3-I** (330 mg, 0.89 mmol), Pd(PPh₃)₂Cl₂ (62 mg), CuI (30 mg), and TEA (9 drop) were dissolved in anhydrous DMF (8 mL), and the mixture was reacted at 80°C for 1 hour under N₂ protection. The reaction solution was concentrated and isolated by column chromatography (methanol/dichloromethane = 1/10) to obtain target product **UB-181004b** (350 mg, yield 68%) as a yellow solid. LCMS [M+H]⁺ = 579.3

### Step 2: UB-181004c (V2240-115)

Compound **P11** (245 mg, 0.6 mmol), **and UB-181004b** (350 mg, 0.6 mmol) were dissolved in t-BuOH (10 mL) and water (5 mL), TBTA (10 mg) and [Cu(CH3CN)₄]PF₆ (15 mg) were added, and the mixture was reacted at room temperature for 2 days. Water (30 mL) was added, the mixture was extracted with EtOAc (30 mL*2), and the organic phases were combined and concentrated. The mixture was isolated by column chromatography (methanol/dichloromethane = 1/10) to obtain target product **UB-181004c** (350 mg, yield 59%) as a yellow solid. LCMS [M+H]⁺ = 984.5

¹H NMR (400 MHz, DMSO-*d*₆) δ 10.99 (s, 1H), 8.66 (s, 1H), 8.32 (d, *J* = 8.3 Hz, 1H), 7.81 (s, 1H), 7.70 -7.32 (m, 6H), 5.10 (dd, *J* = 13.3, 5.1 Hz, 1H), 4.61 (s, 1H), 4.41 (d, *J* = 17.4 Hz, 1H), 4.37 - 4.18 (m, 3H), 3.94 (s, 3H), 3.55 (t, *J* = 6.6 Hz, 2H), 3.46 (t, *J=* 6.1 Hz, 2H), 3.24 (s, 7H), 2.90 (t, *J*= 12.6 Hz, 1H), 2.64 (t, *J=* 6.7 Hz, 4H), 2.44 - 2.31 (m, 2H), 2.08 - 1.85 (m, 6H), 1.74 (d, *J=* 11.3 Hz, 5H), 1.68 - 1.51 (m, 5H), 1.27 (d, *J=* 28.2 Hz, 9H), 0.76 (t, *J=* 7.4 Hz, 3H).

### Step 3: UB-181004 (V2240-121)

Compound **UB-181004c** (25 mg, 0.025 mmol) was dissolved in DCM (1 mL), 4M HCl/dioxane (1.5 mL) was added, and the mixture was reacted at room temperature for 15 minutes. The reaction supernatant was removed, and the remaining solid was dried to obtain target product **UB-181004** (22 mg, yield 92%) as a light yellow solid. LCMS [M+H]⁺ = 885.0 ¹H NMR (400 MHz, DMSO-*d*₆) δ 12.8 (br, 1H), 11.00 (s, 1H), 9.58 (s, 1H), 8.92 (m, 3H), 7.78 - 7.62 (m, 4H), 7.51 (m, 2H), 5.11 (dd, *J* = 13.3, 5.1 Hz, 1H), 4.72 (m, 1H), 4.53 - 4.40 (m, 2H), 4.32 (d, *J* = 17.5 Hz, 1H), 4.09 (m, 1H), 3.91 (s, 3H), 3.79 (t, *J* = 5.2 Hz, 2H), 3.68 (t, *J* = 6.6 Hz, 2H), 3.32 (m, 2H), 3.19 (m, 5H), 2.91 (m, 2H), 2.78 (t, *J=* 6.6 Hz, 2H), 2.65 - 2.55 (m, 2H), 2.47 - 2.36 (m, 2H), 1.99 (m, 6H), 1.90 - 1.73 (m, 6H), 1.53 - 1.34 (m, 4H), 0.75 (t, *J=* 7.4 Hz, 3H).

### Synthesis of Compound UB-181045

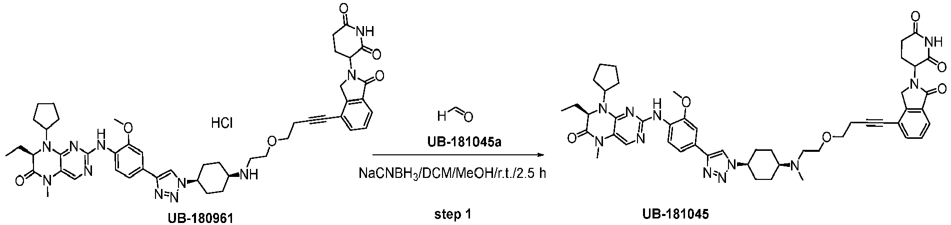

### Step 1: UB-181045 ((V2531-030)

Compound **UB-180961** (30 mg, 0.032 mmol), and **UB-181045a** (20 mg, 0.22 mmol) were dissolved in DCM (5 mL) and MeOH(0.5 mL), and the mixture was reacted at room temperature for 2 hours. Then NaBH₃CN (30 mg, 0.47 mmol) was added, and the mixture was reacted at room temperature for 30 minutes. Brine (15 mL) was added, the mixture was extracted with EtOAc (20 mL), and the organic phases were combined and concentrated. The crude product was prepared to obtain target product **UB-181045** (12 mg, yield 40%) as a white solid. LCMS [M/2]⁺ = 450.3

¹H NMR (400 MHz, DMSO-*d*₆) δ 11.01 (s, 1H), 10.51 (s, 1H), 9.70 (s, 1H), 8.91 (s, 1H), 7.81 - 7.61 (m, 5H), 7.57 - 7.49 (m, 2H), 5.16 (dd, *J=* 13.3, 5.1 Hz, 1H), 5.11 - 5.00 (m, 1H), 4.72 (q, *J* = 3.5 Hz, 1H), 4.53 - 4.44 (m, 2H), 4.33 (d, *J* = 17.8 Hz, 1H), 4.07 (t, *J* = 8.8 Hz, 1H), 3.91 (m, 5H), 3.69 (m, 2H), 3.37 - 3.33 (m, 1H), 3.21 (m, 5H), 2.97 - 2.87 (m, 1H), 2.80 (m, 2H), 2.73 (d, *J* = 4.9 Hz, 3H), 2.66 - 2.56 (m, 1H), 2.49 - 2.42 (m, 1H), 2.18 - 1.64 (m, 14H), 1.52 - 1.32 (m, 4H), 0.75 (t, *J* = 7.4 Hz, 3H).

### Synthesis of Compound UB-181052

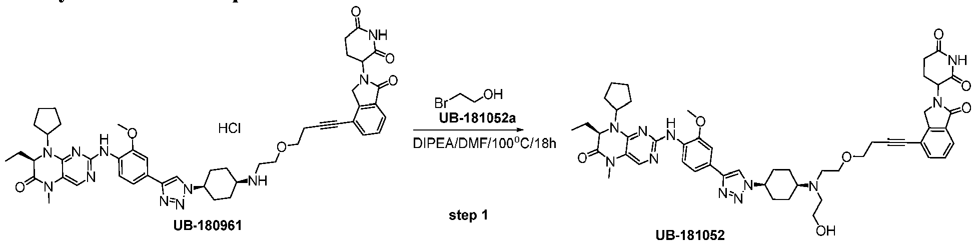

### Step 1: UB-181052 (V2531-035)

Compound **UB-180961** (40 mg, 0.043 mmol), **UB-181052a** (54 mg, 0.43 mmol), and DIPEA (55 mg, 0.43 mmol) were dissolved in DMF (2 mL), and the mixture was reacted at 100°C overnight. The resulting crude product was prepared to obtain target product **UB-181052** (4 mg, yield 10%) as a pink solid. LCMS [M/2]⁺ = 465.2.

¹H NMR (400 MHz, DMSO-*d*₆) δ 11.01 (s, 1H), 9.64 (s, 1H), 8.91 (s, 1H), 8.78 (br, 1H), 7.80 (s, 1H), 7.77 - 7.70 (m, 1H), 7.68 - 7.60 (m, 2H), 7.53 (m, 2H), 7.38 (m, 1H), 5.16 (dd, *J=* 13.3, 5.2 Hz, 1H), 4.71 (br, 1H), 4.52 - 4.42 (m, 2H), 4.32 (m, 3H), 3.84 (s, 3H), 3.78 (t, *J* = 5.2 Hz, 1H), 3.70 (m, 2H), 3.40 - 3.37 (m, 3H), 3.32 (m, 3H), 3.30 (m, 1H), 3.21 (s, 3H), 3.18 (m, 2H), 2.79 (t, *J* = 6.7 Hz, 2H), 2.62 (m, 1H), 2.43 (m, 1H), 2.06 - 1.73 (m, 12H), 1.57 (m, 2H), 1.07 (m, 4H), 0.72 (t, *J* = 7.4 Hz, 3H).

### Synthesis of Compound UB-181063

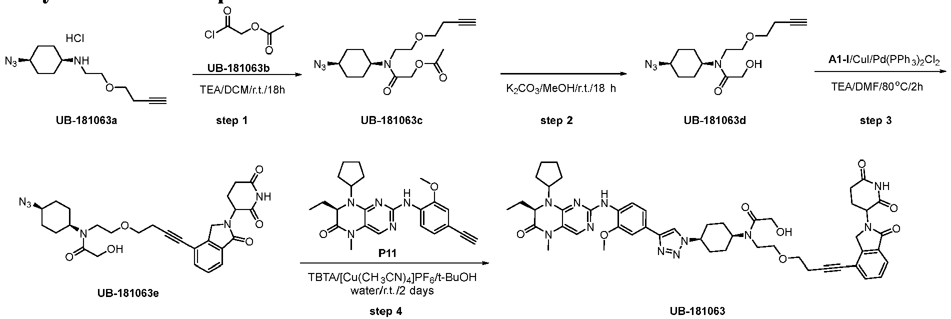

### Step 1: UB-181063c (V2531-052)

Compound **UB-181063a** (0.5 g, 1.8 mmol), **UB-181063b** (0.72 g, 5.4 mmol), and TEA (0.5 g, 5.4 mmol) were dissolved in DCM (20 mL), and the mixture was reacted at room temperature overnight. The reaction was extracted with EtOAc (30 mL*2), the organic phases were combined, and the organic phase was dried over anhydrous sodium sulfate and filtered, and the filtrate was dried by rotary dryer and separated by column chromatography (petroleum ether/ethyl acetate= 0-100%) to obtain target product **UB-181063c** (300 mg mixture) as a yellow solid. LCMS [M+H]⁺ = 295.4,337.4.

### Step 2: UB-181063d (V2531-055)

Compound **UB-181063c** (0.3 g, 0.89 mmol), and K₂CO₃ (0.25 g, 1.78 mmol) were dissolved in MeOH (10 mL), and the mixture was reacted overnight. Then the mixture was extracted with EtOAc (30 mL*2), the organic phases were combined, and the organic phase was dried over anhydrous sodium sulfate and filtered, and the filtrate was dried by rotary dryer and separated by column chromatography (petroleum ether/ethyl acetate= 0-100%) to obtain target product **UB-181063d(80** mg, yield 30%) as yellow oil. LCMS [M+H]⁺ = 295.4.

### Step 4 : UB-181063 (V2531-059)

Compound P11 (50 mg, 0.12 mmol), and **UB-181063d** (65 mg, 0.12 mmol) were dissolved in t-BuOH (10 mL) and water (5 mL), TBTA (3 mg) and [Cu(CH₃CN)₄]PF₆ (5 mg) were added, and the mixture was reacted at room temperature for 2 days. The reaction was extracted with DCM (15 mL*3). The crude product was prepared and purified to obtain target product **UB-181063** (29 mg, yield 25%) as a white solid. LCMS [M+H]⁺ = 943.0

¹H NMR (400 MHz, DMSO-*d*₆) δ 13.8 (br, 1H), 11.04 - 10.94 (s, 1H), 9.60 (s, 1H), 8.80 (d, *J=* 11.3 Hz, 1H), 7.79 - 7.44 (m, 7H), 5.13 (dd, *J* = 13.4, 5.1 Hz, 1H), 4.69 (m, 1H), 4.51 - 4.37 (m, 2H), 4.28 (d, *J* = 17.7 Hz, 1H), 4.17 (s, 1H), 4.10 (m, 3H), 3.90 (d, *J* = 2.7 Hz, 3H), 3.62 (m, 2H), 3.50 (m, 4H), 3.21 (m, 5H), 2.96 - 2.86 (m, 1H), 2.64 (m, 3H), 2.44 (m, 2H), 2.14 - 1.69 (m, 11H), 1.62 (m, 2H), 1.42 (m, 4H), 0.74 (t, *J* = 7.4 Hz, 3H).

### Synthesis of Compound UB-181089

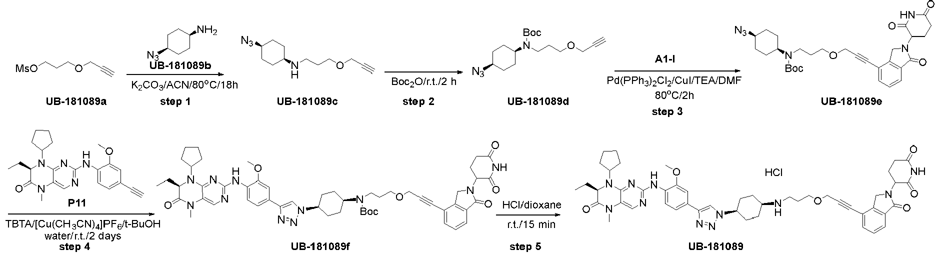

### Steps 1&2: UB-181089d (V2531-111)

Compound **UB-181089a** (1 g, 5.2 mmol) was dissolved in ACN (50 mL), **UB-181089b** (700 mg, 4 mmol), and K₂CO₃ (1.1 g, 8 mmol) were added, and the mixture was reacted at 80°C overnight. The reaction was cooled to room temperature, BoczO (2 mL, 8 mmol) was added, and the mixture was reacted at room temperature for 2 hours. The reaction solution was poured into ice water (50 mL) and dissolved. The mixture was extracted with EtOAc (30 mL*3), and the organic phase was concentrated and dried by rotary dryer and separated by column chromatography (petroleum ether/ethyl acetate= 0-100%) to obtain target product **UB-181089d** (400 mg, yield 30%) as a colorless oil. LCMS [M+H]⁺ = 337.5

### Step 3: UB-181089e (V2531-115)

Compound **UB-181089d** (250 mg, 0.74 mmol), **A1-I** (230 mg, 0.62 mmol), Pd(PPh₃)₂Cl₂ (27 mg, 0.035 mmol), CuI (7 mg, 0.35 mmol), and TEA (75 mg, 0.74 mmol) were dissolved in anhydrous DMF (5 mL), and the mixture was reacted at 80°C for 2 hours under N₂ protection. The reaction solution was concentrated and isolated by column chromatography (dichloromethane/methanol = 15/1) to obtain target product **UB-181089e** (50 mg, yield 14%) as a yellow solid. LCMS [M-56]⁺ = 523.5.

### Step 4: UB-181089f (V2531-117)

Compound **P11** (25 mg, 0.062 mmol), and **UB-181089e** (35 mg, 0.062 mmol) were dissolved in t-BuOH (5 mL) and water (2.5 mL), TBTA (3 mg) and [Cu(CH₃CN)₄]PF₆ (5 mg) were added, and the mixture was reacted at room temperature for 3 days. Water (15 mL) was added, the mixture was extracted with EtOAc (10 mL*2), and the organic phases were combined and concentrated. The resulting crude product was purified by preparative thin layer chromatography (dichloromethane /methanol =15/1) to obtain target product **UB-181089f** (10 mg, yield 16%) as a white solid LCMS [M+H]⁺ = 985.1

### Step 5: UB-181089 (V2531-119)

Compound **UB-181089f** (10 mg, 0.01 mmol) was dissolved in DCM (1 mL), 4M HCl/dioxane (1 mL) was added, and the mixture was reacted at room temperature for 15 minutes. The reaction supernatant was removed, and the remaining solid was dried to obtain target product **UB-181089** (9 mg, yield 97%) as a white solid. LCMS [M/2]⁺ = 443.3

¹H NMR (400 MHz, DMSO-*d*₆) δ 11.01 (s, 1H), 9.59 (s, 1H), 8.99 (br, 3H), 7.80 - 7.67 (m, 4H), 7.63 - 7.55 (m, 2H), 7.51 (m, 1H), 5.15 (dd, *J=* 13.2, 5.1 Hz, 1H), 4.74 (m, 1H), 4.55 - 4.44 (m, 4H), 4.37 (d, *J=* 17.8 Hz, 1H), 4.08 (t, *J=* 8.8 Hz, 1H), 3.91 (s, 3H), 3.65 (m, 2H), 3.21 (s, 3H), 3.06 - 2.88 (m, 4H), 2.60 (m, 1H), 2.48 - 2.42 (m, 2H), 2.06 - 1.74 (m, 16H), 1.41 (m, 4H), 0.75 (t, *J* = 7.4 Hz, 3H).

### Synthesis of Compound UB-181099

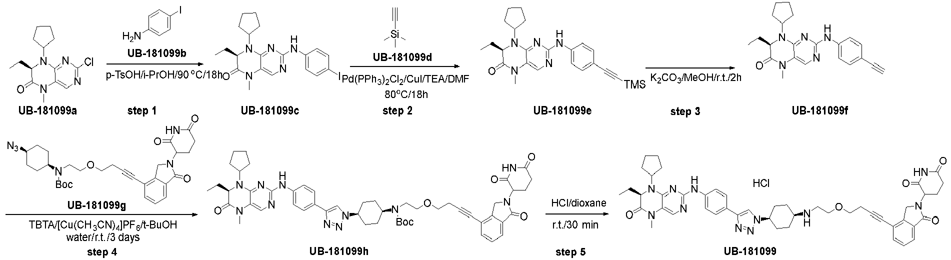

### Step 1: UB-181099c (V2531-134)

Compound **UB-181099a** (550 mg, 1.8 mmol), **and UB-181099b** (394 mg, 1.8 mmol) were dissolved in i-PrOH (30 mL), p-TsOH (100 mg) was added, and the mixture was reacted at 90°C overnight. The solid produced in the reaction was filtered to obtain target product **UB-181099c** (850 mg, yield 95%) as a yellow solid. LCMS [M+H]⁺ = 478.4

¹H NMR (400 MHz, Chloroform-*d*) δ 10.53 (s, 1H), 7.67 - 7.61 (m, 2H), 7.42 (s, 1H), 7.33 (d, *J=* 8.7 Hz, 2H), 4.43 - 4.32 (m, 2H), 3.30 (s, 3H), 2.19 - 2.01 (m, 2H), 1.99 - 1.56 (m, 10H), 0.87 (t, *J*= 7.4 Hz, 3H).

### Step 2: UB-181099e (V2531-136)

Compound **UB-181099c** (100 mg, 0.21 mmol), **UB-181099d** (0.5 mL), Pd(PPh₃)₂Cl₂ (8 mg), CuI (3 mg), and TEA (42 mg, 0.42 mmol) were dissolved in anhydrous DMF (3 mL), and the mixture was reacted at 80°C overnight under N₂ protection. The reaction solution was concentrated and isolated by column chromatography (petroleum ether/ethyl acetate= 0-100%) to obtain target product **UB-181099e** (20 mg, yield 21%) as yellow solid. LCMS [M+H]⁺ = 448.5.

¹H NMR (400 MHz, Chloroform-*d*) δ 7.68 (d, *J=* 10.4 Hz, 1H), 7.59 - 7.50 (m, 2H), 7.44 - 7.33 (m, 2H), 4.24 (dd, *J* = 7.8, 3.7 Hz, 1H), 3.30 (s, 3H), 2.13 (d, *J* = 10.4 Hz, 1H), 1.96 (d, *J* = 8.4 Hz, 1H), 1.84 - 1.57 (m, 10H), 0.87 (t, *J=* 7.5 Hz, 3H), 0.00 (s, 7H).

### Step 3: UB-181099f (V2531-139)

Compound **UB-181099e** (20 mg, 0.04 mmol) was dissolved in MeOH (10 mL), K₂CO₃ (11 mg, 0.08 mmol) was added and the mixture was reacted at room temperature for 2 hours. The mixture was extracted with DCM (50 mL*3), and the organic phase was concentrated and isolated by column chromatography (petroleum ether/ethyl acetate= 0-100%) to obtain target product **UB-181099f** (20 mg crude) as a yellow solid. LCMS [M+H]⁺ = 376.5

### Step 4: UB-181099h (V2531-141)

Compound **P12** (15 mg, 0.04 mmol), and **UB-181099f** (34 mg, 0.058 mmol) were dissolved in t-BuOH (6 mL) and water (3 mL), TBTA (3 mg) and [Cu(CH₃CN)₄]PF₆ (5 mg) were added, and the mixture was reacted at 50°C overnight. Brine (30 mL) was added, the mixture was extracted with EtOAc (30 mL*2), and the organic phases were combined and concentrated. The reaction crude product was purified by preparative thin layer chromatography (dichloromethane /methanol =15/1) to obtain target product **UB-181099h** (10 mg, yield 26%) as a white solid LCMS [M+H]⁺ = 955.1

### Step 5: UB-181099 (V2531-143)

Compound **UB-181099h** (10 mg, 0.01 mmol) was dissolved in DCM (2 mL), 4M HCl/dioxane (2 mL) was added, and the mixture was reacted at room temperature for 30 minutes. The supernatant was removed to obtain target product **UB-181099** (8.7 mg, yield 96%) as a light yellow solid. LCMS [M/2]⁺ = 428.2

¹H NMR (400 MHz, DMSO-*d*₆) δ 11.01 (s, 1H), 10.32 (s, 1H), 8.84 (m, 3H), 7.87 (d, *J=* 8.3 Hz, 2H), 7.75 (d, *J* = 11.6 Hz, 2H), 7.67 - 7.50 (m, 4H), 5.16 (dd, *J=* 13.3, 5.1 Hz, 1H), 4.69 (m, 1H), 4.51 - 4.42 (m, 2H), 4.32 (d, *J* = 17.7 Hz, 1H), 4.23 (t, *J* = 8.8 Hz, 1H), 3.79 (t, *J* = 5.2 Hz, 2H), 3.69 (t, *J=* 6.7 Hz, 2H), 3.22 (s, 3H), 3.16 (t, *J* = 5.7 Hz, 2H), 2.93 (m, 1H), 2.79 (m, 2H), 2.63 - 2.56 (m, 1H), 2.49 - 2.37 (m, 2H), 2.08 - 1.68 (m, 14H), 1.59 (m, 2H), 1.47 (m, 2H), 0.77 (t, *J*= 7.4 Hz, 3H).

### Synthesis of Compound UB-181108

### The synthesis method is similar to the synthesis of UB-180961

### Step 1: UB-181108 (V2531-144)

¹H NMR (400 MHz, DMSO-*d*₆) δ 11.02 (s, 1H), 9.50 (s, 1H), 8.97 (s, 2H), 8.69 (s, 1H), 7.79 - 7.63 (m, 4H), 7.60 - 7.43 (m, 3H), 5.17 (dd, *J=* 13.3, 5.1 Hz, 1H), 4.58 - 4.44 (m, 3H), 4.33 (d, *J=* 17.8 Hz, 1H), 4.09 (m, 1H), 3.91 (s, 3H), 3.80 (t, *J=* 5.1 Hz, 2H), 3.73 (t, *J=* 6.7 Hz, 2H), 3.22 (s, 6H), 2.93 (m, 1H), 2.82 (t, *J* = 6.7 Hz, 2H), 2.61 (m, 1H), 2.48 - 2.43 (m, 1H), 2.26 (m, 4H), 2.09 - 1.75 (m, 10H), 1.72 - 1.60 (m, 2H), 1.53 - 1.35 (m, 4H), 0.75 (t, *J=* 7.4 Hz, 3H). LCMS [M/2]⁺ = 443.2

### Synthesis of Compound UB-181113

### The synthesis is similar to the synthesis of UB-180937

### Step 1: UB-181113 (V2768-004)

¹H NMR (400 MHz, DMSO-*d*₆) δ 11.02 (s, 1H), 8.89 (m, 3H), 8.30 (m, 1H), 7.91 (s, 1H), 7.85 - 7.79 (m, 1H), 7.73 (d, *J* = 7.5 Hz, 1H), 7.66 (d, *J* = 7.5 Hz, 1H), 7.59 - 7.50 (m, 3H), 5.17 (dd, *J* = 13.3, 5.1 Hz, 1H), 4.47 (m, 2H), 4.33 (d, *J* = 17.8 Hz, 1H), 4.24 - 4.17 (m, 1H), 3.91 (s, 3H), 3.79 (t, *J=* 5.2 Hz, 2H), 3.71 (t, *J=* 6.7 Hz, 2H), 3.22 (s, 3H), 3.16 (m, 2H), 3.05 (m, 2H), 2.93 (t, *J=* 12.8 Hz, 1H), 2.81 (t, *J=* 6.7 Hz, 2H), 2.64 - 2.54 (m, 2H), 2.47 - 2.39 (m, 1H), 2.14 (m, 2H), 1.93 (m, 4H), 1.81 (m, 4H), 1.47 (m, 8H), 0.76 (t, *J=* 7.4 Hz, 3H). LCMS [M/2]⁺ = 431.2

### Synthesis of Compound UB-181118

### The synthesis method is similar to the synthesis of UB-181099

### Step 1: UB-181118 (V2768-021)

¹H NMR (400 MHz, DMSO-*d*₆) δ 11.02 (s, 1H), 10.38 (s, 1H), 9.03 (brs, 2H), 8.61 (s, 1H), 7.85 (d, *J=* 8.2 Hz, 2H), 7.80 - 7.70 (m, 2H), 7.66 (d, *J=* 7.4 Hz, 1H), 7.62 - 7.50 (m, 3H), 5.17 (dd, *J=* 13.3, 5.1 Hz, 1H), 4.56 - 4.44 (m, 3H), 4.34 (d, *J=* 17.7 Hz, 1H), 4.22 (p, *J=* 8.9 Hz, 1H), 3.80 (d, *J* = 5.4 Hz, 2H), 3.72 (t, *J* = 6.7 Hz, 2H), 3.21 (m, 5H), 2.99 - 2.89 (m, 1H), 2.82 (m, 2H), 2.64 - 2.57 (m, 1H), 2.48 - 2.43 (m, 1H), 2.26 (m, 4H), 2.08 - 1.76 (m, 10H), 1.65 (m, 4H), 1.47 (m, 2H), 0.77 (t, *J=* 7.4 Hz, 3H). LCMS [M/2]⁺ = 428.1

### Synthesis of Compound UB-181126

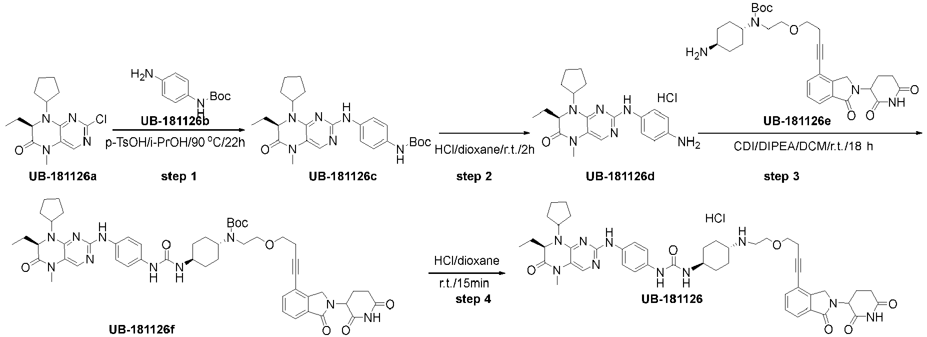

### Step 1: UB-181126c (V2768-013)

Compound **UB-181126a** (295 mg, 1 mmol), **and UB-181126b** (170 mg, 1 mmol) were dissolved in i-PrOH (10 mL), p-TsOH (60 mg, 0.34 mmol) was added, and the mixture was reacted at 90°C overnight. The reaction solution was concentrated to obtain crude product **UB-181126c** (250 mg, yield 53%) as a purple solid. LCMS [M+H]⁺ = 367.5/467.6

### Step 2: UB-181126d (V2768-015)

Compound **UB-181126c** (250 mg, 0.53 mmol) was dissolved in DCM (5 mL), HCl/dioxane (5 mL) was added, and the mixture was reacted at room temperature for 2 hours. The supernatant was removed to obtain target product **UB-181126d** (200 mg, yield 100%) as a green solid. LCMS [M+H]⁺ = 367.4.

### Step 3: UB-181126f (V2768-020)

Compound **UB-181126e** (20 mg, 0.049 mmol), and DIPEA (40 mg, 0.3 mmol) were dissolved in DCM (5 mL), CDI (20 mg, 0.1 mmol) was added, and the mixture was reacted at room temperature for 2 hours. **UB-181126d** (270 mg, 0.049 mmol) was added, and the mixture was reacted at room temperature for 1 hour. The crude product of concentrated reaction solution was purified by preparative thin layer chromatography (dichloromethane /methanol =15/1) to obtain white product **UB-181126f** (5 mg, yield 10%). LCMS [M+H]⁺ = 946

### Step 4: UB-181126 (V2768-023)

Compound **UB-181126f** (5 mg, 0.005 mmol) was dissolved in DCM (2 mL), 4M HCl/dioxane (1.5 mL) was added, and the mixture was reacted at room temperature for 15 minutes. The supernatant was removed to obtain target product **UB-181126** (4 mg, yield 81%) as a white solid. LCMS [M/2]⁺ = 423.6

¹H NMR (400 MHz, DMSO-*d*₆) δ 11.02 (s, 1H), 9.79 (s, 1H), 8.75 (s, 2H), 8.63 (s, 1H), 7.73 (d, *J* = 7.5 Hz, 1H), 7.68 - 7.60 (m, 2H), 7.54 (t, *J* = 7.6 Hz, 1H), 7.39 (d, *J* = 8.5 Hz, 2H), 7.30 (d, *J=* 8.6 Hz, 2H), 6.26 (d, *J=* 7.4 Hz, 1H), 5.17 (dd, *J=* 13.3, 5.1 Hz, 1H), 4.45 (m, 2H), 4.32 (d, *J* = 17.7 Hz, 1H), 4.13 (t, *J* = 8.9 Hz, 1H), 3.82 - 3.67 (m, 4H), 3.20 (s, 3H), 3.15 (m, 2H), 3.03 (m, 1H), 2.94 (m, 1H), 2.80 (t, *J* = 6.7 Hz, 2H), 2.60 (m, 1H), 2.47 - 2.41 (m, 1H), 2.14 - 1.71 (m, 12H), 1.56 (m, 2H), 1.46 (m, 4H), 1.22 - 1.11 (m, 2H), 0.75 (t, *J* = 7.4 Hz, 3H).

### Synthesis of Compound UB-181130

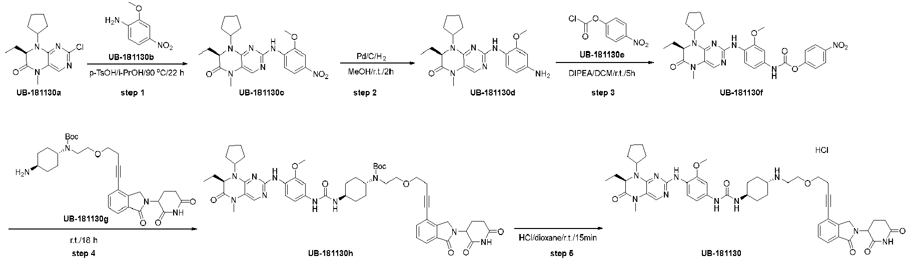

### Step 1: UB-181130c (V2768-008)

Compound **UB-181130a** (1 g, 3.4 mmol), **and UB-181130b** (572 mg, 3.4 mmol) were dissolved in i-PrOH (20 mL), p-TsOH (200 mg, 1.15 mmol) was added, and the mixture was reacted at 90°C overnight. The reaction solution was concentrated and isolated by column chromatography (petroleum ether/ethyl acetate= 0-100%) to obtain target product **UB-181130c** (130 mg, yield 7%) as a yellow solid. LCMS [M+H]⁺ = 427.4

¹H NMR (400 MHz, Chloroform-*d*) δ 8.69 (d, *J* = 9.1 Hz, 1H), 7.94 (dd, *J* = 9.1, 2.5 Hz, 1H), 7.76 - 7.65 (m, 2H), 4.50 (t, *J* = 7.9 Hz, 1H), 4.26 (dd, *J* = 7.9, 3.7 Hz, 1H), 4.03 (s, 3H), 3.34 (s, 3H), 2.17 (q, *J=* 7.6, 6.9 Hz, 1H), 2.01 (m, 1H), 1.93 - 1.81 (m, 4H), 1.71 (m, 4H), 0.88 (t, *J* = 7.5 Hz, 3H).

### Step 2: UB-181130d (V2768-010)

Compound **UB-181130c** (30 mg, 0.07 mmol) was dissolved in DCM (15 mL) and MeOH (5 mL), Pd/C (20 mg) was added, and the mixture was reacted at room temperature for 2 hours under H₂ protection. The reaction solution was filtered, and the filtrate was prepared to obtain target product **UB-181130d** (27 mg, yield 100%) as a white solid. LCMS [M+H]⁺ = 397.4 ¹H NMR (400 MHz, Chloroform-*d*) δ 7.84 (t, *J=* 7.2 Hz, 1H), 7.51 (s, 1H), 6.34 - 6.24 (m, 2H), 4.38 - 4.28 (m, 1H), 4.21 (dd, *J=* 7.6, 3.6 Hz, 1H), 3.83 (s, 3H), 3.28 (s, 3H), 2.08 - 2.01 (m, 1H), 1.97 - 1.78 (m, 4H), 1.72 (q, *J=* 7.1 Hz, 4H), 1.66 - 1.56 (m, 2H), 0.86 (t, *J=* 7.5 Hz, 3H).

### Steps 3&4: UB-181130h (V2768-026)

Compound **UB-181130d** (70 mg, 0.17 mmol), and TEA (35 mg, 0.35 mmol) were dissolved in DCM (10 mL), **UB-181130e** (71 mg, 0.35 mmol) was added, and the mixture was reacted at room temperature for 5 hours. Then **UB-181130g** (90 mg, 0.16 mmol) was added, and the mixture was reacted at room temperature overnight. The reaction solution was concentrated and isolated by column chromatography (methanol/dichloromethane = 1/10) to obtain target product **UB-181130h** (38 mg, yield 24%) as a yellow solid. LCMS [M+H]⁺ = 976

### Step 5: UB-181130 (V2768-028)

Compound **UB-181130h** (38 mg, 0.039 mmol) was dissolved in DCM (2 mL), 4M HCl/dioxane (2 mL) was added, and the mixture was reacted at room temperature for 15 minutes. The supernatant was removed to obtain target product **UB-181130** (25 mg, yield 71%) as a white solid. LCMS [M/2]⁺ = 438.2

¹H NMR (400 MHz, DMSO-*d*₆) δ 11.02 (s, 1H), 8.79 (brs, 3H), 7.73 (dd, *J* = 7.6, 1.1 Hz, 1H), 7.68 - 7.51 (m, 3H), 7.41 (d, *J* = 2.2 Hz, 1H), 7.29 (d, *J=* 8.3 Hz, 1H), 6.85 (dd, *J=* 8.6, 2.2 Hz, 1H), 6.35 (d, *J* = 7.5 Hz, 1H), 5.17 (dd, *J* = 13.3, 5.1 Hz, 1H), 4.47 (m, 2H), 4.32 (d, *J* = 17.7 Hz, 1H), 4.00 (m, 1H), 3.77 (t, *J=* 5.2 Hz, 2H), 3.72 (m, 4H), 3.39 (d, *J=* 4.2 Hz, 1H), 3.29 (m, 1H), 3.19 (s, 3H), 3.14 (m, 2H), 3.03 (m, 1H), 2.97 - 2.89 (m, 1H), 2.80 (t, *J=* 6.7 Hz, 2H), 2.61 (m, 1H), 2.47 - 2.42 (m, 1H), 1.92 (m, 12H), 1.53 - 1.30 (m, 6H), 1.23 - 1.14 (m, 2H), 0.73 (t, *J* = 7.4 Hz, 3H).

### Synthesis of Compound UB-181143

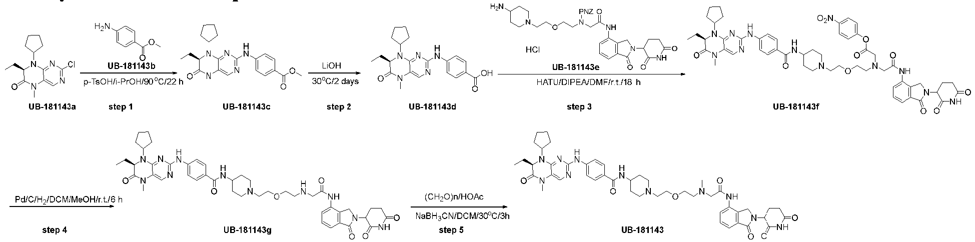

### Step 1: UB-181143c (V2876-025)

Compound **UB-181143a** (1 g, 3.4 mmol) was dissolved in i-prOH (20 ml), **UB-181143b** (5.6 g, 3.7 mmol) and p-TsOH (292 mg. 1.7 mmol) were added, and the mixture was reacted for 22 hours under N₂ protection. The solid produced in the reaction was filtered, then dried to obtain product **UB-181143c** (1.48 g, yield 90%) as white solid. LCMS [M+1] ⁺ = 410.5

### Step 2: UB-181143d (V2876-034)

Compound **UB-181143c** (1000 mg, 2.445 mmol) was dissolved in MeOH/THF/H₂O =1/3/1 (15 ml), LiOH (410 mg, 9.8 mmol) was added, and the mixture was reacted at room temperature overnight. The reaction solution was poured to small amount of water, the mixture was extracted with small amount of ethyl acetate, the aqueous phase was kept and adjusted the pH to 6 with 3M aqueous solution of hydrochloric acid, then extracted with dichloromethane /methanol =10/1, and the organic phase was dried, then concentrated to obtain **product UB-181143d** (850 mg, yield 88%) as a white solid. LCMS [M+1] ⁺ = 396.4

### Step 3: UB-181143f (V2768-051)

Compound **UB-181143e** (500 mg, 0.71 mmol), **UB-1810143d** (250 mg, 0.63 mmol), HATU (500 mg, 1.3 mmol), and DIPEA (0.5 mL) were dissolved in DMF (13 mL), and reacted at room temperature overnight. Solid was precipitated by adding water (60 mL), and the solid was purified by reversed-phase column chromatography (ACN/water=60/40) to obtain target product **UB-1810143f** (300 mg, yield 45%) as a yellow solid. LCMS [M+H]⁺ = 1044

### Step 4: UB-181143g (V2768-045)

Compound **UB-181143f** (70 mg, 0.067 mmol) was dissolved in dichloromethane /methanol (10 mL /3 mL), Pd/C (30 mg) was added, and the mixture was reacted at room temperature for 6 hours under H₂ protection. After completion of the reaction, the reaction was filtrated, and the filtrate was dried by rotary dryer to obtain crude product **UB-181143g** (50 mg crude) as a yellow solid. LCMS [M+H]⁺ = 433.0

### Step 5: UB-181143 (V2768-047)

Compound **UB-181143g** (50 mg, 0.057 mmol), (CH₂O)ₙ (15 mg, 0.17 mmol), and AcOH (1d) were dissolved in DCM (10 mL), and the mixture was reacted at room temperature for 1 hour. Then NaBH₃CN (10 mg, 0.17 mmol) was added, and the mixture was reacted at 30°C for 2 hours. The reaction solution was concentrated, then purified by reversed- phase column chromatography (ACN/H₂O =60/40) to obtain target product **UB-1810143** (20 mg, yield 40%) as a white solid LCMS [M/2+H]⁺ = 440.1

¹H NMR (400 MHz, DMSO-*d*₆) δ 11.02 (s, 1H), 9.74 (s, 1H), 9.30 (s, 1H), 7.92 (d, *J* = 7.7 Hz, 1H), 7.87 - 7.70 (m, 6H), 7.56 - 7.49 (m, 2H), 5.14 (dd, *J=* 13.3, 5.1 Hz, 1H), 4.48 - 4.33 (m, 3H), 4.22 (dd, *J* = 7.9, 3.6 Hz, 1H), 3.67 (d, *J* = 7.6 Hz, 1H), 3.53 (m, 4H), 3.25 (m, 4H), 2.97 - 2.75 (m, 4H), 2.68 (m, 2H), 2.62 (m, 1H), 2.40 (m, 5H), 2.08 - 1.89 (m, 6H), 1.84 - 1.67 (m, 7H), 1.62 (m, 3H), 1.48 (t, *J* = 11.0 Hz, 2H), 0.77 (t, *J* = 7.4 Hz, 3H).

### Synthesis of Compound UB-181144

### Step 1: UB-181144 (V2768-048)

¹H NMR (400 MHz, DMSO-*d*₆) δ 11.02 (s, 1H), 10.56 (s, 1H), 8.96 (q, *J=* 5.7 Hz, 2H), 8.25 (d, *J* = 7.8 Hz, 1H), 7.92 - 7.82 (m, 3H), 7.73 (d, *J* = 7.5 Hz, 1H), 7.64 (m, 3H), 7.54 (t, *J* = 7.6 Hz, 1H), 5.16 (dd, *J* = 13.3, 5.1 Hz, 1H), 4.51 - 4.45 (m, 2H), 4.37 - 4.25 (m, 2H), 3.80 (t, *J* = 5.2 Hz, 2H), 3.72 (d, *J=* 6.7 Hz, 2H), 3.22 (s, 3H), 3.15 (t, *J* = 6.1 Hz, 2H), 3.08 - 3.02 (m, 1H), 2.97 - 2.89 (m, 1H), 2.81 (t, *J* = 6.7 Hz, 2H), 2.64 - 2.58 (m, 1H), 2.48 - 2.41 (m, 1H), 2.19 - 2.11 (m, 2H), 2.07 - 1.75 (m, 10H), 1.68 - 1.60 (m, 2H), 1.51 (m, 4H), 1.45 - 1.35 (m, 2H), 0.77 (t, *J* = 7.4 Hz, 3H). LCMS [M/2+H]⁺ = 416.

### Synthesis of Compound UB-181150

### The synthesis method is similar to UB-181144

### Step 1: UB-181150 (V2768-061)

¹H NMR (400 MHz, DMSO-*d*₆) δ 11.01 (s, 1H), 10.24 (s, 1H), 8.87 - 8.73 (m, 2H), 7.95 - 7.78 (m, 4H), 7.73 (d, *J* = 7.5 Hz, 1H), 7.65 (d, *J* = 7.9 Hz, 3H), 7.53 (t, *J* = 7.6 Hz, 1H), 5.16 (dd, *J* = 13.2, 5.1 Hz, 1H), 4.51- 4.39 (m, 2H), 4.36 - 4.28 (m, 2H), 3.93 (q, *J* = 4.3 Hz, 1H), 3.79 (t, *J=* 5.2 Hz, 2H), 3.71 (t, *J=* 6.7 Hz, 2H), 3.23 (s, 3H), 3.15 (d, *J=* 6.6 Hz, 4H), 2.97 - 2.89 (m, 1H), 2.81 (t, *J=* 6.7 Hz, 2H), 2.60 (d, *J=* 14.7 Hz, 1H), 2.47 - 2.42 (m, 1H), 2.06 - 1.80 (m, 12H), 1.69 - 1.50 (m, 6H), 0.77 (t, *J=* 7.4 Hz, 3H). LCMS [M/2+H]⁺ = 416.1.

### Synthesis of Compound UB-181175

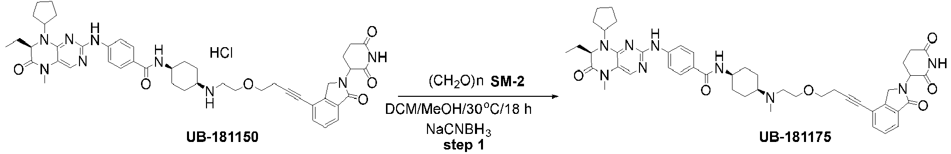

### Step 1: UB-181175 (V2768-077)

Compound **UB-181150** (20 mg, 0.023 mmol), and (CH₂O)ₙ (10 mg, 0.11 mmol) were dissolved in DCM (2 mL) and MeOH (0.2 mL), and the mixture was reacted at 30°C overnight. Then NaBH₃CN (7 mg, 0.077 mmol) was added, and the mixture was reacted at 30°C for 1 hour. The reaction solution was concentrated, then crude product was purified by preparative thin layer chromatography (dichloromethane /methanol =10/1) to obtain target product **UB-181175** (5 mg, yield 26%) as a white solid LCMS [M/2+H]⁺ = 423.1

¹H NMR (400 MHz, DMSO-*d*₆) δ 11.02 (s, 1H), 9.30 (s, 1H), 7.87 - 7.78 (m, 4H), 7.78 - 7.70 (m, 3H), 7.67 - 7.62 (m, 1H), 7.53 (t, *J=* 7.6 Hz, 1H), 5.16 (dd, *J=* 13.3, 5.1 Hz, 1H), 4.50 - 4.40 (m, 2H), 4.32 (d, *J=* 17.7 Hz, 1H), 4.22 (dd, *J=* 7.8, 3.6 Hz, 1H), 3.93 (d, *J=* 65.6 Hz, 3H), 3.67 (d, *J=* 7.9 Hz, 2H), 3.38 (s, 1H), 3.25 (s, 3H), 2.99 - 2.88 (m, 1H), 2.78 (s, 3H), 2.64 - 2.58 (m, 1H), 2.47 - 2.40 (m, 1H), 2.09 - 1.89 (m, 6H), 1.86 - 1.71 (m, 8H), 1.62 (dt, *J* = 14.6, 4.0 Hz, 6H), 1.35 (s, 2H), 0.77 (t, *J* = 7.5 Hz, 3H).

### Synthesis of Compound UB-181176

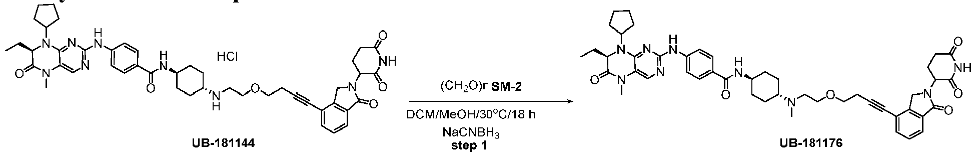

### Step 1: UB-181176 (V2768-078)

Compound **UB-181144** (25 mg, 0.028 mmol), and (CH₂O)ₙ (7.8 mg, 0.086 mmol) were dissolved in DCM (2 mL) and MeOH (0.2 mL), and the mixture was reacted at 30°C overnight. Then NaBH₃CN (5.4 mg, 0.086 mmol) was added, and the mixture was reacted at 30°C for 1 hour. The reaction solution was concentrated, then crude product was purified by preparative thin layer chromatography (dichloromethane /methanol =10/1) to obtain target product **UB-181176** (6 mg, yield 25%) as white solid LCMS [M/2+H]⁺ = 423.1

¹H NMR (400 MHz, DMSO-*d*₆) δ 11.02 (s, 1H), 9.31 (s, 1H), 8.01 (s, 1H), 7.85 (s, 1H), 7.80 (d, *J* = 9.0 Hz, 2H), 7.76 - 7.70 (m, 3H), 7.65 (dd, *J* = 7.6, 1.1 Hz, 1H), 7.53 (t, *J* = 7.6 Hz, 1H), 5.17 (dd, *J=* 13.3, 5.1 Hz, 1H), 4.46 (d, *J=* 17.8 Hz, 2H), 4.32 (d, *J=* 17.7 Hz, 1H), 4.22 (dd, *J* = 7.7, 3.6 Hz, 1H), 3.77 (d, *J=* 58.0 Hz, 5H), 3.25 (s, 4H), 2.94 (td, *J* = 13.0, 12.5, 6.7 Hz, 1H), 2.78 (d, *J=* 8.3 Hz, 4H), 2.62 (s, 1H), 2.43 (d, *J=* 4.4 Hz, 1H), 2.09 - 1.91 (m, 8H), 1.77 (d, *J=* 15.1 Hz, 6H), 1.62 (dt, *J=* 14.6, 7.0 Hz, 5H), 1.42 (d, *J=* 12.2 Hz, 3H), 0.77 (t, *J=* 7.4 Hz, 3H).

### Synthesis of Compound UB-180968

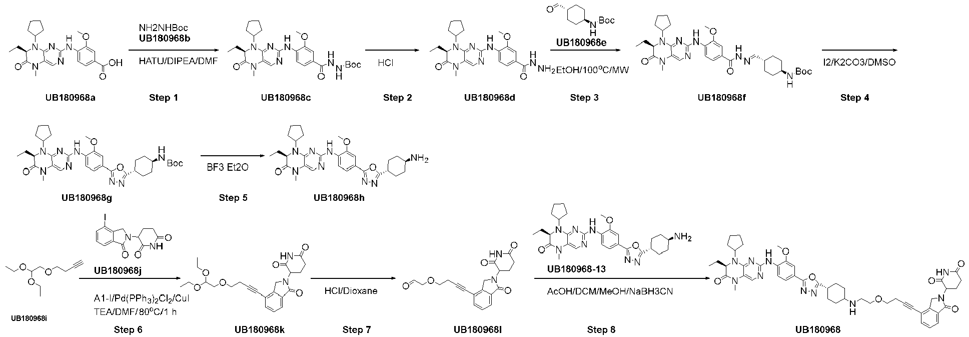

### Step 1: UB-1810968c (V2141-055)

Compound **UB-1810968a** (3g, 7mmol), **UB-1810968b** (1.85g, 14mmol) and HATU (5.3g, 14mmol) were dissolved in DIPEA (3.5mL) and DMF (20mL), and the mixture was reacted at room temperature for 18 hours. The reaction solution was added NaHCO₃ aqueous solution, and extracted with dichloromethane (5 mL*3). The organic phases were combined, washed with water, dried over Na₂SO₄ and concentrated to give the crude product. The crude product was purified by reversed-phase column chromatography (MeOH/water= 5% to 95%, 45min), then purified by silica gel column (DCM/ (DCM/MeOH/H2O 10:1:1) = 30% to obtain target product **UB-1810968c** (200mg, 84.2% yield) as a white solid. ¹H NMR (400 MHz, Chloroform-*d*) δ 8.56 (d, *J* = 8.4 Hz, 1H), 7.66 (d, *J* = 9.5 Hz, 2H), 7.39 (d, *J* = 1.9 Hz, 1H), 7.34 (s, 1H), 7.29 - 7.24 (m, 2H), 4.54 - 4.42 (m, 1H), 4.29 - 4.17 (m, 1H), 3.98 (s, 3H), 3.33 (s, 3H), 2.20 - 1.95 (m, 2H), 1.89 - 1.67 (m, 9H), 0.88 (t, *J=* 7.5 Hz, 3H). LC-MS: (M+H) ⁺=540.2

### Step 2: UB-1810968d (V2141-057)

Compound **UB-1810968c** (3.2g, 5.9mmol) was dissolved in DCM (100mL), HCl/dioxane (1M, 10mL) was added. The reaction was reacted at room temperature for 1 hour. The reaction solution was concentrated to obtain crude product. The crude product was recrystallized with ether to obtain target compound **UB-1810968d** (2.5g, 96% yield). LC-MS: (M+H) ⁺=440.2

### Step 3: UB-1810968f (V2141-059)

Compound **UB-1810968d** (500mg, 1.1mmol) and **UB-1810968e** (200mg, 1.1mmol) were dissolved in ethanol(20mL), and the mixture was reacted at 100°C under microwave for 1 hour. The reaction solution was concentrated to obtain target product. LC-MS: (M+H) ⁺=595.3

### Step 4: UB-1810968g (V2141-063)

**Compound UB-1810968d** (1g, 1.68mmol) was dissolved in DMSO (30mL), I₂ (428mg, 1.68mmol) and K₂CO₃ (700mg, 5.04mmol) were added. The mixture was reacted at 80°C for 16 hours. The reaction solution was concentrated, then purified by silica gel column (DCM/MeOH=20/1) to obtain target product **UB-1810968d** (450mg, 45.3% yield). LC-MS: (M+H) ⁺=593.5

### Step 5: UB-1810968h (V2141-069)

Compound **UB-1810968g** (180mg, 0.3mmol) was dissolved in DCM (3mL), BF₃ Et₂O (0.3mL) was added, and the mixture was reacted at room temperature for 2 hours. The reaction solution was added NaHCO₃ aqueous solution, and extracted with dichloromethane (10 mL*3). The organic phases were combined, dried and concentrated to give the crude product. The crude product was purified by silica gel column to obtain target product **UB-1810968h** (150mg, 100% yield) as a white solid. LC-MS: (M+H) ⁺=493.5

### Step 6: UB-1810968k (V2141-076)

**UB-1810968i** (2g, 10.8mmol), **UB-1810968j** (2g, 5.4mmol), Pd(PPh3)2Cl2 (378mg, 0.54mmol), and CuI (205mg, 1.08mmol) were dissolved in TEA (1.6mL) and DMF (60mL), and the mixture was reacted at 40°C for 16 hours under N₂ protection. The reaction solution was concentrated to obtain crude product. The crude product was purified by silica gel column (DCM/MeOH=10/1) to obtain target product **UB-1810968k** (2g, 43.3% yield) as a white solid. LC-MS: M+H) ⁺= 430.5

### Step 7: UB-1810968i (V2141-079)

**UB-1810968k** (2g, 4.6mmol) was dissolved in THF, concentrated hydrochloric acid (3mL) and water 12 (12mL) were added, and the mixture was reacted at 40°C for 2 hours. Then NaHCO₃ aqueous solution was added till pH=7. The mixture was extracted with ethyl acetate(120mL*3), and the organic phase was dried and concentrated to obtain crude product. The crude product was isolated and purified by silica gel column (PE/EA=1/1) to obtain target product **UB-1810968i** (850mg, 52.5% yield) as a white solid. LC-MS: M+H) ⁺= 355.

### Step 8: UB-1810968 (V2141-081)

**UB-1810968h** (150mg, 0.28mmol), and **UB-1810968i** (100mg, 0.28mmol) were dissolved in DCM (40mL) and MeOH (4mL). 1 drop of acetic acid was added and the reaction was carried out at 40°C for 16 h. Then NaBH³CN (40 mg, 0.56 mmol) was added. The mixture was reacted at 40°C for 2 hours. The reaction solution was added with saturated brine (2mL), concentrated to obtain the crude product. The crude product was isolated and purified by silica gel column (DCM/MeOH=20/1) to obtain target product **UB-1810968** (30mg, 12% yield) as a white solid. LC-MS: M+H) ⁺= 885

### Synthesis of Compound UB-181148(V2537-150)

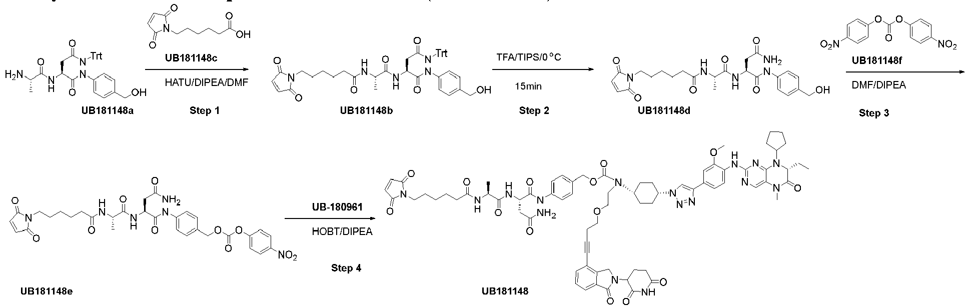

### Step 1: UB-181148b (V2790-025)

**UB-181148c,** and HOBT were dissolved in DMF, then DIEA (0.96g, 7.4 mmol) was added. The reaction solution was cooled to 0 °C, then **UB-181148a and** EDCI in DMF were added dropwise, and the mixture was reacted at room temperature for 7 hours. The reaction solution was poured into water, the mixture was extracted with ethyl acetate, the obtained organic phase was washed once with diluted hydrochloric acid and saturated brine, and dried over anhydrous sodium sulfate, combined organic phase was evaporated to dryness in vacuum to obtain **UB-181148b** (80 mg, 60% yield) as a yellow solid. LCMS [M+H] ⁺ =744.

### Step 2: UB-181148d (V2537-147)

Compound **UB-181148b** (1g, 13mmol) was dissolved in TIPS (6mL), TFA (10mL) was added at 0°C. The mixture was reacted at 0°Cfor 15 minutes, then NaHCO₃ aqueous solution (120mL water) was added till PH=6. The reaction solution was filtered, and the filtrate was directly purified via reversed-phase column chromatography to obtain target product **UB-181148d** (620mg, yield 92.5%) as a white solid. M+H= 502

### Step 3: UB-181148e (V2537-149)

Compound **UB-181148d** (120mg, 0.24mmol), and **UB-181148d** (73mg, 0.24mmol) were dissolved in DIPEA (62mg, 0.48mmol) and DMF (2mL), and the mixture was reacted at 30°C for 16 hours. The reaction solution was concentrated to obtain crude product. The crude was added with ether to be washed, the mixture was filtered to obtain solid, and the solid was washed with ethyl acetate to obtain the white target product **UB-181148e** (50 mg, 31% yield). LC-MS: (M+H) ⁺= 667

### Step 4: UB-181148 (V2537-150)

Compound **UB-181148e** (50mg, 0.075mmol), **UB-180961** (70mg, 0.075mmol), and HOBT (20mg, 0.15mmol) were dissolved in DIPEA (29mg, 0.225mmol) and DMF (3mL), the mixture was reacted at 30°C for 16 hours, and 1 drop of HCl (0.5M) was added to the reaction solution to adjust PH=6. The mixture was directly purified by prep-HPLC to obtain target compound **UB-181148** (12mg, yield 11.3 %) as a white solid. (M+H) ⁺= 707,1412

### Synthesis of Compound UB-181145

### Step 1: UB-1811450 (V2777-048)

### General Method 3:

¹H NMR (400 MHz, DMSO-*d*6) δ 11.01 (s, 1H), 8.90 (d, *J* = 18.2 Hz, 3H), 8.08 (s, 1H), 7.94 - 7.25 (m, 7H), 7.03 (d, *J* = 48.4 Hz, 2H), 5.25 - 5.04 (m, 2H),4.81 - 4.60 (m, 2H), 4.52 - 4.38 (m, 2H), 4.32 (d, *J=* 17.8 Hz, 1H), 3.96 (s, 3H), 3.80 (s, 3H), 3.69 (t, *J=* 6.8 Hz, 2H), 3.23 (s, 3H), 3.16 (s, 2H), 2.92 (s, 2H),2.78 (d, *J=* 6.9 Hz, 2H), 2.11 - 1.70 (m, 10H), 0.67 (t, *J* = 7.4 Hz, 3H). LCMS [M+H]⁺ = 946.9

### Synthesis of Compound UB-181153

### Step 1: UB-181153 (V2777-055), General Method 3

LCMS [M+H]⁺ = 855.9

### Synthesis of Compound UB-181154

### Step 1: UB-181154 (V2777-064), General Method 1

¹H NMR (400 MHz, DMSO-*d*6) δ 11.02 (s, 1H), 10.01 (s, 1H), 9.71 (s, 1H), 9.17 (s, 3H), 8.95 (s, 2H), 8.84 (d, *J* = 2.6 Hz, 1H), 8.50 - 8.28 (m, 2H),7.93 (d, *J* = 8.7 Hz, 1H), 7.86 (s, 1H), 7.73 (d, *J* = 7.5 Hz, 1H), 7.66 (d, *J* = 7.5 Hz, 1H), 7.54 (t, *J* = 7.6 Hz, 1H), 5.16 (dd, *J* = 13.3, 5.1 Hz, 1H), 4.52 -4.31 (m, 3H), 4.25 (dd, *J=* 7.7, 3.6 Hz, 1H), 3.25 (s, 3H), 3.13 (t, *J* = 3.7 Hz, 4H), 3.06 (d, *J=* 2.4 Hz, 2H), 2.83 - 2.78 (m, 2H), 2.62 (d, *J=* 3.6 Hz,1H), 2.43 (d, *J=* 12.7 Hz, 1H), 2.12 (s, 2H), 2.00 (td, *J* = 9.6, 8.9, 4.6 Hz, 4H), 1.87 (d, *J* = 12.0 Hz, 3H), 1.76 (q, *J* = 7.2 Hz, 3H), 1.60 - 1.44 (m, 7H),0.77 (t, *J* = 7.4 Hz, 3H). LCMS [M+H]⁺ = 831.9

### Synthesis of Compound UB-181171

### Step 1: UB-181171 (V2777-063), General Method 1

LCMS [M+H]⁺ = 879.5

### Synthesis of Compound UB-181177

### Step 1: UB-181177 (V2777-078)

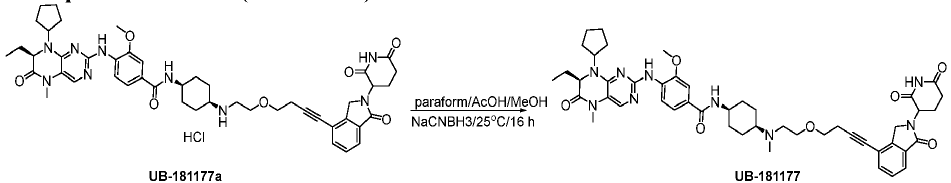

**UB-181177a** (20 mg, 0.02 mmol) was dissolved in methanol (2 mL), acetic acid(0.1 mL) and paraformaldehyde(3.5 mg, 0.12mmol) were added, and the mixture was reacted at room temperature for 1 hour. Sodium cyanoborohydride (2.9mg, 0.05mmol) was added, and the mixture was reacted for another 16 hours. The reaction solution was concentrated and subjected to thin-layer chromatography (MeOH /DCM =10%) to obtain **UB-181185** (5.8 mg, yield 30%) as a white solid.

¹H NMR (400 MHz, DMSO-*d*6) δ 11.01 (s, 1H), 10.52 (s, 1H), 8.30 (s, 1H), 7.97 (s, 1H), 7.85 (s, 1H), 7.72 (d, *J=* 7.6 Hz, 1H), 7.65 (d, *J=* 7.7 Hz, 1H), 7.58- 7.38 (m, 3H), 5.24 - 5.09 (m, 1H), 4.40 (dd, *J=* 59.2, 18.1 Hz, 4H), 4.00 (d, *J=* 40.8 Hz, 6H), 3.69 (t, *J=* 6.6 Hz, 2H), 3.64 - 3.47 (m, 2H), 3.11 (s, 2H),2.90 (d, *J* = 14.9 Hz, 1H), 2.77 (dd, *J* = 13.2, 5.8 Hz, 5H), 2.62 (s, 1H), 1.94 (s, 4H), 1.87 (s, 4H), 1.70 (s, 2H), 1.29 (dd, *J=* 14.2, 6.7 Hz, 8H). LCMS [M+H]⁺ =874.9

### Synthesis method of Compound UB-181178

### Step 1: UB-181178 (V2777-079)

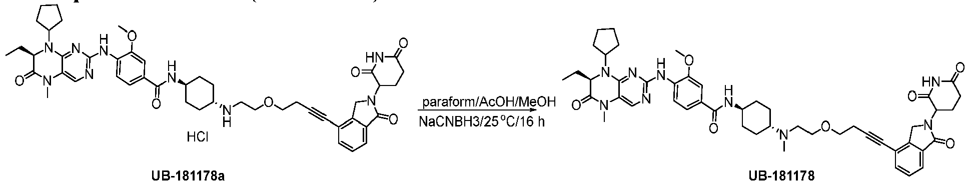

**UB-181178a** (5 mg, 0.01mmol) was dissolved in methanol (2 mL), acetic acid(0.1 mL), and paraformaldehyde(1 mg, 0.03mmol) were added, and the mixture was reacted at room temperature for 1 hour. Sodium cyanoborohydride (1mg, 0.1mmol) was added, and the mixture was reacted for another 16 hours. The reaction solution was concentrated and subjected to thin-layer chromatography (MeOH /DCM =10%) to obtain **UB-181178** (2.4 mg, yield 48%) as a white solid. LCMS [M+H]⁺ =874.9

As used herein Compound No.UB-18XXXX, can also be simplified to No. XXXX, for example **UB-181052 is Compound 1052.** Other compounds shown in Table A3 were prepared by similar methods.

**Table A3**

| **Example** | **Structure and Data analysis** |
|---|---|
| **938** | 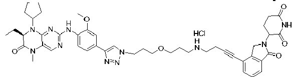 |
| | ¹H NMR (400 MHz, DMSO-*d*₆) δ 11.01 (s, 1H), 9.51 (s, 1H), 9.20 (s, 1H), 9.04 (s, 1H), 8.97 - 8.87 (m, 1H), 7.84 - 7.60 (m, 5H), 7.57 - 7.45 (m, 2H), 5.16 (m, 1H), 4.97 (m, 1H), 4.53 (m, 3H), 4.32 (m, 1H), 4.06 (m, 2H), 3.94 - 3.89 (m, 3H), 3.83 (m, 2H), 3.21 (s, 3H), 2.94 (m, 2H),2.92 (m, 3H), 1.86 (m, 8H), 1.61 - 1.53 (m, 2H), 1.42 (m, 4H), 1.26 - 1.03 (m, 5H), 0.74 (t, *J* = 7.3 Hz, 3H). LCMS [M+H]⁺ = 857 |
| **939** | 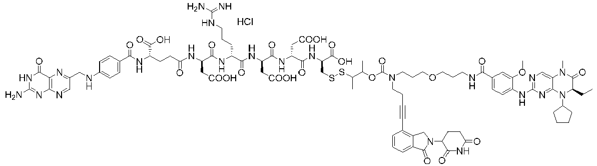 |
| | LCMS [M/2+H]⁺ =1005.9, LCMS \|M/3+H]⁺ =671 |
| **949** | 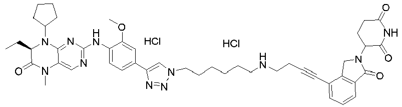 |
| | ¹H NMR (400 MHz, DMSO-*d*₆) δ 13.46 - 12.78 (m, 1H), 11.02 (s, 1H), 9.71 (s, 1H), 9.35 (m, 2H), 8.74 (d, *J* = 1.2 Hz, 1H), 7.82 - 7.65 (m, 4H), 7.62 - 7.44 (m, 3H), 5.16 (dd, *J* = 13.3, 5.1 Hz, 1H), 4.58 - 4.46 (m, 2H), 4.44 - 4.31 (m, 3H), 4.08 (q, *J* = 8.9 Hz, 1H), 3.90 (s, 3H), 3.21 (s, 3H), 3.15 (q, *J* = 6.7 Hz, 2H), 2.94 (m, 5H), 2.64 - 2.58 (m, 1H), 2.44 (m, 1H), 2.07 - 1.96 (m, 2H), 1.94 - 1.74 (m, 7H), 1.67 (t, *J* = 7.6 Hz, 2H), 1.37 (m, 8H), 0.74 (t, *J* = 7.4 Hz, 3H). LCMS [M+H]⁺ = 842.6 |
| **968** | 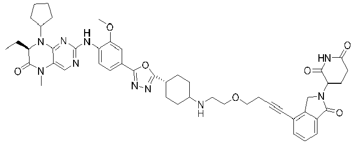 |
| | LC-MS: M+H) ⁺= 885 |
| **969** | 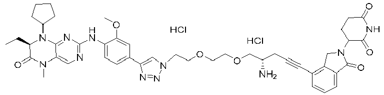 |
| | ¹H NMR (400 MHz, DMSO-*d*₆) δ 11.03 (s, 1H), 9.66 (s, 1H), 8.67 (s, 1H), 8.41 (d, *J* = 5.6 Hz, 3H), 7.80 - 7.65 (m, 4H), 7.59 - 7.44 (m, 3H), 5.16 (dd, *J* = 13.4, 5.2 Hz, 1H), 4.59 (t, *J* = 5.2 Hz, 2H), 4.54 - 4.45 (m, 2H), 4.35 (m, 1H), 4.06 (m, 1H), 3.90 (m, 5H), 3.69 (m, 3H), 3.61 (s, 3H), 3.21 (s, 3H), 2.98 - 2.83 (m, 3H), 2.64 - 2.57 (m, 1H), 2.43 - 2.35 (m, 1H), 1.88 (m, 8H), 1.49 - 1.32 (m, 4H), 0.74 (t, *J* = 7.4 Hz, 3H). LCMS [M+H]⁺ = 860.5 |
| **977** | 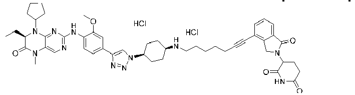 |
| | ¹H NMR (400 MHz, DMSO-*d*₆) δ 11.01 (s, 1H), 9.50 (s, 2H), 8.94 (s, 1H), 8.76 (s, 2H), 7.82 - 7.70 (m, 3H), 7.68 - 7.59 (m, 2H), 7.57 - 7.47 (m, 2H), 5.16 (dd, *J* = 13.2, 5.1 Hz, 1H), 4.74 (s, 1H), 4.48 (d, *J* = 17.6 Hz, 2H), 4.33 (d, *J* = 17.7 Hz, 1H), 4.11 (t, *J* = 8.9 Hz, 1H), 3.92 (s, 3H), 3.26 (m, 4H),3.23 (s, 3H), 2.99 - 2.89 (m, 3H), 2.60 (m, 1H), 2.46 (m, 1H), 2.10 - 1.59 (m, 18H), 1.45 (d, *J* = 38.9 Hz, 6H), 0.76 (t, *J* = 7.4 Hz, 3H). LCMS [M+H]⁺ = 883.0 |
| **978** | 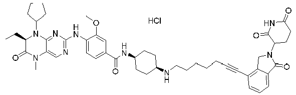 |
| | ¹H NMR (400 MHz, DMSO-*d*₆) δ 11.01 (s, 1H), 8.64 (br, 2H), 7.95 (m, 3H), 7.81 (s, 1H), 7.73 (d, *J* = 7.5 Hz, 1H), 7.65 (d, *J* = 7.5 Hz, 1H), 7.56 (m, 3H), 5.21 - 5.15 (m, 1H), 4.48 (m, 2H), 4.33 (d, *J* = 17.7 Hz, 1H), 4.24 (m, 1H), 3.95 (m, 4H), 3.24 (s, 3H), 3.16 (m, 2H), 2.95 (m, 3H), 2.60 (m, 1H), 2.46 (m, 1H), 2.08 - 1.78 (m, 14H), 1.75 - 1.59 (m, 8H), 1.52 (m, 4H), 0.77 (t, *J* = 7.4 Hz, 3H). LCMS [M+H]⁺ = 858.9 |
| **979** | 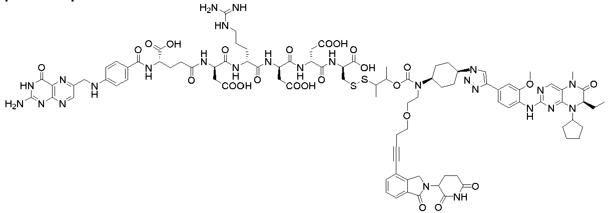 |
| | LCMS [M/3]⁺ = 687.8 ¹H NMR: NA |
| **984** | 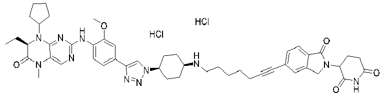 |
| | ¹H NMR (400 MHz, DMSO-*d*₆) δ 13.08 (s, 1H), 11.01 (s, 1H), 9.62 (s, 1H), 8.96 (m, 3H), 7.76 - 7.68 (m, 3H), 7.66 - 7.61 (m, 2H), 7.56 - 7.49 (m, 2H), 5.16 (dd, *J* = 13.3, 5.1 Hz, 1H), 4.71 (m, 1H), 4.51 - 4.44 (m, 2H), 4.32 (d, *J* = 17.8 Hz, 1H), 3.91 (s, 3H), 3.80 (t, *J* = 5.3 Hz, 2H), 3.69 (t, *J* = 6.7 Hz, 2H), 3.32 (m, 1H), 3.21 (s, 3H), 3.16 (m, 2H), 2.97 - 2.89 (m, 1H), 2.79 (t, *J* = 6.7 Hz, 2H), 2.59 (m, 1H), 2.44 (m, 1H), 2.05 - 1.73 (m, 14H), 1.46 (m, 2H), 1.41 - 1.33 (m, 2H), 0.75 (t, *J* = 7.4 Hz, 3H). LCMS [M+H]⁺ = 884.6 |
| **985** | 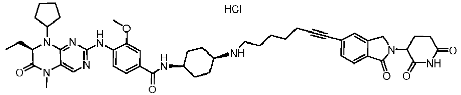 |
| | ¹H NMR (400 MHz, DMSO-*d*₆) δ 11.00 (s, 1H), 8.67 (br, 3H), 7.95 (br, 2H), 7.80 (d, *J* = 2.2 Hz, 1H), 7.72 - 7.66 (m, 1H), 7.63 (s, 1H), 7.57 (m, 2H), 7.51 (d, *J* = 7.9 Hz, 1H), 5.11 (dd, *J* = 13.3, 5.1 Hz, 1H), 4.44 (m, 2H), 4.31 (d, *J* = 17.5 Hz, 1H), 4.22 (m, 1H), 3.94 (m, 4H), 3.23 (s, 3H), 3.13 (m, 2H), 2.93 (m, 3H), 2.60 (m, 1H), 2.42 - 2.37 (m, 1H), 2.05 - 1.76 (m, 14H), 1.61 (m, 8H), 1.51 (m, 4H), 0.76 (t, *J* = 7.4 Hz, 3H). LCMS [M+H]⁺ = 858.8 |
| **992** | 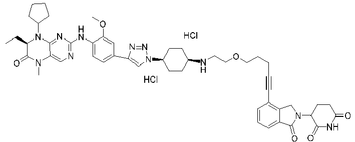 |
| | ¹H NMR (400 MHz, DMSO-*d*₆) δ 12.8 (m, 1H), 11.00 (s, 1H), 9.48 (m, 1H), 8.91 (m, 1H), 8.78 (m, 2H), 7.72 (m, 3H), 7.67 - 7.58 (m, 2H), 7.54 - 7.45 (m, 2H), 5.15 (dd, *J* = 13.2, 5.2 Hz, 1H), 4.72 (m, 1H), 4.51 - 4.42 (m, 2H), 4.31 (m, 1H), 4.10 (m, 1H), 3.91 (s, 3H), 3.70 (m, 2H), 3.59 (m, 2H), 3.22 (s, 3H), 3.16 (m, 2H), 2.97 - 2.88 (m, 1H), 2.58 (m, 4H), 2.45 (m, 4H), 2.06 - 1.93 (m, 6H), 1.89 - 1.71 (m, 8H), 1.44 (m, 4H), 0.75 (t, *J* = 7.4 Hz, 3H). |
| **993** | 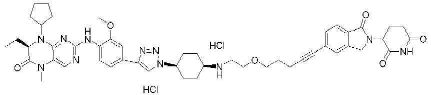 |
| | ¹H NMR (400 MHz, DMSO-*d*₆) δ 11.00 (s, 1H), 9.48 (br, 2H),8.87 (m, 1H), 8.69 (m, 2H), 7.81 - 7.66 (m, 3H), 7.62 (d, *J* = 8.7 Hz, 2H), 7.51 (dt, *J* = 7.9, 2.1 Hz, 2H), 5.11 (dd, *J* = 13.3, 5.1 Hz, 1H), 4.71 (m, 1H), 4.44 (m, 2H), 4.31 (d, *J* = 17.5 Hz, 1H), 4.10 (m, 1H), 3.91 (s, 3H), 3.69 (m, 2H), 3.59 (m, 2H), 3.22 (s, 3H), 3.16 (m, 2H), 2.95 - 2.87 (m, 1H), 2.64 - 2.53 (m, 6H), 2.38 (m, 2H), 2.00 (m, 6H), 1.82 (m, 8H), 1.48 (m, 4H), 0.75 (t, *J* = 7.4 Hz, 3H). LCMS [M+H]⁺ = 898.8 |
| **998** | 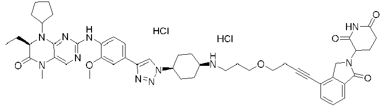 |
| | ¹H NMR (400 MHz, DMSO-*d*₆) δ 12.97 (br, 1H), 11.01 (s, 1H), 9.65 (s, 1H), 9.01 (s, 3H), 7.80 - 7.60 (m, 5H), 7.58 - 7.47 (m, 2H), 5.15 (dd, *J* = 13.3, 5.1 Hz, 1H), 4.72 (m, 1H), 4.52 - 4.41 (m, 2H), 4.32 (d, *J* = 17.7 Hz, 1H), 4.09 (q, *J* = 8.9 Hz, 1H), 3.91 (s, 3H), 3.62 (t, *J* = 6.6 Hz, 2H), 3.57 (d, *J* = 5.8 Hz, 2H), 3.21 (m, 4H), 3.04 - 2.87 (m, 3H), 2.75 (t, *J* = 6.6 Hz, 2H), 2.63 - 2.57 (m, 1H), 2.48 - 2.38 (m, 3H), 2.06 - 1.74 (m, 15H), 1.53 - 1.34 (m, 4H), 0.75 (t, *J* = 7.4 Hz, 3H). LCMS [M+H]⁺ = 898.8 |
| **999** | 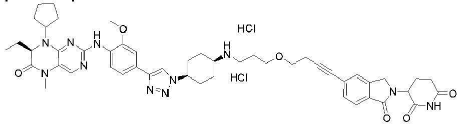 |
| | ¹H NMR (400 MHz, DMSO-*d*₆) δ 13.00 (br, 1H), 11.00 (s, 1H), 9.65 (s, 1H), 8.99 (m, 3H), 7.78 -7.60 (m, 5H), 7.51 (dt, *J* = 8.1, 2.1 Hz, 2H), 5.11 (dd, *J* = 13.3, 5.1 Hz, 1H), 4.78 - 4.69 (m, 1H), 4.52 - 4.40 (m, 2H), 4.32 (m, 1H), 4.11 (d, *J* = 8.9 Hz, 1H), 3.91 (s, 3H), 3.61 (t, *J* = 6.6 Hz, 2H), 3.56 (t, *J* = 6.0 Hz, 2H), 3.21 (m, 4H), 3.05 - 2.97 (m, 2H), 2.94 - 2.85 (m, 1H), 2.73 (t, *J* = 6.5 Hz, 2H), 2.63 - 2.56 (m, 1H), 2.48 - 2.35 (m, 3H), 2.04 - 1.74 (m, 15H), 1.50 - 1.33 (m, 4H), 0.75 (t, *J* = 7.4 Hz, 3H). LCMS [M+H]⁺ = 898.8 |
| **1004** | 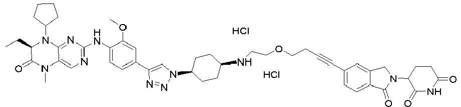 |
| | ¹H NMR (400 MHz, DMSO-*d*₆) δ 12.8 (br, 1H), 11.00 (s, 1H), 9.58 (s, 1H), 8.92 (m, 3H), 7.78 - 7.62 (m, 4H), 7.51 (m, 2H), 5.11 (dd, *J* = 13.3, 5.1 Hz, 1H), 4.72 (m, 1H), 4.53 - 4.40 (m, 2H), 4.32 (d, *J* = 17.5 Hz, 1H), 4.09 (m, 1H), 3.91 (s, 3H), 3.79 (t, *J* = 5.2 Hz, 2H), 3.68 (t, *J* = 6.6 Hz, 2H), 3.32 (m, 2H), 3.19 (m, 5H), 2.91 (m, 2H), 2.78 (t, *J* = 6.6 Hz, 2H), 2.65 - 2.55 (m, 2H), 2.47 - 2.36 (m, 2H), 1.99 (m, 6H), 1.90 - 1.73 (m, 6H), 1.53 - 1.34 (m, 4H), 0.75 (t, *J* = 7.4 Hz, 3H). LCMS [M+H]⁺ = 885.0 |
| **1045** | 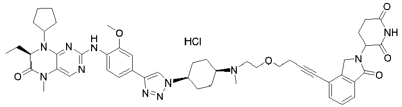 |
| | ¹H NMR (400 MHz, DMSO-*d*₆) δ 11.01 (s, 1H), 10.51 (s, 1H), 9.70 (s, 1H), 8.91 (s, 1H), 7.81 - 7.61 (m, 5H), 7.57 - 7.49 (m, 2H), 5.16 (dd, *J* = 13.3, 5.1 Hz, 1H), 5.11 - 5.00 (m, 1H), 4.72 (q, *J* = 3.5 Hz, 1H), 4.53 - 4.44 (m, 2H), 4.33 (d, *J* = 17.8 Hz, 1H), 4.07 (t, *J* = 8.8 Hz, 1H), 3.91 (m, 5H), 3.69 (m, 2H), 3.37 - 3.33 (m, 1H), 3.21 (m, 5H), 2.97 - 2.87 (m, 1H), 2.80 (m, 2H), 2.73 (d, *J* = 4.9 Hz, 3H), 2.66 - 2.56 (m, 1H), 2.49 - 2.42 (m, 1H), 2.18 - 1.64 (m, 14H), 1.52 - 1.32 (m, 4H), 0.75 (t, *J* = 7.4 Hz, 3H). LCMS [M/2]⁺ = 450.3 |
| **1052** | 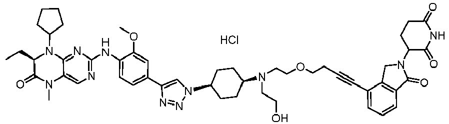 |
| | ¹H NMR (400 MHz, DMSO-*d*₆) δ 11.01 (s, 1H), 9.64 (s, 1H), 8.91 (s, 1H), 8.78 (br, 1H), 7.80 (s, 1H), 7.77 - 7.70 (m, 1H), 7.68 - 7.60 (m, 2H), 7.53 (m, 2H), 7.38 (m, 1H), 5.16 (dd, *J* = 13.3, 5.2 Hz, 1H), 4.71 (br, 1H), 4.52 - 4.42 (m, 2H), 4.32 (m, 3H), 3.84 (s, 3H), 3.78 (t, *J* = 5.2 Hz, 1H), 3.70 (m, 2H), 3.40 - 3.37 (m, 3H), 3.32 (m, 3H), 3.30 (m, 1H), 3.21 (s, 3H), 3.18 (m, 2H), 2.79 (t, *J* = 6.7 Hz, 2H), 2.62 (m, 1H), 2.43 (m, 1H), 2.06 - 1.73 (m, 12H), 1.57 (m, 2H), 1.07 (m, 4H), 0.72 (t, *J* = 7.4 Hz, 3H). LCMS [M/2]⁺ = 465.2. |
| **1063** | 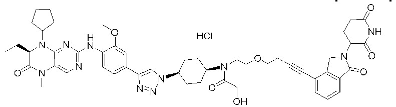 |
| | ¹H NMR (400 MHz, DMSO-*d*₆) δ 13.8 (br, 1H), 11.04 - 10.94 (s, 1H), 9.60 (s, 1H), 8.80 (d, *J* = 11.3 Hz, 1H), 7.79 - 7.44 (m, 7H), 5.13 (dd, *J* = 13.4, 5.1 Hz, 1H), 4.69 (m, 1H), 4.51 - 4.37 (m, 2H), 4.28 (d, *J* = 17.7 Hz, 1H), 4.17 (s, 1H), 4.10 (m, 3H), 3.90 (d, *J* = 2.7 Hz, 3H), 3.62 (m, 2H), 3.50 (m, 4H), 3.21 (m, 5H), 2.96 - 2.86 (m, 1H), 2.64 (m, 3H), 2.44 (m, 2H), 2.14 - 1.69 (m, 11H), 1.62 (m, 2H), 1.42 (m, 4H), 0.74 (t, *J* = 7.4 Hz, 3H). LCMS [M+H]⁺ = 943.0 |
| **1089** | 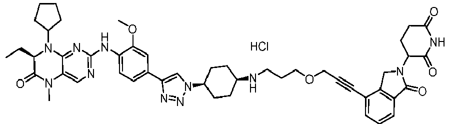 |
| | ¹H NMR (400 MHz, DMSO-*d*₆) δ 11.01 (s, 1H), 9.59 (s, 1H), 8.99 (br, 3H), 7.80 - 7.67 (m, 4H), 7.63 - 7.55 (m, 2H), 7.51 (m, 1H), 5.15 (dd, *J* = 13.2, 5.1 Hz, 1H), 4.74 (m, 1H), 4.55 - 4.44 (m, 4H), 4.37 (d, *J* = 17.8 Hz, 1H), 4.08 (t, *J* = 8.8 Hz, 1H), 3.91 (s, 3H), 3.65 (m, 2H), 3.21 (s, 3H), 3.06 - 2.88 (m, 4H), 2.60 (m, 1H), 2.48 - 2.42 (m, 2H), 2.06 - 1.74 (m, 16H), 1.41 (m, 4H), 0.75 (t, *J* = 7.4 Hz, 3H). LCMS [M/2]⁺ = 443.3 |
| **1099** | 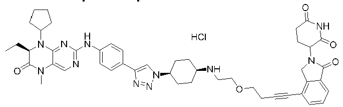 |
| | ¹H NMR (400 MHz, DMSO-*d*₆) δ 11.01 (s, 1H), 10.32 (s, 1H), 8.84 (m, 3H), 7.87 (d, *J* = 8.3 Hz, 2H), 7.75 (d, *J* = 11.6 Hz, 2H), 7.67 - 7.50 (m, 4H), 5.16 (dd, *J* = 13.3, 5.1 Hz, 1H), 4.69 (m, 1H), 4.51 - 4.42 (m, 2H), 4.32 (d, *J* = 17.7 Hz, 1H), 4.23 (t, *J* = 8.8 Hz, 1H), 3.79 (t, *J* = 5.2 Hz, 2H), 3.69 (t, *J* = 6.7 Hz, 2H), 3.22 (s, 3H), 3.16 (t, *J* = 5.7 Hz, 2H), 2.93 (m, 1H), 2.79 (m, 2H), 2.63 - 2.56 (m, 1H), 2.49 - 2.37 (m, 2H), 2.08 - 1.68 (m, 14H), 1.59 (m, 2H), 1.47 (m, 2H), 0.77 (t, *J* = 7.4 Hz, 3H). LCMS [M/2]⁺ = 428.2 |
| **1108** | 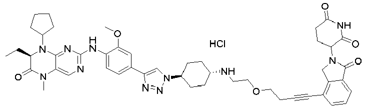 |
| | ¹H NMR (400 MHz, DMSO-*d*₆) δ 11.02 (s, 1H), 9.50 (s, 1H), 8.97 (s, 2H), 8.69 (s, 1H), 7.79 - 7.63 (m, 4H), 7.60 - 7.43 (m, 3H), 5.17 (dd, *J* = 13.3, 5.1 Hz, 1H), 4.58 - 4.44 (m, 3H), 4.33 (d, *J* = 17.8 Hz, 1H), 4.09 (m, 1H), 3.91 (s, 3H), 3.80 (t, *J* = 5.1 Hz, 2H), 3.73 (t, *J* = 6.7 Hz, 2H), 3.22 (s, 6H), 2.93 (m, 1H), 2.82 (t, *J* = 6.7 Hz, 2H), 2.61 (m, 1H), 2.48 - 2.43 (m, 1H), 2.26 (m, 4H), 2.09 - 1.75 (m, 10H), 1.72 - 1.60 (m, 2H), 1.53 - 1.35 (m, 4H), 0.75 (t, *J* = 7.4 Hz, 3H). LCMS [M/2]⁺ = 443.2 |
| **1113** | 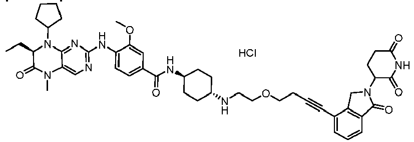 |
| | ¹H NMR (400 MHz, DMSO-*d*₆) δ 11.02 (s, 1H), 8.89 (m, 3H), 8.30 (m, 1H), 7.91 (s, 1H), 7.85 - 7.79 (m, 1H), 7.73 (d, *J* = 7.5 Hz, 1H), 7.66 (d, *J* = 7.5 Hz, 1H), 7.59 - 7.50 (m, 3H), 5.17 (dd, *J* = 13.3, 5.1 Hz, 1H), 4.47 (m, 2H), 4.33 (d, *J* = 17.8 Hz, 1H), 4.24 - 4.17 (m, 1H), 3.91 (s, 3H), 3.79 (t, *J* = 5.2 Hz, 2H), 3.71 (t, *J* = 6.7 Hz, 2H), 3.22 (s, 3H), 3.16 (m, 2H), 3.05 (m, 2H), 2.93 (t, *J* = 12.8 Hz, 1H), 2.81 (t, *J* = 6.7 Hz, 2H), 2.64 - 2.54 (m, 2H), 2.47 - 2.39 (m, 1H), 2.14 (m, 2H), 1.93 (m, 4H), 1.81 (m, 4H), 1.47 (m, 8H), 0.76 (t, *J* = 7.4 Hz, 3H). LCMS [M/2]⁺ = 431.2 |
| **1118** | 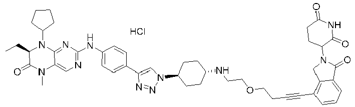 |
| | ¹H NMR (400 MHz, DMSO-*d*₆) δ 11.02 (s, 1H), 10.38 (s, 1H), 9.03 (brs, 2H), 8.61 (s, 1H), 7.85 (d, *J* = 8.2 Hz, 2H), 7.80 - 7.70 (m, 2H), 7.66 (d, *J* = 7.4 Hz, 1H), 7.62 - 7.50 (m, 3H), 5.17 (dd, *J* = 13.3, 5.1 Hz, 1H), 4.56 - 4.44 (m, 3H), 4.34 (d, *J* = 17.7 Hz, 1H), 4.22 (p, *J* = 8.9 Hz, 1H), 3.80 (d, *J* = 5.4 Hz, 2H), 3.72 (t, *J* = 6.7 Hz, 2H), 3.21 (m, 5H), 2.99 - 2.89 (m, 1H), 2.82 (m, 2H), 2.64 - 2.57 (m, 1H), 2.48 - 2.43 (m, 1H), 2.26 (m, 4H), 2.08 - 1.76 (m, 10H), 1.65 (m, 4H), 1.47 (m, 2H), 0.77 (t, *J* = 7.4 Hz, 3H). LCMS [M/2]⁺ = 428.1 |
| **1126** | 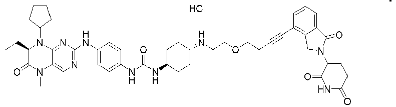 |
| | ¹H NMR (400 MHz, DMSO-*d*₆) δ 11.02 (s, 1H), 9.79 (s, 1H), 8.75 (s, 2H), 8.63 (s, 1H), 7.73 (d, *J* = 7.5 Hz, 1H), 7.68 - 7.60 (m, 2H), 7.54 (t, *J* = 7.6 Hz, 1H), 7.39 (d, *J* = 8.5 Hz, 2H), 7.30 (d, *J* = 8.6 Hz, 2H), 6.26 (d, *J* = 7.4 Hz, 1H), 5.17 (dd, *J* = 13.3, 5.1 Hz, 1H), 4.45 (m, 2H), 4.32 (d, *J* = 17.7 Hz, 1H), 4.13 (t, *J* = 8.9 Hz, 1H), 3.82 - 3.67 (m, 4H), 3.20 (s, 3H), 3.15 (m, 2H), 3.03 (m, 1H), 2.94 (m, 1H), 2.80 (t, *J* = 6.7 Hz, 2H), 2.60 (m, 1H), 2.47 - 2.41 (m, 1H), 2.14 - 1.71 (m, 12H), 1.56 (m, 2H), 1.46 (m, 4H), 1.22 - 1.11 (m, 2H), 0.75 (t, *J* = 7.4 Hz, 3H). LCMS [M/2]⁺ = 423.6 |
| **1130** | 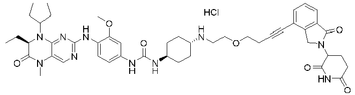 |
| | ¹H NMR (400 MHz, DMSO-*d*₆) δ 11.02 (s, 1H), 8.79 (brs, 3H), 7.73 (dd, *J* = 7.6, 1.1 Hz, 1H), 7.68 - 7.51 (m, 3H), 7.41 (d, *J* = 2.2 Hz, 1H), 7.29 (d, *J* = 8.3 Hz, 1H), 6.85 (dd, *J* = 8.6, 2.2 Hz, 1H), 6.35 (d, *J* = 7.5 Hz, 1H), 5.17 (dd, *J* = 13.3, 5.1 Hz, 1H), 4.47 (m, 2H), 4.32 (d, *J* = 17.7 Hz, 1H), 4.00 (m, 1H), 3.77 (t, *J* = 5.2 Hz, 2H), 3.72 (m, 4H), 3.39 (d, *J* = 4.2 Hz, 1H), 3.29 (m, 1H), 3.19 (s, 3H), 3.14 (m, 2H), 3.03 (m, 1H), 2.97 - 2.89 (m, 1H), 2.80 (t, *J* = 6.7 Hz, 2H), 2.61 (m, 1H), 2.47 - 2.42 (m, 1H), 1.92 (m, 12H), 1.53 - 1.30 (m, 6H), 1.23 - 1.14 (m, 2H), 0.73 (t, *J=* 7.4 Hz, 3H). LCMS [M/2]⁺ = 438.2 |
| **1143** | 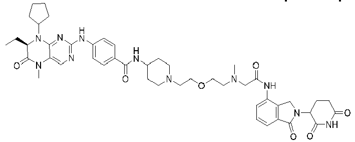 |
| | ¹H NMR (400 MHz, DMSO-*d*₆) δ 11.02 (s, 1H), 9.74 (s, 1H), 9.30 (s, 1H), 7.92 (d, *J* = 7.7 Hz, 1H), 7.87 - 7.70 (m, 6H), 7.56 - 7.49 (m, 2H), 5.14 (dd, *J* = 13.3, 5.1 Hz, 1H), 4.48 - 4.33 (m, 3H), 4.22 (dd, *J* = 7.9, 3.6 Hz, 1H), 3.67 (d, *J* = 7.6 Hz, 1H), 3.53 (m, 4H), 3.25 (m, 4H), 2.97 - 2.75 (m, 4H), 2.68 (m, 2H), 2.62 (m, 1H), 2.40 (m, 5H), 2.08 - 1.89 (m, 6H), 1.84 - 1.67 (m, 7H), 1.62 (m, 3H), 1.48 (t, *J* = 11.0 Hz, 2H), 0.77 (t, *J* = 7.4 Hz, 3H). LCMS [M/2+H]⁺ = 440.1 |
| **1144** | 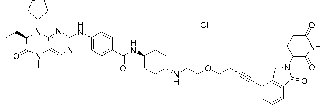 |
| | ¹H NMR (400 MHz, DMSO-*d*₆) δ 11.01 (s, 1H), 10.24 (s, 1H), 8.87 - 8.73 (m, 2H), 7.95 - 7.78 (m, 4H), 7.73 (d, *J* = 7.5 Hz, 1H), 7.65 (d, *J* = 7.9 Hz, 3H), 7.53 (t, *J* = 7.6 Hz, 1H), 5.16 (dd, *J* = 13.2, 5.1 Hz, 1H), 4.51 - 4.39 (m, 2H), 4.36 - 4.28 (m, 2H), 3.93 (q, *J* = 4.3 Hz, 1H), 3.79 (t, *J* = 5.2 Hz, 2H), 3.71 (t, *J* = 6.7 Hz, 2H), 3.23 (s, 3H), 3.15 (d, *J* = 6.6 Hz, 4H), 2.97 - 2.89 (m, 1H), 2.81 (t, *J* = 6.7 Hz, 2H), 2.60 (d, *J* = 14.7 Hz, 1H), 2.47 - 2.42 (m, 1H), 2.06 - 1.80 (m, 12H), 1.69 - 1.50 (m, 6H), 0.77 (t, *J* = 7.4 Hz, 3H). LCMS [M/2+H]⁺ = 416.1. |
| **1145** | 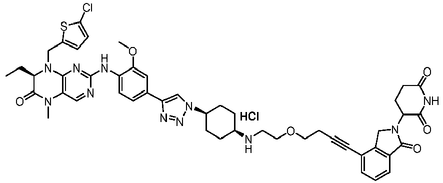 |
| | 1H NMR (400 MHz, DMSO-d6) δ 11.01 (s, 1H), 8.90 (d, J = 18.2 Hz, 3H), 8.08 (s, 1H), 7.94 - 7.25 (m, 7H), 7.03 (d, J = 48.4 Hz, 2H), 5.25 - 5.04 (m, 2H),4.81 - 4.60 (m, 2H), 4.52 - 4.38 (m, 2H), 4.32 (d, J = 17.8 Hz, 1H), 3.96 (s, 3H), 3.80 (s, 3H), 3.69 (t, J = 6.8 Hz, 2H), 3.23 (s, 3H), 3.16 (s, 2H), 2.92 (s, 2H),2.78 (d, J = 6.9 Hz, 2H), 2.11 - 1.70 (m, 10H), 0.67 (t, J = 7.4 Hz, 3H). LCMS [M+H]+ = 946.9 |
| **1148** | 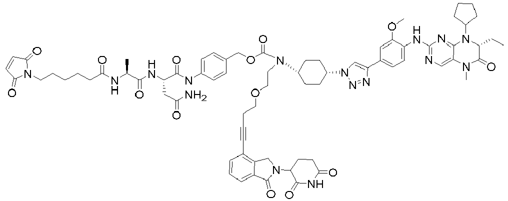 |
| | LC-MS: (M+H) ⁺= 707,1412 |
| | ¹H NMR (400 MHz, DMSO-*d*₆) δ 11.01 (s, 1H), 10.24 (s, 1H), 8.87 - 8.73 (m, 2H), 7.95 - 7.78 (m, 4H), 7.73 (d, *J* = 7.5 Hz, 1H), 7.65 (d, *J* = 7.9 Hz, 3H), 7.53 (t, *J* = 7.6 Hz, 1H), 5.16 (dd, *J* = 13.2, 5.1 Hz, 1H), 4.51 - 4.39 (m, 2H), 4.36 - 4.28 (m, 2H), 3.93 (q, *J* = 4.3 Hz, 1H), 3.79 (t, *J* = 5.2 Hz, 2H), 3.71 (t, *J* = 6.7 Hz, 2H), 3.23 (s, 3H), 3.15 (d, *J* = 6.6 Hz, 4H), 2.97 - 2.89 (m, 1H), 2.81 (t, *J* = 6.7 Hz, 2H), 2.60 (d, *J* = 14.7 Hz, 1H), 2.47 - 2.42 (m, 1H), 2.06 - 1.80 (m, 12H), 1.69 - 1.50 (m, 6H), 0.77 (t, *J* = 7.4 Hz, 3H). LCMS [M/2+H]⁺ = 416.1. |
| **1153** | 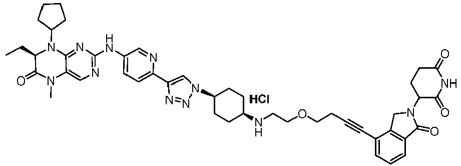 |
| | LCMS [M+H]⁺ = 855.9 |
| **1154** | 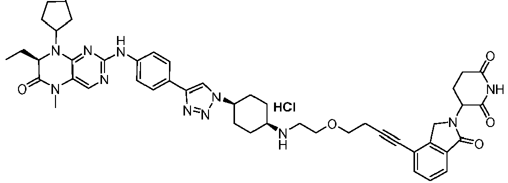 |
| | ¹H NMR (400 MHz, DMSO-*d*6) δ 11.02 (s, 1H), 10.01 (s, 1H), 9.71 (s, 1H), 9.17 (s, 3H), 8.95 (s, 2H), 8.84 (d, *J* = 2.6 Hz, 1H), 8.50 - 8.28 (m, 2H),7.93 (d, *J* = 8.7 Hz, 1H), 7.86 (s, 1H), 7.73 (d, *J* = 7.5 Hz, 1H), 7.66 (d, *J* = 7.5 Hz, 1H), 7.54 (t, *J* = 7.6 Hz, 1H), 5.16 (dd, *J* = 13.3, 5.1 Hz, 1H), 4.52 -4.31 (m, 3H), 4.25 (dd, *J* = 7.7, 3.6 Hz, 1H), 3.25 (s, 3H), 3.13 (t, *J* = 3.7 Hz, 4H), 3.06 (d, *J* = 2.4 Hz, 2H), 2.83 - 2.78 (m, 2H), 2.62 (d, *J* = 3.6 Hz, 1H), 2.43 (d, *J* = 12.7 Hz, 1H), 2.12 (s, 2H), 2.00 (td, *J* = 9.6, 8.9, 4.6 Hz, 4H), 1.87 (d, *J* = 12.0 Hz, 3H), 1.76 (q, *J* = 7.2 Hz, 3H), 1.60 - 1.44 (m, 7H),0.77 (t, *J* = 7.4 Hz, 3H). LCMS [M+H]⁺ = 831.9 |
| **1171** | 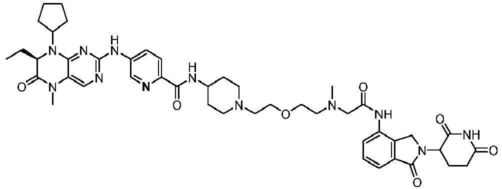 |
| | LCMS [M+H]⁺ = 879.5 |
| **1175** | 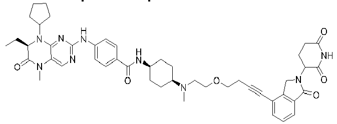 |
| | ¹H NMR (400 MHz, DMSO-*d*₆) δ 11.02 (s, 1H), 9.30 (s, 1H), 7.87 - 7.78 (m, 4H), 7.78 - 7.70 (m, 3H), 7.67 - 7.62 (m, 1H), 7.53 (t, *J* = 7.6 Hz, 1H), 5.16 (dd, *J* = 13.3, 5.1 Hz, 1H), 4.50 - 4.40 (m, 2H), 4.32 (d, *J* = 17.7 Hz, 1H), 4.22 (dd, *J* = 7.8, 3.6 Hz, 1H), 3.93 (d, *J* = 65.6 Hz, 3H), 3.67 (d, *J* = 7.9 Hz, 2H), 3.38 (s, 1H), 3.25 (s, 3H), 2.99 - 2.88 (m, 1H), 2.78 (s, 3H), 2.64 - 2.58 (m, 1H), 2.47 - 2.40 (m, 1H), 2.09 - 1.89 (m, 6H), 1.86 - 1.71 (m, 8H), 1.62 (dt, *J* = 14.6, 4.0 Hz, 6H), 1.35 (s, 2H), 0.77 (t, *J* = 7.5 Hz, 3H). LCMS [M/2+H]⁺ = 423.1 |
| **1176** | 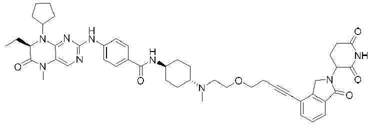 |
| | ¹H NMR (400 MHz, DMSO-*d*₆) δ 11.02 (s, 1H), 9.31 (s, 1H), 8.01 (s, 1H), 7.85 (s, 1H), 7.80 (d, *J* = 9.0 Hz, 2H), 7.76 - 7.70 (m, 3H), 7.65 (dd, *J* = 7.6, 1.1 Hz, 1H), 7.53 (t, *J* = 7.6 Hz, 1H), 5.17 (dd, *J* = 13.3, 5.1 Hz, 1H), 4.46 (d, *J* = 17.8 Hz, 2H), 4.32 (d, *J* = 17.7 Hz, 1H), 4.22 (dd, *J* = 7.7, 3.6 Hz, 1H), 3.77 (d, *J* = 58.0 Hz, 5H), 3.25 (s, 4H), 2.94 (td, *J* = 13.0, 12.5, 6.7 Hz, 1H), 2.78 (d, *J* = 8.3 Hz, 4H), 2.62 (s, 1H), 2.43 (d, *J* = 4.4 Hz, 1H), 2.09 - 1.91 (m, 8H), 1.77 (d, *J* = 15.1 Hz, 6H), 1.62 (dt, *J* = 14.6, 7.0 Hz, 5H), 1.42 (d, *J* = 12.2 Hz, 3H), 0.77 (t, *J* = 7.4 Hz, 3H). LCMS [M/2+H]⁺ = 423.1 |
| **1177** | 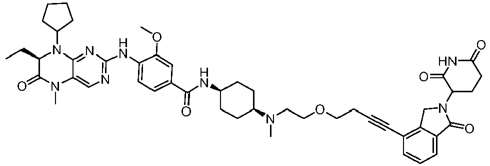 |
| | ¹H NMR (400 MHz, DMSO-*d*6) δ 11.01 (s, 1H), 10.52 (s, 1H), 8.30 (s, 1H), 7.97 (s, 1H), 7.85 (s, 1H), 7.72 (d, *J* = 7.6 Hz, 1H), 7.65 (d, *J* = 7.7 Hz, 1H), 7.58- 7.38 (m, 3H), 5.24 - 5.09 (m, 1H), 4.40 (dd, *J* = 59.2, 18.1 Hz, 4H), 4.00 (d, *J* = 40.8 Hz, 6H), 3.69 (t, *J* = 6.6 Hz, 2H), 3.64 - 3.47 (m, 2H), 3.11 (s, 2H),2.90 (d, *J* = 14.9 Hz, 1H), 2.77 (dd, *J* = 13.2, 5.8 Hz, 5H), 2.62 (s, 1H), 1.94 (s, 4H), 1.87 (s, 4H), 1.70 (s, 2H), 1.29 (dd, *J* = 14.2, 6.7 Hz, 8H). LCMS [M+H]⁺ =874.9 |
| **1178** | 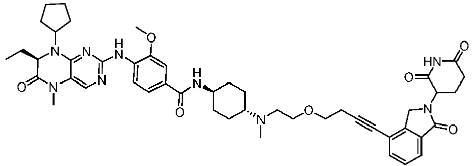 |
| | LCMS [M+H]⁺ =874.9 |

### Synthesis method of Compound UB-181193 (PEG-Ala-Ala-Asn-PAB-961)

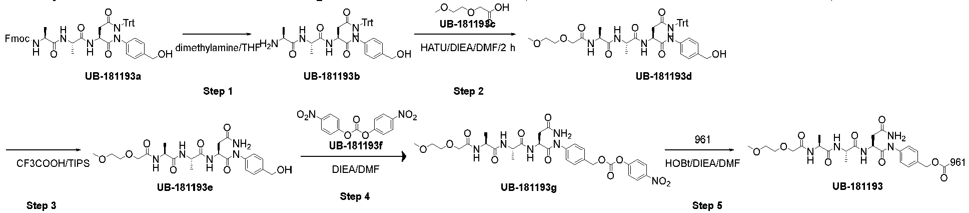

### Step 1: UB-181193b (V2790-095)

**UB-181193a(7.5** g, 8.89mmol) was dissolved in THF(150 ml), then **dimethylamine**(20 ml) was added, and the mixture was reacted at room temperature for 2 h. The reaction solution was dried in vacuum to obtain crude product, then it was slurried with ether to obtain **UB-181193b** (1.0 g, 80% yield) as a yellow solid. LCMS: [M+H] ⁺ = 622.6

### Step 2: UB-181193d (V2790-99)

**UB-181193b** (2.7 g, 4.3 mmol), **UB-181193c**(580 mg, 4.3 mmol) and HATU(2.1 g, 6.5 mmol) were dissolved in DMF(15 ml), then DIEA(1.5 g, 13 mmol) was added and the mixture was reacted at room temperature for 7 hours. The reaction solution was poured into water, the mixture was extracted with ethyl acetate, the obtained organic phase was washed once with diluted hydrochloric acid and saturated brine, and dried over anhydrous sodium sulfate, and combined organic phase was evaporated to dryness in vacuum and purified via silica gel chromatography (DCMIDCM:MeOH:THF(10:0.5:0.5)=0-96%) to obtain **UB-181193d** (1.4 g, 50 % yield) as a white solid. LCMS: [M+H] ⁺ =738.6.

### Step 3: UB-181193e (V2790-101)

**UB-181193d** was dissolved in TIS, cooled to 0 °C, then CF3COOH was added, and the mixture was reacted at 0 °C for 15min. The reaction solution was added NaHCO3 aqueous solution. The reaction solution was filtered, and purified via silica gel chromatography (H2O: acetonitrile =0%-12%) to obtain **UB-181193e** (128 mg, 30% yield) as a white solid. LCMS: [M+H] ⁺ =496.4.

### Step 4: UB-181193g (V2790-103)

**UB-181193e** (128 mg, 0.23mmol) and **UB-181193f** (158 mg, 0.46mmol) were dissolved in DMF(2 ml), then DIPEA (90 mg, 0.69mmol) was added, and the mixture was reacted at room temperature for 18 h. The reaction solution was dried by rotary dryer under vacuum, and slurried three times with ether to obtain **UB-181193g** (150 mg, 75% yield) as a white solid. LCMS: [M+H]⁺ =661.5

### Step 5: UB-181193 (V2790-104)

**UB-181193g** (150 mg, 0.23mmol), 961 (228 mg, 0.22mmol), HOBt (65 mg, 0.46mmol), and DIPEA (95 mg, 0.69mmol) were dissolved in DMF (2 mL), and the mixture was reacted at room temperature for 18 hours. The reaction solution was purified by HPLC to obtain **UB-181193** (120 mg, 45% yield) as a white solid. LCMS: [M+H] ⁺ = 1407.2

### Synthesis method of Compound UB-181210(M-PEG4-VC-PAB-961)

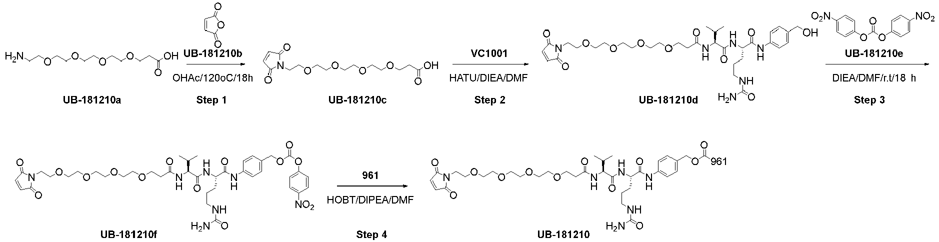

### Step 1: UB-181210c (V2790-134)

**UB-181210a** (1.11 g, 11.3 mmol) and **UB-181210b** (2 g, 7.55 mmol) were dissolved in acetic acid(15 ml), and the mixture was reacted at 120°C for 18h. The reaction solution was dried by rotary dryer under vacuum, and subjected to silica gel column chromatography (water: acetonitrile =0%-100%) to obtain **UB-181210c** (400mg, 75% yield) as a colorless oil. LCMS: [M+H] ⁺ =346.3

### Step 2: UB-181210d (V2790-140)

To a solution of **UB-181210c** (560 mg, 1.5 mmol), VC1001(585 mg, 1.5 mmol) and HATU (1.5 g, 3.8 mmol) in DMF (5 ml) was added DIEA(600 mg, 4.6 mmol). The reaction solution was stirred at room temperature for 2 hours. The reaction solution was dried by rotary dryer under vacuum, and purified via silica gel column chromatography (DCM/MeOH=0-30%) to obtain **UB-181210d** 200 mg, 55% yield). LCMS: [M+H]⁺ =707.7

### Step 3: UB-181210f (V2790-144)

**UB-181210d(200** mg, 0.28 mmol) and **UB-181210e** (172 mg, 0.56 mmol) were dissolved in DMF(2.5 ml), then DIPEA (110 mg, 0.85 mmol) was added, and the mixture was reacted at room temperature for 18 h. The reaction solution was dried by rotary dryer under vacuum, and purified via thin-layer chromatography (DCM/MeOH=10/1) to obtain **UB-181210f** (100 mg, 75% yield) as a yellow solid. LCMS: [M+H] ⁺ = 872.7

### Step 4: UB-181210 (V2790-145)

**UB-181210f** (100 mg, 0.11 mmol), 961 (110 mg, 0.11 mmol), HOBt (31 mg, 0.22 mmol), and DIPEA (45 mg, 0.33 mmol) were dissolved in DMF (5 mL), and the mixture was reacted at room temperature for 18 h. The reaction solution was purified by HPLC to obtain **UB-181210** (90mg, 50% yield) as a white solid. LCMS: [M+H] ⁺ =1618.6

### Synthesis method of Compound UB-181211(M-PEG6-VC-PAB-961)

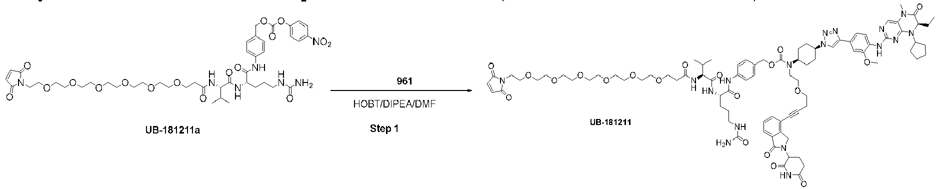

### Step 1: UB-181211 (V2891-089)

Compound **UB-181211a** (60 mg, 0.06 mmol) was dissolved in DMF (1 mL), then **961** (55 mg, .0.06 mmol), HOBT (8 mg, 0.036 mmol) and DIEA (16 mg, 0.13 mmol) were added, and the mixture was reacted at room temperature for 2 days. The reaction solution was subjected to reversed-phase column chromatography (MeCN/H₂O=40%) to obtain target product **UB-181210** (50 mg, 47% yield) as a white solid. LCMS [M+H]⁺ =1706.7

### Synthesis method of Compound UB-181212(Mal-PEG8-VC-PAB-937)

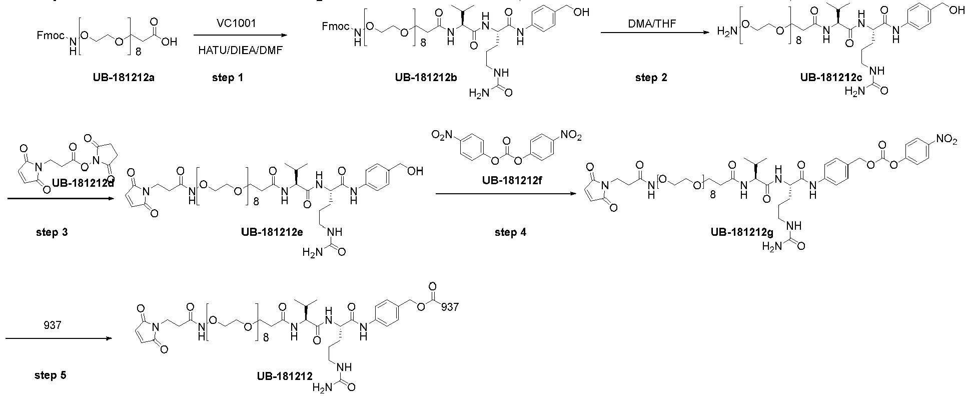

### Step 1: UB-181212b (V2891-084)

Compound **UB-181212a** (220 mg, 0.33 mmol) was dissolved in DMF (10 mL), then HATU (189 mg, 0.50 mmol) and DIEA (128 mg, 1.0 mmol) were added, and the mixture was reacted at room temperature for 1 hour, followed by adding **VC1001** (125 mg, 0.33 mmol) and continued to react at room temperature for 2 hours. The reaction solution was concentrated, then subjected to reversed-phase column chromatography (collected by MeCN/H₂O=30-40%) to obtain white target product **UB-181212b** (270 mg, 79% yield). LCMS [M+H]⁺ = 1026.1

### Step 2: UB-181212c (V2891-085)

Compound **UB-181212b** (30 mg, 0.03 mmol) was dissolved in THF (3 mL), then DMA/THF (5 mL) was added. Then the mixture was reacted at 40°C for 2 hours. The reaction solution was concentrated to obtain white crude product **UB-181212c** (20 mg, yield 87%). LCMS [M+H]⁺ = 803.8

### Step 3: UB-181212e (V2891-086)

Compound **UB-181212c** (200 mg, 0.25 mmol) was dissolved in DMF (5 mL), then **UB-181212d** (56 mg, 0.25 mmol) and DIEA (48 mg, 0.37 mmol) were added, and the mixture was reacted at room temperature for 2 hours. The reaction solution was concentrated, then subjected to reversed-phase column chromatography to obtain white target product **UB-181212e** (270 mg, 100% yield). LCMS [M+H]⁺ = 955.0

### Step 4: UB-181212g (V2891-092)

Compound **UB-181212e** (110 mg, 0.12 mmol) was dissolved in DMF (3 mL), then **UB-181212f** (105 mg, 0.35 mmol) and DIEA (30 mg, 0.23 mmol) were added. The mixture was reacted at room temperature for 2 days, then concentrated and isolated by column chromatography (MeOH/DCM=1/7) to obtain target product **UB-181212g** (50 mg, 39% yield) as a white solid. LCMS [M+H]⁺ = 1119.8

### Step 5: UB-181212 (V2891-093)

Compound **UB-181212g** (50 mg, 0.04 mmol) was dissolved in DMF (1 mL), then **937** (38 mg, 0.04 mmol), HOBT (6 mg, 0.04 mmol) and DIEA (12 mg, 0.09 mmol) were added, and the mixture was reacted at 30°C overnight. The reaction solution was prepared to obtain target product **UB-181212g(24** mg, yield 29%) as a white solid. LCMS [M+H]⁺ =1841.7

### Synthesis method of Compound UB-181229 (Octreotide - Cys - M - PEG4 - VC - PAB - 961)

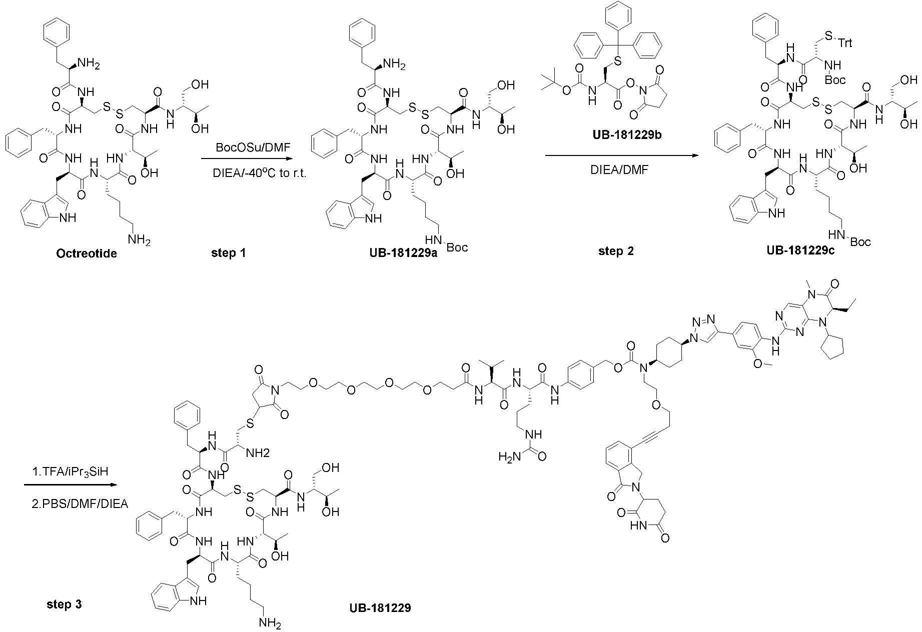

### Step 1: UB-181229a (V2891-099)

Compound **Octreotide** (750 mg, 0.70 mmol) and DIEA (179 mg, 1.39 mmol) were dissolved in DMF (10 mL), then the mixture was cooled to -40°C followed by slowly adding BocOSu (149 mg, 0.70 mmol), then slowly warmed to room temperature and reacted for 2 hours. The reaction solution was concentrated, then subjected to reversed-phase column chromatography (MeCN/0.1% AcOH in H₂O=5-95%, collected at 40 %) to obtain target product **UB-181229a** (1 g, 100% yield) as a white solid. LCMS [M+H]⁺ = 1120.0

### Step 2: UB-181229c (V2891-101)

Compound **UB-181229a** (200 mg, 0.18 mmol) was dissolved in DMF (5 mL), then **UB-181229b** (100 mg, 0.18 mmol) and DIEA (35 mg, 0.27 mmol) were added, and the mixture was reacted at room temperature overnight. The reaction solution was subjected to reversed-phase column chromatography (MeCN/0.5% AcOH in H₂O=5-95%, collected at 40%) to obtain target product **UB-181229c** (130 mg, 47% yield) as a white solid. LCMS [M+H]⁺ = 1565.5

### Step 3: UB-181229 (V2891-100)

Compound **UB-181229a** (25 mg, 0.02 mmol) was dissolved in TFA (0.8 mL), then a catalytic amount of iPr₂SiH was added, and the mixture was reacted at room temperature for 5 minutes. The reaction solution was dried by an oil pump, then dissolved in DMF (1 mL) and DIEA (0.2 mL), and then **UB-181229d** (26 mg, 0.02 mmol) was added and the mixture was reacted at room temperature overnight. The reaction solution was prepared to obtain product **UB-181229**(17.3 mg, yield 39 %) as a white solid. LCMS [M/2+H] ⁺ = 1370.8

### Synthesis method of Compound UB-181233 (Octreotide-C-S-S-961)

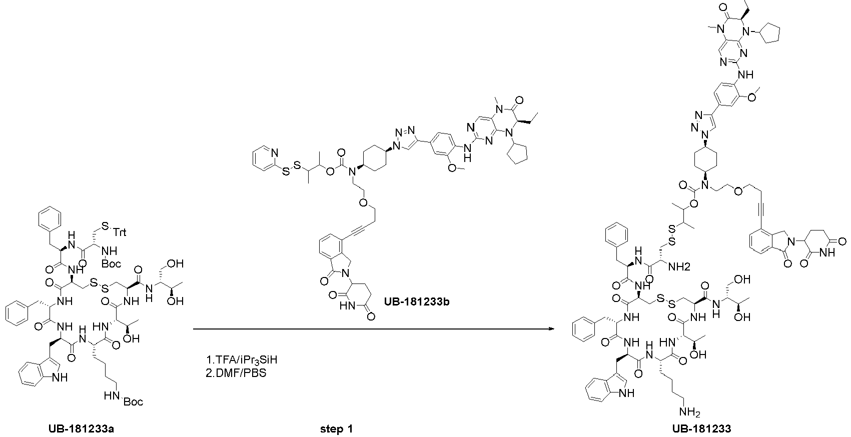

### Step 1: UB-181233 (V2891-103)

Compound **UB-181233a** (25 mg, 0.02 mmol)was dissolved in TFA (0.8 mL), then a catalytic amount of iPr₂SiH was added, and the mixture was reacted at room temperature for 5 minutes. The reaction solution was dried by an oil pump, then dissolved in DMF (1 mL) and DIEA (0.2 mL), and then **UB-181233b** (18 mg, 0.02 mmol) was added and the mixture was reacted at room temperature overnight. The reaction solution was prepared to obtain product **UB-UB-181233(4.4** mg, yield 13%) as a white solid. LCMS [M/2+H] ⁺ = 1069.2

As used herein Compound No.UB-18XXXX, can also be simplified to No. XXXX, for example **UB-181229 is Compound 1229.** Other compounds shown in Table D were prepared by similar methods.

**Table D**

| | |
|---|---|
| **1193** | 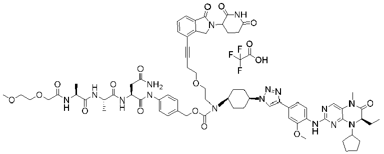 |
| | PEG-AAN-PAB-961 |
| | LCMS [M+H]⁺ = 1407.2 |
| **1201** | 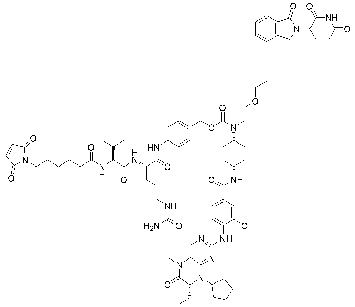 |
| | MC-VC-PAB-937 |
| | LCMS [M+H]⁺ = 1459.5 |
| **1209** | 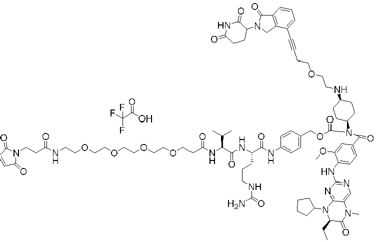 |
| | Mal-4PEG-VC-PAB-937 |
| | LCMS [M+H]⁺ = 1664.6 |
| **1210** | 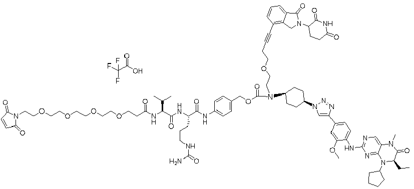 |
| | M-4PEG-VC-PAB-961 |
| | LCMS [M+H]⁺ = 1618.6 |
| **1211** | 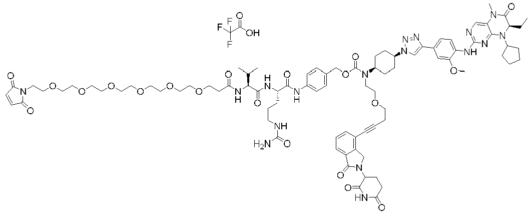 |
| | M-6PEG-VC-PAB-961 |
| | LCMS [M+H]⁺ = 1706.7 |
| **1212** | 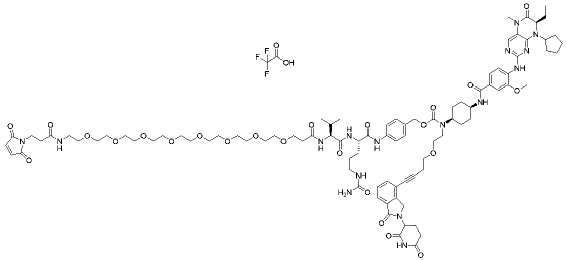 |
| | Mal-8PEG-VC-PAB-937 |
| | LCMS [M+H]⁺ =1841.7 |
| **1215** | 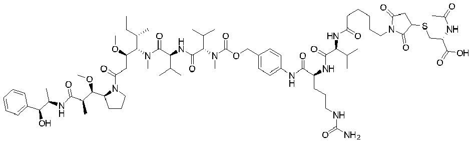 |
| | N-acetyl-L-cysteine-MC-VC-PAB-MMAE |
| | LCMS [M+H]⁺ = 1480.6 |
| **1216** | 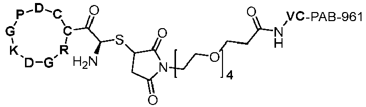 |
| | iRGD-C-M-PEG4-VC-PAB-961 |
| | LCMS [M+H]⁺ = 1334.9 |
| **1228** | 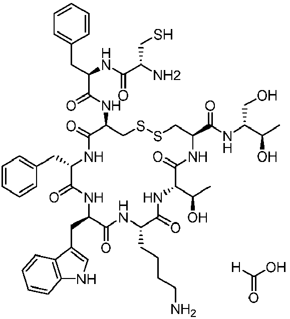 |
| | LCMS [M+H]⁺ =1122.8 |
| **1229** | 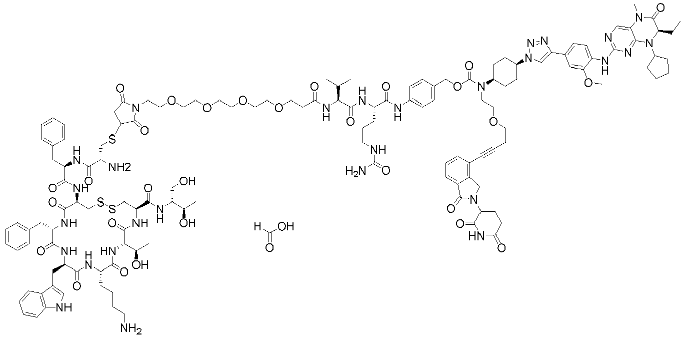 |
| | LCMS [M/2+H]⁺ =1370.8 |
| **1231** | 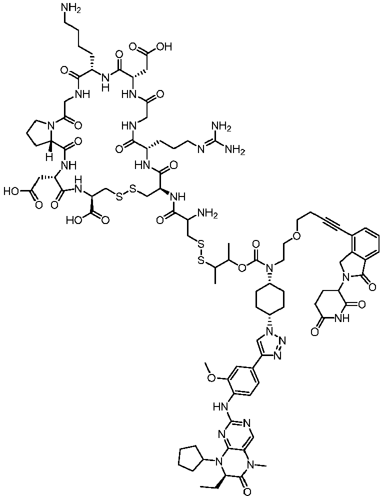 |
| | LCMS [M/2+H]⁺ = 1033.6 |
| **1233** | 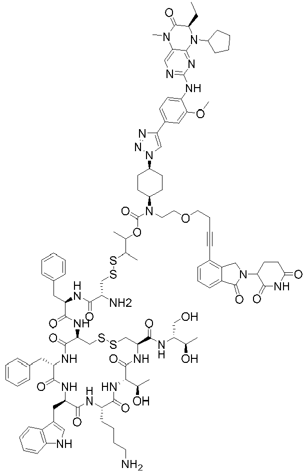 |
| | LCMS [M/2+H]⁺ =1069.2 |
| **1234** | 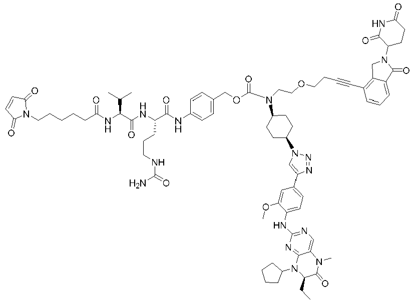 |
| | LCMS [M+H]⁺ =1482.9 |

### Test Example

### Test Example 1 Cell Proliferation Experiment

Reagents: RPMI-1640 medium, McCoy's 5A medium, IMDM medium, MEM medium, L-15 medium, fetal bovine serum, Penicillin-Streptomycin double antibody, trypsin, etc., 2-mercaptoethanol, NEAA, pyruvate, etc.

Some of the cell lines used in this experiment are listed in Table 1 below.

**Table1. List of Cell Lines**

| **No.** | **Cell Name** | **Cell Source** |
|---|---|---|
| 1 | Human Colon Cancer HT-29 | Shanghai Cell Bank of Chinese Academy of Sciences (CAS) |
| 2 | Human Colon Cancer HCT-116 | Shanghai Cell Bank of Chinese Academy of Sciences (CAS) |
| 3 | Human Small Cell Lung Cancer NCI-H82 | American Type Culture Collection (ATCC) |
| 4 | Human Monocytic Leukemia THP1-1 | Shanghai Cell Bank of Chinese Academy of Sciences (CAS) |
| 5 | Human Acute Myelo-monocytic Leukemia HL-60 | Shanghai Cell Bank of Chinese Academy of Sciences (CAS) |
| 6 | Human Glioma Cells U-87 MG | Shanghai Cell Bank of Chinese Academy of Sciences (CAS) |
| 7 | Human cervical adenocarcinoma cells Hela | Shanghai Cell Bank of Chinese Academy of Sciences (CAS) |
| 8 | Human Breast Cancer MDA-MB-231 | Shanghai Cell Bank of Chinese Academy of Sciences (CAS) |
| 9 | Human Acute Lymphoblastic Leukemia Cells MOLT-4 | Shanghai Cell Bank of Chinese Academy of Sciences (CAS) |
| 10 | Human Ovarian Cancer SK-OV-3 | Shanghai Cell Bank of Chinese Academy of Sciences (CAS) |
| 11 | Human Monocytic Leukemia HL-60 | Shanghai Cell Bank of Chinese Academy of Sciences (CAS) |
| 12 | Human Monocytic Leukemia MV4-11 | American Type Culture Collection (ATCC) |
| 13 | Human umbilical vein endothelial cells HUVEC | Allcells |
| 14 | Human peripheral blood mononuclear cells PBMC | Extracted from peripheral blood of healthy volunteers |
| 15 | Human lymphocytic leukemia cells Daudi | Shanghai Cell Bank of Chinese Academy of Sciences (CAS) |
| 16 | Human prostate cancer cells PC-3 | Shanghai Cell Bank of Chinese Academy of Sciences (CAS) |

The cells were cultured in conventional ways, and the cells should be passed at least 2 generations before plating. Cells at the logarithmic growth phase were collected, prepared into single cell suspensions and counted. The concentration of cells was adjusted to the desired concentration, and cells were inoculated into a 96-well cell culture plate at 100 µl per well. 100 µL of complete medium of the test compound were added to each well, set up 2 duplicate wells for each concentration, and diluted with a 5-fold gradient, and continued to culture for 72 h. All cells were subjected to IC₅₀ test for corresponding samples. The experimental results are shown in **Test Example 4.**

The fluorescence intensity of each well was detected using the Alarm blue method, and IC₅₀ was calculated.

IC₅₀ was calculated according to the following formula: $Y = Max + \left(Min-Max\right) / \left[1+\left(X/{IC}_{50}\right)\times Slope\right]$ wherein Min, Max and Slope represent the minimum, maximum and slope respectively.

### Test Example 2 Western Blot

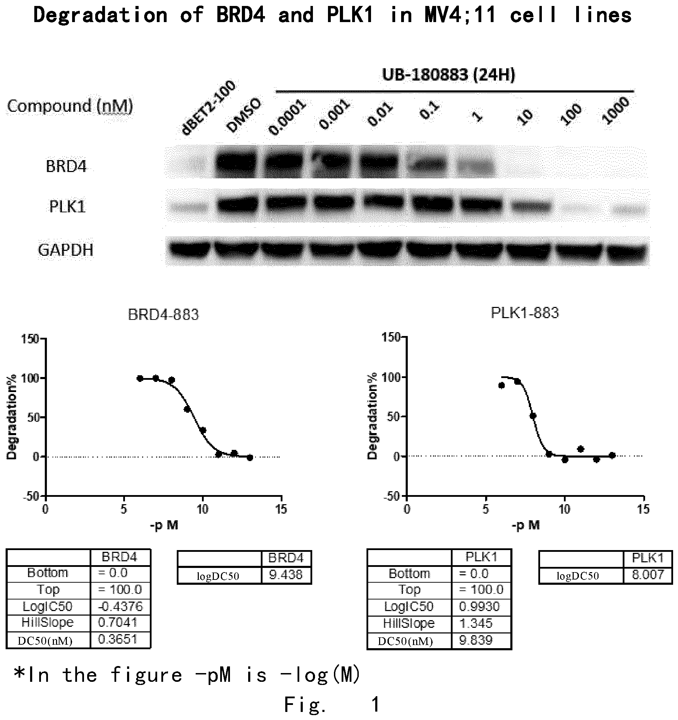

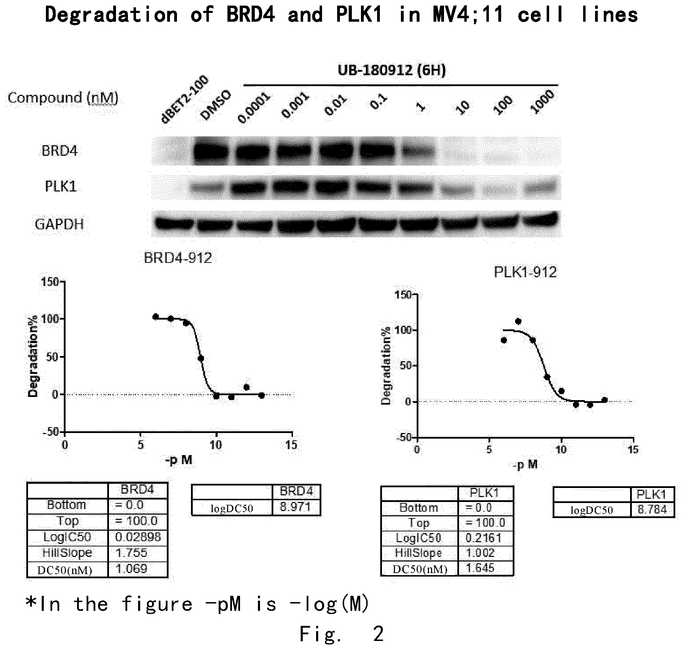

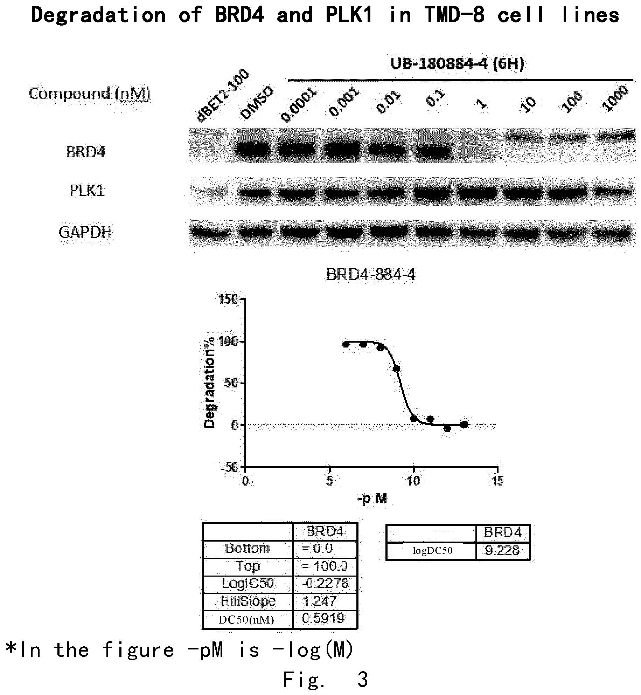

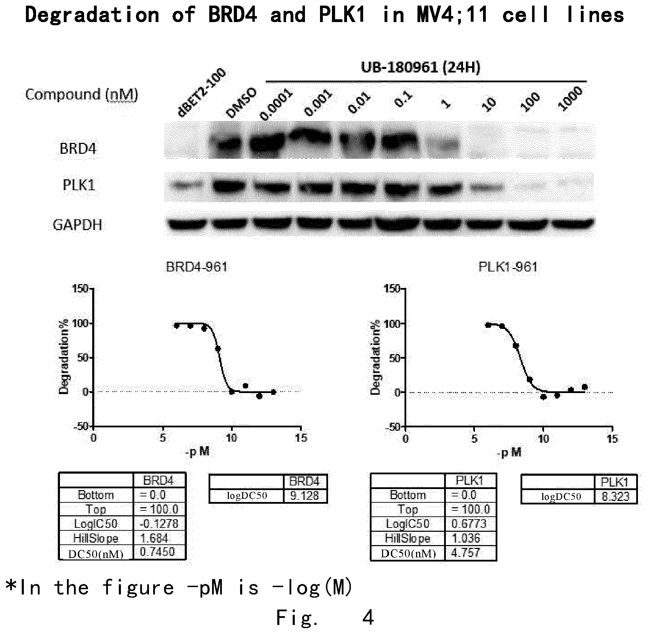

Cells were treated with the compound for a period of time, after that the cells were collected by centrifugation. After washing with PBS, the cells were lysed by adding RIPA buffer; the cell lysate were added to the loading buffer and then appropriate volume were taken and slowly added to the corresponding wells of the gel plate, and run the SDS-PAGE gel (4%-12%). After running the gel, transferred to the PVDF membrane and sealed with 5% skimmed milk powder at room temperature for 1 hour. The membrane was placed in the primary antibody diluted with 5% skimmed milk powder and shook slowly overnight at 4°C. After incubation with primary antibodies, the membrane was washed 3 times using a TBST shaker; added secondary antibodies diluted with 5% skimmed milk powder corresponding to the primary antibodies, and shook slowly at room temperature for 1 hour. After incubation with secondary antibodies, the membrane was washed 3 times using the TBST shaker again. The PVDF membrane was flatted in the cassette, the strip was evenly infiltrated with ECL developer solution, and placed in ChemDoc XRS+ gel imager for taking pictures. The intensity of protein bands was analyzed quantitatively using ImageJ software. The results are shown in Figure 1, Figure 2, Figure 3 and Figure 4, and **Test Example** 4.

It can be seen that the conjugate (or TED molecules) of the present invention exhibit concentration-dependent degradation activity against the target protein.

### Test Example 3 In vitro kinase activity test

The compound, enzyme, substrate and ATP were diluted using 1x reaction buffer to the desired concentration. 1 µL of compounds with different concentrations, 2 µL of enzyme, and 2 µL of substrate/ATP mixed solution were added to the 384-well plate, and incubated for 1 hour at room temperature. Then 5 µL of ADP1-Glo^{™} reagent was added to each well and incubated at room temperature for 40 minutes. Finally, 10 µL of detection reagents were added, incubated at room temperature for 30 minutes, and chemiluminescence signals were detected using Envision. The results are shown in **Test Example 4.**

It can be seen that the TED molecule synthesized and prepared in the present invention exhibits strong cell proliferation inhibitory activity in a variety of tumor cell lines and has the prospect of becoming an antitumor drug.

### Test Example 4

The activity of part of the compounds (or conjugates) in Table A1 was tested according to the method of above-mentioned test examples, and the results are summarized below or as shown in Table 2.

### (4.1) Inhibitory activity IC50 of tested compounds on MV4;11 cells

**Compounds with 0 nM<IC50≤100nM are as follows:** 928, 893, 884, 888, 932, 930, 883, 889, 912, 927, 864, 900, 903, 899, 890, 882, 892, 920, 913, 885, 886, 866, 924, 894, 865, 911, 908, 923, 914, 907, 921, 917, 867, 916, 895, 909, 910, 919, 897, 926, 905, 915, 933, 934, 918, 901, 906, 874, 898, 873, 931, 925, 922, 869, 904, 876, 857.

**Compounds with 100nM<IC50≤1000nM are as follows:** 868, 863, 881, 891, 870, 855, 856, 854, 851, 859, 872, 871.

**The compounds with IC50>1000nM are as follows:** 860, 850, 852, 853, 858.

### (4.2) Inhibitory activity IC50 of tested compounds on Hela cells

**Compounds with 0 nM<IC50≤1000nM are as follows:** 883, 889, 892, 884, 888, 893.

### (4.3) Inhibitory activity IC50 of tested compounds on HL-60 cells

**Compounds with 0 nM<IC50≤1000nM are** as follows:889, 900, 883, 884, 899, 893, 892, 890, 888, 903, 902, 897, 895, 874, 905, 901, 891, 904.

### (4.4) Inhibitory activity IC50 of tested compounds on Daudi cells

**Compounds with IC50≤100nM are as follows:** 928, 883, 884, 889, 864, 900, 930, 932, 865, 912, 927, 890, 902, 913, 899, 920, 911, 926, 924, 909, 903, 908, 910, 893, 916, 866, 923, 888, 914, 907, 917, 918, 867, 919, 915.

**Compounds with 100nM<IC50≤1000nM are as follows:** 906, 886, 874, 892, 882, 933, 922, 921, 897, 895, 925, 885, 931, 873, 863, 857, 894, 876, 869, 868, 896.

**Compounds with IC50>1000nM are as follows:** 898, 881, 870, 855, 856, 854, 851, 859, 872, 871, 850, 852, 853, 858.

### (4.5) Inhibitory activity IC50 of tested compounds on NCIH82 cells

**Compounds with 0nM<IC50≤1000nM are as follows:** 864, 865, 889, 928, 866, 884, 883, 932, 910, 902, 873, 909, 927, 916, 900, 863, 893, 930, 912, 914, 874, 891, 897, 920, 913, 926, 908, 903, 915, 923, 911, 924, 890, 906, 925, 899, 867, 917, 892, 918, 933, 895, 919, 901, 907, 922, 931, 888, 857, 868, 872, 882, 869, 871.

**Compounds with IC50>1000nM are as follows:** 886, 921, 885, 894, 876, 896, 898, 881, 870, 855, 856, 854, 851, 859, 850, 852, 853, 858, 904, 905.

### (4.6) Inhibitory activity IC50 of tested compounds on HT-29 cells

**Compounds with 0nM<IC50≤300nM are as follows:** 864, 865, 928, 889, 902, 866, 910, 932, 914, 930, 900, 923, 893, 925, 927, 916, 909, 873, 924, 899, 897.

**Compounds with 300nM<IC50≤1000nM are as follows:** 911, 883, 913, 874, 912, 903, 892, 891, 867, 884, 906, 920, 908, 926, 895, 917, 915, 918, 919, 882, 922, 890, 907, 933, 888, 901.

**Compounds with IC50>1000nM are** as **follows****:** 863, 931, 857, 868, 872, 869, 871, 886, 885, 921, 894, 876, 896, 898, 881, 870, 855, 854, 851, 859, 850, 852, 853, 858, 904, 905.

### (4.7) Inhibitory activity IC50 of tested compounds on U-87MG cells

**Compounds with 0nM<IC50≤100nM are as follows:** 932, 864, 900, 927, 930, 889, 865, 902, 884, 883, 928, 909, 912, 899, 892, 913, 893, 882, 920, 911, 903, 886.

**Compounds with 100nM<IC50≤500nM are as follows:** 895, 914, 916, 890, 897, 926, 910, 924, 906, 918, 907, 908, 917, 873, 874, 888, 915, 923, 919, 933, 901, 869, 876, 894, 885, 925, 866, 922, 898, 891, 921, 905.

**500nM<IC50≤1000nM:** 896, 868, 867, 931, 857, 904.

**IC50>1000nM:** 863, 872, 871, 881, 870, 855, 856, 854, 851, 859, 850, 852, 853, 858.

### (4.8) Inhibitory activity IC50 of tested compounds on MDA-MB-231 cells

**Compounds with 0nM<IC50≤500nM are as follows:** 883, 884, 890, 893, 888, 889, 892.

### (4.9) Inhibitory activity IC50 of tested compounds on THP-1 cells

**Compounds with 0nM<IC50≤1000nM** are as **follows:** 883, 884, 888, 874, 889.

### (4.10) Inhibitory activity IC50 of tested compounds on MOL4-4 cells

**0nM<IC50≤100nM:** 883, 884, 888, 889.

### (4.11) Inhibitory activity IC50 of tested compounds on HUVEC cells

**0nM<EC50≤1000nM are as follows:** 889, 928, 930, 932, 883, 884, 927, 888, 893, 892, 920, 890, 924, 933, 923, 926, 919, 921, 922, 925.

### (4.12) Degradation activity DC50 on PLK1 of tested compounds in MV4;11 cells

**Compounds with DC50<100nM are as follows:** 869, 874, 883, 884, 889, 912.

### (4.13) Degradation activity DC50 on BRD4 of tested compounds (part) in MV4;11 cells

**Compounds with DC50<100nM are as follows:** 869, 874, 883, 884, 889, 912, 928.

### (4.14) Degradation activity DC50 on PLK1 of tested compounds in TMD-8 cells

**Compounds with DC50<100nM are as follows:883,** 884.

### (4.15) Degradation activity DC50 on BRD4 of tested compounds in TMD-8 cells

**Compounds with DC50<100nM are as follows:**883, 884.

**Table1 Inhibitory activity IC50 of tested compounds on MV4;11, Daudi, MDA-MB-231, PC-3 and NCI-H82**

| In table, A≤10 nM, 10 nM<B≤100 nM, 100 nM<C≤1000 nM, D> 1000 nM, "-" represent not tested | | | | | | | | | | | |
|---|---|---|---|---|---|---|---|---|---|---|---|
| UB No. | MV4; 11 (nM) | Daudi | MDA-MB-231 | PC-3 | NCI-H82 | UB No. | MV4; 11 (nM) | Daudi | MDA-MB-231 | PC-3 | NCI-H82 |
| UB-180934 | B | B | C | - | - | UB-181060 | B | - | D | D | B |
| UB-180935 | C | - | - | - | - | UB-181061 | A | - | C | D | C |
| UB-180936 | B | B | C | D | A | UB-181062 | B | - | D | D | C |
| UB-180937 | A | B | B | B | - | UB-181063 | A | - | C | C | - |
| UB-180938 | B | B | - | - | - | UB-181064 | D | - | D | D | D |
| UB-180939 | B | C | D | - | - | UB-181065 | B | - | D | D | C |
| UB-180940 | C | - | - | - | - | UB-181066 | B | - | D | D | D |
| UB-180941 | B | - | - | - | - | UB-181067 | B | - | D | D | C |
| UB-180942 | C | D | - | - | D | UB-181068 | B | - | D | D | D |
| UB-180944 | C | D | - | - | D | UB-181069 | B | - | D | D | D |
| UB-180945 | C | D | - | - | D | UB-181070 | B | - | C | D | C |
| UB-180946 | C | D | - | - | C | UB-181071 | C | - | D | D | D |
| UB-180947 | B | C | - | - | D | UB-181072 | C | - | D | D | D |
| UB-180948 | B | D | - | - | D | UB-181073 | A | - | D | D | D |
| UB-180949 | A | B | B | - | - | UB-181074 | B | - | D | D | D |
| UB-180950 | A | C | D | - | C | UB-181075 | D | - | D | D | C |
| UB-180951 | C | D | - | - | C | UB-181076 | A | B | D | D | C |
| UB-180952 | D | D | - | - | D | UB-181077 | C | - | D | D | D |
| UB-180953 | C | D | - | - | D | UB-181078 | A | B | C | B | C |
| UB-180954 | C | D | - | - | D | UB-181079 | A | - | D | D | C |
| UB-180955 | C | D | - | - | D | UB-181080 | A | B | C | B | C |
| UB-180956 | C | D | - | - | D | UB-181081 | B | - | D | D | D |
| UB-180957 | B | C | D | D | C | UB-181082 | B | - | D | D | D |
| UB-180958 | C | D | - | - | D | UB-181083 | B | - | D | D | D |
| UB-180959 | C | D | - | - | D | UB-181084 | A | - | C | C | C |
| UB-180960 | C | D | - | - | D | UB-181086 | B | - | D | D | C |
| UB-180961 | A | A | A | A | - | UB-181087 | A | - | D | C | C |
| UB-180962 | C | D | - | - | D | UB-181088 | A | - | D | D | D |
| UB-180963 | B | D | - | - | D | UB-181089 | B | - | B | B | - |
| UB-180964 | C | D | - | - | D | UB-181090 | B | - | D | D | D |
| UB-180965 | C | D | - | - | C | UB-181091 | A | - | C | D | C |
| UB-180966 | D | D | - | - | D | UB-181092 | B | - | D | D | D |
| UB-180967 | C | C | - | - | D | UB-181093 | B | - | D | D | D |
| UB-180968 | A | C | - | - | - | UB-181094 | A | - | D | D | C |
| UB-180969 | B | C | - | - | - | UB-181095 | A | - | D | C | C |
| UB-180970 | C | D | - | - | D | UB-181096 | A | - | C | C | C |
| UB-180971 | C | D | - | - | D | UB-181097 | C | - | D | D | D |
| UB-180972 | B | C | - | - | C | UB-181098 | A | - | D | C | B |
| UB-180973 | C | D | - | - | D | UB-181099 | A | B | B | B | - |
| UB-180974 | C | D | - | - | D | UB-181100 | A | B | C | B | B |
| UB-180975 | D | D | - | - | D | UB-181101 | A | B | C | C | B |
| UB-180976 | C | D | - | - | D | UB-181102 | B | C | D | D | C |
| UB-180977 | A | B | A | - | - | UB-181103 | A | B | C | B | B |
| UB-180978 | A | B | A | - | - | UB-181104 | A | B | C | B | B |
| UB-180979-2 | B | B | C | - | - | UB-181108 | A | B | B | B | - |
| UB-180980 | A | C | D | - | D | UB-181109 | B | - | D | D | C |
| UB-180981 | D | D | - | - | D | UB-181110 | B | - | D | D | C |
| UB-180982 | C | D | - | - | D | UB-181111 | A | - | D | C | B |
| UB-180983 | B | C | - | - | C | UB-181112 | A | - | D | D | C |
| UB-180984 | A | B | - | - | - | UB-181113 | A | B | B | B | - |
| UB-180985 | A | A | - | - | - | UB-181114 | A | - | D | D | C |
| UB-180986 | B | C | D | - | C | UB-181115 | A | B | C | B | B |
| UB-180987 | D | D | - | - | D | UB-181116 | C | - | D | D | D |
| UB-180988 | D | D | - | - | D | UB-181117 | B | - | D | D | D |
| UB-180989 | D | D | - | - | D | UB-181118 | A | C | B | B | - |
| UB-180990 | C | D | - | - | D | UB-181119 | A | - | C | B | C |
| UB-180991 | C | D | - | - | D | UB-181120 | A | - | C | B | C |
| UB-180992 | A | B | B | - | - | UB-181121 | A | C | C | C | C |
| UB-180993 | B | B | B | - | - | UB-181122 | A | C | C | C | C |
| UB-180994 | C | D | - | - | D | UB-181123 | A | - | C | B | C |
| UB-180995 | D | D | - | - | D | UB-181124 | B | - | D | B | D |
| UB-180996 | D | D | - | - | D | UB-181125 | B | - | D | C | D |
| UB-180997 | D | D | - | - | D | UB-181126 | A | - | C | C | - |
| UB-180998 | A | B | B | - | - | UB-181127 | A | B | B | B | B |
| UB-180999 | A | B | B | - | - | UB-181128 | A | - | D | D | D |
| UB-181000 | D | D | - | - | D | UB-181129 | B | - | D | D | C |
| UB-181001 | D | D | - | - | D | UB-181130 | A | - | C | C | - |
| UB-181002 | D | D | - | - | D | UB-181131 | B | C | D | D | B |
| UB-181003 | C | D | - | - | D | UB-181132 | A | - | C | C | C |
| UB-181004 | A | B | B | - | - | UB-181133 | B | - | D | C | C |
| UB-181005 | D | D | - | - | D | UB-181134 | B | - | D | D | D |
| UB-181006 | D | D | - | - | D | UB-181135 | B | - | D | D | D |
| UB-181007 | C | C | - | - | D | UB-181136 | D | - | D | D | - |
| UB-181008 | C | C | - | - | D | UB-181137 | A | C | C | B | C |
| UB-181009 | C | D | - | - | D | UB-181138 | A | C | C | B | B |
| UB-181010 | C | D | - | - | D | UB-181139 | A | - | C | C | C |
| UB-181011 | C | B | - | - | D | UB-181140 | A | - | C | C | C |
| UB-181012 | C | D | - | - | D | UB-181141 | A | C | C | C | C |
| UB-181013 | C | D | - | - | D | UB-181142 | A | C | C | C | C |
| UB-181014 | C | D | - | - | D | UB-181143 | B | C | C | C | - |
| UB-181015 | C | D | - | - | D | UB-181144 | A | B | B | B | - |
| UB-181016 | C | D | - | - | D | UB-181145 | A | A | A | A | - |
| UB-181017 | C | D | - | - | D | UB-181146 | A | - | D | D | D |
| UB-181018 | D | D | - | - | D | UB-181147 | A | - | C | C | C |
| UB-181019 | C | D | - | - | D | UB-181149 | C | C | C | C | - |
| UB-181020 | C | D | - | - | D | UB-181150 | A | B | C | B | - |
| UB-181021 | B | C | C | C | - | UB-181151 | A | - | D | D | C |
| UB-181022 | B | C | C | C | - | UB-181152 | A | - | C | C | C |
| UB-181023 | B | C | C | C | - | UB-181153 | B | - | C | C | - |
| UB-181024 | B | C | C | C | - | UB-181154 | A | C | C | B | - |
| UB-181025 | D | D | D | D | - | UB-181168 | A | B | C | C | C |
| UB-181026 | D | - | D | D | - | UB-181169 | B | C | D | B | C |
| UB-181027 | D | D | - | - | D | UB-181170 | A | B | B | B | - |
| UB-181028 | C | D | - | - | D | UB-181171 | B | D | D | D | - |
| UB-181029 | D | D | - | - | D | UB-181172 | A | B | C | C | C |
| UB-181030 | D | D | - | - | D | UB-181173 | B | D | D | B | D |
| UB-181031 | D | - | D | D | D | UB-181174 | B | B | D | B | D |
| UB-181032 | C | - | D | D | D | UB-181175 | A | B | B | B | - |
| UB-181033 | C | - | D | D | D | UB-181176 | A | B | B | B | - |
| UB-181034 | C | - | D | D | D | UB-181177 | A | B | A | A | - |
| UB-181035 | C | - | D | D | D | UB-181178 | A | A | B | B | - |
| UB-181036 | D | - | D | D | D | UB-181179 | A | B | B | B | - |
| UB-181037 | D | - | D | D | D | UB-181181 | B | D | D | B | C |
| UB-181038 | D | - | D | D | D | UB-181182 | A | C | C | C | B |
| UB-181039 | B | - | D | D | C | UB-181183 | A | B | C | B | B |
| UB-181040 | B | - | D | D | D | UB-181183 | A | B | B | B | B |
| UB-181041 | D | - | D | D | D | UB-181184 | A | B | C | B | B |
| UB-181042 | B | - | D | D | C | UB-181185 | A | B | B | B | B |
| UB-181043 | A | - | D | D | C | UB-181186 | A | B | C | A | B |
| UB-181044 | B | - | D | D | C | UB-181187 | B | D | D | B | C |
| UB-181045 | A | - | A | C | - | UB-181188 | B | B | D | B | B |
| UB-181046 | A | - | D | D | C | UB-181189 | A | A | B | A | B |
| UB-181047 | B | - | D | D | C | UB-181190 | A | B | B | B | B |
| UB-181048 | A | B | D | D | C | UB-181193 | B | C | C | - | - |
| UB-181049 | B | B | D | C | B | UB-181194 | A | D | D | D | D |
| UB-181050 | C | - | D | D | C | UB-181195 | D | D | D | D | D |
| UB-181051 | B | - | D | D | D | UB-181198 | A | B | B | B | B |
| UB-181052 | B | - | C | A | - | UB-181199 | - | B | - | B | A |
| UB-181053 | B | - | D | D | C | UB-181203 | - | C | - | C | C |
| UB-181054 | A | B | D | D | C | UB-181205 | - | C | - | C | C |
| UB-181055 | B | - | D | D | C | UB-181206 | - | C | - | - | C |
| UB-181056 | B | - | D | D | C | UB-181207 | - | D | - | - | D |
| UB-181057 | A | - | D | D | C | UB-181208 | - | B | - | - | C |
| UB-181058 | B | - | D | D | C | | | | | | |

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A conjugate of formula I, and the pharmaceutically acceptable salts thereof, wherein
R_{T}-L1-R_{E3} (I)
wherein
(a) the R_{E3} is a moiety of E3 Ligase Ligand;
(b) the R_{T} is a moiety of target molecule;
(c) the L1 is a linker connecting the moieties of R_{E3} and R_{T}, and L1 is shown in formula II;
-W¹-L2-W²- (II)
wherein
W¹ and W² are each independently -(W)ₛ-;
W is each independently a divalent group selected from the group consisting of null, - C(R^{b})₂-, -O-, -S-, -N(R^{a})-, -C(=O)-, -SO₂-, -SO-, -PO₃-, -C(R^{b})=C(R^{b})-, -C≡C-, NR, substituted or unsubstituted C3-8 cycloalkyl, substituted or unsubstituted 4 to 10 membered heterocycloalkyl, substituted or unsubstituted C6-10 aryl, and substituted or unsubstituted 5 to 10 membered heteroaryl;
s = 0, 1, 2, 3, or 4;
L2 is shown in formula III,
-(M^{L})ₒ- (III)
wherein,
M^{L} is each independently M, M^{T} or M^{N};
wherein,
o is an integer of 5 to 50;
M is each independently a divalent group selected from the group consisting of -C(R^{b})₂-, - O-, -S-, -N(R^{a})-, -C(=O)-, -SO₂-, -SO-, -PO₃-, -C(R^{b})=C(R^{b})-, -C≡C-, substituted or unsubstituted C₃₋₈ cycloalkyl, substituted or unsubstituted 4 to 10 membered heterocycloalkyl, substituted or unsubstituted C₆₋₁₀ aryl, substituted or unsubstituted 5 to 10 membered heteroaryl, and amino acid residue;
M^{N} is each independently a divalent group selected from the group consisting of -N(R')-, - N(4 to 10 membered heterocycloalkyl containing N(R') as ring atom)-, 4 to 10 membered heterocycloalkyl containing N(R') as ring atom, and -C(R^{b})₂- substituted with at least one - N(R^{b})R' (preferably, -NHR'), C₃₋₈ cycloalkyl, 4 to 10 membered heterocycloalkyl, C₆₋₁₀ aryl, and 5 to 10 membered heteroaryl;
M^{T} is each independently divalent group selected from the group consisting of -N(R")-, - N(4 to 10 membered heterocycloalkyl containing N(R") as ring atom)-, 4 to 10 membered heterocycloalkyl containing N(R") as ring atom, and -C(R^{b})₂- substituted with at least one - N(R^{b})R" (preferably, -NHR"), C₃₋₈ cycloalkyl, 4 to 10 membered heterocycloalkyl, C₆₋₁₀ aryl, and 5 to 10 membered heteroaryl;
R is R' or R";
R' is each independently selected from the group consisting of H, C₁₋₆ alkyl, OH, SH, - COO-C₁₋₆ alkyl, -OC(O)-C₁₋₆ alkyl, and amino protecting group;
R" is -W³-L3-W⁴-(R_{P})_{q};
W³ and W⁴ are each independently -(W)ₛ-; and the definitions of W and s are the same as definitions used in W¹ and W²;
L3 is a divalent linker group;
R_{P} is a polypeptide element or target molecule T;
q is >0 (preferably, m is 0.1-10, more preferably, 0.2-5);
R^{a} is each independently selected from the group consisting of H, OH, SH, substituted or unsubstituted C₁₋₆ alkyl, amino protecting group, 4 to 10 membered heterocycloalkyl containing N(R^{c}) as ring atom;
R^{b} is each independently selected from the group consisting of H, halogen, OH, SH, substituted or unsubstituted C₁₋₆ alkyl, substituted or unsubstituted C₂₋₆ alkenyl, substituted or unsubstituted C₂₋₆ alkynyl, substituted or unsubstituted C₁₋₆ alkoxy, substituted or unsubstituted C₁₋₆ alkanoyl (-C(O)-C₁₋₆ alkyl), carboxyl, -COO-C₁₋₆ alkyl, and -OC(O)-C₁₋₆ alkyl; or, two R^{b} on the same atom together with the carbon to which they are attached form substituted or unsubstituted C₃₋₈ cycloalkyl, substituted or unsubstituted 4 to 10 membered heterocycloalkyl.
R^{c} is each independently selected from the group consisting of H, OH, SH, substituted or unsubstituted C₁₋₆ alkyl, and amino protecting group;
unless otherwise specified, the substituted means that one or more (such 1, 2, or 3) hydrogen atoms in the group are substituted with substituents selected from the group consisting of halogen (preferably, F, Cl, Br or I), cyano(CN), oxo (=O), thio (=S), C₁₋₆ alkyl, C₁₋₆ haloalkyl, C₂₋₆ alkenyl, C₂₋₆ alkynyl, C₁₋₆ alkoxy, C₁₋₆ alkanoyl (C₁₋₆ alkyl-C(O)-), -COO-C₁₋₆ alkyl, -OC(O)-C₁₋₆ alkyl, NH₂, NH(C₁₋₆ alkyl), and N(C₁₋₆ alkyl)₂;
and the conjugate is not those specific compounds described in Table B1-11 and the specific compounds described in Table D in PCT/CN2019/110225.

2. The conjugate of Claim 1, wherein L2 is L7, and L7 is shown in formula IIIb;
-(M)ₒ₁-(M^{T})-(M)ₒ₂- (IIIb)
wherein M, and M^{T} are defined as above;
o1 and o2 are each independently integers of 1 to 50, and 4≤o1+o2≤49.

3. The conjugate of Claim 1, wherein the conjugate is shown in formula 1b-1, 1b-2, 1b-3, 2b or 3b;
R_{T}-W¹-L7-W^{b}-C≡C-R_{E3} (1b-1);
R_{T}-W¹-L7-CO-R_{E3} (1b-2);
R_{T}-W¹-L7-CONH-R_{E3} (1b-3);
R_{T}-W^{a}-Cr¹-W^{a}-Cr²-L7-W²-R_{E3} (2b)
R_{T}-Ar1-L7-W²-R_{E3} (3b)
wherein,
Ar1 is 5 or 6 membered heteroaryl containing nitrogen atom;
Cr¹ is null, or C₄₋₇ cycloalkyl unsubstituted or substituted with C₁₋₄ alkyl, or 4 to 6 membered heterocyclyl unsubstituted or substituted with C₁₋₄ alkyl;
Cr² is 4 to 6 membered heterocyclyl containing nitrogen unsubstituted or substituted with C₁₋₄ alkyl, and at least one of nitrogen heteroatom in Cr² is attached with L7;
the definition of W^{a} and W^{b} is the same as W; and W, W¹, W², R_{T}, R_{E3} and L7 are defined as in formula I.

4. The conjugate of Claim 1, wherein the conjugate comprises one or more features selected from the group consisted of:
a. when the heterocycloalkyl is a divalent group, the 4 to 10 membered heterocycloalkyl includes 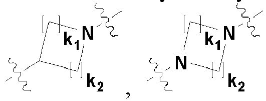 ; wherein k1 and k2 are each independently 1 or 2; and/or
b. when the cycloalkyl is a divalent group, the cycloalkyl includes 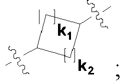 wherein k1 and k2 are each independently 0 1 2 or 3; and/or
c. when the heteroaryl is a divalent group, the heteroaryl is 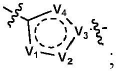 wherein V₁, V₂ and V₄ are each independently selected from -O-, -S-, -N=, -NH-, -CH=, -CH₂-; V₃ is selected from the group consisting of -N=, and -CH=.

5. The conjugate of Claim 1, wherein the conjugate is a conjugate selected from Group 1, Group 2 and Group 3; wherein Rand R¹ are R".

6. The conjugate of Claim 1, wherein L3 is -(M^{a})ₚ-; wherein M^{a} is defined as M, p is an integer of 1 to 50.

7. A pharmaceutical composition comprisingthe conjugate of Claim 1 and pharmaceutically acceptable carriers.

8. A use of the conjugate of Claim 1 in preparation of a drug for the treatment or prevention of diseases associated with an excess of a target protein.

9. A method for reducing the content of target proteins in a cell, wherein the cell is contacted with the conjugate of Claim 1, thereby reducing the content of the target proteins in the cell.

10. A TED compound or the pharmaceutically acceptable salts thereof, wherein the TED compound is shown in formula VI;
R_{T}W¹-(M^{L})ₒ-W²-R_{E3} (VI)
wherein,
M^{L} is each independently M or M^{N};
M, M^{N}, R_{E3}, R_{T}, W¹, W² and subscript o are defined as in formula I.

11. The TED compound of Claim 10, wherein the TED compound is a compound selected from Table A1, A2 , A3, Group 1a, Group 2a and Group 3a.
